# EUROPEAN PATENT APPLICATION

(11) **EP 1 538 138 A1**
(43) Date of publication of application: **08.06.2005**
(21) Application number: 03792835.5
(22) Date of filing: 25.08.2003
(51) Int. Cl.: C07C 1/00

(54) **SUBSTITUTED BENZANILIDE COMPOUND AND PEST CONTROL AGENT**

(30) Priority: 26.08.2002 JP 2002244619; 26.09.2002 JP 2002281294; 28.11.2002 JP 2002344987; 25.03.2003 JP 2003083371; 26.06.2003 JP 2003182013
(71) Applicant: NISSAN CHEMICAL INDUSTRIES, LIMITED, Chiyoda-ku Tokyo 101-0054 (JP)
(72) Inventor: MITA, Takeshi NISSAN CHEMICAL INDUSTRIES, LTD., Funabashi-shi, Chiba 274-8507 (JP); KUDO, Yoshihiro NISSAN CHEMICAL INDUSTRIES, LTD., Funabashi-shi, Chiba 274-8507 (JP); MIZUKOSHI, T. NISSAN CHEMICAL INDUSTRIES, LTD., Funabashi-shi, Chiba 274-8507 (JP); HOTTA, H. NISSAN CHEMICAL INDUSTRIES, LTD., Funabashi-shi, Chiba 274-8507 (JP); MAEDA, Kazushige NISSAN CHEMICAL INDUSTRIES, LTD., Funabashi-shi, Chiba 274-8507 (JP); TAKII, Shinji NISSAN CHEMICAL INDUSTRIES, LTD., Funabashi-shi, Chiba 274-8507 (JP)
(74) Representative: Murphy, Colm Damien
(86) International application number: PCT/JP2003/010708
(87) International publication number: WO 2004/018410

(57) **Abstract**

The present invention is to provide a novel agricultural chemical, in particular an insecticide or an acaricide, and relates to a substituted benzanilide compound represented by the formula (1): 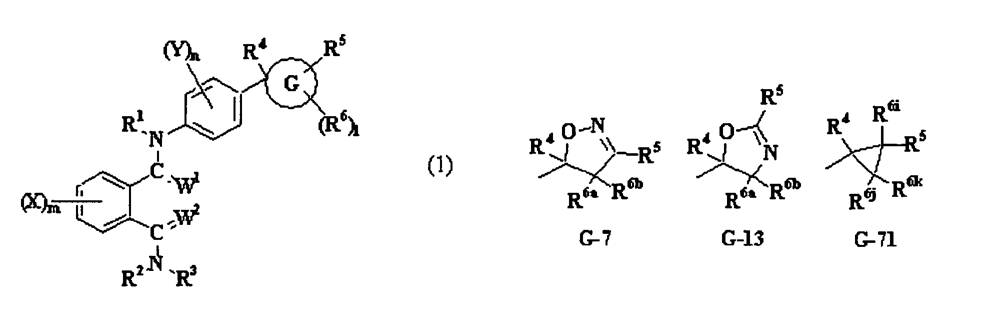 [wherein G represents a ring such as G-7, G-13 and G-71, etc., W¹ and W² each independently represent oxygen atom or sulfur atom, X represents halogen atom, etc., Y represents C₁ to C₆ alkyl, etc., R¹, R² and R³ each independently represent hydrogen atom, a C₁ to C₁₂ alkyl or a C₁ to C₆ alkylthio(C₁ to C₆) alkyl, etc., R⁴, R⁵, R^{6a} and R^{6b} each independently represent hydrogen atom, a C₁ to C₆ alkyl, a C₁ to C₆ haloalkyl or phenyl which may be substituted by (Z²)ₚ₁, etc., R⁶ⁱ, R^{6j} and R^{6k} each independently represent hydrogen atom or halogen atom, etc., Z² represents halogen atom, a C₁ to C₆ haloalkoxy or a C₁ to C₆ alkylsulfonyl, etc., m and n each independently represent integer of 0 to 4, and p1 represents integer of 1 to 5.] or a salt thereof, and a noxious organism controlling agent containing these.

## Description

### TECHNICAL FIELD

The present invention relates to a novel substituted benzanilide compound and a salt thereof, and a noxious organism controlling agent containing said compound as an effective ingredient. The noxious organism controlling agent in the present invention means an noxious organism controlling agent which is to control noxious arthropods in the agricultural and horticultural fields or in the farming, sanitation fields (a medicine for animals or an insecticide for domestic use or for business use). Also, the agricultural chemicals according to the present invention means an insecticide and acaricide , a nematocide, a herbicide and a fungicide in the agricultural and horticultural fields.

### BACKGROUND ART

It has heretofore been known that specific substituted benzanilide derivatives have cytokine production-inhibiting activity, vasopressin antagonistic activity and the like and have been used as a medicine (e.g., see Patent literature 1 to 3.). Also, it has been known that specific substituted benzanilide derivatives have insecticidal activity (e.g., see Patent literature 4 to 10.). However, it has been never disclosed about the substituted benzanilide compounds according to the present invention.
Patent literature 1 WO 98/024771 pamphlet
Patent literature 2 WO 99/051580 pamphlet
Patent literature 3 JP-A-2002-249473 publication
Patent literature 4 EP-A-0919542 specification
Patent literature 5 EP-A-1006107 specification
Patent literature 6 WO 01/021576 pamphlet
Patent literature 7 WO 01/046124 pamphlet
Patent literature 8 JP-A-2001-335559 publication
Patent literature 9 WO 02/062807 pamphlet
Patent literature 10 WO 02/094765 pamphlet

### DISCLOSURE OF THE INVENTION

### Problems to be solved by the invention

Due to use of noxious organism controlling agents such as an insecticide and a fungicide for a long period of time, noxious insects have obtained resistivity thereto in recent years, so that prevention thereof by the conventionally used insecticides or fungicides becomes difficult. Also, a part of the known noxious organism controlling agents has high toxicity, or some of them are putting an ecological system in confusion due to their long residual activity. Under such a circumstance, it has been usually expected to develop a novel noxious organism controlling agent which has low toxicity and low remaining property.

### Means to solve the problems

The present inventors have conducted earnest studies to solve the above-mentioned problems, and as a result, they have found that the novel substituted benzanilide compound represented by the following formula (1) according to the present invention is an extremely useful compound which has an excellent noxious organism controlling activity, in particular, an insecticidal and acaricidal activity, and causing substantially no bad effect against non-target organisms such as mammals, fishes and useful insects, whereby they have accomplished the present invention.

That is, the present invention relates to the following [1] to [19].

[1] A substituted benzanilide compound represented by the formula (1): 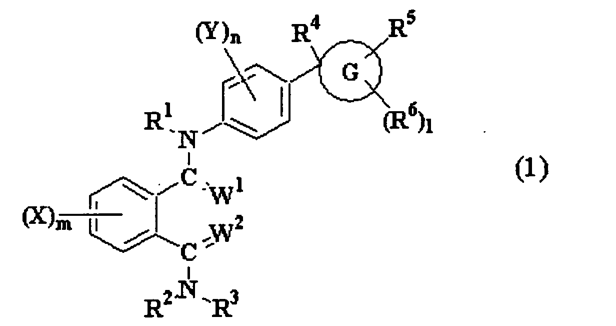 [wherein G represents a 5-membered or 6-membered non-aromatic heterocyclic ring containing at least one atom selected from an oxygen atom, a sulfur atom and a nitrogen atom, and having at least one double bond in the ring, a 5-membered or 6-membered saturated heterocyclic ring containing two atoms selected from an oxygen atom, a sulfur atom and a nitrogen atom or a 3-membered to 6-membered cycloalkyl ring,
W¹ and W² each independently represent an oxygen atom or a sulfur atom,
X represents a halogen atom, cyano, nitro, azide, -SCN, -SF₅, a C₁ to C₆ alkyl, a (C₁ to C₆)alkyl which may be optionally substituted by R⁷, a C₃ to C₈ cycloalkyl, a (C₃ to C₈)cycloalkyl which may be optionally substituted by R⁷, a C₂ to C₆ alkenyl, a (C₂ to C₆)alkenyl which may be optionally substituted by R⁷, a C₃ to C₈ cycloalkenyl, a C₃ to C₈ halocycloalkenyl, a C₂ to C₆ alkynyl, a (C₂ to C₆)alkynyl which may be optionally substituted by R⁷, -OH, -OR⁸, -OS(O)₂R⁸, -SH, -S(O)ᵣR⁸, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, C(S)N(R¹⁰)R⁹, -CH=NOR¹¹, -C(R⁹)=NOR¹¹, -S(O)₂OR⁹, -S(O)₂NHR¹⁰, -S(O)₂N(R¹⁰)R⁹, -Si(R¹³)(R¹⁴)R¹², phenyl which may be substituted by (Z¹)ₚ₁, L or M, when m represents 2, 3 or 4, each of X's may be the same with each other or may be different from each other, further, when two Xs are adjacent to each other, the adjacent two Xs may form -CH₂CH₂CH₂-, -CH₂CH₂O-, -CH₂OCH₂-, -OCH₂O-, -CH₂CH₂S-, -CH₂SCH₂-, -CH₂CH₂N(R¹⁵)-, -CH₂N(R¹⁵)CH₂-, -CH₂CH₂CH₂CH₂-, -CH₂CH₂CH₂O-, -CH₂CH₂OCH₂-, -CH₂OCH₂O-, -OCH₂CH₂O-, -OCH₂CH₂S-, -CH₂CH=CH-, -OCH=CH-, -SCH=CH-, -N(R¹⁵)CH=CH-, -OCH=N-, -SCH=N-, -N(R¹⁵)CH=N-, -N(R¹⁵)N=CH-, -CH=CHCH=CH-, -OCH₂CH=CH-, -N=CHCH=CH-, -N=CHCH=N- or -N=CHN=CH-, so that the two Xs form a 5-membered ring or 6-membered ring with the carbon atoms to which the two Xs are bonded, and at this time, the hydrogen atom(s) bonded to each of the carbon atom(s) which form(s) the ring which may be optionally substituted by Z¹, and further, when it is substituted by two or more Z¹s simultaneously, each of Z¹s may be the same with each other or may be different from each other,
and, when G represents a 5-membered or 6-membered non-aromatic heterocyclic ring containing at least one atom selected from an oxygen atom, a sulfur atom and a nitrogen atom, and having at least one double bond in the ring, X may further represent -N(R¹⁷)R¹⁶, -N=CHOR¹² or -N=C(R⁹)OR¹²,
Y represents a halogen atom, cyano, nitro, azide, -SCN, -SF₅, a C₁ to C₆ alkyl, a (C₁ to C₆)alkyl which may be optionally substituted by R⁷, a C₃ to C₈ cycloalkyl, a (C₃ to C₈)cycloalkyl which may be optionally substituted by R⁷, -OH, -OR⁸, -OS(O)₂R⁸, -SH, -S(O)ᵣR⁸, -NH₂, a C₁ to C₆ alkylamino, a di(C₁ to C₆ alkyl)amino, -Si(R¹³)(R¹⁴)R¹², phenyl which may be substituted by (Z¹)ₚ₁, L or M, when n represents 2, 3 or 4, each of Ys may be the same with each other or may be different from each other, and further, when two Ys are adjacent to each other, the adjacent two Ys may form -CH₂CH₂CH₂-, -CH₂CH₂O-, -CH₂OCH₂-, -OCH₂O-, -CH₂CH₂S-, -CH₂SCH₂-, -SCH₂S-, -CH₂CH₂CH₂CH₂-, -CH₂CH₂CH₂O-, -CH₂CH₂OCH₂-, -CH₂OCH₂O-, -OCH₂CH₂O-, -OCH₂CH₂S-, -SCH₂CH₂S-, -OCH=N- or -SCH=N-, so that the two Ys may form a 5-membered ring or 6-membered ring with carbon atoms to which the two Ys are bonded, and at this time, the hydrogen atom(s) bonded to each of the carbon atom(s) which form(s) the ring which may be optionally substituted by Z¹, and further, when it is substituted by two or more Z¹s simultaneously, each of Z¹s may be the same with each other or may be different from each other,
R¹, R² and R³ each independently represent a hydrogen atom, cyano, a C₁ to C₁₂ alkyl, a (C₁ to C₁₂)alkyl which may be optionally substituted by R¹⁸, a C₃ to C₁₂ cycloalkyl, a (C₃ to C₁₂)cycloalkyl which may be optionally substituted by R¹⁸, a C₃ to C₁₂ alkenyl, a (C₃ to C₁₂)alkenyl which may be optionally substituted by R¹⁸, C₃ to C₁₂ a cycloalkenyl, C₃ to C₁₂ halocycloalkenyl, a C₃ to C₁₂ alkynyl, a (C₃ to C₁₂)alkynyl which may be optionally substituted by R¹⁸, -OH, a C₁ to C₈ alkoxy, a C₃ to C₈ alkenyloxy, a C₃ to C₈ haloalkenyloxy, phenoxy which may be substituted by (Z¹)ₚ₁, a phenyl(C₁ to C₄)alkoxy which may be substituted by (Z¹)ₚ₁, a C₁ to C₆ alkylthio, a C₁ to C₆ haloalkylthio, phenylthio which may be substituted by (Z¹)ₚ₁, -S(O)₂R⁹, -SN(R²⁰)R¹⁹, -S(O)₂N(R¹⁰)R⁹, -N(R²²)R²¹, -C(O)R⁹, -C(O)OR⁹, -C(O)SR⁹, -C(O)N(R¹⁰)R⁹, -C(S)OR⁹, -C(S)SR⁹, -C(S)N(R¹⁰)R⁹, phenyl which may be substituted by (Z¹)ₚ₁, L or M, or R² and R³ are combined to form a C₂ to C₆ alkylene chain, so that they may form a 3- to 7-membered ring with the nitrogen atom(s) to which they are bonded, the alkylene chain at this time may contain one oxygen atom, sulfur atom or nitrogen atom, and may be optionally substituted by a halogen atom, a C₁ to C₆ alkyl group, a C₁ to C₆ haloalkyl group, a C₁ to C₆ alkoxy group, a C₁ to C₆ alkylcarbonyl group or a C₁ to C₆ alkoxycarbonyl group,
R⁴ represents a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², -P(O)(OR²⁴)₂, phenyl which may be substituted by (Z²)ₚ₁, 1-naphthyl, 2-naphthyl, L or M,
R⁵ represents
(a). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, phenyl which may be substituted by (Z²)ₚ₁, 1-naphthyl, 2-naphthyl, L or M, when G represents a 5-membered or 6-membered non-aromatic heterocyclic ring containing at least one atom selected from an oxygen atom, a sulfur atom and a nitrogen atom, and having at least one double bond in the ring, or a 5-membered or 6-membered saturated heterocyclic ring containing two atoms selected from an oxygen atom, a sulfur atom and a nitrogen atom,
(b). cyano, a (C₁ to C₆)alkyl which may be optionally substituted by R²³, a (C₁ to C₆)-haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents cyano, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², -P(O)(OR²⁴)₂, phenyl which may be substituted by (Z²)ₚ₁, 1-naphthyl, 2-naphthyl, L or M,
R⁶ represents a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₁ to C₆ alkoxy, a C₁ to C₆ haloalkoxy, a C₁ to C₆ alkylthio, a C₁ to C₆ haloalkylthio, -S(O)₂R⁹, -C(O)R⁹, -C(O)OR⁹, -C(O)SR⁹, -C(S)OR⁹, -C(S)SR⁹, -C(O)N(R¹⁰)R⁹, -C(S)N(R¹⁰)R⁹, -P(O)(OR²⁴)₂, -P(S)(OR²⁴)₂, phenyl which may be substituted by (Z²)ₚ₁, 1-naphthyl, 2-naphthyl, L or M, when I represents an integer of 2 or more, each of R⁶s may be the same with each other or may be different from each other, and when R⁶ and R⁴ are adjacent to each other, the adjacent R⁴ and R⁶ are combined to form a C₃ to C₅ alkylene chain, so that they may form a 5 to 7-membered ring with atoms to which they are bonded, and the alkylene chain at this time may contain one oxygen atom or sulfur atom, and may be which may be optionally substituted by a C₁ to C₆ alkyl group,
L represents an aromatic heterocyclic ring represented by either one of the formula L-1 to the formula L-58, 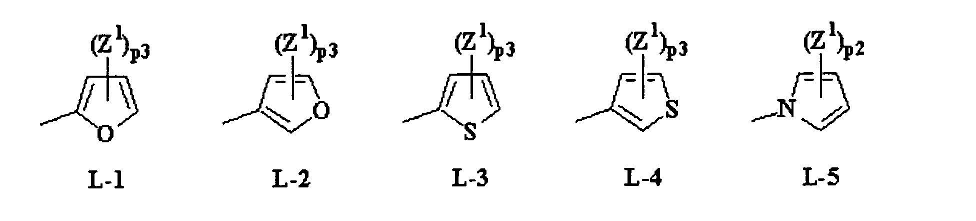 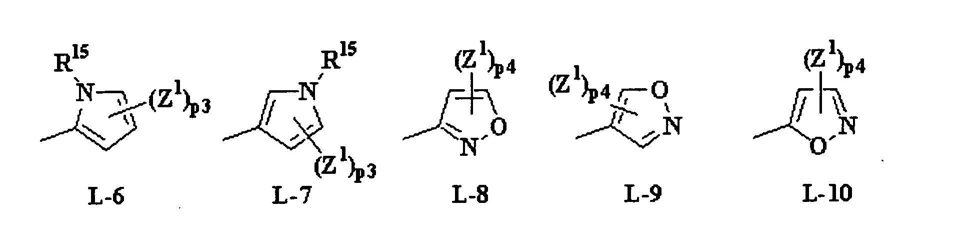 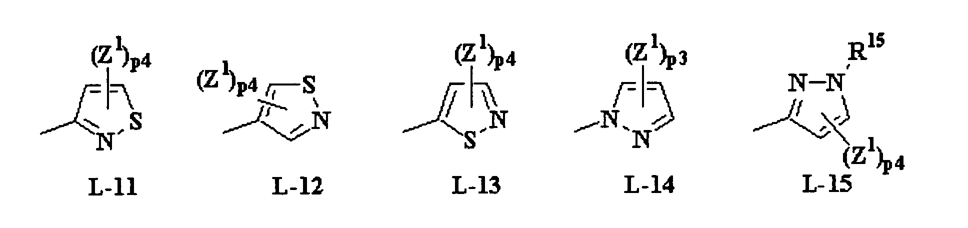 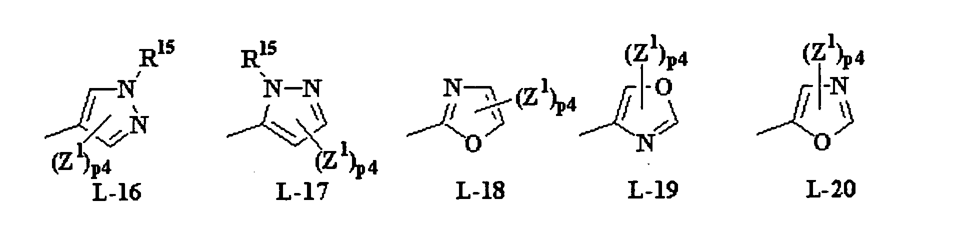 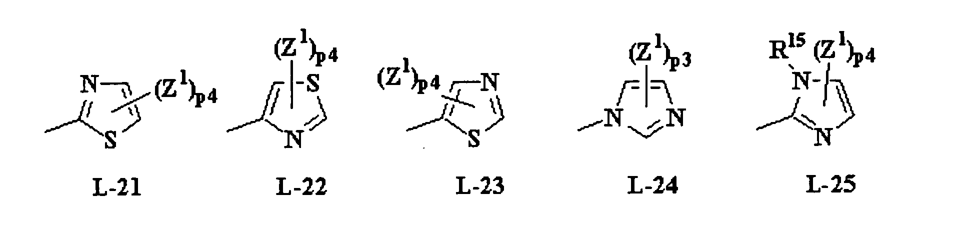 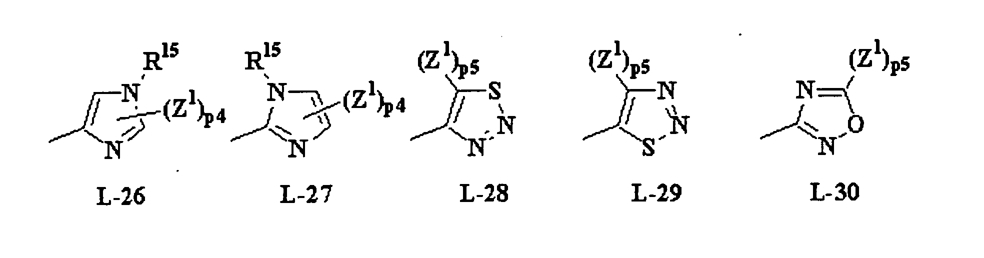 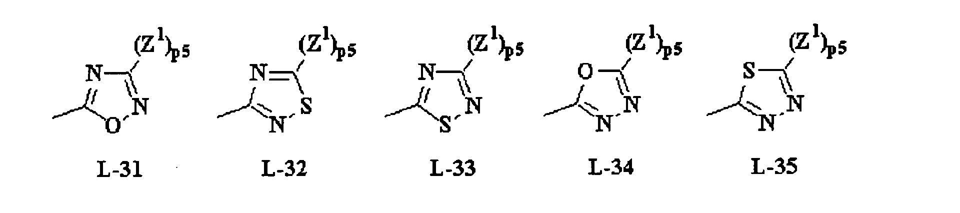 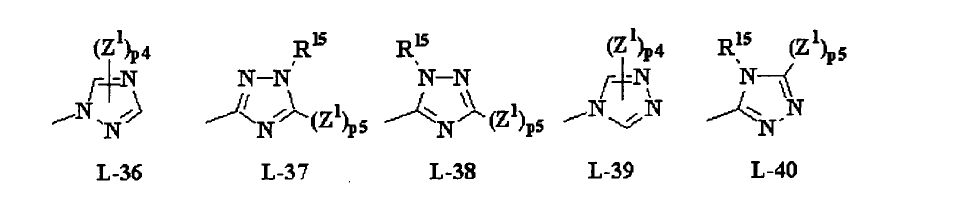 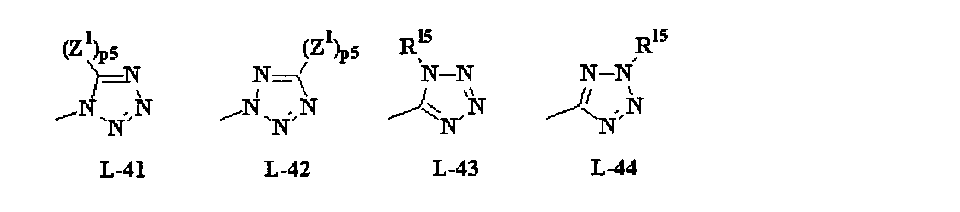 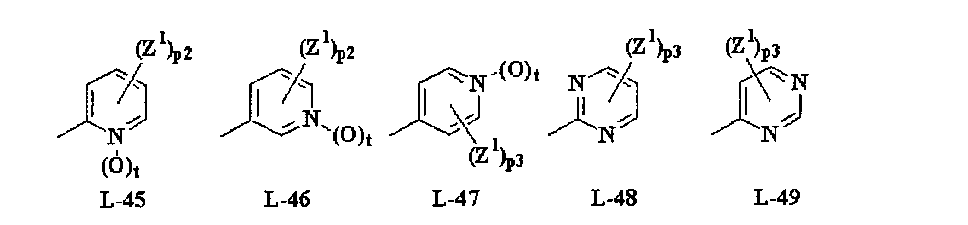 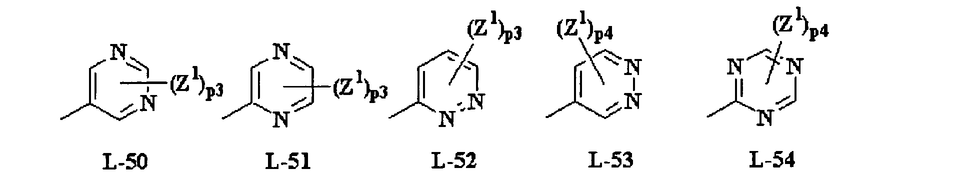 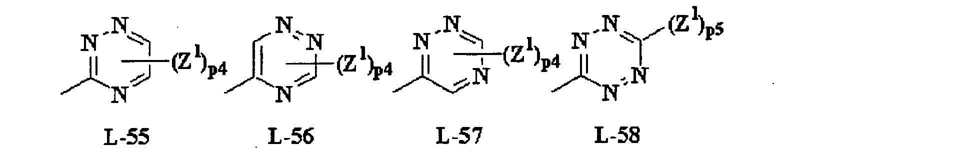 M represents a saturated heterocyclic ring represented by either one of the formula M-1 to the formula M-28, 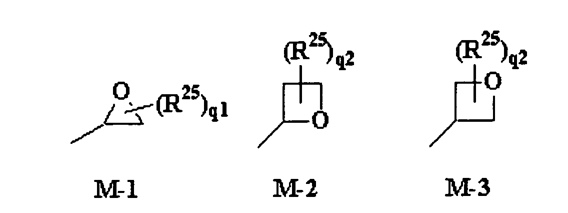 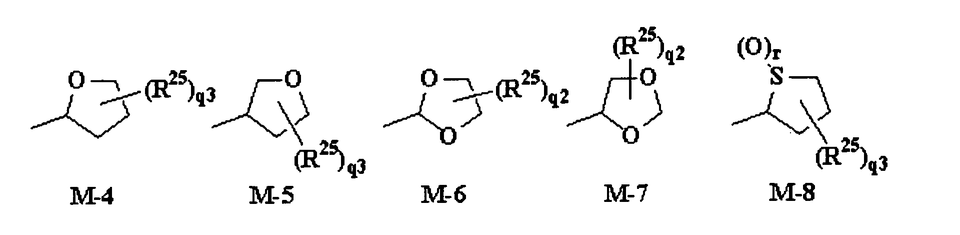 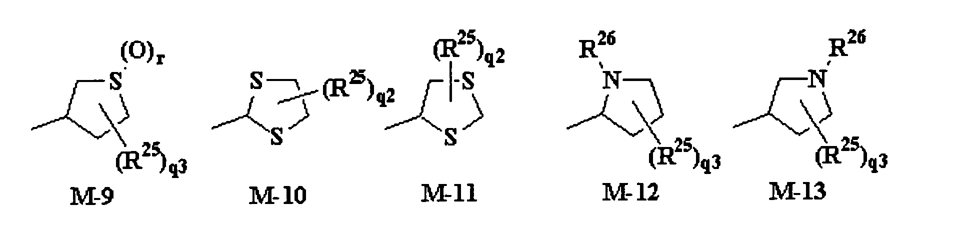 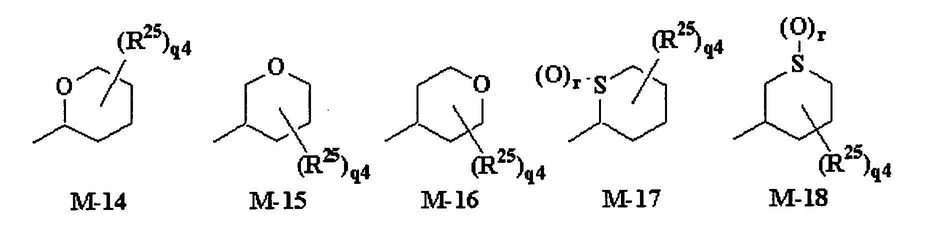 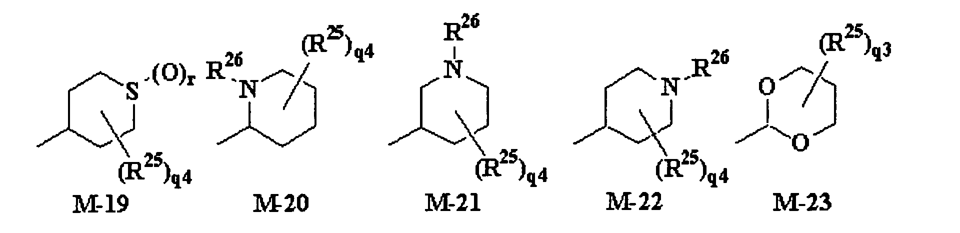 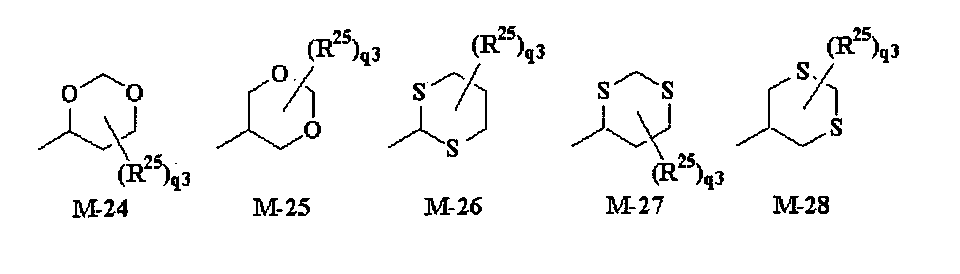 Z¹ represents a halogen atom, cyano, nitro, a C₁ to C₆ alkyl, a C₁ to C₆ haloalkyl, a C₃ to C₆ cycloalkyl, a C₃ to C₆ halocycloalkyl, a C₂ to C₆ alkenyl, a C₂ to C₆ haloalkenyl, a C₂ to C₆ alkynyl, a C₂ to C₆ haloalkynyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a C₁ to C₆ alkylamino, a di(C₁ to C₆ alkyl)amino, a C₁ to C₆ alkoxycarbonyl, a C₁ to C₆ haloalkoxycarbonyl or phenyl which may be optionally substituted by a halogen atom, when p1, p2, p3 or p4 represents an integer of 2 or more, each of Z¹s may be the same with each other or may be different from each other,
Z² represents a halogen atom, cyano, nitro, amino, azide, -SCN, -SF₅, a C₁ to C₆ alkyl, a (C₁ to C₆)alkyl which may be optionally substituted by R⁷, a C₃ to C₈ cycloalkyl, a (C₃ to C₈)cycloalkyl which may be optionally substituted by R⁷, a C₂ to C₆ alkenyl, a (C₂ to C₆)alkenyl which may be optionally substituted by R⁷, a C₃ to C₈ cycloalkenyl, a C₃ to C₈ halocycloalkenyl, a C₂ to C₆ alkynyl, a (C₂ to C₆)alkynyl which may be optionally substituted by R⁷, -OH, -OR⁸, -OS(O)₂R⁸, -SH, -S(O)ᵣR⁸, -N(R¹⁰)R⁹, -N(R¹⁰)CHO, -N(R¹⁰)C(O)R⁹, -N(R¹⁰)C(O)OR⁹, -N(R¹⁰)C(O)SR⁹, -N(R¹⁰)C(S)OR⁹, -N(R¹⁰)C(S)SR⁹, -N(R¹⁰)S(O)₂R⁹, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -S(O)₂OR⁹, -S(O)₂NHR¹⁰, -S(O)₂N(R¹⁰)R⁹, -Si(R¹³)(R¹⁴)R¹², phenyl which may be substituted by (Z¹)ₚ₁, L or M, when p1 represents an integer of 2 or more, each of Z²s may be the same with each other or may be different from each other, and further, when two Z²s are adjacent to each other, the adjacent two Z²s form -CH₂CH₂CH₂-, -CH₂CH₂O-, -CH₂OCH₂-, -OCH₂O-, -CH₂CH₂S-, -CH₂SCH₂-, -CH₂CH₂CH₂CH₂-, -CH₂CH₂CH₂O-, -CH₂CH₂OCH₂-, -CH₂OCH₂O-, -OCH₂CH₂O- or -OCH₂CH₂S-, so that the two Z²s form a 5-membered ring or 6-membered ring with carbon atoms to which the two Z²s are bonded, and at this time, the hydrogen atom(s) bonded to each of the carbon atom(s) which form(s) the ring may be optionally substituted by a halogen atom or a C₁ to C₆ alkyl group,
R⁷ represents a halogen atom, cyano, a C₃ to C₈ cycloalkyl, a C₃ to C₈ halocycloalkyl, -OH, -OR⁸, -SH, -S(O)ᵣR⁸, -N(R¹⁰)R⁹, -N(R¹⁰)CHO, -N(R¹⁰)C(O)R⁹, -N(R¹⁰)C(O)OR⁹, -N(R¹⁰)C(O)SR⁹, -N(R¹⁰)C(S)OR⁹, -N(R¹⁰)C(S)SR⁹, -N(R¹⁰)S(O)₂R⁹, -C(O)OR⁹, -C(O)N(R¹⁰)R⁹, -Si(R¹³)(R¹⁴)R¹², phenyl which may be substituted by (Z¹)ₚ₁, L or M,
R⁸ represents a C₁ to C₆ alkyl, a (C₁ to C₆)alkyl which may be optionally substituted by R²⁷, a C₃ to C₈ cycloalkyl, a (C₃ to C₈)cycloalkyl which may be optionally substituted by R²⁷, a C₂ to C₆ alkenyl, a (C₂ to C₆)alkenyl which may be optionally substituted by R²⁷, a C₃ to C₈ cycloalkenyl, a C₃ to C₈ halocycloalkenyl, a C₃ to C₆ alkynyl, a (C₃ to C₆)alkynyl which may be optionally substituted by R²⁷, phenyl which may be substituted by (Z¹)ₚ₁, L or M,
R⁹ represents a C₁ to C₆ alkyl, a C₁ to C₆ haloalkyl, a C₃ to C₆ cycloalkyl(C₁ to C₄)alkyl, a C₁ to C₆ alkoxy(C₁ to C₄)alkyl, a C₁ to C₆ alkylthio(C₁ to C₄)alkyl, a cyano(C₁ to C₆)alkyl, a phenyl(C₁ to C₄)alkyl which may be substituted by (Z¹)ₚ₁, a L-(C₁ to C₄)alkyl, a M-(C₁ to C₄)alkyl, a C₃ to C₈ cycloalkyl, a C₃ to C₈ halocycloalkyl, a C₃ to C₆ alkenyl, a C₃ to C₆ haloalkenyl, a C₃ to C₆ alkynyl or phenyl which may be substituted by (Z¹)ₚ₁,
R¹⁰ represents a hydrogen atom or a C₁ to C₆ alkyl, or R⁹ and R¹⁰ are combined to form a C₂ to C₆ alkylene chain, so that they may form a 3- to 7-membered ring with the atom(s) to which they are bonded, and the alkylene chain at this time may contain one oxygen atom, sulfur atom or nitrogen atom, and may be optionally substituted by a halogen atom, a C₁ to C₆ alkyl group, a C₁ to C₆ alkoxy group, formyl group, a C₁ to C₆ alkylcarbonyl group or a C₁ to C₆ alkoxycarbonyl group,
R¹¹ represents a hydrogen atom, a C₁ to C₆ alkyl, a C₁ to C₆ haloalkyl, a phenyl(C₁ to C₄)alkyl which may be substituted by (Z¹)ₚ₁, a C₃ to C₆ alkenyl, a C₃ to C₆ haloalkenyl, a C₃ to C₆ alkynyl or a C₃ to C₆ haloalkynyl, or R⁹ and R¹¹ are combined to form a C₂ to C₄ alkylene chain, so that they may form a 5 to 7-membered ring with the atom(s) to which they are bonded, and the alkylene chain at this time may contain one oxygen atom, sulfur atom or nitrogen atom, and may be optionally substituted by a halogen atom or a C₁ to C₆ alkyl group,
R¹² represents a C₁ to C₆ alkyl, a C₁ to C₆ haloalkyl, a C₃ to C₆ alkenyl or phenyl which may be substituted by (Z¹)ₚ₁,
R¹³ and R¹⁴ each independently represent a C₁ to C₆ alkyl or a C₁ to C₆ haloalkyl,
R¹⁵ represents a hydrogen atom, a C₁ to C₆ alkyl, a C₁ to C₆ haloalkyl, a C₁ to C₆ alkoxycarbonyl(C₁ to C₄)alkyl, a C₁ to C₆ haloalkoxycarbonyl(C₁ to C₄)alkyl, a phenyl(C₁ to C₄)alkyl which may be substituted by (Z¹)ₚ₁, a C₃ to C₆ alkenyl, a C₃ to C₆ haloalkenyl, a C₃ to C₆ alkynyl, a C₃ to C₆ haloalkynyl, a C₁ to C₆ alkoxy, a C₁ to C₆ alkoxycarbonyl, a C₁ to C₆ haloalkoxycarbonyl or phenyl which may be substituted by (Z¹)ₚ₁,
R¹⁶ represents a hydrogen atom, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ alkylthio(C₁ to C₄)alkyl, a phenylthio(C₁ to C₄)alkyl which may be substituted by (Z¹)ₚ₁, a cyano(C₁ to C₆)alkyl, a C₁ to C₆ alkoxycarbonyl(C₁ to C₄)alkyl, a C₁ to C₆ alkylaminocarbonyl(C₁ to C₄)alkyl, a di(C₁ to C₆ alkyl)aminocarbonyl(C₁ to C₄)alkyl, a phenyl(C₁ to C₄)alkyl which may be substituted by (Z¹)ₚ₁, a L-(C₁ to C₄)alkyl, a M-(C₁ to C₄)alkyl, a C₃ to C₆ cycloalkyl, a C₃ to C₆ alkenyl, a C₃ to C₆ haloalkenyl, a C₃ to C₆ alkynyl, a C₃ to C₆ haloalkynyl, -OH, a C₁ to C₆ alkylcarbonyloxy, a C₁ to C₆ alkylthio, a C₁ to C₆ haloalkylthio, phenylthio which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, -SN(R²⁰)R¹⁹, a C₁ to C₆ alkylaminosulfonyl, a di(C₁ to C₆ alkyl)aminosulfonyl, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -C(O)C(O)R⁹, -C(O)C(O)OR⁹, -P(O)(OR²⁴)₂ or -P(S)(OR²⁴)₂,
R¹⁷ represents a hydrogen atom, a C₁ to C₆ alkyl, a C₁ to C₆ haloalkyl, a C₃ to C₈ cycloalkyl(C₁ to C₆)alkyl, a C₃ to C₈ cycloalkyl, a C₃ to C₆ alkenyl, a C₃ to C₆ haloalkenyl, a C₃ to C₆ alkynyl, a C₃ to C₆ haloalkynyl, -CHO, a C₁ to C₆ alkylcarbonyl, a C₁ to C₆ haloalkylcarbonyl or a C₁ to C₆ alkoxycarbonyl, or R¹⁶ and R¹⁷ are combined to form a C₂ to C₆ alkylene chain, so that they may form a 3- to 7-membered ring with the nitrogen(s) atom to which they are bonded, and the alkylene chain at this time may contain one oxygen atom, sulfur atom or nitrogen atom, and may be optionally substituted by a halogen atom, a C₁ to C₆ alkyl group or a C₁ to C₆ haloalkyl group, or further, R¹⁷ is combined with R² to form a C₁ to C₂ alkylene chain, so that they may form a 6-membered or 7-membered hetero ring which fuses with a benzene ring with the atom(s) to which they are bonded, and the alkylene chain at this time may be optionally substituted by an oxygen atom or a C₁ to C₆ alkyl group,
R¹⁸ represents a halogen atom, cyano, nitro, a C₃ to C₈ cycloalkyl, a C₃ to C₈ halocycloalkyl, -OR²⁸, -N(R²⁹)R²⁸, -SH, -S(O)ᵣR³⁰, -CHO, -C(O)R³¹, -C(O)OH, -C(O)OR³¹, -C(O)SR³¹, -C(O)NHR³², -C(O)N(R³²)R³¹, -C(O)C(O)OR³¹, -C(R³⁴)=NOH, -C(R³⁴)=NOR³³, -Si(R¹³)(R¹⁴)R¹², -P(O)(OR²⁴)₂, -P(S)(OR²⁴)₂, -P(phenyl)₂, -P(O)(phenyl)₂, phenyl which may be substituted by (Z¹)ₚ₁, L or M,
R¹⁹ represents a C₁ to C₁₂ alkyl, a C₁ to C₁₂ haloalkyl, a C₁ to C₁₂ alkoxy(C₁ to C₁₂)alkyl, a cyano(C₁ to C₁₂)alkyl, a C₁ to C₁₂ alkoxycarbonyl(C₁ to C₁₂)alkyl, a phenyl(C₁ to C₄)alkyl which may be substituted by (Z¹)ₚ₁, a C₃ to C₁₂ alkenyl, a C₃ to C₁₂ haloalkenyl, a C₃ to C₁₂ alkynyl, a C₃ to C₁₂ haloalkynyl, a C₁ to C₁₂ alkylcarbonyl, a C₁ to C₁₂ alkoxycarbonyl or phenyl which may be substituted by (Z¹)ₚ₁,
R²⁰ represents a C₁ to C₁₂ alkyl, a C₁ to C₁₂ haloalkyl, a C₁ to C₁₂ alkoxy(C₁ to C₁₂)alkyl, a cyano(C₁ to C₁₂)alkyl, a C₁ to C₁₂ alkoxycarbonyl(C₁ to C₁₂)alkyl, a phenyl(C₁ to C₄)alkyl which may be substituted by (Z¹)ₚ₁, a C₃ to C₁₂ alkenyl, a C₃ to C₁₂ haloalkenyl, a C₃ to C₁₂ alkynyl, a C₃ to C₁₂ haloalkynyl or phenyl which may be substituted by (Z¹)ₚ₁, or R¹⁹ and R²⁰ are combined to form a C₄ to C₇ alkylene chain, so that they may form a 5 to 8-membered ring with the nitrogen atom(s) to which they are bonded, and the alkylene chain at this time may contain one oxygen atom or sulfur atom, and may be optionally substituted by a C₁ to C₄ alkyl group or a C₁ to C₄ alkoxy group,
R²¹ represents a hydrogen atom, a C₁ to C₆ alkyl, a C₁ to C₆ haloalkyl, a phenyl(C₁ to C₄)alkyl which may be substituted by (Z¹)ₚ₁, a C₃ to C₆ alkenyl, a C₃ to C₆ haloalkenyl, a C₃ to C₆ alkynyl, -CHO, a C₁ to C₆ alkylcarbonyl, a C₁ to C₆ haloalkylcarbonyl, a C₁ to C₆ alkoxycarbonyl, a C₁ to C₆ haloalkoxycarbonyl, a phenyl(C₁ to C₄)alkoxycarbonyl which may be substituted by (Z¹)ₚ₁, phenoxycarbonyl which may be substituted by (Z¹)ₚ₁, phenylcarbonyl which may be substituted by (Z¹)ₚ₁ or phenyl which may be substituted by (Z¹)ₚ₁,
R²² represents a hydrogen atom, a C₁ to C₆ alkyl, -CHO, a C₁ to C₆ alkylcarbonyl, a C₁ to C₆ haloalkylcarbonyl or a C₁ to C₆ alkoxycarbonyl,
R²³ represents cyano, a C₃ to C₈ cycloalkyl, a C₃ to C₈ halocycloalkyl, -OH, -OR⁸, -SH, -S(O)ᵣR⁸, -N(R¹⁰)R⁹, -N(R¹⁰)CHO, -N(R¹⁰)C(O)R⁹, -N(R¹⁰)C(O)OR⁹, -N(R¹⁰)C(O)SR⁹, -N(R¹⁰)C(S)OR⁹, -N(R¹⁰)C(S)SR⁹, -N(R¹⁰)S(O)₂R⁹, -C(O)OR⁹, -C(O)N(R¹⁰)R⁹, -Si(R¹³)(R¹⁴)R¹², phenyl which may be substituted by (Z¹)ₚ₁, L or M,
R²⁴ represents a C₁ to C₆ alkyl or a C₁ to C₆ haloalkyl,
R²⁵ represents a halogen atom, cyano, a C₁ to C₆ alkyl, a C₁ to C₆ haloalkyl, a hydroxy(C₁ to C₆)alkyl, a C₁ to C₄ alkoxy(C₁ to C₄)alkyl, a C₁ to C₄ alkoxycarbonyl(C₁ to C₄)alkyl, a C₁ to C₆ alkoxy, a C₁ to C₆ alkoxycarbonyl or phenyl which may be substituted by (Z¹)ₚ₁, when q1, q2, q3 or q4 represents an integer of 2 or more, each of R²⁵s may be the same with each other or may be different from each other,
R²⁶ represents a hydrogen atom, a C₁ to C₆ alkyl, a C₁ to C₆ haloalkyl, -CHO, a C₁ to C₆ alkylcarbonyl, a C₁ to C₆ haloalkylcarbonyl, a phenyl(C₁ to C₄)alkylcarbonyl which may be substituted by (Z¹)ₚ₁, a C₁ to C₆ alkoxycarbonyl, a C₁ to C₆ haloalkoxycarbonyl, a phenyl(C₁ to C₄)alkoxycarbonyl which may be substituted by (Z¹)ₚ₁, a C₁ to C₆ alkylaminocarbonyl, a di(C₁ to C₆ alkyl)aminocarbonyl, a C₁ to C₆ alkylaminothiocarbonyl, a di(C₁ to C₆ alkyl)aminothiocarbonyl, phenylcarbonyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, -P(O)(OR²⁴)₂ or -P(S)(OR²⁴)₂,
R²⁷ represents a halogen atom, cyano, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfonyl, a C₁ to C₆ haloalkylsulfonyl, a C₁ to C₆ alkylamino, a di(C₁ to C₆ alkyl)amino, -CHO, a C₁ to C₆ alkylcarbonyl, a C₁ to C₆ haloalkylcarbonyl, a C₁ to C₆ alkoxycarbonyl, a C₁ to C₆ haloalkoxycarbonyl, -CH=NOR¹¹, -C(R⁹)=NOR¹¹, phenyl which may be substituted by (Z¹)ₚ₁, L or M,
R²⁸ represents a hydrogen atom, a C₁ to C₈ alkyl, a (C₁ to C₈)alkyl which may be optionally substituted by R³⁵, a C₃ to C₈ cycloalkyl, a (C₃ to C₈)cycloalkyl which may be optionally substituted by R³⁵, a C₃ to C₈ alkenyl, a (C₃ to C₈)alkenyl which may be optionally substituted by R³⁵, a C₃ to C₈ alkynyl, a (C₃ to C₈)alkynyl which may be optionally substituted by R³⁵, -CHO, -C(O)R³¹, -C(O)OR³¹, -C(O)SR³¹, -C(O)NHR³², -C(O)N(R³²)R³¹, -C(O)C(O)R³¹, -C(O)C(O)OR³¹, -C(S)R³¹, -C(S)OR³¹, -C(S)SR³¹, -C(S)NHR³², -C(S)N(R³²)R³¹, -S(O)₂R³¹, -S(O)₂N(R³²)R³¹, -Si(R¹³)(R¹⁴)R¹², -P(O)(OR²⁴)₂, -P(S)(OR²⁴)₂, phenyl which may be substituted by (Z¹)ₚ₁, L or M,
R²⁹ represents a hydrogen atom, a C₁ to C₆ alkyl, a C₁ to C₆ haloalkyl, a C₃ to C₆ cycloalkyl or a C₁ to C₆ alkoxy, or R²⁸ and R²⁹ are combined to form a C₂ to C₅ alkylene chain, so that they may form a 3- to 6-membered ring with the nitrogen atom(s) to which they are bonded, the alkylene chain at this time may contain one oxygen atom or sulfur atom, and may be optionally substituted by a halogen atom, C₁ to C₆ alkyl group, C₁ to C₆ alkoxy group or a phenyl group which may be substituted by (Z¹)ₚ₁,
R³⁰ represents a C₁ to C₈ alkyl, a (C₁ to C₈)alkyl which may be optionally substituted by R³⁵, a C₃ to C₈ cycloalkyl, a (C₃ to C₈)cycloalkyl which may be optionally substituted by R³⁵, a C₃ to C₈ alkenyl, a (C₃ to C₈)alkenyl which may be optionally substituted by R³⁵, a C₃ to C₈ alkynyl, a (C₃ to C₈)alkynyl which may be optionally substituted by R³⁵, -SH, a C₁ to C₆ alkylthio, a C₁ to C₆ haloalkylthio, phenylthio which may be substituted by (Z¹)ₚ₁, -CHO, -C(O)R³¹, -C(O)OR³¹, -C(O)SR³¹, -C(O)NHR³², -C(O)N(R³²)R³¹, -C(O)C(O)R³¹, -C(O)C(O)OR³¹, -C(S)R³¹, -C(S)OR³¹, -C(S)SR³¹, -C(S)NHR³², -C(S)N(R³²)R³¹, -P(O)(OR²⁴)₂, -P(S)(OR²⁴)₂, phenyl which may be substituted by (Z¹)ₚ₁, L-18, L-21, L-25, L-30 to L-35, L-45, L-48, L-49 or M,
R³¹ represents a C₁ to C₆ alkyl, a C₁ to C₆ haloalkyl, a C₃ to C₈ cycloalkyl(C₁ to C₄)alkyl, a C₁ to C₆ alkoxy(C₁ to C₄)alkyl, a C₁ to C₆ haloalkoxy(C₁ to C₄)alkyl, a C₁ to C₆ alkylthio(C₁ to C₄)alkyl, a C₁ to C₆ haloalkylthio(C₁ to C₄)alkyl, a C₁ to C₆ alkylsulfonyl(C₁ to C₄)alkyl, a C₁ to C₆ haloalkylsulfonyl(C₁ to C₄)alkyl, a cyano(C₁ to C₆)alkyl, a C₁ to C₆ alkylcarbonyl(C₁ to C₄)alkyl, a C₁ to C₆ haloalkylcarbonyl(C₁ to C₄)alkyl, a C₁ to C₆ alkoxycarbonyl(C₁ to C₄)alkyl, a di(C₁ to C₆ alkyl)aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a L-(C₁ to C₄)alkyl, a M-(C₁ to C₄)alkyl, a C₃ to C₈ cycloalkyl, a C₃ to C₈ halocycloalkyl, a C₂ to C₆ alkenyl(C₃ to C₈)cycloalkyl, a C₂ to C₆ haloalkenyl(C₃ to C₈)cycloalkyl, a C₂ to C₆ alkenyl, a C₂ to C₆ haloalkenyl, a C₂ to C₆ alkynyl, a C₂ to C₆ haloalkynyl, phenyl which may be substituted by (Z¹)ₚ₁, L or M,
R³² represents a hydrogen atom, a C₁ to C₆ alkyl, a C₁ to C₆ haloalkyl or phenyl which may be substituted by (Z¹)ₚ₁, or R³¹ and R³² are combined to form a C₂ to C₅ alkylene chain, so that they may form a 3- to 6-membered ring with the nitrogen atom(s) to which they are bonded, and the alkylene chain at this time may contain one oxygen atom, sulfur atom or nitrogen atom, and may be optionally substituted by a halogen atom, a C₁ to C₆ alkyl group, a C₁ to C₆ alkoxy group, formyl group, a C₁ to C₆ alkylcarbonyl group, a C₁ to C₆ alkoxycarbonyl group or a phenyl group which may be substituted by (Z¹)ₚ₁,
R³³ represents a hydrogen atom, a C₁ to C₈ alkyl, a (C₁ to C₈)alkyl which may be optionally substituted by R³⁵, a C₃ to C₈ cycloalkyl, a C₃ to C₈ alkenyl, a (C₃ to C₈)alkenyl which may be optionally substituted by R³⁵, a C₃ to C₈ alkynyl or a (C₃ to C₈)alkynyl which may be optionally substituted by R³⁵,
R³⁴ represents a hydrogen atom, a C₁ to C₆ alkyl, a C₁ to C₆ haloalkyl, a C₃ to C₈ cycloalkyl(C₁ to C₄)alkyl, a C₁ to C₆ alkoxy(C₁ to C₄)alkyl, a C₁ to C₆ haloalkoxy(C₁ to C₄)alkyl, a C₁ to C₆ alkylthio(C₁ to C₄)alkyl, a C₁ to C₆ haloalkylthio(C₁ to C₄)alkyl, a C₁ to C₆ alkylsulfonyl(C₁ to C₄)alkyl, a C₁ to C₆ haloalkylsulfonyl(C₁ to C₄)alkyl, a phenyl(C₁ to C₄)alkyl which may be substituted by (Z¹)ₚ₁ or phenyl which may be substituted by (Z¹)ₚ₁,
R³⁵ represents a halogen atom, cyano, nitro, a C₃ to C₈ cycloalkyl, a C₃ to C₈ halocycloalkyl, -OH, -OR³⁶, -SH, -S(O)ᵣR³⁶, -NHR³⁷, -N(R³⁷)R³⁶, -CHO, -C(O)R³¹, -C(O)OR³¹, -C(O)SR³¹, -C(O)NHR³², -C(O)N(R³²)R³¹, -C(O)C(O)OR³¹, -CH=NOR¹¹, -C(R⁹)=NOR¹¹, -Si(R¹³)(R¹⁴)R¹², -P(O)(OR²⁴)₂, -P(S)(OR²⁴)₂, -P(phenyl)₂, -P(O)(phenyl)₂, phenyl which may be substituted by (Z¹)ₚ₁, L or M,
R³⁶ represents a C₁ to C₆ alkyl, a C₁ to C₆ haloalkyl, a C₁ to C₆ alkoxy(C₁ to C₄)alkyl, a C₁ to C₆ alkylthio(C₁ to C₄)alkyl, a phenyl(C₁ to C₄)alkyl which may be substituted by (Z¹)ₚ₁, a C₃ to C₈ cycloalkyl, a C₃ to C₈ halocycloalkyl, a C₃ to C₆ alkenyl, a C₃ to C₆ haloalkenyl, a C₃ to C₈ cycloalkenyl, a C₃ to C₈ halocycloalkenyl, a C₃ to C₆ alkynyl, a C₃ to C₆ haloalkynyl, -CHO, a C₁ to C₆ alkylcarbonyl, a C₁ to C₆ haloalkylcarbonyl, a C₁ to C₆ alkoxycarbonyl, a C₁ to C₆ haloalkoxycarbonyl, a C₁ to C₆ alkylaminocarbonyl, a di(C₁ to C₆ alkyl)aminocarbonyl, phenylcarbonyl which may be substituted by (Z¹)ₚ₁, a C₁ to C₆ alkylaminothiocarbonyl, a di(C₁ to C₆ alkyl)aminothiocarbonyl, phenyl which may be substituted by (Z¹)ₚ₁, L or M,
R³⁷ represents a hydrogen atom, a C₁ to C₆ alkyl, a C₁ to C₆ haloalkyl, a C₃ to C₈ cycloalkyl, a C₃ to C₆ alkenyl, a C₃ to C₆ alkynyl, a C₁ to C₆ alkylcarbonyl, a C₁ to C₆ haloalkylcarbonyl, a C₁ to C₆ alkoxycarbonyl, a C₁ to C₆ haloalkoxycarbonyl, phenoxycarbonyl which may be substituted by (Z¹)ₚ₁, phenylcarbonyl which may be substituted by (Z¹)ₚ₁, a C₁ to C₆ alkylsulfonyl, a C₁ to C₆ haloalkylsulfonyl, phenyl which may be substituted by (Z¹)ₚ₁, L or M, or R³⁶ and R³⁷ are combined to form a C₂ to C₅ alkylene chain, so that they may form a 3- to 6-membered ring with the nitrogen atom(s) to which they are bonded, the alkylene chain at this time may contain one oxygen atom or sulfur atom, and may be substituted by a halogen atom or a methyl group,
I represents an integer of 0 to 9,
m represents an integer of 0 to 4,
n represents an integer of 0 to 4,
p1 represents an integer of 1 to 5,
p2 represents an integer of 0 to 4,
p3 represents an integer of 0 to 3,
p4 represents an integer of 0 to 2,
p5 represents an integer of 0 or 1,
q1 represents an integer of 0 to 3,
q2 represents an integer of 0 to 5,
q3 represents an integer of 0 to 7,
q4 represents an integer of 0 to 9,
r represents an integer of 0 to 2,
t represents an integer of 0 or 1.]
or a salt thereof.

[2] The substituted benzanilide compound or a salt thereof of the above-mentioned [1],
wherein X represents a halogen atom, cyano, nitro, -SF₅, a C₁ to C₆ alkyl, a C₁ to C₆ haloalkyl, a C₂ to C₆ alkynyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a C₁ to C₆ alkoxycarbonyl, a C₁ to C₆ alkylaminocarbonyl, a di(C₁ to C₆ alkyl)aminocarbonyl or phenyl which may be substituted by (Z¹)ₚ₁, when m represents 2, 3 or 4, each of Xs may be the same with each other or may be different from each other, and further, when two Xs are adjacent to each other, the adjacent two Xs may form -CH₂CH₂CH₂-, -CH₂CH₂O-, -CH₂OCH₂-, -OCH₂O-, -CH₂CH₂S-, -CH₂SCH₂-, -CH₂CH₂CH₂CH₂-, -CH₂CH₂CH₂O-, -CH₂CH₂OCH₂-, -CH₂OCH₂O-, -OCH₂CH₂O-, -OCH₂CH₂S- or -CH=CHCH=CH-, so that the two Xs may form a 5-membered ring or 6-membered ring with the carbon atom(s) to which they are bonded, and at this time, the hydrogen atom(s) bonded to the respective carbon atoms which form the ring may be optionally substituted by a halogen atom, a C₁ to C₄ alkyl group or a C₁ to C₄ haloalkyl group,
and when G represents a 5-membered or 6-membered non-aromatic heterocyclic ring containing at least one atom selected from an oxygen atom, a sulfur atom and a nitrogen atom, and having at least one double bond in the ring, X may further represent -N(R¹⁷)R¹⁶, -N=CHOR¹² or -N=C(R⁹)OR¹²,
Y represents a halogen atom, cyano, a C₁ to C₆ alkyl, a C₁ to C₆ haloalkyl, a hydroxy(C₁ to C₆)alkyl, a C₁ to C₃ alkoxy(C₁ to C₃)alkyl, a C₁ to C₆ alkoxy, a C₁ to C₆ haloalkoxy, a C₁ to C₆ alkylthio, a C₁ to C₆ haloalkylthio, a C₁ to C₆ alkylamino, a di(C₁ to C₆ alkyl)amino, phenyl which may be substituted by (Z¹)ₚ₁ or phenoxy which may be substituted by (Z¹)ₚ₁, and when n represents 2, 3 or 4, each of Ys may be the same with each other or may be different from each other, and further, when two Ys are adjacent to each other, the adjacent two Ys may form -CH₂CH₂CH₂-, -CH₂CH₂O-, -CH₂OCH₂-, -OCH₂O-, -CH₂CH₂CH₂CH₂-, -CH₂CH₂CH₂O-, -CH₂CH₂OCH₂-, -CH₂OCH₂O-, -OCH₂CH₂O-, -OCH=N- or -SCH=N-, so that the two Ys may form a 5-membered ring or 6-membered ring with carbon atoms to which the two Ys are bonded, and at this time, the hydrogen atom(s) bonded to each of the carbon atom(s) which form(s) the ring may be optionally substituted by a halogen atom, a C₁ to C₄ alkyl group or a C₁ to C₄ haloalkyl group,
R¹ and R² each independently represent a hydrogen atom, a C₁ to C₆ alkyl, a C₁ to C₆ haloalkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfonyl(C₁ to C₄)alkyl, a C₁ to C₄ haloalkylsulfonyl(C₁ to C₄)alkyl, phenylthio(C₁ to C₄)alkyl which may be substituted by (Z¹)ₚ₁, a C₃ to C₆ alkenyl, a C₃ to C₆ haloalkenyl, a C₃ to C₆ alkynyl, a C₃ to C₆ haloalkynyl, a C₁ to C₆ alkylthio, a C₁ to C₆ haloalkylthio, phenylthio which may be substituted by (Z¹)ₚ₁ or -SN(R²⁰)R¹⁹,
R³ represents a C₁ to C₈ alkyl, a (C₁ to C₈)alkyl which may be optionally substituted by R¹⁸, a C₃ to C₈ cycloalkyl, a C₁ to C₄ alkylthio(C₃ to C₆)cycloalkyl, a C₁ to C₄ alkylsulfinyl(C₃ to C₆)cycloalkyl, a C₁ to C₄ alkylsulfonyl(C₃ to C₆)cycloalkyl, a hydroxymethyl(C₃ to C₆)cycloalkyl, a C₁ to C₄ alkoxymethyl(C₃ to C₆)cycloalkyl, a C₁ to C₄ alkylthiomethyl(C₃ to C₆)cycloalkyl, a C₁ to C₄ alkylsulfinylmethyl(C₃ to C₆)cycloalkyl, a C₁ to C₄ alkylsulfonylmethyl(C₃ to C₆)cycloalkyl, a C₃ to C₈ alkenyl, a C₁ to C₆ alkylaminocarbonyl(C₃ to C₆)alkenyl, a phenyl(C₃ to C₆)alkenyl which may be substituted by (Z¹)ₚ₁, a C₃ to C₈ alkynyl, a phenyl(C₃ to C₆)alkynyl which may be substituted by (Z¹)ₚ₁, a naphthyl(C₃ to C₆)alkynyl, a L-(C₃ to C₆)alkynyl, a C₁ to C₈ alkoxy, a C₃ to C₈ haloalkenyloxy, M-4, M-5, M-8, M-9, M-13 to M-19, M-21, M-22, M-25 or M-28, or R² and R³ are combined to form a C₂ to C₆ alkylene chain, so that they may form a 3- to 7-membered ring with a nitrogen atom(s) to which they are bonded, the alkylene chain at this time may contain one oxygen atom or sulfur atom, and may be optionally substituted by a C₁ to C₆ alkyl group,
R⁴ represents a hydrogen atom, a halogen atom, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, a C₁ to C₆ alkoxycarbonyl, -CH=NOR¹¹, -C(R⁹)=NOR¹¹, -Si(R¹³)(R¹⁴)R¹², -P(O)(OR²⁴)₂, phenyl which may be substituted by (Z²)ₚ₁, 1-naphthyl, 2-naphthyl, L or M,
R⁵ represents
(a). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)N(R¹⁰)R⁹, -C(S)N(R¹⁰)R⁹, -CH=NOR¹¹, -C(R⁹)=NOR¹¹, phenyl which may be substituted by (Z²)ₚ₁, 1-naphthyl, 2-naphthyl, L or M, when G represents a 5-membered or 6-membered non-aromatic heterocyclic ring containing at least one atom selected from an oxygen atom, a sulfur atom and a nitrogen atom, and having at least one double bond in the ring, or a 5-membered or 6-membered saturated heterocyclic ring containing two atoms selected from an oxygen atom, a sulfur atom and a nitrogen atom,
(b). cyano, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents cyano, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, a C₁ to C₆ alkoxycarbonyl, -Si(R¹³)(R¹⁴)R¹², -P(O)(OR²⁴)₂, phenyl which may be substituted by (Z²)ₚ₁, 1-naphthyl, 2-naphthyl, L or M,
R⁶ represents a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, -S(O)₂R⁹, a C₁ to C₆ alkylcarbonyl, a C₁ to C₆ haloalkylcarbonyl, phenylcarbonyl which may be substituted by (Z¹)ₚ₁, -C(O)OR⁹, -C(O)SR⁹, -C(O)N(R¹⁰)R⁹, a di(C₁ to C₆ alkyl)phosphoryl, a di(C₁ to C₆ alkyl)thiophosphoryl, phenyl which may be substituted by (Z²)ₚ₁, L or M, or when R⁶ and R⁴ are adjacent to each other, the adjacent R⁴ and R⁶ are combined to form a C₃ to C₅ alkylene chain, so that they may form a 5- to 7-membered ring with atoms to which they are bonded, and the alkylene chain at this time may contain one oxygen atom or sulfur atom, and may be optionally substituted by a C₁ to C₆ alkyl group,
Z² represents a halogen atom, cyano, nitro, amino, a C₁ to C₆ alkyl, a C₁ to C₆ haloalkyl, a C₁ to C₃ alkoxy(C₁ to C₃)alkyl, a C₁ to C₃ haloalkoxy(C₁ to C₃)alkyl, a C₁ to C₃ alkylthio(C₁ to C₃)alkyl, a C₁ to C₃ haloalkylthio(C₁ to C₃)alkyl, a C₁ to C₃ alkylsulfinyl(C₁ to C₃)alkyl, a C₁ to C₃ haloalkylsulfinyl(C₁ to C₃)alkyl, a C₁ to C₃ alkylsulfonyl(C₁ to C₃)alkyl, a C₁ to C₃ haloalkylsulfonyl(C₁ to C₃)alkyl, cyano(C₁ to C₆)alkyl, hydroxy(C₁ to C₃)haloalkyl, a C₁ to C₃ alkoxy(C₁ to C₃)haloalkyl, a C₁ to C₃ haloalkoxy-(C₁ to C₃)haloalkyl, a C₃ to C₈ cycloalkyl, a C₃ to C₈ halocycloalkyl, a C₁ to C₆ alkoxy, a C₁ to C₆ haloalkoxy, a C₁ to C₃ haloalkoxy(C₁ to C₃)haloalkoxy, a C₁ to C₆ alkylsulfonyloxy, a C₁ to C₆ haloalkylsulfonyloxy, phenoxy which may be substituted by (Z¹)ₚ₁, -O(L-45), a C₁ to C₆ alkylthio, a C₁ to C₆ haloalkylthio, a C₃ to C₈ cycloalkylthio, phenylthio which may be substituted by (Z¹)ₚ₁, -S(L-45), a C₁ to C₆ alkylsulfinyl, a C₁ to C₆ haloalkylsulfinyl, a C₃ to C₈ cycloalkylsulfinyl, phenylsulfinyl which may be substituted by (Z¹)ₚ₁, -S(O)(L-45), a C₁ to C₆ alkylsulfonyl, a C₁ to C₆ haloalkylsulfonyl, a C₃ to C₈ cycloalkylsulfonyl, phenylsulfonyl which may be substituted by (Z¹)ₚ₁, -SO₂(L-45), a C₁ to C₆ alkylamino, a di(C₁ to C₆ alkyl)amino, a C₁ to C₆ alkylaminosulfonyl, a di(C₁ to C₆ alkyl)aminosulfonyl, a C₁ to C₆ alkylsulfonylamino, a C₁ to C₆ haloalkylsulfonylamino, -C(O)NH₂, a C₁ to C₆ alkylaminocarbonyl, a di(C₁ to C₆ alkyl)aminocarbonyl, -C(S)NH₂, -Si(R¹⁵)(R¹⁶)R¹⁴, phenyl which may be substituted by (Z¹)ₚ₁, L-5, L-14, L-24, L-36, L-39, L-41, L-42, L-43, L-44 or M, when p1 represents an integer of 2 or more, each of Z²s may be the same with each other or may be different from each other, and further, when the two Z²s are adjacent to each other, the adjacent two Z²s form -CF₂CF₂O-, -CF₂OCF₂- or -OCF₂O- so that they may form a 5-membered ring with the carbon atoms each of which are bonded to,
R⁸ represents a C₁ to C₆ alkyl, a C₁ to C₆ haloalkyl, a C₃ to C₈ cycloalkyl(C₁ to C₄)alkyl, a C₃ to C₈ cycloalkyl, a C₃ to C₈ halocycloalkyl, a C₂ to C₆ alkenyl, a C₂ to C₆ haloalkenyl, a C₃ to C₈ cycloalkenyl, a C₃ to C₈ halocycloalkenyl, a C₃ to C₆ alkynyl, a C₃ to C₆ haloalkynyl, phenyl which may be substituted by (Z¹)ₚ₁, L or M,
R⁹ represents a C₁ to C₆ alkyl, a C₁ to C₆ haloalkyl, a C₃ to C₆ cycloalkyl(C₁ to C₄)alkyl, a phenyl(C₁ to C₄)alkyl which may be substituted by (Z¹)ₚ₁, a L-(C₁ to C₄)alkyl, a M-(C₁ to C₄)alkyl, a C₃ to C₈ cycloalkyl, a C₃ to C₈ halocycloalkyl or phenyl which may be substituted by (Z¹)ₚ₁,
R¹⁰ represents a hydrogen atom or a C₁ to C₆ alkyl, or R⁹ and R¹⁰ are combined to form a C₂ to C₆ alkylene chain, so that they may form a 3- to 7-membered ring with a nitrogen atom(s) to which they are bonded, and the alkylene chain at this time may contain one oxygen atom or sulfur atom,
R¹¹ represents a hydrogen atom, a C₁ to C₆ alkyl, a C₁ to C₆ haloalkyl or phenyl(C₁ to C₄)alkyl which may be substituted by (Z¹)ₚ₁, or R⁹ and R¹¹ are combined to form a C₂ to C₃ alkylene chain, so that they may form a 5-membered ring or 6-membered ring with atoms to which they are bonded, and the alkylene chain at this time may contain one oxygen atom, sulfur atom or nitrogen atom, and may be optionally substituted by a C₁ to C₆ alkyl group,
R¹² represents a C₁ to C₆ alkyl or phenyl which may be substituted by (Z¹)ₚ₁,
R¹³ and R¹⁴ each independently represent a C₁ to C₆ alkyl,
R¹⁵ represents a C₁ to C₆ alkyl, a C₁ to C₆ haloalkyl, a phenyl(C₁ to C₄)alkyl which may be substituted by (Z¹)ₚ₁ or phenyl which may be substituted by (Z¹)ₚ₁,
R¹⁶ represents a hydrogen atom, a C₁ to C₆ alkyl, a C₃ to C₆ cycloalkyl(C₁ to C₄)alkyl, a C₁ to C₄ alkoxy(C₁ to C₄)alkyl, a C₁ to C₄ alkylthio(C₁ to C₄)alkyl, a C₃ to C₆ cycloalkyl, a C₃ to C₆ alkenyl, a C₃ to C₆ haloalkenyl, a C₃ to C₆ alkynyl, a C₃ to C₆ haloalkynyl, a C₁ to C₆ alkylsulfonyl, a C₁ to C₆ haloalkylsulfonyl, -CHO, -C(O)R⁹, -C(O)OR⁹, -C(O)SR⁹, -C(O)N(R¹⁰)R⁹, -C(S)OR⁹, -C(S)SR⁹ or -C(S)N(R¹⁰)R⁹,
R¹⁷ represents a hydrogen atom or a C₁ to C₆ alkyl, or R¹⁷ and R² are combined to form a C₁ to C₂ alkylene chain, so that they may form a 6-membered or 7-membered hetero ring which fuses with a benzene ring with atoms to which they are bonded, and the alkylene chain at this time may be optionally substituted by a C₁ to C₆ alkyl group,
R¹⁸ represents a halogen atom, cyano, a C₃ to C₆ cycloalkyl, -OR²⁸, -N(R²⁹)R²⁸, -SH, -S(O)ᵣR³⁰, -CHO, a C₁ to C₆ alkylcarbonyl, a C₁ to C₆ alkoxycarbonyl, -C(O)N(R³²)R³¹, -C(R³⁴)=NOH, -C(R³⁴)=NOR³³, -Si(R¹³)(R¹⁴)R¹², phenyl which may be substituted by (Z¹)ₚ₁, L-1, L-2, L-3, L-4, L-45, L-46, L-47 or M,
R¹⁹ represents a C₁ to C₆ alkyl, a C₁ to C₆ alkoxycarbonyl(C₁ to C₄)alkyl or a C₁ to C₆ alkoxycarbonyl,
R²⁰ represents a C₁ to C₆ alkyl or, R¹⁹ and R²⁰ are combined to form a C₄ to C₅ alkylene chain, so that they may form a 5-membered ring or 6-membered ring with the nitrogen atom(s) to which they are bonded, and the alkylene chain at this time may contain one oxygen atom or sulfur atom, and may be optionally substituted by a methyl group or a methoxy group,
R²³ represents cyano, a C₃ to C₈ cycloalkyl, a C₃ to C₈ halocycloalkyl, a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, a C₁ to C₆ alkoxycarbonyl, a C₁ to C₆ haloalkoxycarbonyl, a di(C₁ to C₆ alkyl)-aminocarbonyl, -Si(R¹³)(R¹⁴)R¹², phenyl which may be substituted by (Z¹)ₚ₁, L or M,
R²⁵ represents a C₁ to C₆ alkyl, a C₁ to C₆ haloalkyl or a C₁ to C₆ alkoxy, when q1, q2, q3 or q4 represents an interger of 2 or more, each of R²⁵s may be the same with each other or may be different from each other,
R²⁶ represents -CHO, a C₁ to C₆ alkylcarbonyl, a C₁ to C₆ alkoxycarbonyl, a C₁ to C₆ alkylaminocarbonyl, a di(C₁ to C₆ alkyl)aminocarbonyl, phenylcarbonyl which may be substituted by (Z¹)ₚ₁, a C₁ to C₆ alkylsulfonyl or a C₁ to C₆ haloalkylsulfonyl,
R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₃ to C₈ cycloalkyl, a C₃ to C₈ alkenyl, a C₃ to C₈ alkynyl, -CHO, a C₁ to C₆ alkylcarbonyl, a C₁ to C₆ haloalkylcarbonyl, a C₃ to C₆ cycloalkylcarbonyl, phenylcarbonyl which may be substituted by (Z¹)ₚ₁, a C₁ to C₆ alkoxycarbonyl, -C(O)N(R³²)R³¹, -C(S)N(R³²)R³¹, a C₁ to C₆ alkylsulfonyl, a C₁ to C₆ haloalkylsulfonyl, phenylsulfonyl which may be substituted by (Z¹)ₚ₁, a di(C₁ to C₆ alkyl)aminosulfonyl, a di(C₁ to C₆ alkyl)phosphoryl, a di(C₁ to C₆ alkyl)thiophosphoryl, -Si(R¹³)(R¹⁴)R¹² or phenyl which may be substituted by (Z¹)ₚ₁,
R²⁹ represents a hydrogen atom, a C₁ to C₆ alkyl or a C₁ to C₆ alkoxy,
R³⁰ represents a C₁ to C₆ alkyl, a (C₁ to C₄)alkyl which may be optionally substituted by R³⁵, a C₃ to C₆ cycloalkyl, a C₃ to C₆ alkenyl, a C₃ to C₆ alkynyl, a C₁ to C₆ alkylthio, phenylthio which may be substituted by (Z¹)ₚ₁, a C₁ to C₆ alkylcarbonyl, a C₁ to C₆ alkylaminocarbonyl, a di(C₁ to C₄ alkyl)aminocarbonyl, a C₁ to C₆ alkylaminothiocarbonyl, a di(C₁ to C₄ alkyl)aminothiocarbonyl, phenyl which may be substituted by (Z¹)ₚ₁, L-21, L-35, L-45 or L-48,
R³¹ represents a C₁ to C₆ alkyl, a C₁ to C₆ haloalkyl, a C₃ to C₈ cycloalkyl(C₁ to C₄)alkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a (L-45)-(C₁ to C₄)alkyl, a (L-46)-(C₁ to C₄)alkyl, a (L-47)-(C₁ to C₄)alkyl, a C₃ to C₆ cycloalkyl, a C₃ to C₆ alkenyl, a C₂ to C₆ alkynyl or phenyl which may be substituted by (Z¹)ₚ₁,
R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, and the alkylene chain at this time may contain one oxygen atom, sulfur atom or nitrogen atom, and may be optionally substituted by a C₁ to C₆ alkyl group, a C₁ to C₆ alkoxy group, formyl group, a C₁ to C₆ alkylcarbonyl group or a C₁ to C₆ alkoxycarbonyl group,
R³³ represents a C₁ to C₆ alkyl, a C₃ to C₆ cycloalkyl(C₁ to C₄)alkyl, a C₁ to C₄ alkoxy(C₁ to C₄)alkyl, a C₁ to C₄ alkylthio(C₁ to C₄)alkyl, a C₁ to C₄ alkoxycarbonyl(C₁ to C₄)alkyl, a di(C₁ to C₄ alkyl)aminocarbonyl(C₁ to C₄)alkyl, a C₃ to C₆ alkenyl or a phenyl(C₁ to C₄)alkyl which may be substituted by (Z¹)ₚ₁,
R³⁴ represents a hydrogen atom, a C₁ to C₆ alkyl or phenyl which may be substituted by (Z¹)ₚ₁,
R³⁵ represents a halogen atom, -OH, a C₁ to C₄ alkoxy, a C₁ to C₆ alkylcarbonyloxy, a C₁ to C₄ haloalkylcarbonyloxy, a C₁ to C₄ alkylthio, a C₁ to C₄ alkylcarbonyl, a C₁ to C₄ alkoxycarbonyl, a di(C₁ to C₄ alkyl)aminocarbonyl, -Si(R¹³)(R¹⁴)R¹² or phenyl which may be substituted by (Z¹)ₚ₁.

[3] The substituted benzanilide compound or a salt thereof of the above-mentioned [2], wherein G represents a non-aromatic heterocyclic ring represented by either one of the formula G-1, the formula G-4, the formula G-5, the formula G-6, the formula G-7, the formula G-8, the formula G-11, the formula G-12, the formula G-13, the formula G-14, the formula G-15, the formula G-17, the formula G-18, the formula G-21, the formula G-22, the formula G-23, the formula G-32, the formula G-33, the formula G-40, the formula G-41, the formula G-42, the formula G-53 or the formula G-54, a saturated heterocyclic ring represented by the formula G-55 or the formula G-56, or a cycloalkyl ring represented by the formula G-71, 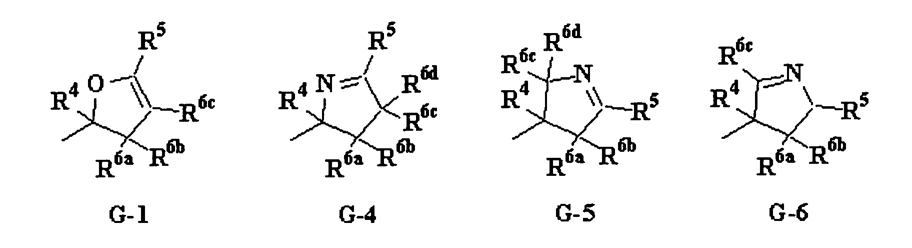 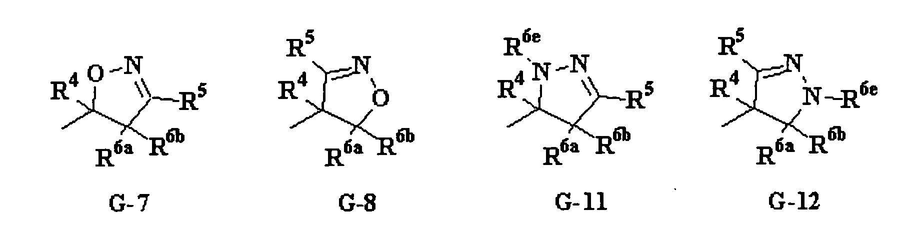 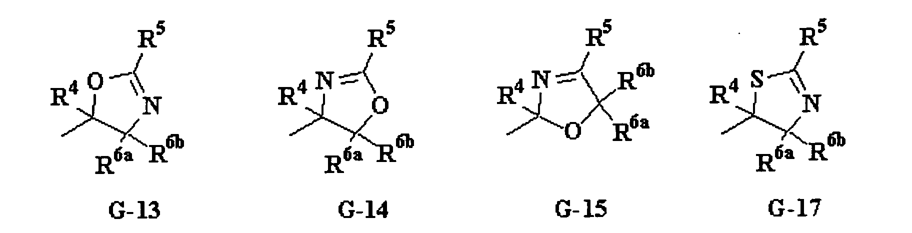 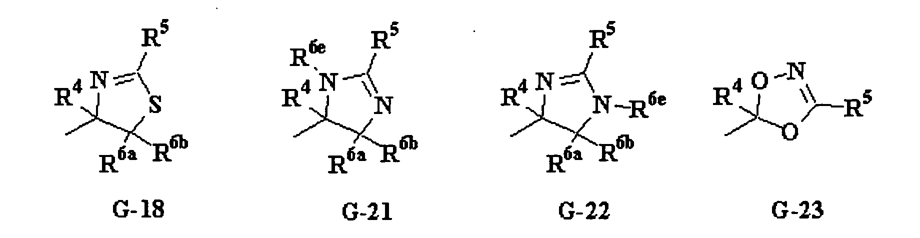 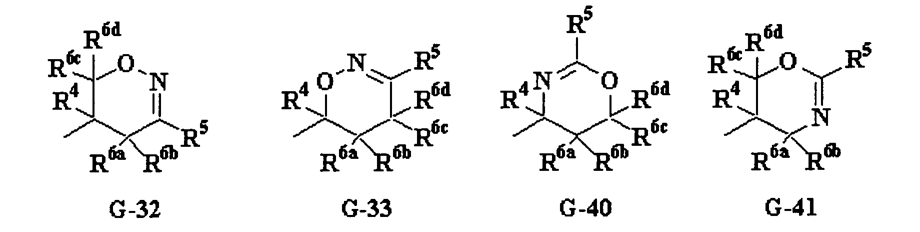 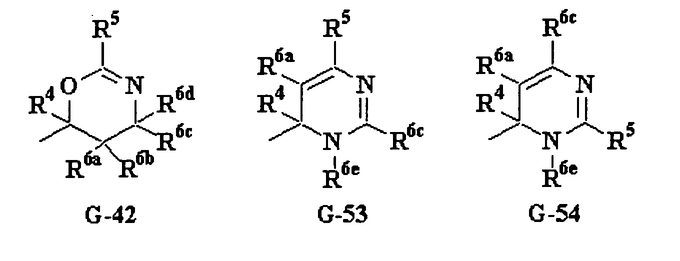 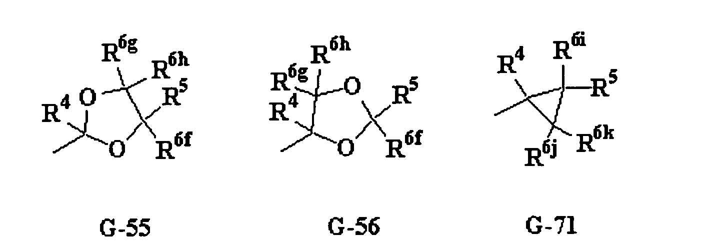 W¹ and W² each represent an oxygen atom,
X represents a halogen atom, nitro, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl or a C₁ to C₆ haloalkylsulfonyl, when m represents 2 or 3, each of Xs may be the same with each other or may be different from each other,
and further, when two Xs are adjacent to each other, the adjacent two Xs may form -OCF₂O- or -OCF₂CF₂O-, so that they may form a 5-membered ring or 6-membered ring with carbon atoms to which they are bonded,
and, when G represents a non-aromatic heterocyclic ring represented by either of the formula G-1, the formula G-4, the formula G-5, the formula G-6, the formula G-7, the formula G-8, the formula G-11, the formula G-12, the formula G-13, the formula G-14, the formula G-15, the formula G-17, the formula G-18, the formula G-21, the formula G-22, the formula G-23, the formula G-32, the formula G-33, the formula G-40, the formula G-41, the formula G-42, the formula G-53 or the formula G-54, X may further represent -N(R¹⁷)R¹⁶,
Y represents a halogen atom, a C₁ to C₆ alkyl, a C₁ to C₆ haloalkyl, a hydroxy(C₁ to C₆)alkyl, a C₁ to C₃ alkoxy(C₁ to C₃)alkyl, a C₁ to C₆ alkoxy or a C₁ to C₆ alkylthio, and when n represents 2 or 3, each of Ys may be the same with each other or may be different from each other,
R¹ represents a hydrogen atom,
R² represents a hydrogen atom, a C₁ to C₆ alkyl, a C₁ to C₄ alkoxy(C₁ to C₄)-alkyl or a C₃ to C₆ alkenyl,
R³ represents a C₁ to C₈ alkyl, a (C₁ to C₈)alkyl which may be optionally substituted by R¹⁸, a C₃ to C₈ cycloalkyl, a C₃ to C₈ alkenyl, a C₁ to C₆ alkylaminocarbonyl(C₃ to C₆)alkenyl, a phenyl(C₃ to C₆)alkenyl which may be substituted by (Z¹)ₚ₁, a C₃ to C₈ alkynyl, a phenyl(C₃ to C₆)alkynyl which may be substituted by (Z¹)ₚ₁, a naphthalen-1-yl-(C₃ to C₆)alkynyl, a naphthalen-2-yl-(C₃ to C₆)alkynyl, a (L-1)-(C₃ to C₆)alkynyl, a (L-2)-(C₃ to C₆)alkynyl, a (L-3)-(C₃ to C₆)alkynyl, a (L-4)-(C₃ to C₆)alkynyl, a (L-45)-(C₃ to C₆)alkynyl, a (L-46)-(C₃ to C₆)alkynyl, a (L-47)-(C₃ to C₆)alkynyl, M-4, M-5, M-8, M-9, M-13 to M-19, M-21 or M-22, or R² and R³ are combined to form a C₂ to C₆ alkylene chain, so that they may form a 3- to 7-membered ring with a nitrogen atom(s) to which they are bonded, and the alkylene chain at this time may contain one oxygen atom or sulfur atom,
R⁴ represents a hydrogen atom, a C₁ to C₆ alkyl, a C₁ to C₆ haloalkyl, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₁ to C₄ haloalkoxy(C₁ to C₄)haloalkyl, a C₃ to C₈ cycloalkyl, a C₃ to C₈ halocycloalkyl, -CH=NOR¹¹, -C(R⁹)=NOR¹¹, phenyl which may be substituted by (Z²)ₚ₁, 1-naphthyl, 2-naphthyl, L-1 to L-4, L-8 to L-13, L-15 to L-23, L-25 to L-35, L-37, L-38, L-40, L-43 to L-58, M-4, M-5, M-8, M-9, M-14 to M-18 or M-19,
and when G represents a cycloalkyl ring represented by the formula G-71, R⁴ may further represent a halogen atom, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl or a C₁ to C₆ haloalkylsulfonyl,
R⁵ represents
(a). a hydrogen atom, a halogen atom, cyano, a C₁ to C₆ alkyl, a C₁ to C₆ haloalkyl, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, cyano(C₁ to C₆)alkyl, a C₃ to C₈ cycloalkyl, a C₃ to C₈ halocycloalkyl, a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfinyl, a C₁ to C₆ haloalkylsulfinyl, phenylsulfinyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, a C₁ to C₆ alkylamino, a di(C₁ to C₆ alkyl)amino, pyrrolidin-1-yl, oxazolidin-3-yl, thiazolidin-3-yl, piperidin-1-yl, morpholin-1-yl, -C(O)R⁹, -C(O)OR⁹, -C(O)SR⁹, -C(O)N(R¹⁰)R⁹, -C(S)N(R¹⁰)R⁹, -CH=NOR¹¹, -C(R⁹)=NOR¹¹, phenyl which may be substituted by (Z²)ₚ₁, 1-naphthyl, 2-naphthyl, L or M, when G represents a non-aromatic heterocyclic ring represented by either one of the formula G-1, the formula G-4, the formula G-5, the formula G-6, the formula G-7, the formula G-8, the formula G-11, the formula G-12, the formula G-13, the formula G-14, the formula G-15, the formula G-17, the formula G-18, the formula G-21, the formula G-22, the formula G-23, the formula G-32, the formula G-33, the formula G-40, the formula G-41, the formula G-42, the formula G-53 or the formula G-54, or a saturated heterocyclic ring represented by the formula G-55 or the formula G-56,
(b). cyano, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio-(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₃ to C₈ cycloalkyl, a C₃ to C₈ halocycloalkyl, phenoxy which may be substituted by (Z¹)ₚ₁, phenylthio which may be substituted by (Z¹)ₚ₁, phenylsulfinyl which may be substituted by (Z¹)ₚ₁, phenylsulfonyl which may be substituted by (Z¹)ₚ₁, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a cycloalkyl ring represented by the formula G-71, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₃ to C₈ cycloalkyl, a C₃ to C₈ halocycloalkyl, a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfinyl, a C₁ to C₆ haloalkylsulfinyl, phenylsulfinyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a cycloalkyl ring represented by the formula G-71, and R⁴ represents a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio-(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₁ to C₄ haloalkoxy(C₁ to C₄)haloalkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, phenyl which may be substituted by (Z²)ₚ₁, 1-naphthyl, 2-naphthyl, L-1 to L-4, L-8 to L-13, L-15 to L-23, L-25 to L-35, L-37, L-38, L-40, L-43 to L-58, M-4, M-5, M-8, M-9, M-14 to M-18 or M-19,
R^{6a}, R^{6b}, R^{6c} and R^{6d} each independently represent a hydrogen atom, a halogen atom, cyano, a C₁ to C₆ alkyl, a C₁ to C₆ haloalkyl or phenyl which may be substituted by (Z²)ₚ₁,
R^{6e} represents a hydrogen atom, a C₁ to C₆ alkyl, a C₁ to C₆ alkylsulfonyl, a C₁ to C₆ haloalkylsulfonyl, phenylsulfonyl which may be substituted by (Z¹)ₚ₁, a C₁ to C₆ alkylcarbonyl, a C₁ to C₆ haloalkylcarbonyl, phenylcarbonyl which may be substituted by (Z¹)ₚ₁, a C₁ to C₆ alkoxycarbonyl, a C₁ to C₆ haloalkoxycarbonyl, phenoxycarbonyl which may be substituted by (Z¹)ₚ₁, a di(C₁ to C₆ alkyl)phosphoryl or phenyl which may be substituted by (Z²)ₚ₁,
R^{6f}, R^{6g} and R^{6h} each independently represent a hydrogen atom, a C₁ to C₆ alkyl, a C₁ to C₆ haloalkyl or phenyl which may be substituted by (Z²)ₚ₁,
R⁶ⁱ represents a hydrogen atom, a halogen atom, cyano, a C₁ to C₆ alkyl, a C₁ to C₆ haloalkyl, a C₁ to C₆ alkoxycarbonyl or a C₁ to C₆ haloalkoxycarbonyl,
R^{6j} and R^{6k} each independently represent hydrogen atom, a halogen atom, cyano, a C₁ to C₆ alkyl or a C₁ to C₆ haloalkyl,
Z² represents a halogen atom, cyano, nitro, amino, a C₁ to C₆ alkyl, a C₁ to C₆ haloalkyl, a C₁ to C₃ alkoxy(C₁ to C₃)alkyl, a C₁ to C₃ haloalkoxy(C₁ to C₃)alkyl, a C₁ to C₃ alkylthio(C₁ to C₃)alkyl, a C₁ to C₃ haloalkylthio(C₁ to C₃)alkyl, a C₁ to C₃ alkylsulfinyl(C₁ to C₃)alkyl, a C₁ to C₃ haloalkylsulfinyl(C₁ to C₃)alkyl, a C₁ to C₃ alkylsulfonyl(C₁ to C₃)alkyl, a C₁ to C₃ haloalkylsulfonyl(C₁ to C₃)alkyl, a cyano(C₁ to C₆)alkyl, a C₃ to C₈ cycloalkyl, a C₃ to C₈ halocycloalkyl, a C₁ to C₆ alkoxy, a C₁ to C₆ haloalkoxy, a C₁ to C₃ haloalkoxy(C₁ to C₃) haloalkoxy, a C₁ to C₆ alkylsulfonyloxy, a C₁ to C₆ haloalkylsulfonyloxy, phenoxy which may be substituted by (Z¹)ₚ₁, -O(L-45), a C₁ to C₆ alkylthio, a C₁ to C₆ haloalkylthio, a C₃ to C₈ cycloalkylthio, a C₁ to C₆ alkylsulfinyl, a C₁ to C₆ haloalkylsulfinyl, a C₃ to C₈ cycloalkylsulfinyl, a C₁ to C₆ alkylsulfonyl, a C₁ to C₆ haloalkylsulfonyl, a C₃ to C₈ cycloalkylsulfonyl, a C₁ to C₆ alkylamino, a di(C₁ to C₆ alkyl)amino, a C₁ to C₆ alkylaminosulfonyl, a di(C₁ to C₆ alkyl)aminosulfonyl, -C(O)NH₂, a C₁ to C₆ alkylaminocarbonyl, a di(C₁ to C₆ alkyl) aminocarbonyl, -C(S)NH₂ or a tri(C₁ to C₆ alkyl)silyl, when p1 represents an integer of 2 or more, each of Z²s may be the same with each other or may be different from each other,
and further, when the two Z²s are adjacent to each other, the adjacent two Z²s form -CF₂CF₂O-, -CF₂OCF₂- or -OCF₂O- so that they may form a 5-membered ring with the carbon atoms each of which are bonded to,
R⁹ represents a C₁ to C₆ alkyl, a C₁ to C₆ haloalkyl, a phenyl(C₁ to C₄)alkyl which may be substituted by (Z¹)ₚ₁, a C₃ to C₈ cycloalkyl or phenyl which may be substituted by (Z¹)ₚ₁,
R¹⁰ represents a hydrogen atom or a C₁ to C₆ alkyl, or R⁹ and R¹⁰ are combined to form a C₄ to C₅ alkylene chain, so that they may form a 5-membered ring or 6-membered ring with the nitrogen atom(s) to which they are bonded, and the alkylene chain at this time may contain one oxygen atom or sulfur atom,
R¹¹ represents a C₁ to C₆ alkyl, a C₁ to C₆ haloalkyl or a phenyl(C₁ to C₄)alkyl which may be substituted by (Z¹)ₚ₁, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group,
R¹⁵ represents a C₁ to C₆ alkyl or phenyl which may be substituted by (Z¹)ₚ₁,
R¹⁶ represents a hydrogen atom, a C₁ to C₆ alkyl, a C₁ to C₄ alkoxy(C₁ to C₄)alkyl, a C₁ to C₄ alkylthio(C₁ to C₄)alkyl, -CHO, a C₁ to C₆ alkylcarbonyl, a C₁ to C₆ haloalkylcarbonyl, a C₃ to C₆ cycloalkylcarbonyl, a C₁ to C₆ alkoxycarbonyl, a C₁ to C₆ haloalkoxycarbonyl or a C₁ to C₆ alkylthiocarbonyl,
R¹⁷ represents a hydrogen atom or a C₁ to C₆ alkyl, or R¹⁷ may be combined with R² to form a -CH₂-,
R¹⁸ represents a halogen atom, cyano, a C₃ to C₆ cycloalkyl, -OR²⁸, -N(R²⁹)R²⁸, -S(O)ᵣR³⁰, a C₁ to C₆ alkoxycarbonyl, -C(O)N(R³²)R³¹, -C(R³⁴)=NOH, -C(R³⁴)=NOR³³, a tri(C₁ to C₄ alkyl)silyl, phenyl which may be substituted by (Z¹)ₚ₁, L-1, L-2, L-3, L-4, L-45, L-46, L-47 or M,
R²⁵ represents a C₁ to C₄ alkyl,
R²⁶ represents a C₁ to C₆ alkylcarbonyl or a C₁ to C₆ alkoxycarbonyl,
R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₁ to C₆ alkylcarbonyl, a C₃ to C₆ cycloalkylcarbonyl, a C₁ to C₆ alkoxycarbonyl, -C(O)N(R³²)R³¹, a C₁ to C₆ alkylsulfonyl, a di(C₁ to C₆ alkyl)-aminosulfonyl, phenylsulfonyl which may be substituted by (Z¹)ₚ₁, a di(C₁ to C₆ alkyl)phosphoryl, a di(C₁ to C₆ alkyl)thiophosphoryl, a tri(C₁ to C₄ alkyl)silyl or phenyl which may be substituted by (Z¹)ₚ₁,
R²⁹ represents a hydrogen atom or a C₁ to C₆ alkyl,
R³⁰ represents a C₁ to C₆ alkyl, a C₁ to C₆ haloalkyl, a hydroxy(C₁ to C₄)alkyl, a C₁ to C₄ alkoxy(C₁ to C₄)alkyl, a C₁ to C₄ alkylthio(C₁ to C₄)alkyl, a C₁ to C₄ alkylcarbonyl(C₁ to C₄)alkyl, a C₁ to C₄ alkoxycarbonyl(C₁ to C₄)alkyl, a di(C₁ to C₄ alkyl)aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a C₃ to C₆ alkenyl, a C₃ to C₆ alkynyl, a C₁ to C₆ alkylthio, phenyl which may be substituted by (Z¹)ₚ₁, L-21 or L-45,
R³¹ represents a C₁ to C₆ alkyl, a C₁ to C₄ alkylthio(C₁ to C₄)alkyl, a phenyl(C₁ to C₄)alkyl which may be substituted by (Z¹)ₚ₁, a C₃ to C₆ cycloalkyl, a C₃ to C₆ alkenyl or phenyl which may be substituted by (Z¹)ₚ₁,
R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, and the alkylene chain at this time may contain one oxygen atom or sulfur atom,
R³³ represents a C₁ to C₆ alkyl or a phenyl(C₁ to C₄)alkyl which may be substituted by (Z¹)ₚ₁,
R³⁴ represents a hydrogen atom or a C₁ to C₆ alkyl,
m represents an integer of 0 to 3,
n represents an integer of 0 to 3,
q2 represents an integer of 0 to 3,
q3 represents an integer of 0 to 2, and
q4 represents an integer of 0 to 2.

[4] The substituted benzanilide compound or a salt thereof of the above-mentioned [3], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, or a cycloalkyl ring represented by the formula G-71,
X represents a halogen atom, nitro, a C₁ to C₄ alkyl, a C₁ to C₄ haloalkyl, a C₁ to C₄ alkoxy, a C₁ to C₄ haloalkoxy, a C₁ to C₄ alkylthio, a C₁ to C₄ haloalkylthio, a C₁ to C₄ alkylsulfinyl, a C₁ to C₄ haloalkylsulfinyl, a C₁ to C₄ alkylsulfonyl or a C₁ to C₄ haloalkylsulfonyl, and when m represents 2, each of Xs may be the same with each other or may be different from each other,
Y represents a halogen atom, a C₁ to C₄ alkyl, a C₁ to C₄ haloalkyl, a C₁ to C₄ alkoxy or a C₁ to C₄ alkylthio, and when n represents 2, each of Ys may be the same with each other or may be different from each other,
R² represents a hydrogen atom or a C₁ to C₆ alkyl,
R³ represents a C₁ to C₈ alkyl, a (C₁ to C₈)alkyl which may be optionally substituted by R¹⁸, a C₃ to C₈ alkenyl or a C₃ to C₈ alkynyl,
R⁴ represents a hydrogen atom, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₃ to C₈ cycloalkyl, a C₃ to C₈ halocycloalkyl, phenyl which may be substituted by (Z²)ₚ₁, 2-naphthyl, L-1 to L-4, L-15 to L-17, L-19, L-20, L-22, L-23, L-45 to L-47, L-50, M-4, M-5, M-8, M-9, M-14 to M-18 or M-19,
and when G represents a cycloalkyl ring represented by the formula G-71, R⁴ may further represent a halogen atom, a C₁ to C₆ alkoxy, a C₁ to C₆ haloalkoxy, a C₁ to C₆ alkylthio, a C₁ to C₆ haloalkylthio, a C₁ to C₆ alkylsulfonyl or a C₁ to C₆ haloalkylsulfonyl,
R⁵ represents
(a). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a di(C₁ to C₆ alkyl)amino, pyrrolidin-1-yl, oxazolidin-3-yl, thiazolidin-3-yl, piperidin-1-yl, morpholin-1-yl, -C(R⁹)=NOR¹¹, phenyl which may be substituted by (Z²)ₚ₁, 1-naphthyl, 2-naphthyl, L, M-4, M-5, M-8, M-9, M-14 to M-18 or M-19, when G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
(b). a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, -C(R⁹)=NOR¹¹, -Si(R¹³)(R¹⁴)R¹², phenyl which may be substituted by (Z²)ₚ₁, 1-naphthyl, 2-naphthyl, L, M-4, M-5, M-8, M-9, M-14 to M-18 or M-19, when G represents a cycloalkyl ring represented by the formula G-71, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, -C(R⁹)=NOR¹¹, -Si(R¹³)(R¹⁴)R¹², M-4, M-5, M-8, M-9, M-14 to M-18 or M-19, when G represents a cycloalkyl ring represented by the formula G-71, and R⁴ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfonyl, a C₁ to C₆ haloalkylsulfonyl, M-4, M-5, M-8, M-9, M-14 to M-18 or M-19,
(d). a hydrogen atom, a halogen atom, cyano, a C₁ to C₆ alkyl or a C₁ to C₆ haloalkyl, when G represents a cycloalkyl ring represented by the formula G-71, and R⁴ represents phenyl which may be substituted by (Z²)ₚ₁, 1-naphthyl, 2-naphthyl, L-1 to L-4, L-15 to L-17, L-19, L-20, L-22, L-23, L-45 to L-47 or L-50,
R^{6a}, R^{6b}, R^{6c} and R^{6d} each represent a hydrogen atom,
R^{6e} represents a C₁ to C₆ alkyl, a C₁ to C₆ alkylsulfonyl, a C₁ to C₆ alkoxycarbonyl, a di(C₁ to C₆ alkyl)phosphoryl or phenyl which may be substituted by (Z²)ₚ₁,
R⁶ⁱ represents a hydrogen atom, a halogen atom or a C₁ to C₆ alkyl,
R^{6j} and R^{6k} each independently represent a hydrogen atom, a halogen atom, cyano or methyl,
R⁹ represents a C₁ to C₆ alkyl, a C₁ to C₆ haloalkyl, a C₃ to C₈ cycloalkyl or phenyl which may be substituted by (Z¹)ₚ₁,
R¹¹ represents a C₁ to C₆ alkyl or a C₁ to C₆ haloalkyl, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group,
R¹⁸ represents a -OR²⁸, -N(R²⁹)R²⁸, -S(O)ᵣR³⁰, -C(R³⁴)=NOH, -C(R³⁴)=NOR³³ or phenyl which may be substituted by (Z¹)ₚ₁,
R²⁸ represents a hydrogen atom, a C₁ to C₆ alkyl, a C₁ to C₆ alkylcarbonyl, a C₁ to C₆ alkoxycarbonyl, a C₁ to C₆ alkylaminocarbonyl, a C₃ to C₆ cycloalkylaminocarbonyl, a di(C₁ to C₆ alkyl)aminocarbonyl, phenylaminocarbonyl which may be substituted by (Z¹)ₚ₁, a phenyl(C₁ to C₄)alkylaminocarbonyl which may be substituted by (Z¹)ₚ₁, a C₁ to C₆ alkylsulfonyl, a di(C₁ to C₆ alkyl)thiophosphoryl or phenyl which may be substituted by (Z¹)ₚ₁,
R³⁰ represents a C₁ to C₆ alkyl, a C₁ to C₆ alkylthio, phenyl which may be substituted by (Z¹)ₚ₁ or L-45,
R³³ represents a C₁ to C₆ alkyl,
R³⁴ represents a hydrogen atom,
m represents an integer of 0 to 2, and
n represents an integer of 0 to 2.

[5] The substituted benzanilide compound or a salt thereof of the above-mentioned [1] to [4], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, and
R⁵ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, pyrrolidin-1-yl, oxazolidin-3-yl, thiazolidin-3-yl, piperidin-1-yl, morpholin-1-yl, -C(R⁹)=NOR¹¹, phenyl which may be substituted by (Z²)ₚ₁, 1-naphthyl, 2-naphthyl, L, M-4, M-5, M-8, M-9, M-14 to M-18 or M-19.

[6] The substituted benzanilide compound or a salt thereof of the above-mentioned [1] to [4], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, and
R⁴ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₃ to C₈ cycloalkyl, a C₃ to C₈ halocycloalkyl, phenyl which may be substituted by (Z²)ₚ₁, 2-naphthyl, L-1 to L-4, L-15 to L-17, L-19, L-20, L-22, L-23, L-45 to L-47, L-50, M-4, M-5, M-8, M-9, M-14 to M-18 or M-19.

[7] The substituted benzanilide compound or a salt thereof of the above-mentioned [1] to [3], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R³ represents a C₁ to C₈ alkyl, a C₁ to C₈ haloalkyl, a cyano(C₁ to C₈)alkyl, a C₃ to C₆ cycloalkyl(C₁ to C₈)alkyl, a tri(C₁ to C₄ alkyl)silyl(C₁ to C₈)alkyl, a phenyl(C₁ to C₈)alkyl which may be substituted by (Z¹)ₚ₁, a (L-1)-(C₁ to C₈)alkyl, a (L-2)-(C₁ to C₈)alkyl, a (L-3)-(C₁ to C₈)alkyl, a (L-4)-(C₁ to C₈)alkyl, a (L-45)-(C₁ to C₈)alkyl, a (L-46)-(C₁ to C₈)alkyl, a (L-47)-(C₁ to C₈)alkyl, a C₃ to C₈ cycloalkyl, a C₃ to C₈ alkenyl, a phenyl(C₃ to C₆)alkenyl which may be substituted by (Z¹)ₚ₁, a C₃ to C₈ alkynyl, a phenyl(C₃ to C₆)alkynyl which may be substituted by (Z¹)ₚ₁, a naphthalen-1-yl-(C₃ to C₆)alkynyl, a naphthalen-2-yl-(C₃ to C₆)alkynyl, a (L-1)-(C₃ to C₆)alkynyl, a (L-2)-(C₃ to C₆)alkynyl, a (L-3)-(C₃ to C₆)alkynyl, a (L-4)-(C₃ to C₆)alkynyl, a (L-45)-(C₃ to C₆)alkynyl, a (L-46)-(C₃ to C₆)alkynyl or a (L-47)-(C₃ to C₆)alkynyl, or R² and R³ are combined to form a C₂ to C₆ alkylene chain, so that they may form a 3- to 7-membered ring with the nitrogen atoms to which they are bonded.

[8]. The substituted benzanilide compound or a salt thereof of the above-mentioned [4], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, and
R³ represents a C₁ to C₈ alkyl, a phenyl(C₁ to C₈)alkyl which may be substituted by (Z¹)ₚ₁, a C₃ to C₈ alkenyl or a C₃ to C₈ alkynyl.

[9] The substituted benzanilide compound or a salt thereof of the above-mentioned [1] to [3], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R³ represents a (C₁ to C₈)alkyl which may be optionally substituted by R¹⁸, a C₁ to C₆ alkylaminocarbonyl(C₃ to C₆)alkenyl, M-4, M-5, M-8, M-9, M-13 to M-19, M-21 or M-22, or R² and R³ are combined to form a C₂ to C₆ alkylene chain containing one oxygen atom or sulfur atom, so that they may form a 3- to 7-membered ring with a nitrogen atom(s) to which they are bonded,
R⁴ represents a hydrogen atom, a C₁ to C₆ alkyl or a C₁ to C₆ haloalkyl,
R⁵ represents a hydrogen atom, a halogen atom, cyano, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl or a di(C₁ to C₆ alkyl)amino,
R¹⁸ represents -OR²⁸, -N(R²⁹)R²⁸, -S(O)ᵣR³⁰, a C₁ to C₆ alkoxycarbonyl, a C₁ to C₆ alkylaminocarbonyl, -C(O)N(R³²)R³¹, -C(R³⁴)=NOH, -C(R³⁴)=NOR³³ or M,
R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₁ to C₆ alkylcarbonyl, a C₃ to C₆ cycloalkylcarbonyl, a C₁ to C₆ alkoxycarbonyl, -C(O)N(R³²)R³¹, a C₁ to C₆ alkylsulfonyl, a di(C₁ to C₆ alkyl)aminosulfonyl, phenylsulfonyl which may be substituted by (Z¹)ₚ₁, a di(C₁ to C₆ alkyl)phosphoryl, a di(C₁ to C₆ alkyl)thiophosphoryl, a tri(C₁ to C₄ alkyl)silyl or phenyl which may be substituted by (Z¹)ₚ₁,
R²⁹ represents a hydrogen atom or a C₁ to C₆ alkyl,
R³⁰ represents a C₁ to C₆ alkyl, a C₁ to C₆ haloalkyl, a hydroxy(C₁ to C₄)alkyl, a C₁ to C₄ alkoxy(C₁ to C₄)alkyl, a C₁ to C₄ alkylthio(C₁ to C₄)alkyl, a C₁ to C₄ alkylcarbonyl(C₁ to C₄)alkyl, a C₁ to C₄ alkoxycarbonyl(C₁ to C₄)alkyl, a di(C₁ to C₄ alkyl)aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a C₃ to C₆ alkenyl, a C₃ to C₆ alkynyl, a C₁ to C₆ alkylthio, phenyl which may be substituted by (Z¹)ₚ₁, L-21 or L-45,
R³¹ represents a C₁ to C₆ alkyl, a C₁ to C₄ alkylthio(C₁ to C₄)alkyl, a phenyl(C₁ to C₄)alkyl which may be substituted by (Z¹)ₚ₁, a C₃ to C₆ cycloalkyl, a C₃ to C₆ alkenyl or phenyl which may be substituted by (Z¹)ₚ₁,
R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, and further the alkylene chain may contain one oxygen atom or sulfur atom,
R³³ represents a C₁ to C₆ alkyl or a phenyl(C₁ to C₄)alkyl which may be substituted by (Z¹)ₚ₁,
R³⁴ represents a hydrogen atom or a C₁ to C₆ alkyl,
q2 represents an integer of 0 to 3,
q3 represents an integer of 0 to 2, and
q4 represents an integer of 0 to 2.

[10] The substituted benzanilide compound or a salt thereof of the above-mentioned [4], wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R³ represents a C₁ to C₈ alkyl which may be optionally substituted by -OR²⁸, -N(R²⁹)R²⁸, -S(O)ᵣR³⁰, -C(R³⁴)=NOH or -C(R³⁴)=NOR³³,
R⁴ represents a hydrogen atom, a C₁ to C₆ alkyl or a C₁ to C₆ haloalkyl,
R⁵ represents a hydrogen atom, a halogen atom, cyano, a C₁ to C₆ alkyl, a C₁ to C₆ haloalkyl, a C₁ to C₆ alkoxy; a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl or a di(C₁ to C₆ alkyl)amino,
R²⁸ represents a hydrogen atom, a C₁ to C₆ alkyl, a C₁ to C₆ alkylcarbonyl, a C₁ to C₆ alkoxycarbonyl, a C₁ to C₆ alkylaminocarbonyl, a C₃ to C₆ cycloalkylaminocarbonyl, a di(C₁ to C₆ alkyl)aminocarbonyl, phenylaminocarbonyl which may be substituted by (Z¹)ₚ₁, a phenyl(C₁ to C₄)alkylaminocarbonyl which may be substituted by (Z¹)ₚ₁, a C₁ to C₆ alkylsulfonyl, a di(C₁ to C₆ alkyl)thiophosphoryl or phenyl which may be substituted by (Z¹)ₚ₁,
R³⁰ represents a C₁ to C₆ alkyl, a C₁ to C₆ alkylthio, phenyl which may be substituted by (Z¹)ₚ₁ or L-45,
R³³ represents a C₁ to C₆ alkyl, and
R³⁴ represents a hydrogen atom.

[11] The substituted benzanilide compound or a salt thereof of the above-mentioned [1] to [5] or the above-mentioned [7] to [8], wherein G represents a non-aromatic heterocyclic ring represented by either one of the formula G-7, the formula G-13, the formula G-14, the formula G-15, the formula G-17 or the formula G-18,
X represents a halogen atom, nitro, a C₁ to C₄ alkyl, a C₁ to C₄ haloalkyl, a C₁ to C₄ alkylthio, a C₁ to C₄ alkylsulfinyl or a C₁ to C₄ alkylsulfonyl, and when m represents 2, each of Xs may be the same with each other or may be different from each other,
Y represents a halogen atom or a C₁ to C₄ alkyl, and when n represents 2, each of Ys may be the same with each other or may be different from each other,
R⁴ represents a C₁ to C₆ alkyl or a C₁ to C₆ haloalkyl,
R⁵ represents phenyl which may be substituted by (Z²)ₚ₁, 1-naphthyl, 2-naphthyl, L-1 to L-5, L-8 to L-24, L-28 to L-36, L-39, L-41, L-42, L-45 to L-47 or L-50.

[12] The substituted benzanilide compound or a salt thereof of the above-mentioned [1] to [4] or the above-mentioned [6] to [8], wherein G represents a non-aromatic heterocyclic ring represented by either one of the formula G-7, the formula G-13, the formula G-14, the formula G-15, the formula G-17 or the formula G-18,
X represents a halogen atom, nitro, a C₁ to C₄ alkyl, a C₁ to C₄ haloalkyl, a C₁ to C₄ alkylthio, a C₁ to C₄ alkylsulfinyl or a C₁ to C₄ alkylsulfonyl, and when m represents 2, each of Xs may be the same with each other or may be different from each other,
Y represents a halogen atom or a C₁ to C₄ alkyl, and when n represents 2, each of Ys may be the same with each other or may be different from each other,
R⁴ represents phenyl which may be substituted by (Z²)ₚ₁, 2-naphthyl, L-1 to L-4, L-15 to L-17, L-19, L-20, L-22, L-23, L-45, L-46 or L-47,
R⁵ represents a halogen atom, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ alkylsulfinyl, a C₁ to C₆ alkylsulfonyl or a di(C₁ to C₆ alkyl)amino.

[13] The substituted benzanilide compound or a salt thereof of the above-mentioned [4], wherein G represents a cycloalkyl ring represented by the formula G-71,
X represents a halogen atom, nitro, a C₁ to C₄ alkyl, a C₁ to C₄ haloalkyl, a C₁ to C₆ alkylthio, a C₁ to C₆ alkylsulfinyl or a C₁ to C₆ alkylsulfonyl, and when m represents 2, each of Xs may be the same with each other or may be different from each other,
Y represents a halogen atom or a C₁ to C₆ alkyl, and when n represents 2, each of Ys may be the same with each other or may be different from each other,
R³ represents a C₁ to C₈ alkyl which may be optionally substituted by -OR²⁸, -N(R²⁹)R²⁸, -S(O)ᵣR³⁰, -C(R³⁴)=NOH or -C(R³⁴)=NOR³³,
R⁴ represents a hydrogen atom, a halogen atom, a C₁ to C₆ alkyl or a C₁ to C₆ haloalkyl,
R⁵ represents phenyl which may be substituted by (Z²)ₚ₁, 1-naphthyl, 2-naphthyl, L-1 to L-4, L-15 to L-17, L-19, L-20, L-22, L-23, L-45, L-46 or L-47,
R²⁸ represents a hydrogen atom, a C₁ to C₆ alkyl, a C₁ to C₆ alkylcarbonyl, a C₁ to C₆ alkoxycarbonyl, a C₁ to C₆ alkylaminocarbonyl, a C₃ to C₆ cycloalkylaminocarbonyl, a di(C₁ to C₆ alkyl)aminocarbonyl, phenylaminocarbonyl which may be substituted by (Z¹)ₚ₁, a phenyl(C₁ to C₄)alkylaminocarbonyl which may be substituted by (Z¹)ₚ₁, a C₁ to C₆ alkylsulfonyl, a di(C₁ to C₆ alkyl)thiophosphoryl or phenyl which may be substituted by (Z¹)ₚ₁,
R³⁰ represents a C₁ to C₆ alkyl, a C₁ to C₆ alkylthio, phenyl which may be substituted by (Z¹)ₚ₁ or L-45,
R³³ represents a C₁ to C₆ alkyl, and
R³⁴ represents a hydrogen atom.

[14] The substituted benzanilide compound or a salt thereof of the above-mentioned [4], wherein G represents a cycloalkyl ring represented by the formula G-71,
R⁴ represents phenyl which may be substituted by (Z²)ₚ₁, 2-naphthyl, L-1 to L-4, L-15 to L-17, L-19, L-20, L-22, L-23, L-45, L-46 or L-47, and
R⁵ represents a hydrogen atom, halogen atom or a C₁ to C₆ alkyl.

[15] The substituted benzanilide compound or a salt thereof of the above-mentioned [14], wherein X represents a halogen atom, nitro, a C₁ to C₄ alkyl, a C₁ to C₄ haloalkyl, a C₁ to C₆ alkylthio, a C₁ to C₆ alkylsulfinyl or a C₁ to C₆ alkylsulfonyl, and when m represents 2, each of Xs may be the same with each other or may be different from each other,
Y represents a halogen atom or a C₁ to C₆ alkyl, and when n represents 2, each of Ys may be the same with each other or may be different from each other,
R⁵ represents a hydrogen atom,
R⁶ⁱ represents a hydrogen atom, and
R^{6j} and R^{6k} each independently represent a hydrogen atom, a halogen atom or cyano.

[16] An N-substituted phenyl-3-nitrophthalimide or a substituted aniline represented by the formula (2) or the formula (3): 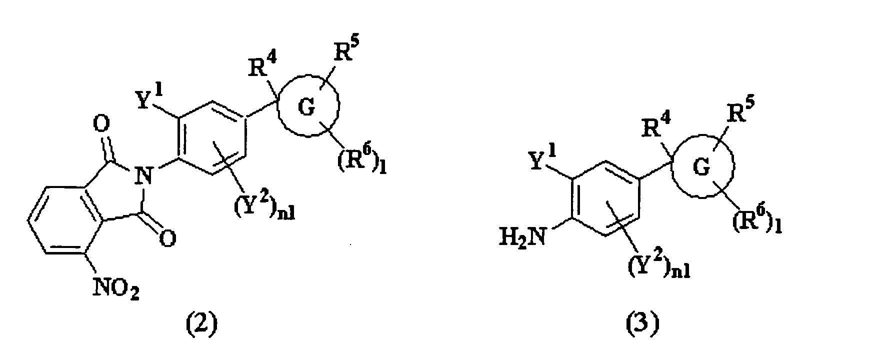 [wherein G represents a non-aromatic heterocyclic ring represented by either of the formula G-4, the formula G-5, the formula G-7, the formula G-11, the formula G-13, the formula G-14, the formula G-15, the formula G-17, the formula G-18 or the formula G-23, or a cycloalkyl ring represented by the formula G-71, 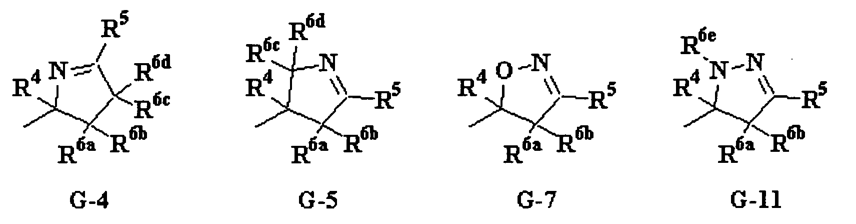 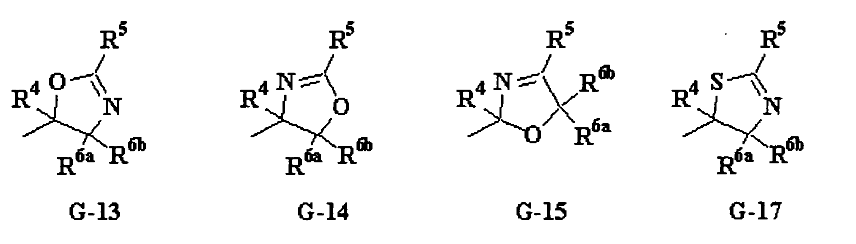 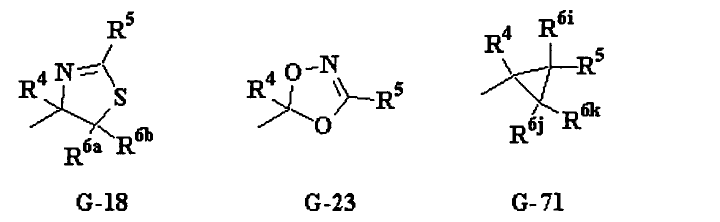 Y¹ represents a halogen atom, a C₁ to C₆ alkyl, a C₁ to C₆ haloalkyl, a hydroxy(C₁ to C₆)alkyl, a C₁ to C₃ alkoxy(C₁ to C₃)alkyl, a C₁ to C₆ alkoxy, a C₁ to C₆ haloalkoxy, a C₁ to C₆ alkylthio or a C₁ to C₆ haloalkylthio,
Y² represents a halogen atom, a C₁ to C₆ alkyl, a C₁ to C₆ alkoxy or a C₁ to C₆ alkylthio, and when n1 represents 2, each of Y²s may be the same with each other or may be different from each other,
R⁴ represents
(a). a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a non-aromatic heterocyclic ring represented by either of the formula G-4, the formula G-5, the formula G-7, the formula G-11, the formula G-13, the formula G-15, the formula G-17, the formula G-18 or the formula G-23,
(b). a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a non-aromatic heterocyclic ring represented by the formula G-14,
(c). a halogen atom, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)-alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a cycloalkyl ring represented by the formula G-71,
R⁵ represents
(a). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a di(C₁ to C₆ alkyl)amino, pyrrolidin-1-yl, oxazolidin-3-yl, thiazolidin-3-yl, piperidin-1-yl, morpholin-1-yl, -C(R⁹)=NOR¹¹, Q, 1-naphthyl, 2-naphthyl, L, M-4, M-5, M-8, M-9, M-14 to M-18 or M-19, when G represents a non-aromatic heterocyclic ring represented by either of the formula G-4, the formula G-5, the formula G-7, the formula G-11, the formula G-13, the formula G-14, the formula G-15, the formula G-17, the formula G-18 or the formula G-23,
(b). a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, -C(R⁹)=NOR¹¹, -Si(R¹³)(R¹⁴)R¹², Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a cycloalkyl ring represented by the formula G-71, and R⁴ represents a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, -C(R⁹)=NOR¹¹, -Si(R¹³)(R¹⁴)R¹², M-4, M-5, M-8, M-9, M-14 to M-18 or M-19, when G represents a cycloalkyl ring represented by the formula G-71, and R⁴ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, M-4, M-5, M-8, M-9, M-14 to M-18 or M-19,
(d). a hydrogen atom, a halogen atom, cyano, a C₁ to C₆ alkyl or a C₁ to C₆ haloalkyl, when G represents a cycloalkyl ring represented by the formula G-71, and R⁴ represents Q, 1-naphthyl, 2-naphthyl, L-1 to L-4, L-8 to L-13, L-15 to L-23, L-30 to L-35, L-45 to L-52 or L-53,
R^{6a}, R^{6b}, R^{6c} and R^{6d} each represent a hydrogen atom,
R^{6e} represents a C₁ to C₆ alkyl, a C₁ to C₆ alkylsulfonyl, a C₁ to C₆ alkoxycarbonyl, a di(C₁ to C₆ alkyl)phosphoryl or Q,
R⁶ⁱ represents a hydrogen atom, a halogen atom or a C₁ to C₆ alkyl,
R^{6j} and R^{6k} each independently represent a hydrogen atom, a halogen atom, cyano or methyl,
Q represents a phenyl group which may be substituted represented by either of Q-1 to Q-11, 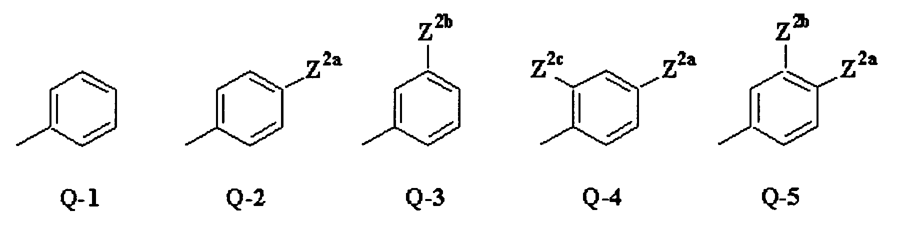 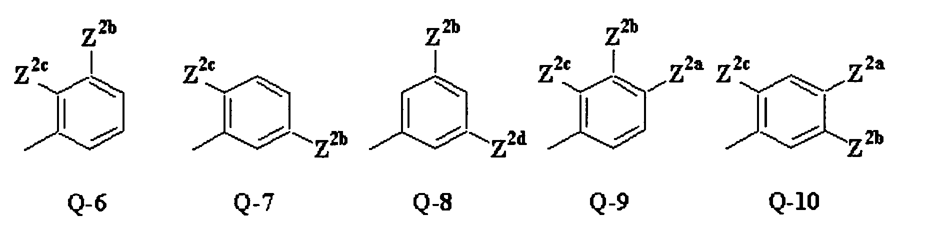 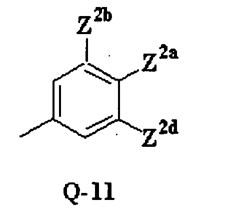 L represents an aromatic heterocyclic ring represented by either of the formula L-1 to the formula L-58, 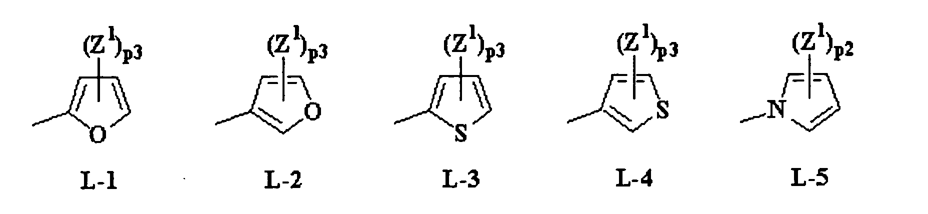 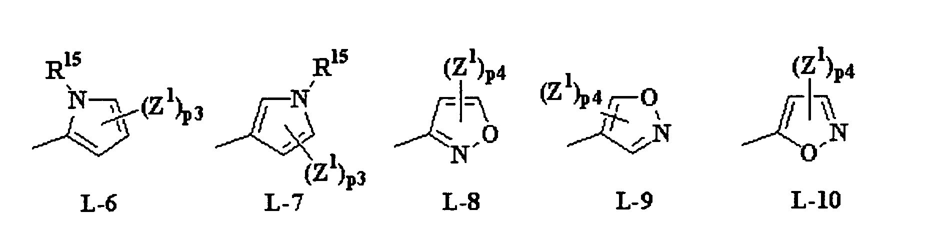 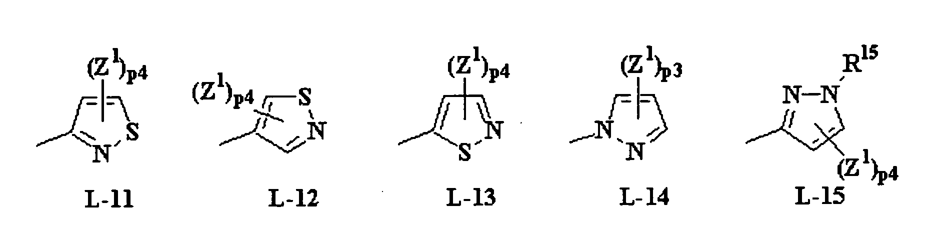 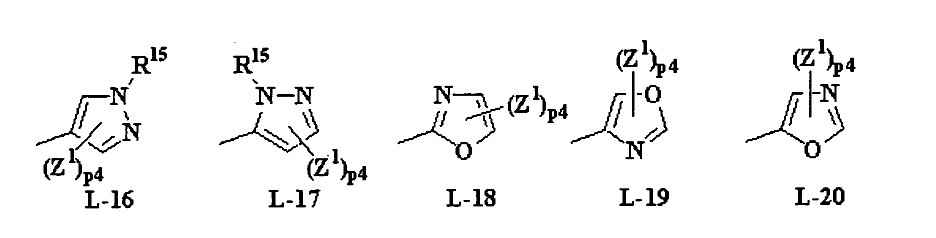 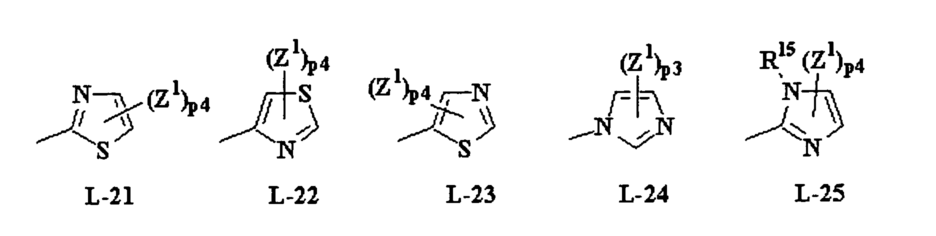 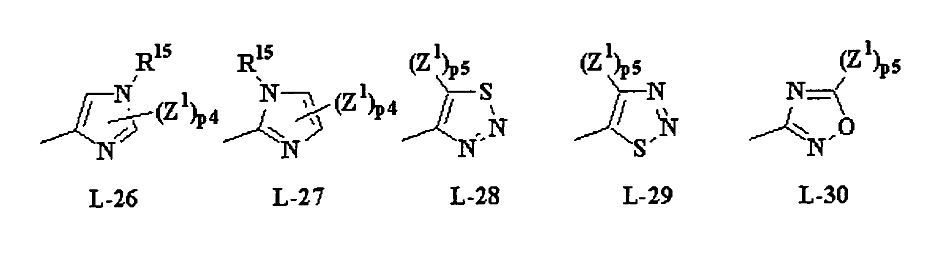 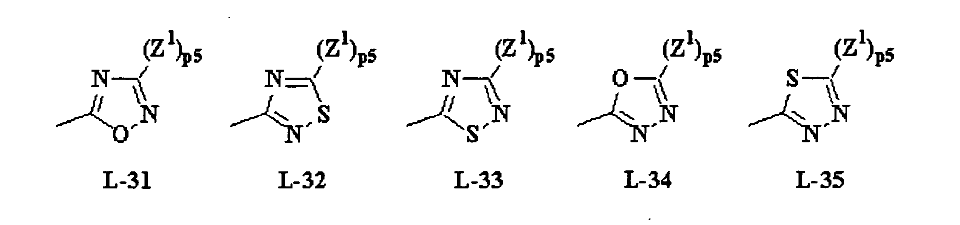 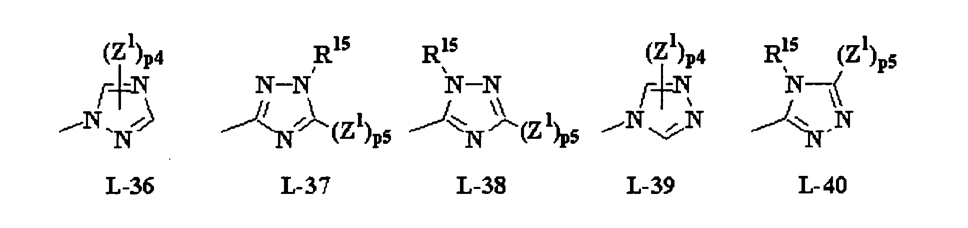 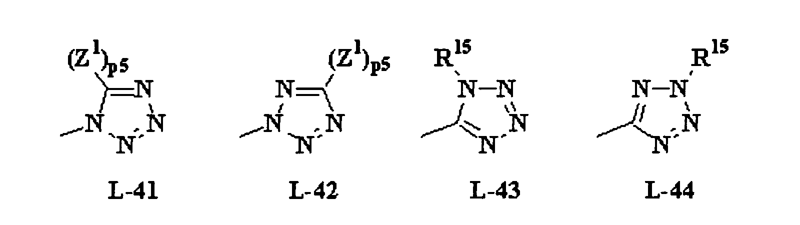 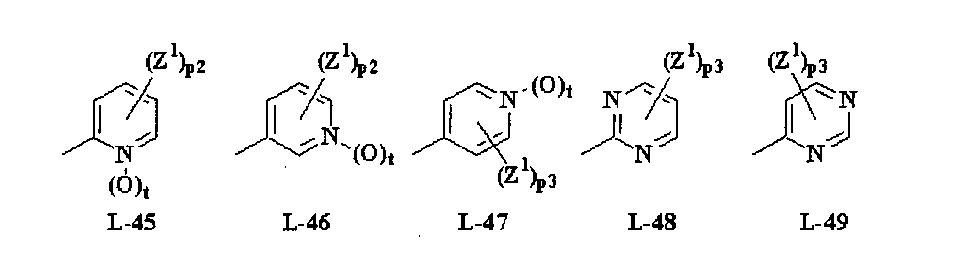 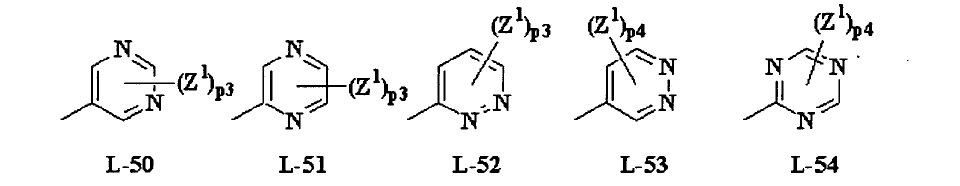 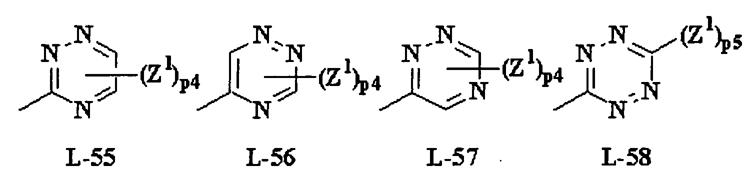 M-4, M-5, M-8, M-9, M-14 to M-18 or M-19 each represent the following saturated heterocyclic ring, 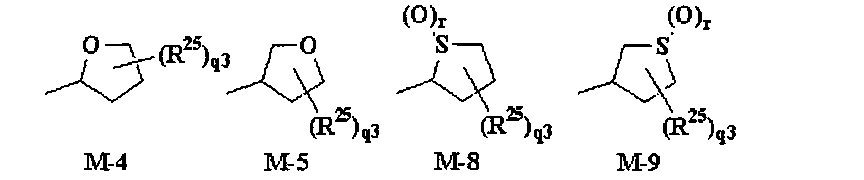 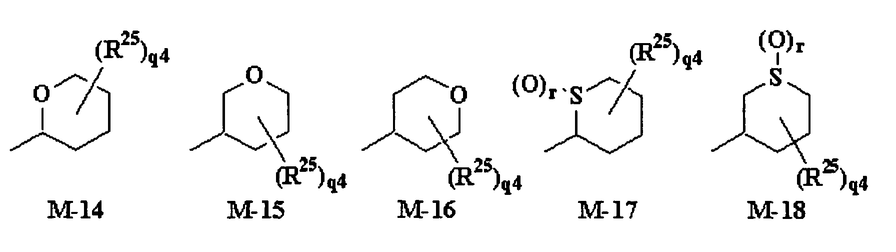 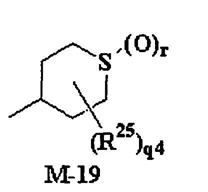 Z¹ represents a halogen atom, cyano, nitro, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a C₁ to C₆ alkylamino or di(C₁ to C₆ alkyl)amino, when p1, p2, p3 or p4 represents an interger of 2 or more, each of Z¹s may be the same with each other or may be different from each other,
Z^{2a}, Z^{2b} and R^{2d} each independently represent a halogen atom, cyano, nitro, amino, a C₁ to C₆ alkyl, a C₁ to C₆ haloalkyl, a C₁ to C₃ alkoxy(C₁ to C₃)alkyl, a C₁ to C₃ haloalkoxy(C₁ to C₃)alkyl, a C₁ to C₃ alkylthio(C₁ to C₃)alkyl, a C₁ to C₃ haloalkylthio(C₁ to C₃)alkyl, a C₁ to C₃ alkylsulfinyl(C₁ to C₃)alkyl, a C₁ to C₃ haloalkylsulfinyl(C₁ to C₃)alkyl, a C₁ to C₃ alkylsulfonyl(C₁ to C₃)alkyl, a C₁ to C₃ haloalkylsulfonyl(C₁ to C₃)alkyl, a cyano(C₁ to C₆)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylsulfonyloxy, a C₁ to C₆ haloalkylsulfonyloxy, phenoxy which may be substituted by (Z¹)ₚ₁, 5-trifluoromethylpyridin-2-yloxy, 3-chloro-5-trifluoromethylpyridin-2-yloxy, a C₁ to C₆ alkylthio, a C₁ to C₆ haloalkylthio, a C₃ to C₈ cycloalkylthio, a C₁ to C₆ alkylsulfinyl, a C₁ to C₆ haloalkylsulfinyl, a C₃ to C₈ cycloalkylsulfinyl, a C₁ to C₆ alkylsulfonyl, a C₁ to C₆ haloalkylsulfonyl, a C₃ to C₈ cycloalkylsulfonyl, a C₁ to C₆ alkylamino, a di(C₁ to C₆ alkyl)amino, a C₁ to C₆ alkylaminosulfonyl, a di(C₁ to C₆ alkyl)aminosulfonyl, -C(O)NH₂, a C₁ to C₆ alkylaminocarbonyl, a di(C₁ to C₆)alkylaminocarbonyl, -C(S)NH₂ or a tri(C₁ to C₆ alkyl)silyl,
and further Z^{2a} and Z^{2b} or Z^{2a} and Z^{2d} may form -OCF₂O-, so that they may form a 5-membered ring with the carbon atoms to which they are bonded,
Z^{2c} represents a halogen atom, a C₁ to C₆ alkyl or a C₁ to C₆ haloalkyl,
R⁹ represents a C₁ to C₆ alkyl, a C₁ to C₆ haloalkyl, a C₃ to C₈ cycloalkyl or phenyl which may be substituted by (Z¹)ₚ₁,
R¹¹ represents a C₁ to C₆ alkyl or a C₁ to C₆ haloalkyl, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group,
R¹² represents a C₁ to C₆ alkyl or phenyl which may be substituted by (Z¹)ₚ₁,
R¹³ and R¹⁴ each independently represent a C₁ to C₆ alkyl,
R¹⁵ represents a C₁ to C₆ alkyl or phenyl which may be substituted by (Z¹)ₚ₁,
R²⁵ represents a C₁ to C₄ alkyl,
n1 represents an integer of 0 to 3,
p1 represents an integer of 1 to 5,
p2 represents an integer of 0 to 4,
p3 represents an integer of 0 to 3,
p4 represents an integer of 0 to 2,
p5 represents an integer of 0 or 1,
q3 represents an integer of 0 to 2,
q4 represents an integer of 0 to 2,
r represents an integer of 0 to 2,
t represents an integer of 0 or 1.]
or a salt of these.

[17] A noxious organism controlling agent which comprises one or more kinds selected from the group consisting of a substituted benzanilide compound and a salt thereof of the above-mentioned [1] to [15] as an effective ingredient.

[18] An agricultural chemical which comprises one or more kinds selected from the group consisting of a substituted benzanilide compound and a salt thereof of the above-mentioned [1] to [15] as an effective ingredient.

[19] A insecticide or acaricide which comprises one or more kinds selected from the group consisting of a substituted benzanilide compound and a salt thereof of the above-mentioned [1] to [15] as an effective ingredient.

### Effects of the invention

Due to use of an insecticide or a fungicide for a long period of time, noxious insects have obtained resistivity thereto in recent years, so that it becomes difficult to prevent them by the conventional insecticides or fungicides. Also, in a part of the insecticides, there exist those having high toxicity or having long residual activity in an environment, so that there is a problem that they disturb an ecological system. On the other hand, the compounds of the present invention have excellent insecticidal and acaricidal activities against many agricultural noxious insects and spider mites, and show sufficient preventing effects against noxious insects which obtained resistivity to the conventional insecticides. Moreover, the compounds do not substantially show bad influence against mammals, fishes and useful insects, and are low residual activity so that load against the environment is low.

Accordingly, the present invention can provide a useful and novel noxious organism controlling agent.

### Best mode for carrying out the invention

In the compounds included in the present invention, there are some cases in which geometric isomers of E-isomer and Z-isomer depending on the kind of the substituent(s), and the present invention includes these E-isomer, Z-isomer or a mixture of E-isomer and Z-isomer in an optional ratio. Also, in the compounds contained in the present invention, there exist optical isomers depending on the presence of one or more asymmetric carbon atoms, the present invention includes all the optical isomers or racemic mixtures. Moreover, in the compounds of the present invention represented by the formula (1), when R¹ or R² is a hydrogen atom, it can conceive the presence of tautomeric isomers represented by the following formula, and the present invention also includes these structures.

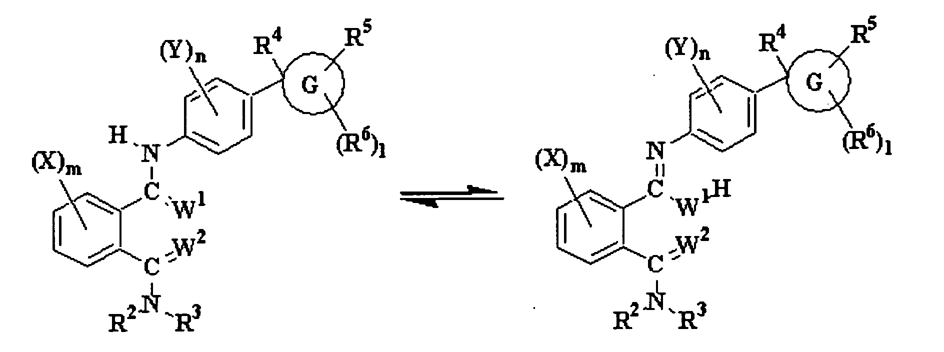

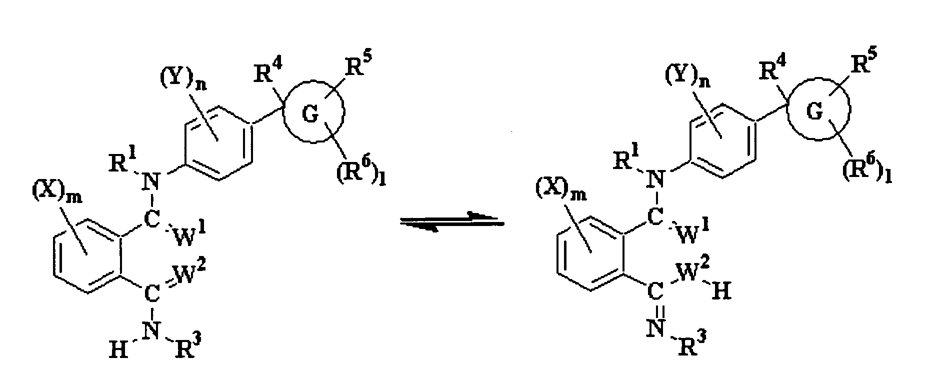

Among the compounds included in the present invention, those which can be an acid addition salt according to the conventional method may include, for example, a salt of a hydrohalogenic acid such as hydrofluoric acid, hydrochloric acid, hydrobromic acid , hydroiodic acid, etc., a salt of an inorganic acid such as nitric acid, sulfuric acid, phosphoric acid, chloric acid, perchloric acid, etc., a salt of a sulfonic acid such as methanesulfonic acid, ethanesulfonic acid, trifluoromethanesulfonic acid, benzenesulfonic acid, p-toluenesulfonic acid, etc., a salt of a carbonic acid such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, citric acid, etc., or a salt of an amino acid such as glutamic acid, aspartic acid, etc.

Or else, among the compounds included in the present invention, those which can be made a metal salt according to the conventional manner may include, for example, a salt of an alkali metal such as lithium, sodium and potassium, a salt of an alkaline earth metal such as calcium, barium and magnesium or a salt of aluminum.

Next, specific examples of the respective substituent(s) shown in the present specification are shown below. Here, n- means normal, i- means iso, s- means secondary and t- means tertiary, respectively, and Ph means phenyl.

As G which is a 5-membered or 6-membered non-aromatic heterocyclic ring containing at least one atom selected from an oxygen atom, sulfur atom and nitrogen atom, and existing at least one double bond in the ring in the compounds of the present invention, there may be mentioned, for example, a non-aromatic heterocyclic ring represented by the formula G-1 to the formula G-54, etc.

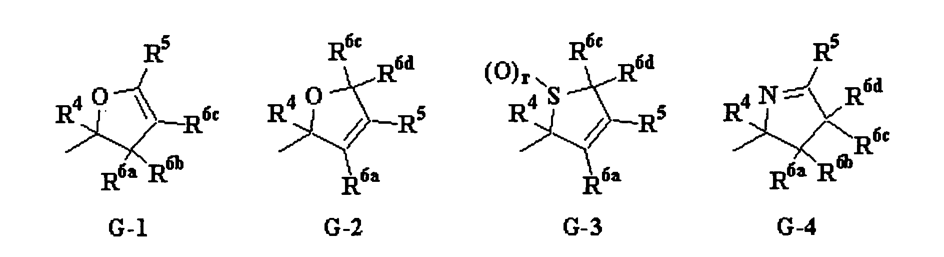

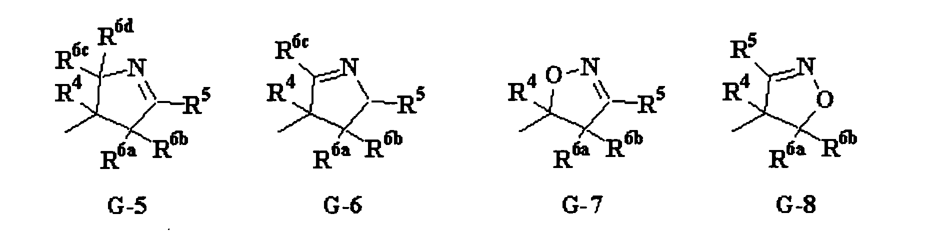

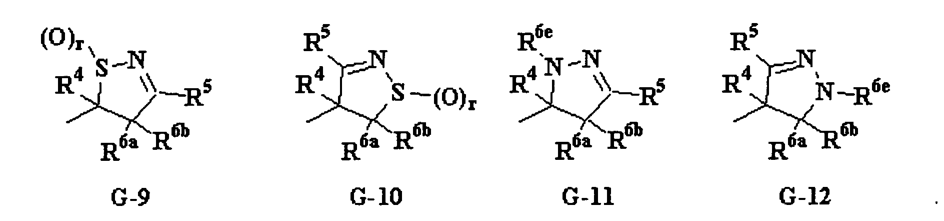

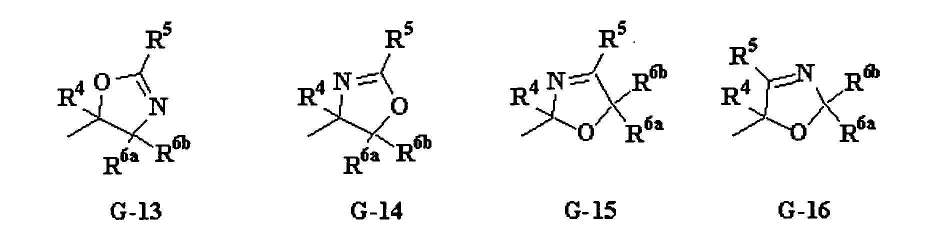

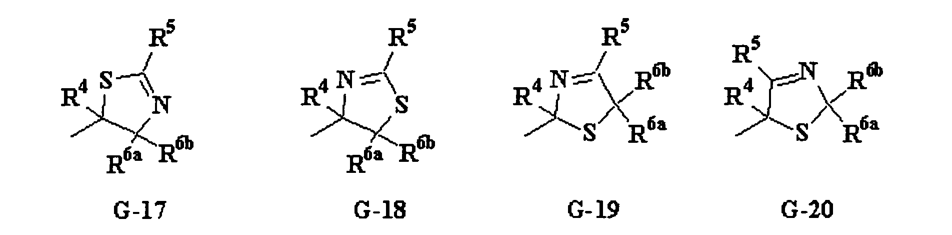

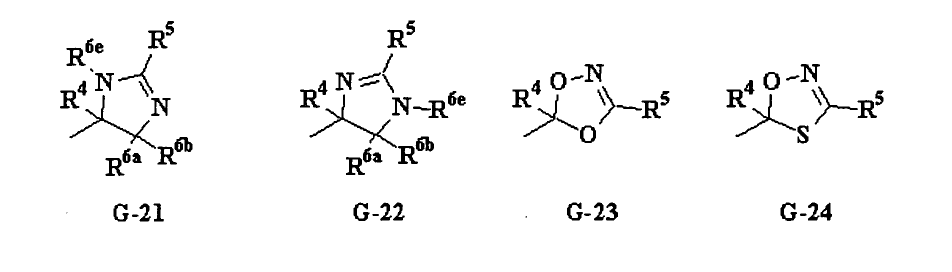

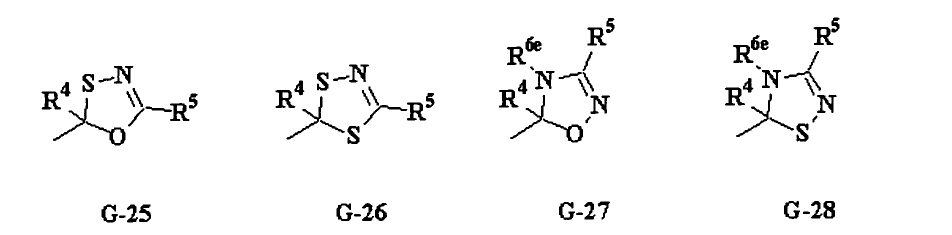

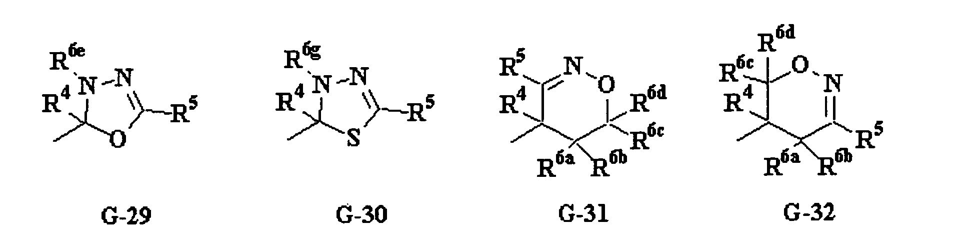

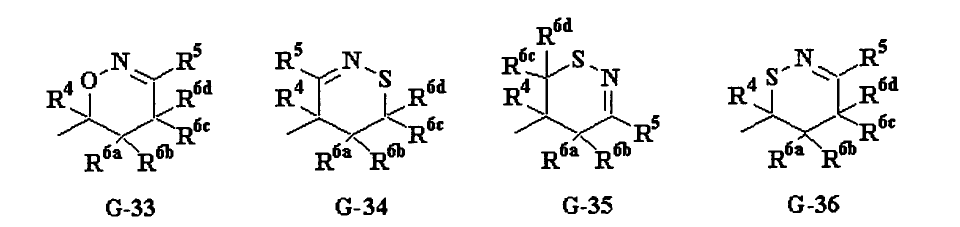

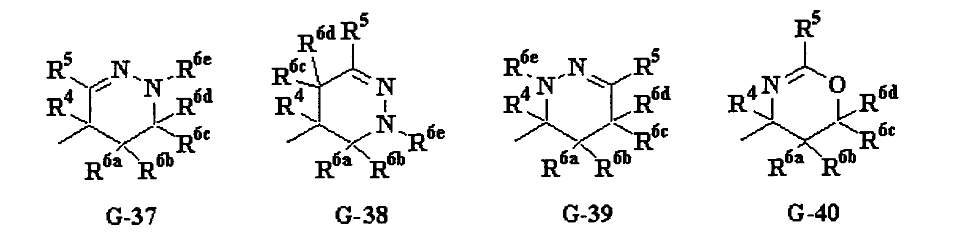

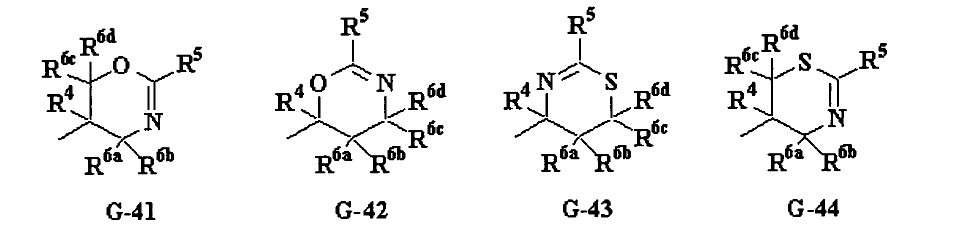

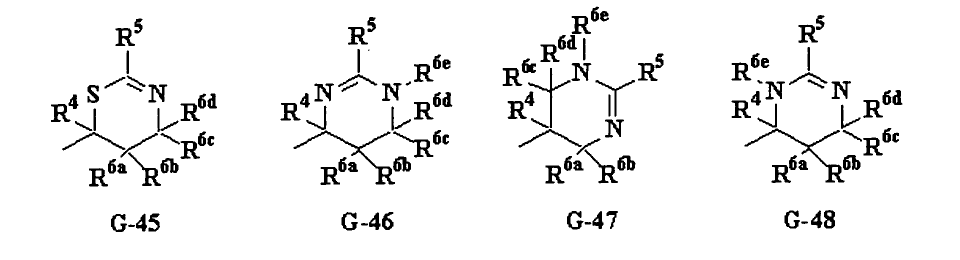

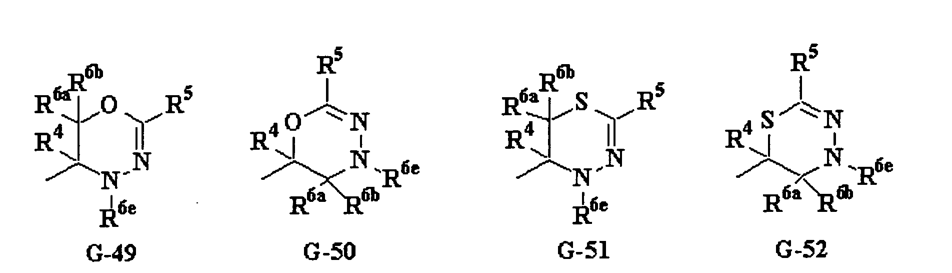

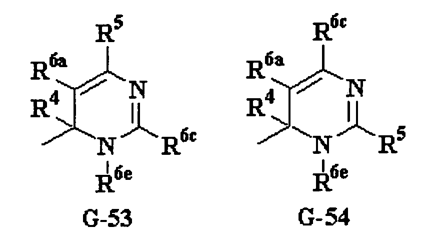

As G which is a 5-membered or 6-membered saturated heterocyclic ring containing two atoms selected from an oxygen atom, sulfur atom and nitrogen atom in the compounds of the present invention, there may be mentioned, for example, a saturated heterocyclic ring represented by the formula G-55 to the formula G-70, and the like.

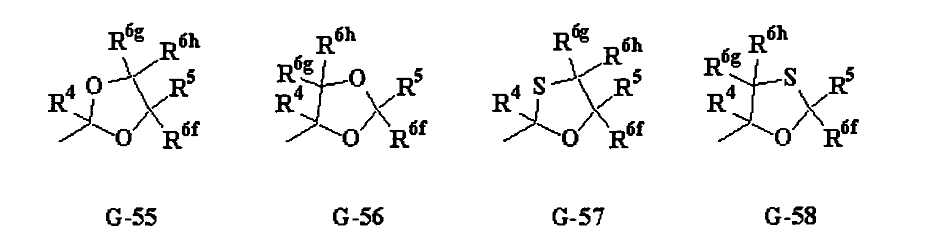

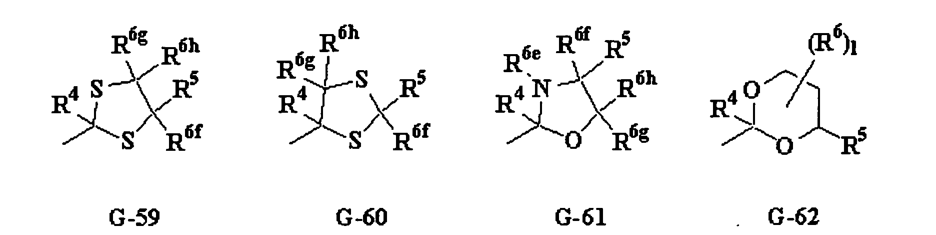

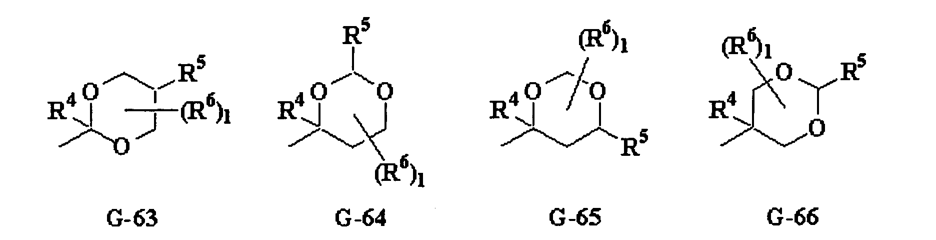

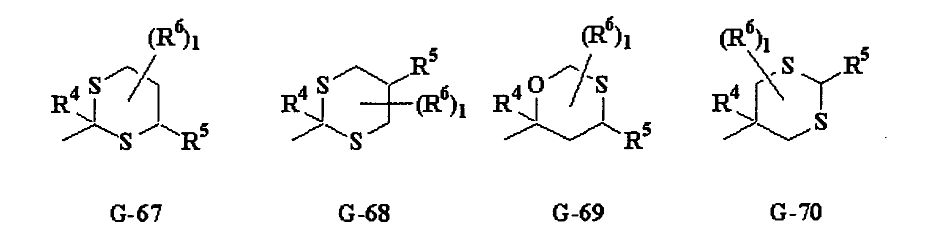

As G which is a 3-membered to 6-membered cycloalkyl ring in the compounds of the present invention, there may be mentioned, for example, a cycloalkyl ring represented by the formula G-71 to the formula G-78, and the like.

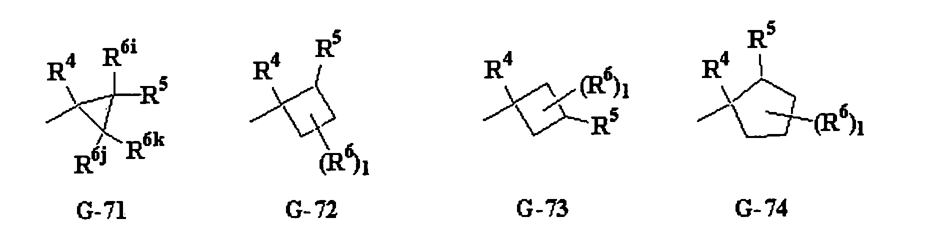

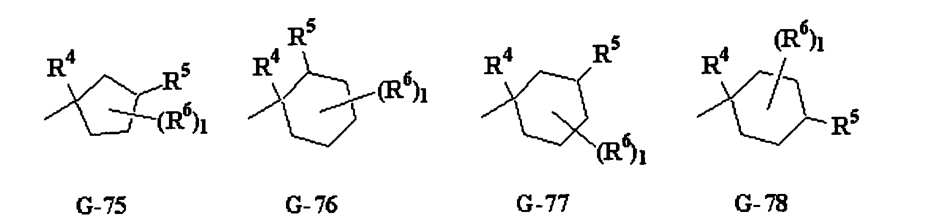

As the halogen atom in the compounds of the present invention, there may be mentioned a fluorine atom, chlorine atom, bromine atom and iodine atom. Incidentally, the expression "halo" in the present specification also represents these halogen atoms.

The expression Cₐ to C_{b} alkyl in the present specification represents a linear or branched hydrocarbon group having a to b carbon atoms, and there may be mentioned, for example, methyl group, ethyl group, n-propyl group, i-propyl group, n-butyl group, s-butyl group, i-butyl group, t-butyl group, n-pentyl group, 1-methylbutyl group, 2-methylbutyl group, 3-methylbutyl group, 1-ethylpropyl group, 1,1-dimethylpropyl group, 1,2-dimethylpropyl group, neopentyl group, n-hexyl group, 1-methylpentyl group, 2-methylpentyl group, 3-methylpentyl group, 4-methylpentyl group, 1-ethylbutyl group, 2-ethylbutyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 1,1,2-trimethylpropyl group, 1,2,2-trimethylpropyl group, 1-ethyl-1-methylpropyl group, 1-ethyl-2-methylpropyl group, heptyl group, 1-methylhexyl group, 1,1-dimethylpentyl group, 1,1,3-trimethylbutyl group, octyl group, 1-methylheptyl group, nonyl group, 1-methyloctyl group, 1,1-dimethylheptyl group, decyl group, 1-methylnonyl group, undecyl group, 1-methyldecyl group, dodecyl group, 1-methylundecyl group, etc. as specific examples, and each may be selected from the range of the carbon numbers as designated, respectively.

The expression Cₐ to C_{b} haloalkyl in the present specification represents a linear or branched hydrocarbon group having a to b carbon atoms in which the hydrogen atom bonded to the carbon atom is optionally substituted by a halogen atom, and when it is substituted by 2 or more halogen atoms, these halogen atoms may be the same with each other or may be different from each other. Specific examples may include, for example, fluoromethyl group, chloromethyl group, bromomethyl group, difluoromethyl group, dichloromethyl group, trifluoromethyl group, trichloromethyl group, chlorodifluoromethyl group, bromodifluoromethyl group, 2-fluoroethyl group, 1-chloroethyl group, 2-chloroethyl group, 1-bromoethyl group, 2-bromoethyl group, 2,2-difluoroethyl group, 1,2-dichloroethyl group, 2,2-dichloroethyl group, 2-bromo-2-chloroethyl group, 2,2,2-trifluoroethyl group, 2,2,2-trichloroethyl group, 1,1,2,2-tetrafluoroethyl group, 2-chloro-1,1,2-trifluoroethyl group, 2-bromo-1,1,2-trifluoroethyl group, pentafluoroethyl group, 2-chloro-1,1,2,2-tetrafluoroethyl group, 1-chloro-1,2,2,2-tetrafluoroethyl group, 2-bromo-1,1,2,2-tetrafluoroethyl group, 2,2-dichloro-1,1,2-trifluoroethyl group, 2,2,2-trichloro-1,1-difluoroethyl group, 1-chloropropyl group, 2-chloropropyl group, 3-chloropropyl group, 3-bromopropyl group, 2-fluoro-1-methylethyl group, 2-chloro-1-methylethyl group, 2-bromo-1-methylethyl group, 2,2,3,3,3-pentafluoropropyl group, 1,1,2,3,3,3-hexafluoropropyl group, 2,2,2-trifluoro-1-trifluoromethylethyl group, heptafluoropropyl group, 1,2,2,2-tetrafluoro-1-trifluoromethylethyl group, 2-bromo-1,1,2,3,3,3-hexafluoropropyl group, 4-chlorobutyl group, 2-chloro-1,1-dimethylethyl group, 2-bromo-1,1-dimethylethyl group, 3,3,3-trifluoro-1-methylpropyl group, nonafluorobutyl group, 5-chloropentyl group, 2,3-dibromo-1,1-dimethylpropyl group, 6-chlorohexyl group, tridecafluorohexyl group, 7-bromoheptyl group, 8-chlorooctyl group, 9-bromononyl group, 10-chlorodecyl group, 11-bromoundecyl group, 12-bromododecyl group, etc., and each may be selected from the range of the carbon numbers as designated, respectively.

The expression hydroxy(Cₐ to C_{b}) alkyl in the present specification represents a linear or branched alkyl group having a to b carbon atoms in which the hydrogen atom bonded to the carbon atom is optionally substituted by a hydroxyl group, and there may be specifically mentioned, for example, hydroxymethyl group, 1-hydroxyethyl group, 2-hydroxyethyl group, 3-hydroxypropyl group, 2-hydroxy-1-methylethyl group, 4-hydroxybutyl group, 2-hydroxy-1,1-dimethylethyl group, 3-hydroxy-1-methylpropyl group, 6-hydroxyhexyl group, etc., and each may be selected from the range of the carbon numbers as designated, respectively.

The expression cyano(Cₐ to C_{b}) alkyl in the present specification represents a linear or branched alkyl group having a to b carbon atoms in which the hydrogen atom bonded to the carbon atom is optionally substituted by a cyano group, and there may be specifically mentioned, for example, cyanomethyl group, 1-cyanoethyl group, 2-cyanoethyl group, 3-cyanopropyl group, 1-cyano-1-methylethyl group, 4-cyanobutyl group, 2-cyano-1,1-dimethylethyl group, 1-cyano-1-methylpropyl group, 6-cyanohexyl group, etc., and each may be selected from the range of the carbon numbers as designated, respectively.

The expression Cₐ to C_{b} cycloalkyl in the present specification represents a cyclic hydrocarbon group having a to b carbon atoms, and may form a monocyclic or heterocyclic structure from a 3-membered ring to a 6-membered ring. Also, respective rings may be optionally substituted by an alkyl group(s) in the range of the designated number of the carbon atoms. There may be specifically mentioned, for example, cyclopropyl group, 1-methylcyclopropyl group, 2-methylcyclopropyl group, 2,2-dimethylcyclopropyl group, 2,2,3,3-tetramethylcyclopropyl group, cyclobutyl group, cyclopentyl group, 1-methylcyclopentyl group, 2-methylcyclopentyl group, 3-methylcyclopentyl group, cyclohexyl group, 1-methylcyclohexyl group, 2-methylcyclohexyl group, 3-methylcyclohexyl group, 4-methylcyclohexyl group, bicyclo[2.2.1]heptan-2-yl group, etc., and each may be selected from the range of the carbon numbers as designated, respectively.

The expression Cₐ to C_{b} halocycloalkyl in the present specification represents a cyclic hydrocarbon group having a to b carbon atoms in which the hydrogen atom bonded to the carbon atom is optionally substituted by a halogen atom, and may form a monocyclic or heterocyclic structure from a 3-membered ring to a 6-membered ring. Also, respective rings may be optionally substituted by an alkyl group(s) in the range of the designated number of the carbon atoms, substitution by the halogen atom may be at the ring structure portion, a side chain portion, or may be both of the portions, and further, when it is substituted by 2 or more halogen atoms, these halogen atoms may be the same with each other or may be different from each other. There may be specifically mentioned, for example, 1-bromocyclopropyl group, 2,2-dichlorocyclopropyl group, 2,2-dibromocyclopropyl group, 2,2-difluoro-1-methylcyclopropyl group, 2,2-dichloro-1-methylcyclopropyl group, 2,2-dibromo-1-methylcyclopropyl group, 2,2-dichloro-3,3-dimethylcyclopropyl group, 2,2,3,3-tetrafluorocyclobutyl group, 2-fluorocyclohexyl group, 2-chlorocyclohexyl group, 3-chlorocyclohexyl group, 4-chlorocyclohexyl group, 2-trifluoromethylcyclohexyl group, 3-trifluoromethylcyclohexyl group, 4-trifluoromethylcyclohexyl group, etc., and each may be selected from the range of the carbon numbers as designated, respectively.

The expression Cₐ to C_{b} alkenyl in the present specification represents an unsaturated hydrocarbon group which is linear or branched having a to b carbon atoms, and having one or more double bonds in the molecule, and there may be specifically mentioned, for example, vinyl group, 1-propenyl group, 1-methylethenyl group, 2-propenyl group, 1-butenyl group, 1-methyl-1-propenyl group, 2-methyl-1-propenyl group, 2-butenyl group, 1-methyl-2-propenyl group, 2-methyl-2-propenyl group, 3-butenyl group, 1,3-butadienyl group, 1-methyl-2-butenyl group, 2-methyl-2-butenyl group, 3-methyl-2-butenyl group, 1,1-dimethyl-2-propenyl group, 2-hexenyl group, 2-methyl-2-pentenyl group, 1,3-dimethyl-2-butenyl group, 1,1,2-trimethyl-2-propenyl group, 1,1-dimethyl-3-butenyl group, 2,4-hexadienyl group, 2-heptenyl group, 2-octenyl group, 1-methyl-2-heptenyl group, 2-undecenyl group, 10-undecenyl group, 2-dodecenyl group, 11-dodecenyl group, etc., and each may be selected from the range of the carbon numbers as designated, respectively.

The expression Cₐ to C_{b} haloalkenyl in the present specification represents an unsaturated hydrocarbon group which is linear or branched having a to b carbon atoms, and having one or more double bonds in the molecule in which the hydrogen atom bonded to the carbon atom is optionally substituted by a halogen atom. At this time, when it is substituted by 2 or more halogen atoms, these halogen atoms may be the same with each other or may be different from each other. There may be specifically mentioned, for example, 2-chlorovinyl group, 2-bromovinyl group, 2,2-dichlorovinyl group, 2,2-dibromovinyl group, 3-bromo-2-propenyl group, 1-chloromethylvinyl group, 2-bromo-1-methylvinyl group, 1-trifluoromethylvinyl group, 2-chloro-3,3,3-trifluoro-1-propenyl group, 1-trifluoromethyl-2,2-difluorovinyl group, 2-chloro-2-propenyl group, 3,3-difluoro-2-propenyl group, 3,3-dichloro-2-propenyl group, 2,3,3-trifluoro-2-propenyl group, 2,3,3-trichloro-2-propenyl group, 4,4-difluoro-3-butenyl group, 3,4,4-trifluoro-3-butenyl group, 3-chloro-4,4,4-trifluoro-2-butenyl group, 3,3,3-trifluoro-1-methyl-1-propenyl group, 3,3,3-trifluoro-2-trifluoromethyl-1-propenyl group, 5,5-difluoro-4-pentenyl group, 4,5,5-trifluoro-4-pentenyl group, 4,4,5,5,6,6,6-heptafluoro-2-hexenyl group, 2-perfluorohexylethenyl group, etc., and each may be selected from the range of the carbon numbers as designated, respectively.

The expression Cₐ to C_{b} cycloalkenyl in the present specification represents a cyclic unsaturated hydrocarbon group having a to b carbon atoms and having 1 or more double bonds, and may form a monocyclic or heterocyclic structure from a 3-membered ring to a 6-membered ring. Also, respective rings may be optionally substituted by an alkyl group(s) in the range of the designated number of the carbon atoms, and the double bond may be either of the endo- or exo-form. There may be specifically mentioned, for example, cyclopenten-1-yl group, 2-cyclopenten-1-yl group, 3-cyclo-penten-1-yl group, cyclohexen-1-yl group, 2-cyclohexen-1-yl group, 3-cyclohexen-1-yl group, 2-methyl-2-cyclohexen-1-yl group, 3-methyl-2-cyclohexen-1-yl group, bicyclo-[2.2.1]-5-hepten-2-yl group, etc., and each may be selected from the range of the carbon numbers as designated, respectively.

The expression Cₐ to C_{b} halocycloalkenyl in the present specification represents a cyclic unsaturated hydrocarbon group having a to b carbon atoms and having 1 or more double bonds in which the hydrogen atom bonded to the carbon atom is optionally substituted by a halogen atom, and may form a monocyclic or heterocyclic structure from a 3-membered ring to a 6-membered ring. Also, respective rings may be optionally substituted by an alkyl group(s) in the range of the designated number of the carbon atoms, and the double bond may be either of the endo- or exo-form. Also, substitution by the halogen atom may be at the ring structure portion, a side chain portion, or may be both of the portions, and further, when it is substituted by 2 or more halogen atoms, these halogen atoms may be the same with each other or may be different from each other. There may be specifically mentioned, for example, 2-chlorobicyclo[2.2.1]-5-hepten-2-yl group, etc., and each may be selected from the range of the carbon numbers as designated, respectively.

The expression Cₐ to C_{b} alkynyl in the present specification represents a linear or branched unsaturated hydrocarbon group having one or more triple bonds in the molecule with a to b carbon atoms, and there may be specifically mentioned, for example, ethynyl group, 1-propynyl group, 2-propynyl group, 1-methyl-2-propynyl group, 2-butynyl group, 3-butynyl group, 2-pentynyl group, 1-methyl-2-butynyl group, 1-methyl-3-butynyl group, 1,1-dimethyl-2-propynyl group, 1-hexynyl group, 3,3-dimethyl-1-butynyl group, 2-hexynyl group, 1-methyl-2-pentynyl group, 1,1-dimethyl-2-butynyl group, 2-heptynyl group, 1,1-dimethyl-2-pentynyl group, 2-octynyl group, 2-nonynyl group, 2-decynyl group, 2-undecynyl group, etc., and each may be selected from the range of the carbon numbers as designated, respectively.

The expression Cₐ to C_{b} haloalkynyl in the present specification represents a linear or branched unsaturated hydrocarbon group having one or more triple bonds in the molecule with a to b carbon atoms in which the hydrogen atom bonded to the carbon atom is optionally substituted by a halogen atom. At this time, when it is substituted by two or more halogen atoms, these halogen atoms may be the same with each other or may be different from each other. There may be specifically mentioned, for example, 2-chloroethynyl group, 2-bromoethynyl group, 2-iodoethynyl group, 3-chloro-2-propynyl group, 3-bromo-2-propynyl group, 3-iodo-2-propynyl group, 3,3,3-trifluoro-1-propynyl group, 3-chloro-1-methyl-2-propynyl group, 3-bromo-1-methyl-2-propynyl group, 3-iodo-1-methyl-2-propynyl group, 3-chloro-1,1-dimethyl-2-propynyl group, 3-bromo-1,1-dimethyl-2-propynyl group, 3-iodo-1,1-dimethyl-2-propynyl group, etc., and each may be selected from the range of the carbon numbers as designated, respectively.

The expression Cₐ to C_{b} alkoxy in the present specification represents an alkyl-O- group having the above-mentioned meaning with a to b carbon atoms, and there may be specifically mentioned, for example, methoxy group, ethoxy group, n-propyloxy group, i-propyloxy group, n-butyloxy group, s-butyloxy group, i-butyloxy group, t-butyloxy group, n-pentyloxy group, 1-methylbutyloxy group, 2-methylbutyloxy group, 3-methylbutyloxy group, 1-ethylpropyloxy group, 1,1-dimethylpropyloxy group, 1,2-dimethylpropyloxy group, neopentyloxy group, n-hexyloxy group, 1,1-dimethylbutyloxy group, etc., and each may be selected from the range of the carbon numbers as designated, respectively.

The expression Cₐ to C_{b} haloalkoxy in the present specification represents a haloalkyl-O- group having the above-mentioned meaning with a to b carbon atoms, and there may be specifically mentioned, for example, difluoromethoxy group, trifluoromethoxy group, chlorodifluoromethoxy group, bromodifluoromethoxy group, 2-fluoroethoxy group, 2-chloroethoxy group, 2,2,2-trifluoroethoxy group, 1,1,2,2,-tetrafluoroethoxy group, 2-chloro-1,1,2-trifluoroethoxy group, 2-bromo-1,1,2-trifluoroethoxy group, pentafluoroethoxy group, 2-bromo-1,1,2,2-tetrafluoroethoxy group, 2,2-dichloro-1,1,2-trifluoroethoxy group, 2,2,2-trichloro-1,1-difluoroethoxy group, 2-chloropropyloxy group, 3-chloropropyloxy group, heptafluoropropyloxy group, 2,2,2-trifluoro-1-trifluoromethylethoxy group, 2,2,3,3-tetrafluoropropyloxy group, 1,1,2,3,3,3-hexafluoropropyloxy group, 2-bromo-1,1,2,3,3,3-hexafluoropropyloxy group, etc., and each may be selected from the range of the carbon numbers as designated, respectively.

The expression Cₐ to C_{b} alkenyloxy in the present specification represents an alkenyl-O- group having the above-mentioned meaning with a to b carbon atoms, and there may be specifically mentioned, for example, 2-propenyloxy group, 2-butenyloxy group, 2-methyl-2-propenyloxy group, 3-methyl-2-butenyloxy group, etc., and each may be selected from the range of the carbon numbers as designated, respectively.

The expression Cₐ to C_{b} haloalkenyloxy in the present specification represents a haloalkenyl-O- group having the above-mentioned meaning with a to b carbon atoms, and there may be specifically mentioned, for example, 2-chloro-2-propenyl group, 3-chloro-2-propenyl group, 3,3-difluoro-2-propenyl group, 3,3-dichloro-2-propenyl group, 2,3,3-trifluoro-2-propenyl group, etc., and each may be selected from the range of the carbon numbers as designated, respectively.

The expression Cₐ to C_{b} alkylthio in the present specification represents an alkyl-S- group having the above-mentioned meaning with a to b carbon atoms, and there may be specifically mentioned, for example, methylthio group, ethylthio group, n-propylthio group, i-propylthio group, n-butylthio group, s-butylthio group, i-butylthio group, t-butylthio group, n-pentylthio group, 1-methylbutylthio group, 2-methylbutylthio group, 3-methylbutylthio group, 1-ethylpropylthio group, 1,1-dimethylpropylthio group, 1,2-dimethylpropylthio group, neopentylthio group, n-hexylthio group, etc., and each may be selected from the range of the carbon numbers as designated, respectively.

The expression Cₐ to C_{b} haloalkylthio in the present specification represents a haloalkyl-S- group having the above-mentioned meaning with a to b carbon atoms, and there may be specifically mentioned, for example, difluoromethylthio group, trifluoromethylthio group, bromodifluoromethylthio group, 2,2,2-trifluoroethylthio group, 1,1,2,2-tetrafluoroethylthio group, 1,1,2-trifluoro-2-chloroethylthio group, pentafluoroethylthio group, 2-bromo-1,1,2,2-tetrafluoroethylthio group, heptafluoropropylthio group, 1,2,2,2-tetrafluoro-1-trifluoromethylethylthio group, nonafluorobutylthio group, etc., and each may be selected from the range of the carbon numbers as designated, respectively.

The expression Cₐ to C_{b} cycloalkylthio in the present specification represents a cycloalkyl-S- group having the above-mentioned meaning with a to b carbon atoms, and there may be specifically mentioned, for example, cyclopropylthio group, cyclobutylthio group, cyclopentylthio group, cyclohexylthio group, etc., and each may be selected from the range of the carbon numbers as designated, respectively.

The expression Cₐ to C_{b} alkylsulfinyl in the present specification represents an alkyl-S(O)- group having the above-mentioned meaning with a to b carbon atoms, and there may be specifically mentioned, for example, methylsulfinyl group, ethylsulfinyl group, n-propylsulfinyl group, i-propylsulfinyl group, n-butylsulfinyl group, s-butylsulfinyl group, i-butylsulfinyl group, t-butylsulfinyl group, etc., and each may be selected from the range of the carbon numbers as designated, respectively.

The expression Cₐ to C_{b} haloalkylsulfinyl in the present specification represents a haloalkyl-S(O)- group having the above-mentioned meaning with a to b carbon atoms, and there may be specifically mentioned, for example, difluoromethylsulfinyl group, trifluoromethylsulfinyl group, bromodifluoromethylsulfinyl group, 2,2,2-trifluoroethylsulfinyl group, 2-bromo-1,1,2,2-tetrafluoroethylsulfinyl group, 1,2,2,2-tetrafluoro-1-trifluoromethylethylsulfinyl group, nonafluorobutylsulfinyl group, etc., and each may be selected from the range of the carbon numbers as designated, respectively.

The expression Cₐ to C_{b} cycloalkylsulfinyl in the present specification represents a cycloalkyl-S(O)- group having the above-mentioned meaning with a to b carbon atoms, and there may be specifically mentioned, for example, cyclopropylsulfinyl group, cyclobutylsulfinyl group, cyclopentylsulfinyl group, cyclohexylsulfinyl group, etc., and each may be selected from the range of the carbon numbers as designated, respectively.

The expression Cₐ to C_{b} alkylsulfonyl in the present specification represents an alkyl-SO₂- group having the above-mentioned meaning with a to b carbon atoms, and there may be specifically mentioned, for example, methanesulfonyl group, ethanesulfonyl group, n-propylsulfonyl group, i-propylsulfonyl group, n-butylsulfonyl group, s-butylsulfonyl group, i-butylsulfonyl group, t-butylsulfonyl group, n-pentylsulfonyl group, n-hexylsulfonyl group, etc., and each may be selected from the range of the carbon numbers as designated, respectively.

The expression Cₐ to C_{b} haloalkylsulfonyl in the present specification represents a haloalkyl-SO₂- group having the above-mentioned meaning with a to b carbon atoms, and there may be specifically mentioned, for example, difluoromethanesulfonyl group, trifluoromethanesulfonyl group, chlorodifluoromethanesulfonyl group, bromodifluoromethanesulfonyl group, 2,2,2-trifluoroethanesulfonyl group, 1,1,2,2-tetrafluoroethanesulfonyl group, 1,1,2-trifluoro-2-chloroethanesulfonyl group, etc., and each may be selected from the range of the carbon numbers as designated, respectively.

The expression Cₐ to C_{b} cycloalkylsulfonyl in the present specification represents a cycloalkyl-SO₂- group having the above-mentioned meaning with a to b carbon atoms, and there may be specifically mentioned, for example, cyclopropylsulfonyl group, cyclobutylsulfonyl group, cyclopentylsulfonyl group, cyclohexylsulfonyl group, etc., and each may be selected from the range of the carbon numbers as designated, respectively.

The expression Cₐ to C_{b} alkylamino in the present specification represents an amino group in which either one of the hydrogen atoms is substituted by the alkyl group having the above-mentioned meaning with a to b carbon atoms, and there may be specifically mentioned, for example, methylamino group, ethylamino group, n-propylamino group, i-propylamino group, n-butylamino group, i-butylamino group, t-butylamino group, etc., and each may be selected from the range of the carbon numbers as designated, respectively.

The expression di(Cₐ to C_{b} alkyl)amino in the present specification represents an amino group in which both of the hydrogen atoms are substituted by the alkyl group having the above-mentioned meaning with a to b carbon atoms, which may be the same with each other or may be different from each other, and there may be specifically mentioned, for example, dimethylamino group, ethyl(methyl)amino group, diethylamino group, n-propyl(methyl)amino group, i-propyl(methyl)amino group, di(n-propyl)amino group, n-butyl(methyl)amino group, i-butyl(methyl)amino group, t-butyl(methyl)amino group, etc., and each may be selected from the range of the carbon numbers as designated, respectively.

The expression Cₐ to C_{b} alkylcarbonyl in the present specification represents an alkyl-C(O)- group having the above-mentioned meaning with a to b carbon atoms, and there may be specifically mentioned, for example, CH₃C(O)- group, CH₃CH₂C(O)-group, CH₃CH₂CH₂C(O)- group, a (CH₃)₂CHC(O)- group, CH₃(CH₂)₃C(O)- group, a (CH₃)₂CHCH₂C(O)- group, CH₃CH₂CH(CH₃)C(O)- group, a (CH₃)₃CC(O)- group, CH₃(CH₂)₄C(O)- group, CH₃(CH₂)₅C(O)- group, etc., and each may be selected from the range of the carbon numbers as designated, respectively.

The expression Cₐ to C_{b} haloalkylcarbonyl in the present specification represents a haloalkyl-C(O)- group having the above-mentioned meaning with a to b carbon atoms, and there may be specifically mentioned, for example, FCH₂C(O)- group, ClCH₂C(O)- group, F₂CHC(O)- group, Cl₂CHC(O)- group, CF₃C(O)- group, ClCF₂C(O)-group, BrCF₂C(O)- group, CCl₃C(O)- group, CF₃CF₂C(O)- group, ClCH₂CH₂CH₂C(O)-group, CF₃CF₂CF₂C(O)- group, ClCH₂C(CH₃)₂C(O)- group, etc., and each may be selected from the range of the carbon numbers as designated, respectively.

The expression Cₐ to C_{b} cycloalkylcarbonyl in the present specification represents a cycloalkyl-C(O)- group having the above-mentioned meaning with a to b carbon atoms, and there may be specifically mentioned, for example, cyclopropyl-C(O)-group, 1-methylcyclopropyl-C(O)- group, 2-methylcyclopropyl-C(O)- group, 2,2-dimethylcyclopropyl-C(O)- group, 2,2,3,3-tetramethylcyclopropyl-C(O)- group, cyclobutyl-C(O)- group, cyclobutyl-C(O)- group, cyclopentyl-C(O)- group, cyclohexyl-C(O)- group, etc., and each may be selected from the range of the carbon numbers as designated, respectively.

The expression Cₐ to C_{b} alkoxycarbonyl in the present specification represents an alkyl-O-C(O)- group having the above-mentioned meaning with a to b carbon atoms, and there may be specifically mentioned, for example, CH₃OC(O)- group, CH₃CH₂OC(O)- group, CH₃CH₂CH₂OC(O)- group, a (CH₃)₂CHOC(O)- group, CH₃(CH₂)₃OC(O)- group, a (CH₃)₂CHCH₂OC(O)- group, a (CH₃)₃COC(O)- group, etc., and each may be selected from the range of the carbon numbers as designated, respectively.

The expression Cₐ to C_{b} haloalkoxycarbonyl in the present specification represents a haloalkyl-O-C(O)- group having the above-mentioned meaning with a to b carbon atoms, and there may be specifically mentioned, for example, ClCH₂CH₂OC(O)-group, CF₃CH₂OC(O)- group, etc., and each may be selected from the range of the carbon numbers as designated, respectively.

The expression Cₐ to C_{b} alkylthiocarbonyl in the present specification represents an alkyl-S-C(O)- group having the above-mentioned meaning with a to b carbon atoms, and there may be specifically mentioned, for example, CH₃SC(O)- group, CH₃CH₂SC(O)- group, CH₃CH₂CH₂SC(O)- group, a (CH₃)₂CHSC(O)- group, CH₃(CH₂)₃SC(O)- group, a (CH₃)₂CHCH₂SC(O)- group, a (CH₃)₃CSC(O)- group, etc., and each may be selected from the range of the carbon numbers as designated, respectively.

The expression Cₐ to C_{b} alkylaminocarbonyl in the present specification represents a carbamoyl group in which either one of the hydrogen atoms is substituted by the alkyl group having the above-mentioned meaning with a to b carbon atoms, and there may be specifically mentioned, for example, CH₃NHC(O)- group, CH₃CH₂NHC(O)- group, CH₃CH₂CH₂NHC(O)- group, a (CH₃)₂CHNHC(O)- group, CH₃(CH₂)₃NHC(O)- group, a (CH₃)₂CHCH₂NHC(O)- group, CH3CH2CH(CH3)NHC(O)-group, a (CH₃)₃CNHC(O)- group, etc., and each may be selected from the range of the carbon numbers as designated, respectively.

The expression Cₐ to C_{b} cycloalkylaminocarbonyl in the present specification represents a carbamoyl group in which either one of the hydrogen atoms is substituted by the cycloalkyl group having the above-mentioned meaning with a to b carbon atoms, and there may be specifically mentioned, for example, cyclopropyl-NHC(O)- group, cyclobutyl-NHC(O)- group, cyclopentyl-NHC(O)- group, cyclohexyl-NHC(O)- group, etc., and each may be selected from the range of the carbon numbers as designated, respectively.

The expression di(Cₐ to C_{b} alkyl)aminocarbonyl in the present specification represents a carbamoyl group in which both of the hydrogen atoms are substituted by the alkyl group having the above-mentioned meaning with a to b carbon atoms, which may be the same with each other or may be different from each other, and there may be specifically mentioned, for example, a (CH₃)₂NC(O)- group, CH₃CH₂N(CH₃)C(O)- group, a (CH₃CH₂)₂NC(O)- group, a (CH₃CH₂CH₂)₂NC(O)- group, a (CH₃CH₂CH₂CH₂)₂NC(O)-group, etc., and each may be selected from the range of the carbon numbers as designated, respectively.

The expression Cₐ to C_{b} alkylaminothiocarbonyl in the present specification represents a thiocarbamoyl group in which either one of the hydrogen atoms is substituted by the alkyl group having the above-mentioned meaning with a to b carbon atoms, and there may be specifically mentioned, for example, CH₃NHC(S)- group, CH₃CH₂NHC(S)- group, CH₃CH₂CH₂NHC(S)- group, a (CH₃)₂CHNHC(S)- group, CH₃(CH₂)₃NHC(S)- group, a (CH₃)₂CHCH₂NHC(S)- group, CH₃CH₂CH(CH₃)NHC(S)-group, a (CH₃)₃CNHC(S)- group, etc., and each may be selected from the range of the carbon numbers as designated, respectively.

The expression di(Cₐ to C_{b} alkyl)aminothiocarbonyl in the present specification represents a thiocarbamoyl group in which both of the hydrogen atoms are substituted by the alkyl group having the above-mentioned meaning with a to b carbon atoms, which may be the same with each other or may be different from each other, and there may be specifically mentioned, for example, a (CH₃)₂NC(S)- group, CH₃CH₂N(CH₃)C(S)- group, a (CH₃CH₂)₂NC(S)- group, a (CH₃CH₂CH₂)₂NC(S)- group, a (CH₃CH₂CH₂CH₂)₂NC(S)- group, etc., and each may be selected from the range of the carbon numbers as designated, respectively.

The expression Cₐ to C_{b} alkylaminosulfonyl in the present specification represents a sulfamoyl group in which either one of the hydrogen atoms is substituted by the alkyl group having the above-mentioned meaning with a to b carbon atoms, and there may be specifically mentioned, for example, CH₃NHSO₂- group, CH₃CH₂NHSO₂-group, CH₃CH₂CH₂NHSO₂- group, a (CH₃)₂CHNHSO₂- group, CH₃(CH₂)₃NHSO₂- group, a (CH₃)₂CHCH₂NHSO₂- group, CH₃CH₂CH(CH₃)NHSO₂- group, a (CH₃)₃CNHSO₂-group, etc., and each may be selected from the range of the carbon numbers as designated, respectively.

The expression di(Cₐ to C_{b} alkyl)aminosulfonyl in the present specification represents a sulfamoyl group in which both of the hydrogen atoms are substituted by the alkyl group having the above-mentioned meaning with a to b carbon atoms, which may be the same with each other or may be different from each other, and there may be specifically mentioned, for example, a (CH₃)₂NSO₂- group, CH₃CH₂N(CH₃)SO₂- group, a (CH₃CH₂)₂NSO₂- group, a (CH₃CH₂CH₂)₂NSO₂- group, a (CH₃CH₂CH₂CH₂)₂NSO₂-group, etc., and each may be selected from the range of the carbon numbers as designated, respectively.

The expression di(Cₐ to C_{b} alkyl)phosphoryl in the present specification represents a phosphoryl group in which both of the hydrogen atoms are substituted by the alkyl group having the above-mentioned meaning with a to b carbon atoms, which may be the same with each other or may be different from each other, and there may be specifically mentioned, for example, a (CH₃O)₂P(O)- group, a (CH₃CH₂O)₂P(O)- group, etc., and each may be selected from the range of the carbon numbers as designated, respectively.

The expression di(Cₐ to C_{b} alkyl)thiophosphoryl in the present specification represents a thiophosphoryl group in which both of the hydrogen atoms are substituted by the alkyl group having the above-mentioned meaning with a to b carbon atoms, which may be the same with each other or may be different from each other, and there may be specifically mentioned, for example, a (CH₃O)₂P(S)- group, a (CH₃CH₂O)₂P(S)-group, etc., and each may be selected from the range of the carbon numbers as designated, respectively.

The expression tri(Cₐ to C_{b} alkyl)silyl in the present specification represents a silyl group which is substituted by the alkyl group(s) having the above-mentioned meaning with a to b carbon atoms, which may be the same with each other or may be different from each other, and there may be specifically mentioned, for example, trimethylsilyl group, triethylsilyl group, tri(n-propyl)silyl group, ethyldimethylsilyl group, n-propyldimethylsilyl group, n-butyldimethylsilyl group, i-butyldimethylsilyl group, t-butyldimethylsilyl group, etc., and each may be selected from the range of the carbon numbers as designated, respectively.

The expression Cₐ to C_{b} alkylcarbonyloxy in the present specification represents an alkyl-C(O)-O- group having the above-mentioned meaning with a to b carbon atoms, and there may be specifically mentioned, for example, CH₃C(O)-O-group, CH₃CH₂C(O)-O- group, CH₃CH₂CH₂C(O)-O- group, a (CH₃)₂CHC(O)-O- group, CH₃(CH₂)₃C(O)-O- group, a (CH₃)₂CHCH₂C(O)-O- group, CH₃CH₂CH(CH₃)C(O)-O-group, a (CH₃)₃CC(O)-O- group, etc., and each may be selected from the range of the carbon numbers as designated, respectively.

The expression Cₐ to C_{b} haloalkylcarbonyloxy in the present specification represents a haloalkyl-C(O)-O- group having the above-mentioned meaning with a to b carbon atoms, and there may be specifically mentioned, for example, FCH₂C(O)-O-group, ClCH₂C(O)-O- group, F₂CHC(O)-O- group, Cl₂CHC(O)-O- group, CF₃C(O)-O-group, ClCF₂C(O)-O- group, BrCF₂C(O)-O- group, CCl₃C(O)-O- group, CF₃CF₂C(O)-O-group, CF₃CF₂CF₂C(O)-O- group, ClCH₂CH₂CH₂C(O)-O- group, etc., and each may be selected from the range of the carbon numbers as designated, respectively.

The expression Cₐ to C_{b} alkylsulfonyloxy in the present specification represents an alkyl-SO₂-O- group having the above-mentioned meaning with a to b carbon atoms, and there may be specifically mentioned, for example, CH₃SO₂-O- group, CH₃CH₂SO₂-O- group, CH₃CH₂CH₂SO₂-O- group, a (CH₃)₂CHSO₂-O- group, CH₃(CH₂)₃SO₂-O- group, a (CH₃)₂CHCH₂SO₂-O- group, CH₃CH₂CH(CH₃)SO₂-O- group, a (CH₃)₃CSO₂-O- group, etc., and each may be selected from the range of the carbon numbers as designated, respectively.

The expression Cₐ to C_{b} haloalkylsulfonyloxy in the present specification represents a haloalkyl-SO₂-O- group having the above-mentioned meaning with a to b carbon atoms, and there may be specifically mentioned, for example, CF₃SO₂-O- group, CF₃CF₂SO₂-O- group, etc., and each may be selected from the range of the carbon numbers as designated, respectively.

The expressions Cₐ to C_{b} cycloalkyl(C_{d} to Cₑ)alkyl, Cₐ to C_{b} halocycloalkyl(C_{d} to Cₑ)alkyl, Cₐ to C_{b} alkoxy(C_{d} to Cₑ)alkyl, Cₐ to C_{b} haloalkoxy(C_{d} to Cₑ)alkyl, Cₐ to C_{b} alkylthio(C_{d} to Cₑ)alkyl, Cₐ to C_{b} haloalkylthio(C_{d} to Cₑ)alkyl, phenylthio(C_{d} to Cₑ)alkyl which may be substituted by (Z¹)ₚ₁, Cₐ to C_{b} alkylsulfinyl(C_{d} to Cₑ)alkyl, Cₐ to C_{b} haloalkylsulfinyl(C_{d} to Cₑ)alkyl, Cₐ to C_{b} alkylsulfonyl(C_{d} to Cₑ)alkyl, Cₐ to C_{b} haloalkylsulfonyl(C_{d} to Cₑ)alkyl, Cₐ to C_{b} alkylcarbonyl(C_{d} to Cₑ)alkyl, Cₐ to C_{b} haloalkylcarbonyl(C_{d} to Cₑ)alkyl, Cₐ to C_{b} alkoxycarbonyl(C_{d} to Cₑ)alkyl, Cₐ to C_{b} haloalkoxycarbonyl(C_{d} to Cₑ)alkyl, Cₐ to C_{b}alkylaminocarbonyl(C_{d} to Cₑ)alkyl, a di(Cₐ to C_{b} alkyl)aminocarbonyl(C_{d} to Cₑ)alkyl, a tri(Cₐ to C_{b} alkyl)silyl(C_{d} to Cₑ)alkyl, phenyl(C_{d} to Cₑ)alkyl which may be substituted by (Z¹)ₚ₁, L-(C_{d} to Cₑ)alkyl or M-(C_{d} to Cₑ) alkyl, etc., in the present specification each represent a linear or branched hydrocarbon group having d to e carbon atoms in which the hydrogen atom(s) bonded to the carbon atom(s) is/are optionally substituted by the optional Cₐ to C_{b} cycloalkyl group, Cₐ to C_{b} halocycloalkyl group, Cₐ to C_{b} alkoxy group, Cₐ to C_{b} haloalkoxy group, Cₐ to C_{b} alkylthio group, Cₐ to C_{b} haloalkylthio group, phenylthio group which may be substituted by (Z¹)ₚ₁, Cₐ to C_{b} alkylsulfinyl group, Cₐ to C_{b} haloalkylsulfinyl group, Cₐ to C_{b} alkylsulfonyl group, Cₐ to C_{b} haloalkylsulfonyl group, Cₐ to C_{b} alkylcarbonyl group, Cₐ to C_{b} haloalkylcarbonyl group, Cₐ to C_{b} alkoxycarbonyl group, Cₐ to C_{b} haloalkoxycarbonyl group, Cₐ to C_{b} alkylaminocarbonyl group, a di(Cₐ to C_{b} alkyl)aminocarbonyl group, a tri(Cₐ to C_{b} alkyl)silyl group, phenyl group which may be substituted by (Z¹)ₚ₁, L group or M group, each of which have the above-mentioned meanings, and each may be selected from the range of the carbon numbers as designated, respectively.

The expressions (Cₐ to C_{b})alkyl which may be optionally substituted by R⁷, (Cₐ to C_{b})alkyl which may be optionally substituted by R¹⁸, (Cₐ to C_{b})alkyl which may be optionally substituted by R²³, (Cₐ to C_{b})alkyl which may be optionally substituted by R²⁷ or (Cₐ to C_{b})alkyl which may be optionally substituted by R³⁵ in the present specification each represent a linear or branched hydrocarbon group having a to b carbon atoms in which the hydrogen atom(s) bonded to the carbon atom(s) is/are optionally substituted by an optional R⁷, R¹⁸, R²³, R²⁷ or R³⁵, and each may be selected from the range of the carbon numbers as designated, respectively. At this time, when two or more substituent(s) R⁷, R¹⁸, R²³, R²⁷ or R³⁵ exist on the respective (Cₐ to C_{b})alkyl group, the respective R⁷, R¹⁸, R²³, R²⁷ or R³⁵ may be the same with each other or may be different from each other.

The expression Cₐ to C_{b} haloalkoxy(C_{d} to Cₑ)haloalkyl in the present specification represents a haloalkyl group having the above-mentioned meaning with d to e carbon atoms in which the hydrogen atom(s) or halogen atom(s) bonded to the carbon atom(s) is/are optionally substituted by the Cₐ to C_{b} haloalkoxy group having the above-mentioned meaning, and there may be specifically mentioned, for example, 2,2,2-trifluoro-1-(2,2,2-trifluoroethoxy)-1-(trifluoromethyl)ethyl group, 3-(1,2-dichloro-1,2,2-trifluoroethoxy)-1,1,2,2,3,3-hexafluoropropyl group, etc., and each may be selected from the range of the carbon numbers as designated, respectively.

The expression (Cₐ to C_{b})haloalkyl which may be optionally substituted by R²³ in the present specification represents a linear or branched hydrocarbon group having a to b carbon atoms in which the hydrogen atom(s) or halogen atom(s) bonded to the carbon atom(s) is/are optionally substituted by an optional R²³, and each may be selected from the range of the carbon numbers as designated, respectively. At this time, when the substituent(s) R²³ on the respective (Cₐ to C_{b})alkyl groups exists 2 or more, each of R²³s may be the same with each other or may be different from each other.

The expressions hydroxymethyl(C_{d} to Cₑ)cycloalkyl, Cₐ to C_{b} alkoxymethyl(C_{d} to Cₑ)cycloalkyl, Cₐ to C_{b} alkylthiomethyl(C_{d} to Cₑ)cycloalkyl, Cₐ to C_{b} alkylsulfinylmethyl(C_{d} to Cₑ)cycloalkyl, Cₐ to C_{b} alkylsulfonylmethyl(C_{d} to Cₑ)cycloalkyl, Cₐ to C_{b} alkenyl(C_{d} to Cₑ)cycloalkyl, Cₐ to C_{b} haloalkenyl(C_{d} to Cₑ)cycloalkyl, Cₐ to C_{b} alkylthio(C_{d} to Cₑ)cycloalkyl, Cₐ to C_{b} alkylsulfinyl(C_{d} to Cₑ)cycloalkyl or a Cₐ to C_{b} alkylsulfonyl(C_{d} to Cₑ)cycloalkyl, etc. in the present specification represent a cycloalkyl group having d to e carbon atoms in which the hydrogen atom bonded to the carbon atom is optionally substituted by an optional hydroxy group, Cₐ to C_{b} alkoxy group, Cₐ to C_{b} alkenyl group, Cₐ to C_{b} haloalkenyl group, Cₐ to C_{b} alkylthio group, Cₐ to C_{b} alkylsulfinyl group or a Cₐ to C_{b} alkylsulfonyl group each having the above-mentioned meanings, and there may be specifically mentioned, for example, 2-vinylcyclopropyl group, 3,3-dimethyl-2-(2-methyl-1-propenyl)cyclopropyl group, 2-(2,2-dichloroethenyl)-3,3-dimethylcyclopropyl group, 2-(2-chloro-3,3,3-trifluoro-1-propenyl)-3,3-dimethylcyclopropyl group, 1-(methylthiomethyl)cyclopropyl group, 1-(methylsulfinylmethyl)cyclopropyl group, 1-(methylsulfonylmethyl)cyclopropyl group, 1-(methylthiomethyl)cyclobutyl group, 2-allylcyclopentyl group, 1-(hydroxymethyl)cyclopentyl group, 1-(methoxymethyl)cyclopentyl group, 1-(methylthiomethyl)cyclopentyl group, 1-(methylsulfinylmethyl)cyclopentyl group, 1-(methylsulfonylmethyl)cyclopentyl group, 2-(methoxy)cyclopentyl group, 2-(methylthio)cyclopentyl group, 2-(methylthio)cyclohexyl group, etc., and each may be selected from the range of the carbon numbers as designated, respectively.

The expressions (Cₐ to C_{b})cycloalkyl which is optionally substituted by R⁷, (Cₐ to C_{b})cycloalkyl which is optionally substituted by R¹⁸, (Cₐ to C_{b})cycloalkyl which is optionally substituted by R²³, (Cₐ to C_{b})cycloalkyl which is optionally substituted by R²⁷ or (Cₐ to C_{b})cycloalkyl which is optionally substituted by R³⁵, etc. in the present specification represent a cycloalkyl group having a to b carbon atoms in which the hydrogen atom bonded to the carbon atom is optionally substituted by an optional R⁷, R¹⁸, R²³, R²⁷ or R³⁵. At this time, substitution by R⁷, R¹⁸, R²³, R²⁷ or R³⁵ may be at the ring structure portion, at the side chain portion or at the both portions thereof, and further, when the substituent(s) R⁷, R¹⁸, R²³, R²⁷ or R³⁵ on the respective (Cₐ to C_{b})cycloalkyl group exists 2 or more, each of R⁷, R¹⁸, R²³, R²⁷ or R³⁵s may be the same with each other or may be different from each other.

The expression (Cₐ to C_{b})halocycloalkyl which is optionally substituted by R²³ in the present specification represent a cycloalkyl group having the above-mentioned meaning with a to be carbon atoms in which the hydrogen atom or halogen atom bonded to the carbon atom is optionally substituted by an optional R²³. At this time, substitution by R²³ may be at the ring structure portion, a side chain portion, or may be both of the portions, and further, when the substituent(s) R²³ on the respective (Cₐ to C_{b})cycloalkyl groups exist 2 or more, the respective R²³s may be the same with each other or may be different from each other.

The expressions Cₐ to C_{b} alkylaminocarbonyl(C_{d} to Cₑ)alkenyl or phenyl(C_{d} to Cₑ)alkenyl which may be substituted by (Z¹)ₚ₁, etc. in the present specification represent an alkenyl group having the above-mentioned meaning with d to e carbon atoms in which the hydrogen atom bonded to the carbon atom is optionally substituted by an optional Cₐ to C_{b} alkylaminocarbonyl group or phenyl group which may be substituted by (Z¹)ₚ₁ each having the above-mentioned meanings, and each may be selected from the range of the carbon numbers as designated, respectively.

The expressions (Cₐ to C_{b})alkenyl which may be optionally substituted by R⁷, (Cₐ to C_{b})alkenyl which may be optionally substituted by R¹⁸, (Cₐ to C_{b}) alkenyl which may be optionally substituted by R²⁷ or (Cₐ to C_{b})alkenyl which may be optionally substituted by R³⁵ in the present specification represent an alkenyl group having the above-mentioned meaning with a to b carbon atoms in which the hydrogen atom bonded to the carbon atom is optionally substituted by an optional R⁷, R¹⁸, R²⁷ or R³⁵, and each may be selected from the range of the carbon numbers as designated, respectively. At this time, when the substituent(s) R⁷, R¹⁸, R²⁷ or R³⁵ on the respective (Cₐ to C_{b})alkenyl groups exist 2 or more, the respective R⁷, R¹⁸, R²⁷ or R³⁵s may be the same with each other or may be different from each other.

The expressions phenyl(C_{d} to Cₑ)alkynyl which may be substituted by (Z¹)ₚ₁, naphthyl(C_{d} to Cₑ)alkynyl or L-(C_{d} to Cₑ)alkynyl, etc. in the present specification represent an alkynyl group having the above-mentioned meaning with d to e carbon atoms in which the hydrogen atom bonded to the carbon atom is optionally substituted by an optional phenyl group which may be substituted by (Z¹)ₚ₁, naphthyl group or L group, and each may be selected from the range of the carbon numbers as designated, respectively.

The expressions (Cₐ to C_{b})alkynyl which may be optionally substituted by R⁷, (Cₐ to C_{b})alkynyl which may be optionally substituted by R¹⁸, (Cₐ to C_{b})alkynyl which may be optionally substituted by R²⁷ or (Cₐ to C_{b})alkynyl which may be optionally substituted by R³⁵ in the present specification represent an alkynyl group having the above-mentioned meaning with a to b carbon atoms in which the hydrogen atom bonded to the carbon atom is optionally substituted by an optional R⁷, R¹⁸, R²⁷ or R³⁵, and each may be selected from the range of the carbon numbers as designated, respectively. At this time, when the substituent(s) R⁷, R¹⁸, R²⁷ or R³⁵ on the respective (Cₐ to C_{b})alkynyl groups exist 2 or more, the respective R⁷, R¹⁸, R²⁷ or R³⁵s may be the same with each other or may be different from each other.

The expression phenyl(Cₐ to C_{b})alkoxy which may be substituted by (Z¹)ₚ₁ in the present specification represents a (Cₐ to C_{b})alkoxy group having the above-mentioned meaning in which the hydrogen atom bonded to the carbon atom is optionally substituted by a phenyl group which may be substituted by (Z¹)ₚ₁, and as the (Cₐ to C_{b})alkoxy group, there may be mentioned, for example, -CH₂O- group, -CH(CH₃)O- group, -C(CH₃)₂O- group, -CH₂CH₂O- group, -CH(CH₃)CH₂O- group, -C(CH₃)₂CH₂O- group, etc., and each may be selected from the range of the carbon numbers as designated, respectively.

The expression phenyl(Cₐ to C_{b})alkylcarbonyl which may be substituted by (Z¹)ₚ₁ in the present specification represents a (Cₐ to C_{b})alkylcarbonyl group having the above-mentioned meaning in which the hydrogen atom bonded to the carbon atom is optionally substituted by a phenyl group which may be substituted by (Z¹)ₚ₁, and as the (Cₐ to C_{b}) alkylcarbonyl group, there may be mentioned, for example, -CH₂C(O)- group, -CH(CH₃)C(O)- group, -C(CH₃)₂C(O)- group, -CH₂CH₂C(O)- group, -CH(CH₃)CH₂C(O)-group, -C(CH₃)₂CH₂C(O)- group, -CH₂CH(CH₃)C(O)- group, -CH₂C(CH₃)₂C(O)- group, -CH₂CH₂CH₂C(O)- group, etc., and each may be selected from the range of the carbon numbers as designated, respectively.

The expression phenyl(Cₐ to C_{b}) alkoxycarbonyl which may be substituted by (Z¹)ₚ₁ in the present specification represent a (Cₐ to C_{b}) alkoxycarbonyl group having the above-mentioned meaning in which the hydrogen atom bonded to the carbon atom is optionally substituted by a phenyl group which may be substituted by (Z¹)ₚ₁, and as the (Cₐ to C_{b}) alkoxycarbonyl group, there may be mentioned, for example, -CH₂O-C(O)-group, -CH(CH₃)O-C(O)- group, -C(CH₃)₂O-C(O)- group, -CH₂CH₂O-C(O)- group, -CH(CH₃)CH₂O-C(O)- group, -C(CH₃)₂CH₂O-C(O)- group, etc., and each may be selected from the range of the carbon numbers as designated, respectively.

The expression phenyl(Cₐ to C_{b})alkylaminocarbonyl which may be substituted by (Z¹)ₚ₁ in the present specification represent a (Cₐ to C_{b})alkylaminocarbonyl group having the above-mentioned meaning in which the hydrogen atom bonded to the carbon atom is optionally substituted by a phenyl group which may be substituted by (Z¹)ₚ₁, and as the (Cₐ to C_{b})alkylaminocarbonyl group, there may be mentioned, for example, -CH₂NH-C(O)- group, -CH(CH₃)NH-C(O)- group, -C(CH₃)₂NH-C(O)- group, -CH₂CH₂NH-C(O)- group, -CH(CH₃)CH₂NH-C(O)- group, -C(CH₃)₂CH₂NH-C(O)- group, etc., and each may be selected from the range of the carbon numbers as designated, respectively.

As the specific examples of the expressions
[R² and R³ are combined to form a C₂ to C₆ alkylene chain, so that they may form a 3- to 7-membered ring with a nitrogen atom(s) to which they are bonded, and the alkylene chain at this time may contain one oxygen atom, sulfur atom or nitrogen atom,],
[R¹⁹ and R²⁰ are combined to form a C₄ to C₇ alkylene chain, so that they may form a 5 to 8-membered ring with a nitrogen atom(s) to which they are bonded, the alkylene chain at this time may contain one oxygen atom or sulfur atom,],
[R²⁸ and R²⁹ are combined to form a C₂ to C₅ alkylene chain, so that they may form a 3- to 6-membered ring with the nitrogen atom(s) to which they are bonded, the alkylene chain at this time may contain one oxygen atom or sulfur atom,],
[R³¹ and R³² are combined to form a C₂ to C₅ alkylene chain, so that they may form a 3- to 6-membered ring with the nitrogen atom(s) to which they are bonded, and the alkylene chain at this time may contain one oxygen atom, sulfur atom or nitrogen atom,],
and
[R³⁶ and R³⁷ are combined to form a C₂ to C₅ alkylene chain, so that they may form a 3- to 6-membered ring with the nitrogen atom(s) to which they are bonded, and the alkylene chain at this time may contain one oxygen atom or sulfur atom,] in the present specification, there may be mentioned, for example, aziridine, azetidine, pyrrolidine, oxazolidine, thiazolidine, imidazolidine, piperidine, morpholine, thiomorpholine, piperazine, homopiperidine, heptamethyleneimine, etc., and each may be selected from the range of the carbon numbers as designated, respectively.

As the specific examples of the expression
[R⁹ and R¹⁰ are combined to form a C₂ to C₆ alkylene chain, so that they may form a 3- to 7-membered ring with the atom(s) to which they are bonded, and the alkylene chain at this time may contain one oxygen atom, sulfur atom or nitrogen atom,] in the present specification, there may be mentioned, for example, aziridine, azetidine, azetidin-2-one, pyrrolidine, pyrrolidin-2-one, oxazolidine, oxazolidin-2-one, thiazolidine, thiazolidin-2-one, imidazolidine, imidazolidin-2-one, piperidine, piperidin-2-one, morpholine, tetrahydro-1,3-oxazin-2-one, thiomorpholine, tetrahydro-1,3-thiazin-2-one, piperazine, tetrahydropyrimidin-2-one, homopiperidine, homopiperidin-2-one, etc., and each may be selected from the range of the carbon numbers as designated, respectively.

As the specific examples of the expression
[R⁹ and R¹¹ are combined to form a C₂ to C₄ alkylene chain, so that they may form a 5- to 7-membered ring with atoms to which they are bonded, and the alkylene chain at this time may contain one oxygen atom, sulfur atom or nitrogen atom,] in the present specification, there may be mentioned, for example, isoxazoline, 1,4,2-dioxazoline, 1,4,2-oxathiazoline, 1,2,4-oxadiazoline, dihydro-1,2-oxazine, dihydro-1,4,2-dioxazine, dihydro-1,4,2-oxathiazine, dihydro-4H-1,2,4-oxadiazine, tetrahydro-1,2-oxazepine, etc., and each may be selected from the range of the carbon numbers as designated, respectively.

In the compounds included in the present invention, as the scope of the heterocyclic ring and the cycloalkyl ring represented by G, there may be mentioned, for example, the group consisting of G-1, G-4, G-5, G-6, G-7, G-8, G-11, G-12, G-13, G-14, G-15, G-17, G-18, G-21, G-22, G-23, G-32, G-33, G-40, G-41, G-42, G-53, G-54, G-55, G-56 and G-71.

In the compounds included in the present invention, as the substituent(s) represented by W¹ or W², there may be mentioned, for example, an oxygen atom or a sulfur atom, and of these, an oxygen atom is preferred.

In the compounds included in the present invention, as the scope of the substituent(s) represented by X, there may be mentioned, for example, the respective groups as mentioned below.

That is, X-I: a halogen atom.
X-II: cyano and nitro.
X-III: a hydrogen atom, a C₁ to C₆ alkyl and a C₁ to C₆ haloalkyl.
X-IV: a C₁ to C₆ alkoxy and a C₁ to C₆ haloalkoxy.
X-V: a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl and a C₁ to C₆ haloalkylsulfonyl.

X-VI: -N(R¹⁷)R¹⁶ [here, R¹⁶ represents a hydrogen atom, a C₁ to C₆ alkyl, a C₁ to C₄ alkoxy(C₁ to C₄)alkyl, a C₁ to C₄ alkylthio(C₁ to C₄)alkyl, -CHO, a C₁ to C₆ alkylcarbonyl, a C₁ to C₆ haloalkylcarbonyl, a C₃ to C₆ cycloalkylcarbonyl, a C₁ to C₆ alkoxycarbonyl, a C₁ to C₆ haloalkoxycarbonyl or a C₁ to C₆ alkylthiocarbonyl, R¹⁷ represents a hydrogen atom or a C₁ to C₆ alkyl, or R¹⁷ may be combined with R² to form -CH₂-.].

In the compounds included in the present invention, as the scope of the substituent(s) represented by Y, for example, there may be mentioned the respective groups as mentioned below.

That is, Y-I: a hydrogen atom.
Y-II: a halogen atom.
Y-III: a C₁ to C₆ alkyl.
Y-IV: a C₁ to C₆ haloalkyl, a hydroxy(C₁ to C₆)alkyl and a C₁ to C₄ alkoxy(C₁ to C₄)alkyl.
Y-V: a C₁ to C₆ alkoxy.
Y-VI: a C₁ to C₆ alkylthio.

In the compounds included in the present invention, as the substituent(s) represented by R¹, a hydrogen atom is preferred.

In the compounds included in the present invention, as the scope of the substituent(s) represented by R², there may be mentioned, for example, the respective groups as mentioned below.

That is, R²-l:hydrogen atom.
R²-II: a C₁ to C₆ alkyl.
R²-III: a C₁ to C₄ alkoxy(C₁ to C₄)alkyl and a C₁ to C₄ alkylthio(C₁ to C₄) alkyl.
R²-IV: a C₃ to C₆ alkenyl and a C₃ to C₆ alkynyl.

In the compounds included in the present invention, as the scope of the substituent(s) represented by R³, there may be mentioned, for example, the respective groups as mentioned below.

That is, R³-l: a C₁ to C₈ alkyl and a C₃ to C₈ cycloalkyl.
R³-II: a C₃ to C₈ alkenyl and a C₃ to C₈ alkynyl.
R³-III: R²⁸O-(C₁ to C₈)alkyl [here, R²⁸ represents a C₁ to C₆ alkyl or -C(O)N(R³²)R³¹, R³¹ represents a C₁ to C₆ alkyl, R³² represents a hydrogen atom or a C₁ to C₆ alkyl.].
R³-IV: R²⁸O-(C₁ to C₈)alkyl [here, R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₁ to C₆ alkylcarbonyl, a C₃ to C₆ cycloalkylcarbonyl, -C(O)N(R³²)R³¹, a di(C₁ to C₆ alkyl)phosphoryl, a di(C₁ to C₆ alkyl)thiophosphoryl, a tri(C₁ to C₄ alkyl)silyl or phenyl which may be substituted by (Z¹)ₚ₁, R³¹ represents a C₁ to C₆ alkyl, a C₁ to C₄ alkylthio(C₁ to C₄)alkyl, a phenyl(C₁ to C₄)alkyl which may be substituted by (Z¹)ₚ₁, a C₃ to C₆ cycloalkyl, a C₃ to C₆ alkenyl or phenyl which may be substituted by (Z¹)ₚ₁, R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, the alkylene chain at this time may contain one oxygen atom or sulfur atom.], (M-1)-(C₁ to C₈)alkyl, (M-2)-(C₁ to C₈)alkyl, (M-3)-(C₁ to C₈)alkyl, (M-4)-(C₁ to C₈)alkyl, (M-5)-(C₁ to C₈)alkyl, (M-6)-(C₁ to C₈)alkyl, (M-7)-(C₁ to C₈)alkyl, (M-14)-(C₁ to C₈)alkyl, (M-15)-(C₁ to C₈)alkyl, (M-16)-(C₁ to C₈)alkyl, (M-23)-(C₁ to C₈)alkyl, (M-24)-(C₁ to C₈)alkyl, (M-25)-(C₁ to C₈)alkyl, M-4, M-5, M-14, M-15 and M-16.
R³-V: a C₁ to C₈ haloalkyl, a C₃ to C₆ cycloalkyl(C₁ to C₈)alkyl, a tri(C₁ to C₆ alkyl)silyl(C₁ to C₈)alkyl, a phenyl(C₁ to C₈)alkyl which may be substituted by (Z¹)ₚ₁, (L-1)-(C₁ to C₈)alkyl, (L-2)-(C₁ to C₈)alkyl, (L-3)-(C₁ to C₈)alkyl, (L-4)-(C₁ to C₈)alkyl, (L-45)-(C₁ to C₈)alkyl, (L-46)-(C₁ to C₈)alkyl, (L-47)-(C₁ to C₈)alkyl, a C₃ to C₈ alkenyl, a phenyl(C₃ to C₆)alkenyl which may be substituted by (Z¹)ₚ₁, a C₃ to C₈ alkynyl, a phenyl(C₃ to C₆)alkynyl which may be substituted by (Z¹)ₚ₁, a naphthalen-1-yl-(C₃ to C₆)alkynyl, a naphthalen-2-yl-(C₃ to C₆)alkynyl, (L-1)-(C₃ to C₆)alkynyl, (L-2)-(C₃ to C₆)alkynyl, (L-3)-(C₃ to C₆)alkynyl, (L-4)-(C₃ to C₆)alkynyl, (L-45)-(C₃ to C₆)alkynyl, (L-46)-(C₃ to C₆)alkynyl and (L-47)-(C₃ to C₆)alkynyl.
R³-VI: a cyano(C₁ to C₈)alkyl, a C₁ to C₆ alkoxycarbonyl(C₁ to C₈)alkyl, a C₁ to C₆ alkylaminocarbonyl(C₁ to C₈)alkyl, a di(C₁ to C₆ alkyl)aminocarbonyl(C₁ to C₈)alkyl, HON=C(R³⁴)-(C₁ to C₈)alkyl, R³³ON=C(R³⁴)-(C₁ to C₈) alkyl [here, R³³ represents a C₁ to C₆ alkyl or a phenyl(C₁ to C₄)alkyl which may be substituted by (Z¹)ₚ₁, R³⁴ represents a hydrogen atom or a C₁ to C₆ alkyl.] and a C₁ to C₆ alkylaminocarbonyl(C₃ to C₆) alkenyl.
R³-VII: a C₁ to C₄ alkylthio(C₁ to C₄)alkyl, a C₁ to C₄ alkylsulfinyl(C₁ to C₄)alkyl and a C₁ to C₄ alkylsulfonyl(C₁ to C₄) alkyl.
R³-VIII: HON=CH-(C₁ to C₈)alkyl and R³³ON=CH-(C₁ to C₈) alkyl [here, R³³ represents a C₁ to C₆ alkyl.].
R³-IX: R²⁸(R²⁹)N-(C₁ to C₈)alkyl [here, R²⁸ represents a C₁ to C₆ alkoxycarbonyl, a C₁ to C₆ alkylsulfonyl or a di(C₁ to C₆ alkyl)thiophosphoryl, R²⁹ represents a hydrogen atom or a C₁ to C₆ alkyl.].
R³-X: R³⁰S(O)ᵣ-(C₁ to C₈)alkyl [here, R³⁰ represents a C₁ to C₆ alkyl, a C₁ to C₆ haloalkyl, a hydroxy(C₁ to C₄)alkyl, a C₁ to C₄ alkoxy(C₁ to C₄)alkyl, a C₁ to C₄ alkylthio(C₁ to C₄)alkyl, a C₁ to C₄ alkylcarbonyl(C₁ to C₄)alkyl, a C₁ to C₄ alkoxycarbonyl(C₁ to C₄)alkyl, a di(C₁ to C₄ alkyl)aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a C₃ to C₆ alkenyl, a C₃ to C₆ alkynyl, a C₁ to C₆ alkylthio, phenyl which may be substituted by (Z¹)ₚ₁, L-21 or L-45, r represents an integer of 0 to 2.], (M-8)-(C₁ to C₈)alkyl, (M-9)-(C₁ to C₈)alkyl, (M-10)-(C₁ to C₈)alkyl, (M-11)-(C₁ to C₈)alkyl, (M-17)-(C₁ to C₈)alkyl, (M-18)-(C₁ to C₈)alkyl, (M-19)-(C₁ to C₈)alkyl, (M-26)-(C₁ to C₈)alkyl, (M-27)-(C₁ to C₈)alkyl, (M-28)-(C₁ to C₈)alkyl, M-8, M-9, M-17, M-18 and M-19.
R³-XI: R²⁸(R²⁹)N-(C₁ to C₈)alkyl [here, R²⁸ represents a C₁ to C₆ alkylcarbonyl, a C₃ to C₆ cycloalkylcarbonyl, a C₁ to C₆ alkoxycarbonyl, a di(C₁ to C₆ alkyl)aminocarbonyl, a C₁ to C₆ alkylsulfonyl, a di(C₁ to C₆ alkyl)aminosulfonyl, phenylsulfonyl which may be substituted by (Z¹)ₚ₁ or a di(C₁ to C₆ alkyl)thiophosphoryl, R²⁹ represents a hydrogen atom or a C₁ to C₆ alkyl.], (M-12)-(C₁ to C₈)alkyl, (M-13)-(C₁ to C₈)alkyl, (M-20)-(C₁ to C₈)alkyl, (M-21)-(C₁ to C₈)alkyl, (M-22)-(C₁ to C₈)alkyl, M-13, M-21 and M-22.
R³-XII: a 3- to 7-membered ring formed by R² and R³ in combination is aziridine, azetidine, pyrrolidine, oxazolidine, thiazolidine, piperidine, morpholine, thiomorpholine and homopiperidine.
R³-XIII: a C₁ to C₈ alkyl, a C₃ to C₈ cycloalkyl, a C₃ to C₈ alkenyl and a C₃ to C₈ alkynyl.
R³-XIV: a C₁ to C₆ alkyl, a C₁ to C₄ alkylthio(C₁ to C₄)alkyl, a C₁ to C₄ alkylsulfinyl(C₁ to C₄)alkyl and a C₁ to C₄ alkylsulfonyl(C₁ to C₄) alkyl.
R³-XV: R²⁸O-(C₁ to C₈) alkyl [here, R²⁸ represents a C₁ to C₆ alkyl or -C(O)N(R³²)R³¹, R³¹ represents a C₁ to C₆ alkyl, R³² represents a hydrogen atom or a C₁ to C₆ alkyl.], C₁ to C₄ alkylthio(C₁ to C₄)alkyl, C₁ to C₄ alkylsulfinyl(C₁ to C₄)alkyl and C₁ to C₄ alkylsulfonyl(C₁ to C₄) alkyl.
R³-XVI: C₁ to C₆ alkyl, R²⁸O-(C₁ to C₈) alkyl [here, R²⁸ represents a C₁ to C₆ alkyl or -C(O)N(R³²)R³¹, R³¹ represents a C₁ to C₆ alkyl, R³² represents a hydrogen atom or a C₁ to C₆ alkyl.], a C₁ to C₄ alkylthio(C₁ to C₄)alkyl, a C₁ to C₄ alkylsulfinyl(C₁ to C₄)alkyl, a C₁ to C₄ alkylsulfonyl(C₁ to C₄)alkyl, a R²⁸(R²⁹)N-(C₁ to C₈) alkyl [here, R²⁸ represents a C₁ to C₆ alkoxycarbonyl, a C₁ to C₆ alkylsulfonyl or a di(C₁ to C₆ alkyl)-thiophosphoryl, R²⁹ represents a hydrogen atom or a C₁ to C₆ alkyl.], a HON=CH-(C₁ to C₈)alkyl and a R³³ON=CH-(C₁ to C₈)alkyl [here, R³³ represents a C₁ to C₆ alkyl.].
In the compounds included in the present invention, as the scope of the substituent(s) represented by R⁴, for example, there may be mentioned the respective groups as mentioned below.

That is, R⁴-l: a C₁ to C₆ alkyl and a C₁ to C₆ haloalkyl.
R⁴-II: a C₃ to C₆ cycloalkyl(C₁ to C₄)alkyl, a C₃ to C₆ halocycloalkyl(C₁ to C₄)-alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl and a C₁ to C₄ haloalkoxy(C₁ to C₄)haloalkyl.
R⁴-III: a C₃ to C₈ cycloalkyl, a C₃ to C₈ halocycloalkyl, M-4, M-5, M-8, M-9, M-14 to M-18 and M-19.
R⁴-IV: phenyl which may be substituted by (Z²)ₚ₁, 1-naphthyl and 2-naphthyl.
R⁴-V: L-1 to L-4, L-8 to L-13, L-15 to L-23, L-25 to L-35, L-37, L-38, L-40, L-43 to L-57 and L-58.
R⁴-VI: a hydrogen atom, a C₁ to C₆ alkyl and a C₁ to C₆ haloalkyl.
R⁴-VII: a hydrogen atom, a halogen atom, a C₁ to C₆ alkyl and a C₁ to C₆ haloalkyl.
R⁴-VIII: a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl and a C₁ to C₆ haloalkylsulfonyl.
R⁴-IX: a halogen atom and a C₁ to C₆ haloalkyl.

In the compounds included in the present invention, as the scope of the substituent(s) represented by R⁵, for example, there may be mentioned the respective groups as mentioned below.

That is, R⁵-l: a hydrogen atom, a halogen atom, cyano, a C₁ to C₆ alkyl, a C₁ to C₆ haloalkyl, a C₁ to C₆ alkoxy, a C₁ to C₆ haloalkoxy, phenoxy which may be substituted by (Z²)ₚ₁, a C₁ to C₆ alkylthio, a C₁ to C₆ haloalkylthio, phenylthio which may be substituted by (Z²)ₚ₁, a C₁ to C₆ alkylsulfinyl, a C₁ to C₆ haloalkylsulfinyl, phenylsulfinyl which may be substituted by (Z²)ₚ₁, a C₁ to C₆ alkylsulfonyl, a C₁ to C₆ haloalkylsulfonyl, phenylsulfonyl which may be substituted by (Z²)ₚ₁, a C₁ to C₆ alkylamino and a di(C₁ to C₆ alkyl)amino.
R⁵-II: a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl and a cyano(C₁ to C₆)alkyl.
R⁵-III: a C₃ to C₈ cycloalkyl, a C₃ to C₈ halocycloalkyl, pyrrolidin-1-yl, oxazolidin-3-yl, thiazolidin-3-yl, piperidin-1-yl, morpholin-1-yl and M.
R⁵-IV: -CHO, -C(O)R⁹, -C(O)OR⁹, -C(O)SR⁹, -C(O)NHR¹⁰, -C(O)N(R¹⁰)R⁹, -C(S)NHR¹⁰, -C(S)N(R¹⁰)R⁹, -CH=NOR¹¹ and -C(R⁹)=NOR¹¹ [here, R⁹ represents a C₁ to C₆ alkyl, a C₁ to C₆ haloalkyl, phenyl(C₁ to C₄)alkyl which may be substituted by (Z¹)ₚ₁, a C₃ to C₈ cycloalkyl or phenyl which may be substituted by (Z¹)ₚ₁, R¹⁰ represents a hydrogen atom or a C₁ to C₆ alkyl, or R⁹ and R¹⁰ are combined to form a C₄ to C₅ alkylene chain, so that they may form a 5-membered ring or 6-membered ring with the nitrogen atom(s) to which they are bonded, the alkylene chain at this time may contain one oxygen atom or sulfur atom, R¹¹ represents a C₁ to C₆ alkyl, a C₁ to C₆ haloalkyl or a phenyl(C₁ to C₄)alkyl which may be substituted by (Z¹)ₚ₁, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group.].
R⁵-V: phenyl which may be substituted by (Z²)ₚ₁, 1-naphthyl, 2-naphthyl and L.
R⁵-VI: a halogen atom, a C₁ to C₆ alkyl, a C₁ to C₆ haloalkyl, a C₃ to C₆ cycloalkyl, a C₁ to C₆ haloalkoxy, a C₁ to C₆ alkylthio, a C₁ to C₆ alkylsulfinyl, a C₁ to C₆ alkylsulfonyl or a di(C₁ to C₆ alkyl)amino.
R⁵-VII: phenyl which may be substituted by (Z²)ₚ₁, L-1 to L-5, L-8 to L-24, L-28 to L-36, L-39, L-41, L-42, L-45 to L-47 or L-50.
R⁵-VIII: a hydrogen atom, a halogen atom, a C₁ to C₆ alkyl and a C₁ to C₆ haloalkyl.
R⁵-IX: cyano, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)-alkyl, a C₁ to C₄ haloalkylsulfonyl(C₁ to C₄)alkyl and a cyano(C₁ to C₆) alkyl.
R⁵-X: a C₃ to C₈ cycloalkyl, a C₃ to C₈ halocycloalkyl and M.
R⁵-XI: a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfinyl, a C₁ to C₆ haloalkylsulfinyl, phenylsulfinyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁ and -Si(R¹³)(R¹⁴)R¹².
R⁵-XII: phenyl which may be substituted by (Z²)ₚ₁, 1-naphthyl or 2-naphthyl.
R⁵-XIII: L-1 to L-4, L-15 to L-17, L-19, L-20, L-22, L-23, L-45 to L-47 or L-50.
R⁵-XIV: a hydrogen atom.
R⁵-XV: a hydrogen atom, a C₁ to C₆ alkyl and a C₁ to C₆ haloalkyl.
In the compounds included in the present invention, as the scope of the substituent(s) represented by R⁶, for example, there may be mentioned the respective groups as mentioned below.
That is, R⁶-l: R^{6a} and R^{6b} each independently represent a hydrogen atom, a halogen atom, a C₁ to C₆ alkyl or a C₁ to C₆ haloalkyl.
R⁶-II: R^{6a} and R^{6b} each independently represent a hydrogen atom or a C₁ to C₆ alkyl, R^{6c} represents a hydrogen atom, a halogen atom, cyano or a C₁ to C₆ alkyl.
R⁶-III: R^{6a}, R^{6b}, R^{6c} and R^{6d} each independently represent a hydrogen atom, a halogen atom, a C₁ to C₆ alkyl or a C₁ to C₆ haloalkyl.
R⁶-IV: R^{6a} and R^{6b} each independently represent a hydrogen atom, a halogen atom, a C₁ to C₆ alkyl or a C₁ to C₆ haloalkyl, R^{6c} represents a hydrogen atom.
R⁶-V: R^{6a} and R^{6b} each independently represent a hydrogen atom, a halogen atom, a C₁ to C₆ alkyl or a C₁ to C₆ haloalkyl, R^{6c} and R^{6d} each independently represent a hydrogen atom or a C₁ to C₆ alkyl.
R⁶-VI: R^{6a} and R^{6b} each independently represent a hydrogen atom or a C₁ to C₆ alkyl, R^{6e} represents a hydrogen atom, a C₁ to C₆ alkyl, a C₁ to C₆ alkylsulfonyl, a C₁ to C₆ haloalkylsulfonyl, a phenylsulfonyl which may be substituted by (Z¹)ₚ₁, a C₁ to C₆ alkylcarbonyl, a C₁ to C₆ haloalkylcarbonyl, phenylcarbonyl which may be substituted by (Z¹)ₚ₁, a C₁ to C₆ alkoxycarbonyl, a C₁ to C₆ haloalkoxycarbonyl, a phenoxycarbonyl which may be substituted by (Z¹)ₚ₁, a di(C₁ to C₆ alkyl)phosphoryl or phenyl which may be substituted by (Z²)ₚ₁.
R⁶-VII: R^{6a} and R^{6e} each represent a hydrogen atom, R^{6c} represents a hydrogen atom or a C₁ to C₆ alkyl.
R⁶-VIII: R^{6a} and R^{6b} each independently represent a hydrogen atom, a C₁ to C₆ alkyl or a C₁ to C₆ haloalkyl.
R⁶-IX: R^{6a}, R^{6b}, R^{6c} and R^{6d} each independently represent a hydrogen atom, a C₁ to C₆ alkyl or a C₁ to C₆ haloalkyl.
R⁶-X: R^{6f}, R^{6g} and R^{6h} each independently represent a hydrogen atom, a C₁ to C₆ alkyl or a C₁ to C₆ haloalkyl.
R⁶-XI: R⁶ⁱ represents a hydrogen atom, a halogen atom, cyano, a C₁ to C₆ alkyl, a C₁ to C₆ haloalkyl, a C₁ to C₆ alkoxycarbonyl or a C₁ to C₆ haloalkoxycarbonyl, R^{6j} and R^{6k} each independently represent a hydrogen atom, a halogen atom, cyano, a C₁ to C₆ alkyl or a C₁ to C₆ haloalkyl.
R⁶-XII:R⁶ⁱ represents a hydrogen atom, R^{6j} and R^{6k} each independently represent a hydrogen atom, a halogen atom, cyano or a C₁ to C₆ alkyl.

These respective groups showing the scope of the respective substituent(s) in the compounds included in the present invention can be optionally combined to each other, and each represents the scope of the compounds of the present invention. Examples of the combinations of the scope of G, R⁴, R⁵ and R⁶ may be mentioned, for example, the combinations shown in the following Table 1. Provided that, the combination shown in Table 1 is only for the purpose of exemplification, and the present invention is not restricted only to these.

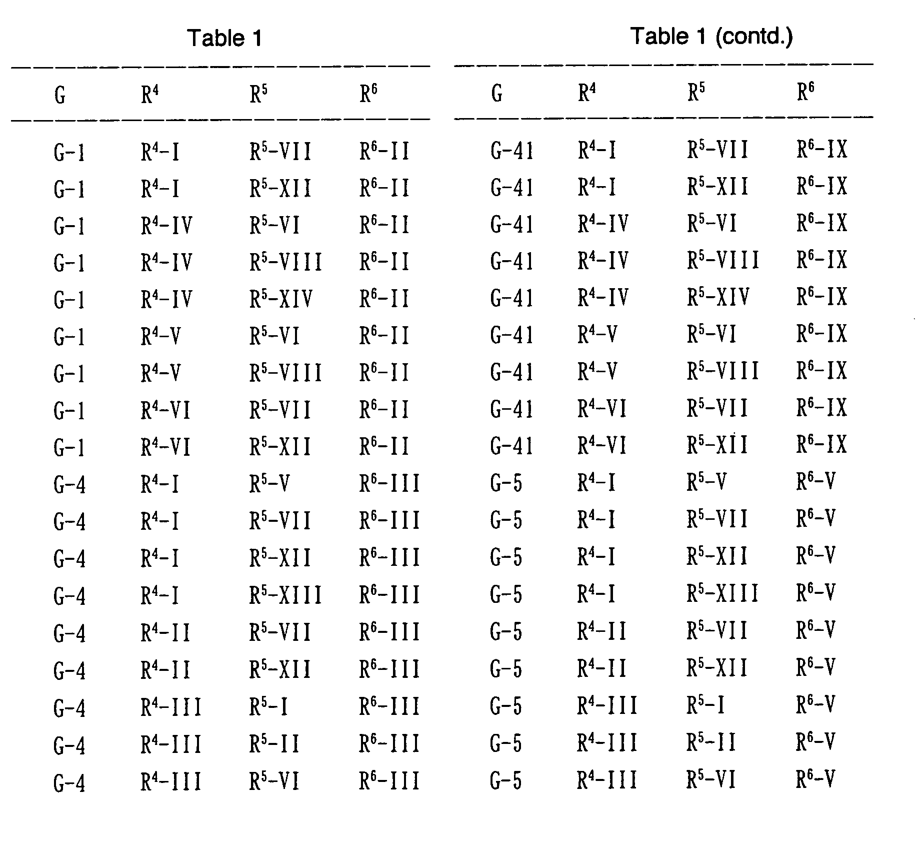

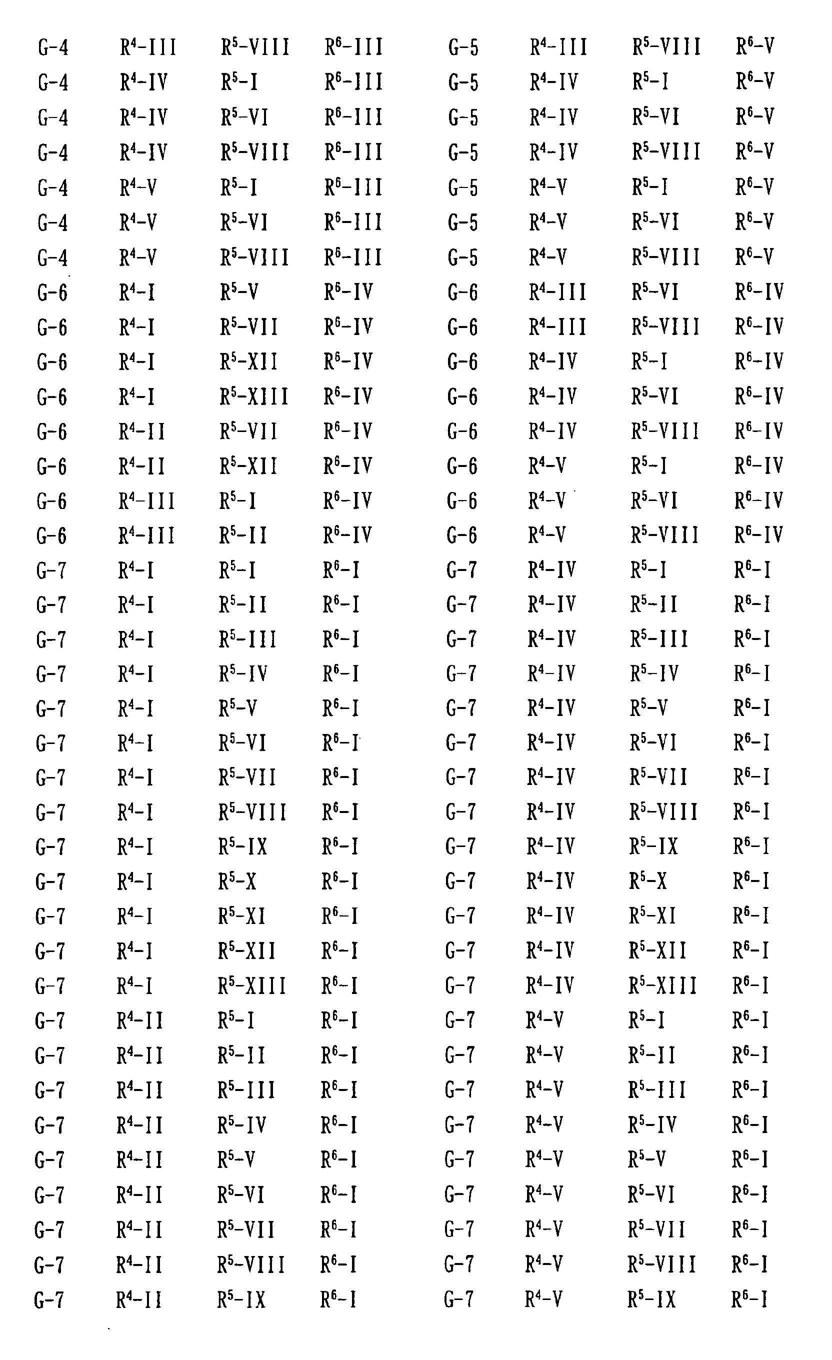

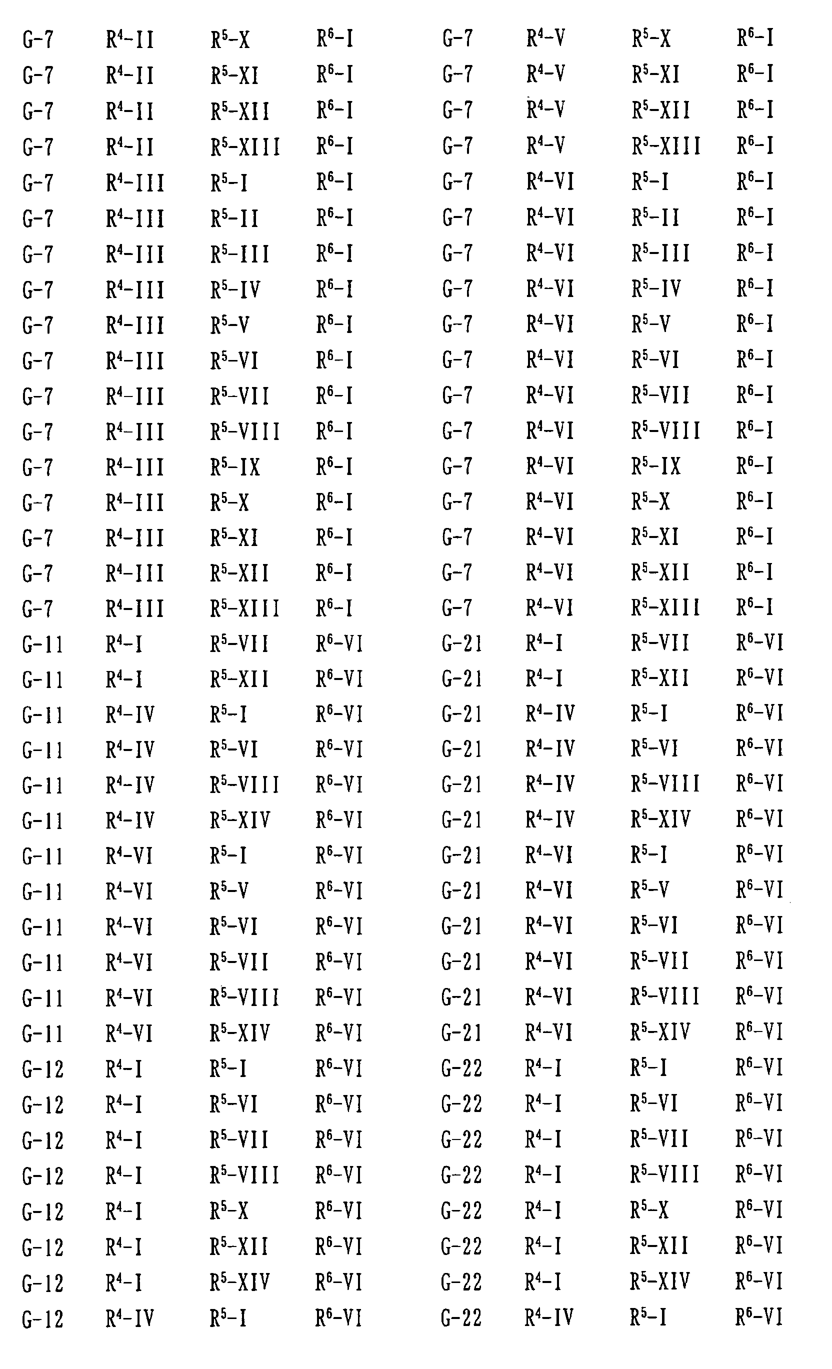

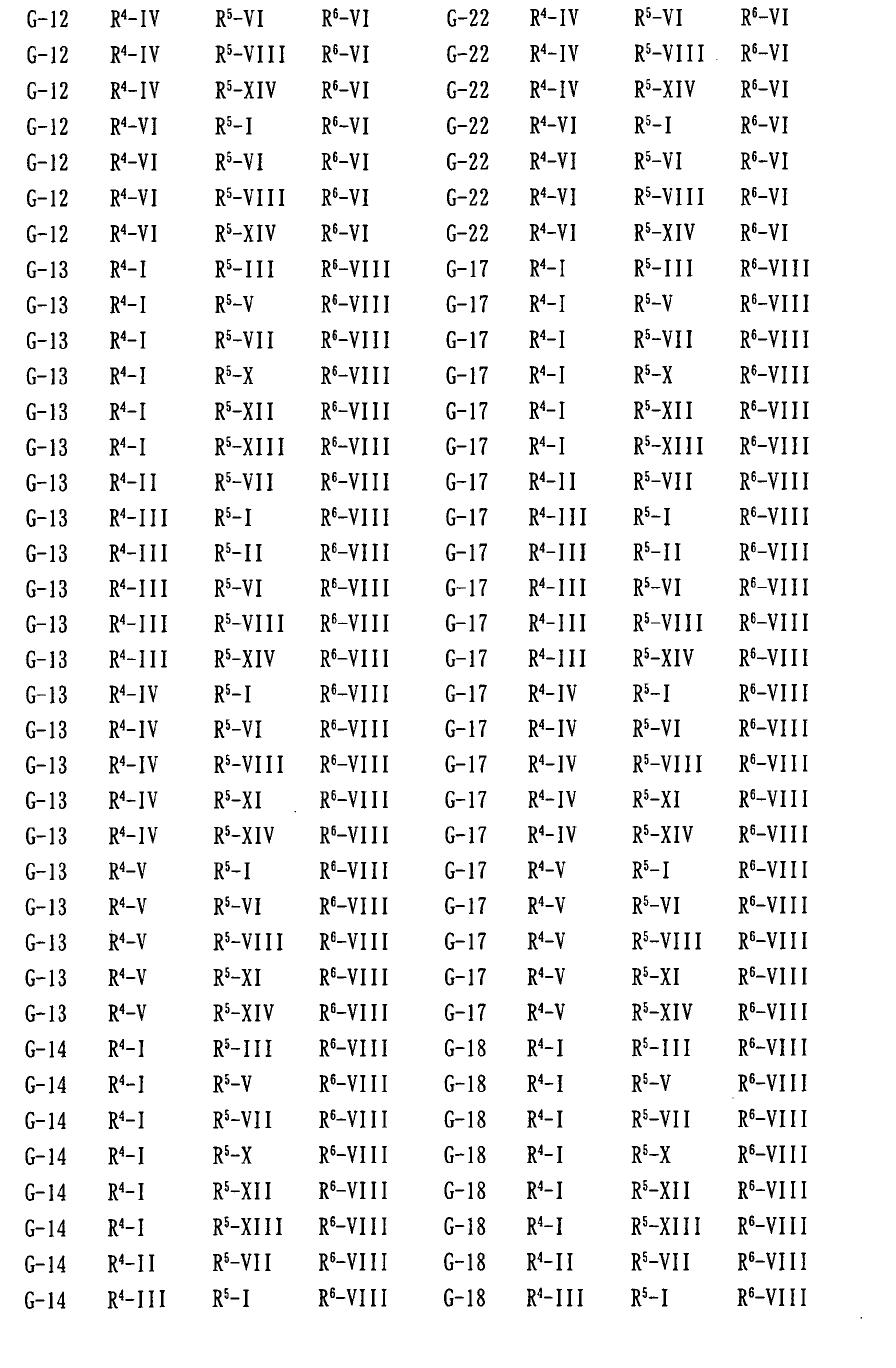

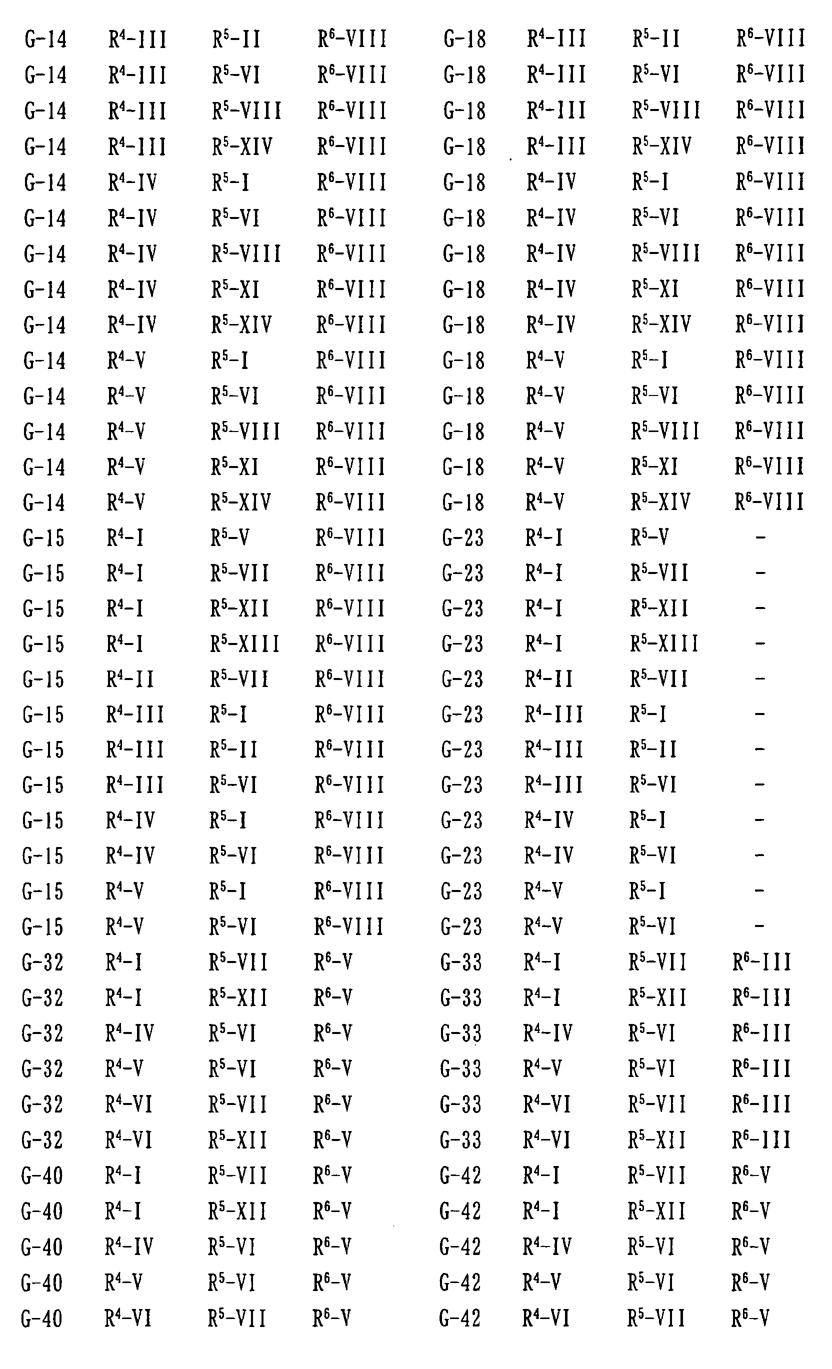

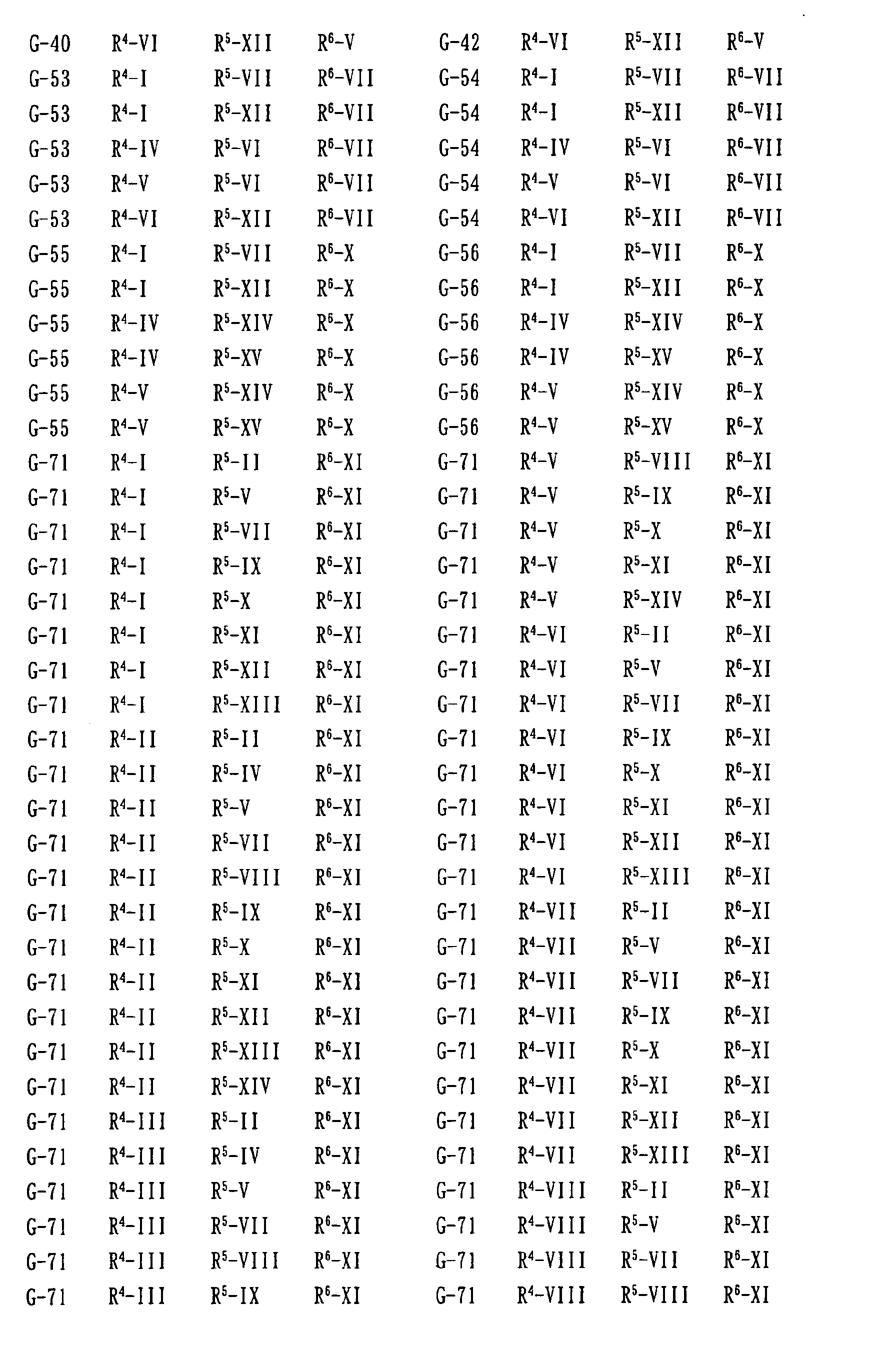

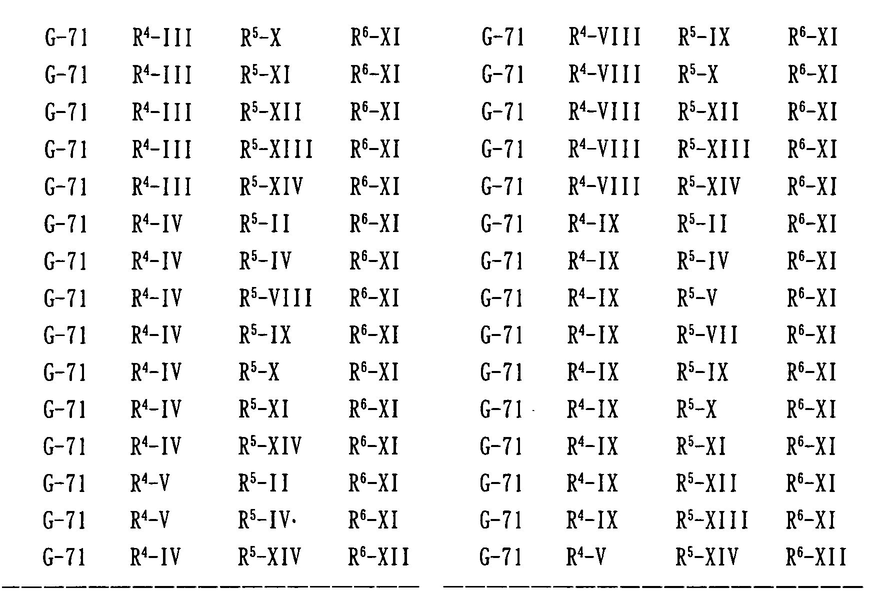

The compounds of the present invention can be prepared by, for example, the following methods.

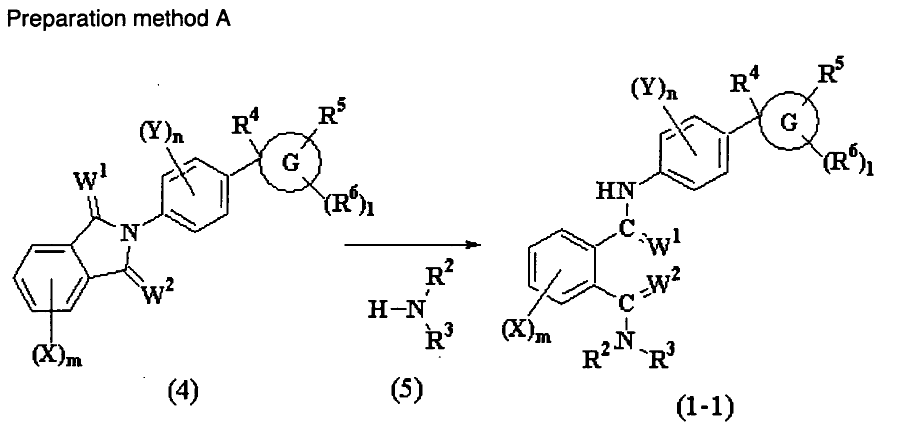

The compound represented by Formula (4) [wherein G, W¹, W², X, Y, R⁴, R⁵, R⁶, I, m and n have the same meanings as defined above.] and the compound represented by Formula (5) [wherein R² and R³ have the same meanings as defined above.] are reacted in a solvent which is inactive to the reaction or in the absence of a solvent, and in the presence of a catalyst if necessary, to obtain the compound of the present invention represented by Formula (1-1) [wherein G, W¹, W², X, Y, R², R³, R⁴, R⁵, R⁶, I, m and n have the same meanings as defined above.] where R¹ in the Formula (1) is a hydrogen atom.

As amounts of the reaction substrates, 1 to 50 equivalents of the compound represented by Formula (5) can be used based on 1 equivalent of the compound represented by Formula (4).

When a solvent is used, the solvent to be used may be any one so long as it does not interfere the progress of the reaction, and there may be mentioned, for example, aromatic hydrocarbons such as benzene, toluene, xylene, etc., aliphatic hydrocarbons such as hexane, heptane, etc., alicyclic hydrocarbons such as cyclohexane, etc., aromatic halogenated hydrocarbons such as chlorobenzene, dichlorobenzene, etc., aliphatic halogenated hydrocarbons such as dichloromethane, chloroform, carbon tetrachloride, 1,2-dichloroethane, 1,1,1-trichloroethane, trichloroethylene, tetrachloroethylene, etc., ethers such as diethyl ether, 1,2-dimethoxyethane, tetrahydrofuran, 1,4-dioxane, etc., esters such as ethyl acetate, ethyl propionate, etc., amides such as dimethylformamide, dimethylacetamide, N-methyl-2-pyrrolidone, etc., carboxylic acids such as formic acid, acetic acid, propionic acid, etc., amines such as triethylamine, tributylamine, N,N-dimethylaniline, etc., pyridines such as pyridine, picoline, etc., alcohols such as methanol, ethanol, ethylene glycol, etc., acetonitrile, dimethylsulfoxide, sulforane, 1,3-dimethyl-2-imidazolidinone and water, etc. These solvents may be used alone, or may be used two or more kinds in admixture.

When a catalyst is used, the catalyst for the reaction may be mentioned, for example, mineral acids such as hydrochloric acid, sulfuric acid, nitric acid, etc., organic acids such as formic acid, acetic acid, propionic acid, trifluoroacetic acid , methanesulfonic acid, benzenesulfonic acid, p-toluene sulfonic acid, etc., acid addition salts of an amine such as triethylamine hydrochloride, pyridine hydrochloride, etc., Lewis acids such as zinc chloride, zinc iodide, titanium tetrachloride, cerium chloride, ytterbium trifrate, boron trifluoride-ether complex, etc., in an amount of 0.001 to 1 equivalent based on the compound represented by Formula (4).

The reaction temperature may be set at an optional temperature from -60°C to a reflux temperature of the reaction mixture, and the reaction time may vary depending on the concentrations of the reaction substrates, and the reaction temperature, and may be optionally set usually in the range of 5 minutes to 100 hours.

In general, the reaction is preferably carried out by using, for example, 1 to 10 equivalents of the compound represented by Formula (5) based on 1 equivalent of the compound represented by Formula (4), in the absence of a solvent, or using a solvent such as tetrahydrofuran or 1,4-dioxane, etc., in the temperature range of from 50°C to a reflux temperature of the reaction mixture for 30 minutes to 24 hours.

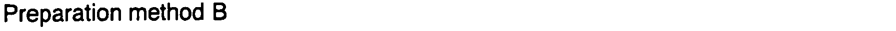

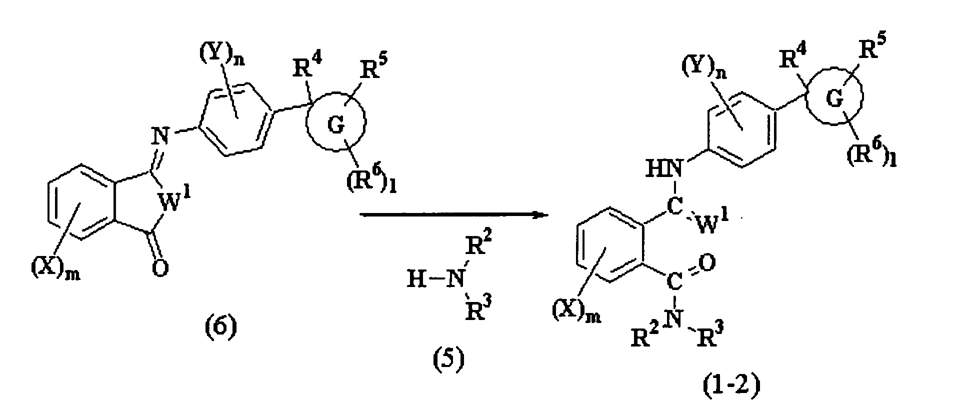

The compound represented by Formula (6) [wherein G, W¹, X, Y, R⁴, R⁵, R⁶, I, m and n have the same meanings as defined above.] and the compound represented by Formula (5) [wherein R² and R³ have the same meanings as defined above.] are reacted under the similar conditions as in Preparation method A to obtain the compound of the present invention represented by Formula (1-2) [wherein G, W¹, X, Y, R², R³, R⁴, R⁵, R⁶, I, m and n have the same meanings as defined above.] where W² is an oxygen atom and R¹ is a hydrogen atom in Formula (1).

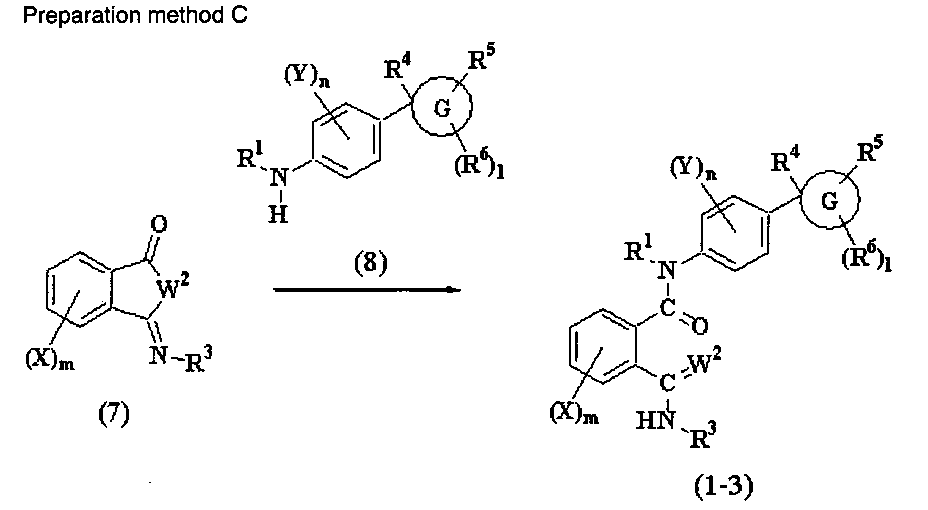

The compound represented by Formula (7) [wherein W², X, R³ and m have the same meanings as defined above.] and the compound represented by Formula (8) [wherein G, Y, R¹, R⁴, R⁵, R⁶, l and n have the same meanings as defined above.] are reacted under the similar conditions as in Preparation method A to obtain the compound of the present invention represented by Formula (1-3) [wherein G, W², X, Y, R¹, R³, R⁴, R⁵, R⁶, l, m and n have the same meanings as defined above.] where W² is an oxygen atom and R² is a hydrogen atom in Formula (1).

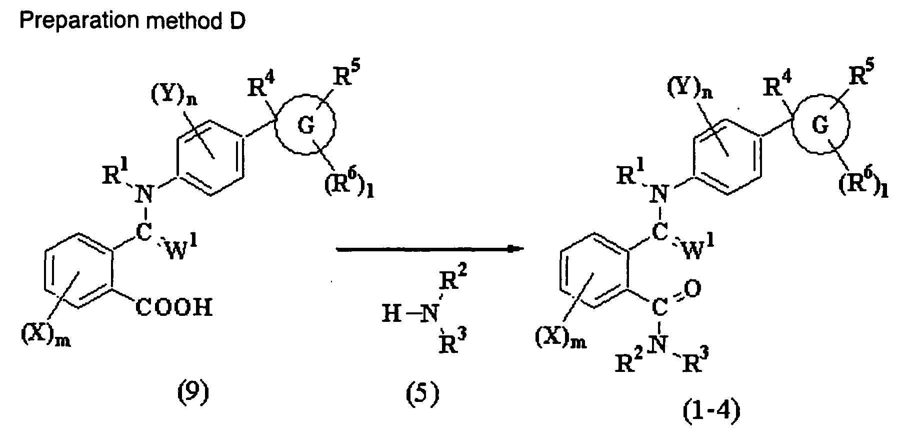

The compound represented by Formula (9) [wherein G, W¹, X, Y, R¹, R⁴, R⁵, R⁶, I, m and n have the same meanings as defined above.] and the compound represented by Formula (5) [wherein R² and R³ have the same meanings as defined above.] are reacted in a solvent which is inactive to the reaction or in the absence of a solvent, if necessary, in the presence of a base and using a condensing agent, to obtain the compound of the present invention represented by Formula (1-4) [wherein G, W¹, X, Y, R¹, R², R³, R⁴, R⁵, R⁶, l, m and n have the same meanings as defined above.] where W² is an oxygen atom in Formula (1).

As amounts of the reaction substrates, 1 to 100 equivalents of the compound represented by Formula (5) can be used based on 1 equivalent of the compound represented by Formula (9).

The condensing agent is not particularly limited so long as it can be used for usual amide synthesis, and there may be mentioned, for example, Mukaiyama reagent (2-chloro-N-methylpyridinium iodide), DCC (1,3-dicyclohexylcarbodiimide), WSC (1-ethyl-3-(3-dimethylaminopropyl)-carbodiimide hydrochloride), CDI (carbonyldiimidazole), dimethylpropynylsulfonium bromide, propargyltriphenyl phosphonium bromide , DEPC (diethyl cyanophosphate), etc., and it can be used in an amount of 1 to 4 equivalents based on the amount of the compound represented by Formula (9).

When a solvent is used, the solvent to be used may be any one so long as it does not interfere the progress of the reaction, and there may be mentioned, for example, aromatic hydrocarbons such as benzene, toluene, xylene, etc., aliphatic hydrocarbons such as hexane, heptane, etc., alicyclic hydrocarbons such as cyclohexane, etc., aromatic halogenated hydrocarbons such as chlorobenzene, dichlorobenzene, etc., aliphatic halogenated hydrocarbons such as dichloromethane, chloroform, carbon tetrachloride, 1,2-dichloroethane, 1,1,1-trichloroethane, trichloroethylene, tetrachloroethylene, etc., ethers such as diethyl ether, 1,2-dimethoxyethane, tetrahydrofuran, 1,4-dioxane, etc., esters such as ethyl acetate, ethyl propionate, etc., amides such as dimethylformamide, dimethylacetamide, N-methyl- 2-pyrrolidone, etc., amines such as triethylamine, tributylamine, N,N-dimethylaniline, etc., pyridines such as pyridine, picoline, etc., acetonitrile and dimethylsulfoxide, etc. These solvents may be used alone, or may be used two or more kinds in admixture.

Addition of a base is not necessarily required, and when the base is used, the base to be used may be mentioned, for example, alkali metal hydroxides such as sodium hydroxide, potassium hydroxide, etc., alkali metal carbonates such as sodium carbonate, potassium carbonate, sodium hydrogen carbonate, etc., organic bases such as triethylamine, tributylamine, N,N-dimethylaniline , pyridine, 4-(dimethylamino)-pyridine, imidazole, 1,8-diazabicyclo[5,4,0]-7-undecene, etc., and it can be used in an amount of 1 to 4 equivalents based on the amount of the compound represented by Formula (9).

The reaction temperature can be set an optional temperature from -60°C to the reflux temperature of the reaction mixture, and the reaction time may vary depending on the concentrations of the reaction substrates, and the reaction temperature, but it can be optionally set usually within the range of from 5 minutes to 100 hours.

In general, the reaction is preferably carried out by using, for example, 1 to 20 equivalents of the compound represented by Formula (5) and 1 to 4 equivalents of a condensing agent such as WSC (1-ethyl-3-(3-dimethylaminopropyl)-carbodiimide hydrochloride), CDI (carbonyldiimidazole), etc., based on 1 equivalent of the compound represented by Formula (9), and if necessary, in the presence of 1 to 4 equivalents of a base such as potassium carbonate, triethylamine, pyridine, 4-(dimethylamino)pyridine, etc., in the absence of a solvent or in the presence of a solvent such as dichloromethane, chloroform, diethyl ether, tetrahydrofuran, 1,4-dioxane, etc., in the range of 0°C to a reflux temperature of these solvents, for 10 minutes to 24 hours.

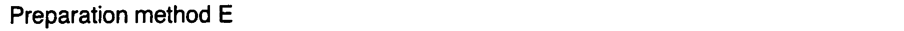

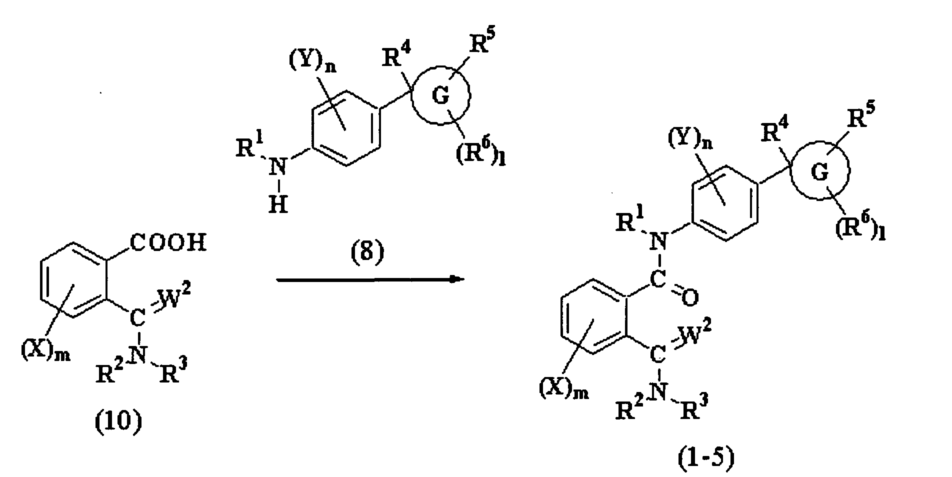

The compound represented by Formula (10) [wherein W², X, R², R³ and m have the same meanings as defined above.] and the compound represented by Formula (8) [wherein G, Y, R¹, R⁴, R⁵, R⁶, l and n have the same meanings as defined above.] are reacted under the similar conditions as in Preparation method D to obtain the compound of the present invention represented by Formula (1-5) [wherein G, W², X, Y, R¹, R², R³, R⁴, R⁵, R⁶, l, m and n have the same meanings as defined above.] where W¹ is an oxygen atom in Formula (1).

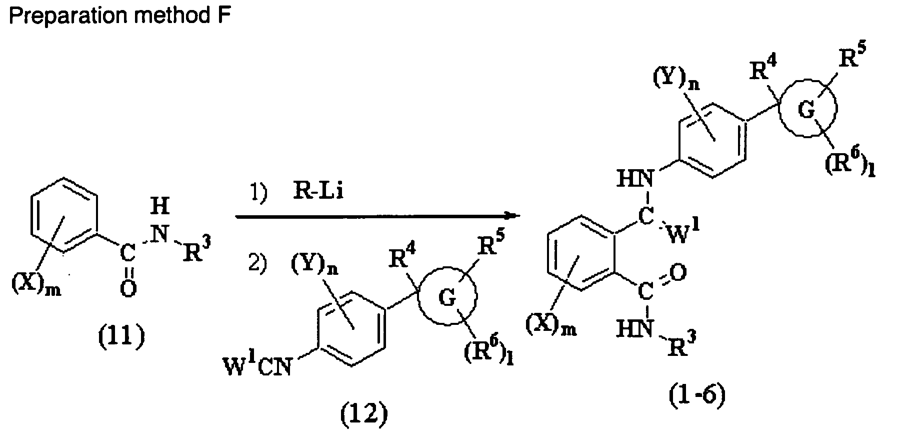

The compound represented by Formula (11) [wherein X, R³ and m have the same meanings as defined above.] is subjected to selective lithiation according to the conventionally known method described in a literature, for example, the method described in Chemical Reviews [Chem. Rev.] 1990, vol. 90, p. 879, etc., and then, reacting with the compound represented by Formula (12) [wherein G, W¹, Y, R⁴, R⁵, R⁶, I and n have the same meanings as defined above.] to obtain the compound of the present invention represented by Formula (1-6) [wherein G, W¹, X, Y, R³, R⁴, R⁵, R⁶, I, m and n have the same meanings as defined above.] where W² is an oxygen atom, and R¹ and R² are hydrogen atoms in Formula (1). Incidentally, R-Li represents an alkyl lithium reagent such as butyl lithium, etc.

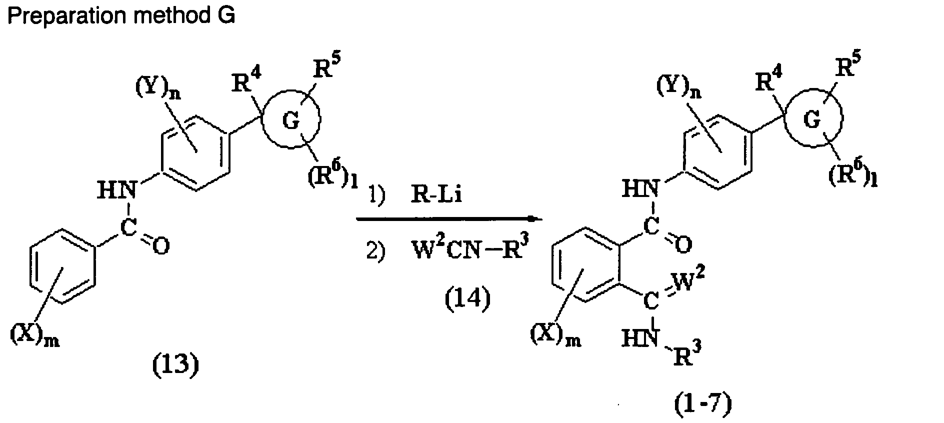

The compound represented by Formula (13) [wherein G, X, Y, R⁴, R⁵, R⁶, I, m and n have the same meanings as defined above.] and the compound represented by Formula (14) [wherein W² and R³ have the same meanings as defined above.] are reacted under similar conditions as in Preparation method F to obtain the compound of the present invention represented by Formula (1-7) [wherein G, W², X, Y, R³, R⁴, R⁵, R⁶, I, m and n have the same meanings as defined above.] where W¹ is an oxygen atom, and R¹ and R² are hydrogen atoms in Formula (1). Incidentally, R-Li represents an alkyl lithium reagent such as butyl lithium, etc.

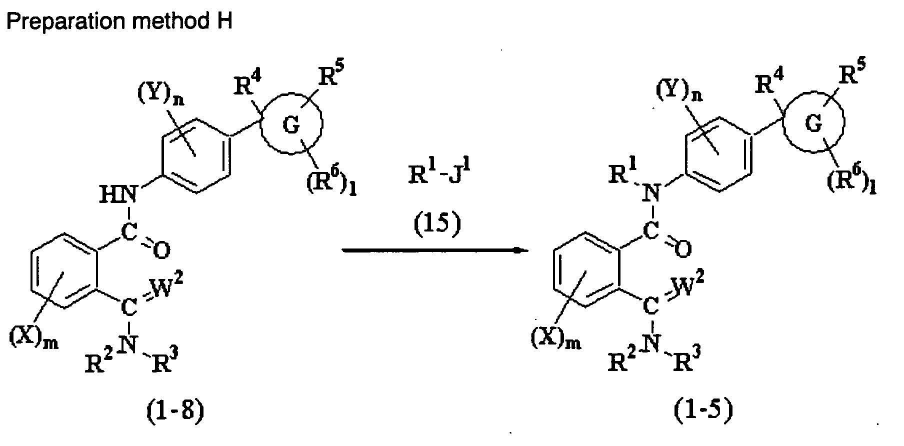

The compound of the present invention represented by Formula (1-8) [wherein G, W², X, Y, R², R³, R⁴, R⁵, R⁶, l, m and n have the same meanings as defined above.] where W¹ is an oxygen atom, and R¹ is a hydrogen atom in the compound represented by Formula (1) and the compound represented by Formula (15) [wherein R¹ has the same meaning as defined above, and J¹ represents a good eliminating group such as chlorine atom, bromine atom, iodine atom, a C₁ to C₄ alkylcarbonyloxy group (e.g., pivaloyloxy group), a C₁ to C₄ alkylsulfonate group (e.g., methanesulfonyloxy group), a C₁ to C₄ haloalkylsulfonate group (e.g., trifluoromethanesulfonyloxy group), an arylsulfonate group (e.g., benzenesulfonyloxy group, p-toluene sulfonyloxy group) or an azolyl group (e.g., imidazole-1-yl group), etc.] are reacted, if necessary, in the presence of a base, and if necessary, by using a solvent which is inactive to said reaction to obtain the compound of the present invention represented by Formula (1-5) [wherein G, W², X, Y, R¹, R², R³, R⁴, R⁵, R⁶, I, m and n have the same meanings as defined above.] where W¹ is an oxygen atom in Formula (1).

As amounts of the reaction substrates, 1 to 50 equivalents of the compound represented by Formula (15) can be used based on 1 equivalent of the compound represented by Formula (1-8).

When a solvent is used, the solvent to be used may be any one so long as it does not interfere the progress of the reaction, and there may be mentioned, for example, aromatic hydrocarbons such as benzene, toluene, xylene, etc., aliphatic hydrocarbons such as hexane, heptane, etc., alicyclic hydrocarbons such as cyclohexane, etc., aromatic halogenated hydrocarbons such as chlorobenzene, dichlorobenzene, etc., aliphatic halogenated hydrocarbons such as dichloromethane, chloroform, carbon tetrachloride, 1,2-dichloroethane, 1,1,1-trichloroethane, trichloroethylene, tetrachloroethylene, etc., ethers such as diethyl ether, 1,2-dimethoxyethane, tetrahydrofuran, 1,4-dioxane, etc., esters such as ethyl acetate, ethyl propionate, etc., amides such as dimethylformamide, dimethylacetamide, N-methyl-2-pyrrolidone, etc., amines such as triethylamine, tributylamine, N,N-dimethylaniline, etc., pyridines such as pyridine, picoline, etc., alcohols such as methanol, ethanol, ethylene glycol, etc., acetonitrile, dimethylsulfoxide, sulforane, 1,3-dimethyl-2-imidazolidinone and water, etc. These solvents may be used alone, or may be used two or more kinds in admixture.

When a base is used, as the base to be used, there may be mentioned, for example, alkali metal hydrides such as sodium hydride, potassium hydrides, etc., alkali metal hydroxides such as sodium hydroxide, potassium hydroxide, etc., alkali metal alkoxides such as sodium ethoxide, potassium tert-butoxide, etc., alkali metal amides such as lithium diisopropylamide, lithium hexamethyldisilazane, sodium amide, etc., organic metal compounds such as tertiary butyl lithium, etc., alkali metal carbonates such as sodium carbonate, potassium carbonate, sodium hydrogen carbonate, etc., organic bases such as triethylamine, tributylamine, N,N-dimethylaniline, pyridine, 4-(dimethylamino)pyridine, imidazole, 1,8-diazabicyclo[5,4,0]-7-undecene, etc., and it can be used in an amount of 1 to 4 equivalents based on the amount of the compound represented by Formula (1-8).

The reaction temperature can be set an optional temperature from -60°C to the reflux temperature of the reaction mixture, and the reaction time may vary depending on the concentrations of the reaction substrates, and the reaction temperature, but it can be optionally set usually within the range of from 5 minutes to 100 hours.

In general, the reaction is preferable carried out by using, for example, 1 to 10 equivalents of the compound represented by Formula (15) based on 1 equivalent of the compound represented by Formula (1-8), in tetrahydrofuran, 1,4-dioxane, acetonitrile or a polar solvent such as N,N-dimethylformamide, etc., if necessary, by using 1 to 3 equivalents of a base such as sodium hydride, potassium tert-butoxide, potassium hydroxide, potassium carbonate, triethylamine or pyridine, etc. based on 1 equivalent of the compound represented by Formula (1-8) in a temperature range of 0 to 90°C for 10 minutes to 24 hours.

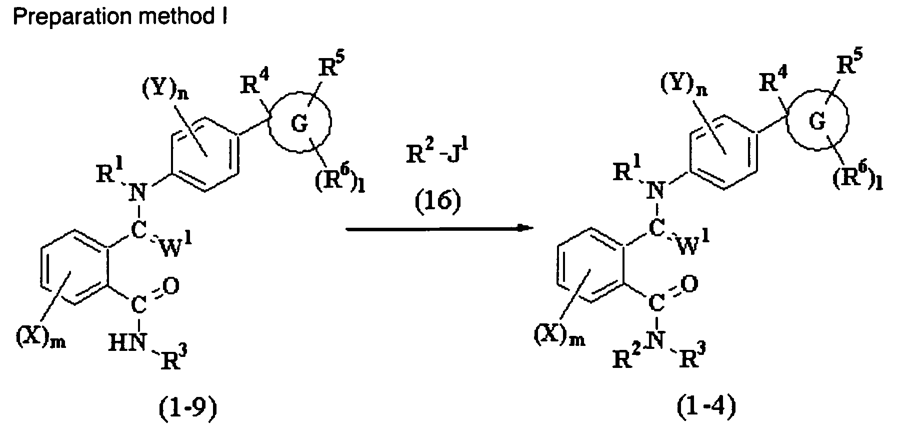

The compound of the present invention represented by Formula (1-9) [wherein G, W¹, X, Y, R¹, R³, R⁴, R⁵, R⁶, I, m and n have the same meanings as defined above.] where W² is an oxygen atom and R² is a hydrogen atom in Formula (1) and the compound represented by Formula (16) [wherein R² and J¹ have the same meanings as defined above.] are reacted under the similar conditions as in Preparation method H to obtain the compound of the present invention represented by Formula (1-4) [wherein G, W¹, X, Y, R¹, R², R³, R⁴, R⁵, R⁶, I, m and n have the same meanings as defined above.] where W² is an oxygen atom in Formula (1).

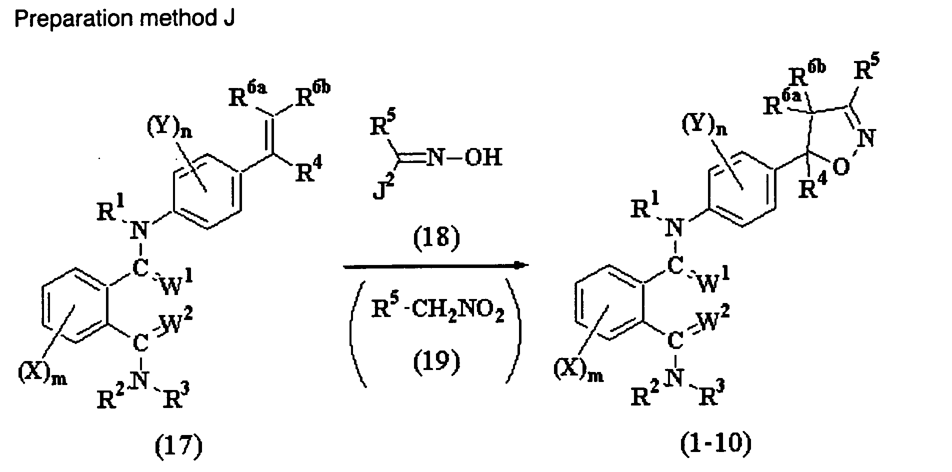

The compound represented by Formula (17) [wherein W¹, W², X, Y, R¹, R², R³, R⁴, R^{6a}, R^{6b}, m and n have the same meanings as defined above.] and the compound represented by Formula (18) [wherein R⁵ has the same meaning as defined above, J² represents a hydrogen atom or a halogen atom such as chlorine atom, bromine atom, etc.] are reacted by the methods according to the conventionally known method described in literatures, for example, the methods as described in Heterocycles, 1996, vol. 43, p. 1771, Journal of Heterocyclic Chemistry [J. Heterocyclic Chem.] 1990, vol. 27, p. 769, Justus Liebigs Annalen der Chemie [Justus Liebigs Ann. Chem.] 1989, p. 985, Tetrahedron: Asymmetry, 1997, vol. 8, p. 245, Tetrahedron Letters [Tetrahedron Lett.] 1986, vol. 27, p. 4647, International Unexamined Patent Publications (WO 01/12613 publication, WO 02/076956 publication), etc.,

Or else, the compound represented by Formula (17) and the compound represented by Formula (19) [wherein R⁵ have the same meanings as defined above.] are reacted by the methods according to the conventionally known method described in literatures, for example, the methods as described in Chemistry Letters [Chem. Lett.], 1990, p. 559, Synthesis, 1997, p. 309, Synthetic Communications [Synth. Commun.], 1988, vol. 18, p. 2315, etc., to obtain the compound represented by Formula (1-10) [wherein W¹, W², X, Y, R¹, R², R³, R⁴, R⁵, R^{6a}, R⁶, m and n have the same meanings as defined above.] where G is G-7 in Formula (1).

In Preparation method A to Preparation method J, the reaction mixture after completion of the reaction can be subjected to usual post-treatment such as direct concentration, or dissolution in an organic solvent, and after washing with water, subjecting to concentration, or pouring in ice-water, extraction with an organic solvent and then concentration, and the like, to obtain the objective compound of the present invention. Also, when purification is required to be carried out, it can be separated and purified by optional purification methods such as recrystallization, column chromatography, thin layer chromatography, fractionation by liquid chromatography, and the like.

In Preparation method A, the compound represented by Formula (4), and the compound represented by Formula (2) which is a compound where W¹ and W² are oxygen atoms and (X)ₘ is a nitro group at the 3-position in the compound represented by Formula (4), which are starting compounds for preparing the compound of the present invention, can be synthesized as mentioned below.

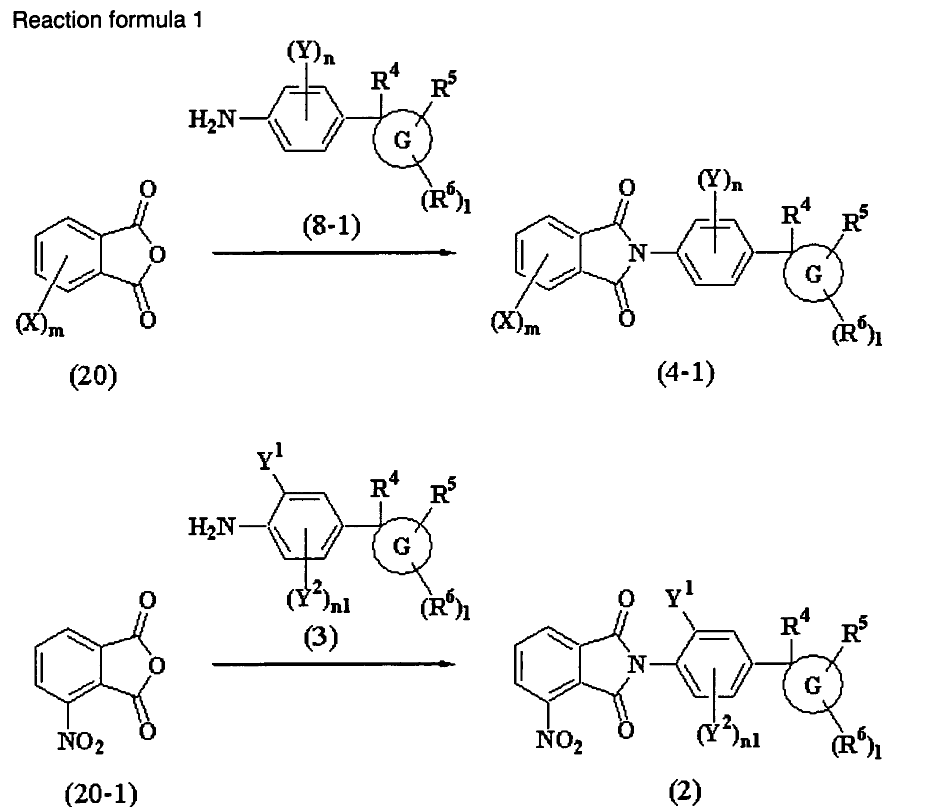

That is, the compound represented by Formula (20) [wherein X and m have the same meanings as defined above.] and the compound represented by Formula (8-1) [wherein G, Y, R⁴, R⁵, R⁶, l and n have the same meanings as defined above.] where R¹ is a hydrogen atom in the compound represented by Formula (8) are reacted in accordance with the conventionally known method described in literatures, for example, the methods as described in Berichte der Deutschen Chemischen Gesellschaft [Ber. Dtsch. Chem. Ges.] 1907, vol. 40, p. 3177, Journal of the Chemical Society [J. Chem. Soc.] 1954, p. 2023, Journal of the Chemical Society Perkin Transactions, 1 [J. Chem. Soc. Perkin Trans. 1] 1994, p. 2975, etc., the compound represented by Formula (4-1) [wherein G, X, Y, R⁴, R⁵, R⁶, l, m and n have the same meanings as defined above.] where W¹ and W² are oxygen atoms in Formula (4) can be easily synthesized.

Also, commercially available 3-nitrophthalic anhydride (20-1) and the compound represented by Formula (3) [wherein G, Y¹, Y², R⁴, R⁵, R⁶, l and n1 have the same meanings as defined above.] are reacted under the similar conditions as mentioned above, the compound represented by Formula (2) [wherein G, X, Y¹, Y², R⁴, R⁵, R⁶, l, m and n1 have the same meanings as defined above.] can be synthesized.

Some of the compound represented by Formula (5) used in Preparation method A, Preparation method B and Preparation method D are conventionally known compounds, and some of which can be available as commercially available products. Also, those other than the above can be synthesized according to the methods described in, for example, the methods as described in Chemical and Pharmaceutical Bulletin [Chem. Pharm. Bull.] 1982, vol. 30, p. 1921, Journal of the American Chemical Society [J. Am. Chem. Soc.] 1986, vol. 108, p. 3811, International Unexamined Patent Publications (WO 01/23350 publication), etc., and the respective synthetic methods of other primary or secondary alkylamines described in literatures.

In Preparation method B, the compound represented by Formula (6) which is a starting compound for preparing the compound of the present invention can be synthesized as mentioned below.

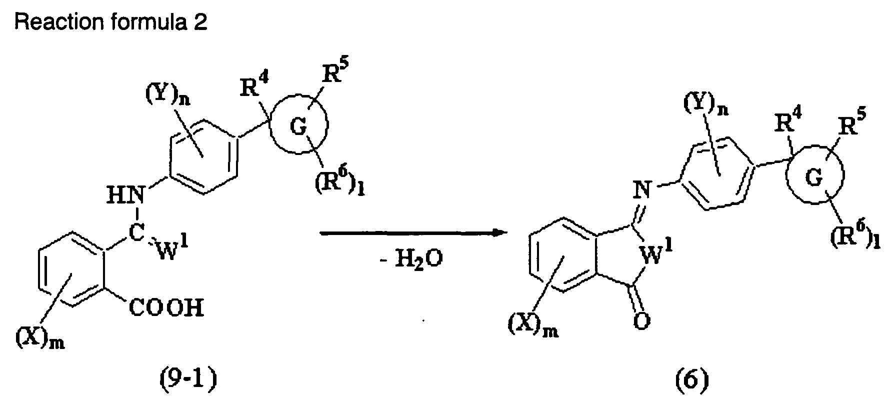

That is, the compound represented by Formula (9-1) [wherein G, W¹, X, Y, R⁴, R⁵, R⁶, l, m and n have the same meanings as defined above.] where R¹ is a hydrogen atom in Formula (9) is subjected to cyclization according to the methods such as a synthetic method of isoimide by general dehydration and cyclization described in literatures, for example, the methods described in Journal of the American Chemical Society [J. Am. Chem. Soc.] 1975, vol. 97, p. 5582, Journal of Medicinal Chemistry [J. Med. Chem.] 1967, vol. 10, p. 982, The Journal of Organic Chemistry [J. Org. Chem.] 1963, vol. 28, p. 2018, etc., the compound represented by Formula (6) [wherein G, W¹, X, Y, R⁴, R⁵, R⁶, I, m and n have the same meanings as defined above.] can be easily synthesized.

In Preparation method C, the compound represented by Formula (7) which is a starting compound for preparing the compound of the present invention can be synthesized as mentioned below.

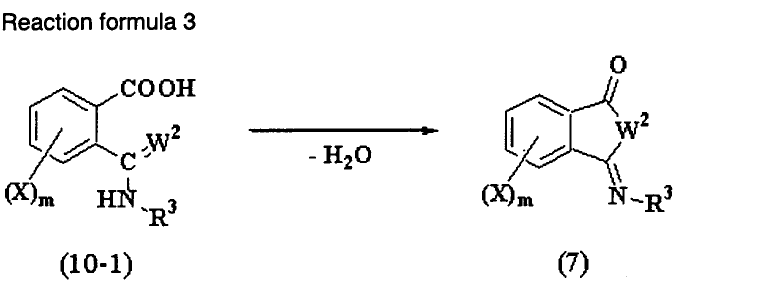

That is, the compound represented by Formula (10-1) [wherein W², X, R³ and m have the same meanings as defined above.] wherein R² is a hydrogen atom in Formula (10) is reacted in the same manner as in Reaction formula 2, the compound represented by Formula (7) [wherein W², X, R³ and m have the same meanings as defined above.] can be easily synthesized.
the compound represented by Formula (8) and the compound represented by Formula (3) used in Preparation method C and Preparation method E can be synthesized by, for example, using the method shown by the following Reaction formula 4 to Reaction formula 16 and the like.

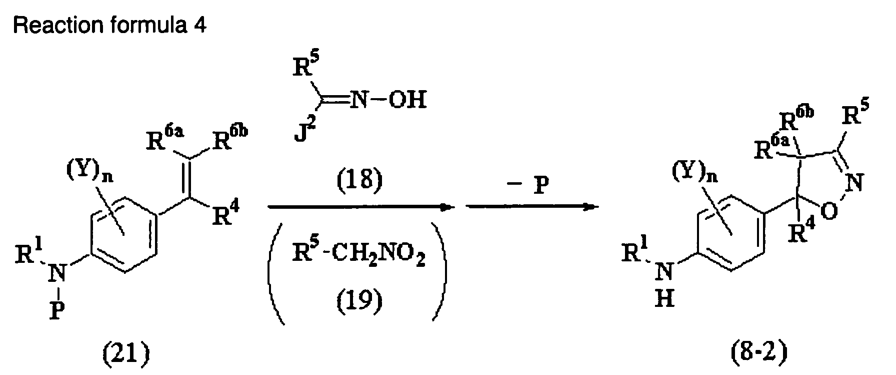

The compound represented by Formula (21) [wherein Y, R¹, R⁴, R^{6a}, R^{6b} and n have the same meanings as defined above, and P represents a protective group for an amino group generally employed such as acetyl group, pivaloyl group, benzoyl group, tert-butoxycarbonyl group, benzyloxycarbonyl group, etc.] and the compound represented by Formula (18) [wherein R⁵ and J² have the same meanings as defined above.] or the compound represented by Formula (19) [wherein R⁵ have the same meanings as defined above.] are reacted in the similar conditions as in Preparation method J to form an isoxazoline ring, and subjecting to deprotection by the method generally employed for the respective protective groups, so that the compound represented by Formula (8-2) [wherein Y, R¹, R⁴, R⁵, R^{6a}, R^{6b} and n have the same meanings as defined above.] wherein G is G-7 in Formula (8) can be obtained.

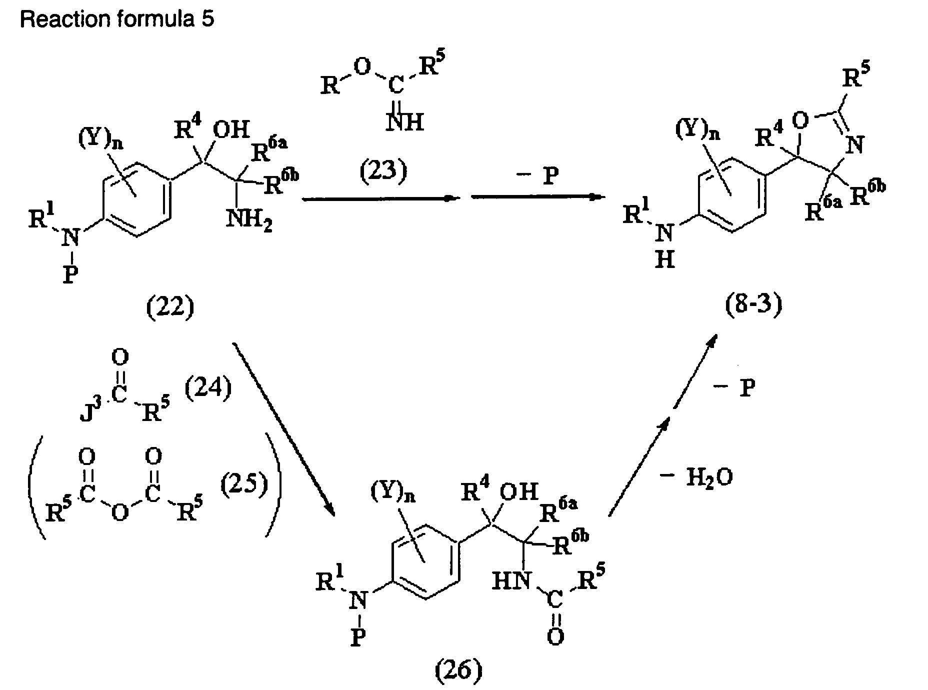

The compound represented by Formula (22) [wherein Y, R¹, R⁴, R^{6a}, R^{6b}, n and P have the same meanings as defined above.] and the compound represented by Formula (23) [wherein R⁵ has the same meaning as defined above, R represents a lower alkyl group such as methyl, ethyl, etc.] are condensed according to the conventionally known methods described in literatures, for example, the methods as described in Chemische Berichte [Chem. Ber.] 1958, vol. 91, p. 1098, etc., and then, subjecting to deprotection by the method generally employed for the respective protective groups, so that the compound represented by Formula (8-3) [wherein Y, R¹, R⁴, R⁵, R^{6a}, R^{6b} and n have the same meanings as defined above.] wherein G is G-13 in Formula (8) can be obtained.

Or else, the compound represented by Formula (22) and the conventionally known compound represented by Formula (24) [wherein R⁵ has the same meaning as defined above, J³ represents an eliminating group such as a halogen atom, a C₁ to C₄ alkoxy group (e.g., methoxy group, ethoxy group), an aryloxy group (e.g., phenoxy group), a C₁ to C₄ alkylcarbonyloxy group (e.g., pivaloyloxy group) or a C₁ to C₄ alkoxycarbonyloxy group (e.g., isobutyloxycarbonyloxy group).] or the conventionally known compound represented by Formula (25) [wherein R⁵ have the same meanings as defined above.] are subjected to general acylation reaction of an amine according to the method described in the literatures, for example, the methods as described in Journal of the American Chemical Society [J. Am. Chem. Soc.] 2000, vol. 122, p. 2149, Tetrahedron: Asymmetry, 1993, vol. 4, p. 205, International Unexamined Patent Publication (WO 99/24393 publication), etc., and the compound represented by Formula (26) [wherein Y, R¹, R⁴, R⁵, R^{6a}, R^{6b}, n and P have the same meanings as defined above.] obtained by the above reaction is subjected to form an oxazoline ring by dehydration and cyclization according to the conventionally known methods described in literatures, for example, the methods as described in Berichte der Deutschen Chemischen Gesellschaft [Ber. Dtsch. Chem. Ges.] 1940, vol. 73, p. 656, Journal of Heterocyclic Chemistry [J. Heterocyclic Chem.] 2000, vol. 37, p. 343, European Patent Publication (EP 0,895,992 Publication), etc., and subjecting to deprotection by the method generally employed for the respective protective groups, so that the compound represented by Formula (8-3) can be obtained.

Here, the compound represented by Formula (23) to be used is a conventionally known compound, some of which can be obtained as a commercially available product. Also, those other than the above can be easily synthesized according to the conventionally known methods described in literatures, for example, the methods as described in Chemische Berichte [Chem. Ber.] 1959, vol. 92, p. 330 and 1985, vol. 118, p. 3089, Journal of the American Chemical Society [J. Am. Chem. Soc.] 1948, vol. 70, p. 165, etc.

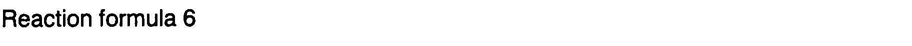

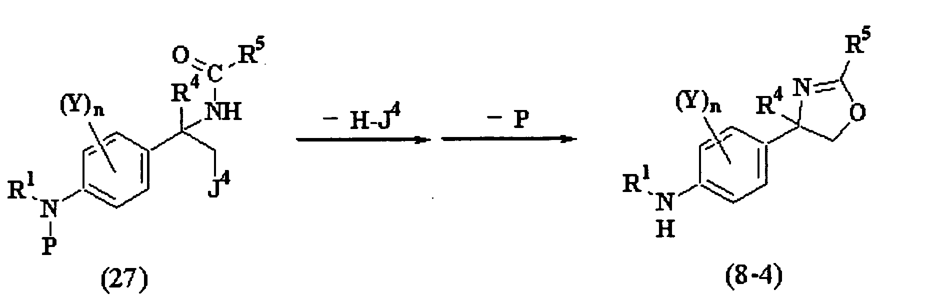

The compound represented by Formula (27) [wherein Y, R¹, R⁴, R⁵, n and P have the same meanings as defined above, J⁴ represents a good eliminating group such as chlorine atom, bromine atom, iodine atom, a C₁ to C₄ alkylsulfonate group (e.g., methanesulfonyloxy group), a C₁ to C₄ haloalkylsulfonate group (e.g., trifluoromethanesulfonyloxy group) or an arylsulfonate group (e.g., benzenesulfonyloxy group, p-toluene sulfonyloxy group).] is subjected to form an oxazoline ring according to the conventionally known methods described in literatures, for example, the methods as described in Bulletin of the Chemical Society of Japan [Bull. Chem. Soc. Jpn.] 1996, vol. 69, p. 3345, German Patent Publication (DE 19528778 Publication), etc., and then, subjecting to deprotection by the generally employed method for the respective protective groups, so that the compound represented by Formula (8-4) [wherein Y, R¹, R⁴, R⁵ and n have the same meanings as defined above.] wherein G is G-14, and R^{6e} and R^{6f} are hydrogen atoms in Formula (8) can be obtained.

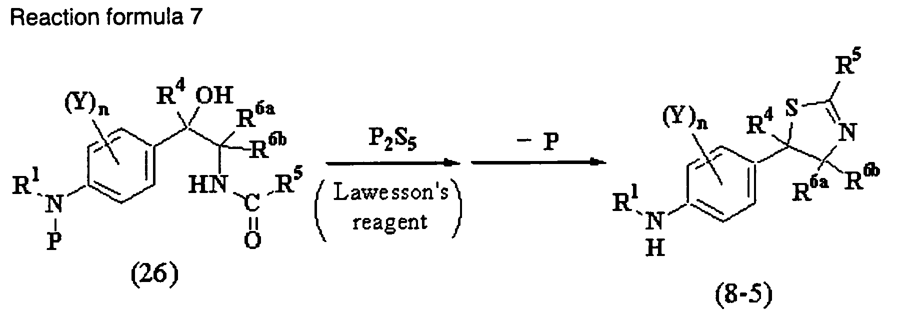

The compound represented by Formula (26) [wherein Y, R¹, R⁴, R⁵, R^{6a}, R^{6b}, n and P have the same meanings as defined above.] is reacted with diphosphorus pentasulfide or Lawesson's Reagent, etc. according to the conventionally known methods described in literatures, for example, the methods as described in The Journal of Organic Chemistry [J. Org. Chem.] 1997, vol. 62, p. 1106, etc. to form a thiazoline ring, and then, subjecting to deprotection by using the generally employed method for the respective protective groups, so that the compound represented by Formula (8-5) [wherein Y, R¹, R⁴, R⁵, R^{6a}, R^{6b} and n have the same meanings as defined above.] wherein G is G-17 in Formula (8) can be obtained.

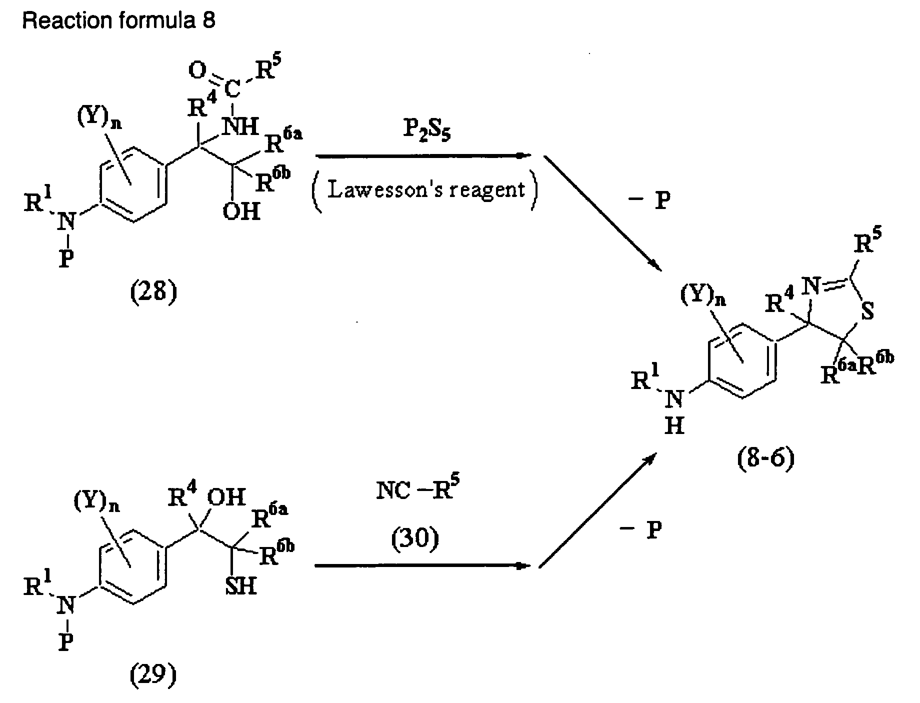

The compound represented by Formula (28) [wherein Y, R¹, R⁴, R⁵, R^{6a}, R^{6b}, n and P have the same meanings as defined above.] is reacted with diphosphorus pentasulfide or Lawesson's Reagent according to the conventionally known methods described in literatures, for example, the methods as described in German Patent Publication (DE 19528778 Publication), etc. to form a thiazoline ring, and then, subjecting to deprotection by the generally employed method for the respective protective groups, so that the compound represented by Formula (8-6) [wherein Y, R¹, R⁴, R⁵, R^{6a}, R^{6b} and n have the same meanings as defined above.] wherein G is G-18 in Formula (8) can be obtained.

Or else, the compound represented by Formula (29) [wherein Y, R¹, R⁴, R^{6a}, R^{6b}, n and P have the same meanings as defined above.] and the conventionally known compound represented by Formula (30) [wherein R⁵ have the same meanings as defined above.] are reacted according to the conventionally known methods described in literatures, for example, the methods as described in The Journal of Organic Chemistry [J. Org. Chem.] 1960, vol. 25, p. 1147, etc. to form a thiazoline ring, and then, subjecting to deprotection by the generally employed method for the respective protective groups, so that the compound represented by Formula (8-6) can be obtained.

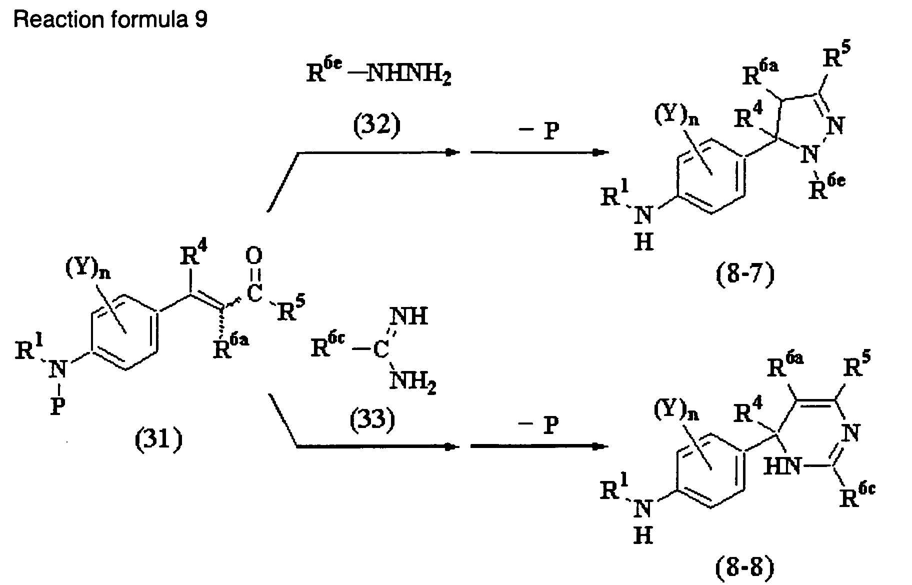

The compound represented by Formula (31) [wherein Y, R¹, R⁴, R⁵, R^{6a}, n and P have the same meanings as defined above.] and a hydrazine compound which is the conventionally known compound represented by Formula (32) [wherein R^{6e} have the same meanings as defined above.] are reacted according to the conventionally known methods described in literatures, for example, the methods as described in Journal of the Chemical Society [J. Chem. Soc.] 1964, p. 6072, etc. to for a pyrazoline ring, and then, subjecting to deprotection by the generally employed method for the respective protective groups, so that the compound represented by Formula (8-7) [wherein Y, R¹, R⁴, R⁵, R^{6a}, R^{6e} and n have the same meanings as defined above.] wherein G is G-11 and R^{6b} is a hydrogen atom in Formula (8) can be obtained.

Also, the compound represented by Formula (31) is reacted with an amidine compound which is the conventionally known compound represented by Formula (33) [wherein R^{6c} have the same meanings as defined above.] according to the conventionally known methods described in literatures, for example, the methods as described in Journal of Heterocyclic Chemistry [J. Heterocyclic Chem.] 1989, vol. 26, p. 251, etc. to form a 3,4-dihydropyrimidine ring, and then, subjecting to deprotection by the generally employed method for the respective protective groups, so that the compound represented by Formula (8-8) [wherein Y, R¹, R⁴, R⁵, R^{6a}, R^{6c} and n have the same meanings as defined above.] wherein G is G-53 and R^{6e} is a hydrogen atom in Formula (8) can be obtained.

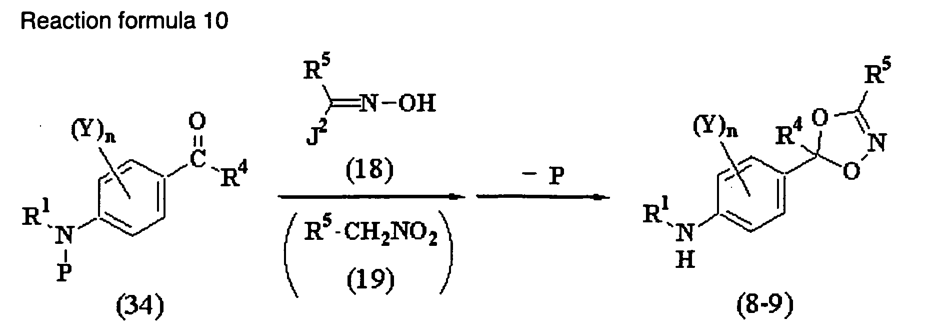

The compound represented by Formula (34) [wherein Y, R¹, R⁴, n and P have the same meanings as defined above.] and the compound represented by Formula (18) [wherein R⁵ and J² have the same meanings as defined above.] or the compound represented by Formula (19) [wherein R⁵ have the same meanings as defined above.] are reacted under the similar conditions as in Preparation method J to form a dioxazoline ring, and then, subjecting to deprotection by the generally employed method for the respective protective groups, so that the compound represented by Formula (8-9) [wherein Y, R¹, R⁴, R⁵ and n have the same meanings as defined above.] wherein G is G-23 in Formula (8) can be obtained.

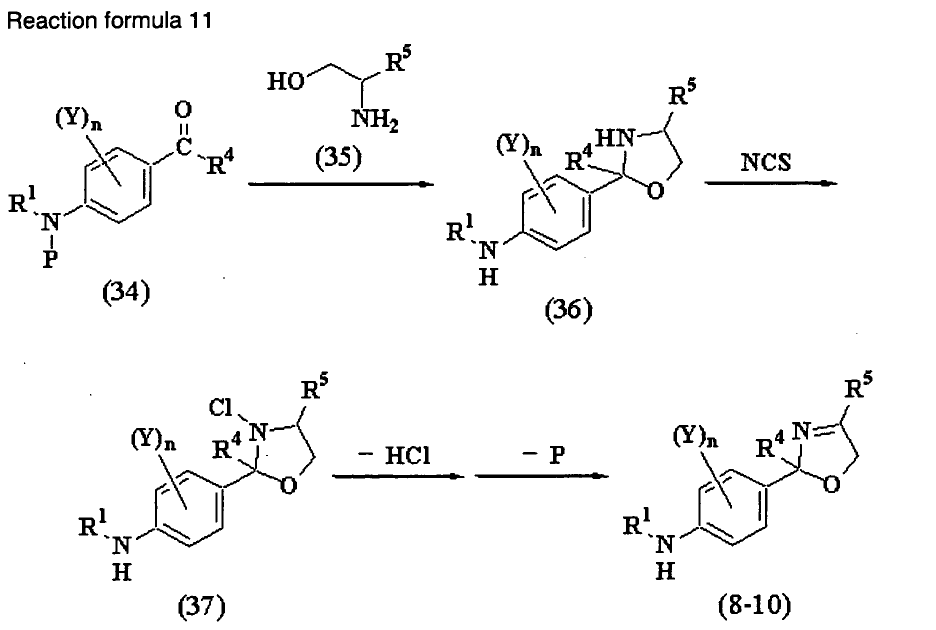

The compound represented by Formula (34) [wherein Y, R¹, R⁴, n and P have the same meanings as defined above.] and the compound represented by Formula (35) [wherein R⁵ have the same meanings as defined above.] are reacted according to the conventionally known methods described in literatures, for example, the methods as described in Chemistry Letters [Chem. Lett.] 1998, p. 119, etc., and the resulting compound represented by Formula (36) [wherein Y, R¹, R⁴, R⁵, n and P have the same meanings as defined above.] is treated according to the conventionally known methods described in literatures, for example, the methods as described in Tetrahedron Letters [Tetrahedron Lett.] 1992, vol. 33, p. 7751, etc. to prepare the N-halogenated compound represented by Formula (37) [wherein Y, R¹, R⁴, R⁵, n and P have the same meanings as defined above.] to form a 1,5-dihydroxazole ring, and then, subjecting to deprotection by the generally employed method for the respective protective groups, so that the compound represented by Formula (8-10) [wherein Y, R¹, R⁴, R⁵ and n have the same meanings as defined above.] G is G-15 in Formula (8) can be obtained.

The compound represented by Formula (35) herein used is a conventionally known compound, and some of which can be obtained as a commercially available product. Also, those other than the above can be easily synthesized from the corresponding conventionally known amino acids according to the conventionally known methods described in literatures, for example, the methods as described in Chemical and Pharmaceutical Bulletin [Chem. Pharm. Bull.] 1965, vol. 13, p. 999, etc.

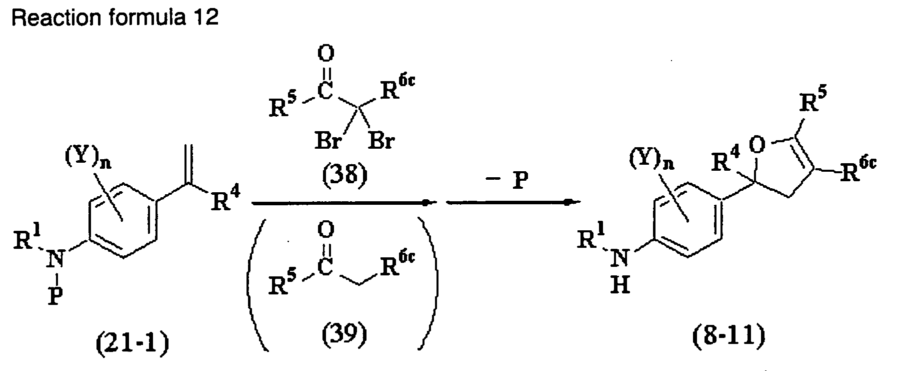

The compound represented by Formula (21-1) [wherein Y, R¹, R⁴, n and P have the same meanings as defined above.] wherein R^{6a} and R^{6b} are hydrogen atoms in Formula (21) and the compound represented by Formula (38) [wherein R⁵ and R^{6c} have the same meanings as defined above.] are reacted according to the conventionally known methods described in literatures, for example, the methods as described in Journal of the Chemical Society Chemical Communications [J. Chem. Soc. Chem. Commun.] 1987, p. 919, etc. to form a 4,5-dihydrofuran ring, and then, subjecting to deprotection by the generally employed method for the respective protective groups, so that the compound represented by Formula (8-11) [wherein Y, R¹, R⁴, R⁵, R^{6c} and n have the same meanings as defined above.] wherein G is G-1 in Formula (8) can be obtained.

Or else, the compound represented by Formula (21-1) and the conventionally known compound represented by Formula (39) [wherein R⁵ has the same meaning as defined above, and R^{6c} represents an electron attractive group such as an alkoxycarbonyl group, cyano group, etc.] are reacted according to the conventionally known methods described in literatures, for example, the methods as described in Tetrahedron Letters [Tetrahedron Lett.] 1996, vol. 37, p. 4949, etc., the compound wherein R^{6c} is an electron attractive group in Formula (8-11) can be obtained.

Some of the compound represented by Formula (38) herein used are conventionally known compounds, and those other than the above can be easily synthesized according to the conventionally known methods described in literatures, for example, the methods as described in Chemistry Letters [Chem. Lett.] 1981, p. 1135, The Journal of Organic Chemistry [J. Org. Chem.] 1992, vol. 57, p. 4555, etc.

Moreover, the compound represented by Formula (39) is also conventionally known compound, some of which can be obtained as a commercially available product. Also, those other than the above can be easily synthesized according to the conventionally known methods described in literatures, for example, the methods as described in Journal of Heterocyclic Chemistry [J. Heterocyclic Chem.] 1984, vol. 21, p. 1849, Journal of Medicinal Chemistry [J. Med. Chem.] 1979, vol. 22, p. 1385, etc.

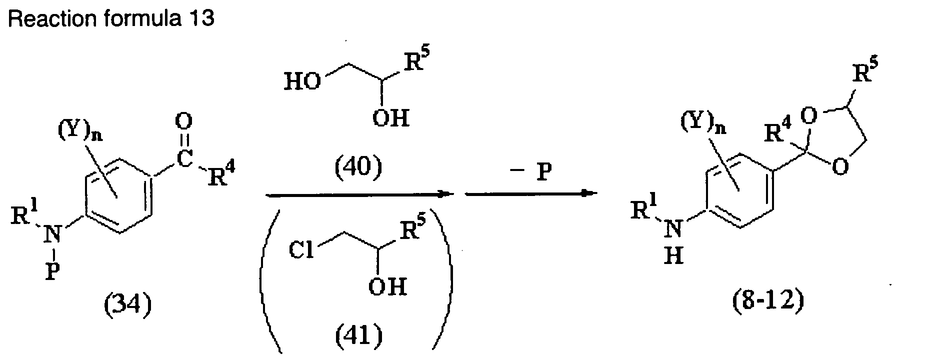

The compound represented by Formula (34) [wherein Y, R¹, R⁴, n and P have the same meanings as defined above.] and the compound represented by Formula (40) [wherein R⁵ have the same meanings as defined above.] or the compound represented by Formula (41) [wherein R⁵ have the same meanings as defined above.] are reacted according to the conventionally known methods described in literatures, for example, the methods as described in Synthesis, 1983, p. 203, Japanese Unexamined Patent Publication (JP 06/092957 Publication), Journal of Fluorine Chemistry [J. Fluorine Chem.] 1989, vol. 44, p. 377, etc. to form a dioxolan ring, and then, subjecting to deprotection by the generally employed method for the respective protective groups, so that the compound represented by Formula (8-12) [wherein Y, R¹, R⁴, R⁵ and n have the same meanings as defined above.] wherein G is G-55 in Formula (8) can be obtained.

The compound represented by Formula (40) herein used is a conventionally known compound, and some of which can be obtained as a commercially available product. Also, those other than the above can be easily synthesized according to the conventionally known methods described in literatures, for example, the methods as described in Journal of the American Chemical Society [J. Am. Chem. Soc.] 1966, vol. 88, p. 2194, Tetrahedron Letters [Tetrahedron Lett.] 1995, vol. 36, p. 3277 and 2000, vol. 41, p. 7847, etc.

Also, some of the compound represented by Formula (41) are conventionally known compounds, and those other than the above can be easily synthesized according to the conventionally known methods described in literatures, for example, the methods as described in Journal of the Chemical Society Perkin Transactions 1 [J. Chem. Soc., Perkin Trans. 1] 1983, p. 3020, etc.

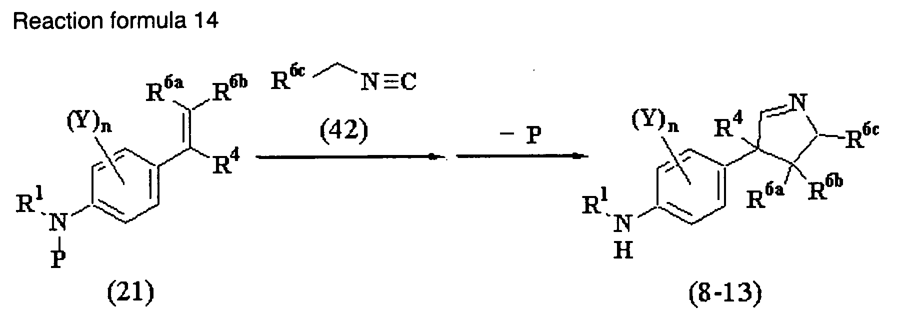

The compound represented by Formula (21) [wherein Y, R¹, R⁴, R^{6a}, R^{6b}, n and P have the same meanings as defined above.] and the compound represented by Formula (42) [wherein R^{6c} represents phenyl group which may be substituted by (Z²)ₚ₁.] are reacted according to the conventionally known methods described in literatures, for example, the methods as described in The Journal of Organic Chemistry [J. Org. Chem.] 1971, vol. 36, p. 3316, etc. to form a 3,4-dihydro-2H-pyrrole ring, and then, subjecting to deprotection by the generally employed method for the respective protective groups, so that the compound represented by Formula (8-13) [wherein Y, R¹, R⁴, R^{6a}, R^{6b} and n have the same meanings as defined above, and R^{6c} represents phenyl group which may be substituted by (Z²)ₚ₁.] wherein G is G-6 and R⁵ is a hydrogen atom in Formula (8) can be obtained.

The compound represented by Formula (42) herein used is a conventionally known compound, and some of which can be obtained as a commercially available product. Also, those other than the above can be easily synthesized according to the conventionally known methods described in literatures, for example, the methods as described in Angewante Chemie [Angew. Chem.] 1965, vol. 77, p. 492, Chemische Berichte [Chem. Ber.] 1960, vol. 93, p. 239, etc.

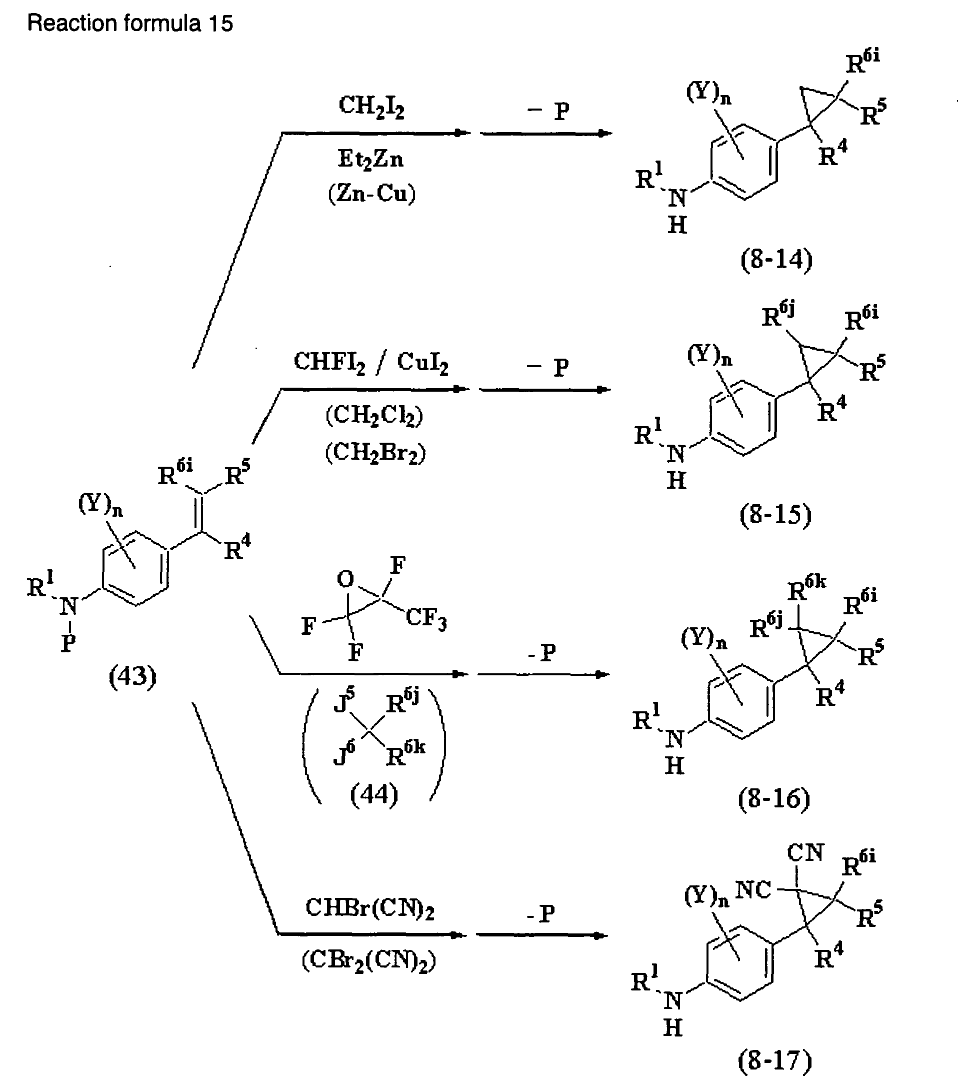

The compound represented by Formula (43) [wherein Y, R¹, R⁴, R⁵, R⁶ⁱ, n and P have the same meanings as defined above.] and diiodomethane are reacted according to the conventionally known methods described in literatures, for example, the methods as described in Chemical and Pharmaceutical Bulletin [Chem. Pharm. Bull.] 1992, vol. 40, p. 3189, etc. to form a cyclopropane ring, and then, subjecting to deprotection by the generally employed method for the respective protective groups, so that the compound represented by Formula (8-14) [wherein Y, R¹, R⁴, R⁵, R⁶ⁱ and n have the same meanings as defined above.] wherein G is G-71 and R^{6j} and R^{6k} are hydrogen atoms in Formula (8) can be obtained.

Also, the compound represented by Formula (43) is reacted with fluorodiiodomethane according to the conventionally known methods described in literatures, for example, the methods as described in Tetrahedron 1979, vol. 35, p. 1919, Tetrahedron Letters [Tetrahedron Lett.] 1975, p. 1820, etc., or reacted with dichloromethane or dibromomethane according to the conventionally known methods described in literatures, for example, the methods as described in The Journal of Organic Chemistry [J. Org. Chem.] 1994, vol. 59, p. 4087, Tetrahedron 1970, vol. 26, p. 4203, Tetrahedron Letters [Tetrahedron Lett.] 1987, vol. 28, p. 5075, etc., so that the compound represented by Formula (8-15) [wherein Y, R¹, R⁴, R⁵, R⁶ⁱ and n have the same meanings as defined above, and R^{6j} represents fluorine atom, chlorine atom or bromine atom.] wherein G is G-71 and R^{6k} is a hydrogen atom in Formula (8) can be obtained.

Or else, the compound represented by Formula (43) and hexafluoro-1,2--epoxypropane or the conventionally known compound represented by Formula (44) [wherein R^{6j} and R^{6k} each independently represent fluorine atom, chlorine atom or bromine atom, J⁵ represents a hydrogen atom, chlorine atom or bromine atom, etc., and J⁶ represents chlorine atom, bromine atom, carboxyl group or alkoxycarbonyl group, etc.] are reacted according to the conventionally known methods described in literatures, for example, the methods as described in Journal of the Chemical Society Perkin Transactions 1 [J. Chem. Soc., Perkin Trans. 1] 1995, p. 653, The Journal of Organic Chemistry [J. Org. Chem.] 1964, vol. 29, p. 1886, 1986, vol. 51, p. 974 and 1990, vol. 55, p. 5420, Tetrahedron 1989, vol. 45, p. 2925, 1990, vol. 46, p. 1911 and 1999, vol. 55, p. 10325, Tetrahedron Letters [Tetrahedron Lett.] 1971, p. 3869, 1988, vol. 29, p. 6749 and 1998, vol. 39, p. 3013, etc., so that the compound represented by Formula (8-16) [wherein Y, R¹, R⁴, R⁵, R⁶ⁱ and n have the same meanings as defined above, R^{6j} and R^{6k} each independently represent fluorine atom, chlorine atom or bromine atom.] wherein G is G-71 in Formula (8) can be obtained.

Moreover, the compound represented by Formula (43) and the conventionally known malononitrile derivative are reacted according to the conventionally known methods described in literatures, for example, the methods as described in Journal of the Chemical Society Chemical Communications [J. Chem. Soc. Chem. Commun.] 1989, p. 1286, Tetrahedron Letters [Tetrahedron Lett.] 1966, p. 1415, etc., so that the compound represented by Formula (8-17) [wherein Y, R¹, R⁴, R⁵, R⁶ⁱ and n have the same meanings as defined above.] wherein G is G-71, and R^{6j} and R^{6k} are cyano groups in Formula (8) can be obtained.

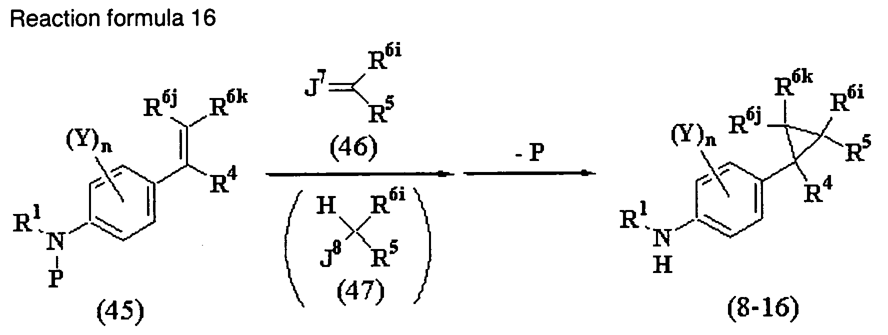

The compound represented by Formula (45) [wherein Y, R¹, R⁴, R^{6j}, R^{6k}, n and P have the same meanings as defined above.] and the compound represented by Formula (46) [wherein R⁵ represents phenyl group which may be substituted by (Z²)ₚ₁, R⁶ⁱ represents a hydrogen atom or an alkoxycarbonyl group, etc., J⁷ represents =N⁺=N⁻ group or =NNHSO₂Ph-4-CH₃ group, etc.] are reacted by an addition reaction using a general rhodium catalyst as described in the literatures, for example, the methods as described in Journal of the American Chemical Society[J. Am. Chem. Soc.] 2001, vol. 123, p. 2695, International Unexamined Patent Publication (WO 98/3470 Publication), etc., or the compound represented by Formula (45) and the compound represented by Formula (47) [wherein R⁵ represents phenyl group which may be substituted by (Z²)ₚ₁, R⁶ⁱ represents fluorine atom, chlorine atom or bromine atom, and J⁸ represents chlorine atom or bromine atom.] are reacted according to the conventionally known methods described in literatures, for example, the methods as described in The Journal of Organic Chemistry [J. Org. Chem.] 1962, vol. 27, 2685, Tetrahedron Letters [Tetrahedron Lett.] 1965, p. 3445 and 1968, p. 11962, etc. to form a cyclopropane ring, and then, subjecting to deprotection by the generally employed method for the respective protective groups, so that the compound represented by Formula (8-16) [wherein Y, R¹, R⁴, R^{6j}, R^{6k} and n have the same meanings as defined above, R⁵ represents phenyl group which may be substituted by (Z²)ₚ₁, and R⁶ⁱ represents a hydrogen atom, fluorine atom, chlorine atom, bromine atom or alkoxycarbonyl group.] wherein G is G-71 in Formula (8) can be obtained.

The compound represented by Formula (46) and the compound represented by Formula (47) herein used can be synthesized according to the conventionally known methods described above.

The compound represented by Formula (9) which is a starting compound for preparing the compound of the present invention in Preparation method D can be synthesized by, for example, the methods shown by the following Reaction formula 17 or Reaction formula 18.

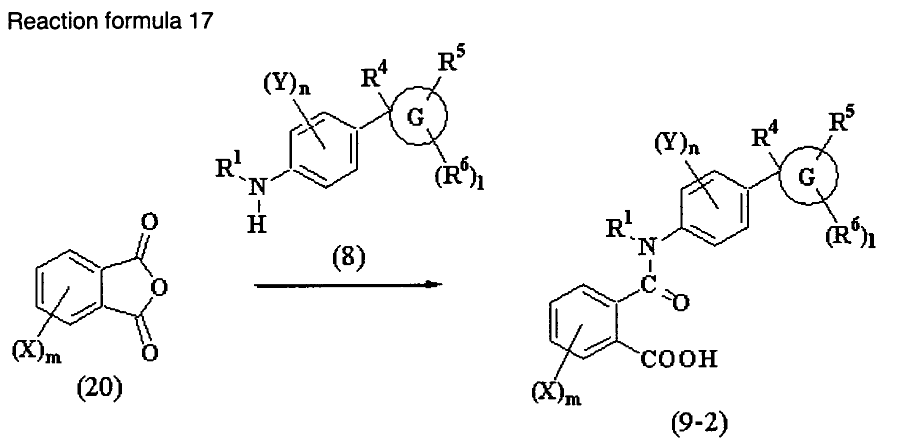

The compound represented by Formula (20) [wherein X and m have the same meanings as defined above.] and the compound represented by Formula (8) [wherein G, Y, R¹, R⁴, R⁵, R⁶, l and n have the same meanings as defined above.] are reacted under the similar conditions as in Preparation method A, so that the compound represented by Formula (9-2) [wherein G, X, Y, R¹, R⁴, R⁵, R⁶, l, m and n have the same meanings as defined above.] wherein W¹ is an oxygen atom in Formula (9) can be obtained.

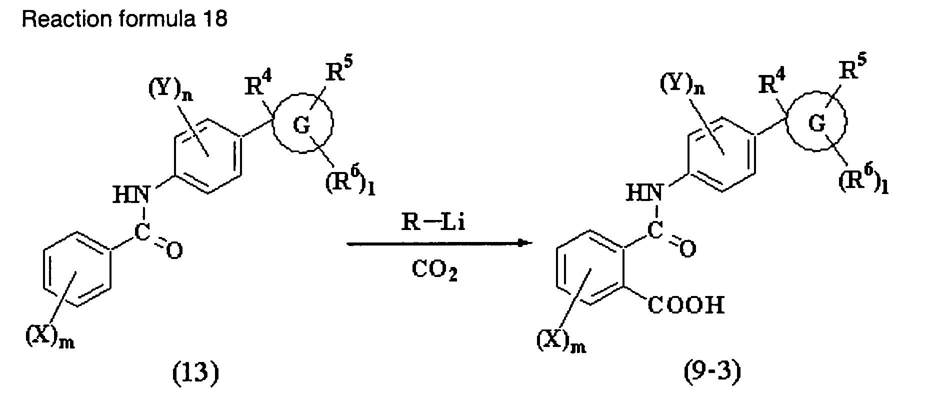

The compound represented by Formula (13) [wherein G, X, Y, R⁴, R⁵, R⁶, l, m and n have the same meanings as defined above.] is subjected to selective lithiation according to the conventionally known methods described in literatures, for example, the methods as described in Chemical Reviews [Chem. Rev.] 1990, vol. 9, p. 879, etc., and then, reacting with a carbon dioxide gas, so that the compound represented by Formula (9-3) [wherein G, X, Y, R⁴, R⁵, R⁶, I, m and n have the same meanings as defined above.] wherein W¹ is an oxygen atom, and R¹ is a hydrogen atom in Formula (9) can be obtained. Incidentally, R-Li represents an alkyl lithium reagent such as butyl lithium, etc.

The compound represented by Formula (10) which is a starting compound for preparing the compound of the present invention in Preparation method E can be synthesized by, for example, the method shown by the following Reaction formula 19 or Reaction formula 20, and the like.

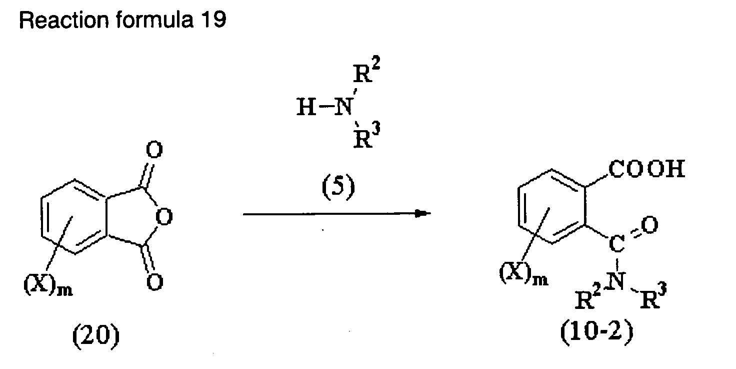

The compound represented by Formula (20) [wherein X and m have the same meanings as defined above.] and the compound represented by Formula (5) [wherein R² and R³ have the same meanings as defined above.] are reacted under the similar conditions as in Preparation method A, so that the compound represented by Formula (10-2) [wherein X, R², R³ and m have the same meanings as defined above.] wherein W² is an oxygen atom in Formula (10) can be obtained.

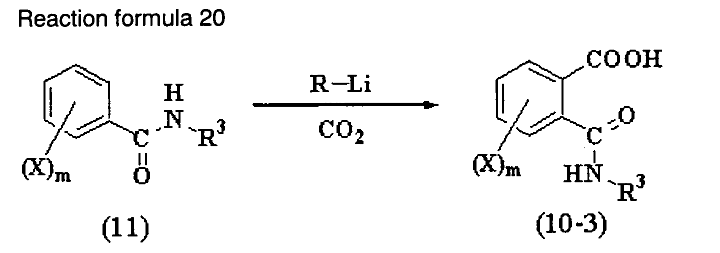

The compound represented by Formula (11) [wherein X, R³ and m have the same meanings as defined above.] is reacted under the similar conditions as in Reaction formula 12, so that the compound represented by Formula (10-3) [wherein X, R³ and m have the same meanings as defined above.] wherein W² is an oxygen atom, and R² is a hydrogen atom in Formula (10) can be obtained. Incidentally, R-Li represents an alkyl lithium reagent such as butyl lithium, etc.

Some of the compound represented by Formula (11) which is a starting compound for preparing the compound of the present invention in Preparation method F are the conventionally known compounds, and some of which can be obtained as a commercially available product. Also, those other than the above can be easily synthesized according to the conventionally known methods described in literatures, for example, the methods as described in Bulletin of the Chemical Society of Japan [Bull. Chem. Soc. Jpn.] 1985, vol. 58, p. 3291, The Journal of Organic Chemistry [J. Org. Chem.] 1991, vol. 56, p. 2395, Tetrahedron Letters [Tetrahedron Lett.] 1994, vol. 35, p. 2113, International Unexamined Patent Publication (WO 98/23581 publication), etc.

The compound represented by Formula (12) used in Preparation method F can be synthesized as mentioned below.

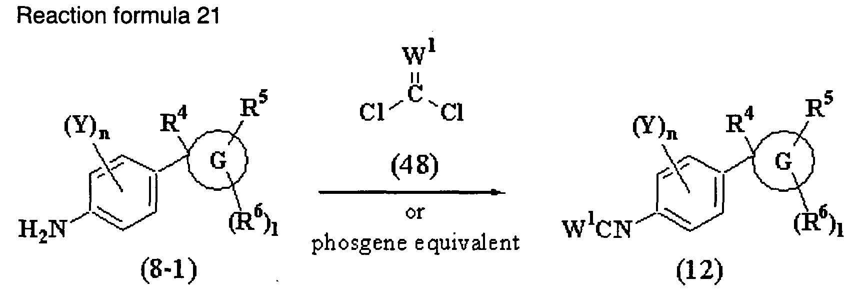

That is, the compound represented by Formula (8-1) [wherein G, Y, R⁴, R⁵, R⁶, I and n have the same meanings as defined above.] wherein R¹ is a hydrogen atom in Formula (8) and the commercially available phosgene, thiophosgene or their equivalents represented by Formula (48) [wherein W¹ represents oxygen atom or sulfur atom.] are reacted according to the conventionally known methods described in literatures, for example, the methods as described in Angewante Chemie International Edition in English [Angew. Chem. Int. Ed. Engl.] 1987, vol. 26, p. 894 and 1995, vol. 34, p. 2497, The Journal of Organic Chemistry [J. Org. Chem.] 1976, vol. 41, p. 2070, Synthesis 1988, p. 990, Tetrahedron Letters [Tetrahedron Lett.] 1997, vol. 38, p. 919, etc., so that the compound represented by Formula (12) [wherein G, W¹, Y, R⁴, R⁵, R⁶, I and n have the same meanings as defined above.] can be easily synthesized.

In Preparation method G, the compound represented by Formula (13) which is a starting compound for preparing the compound of the present invention can be synthesized as mentioned below.

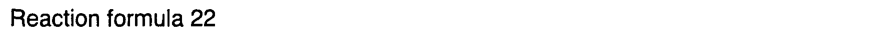

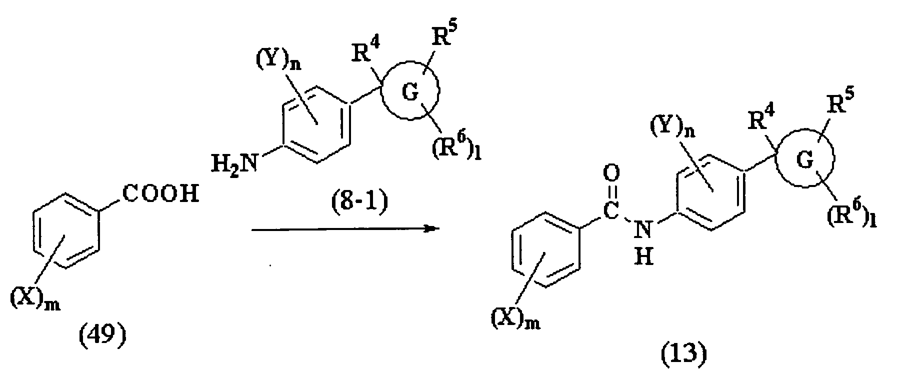

That is, the compound represented by Formula (49) [wherein X and m have the same meanings as defined above.] and the compound represented by Formula (8-1) [wherein G, Y, R⁴, R⁵, R⁶, l and n have the same meanings as defined above.] wherein R¹ is a hydrogen atom in Formula (8) are reacted under the similar conditions as in Preparation method D, or the compound represented by Formula (49) is converted into a corresponding carboxylic acid chloride using the conventionally known methods (e.g., a chlorinating agent such as thionyl chloride, phosphorus pentachloride or oxalyl chloride, etc.), and then, reacting the resulting compound with the compound represented by Formula (8-1), so that the compound represented by Formula (13) [wherein G, X, Y, R⁴, R⁵, R⁶, l, m and n have the same meanings as defined above.] can be easily synthesized.

The compound represented by Formula (49) herein used is a conventionally known compound, and some of which can be obtained as a commercially available product.

Some of the compound represented by the compound represented by Formula (14) used in Preparation method G are the conventionally known compound, and some of which can be obtained as a commercially available product. Also, those other than the above can be easily synthesized according to the general synthetic methods described in, for example, the methods as described in The Journal of Organic Chemistry [J. Org. Chem.] 1996, vol. 61, pp. 3883, 3929 and 6575, Tetrahedron Letters [Tetrahedron Lett.] 1999, vol. 40, pp. 363 and 6121, etc.

Some of the compound represented by Formula (15) used in Preparation method H and some of the compound represented by Formula (16) used in Preparation method I are the conventionally known compounds, and some of which can be obtained as a commercially available product. Also, those other than the above can be easily synthesized according to the general synthetic methods described in, for example, the methods as described in Chemistry Letters [Chem. Lett.] 1976, p. 373, Journal of the American Chemical Society [J. Am. Chem. Soc.] 1964, vol. 86, p. 4383, The Journal of Organic Chemistry [J. Org. Chem.] 1976, vol. 41, p. 4028 and 1978, vol. 43, p. 3244, Organic Synthesis [Org. Synth.] 1988, Collective Volume 6, p. 101, Tetrahedron Letters [Tetrahedron Lett.] 1972, p. 4339, British Patent (GB 2,161,802 Publication), European Patent (EP 0,051,273 Publication), etc.

In Preparation method J, the compound represented by Formula (17) which is a starting compound for preparing the compound of the present invention can be synthesized by, after deprotecting the compound represented by Formula (21) according to the generally employed method, using Preparation method A to Preparation method G in the same manner as in the compounds of the present invention.

Some of the compound represented by Formula (18) and some of the compound represented by Formula (19) used in Preparation method J are the conventionally known compounds, and some of which can be obtained as a commercially available product. Also, those other than the above can be easily synthesized according to the conventionally known methods described in literatures, for example, the methods as described in the compound represented by Formula (18) can be easily synthesized according to the method described in Chemistry Letters [Chem. Lett.] 1986, p. 183, The Journal of Organic Chemistry [J. Org. Chem.] 1980, vol. 45, p. 3916 and 1990, vol. 55, p. 4585, Tetrahedron Letters [Tetrahedron Lett.] 1993, vol. 34, p. 2831 and 1996, vol. 37, p. 5699, etc., and the compound represented by Formula (19) can be easily synthesized according to the method described in Journal of Fluorine Chemistry [J. Fluorine Chem.] 1991, vol. 55, p. 149, The Journal of Organic Chemistry [J. Org. Chem.] 1979, vol. 44, p. 3872, etc.

Some of the compound represented by Formula (20) are the conventionally known compounds, and some of which can be obtained as a commercially available product. Also, those other than the above can be synthesized, for example, as mentioned below.

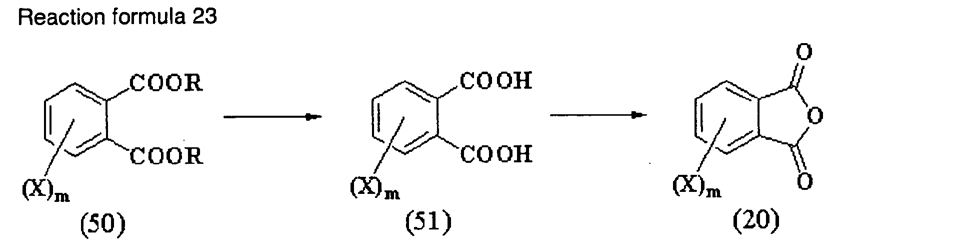

That is, the compound represented by Formula (50) [wherein X and m have the same meanings as defined above, and R represents a lower alkyl group such as methyl group, ethyl group, etc.] is subjected to a general hydrolysis reaction described in literatures, for example, the methods as described in Angewante Chemie [Angew. Chem.] 1951, vol. 63, p. 329, Journal of the American Chemical Society [J. Am. Chem. Soc.] 1929, vol. 51, p. 1865, etc. to form a phthalic acid derivative represented by Formula (51) [wherein X and m have the same meanings as defined above.], and then, subjecting to a general dehydration and cyclization reaction under the conditions described in, for example, the methods as described in The Journal of Organic Chemistry [J. Org. Chem.] 1987, vol. 52, p. 129, etc., so that the compound represented by Formula (20) [wherein X and m have the same meanings as defined above.] can be obtained.

The compound represented by Formula (50) herein used is a conventionally known compound, and some of which can be obtained as a commercially available product.

The compound represented by Formula (21) can be synthesized, for example, as mentioned below.

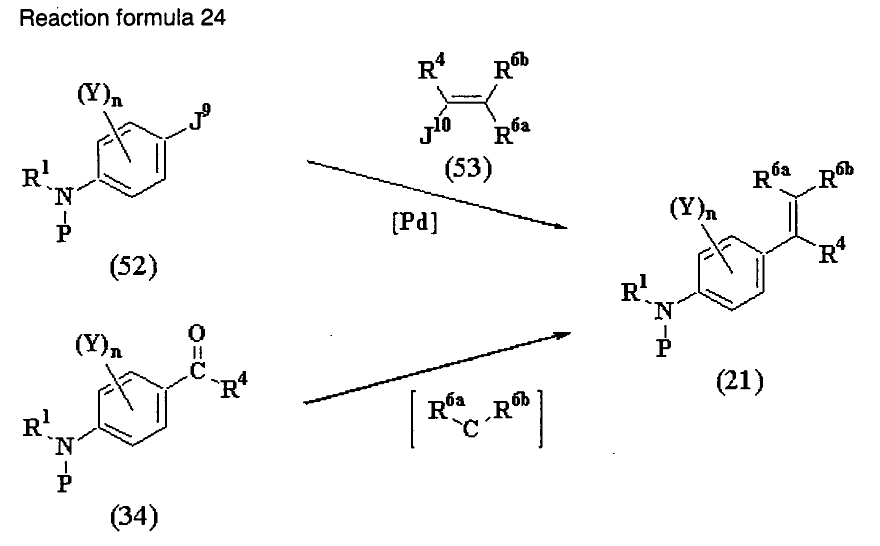

That is, the compound represented by Formula (52) [wherein Y, R¹, n and P have the same meanings as defined above, J⁹ represents an eliminating group such as bromine atom, iodine atom, a halosulfonate group (e.g., fluorosulfonyloxy group), and a C₁ to C₄ haloalkylsulfonate group (e.g., trifluoromethanesulfonyloxy group).] and the compound represented by Formula (53) [wherein R⁴, R^{6a} and R^{6b} have the same meanings as defined above, J¹⁰ represents a halogen atom such as bromine atom, iodine atom, etc.] are reacted in the cross-coupling reaction using a general transition metal catalyst such as palladium according to the methods described in literatures, for example, the methods as described in The Journal of Organic Chemistry [J. Org. Chem.] 1991, vol. 56, p. 7336, Tetrahedron Letters [Tetrahedron Lett.] 2001, vol. 42, p. 4083, etc., so that the compound represented by Formula (21) [wherein Y, R¹, R⁴, R^{6a}, R^{6b}, n and P have the same meanings as defined above.] can be obtained.

The compound represented by Formula (53) herein used is a conventionally known compound, and some of which can be obtained as a commercially available product. Also, those other than the above can be easily synthesized according to the conventionally known methods described in literatures, for example, the methods as described in Journal of the American Chemical Society [J. Am. Chem. Soc.] 1971, vol. 93, p. 1925, Tetrahedron Letters [Tetrahedron Lett.] 1990, vol. 31, p. 1919, etc.

Or else, the compound represented by Formula (34) [wherein Y, R¹, R⁴, n and P have the same meanings as defined above.] is subjected to olefination reaction of the carbonyl group according to the conventionally known methods described in literatures, for example, the methods as described in The Journal of Organic Chemistry [J. Org. Chem.] 1986, vol. 51, p. 5252 and 1994, vol. 59, p. 2898, Synthesis, 1991, p. 29, Tetrahedron Letters [Tetrahedron Lett.] 1985, vol. 26, p. 5579, etc., so that the compound represented by Formula (21) can be obtained.

The compound represented by Formula (22) can be synthesized, for example, as mentioned below.

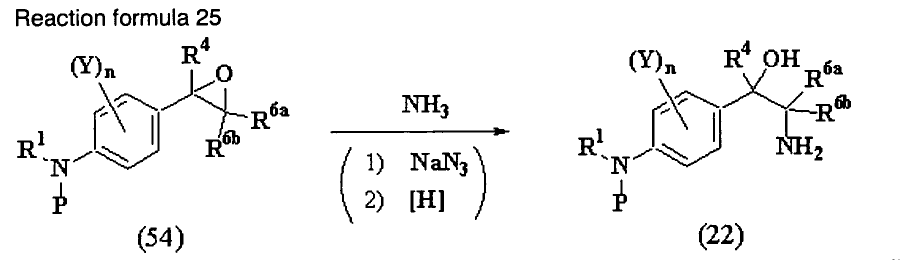

That is, the compound represented by Formula (54) [wherein Y, R¹, R⁴, R^{6a}, R^{6b}, n and P have the same meanings as defined above.] is reacted with ammonia according to the conventionally known methods described in literatures, for example, the methods as described in Journal of the American Chemical Society [J. Am. Chem. Soc.] 1951, vol. 73, p. 96 and 1965, vol. 87, p. 1358, etc., or reacting with an azide according to the method described in, for example, the methods as described in Heterocycles, 1986, vol. 24, p. 931, etc., and then, reducing the resulting compound, so that the compound represented by Formula (22) [wherein Y, R¹, R⁴, R^{6a}, R^{6b}, n and P have the same meanings as defined above.] can be obtained.

The compound represented by Formula (27) can be synthesized, for example, as follows.

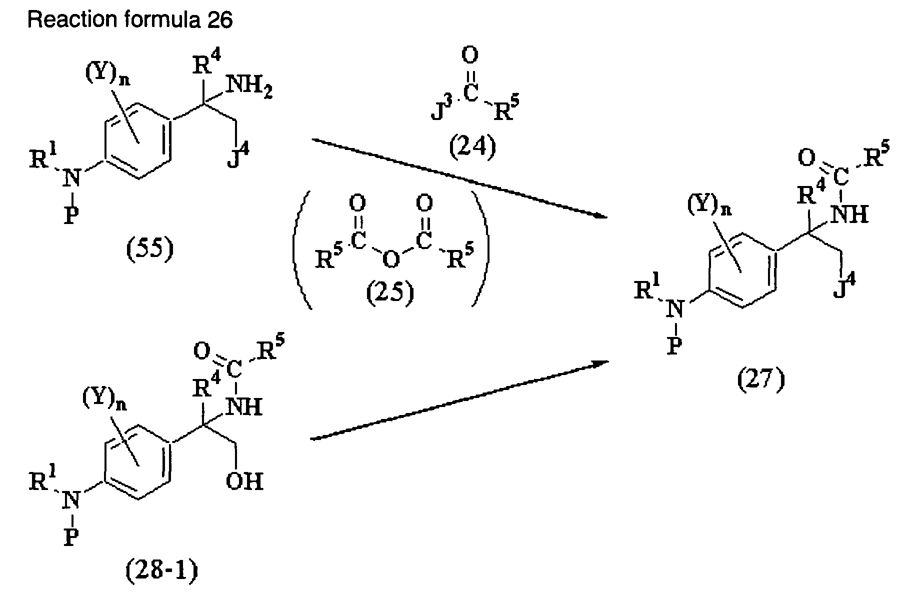

That is, the compound represented by Formula (55) [wherein Y, R¹, R⁴, J⁴, n and P have the same meanings as defined above.] and the conventionally known compound represented by Formula (24) [wherein R⁵ and J³ have the same meanings as defined above.] or the conventionally known compound represented by Formula (25) [wherein R⁵ have the same meanings as defined above.] are reacted under the similar conditions as in Reaction formula 5, so that the compound represented by Formula (27) [wherein Y, R¹, R⁴, R⁵, J⁴, n and P have the same meanings as defined above.] can be obtained.

Or else, the compound represented by Formula (28-1) [wherein Y, R¹, R⁴, R⁵, n and P have the same meanings as defined above.] wherein R^{6a} and R^{6b} are hydrogen atoms in Formula (28) is reacted according to the conventionally known methods described in literatures, for example, the methods as described in The Journal of Organic Chemistry [J. Org. Chem.] 2000, vol. 65, p. 9223, Tetrahedron Letters [Tetrahedron Lett.] 1995, vol. 36, p. 1223, German Patent Publication (DE 19528778 Publication), etc., so that the compound represented by Formula (27) can be obtained.

The compound represented by Formula (28) can be synthesized, for example, as follows.

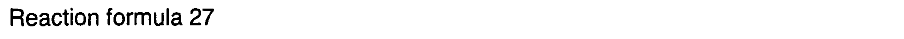

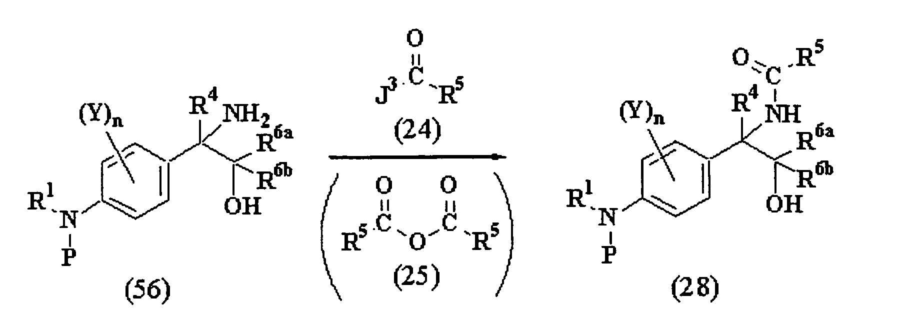

That is, the compound represented by Formula (56) [wherein Y, R¹, R⁴, n and P have the same meanings as defined above.] and the conventionally known compound represented by Formula (24) [wherein R⁵ and J⁶ have the same meanings as defined above.] or the conventionally known compound represented by Formula (25) [wherein R⁵ have the same meanings as defined above.] are reacted according to the conventionally known methods described in literatures, for example, the methods as described in Angewante Chemie International Edition in English [Angew. Chem. Int. Ed. Engl.] 1996, vol. 35, p. 2487, Journal of the American Chemical Society [J. Am. Chem. Soc.] 1953, vol. 75, p. 5896, Synthetic Communications [Synth. Commun.] 1998, vol. 28, p. 3317, etc., so that the compound represented by Formula (28) [wherein Y, R¹, R⁴, R⁵, n and P have the same meanings as defined above.] can be obtained.

The compound represented by Formula (29) can be synthesized, for example, as follows.

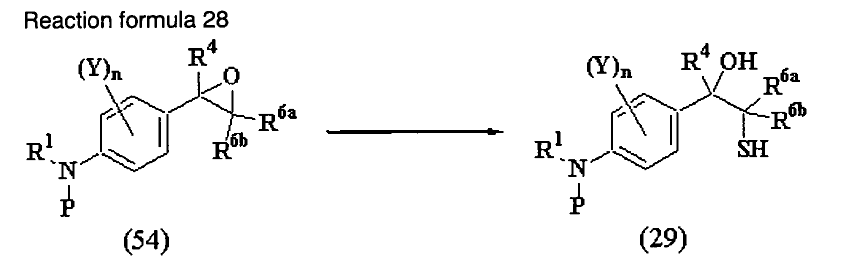

That is, the compound represented by Formula (54) [wherein Y, R¹, R⁴, R^{6a}, R^{6b}, n and P have the same meanings as defined above.] is reacted according to the conventionally known methods described in literatures, for example, the methods as described in Chemical and Pharmaceutical Bulletin [Chem. Pharm. Bull.] 1993, vol. 41, p. 1035, Journal of the Chemical Society [J. Chem. Soc.] 1951, p. 778 and 1960, p. 2653, etc., so that the compound represented by Formula (29) [wherein Y, R¹, R⁴, R^{6a}, R^{6b}, n and P have the same meanings as defined above.] can be obtained.

The compound represented by Formula (31) can be synthesized, for example, as follows.

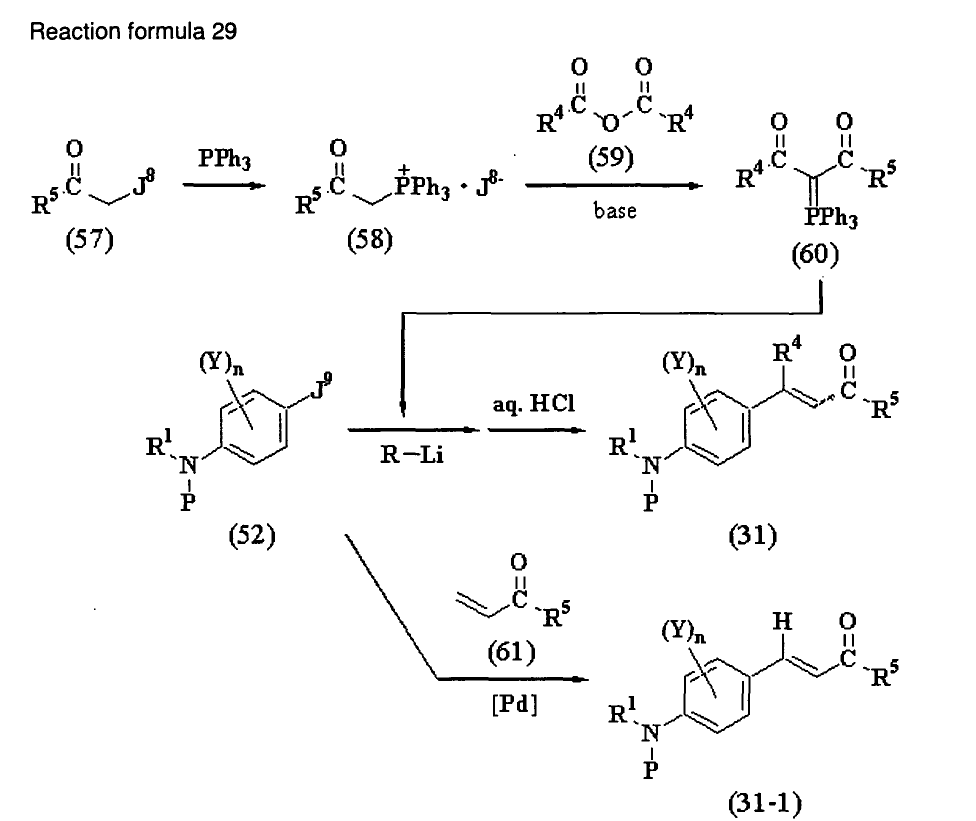

That is, the conventionally known compound represented by Formula (57) [wherein R⁵ and J⁸ have the same meanings as defined above.] is reacted according to the conventionally known methods described in literatures, for example, the methods as described in The Journal of Organic Chemistry [J. Org. Chem.] 1998, vol. 53, p. 5558, etc. to form a phosphonium salt represented by Formula (58) [wherein R⁵ and J⁸ have the same meanings as defined above.], then, reacting the resulting compound with the conventionally known compound represented by Formula (59) [wherein R⁴ have the same meanings as defined above.], and the resulting compound represented by Formula (60) [wherein R⁴ and R⁵ have the same meanings as defined above.] is reacted with a lithium salt of the compound represented by Formula (52) [wherein Y, R¹, n and P have the same meanings as defined above, and J⁹ represents bromine atom or iodine atom.] obtained by a halogen-metal exchange reaction according to the conventionally known methods described in literatures, for example, the methods as described in Journal of the Chemical Society Perkin Transactions 1 [J. Chem. Soc., Perkin Trans. 1] 1996, p. 2531, etc., so that the compound represented by Formula (31) [wherein Y, R¹, R⁴, R⁵, n and P have the same meanings as defined above.] can be obtained.

Also, the compound represented by Formula (52) and the conventionally known compound represented by Formula (61) [wherein R⁵ have the same meanings as defined above.] are reacted according to the conventionally known methods described in literatures, for example, the methods as described in Synthesis 1989, p. 869, etc., so that the compound represented by Formula (31-1) [wherein Y, R¹, R⁵, n and P have the same meanings as defined above.] wherein R⁴ is a hydrogen atom in Formula (31) can be obtained.

The compound represented by Formula (34) can be synthesized, for example, as follows.

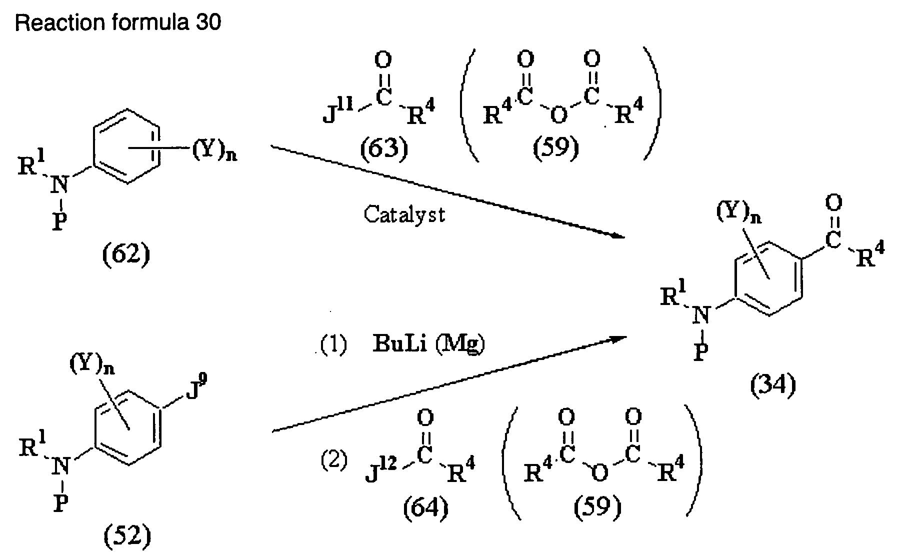

That is, the conventionally known compound represented by Formula (62) [wherein Y, R¹, n and P have the same meanings as defined above.] and the conventionally known compound represented by Formula (63) [wherein R⁴ has the same meanings as defined above, J¹¹ represents an eliminating group such as a halogen atom, trifluoromethanesulfonyloxy group, 2-pyridyloxy group, etc.] or the conventionally known compound represented by Formula (59) [wherein R⁴ have the same meanings as defined above.] are reacted by a general acylation reaction of the aromatic ring according to the methods described in literatures, for example, the methods as described in Chemistry Letters [Chem. Lett.] 1990, p. 783, The Journal of Organic Chemistry [J. Org. Chem.] 1991, vol. 56, p. 1963, etc., so that the compound represented by Formula (34) [wherein Y, R¹, R⁴, n and P have the same meanings as defined above.] can be obtained.

Or else, the compound represented by Formula (52) [wherein Y, R¹, n and P have the same meanings as defined above, J⁹ represents bromine atom or iodine atom.] is reacted according to the general methods described in literatures, for example, subjecting to lithiation, and then, reacting with the conventionally known compound represented by Formula (64) [wherein R⁴ has the same meanings as defined above, J¹² represents a halogen atom, a hydroxyl group, a metal salt (e.g., -OLi, -ONa), a C₁ to C₄ alkoxy group (e.g., methoxy group, ethoxy group), a di(C₁ to C₄ alkyl)amino group (e.g., diethylamino group), a C₁ to C₄ alkoxy(C₁ to C₄ alkyl)amino group (e.g., O,N-dimethyl-hydroxyamino group) or a cyclic amino group (e.g., piperidin-1-yl group, morpholin-4-yl group, 4-methylpiperazin-1-yl group).] or reacting with the conventionally known compound represented by Formula (59) according to the methods as described in Journal of the American Chemical Society[J. Am. Chem. Soc.] 1955, vol. 77, p. 3657, Tetrahedron Letters [Tetrahedron Lett.] 1980, vol. 21, p. 2129 and 1991, vol. 32, p. 2003, U.S. Patent Publication (US Patent No. 5,514,816), etc., or forming a Grignard reagent, and then, reacting with the compound represented by Formula (64) or the compound represented by Formula (59) according to the methods as described in Heterocycles, 1987, vol. 25, p. 221, Synthetic Communications [Synth. Commun.] 1985, vol. 15, p. 1291 and 1990, vol. 20, p. 1469, German Patent Publication (DE 19727042 Publication), etc., so that the compound represented by Formula (34) can be obtained.

The compound represented by Formula (43) and the compound represented by Formula (45) can be synthesized in the same manner as in the compound represented by Formula (21).

The compound represented by Formula (52) can be synthesized, for example, as follows.

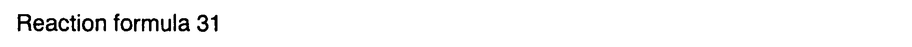

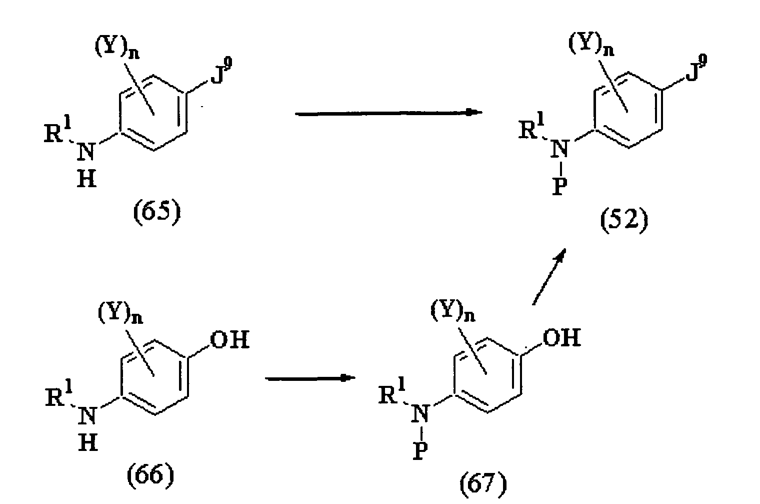

That is, an amino group of the conventionally known substituted aniline represented by Formula (65) [wherein Y, R¹, n and J⁹ have the same meanings as defined above.] is protected according to the general method described in literatures, for example, the methods as described in Journal of Medicinal Chemistry [J. Med. Chem.] 1996, vol. 39, p. 673 and 1997, vol. 40, p. 3542, etc., so that the compound represented by Formula (52) [wherein Y, R¹, J⁹, n and P have the same meanings as defined above.] can be obtained.

Also, an amino group of the conventionally known substituted aminophenol represented by Formula (66) [wherein Y, R¹ and n have the same meanings as defined above.] is protected under the same conditions to prepare the compound represented by Formula (67) [wherein Y, R¹, n and P have the same meanings as defined above.], and then, reacting the resulting compound with anhydrous trifluoromethanesulfonic acid or anhydrous fluorosulfonic acid to carry out a general sulfonylation reaction according to the methods described in literatures, for example, the methods as described in The Journal of Organic Chemistry [J. Org. Chem.] 1991, vol. 56, p. 3493 and 1994, vol. 59, p. 1216, etc., so that the compound represented by Formula (52) where J⁹ is a trifluoromethanesulfonyloxy group or a fluorosulfonyloxy group can be obtained.

The compound represented by Formula (54) can be synthesized, for example, as follows.

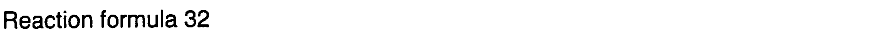

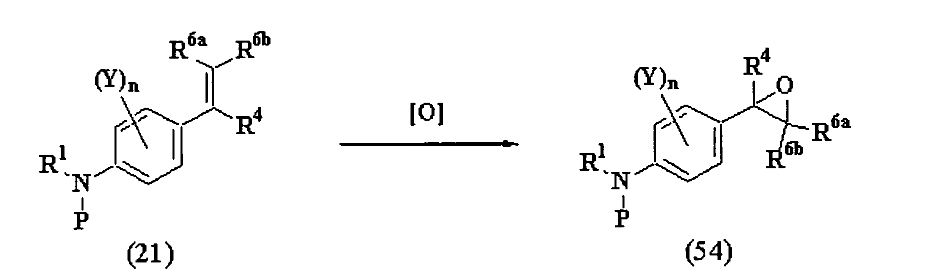

That is, the compound represented by Formula (21) [wherein Y, R¹, R⁴, R^{6a}, R^{6b}, n and P have the same meanings as defined above.] is oxidized according to the conventionally known methods described in literatures, for example, the methods as described in Angewante Chemie International Edition in English [Angew. Chem. Int. Ed. Engl.] 2000, vol. 39, p. 3473, Journal of the American Chemical Society [J. Am. Chem. Soc.] 2001, vol. 123, p. 2933, Synthesis 1999, p. 249, etc., so that the compound represented by Formula (54) [wherein Y, R¹, R⁴, R^{6a}, R^{6b}, n and P have the same meanings as defined above.] can be obtained.

The compound represented by Formula (55) can be synthesized, for example, as follows.

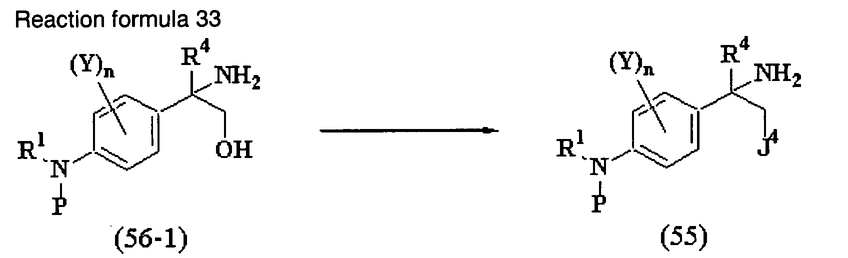

That is, the compound represented by Formula (56-1) [wherein Y, R¹, R⁴, n and P have the same meanings as defined above.] wherein R^{6a} and R^{6b} are hydrogen atoms in Formula (56) is reacted according to the conventionally known methods described in literatures, for example, the methods as described in The Journal of Organic Chemistry [J. Org. Chem.] 1959, vol. 24, p. 527, Synthesis 1987, p. 479, Tetrahedron, 1993, vol. 49, p. 1993, etc., so that the compound represented by Formula (55) [wherein Y, R¹, R⁴, J⁴, n and P have the same meanings as defined above.] can be obtained.

The compound represented by Formula (56) can be synthesized, for example, as follows.

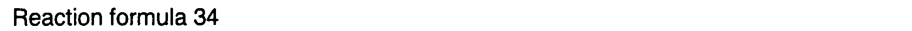

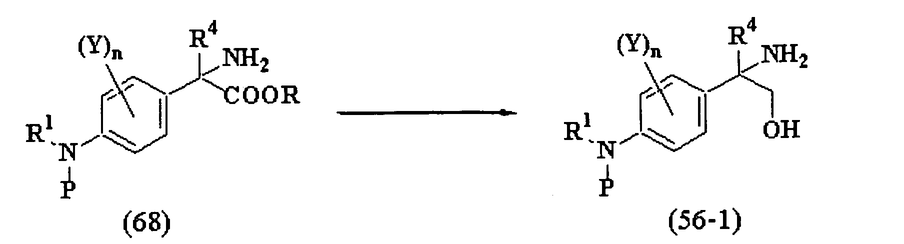

That is, the compound represented by Formula (68) [wherein Y, R¹, R⁴, n and P have the same meanings as defined above, R represents a lower alkyl group such as a hydrogen atom or methyl, ethyl, etc.] is reduced according to the conventionally known methods described in literatures, for example, the methods as described in Chemical and Pharmaceutical Bulletin [Chem. Pharm. Bull.] 1965, vol. 13, p. 999, The Journal of Organic Chemistry [J. Org. Chem.] 1993, vol. 58, p. 3568, German Patent Publication (DE 19528778 Publication), etc., so that the compound represented by Formula (56-1) [wherein Y, R¹, R⁴, n and P have the same meanings as defined above.] wherein R^{6a} and R^{6b} are hydrogen atoms in Formula (56) can be obtained.

The compound represented by Formula (68) can be synthesized, for example, as follows.

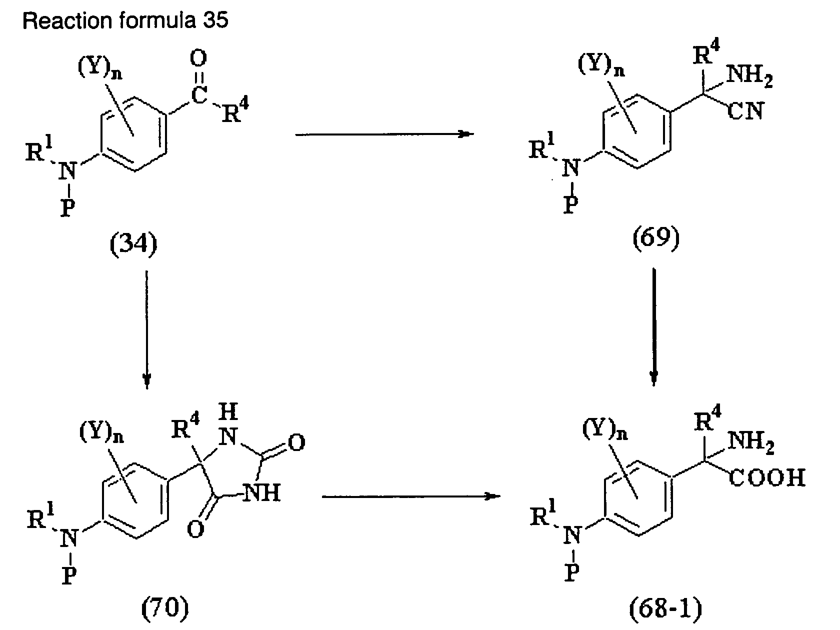

That is, the compound represented by Formula (34) [wherein Y, R¹, R⁴, n and P have the same meanings as defined above.] is converted into the aminonitrile represented by Formula (69) [wherein Y, R¹, R⁴, n and P have the same meanings as defined above.] according to the general method of an amino acid synthesis described in literatures, for example, the methods as described in Journal of the American Chemical Society [J. Am. Chem. Soc.] 1960, vol. 82, p. 698, Tetrahedron Letters [Tetrahedron Lett.] 1996, vol. 37, p. 8655, etc., and then, subjecting to hydrolysis according to the methods, for example, as described in Journal of the Chemical Society [J. Chem. Soc.] 1962, p. 3979, etc., or converting into the imidazolidinedione represented by Formula (70) [wherein Y, R¹, R⁴, n and P have the same meanings as defined above.] according to the methods as described in Journal of the American Chemical Society [J. Am. Chem. Soc.] 1943, vol. 65, p. 324, Tetrahedron: Asymmetry, 1997, vol. 8, p. 2913, German Patent Publication (DE 19528778 Publication), etc., and then, subjecting to hydrolysis, so that the compound represented by Formula (68-1) [wherein Y, R¹, R⁴, n and P have the same meanings as defined above.] wherein R is a hydrogen atom in Formula (68) can be obtained.

In these respective reactions, after completion of the reaction, by carrying out the usual post-treatments, respective preparation intermediates which are starting compounds in Preparation method A to Preparation method J can be obtained.

Also, the respective preparation intermediates prepared by these methods may be used as such in the reaction of the next Step without isolation and purification.

As the compounds included in the present invention, there may be specifically mentioned, for example, the compounds shown in Table 2 to Table 7. Provided that the compounds of Table 2 to Table 7 are only for exemplification purpose, and the present invention is not limited only these.

Incidentally, the description Et in the tables means an ethyl group, hereinafter the same, n-Pr and Pr-n are a normal propyl group, i-Pr and Pr-i are an isopropyl group, c-Pr and Pr-c are a cyclopropyl group, n-Bu and Bu-n are a normal butyl group, s-Bu and Bu-s are a secondary butyl group, i-Bu and Bu-i are an isobutyl group, t-Bu and Bu-t are a tertiary butyl group, c-Bu and Bu-c are a cyclobutyl group, n-Pen and Pen-n are a normal pentyl group, c-Pen and Pen-c are a cyclopentyl group, n-Hex and Hex-n are a normal hexyl group, c-Hex and Hex-c are a cyclohexyl group, Oct is an octyl group, Ph is a phenyl group, 1-Naph is a 1-naphthyl group, and 2-Naph is a 2-naphthyl group, respectively,

In the tables, T-1 to T-24 each represent the following structures,

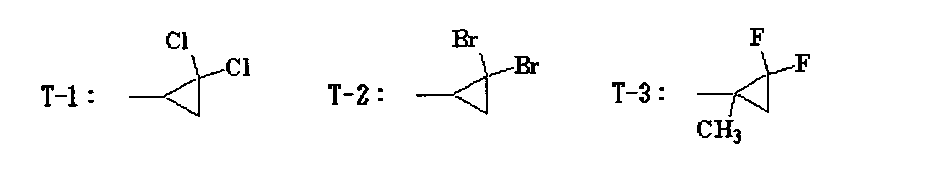

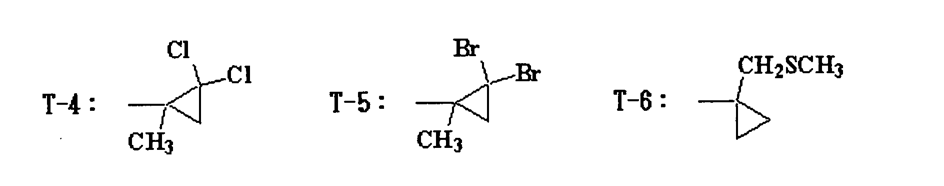

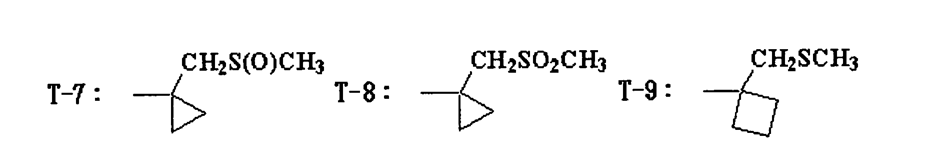

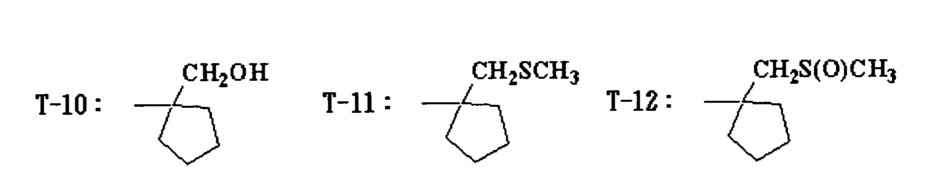

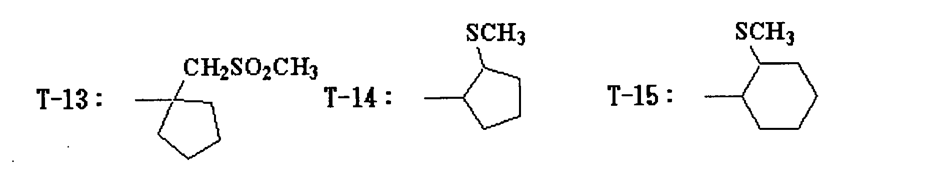

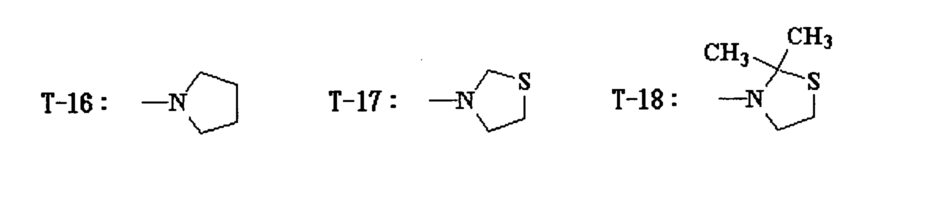

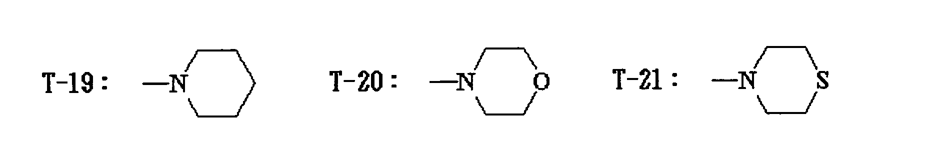

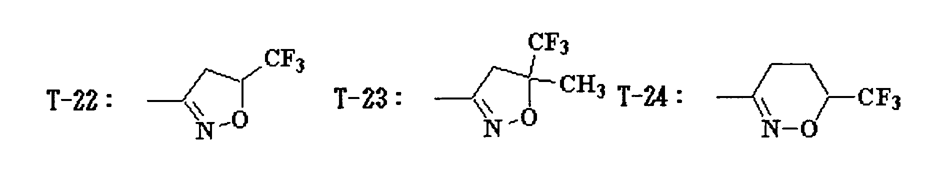

In the tables, aromatic heterocyclic rings represented by L-1 a to L-55a mean the structures shown below, respectively,

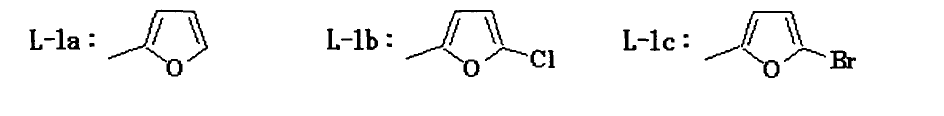

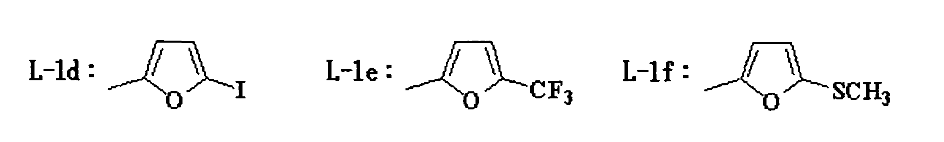

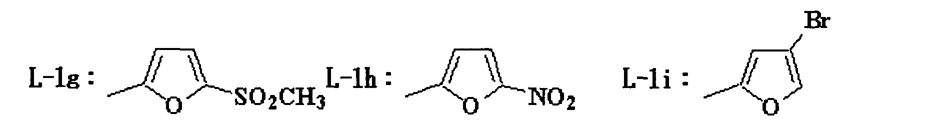

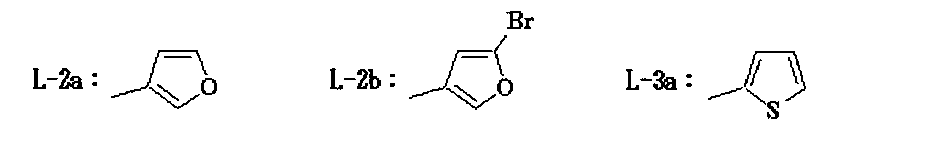

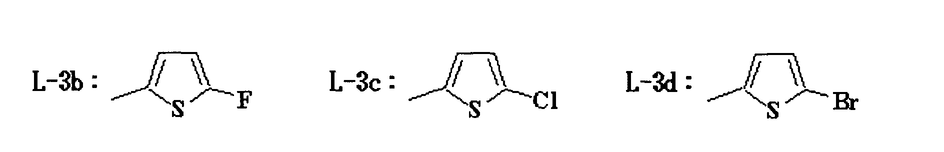

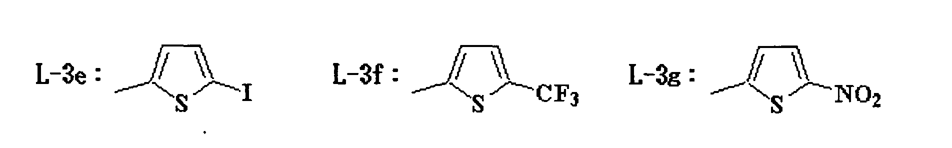

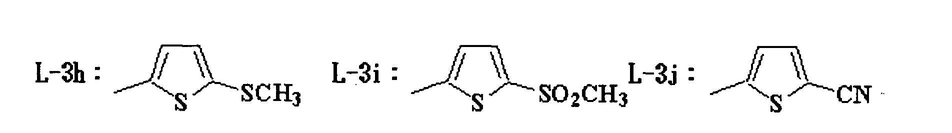

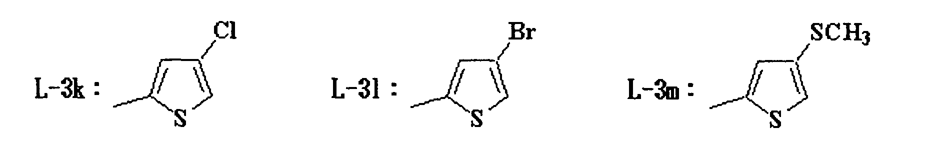

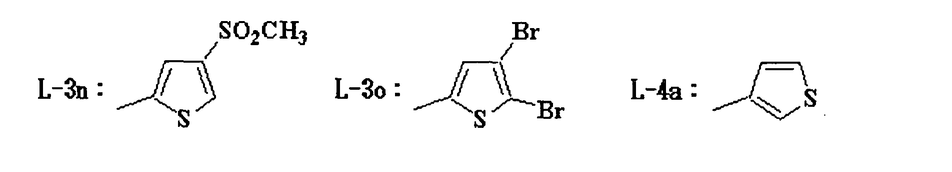

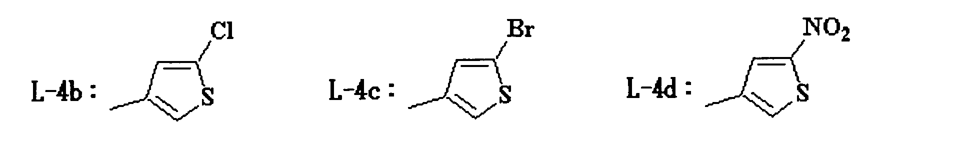

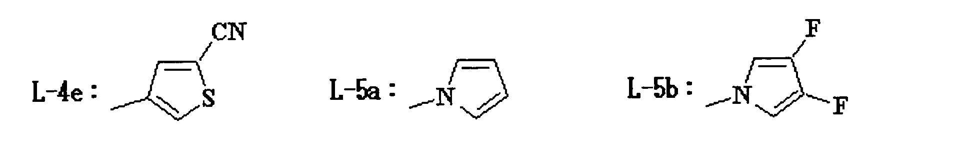

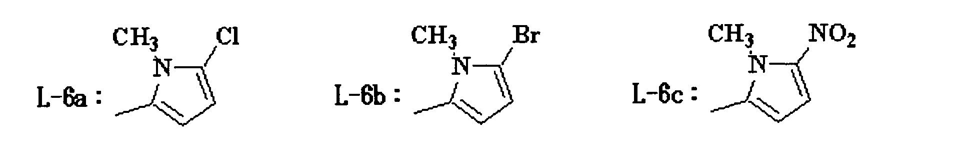

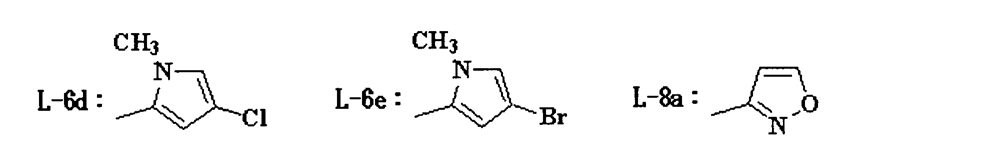

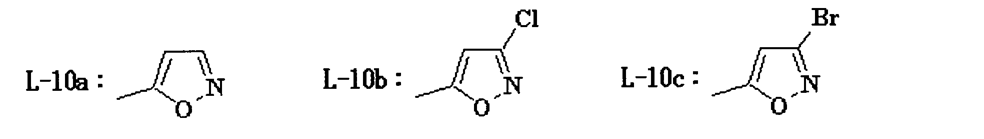

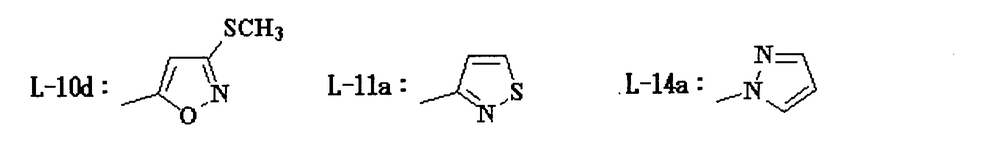

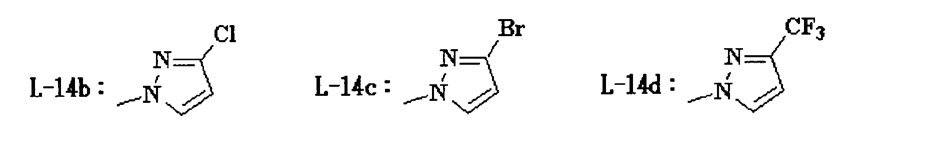

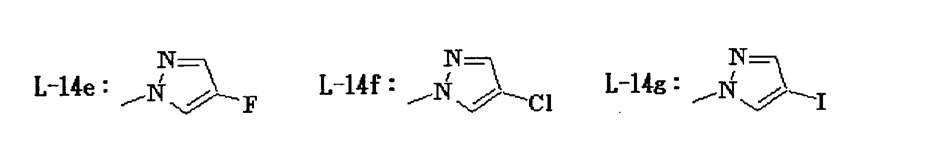

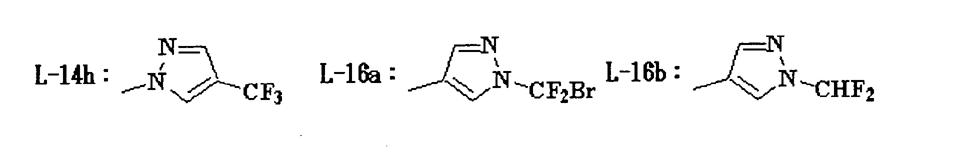

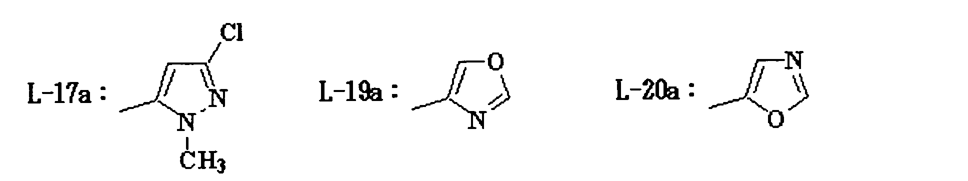

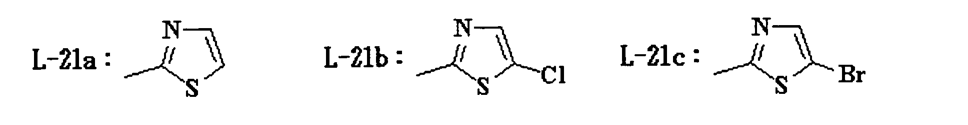

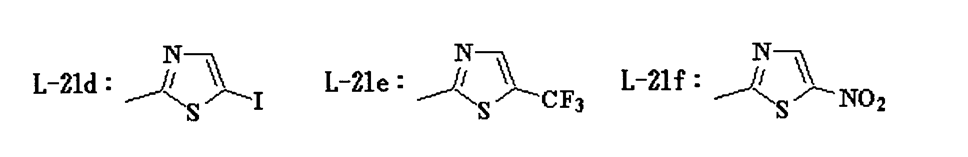

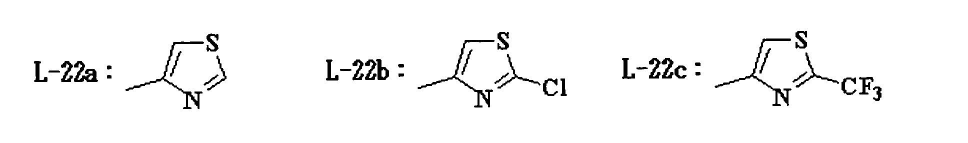

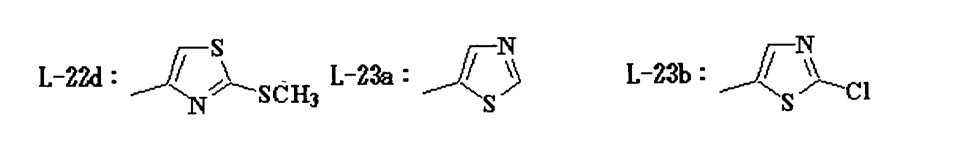

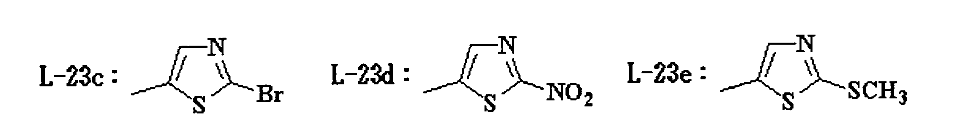

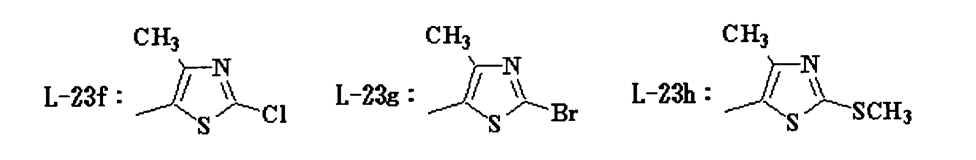

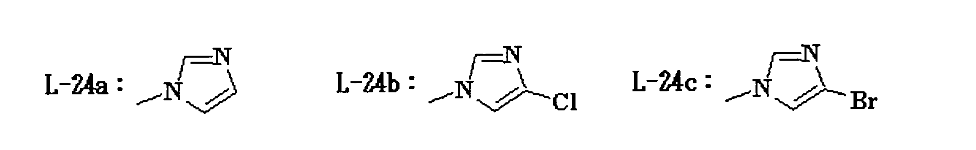

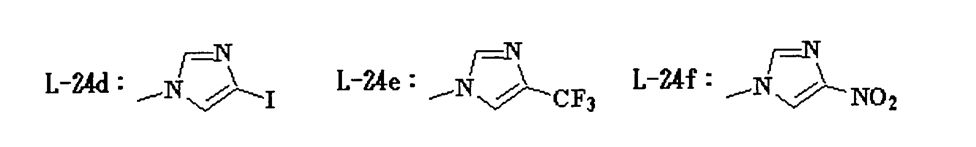

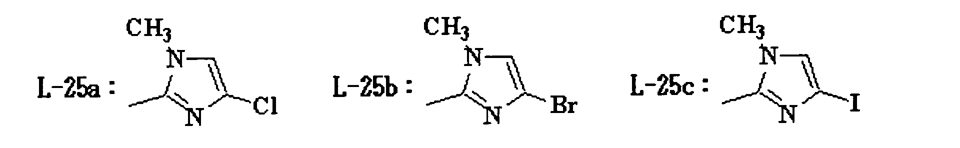

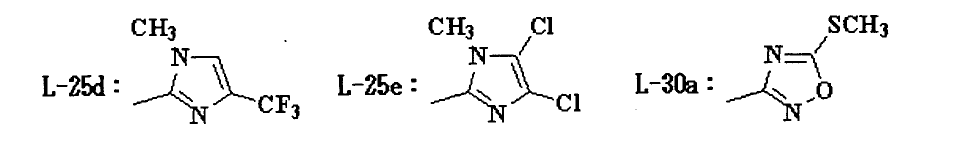

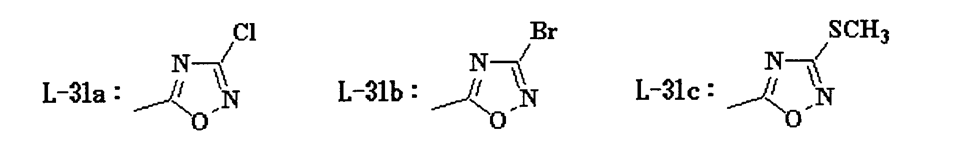

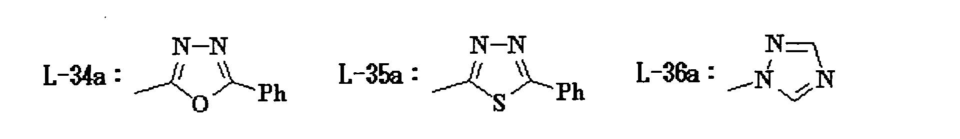

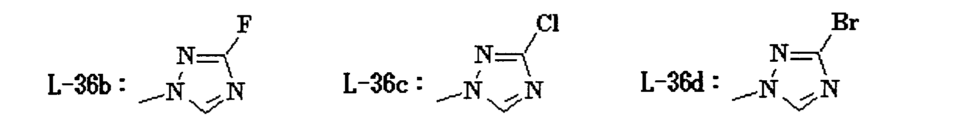

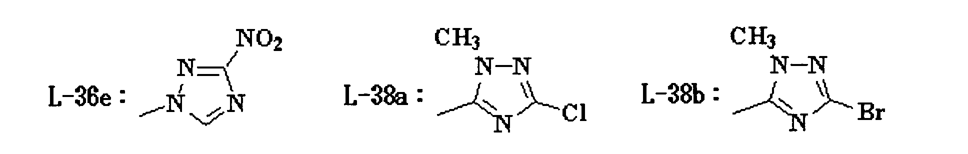

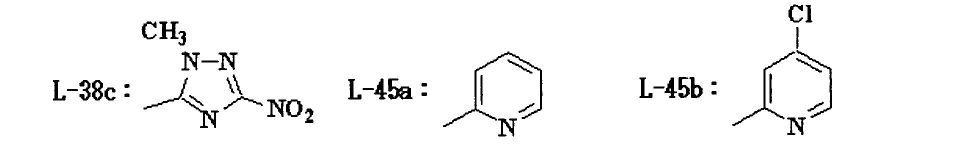

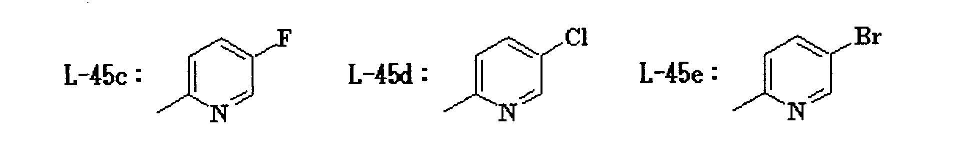

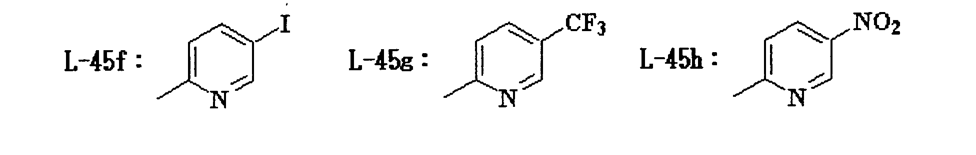

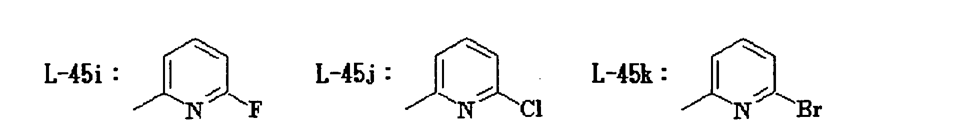

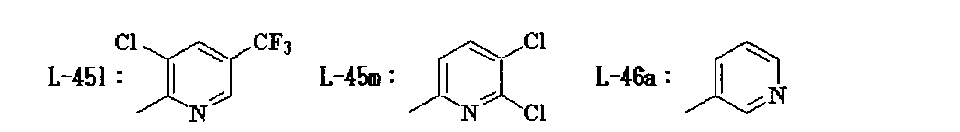

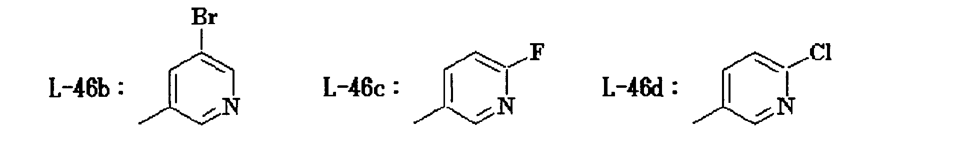

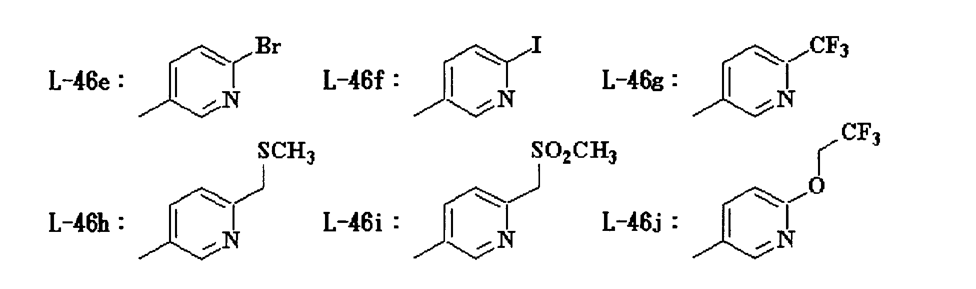

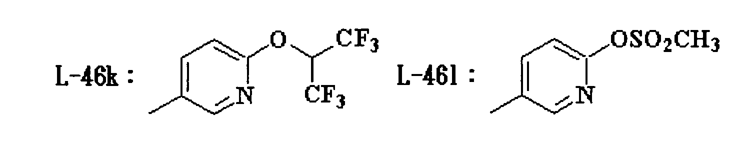

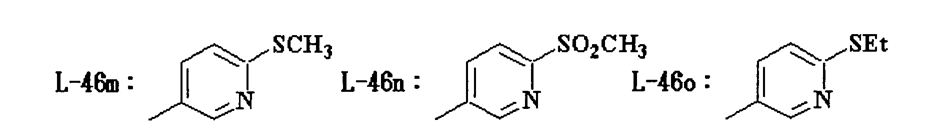

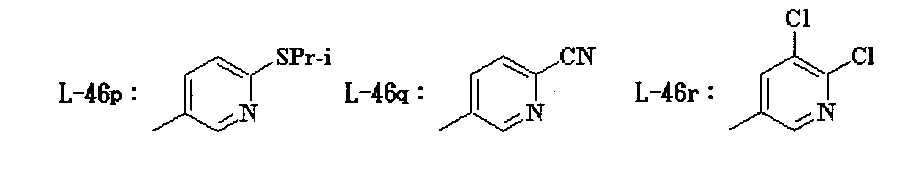

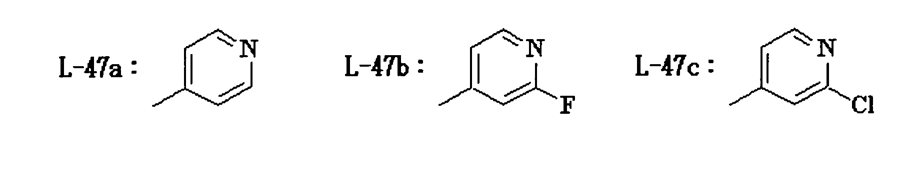

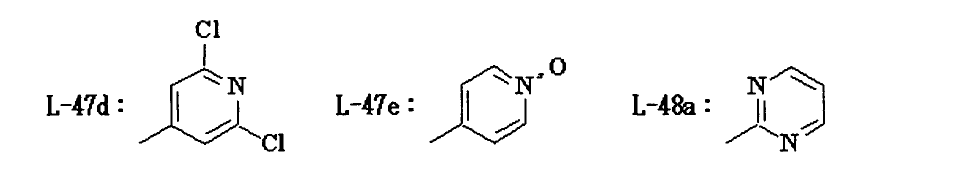

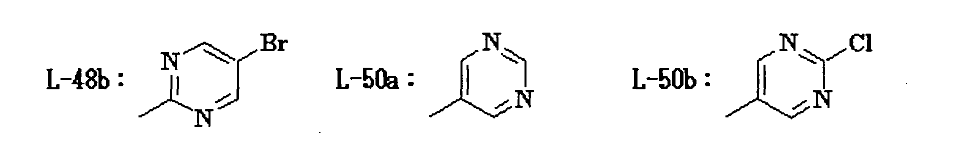

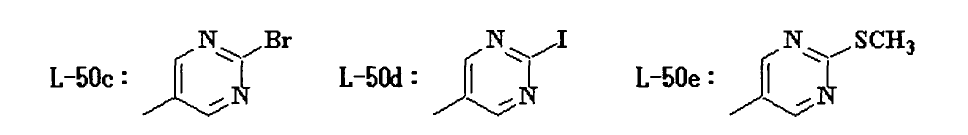

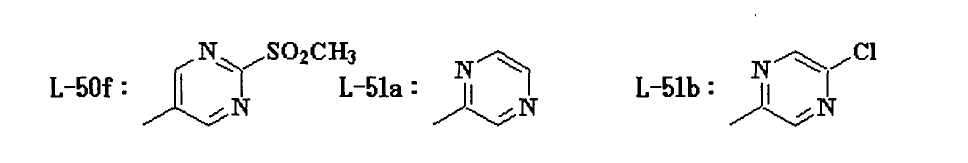

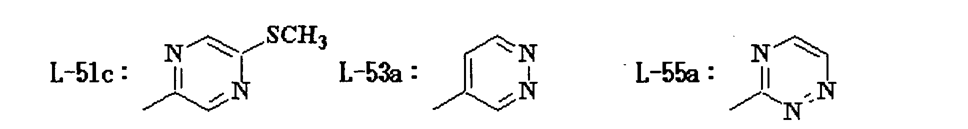

Moreover, in the tables, the aliphatic heterocyclic rings represented by M-4a to M-22a each represent the following structures.

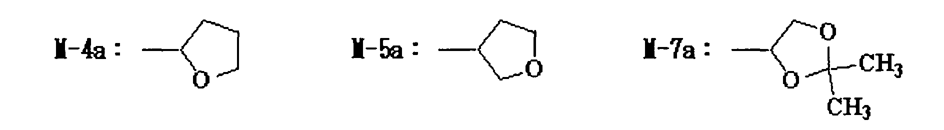

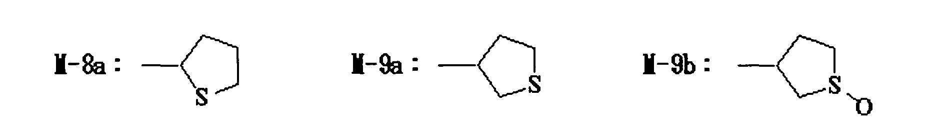

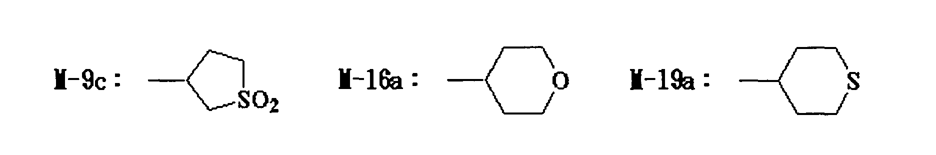

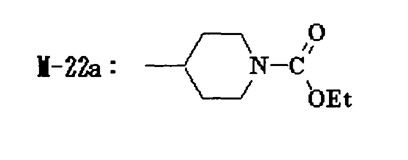

In the following Table 2, with regard to the compound represented by Formula [1]-22, no substituent(s) corresponding to R² exists in the table.

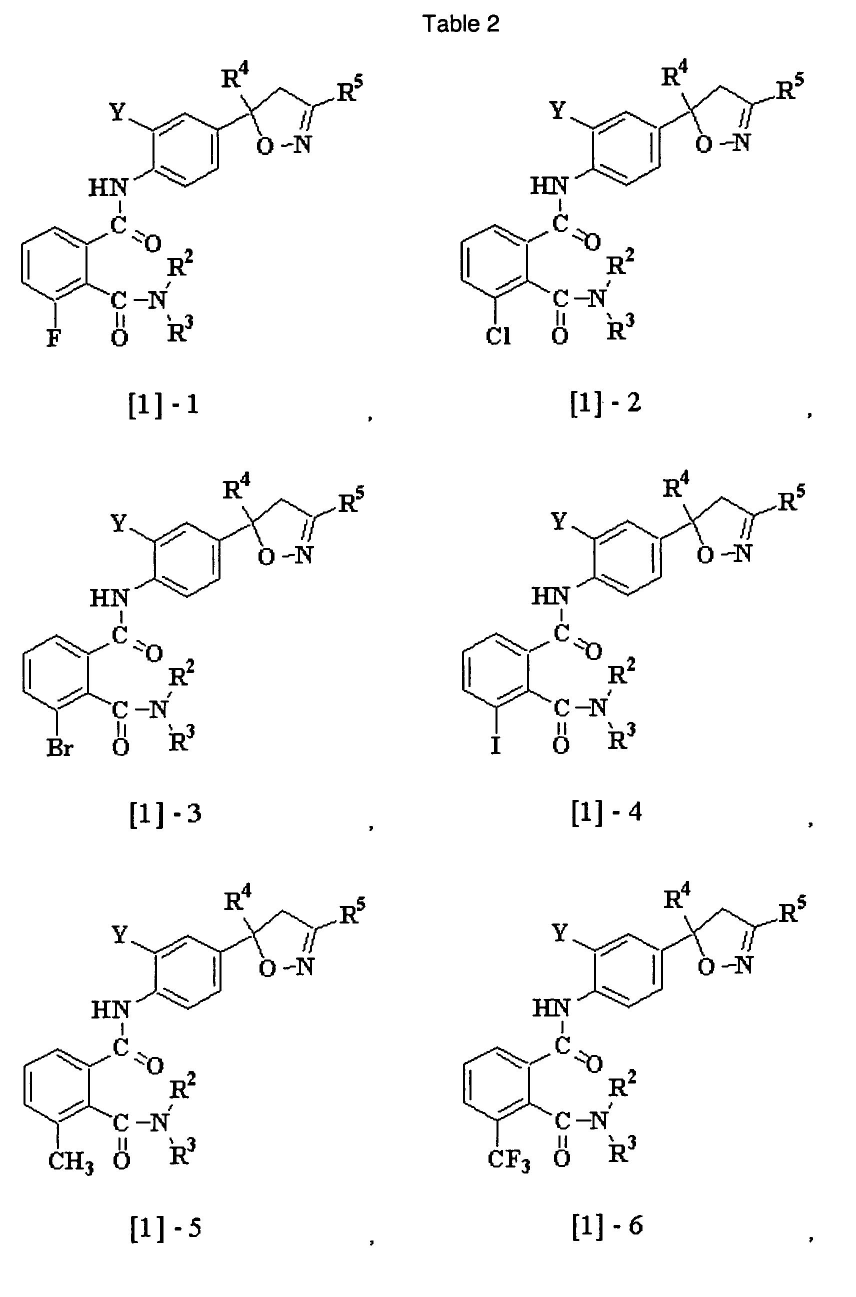

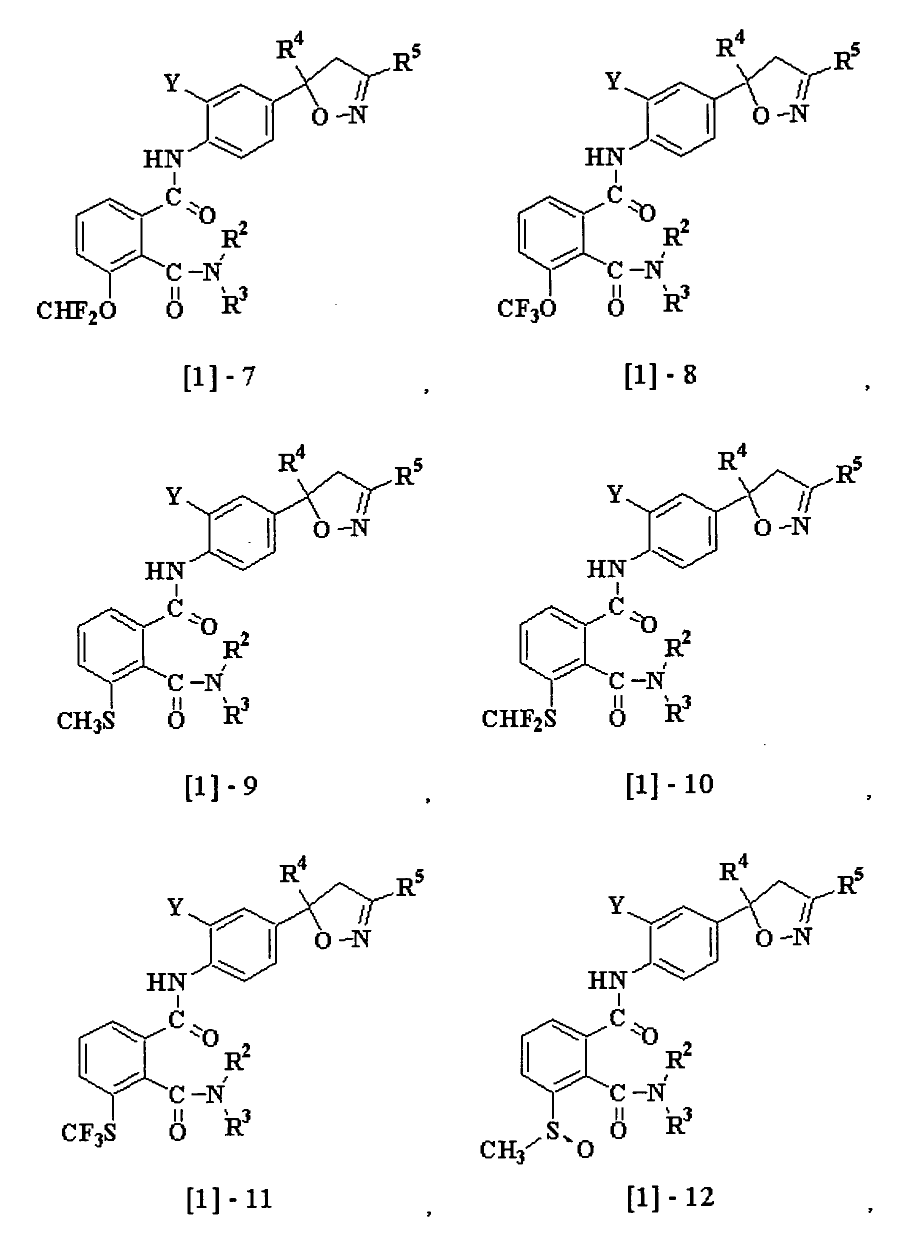

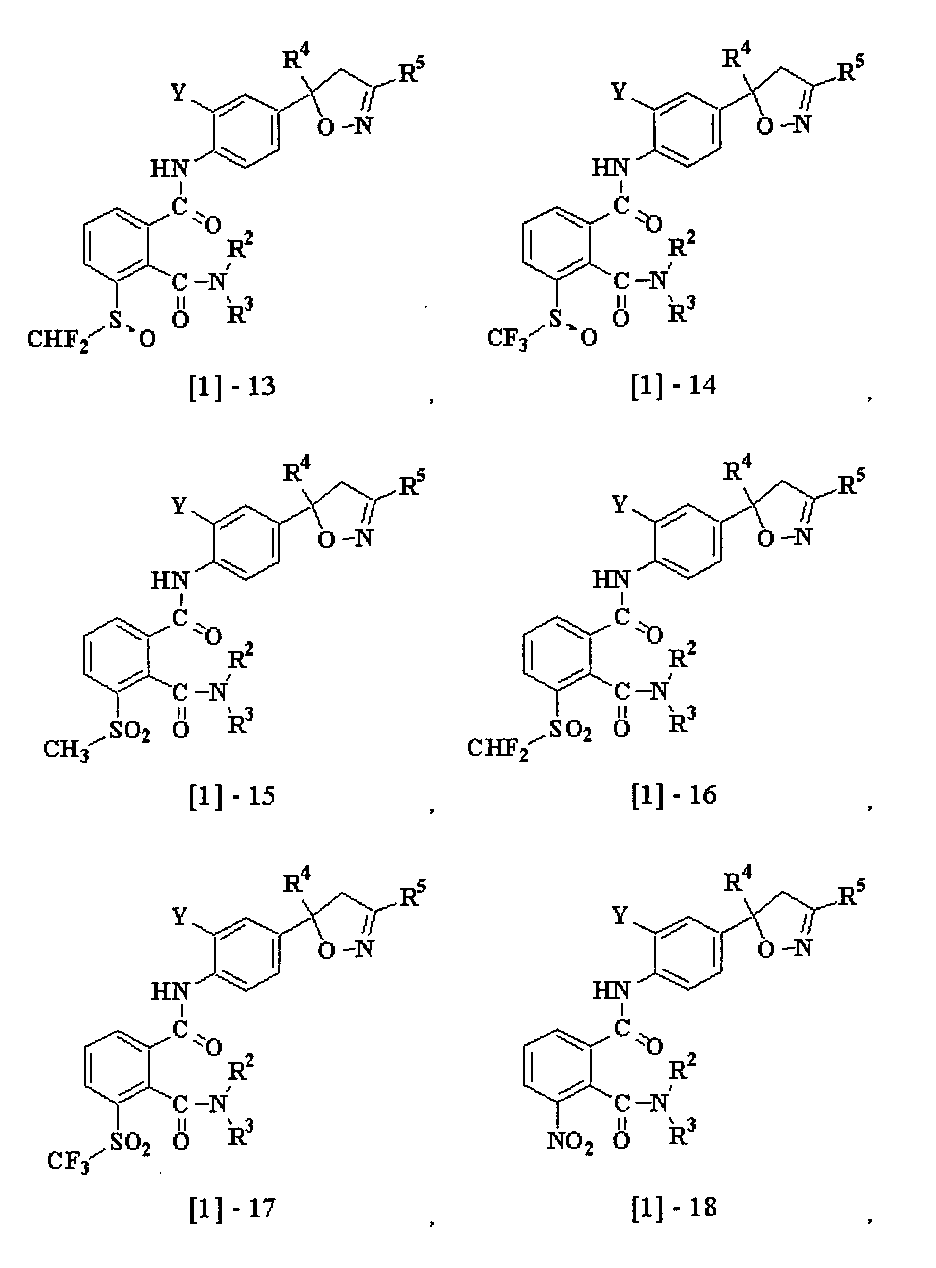

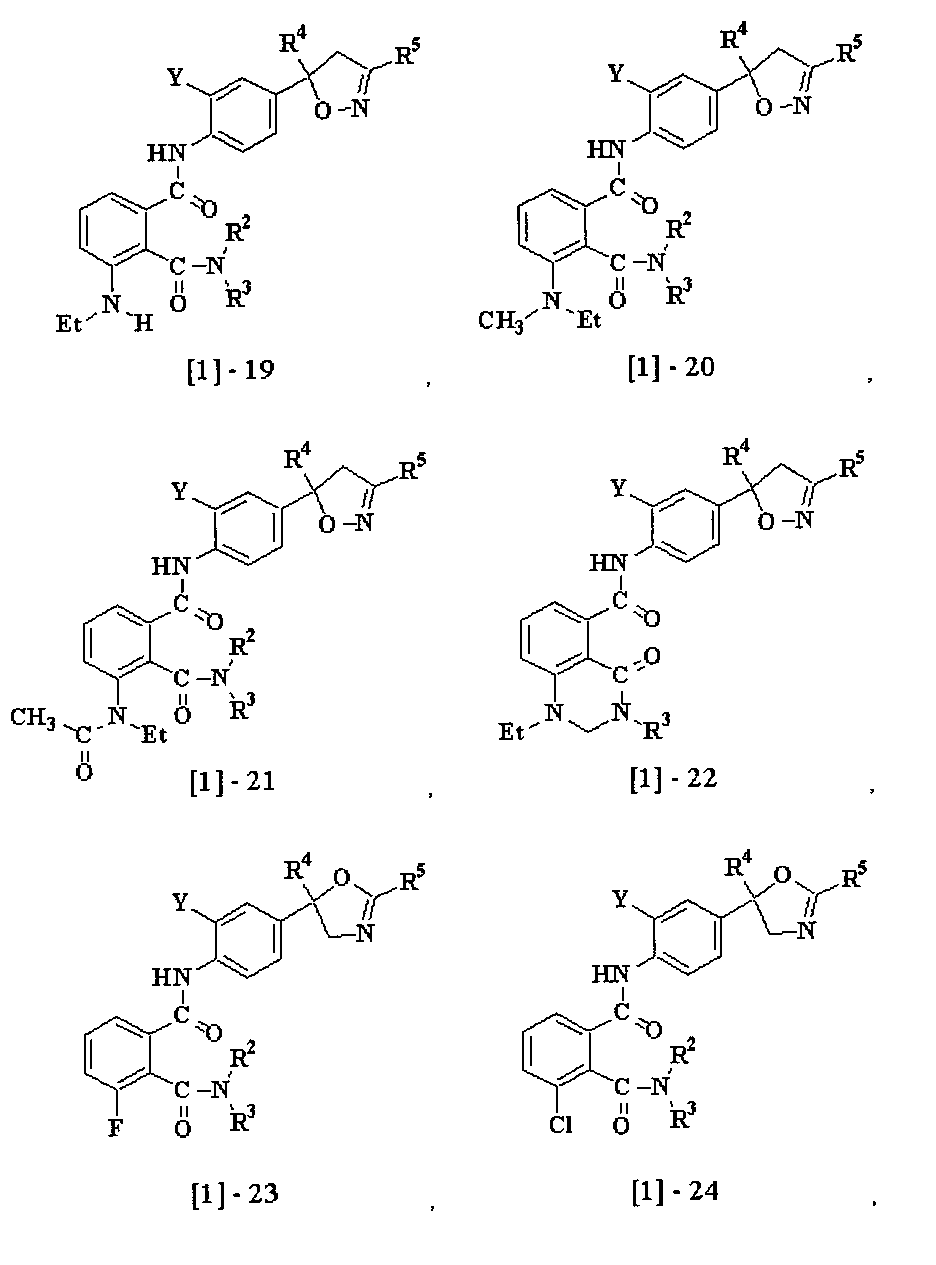

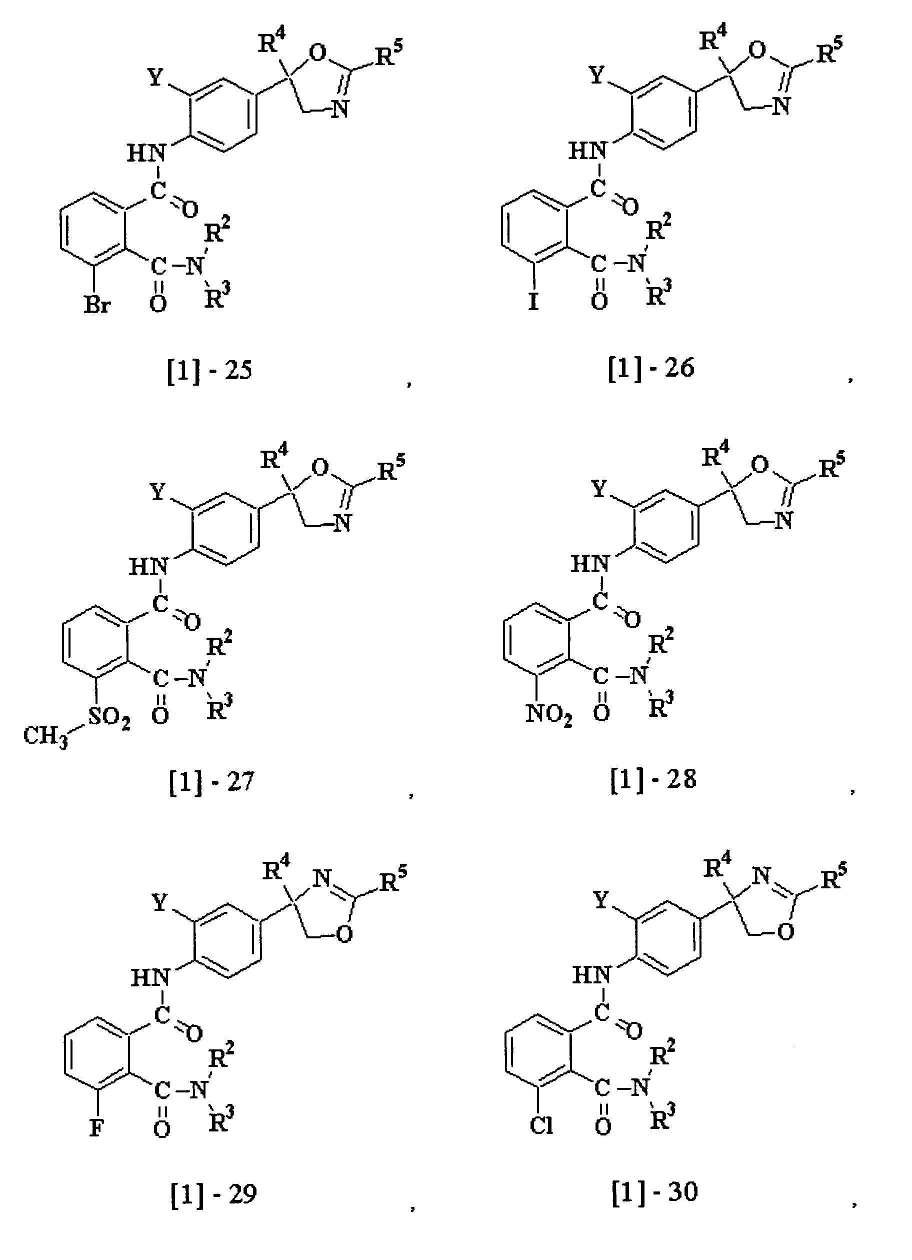

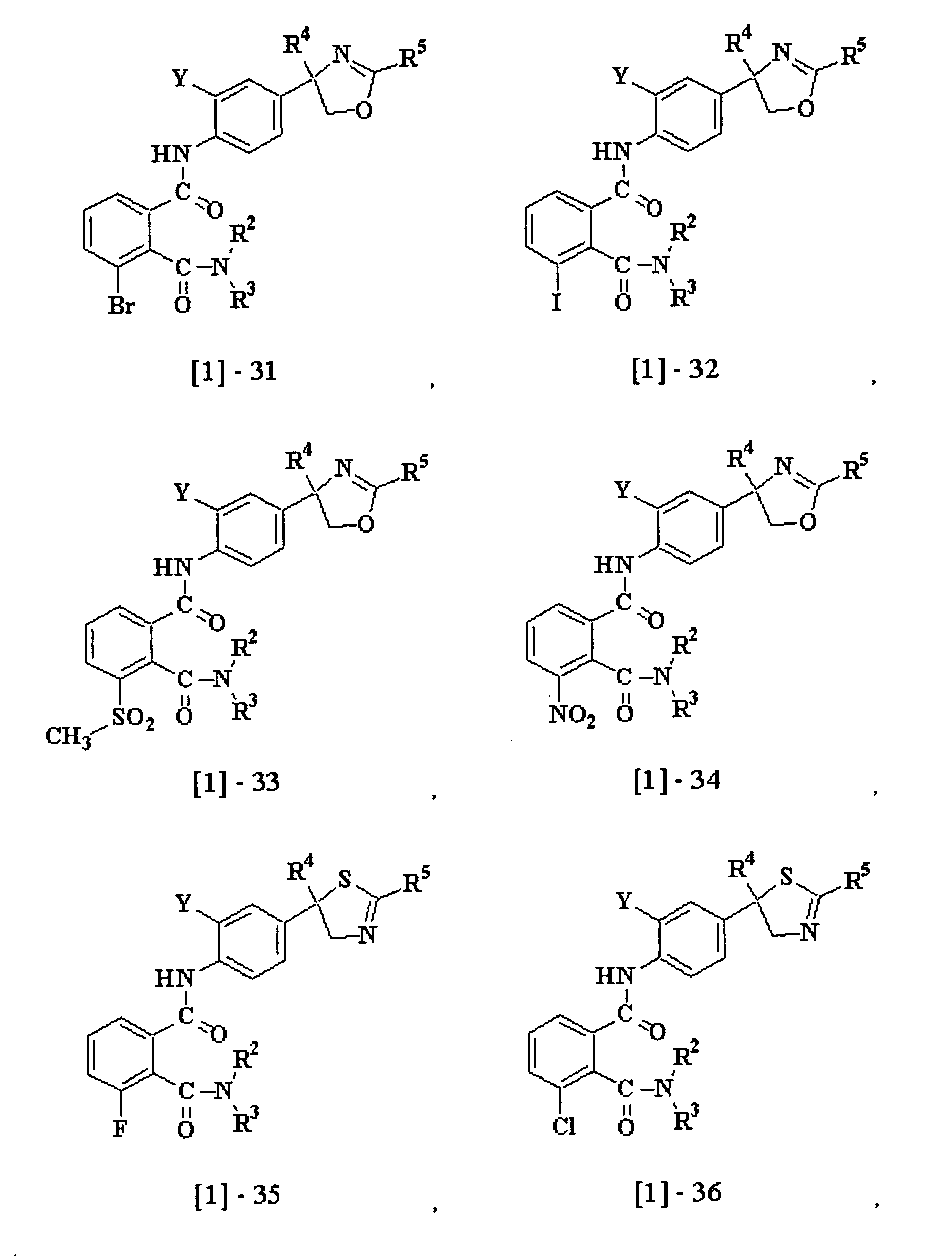

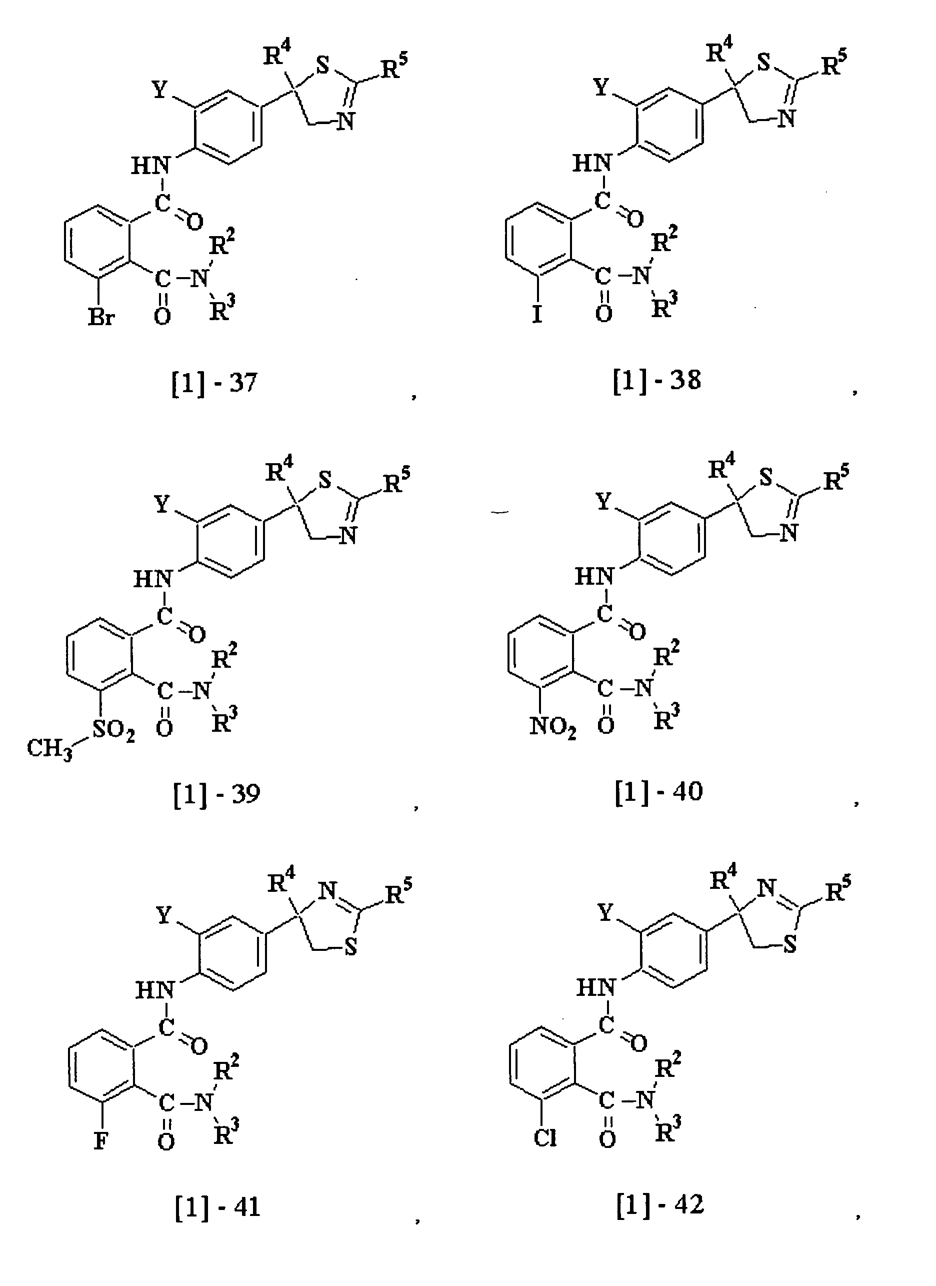

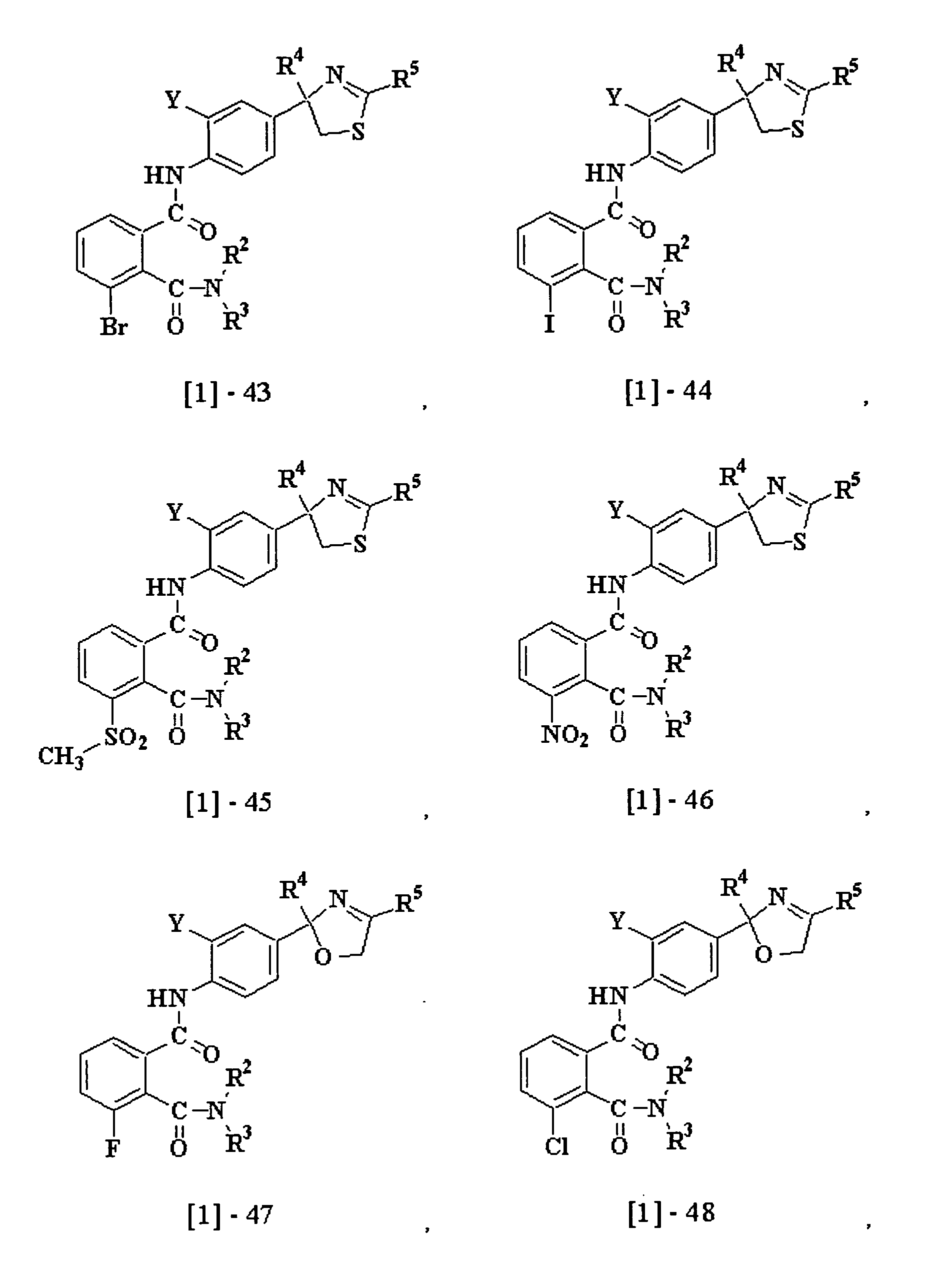

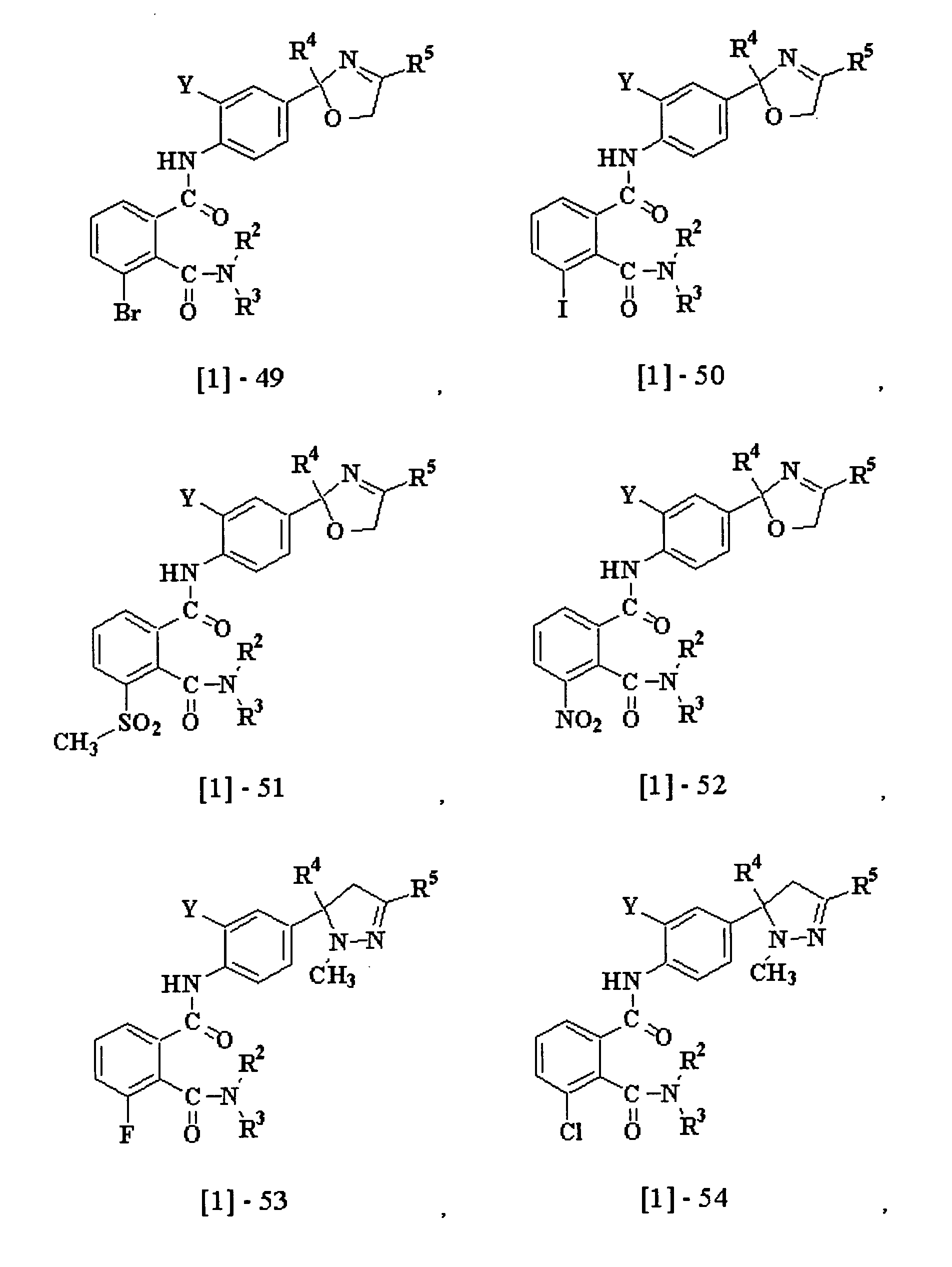

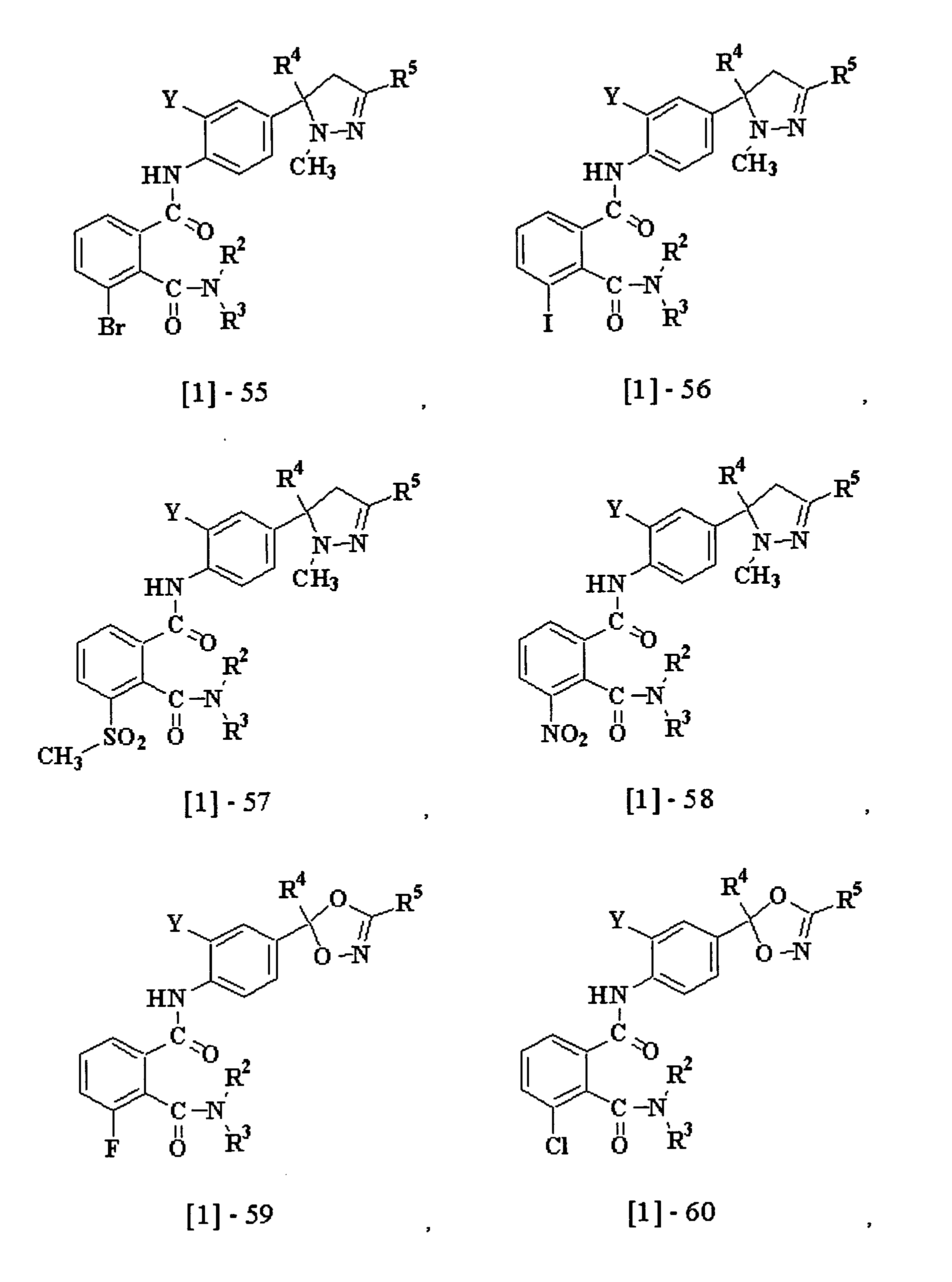

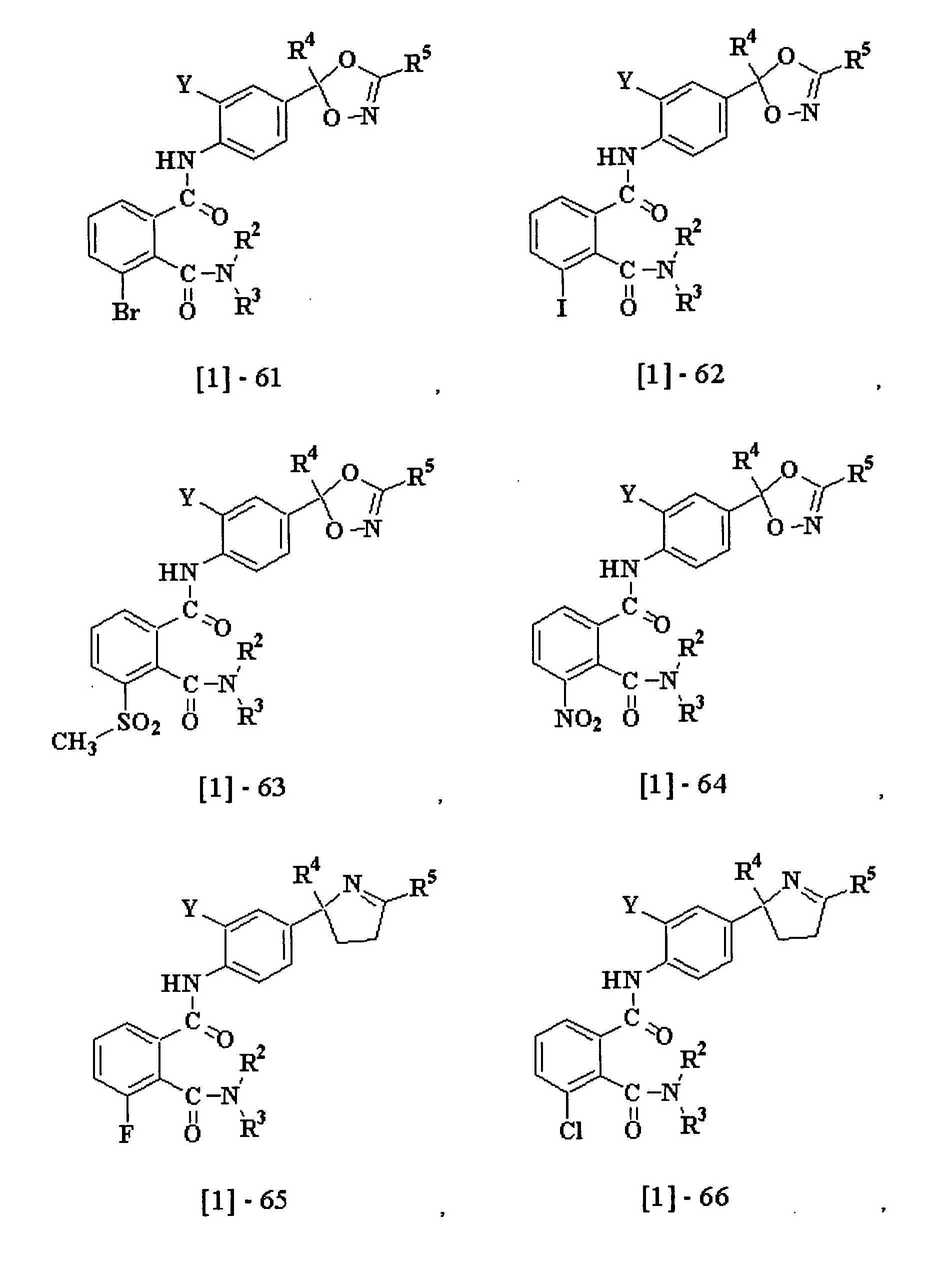

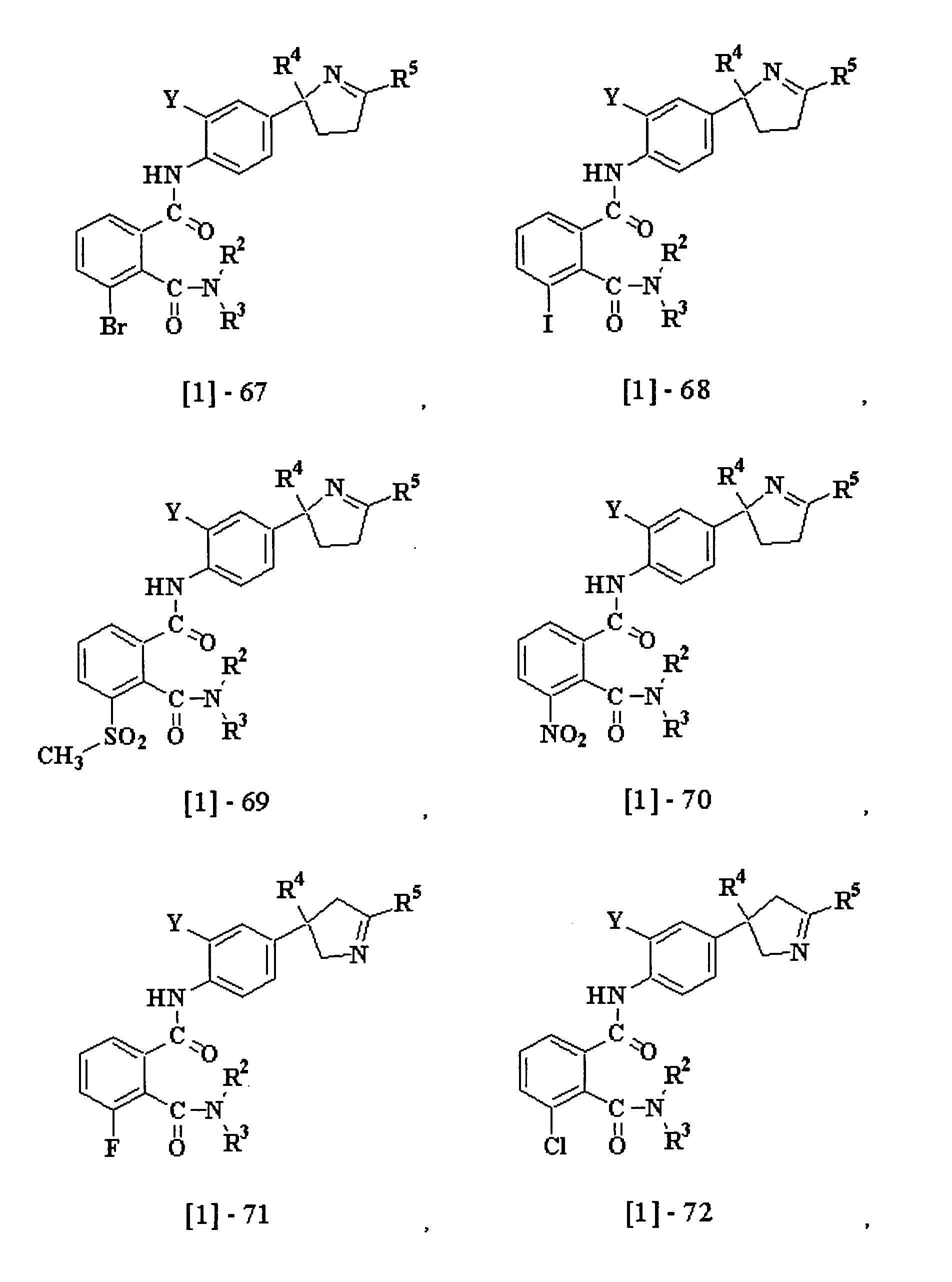

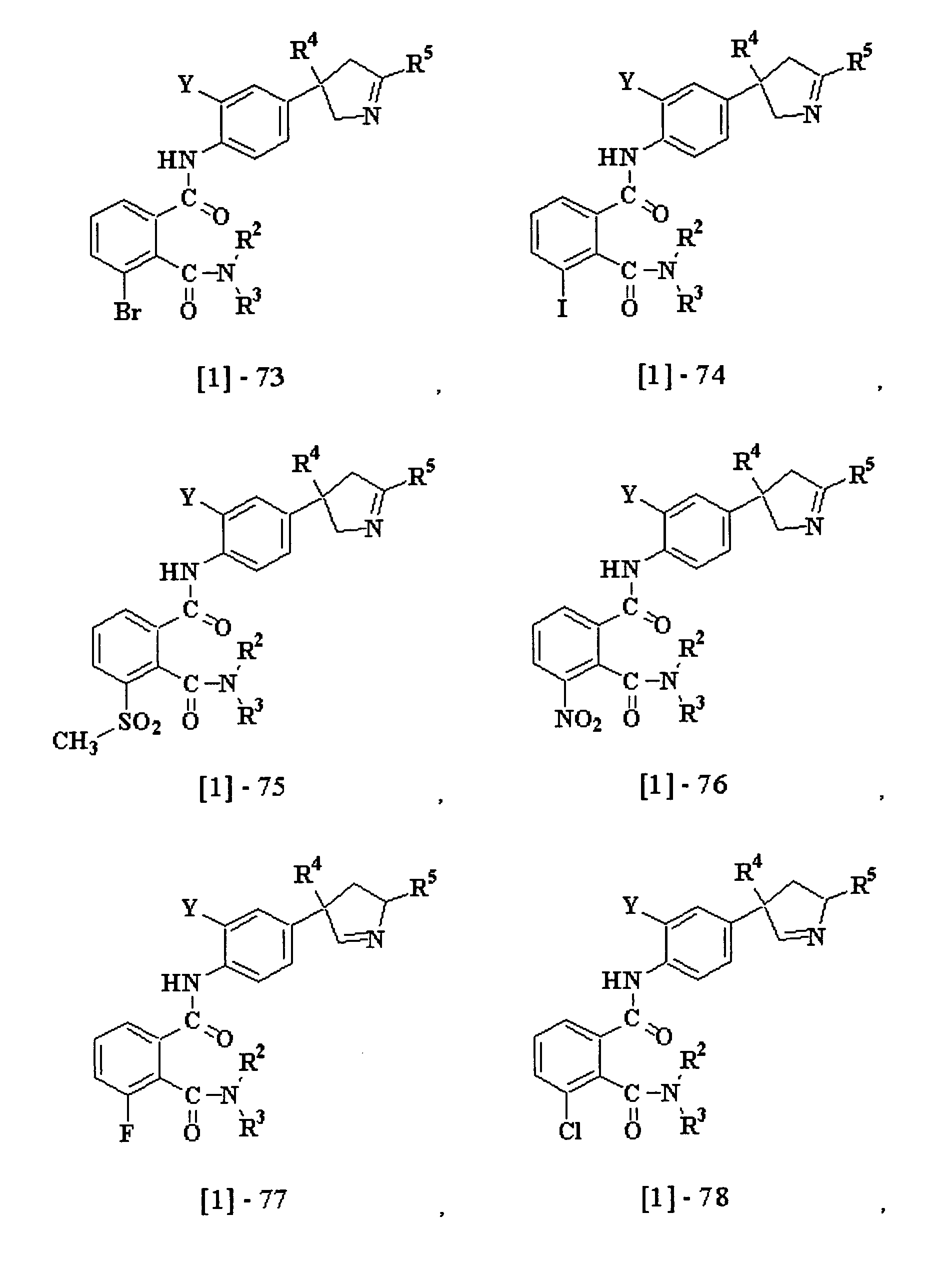

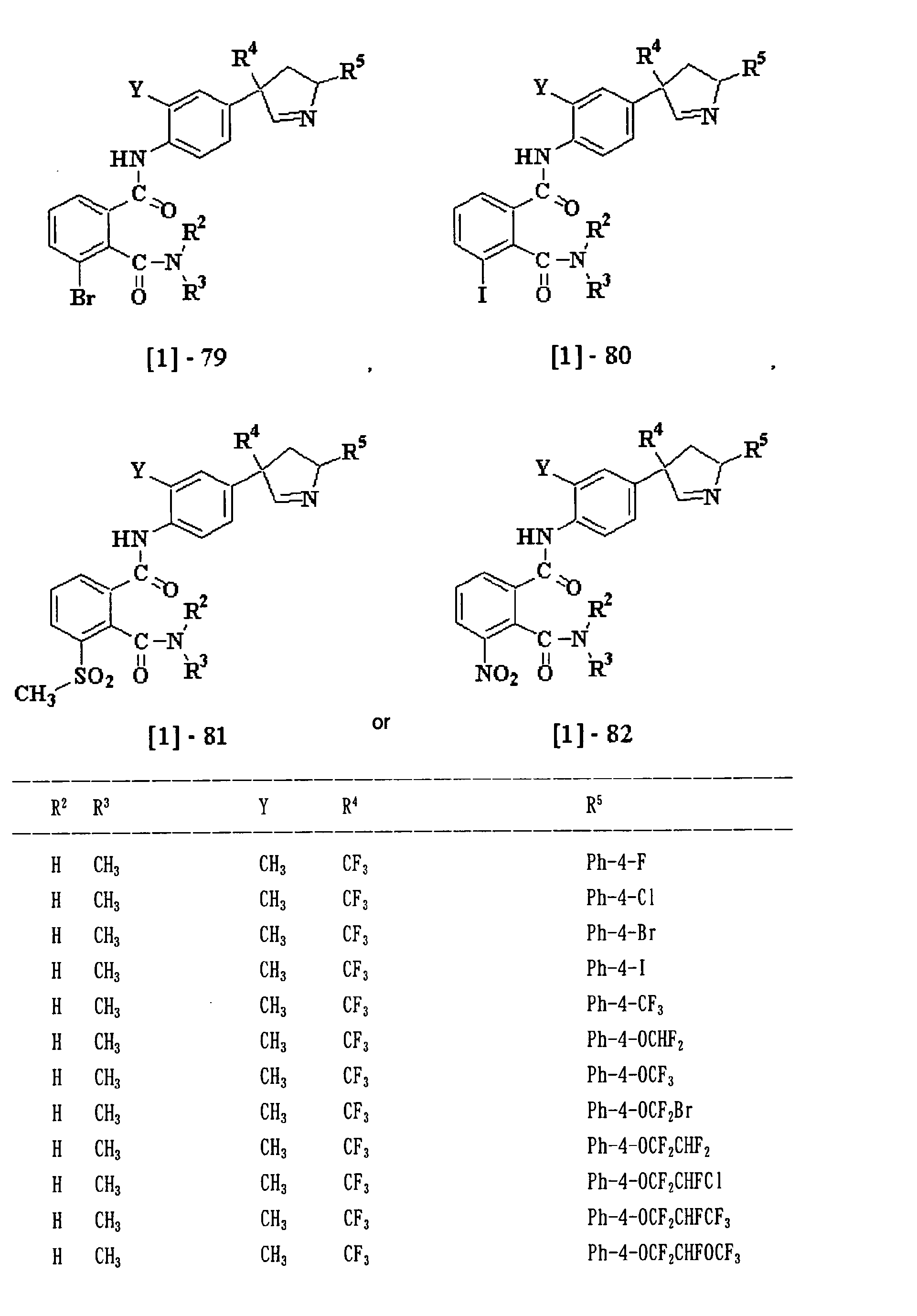

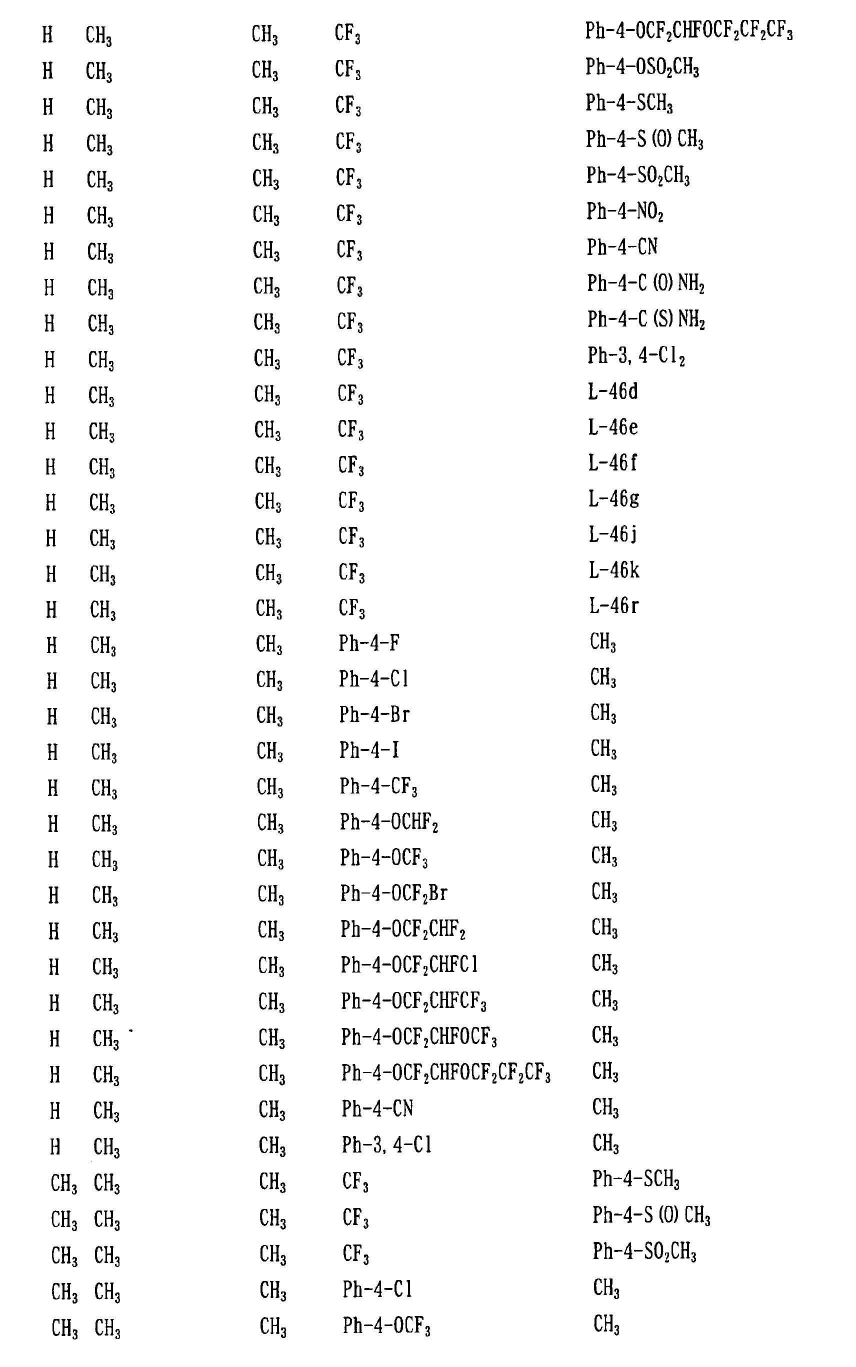

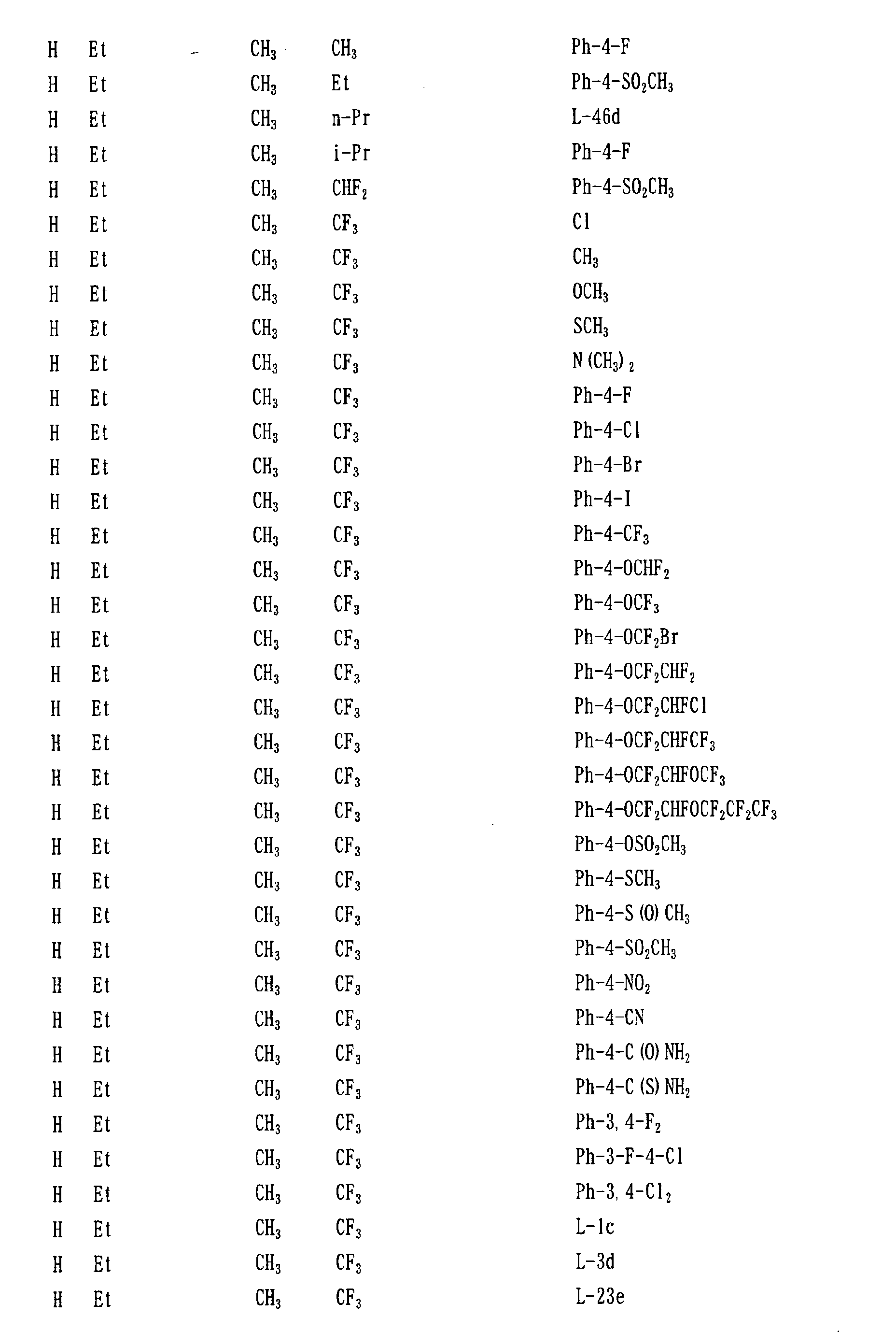

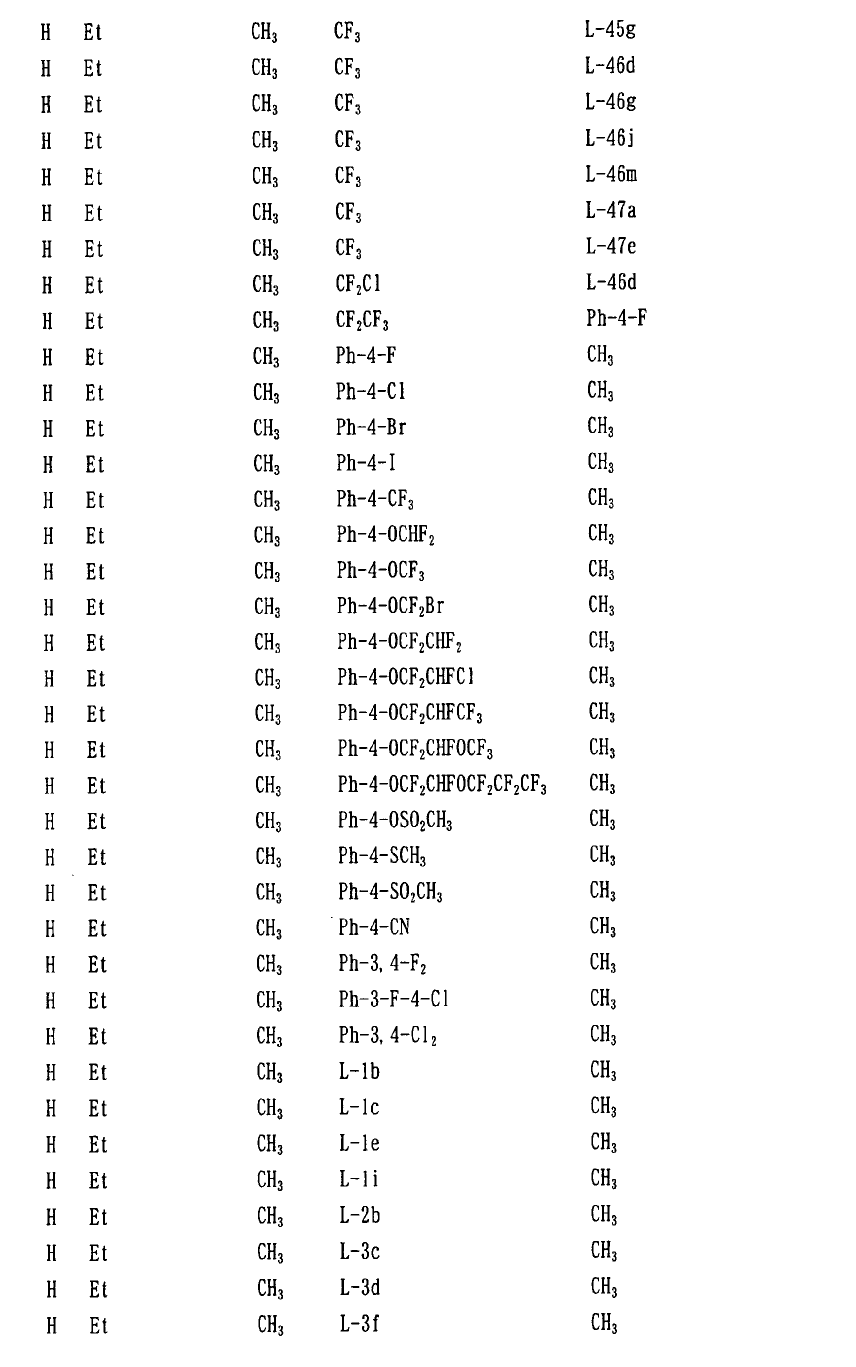

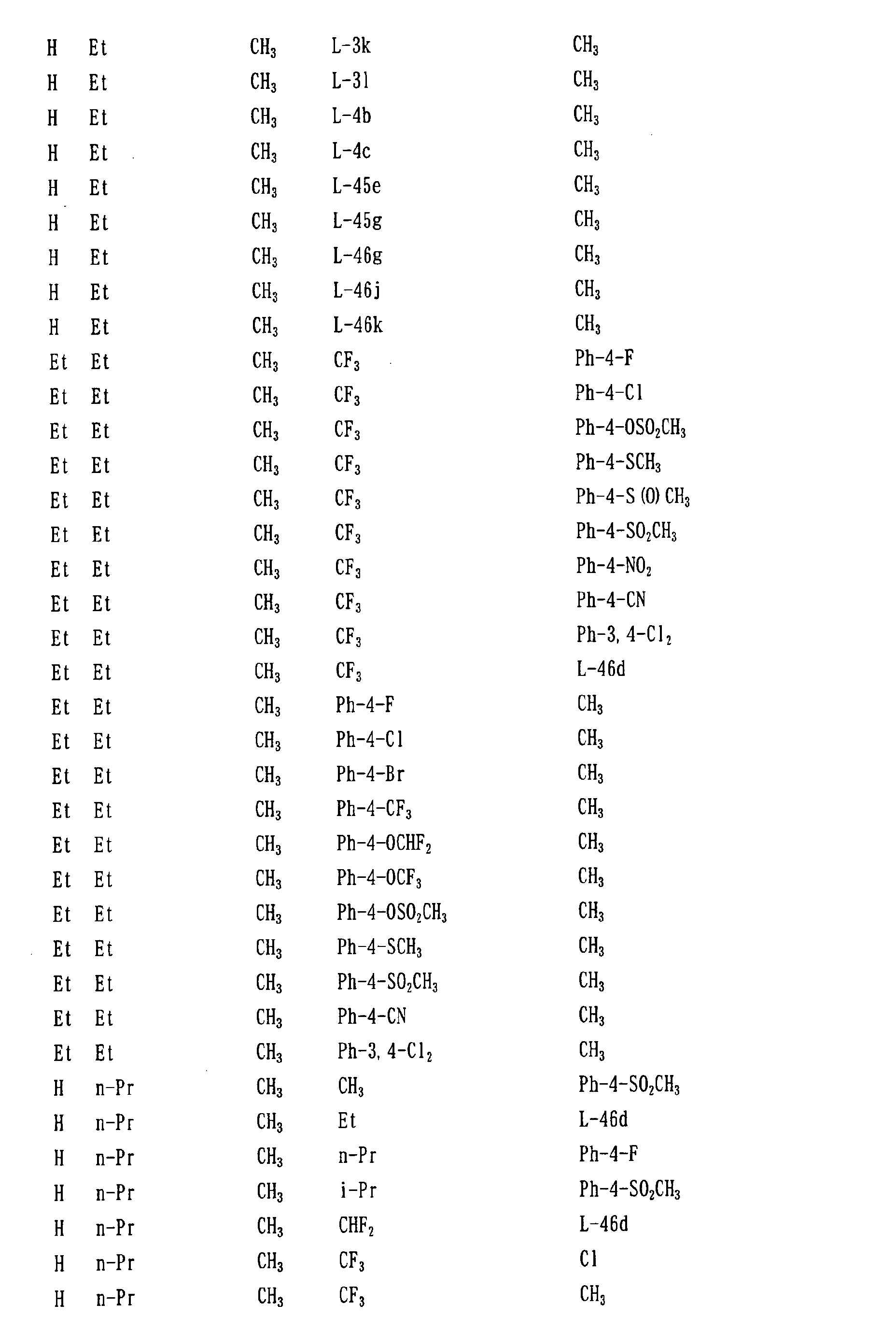

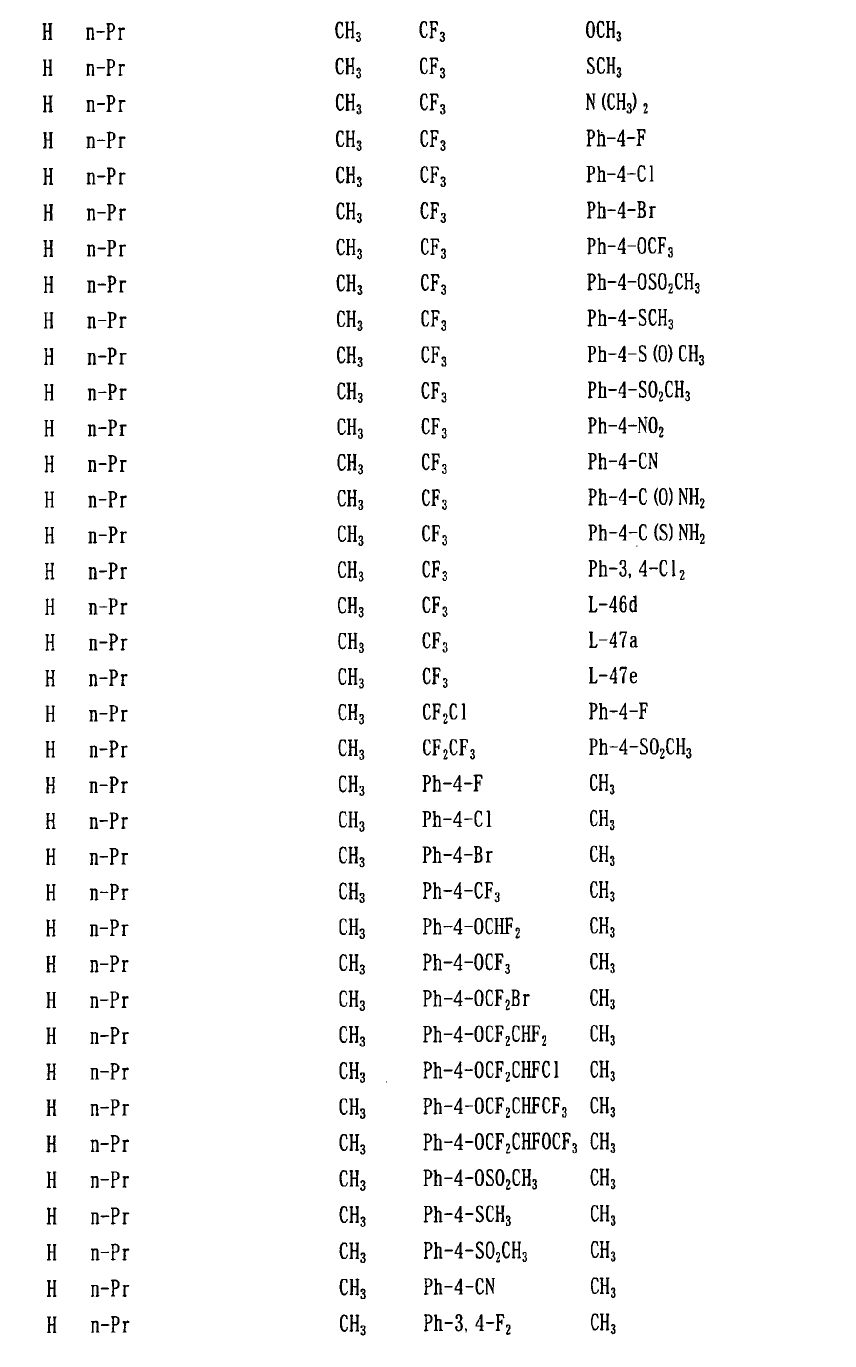

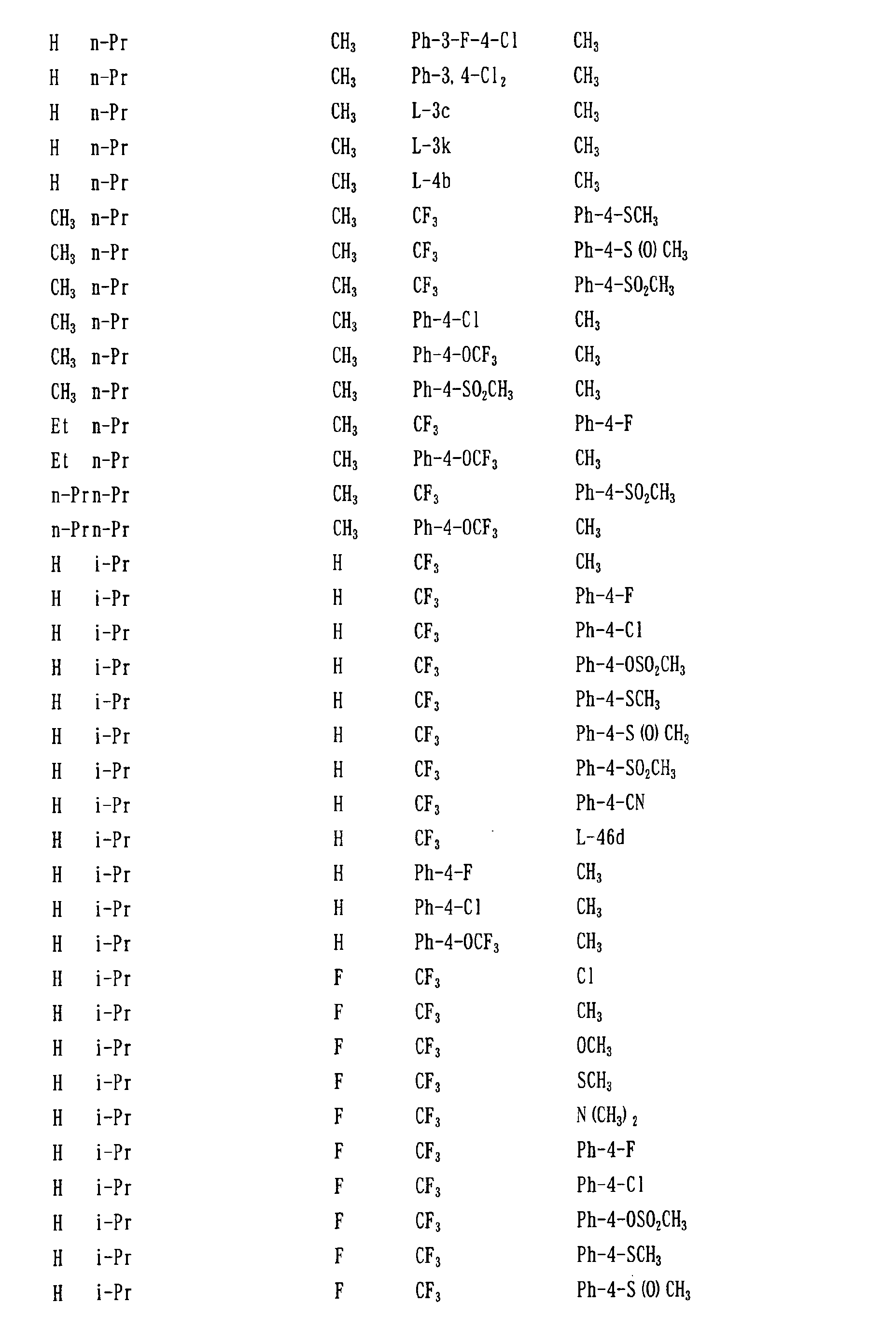

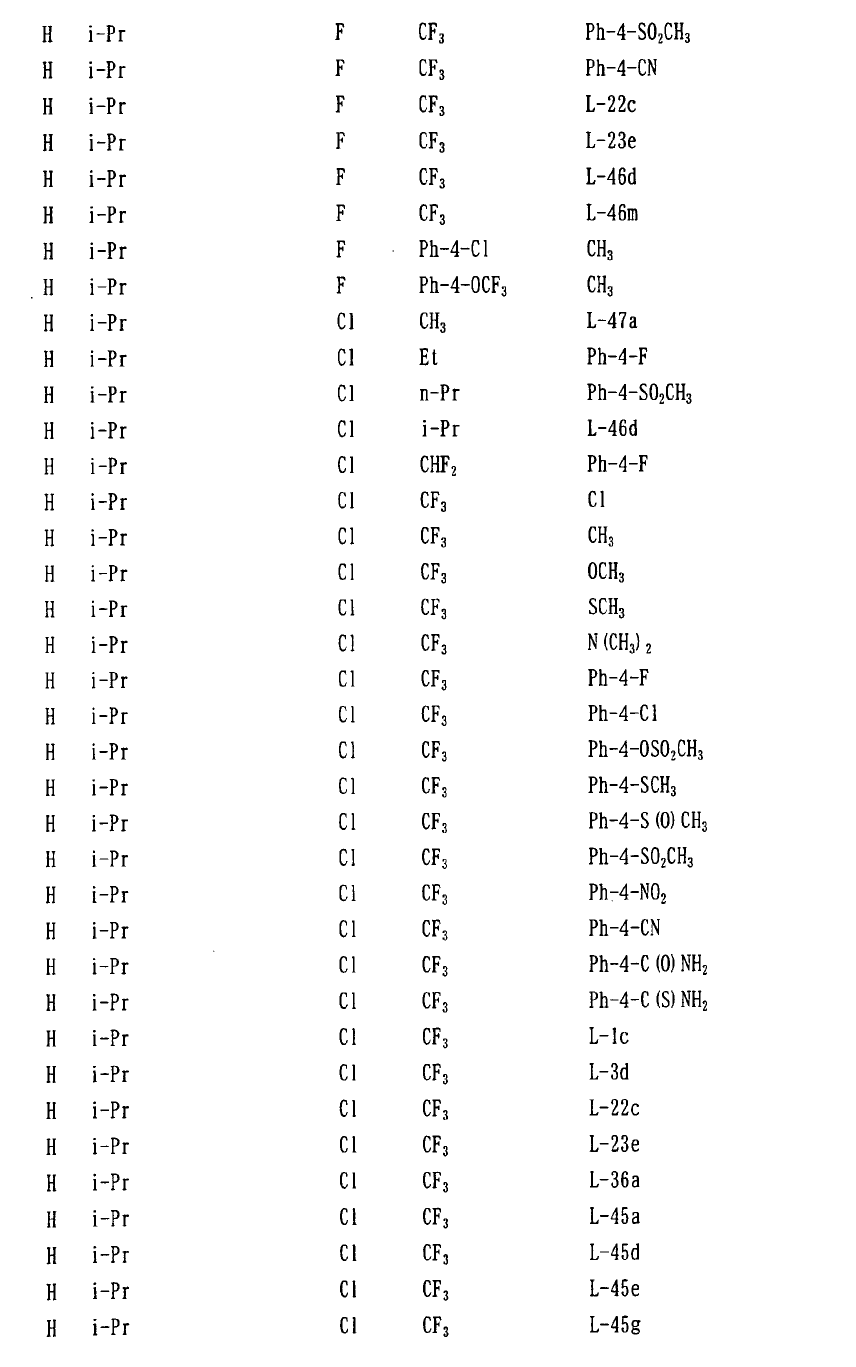

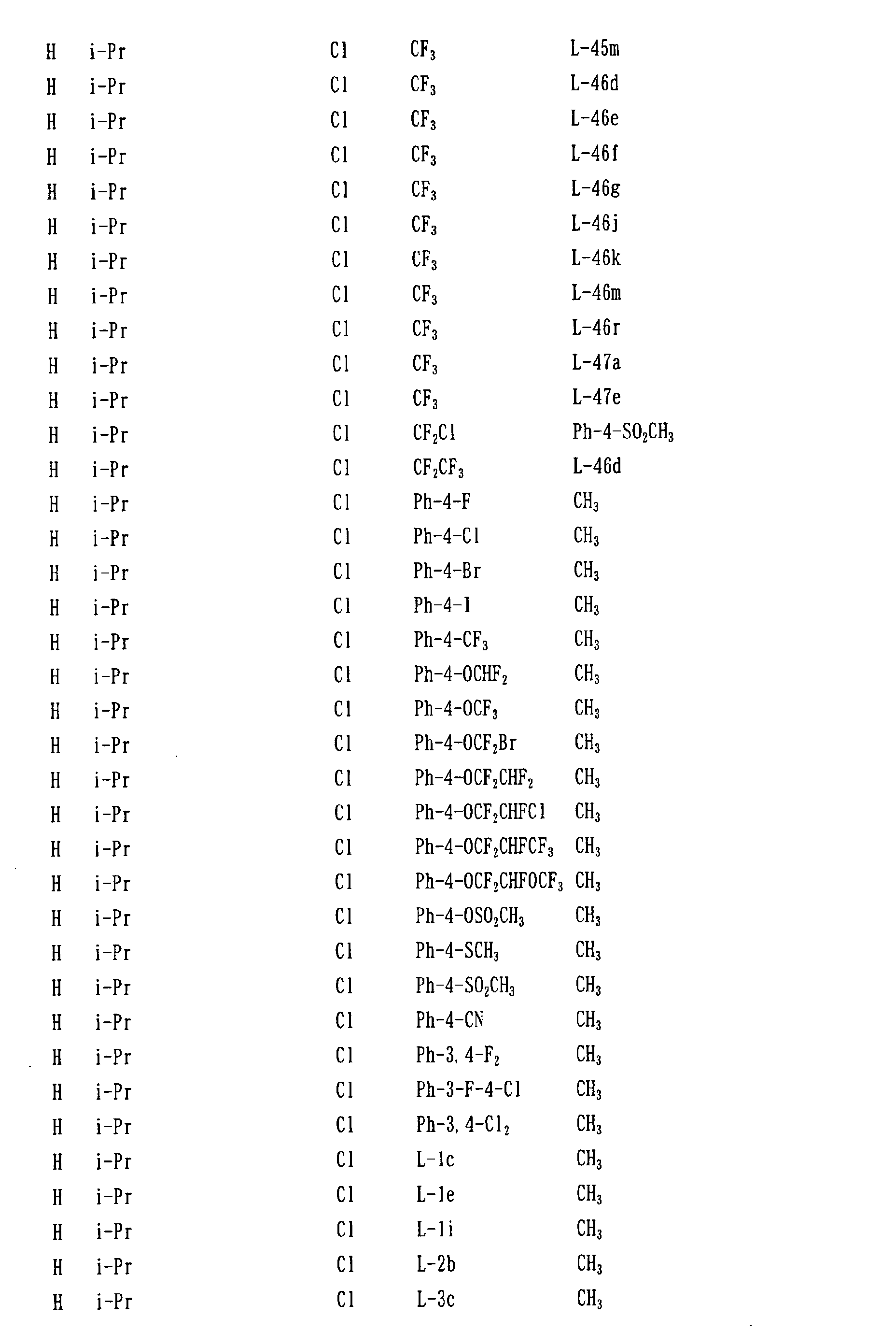

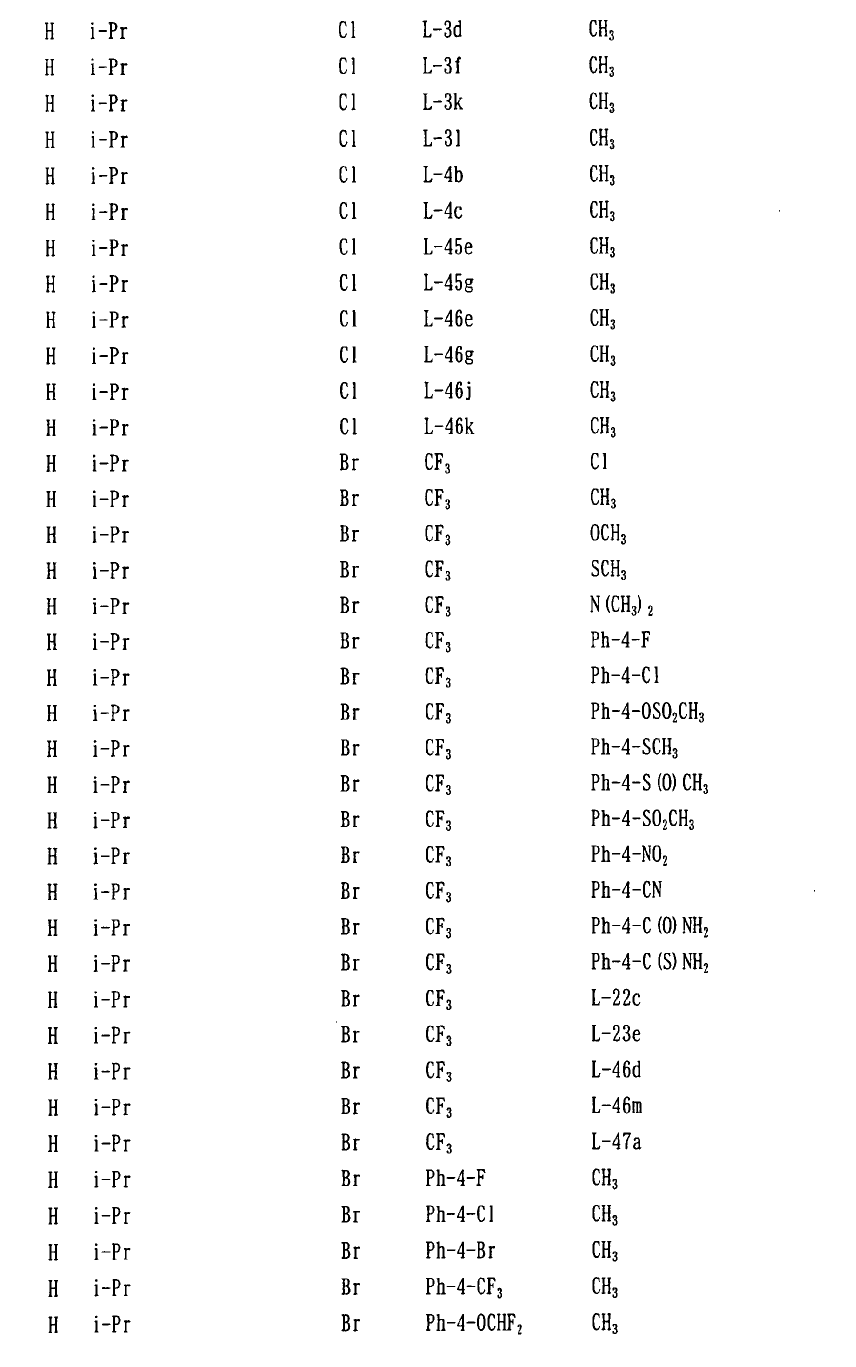

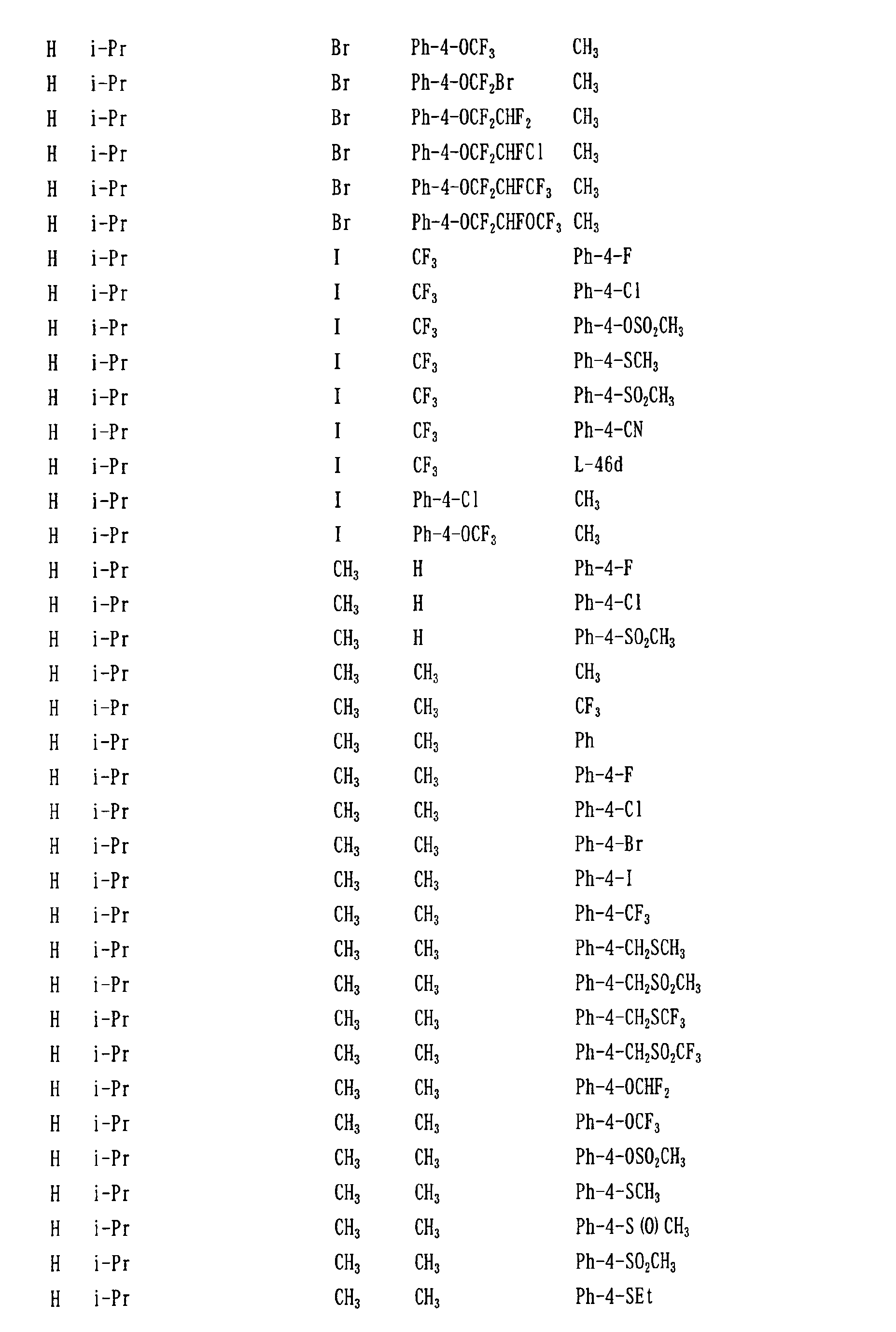

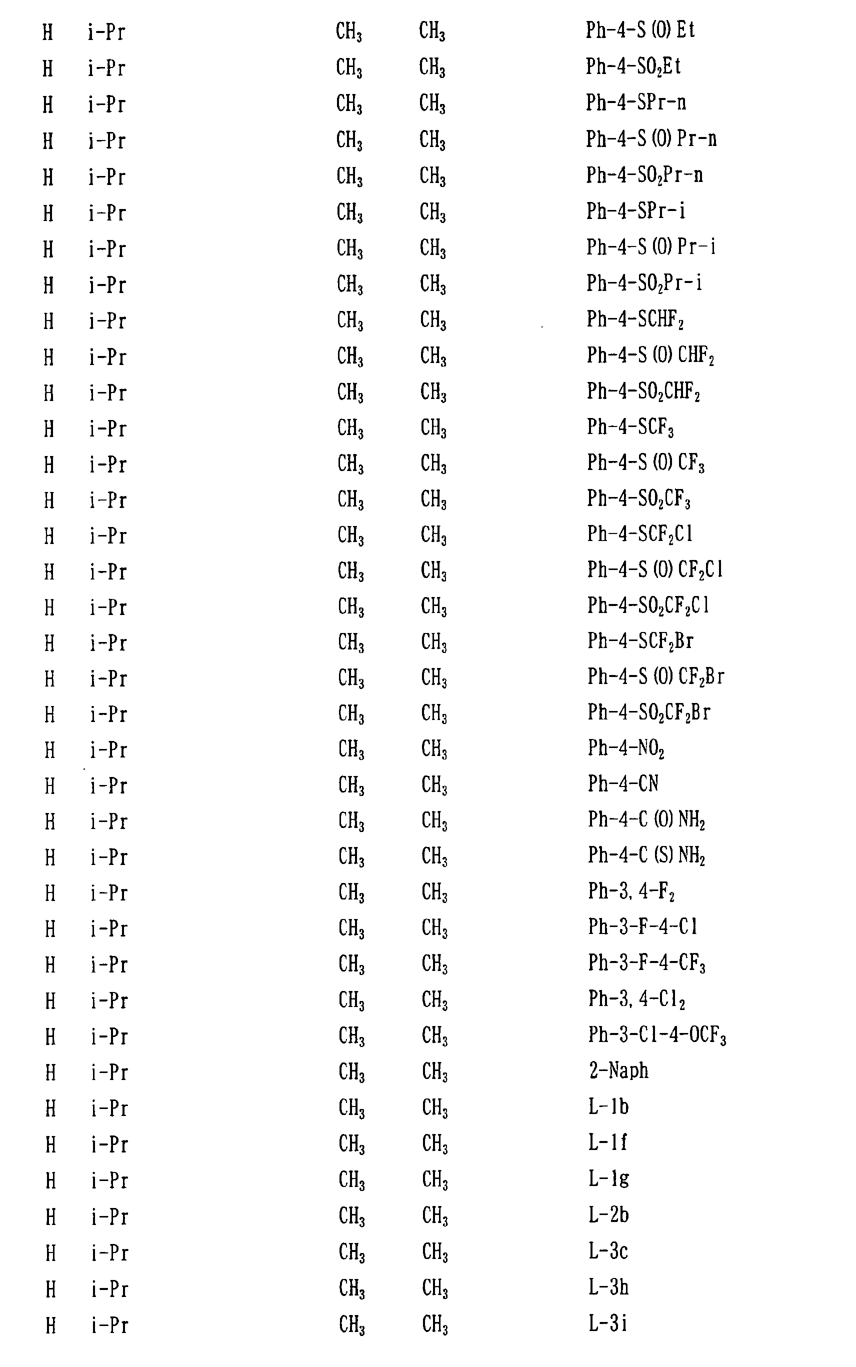

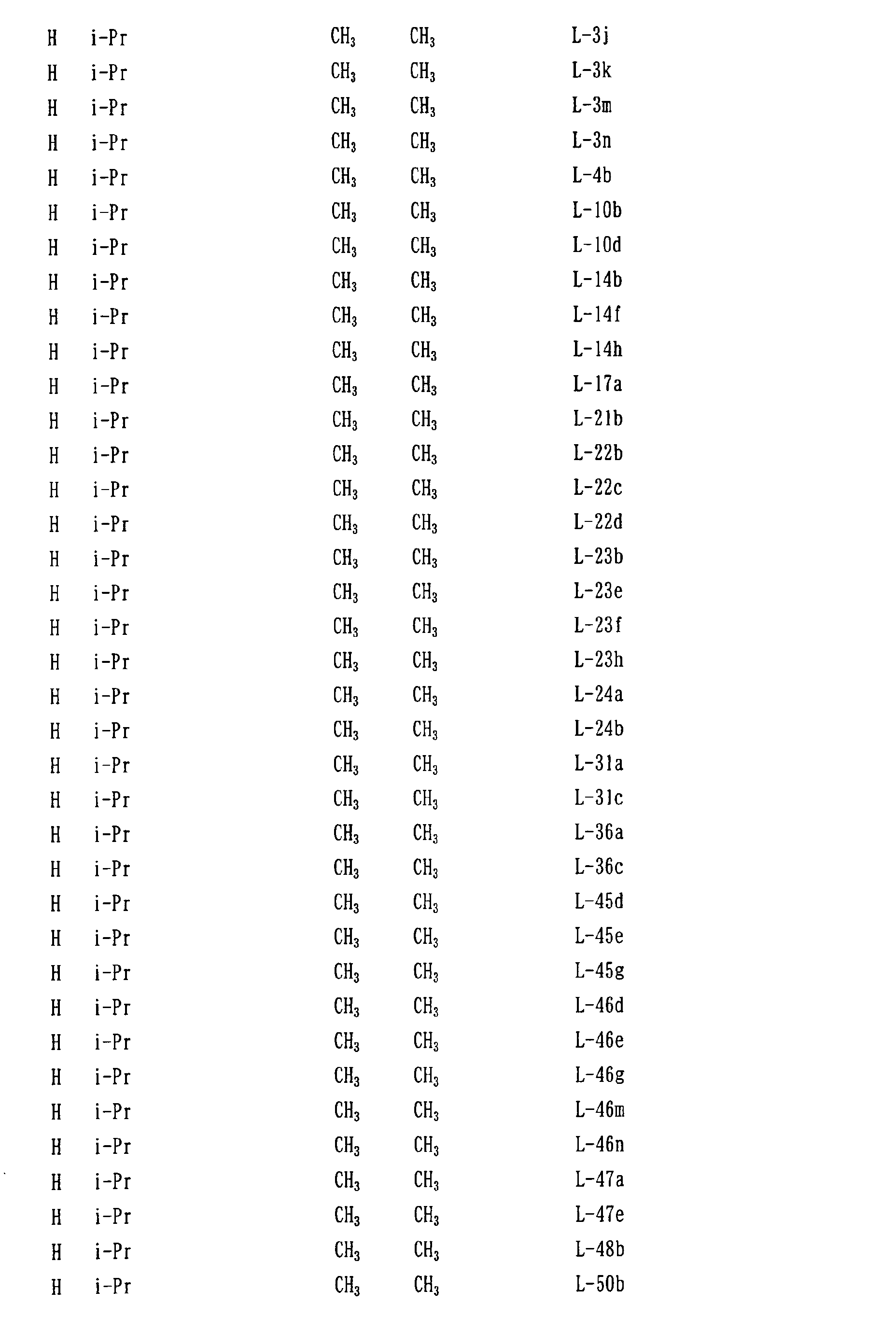

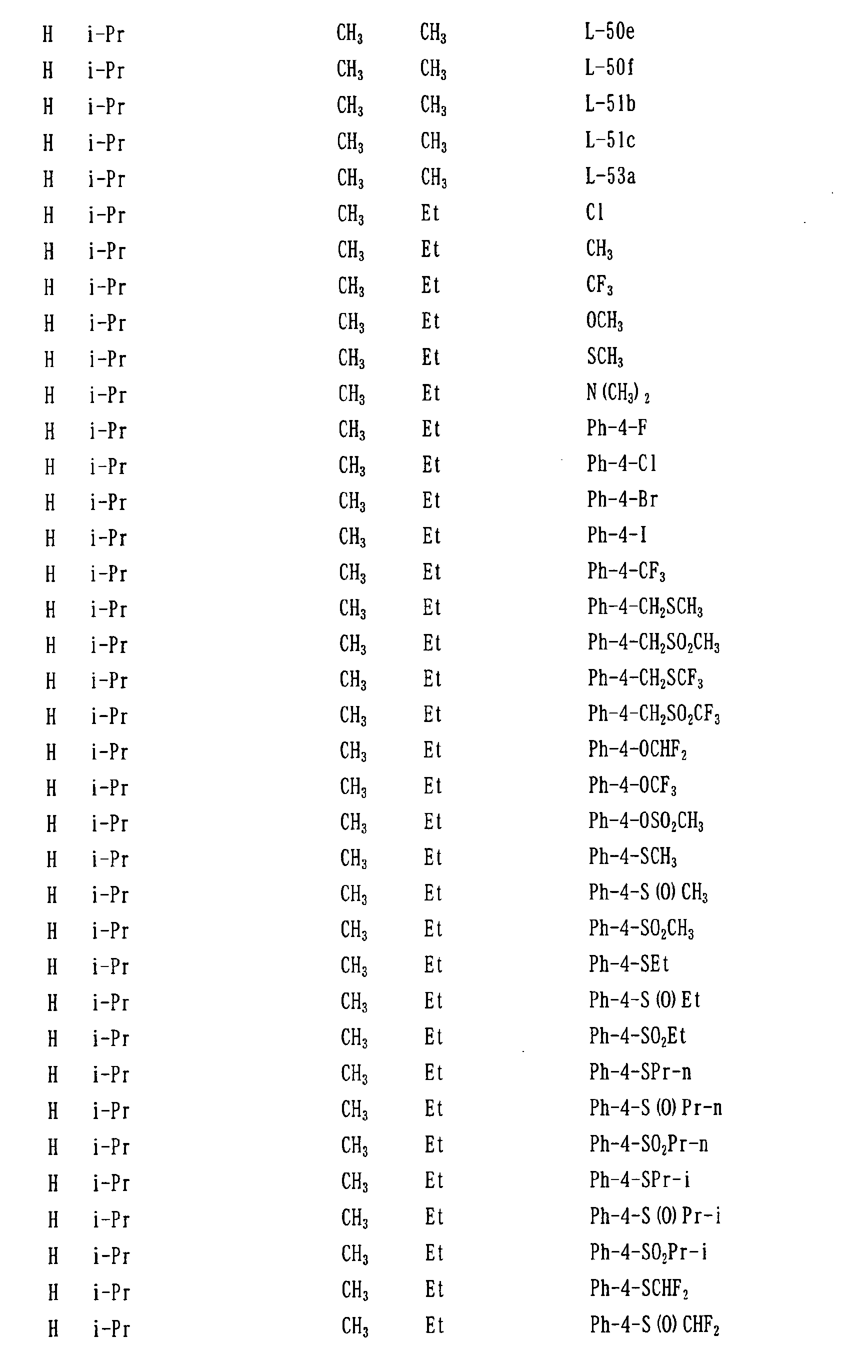

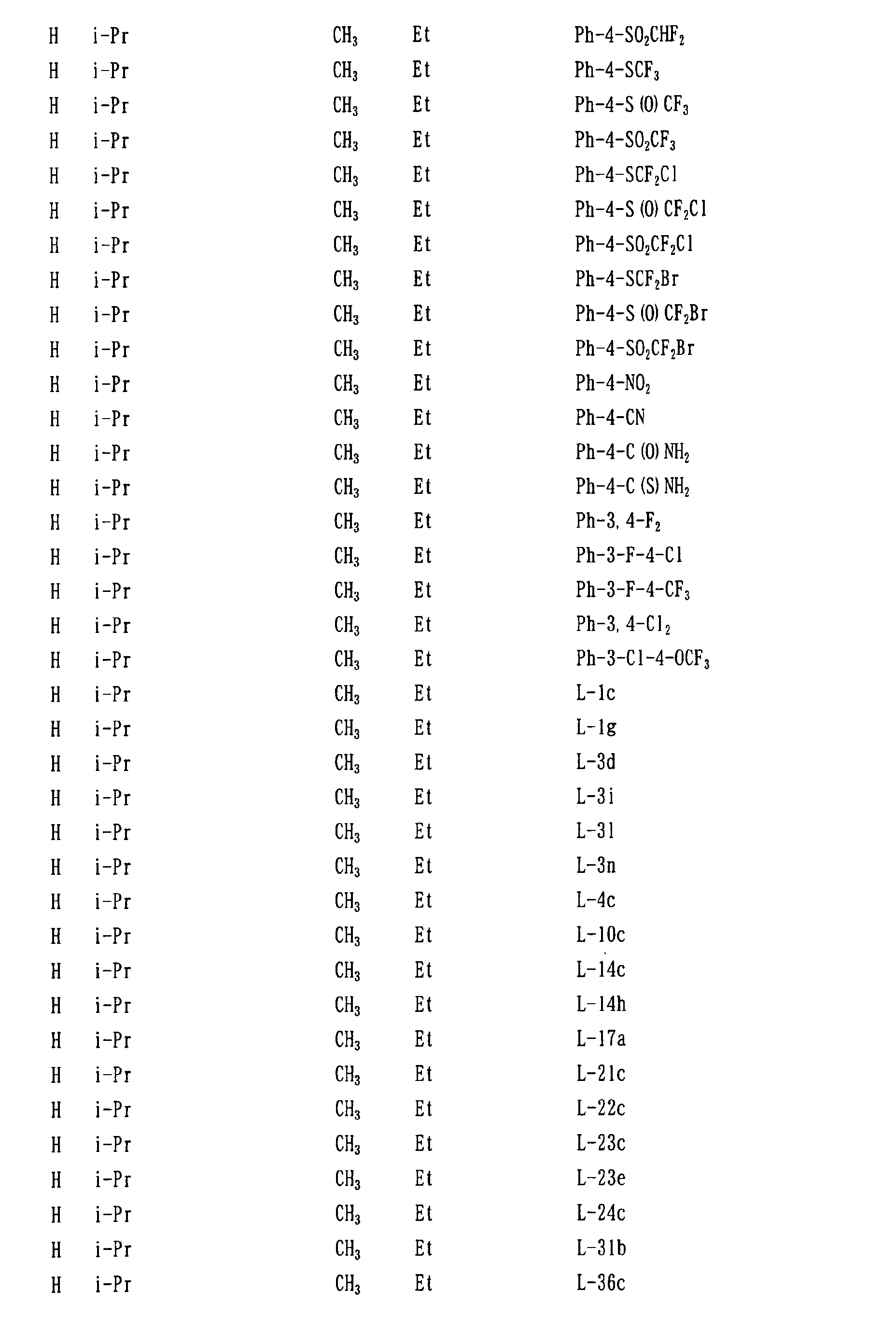

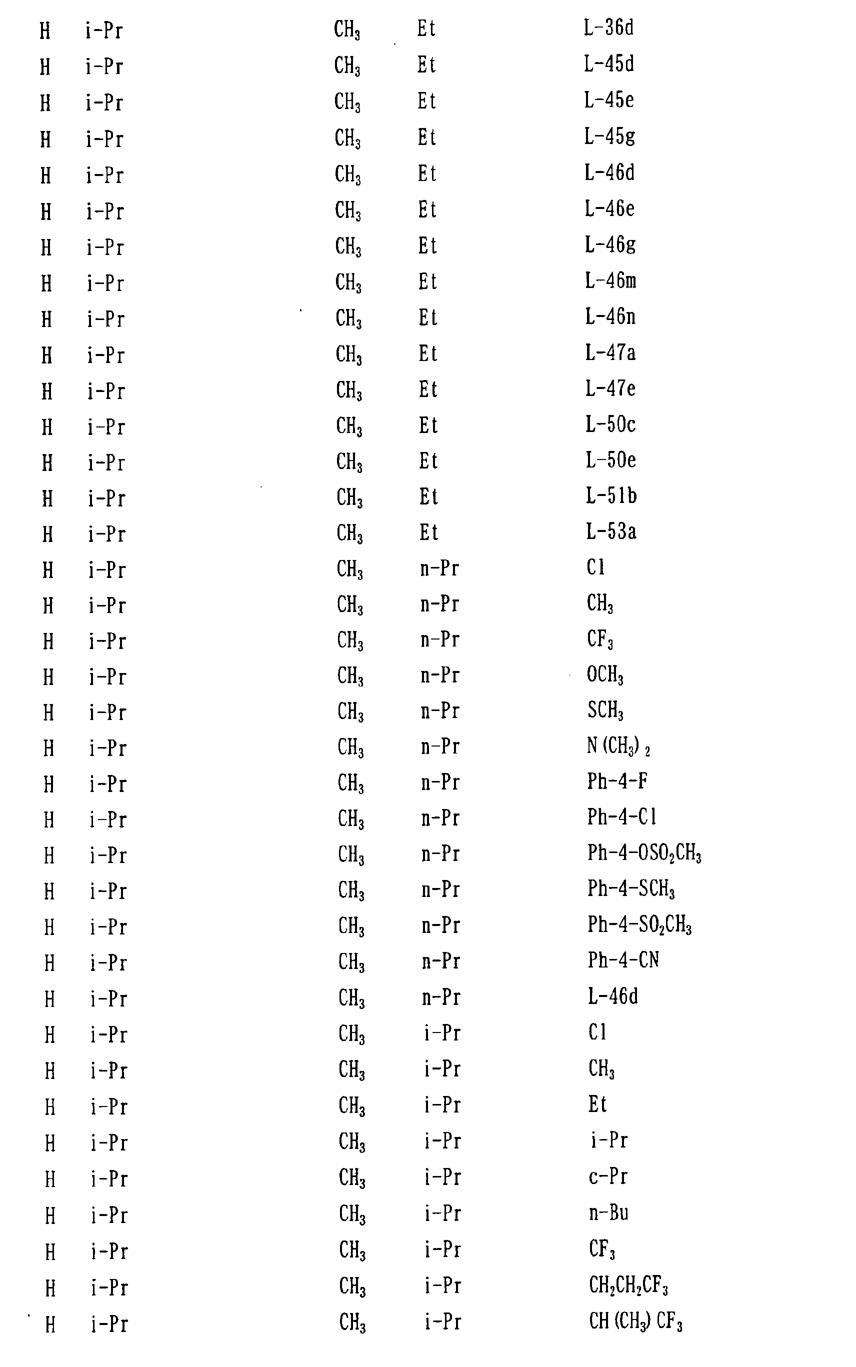

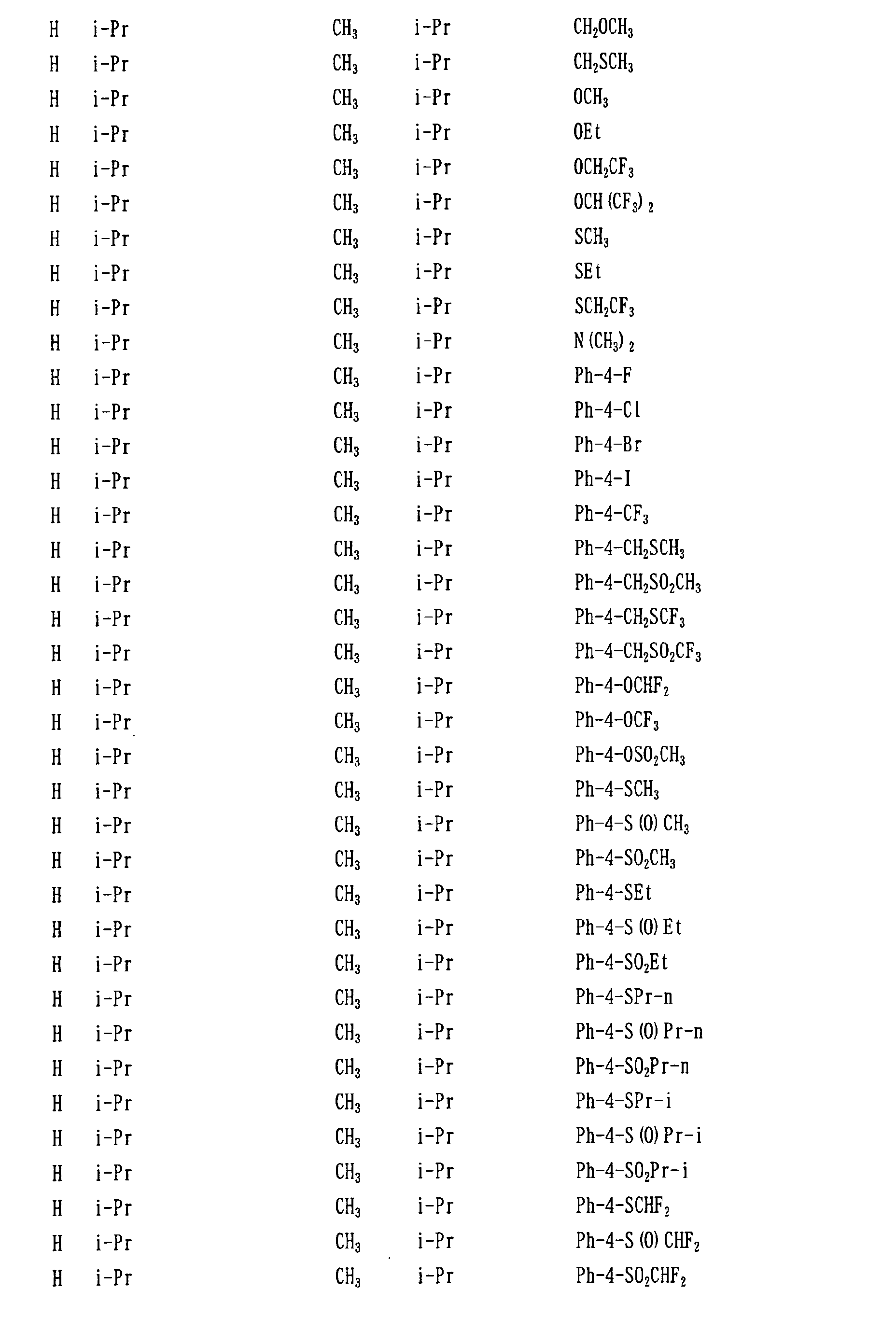

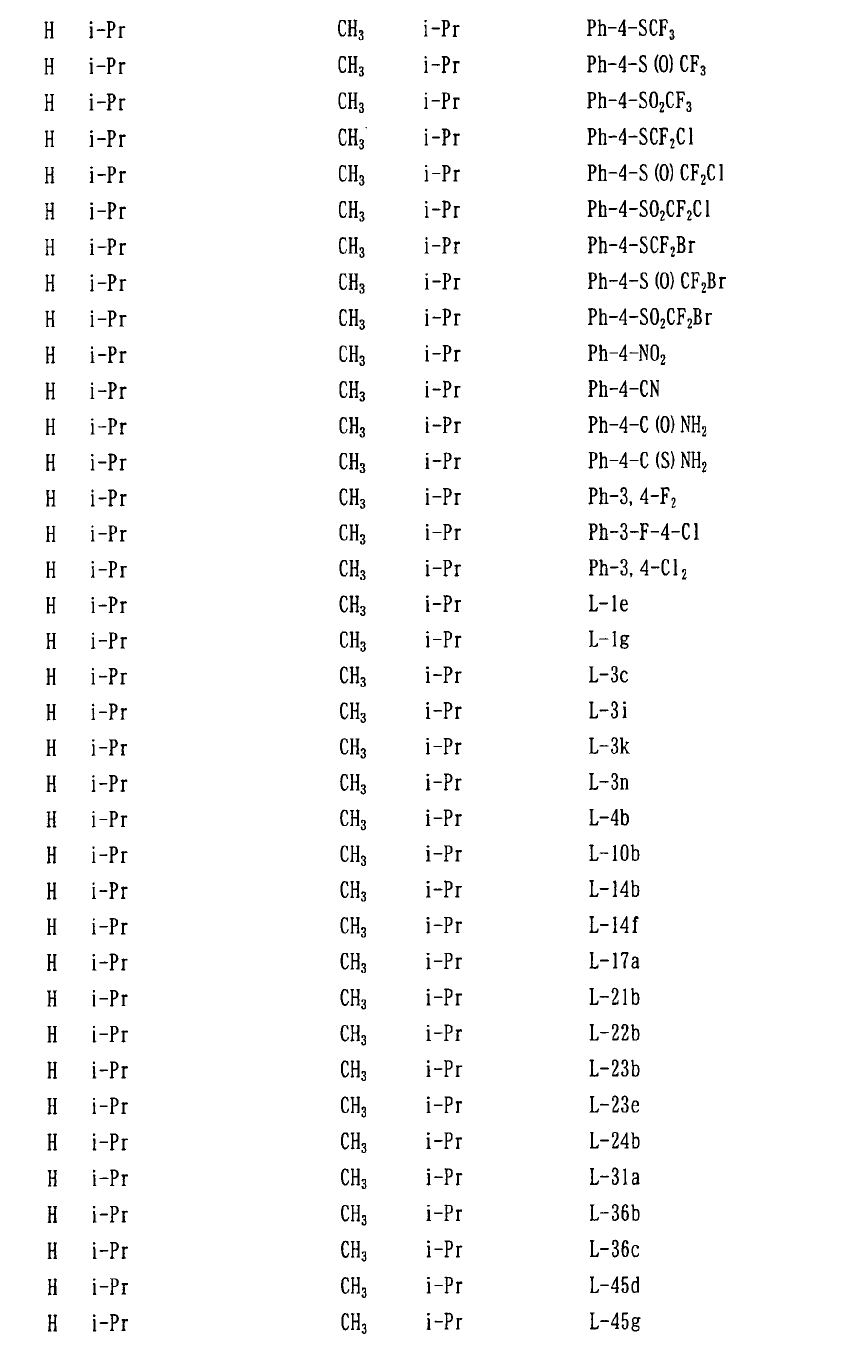

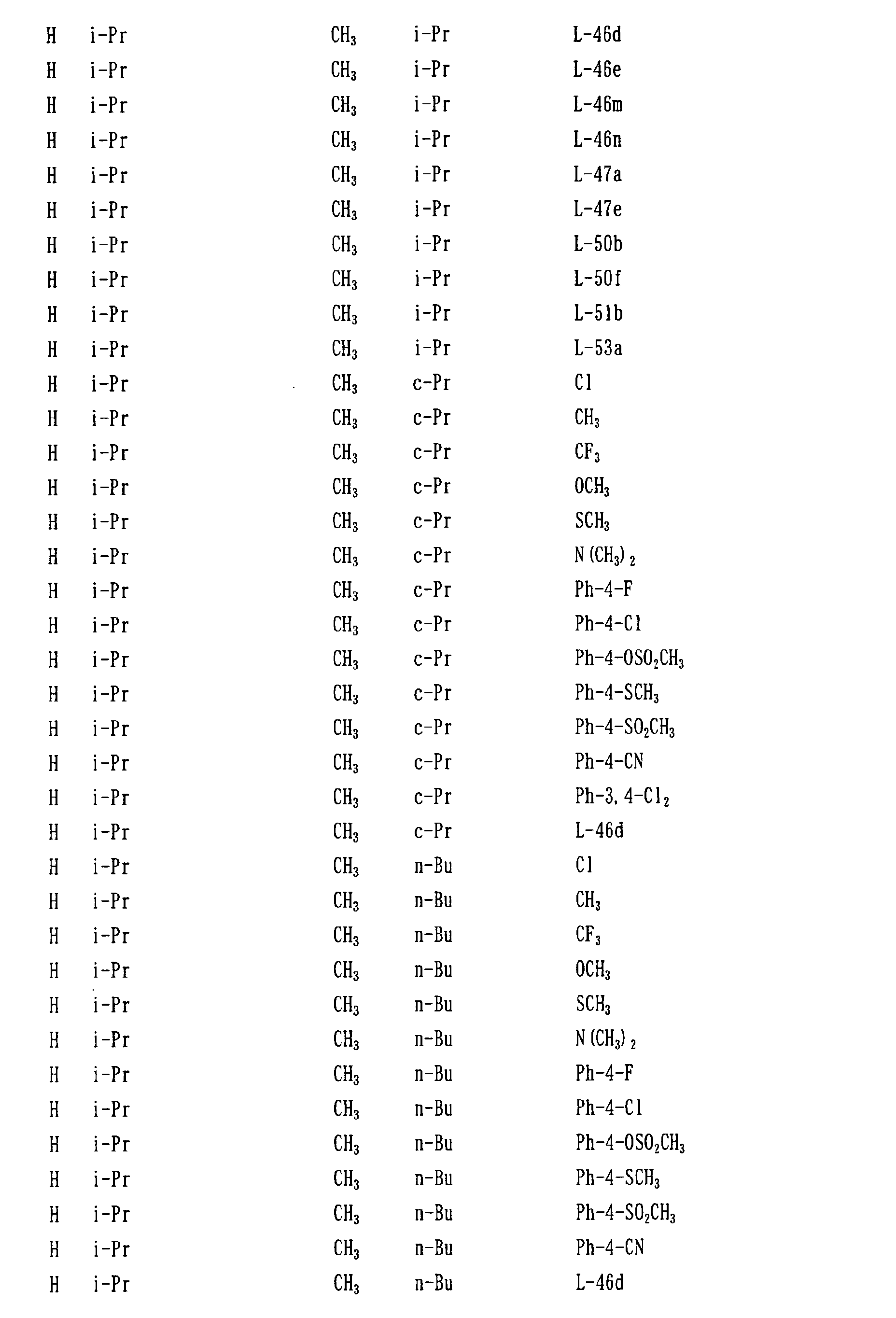

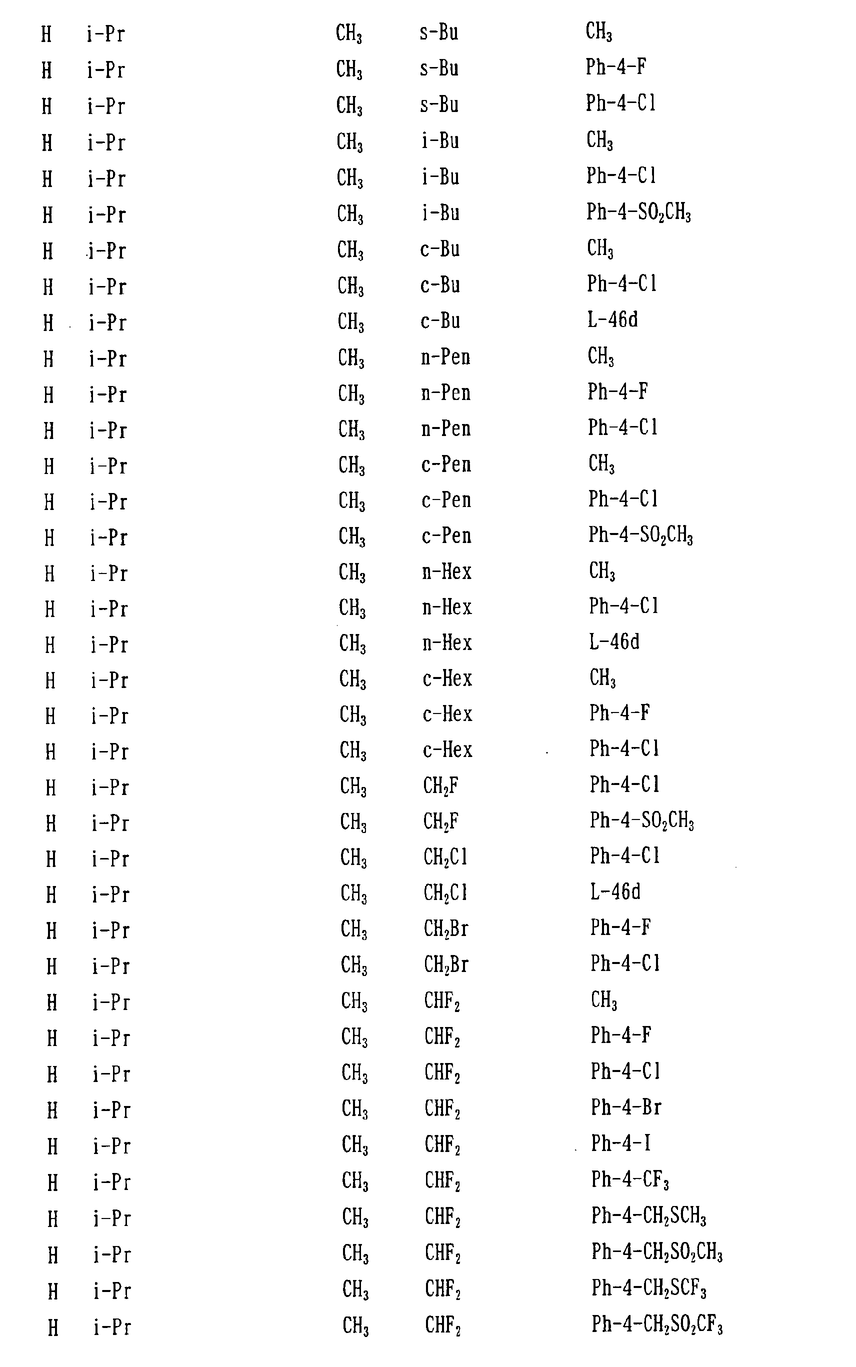

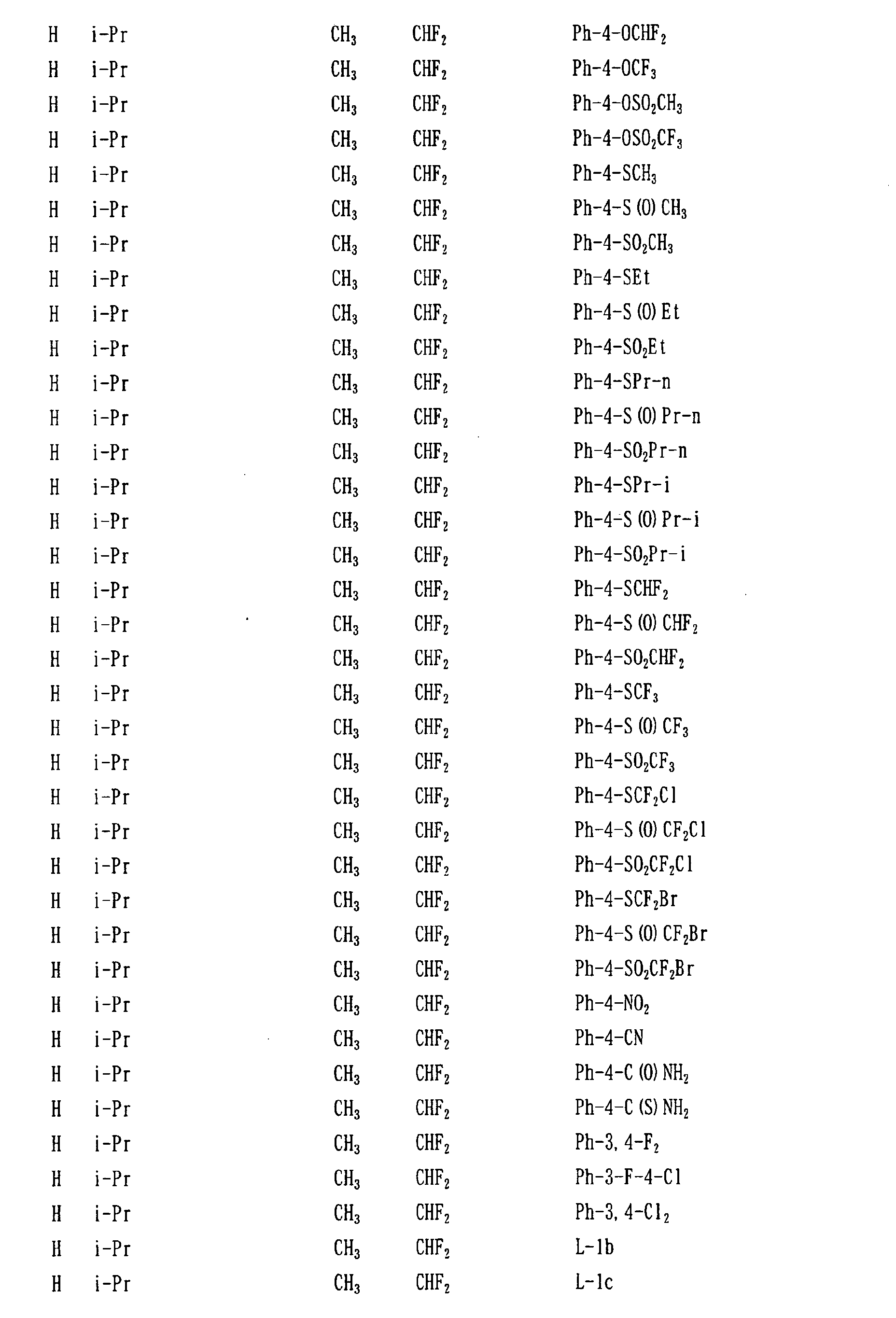

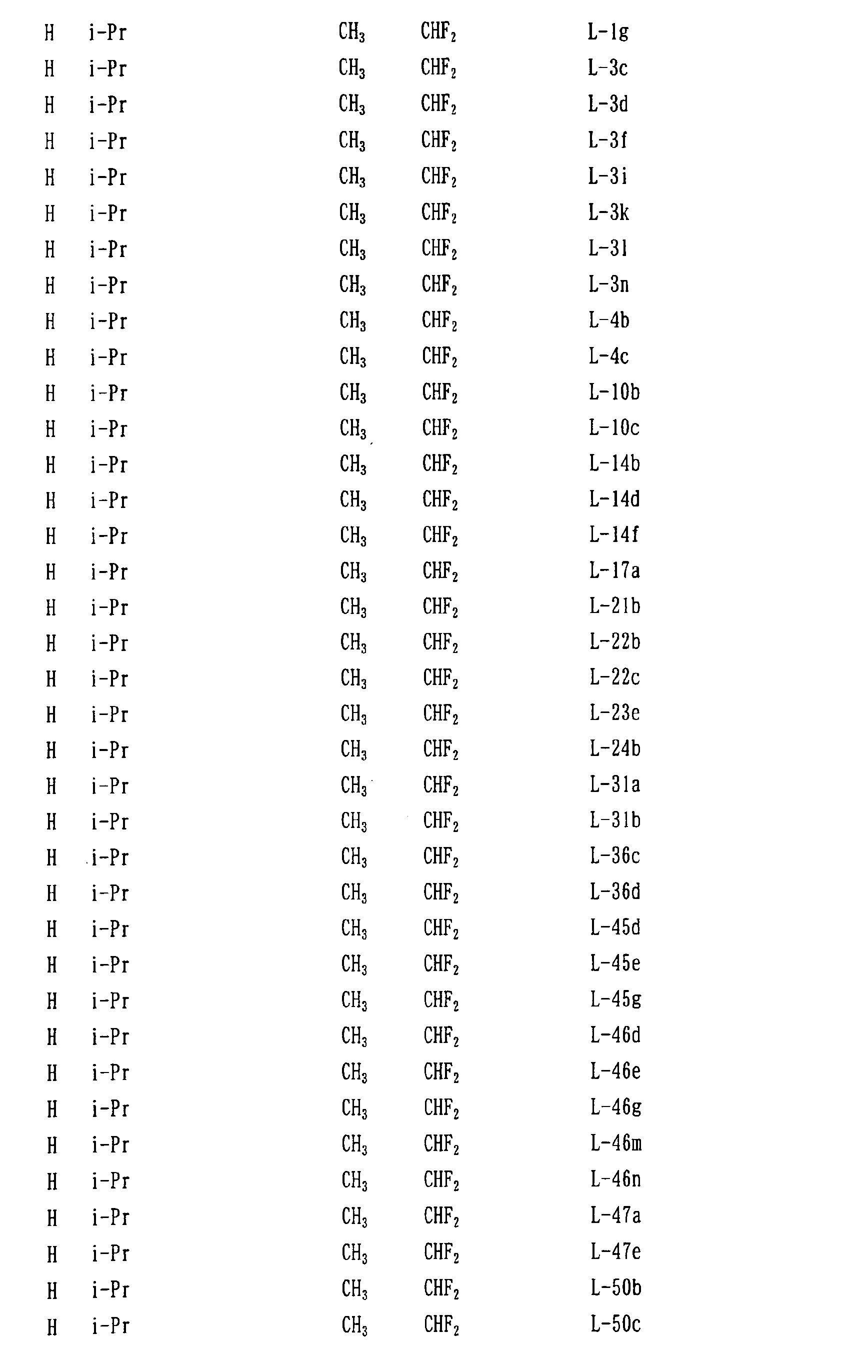

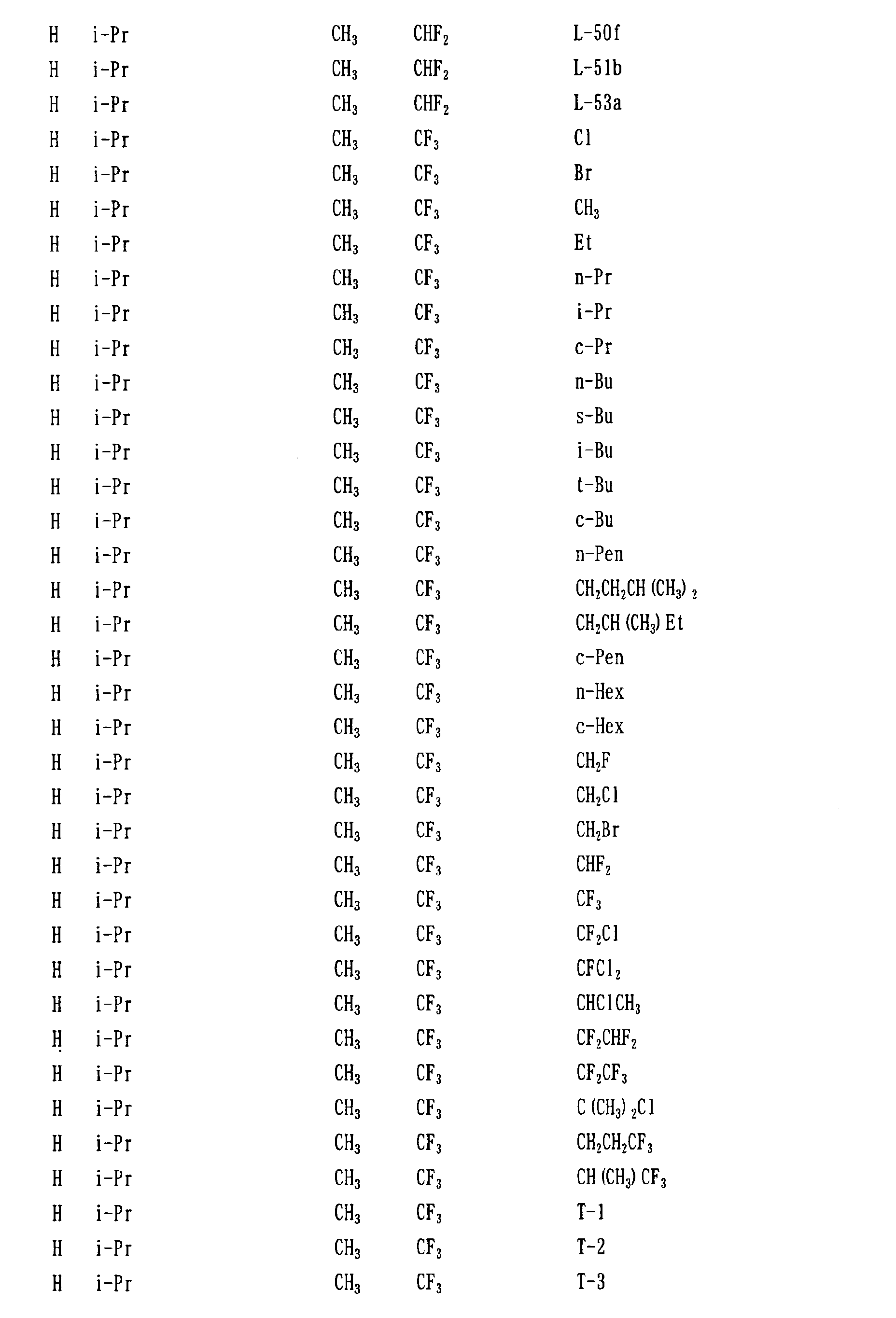

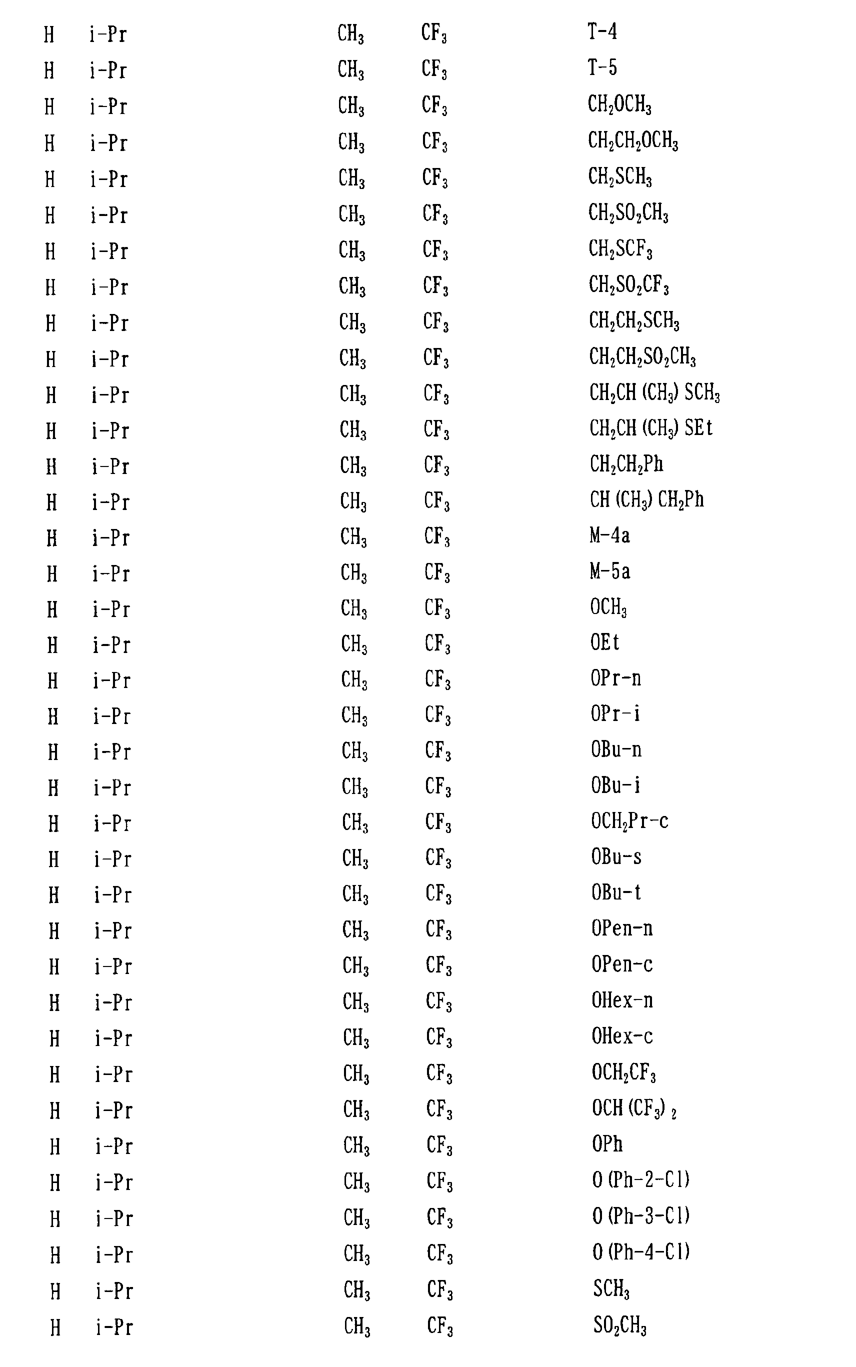

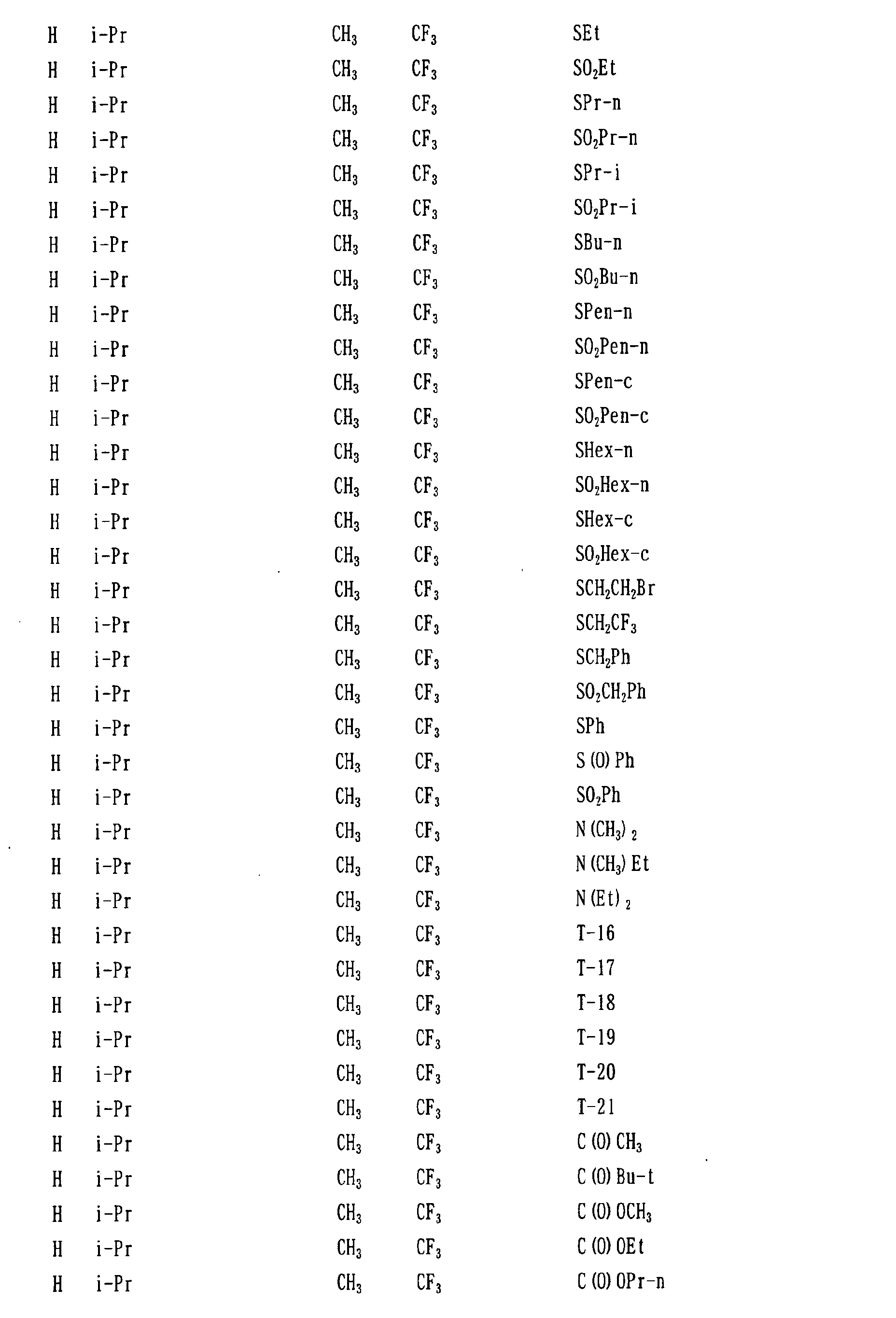

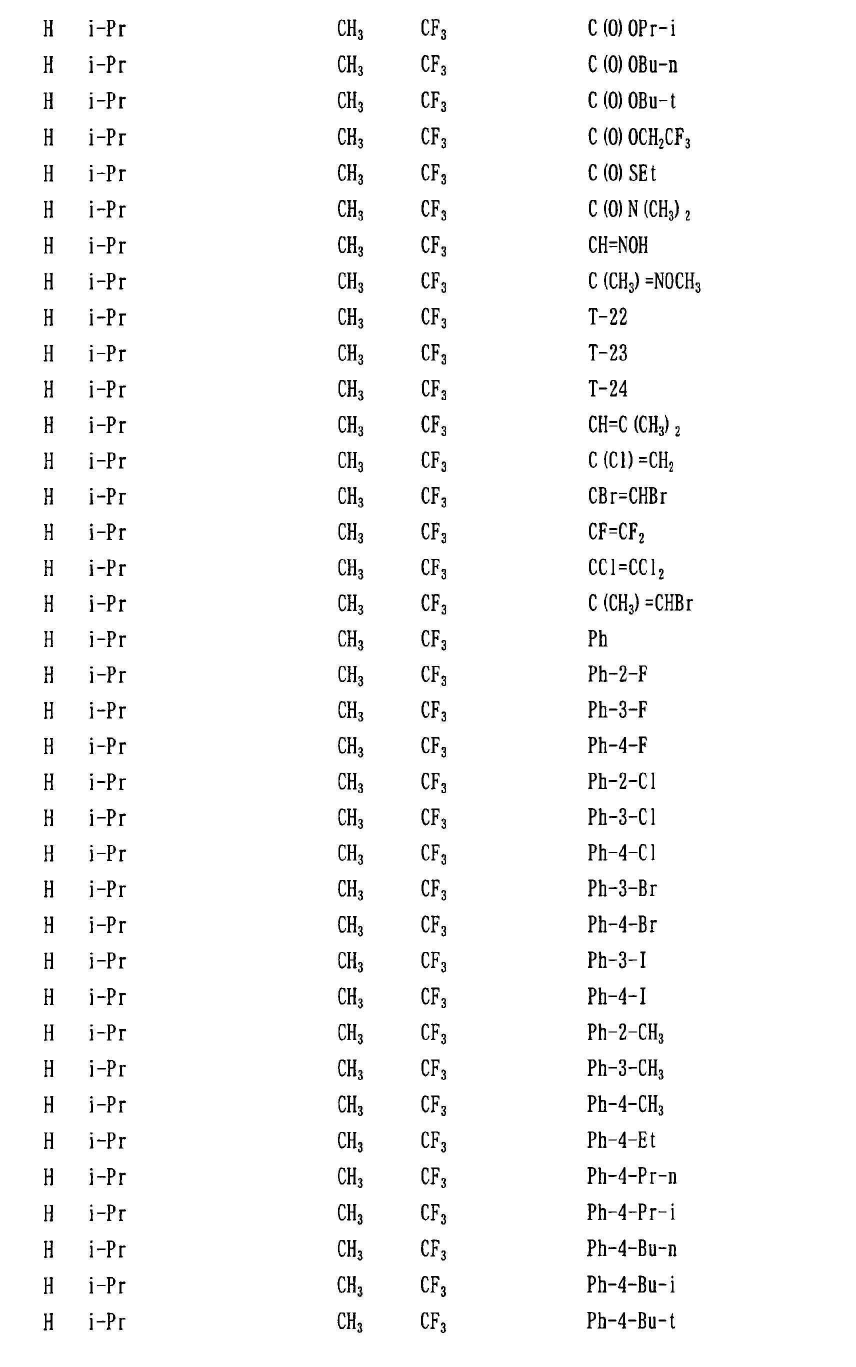

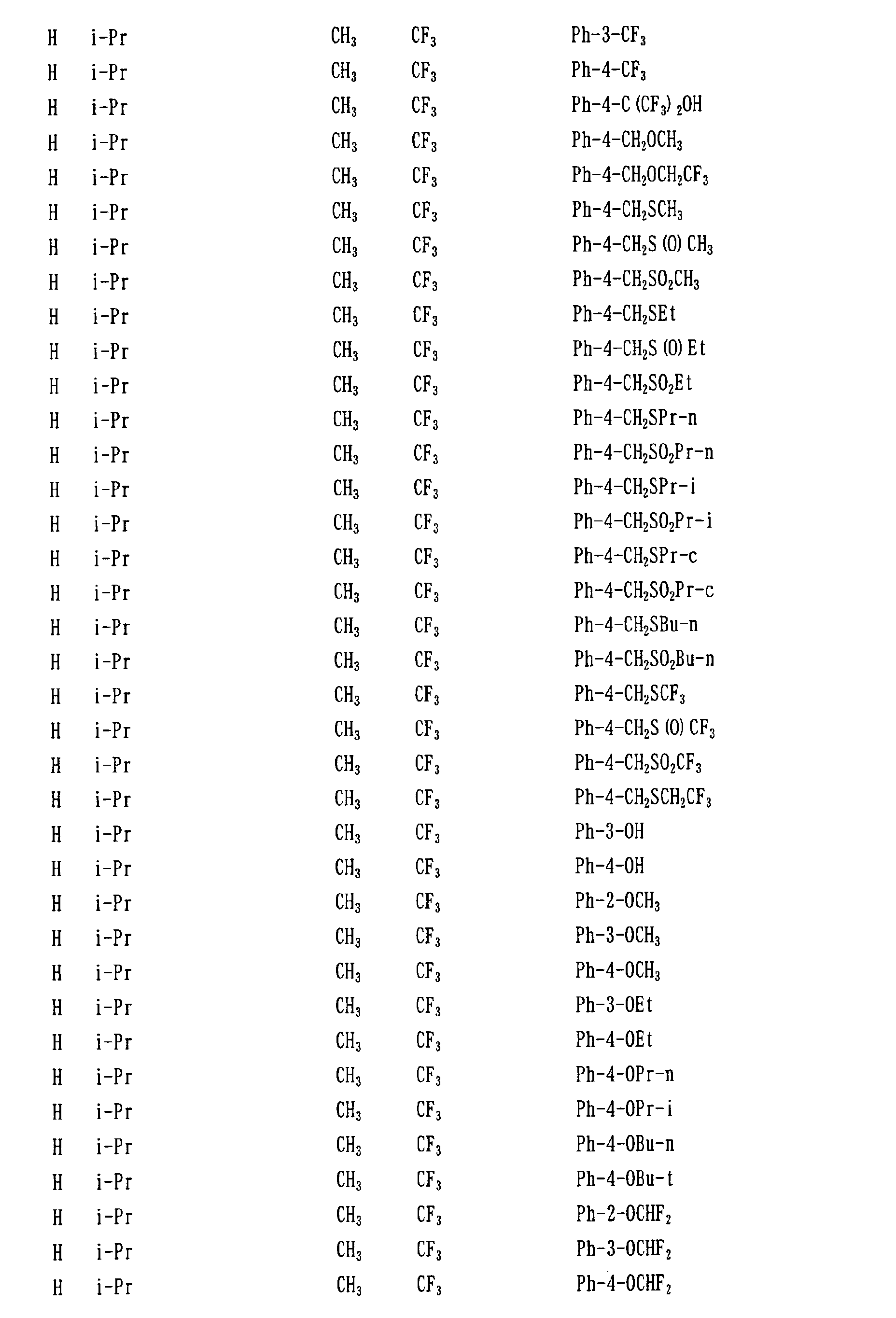

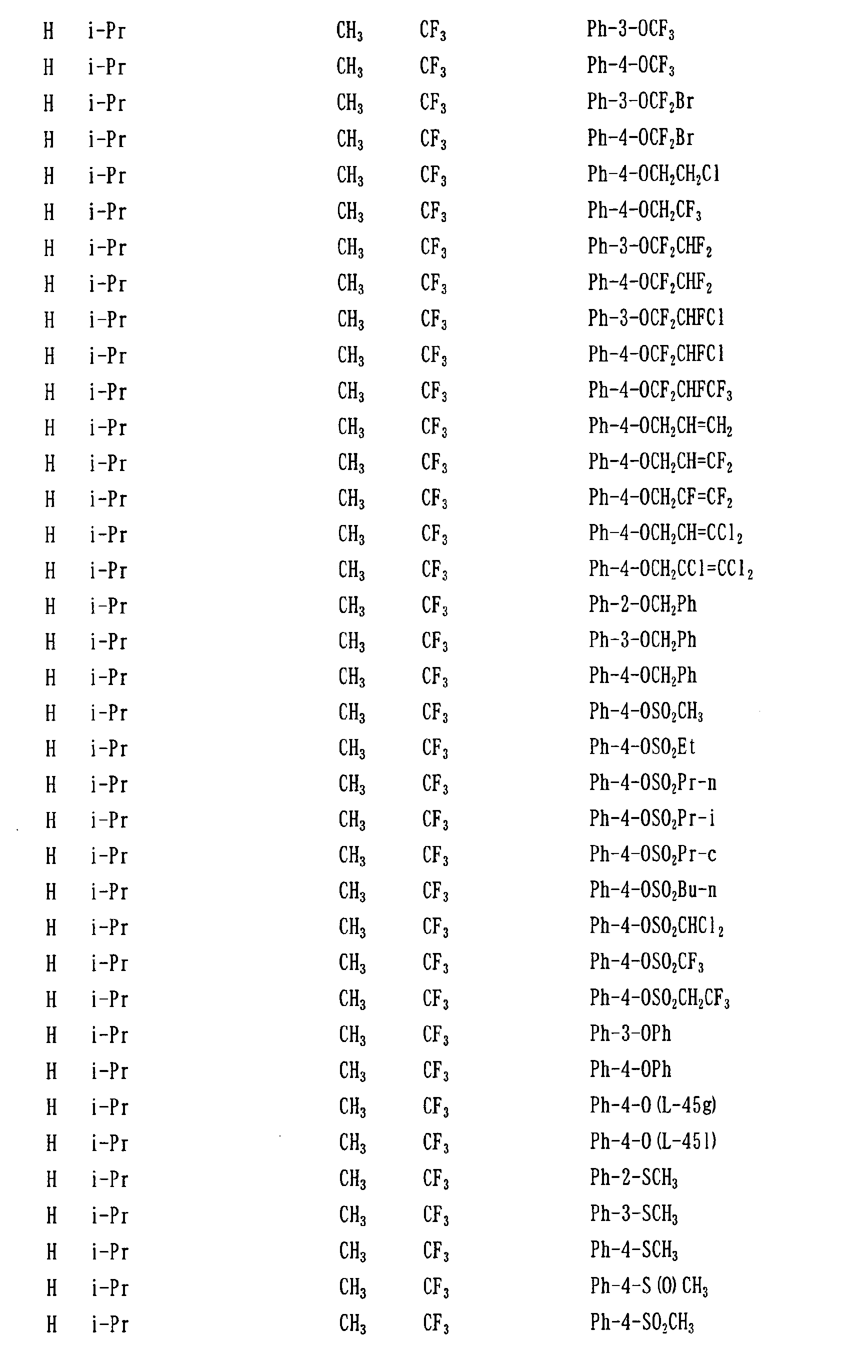

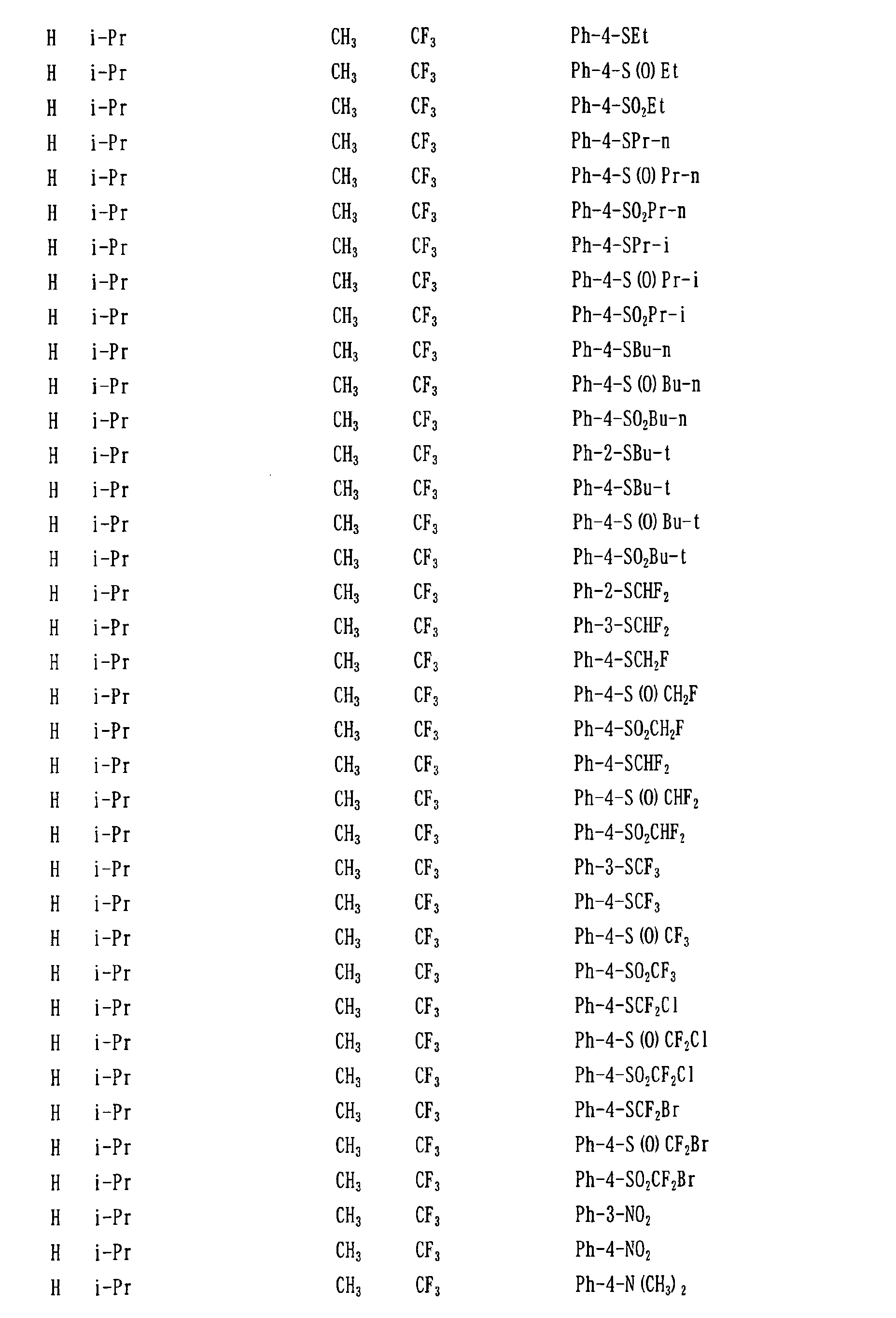

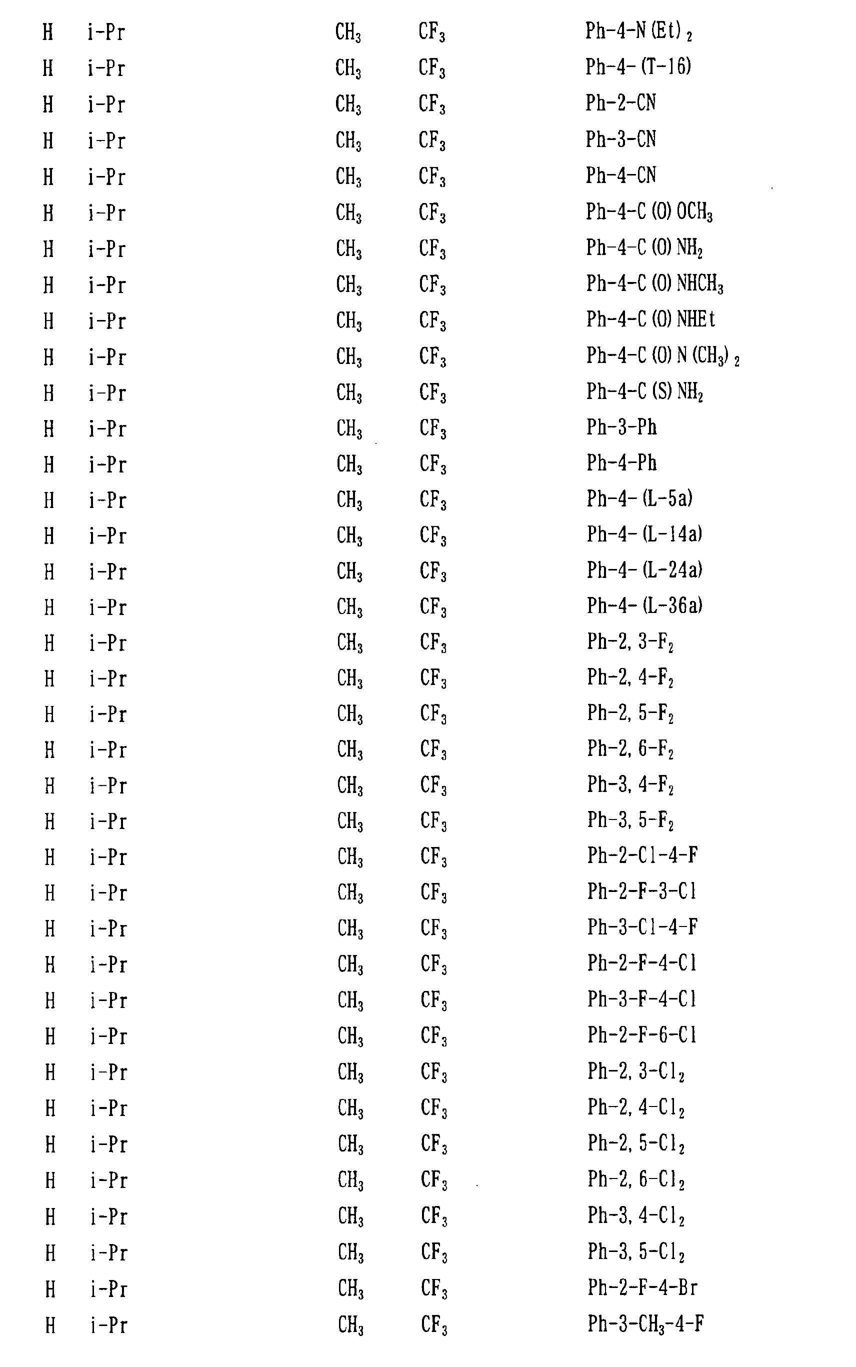

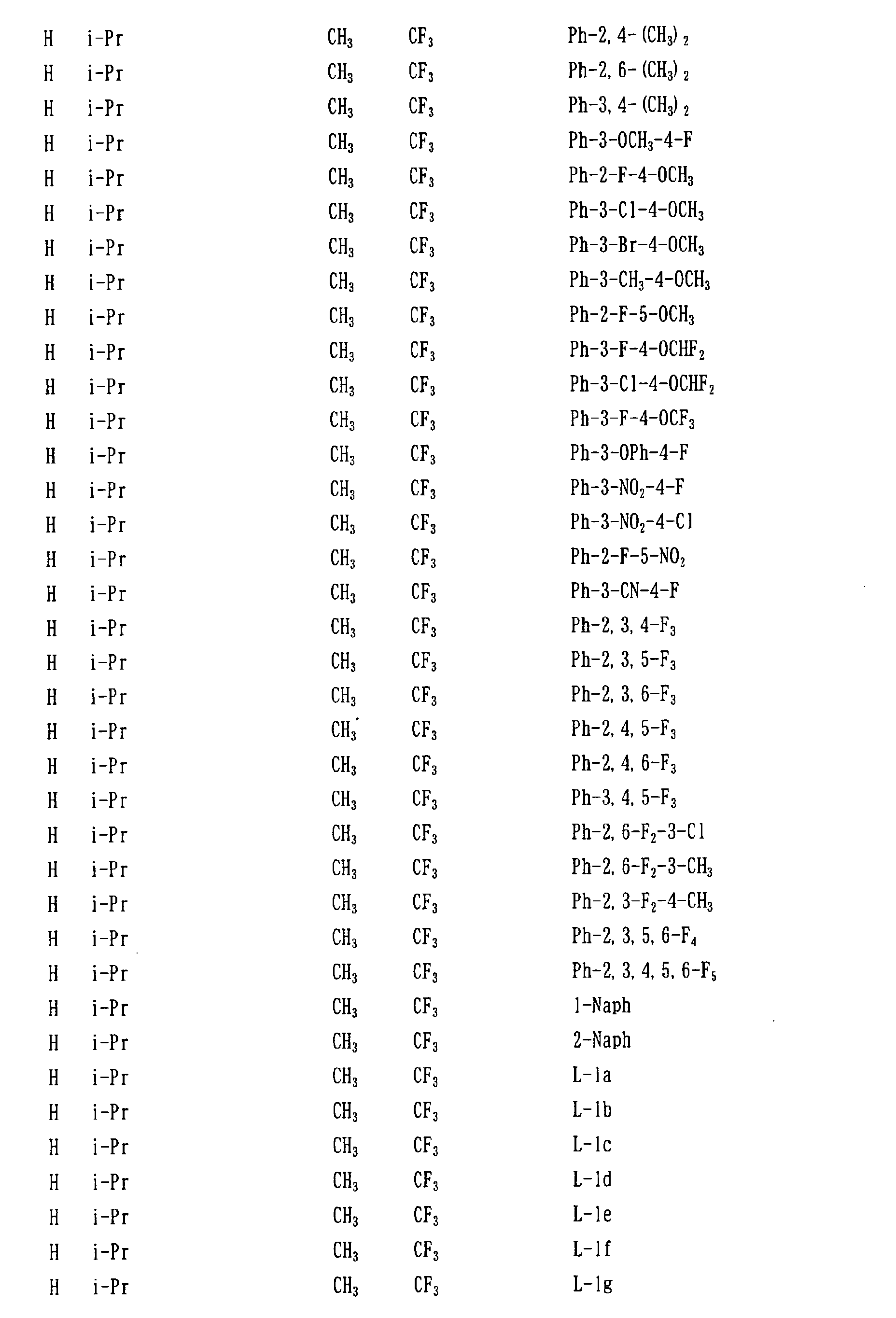

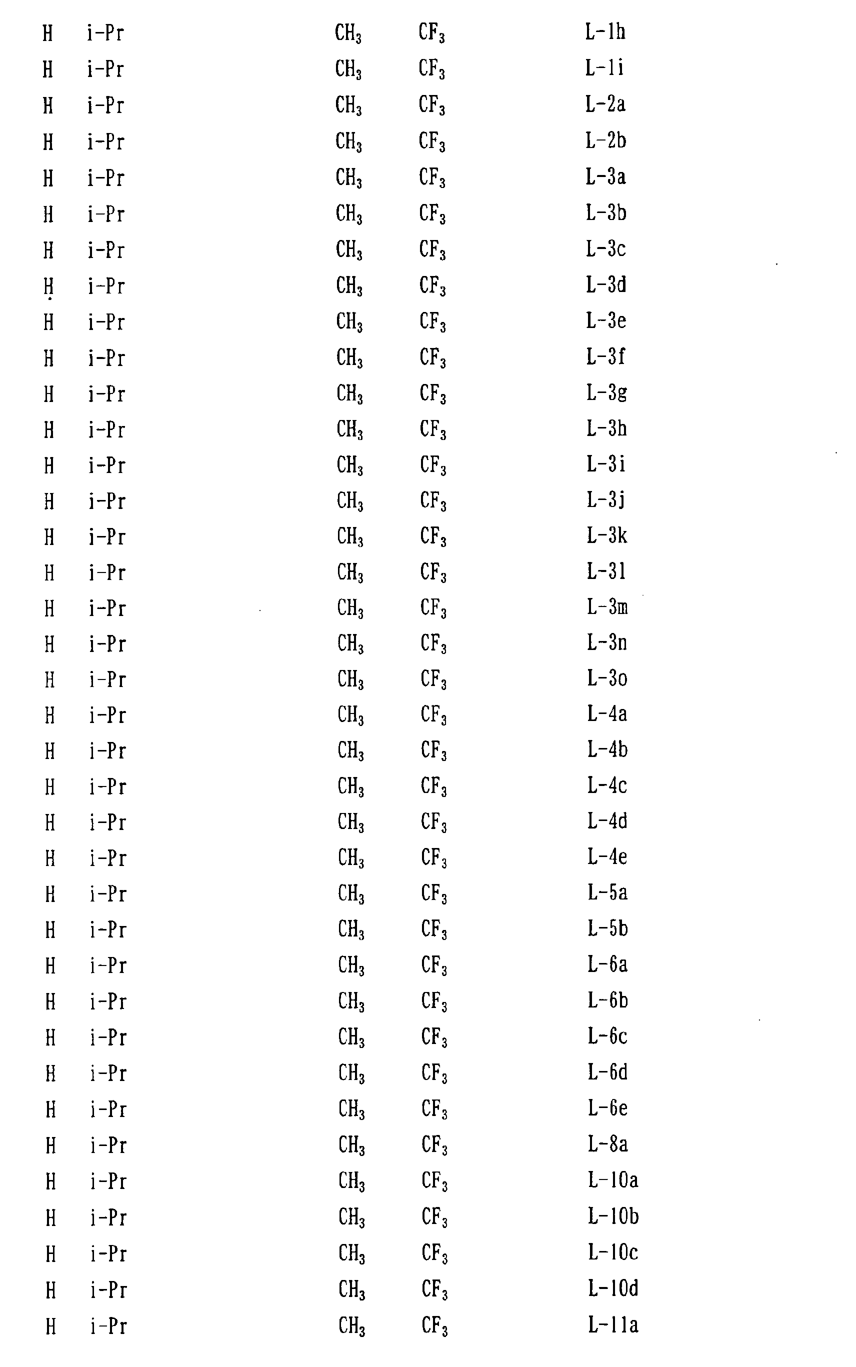

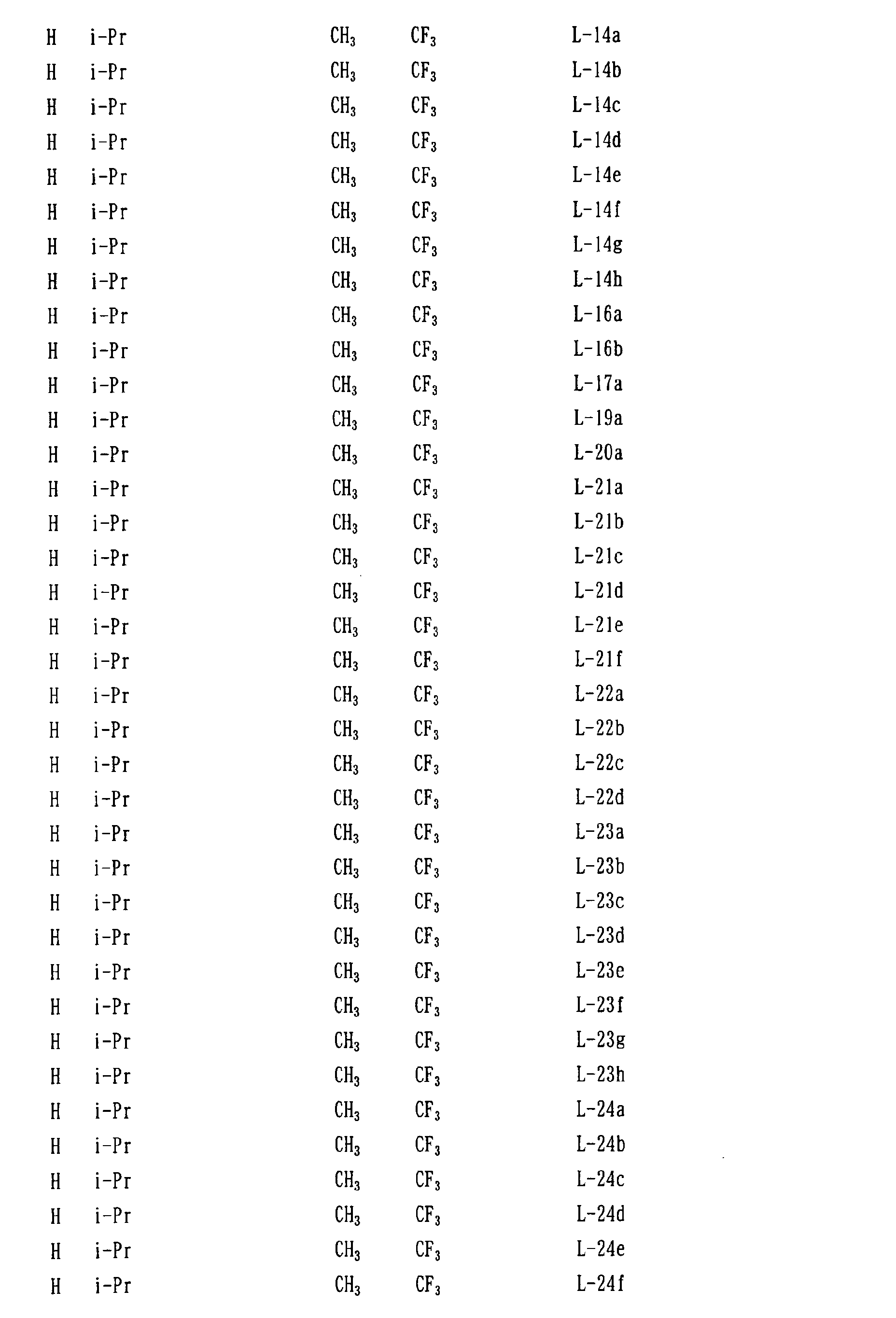

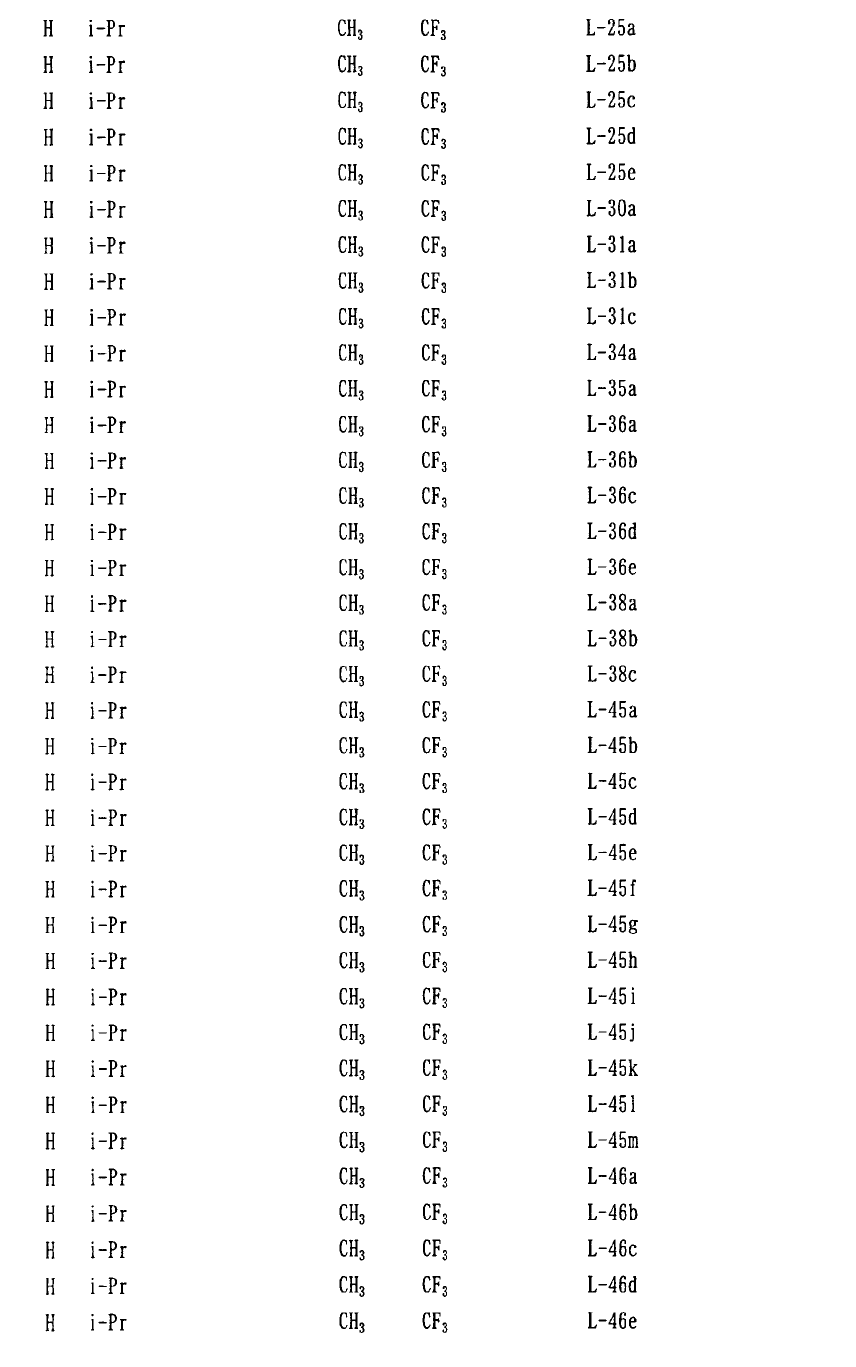

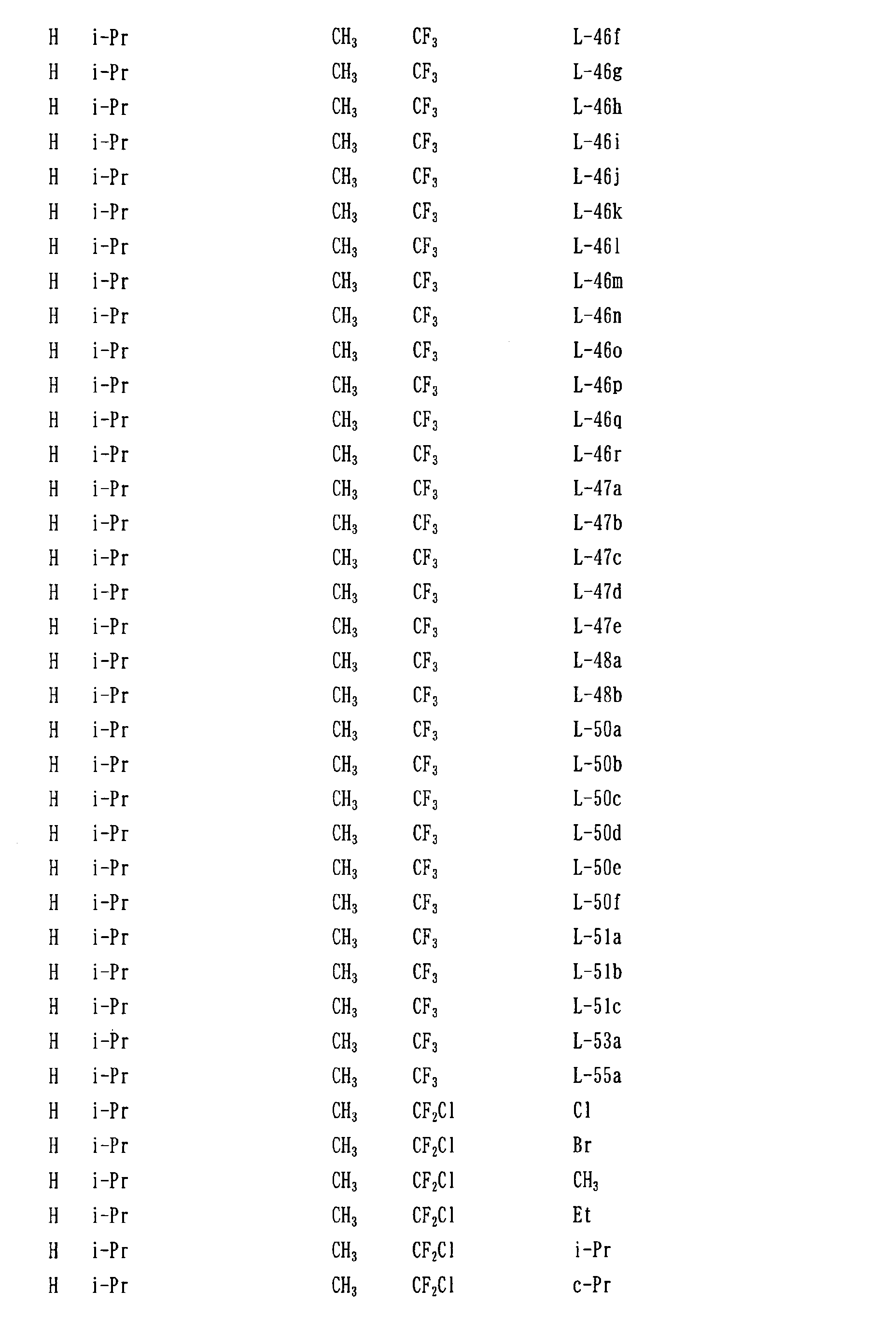

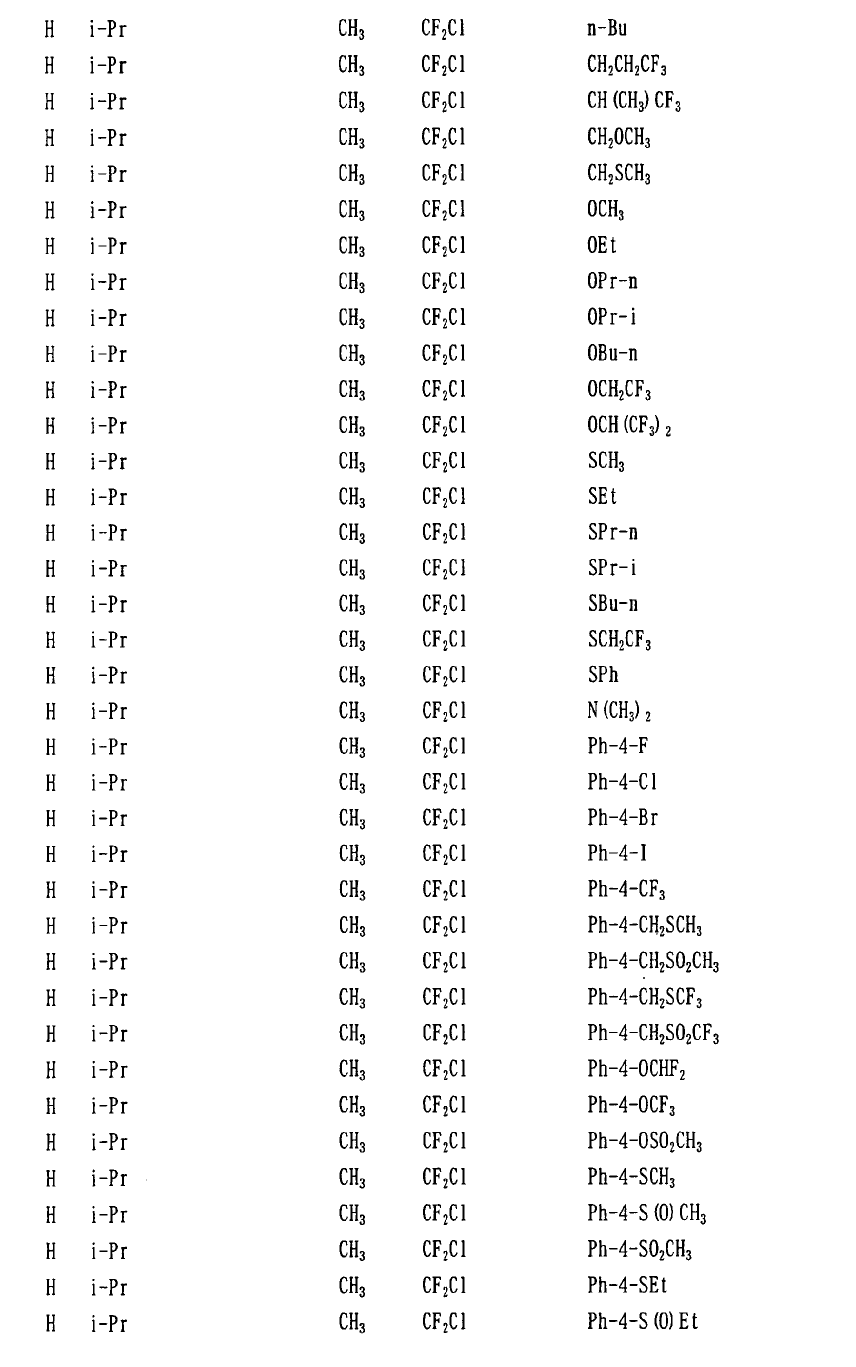

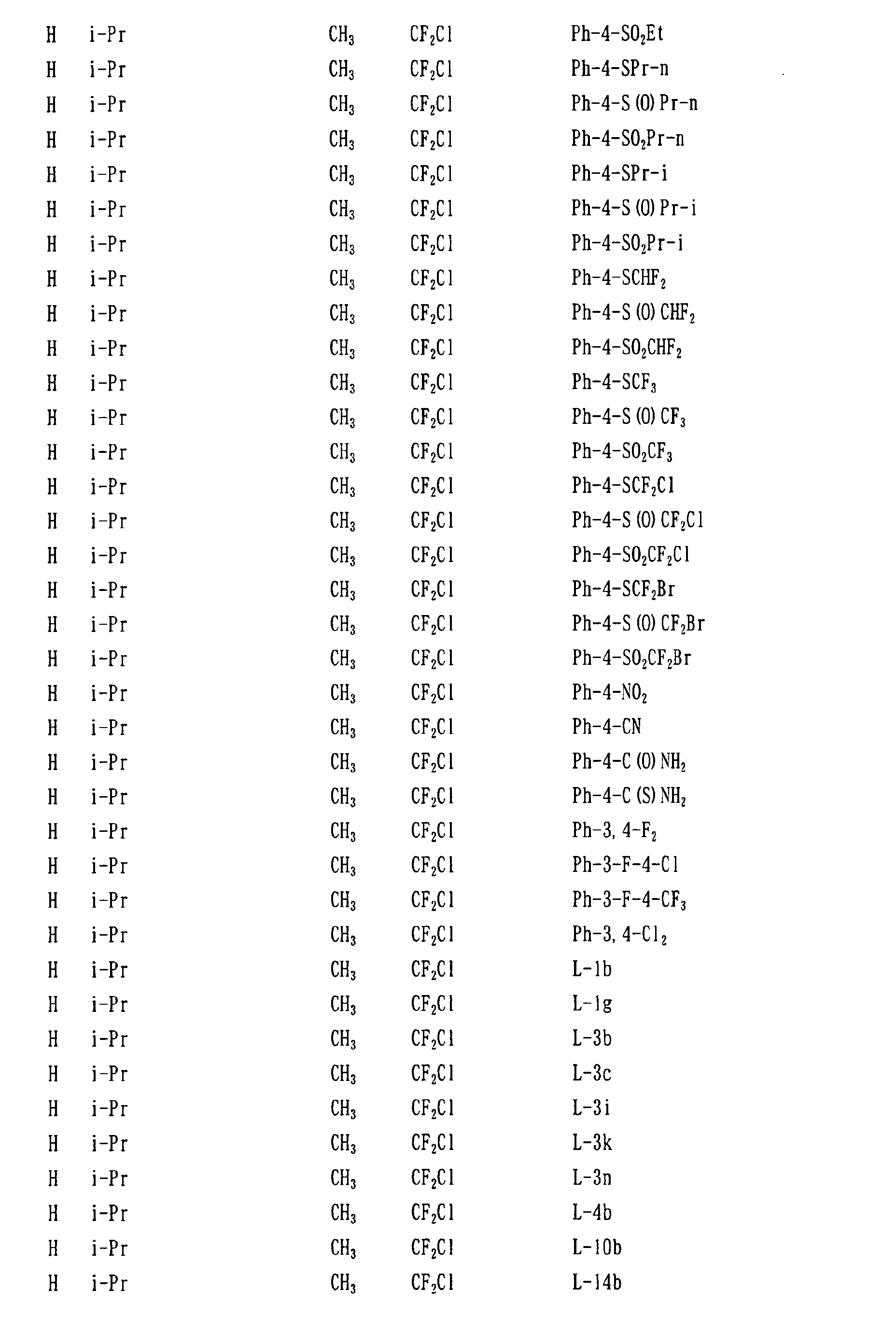

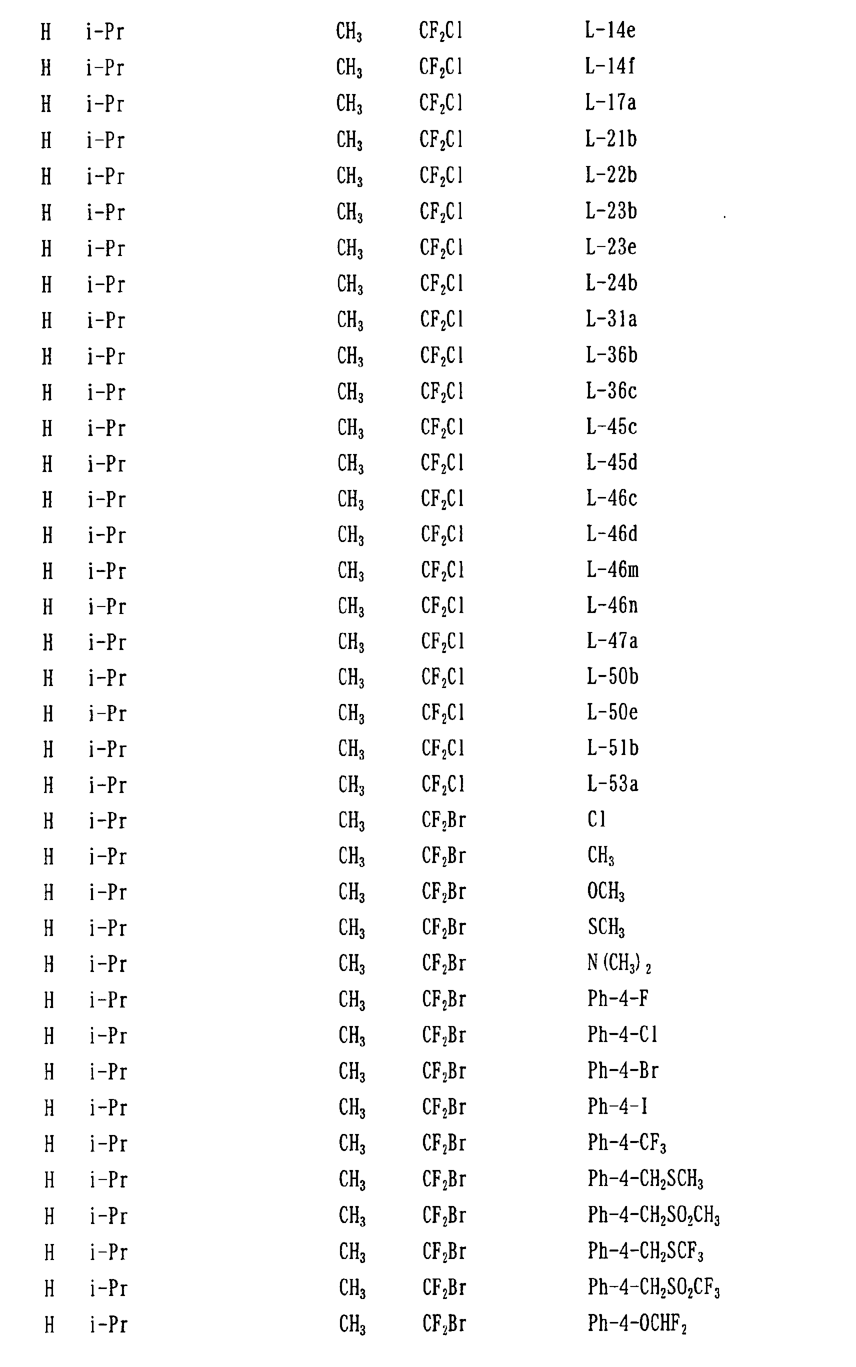

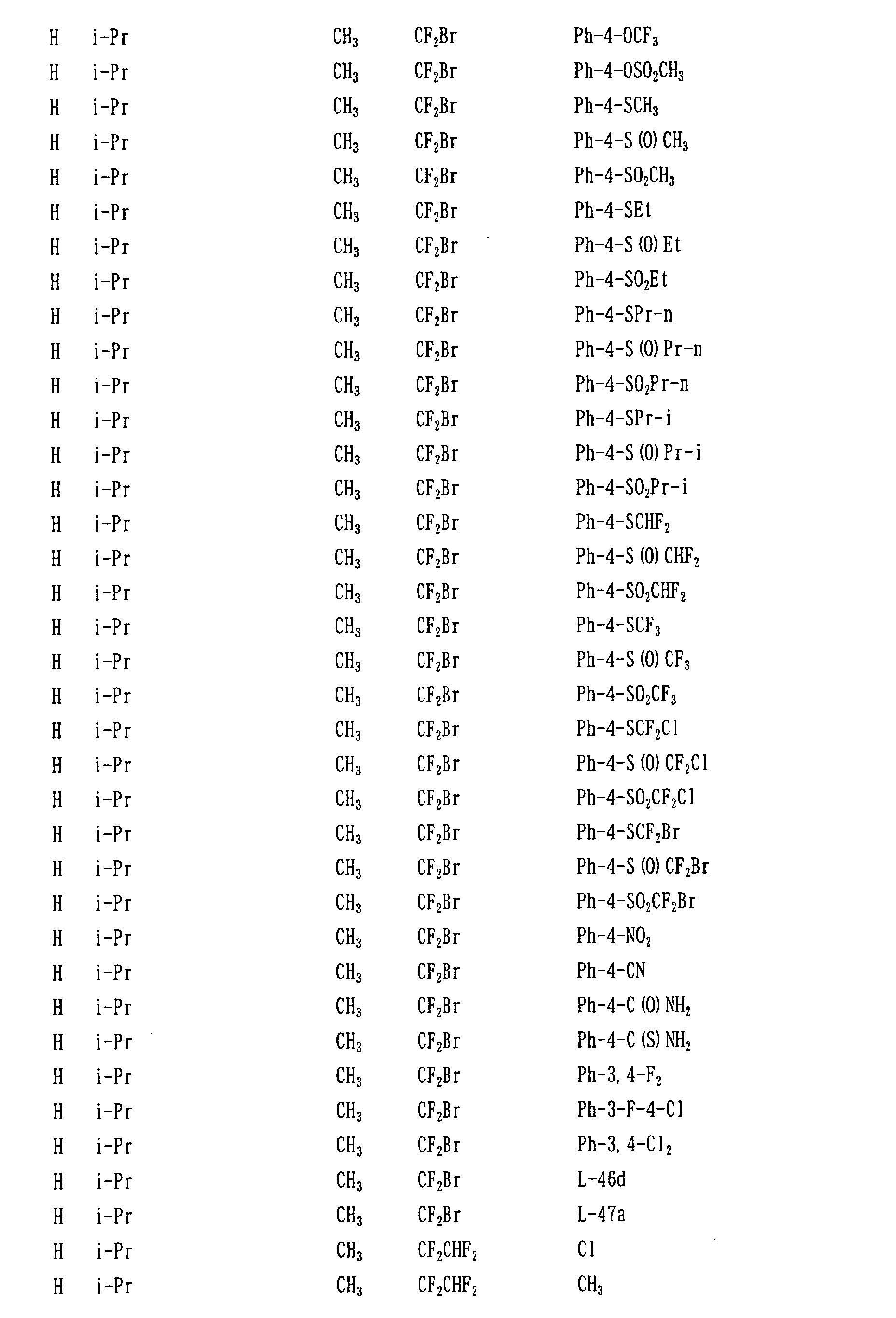

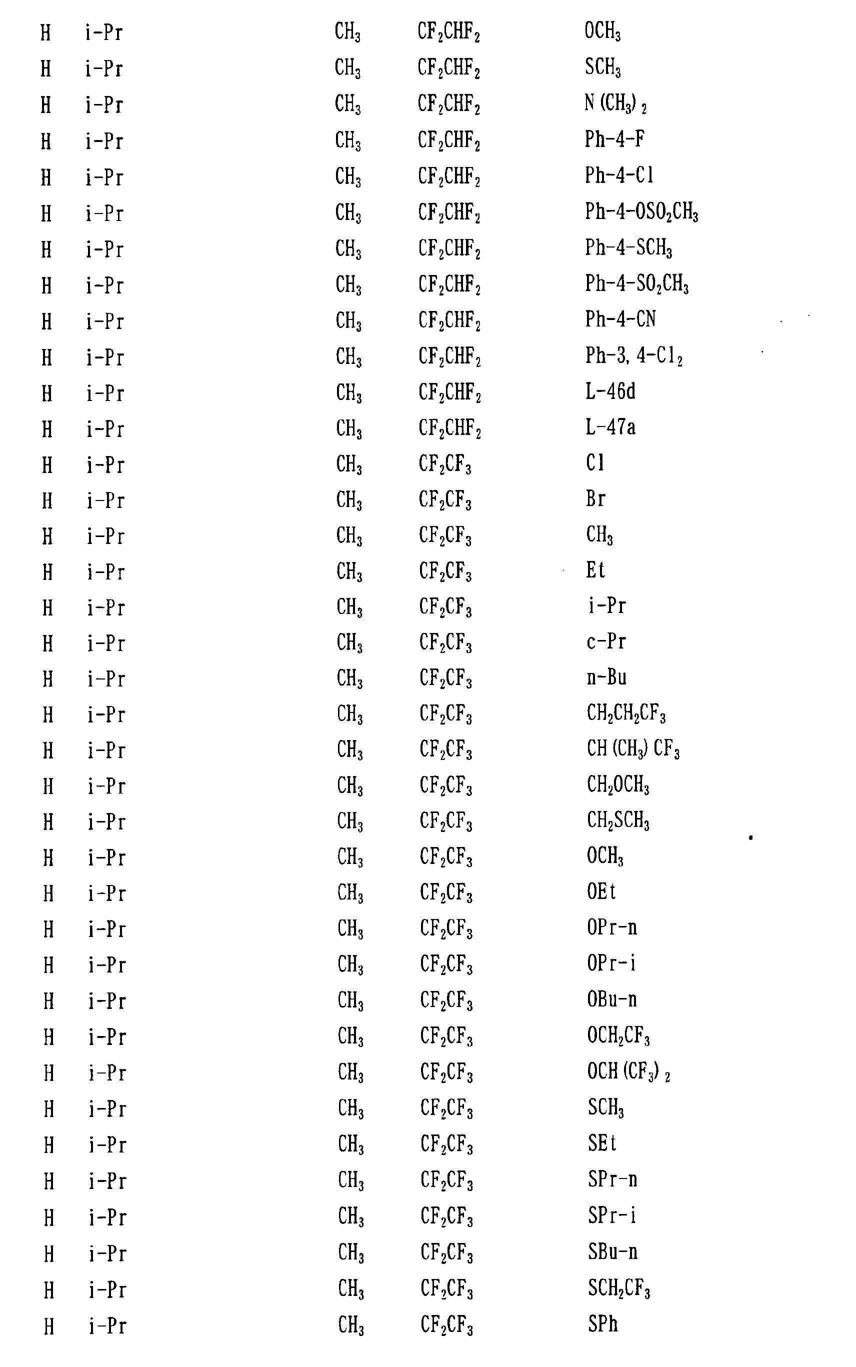

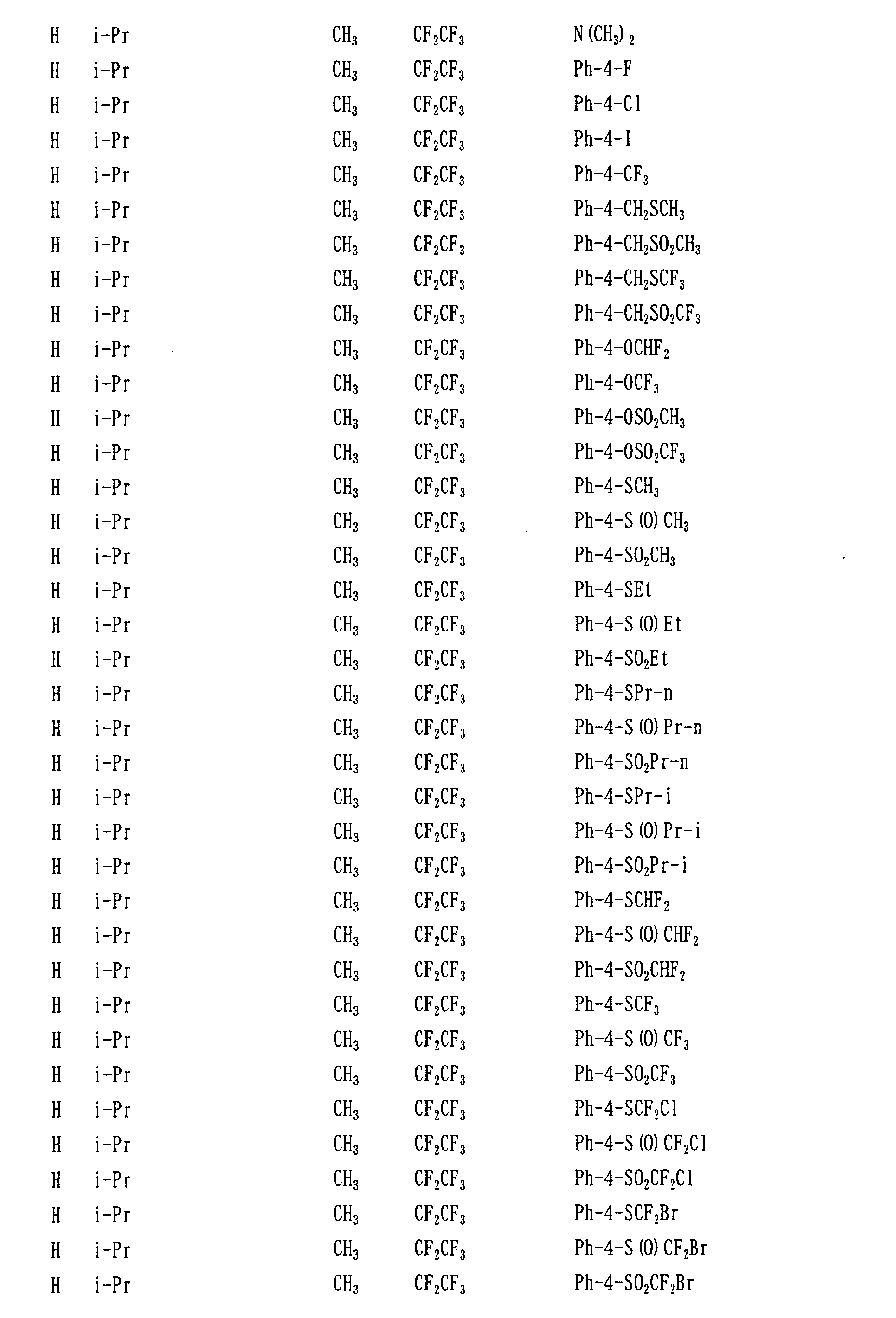

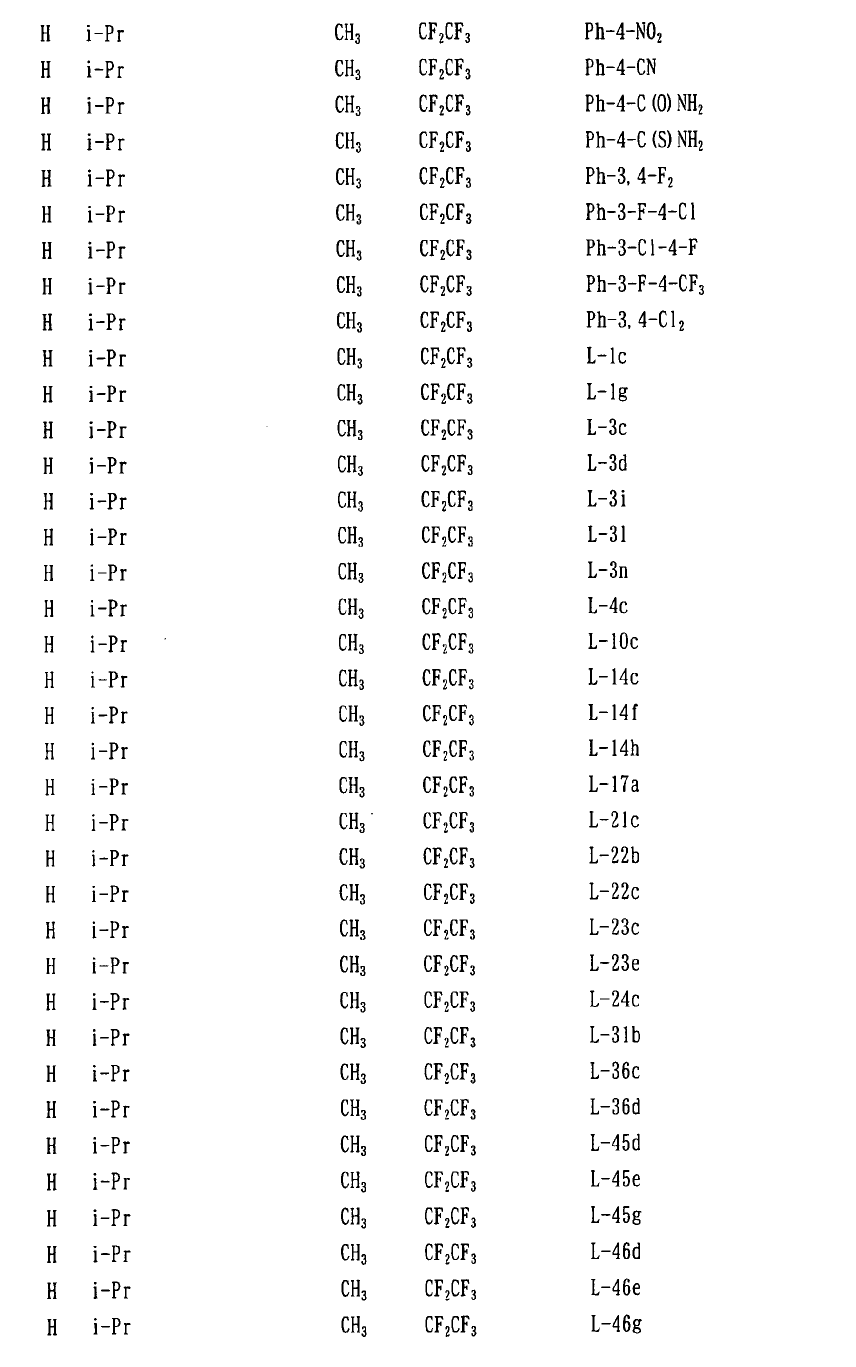

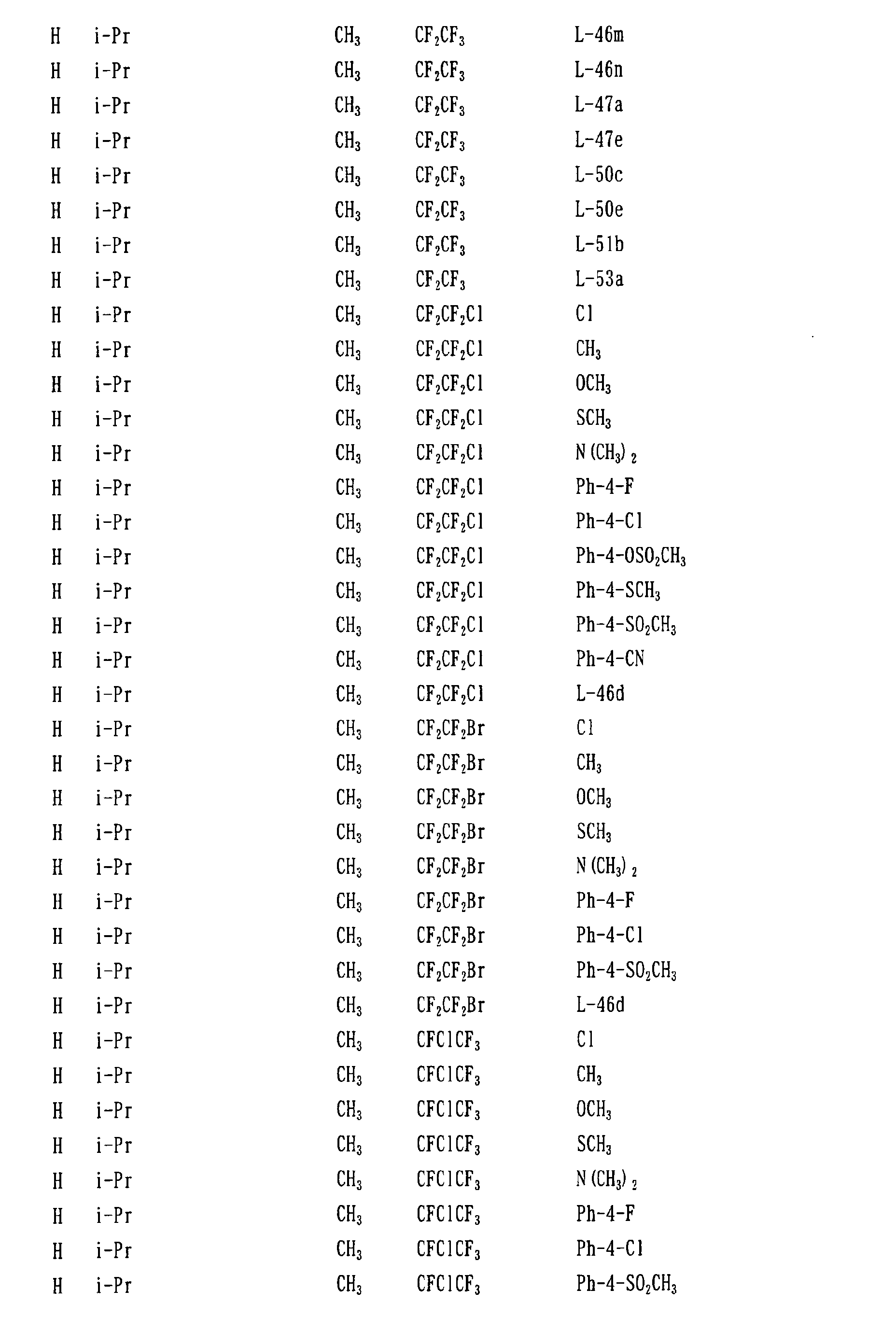

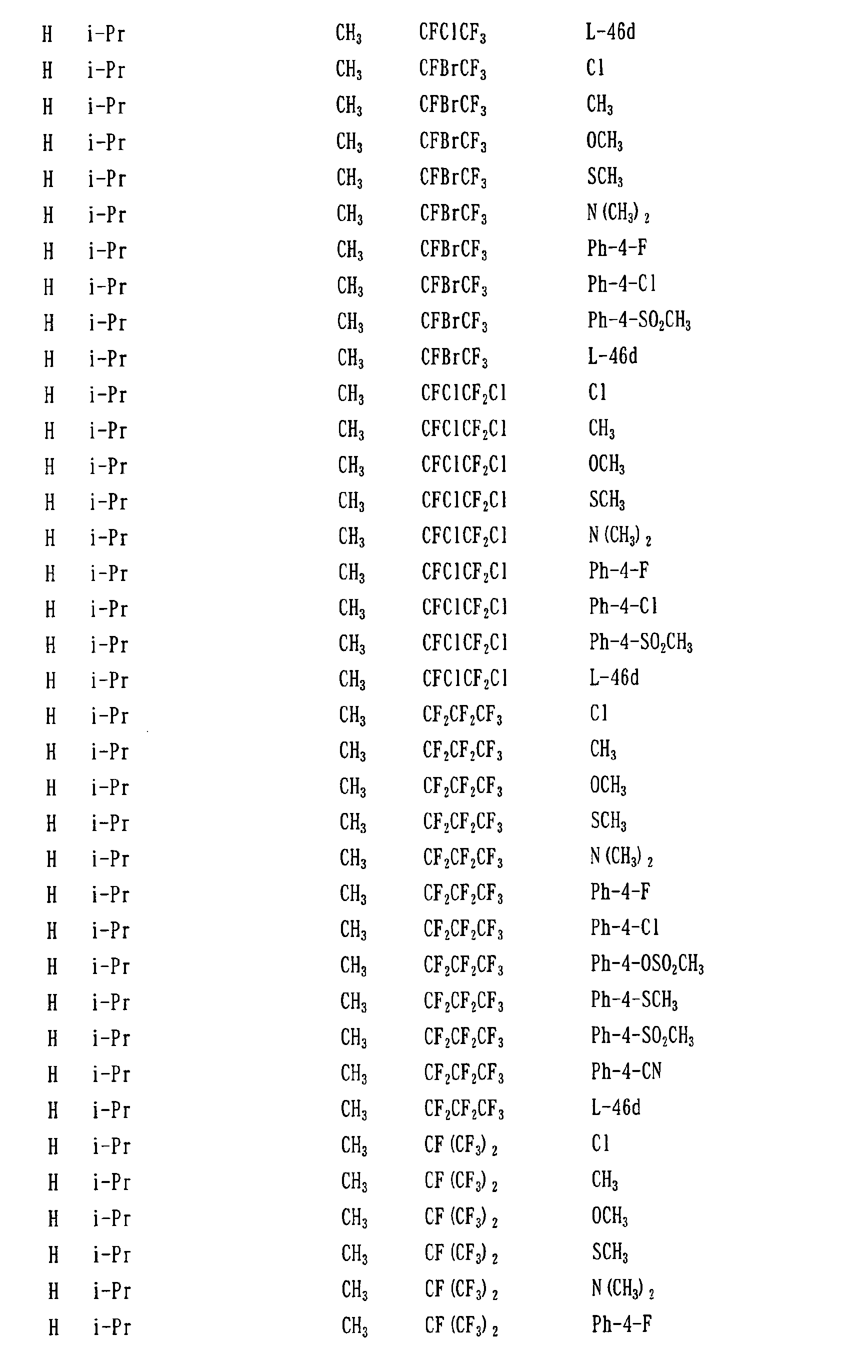

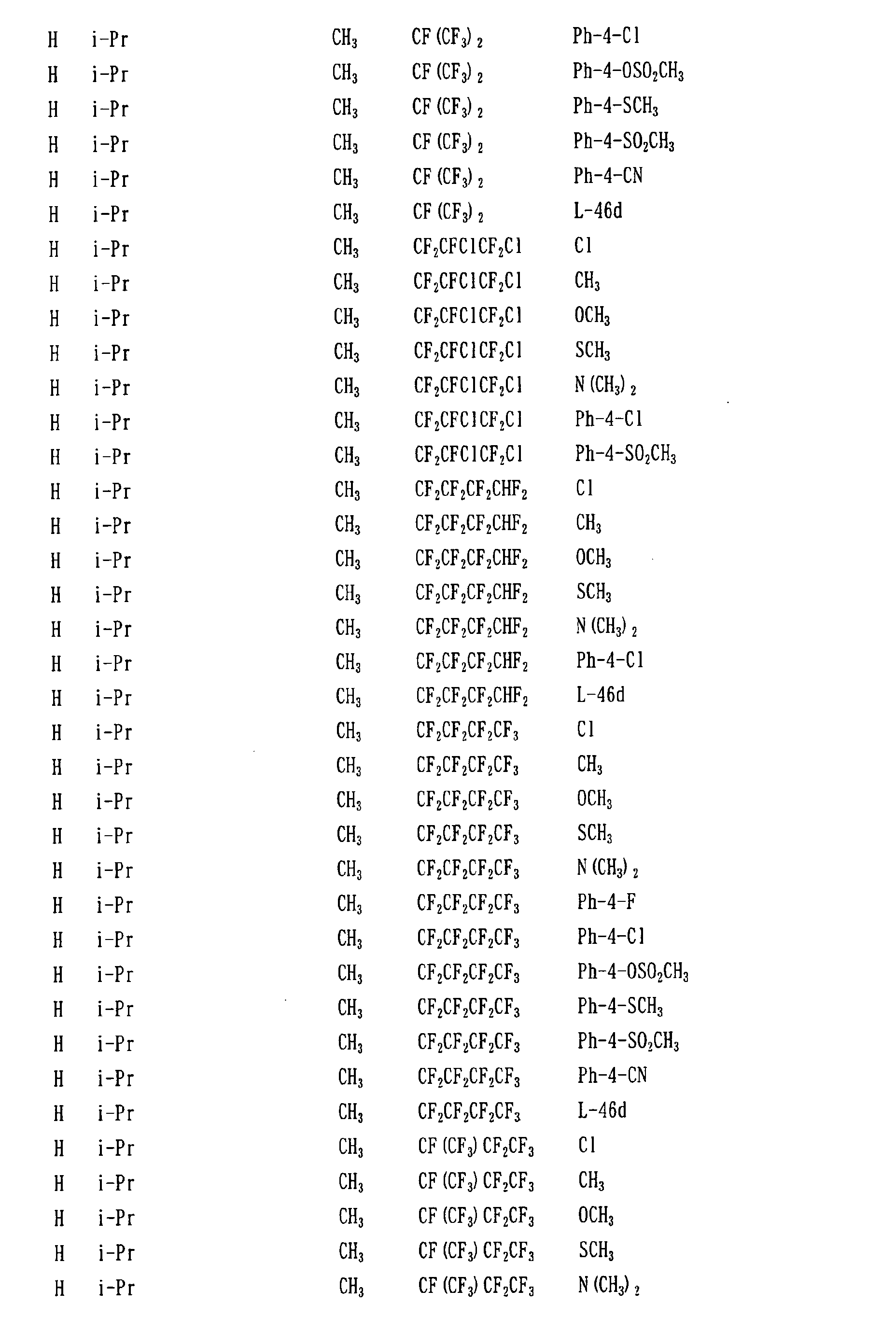

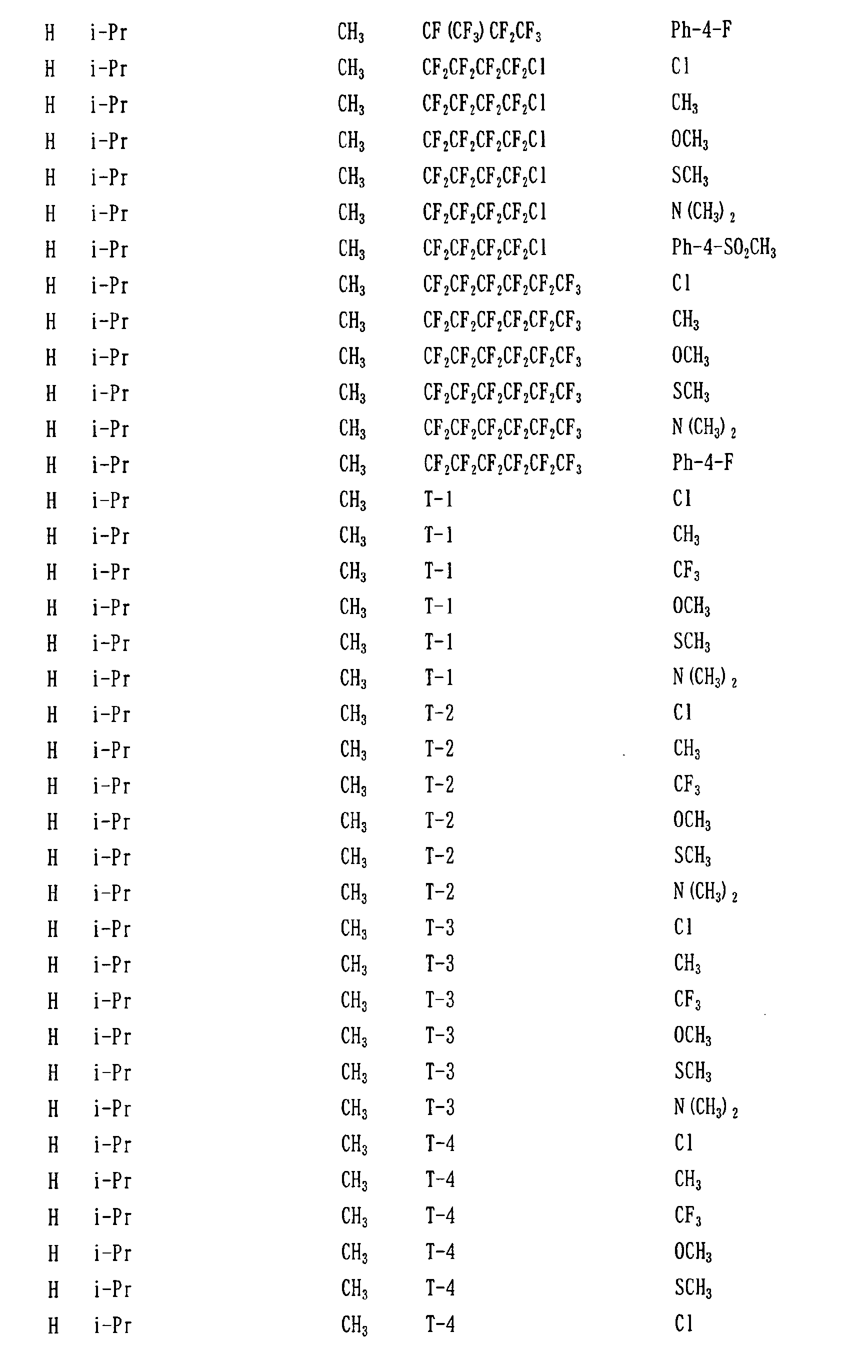

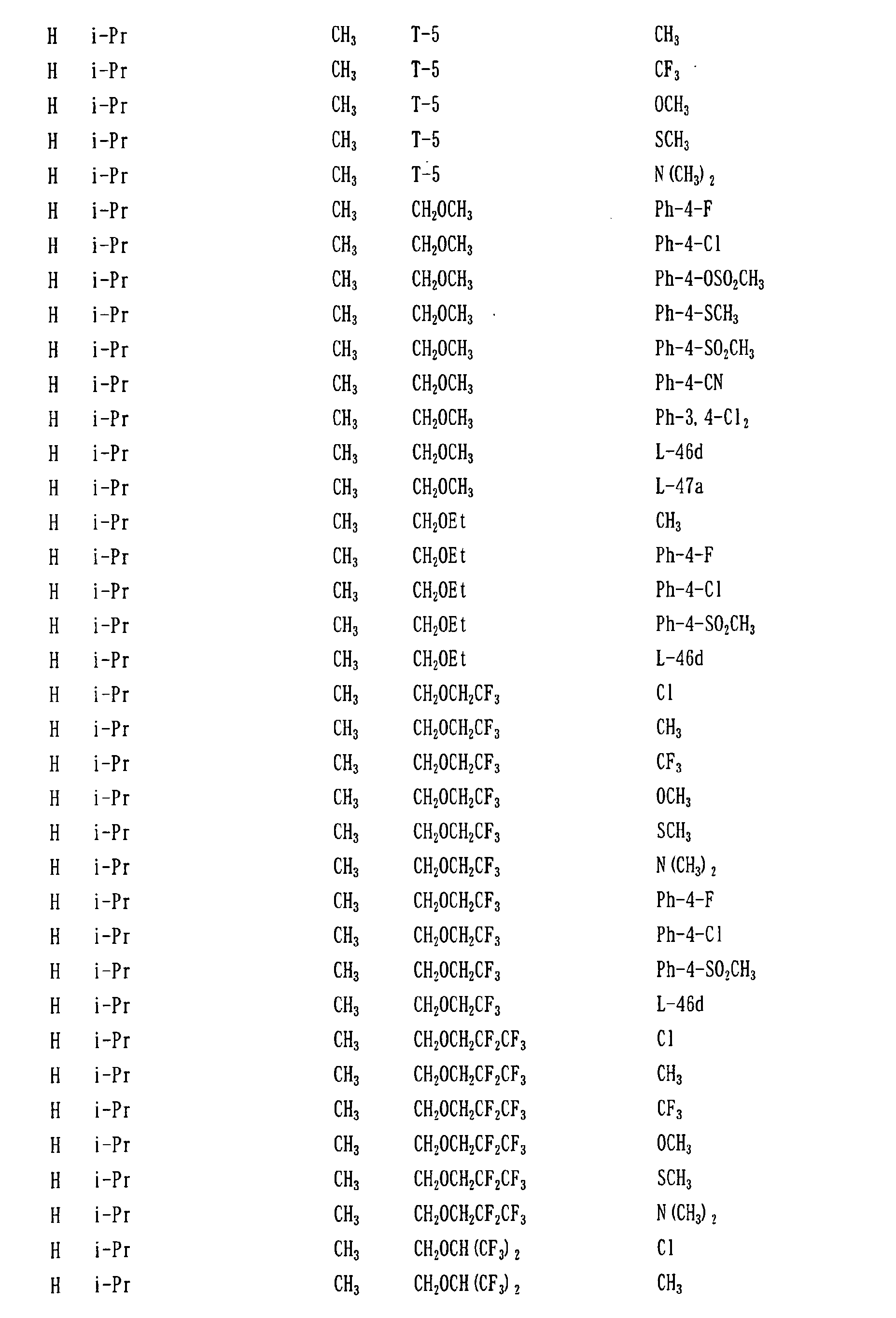

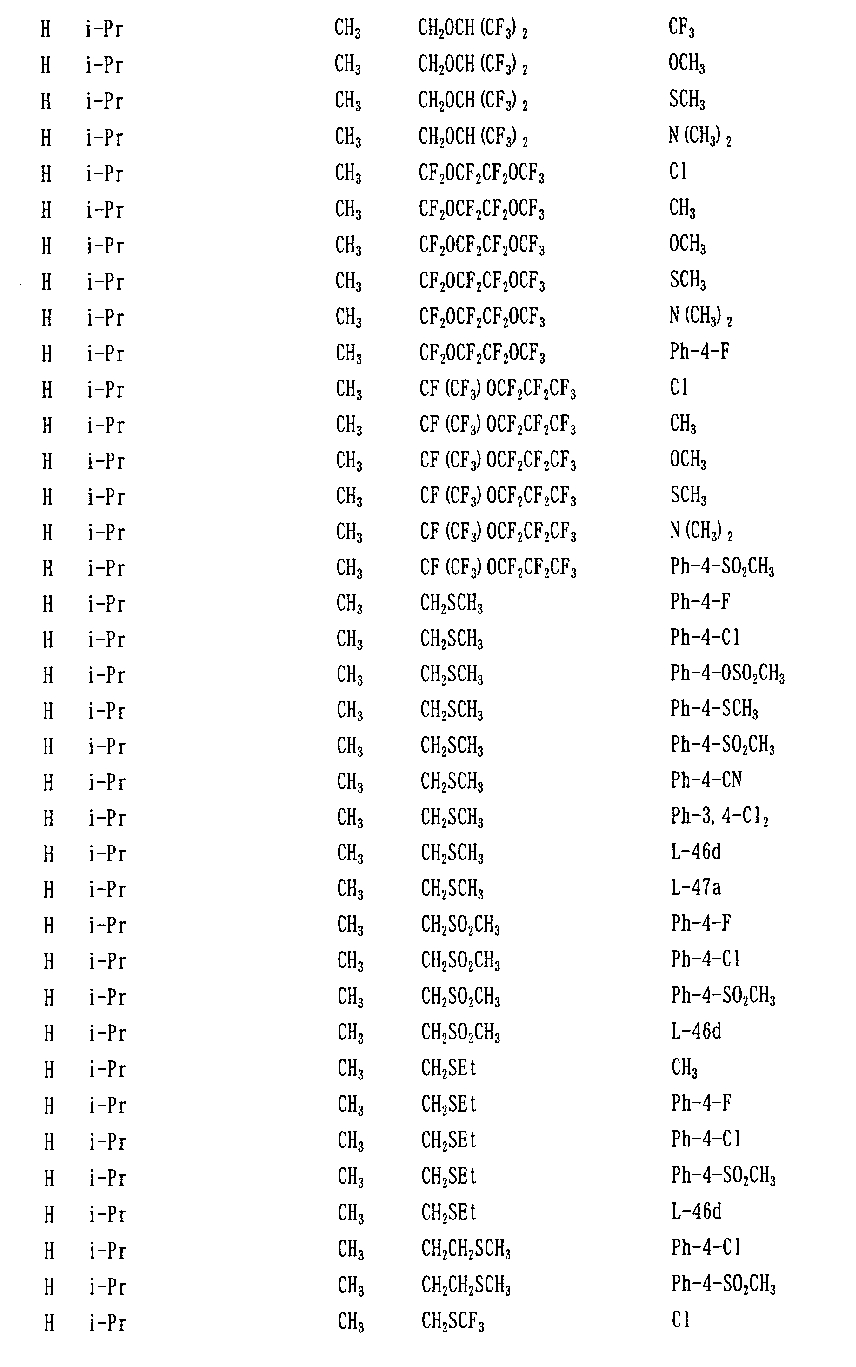

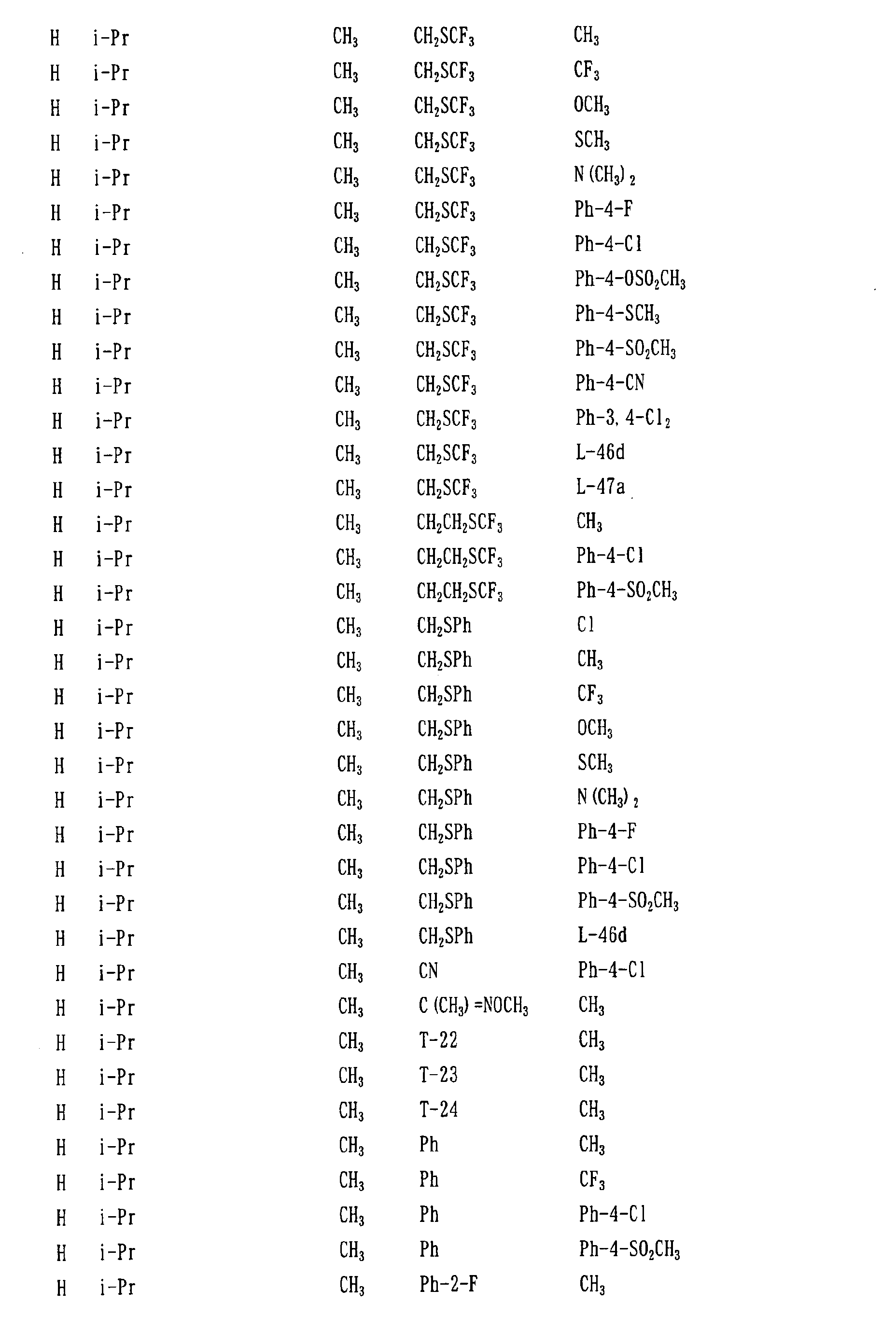

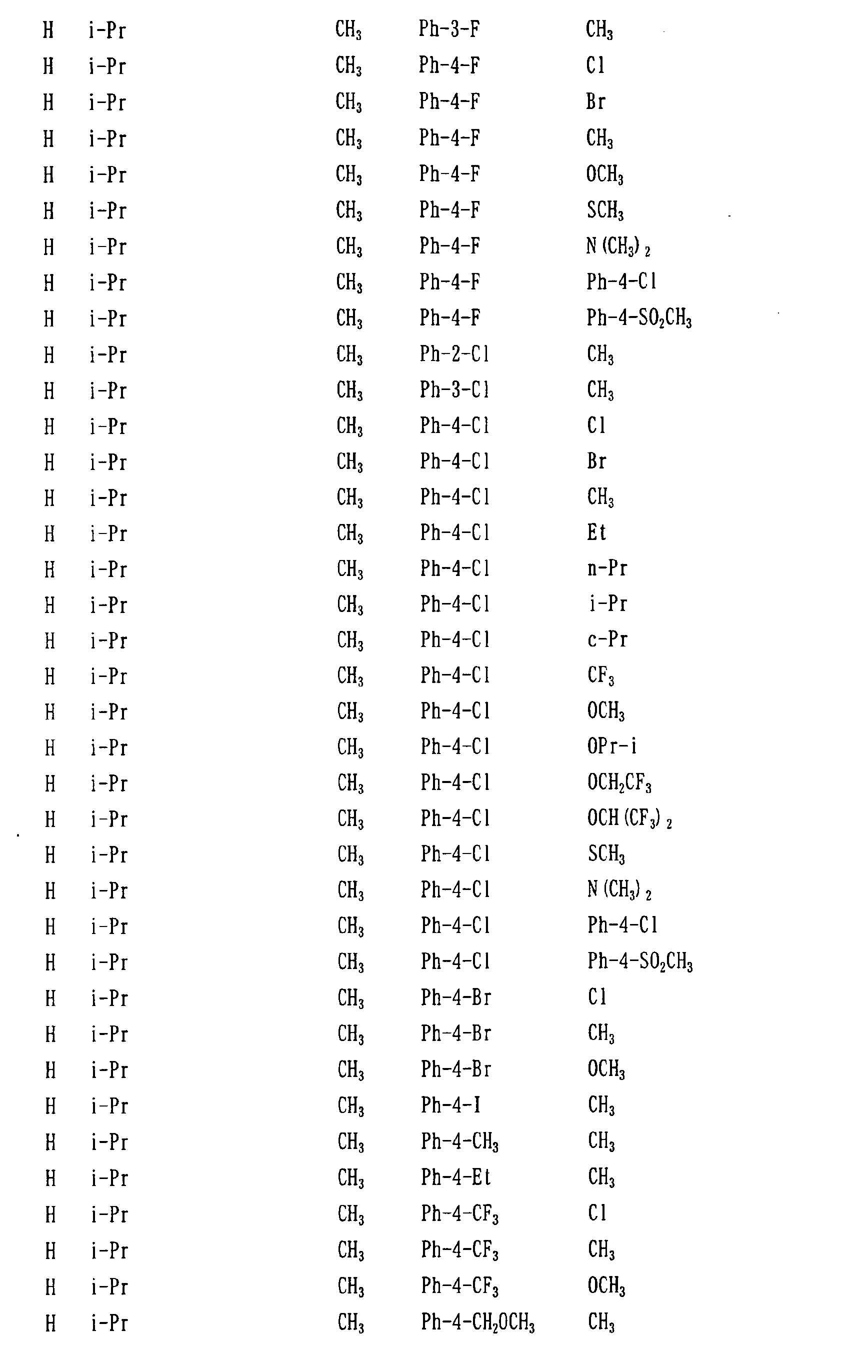

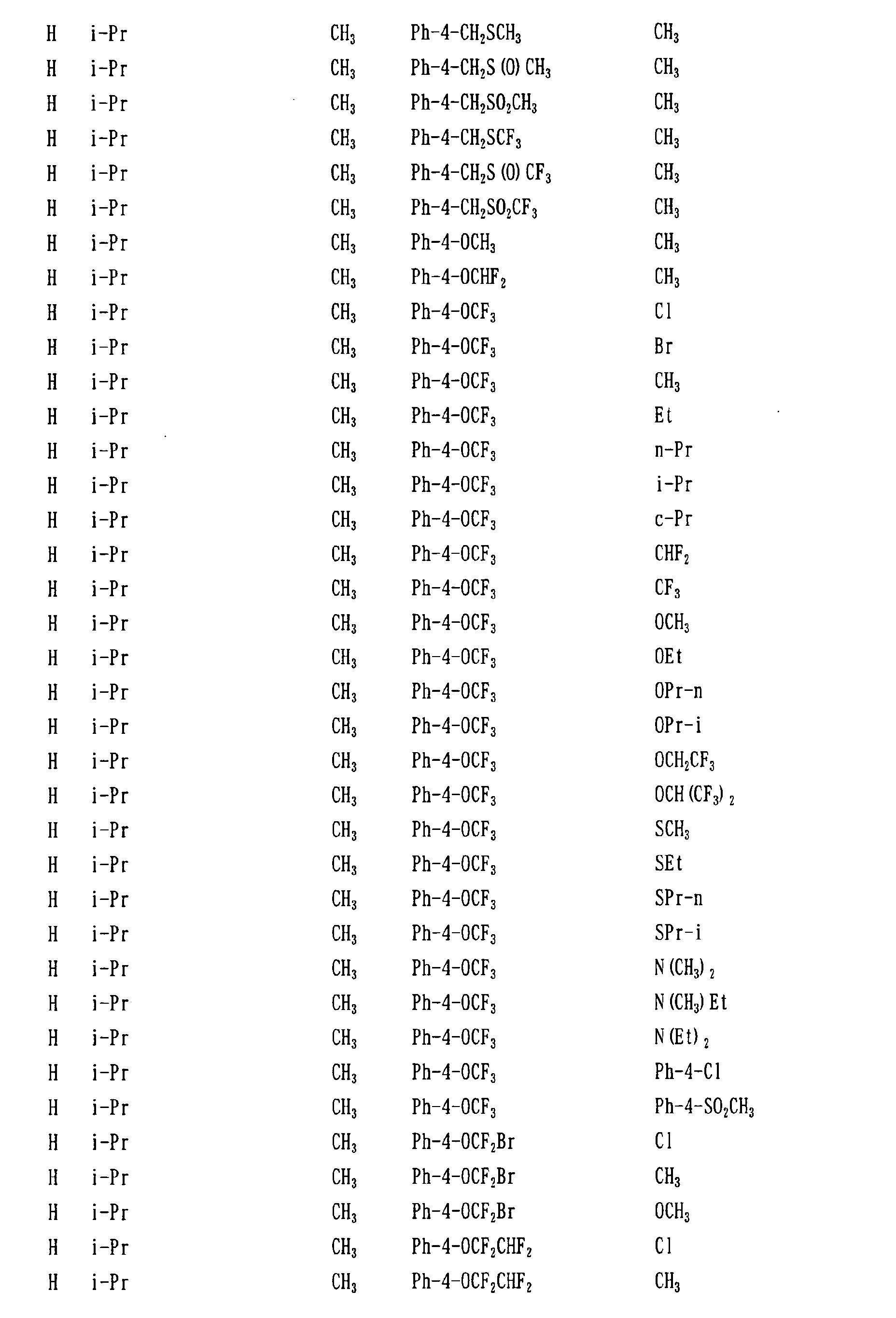

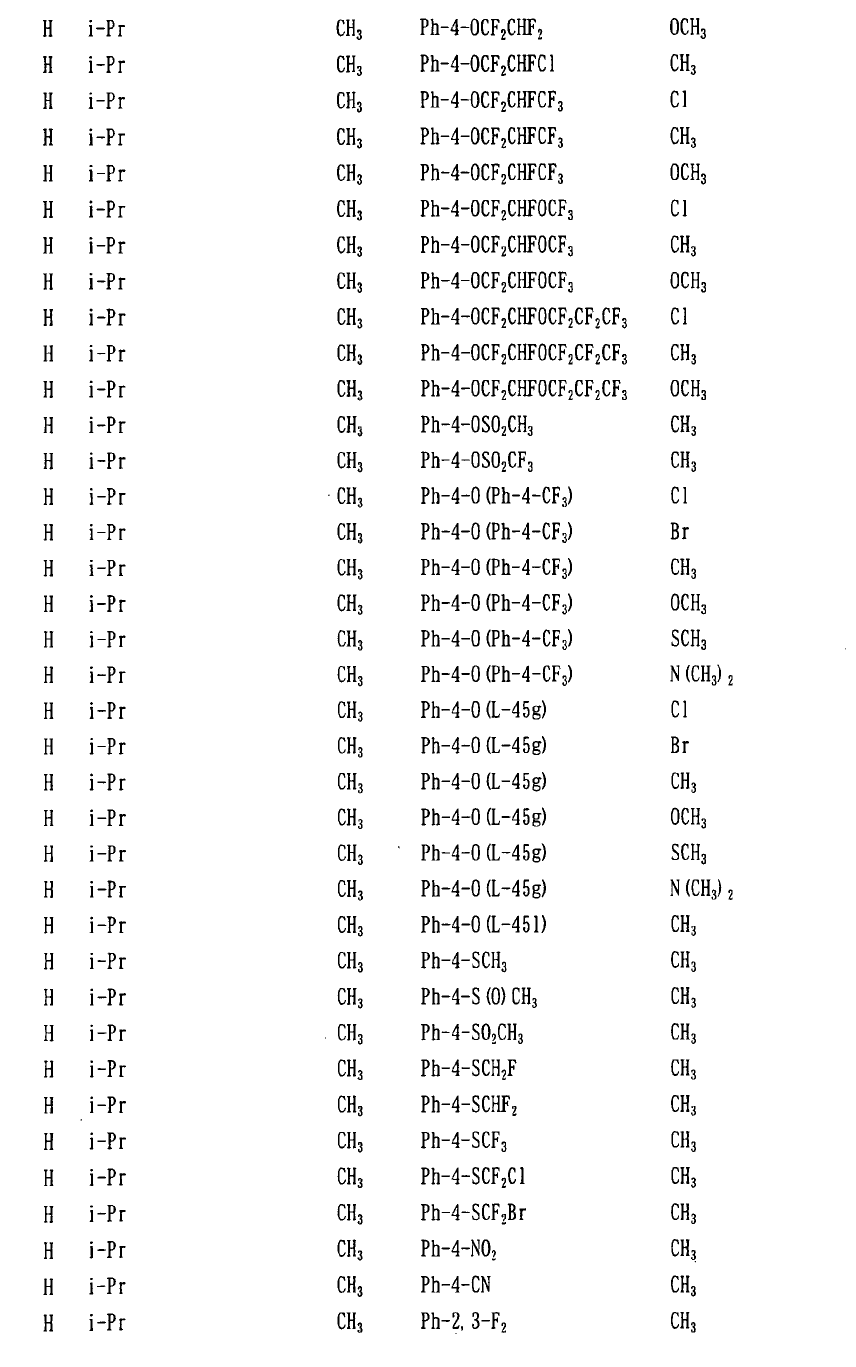

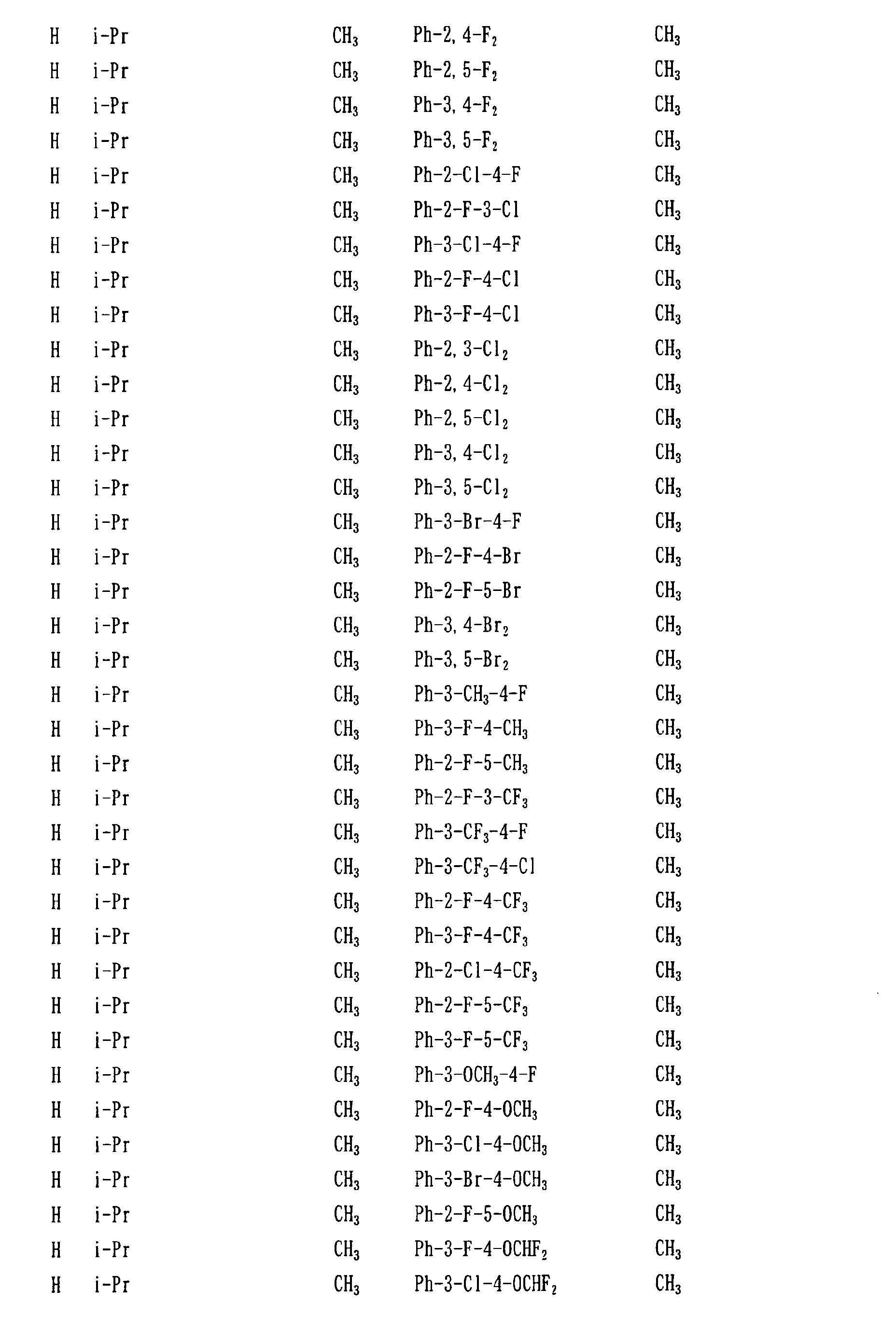

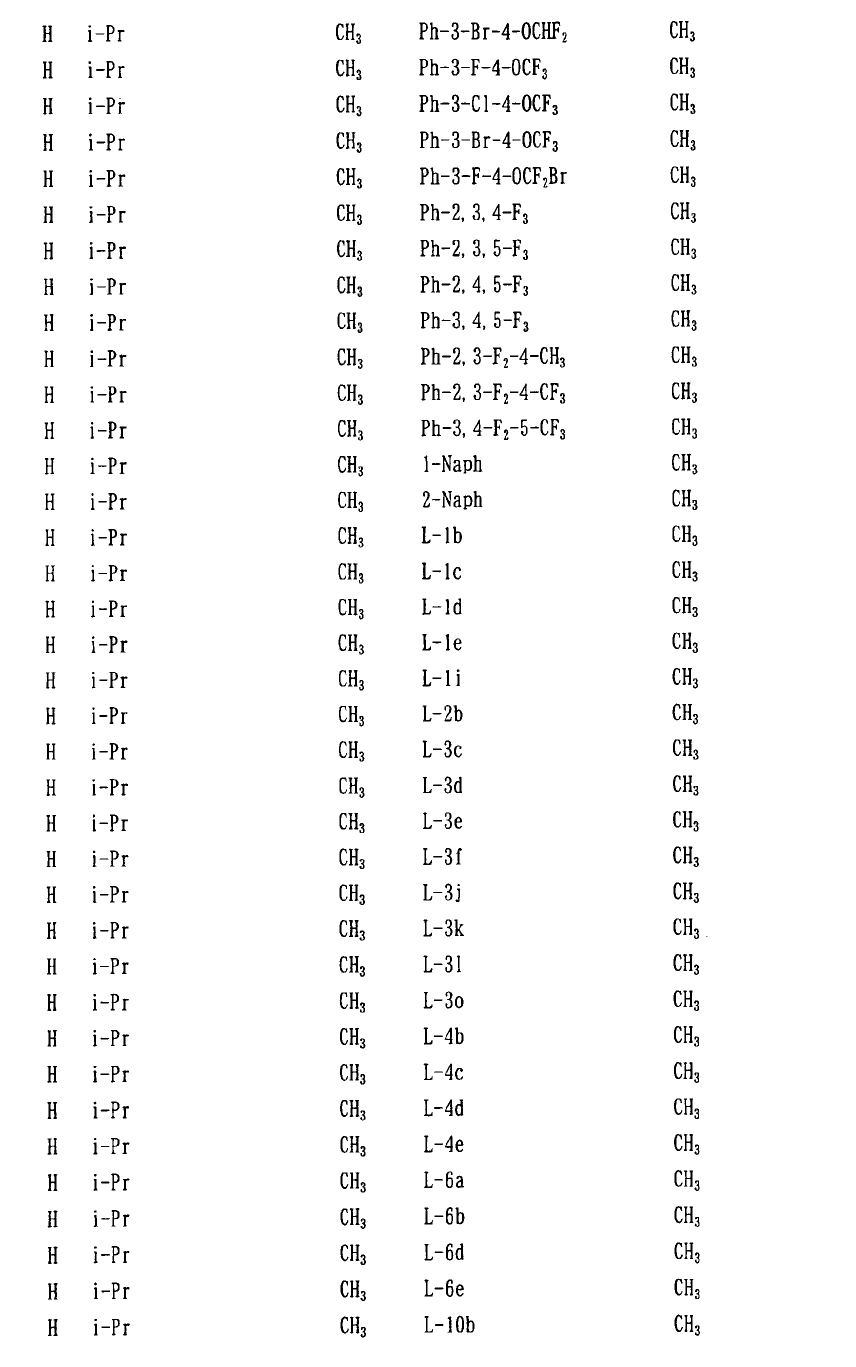

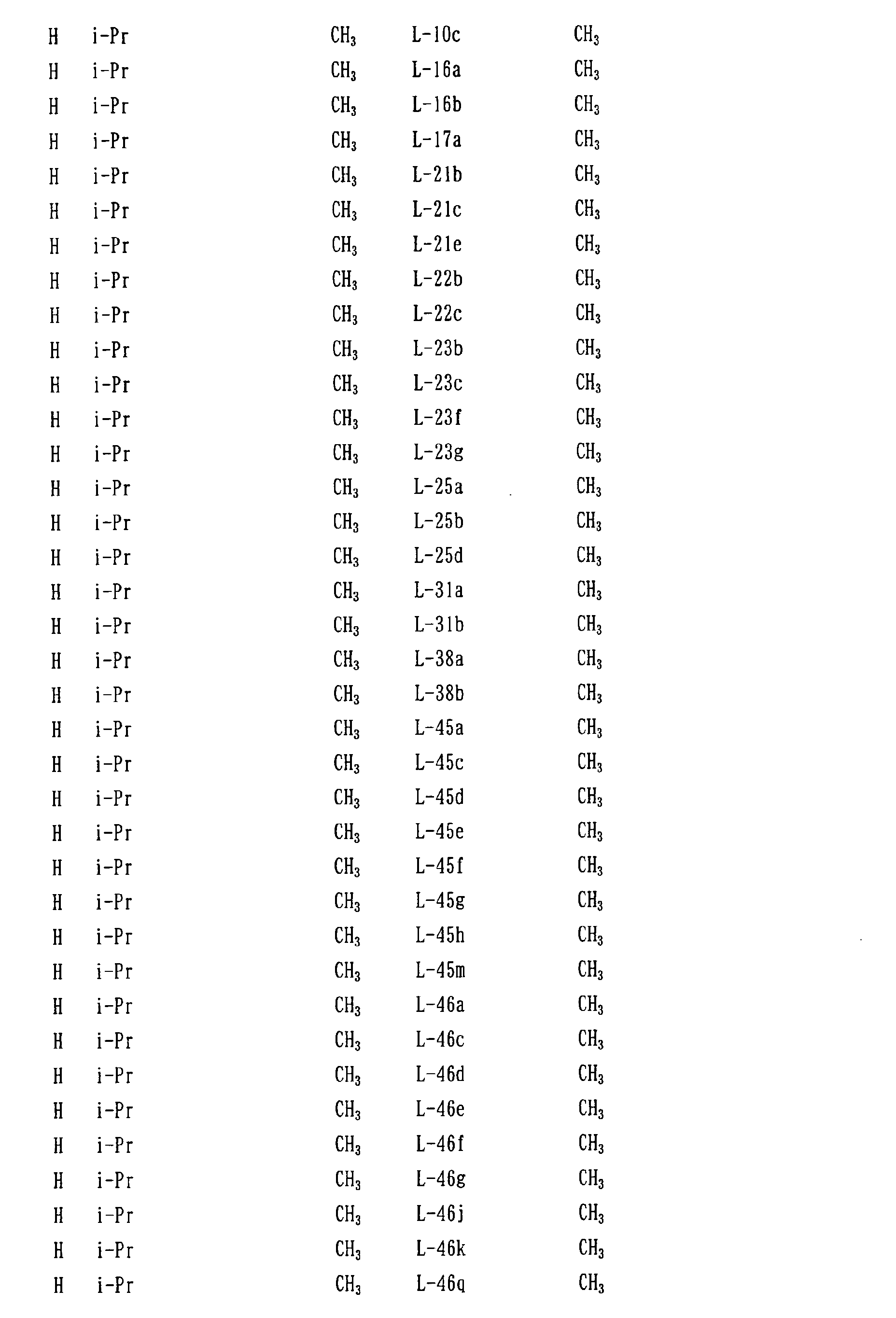

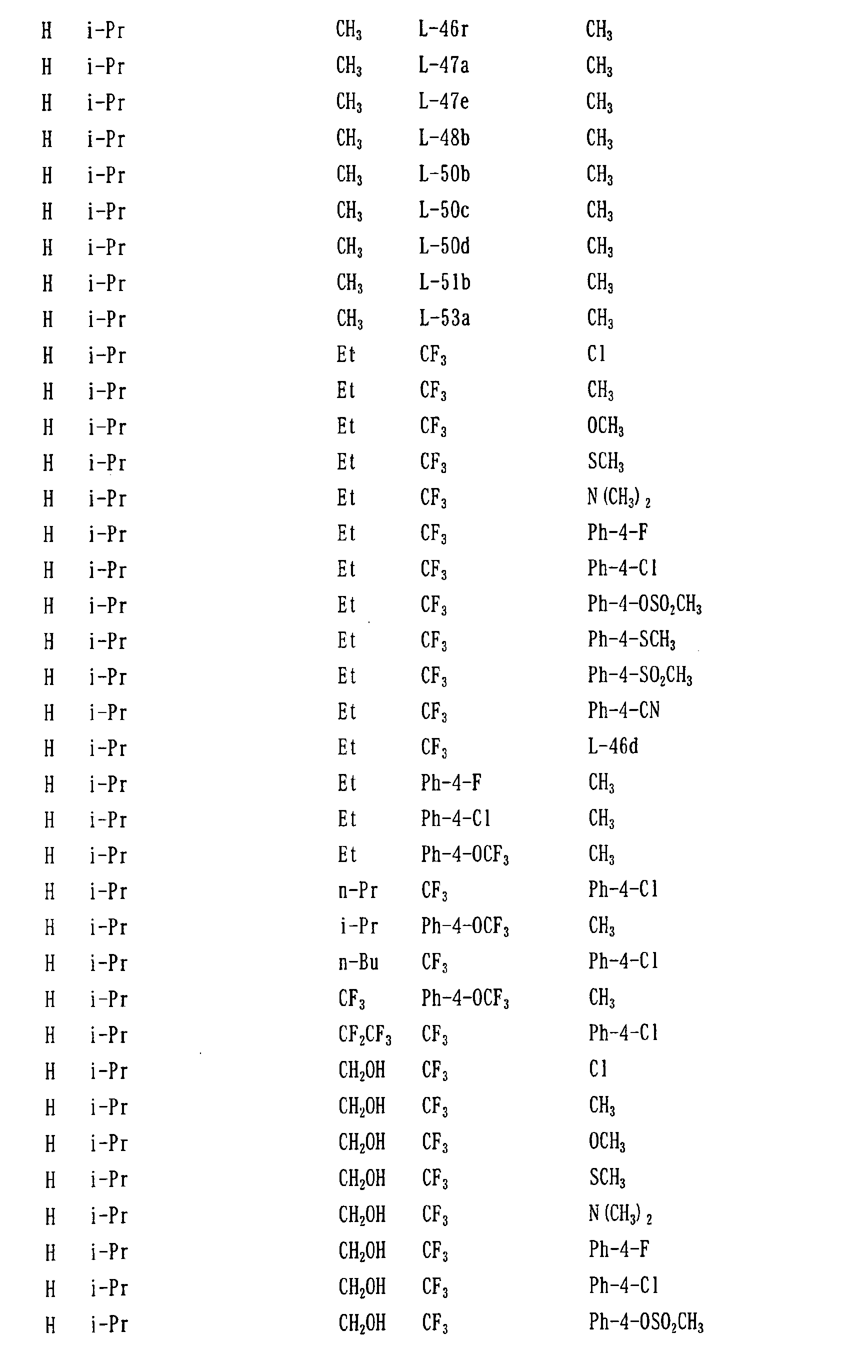

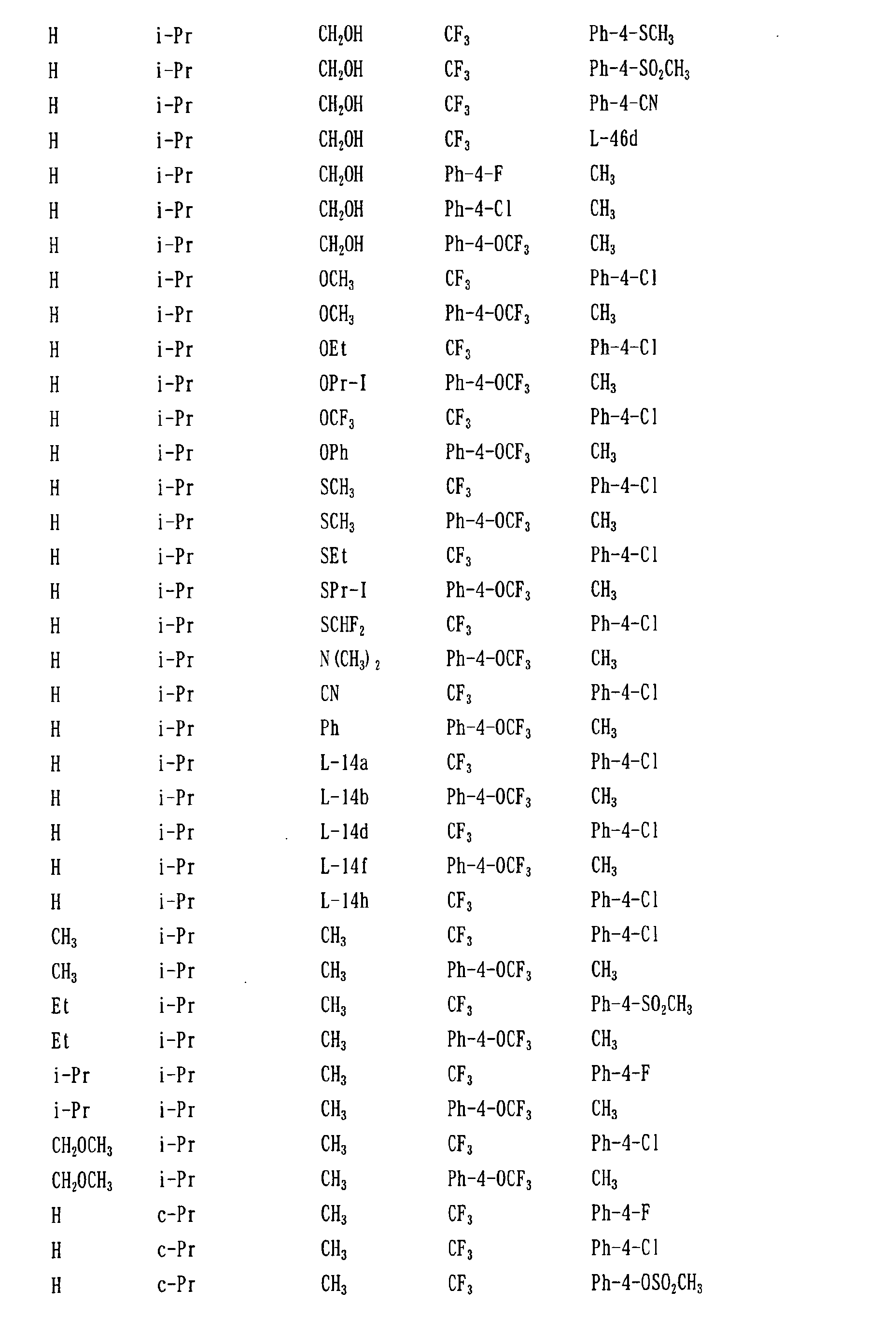

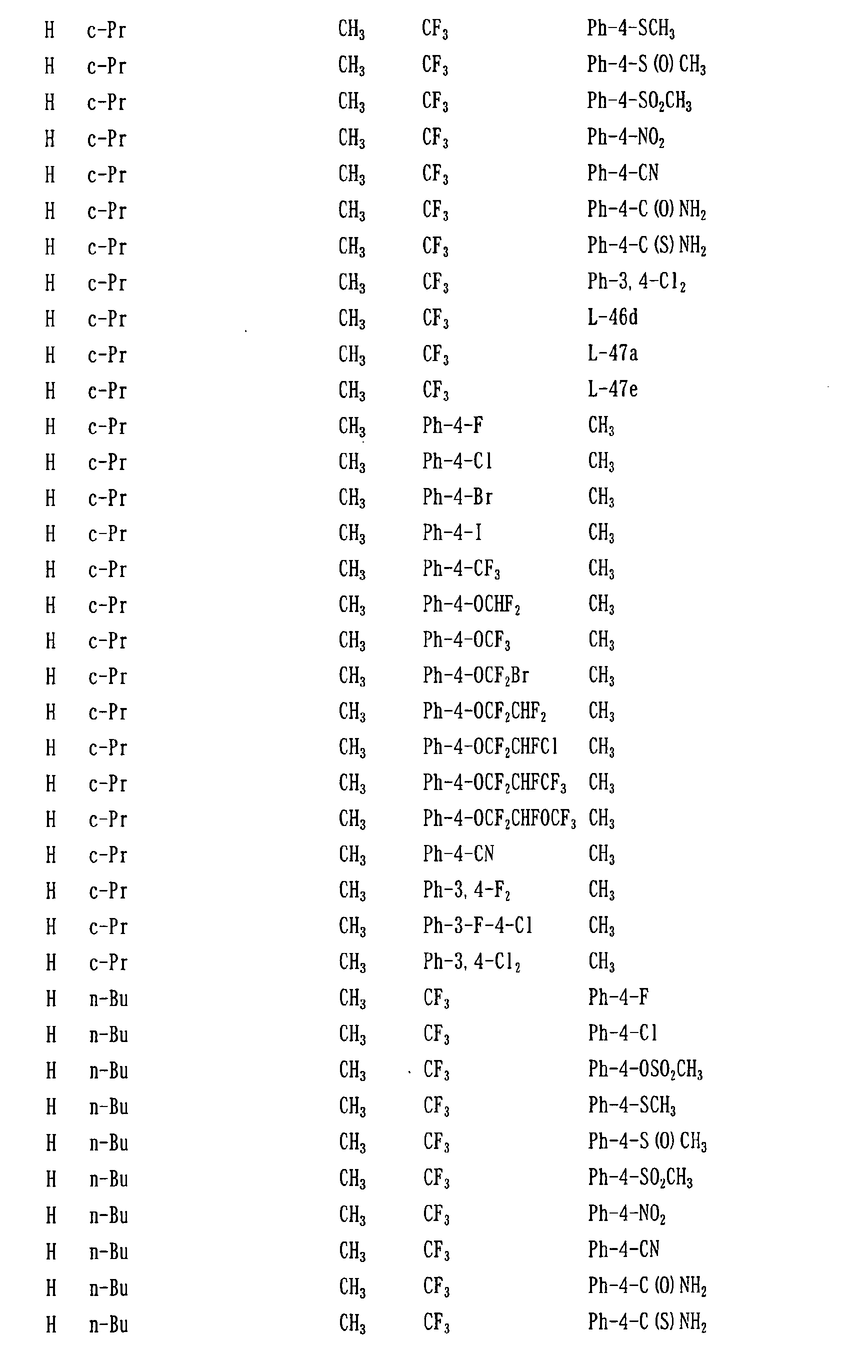

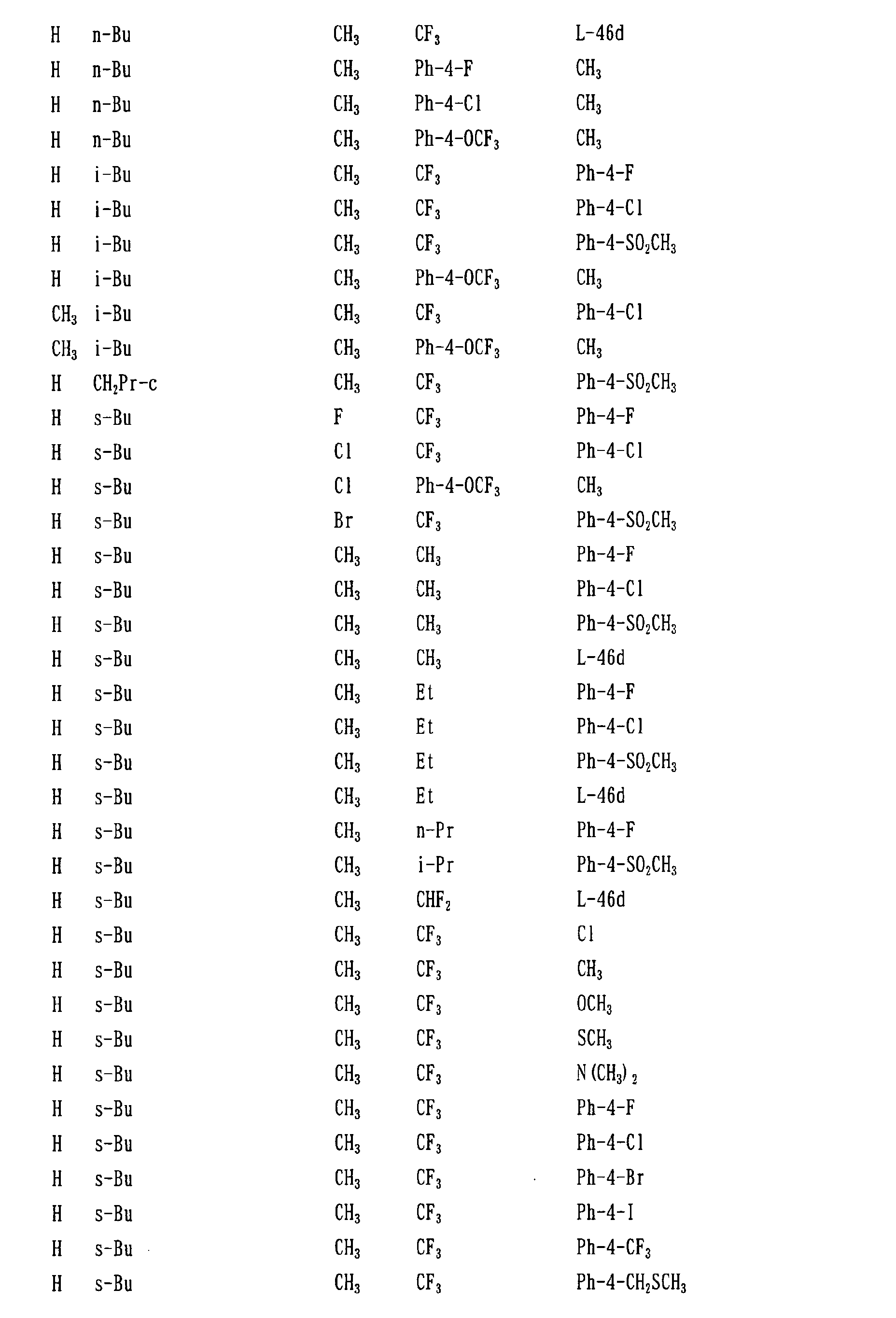

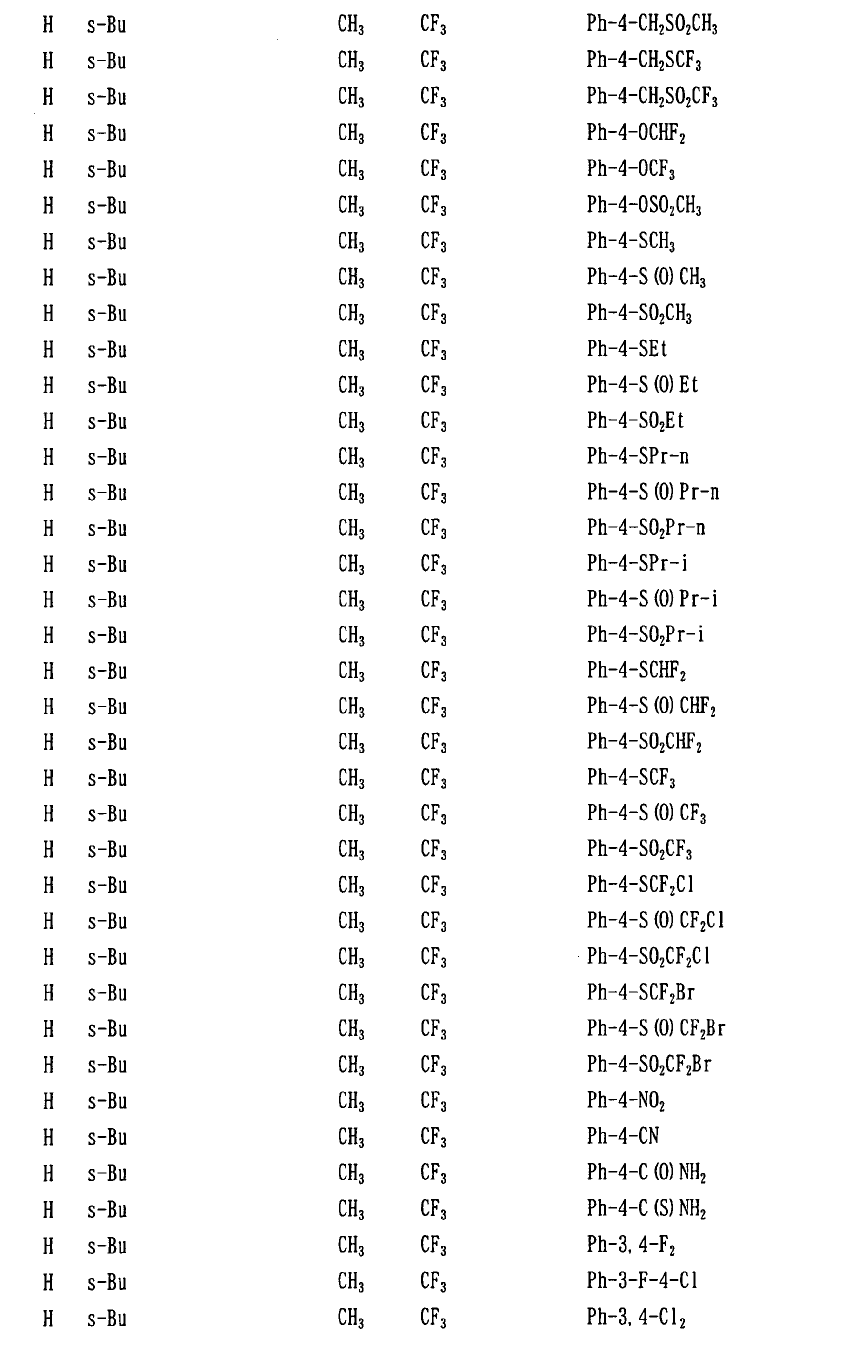

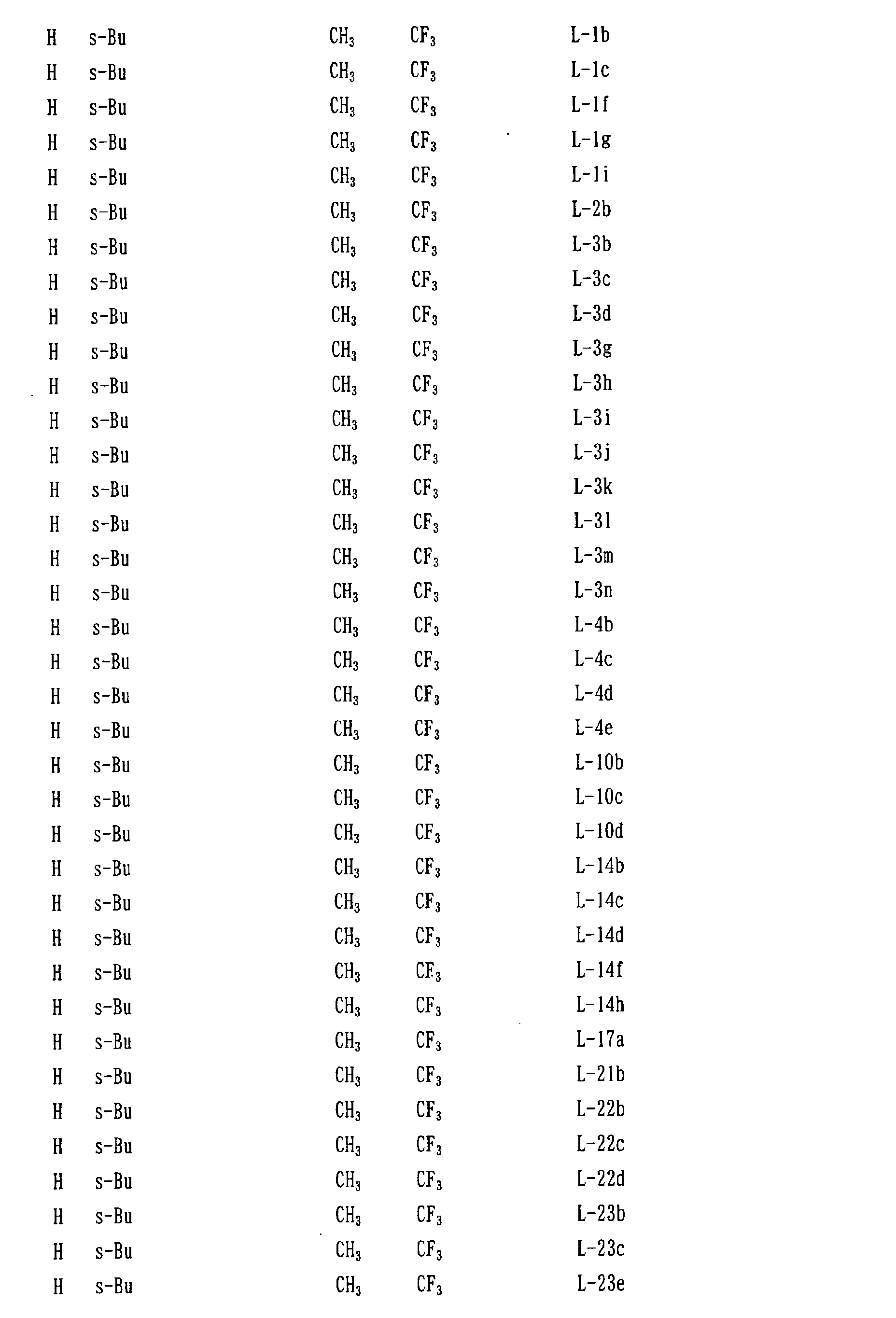

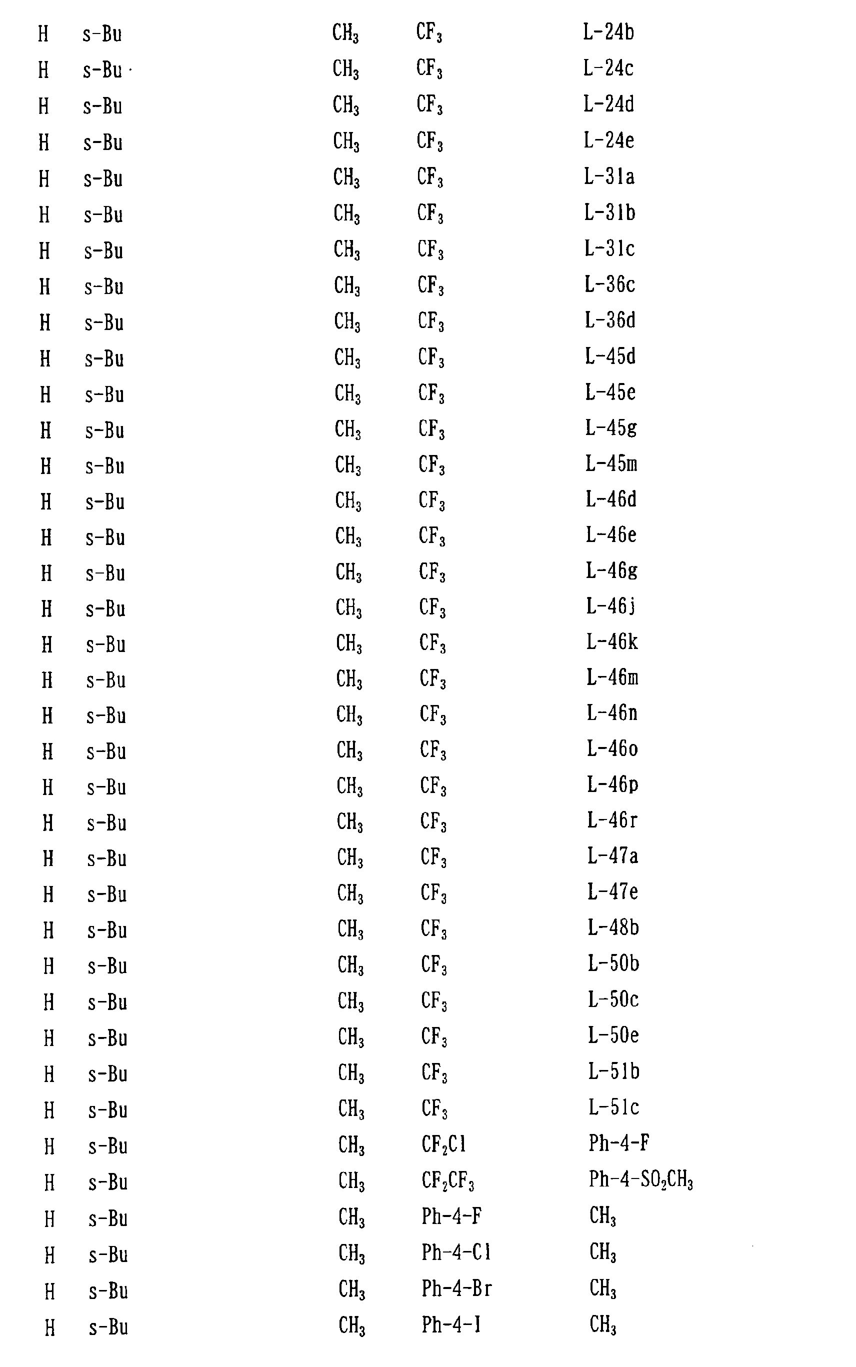

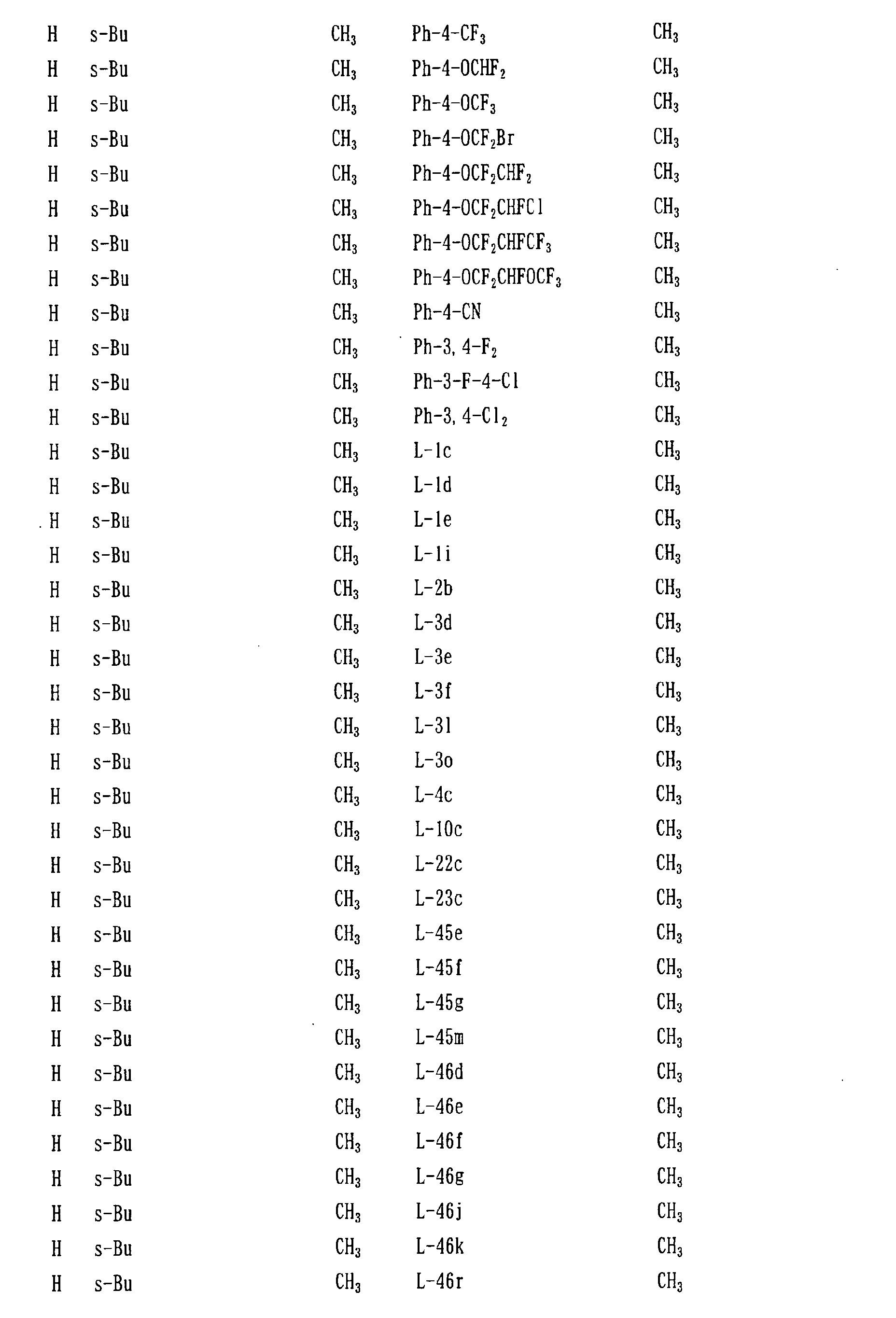

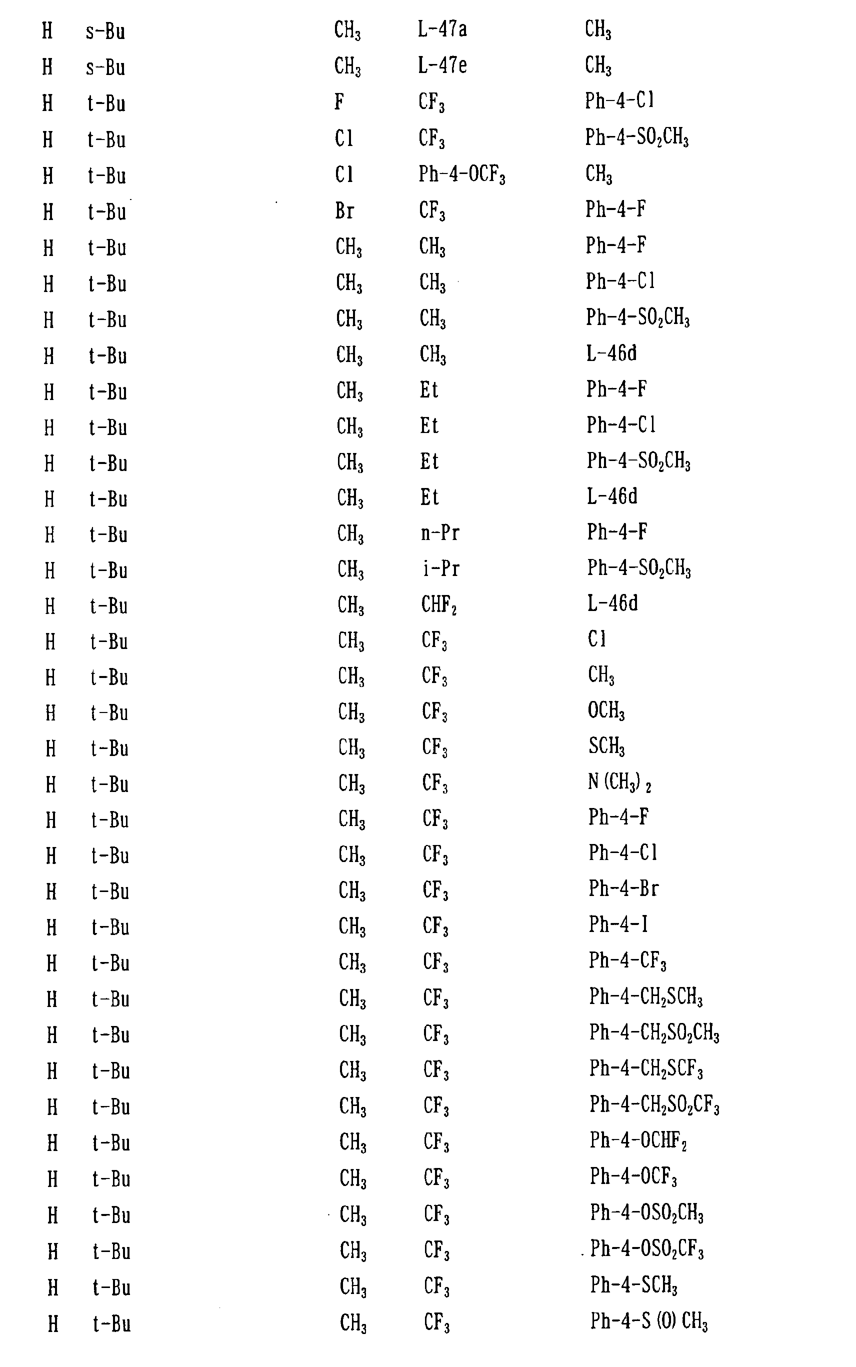

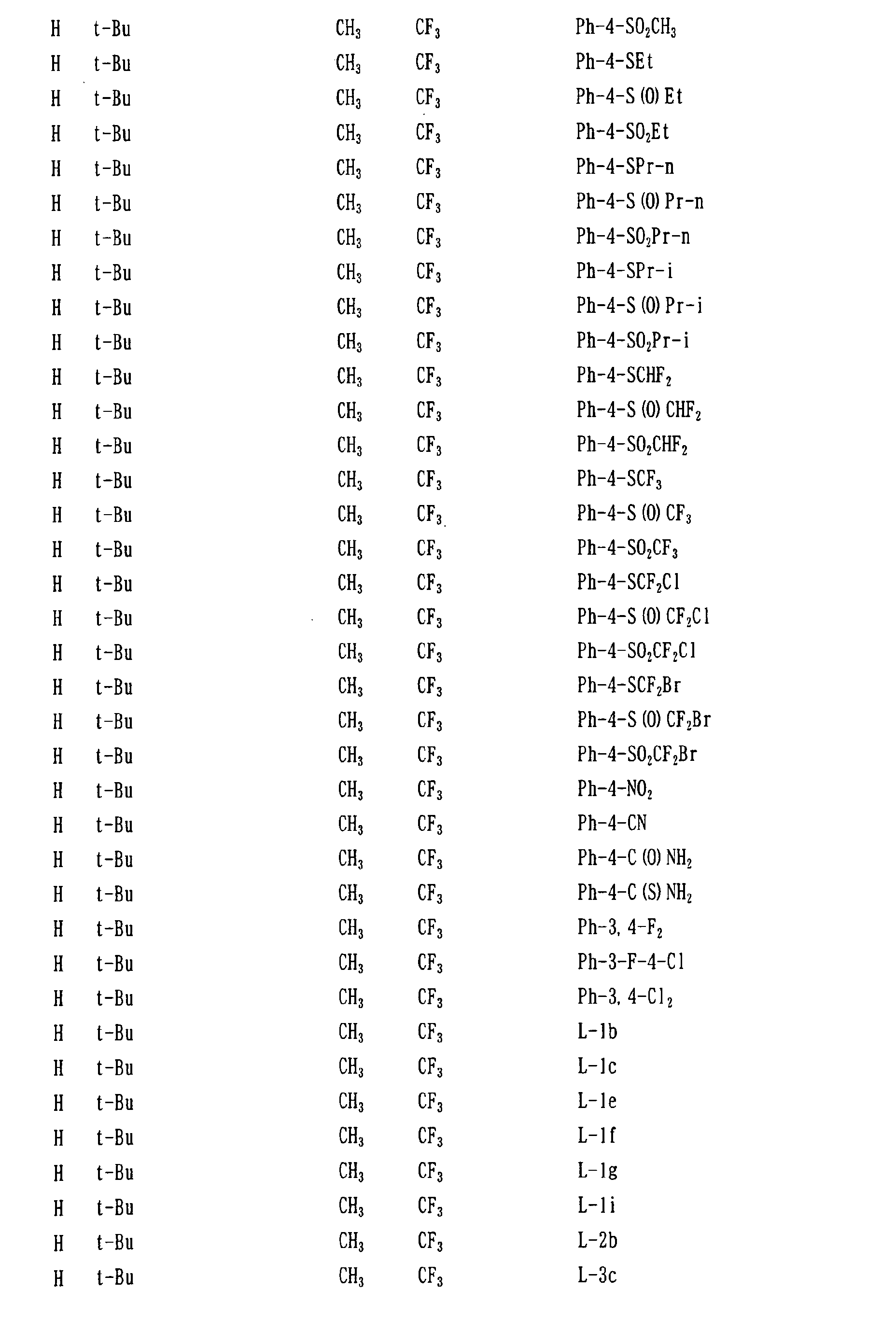

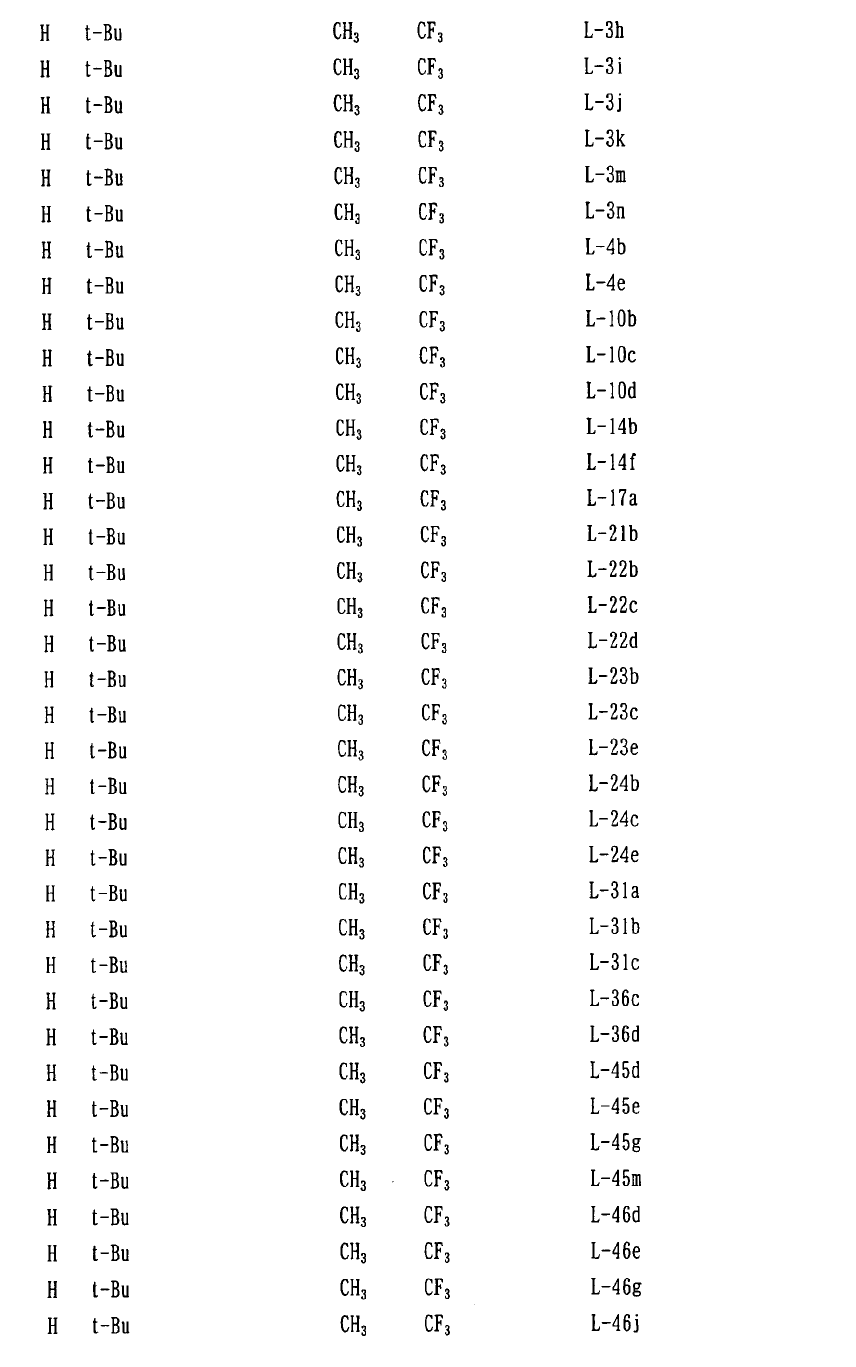

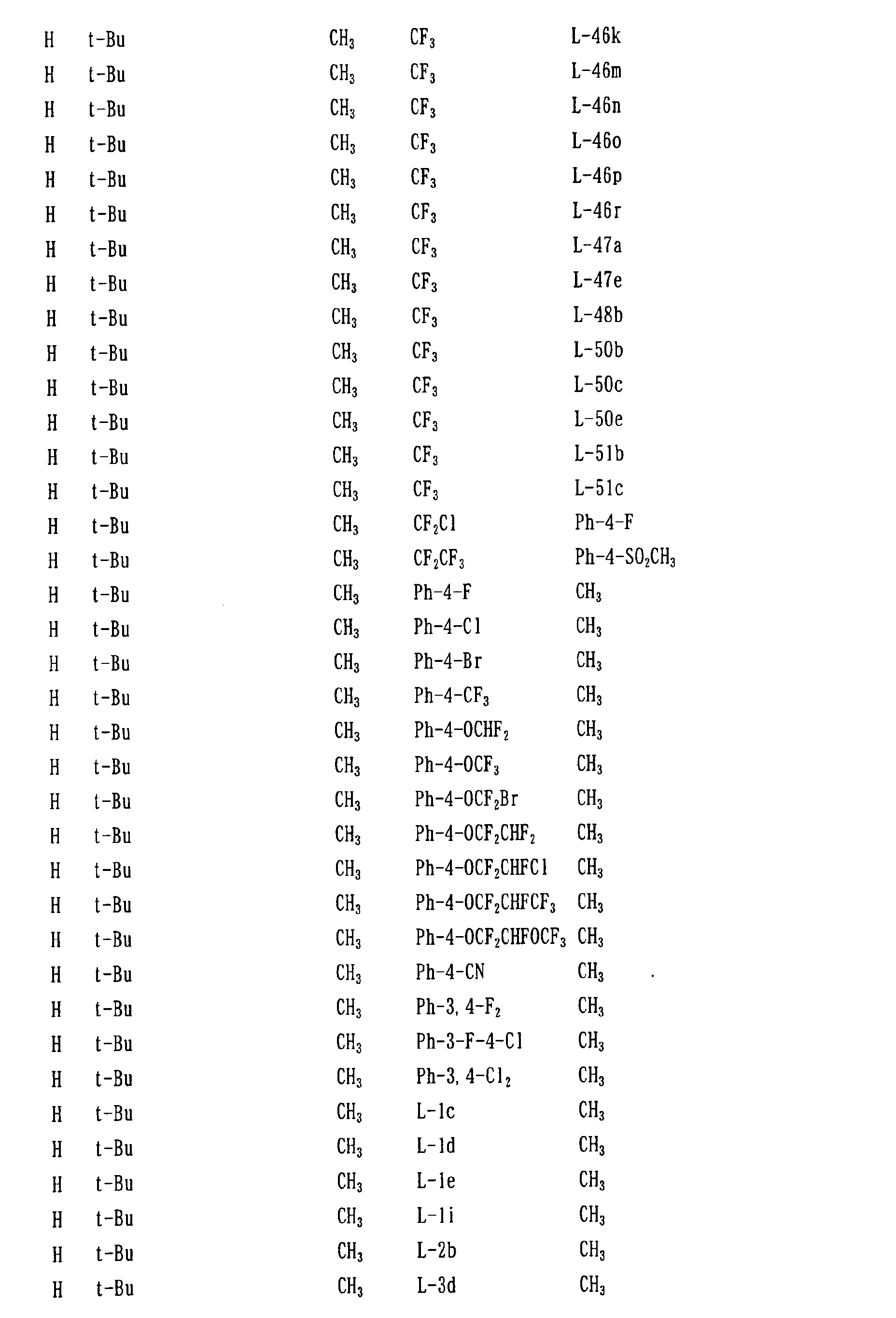

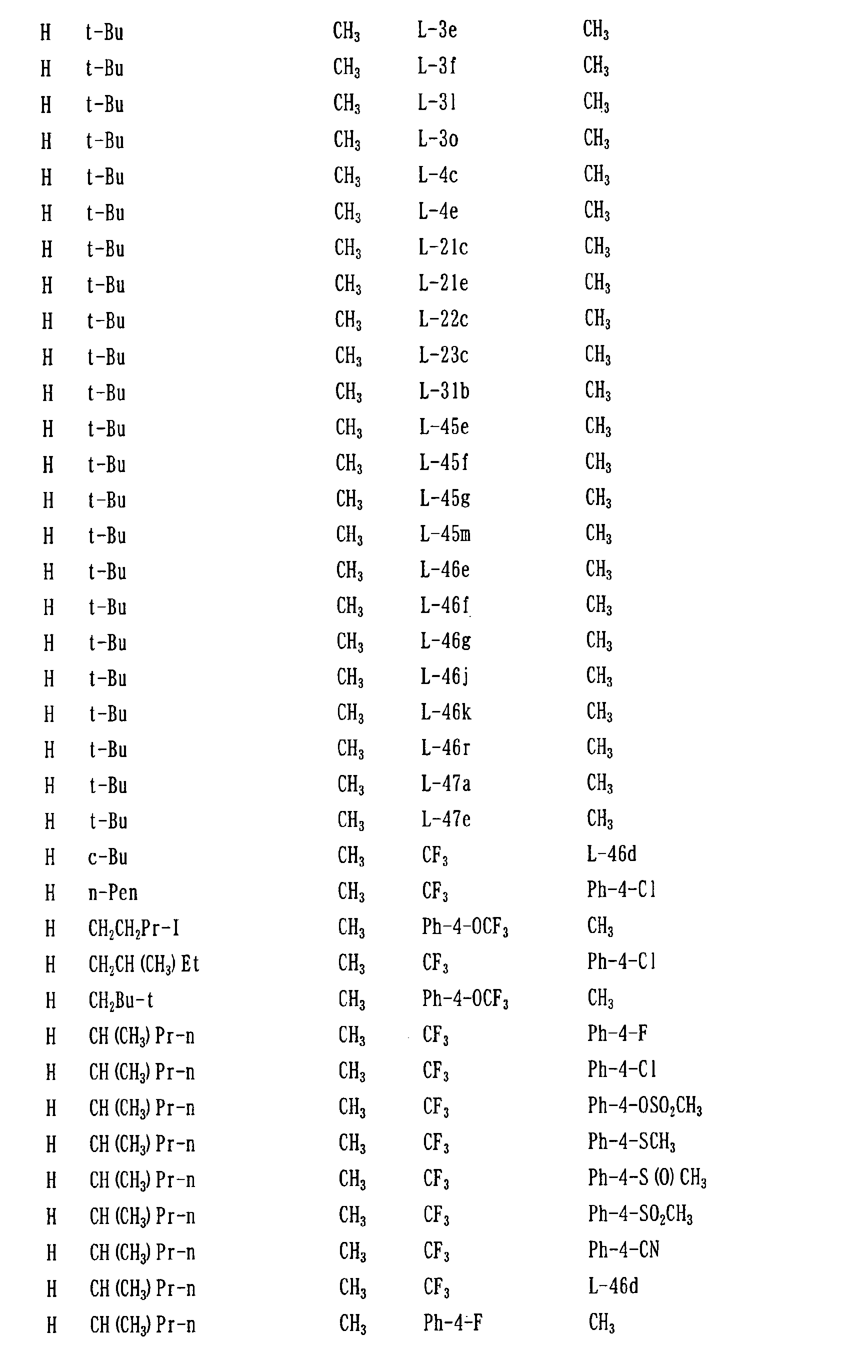

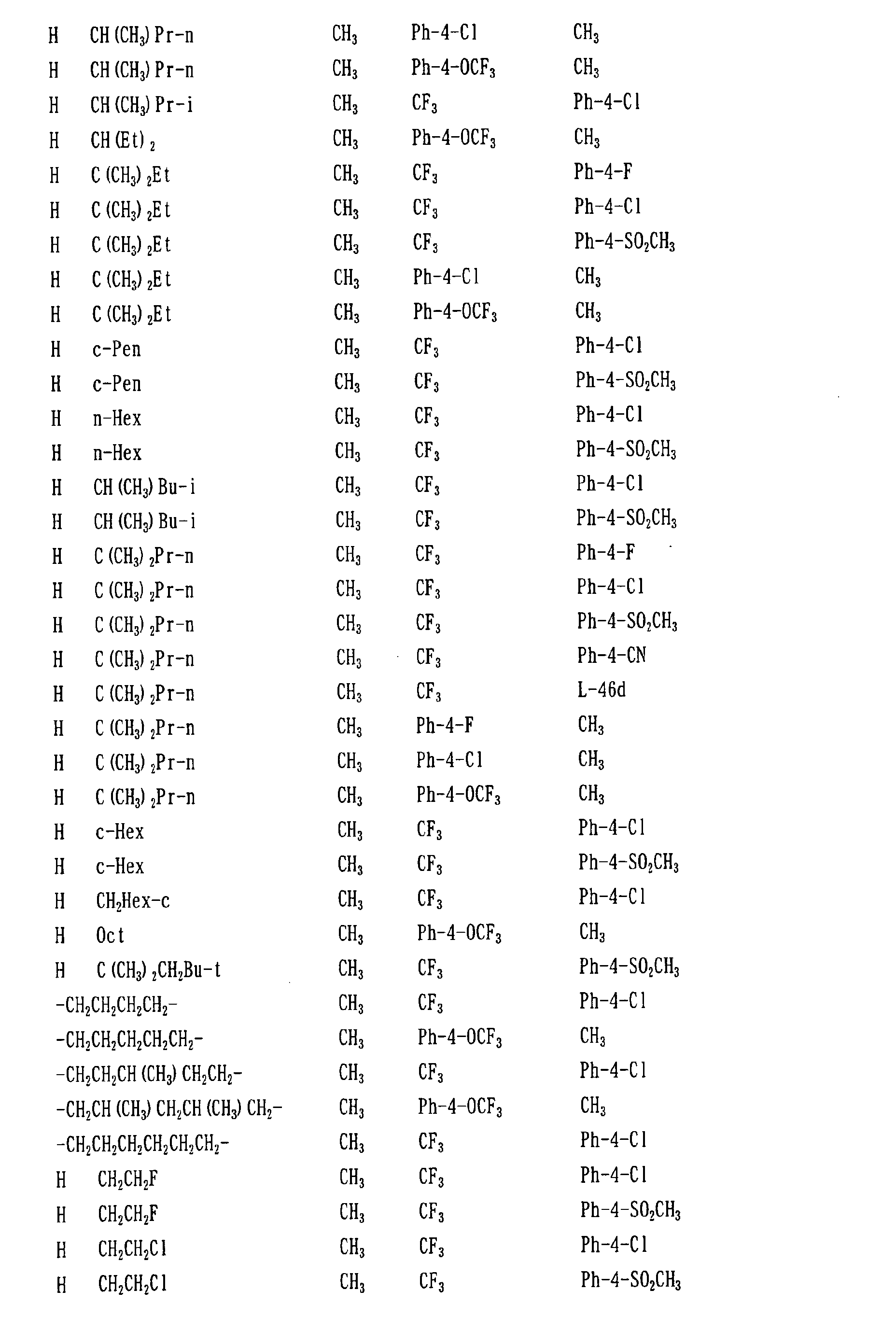

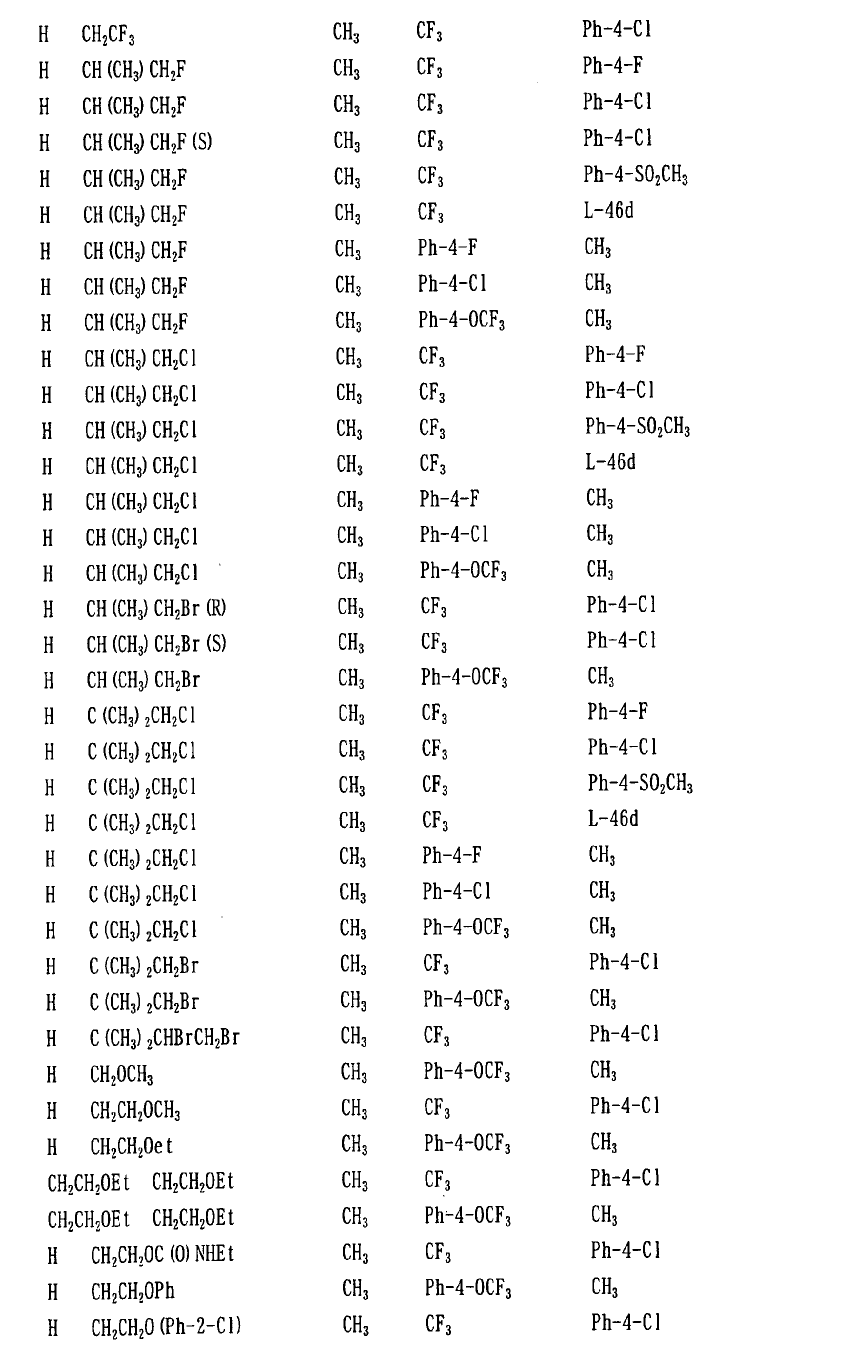

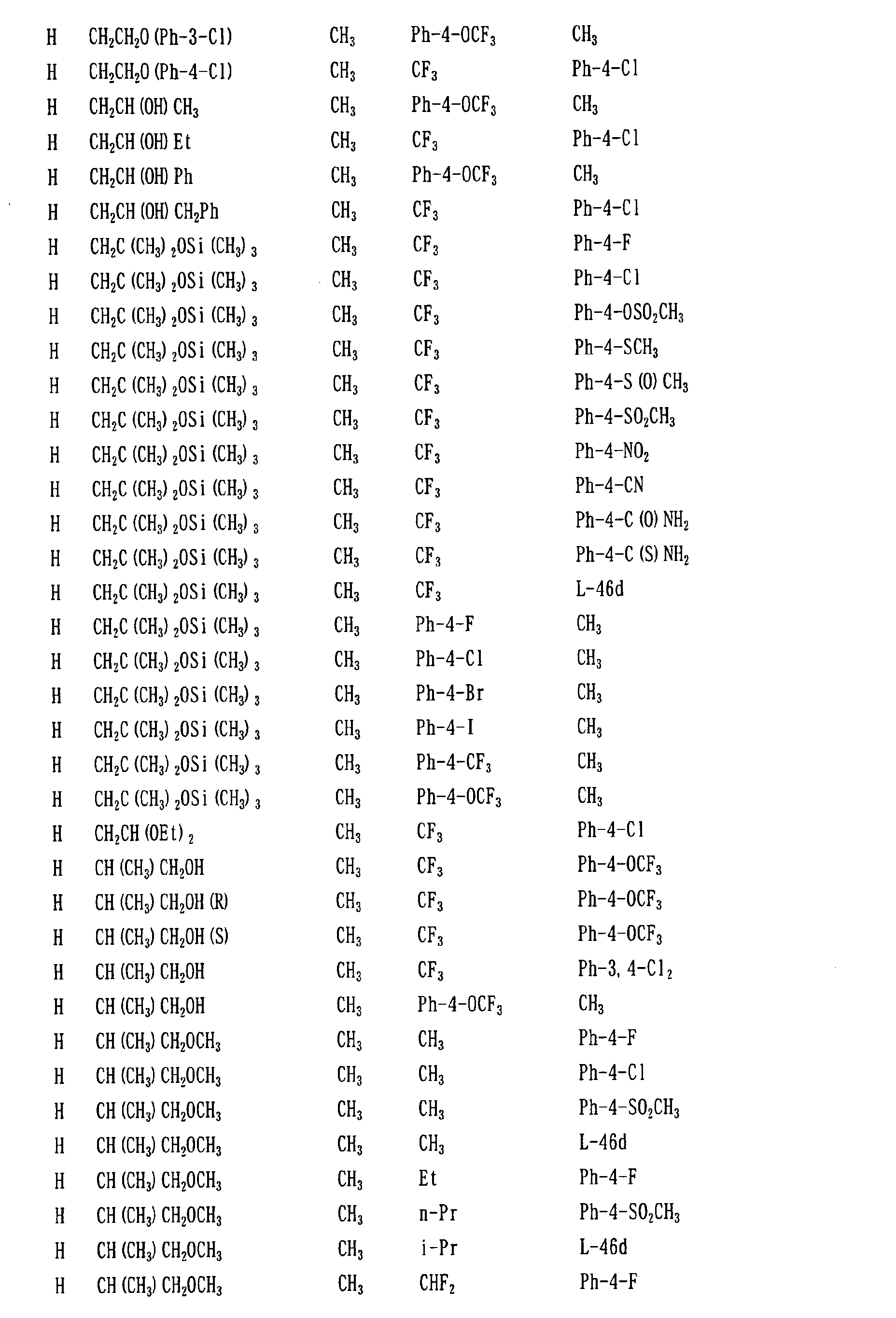

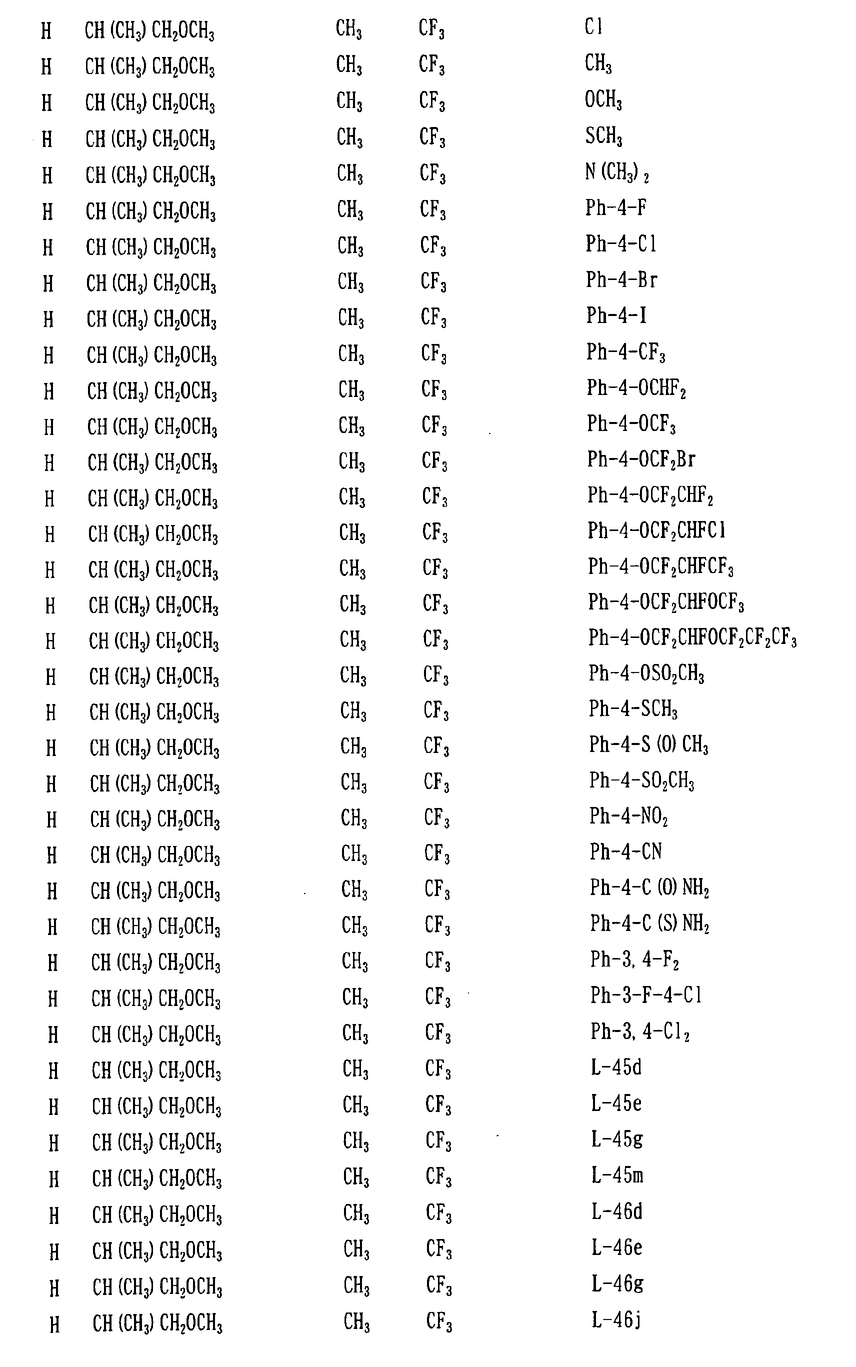

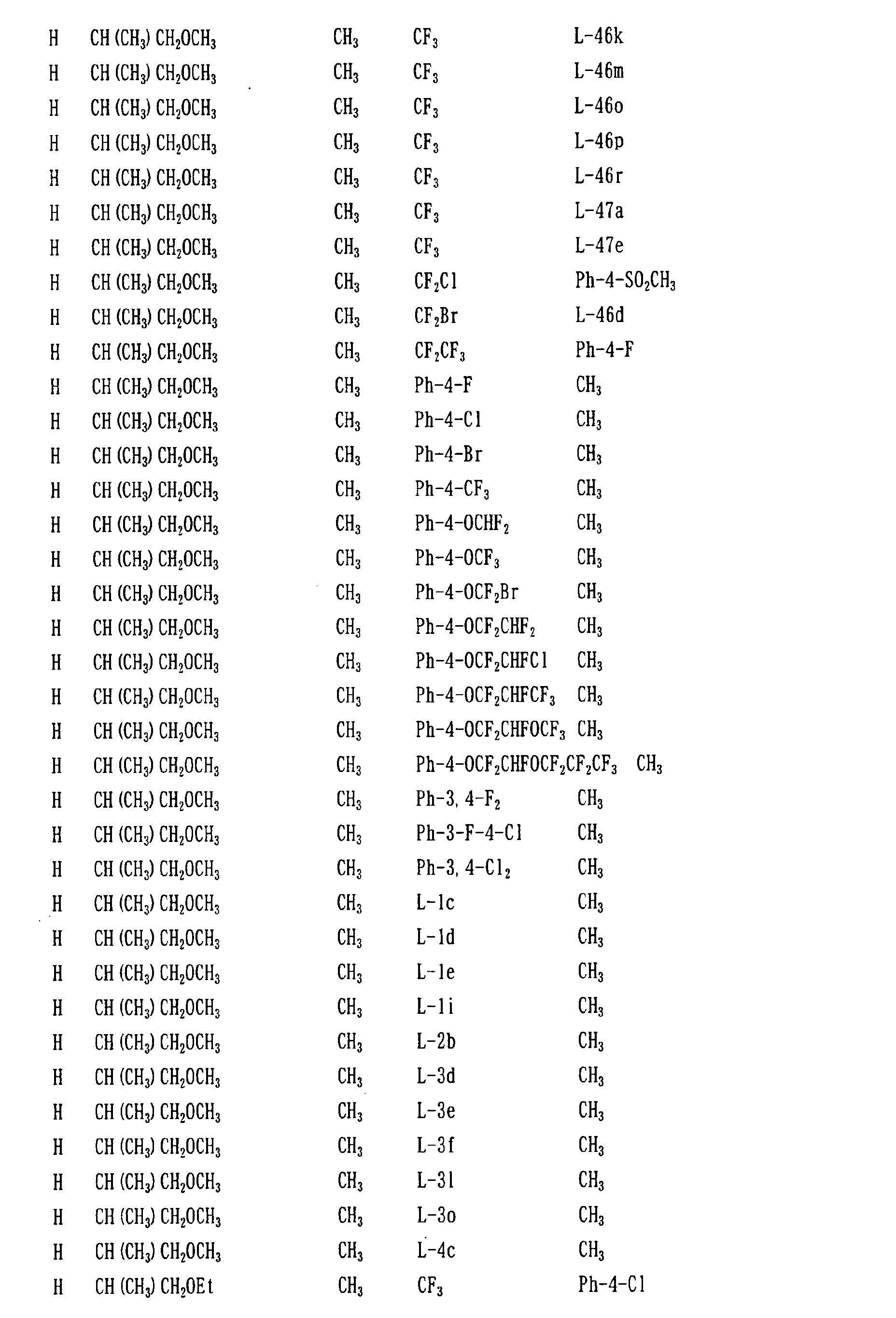

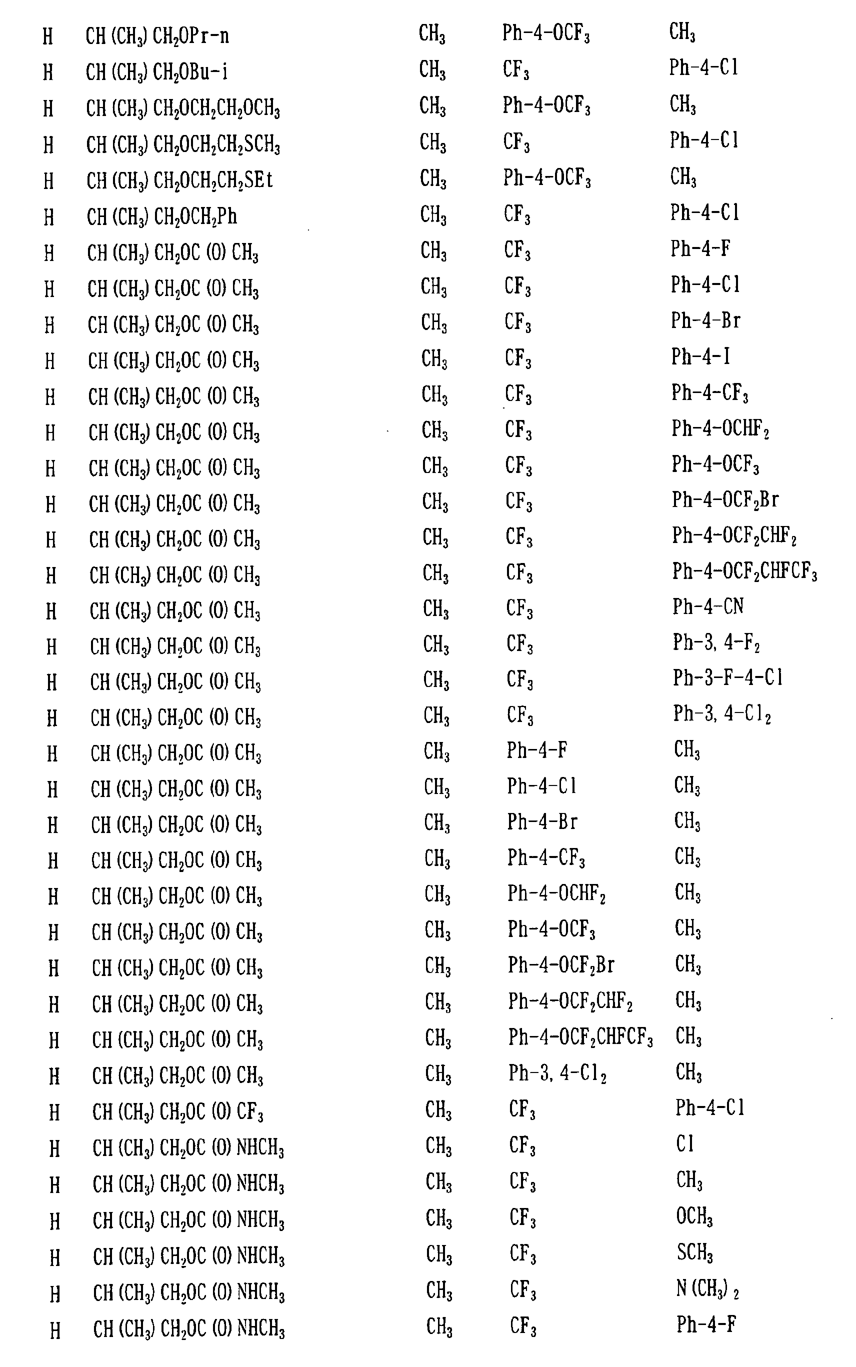

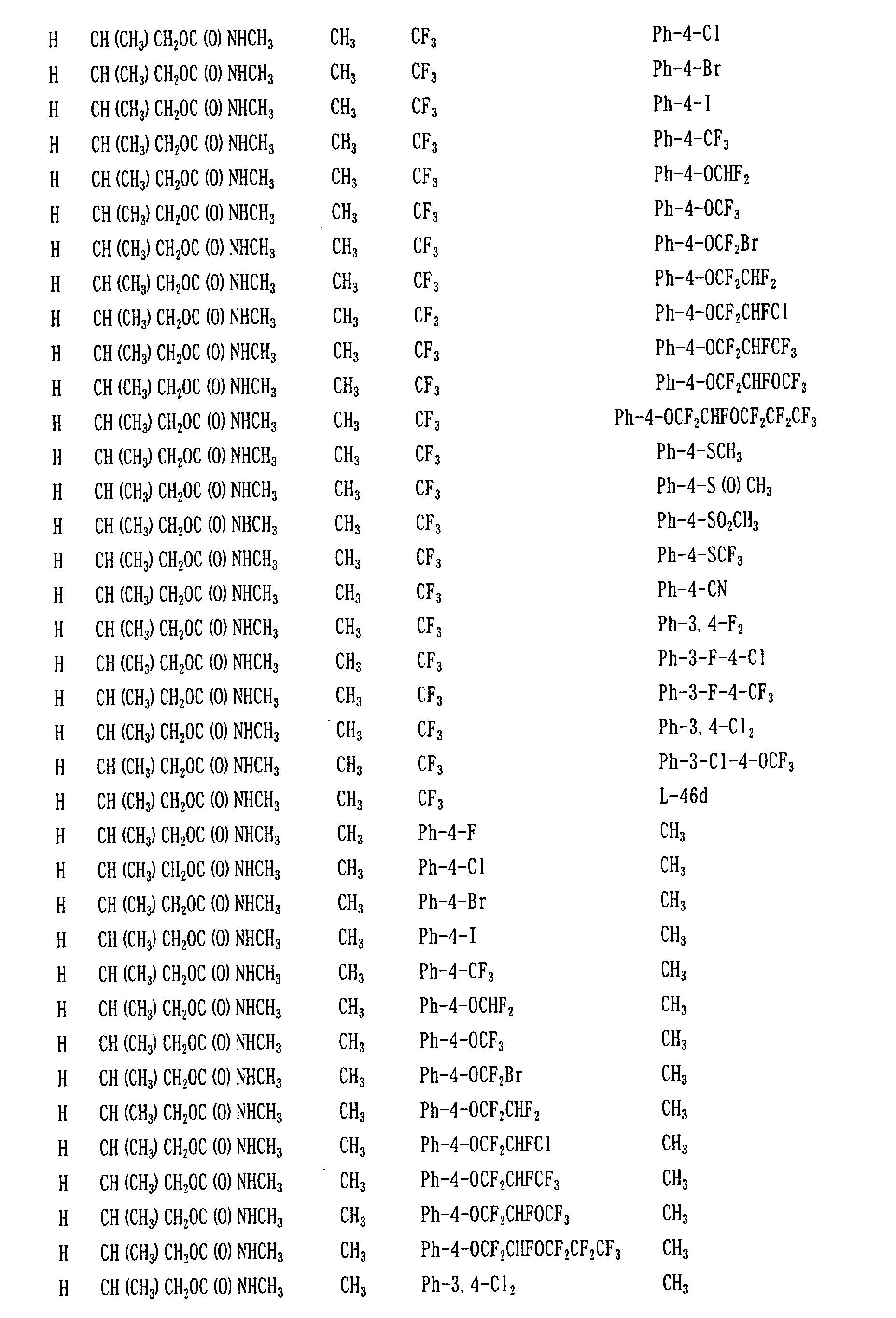

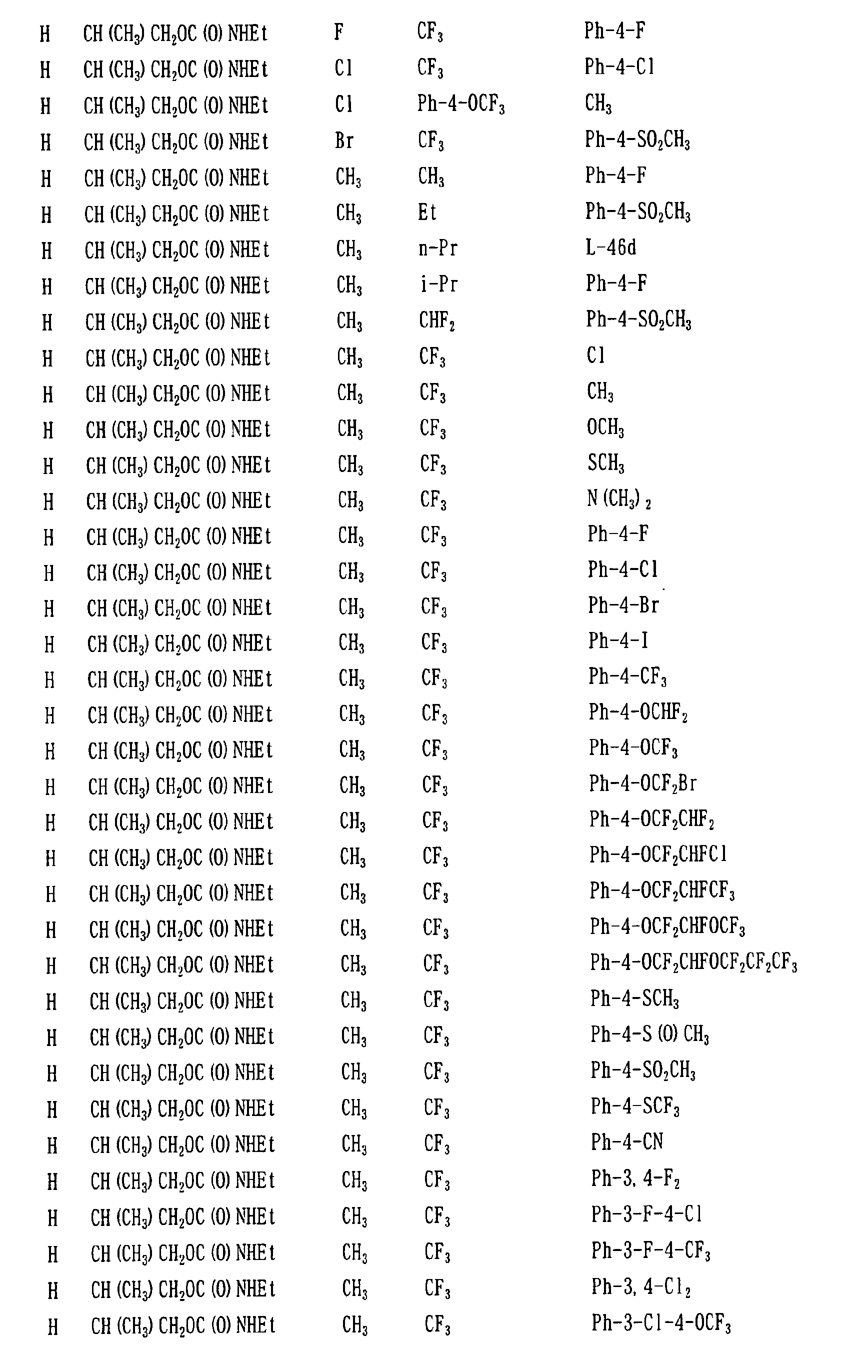

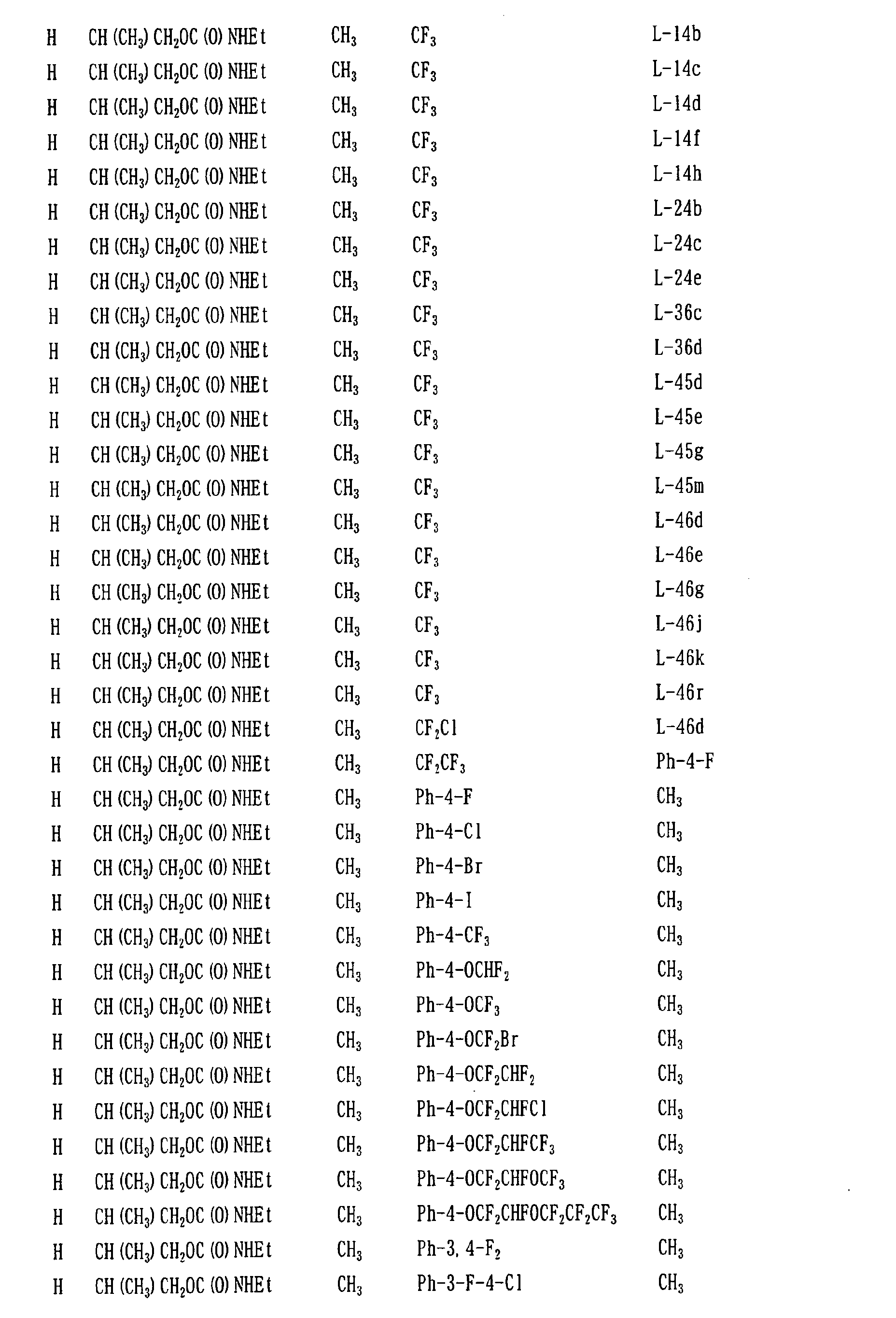

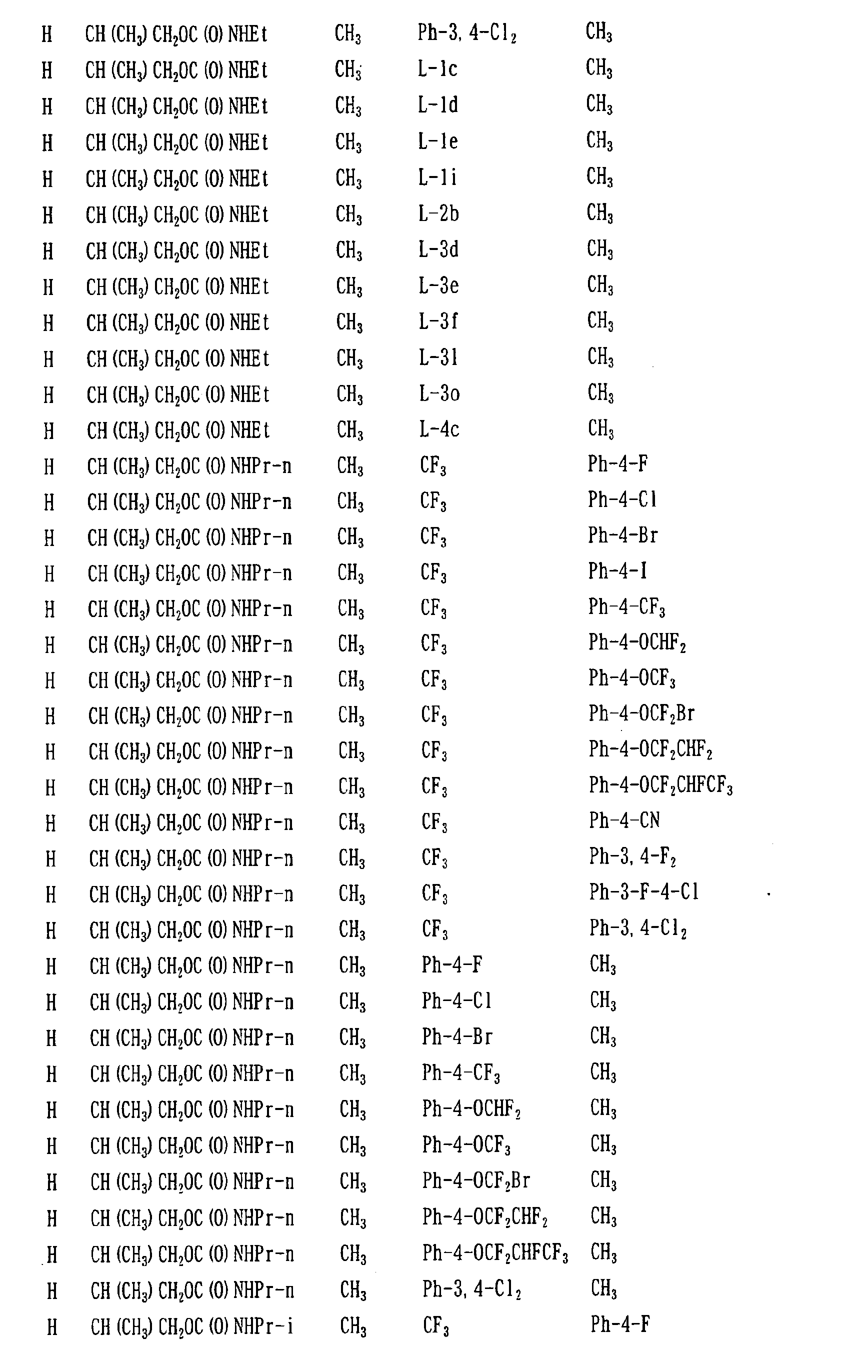

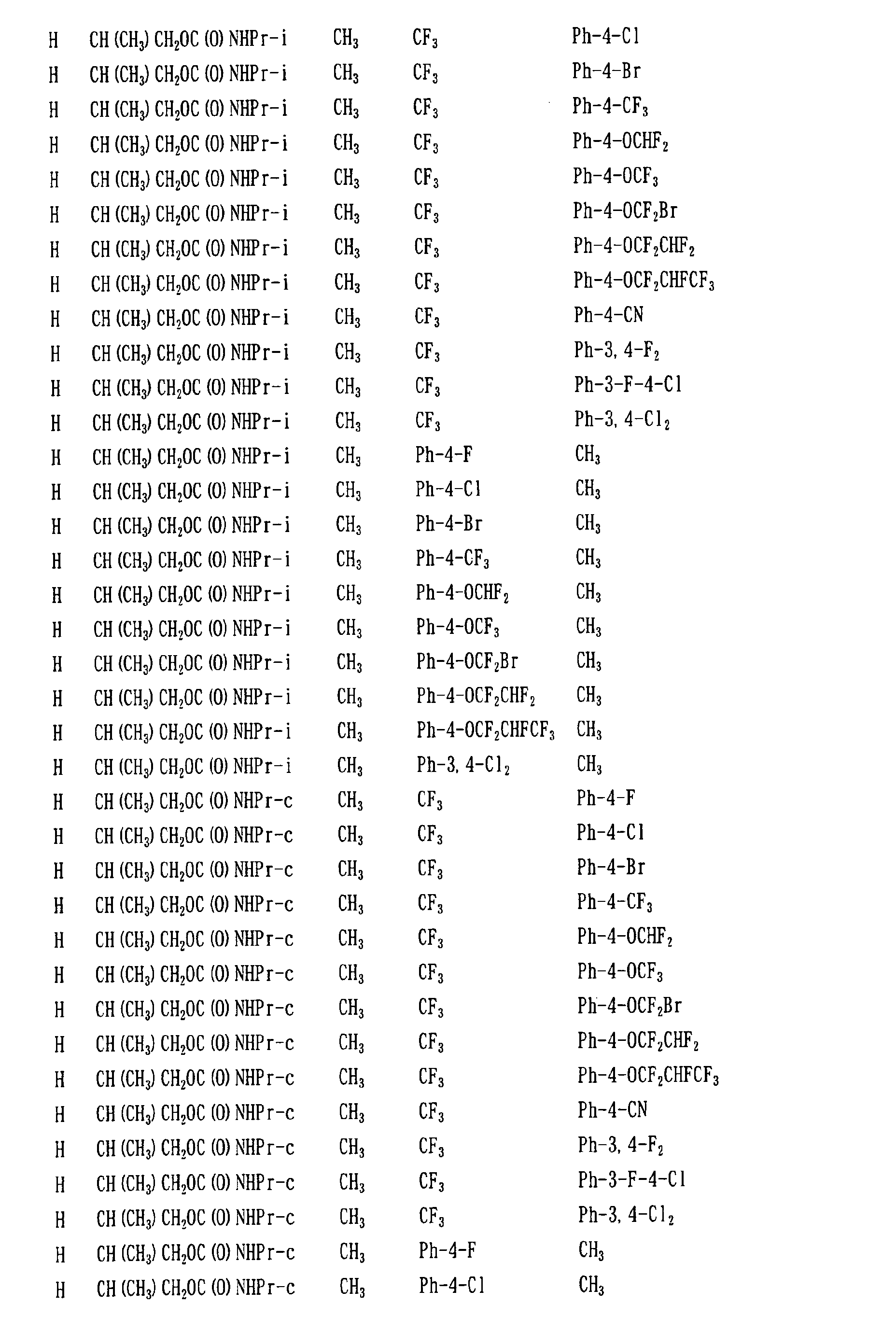

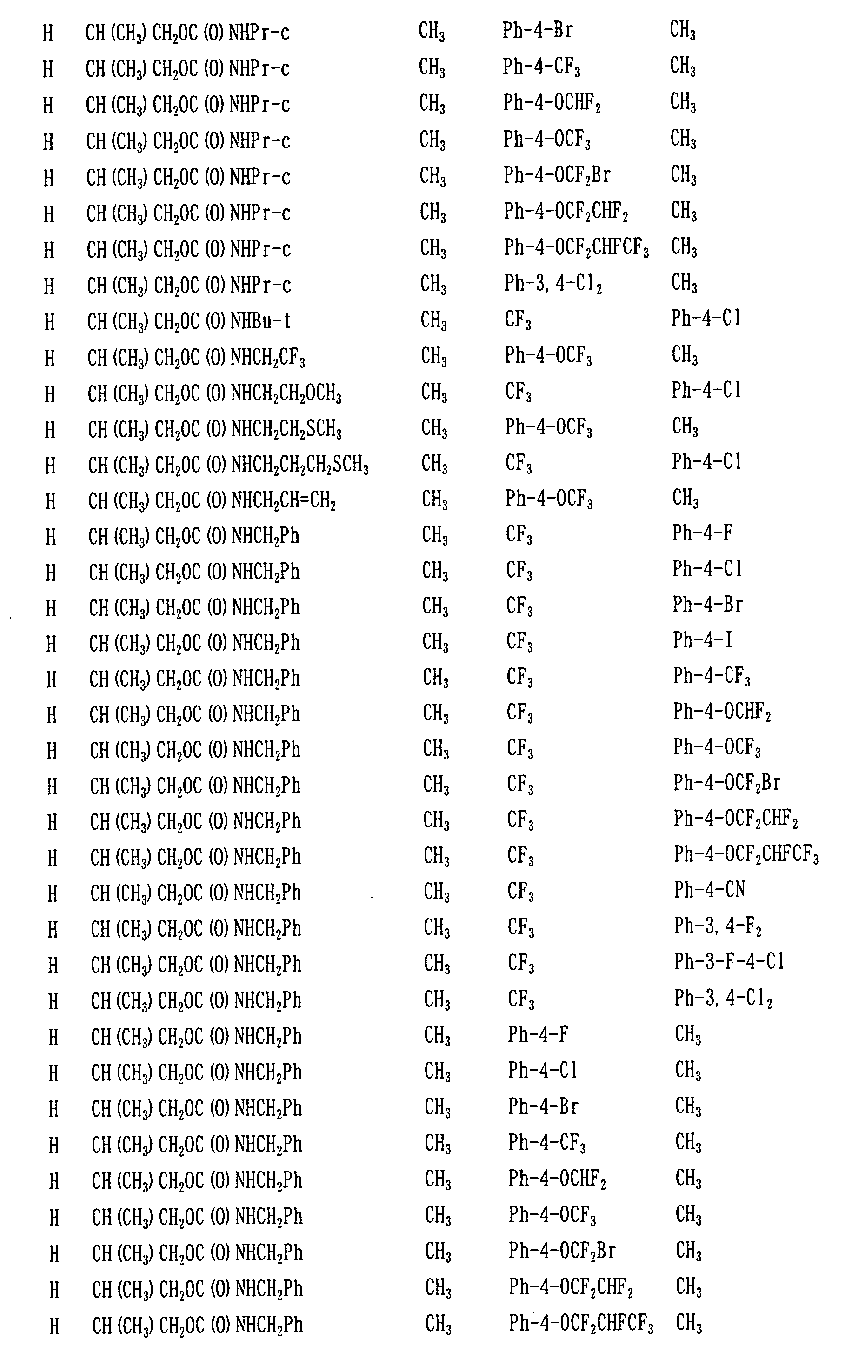

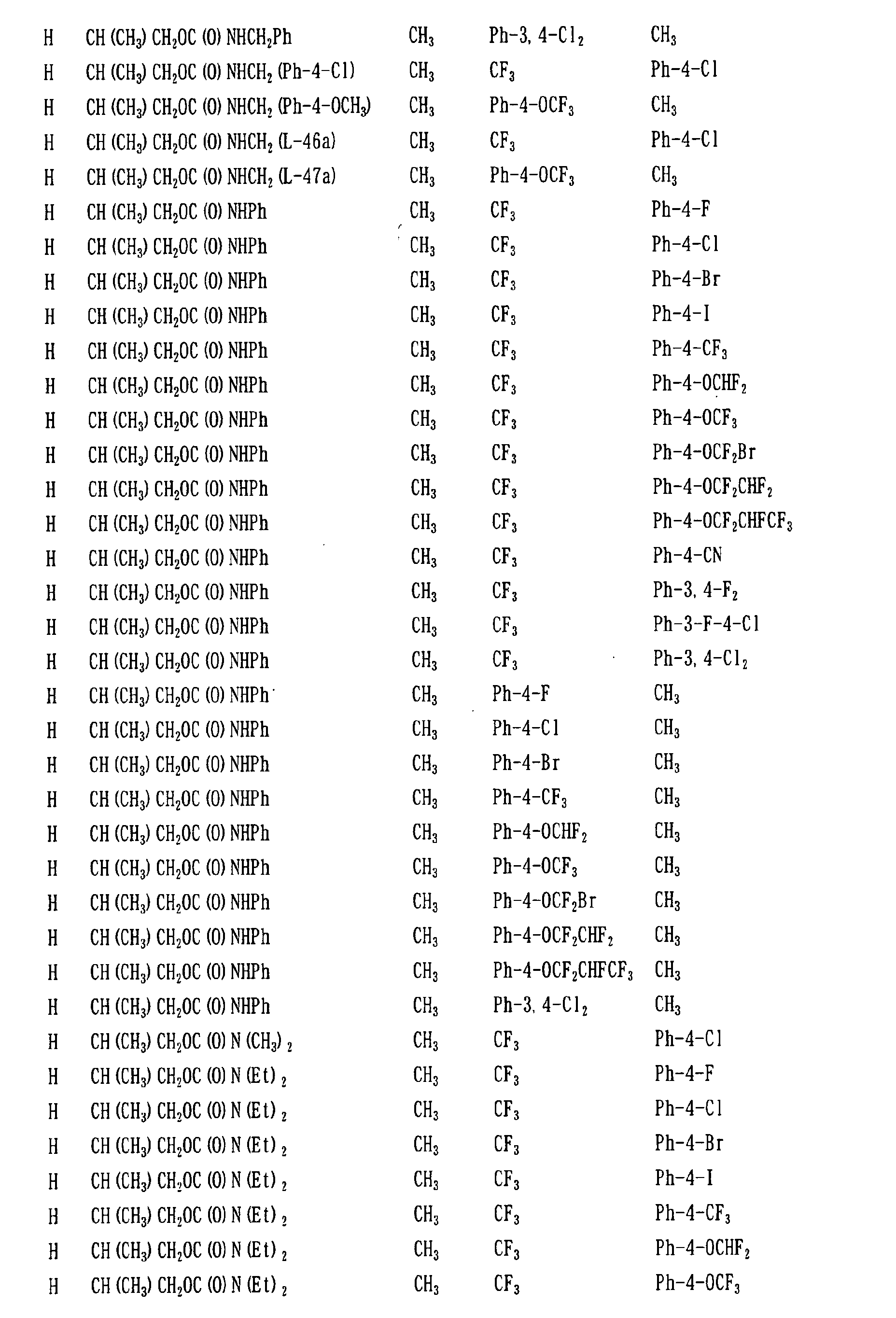

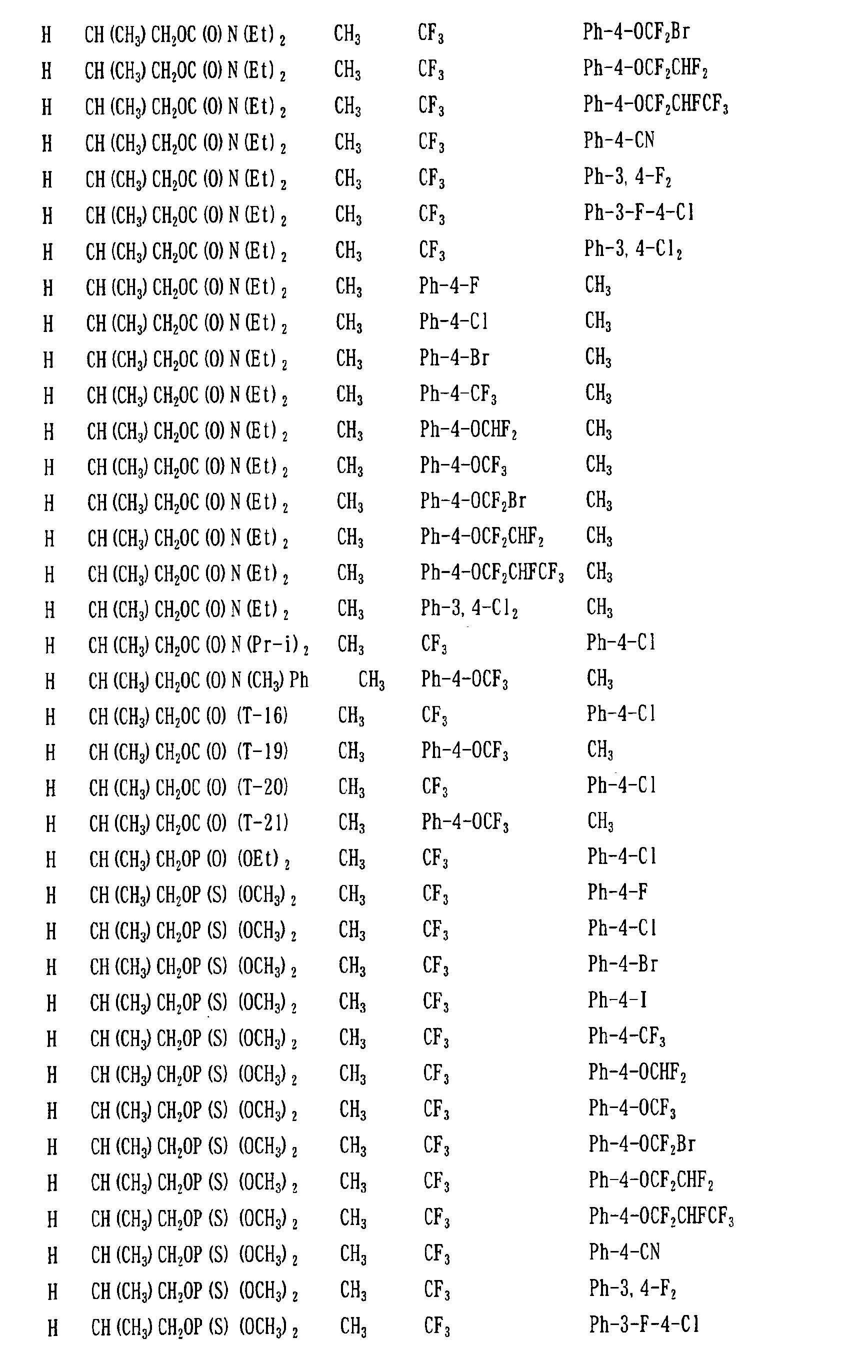

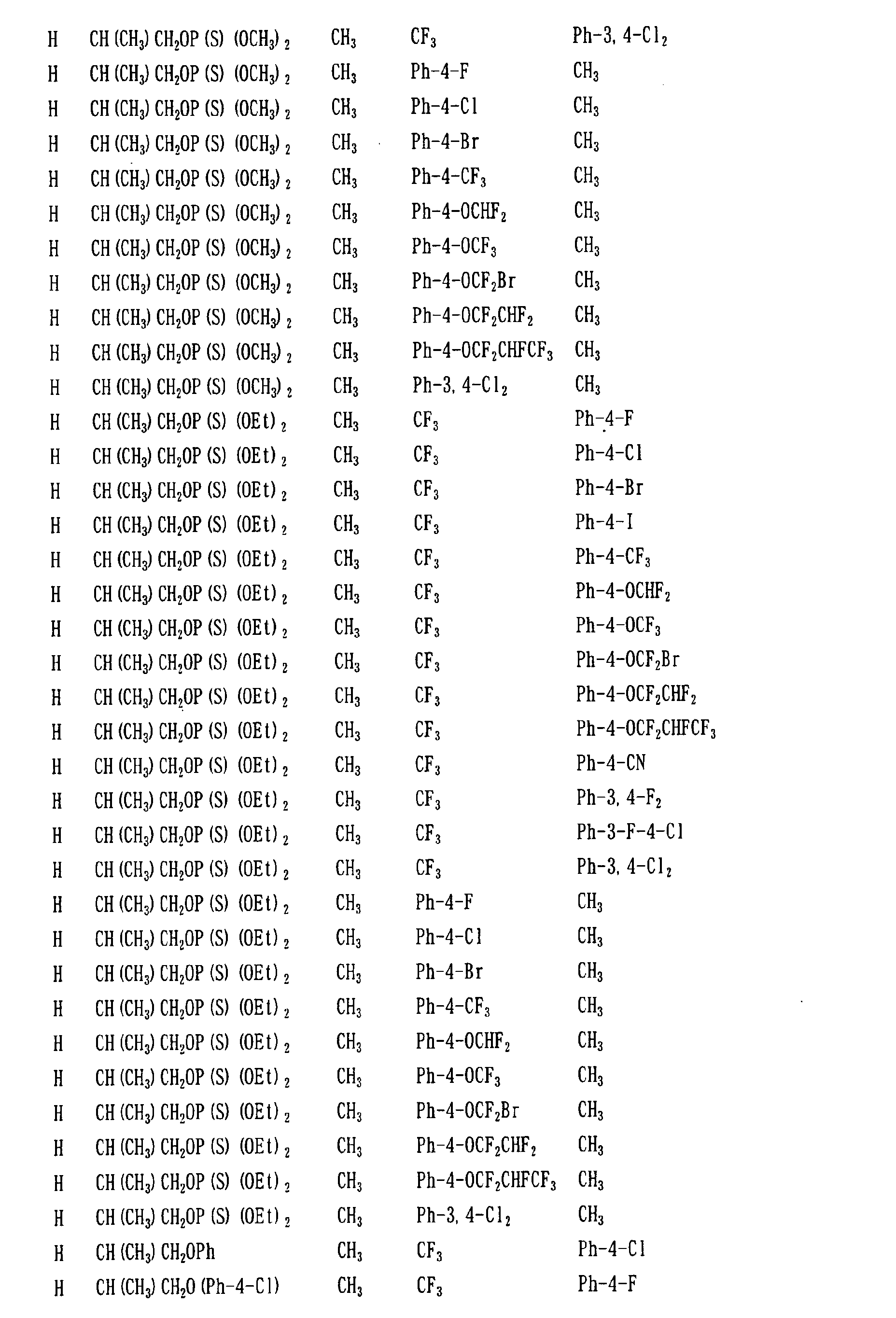

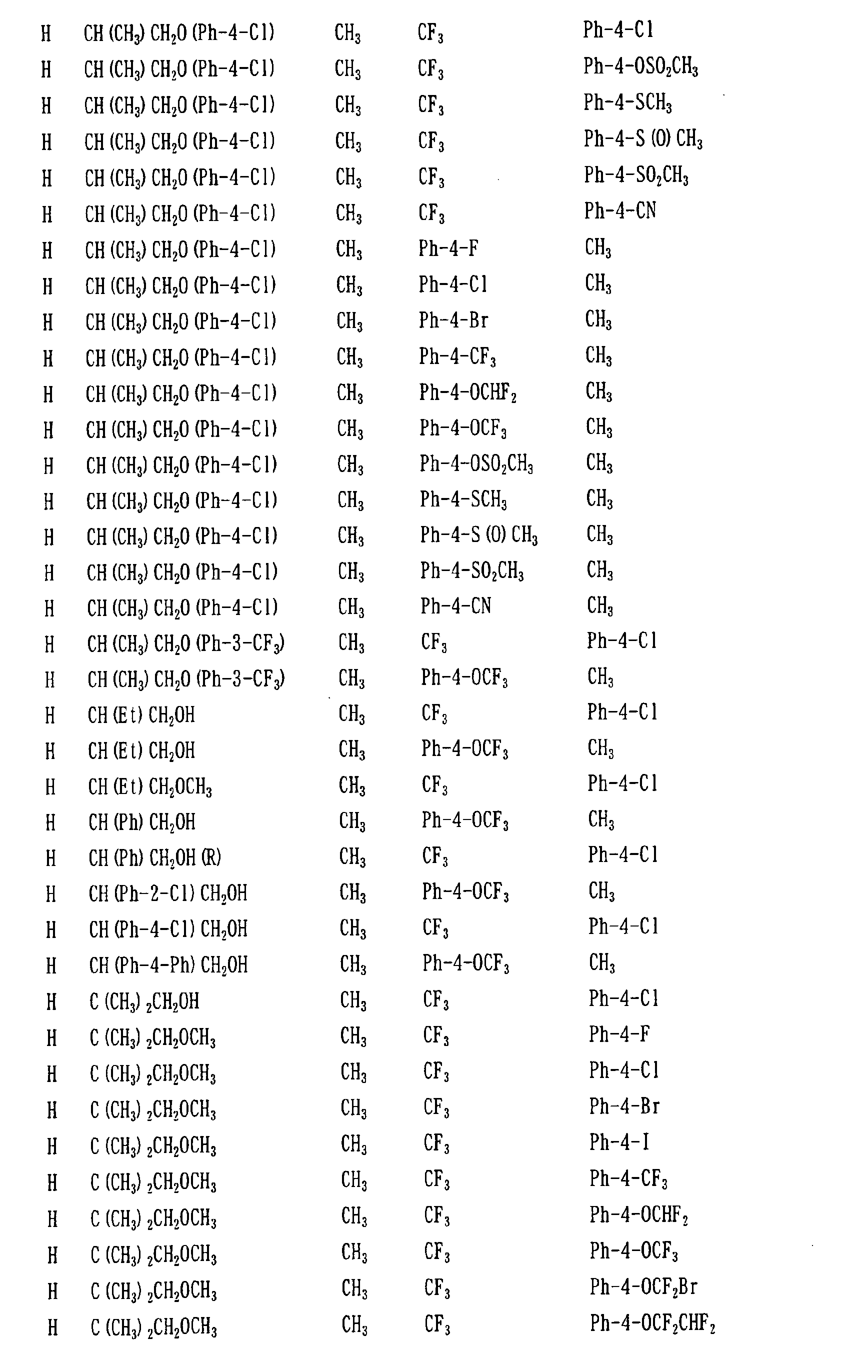

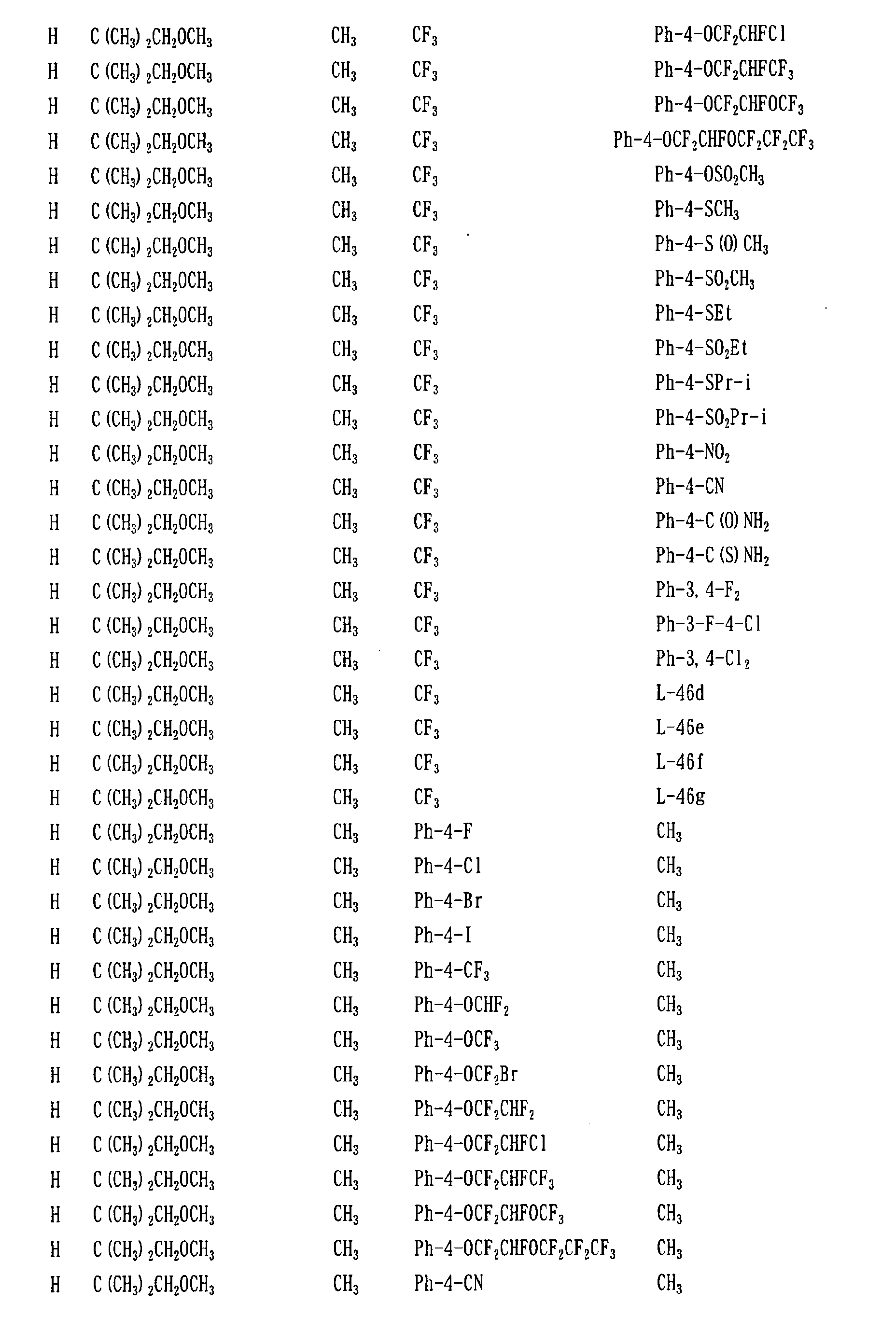

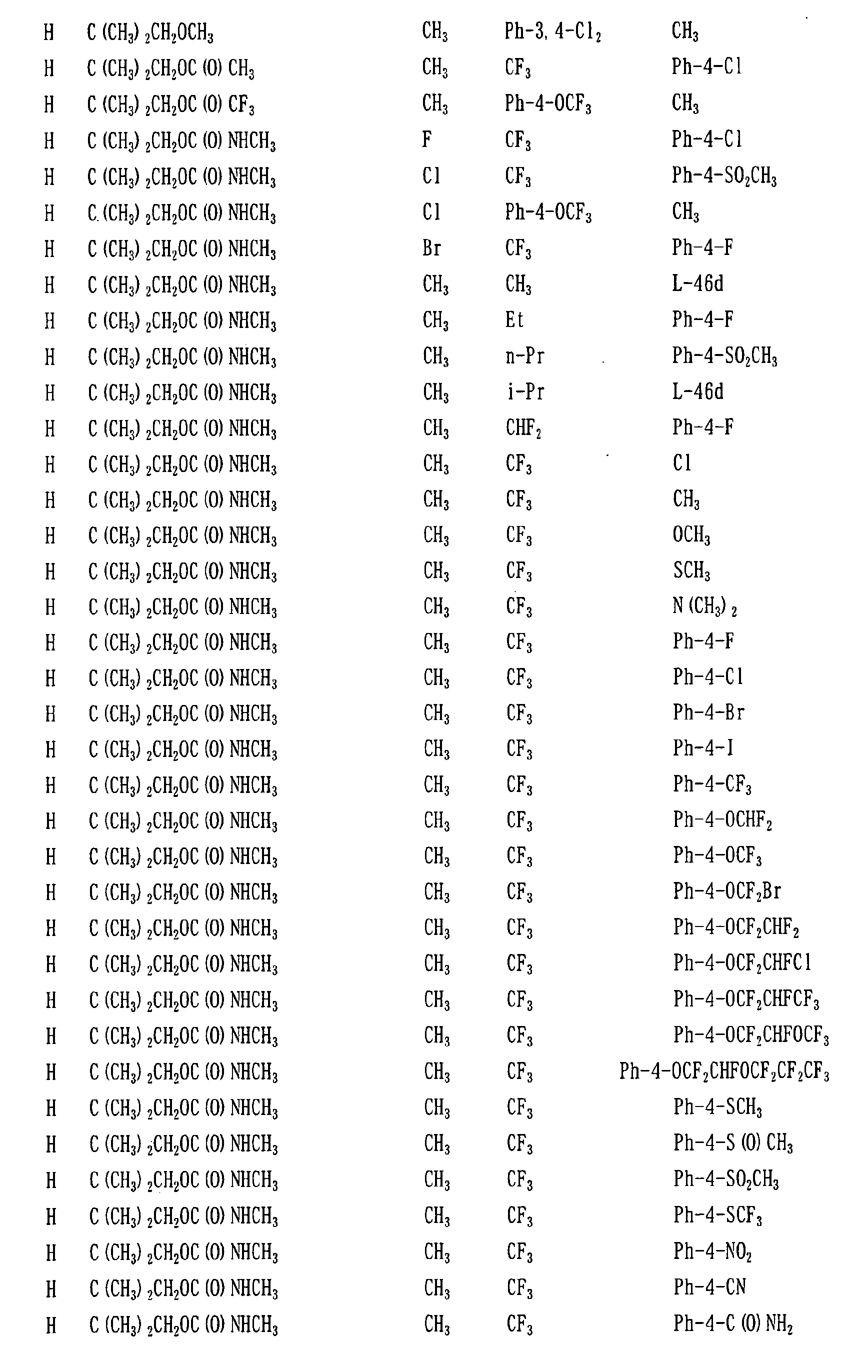

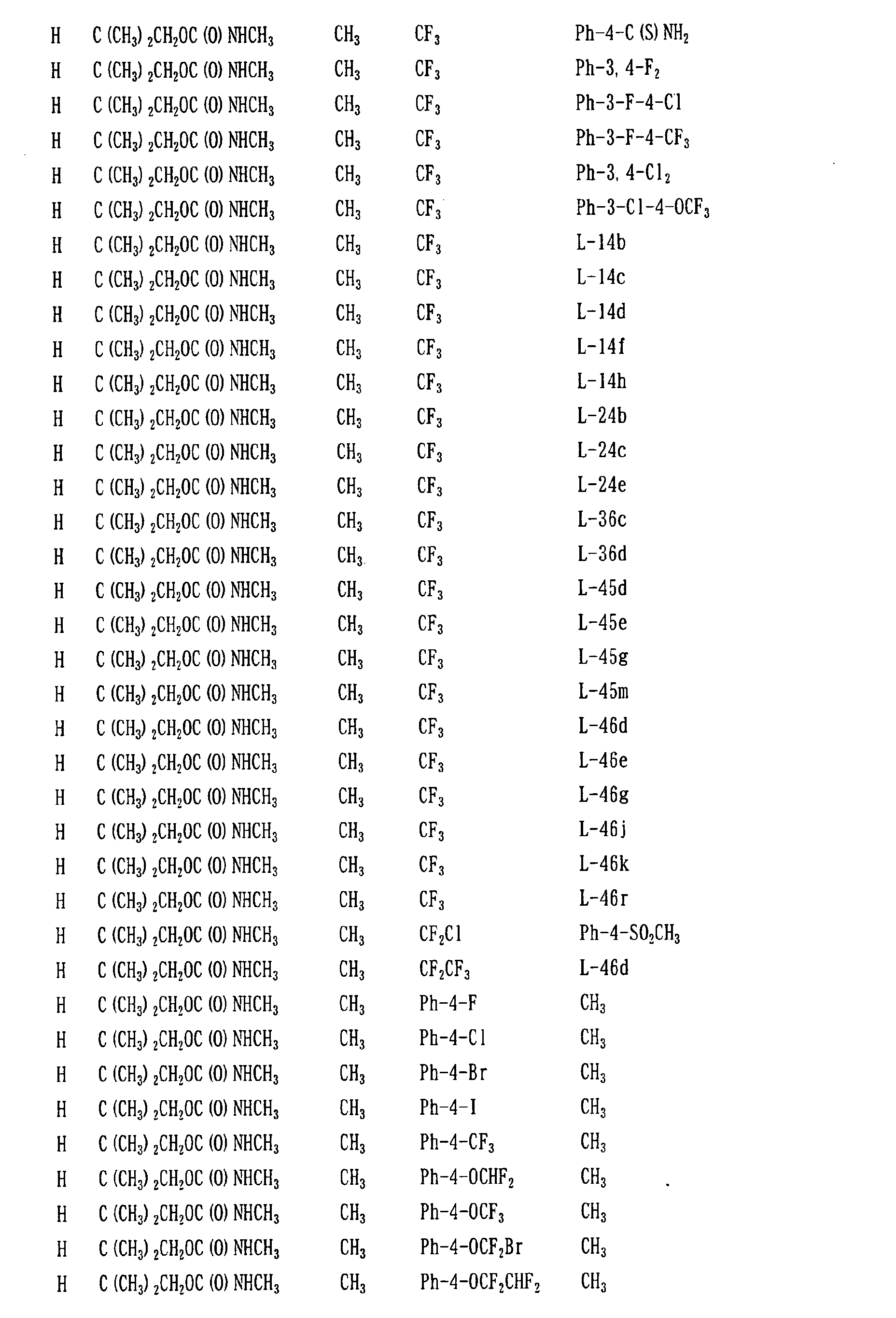

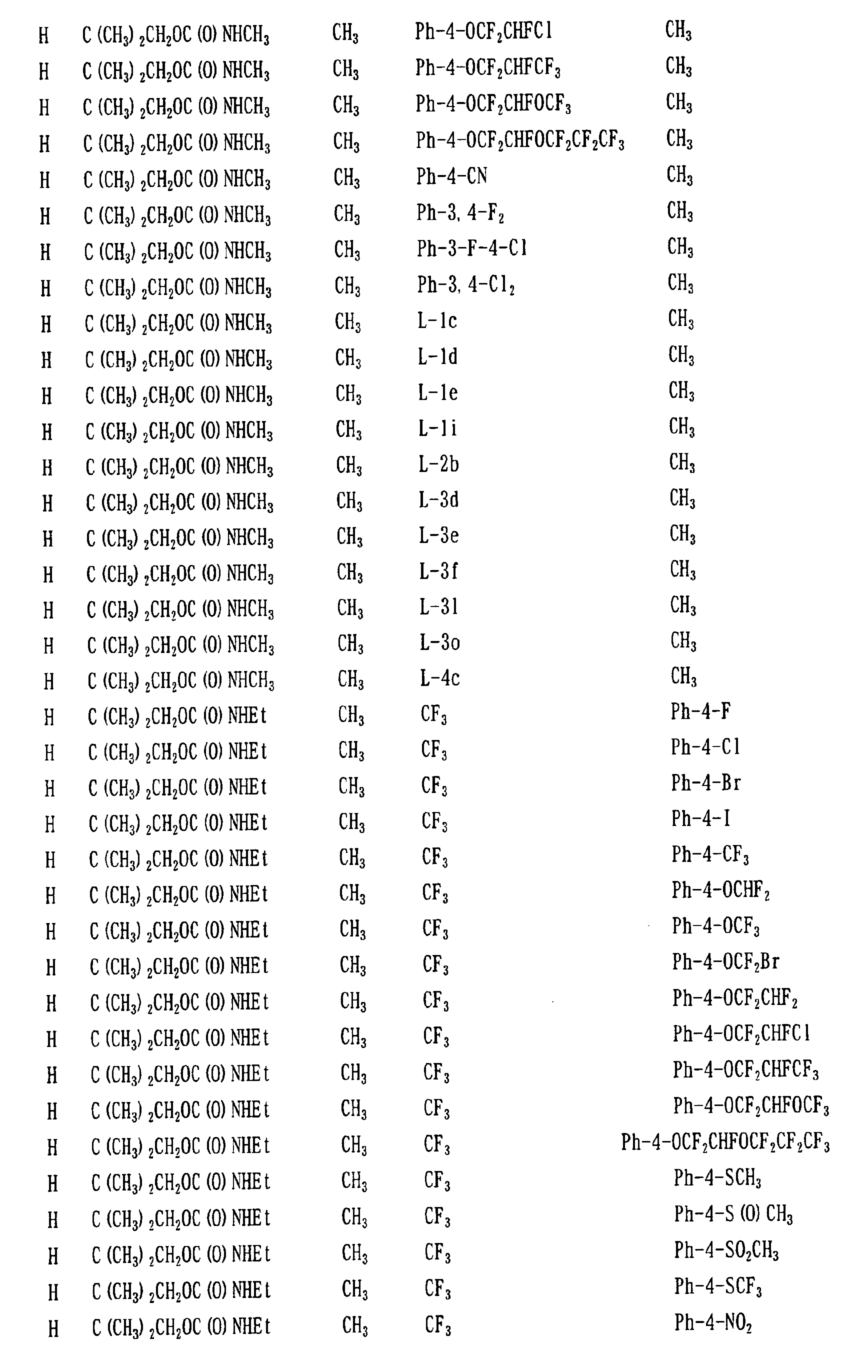

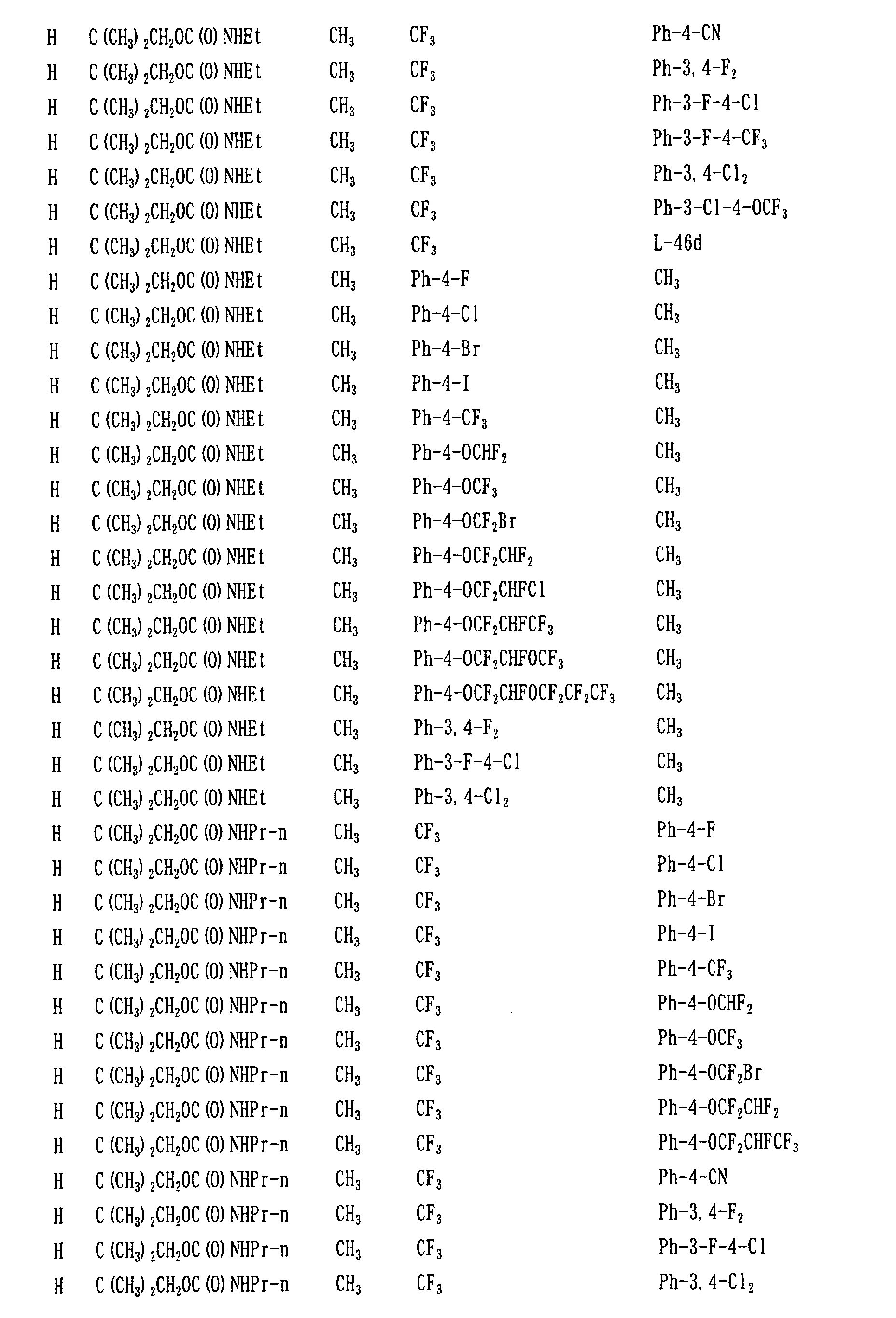

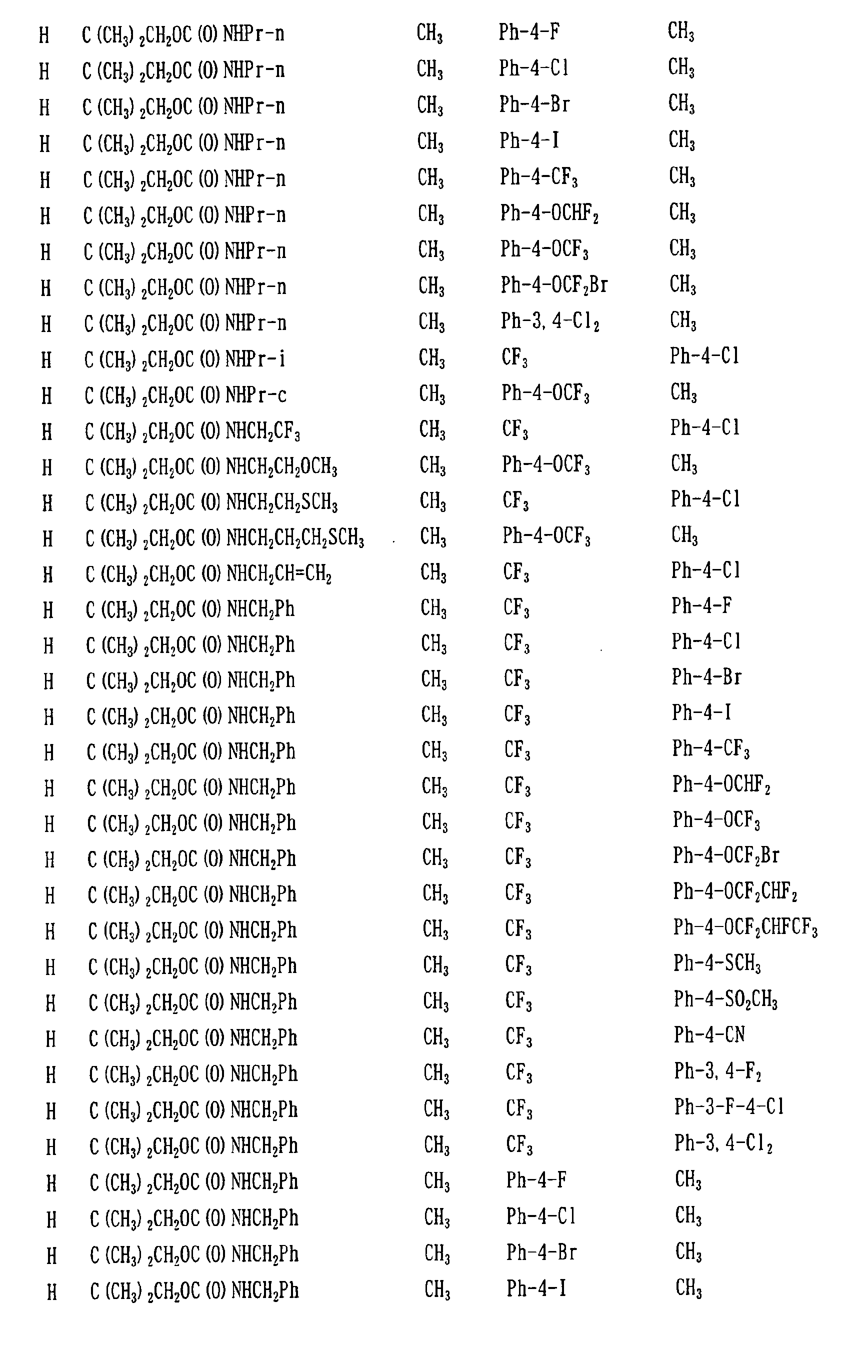

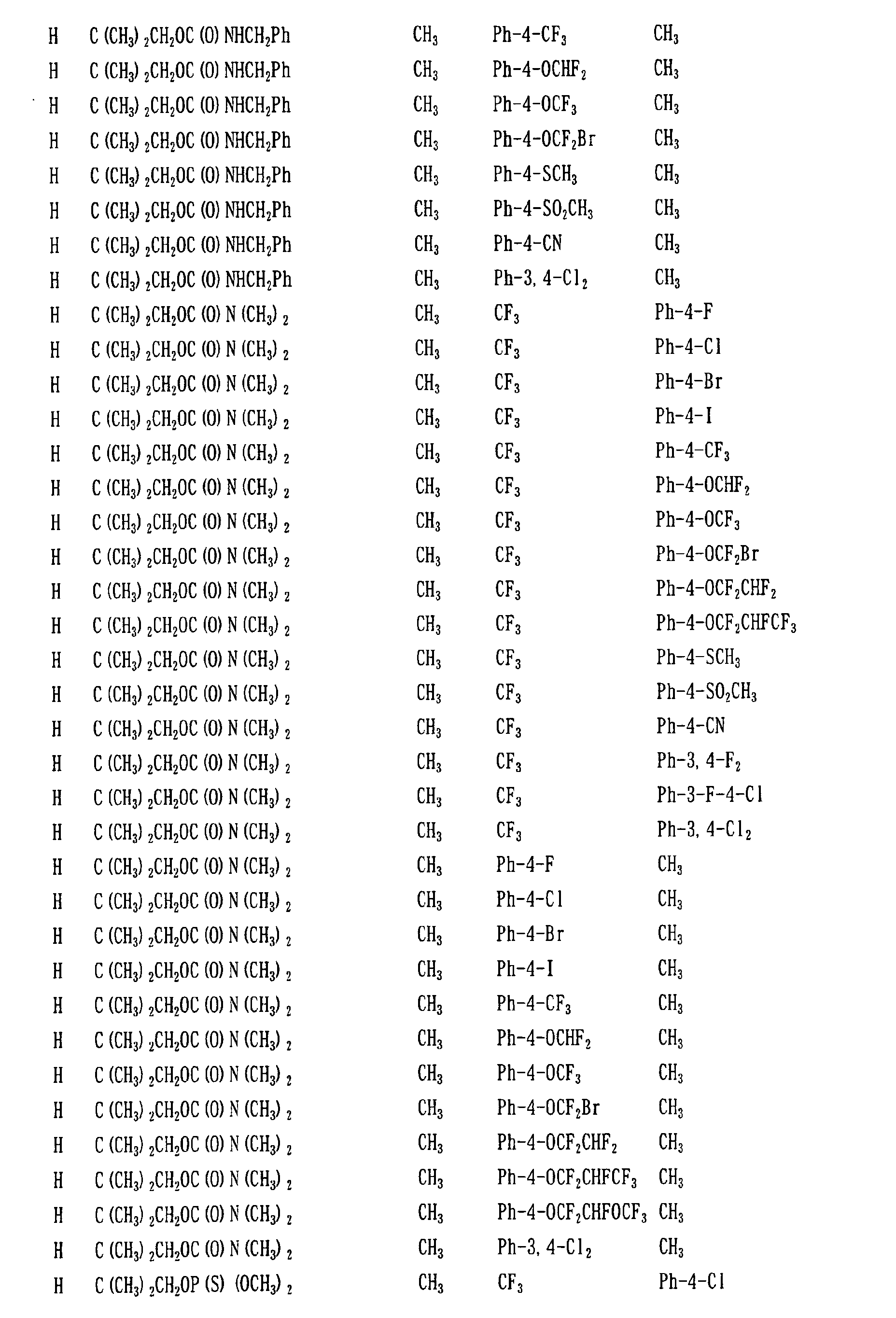

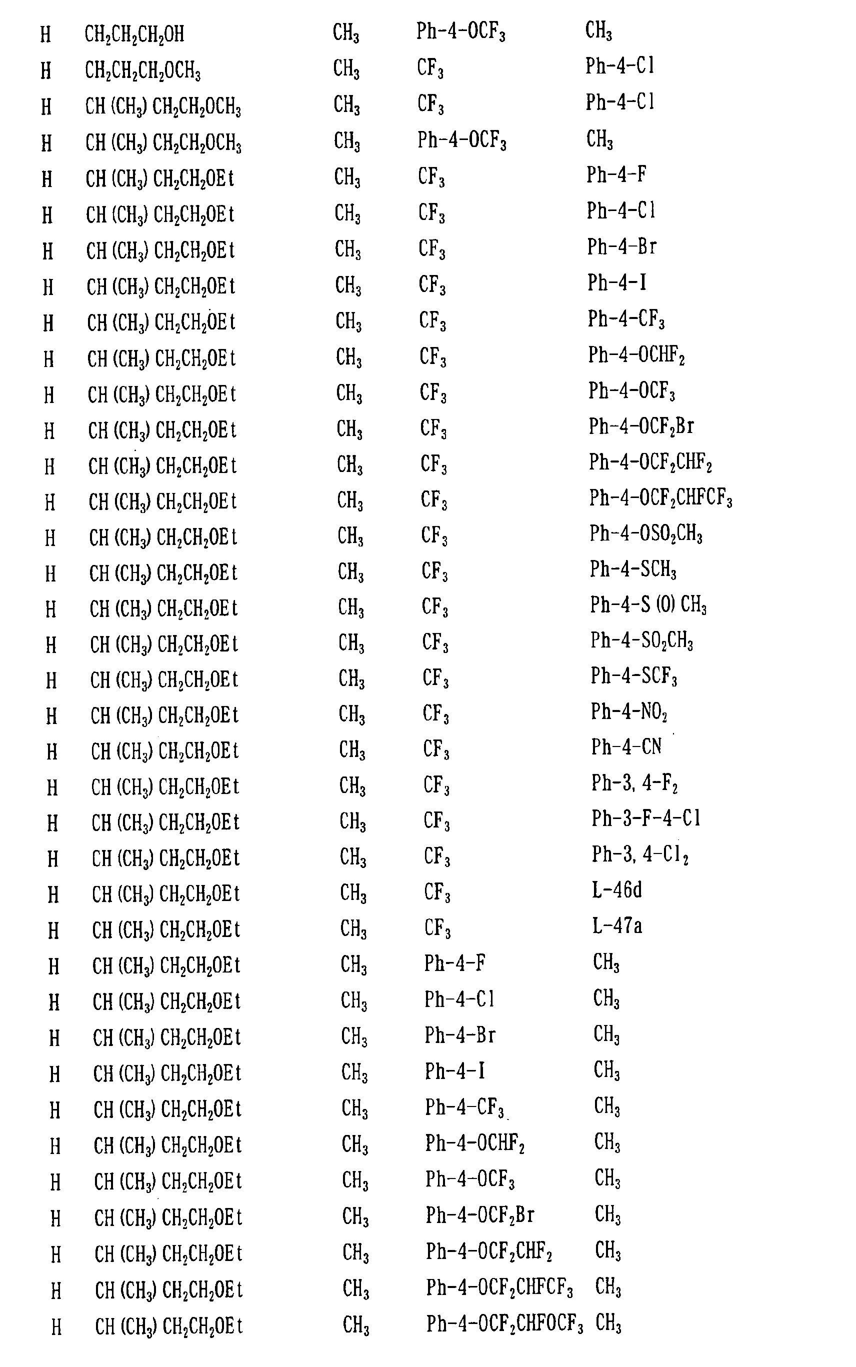

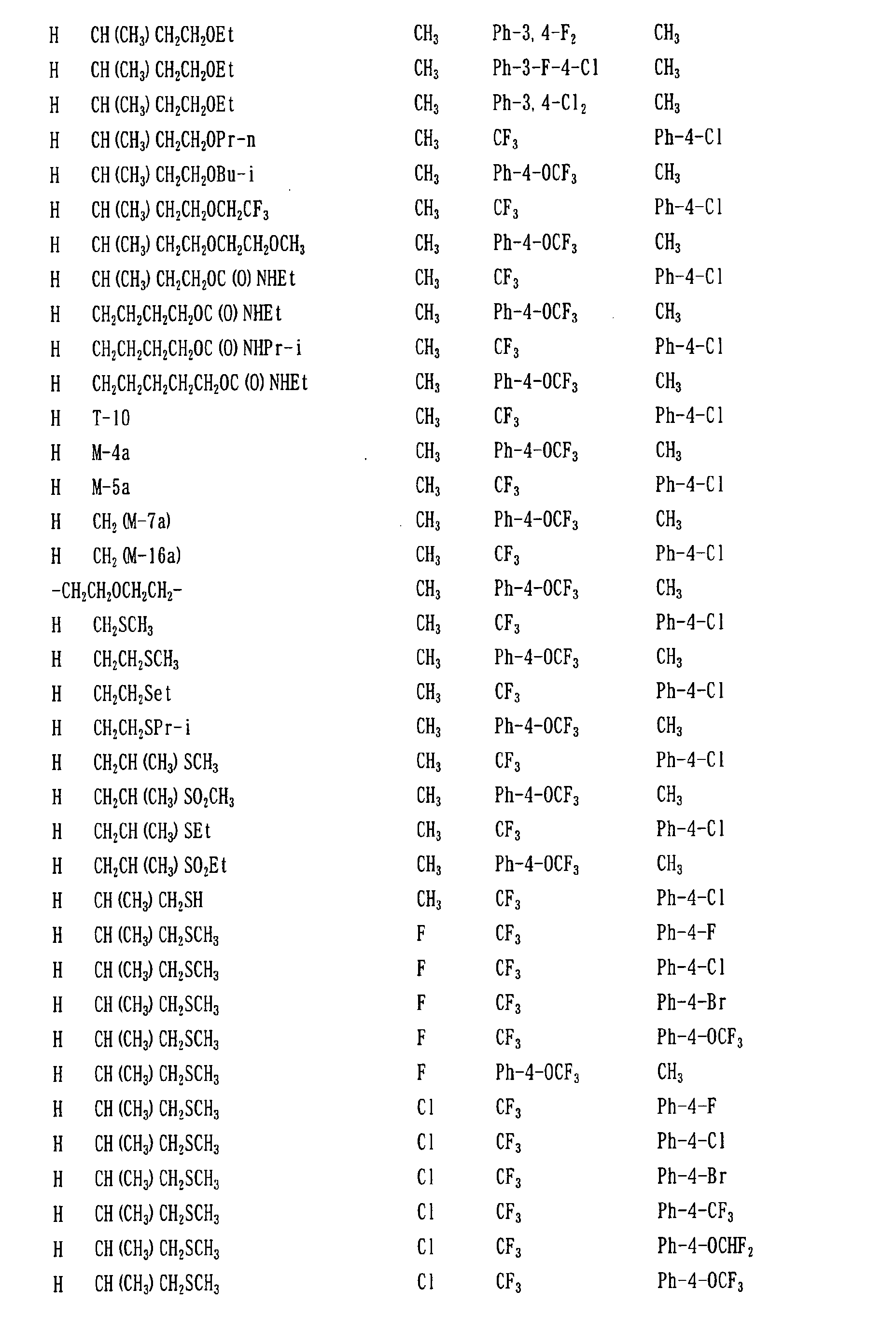

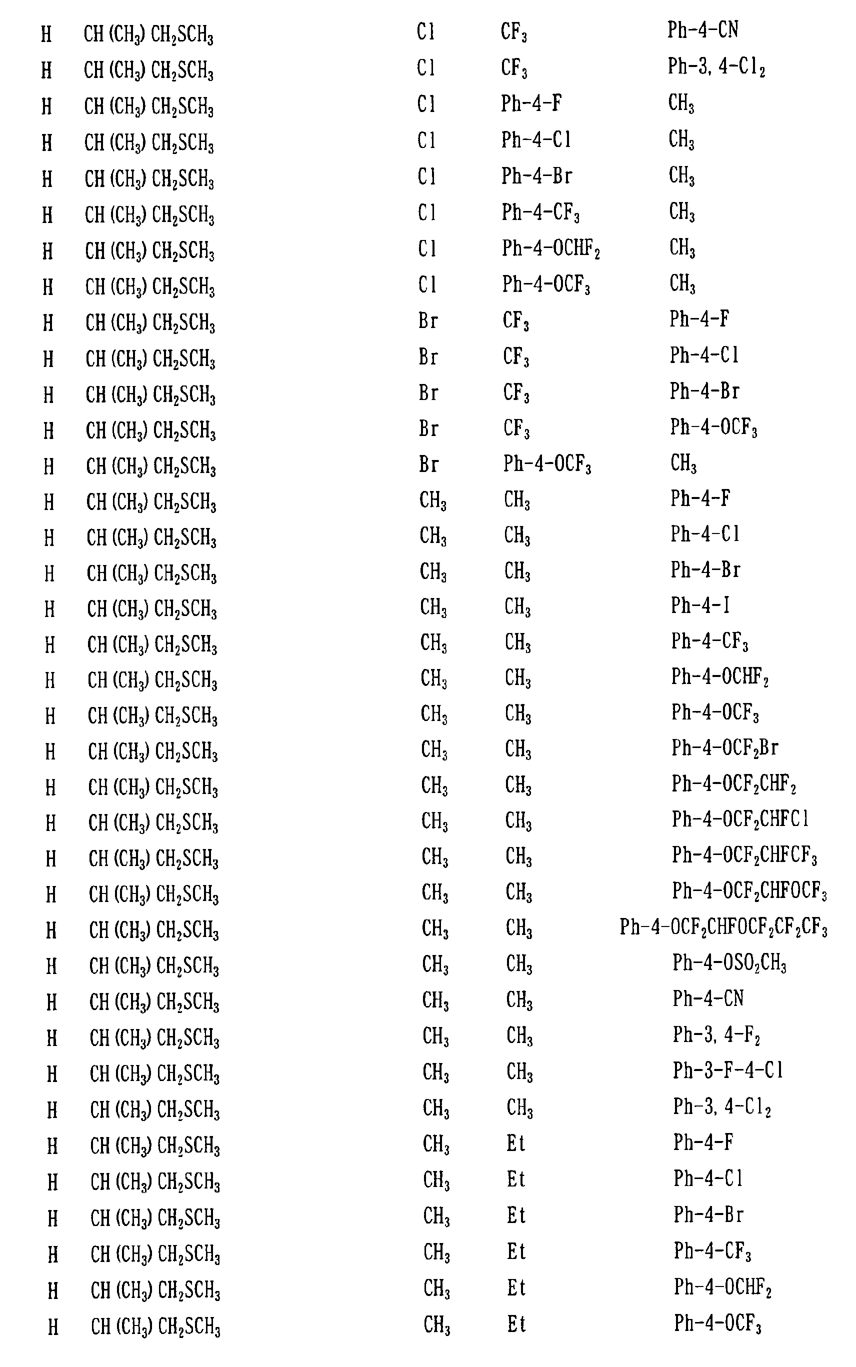

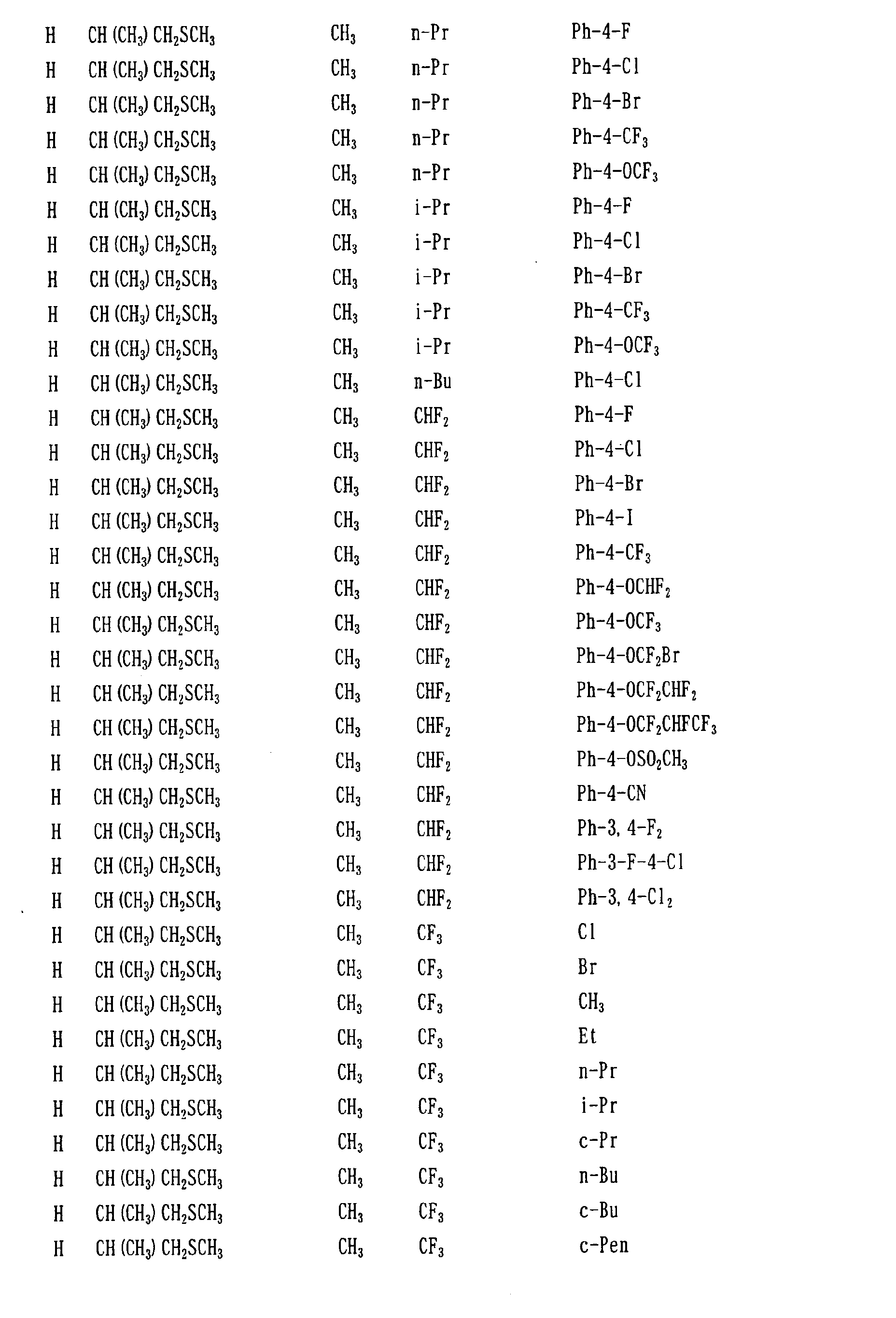

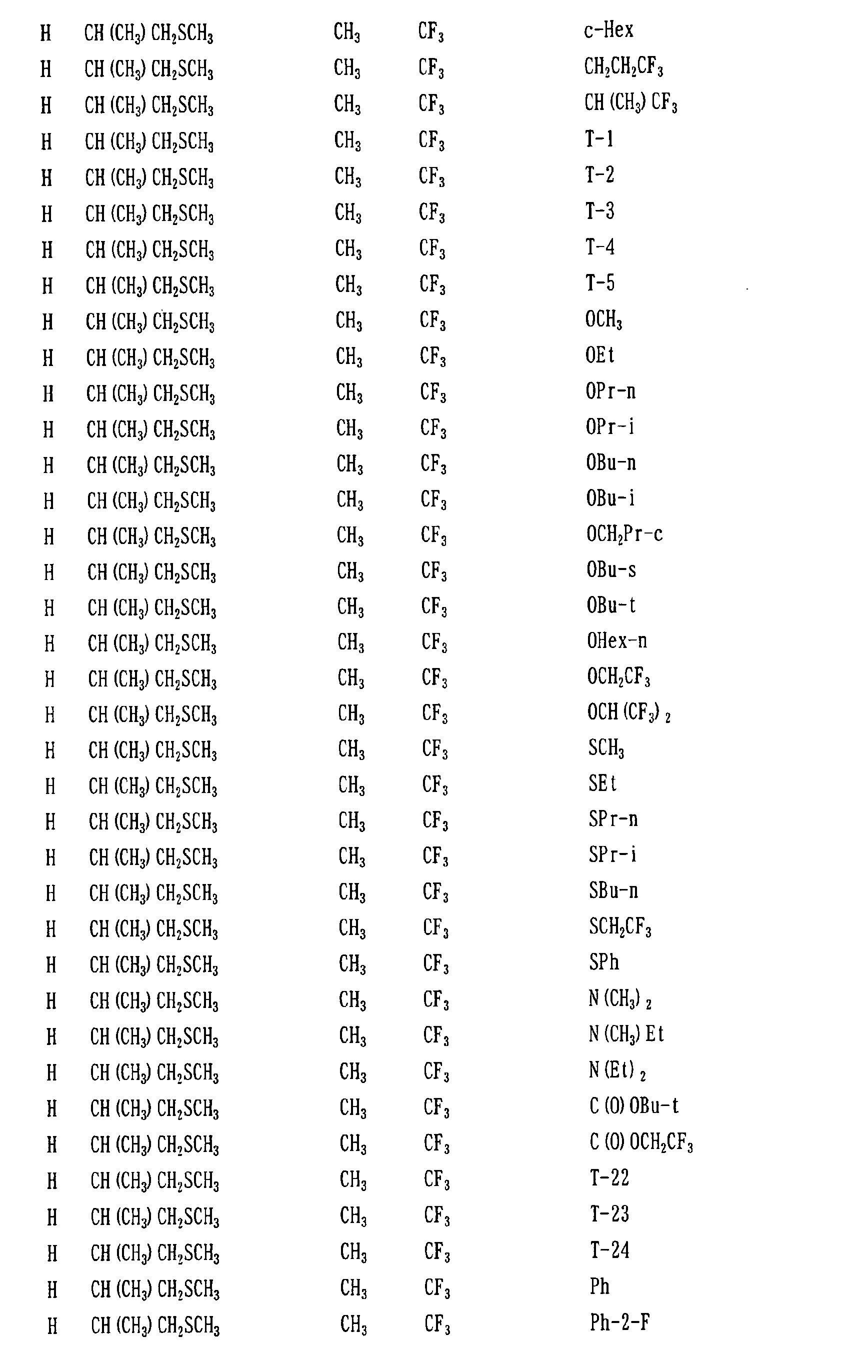

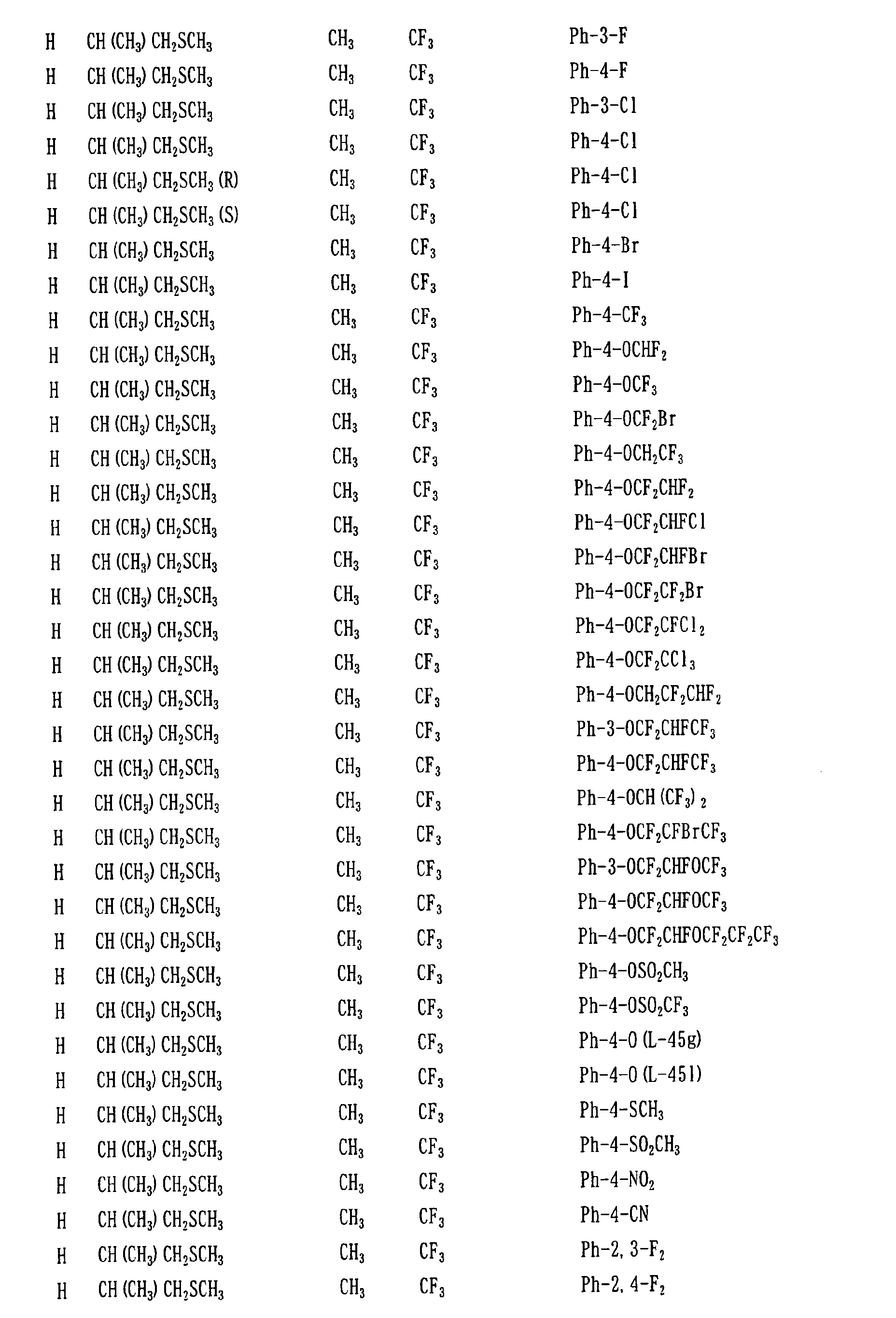

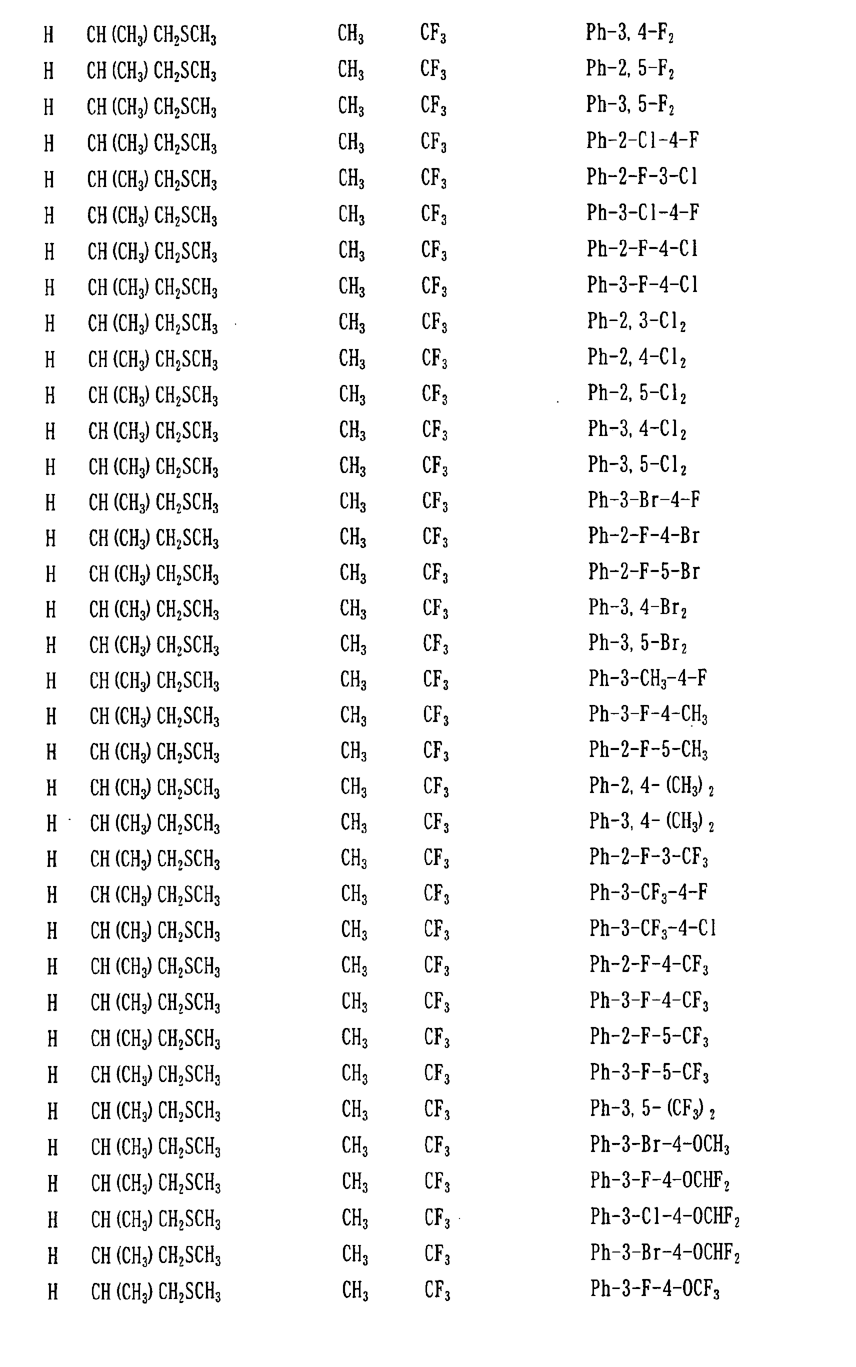

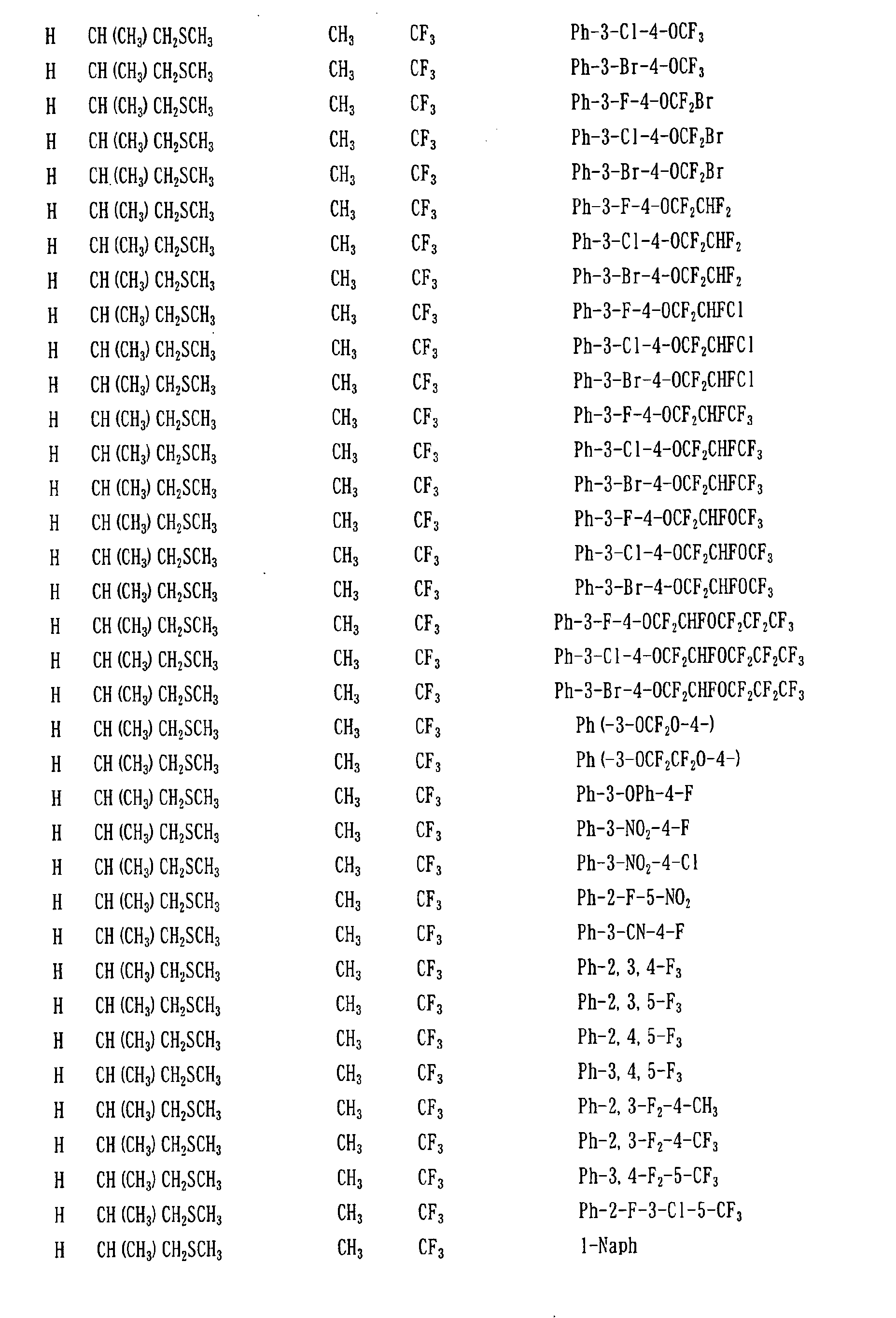

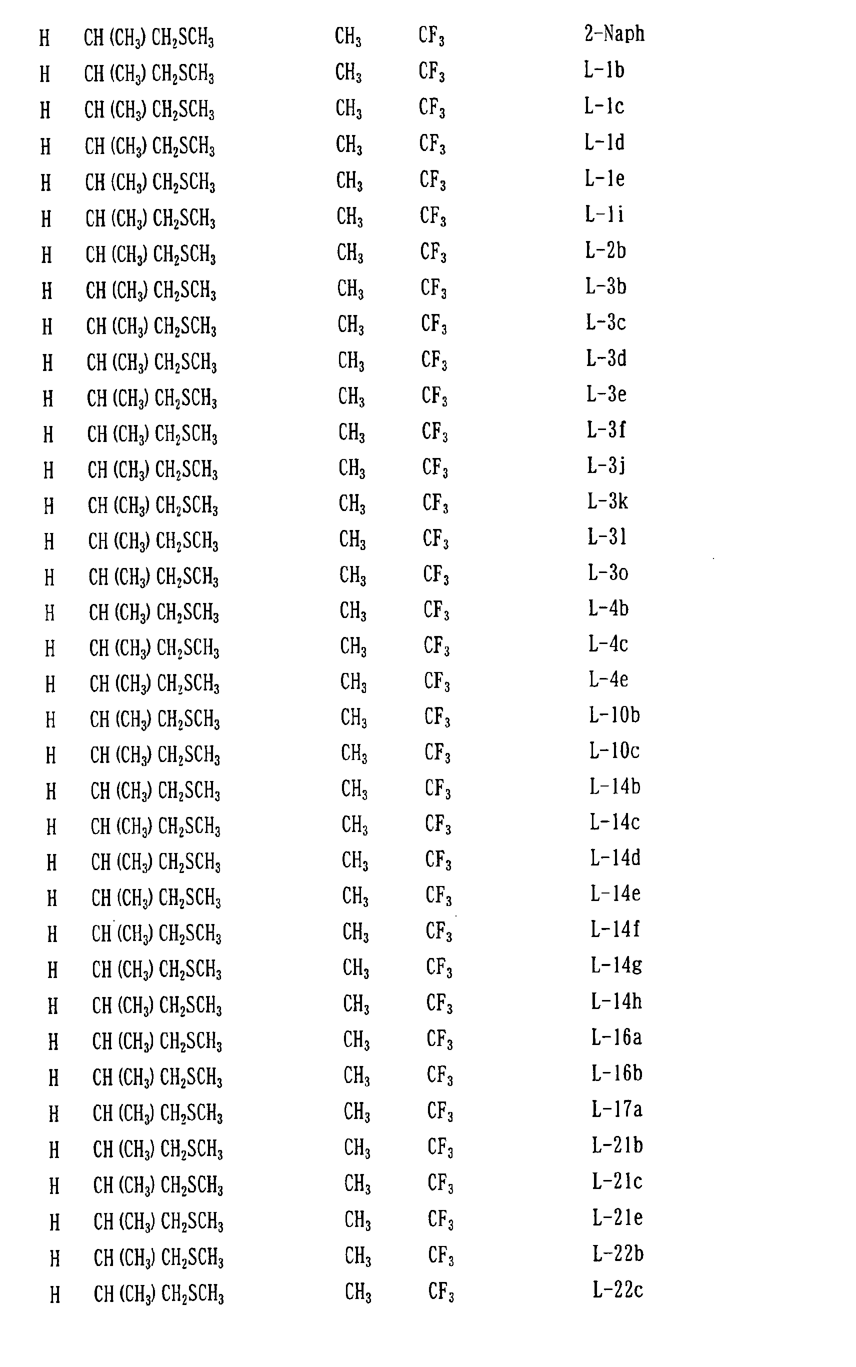

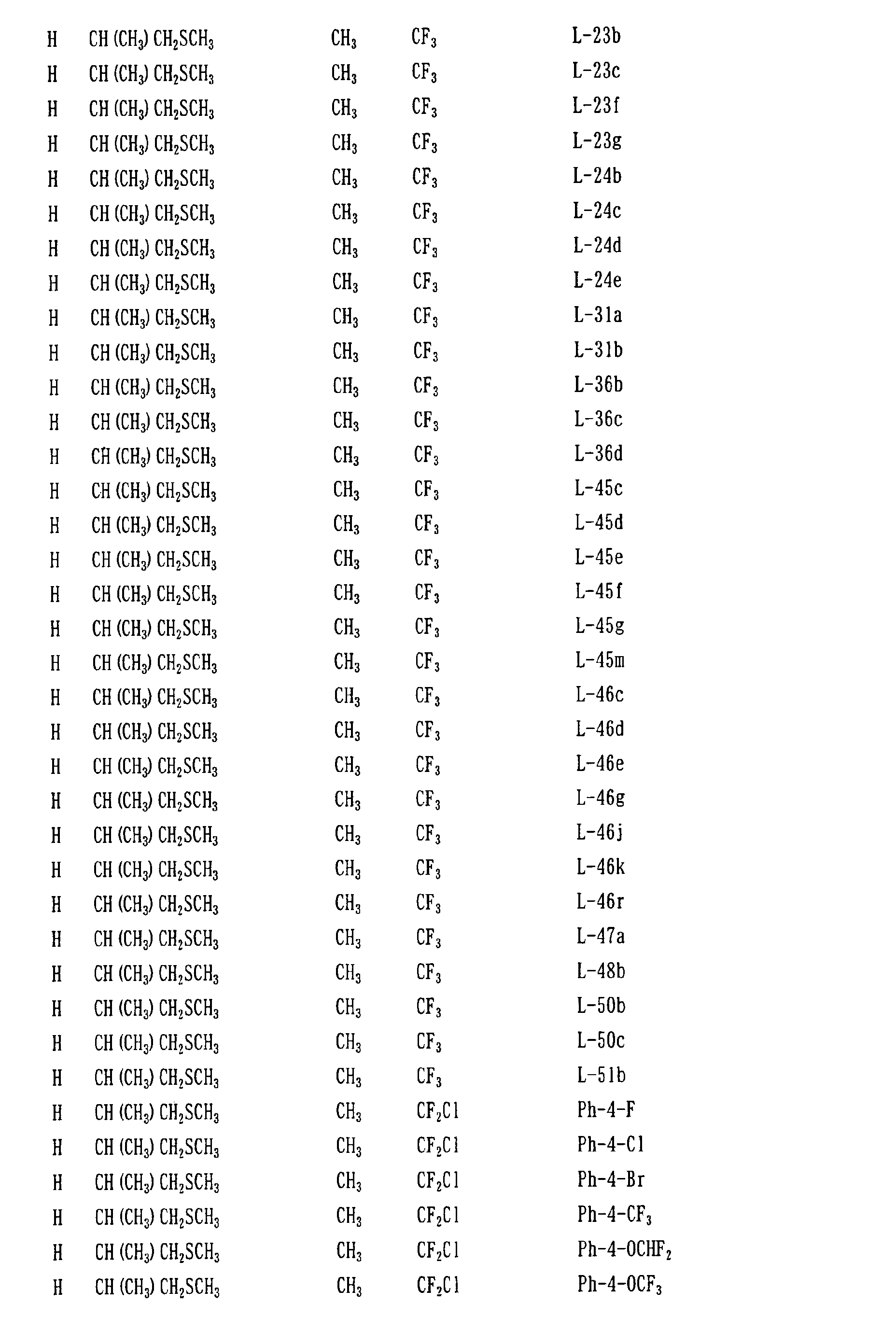

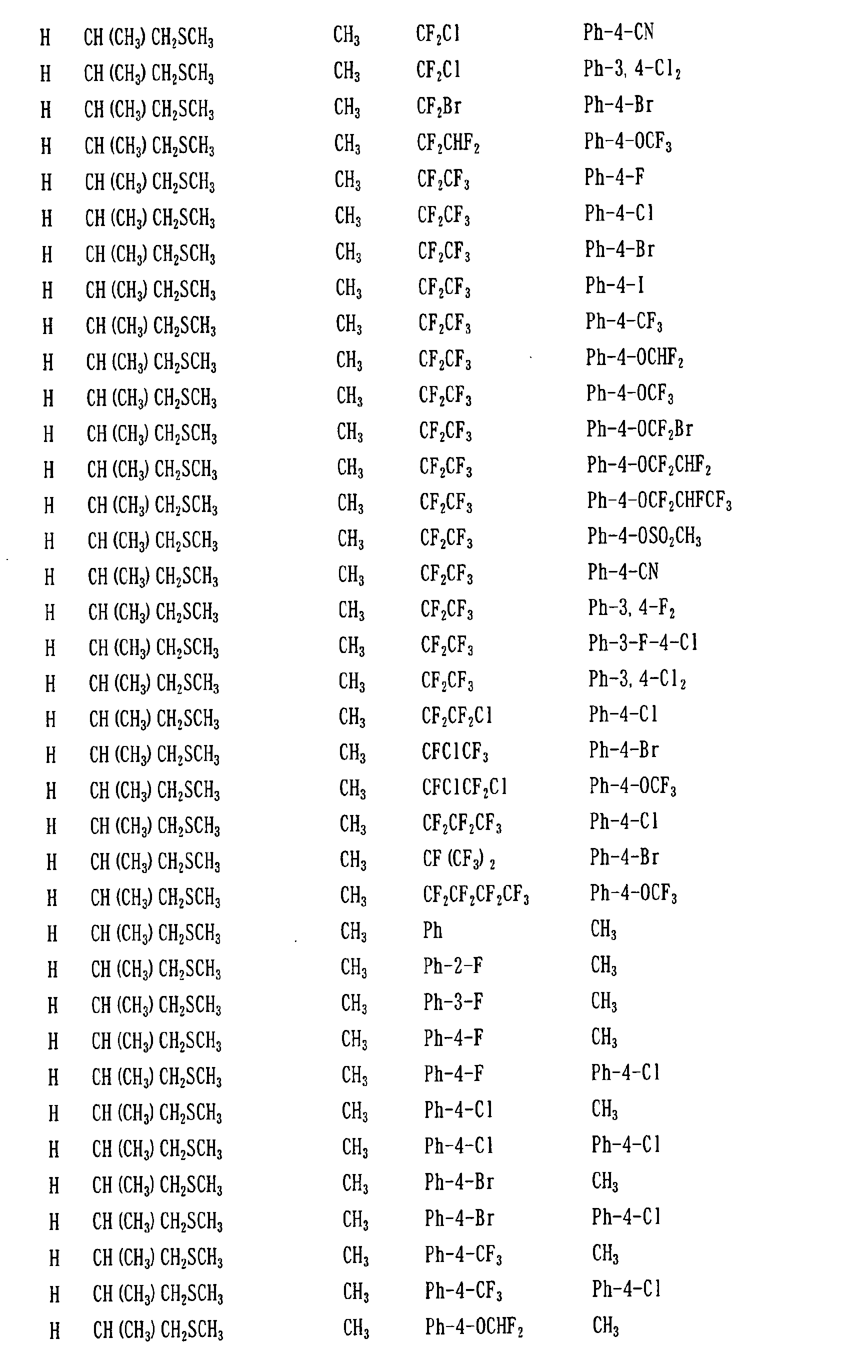

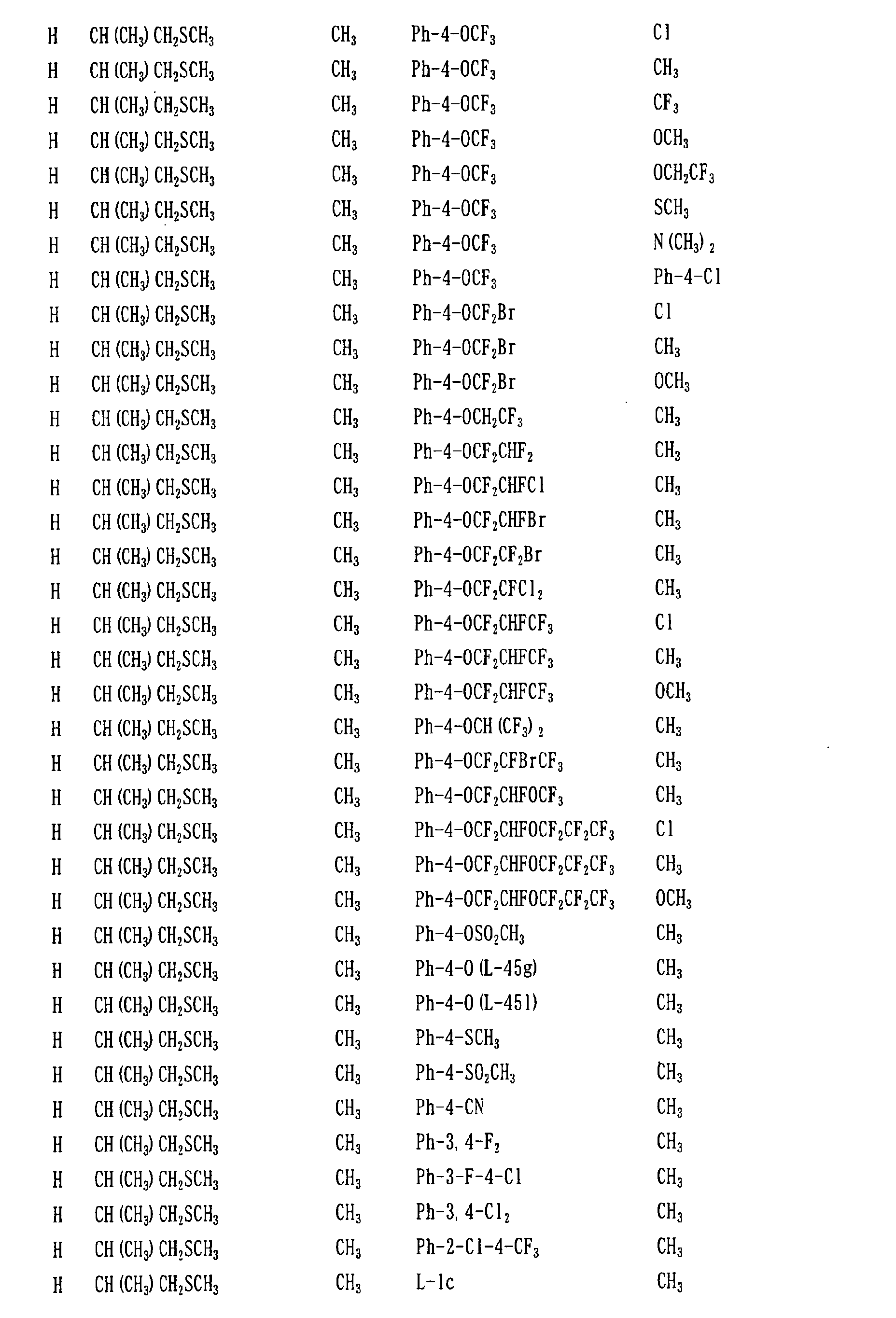

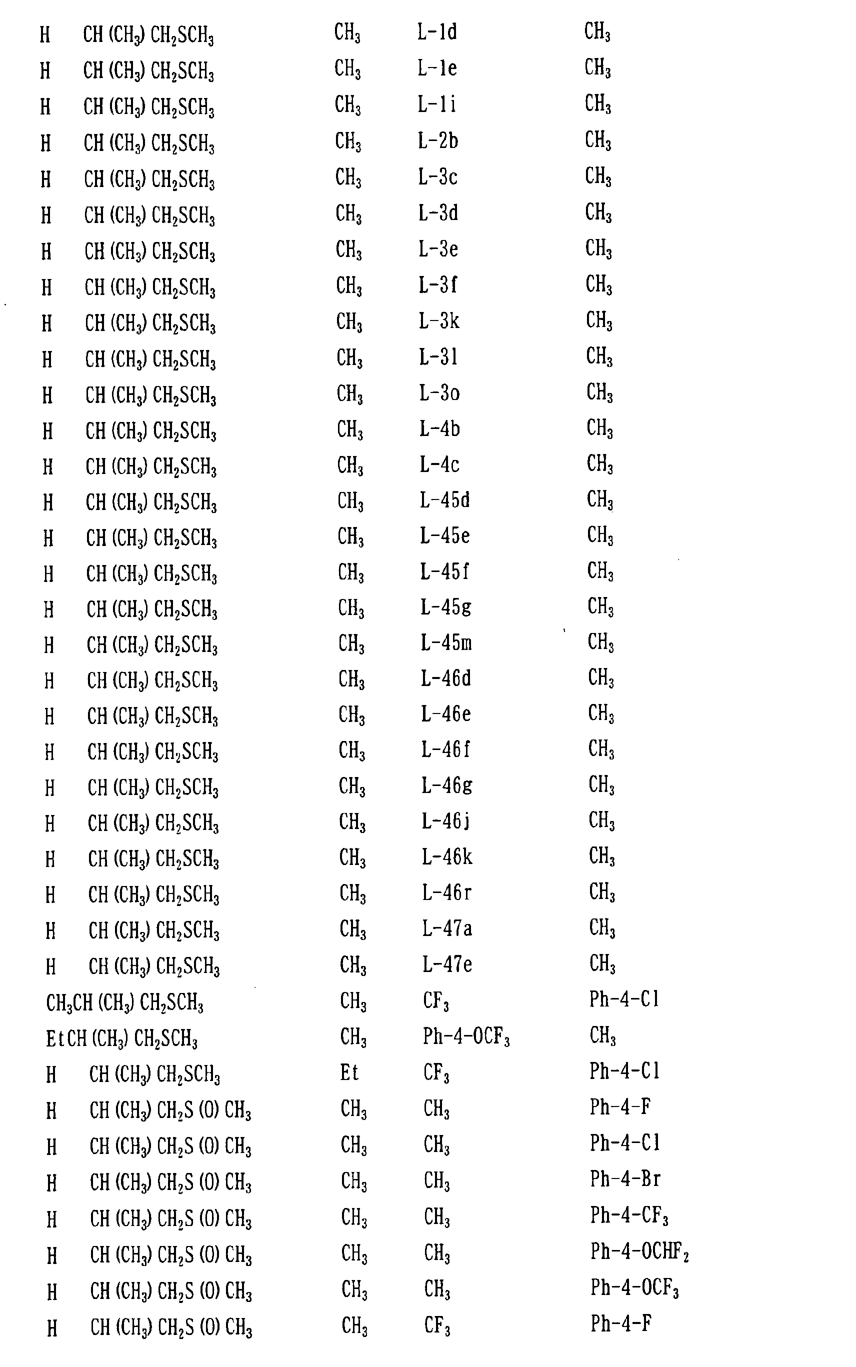

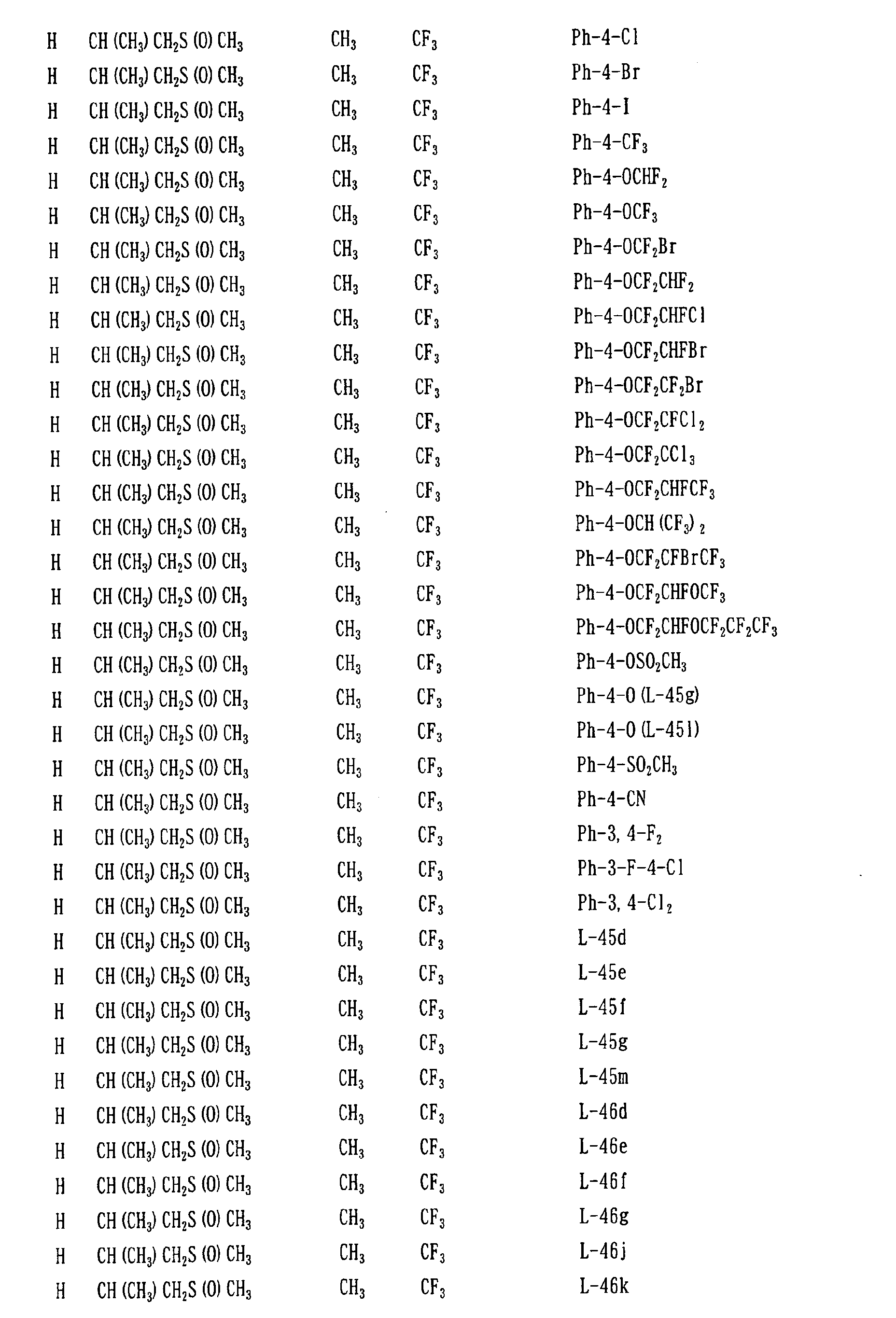

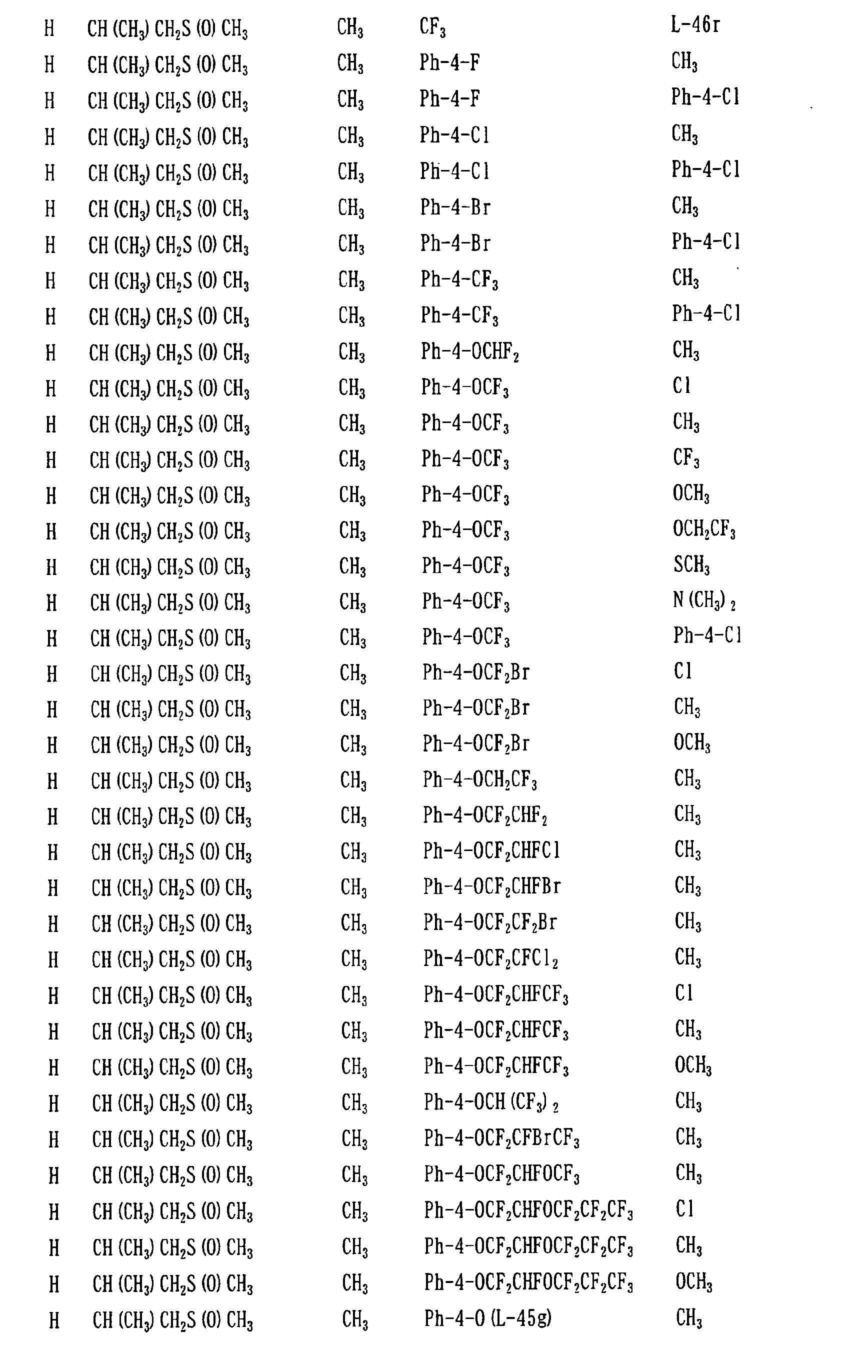

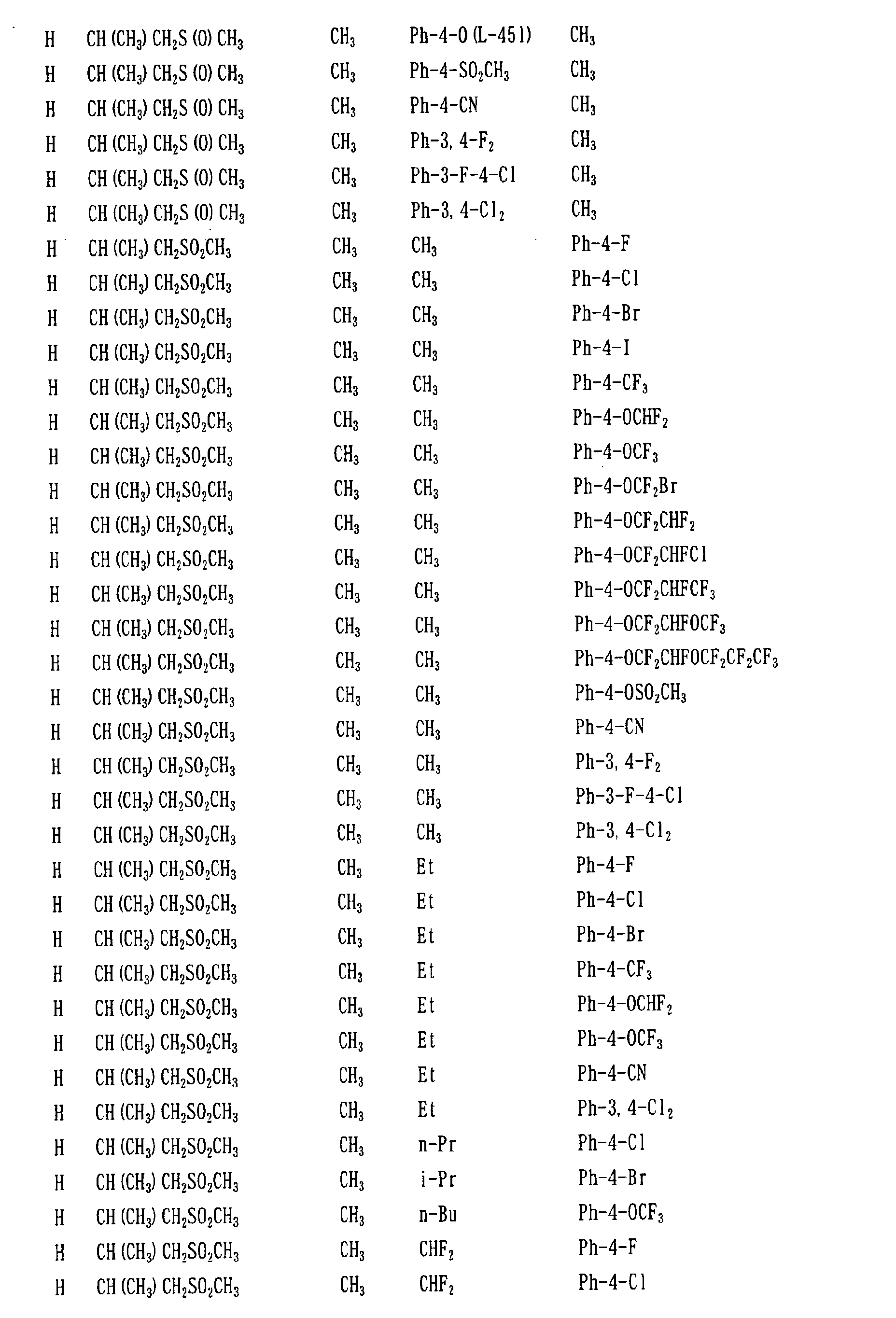

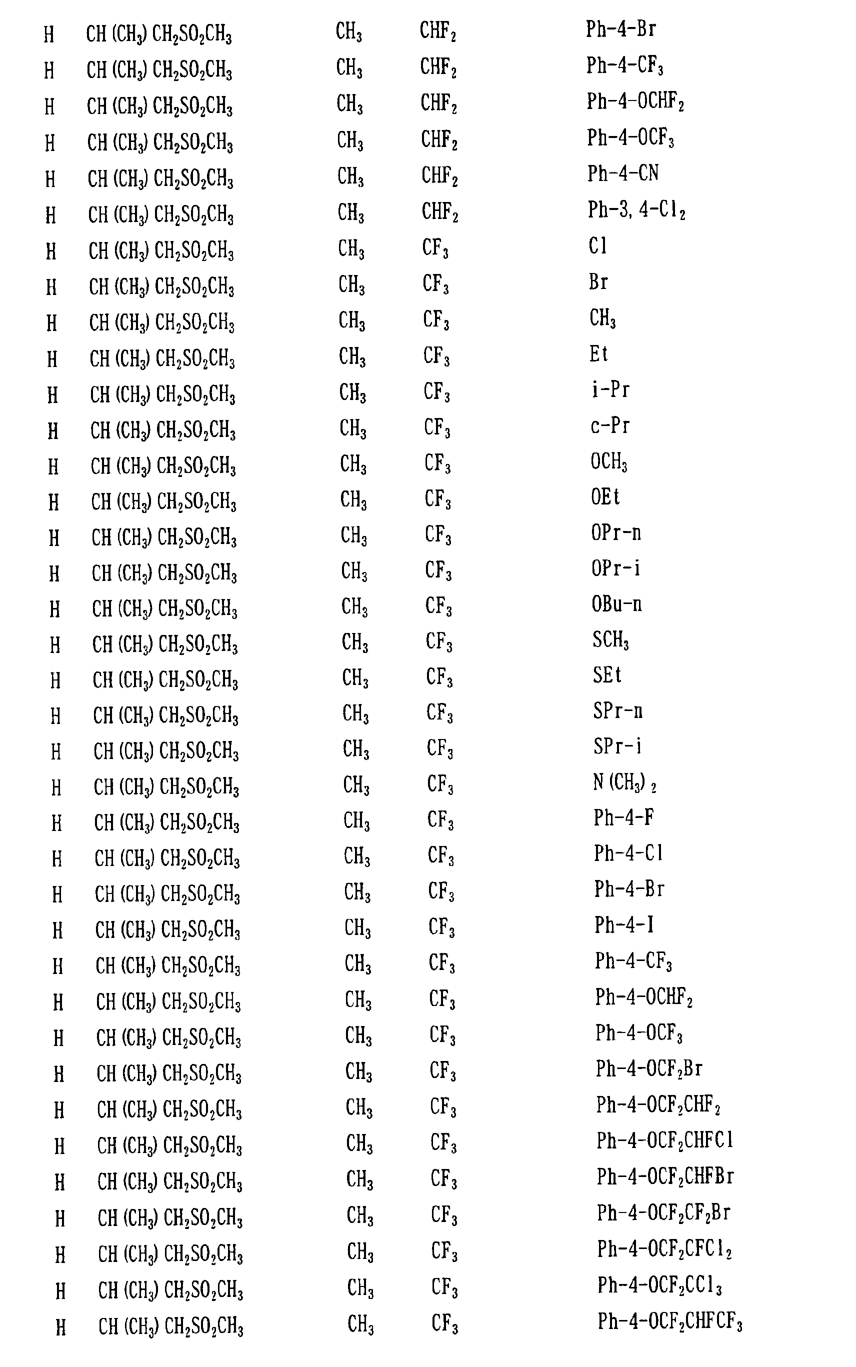

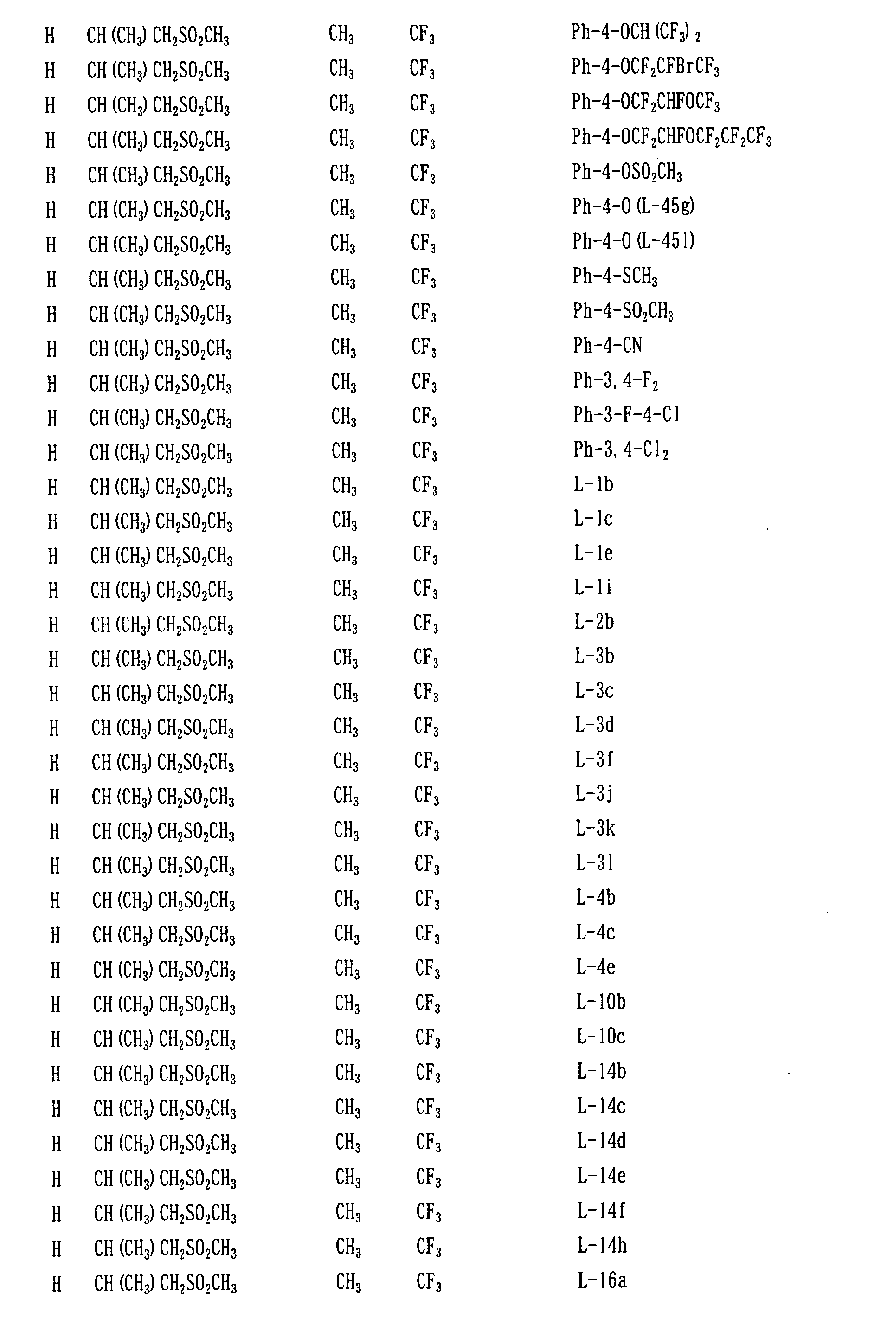

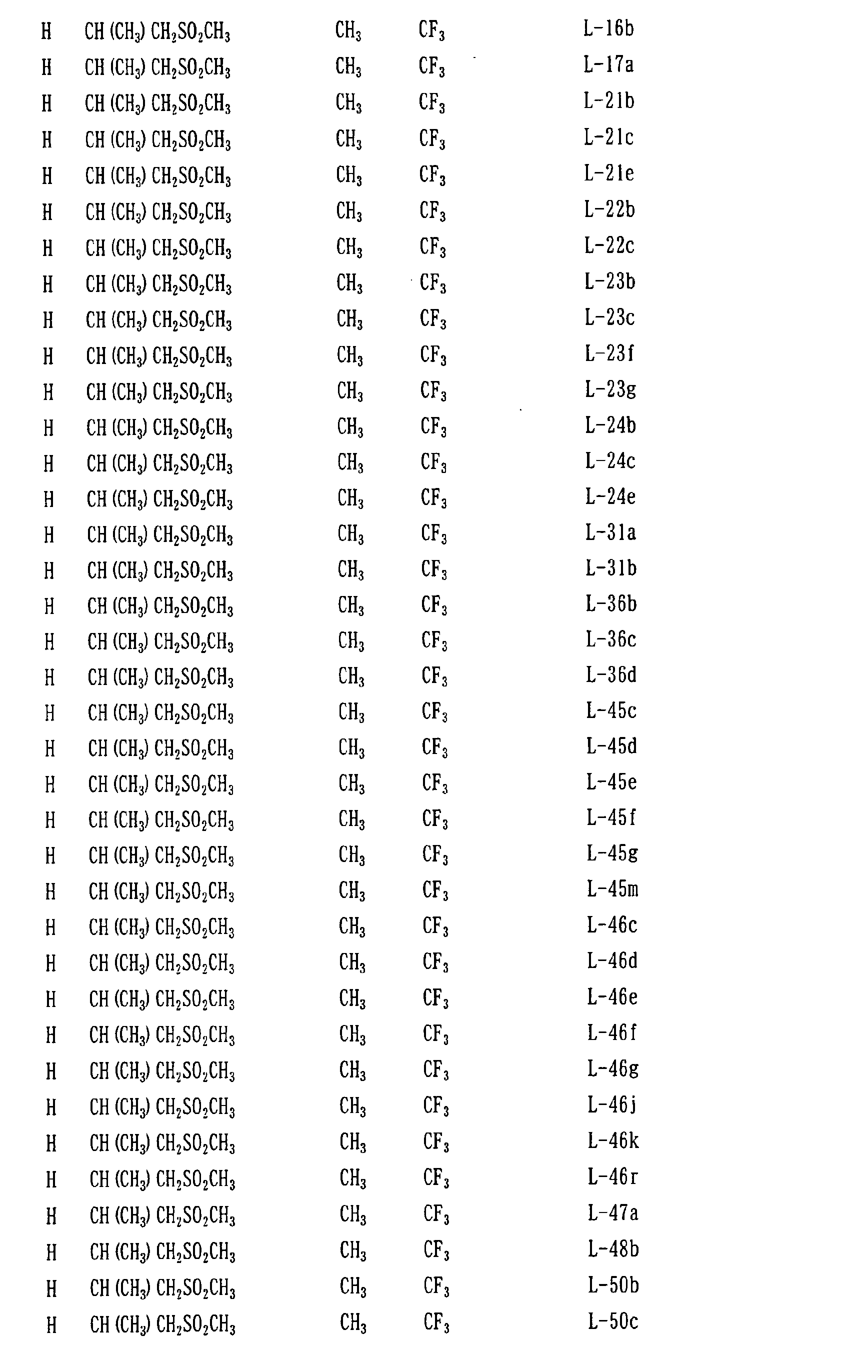

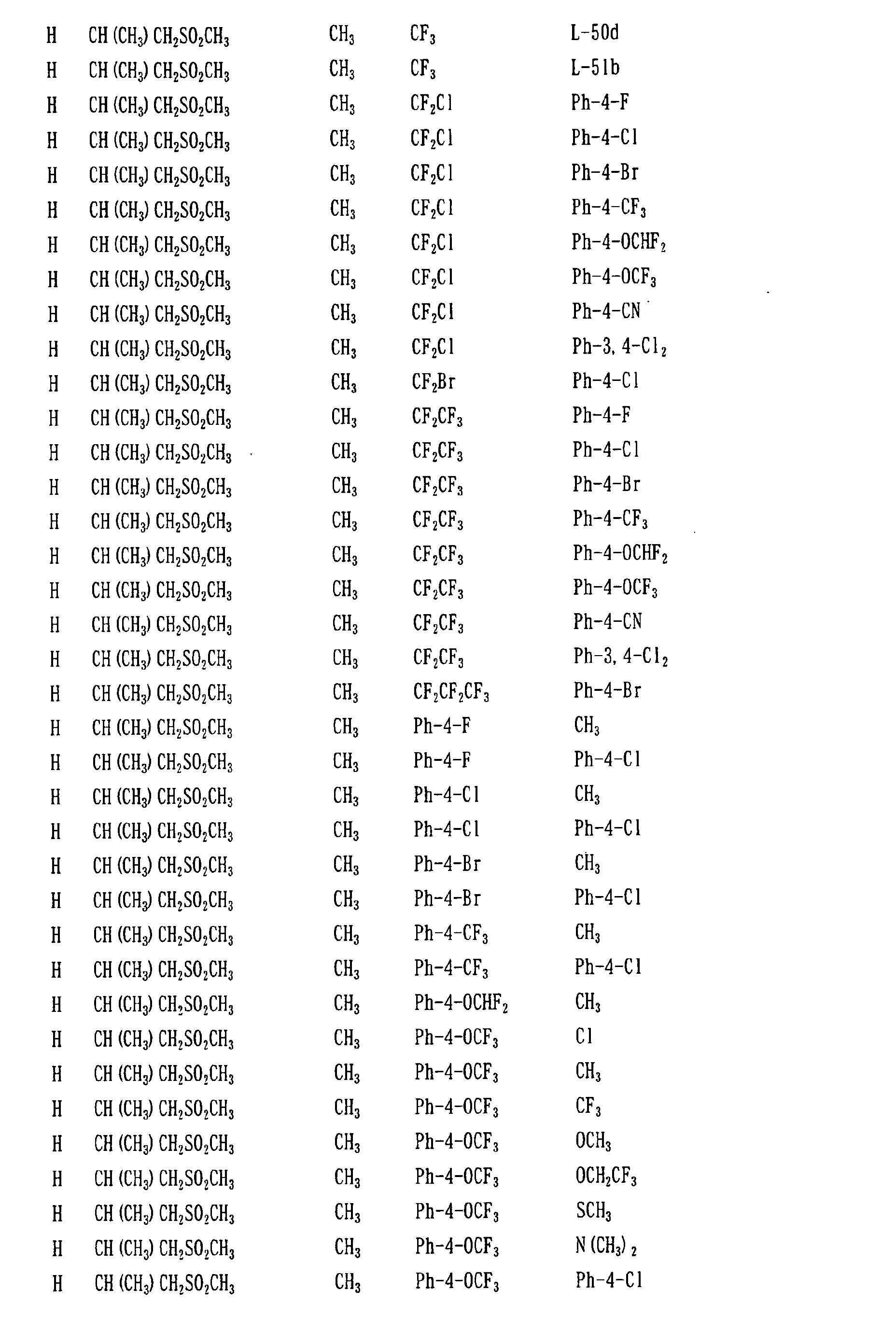

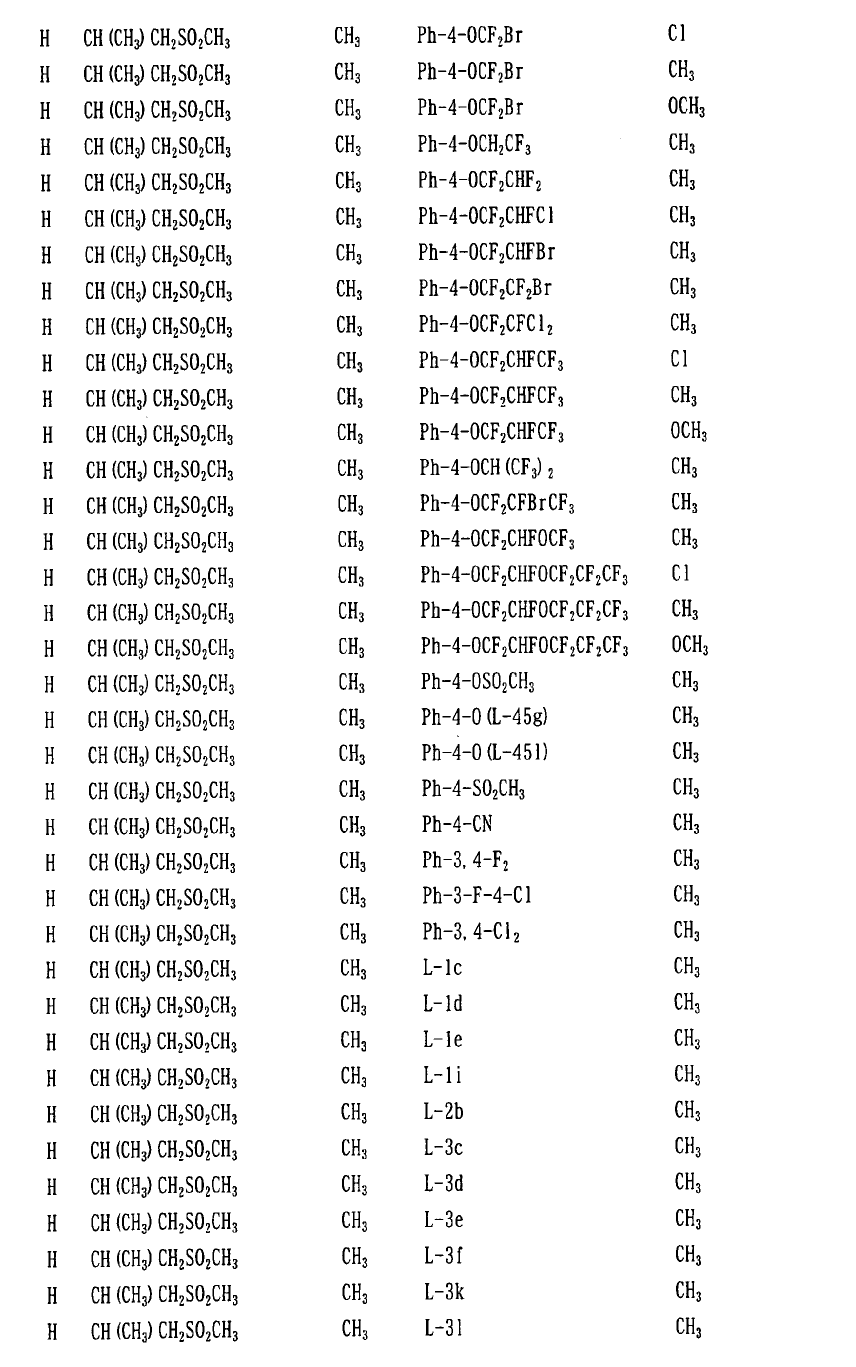

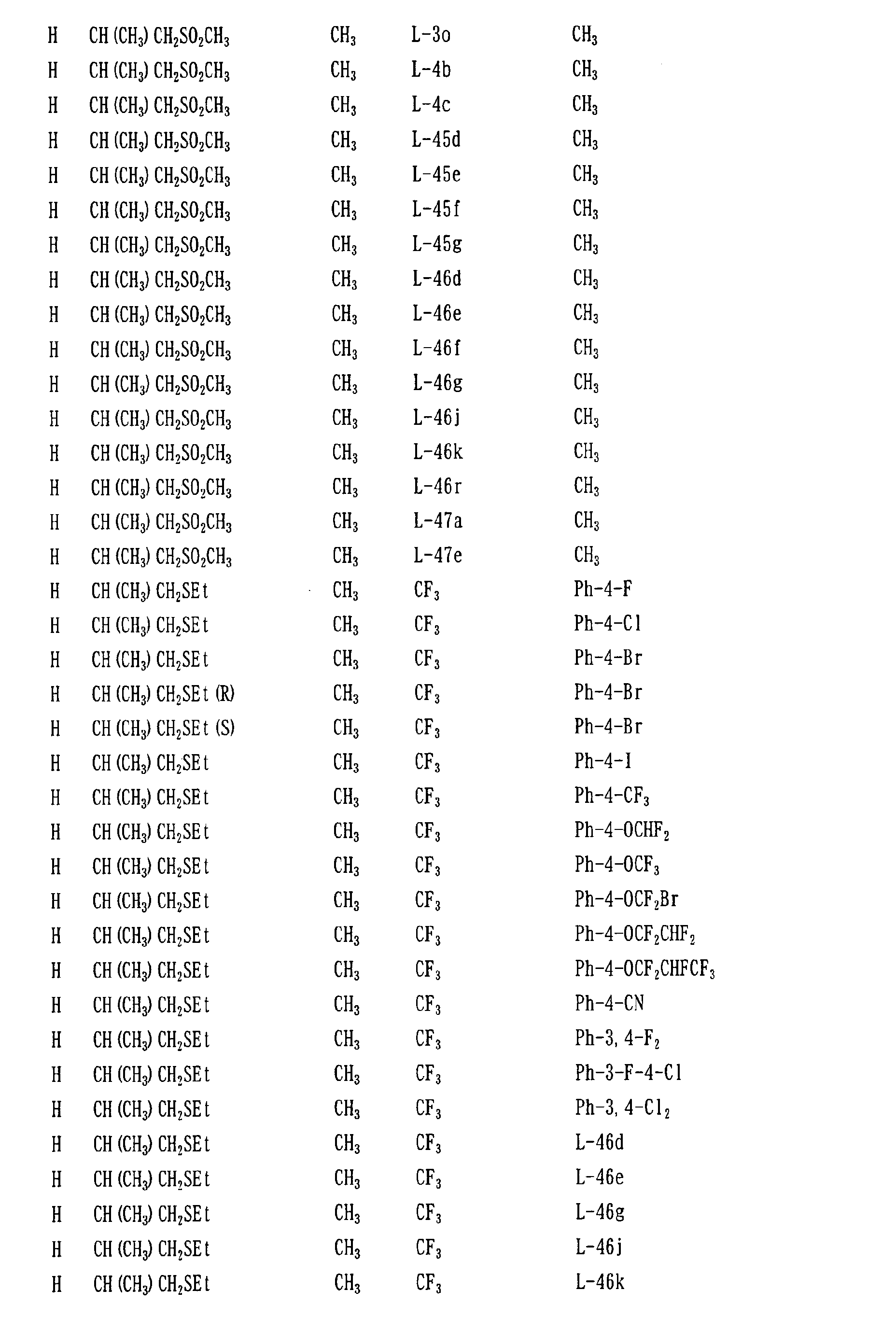

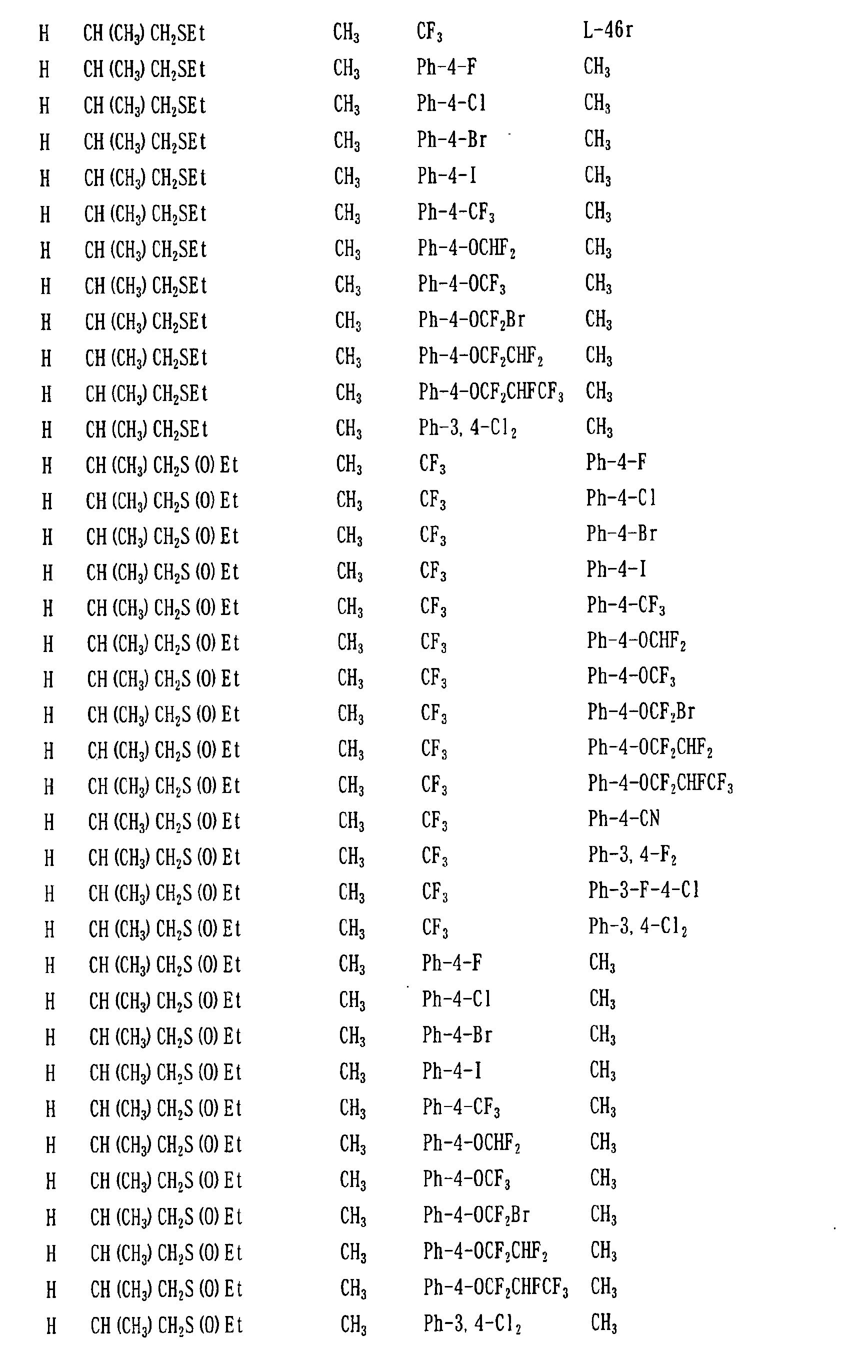

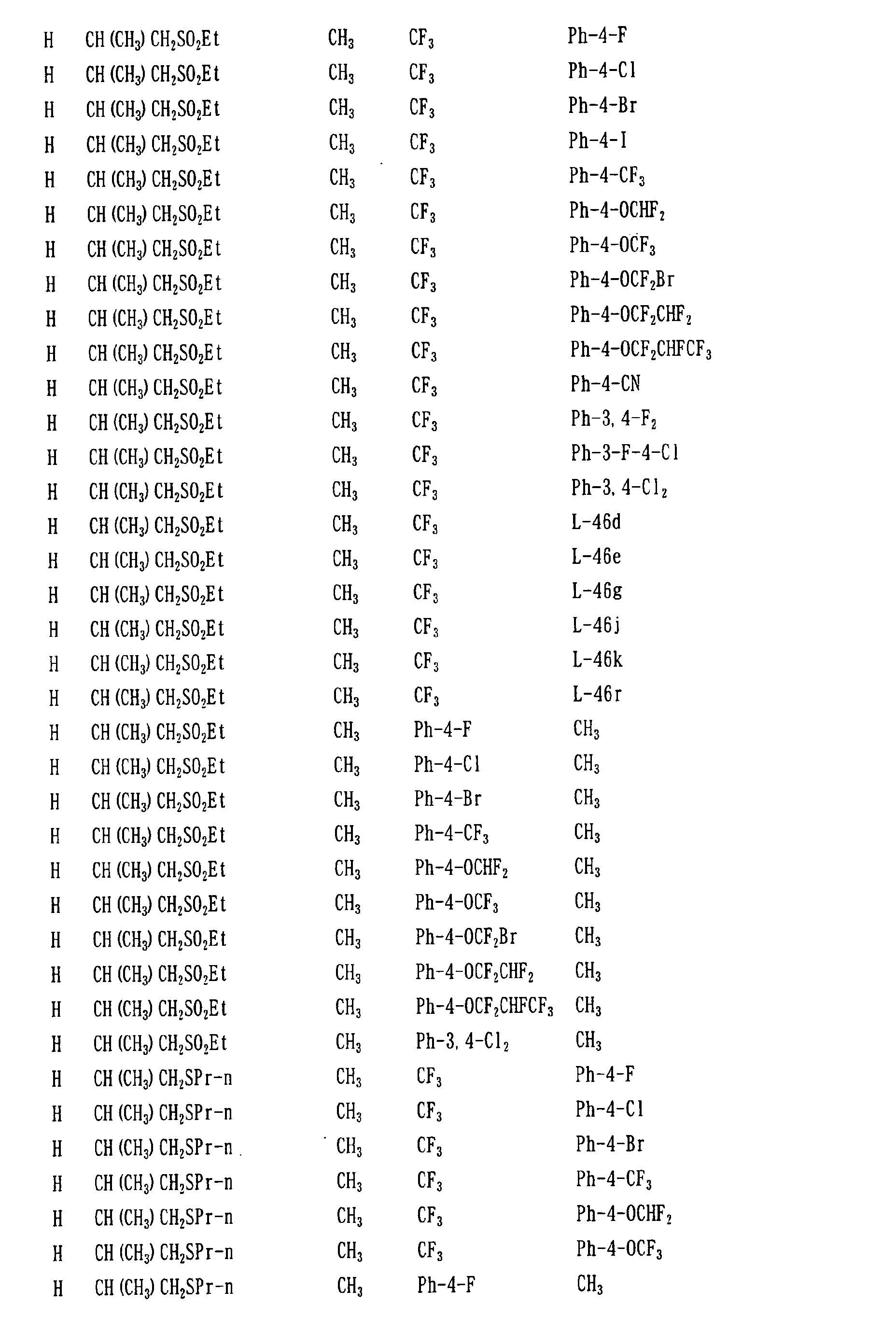

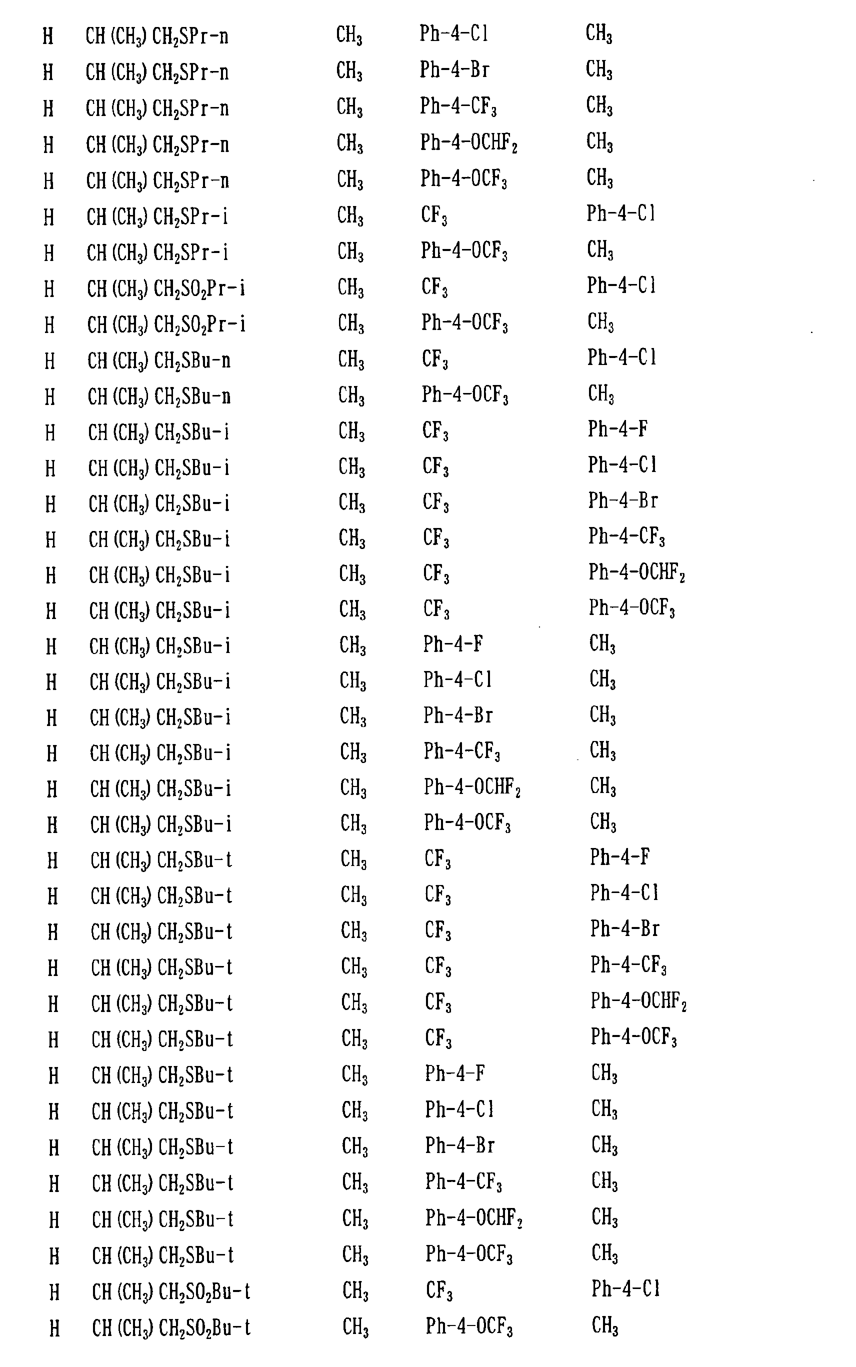

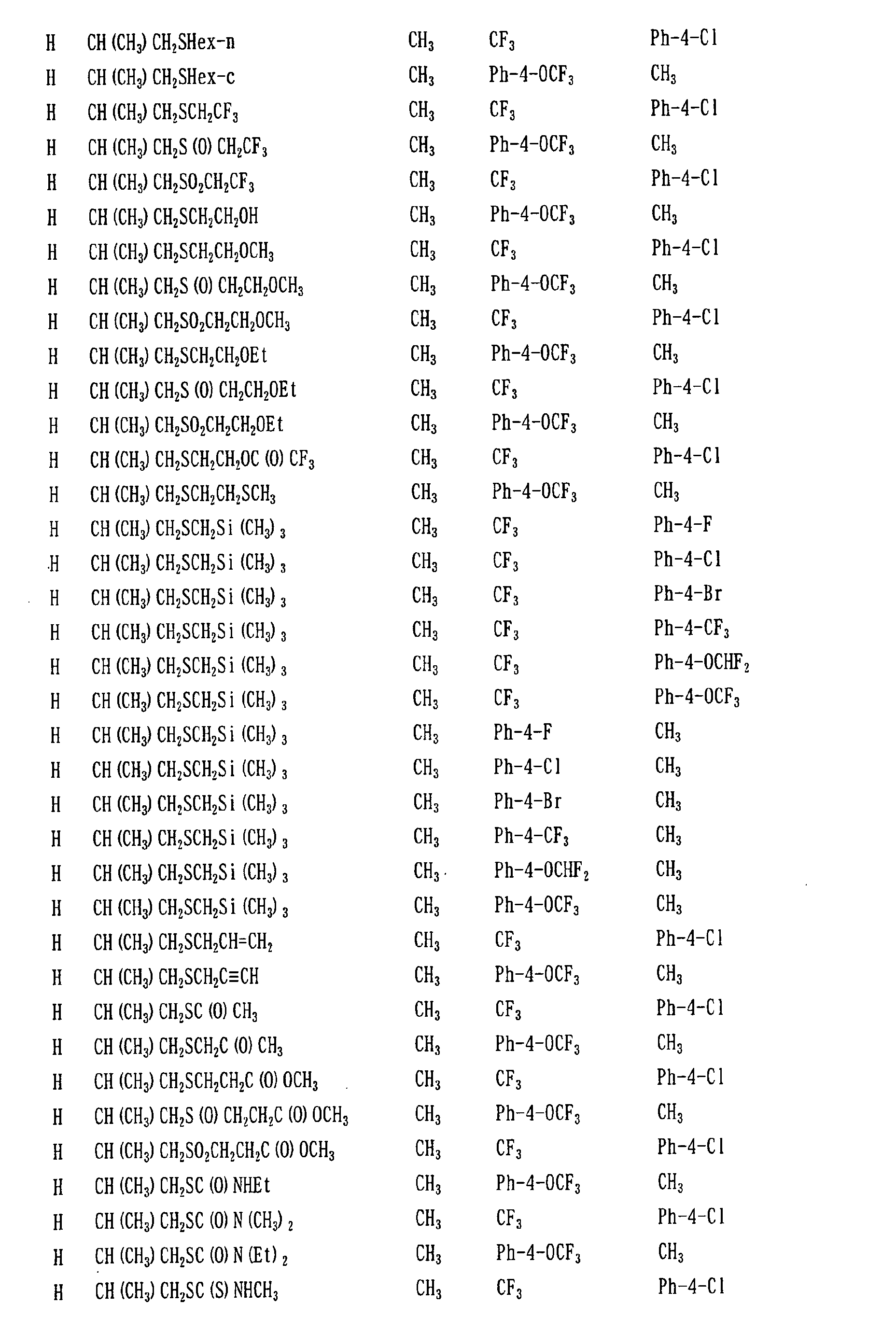

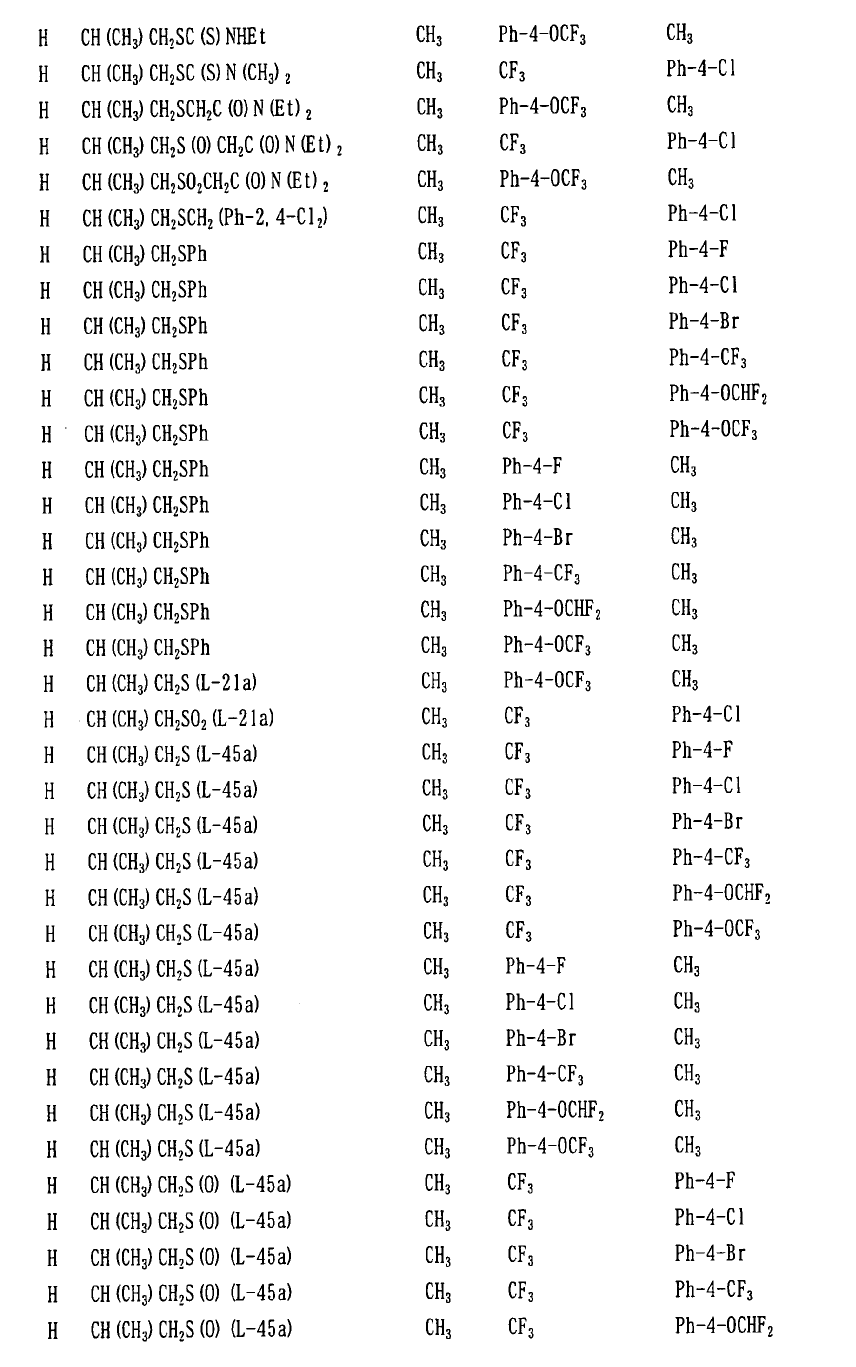

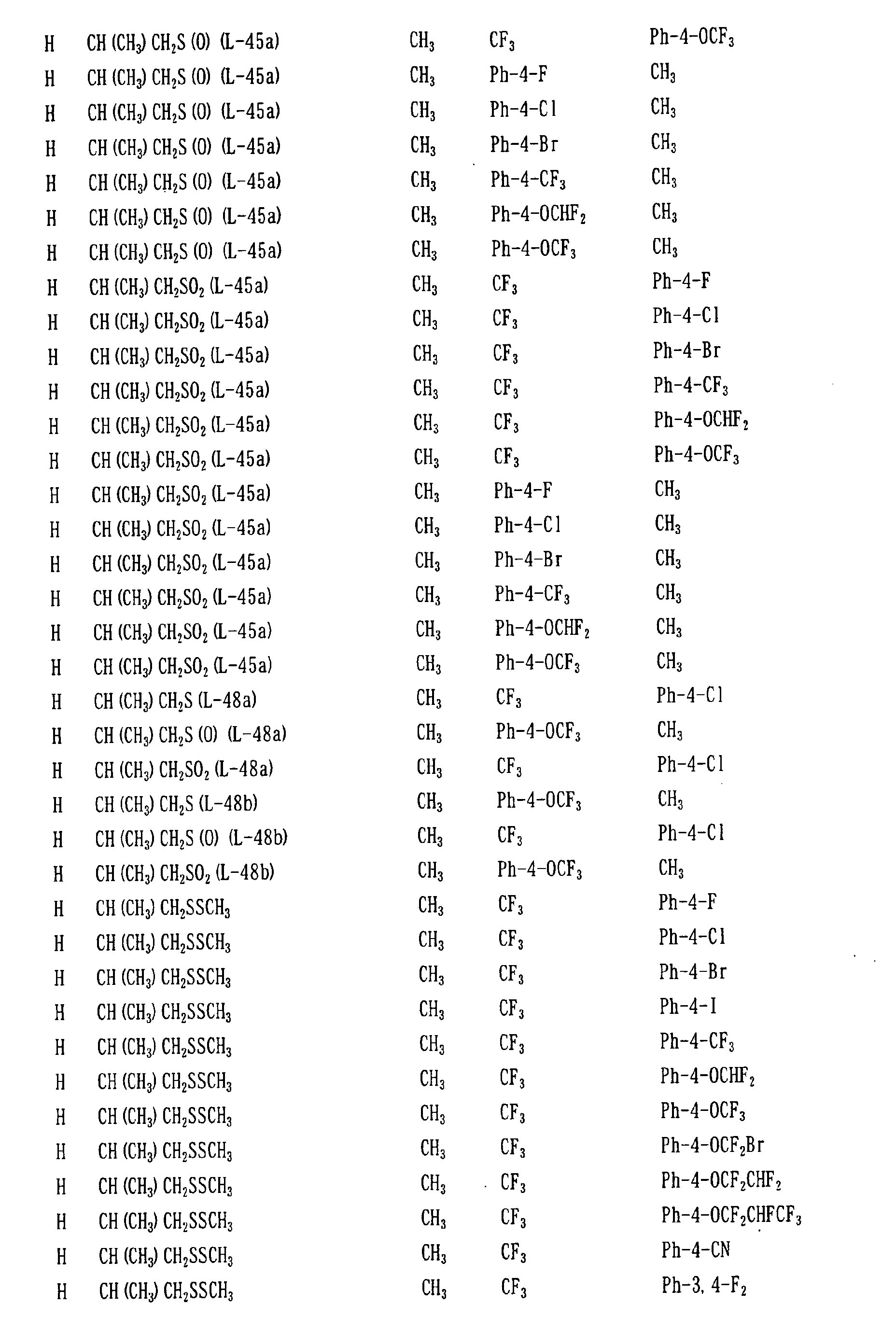

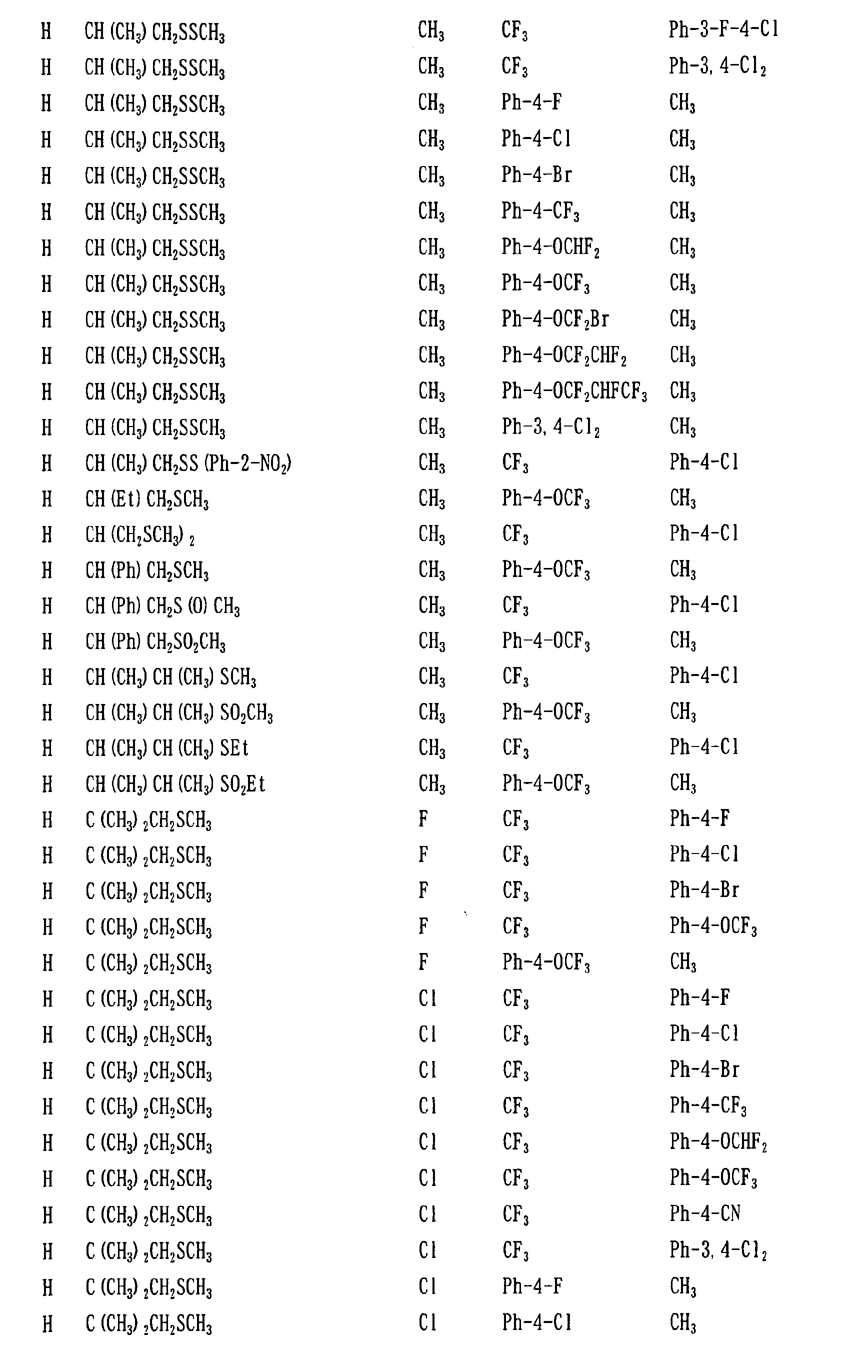

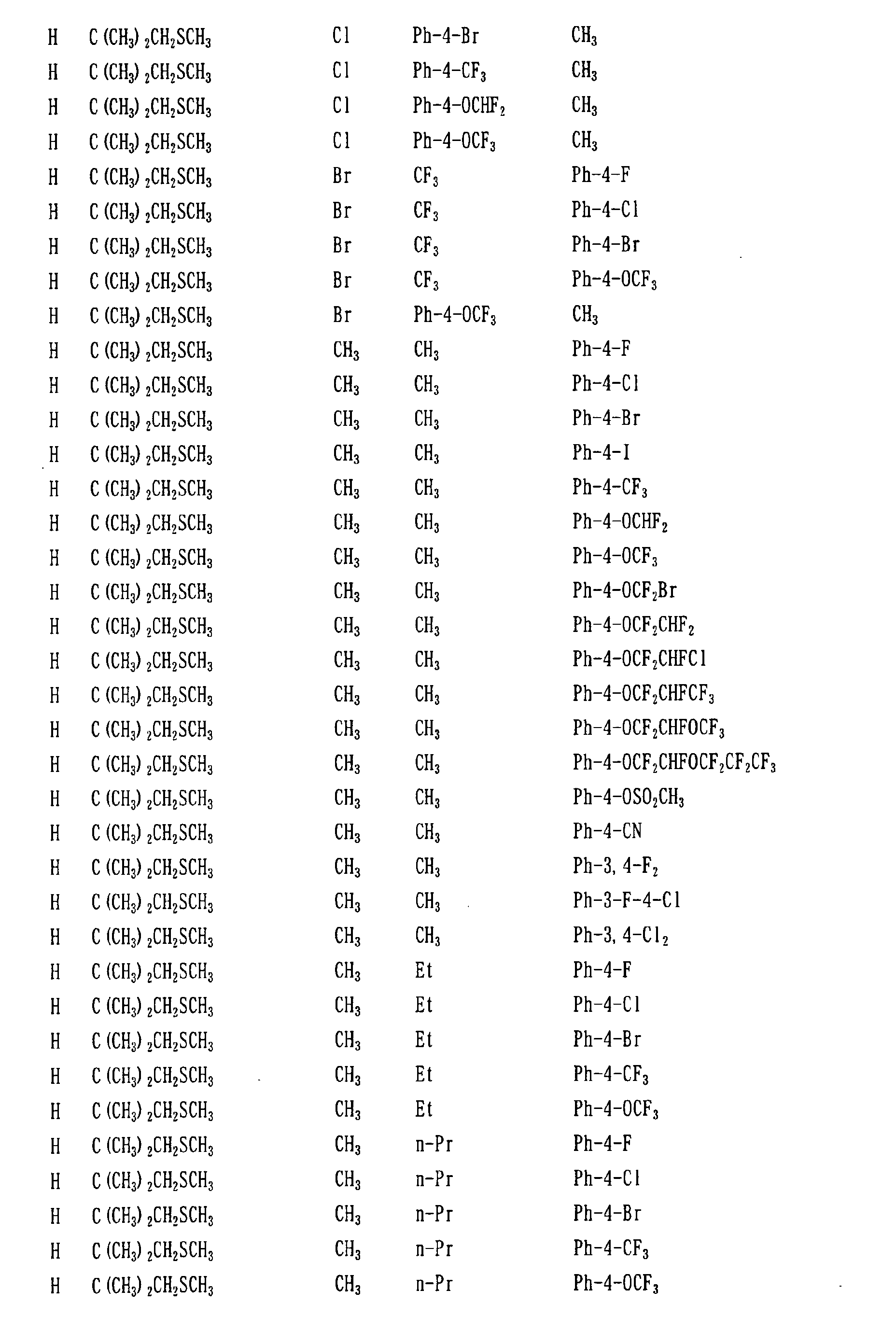

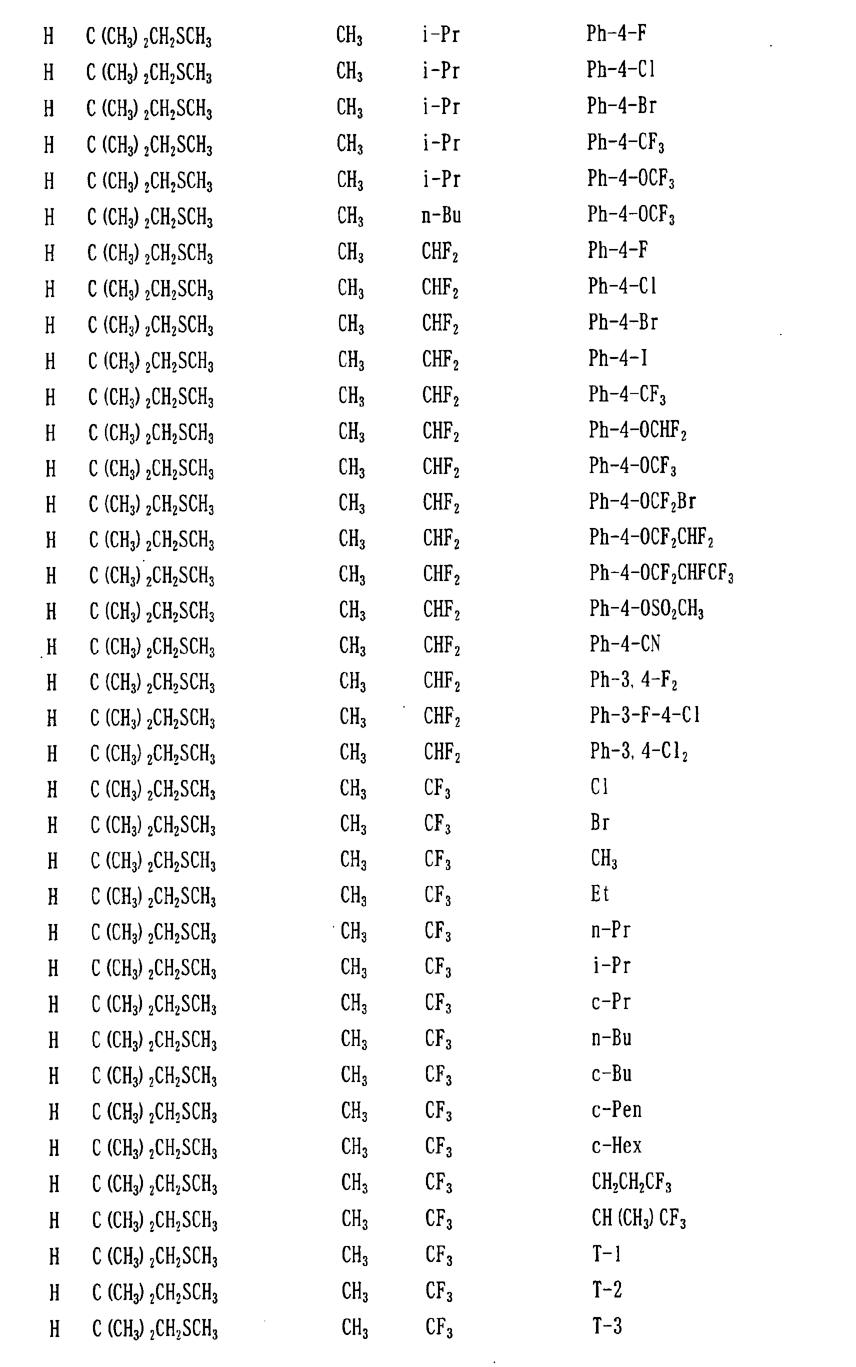

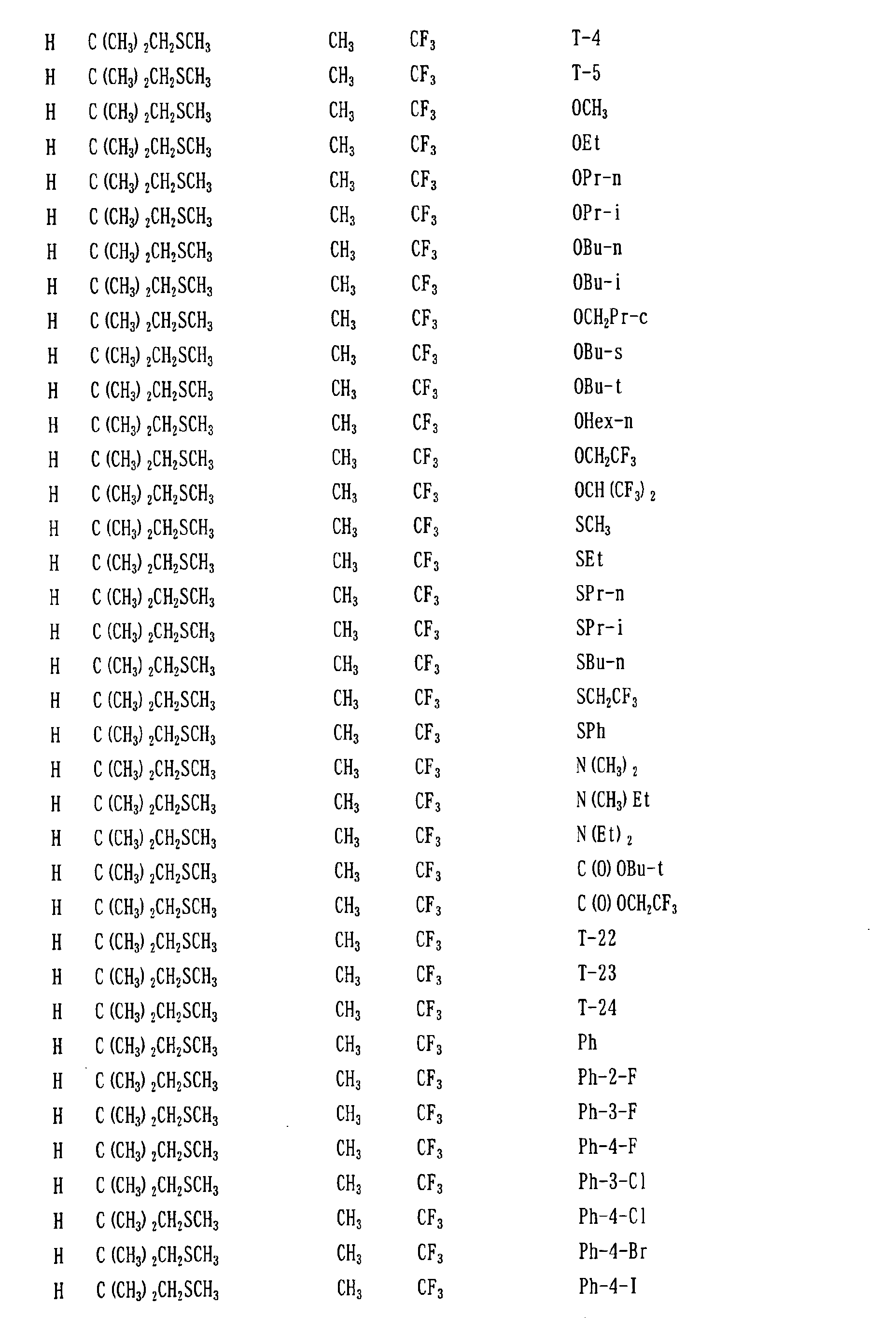

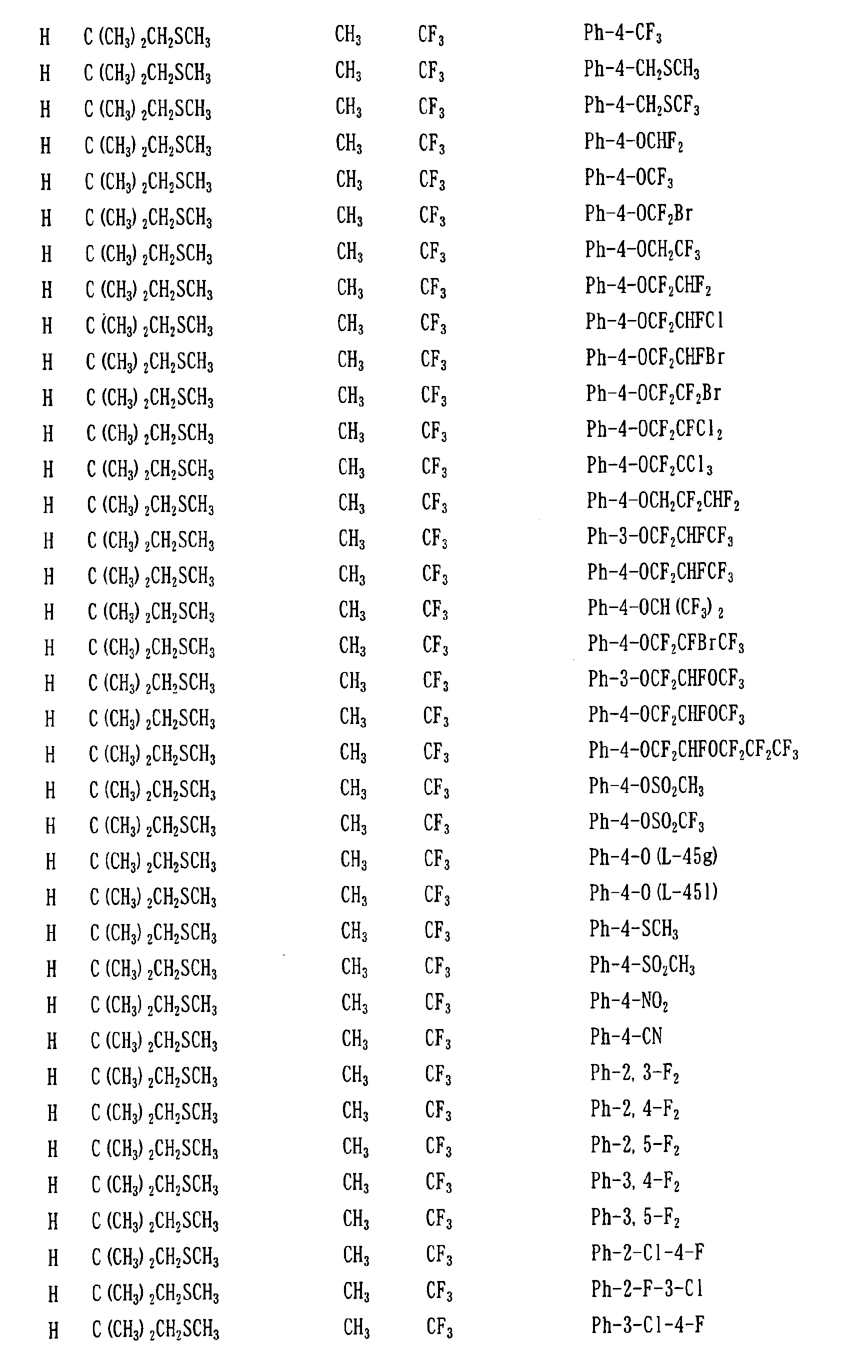

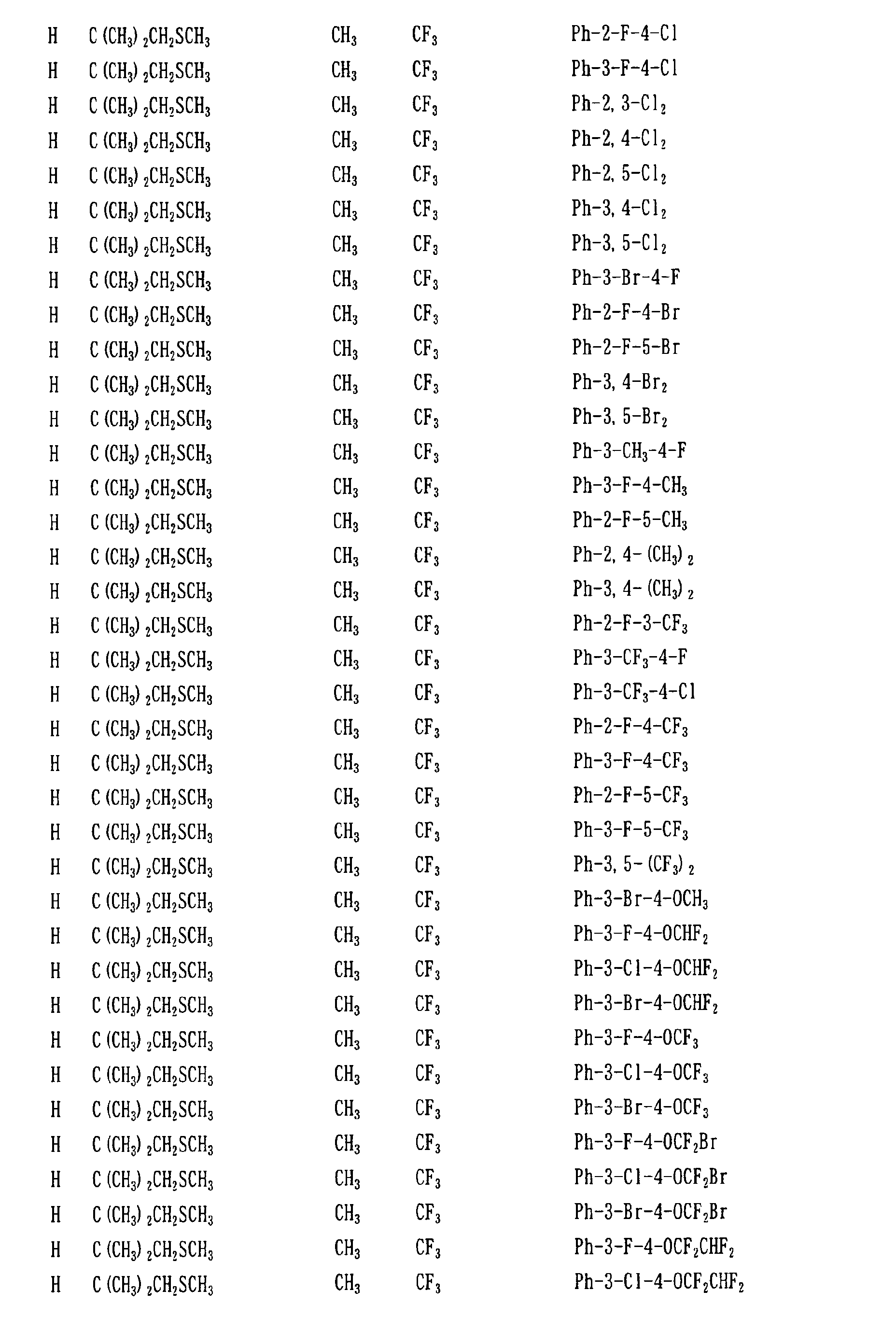

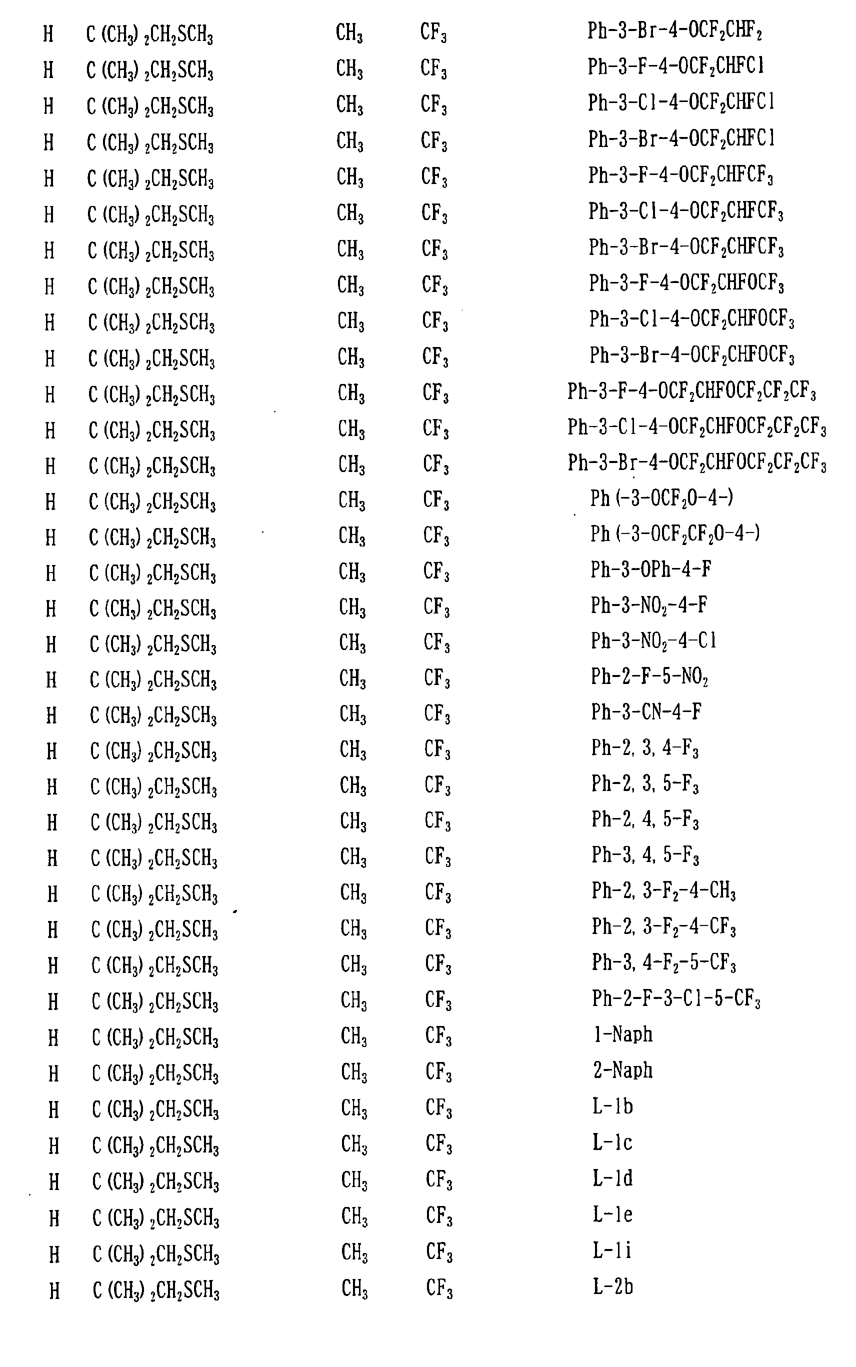

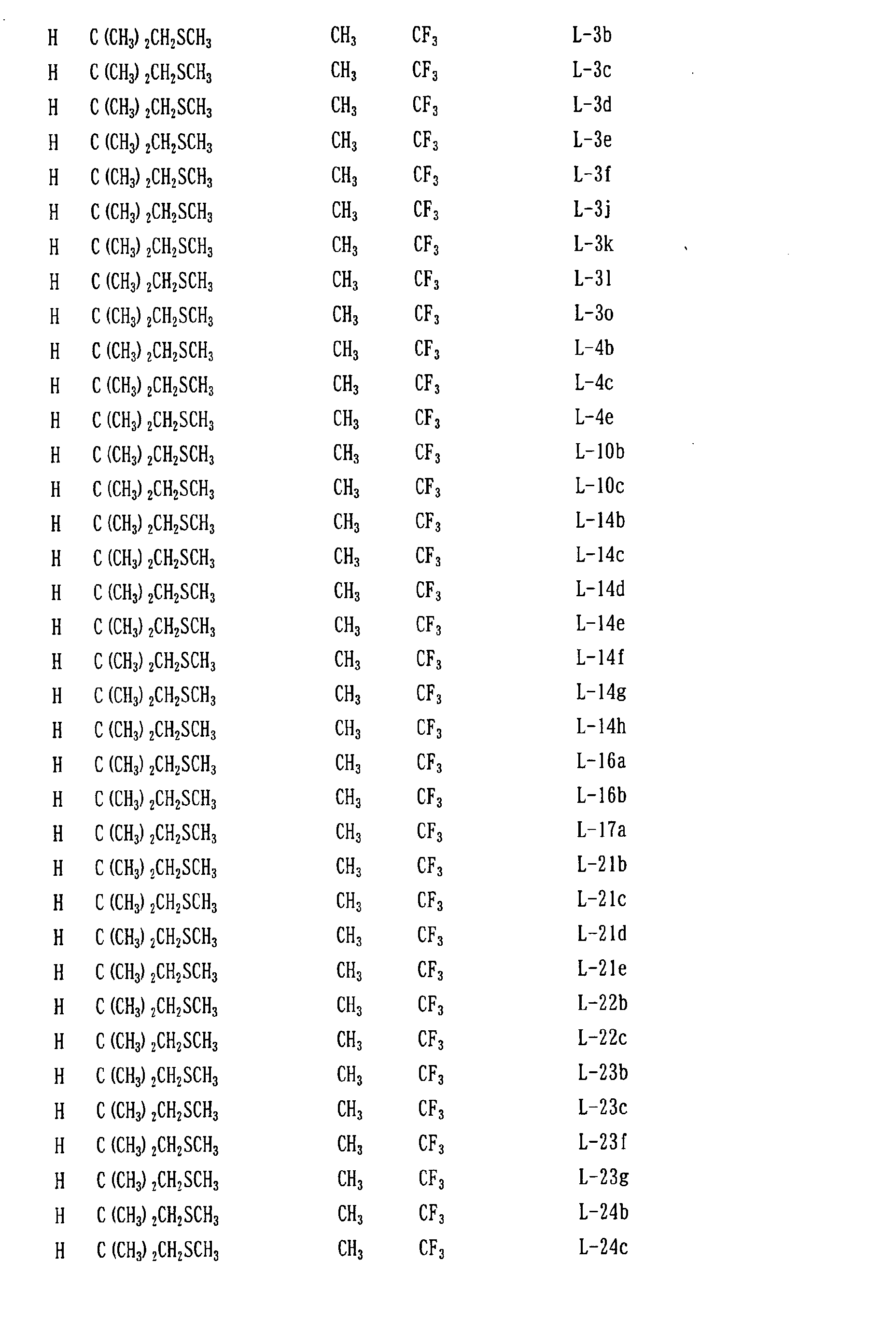

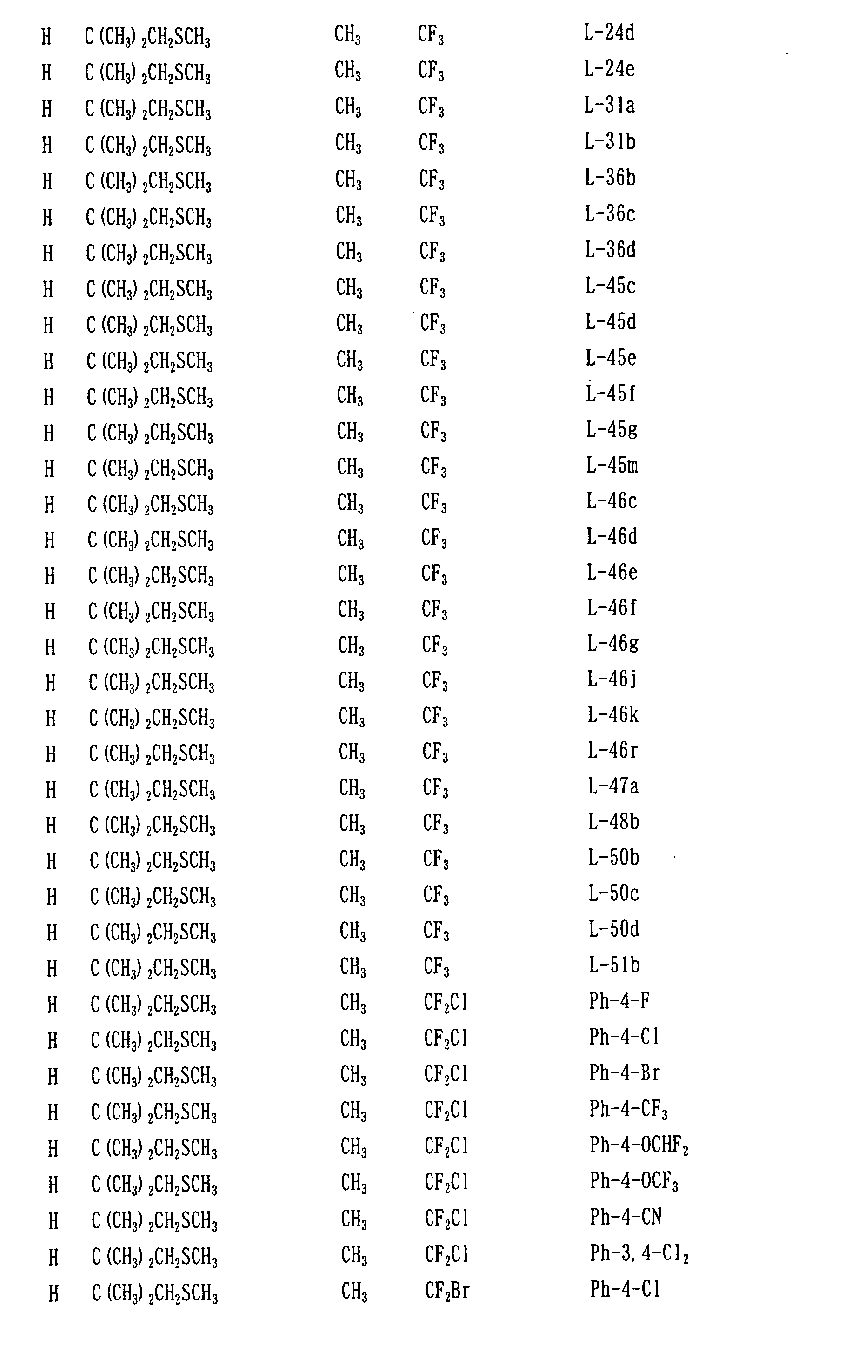

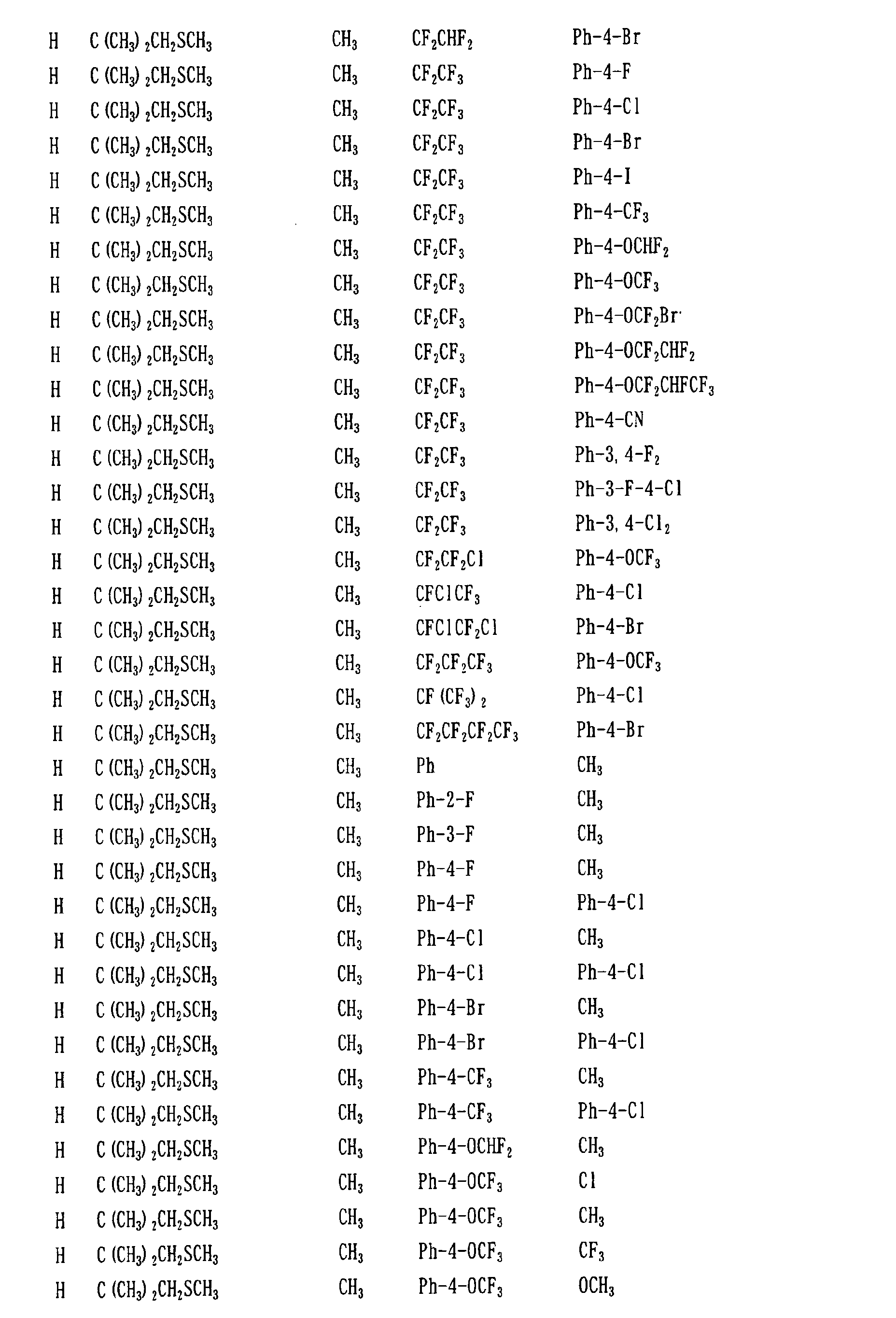

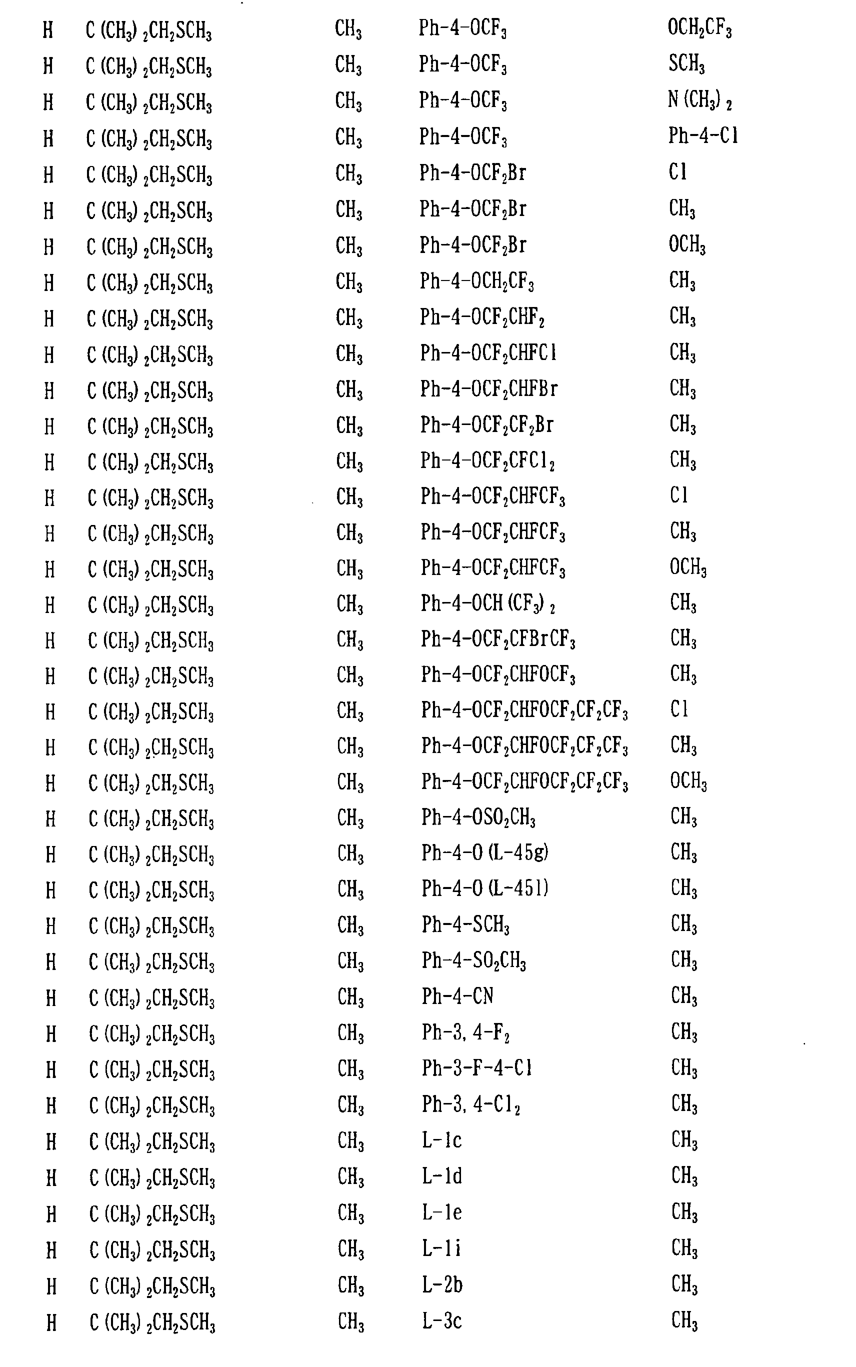

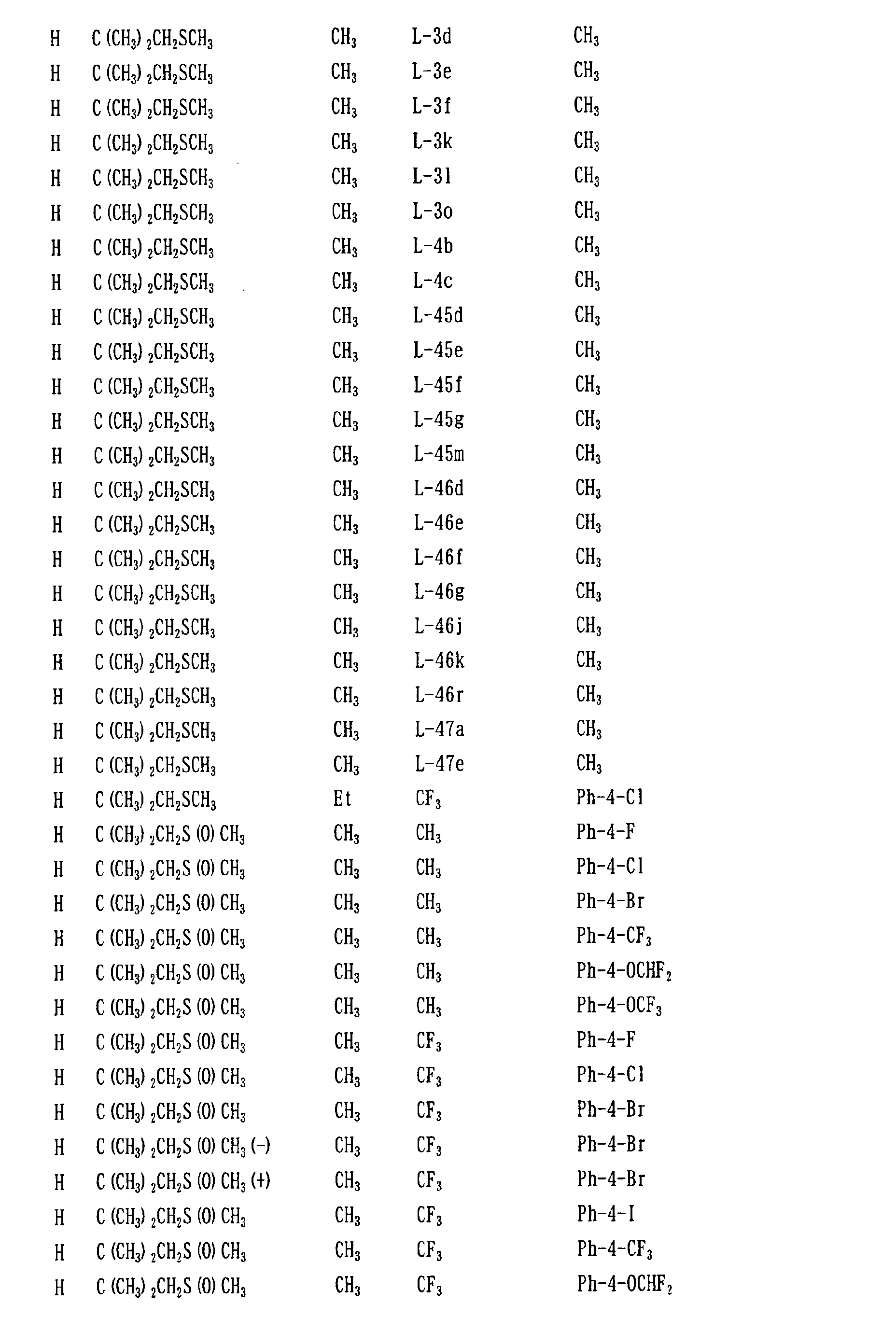

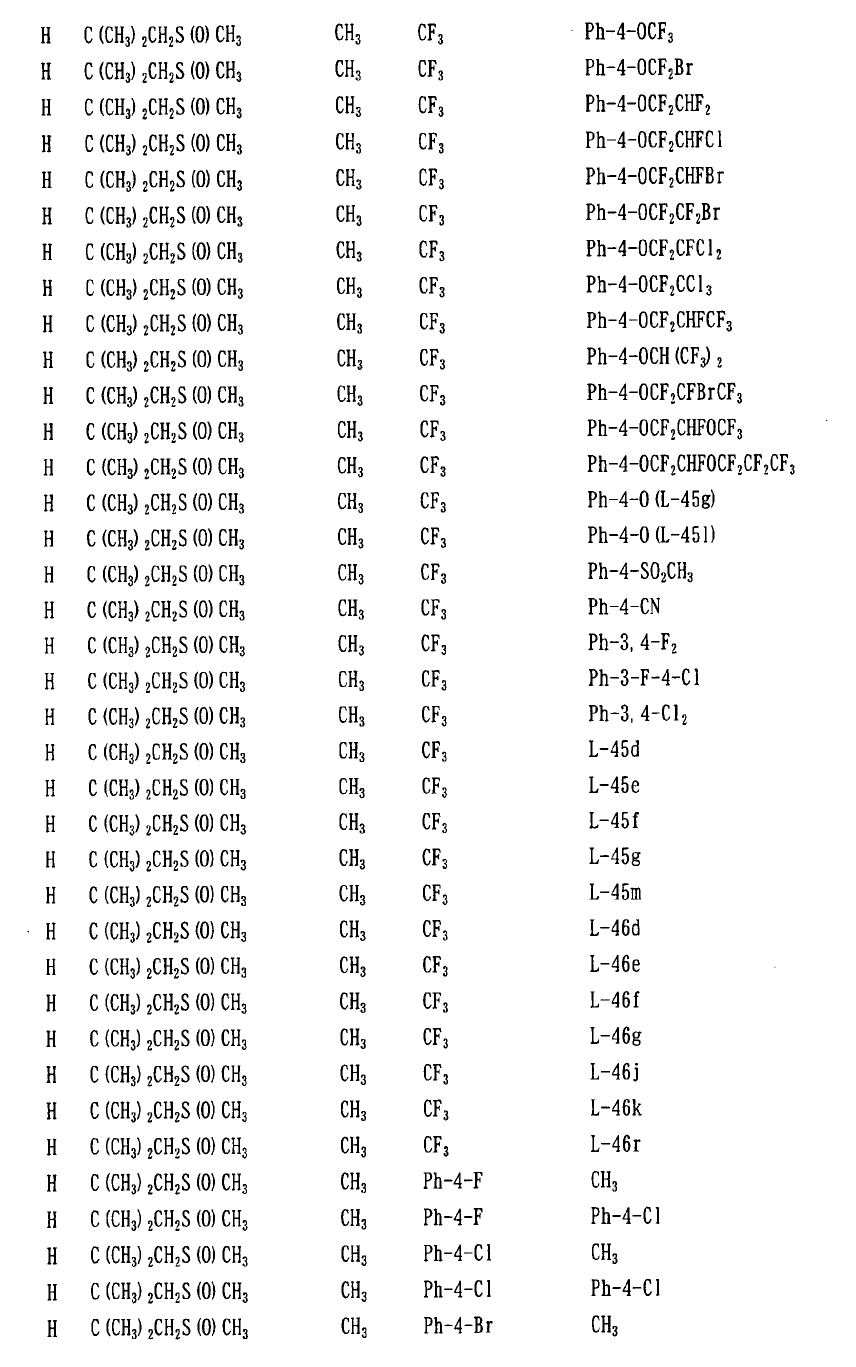

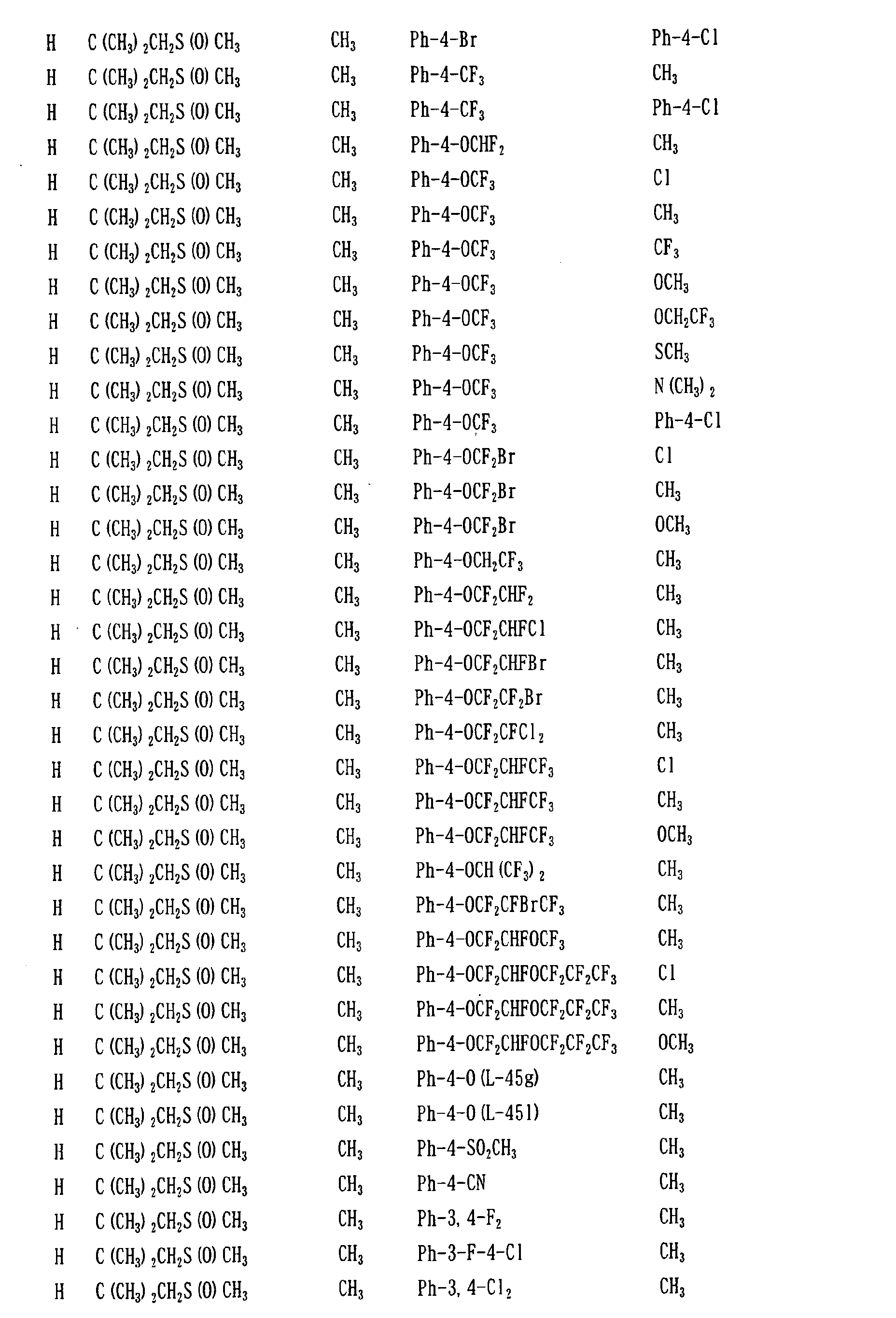

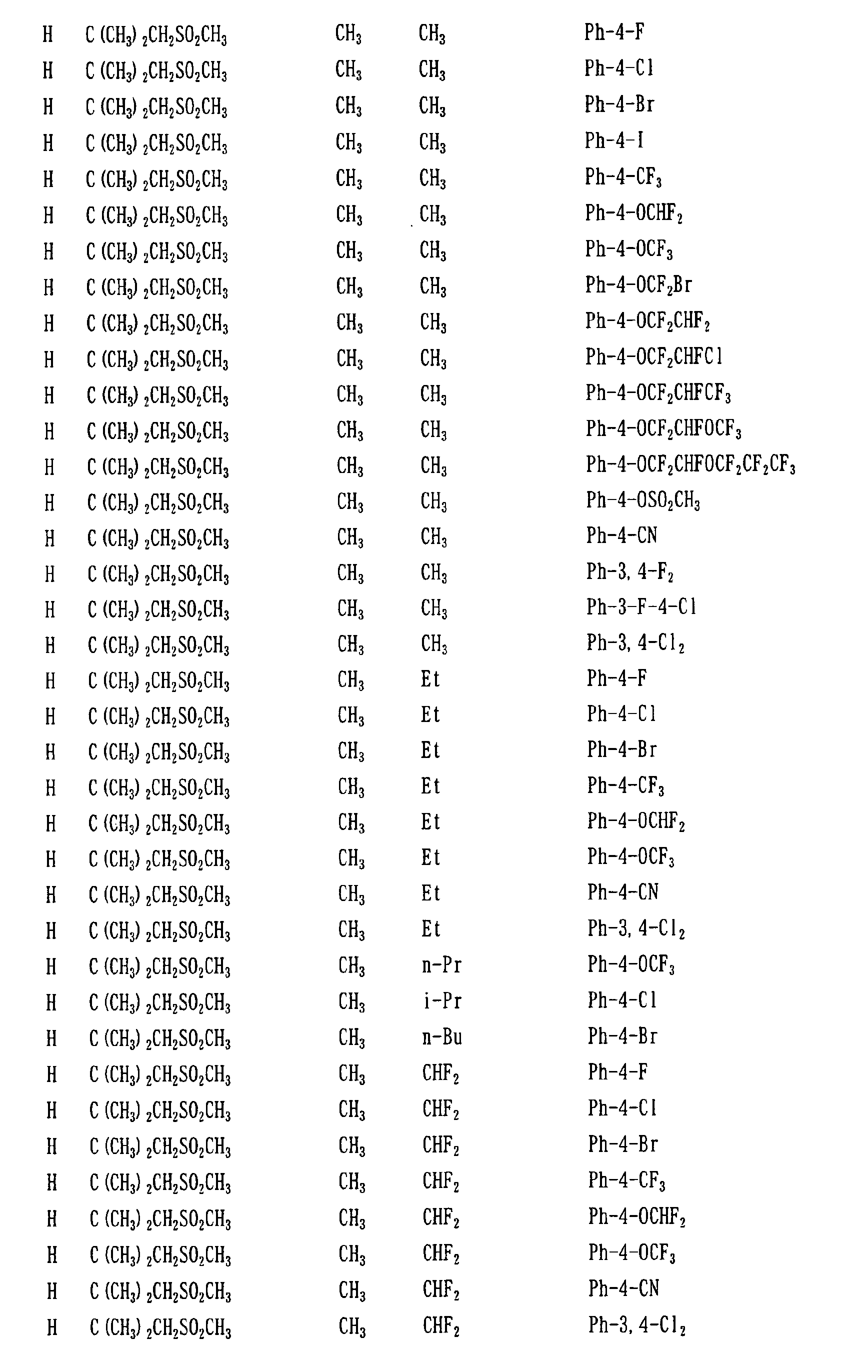

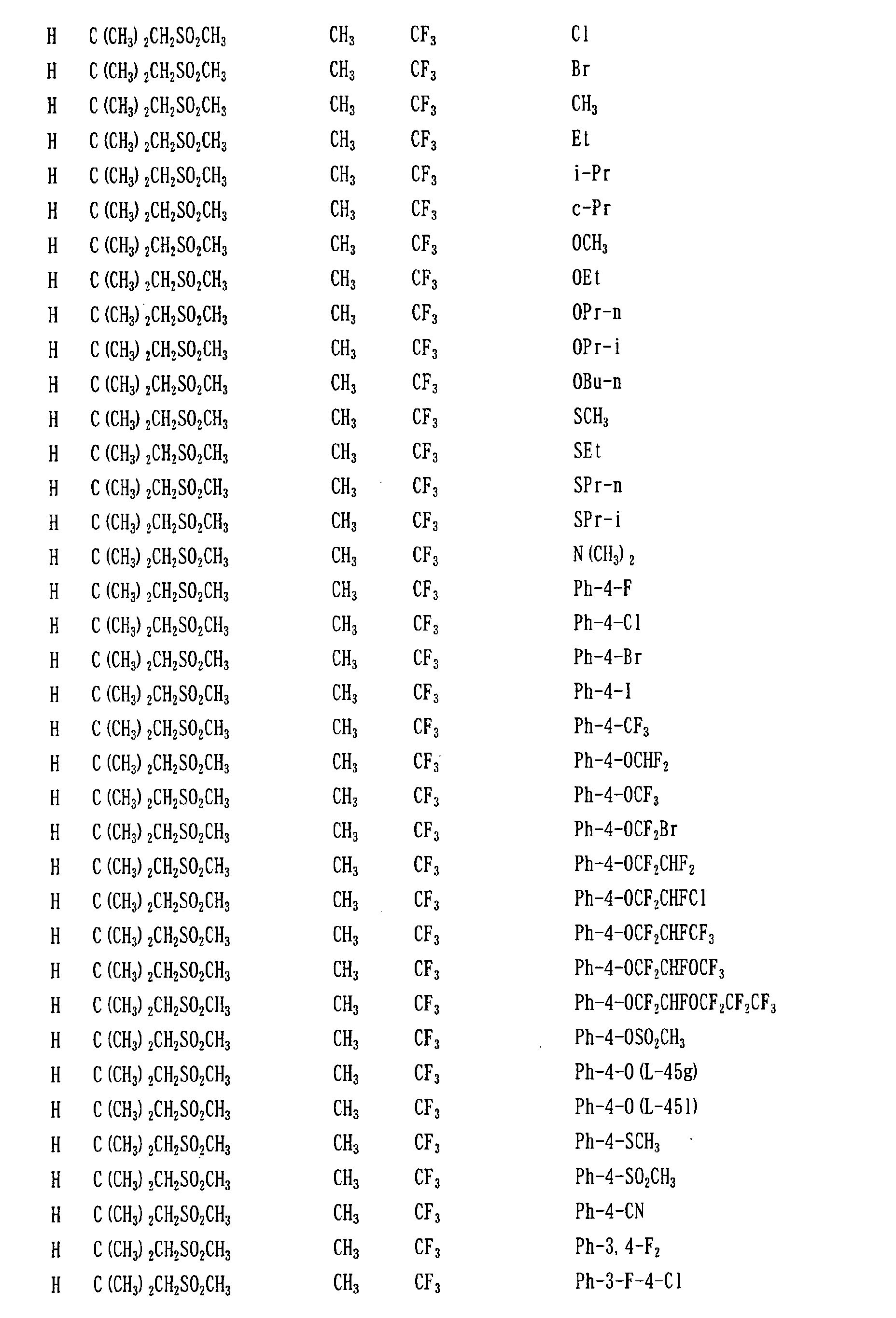

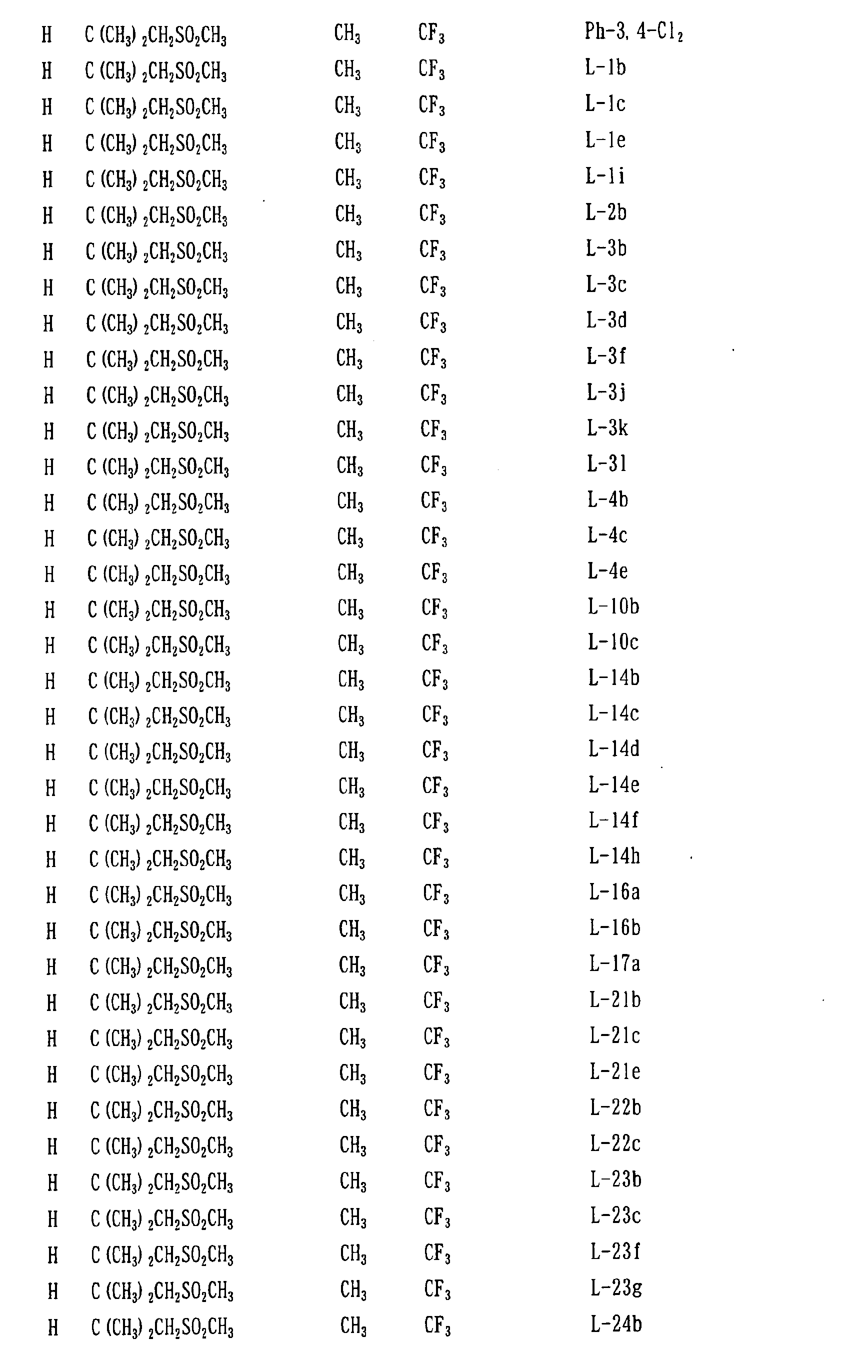

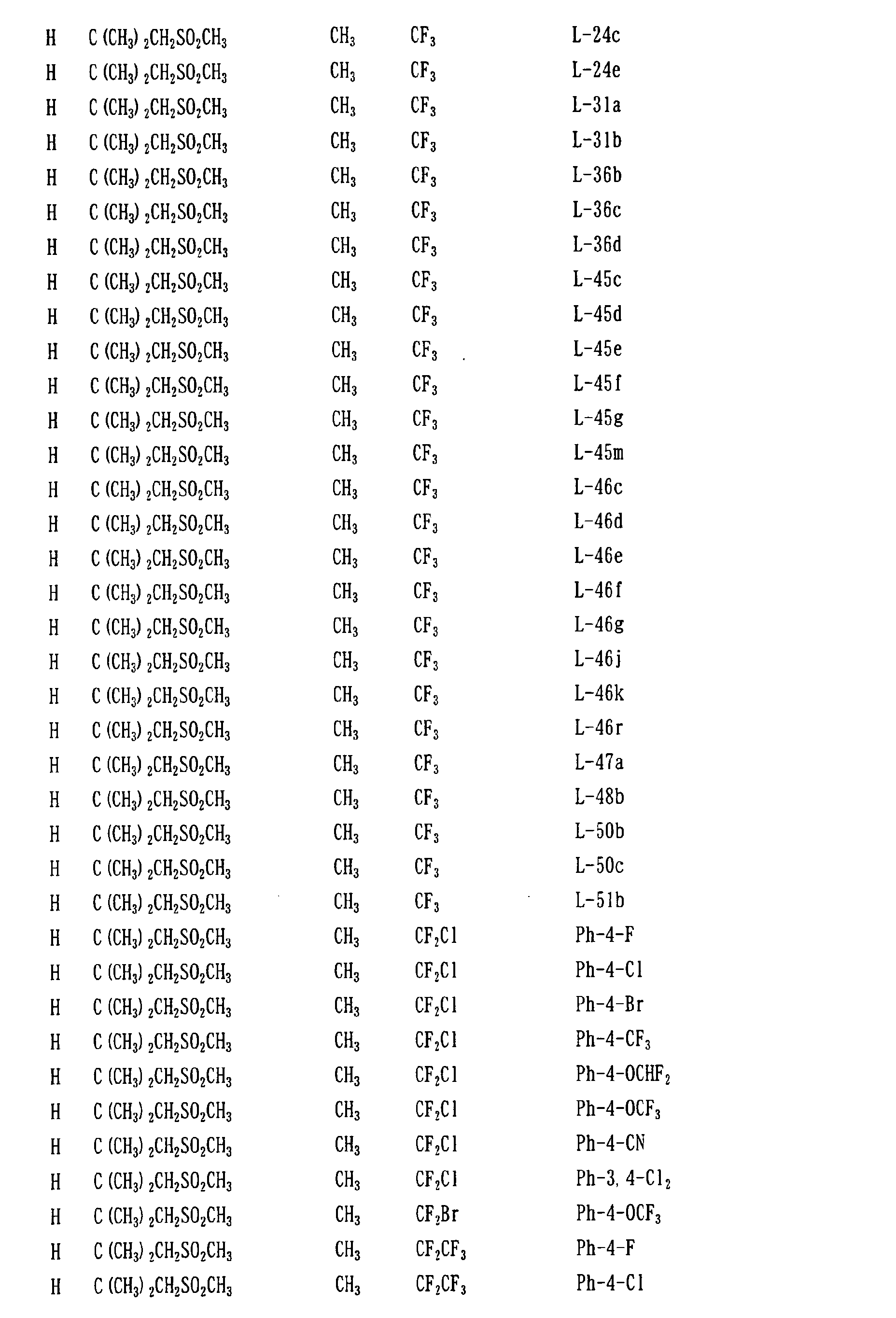

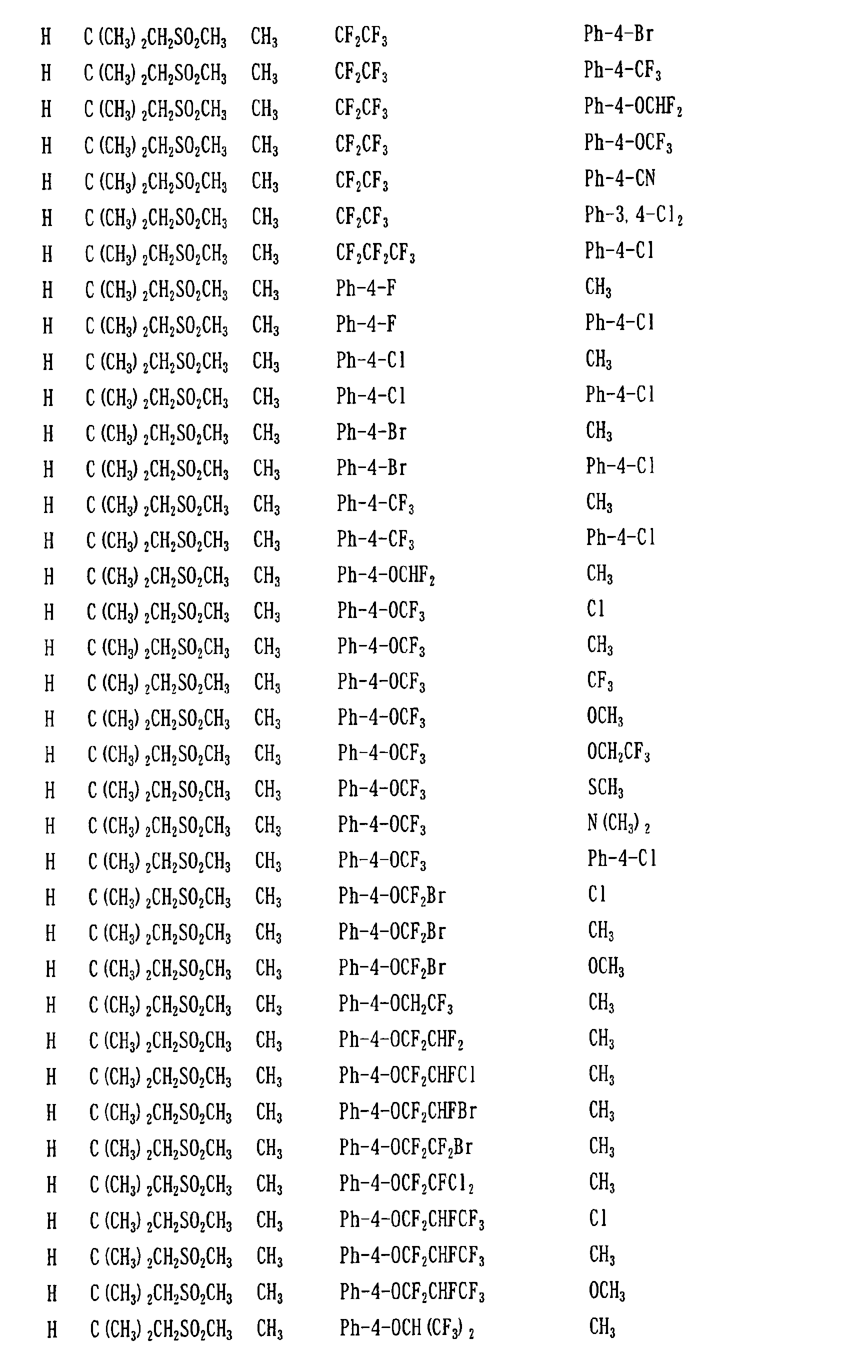

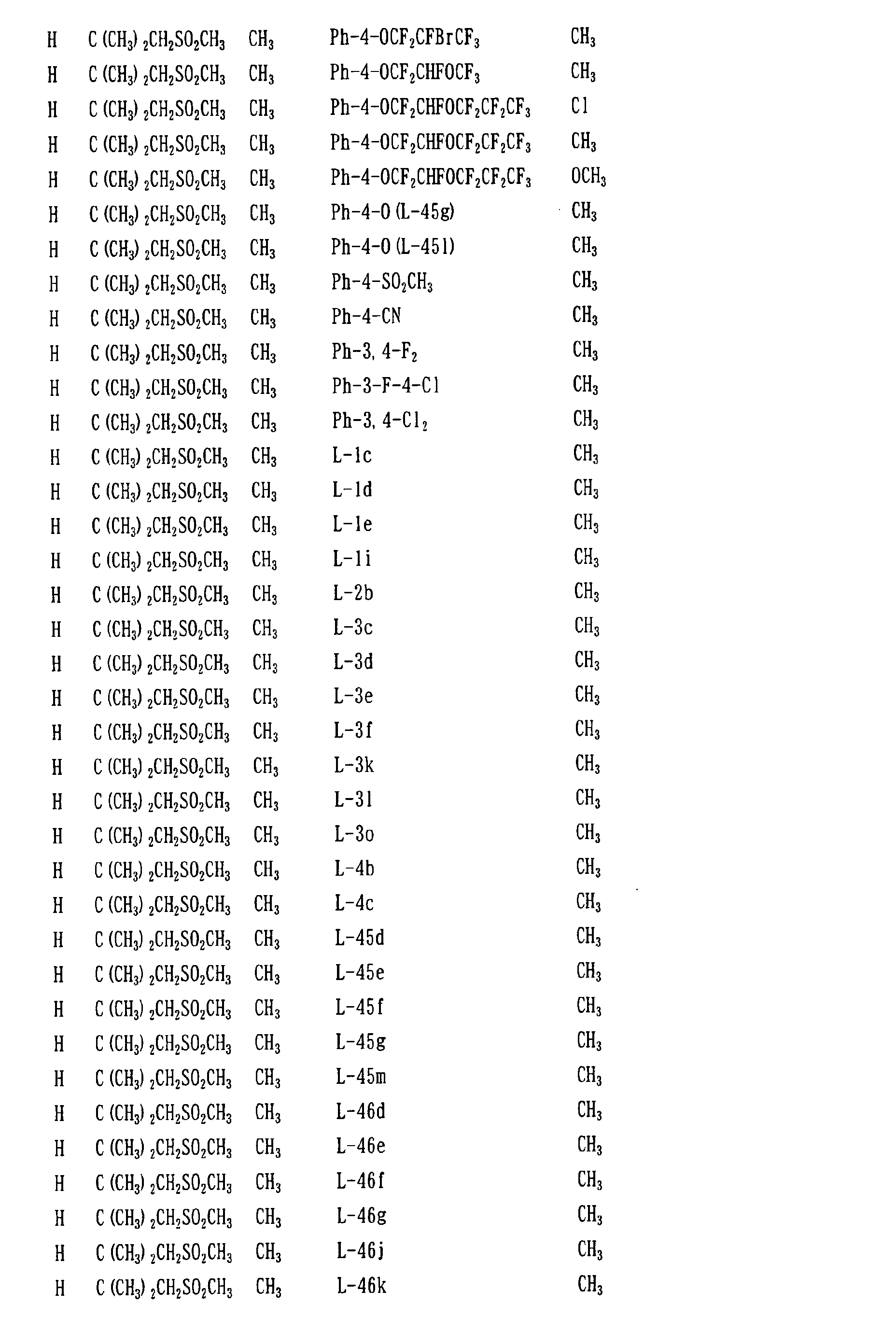

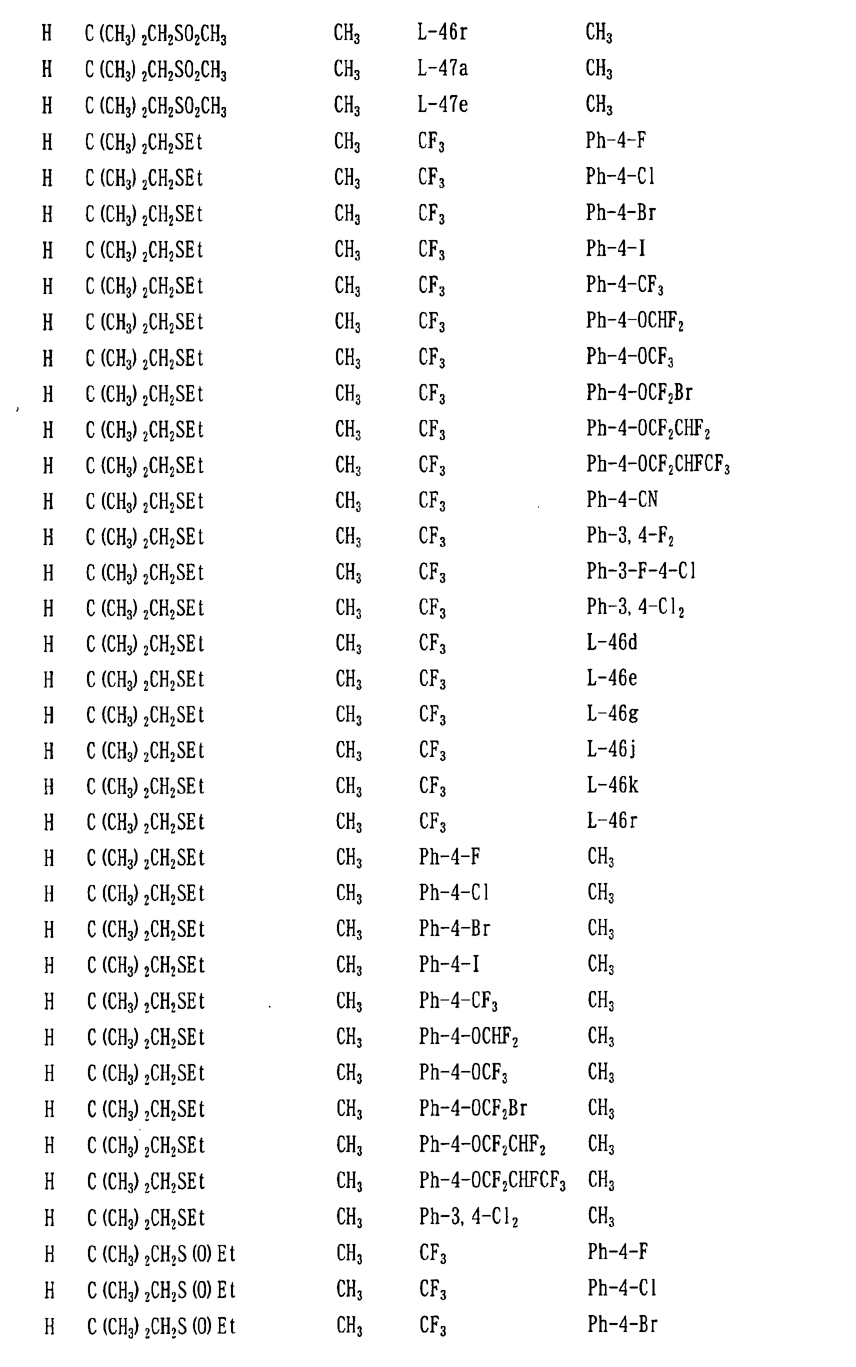

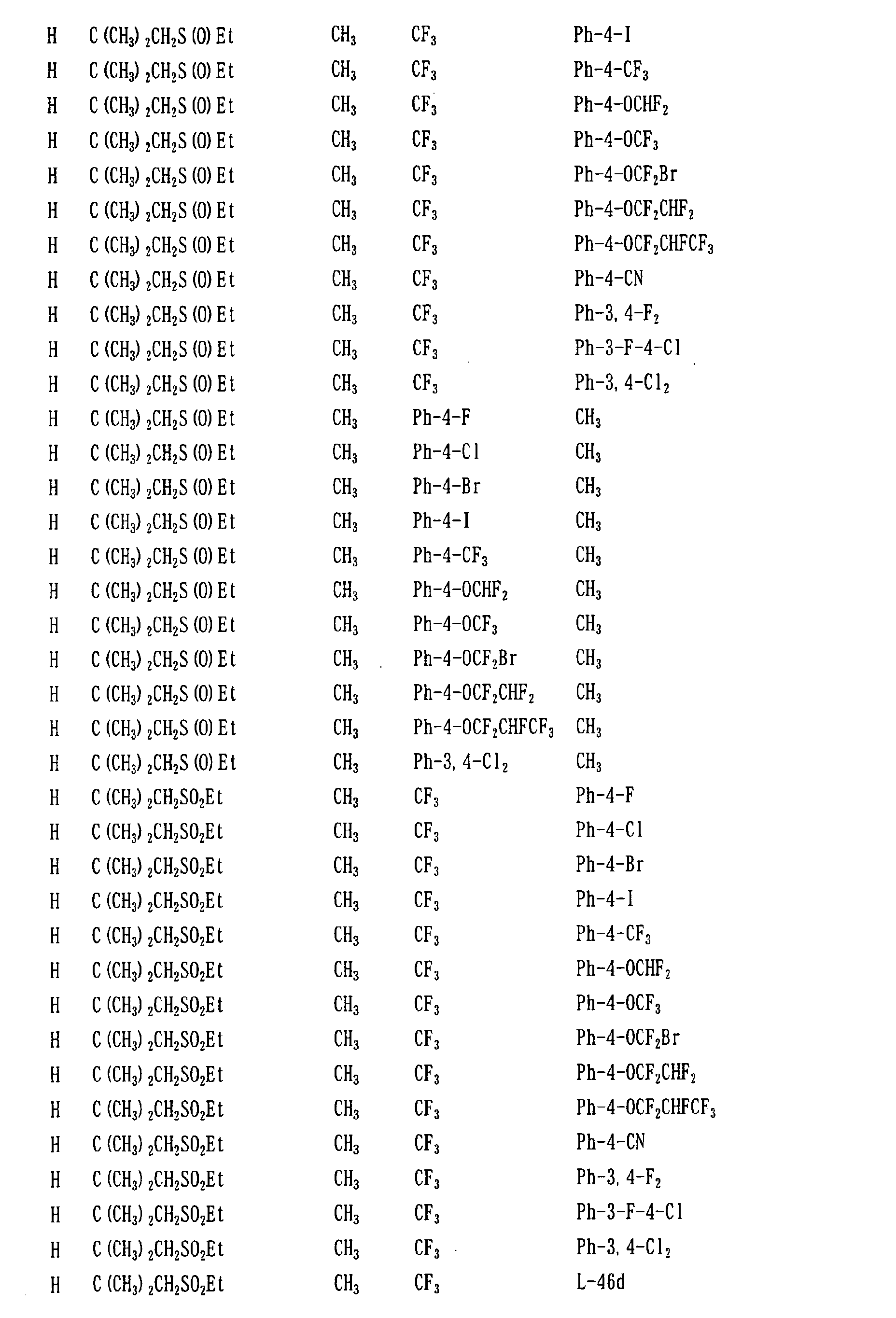

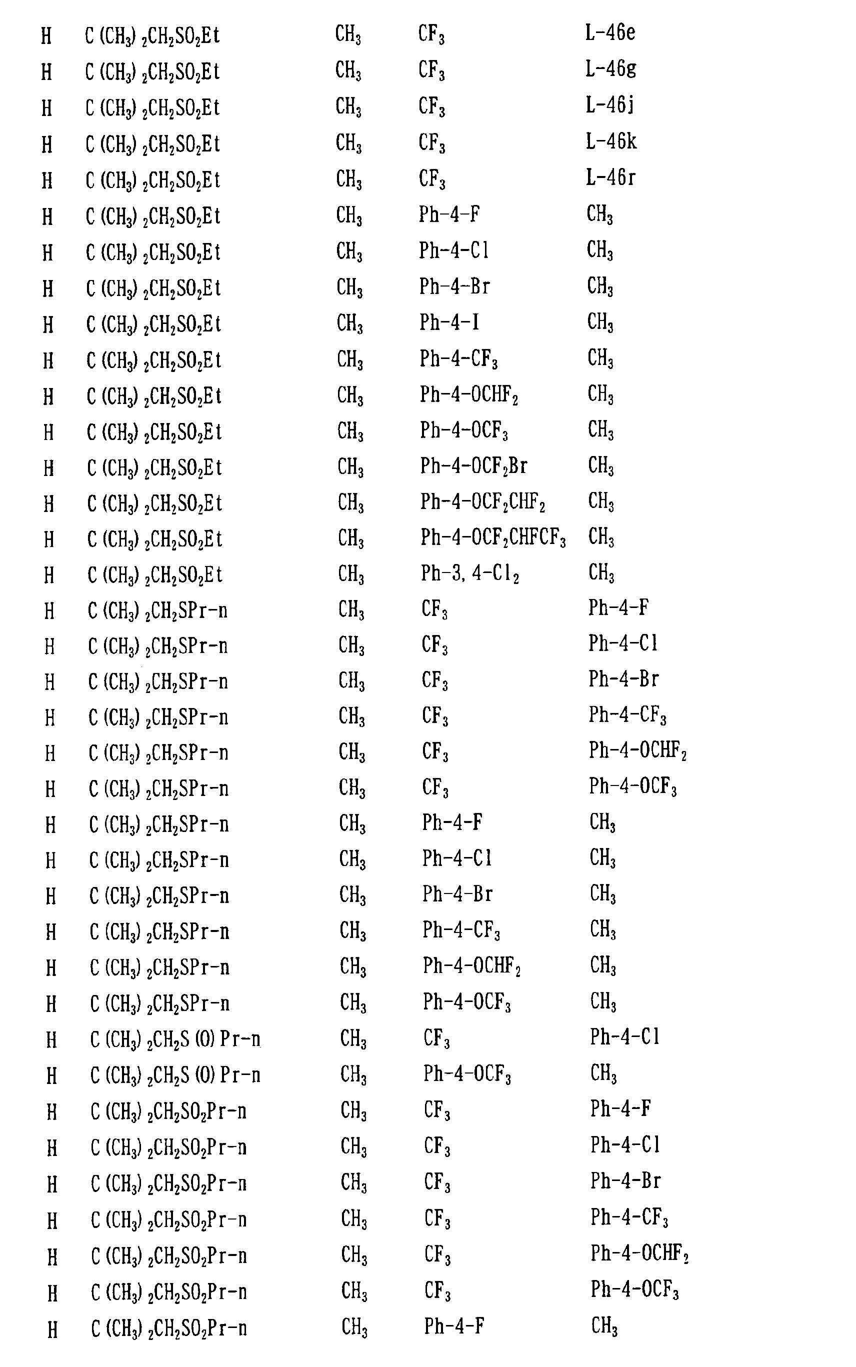

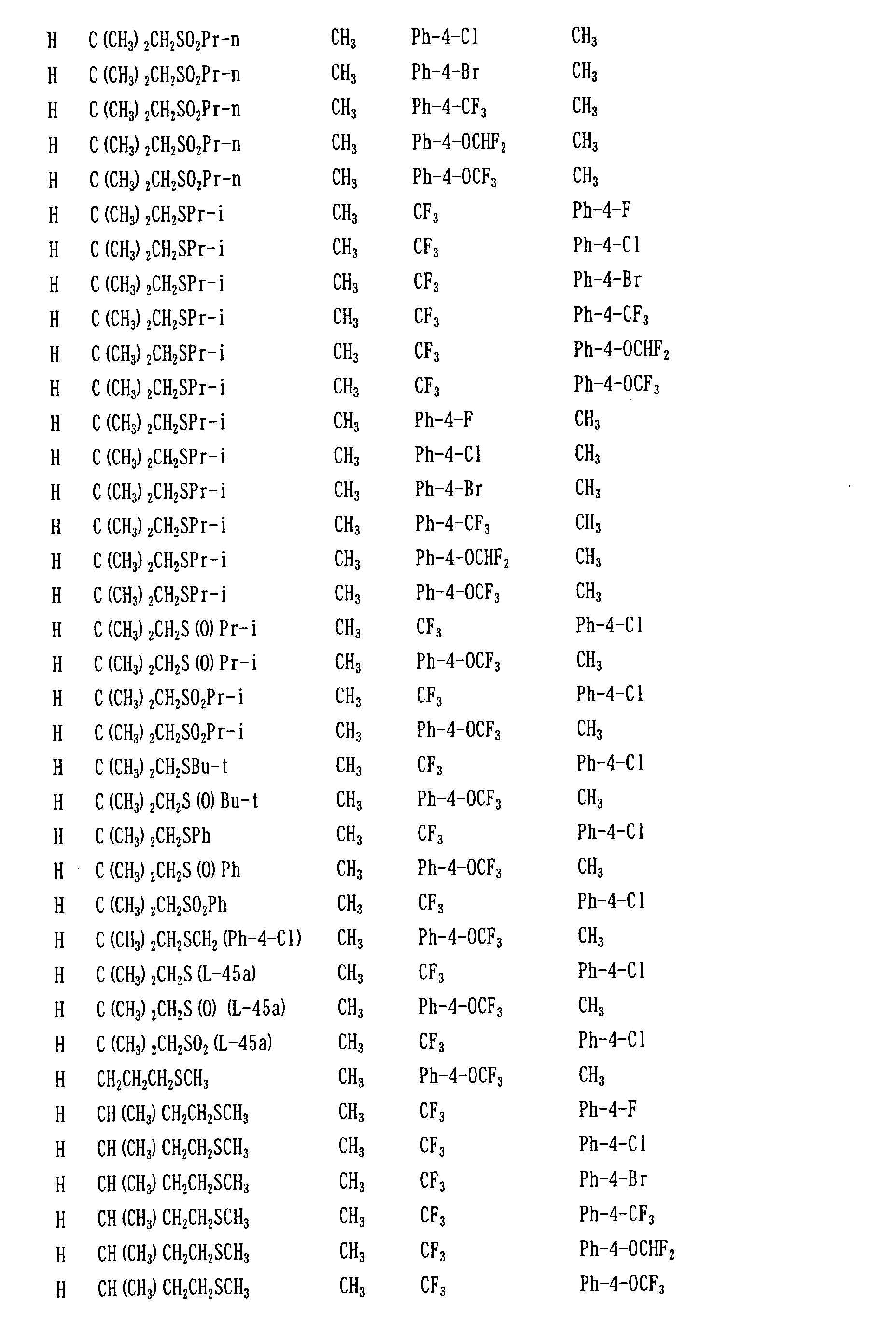

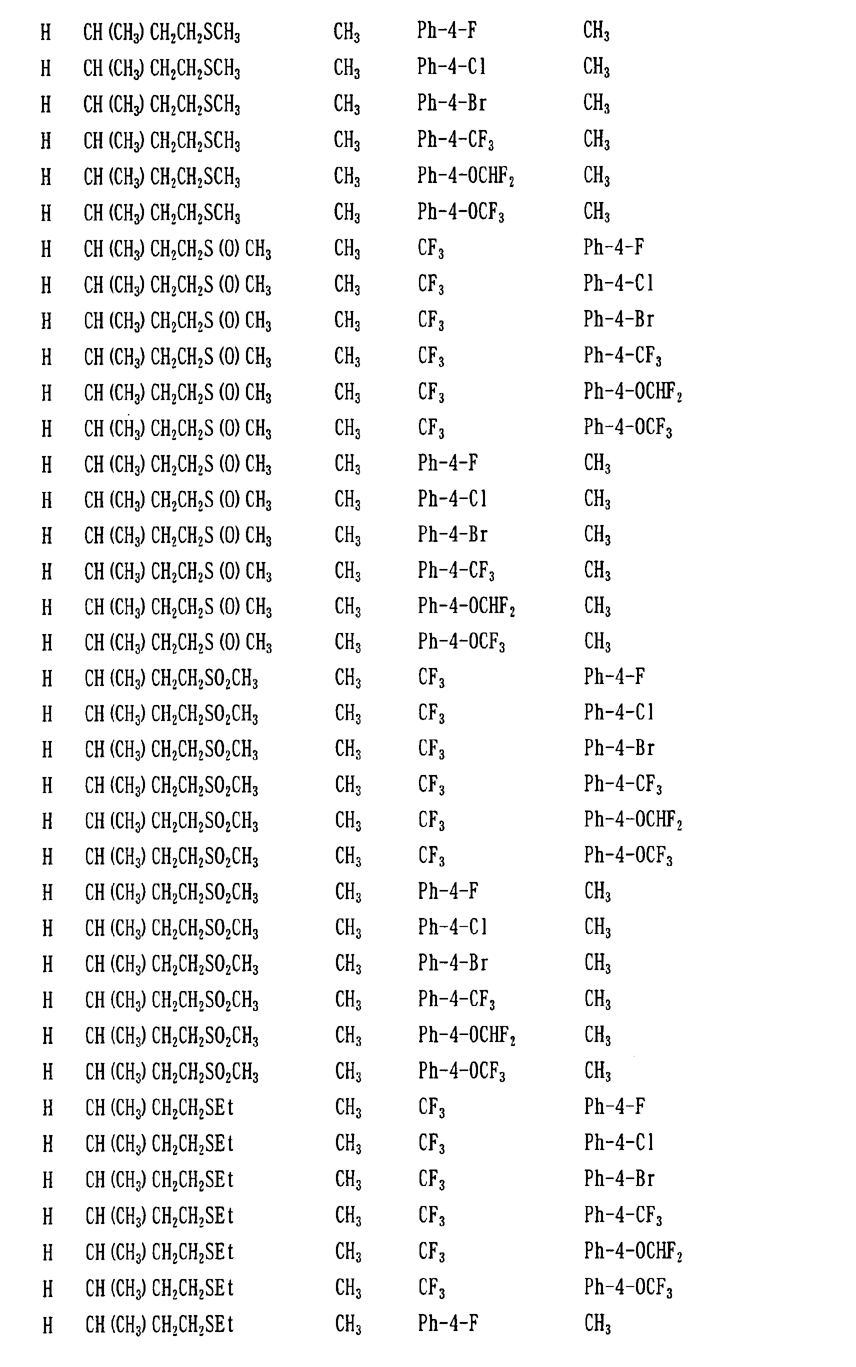

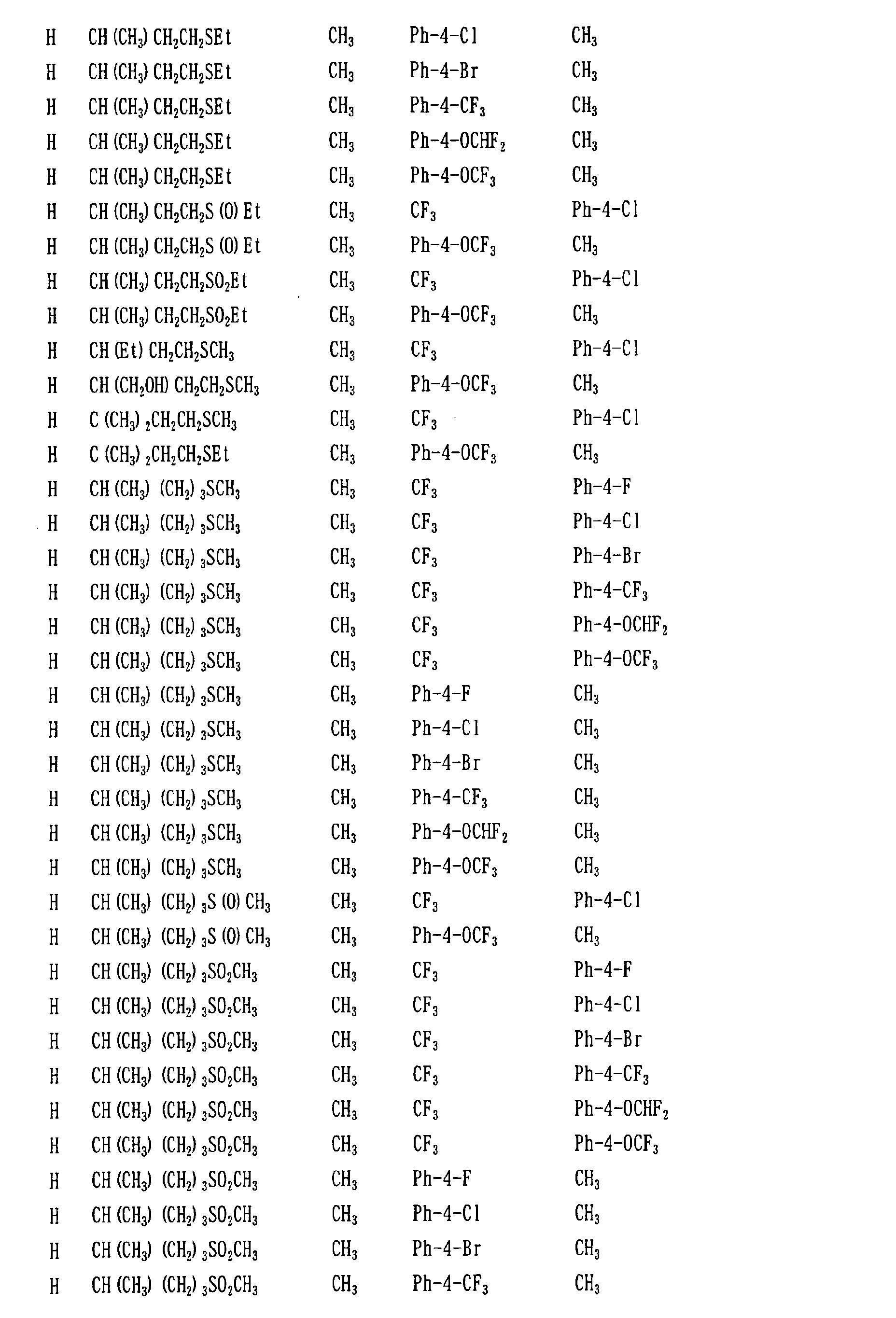

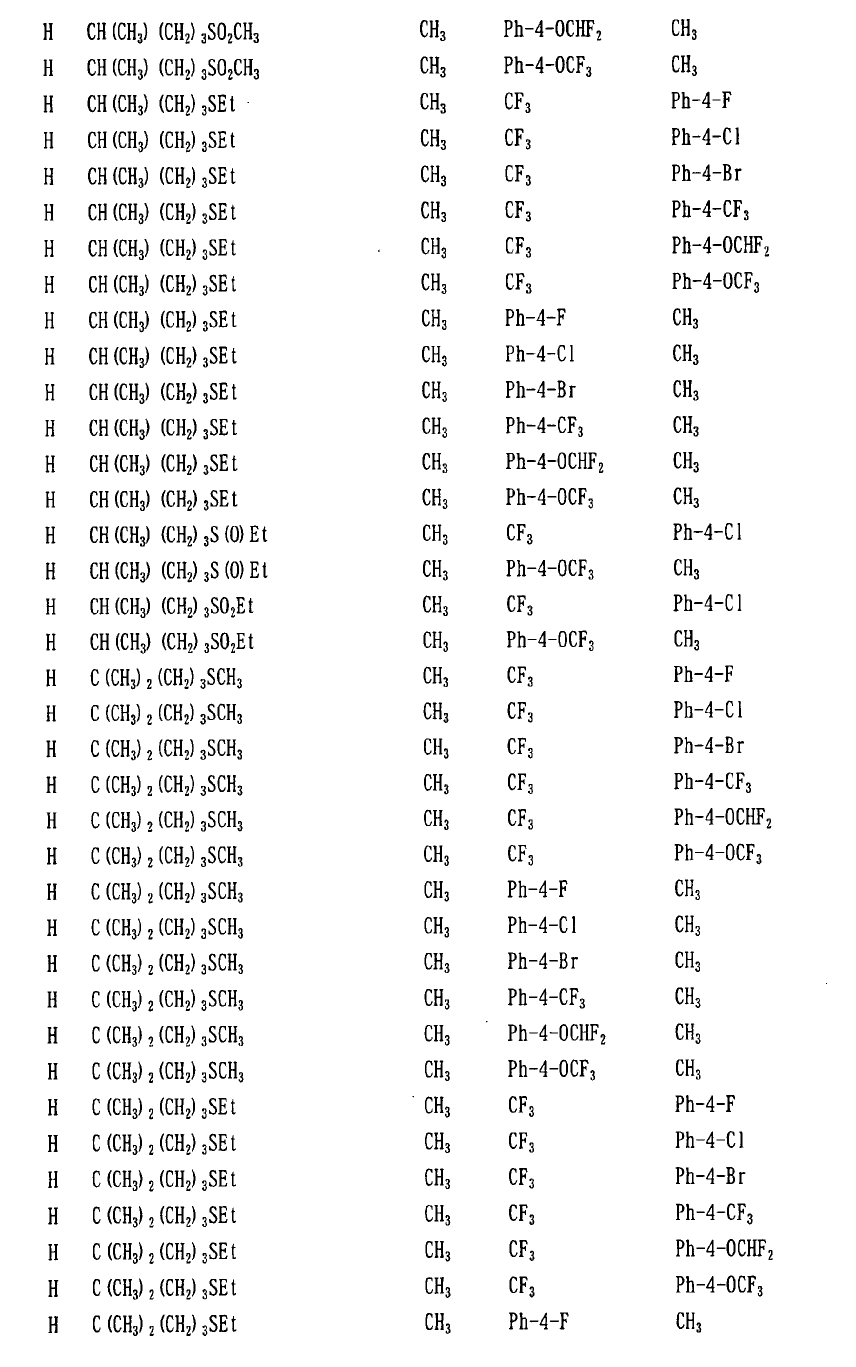

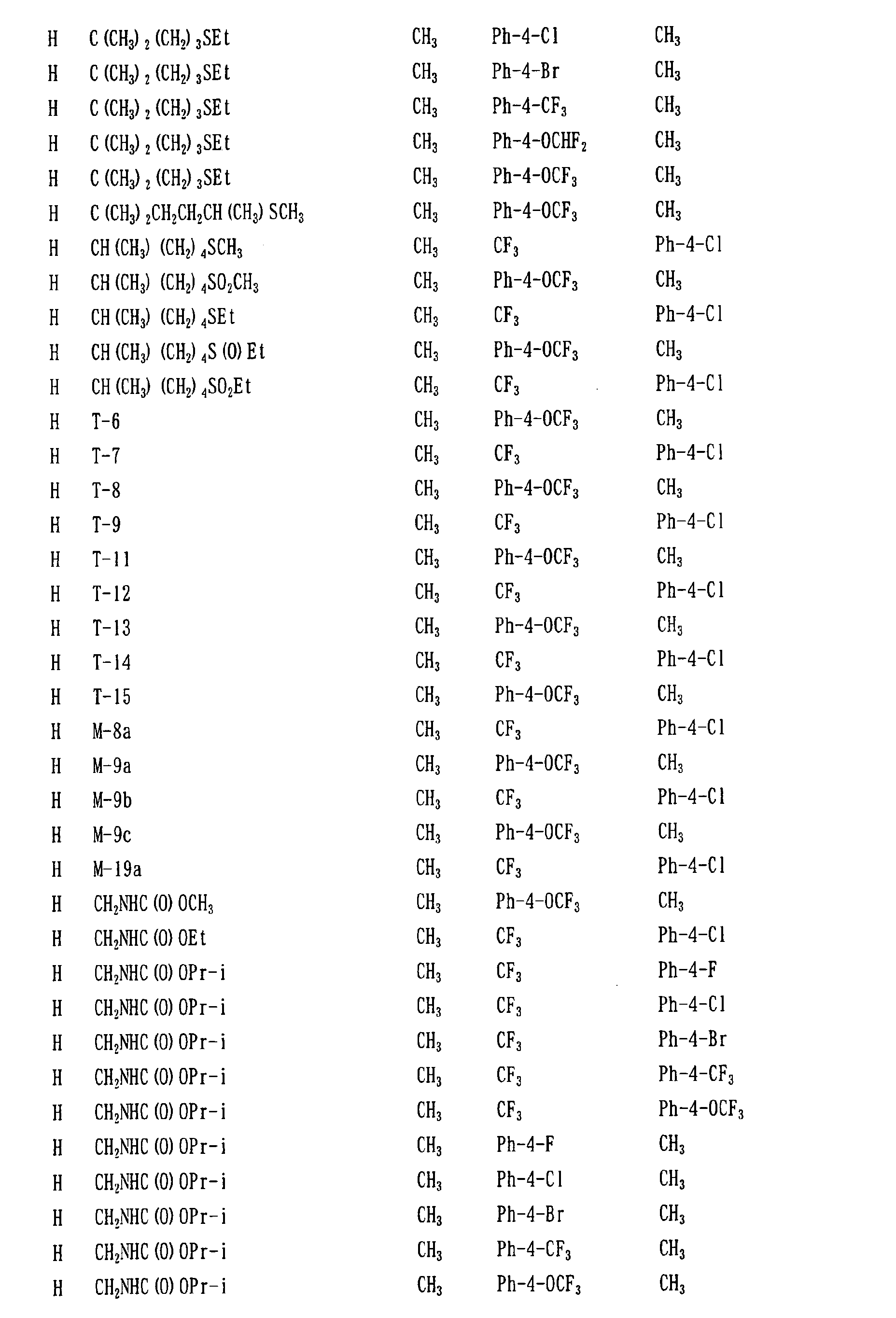

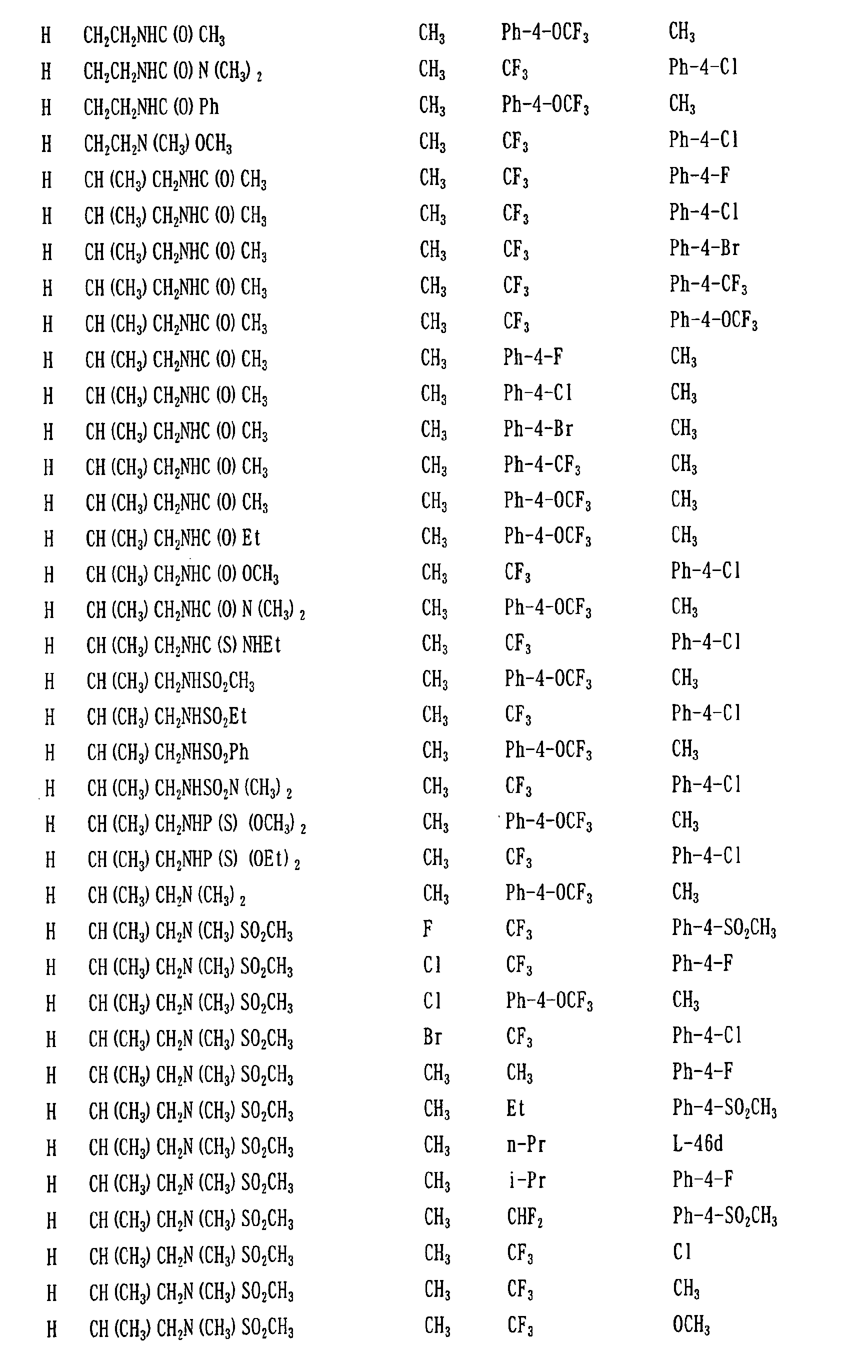

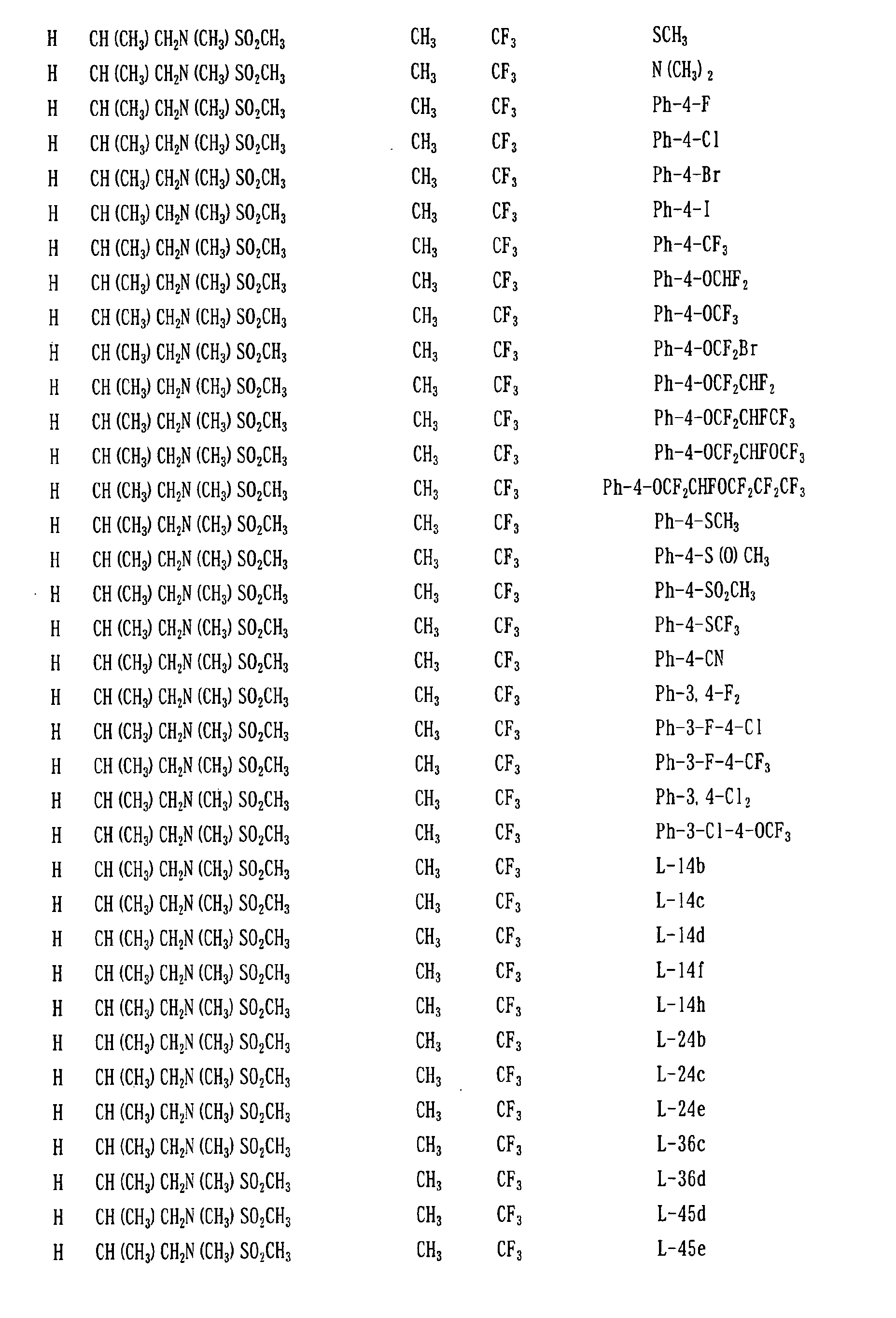

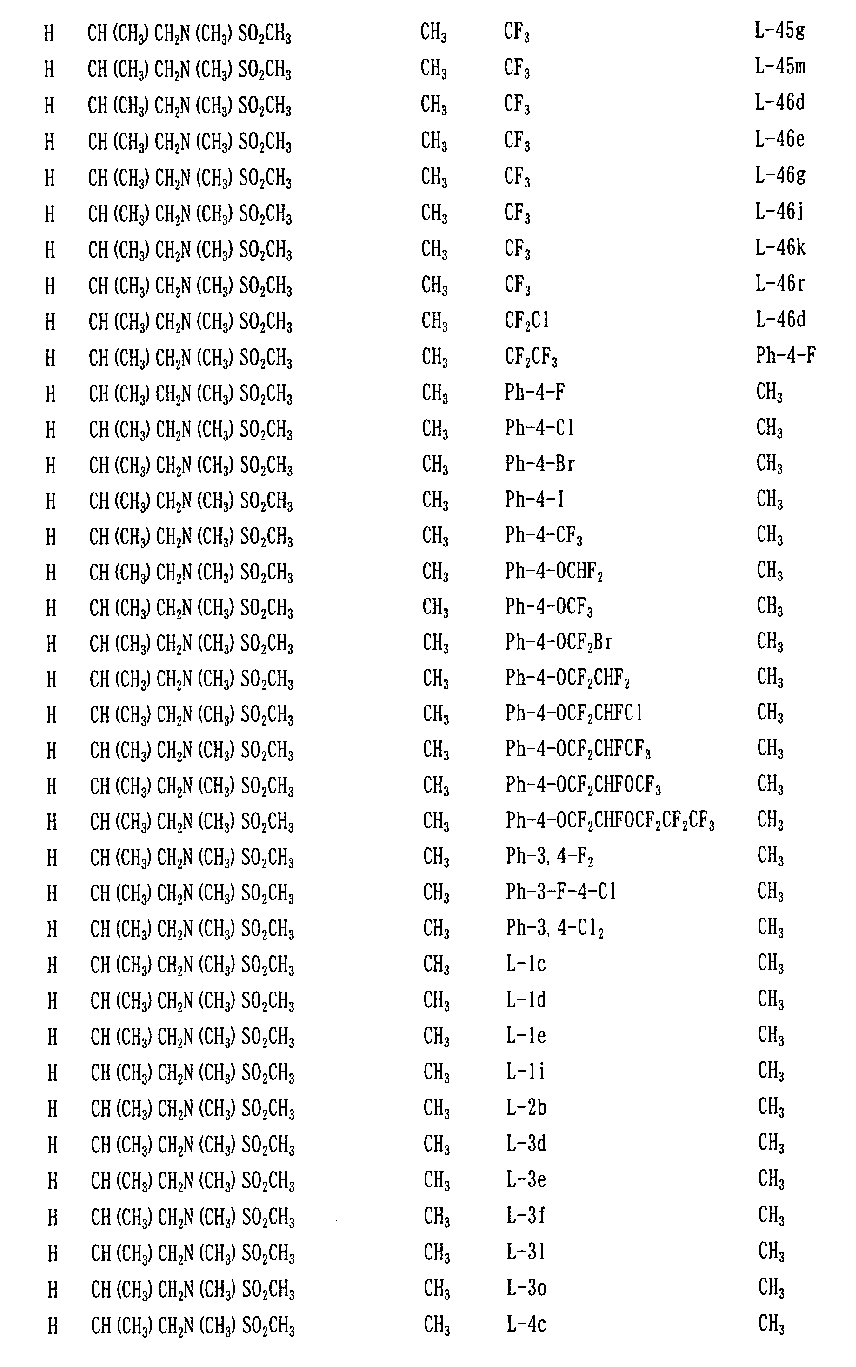

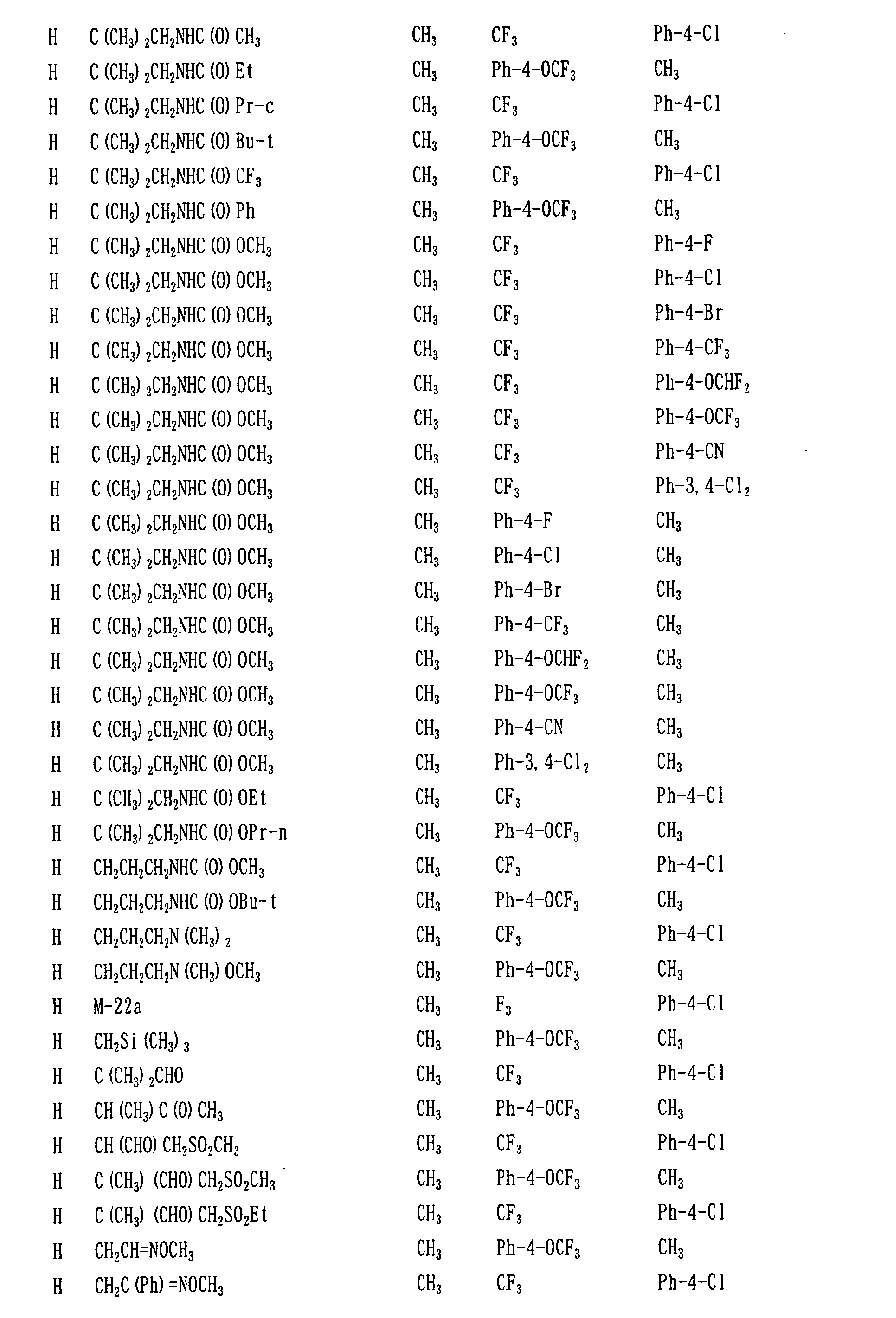

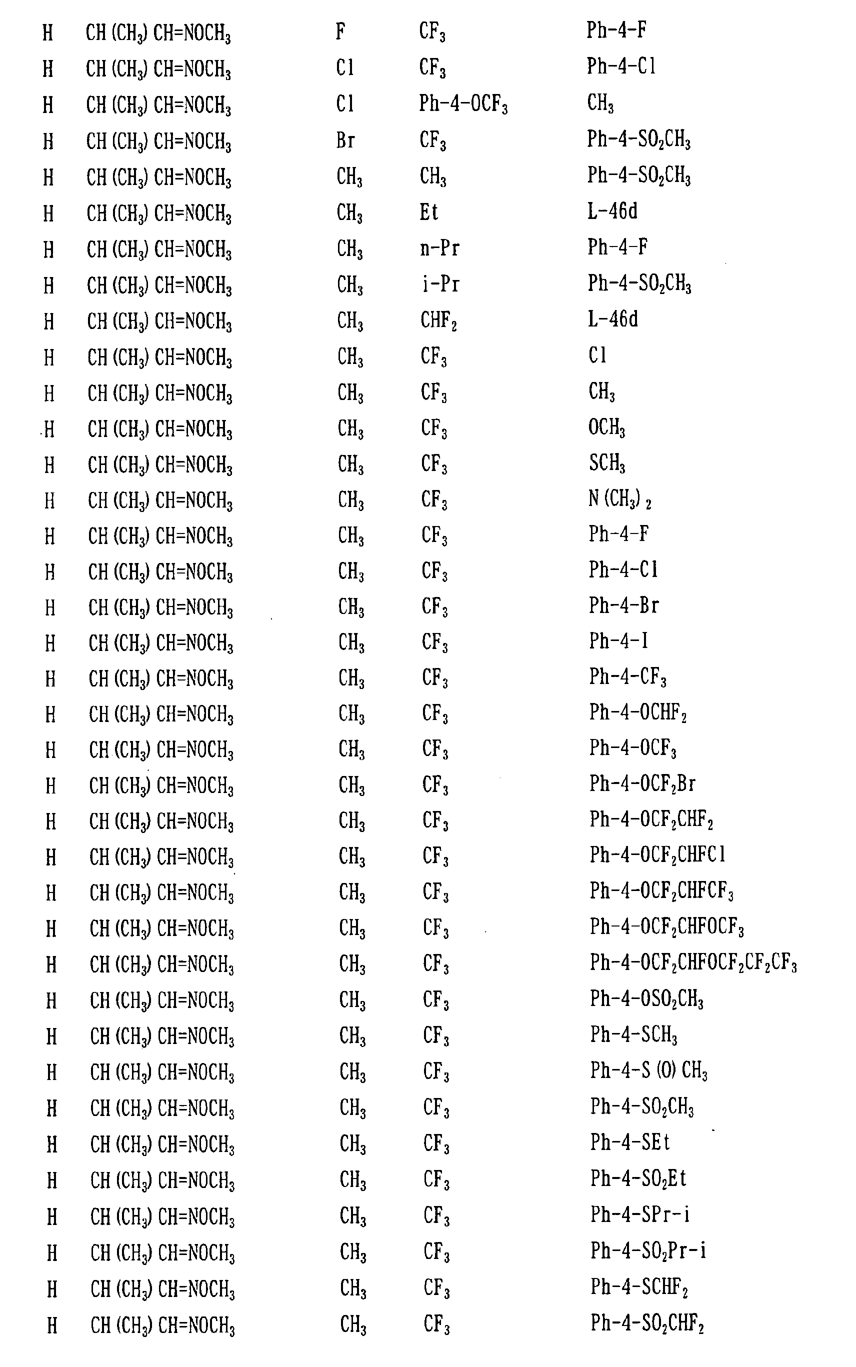

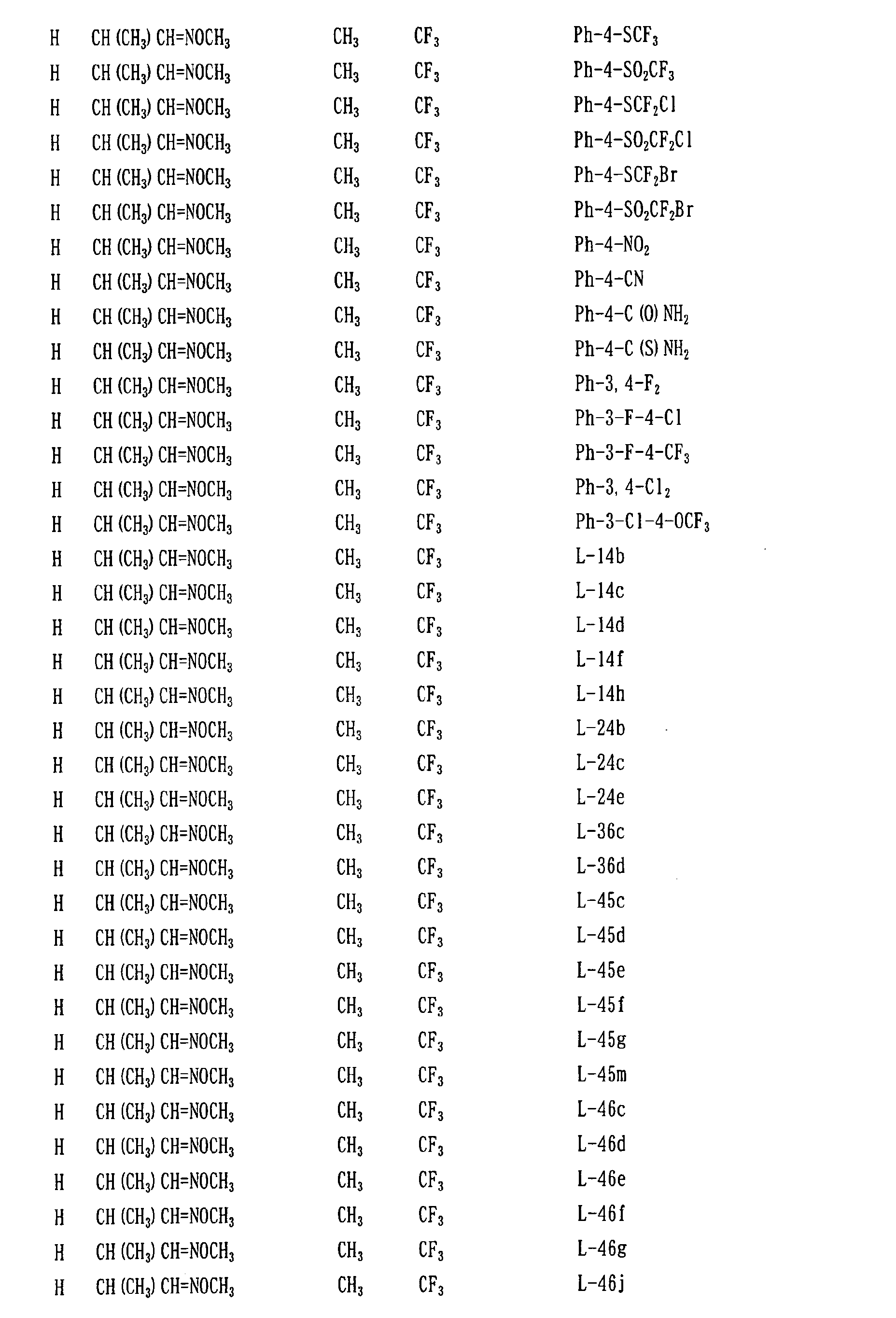

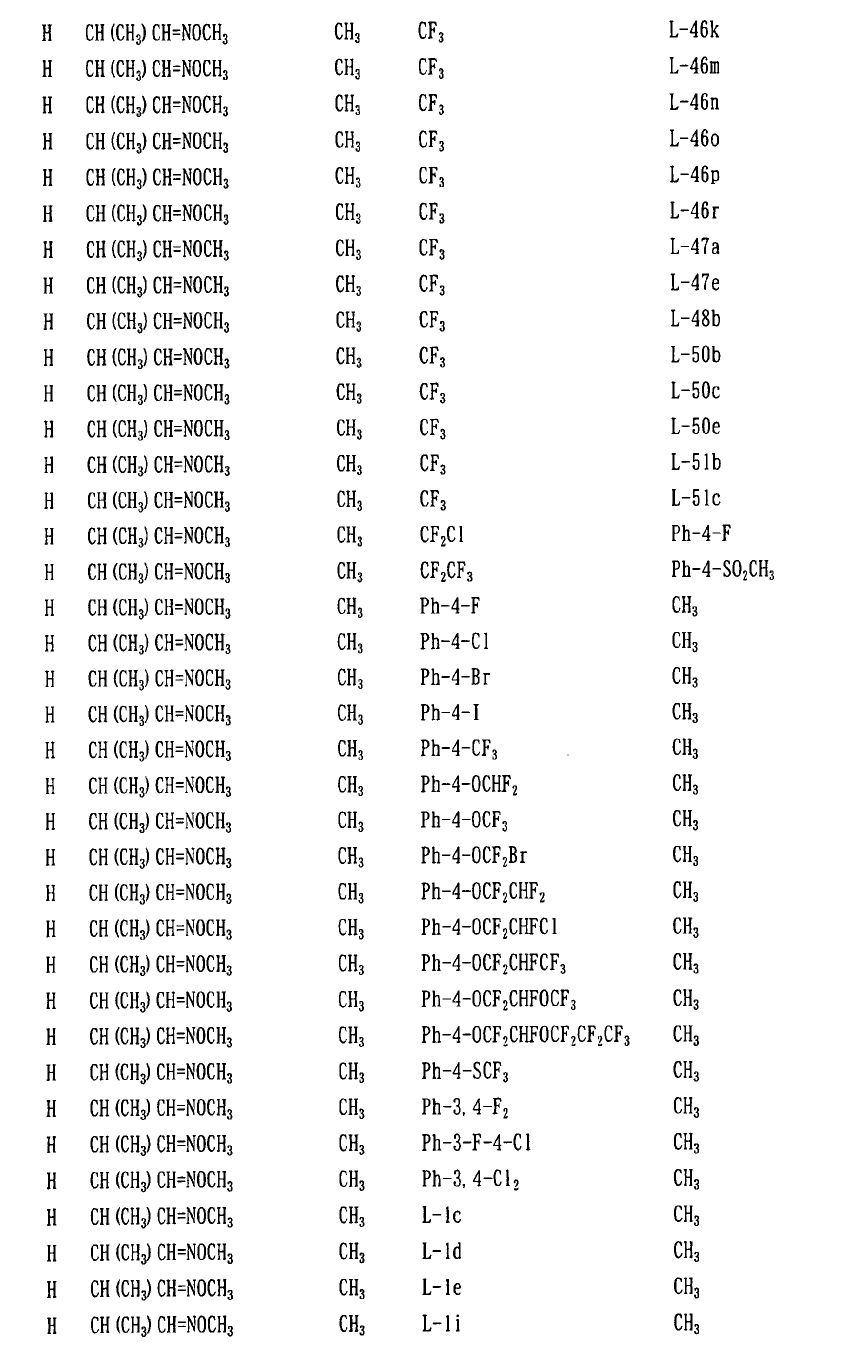

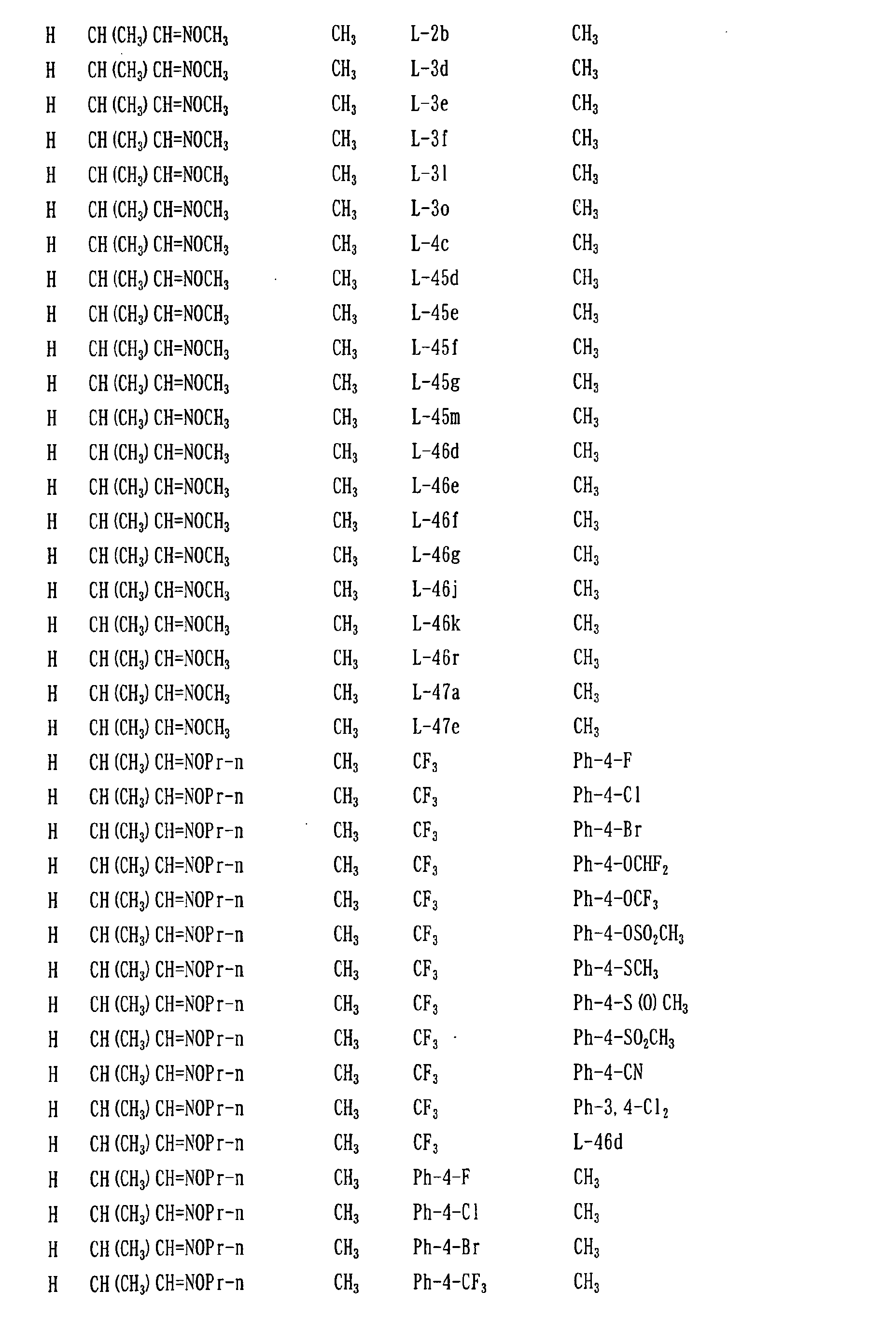

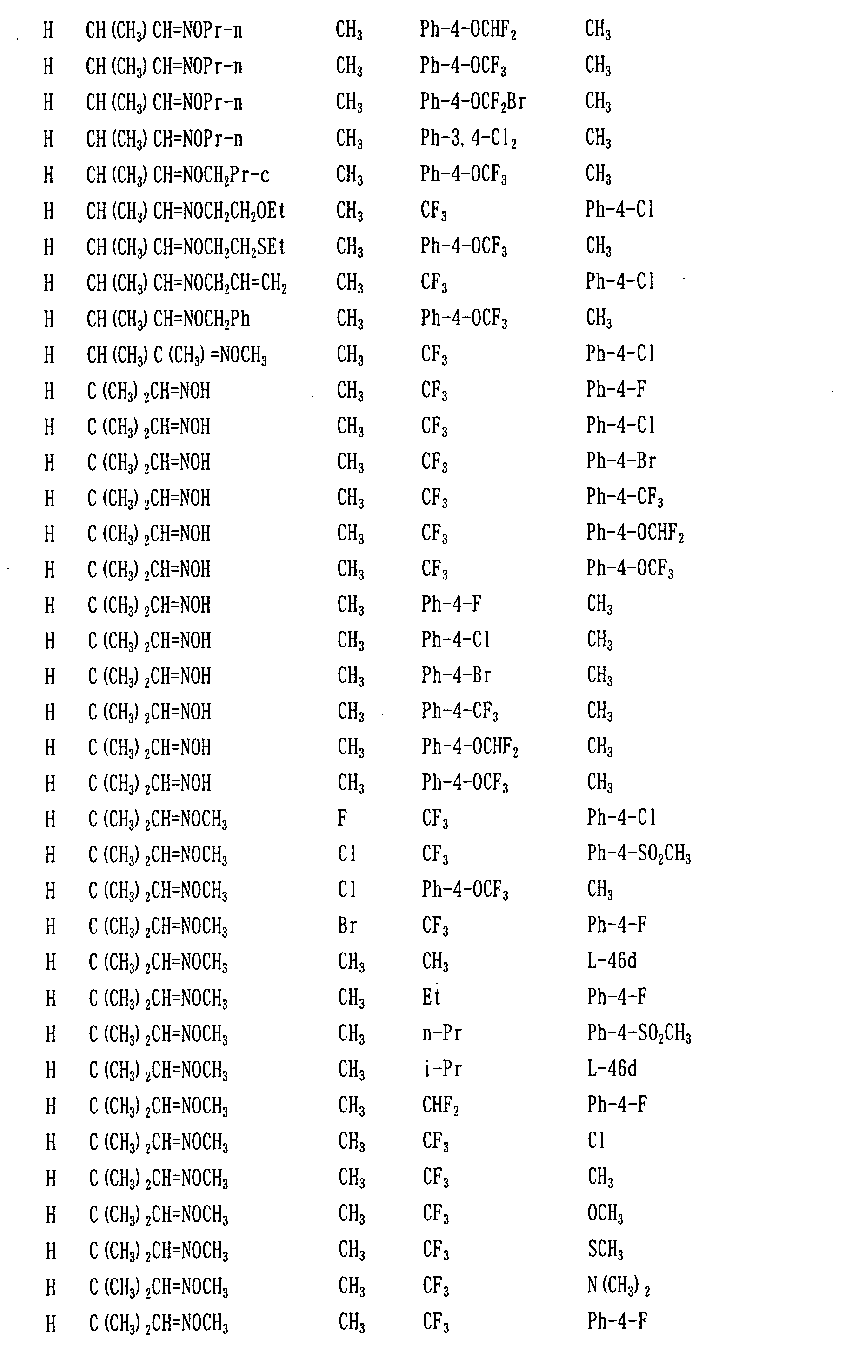

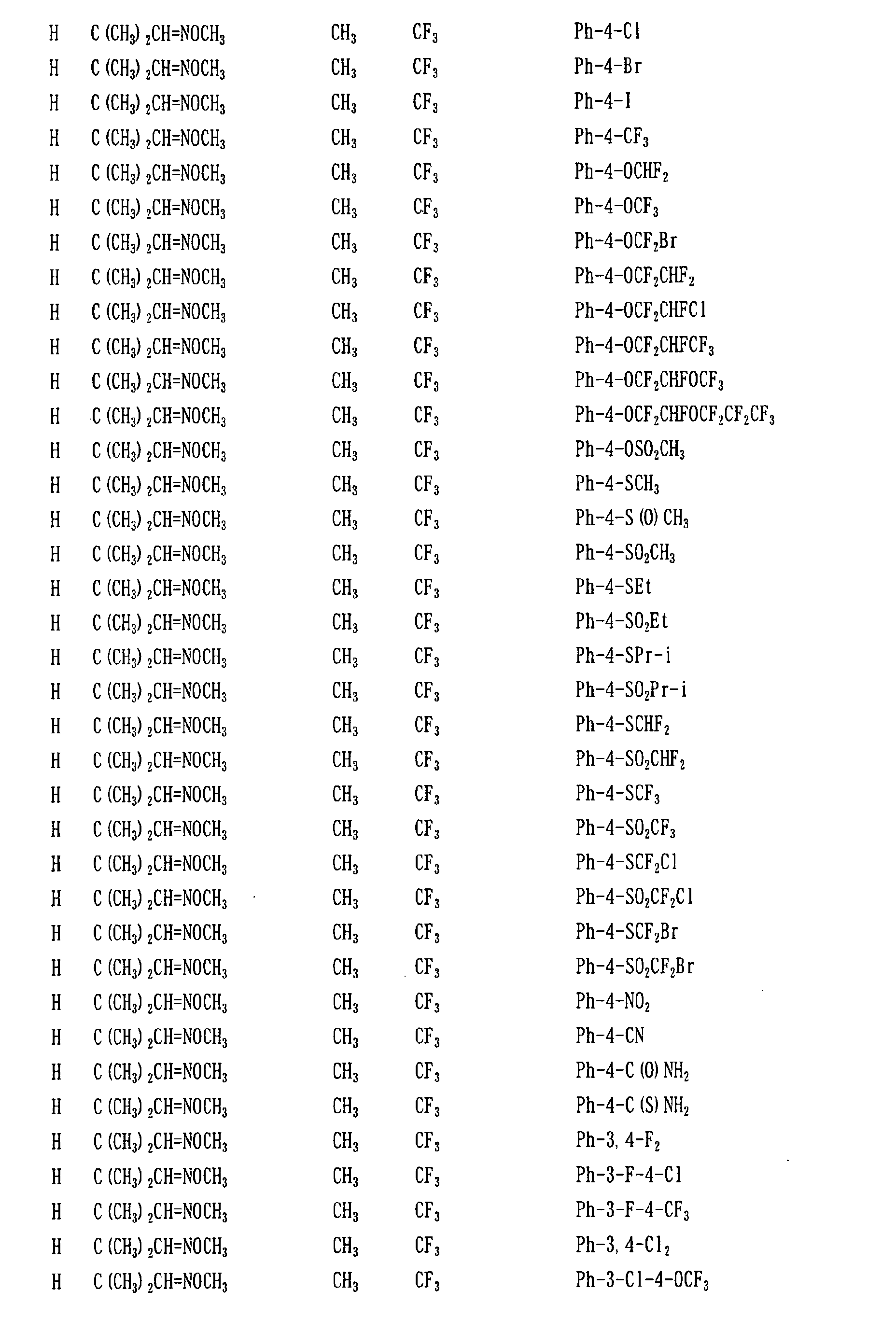

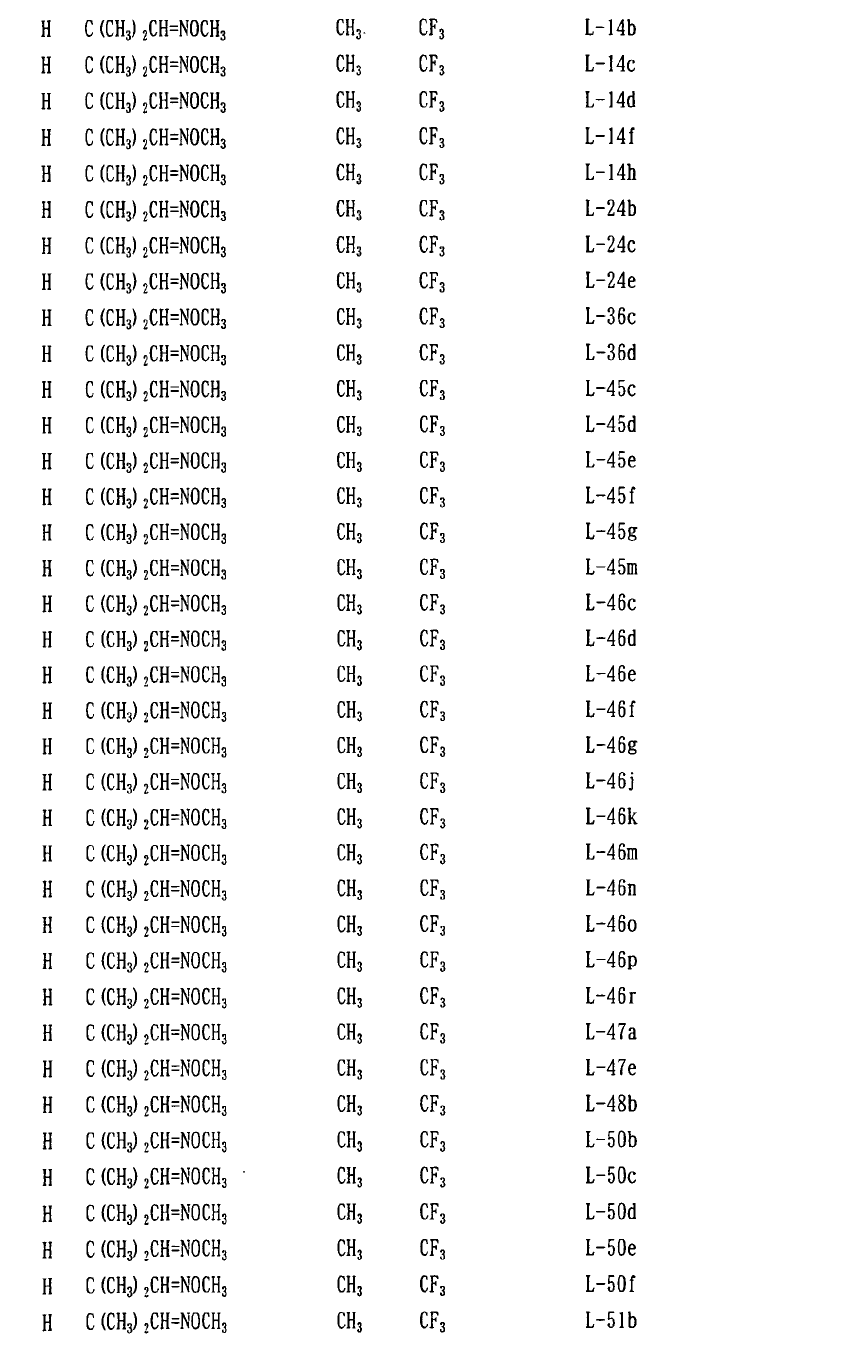

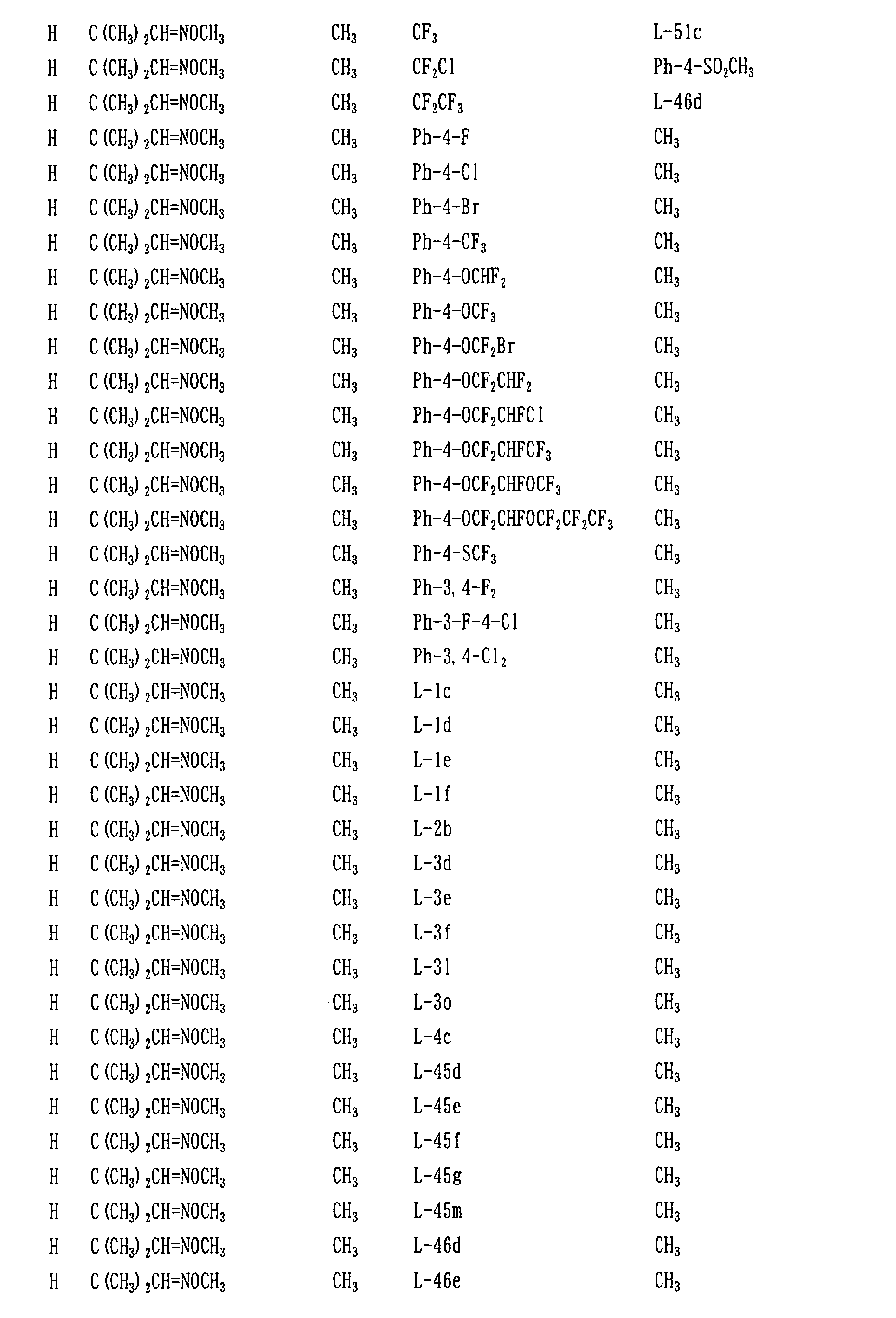

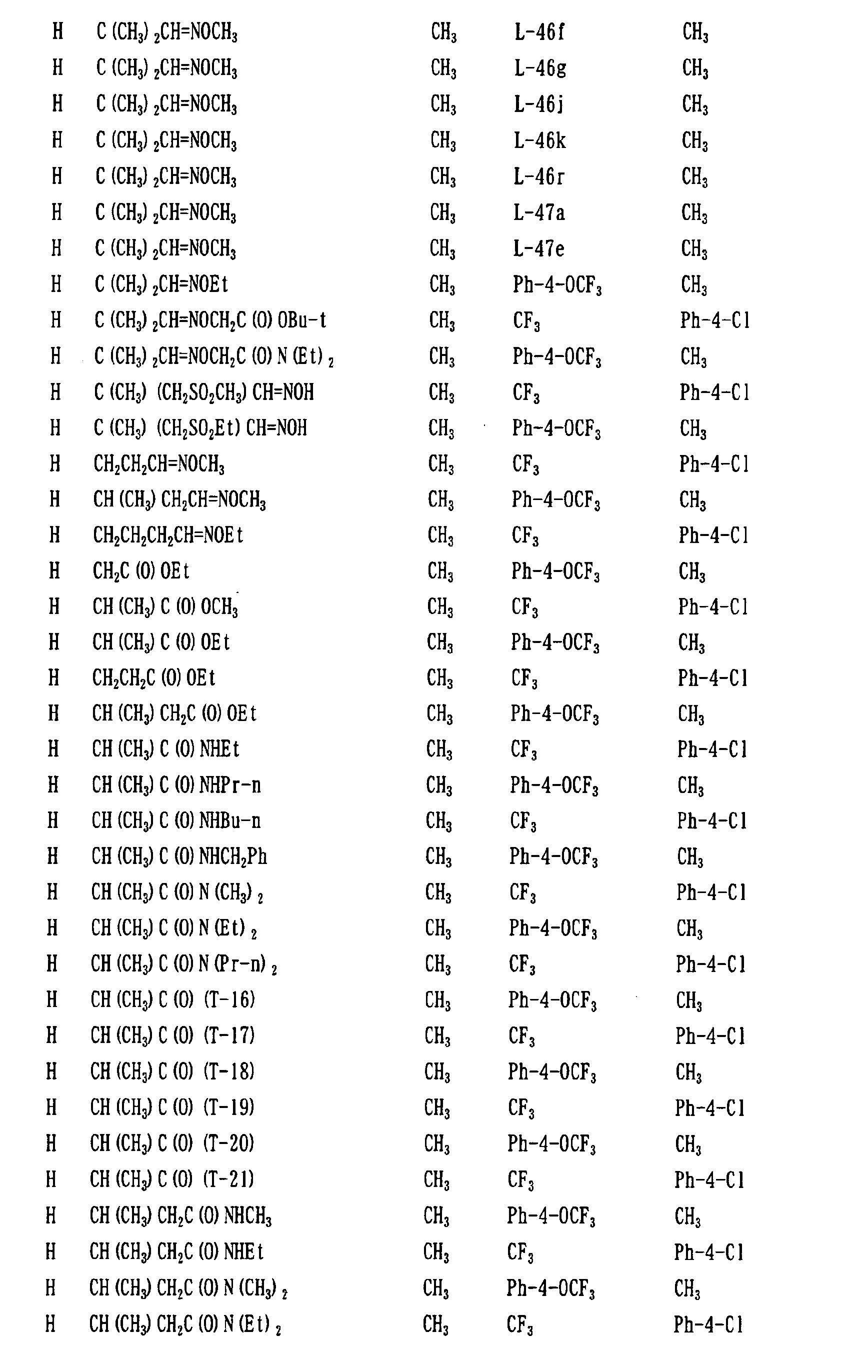

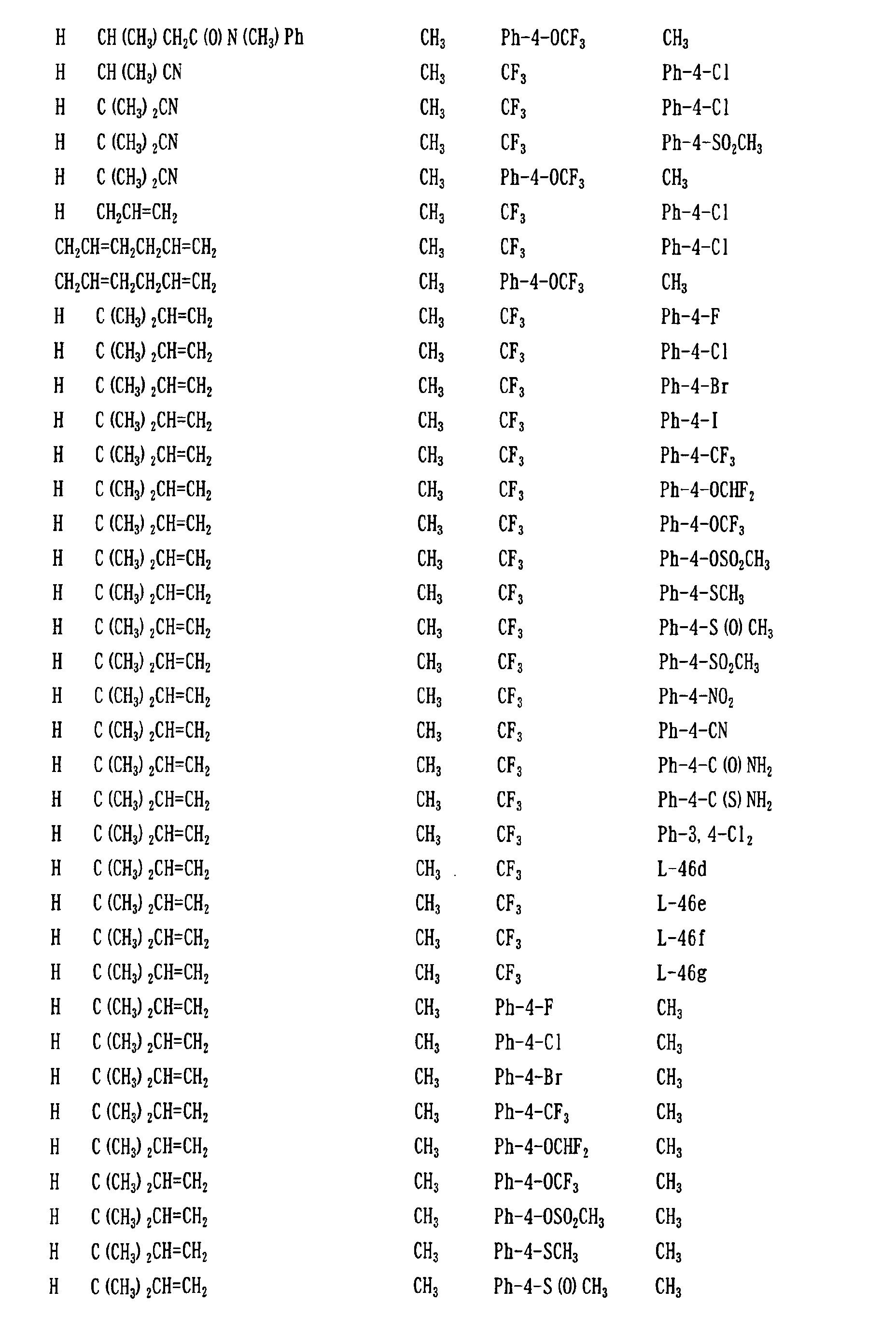

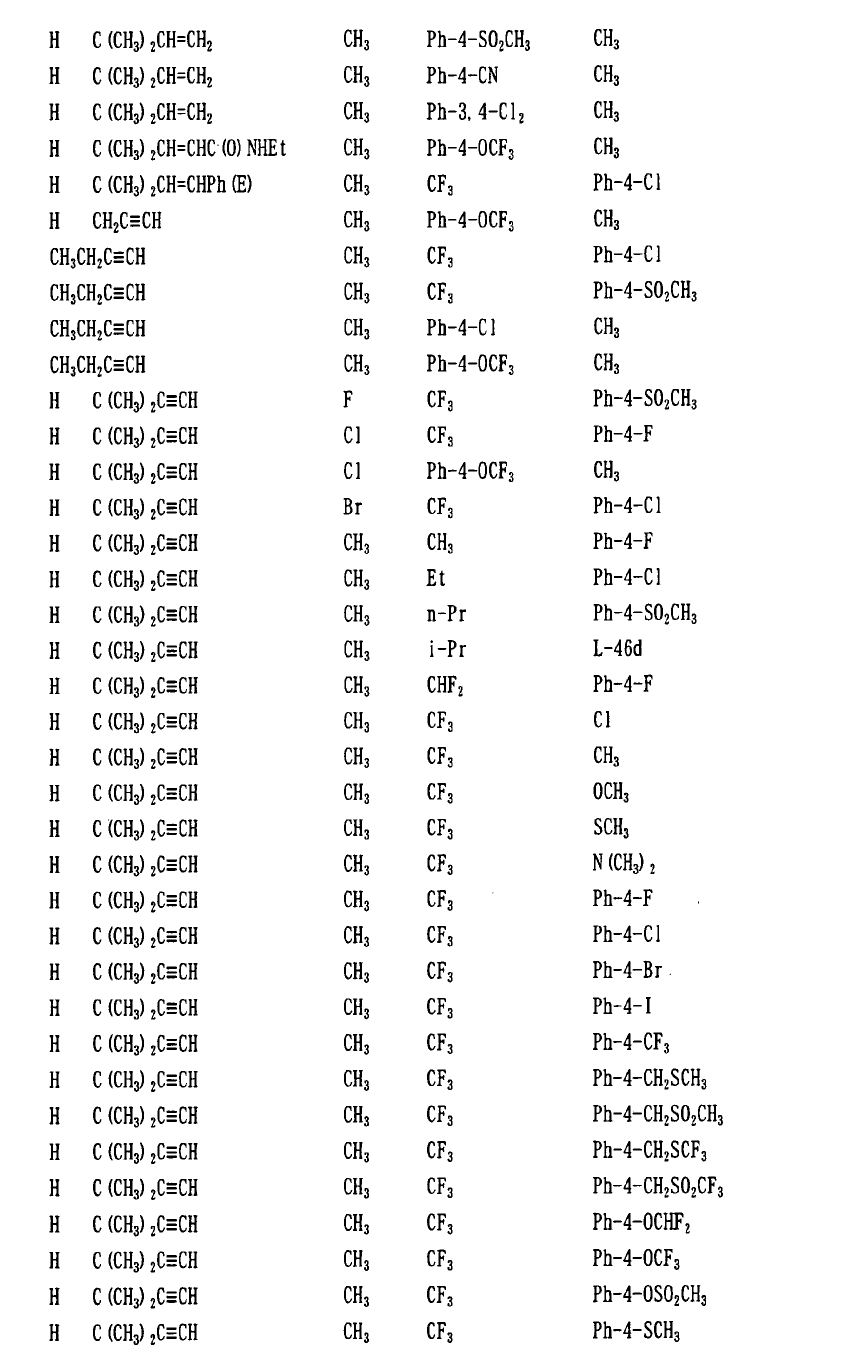

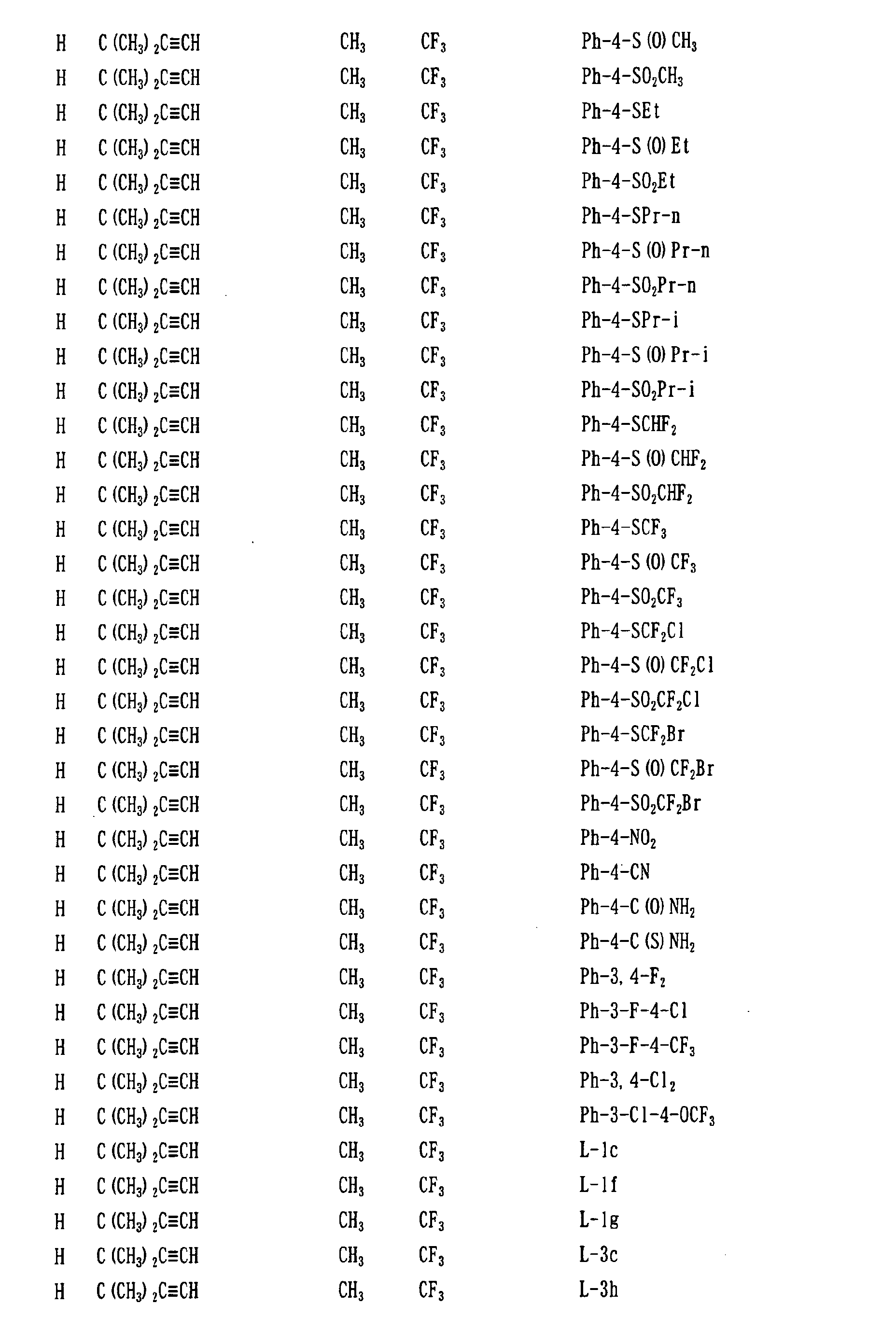

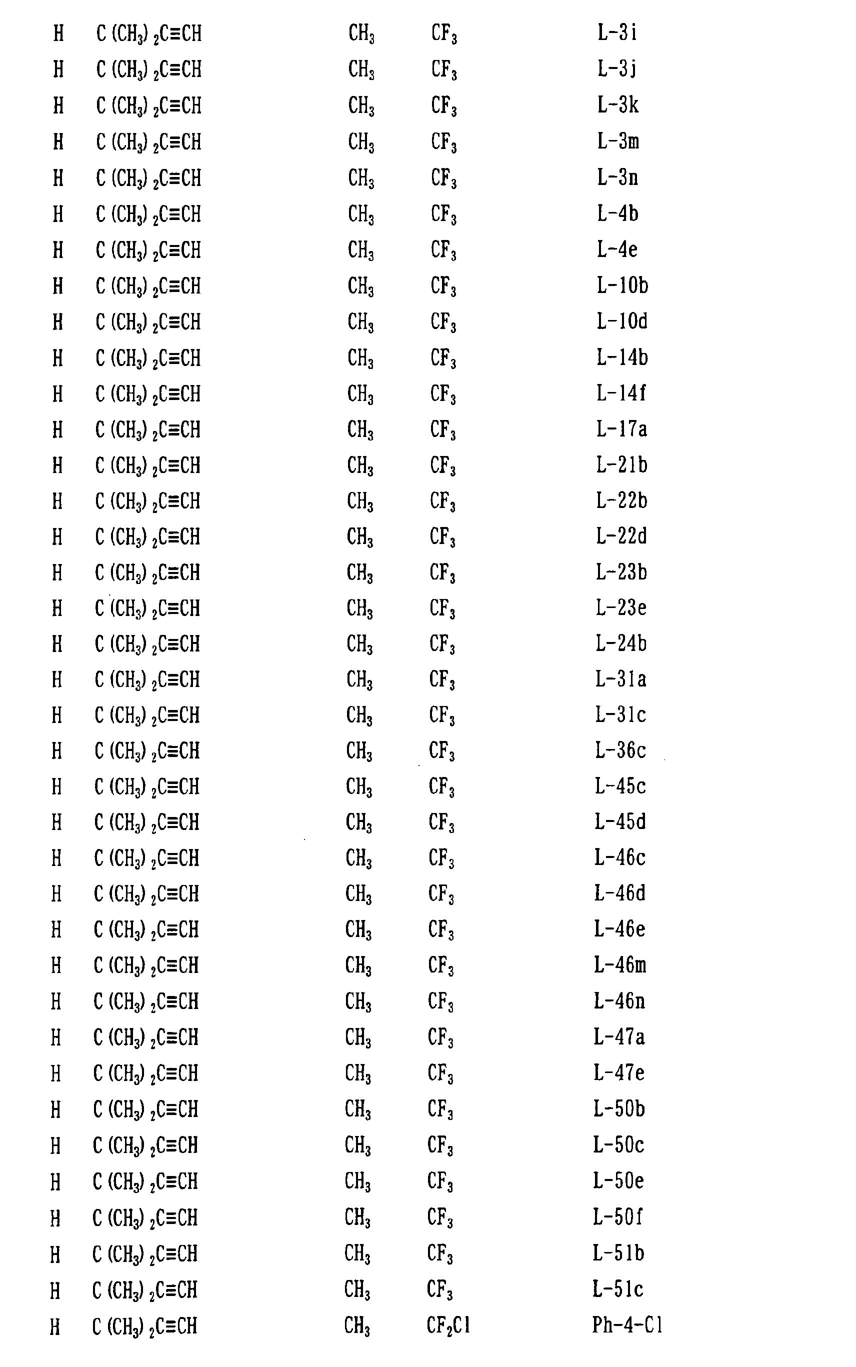

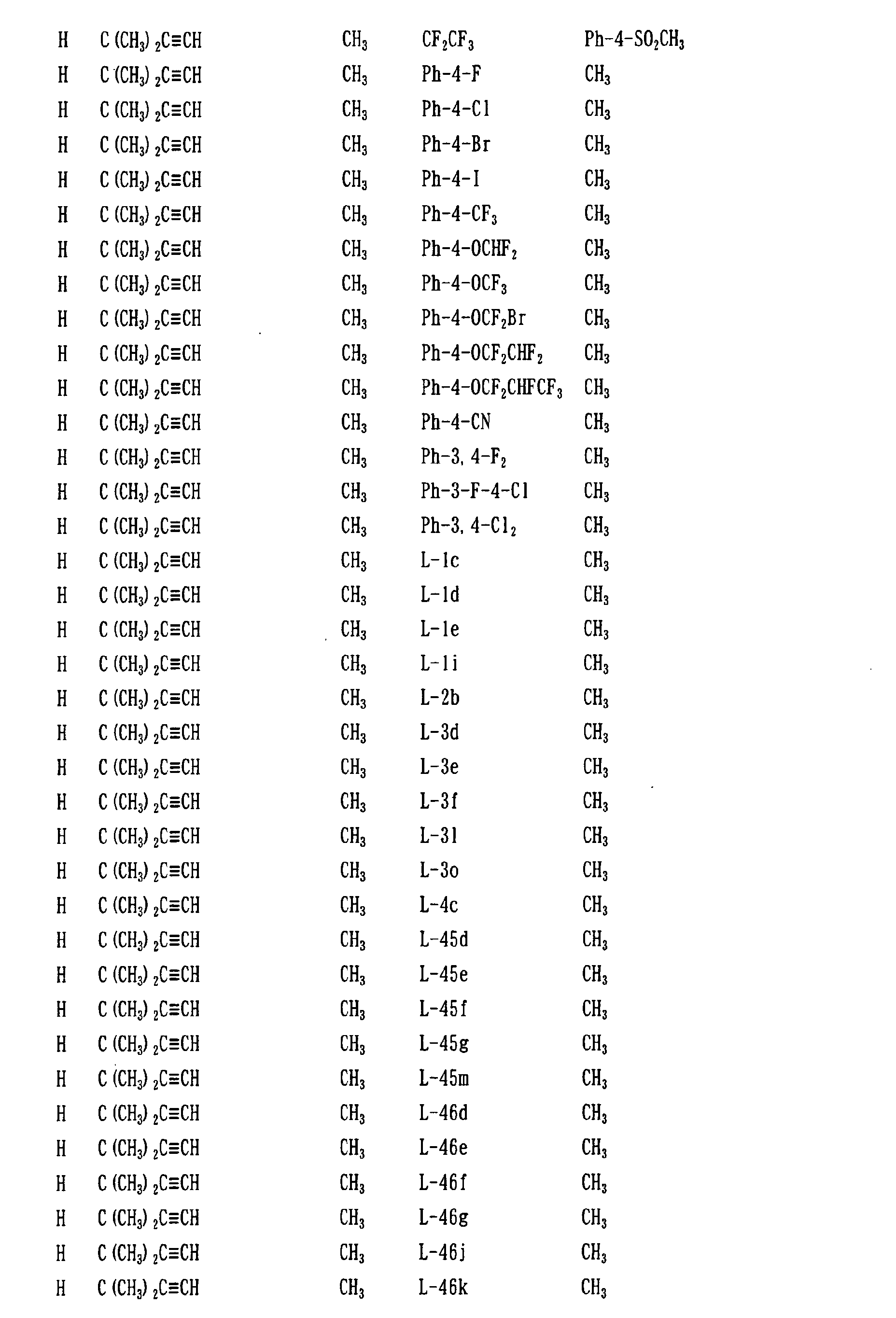

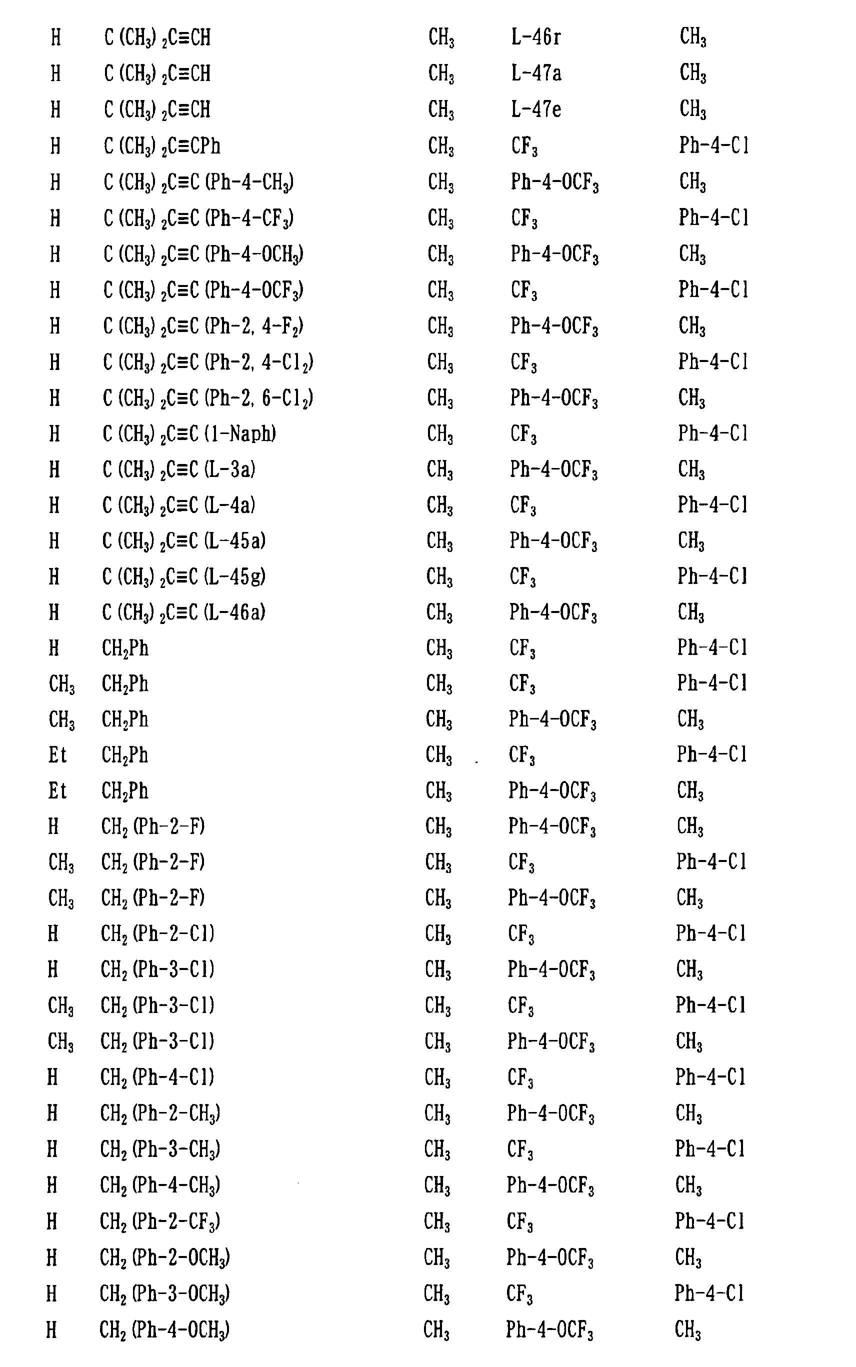

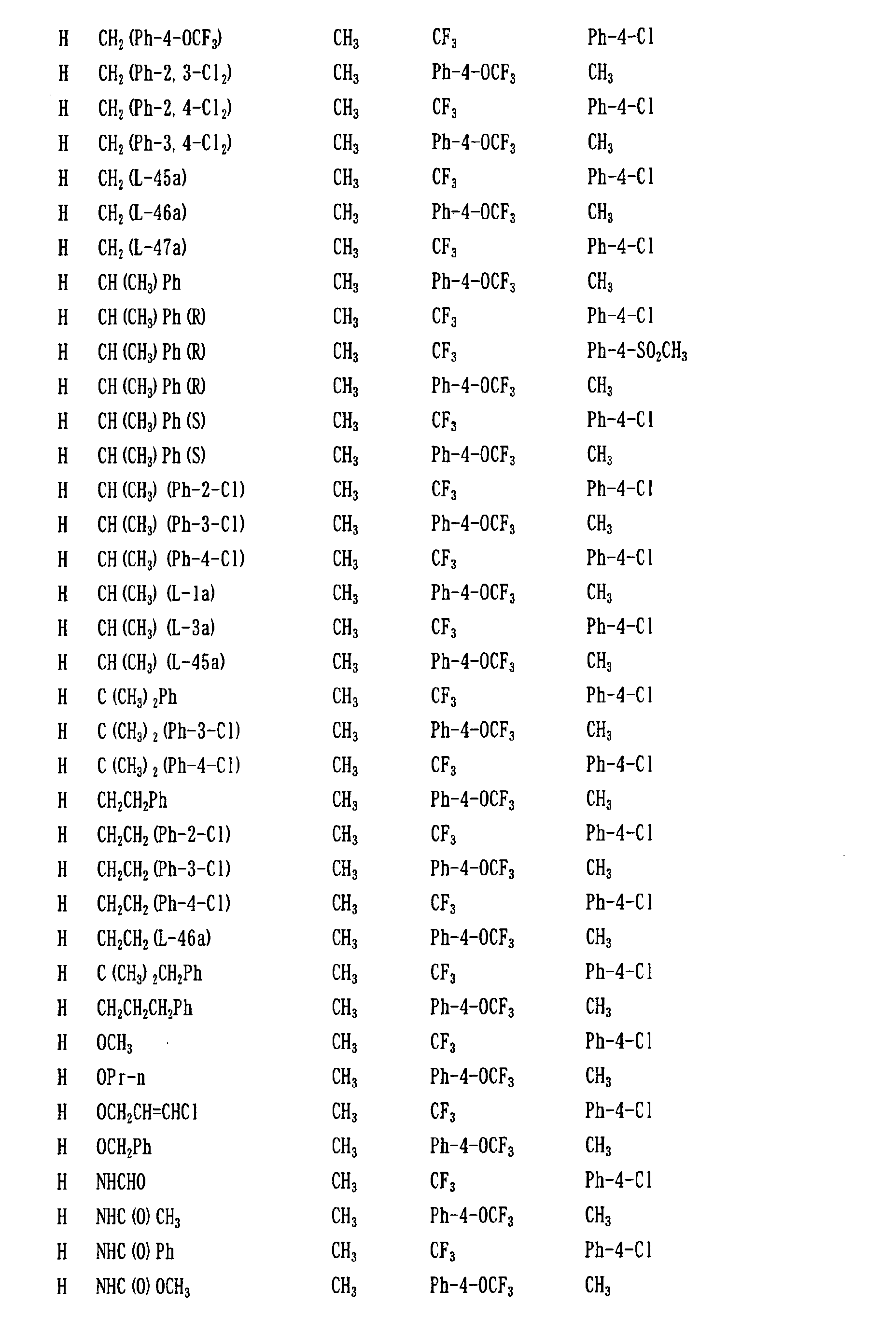

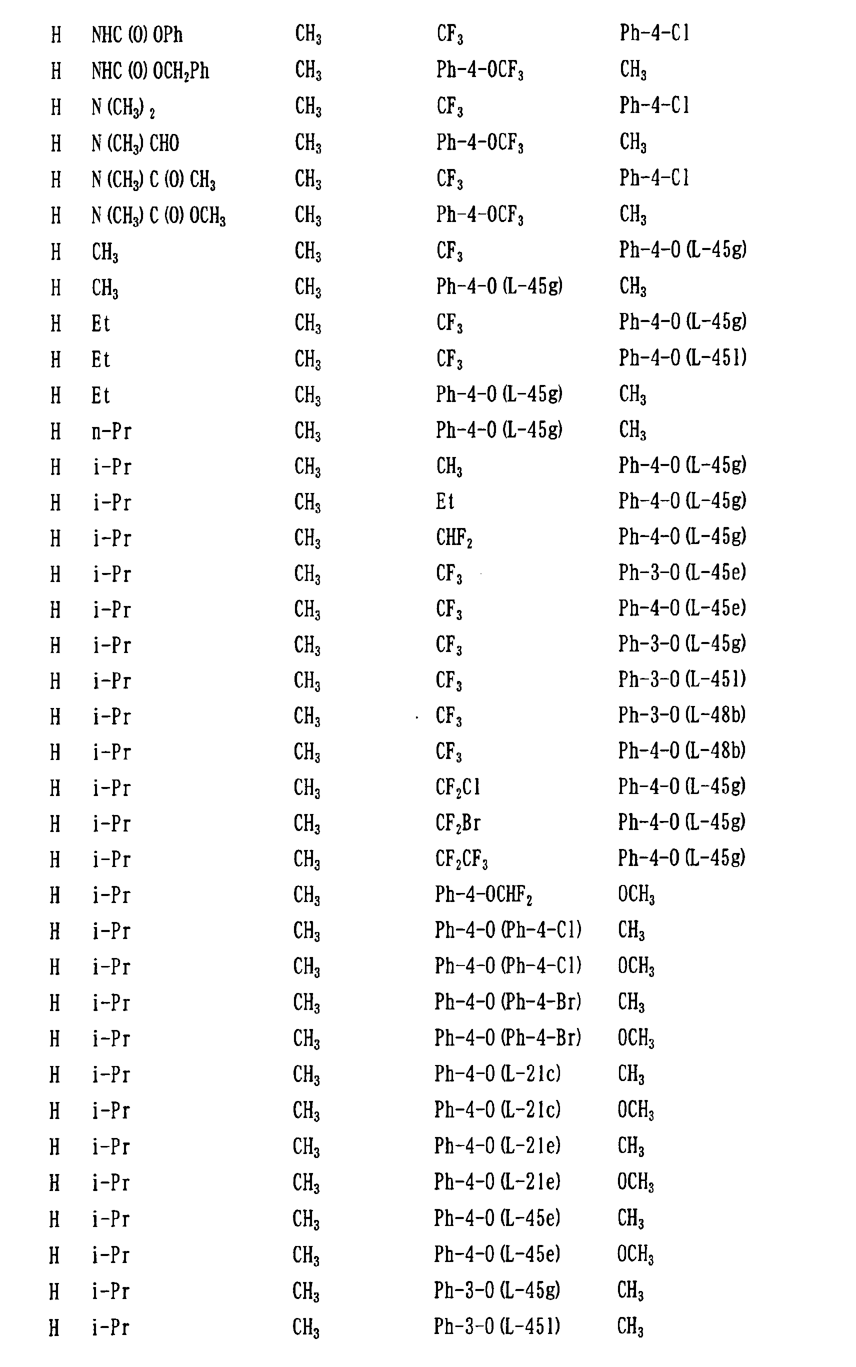

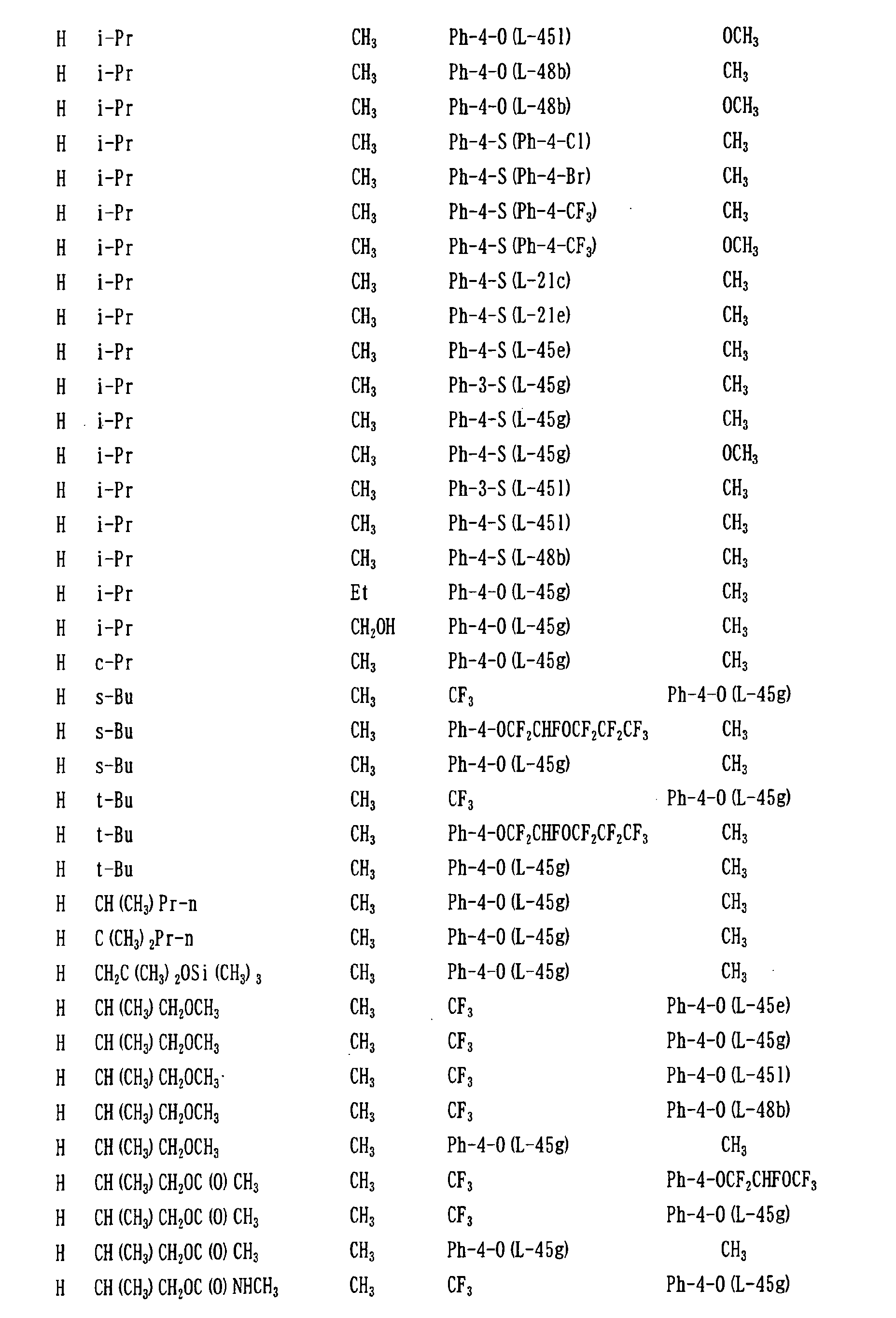

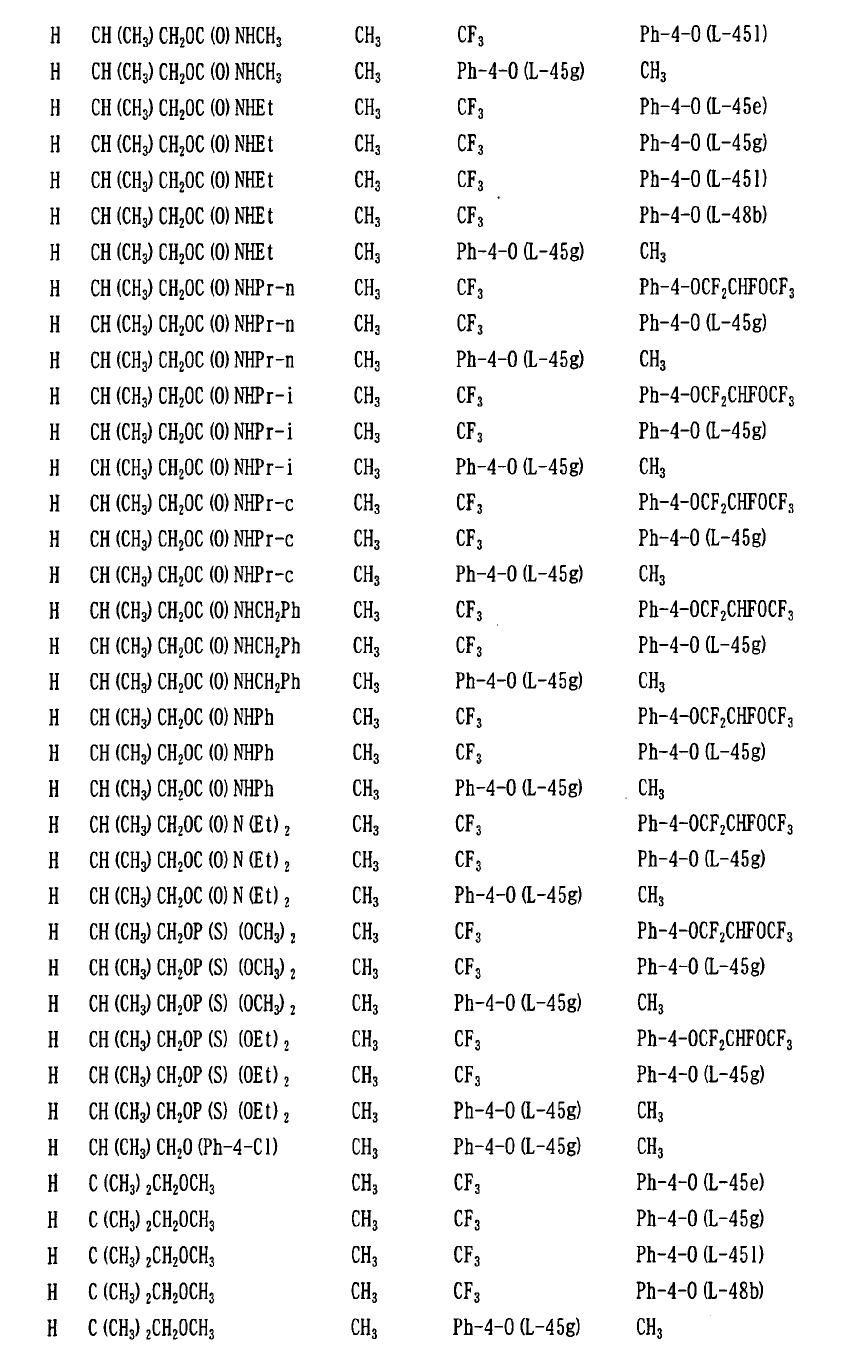

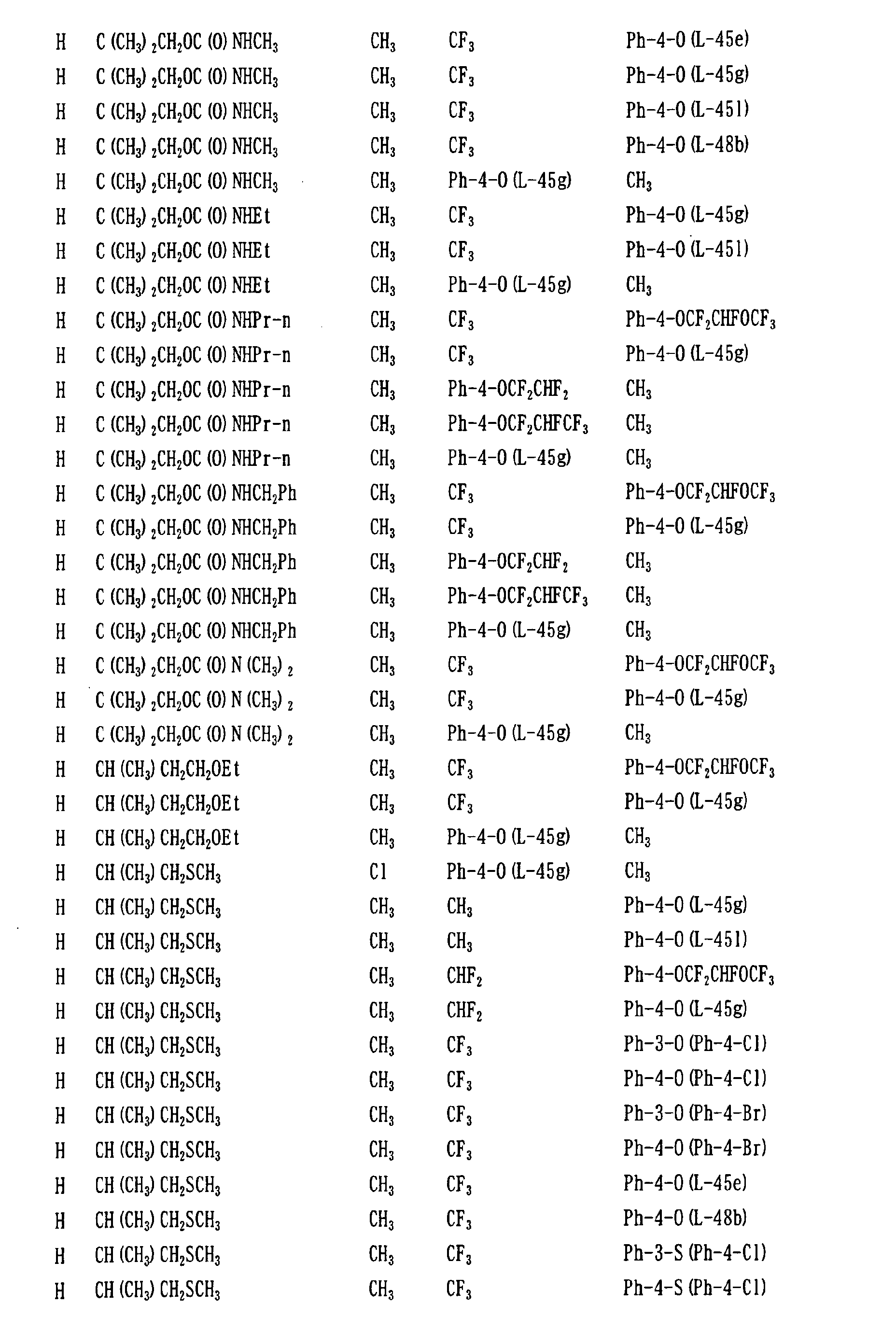

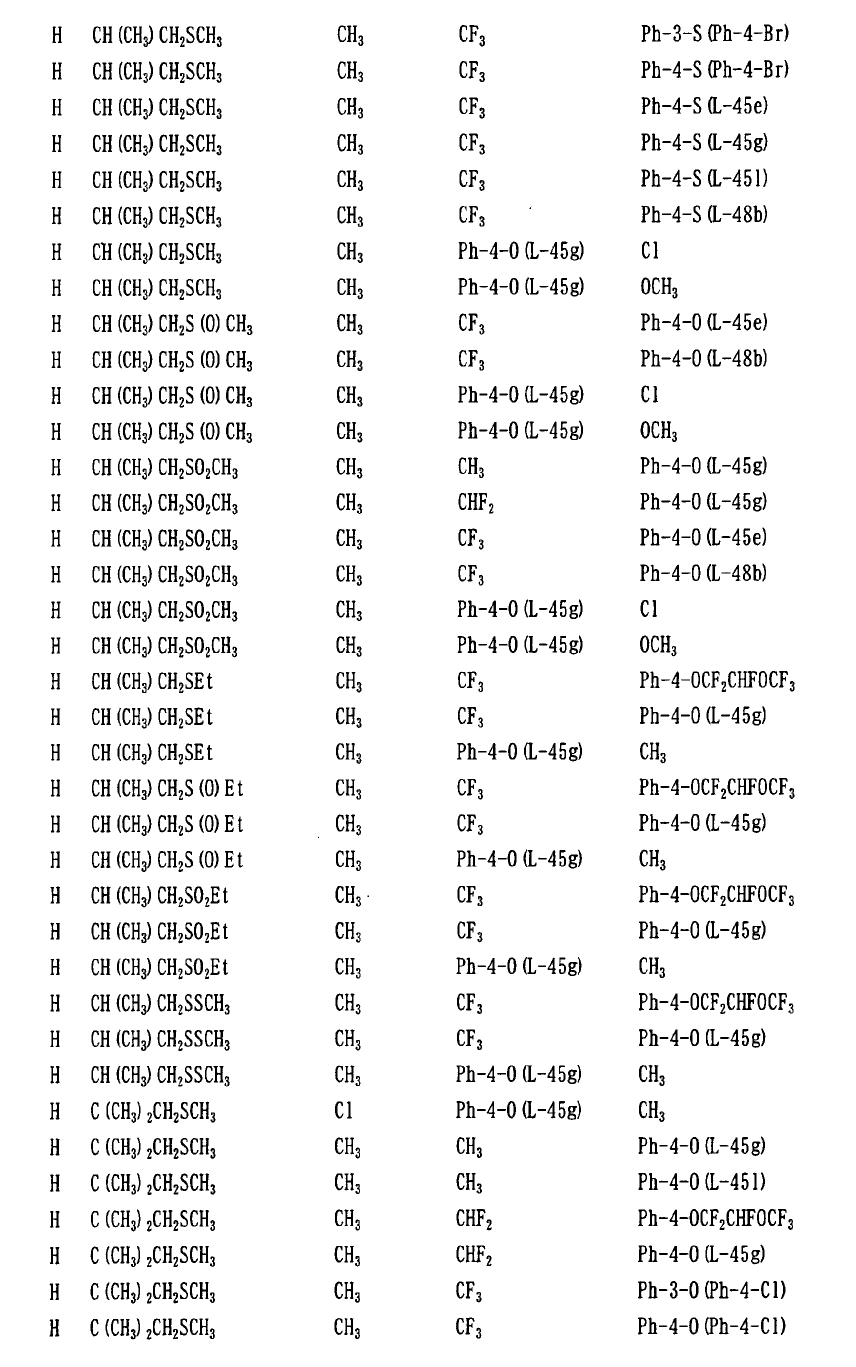

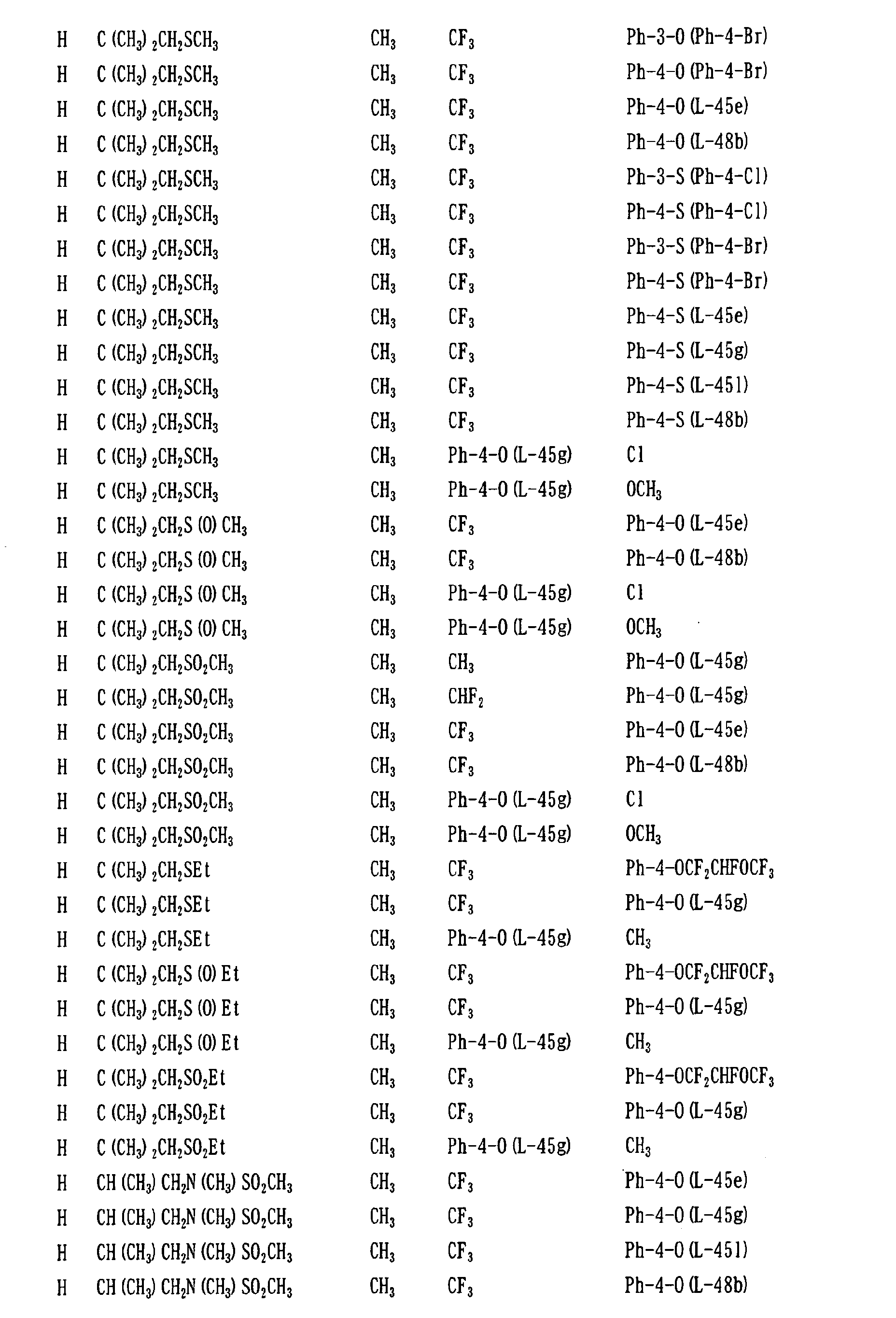

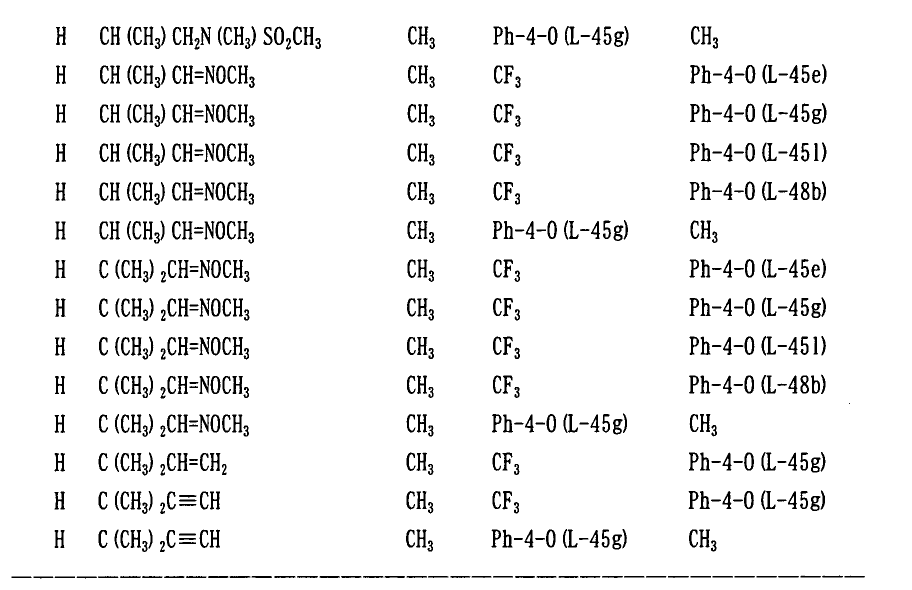

In the following Table 3, the number(s) showing the position(s) of the substituent(s) (X)ₘ and (Y)ₙ correspond to the number(s) shown in the following structural formulae, and the symbol of - means unsubstituted.

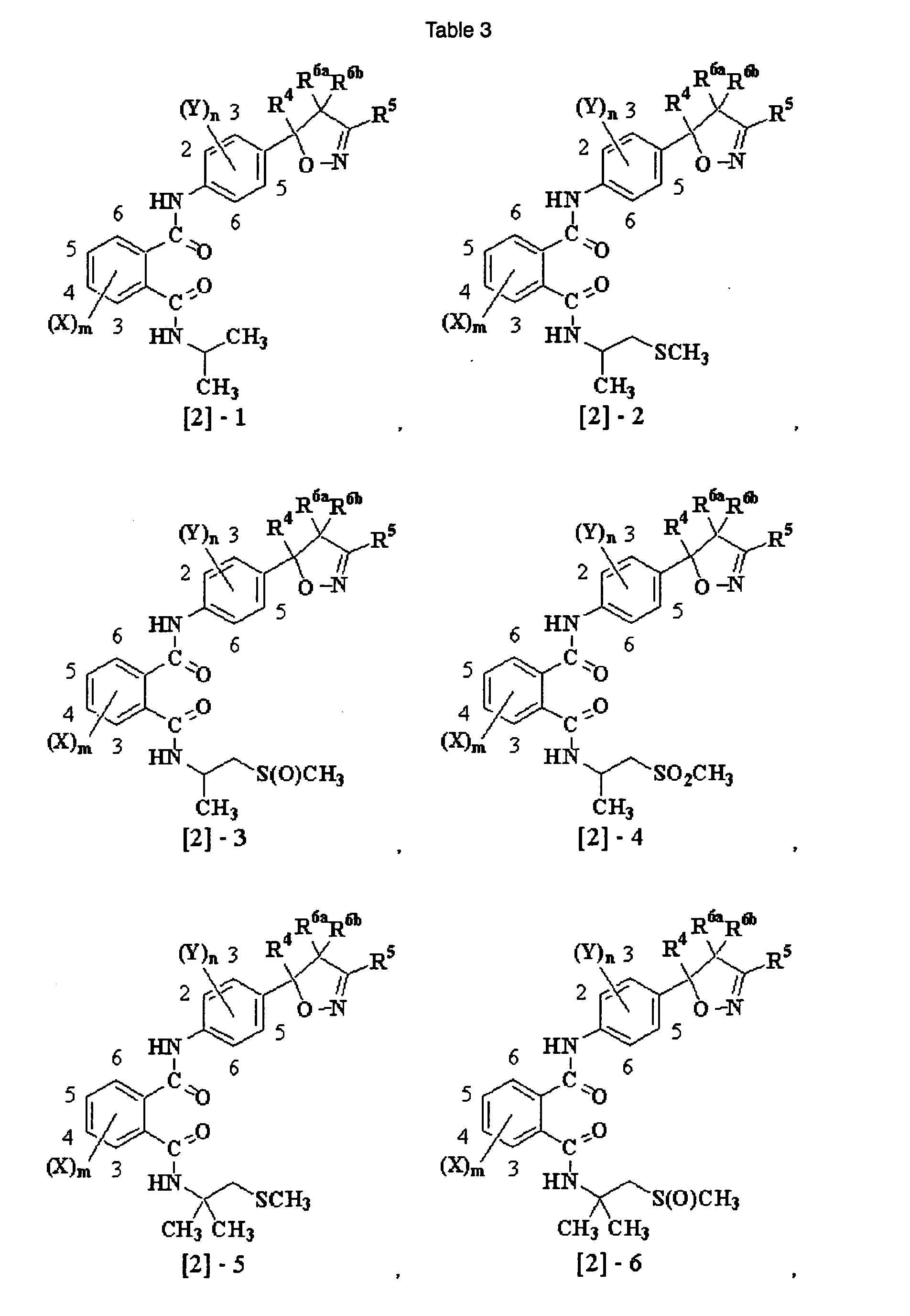

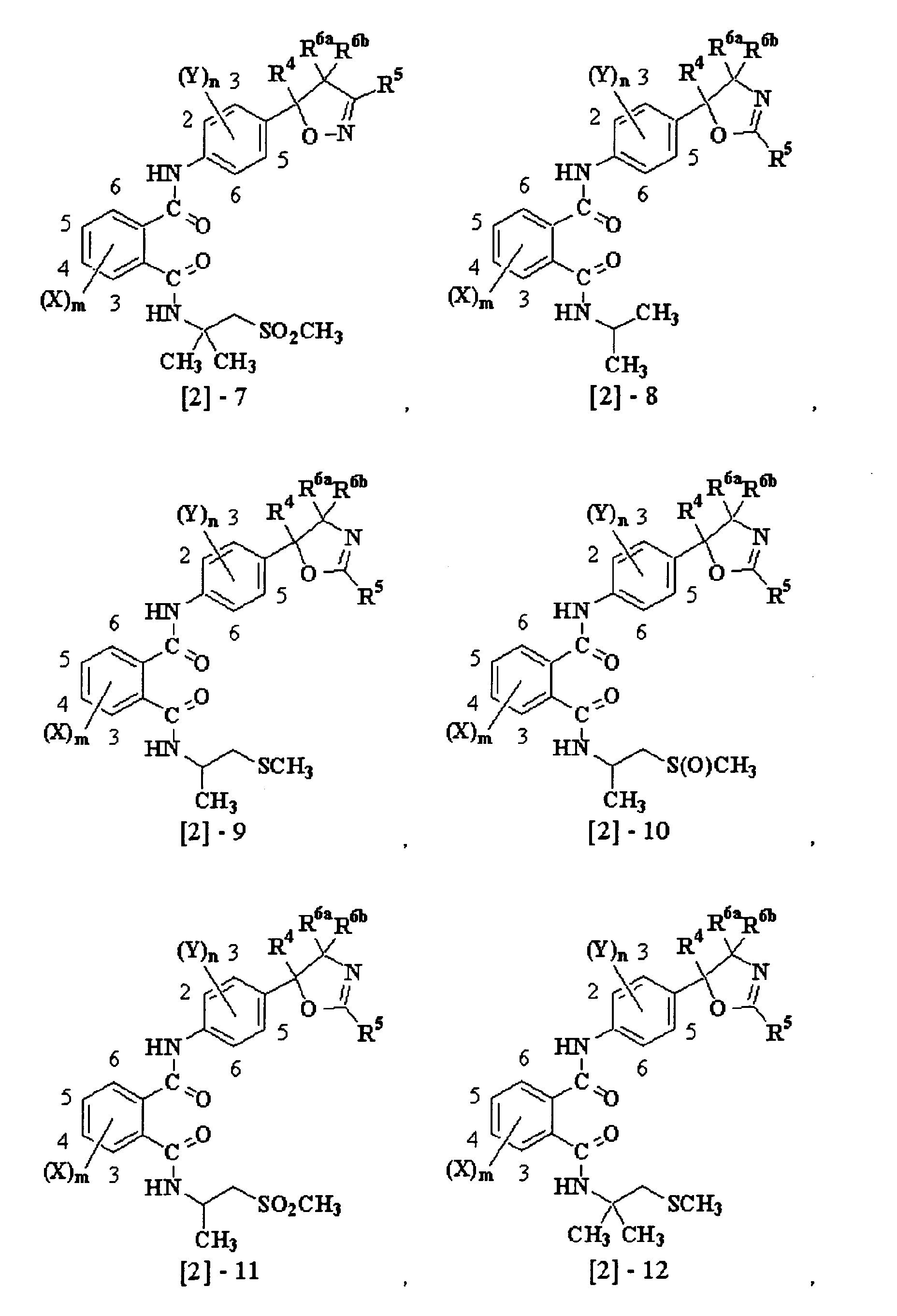

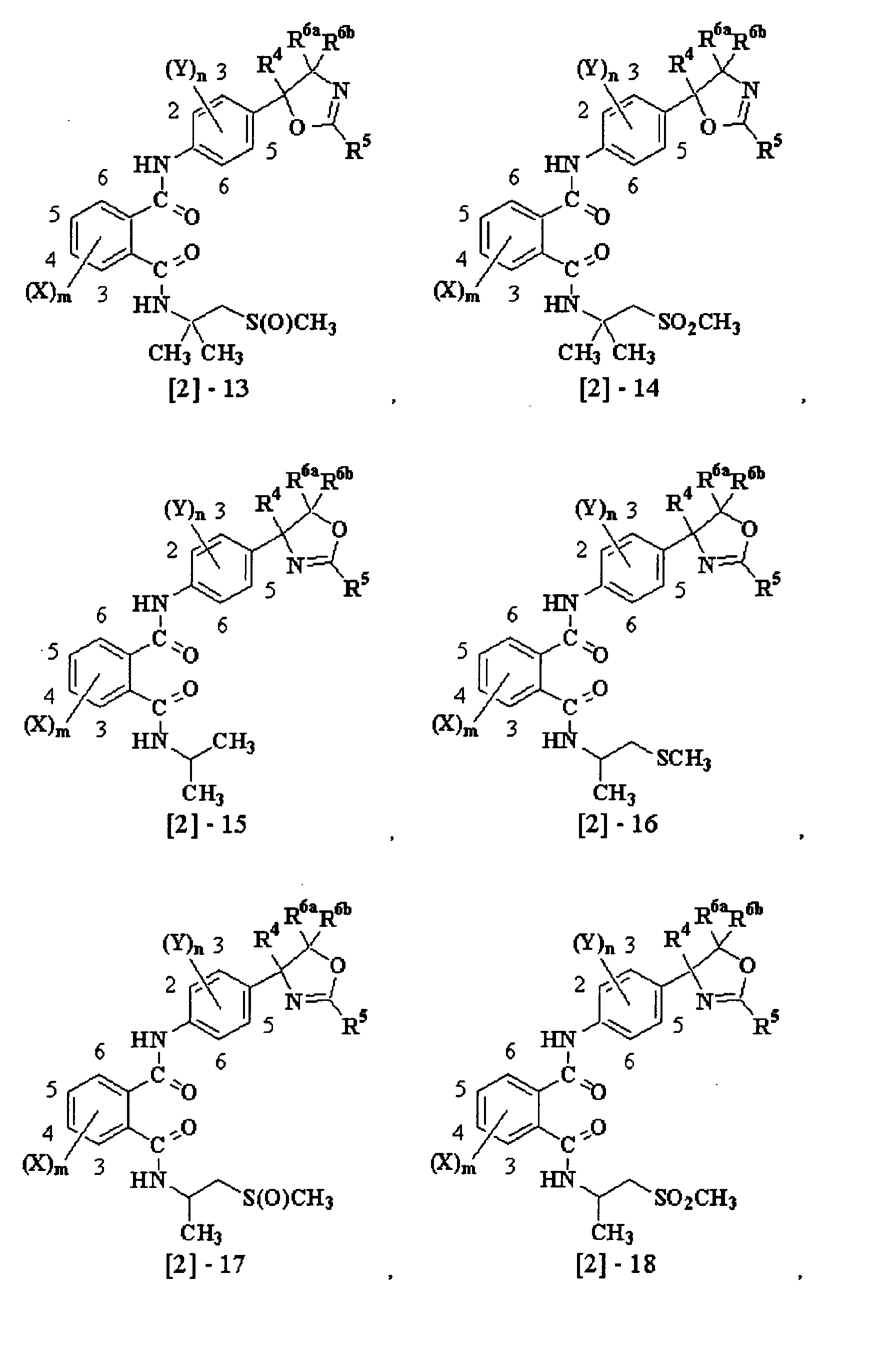

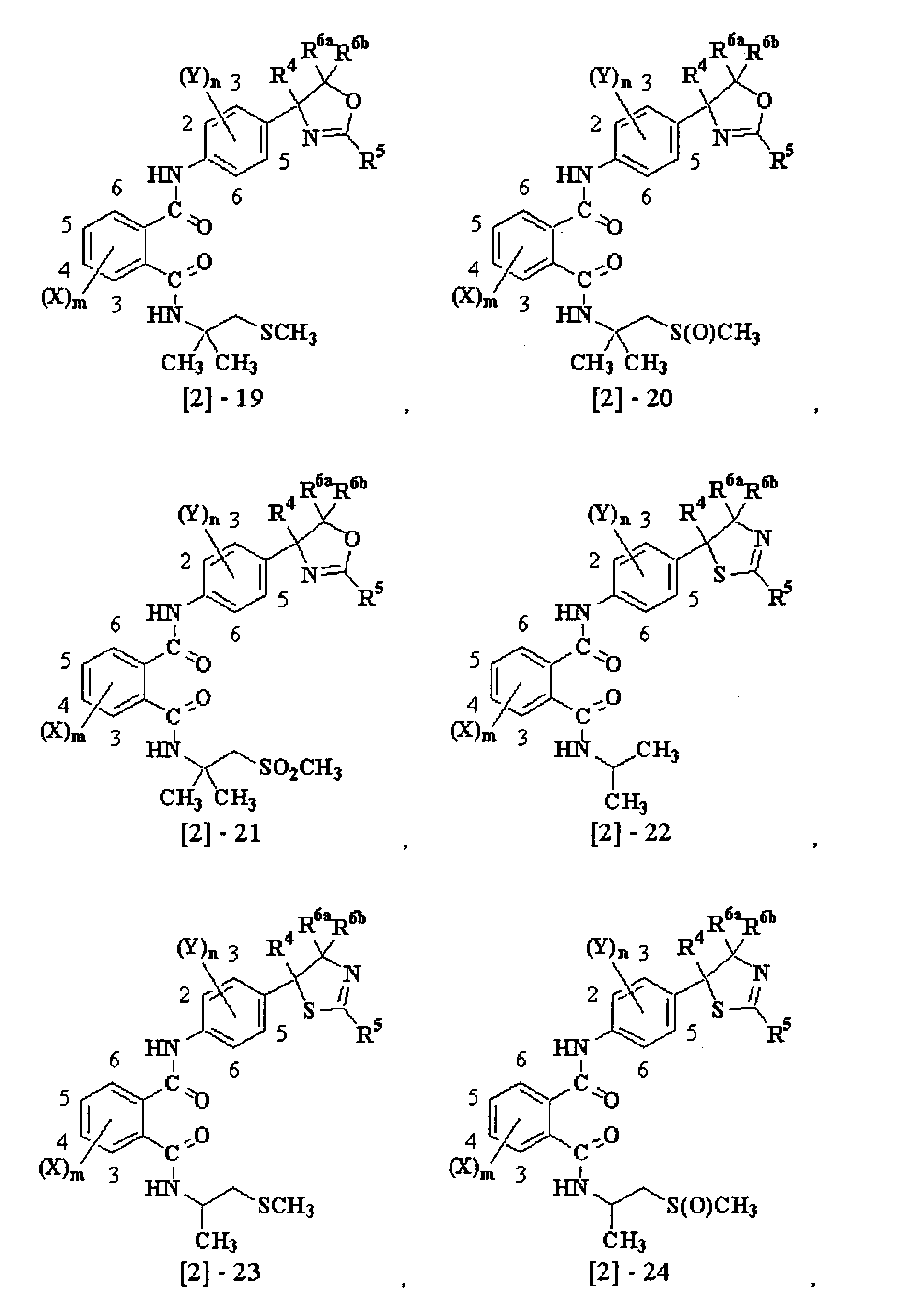

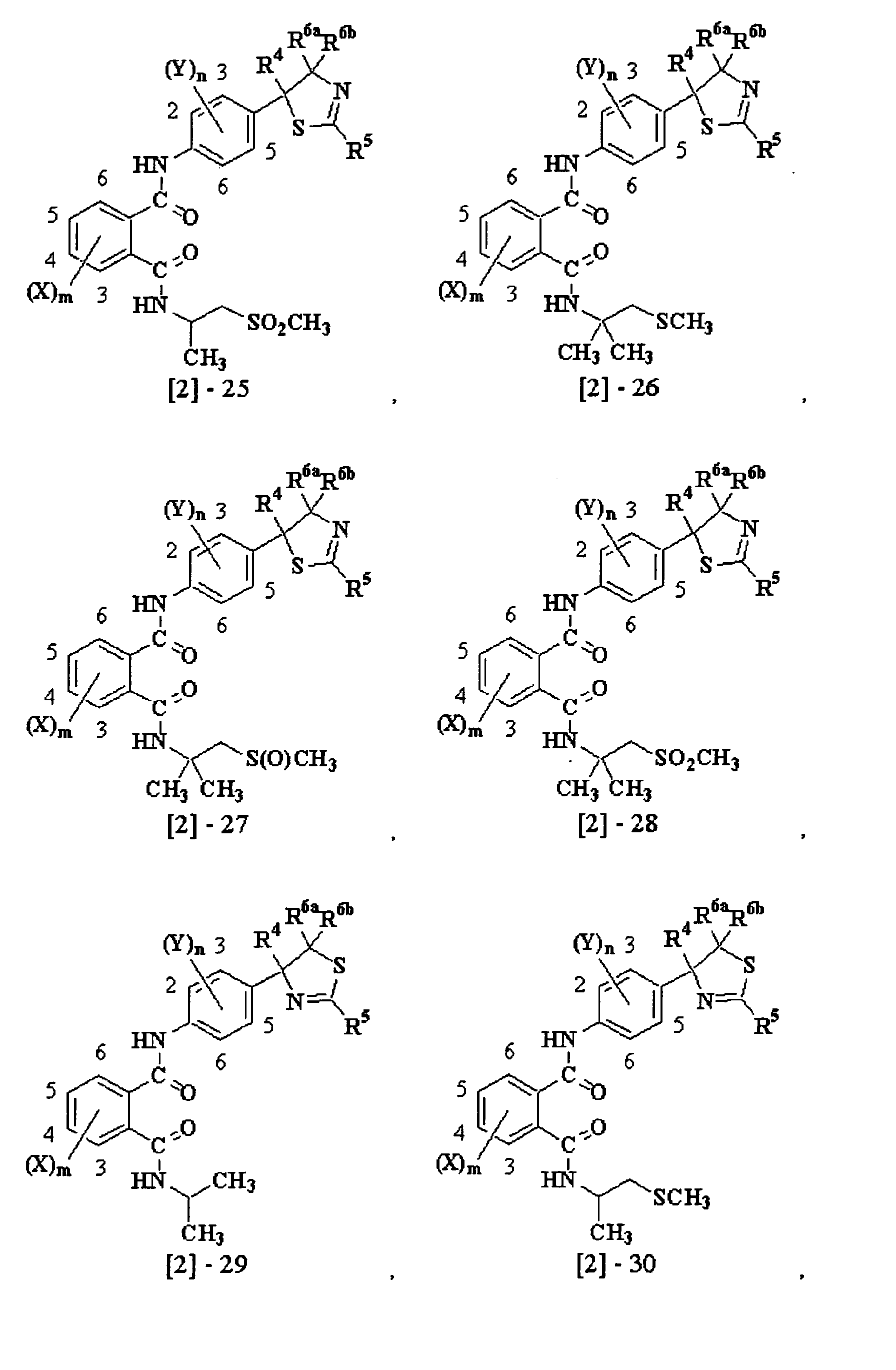

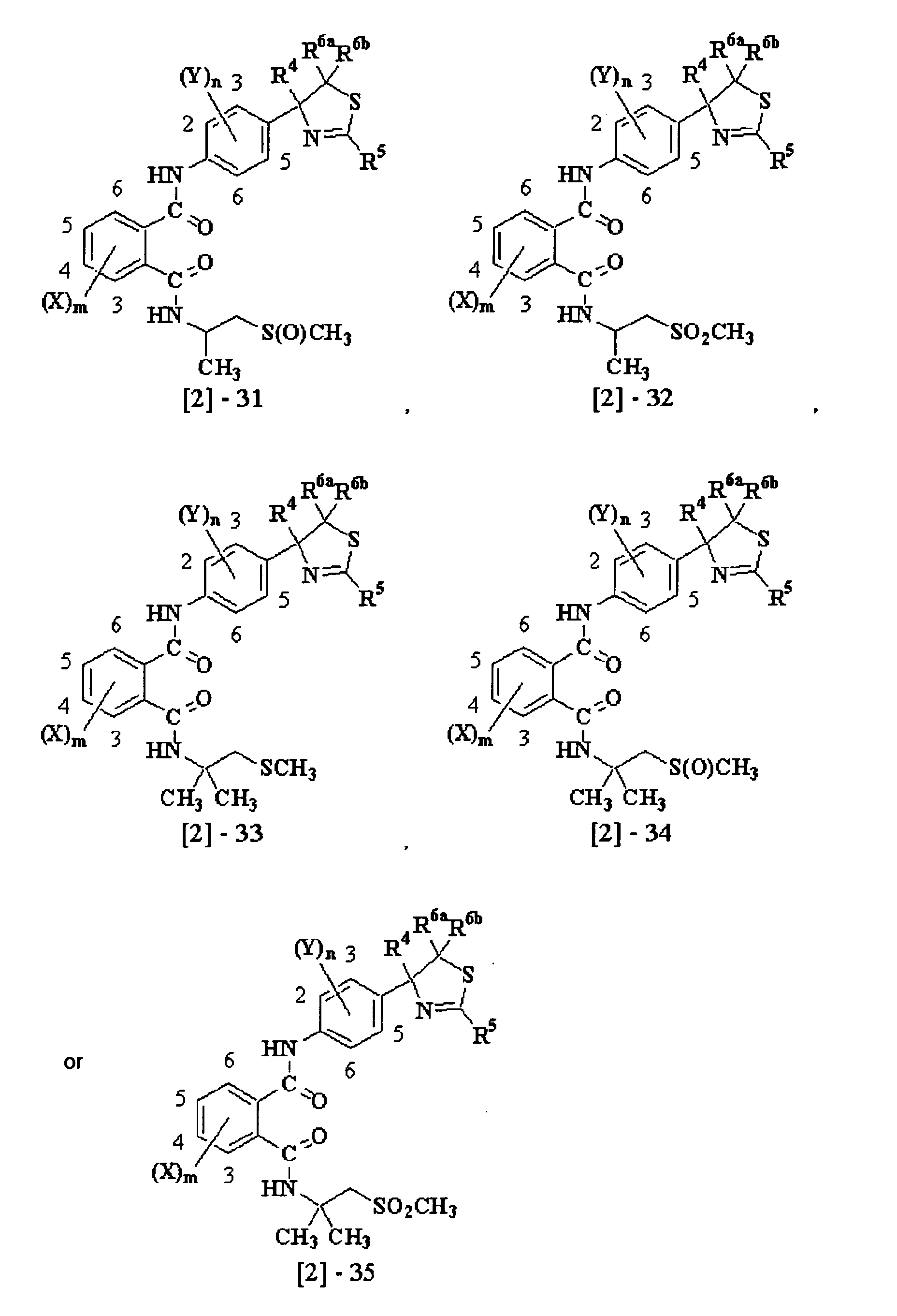

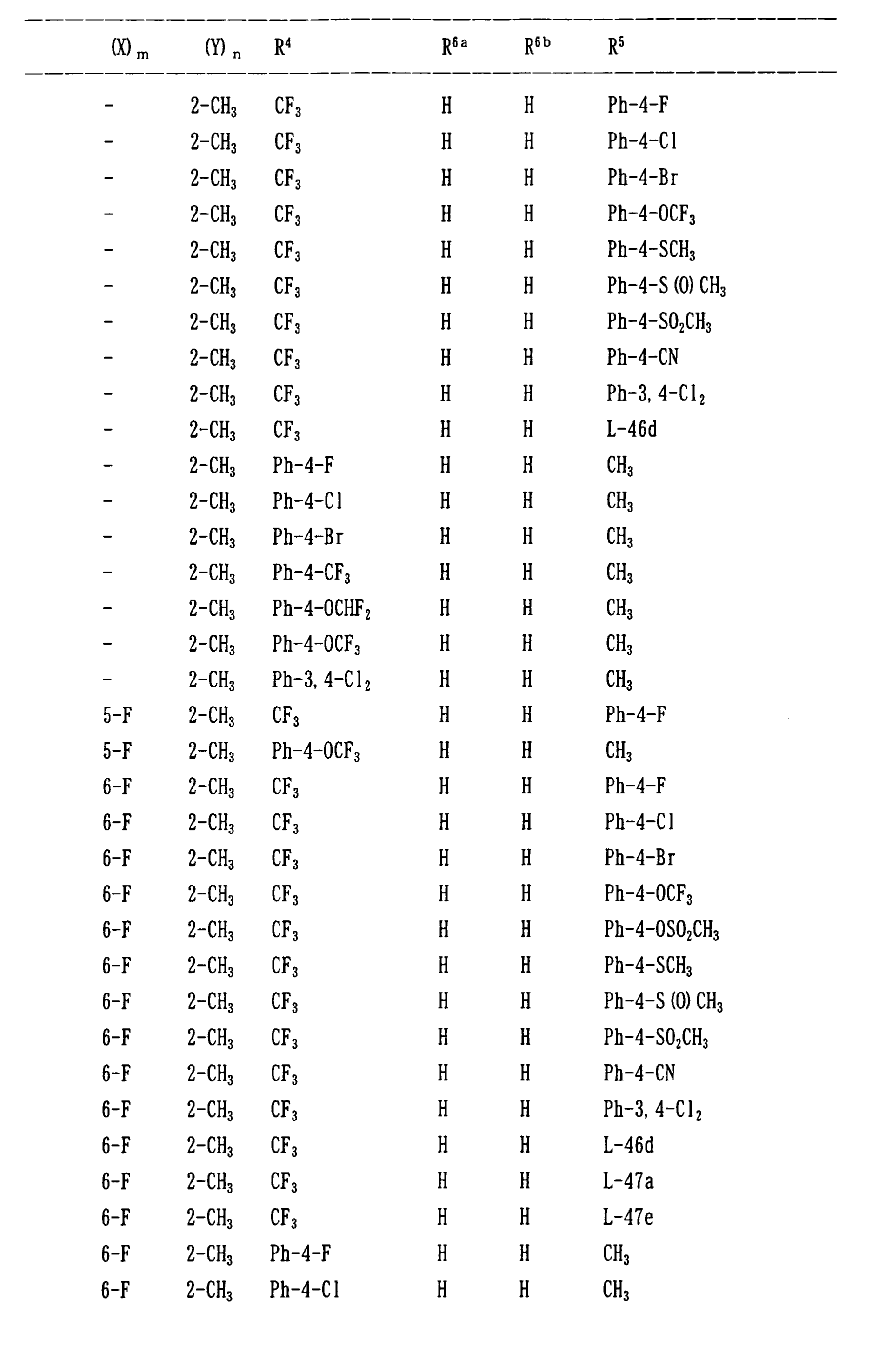

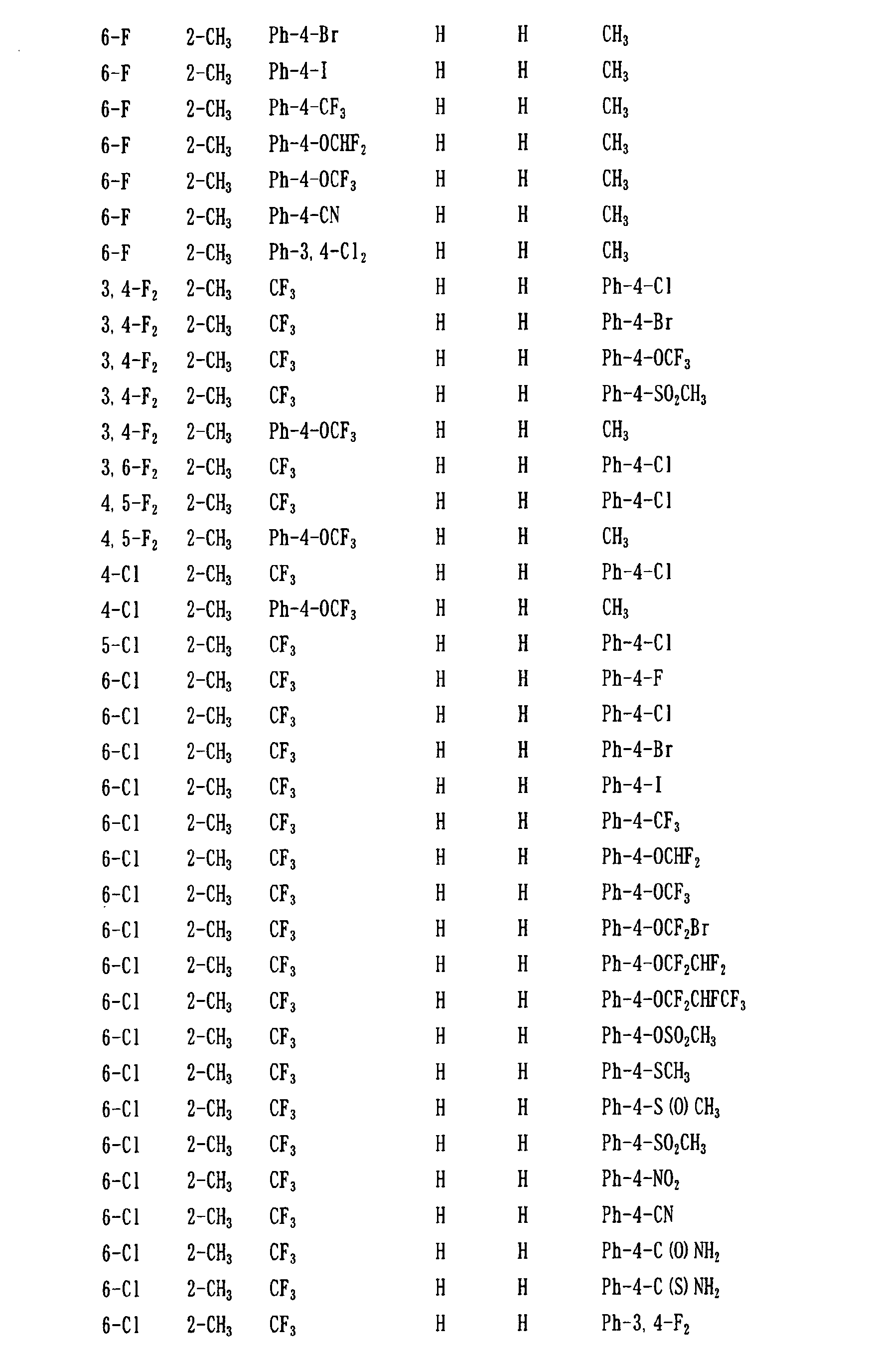

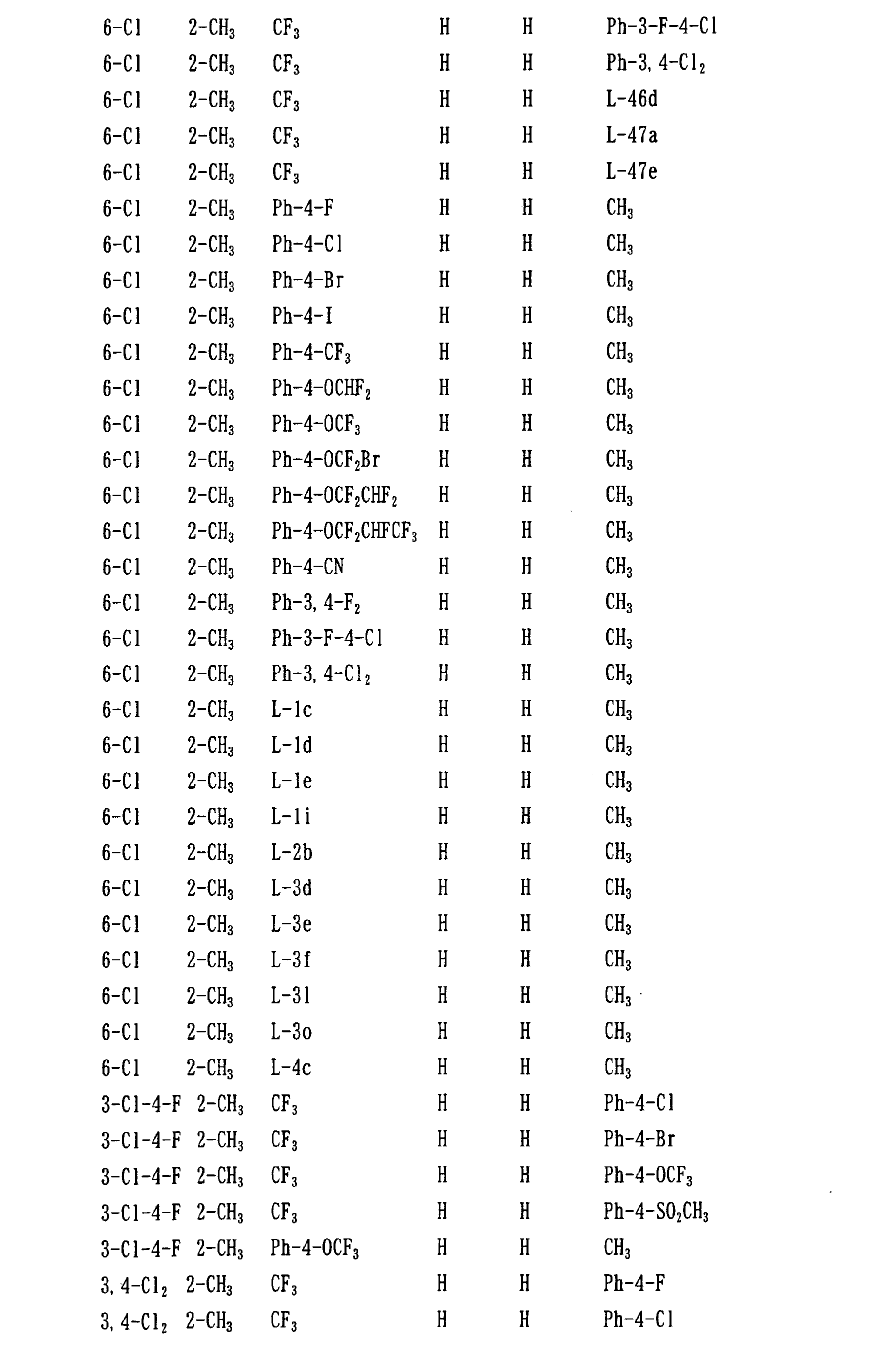

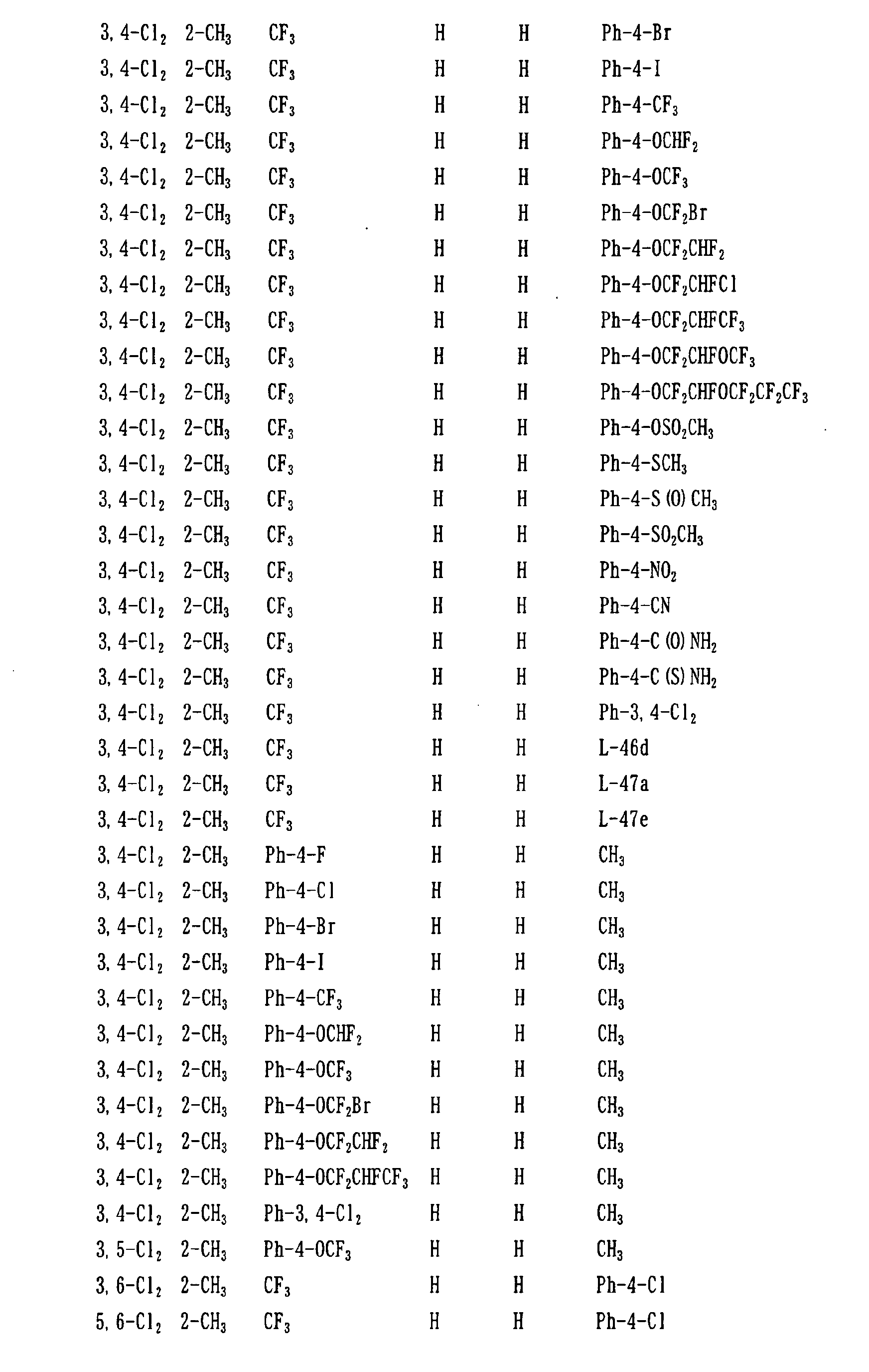

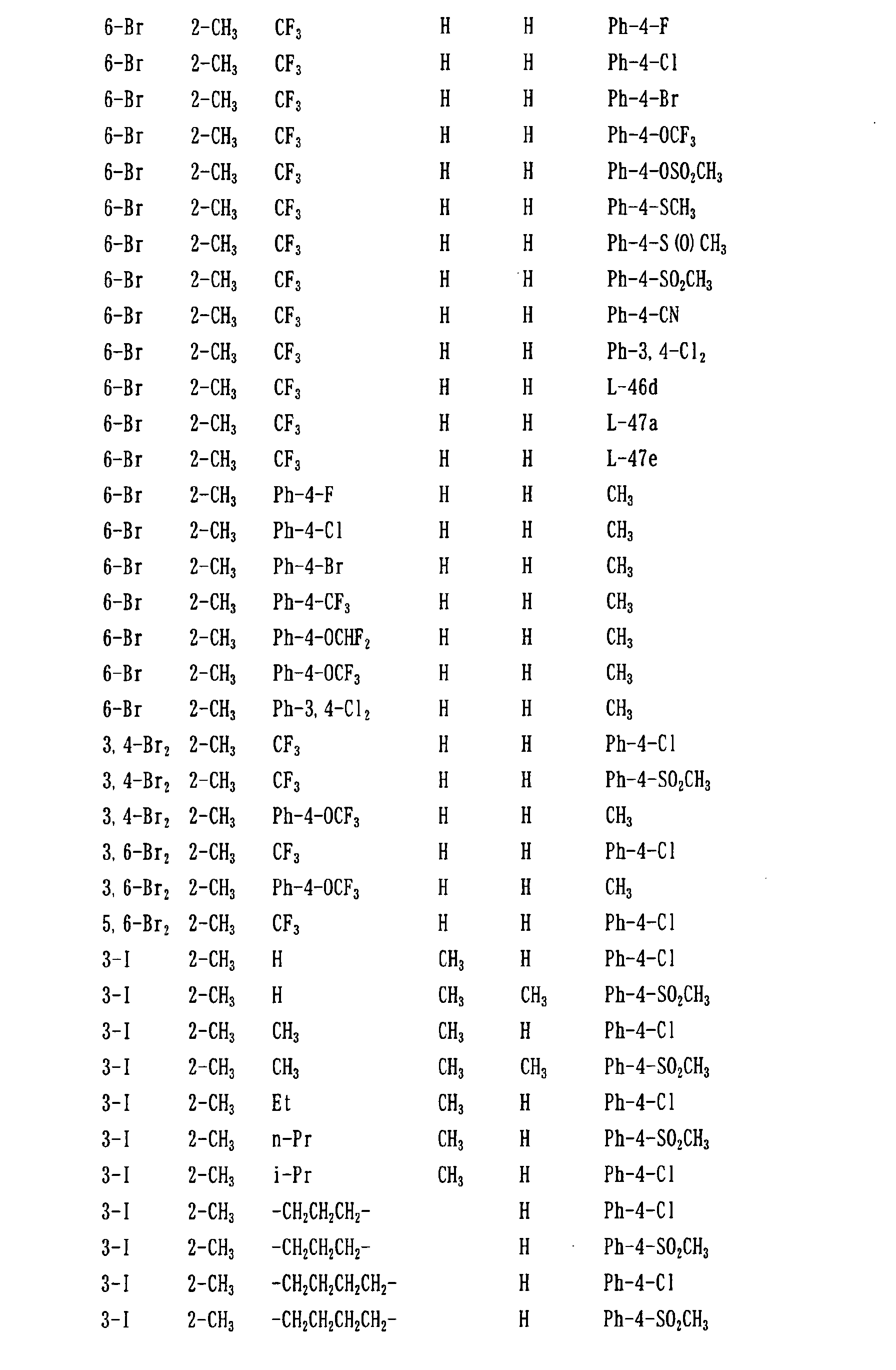

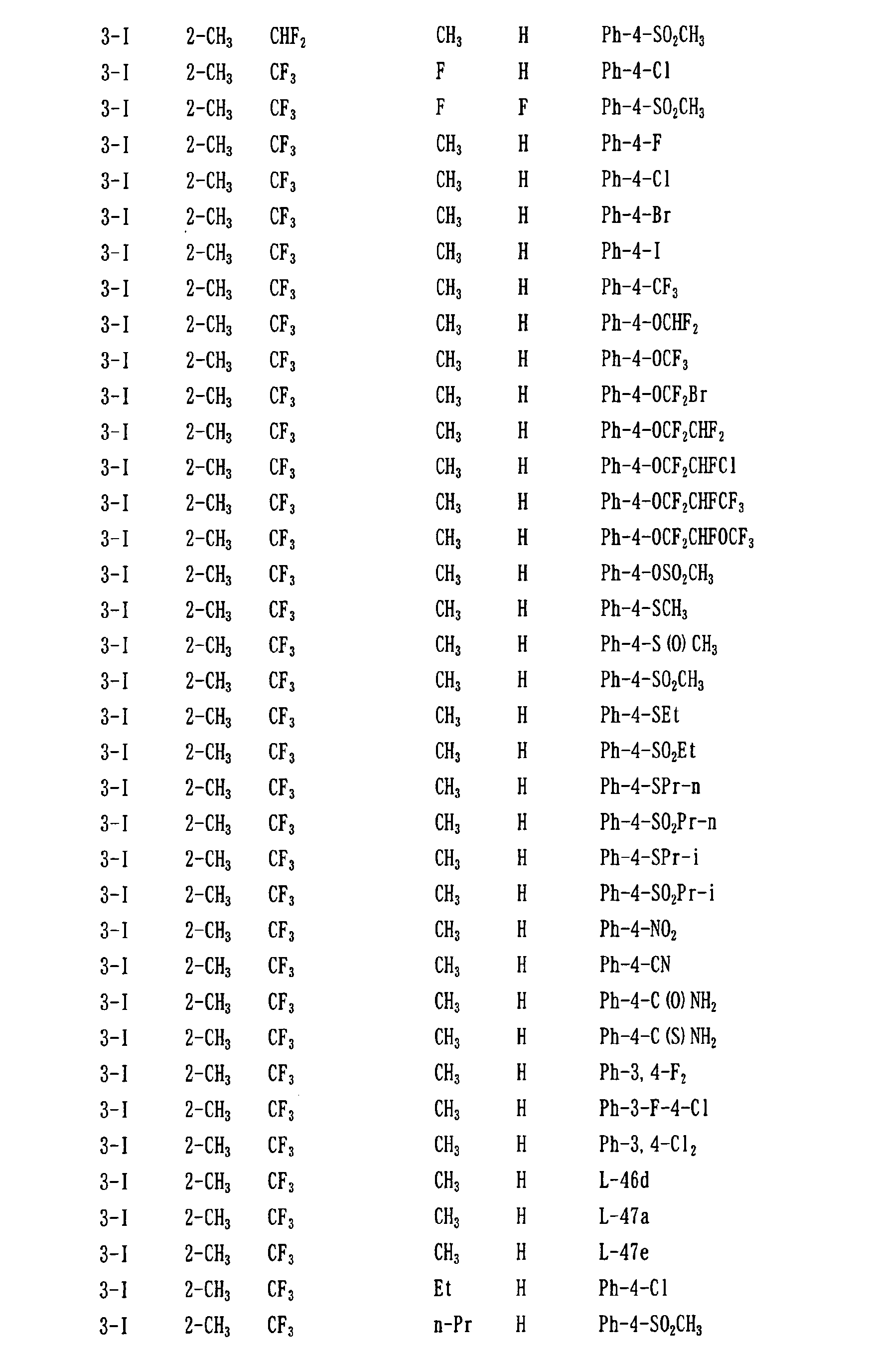

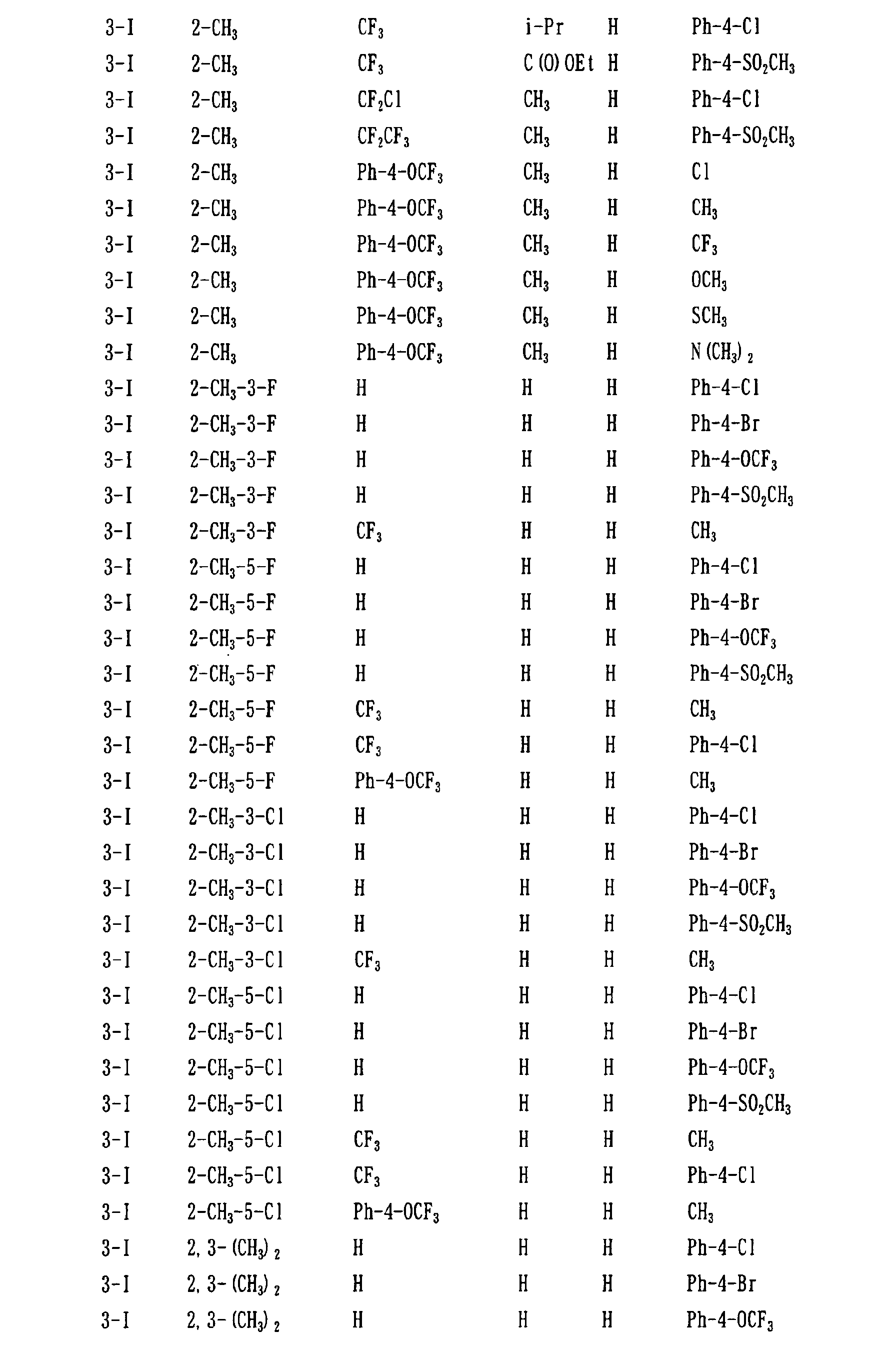

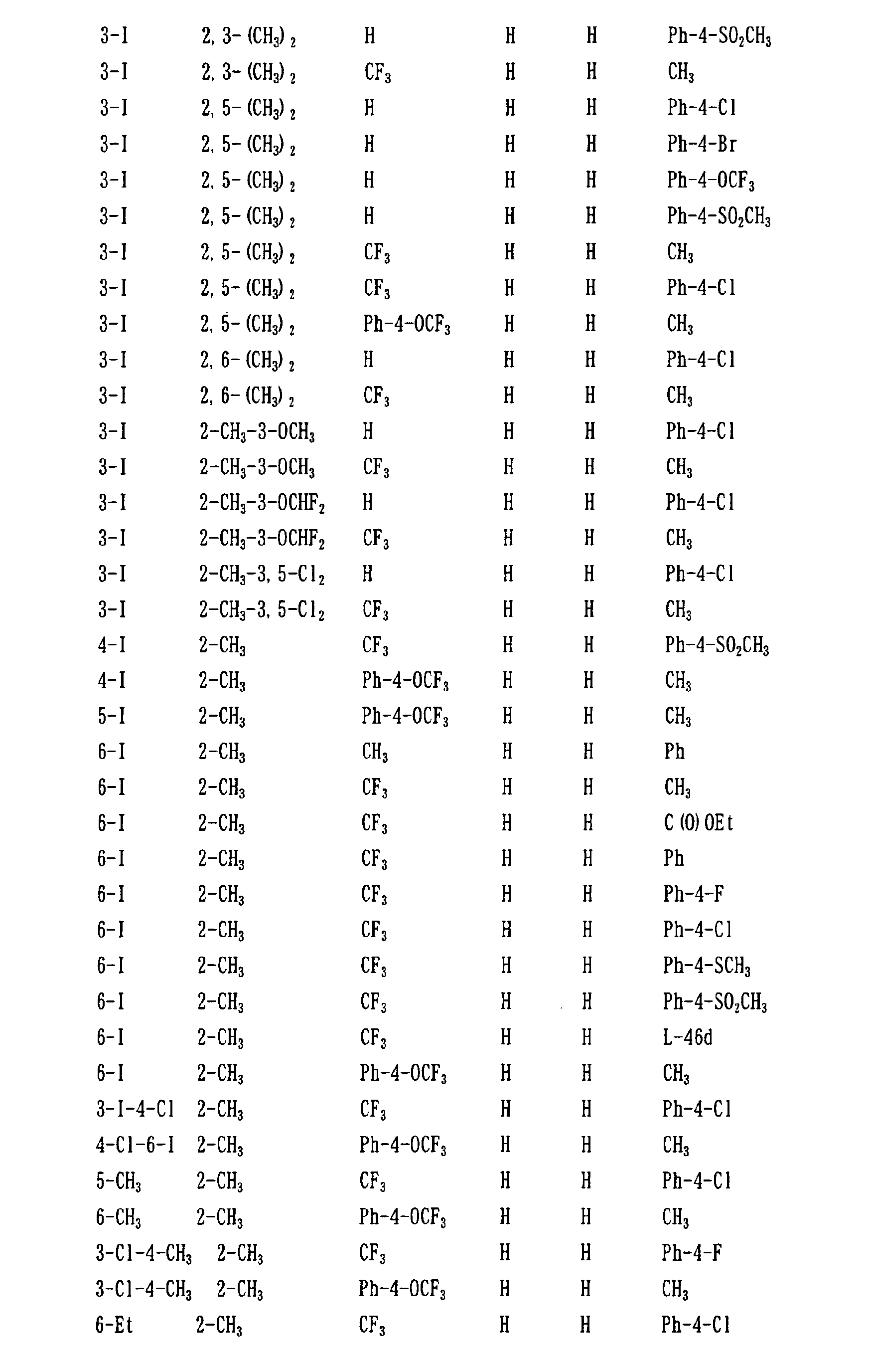

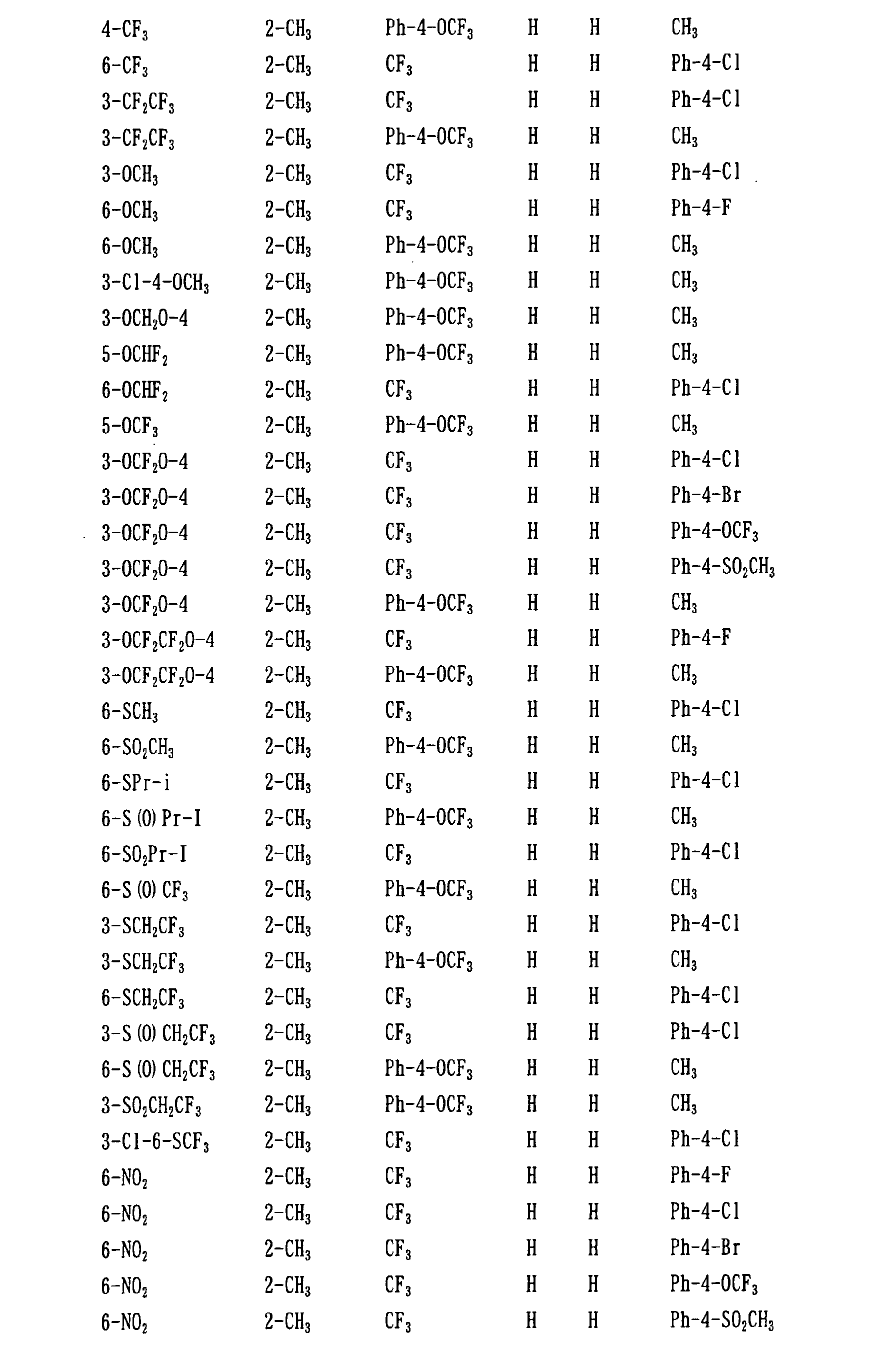

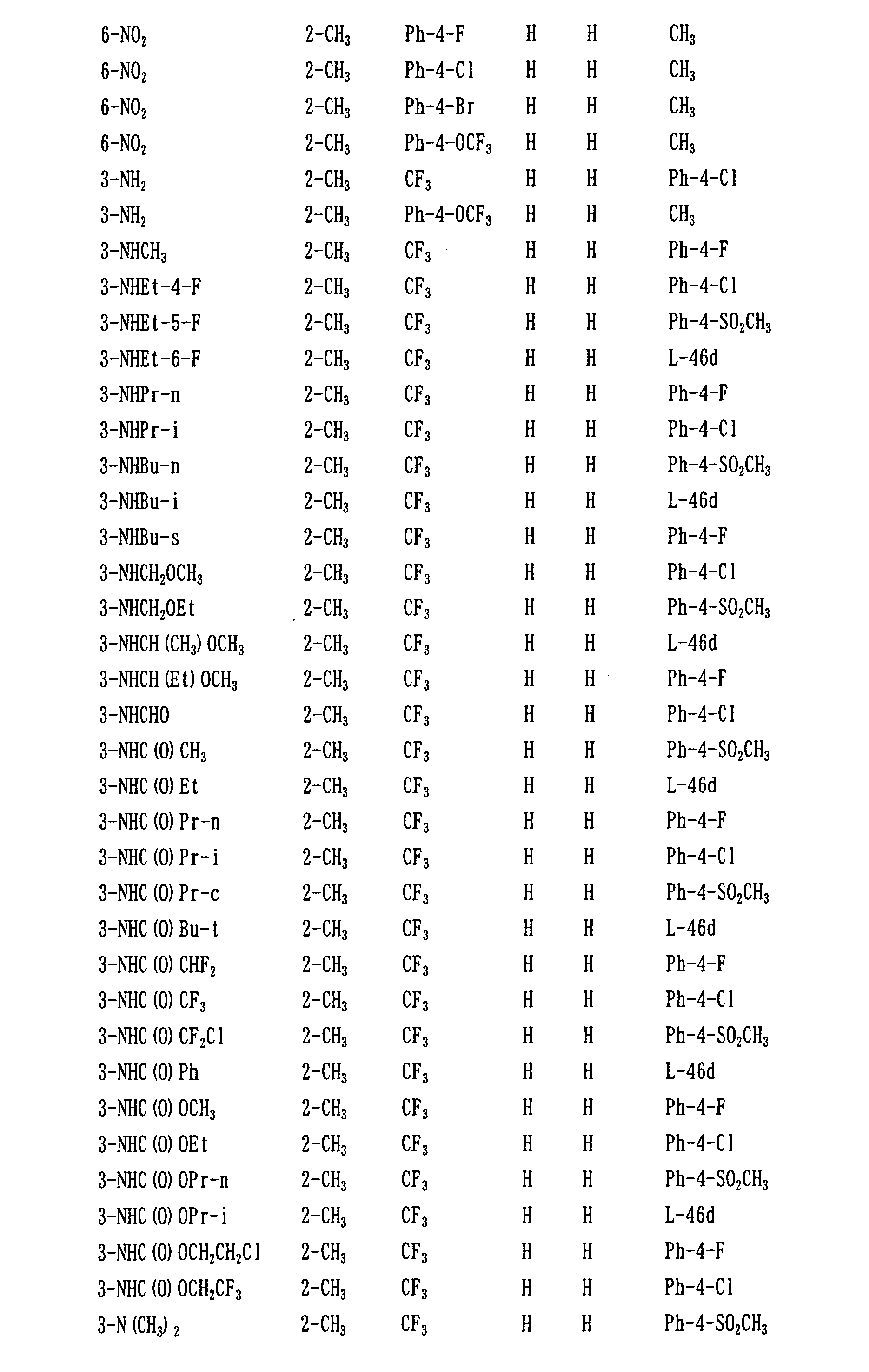

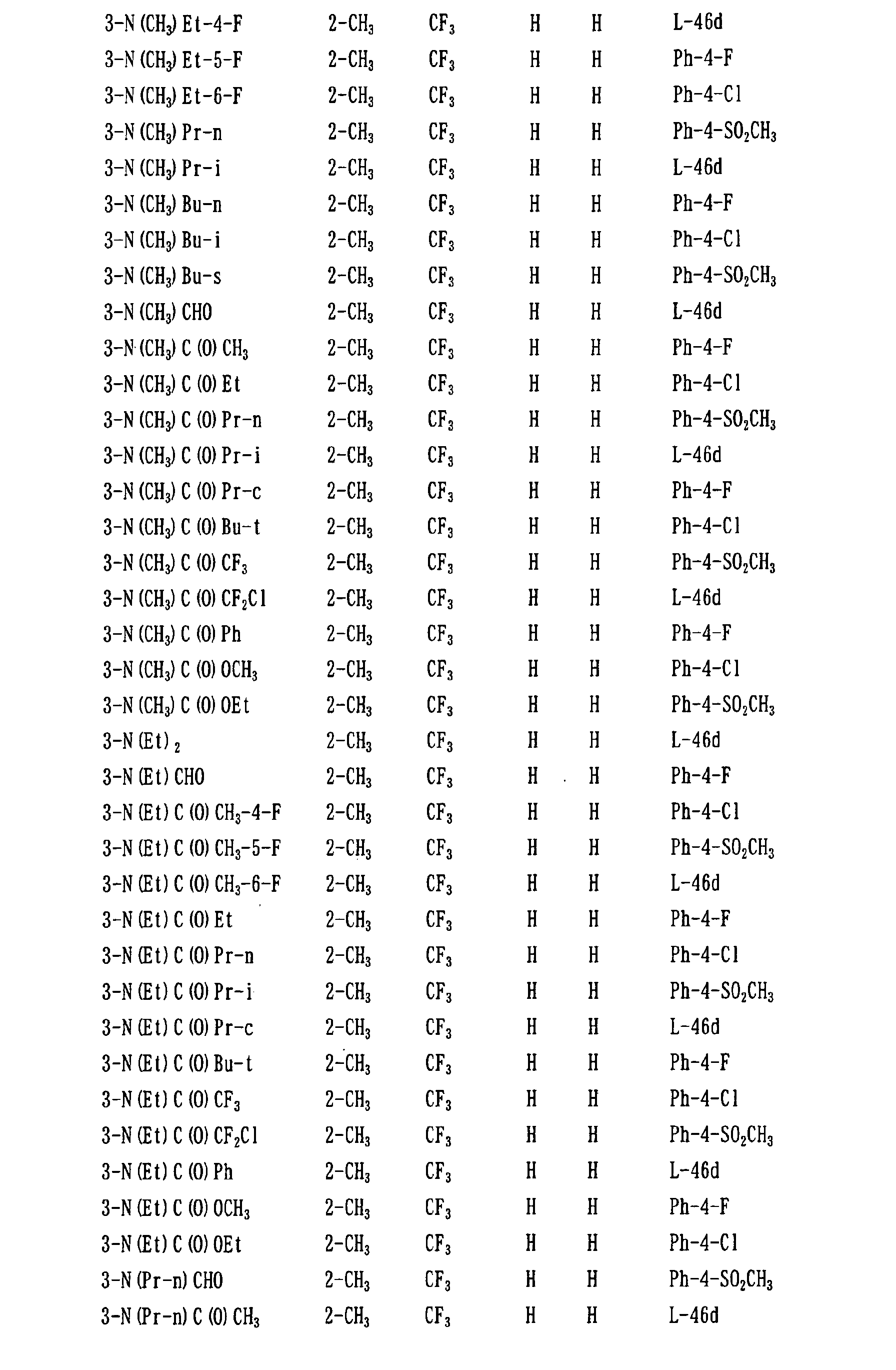

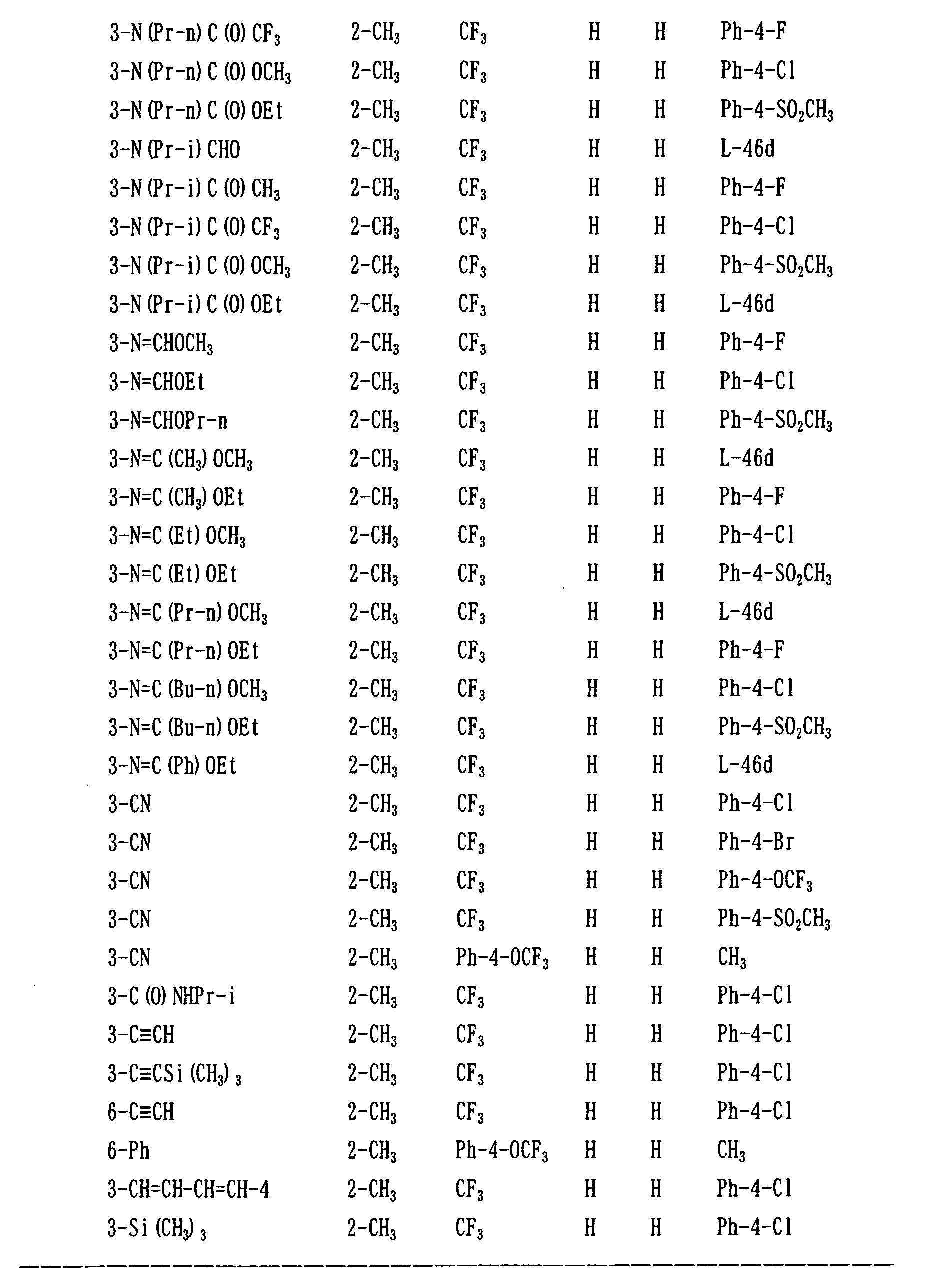

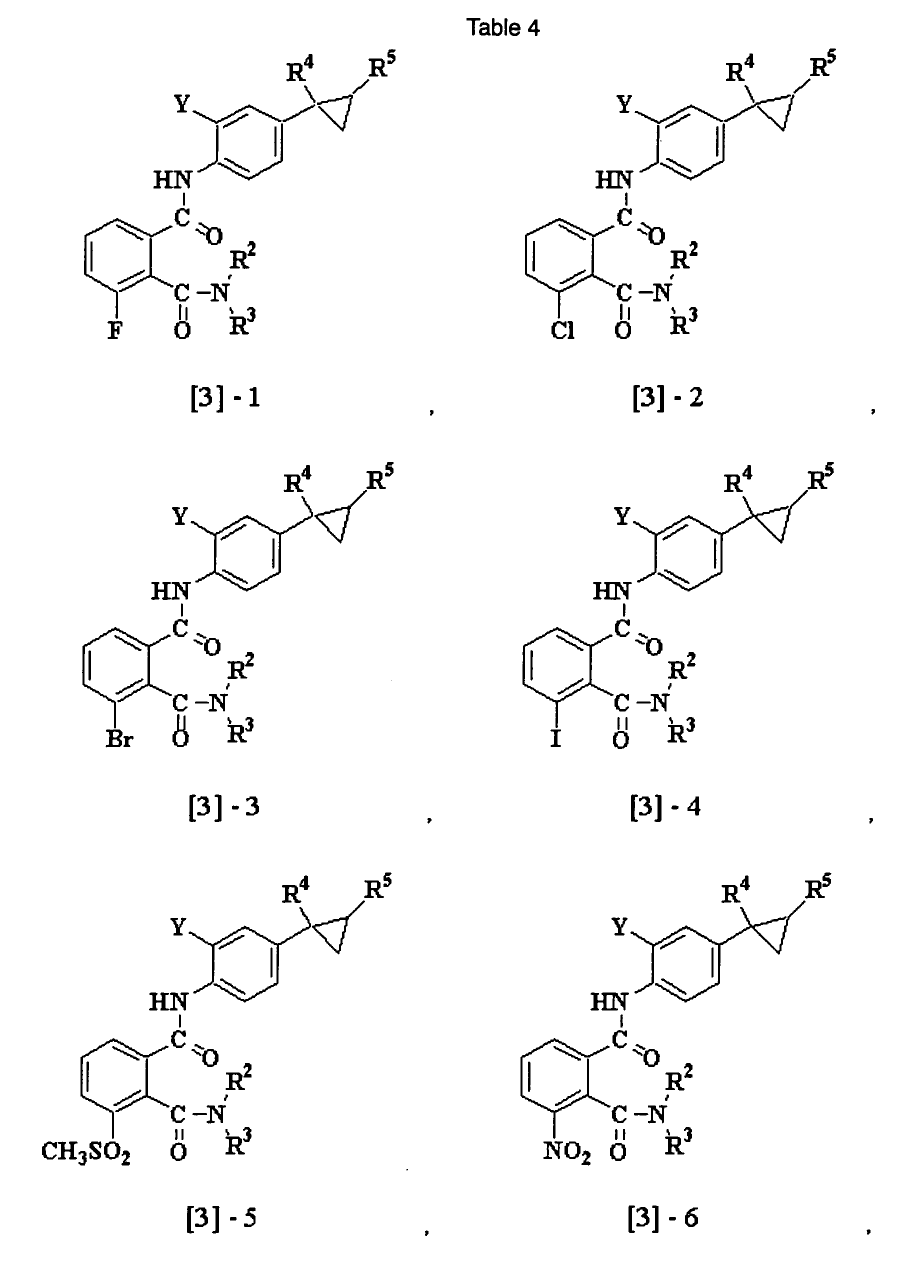

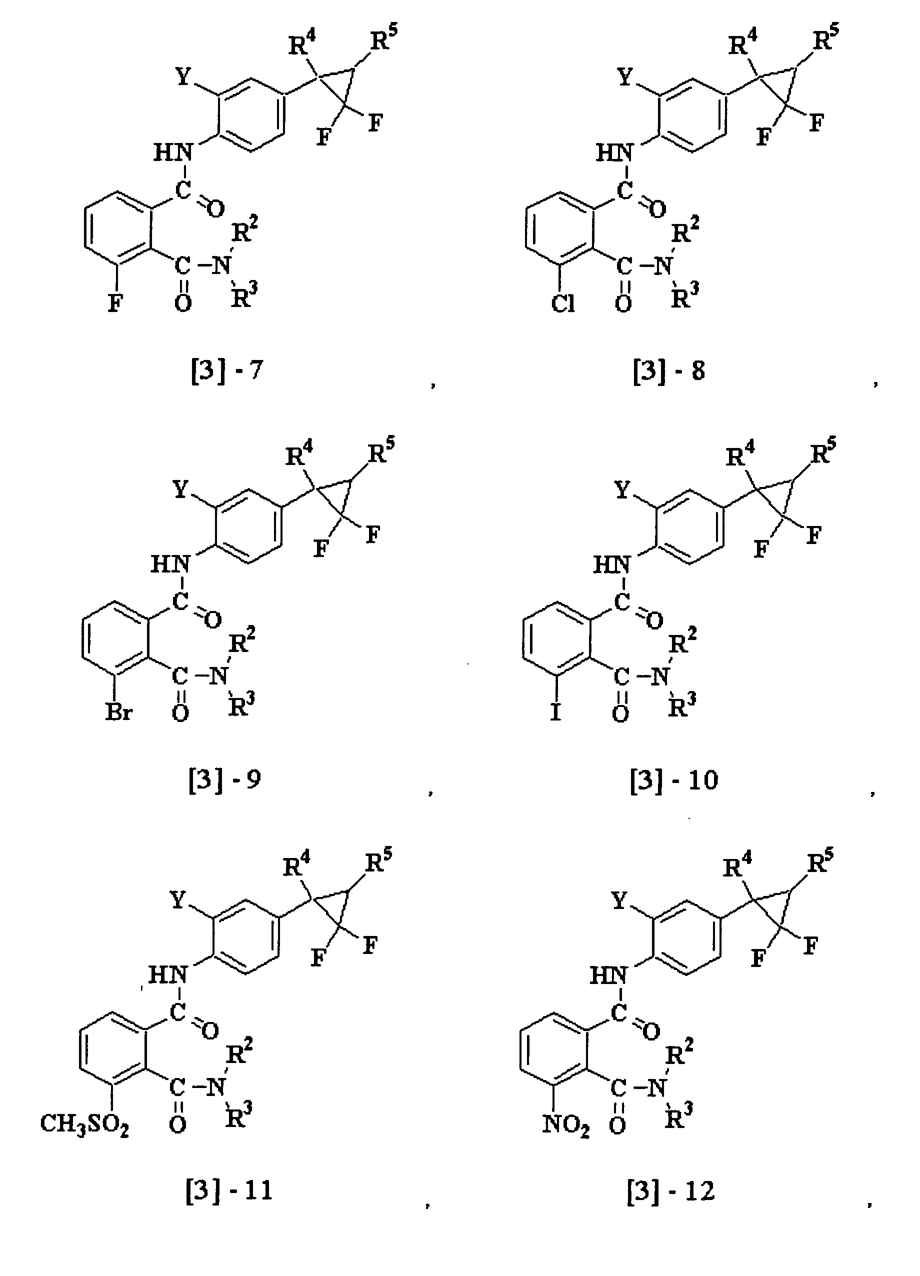

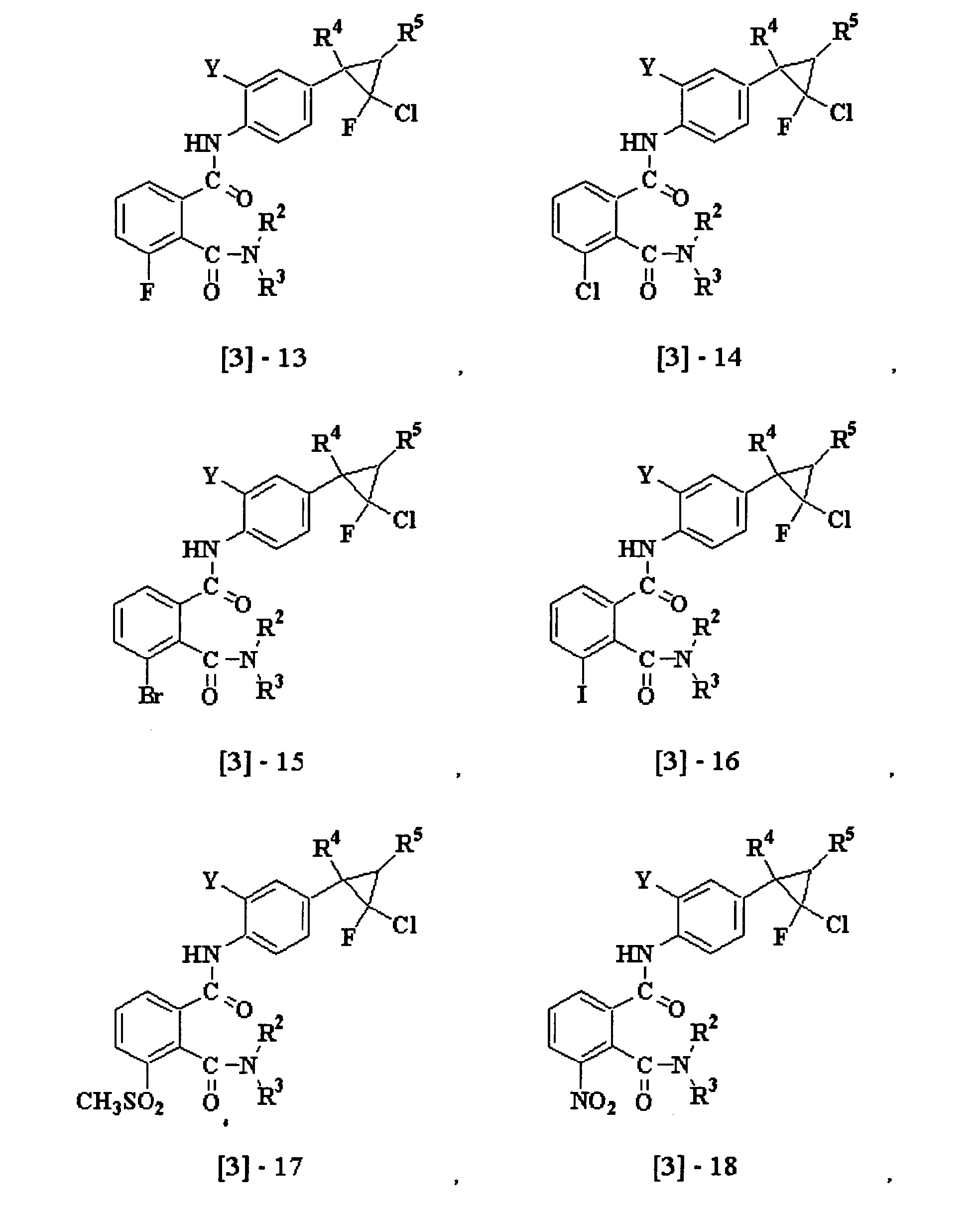

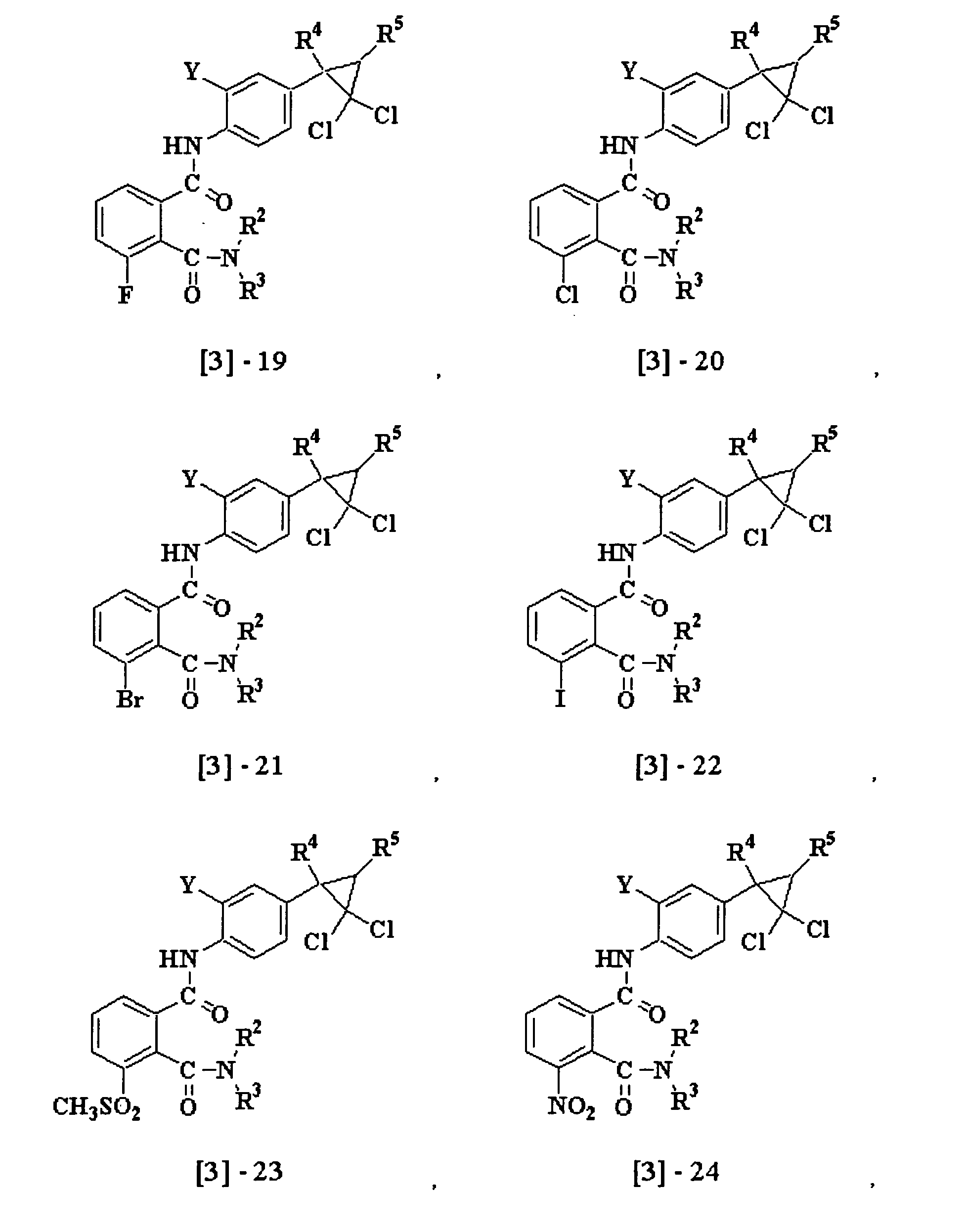

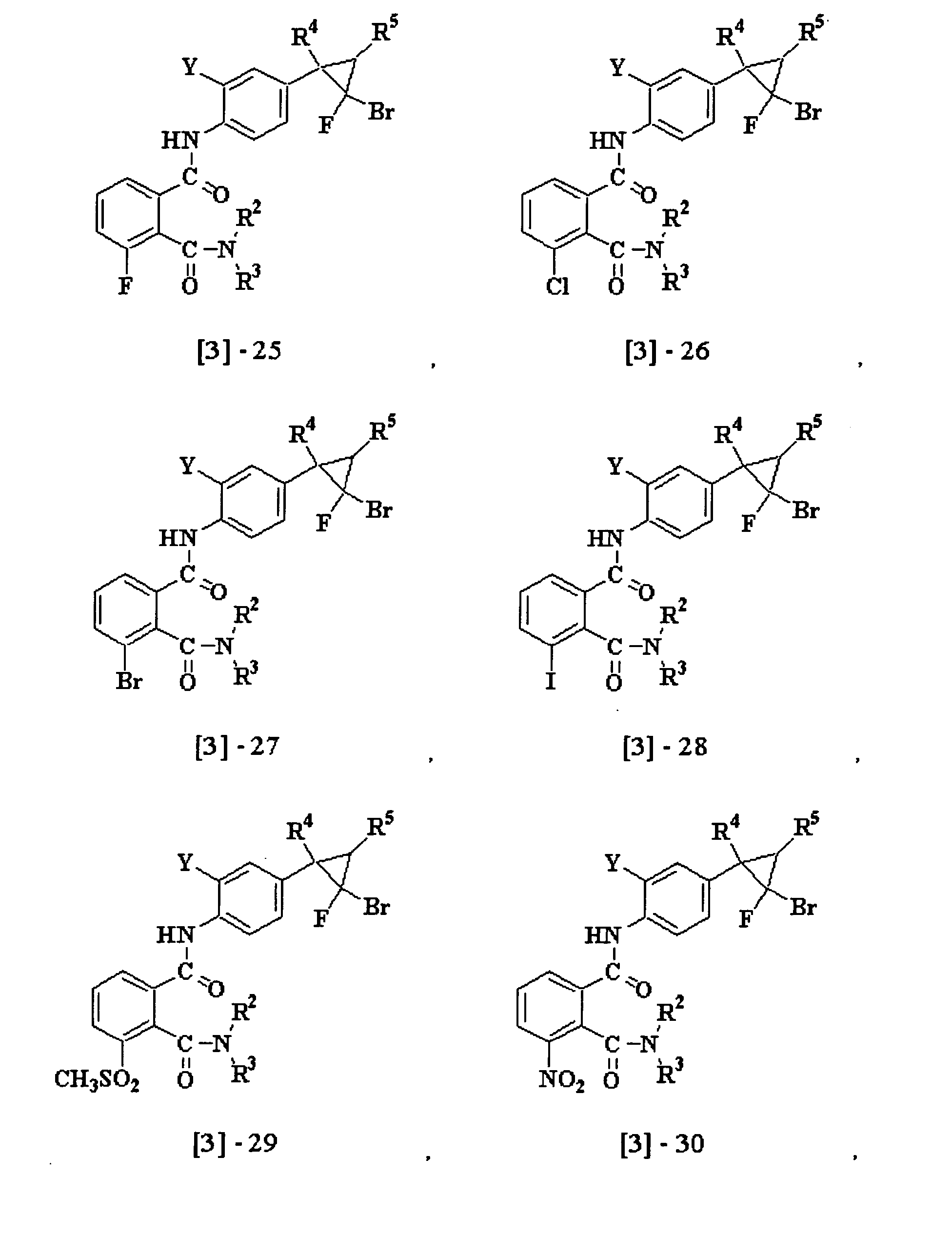

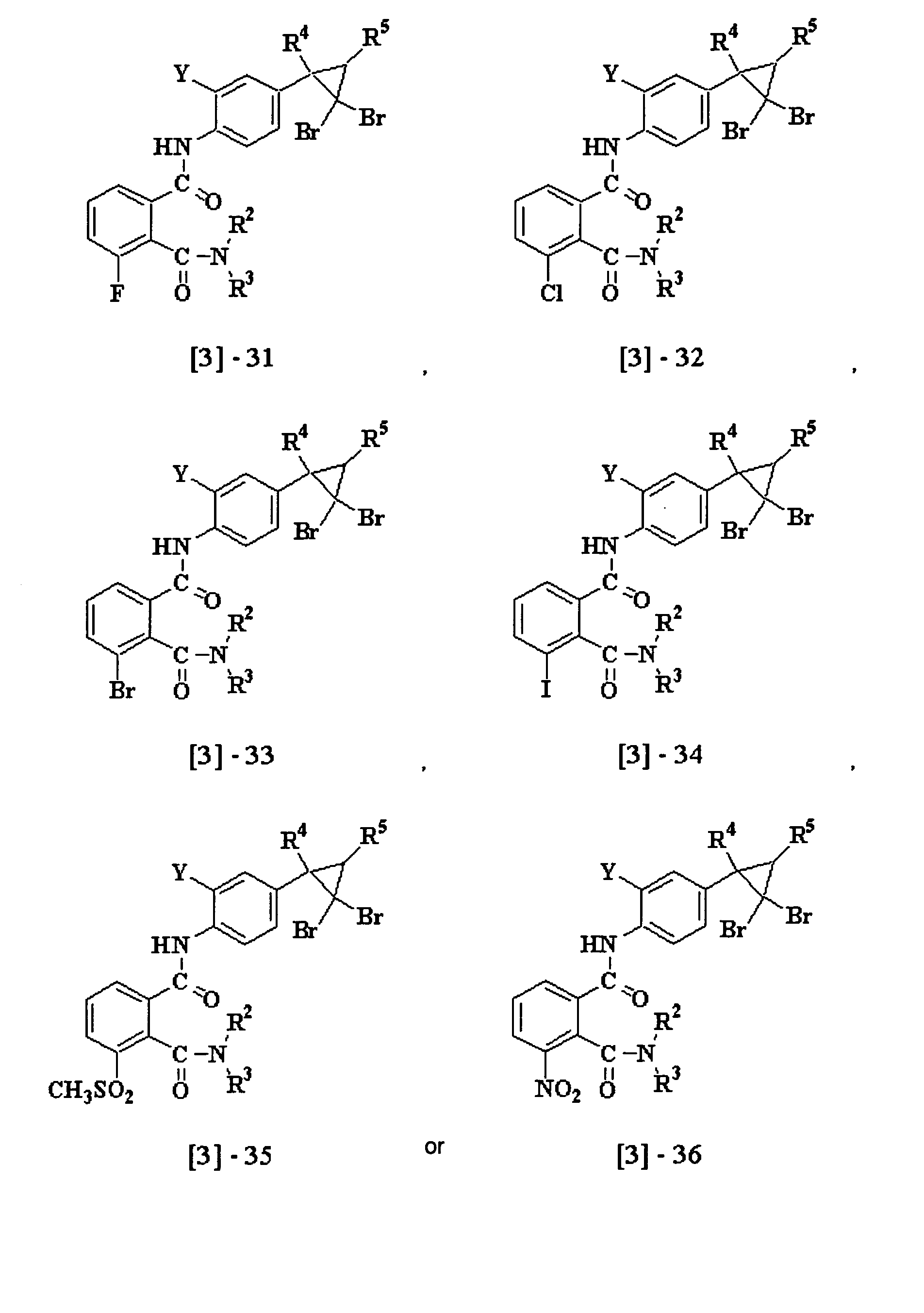

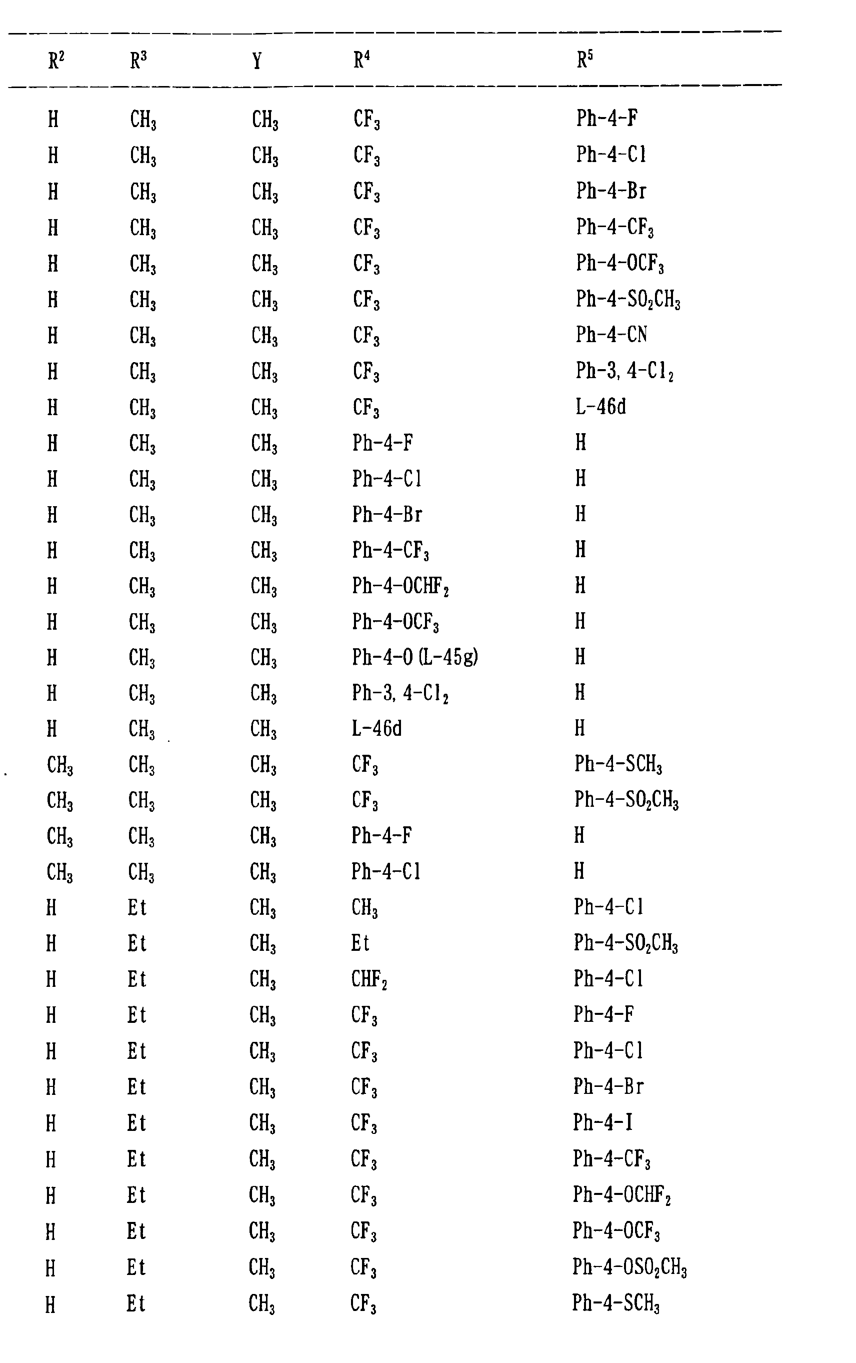

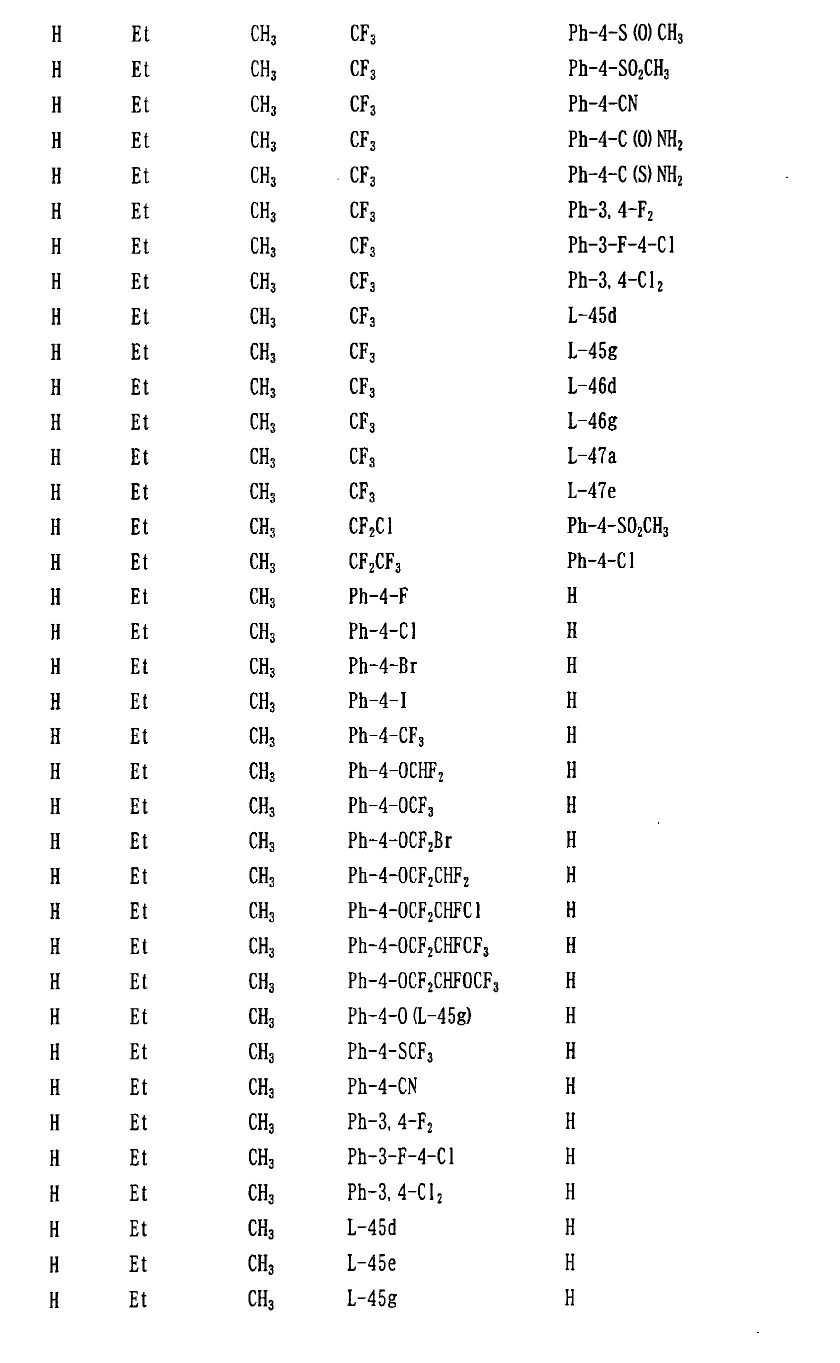

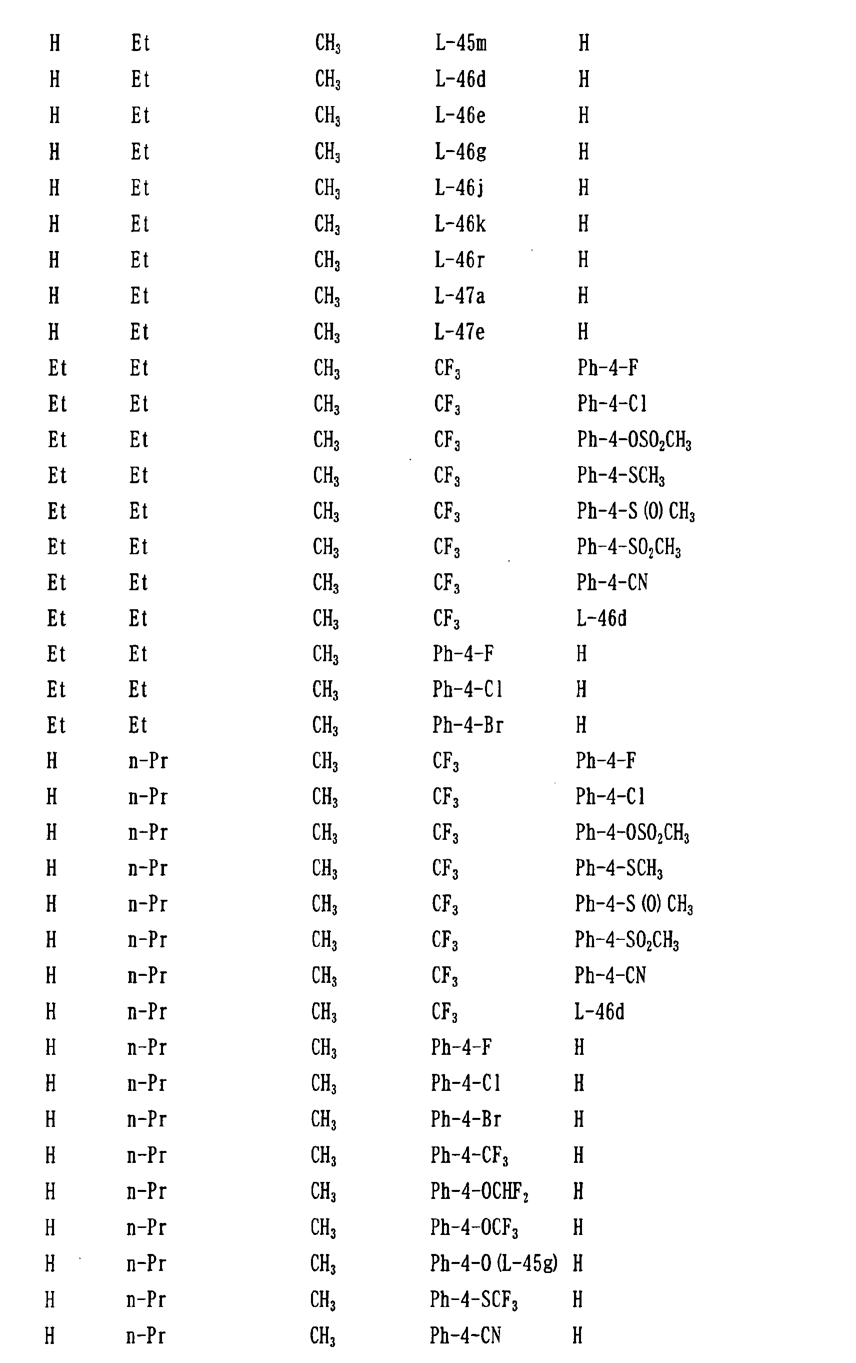

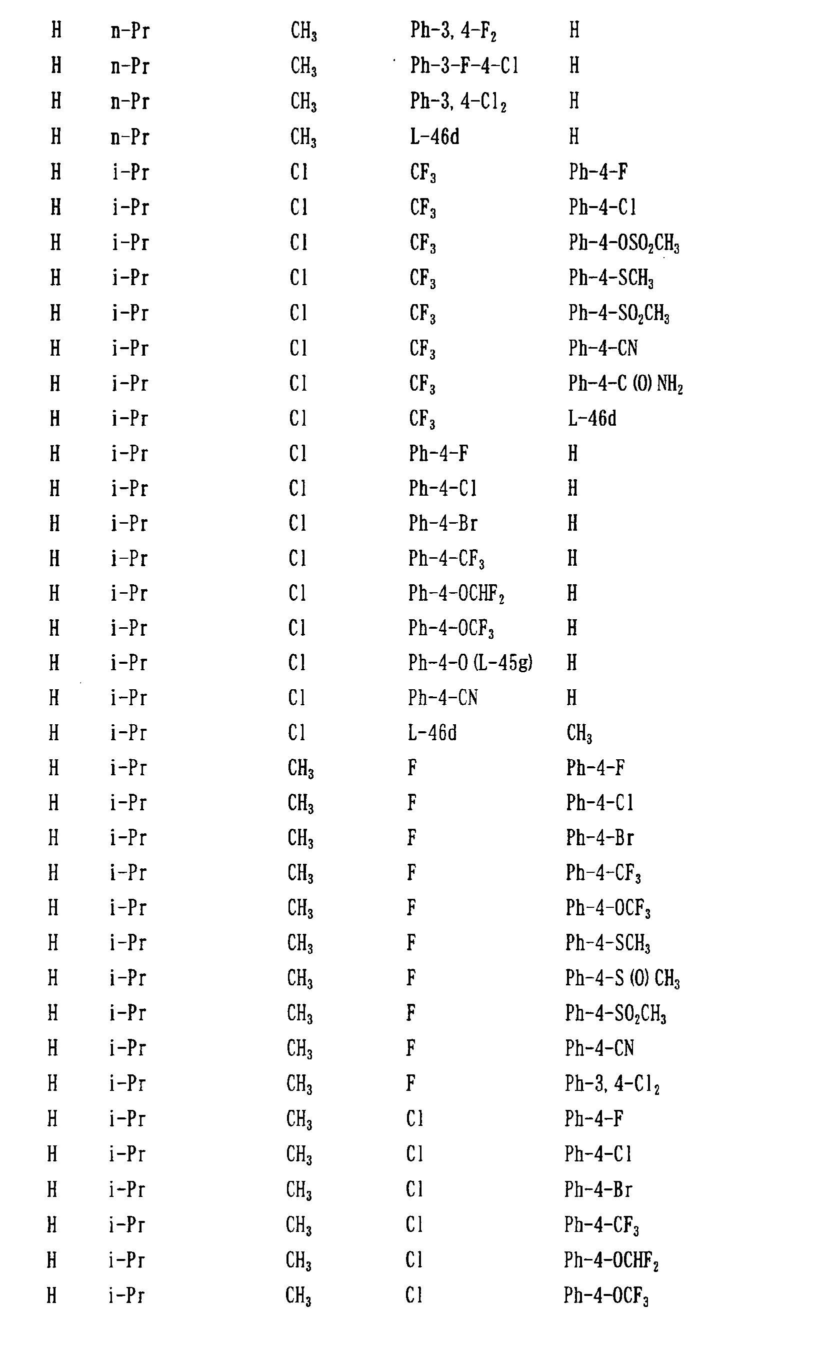

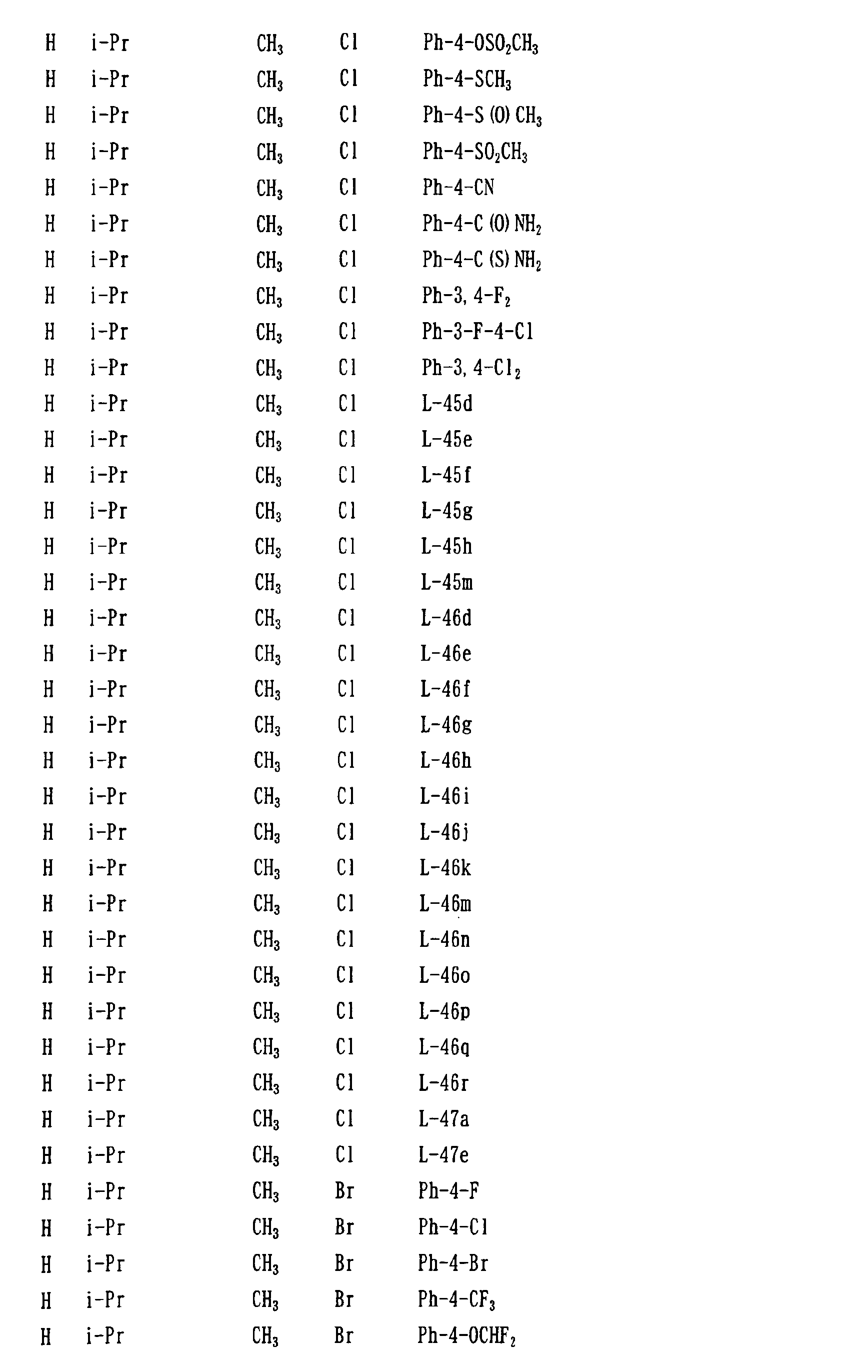

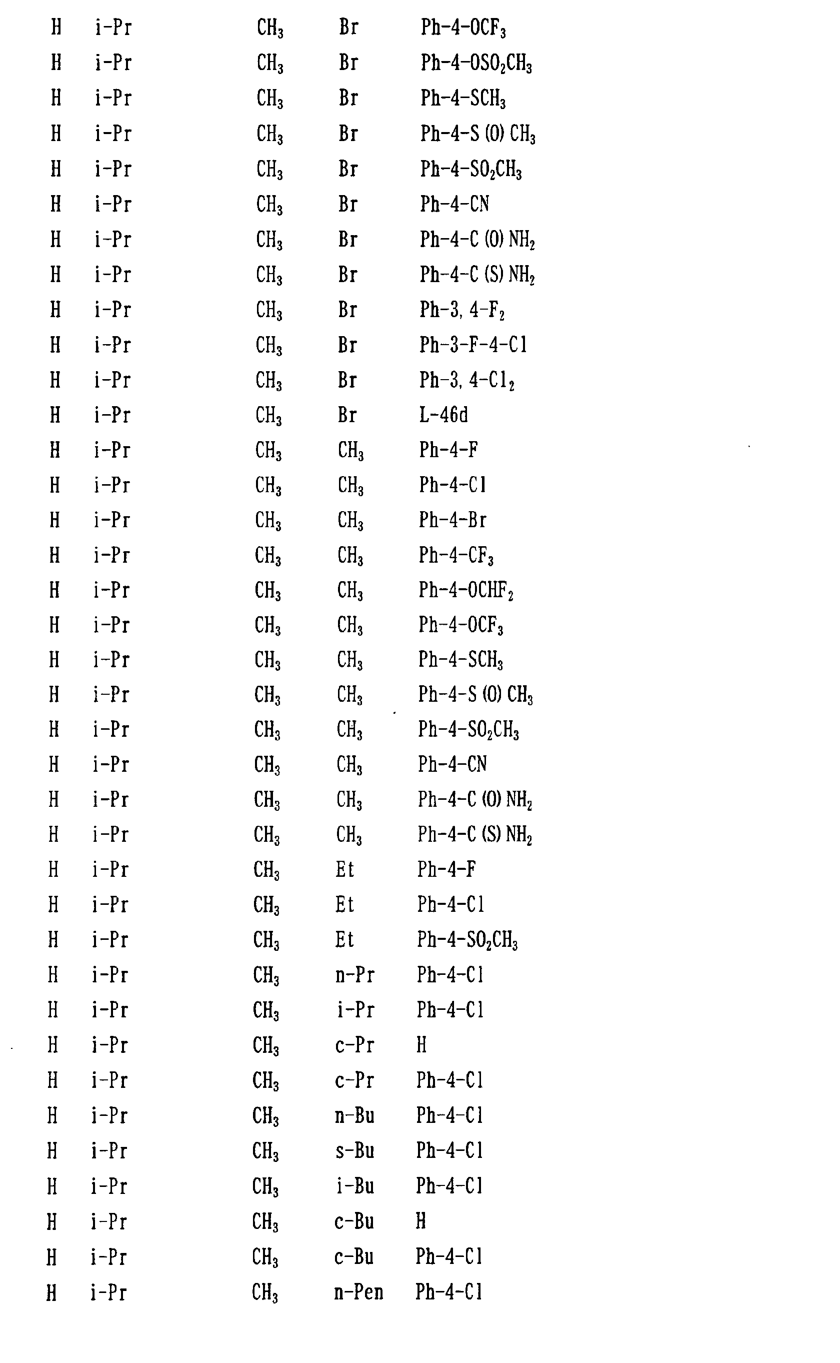

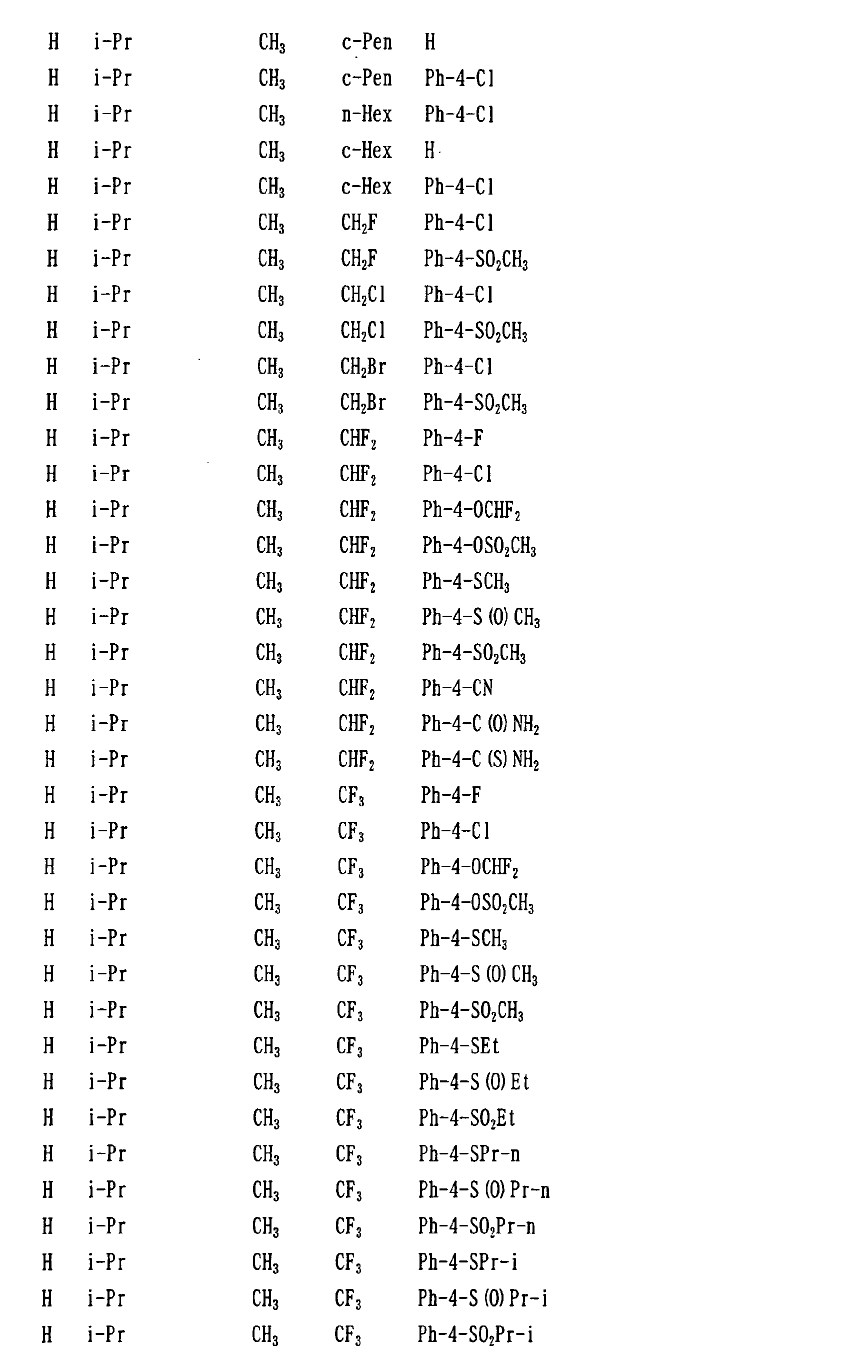

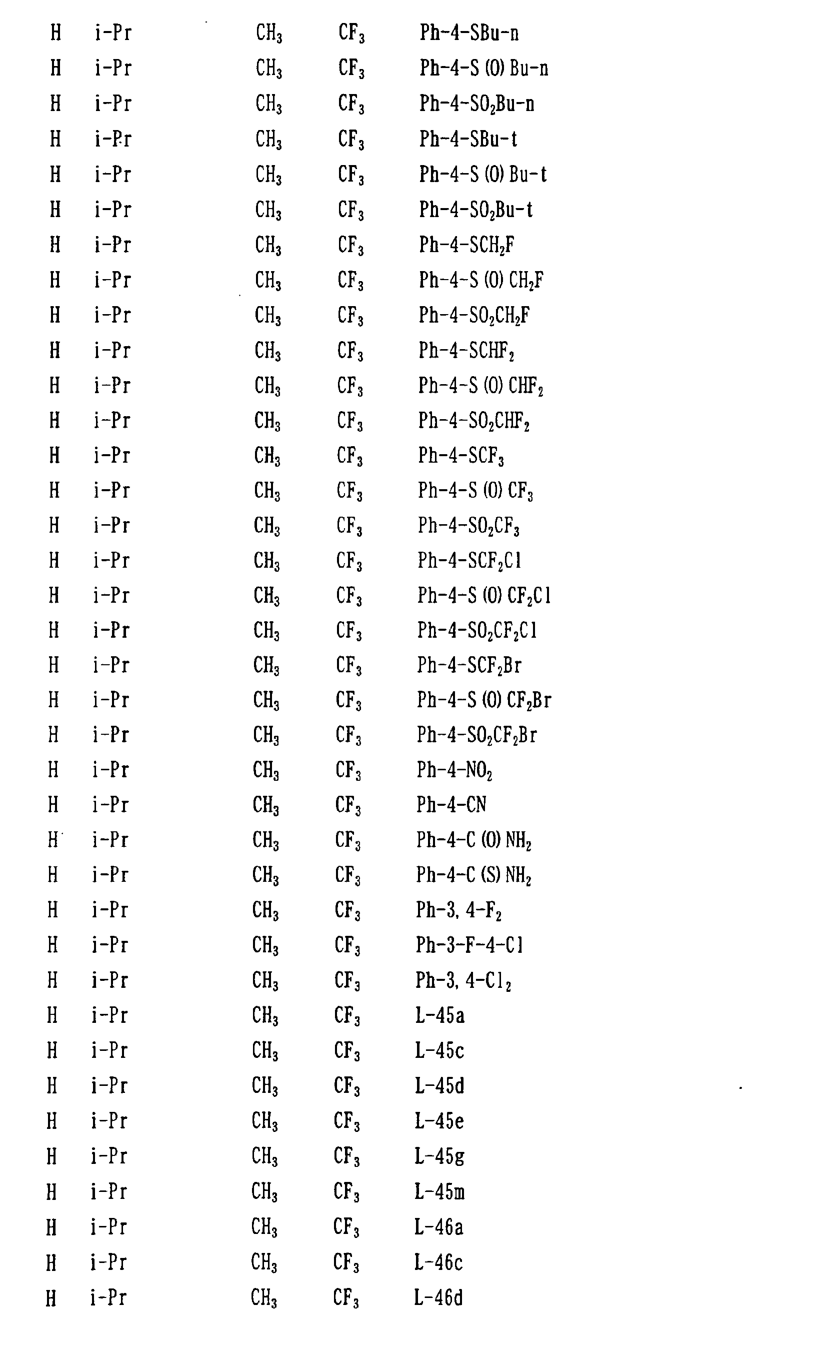

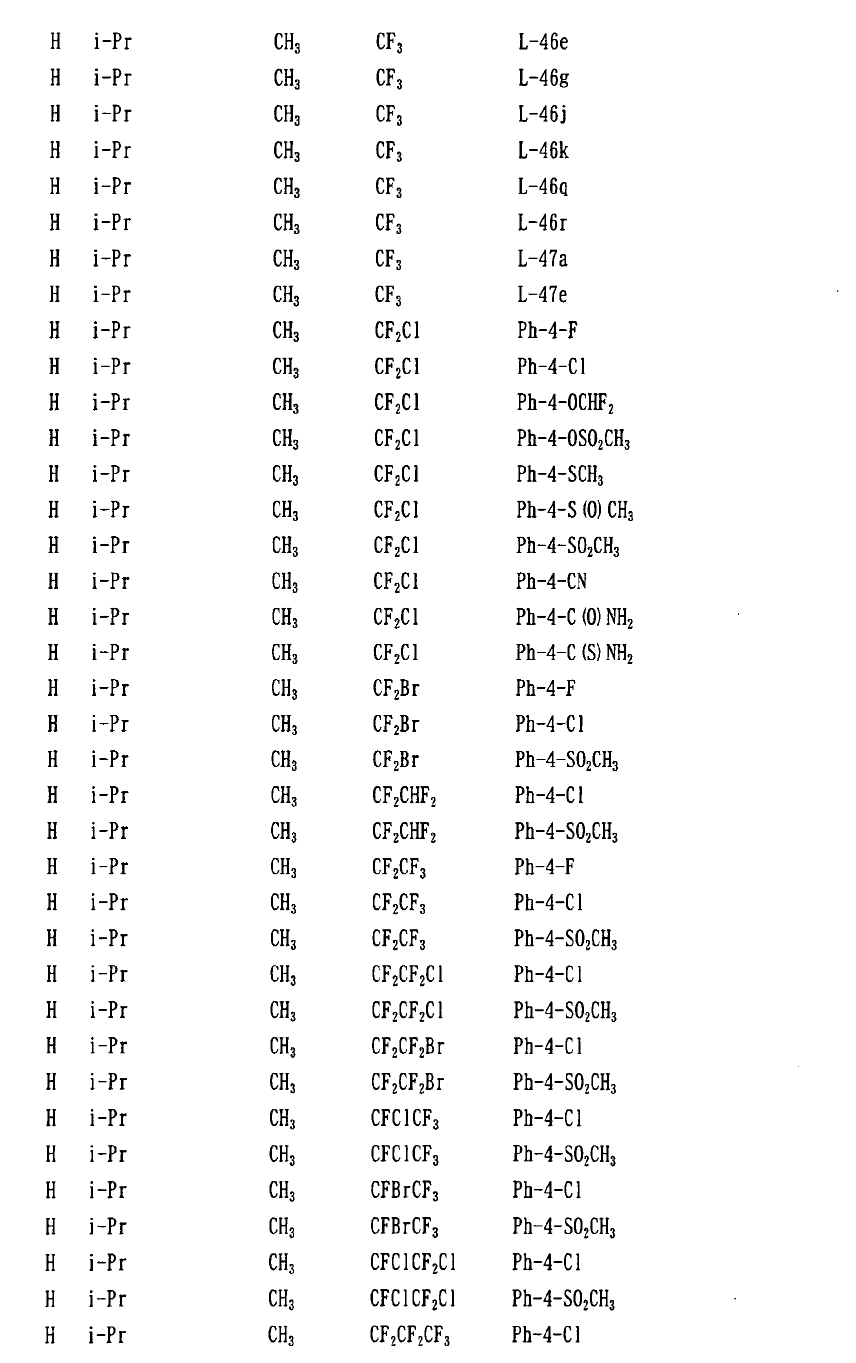

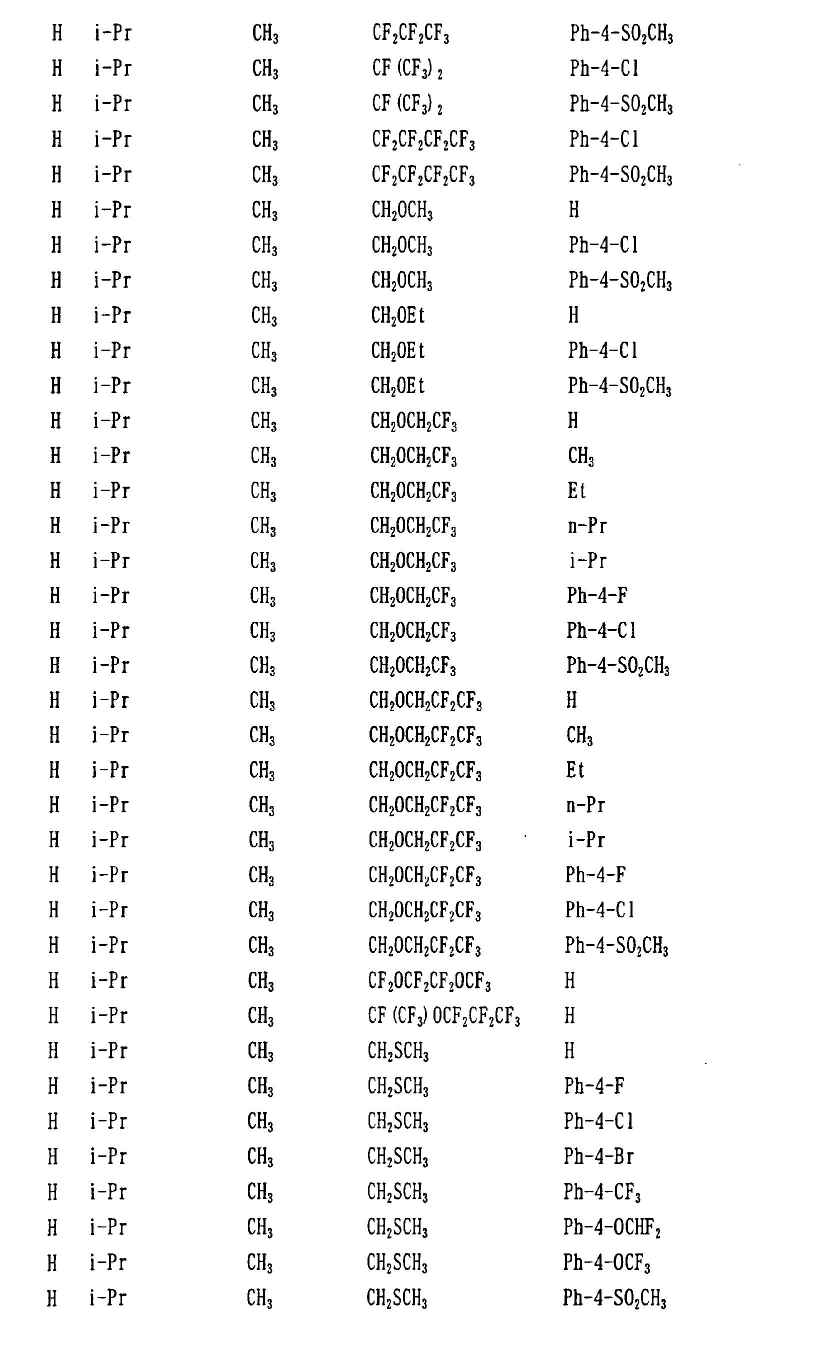

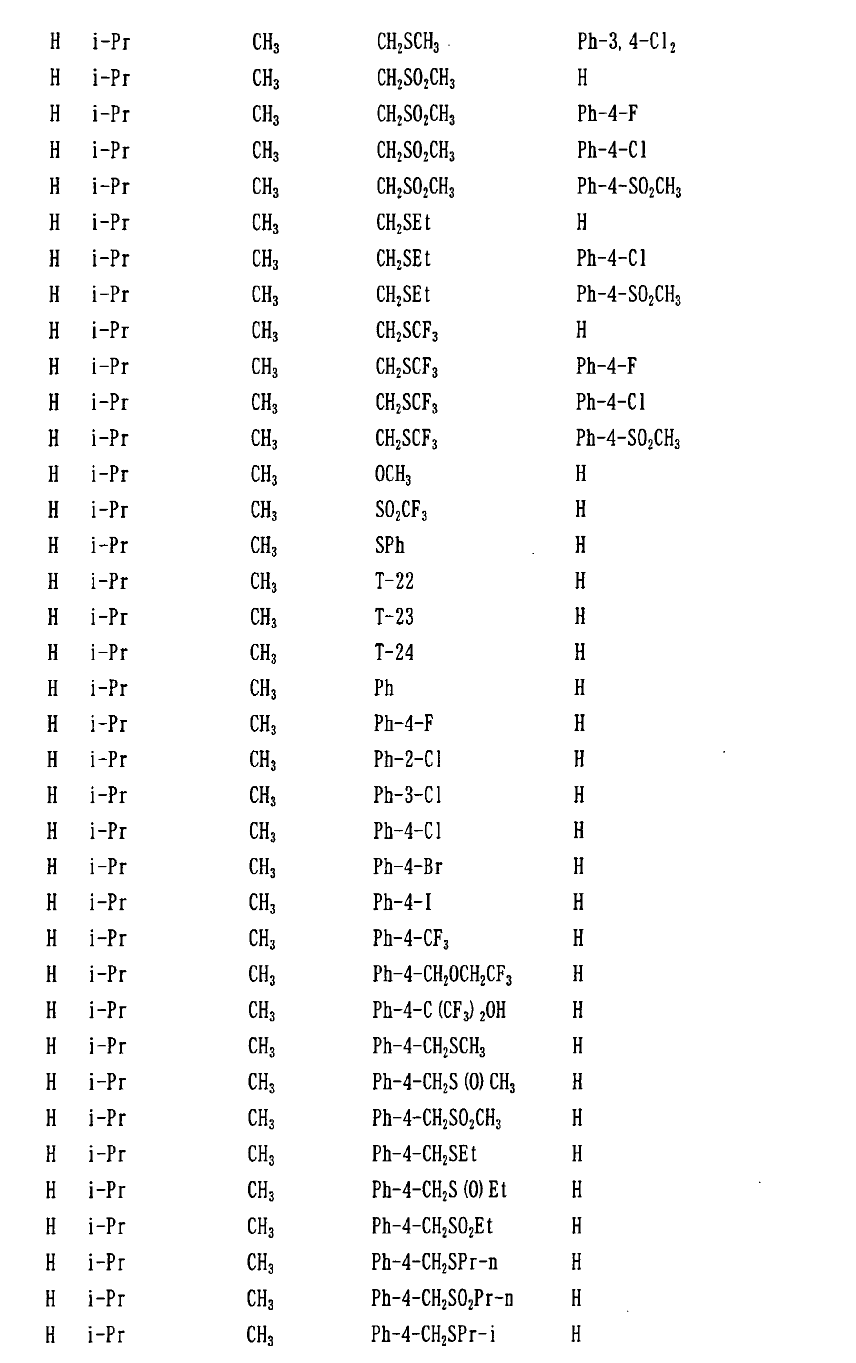

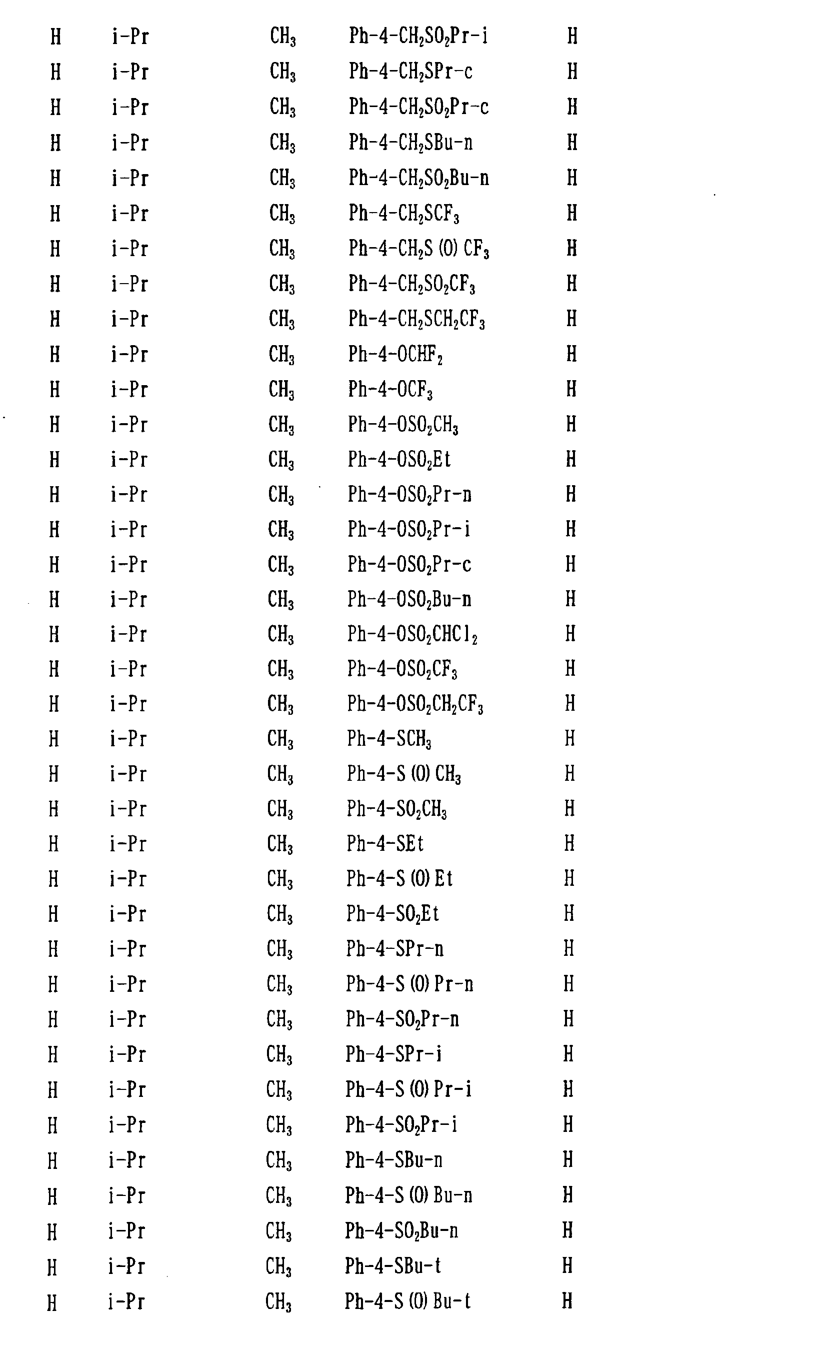

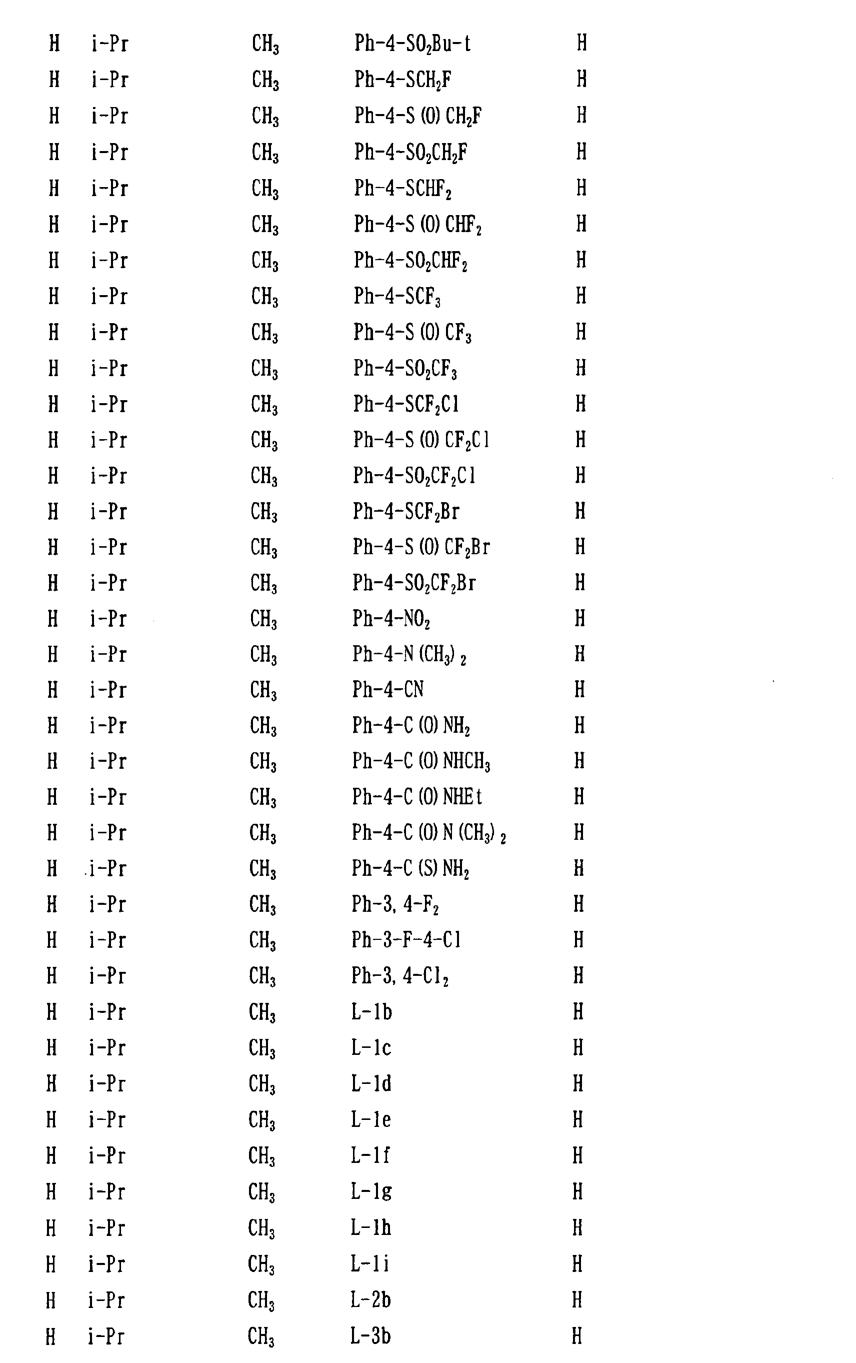

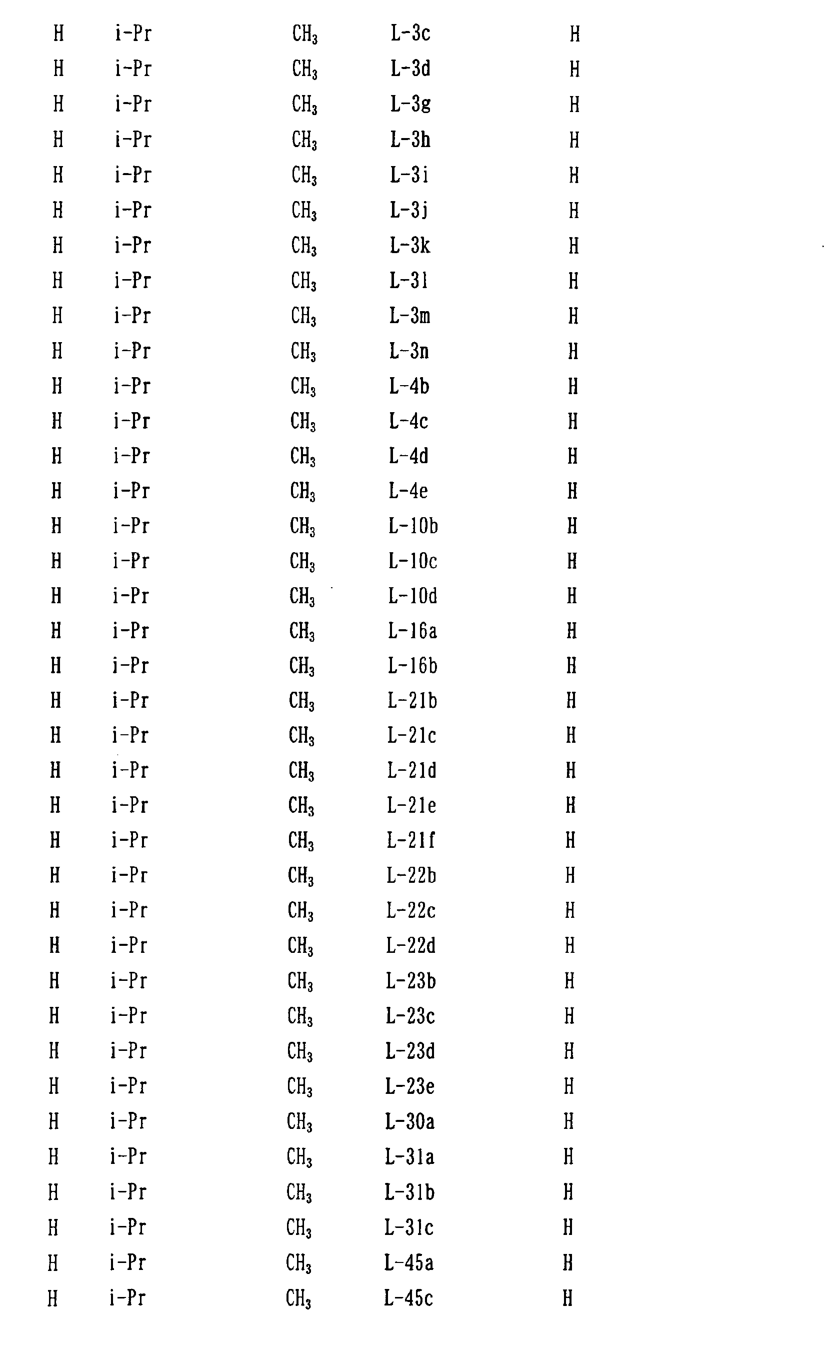

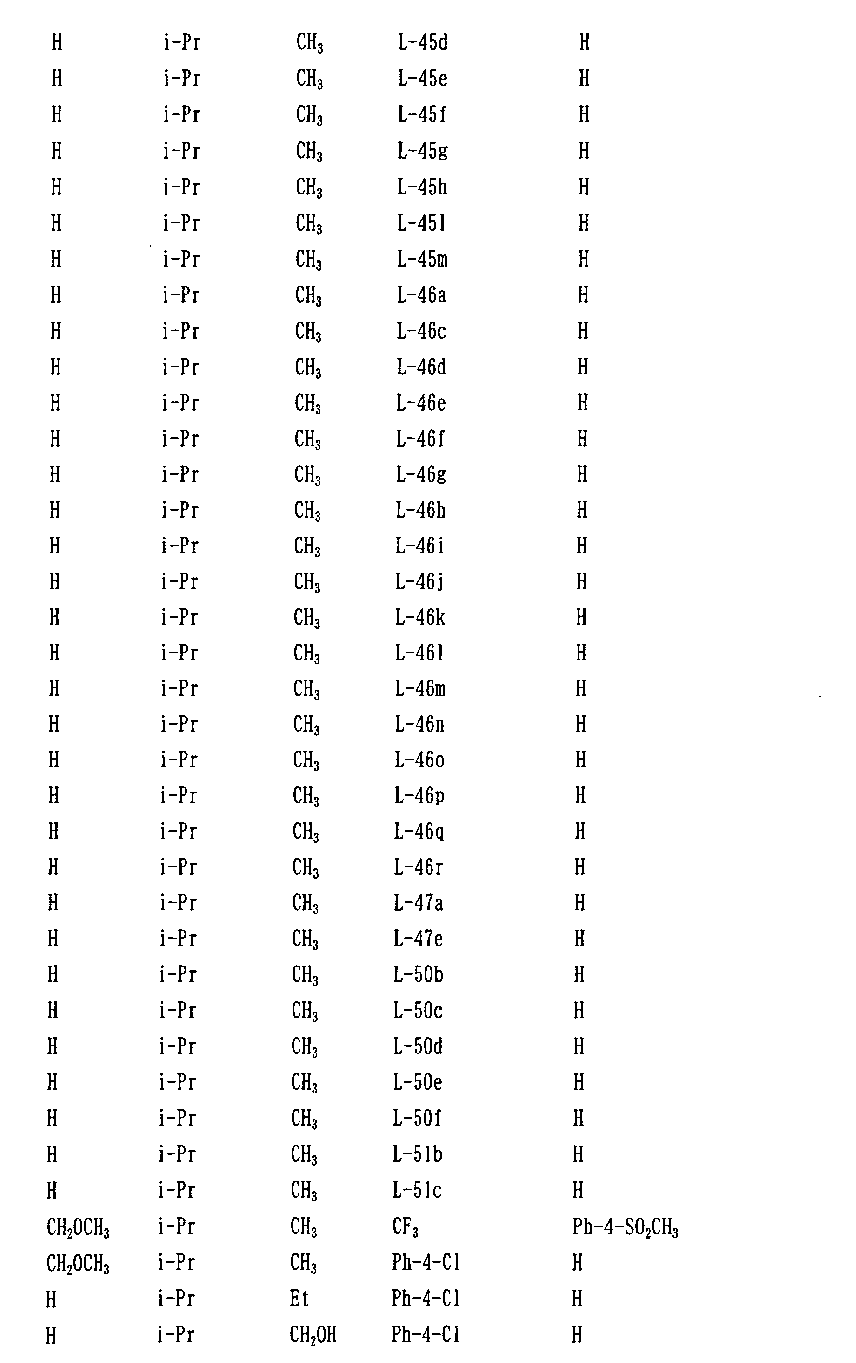

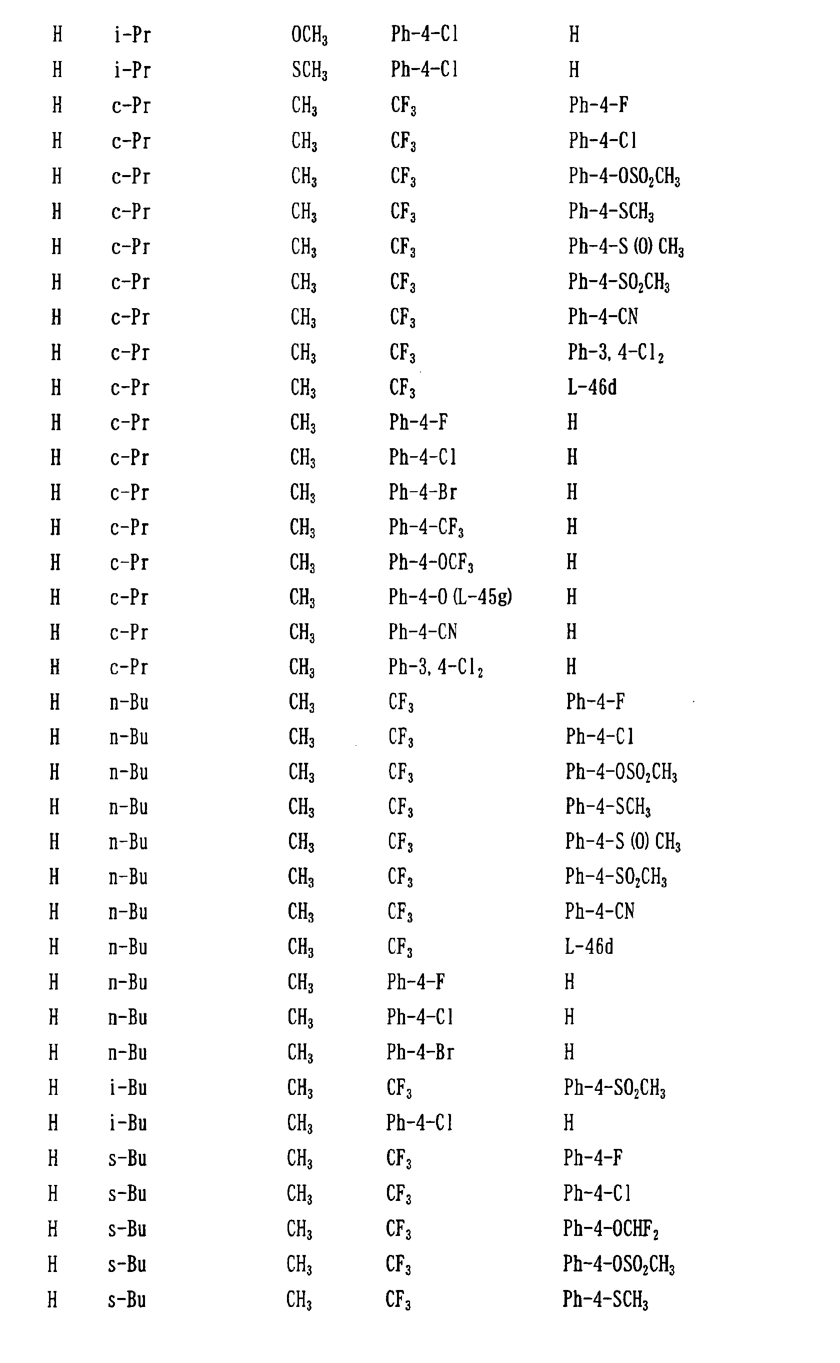

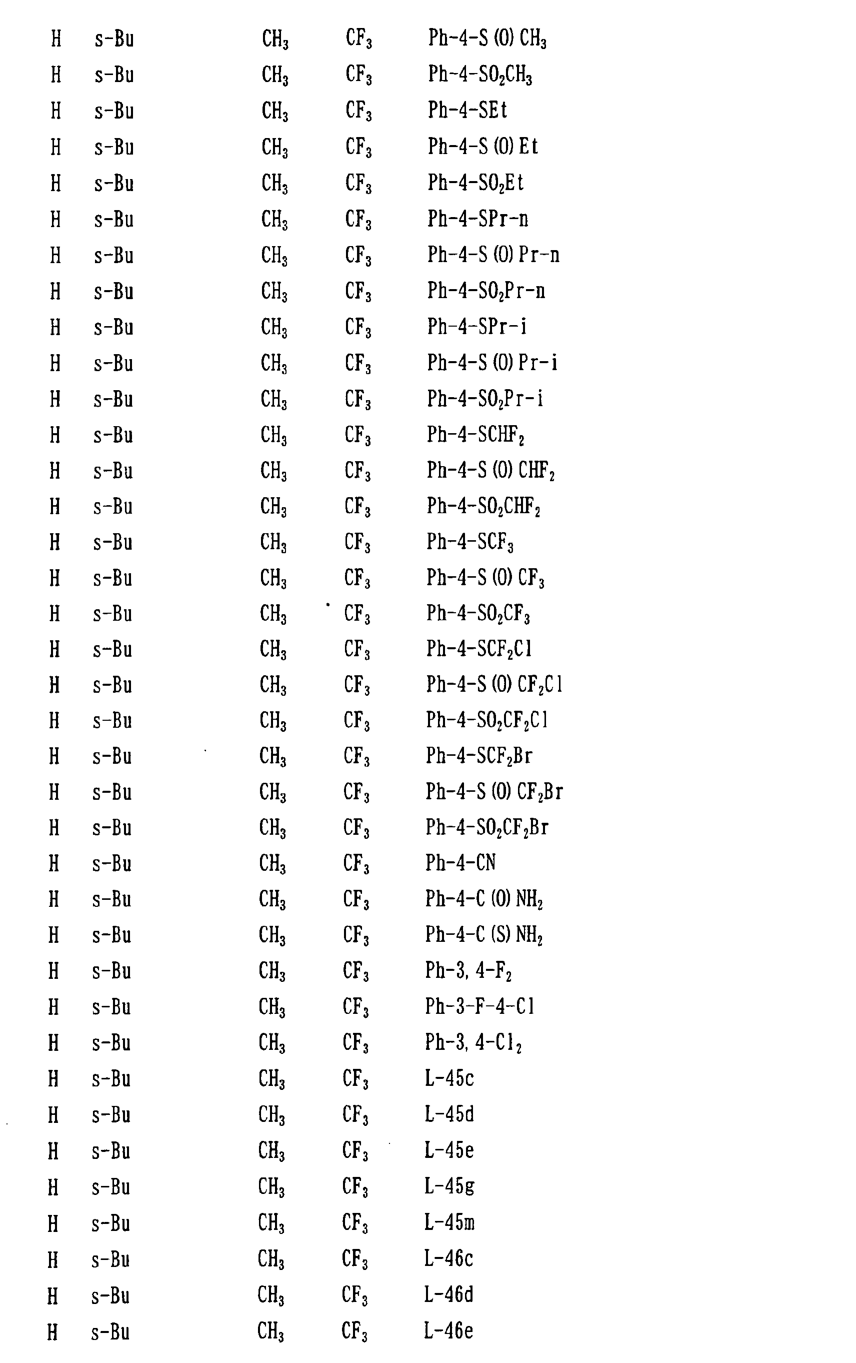

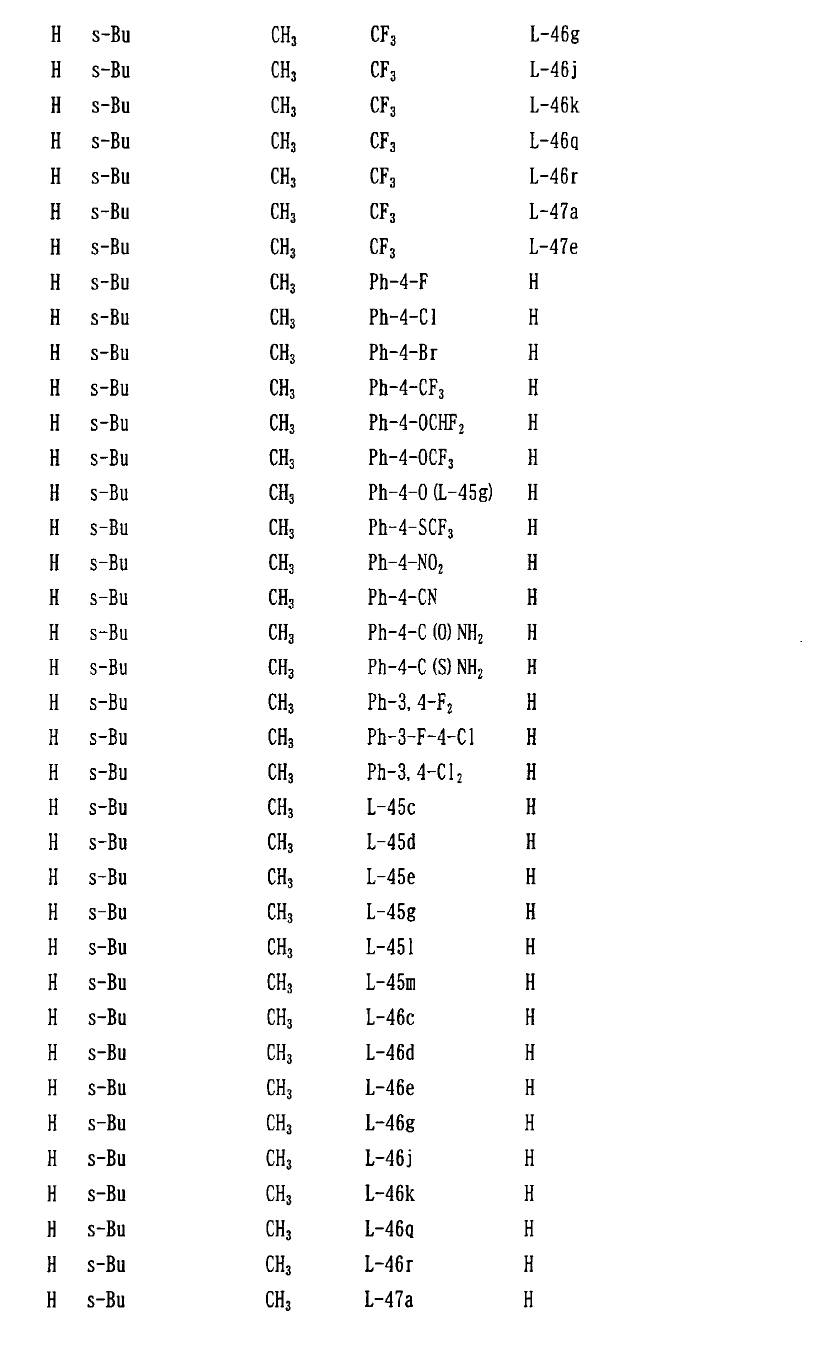

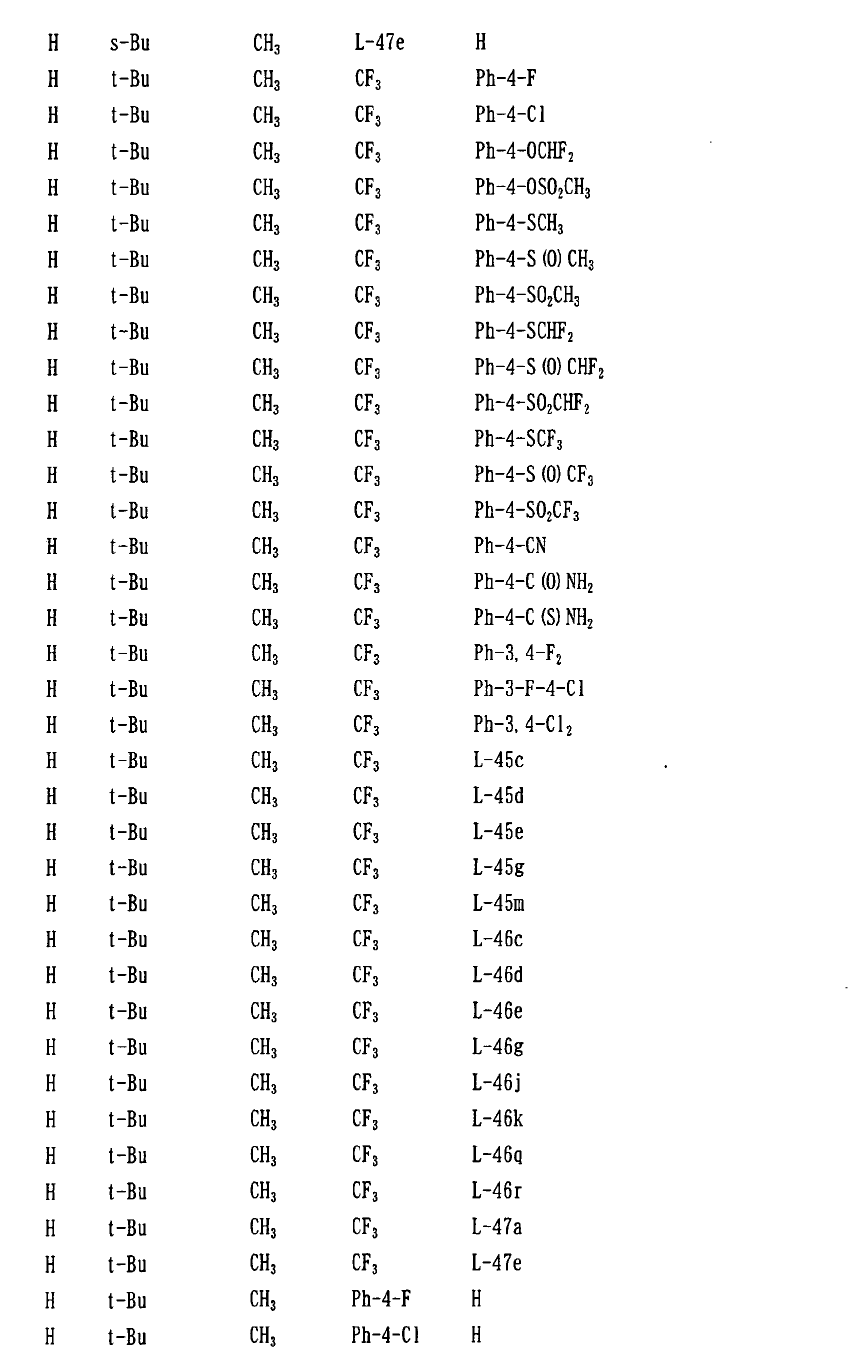

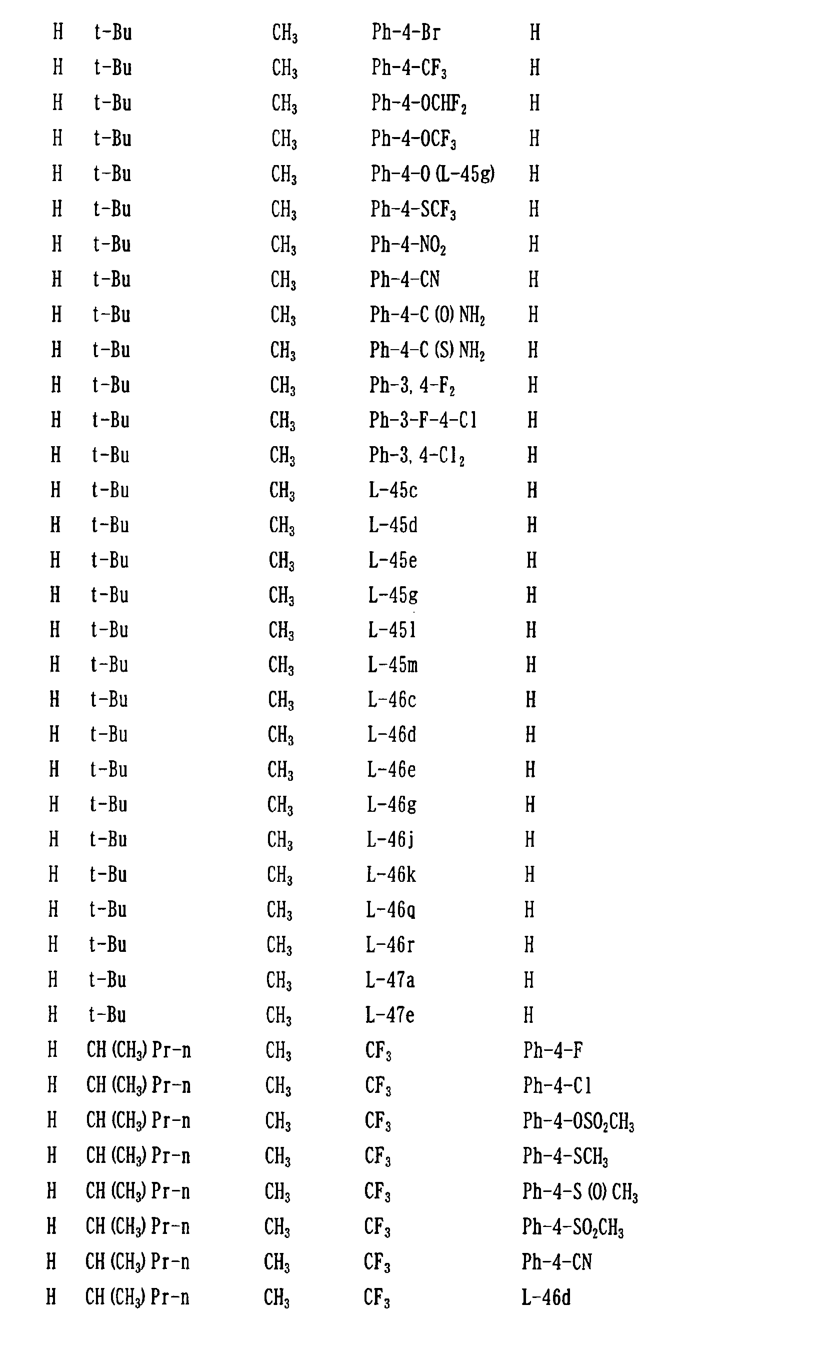

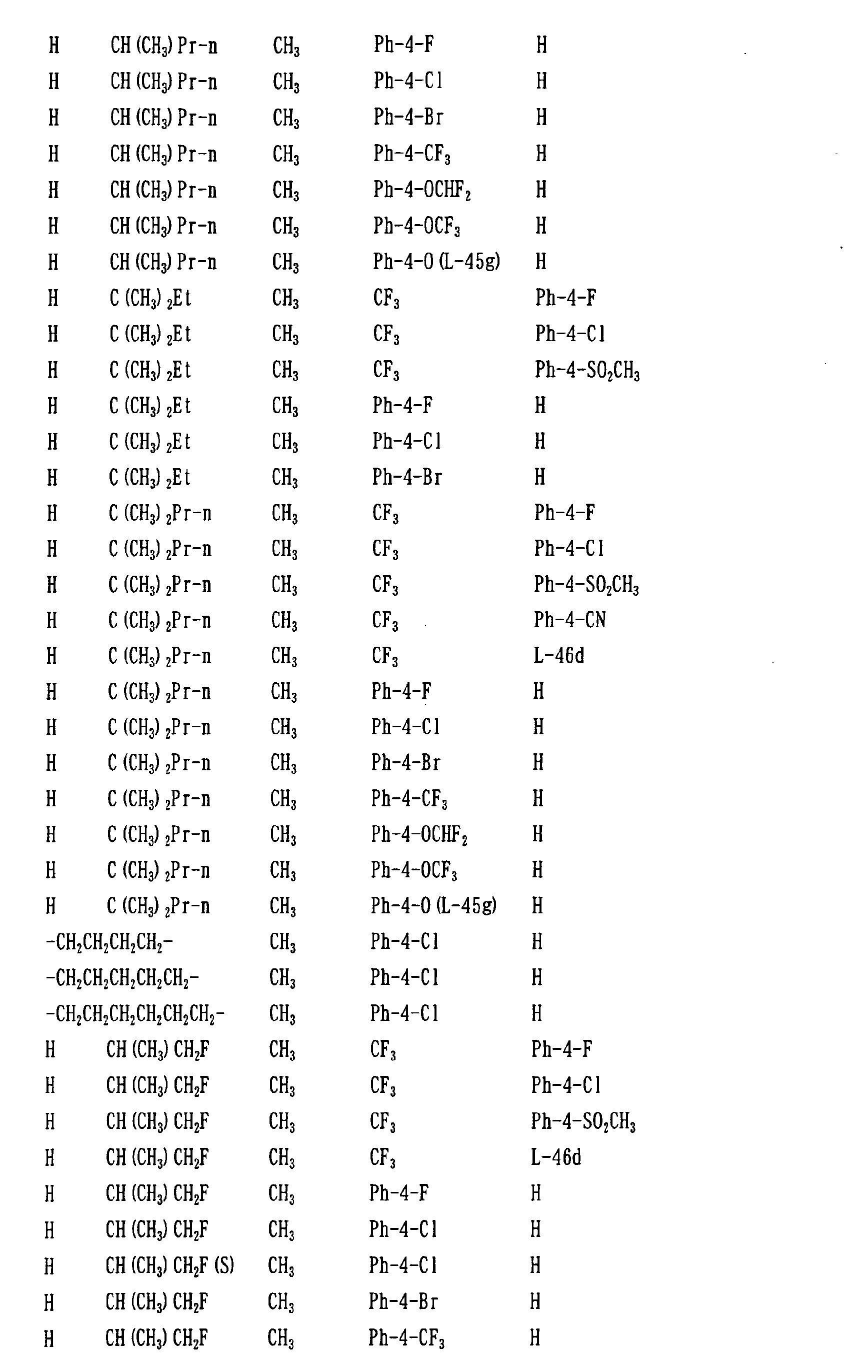

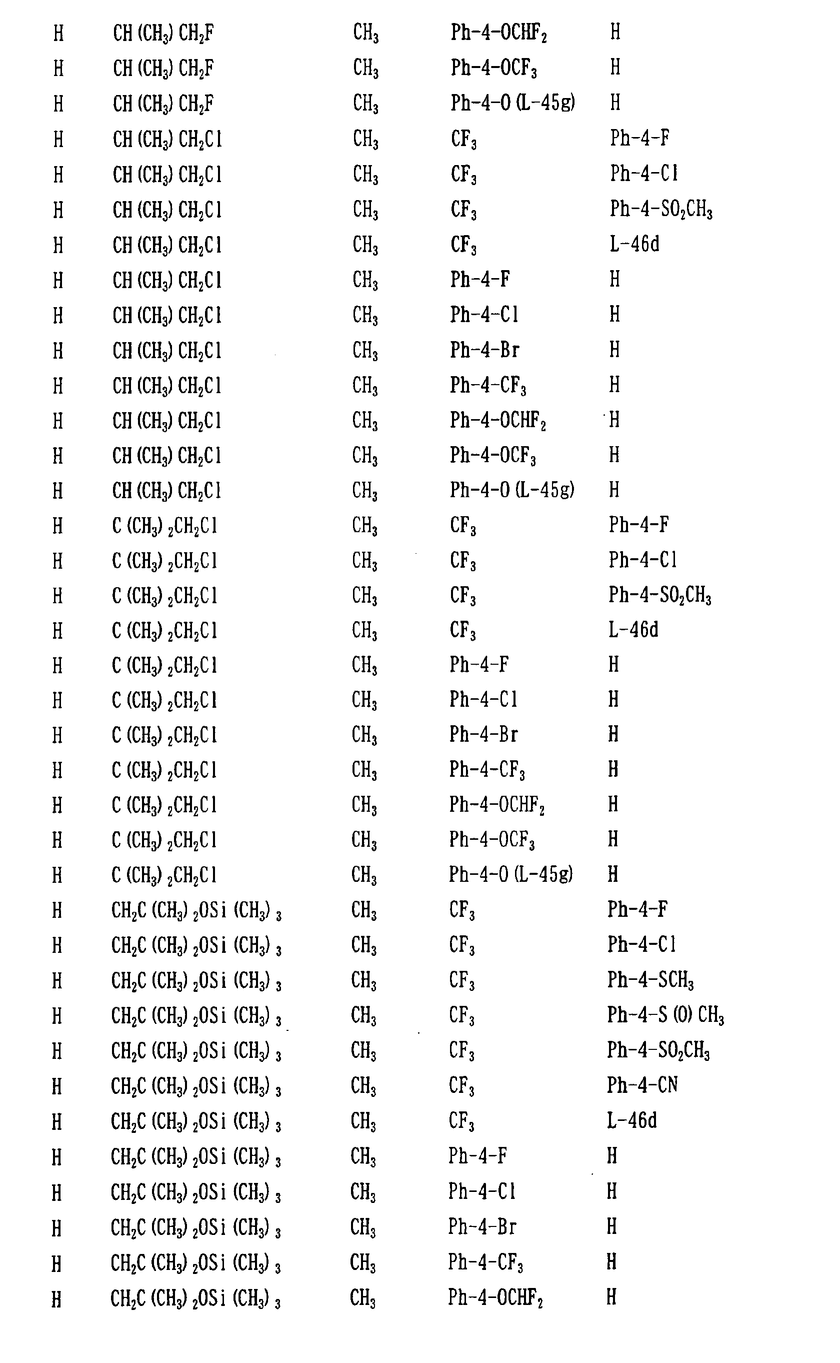

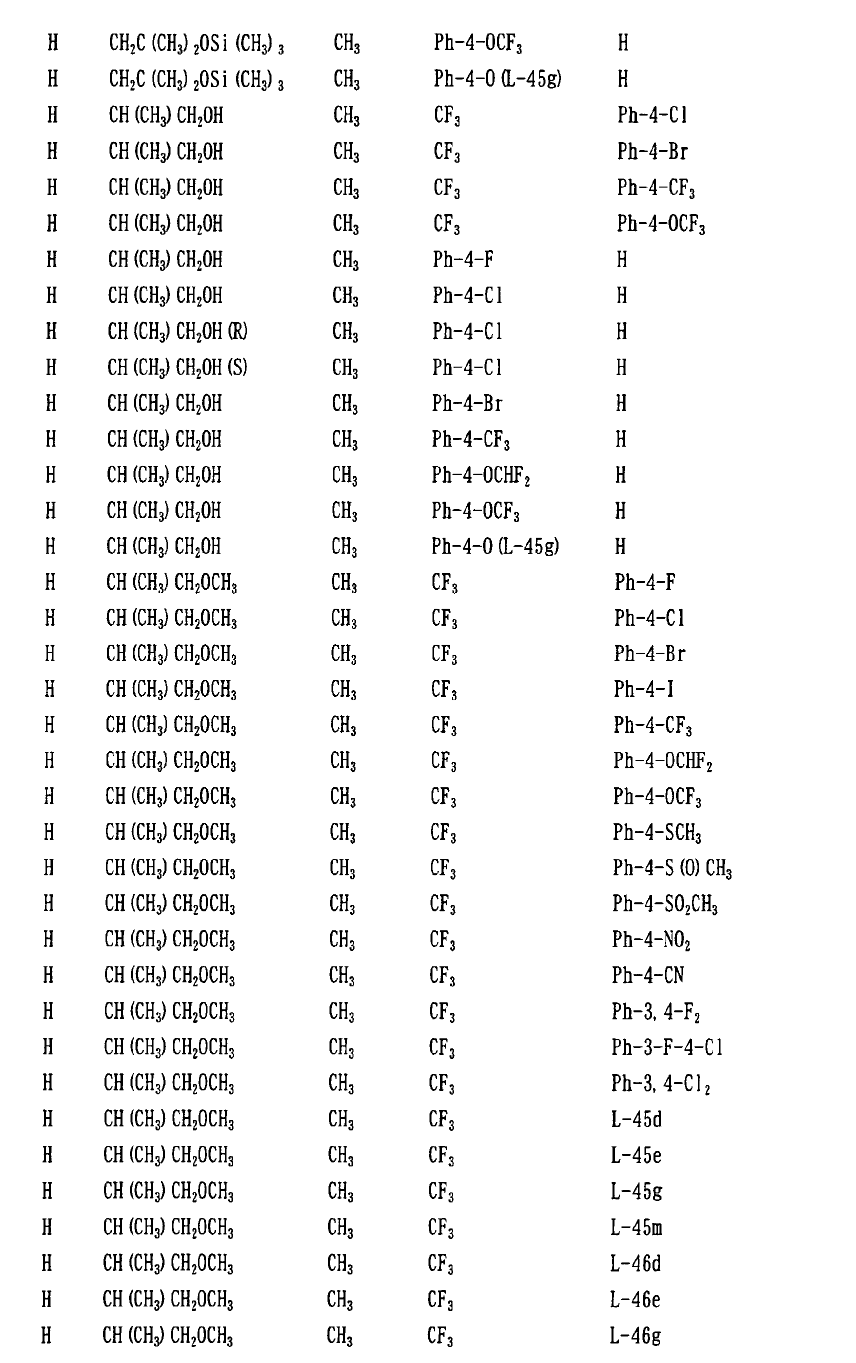

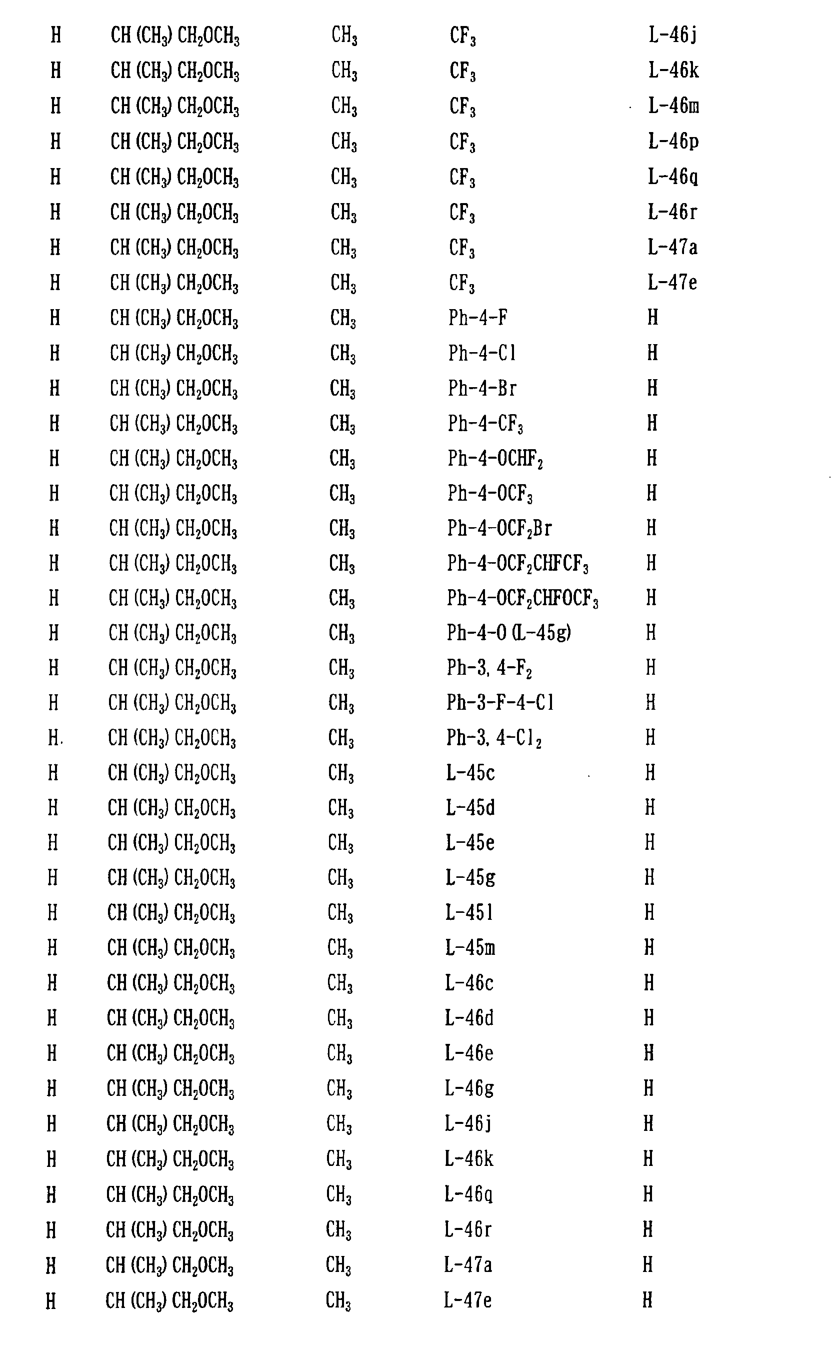

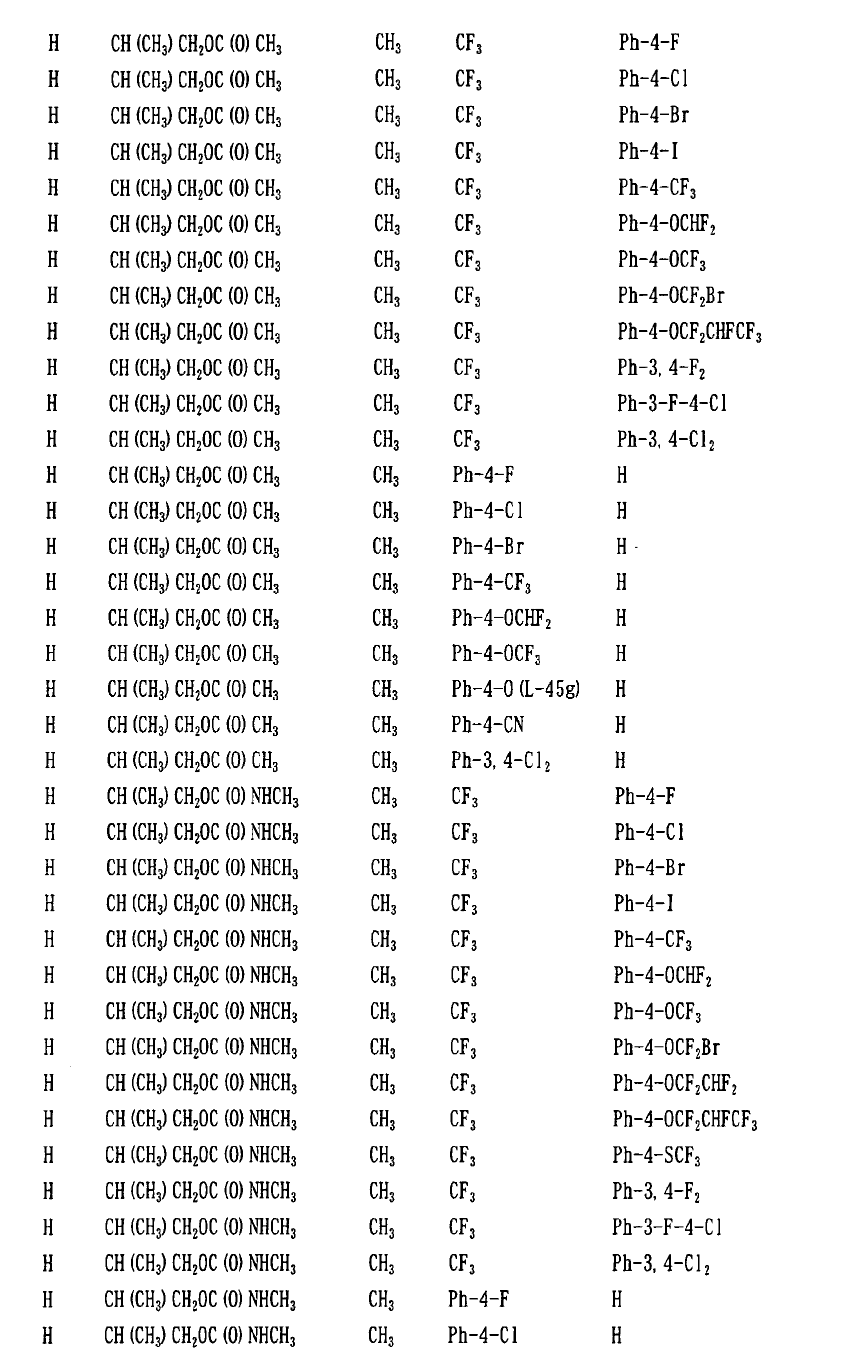

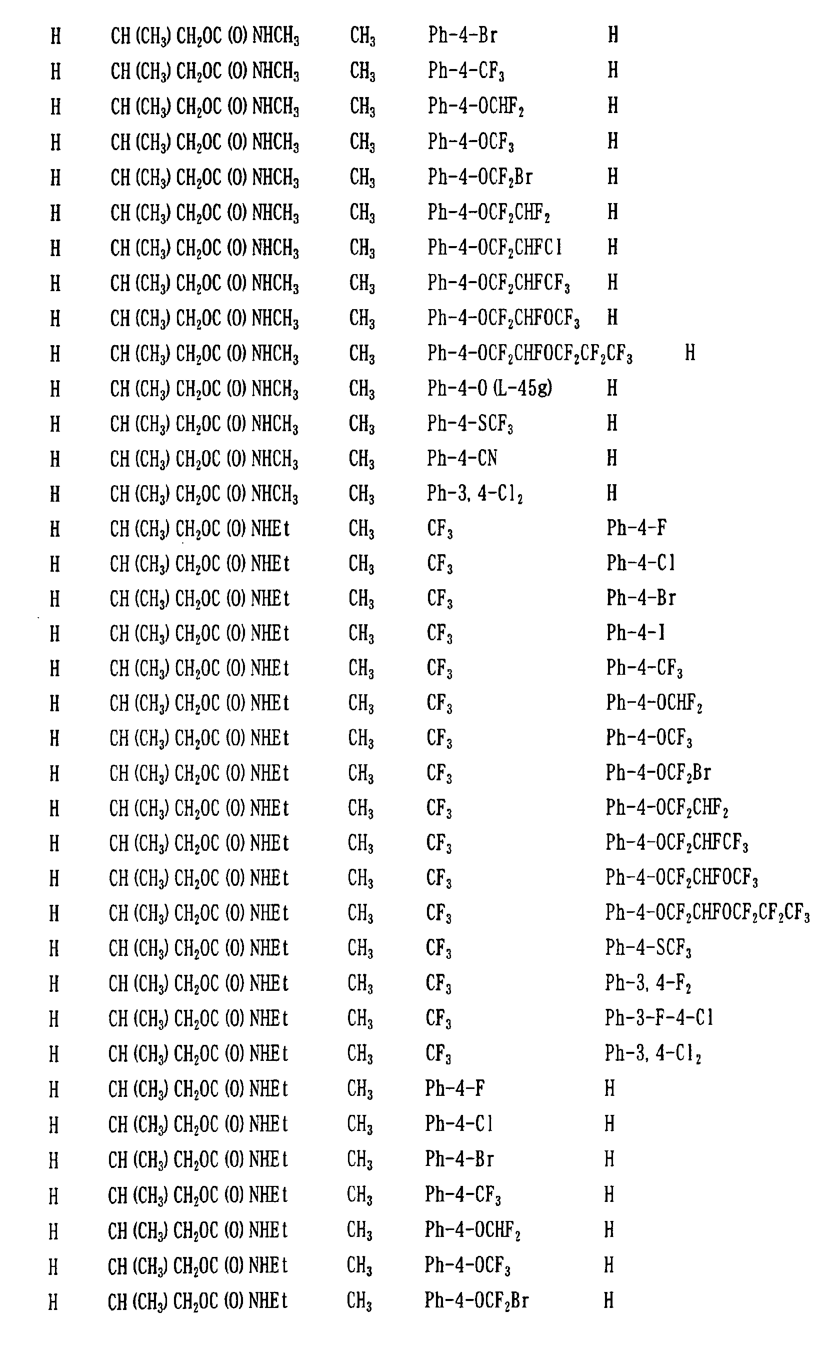

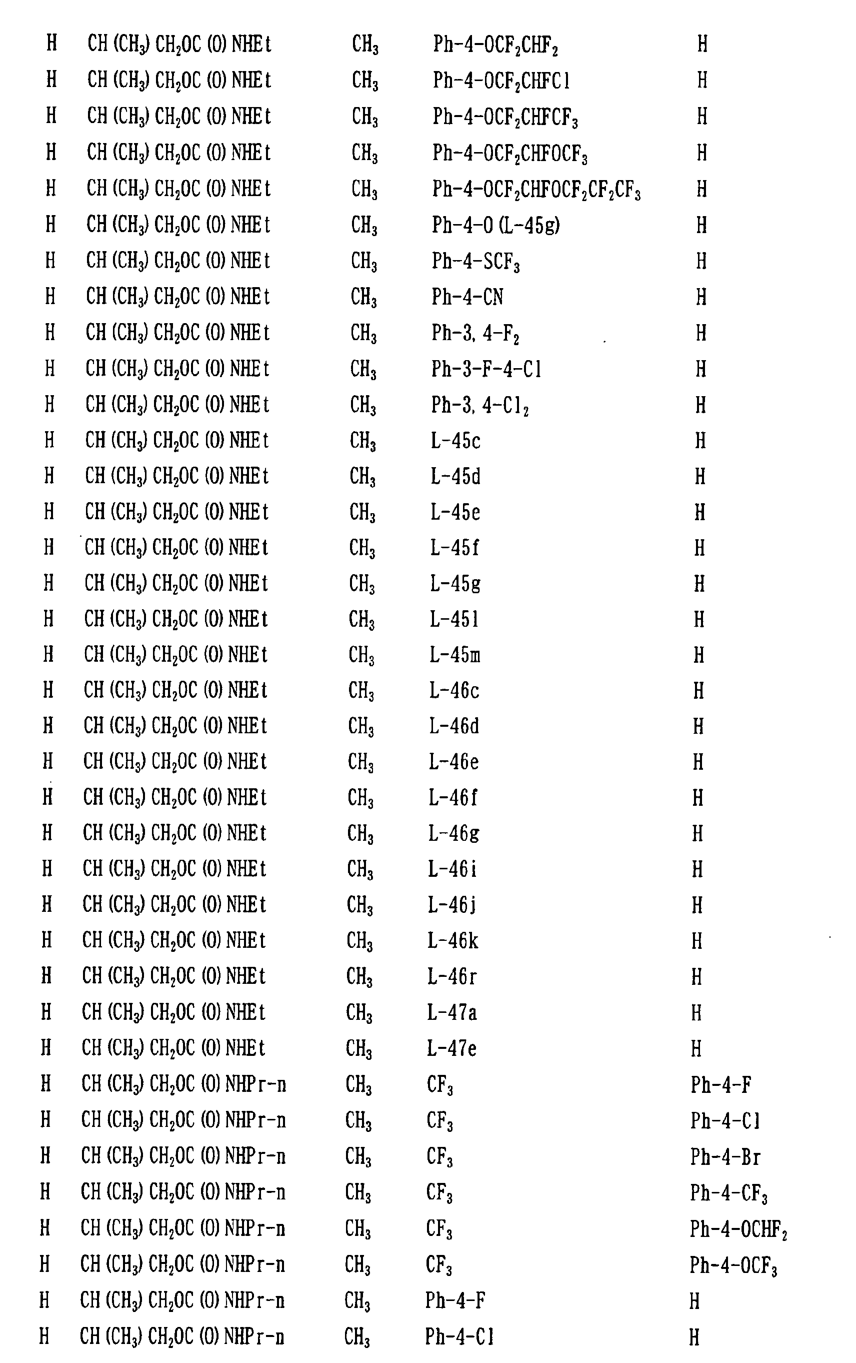

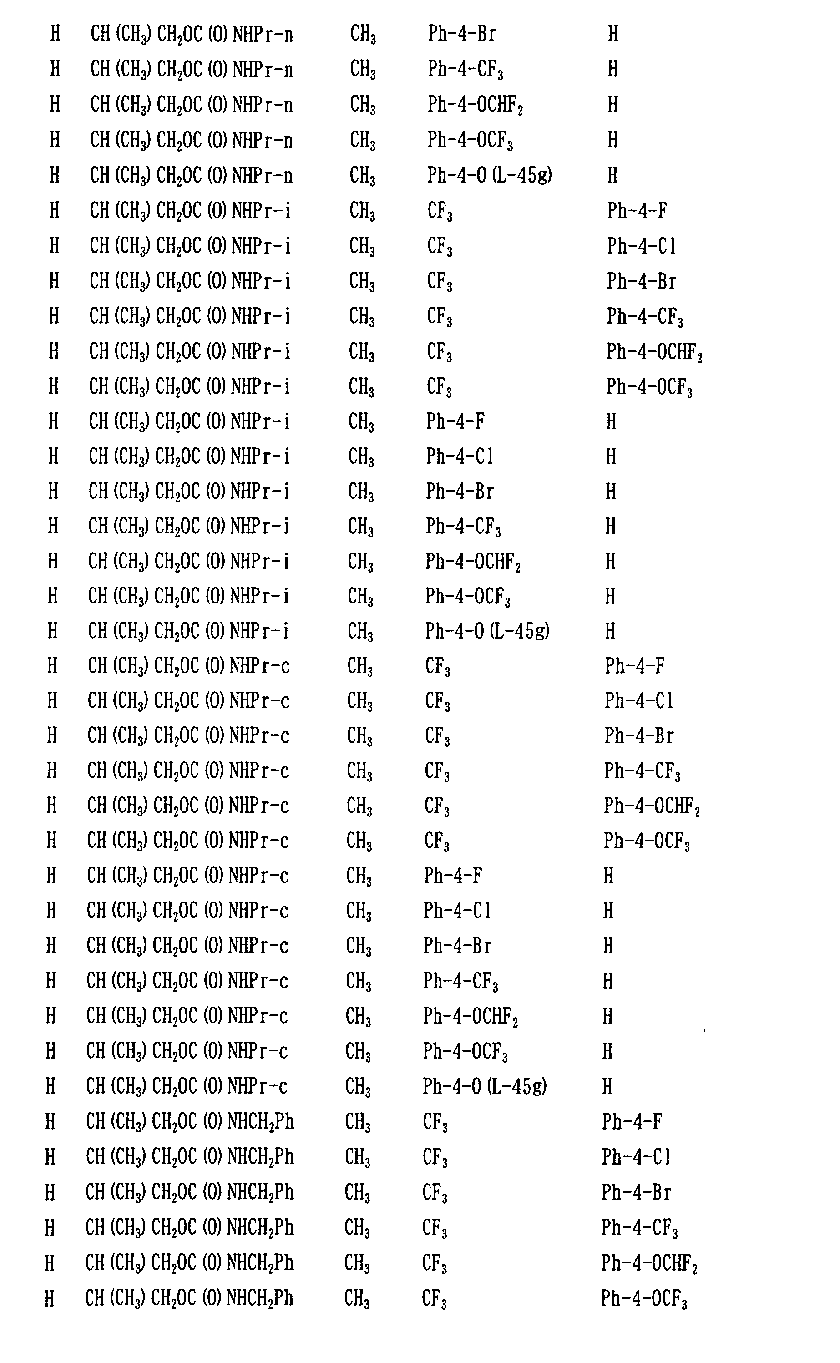

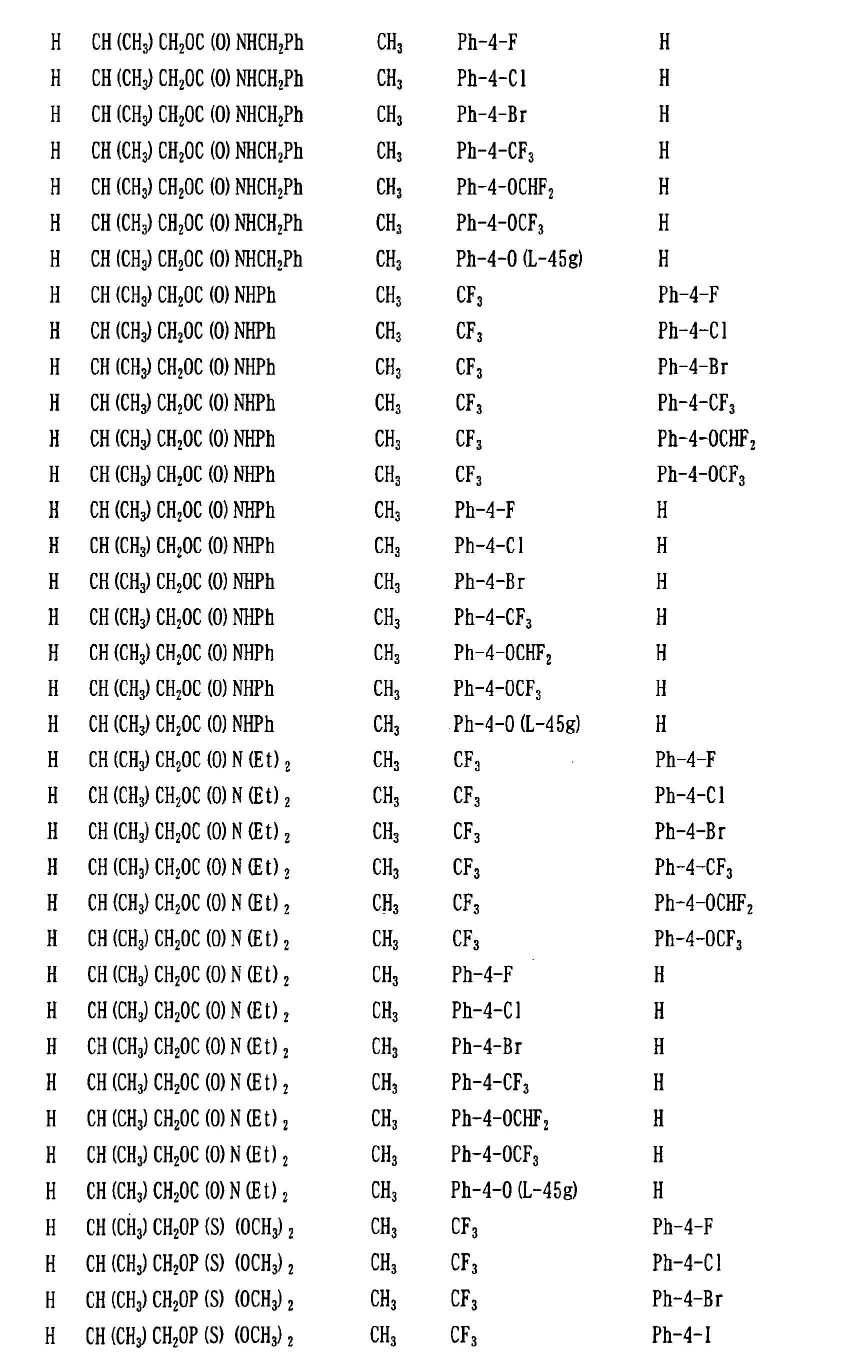

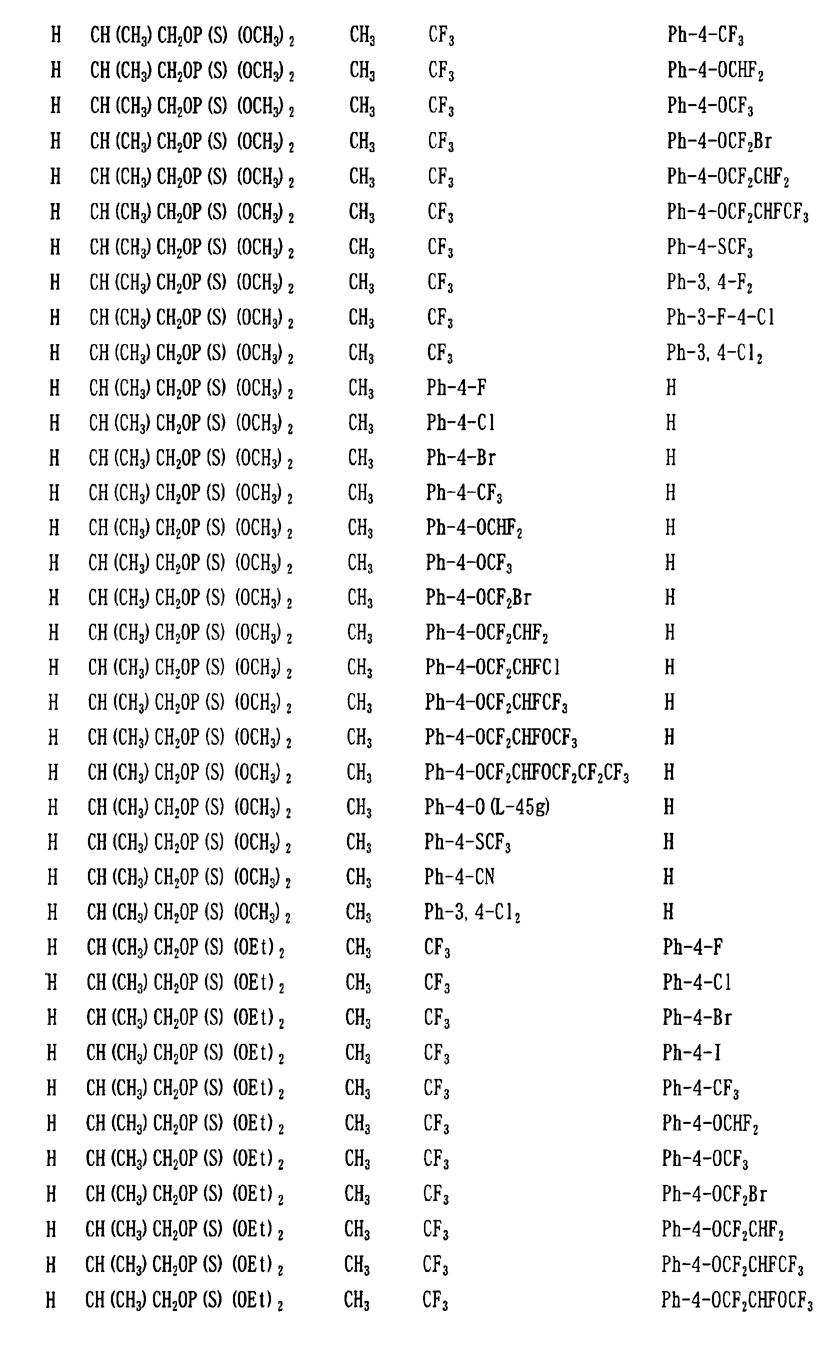

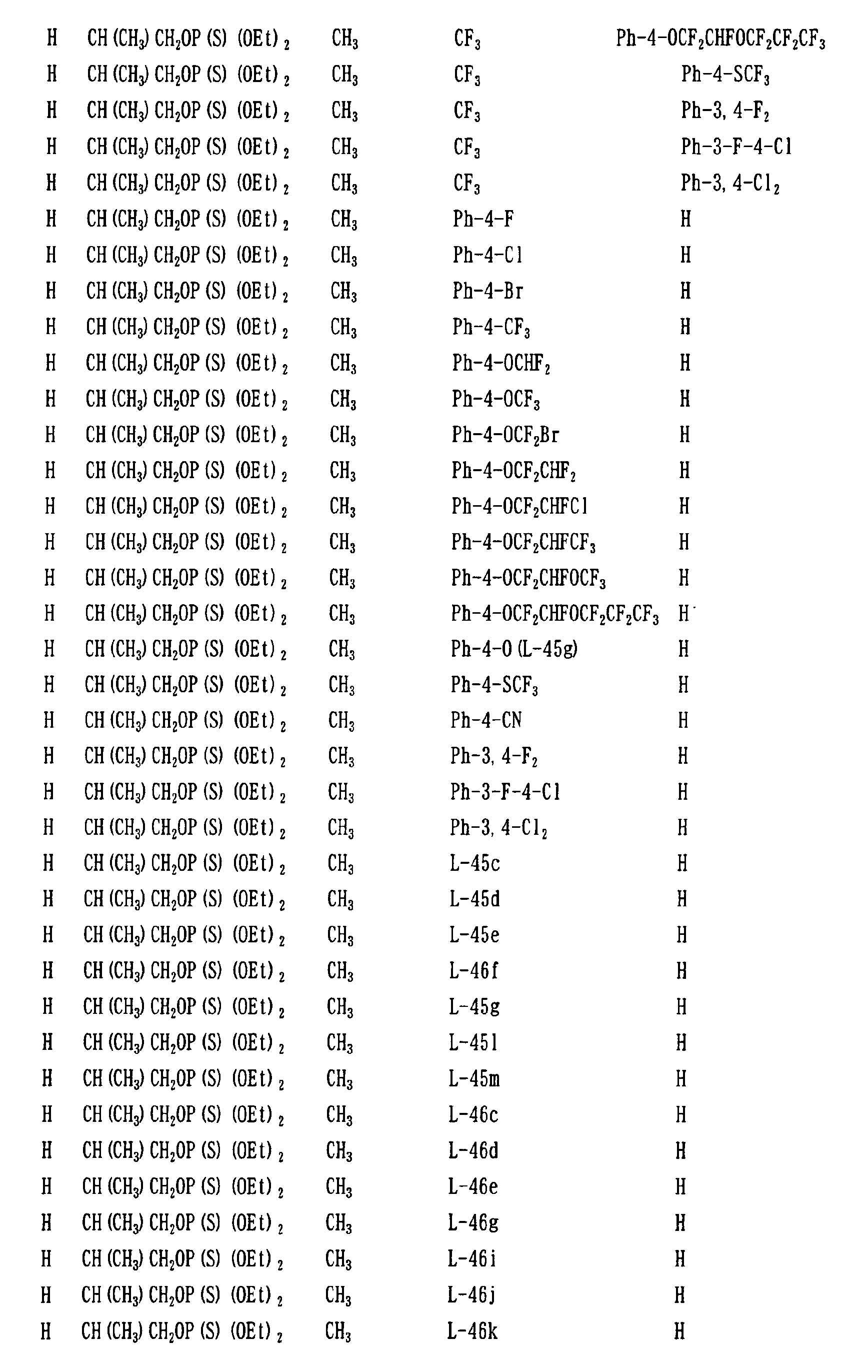

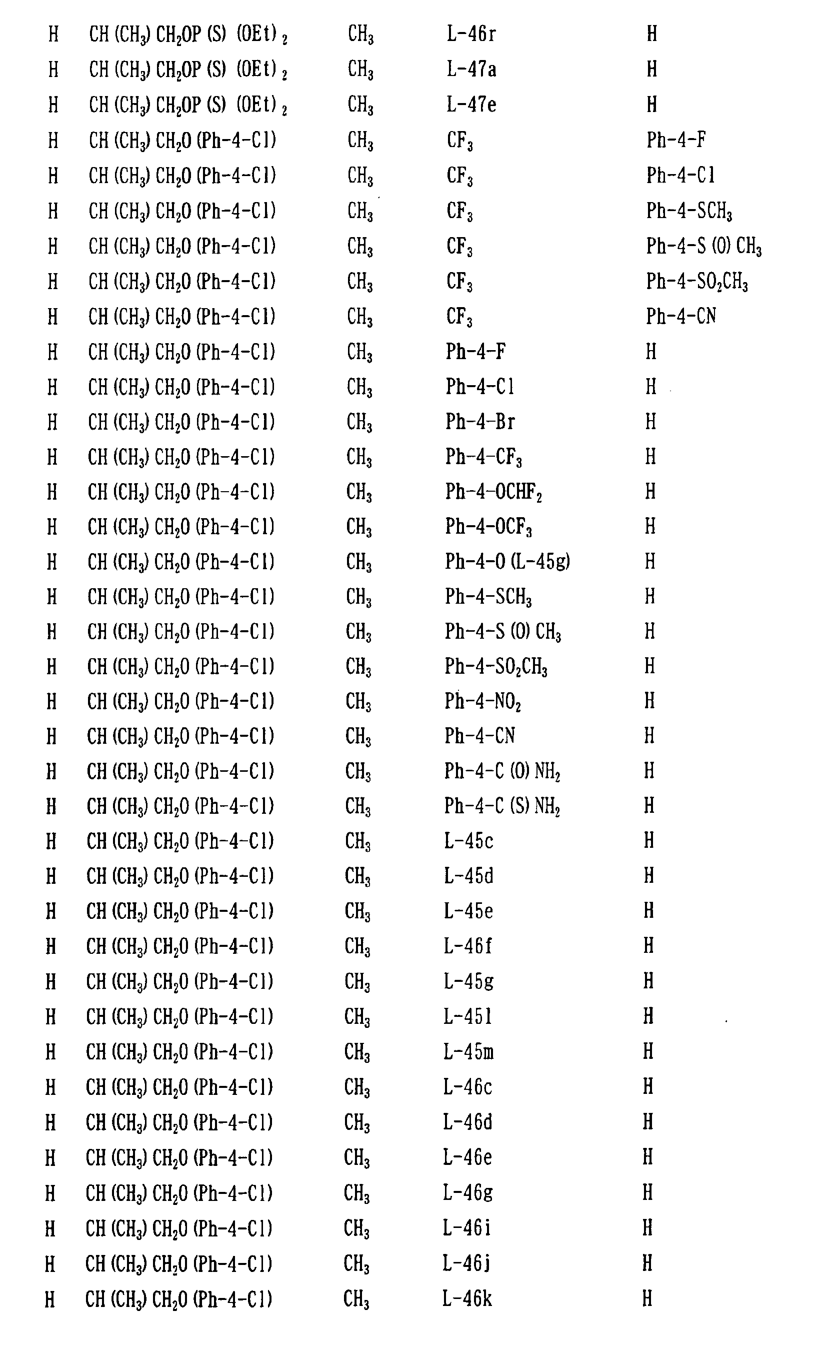

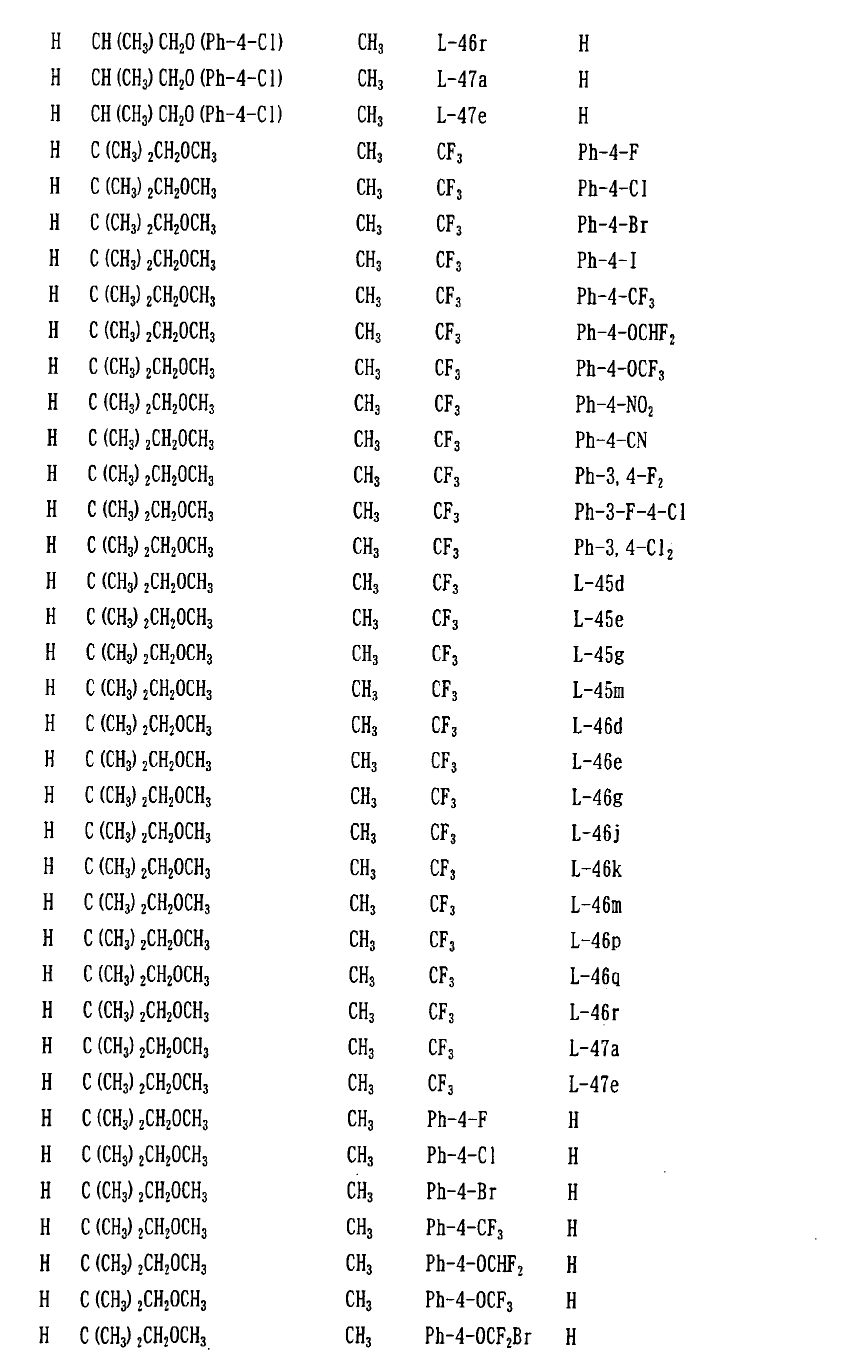

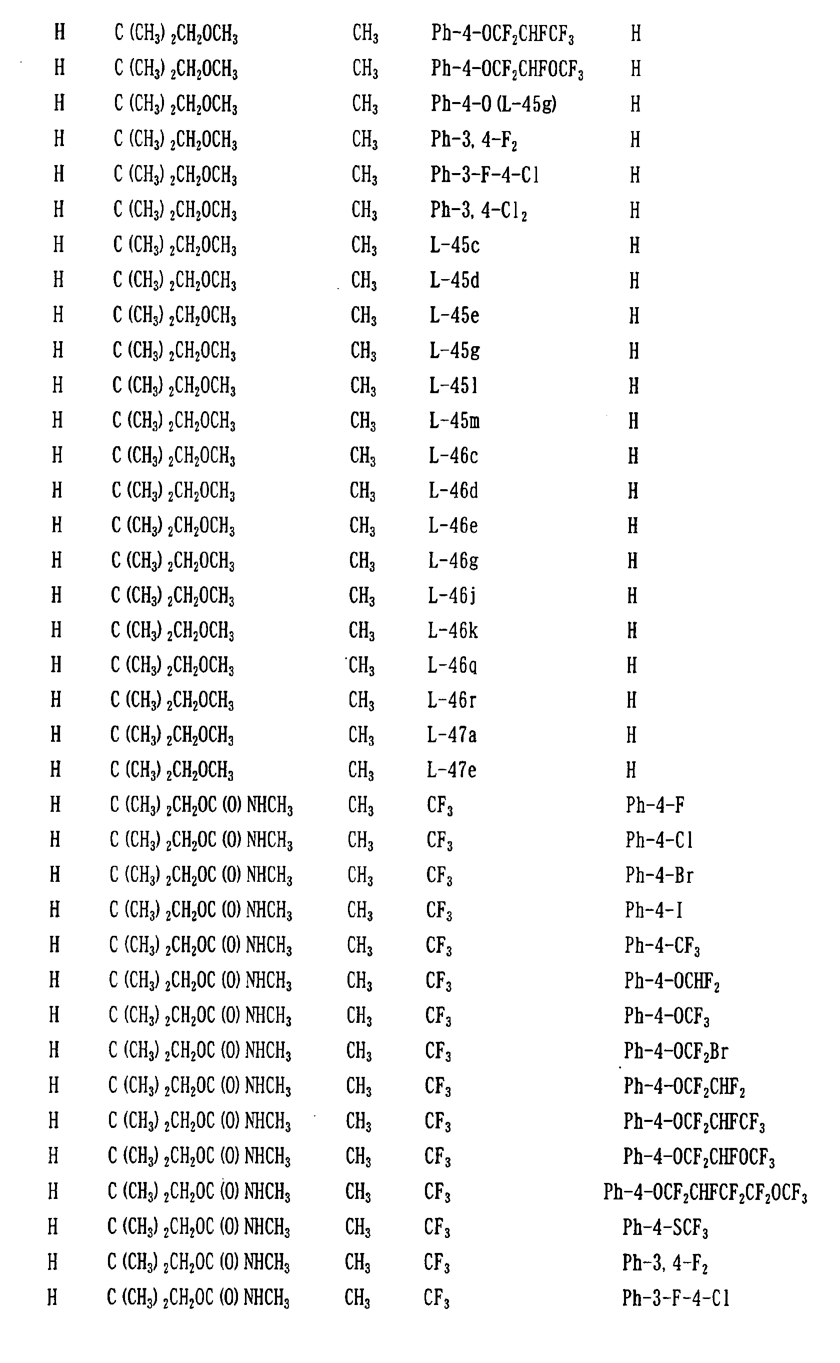

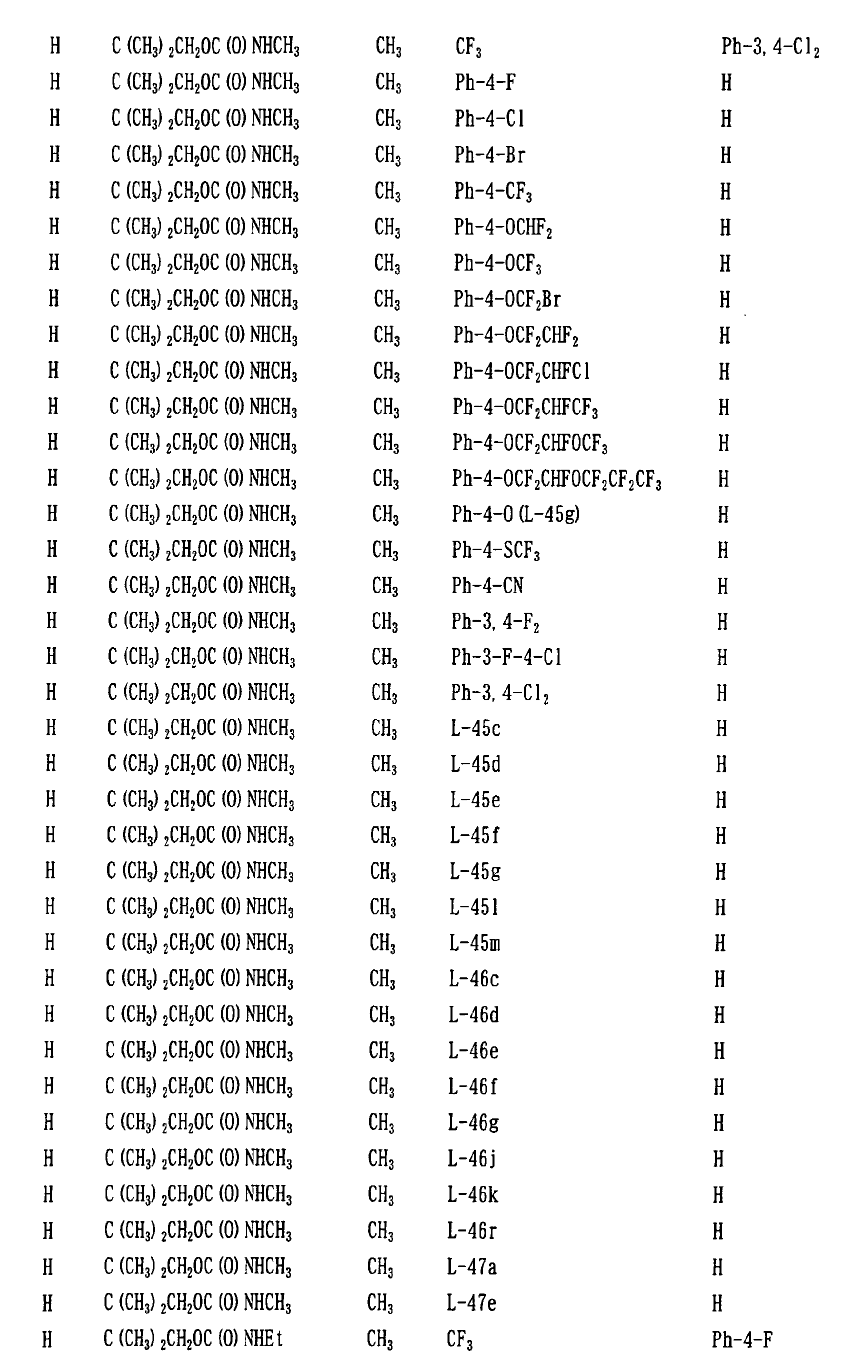

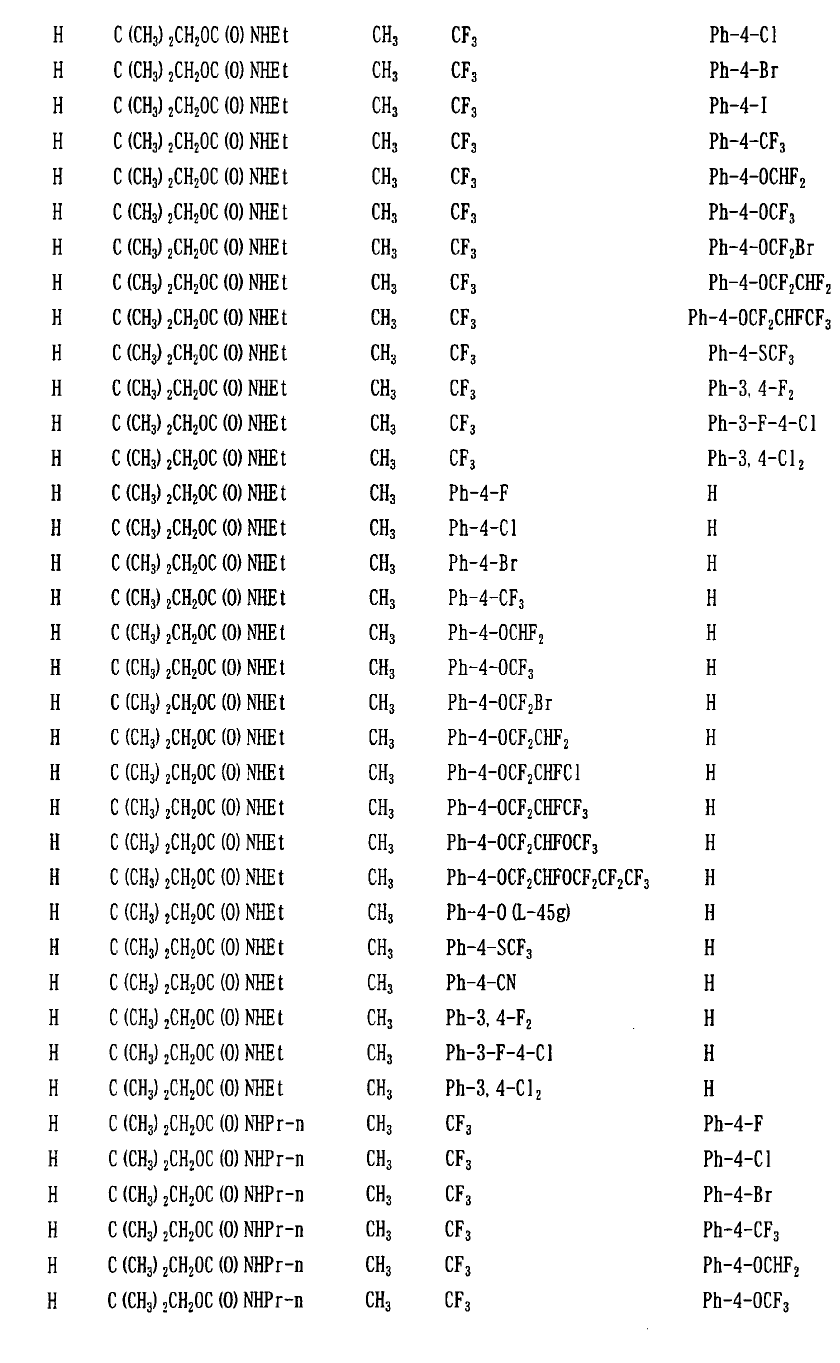

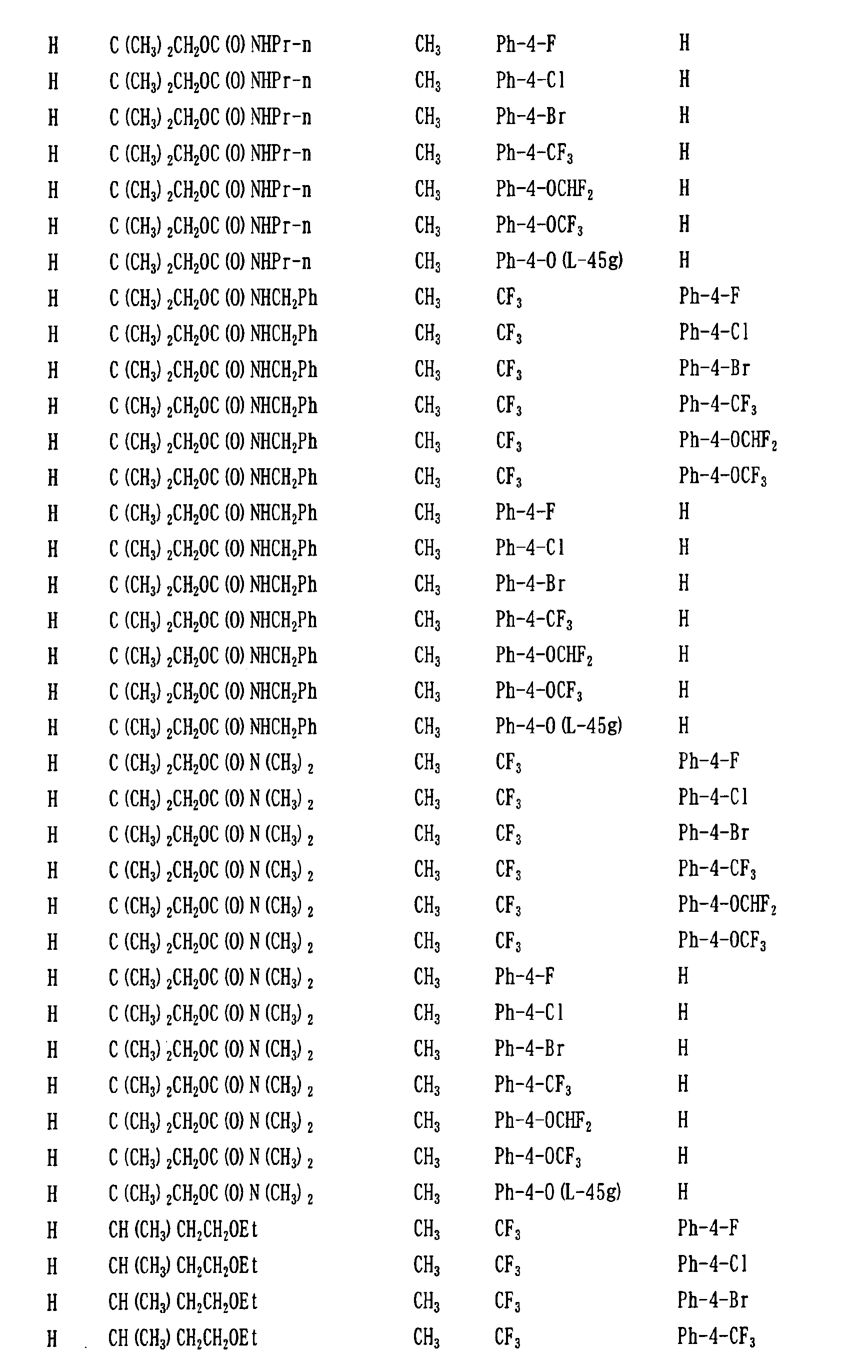

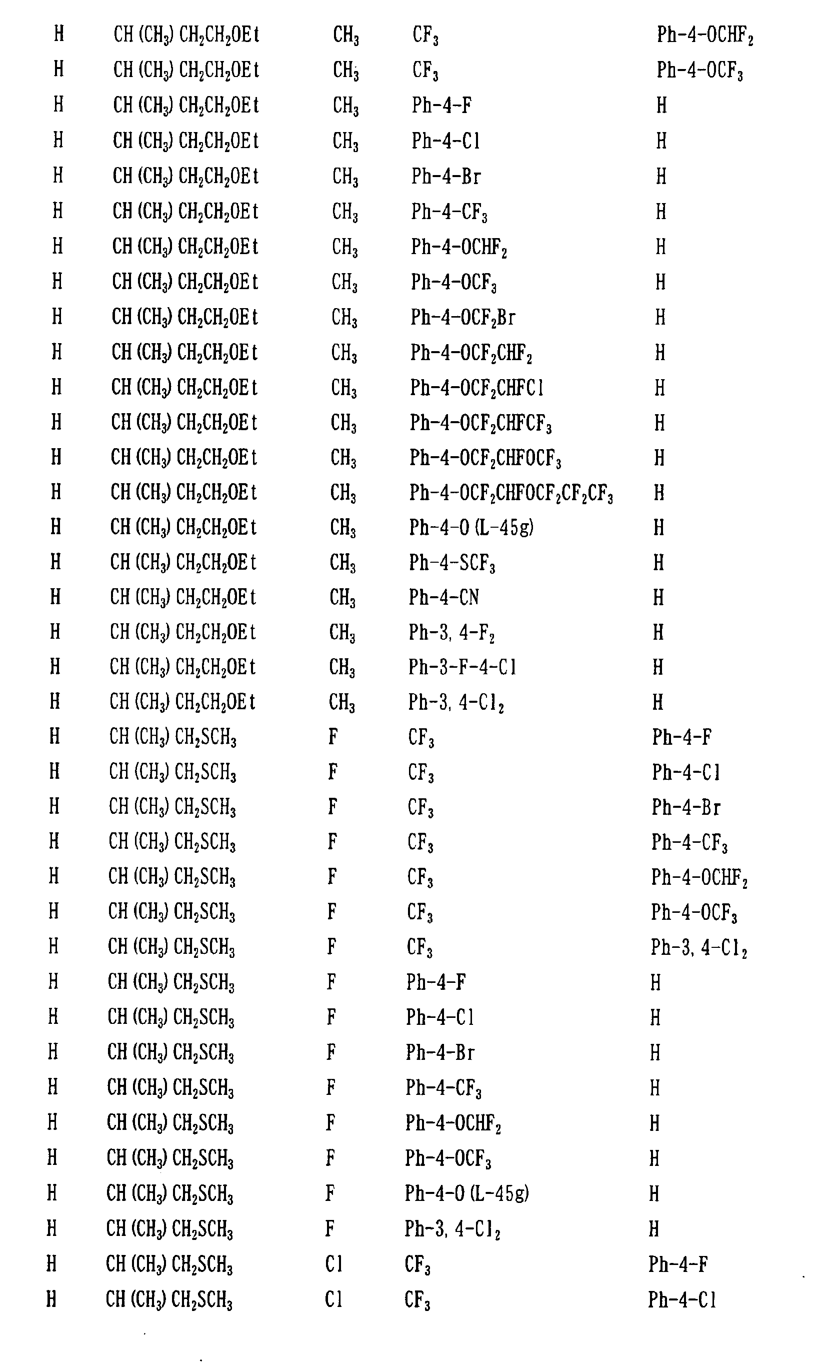

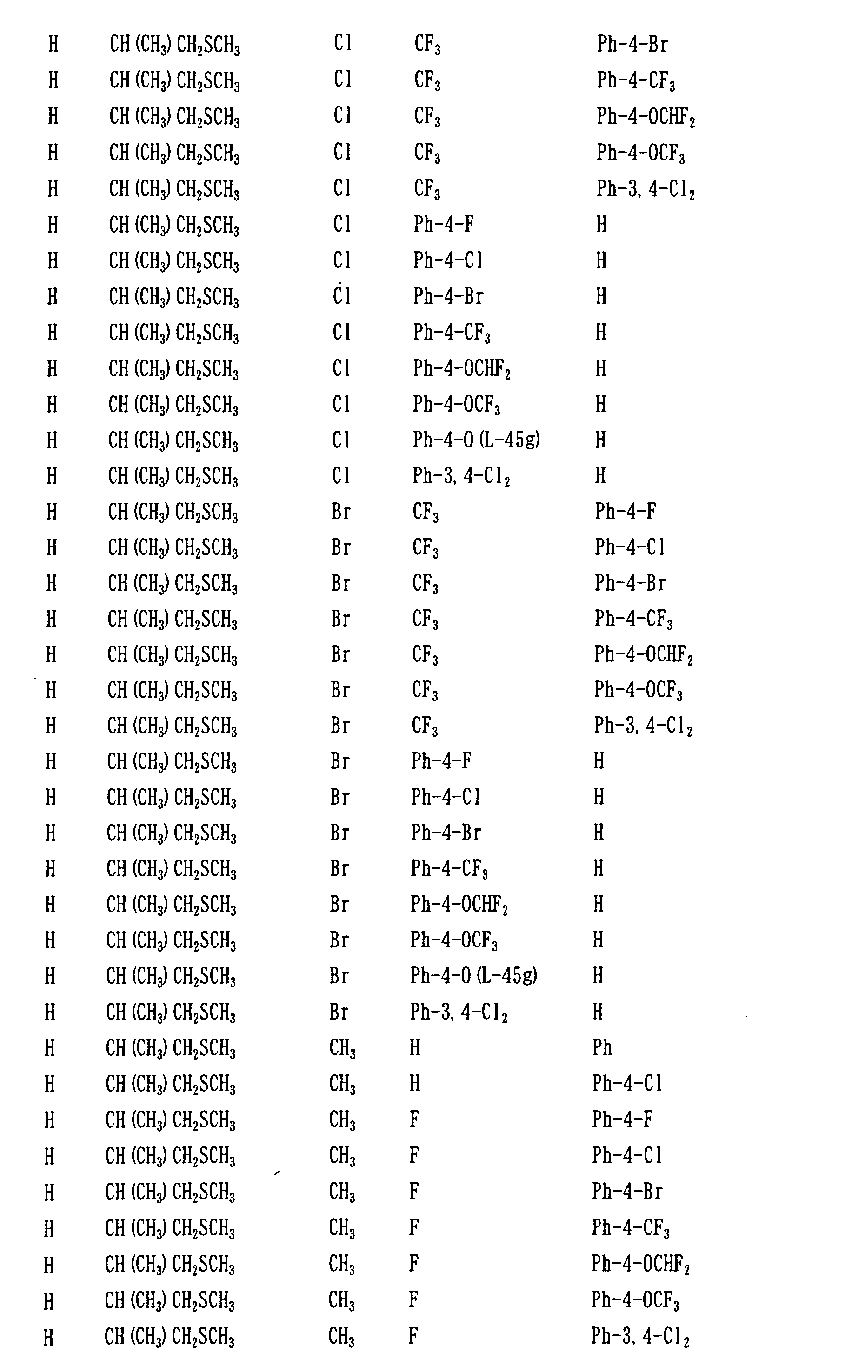

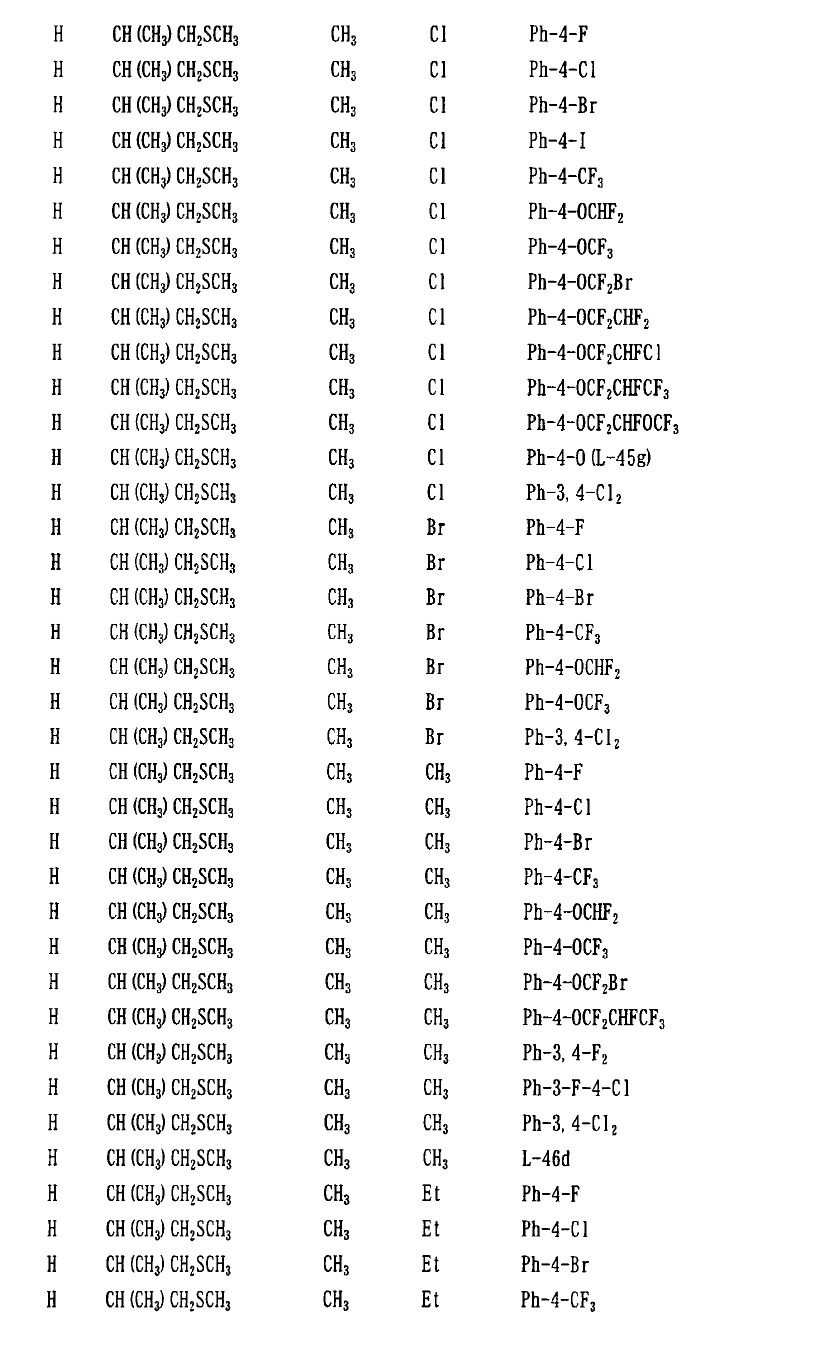

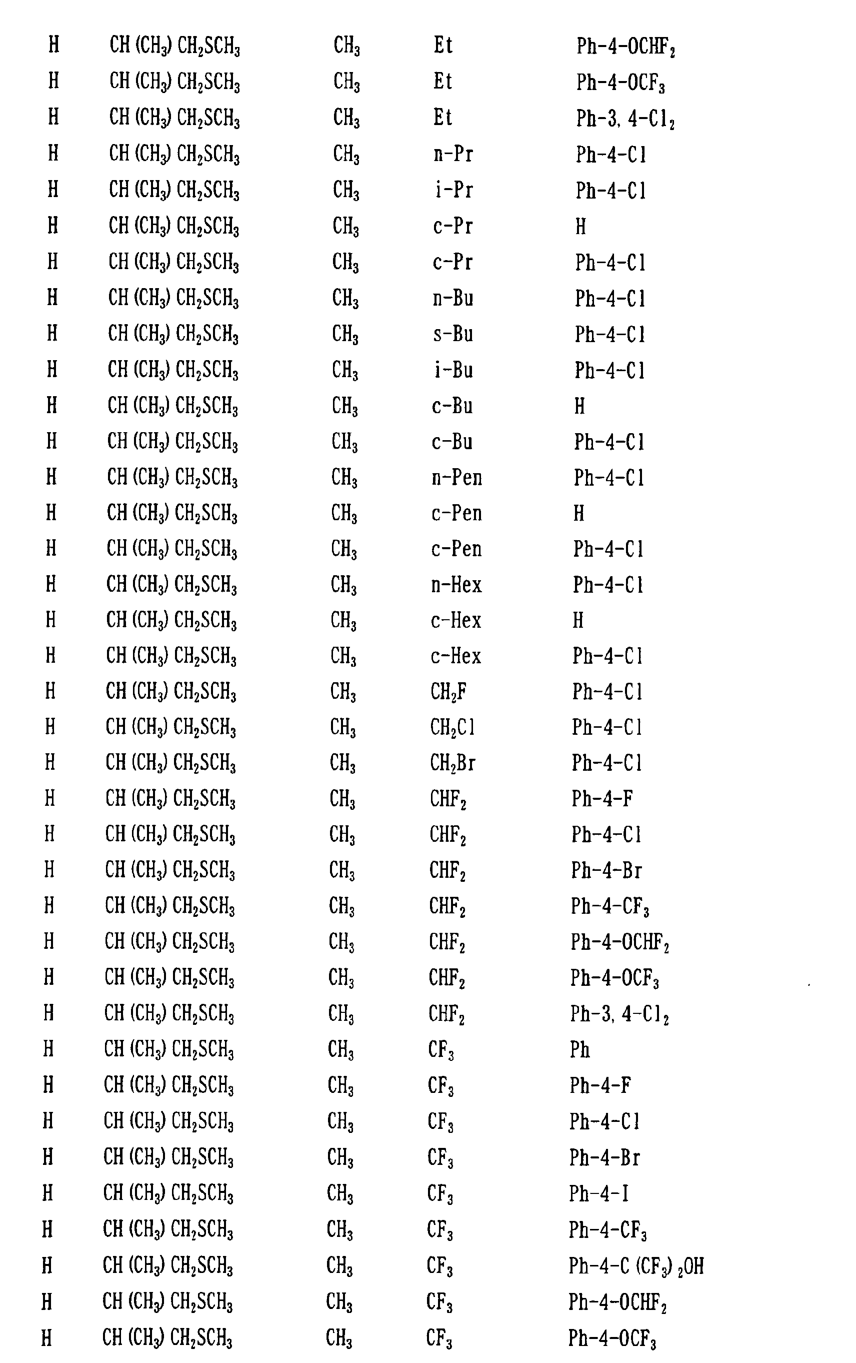

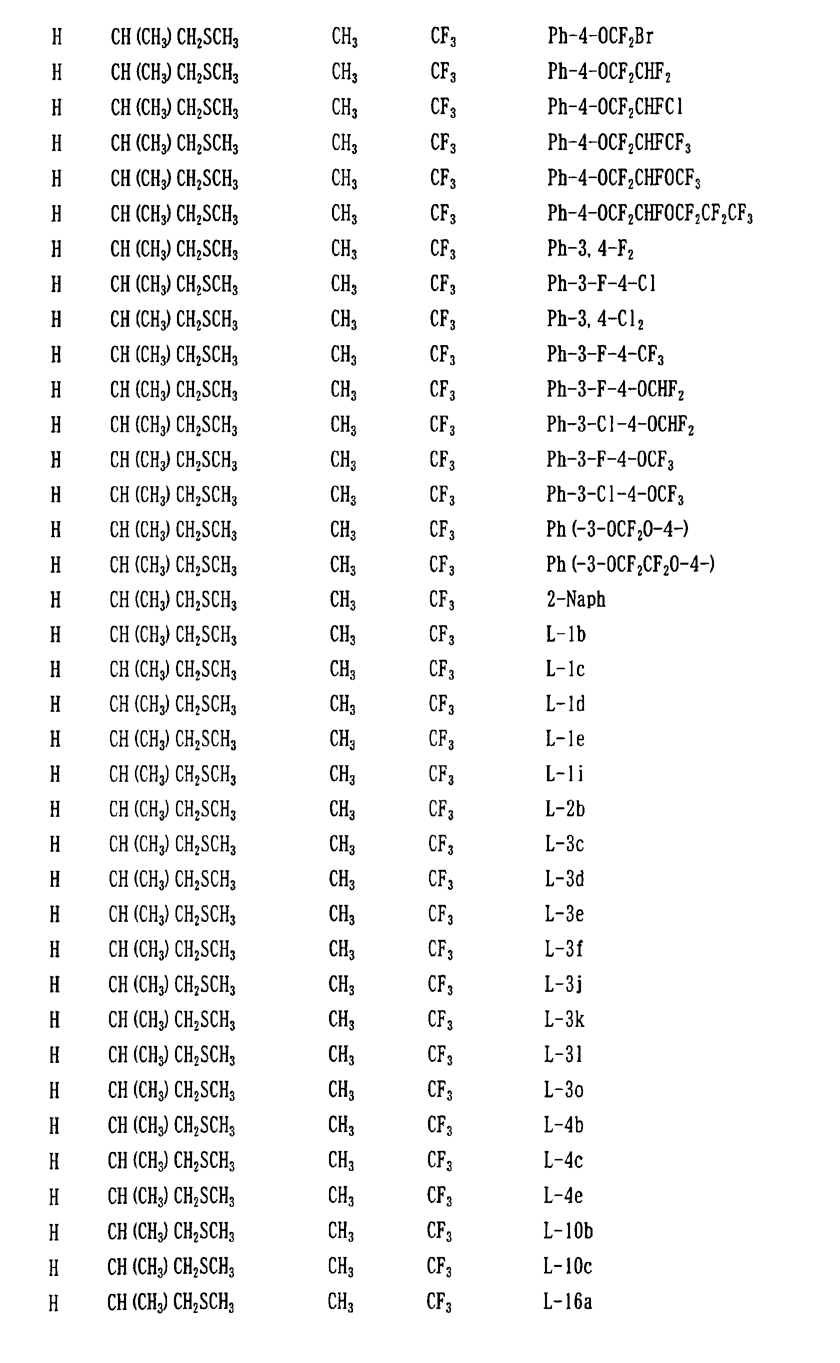

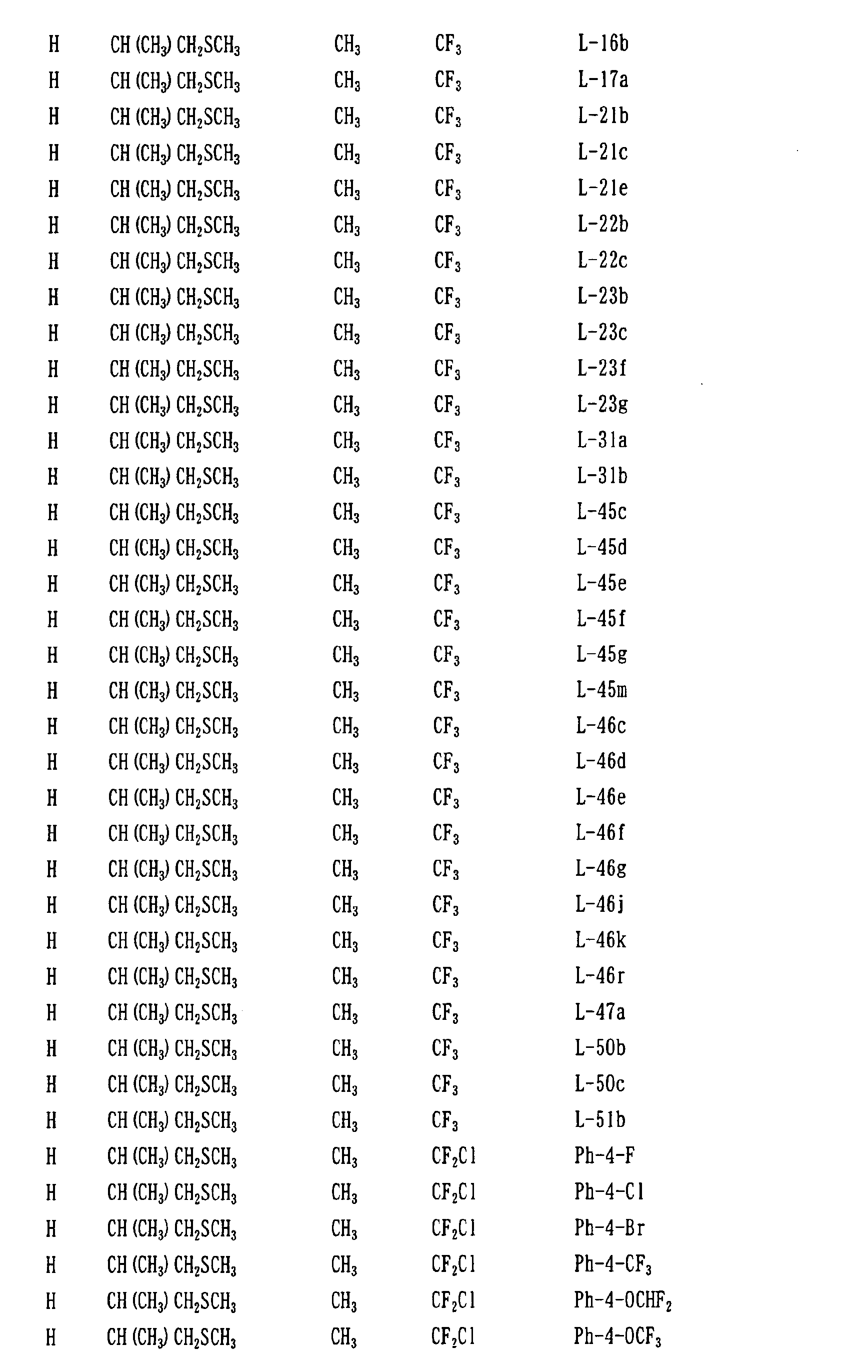

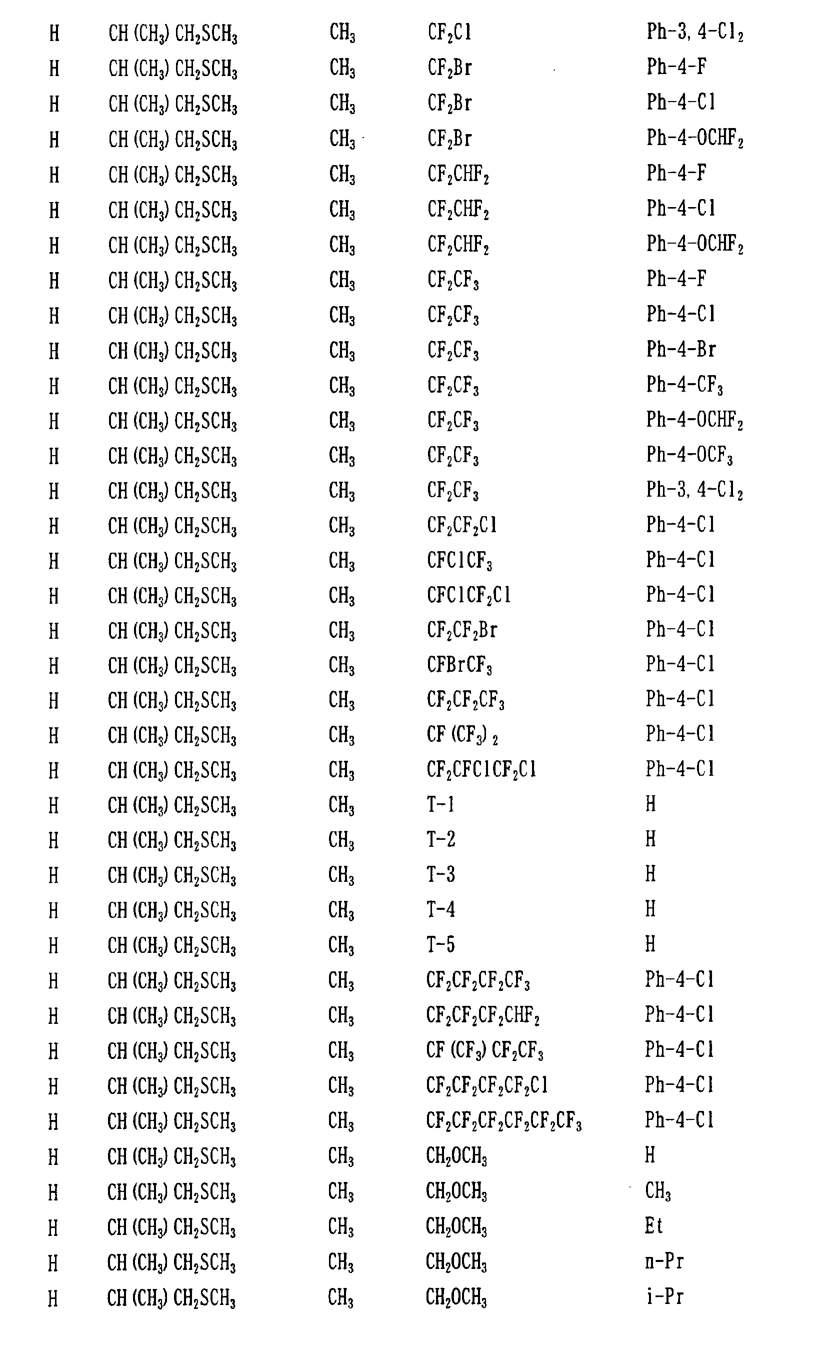

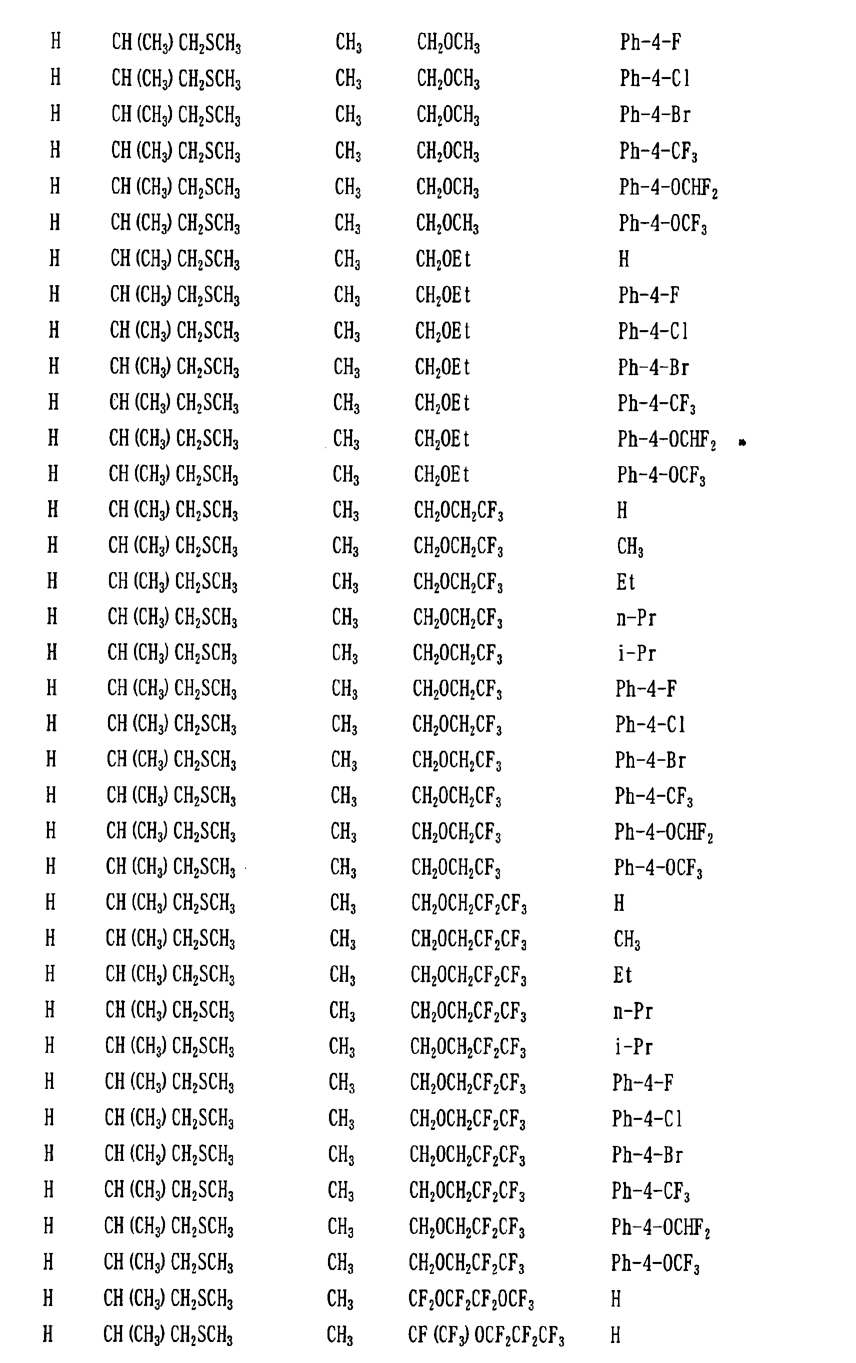

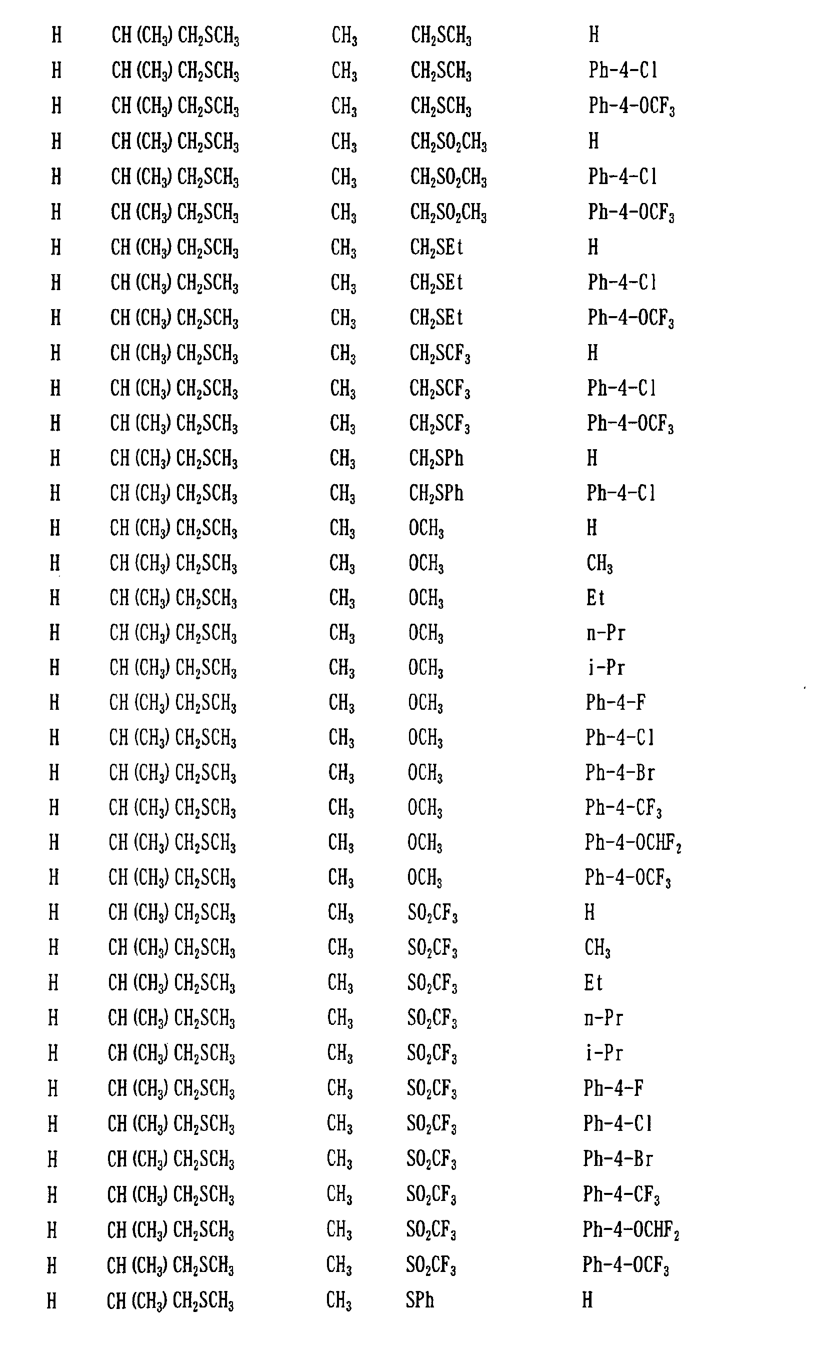

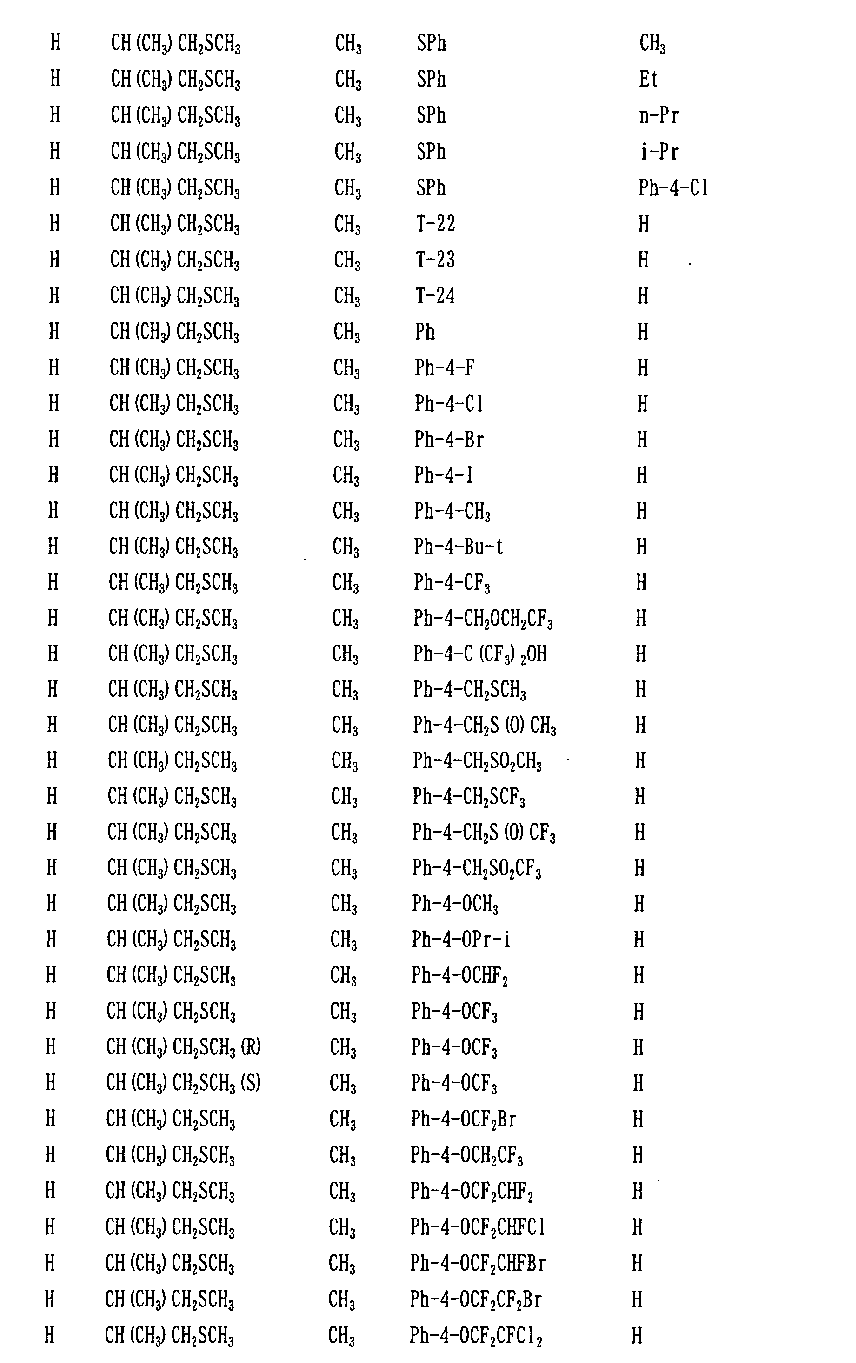

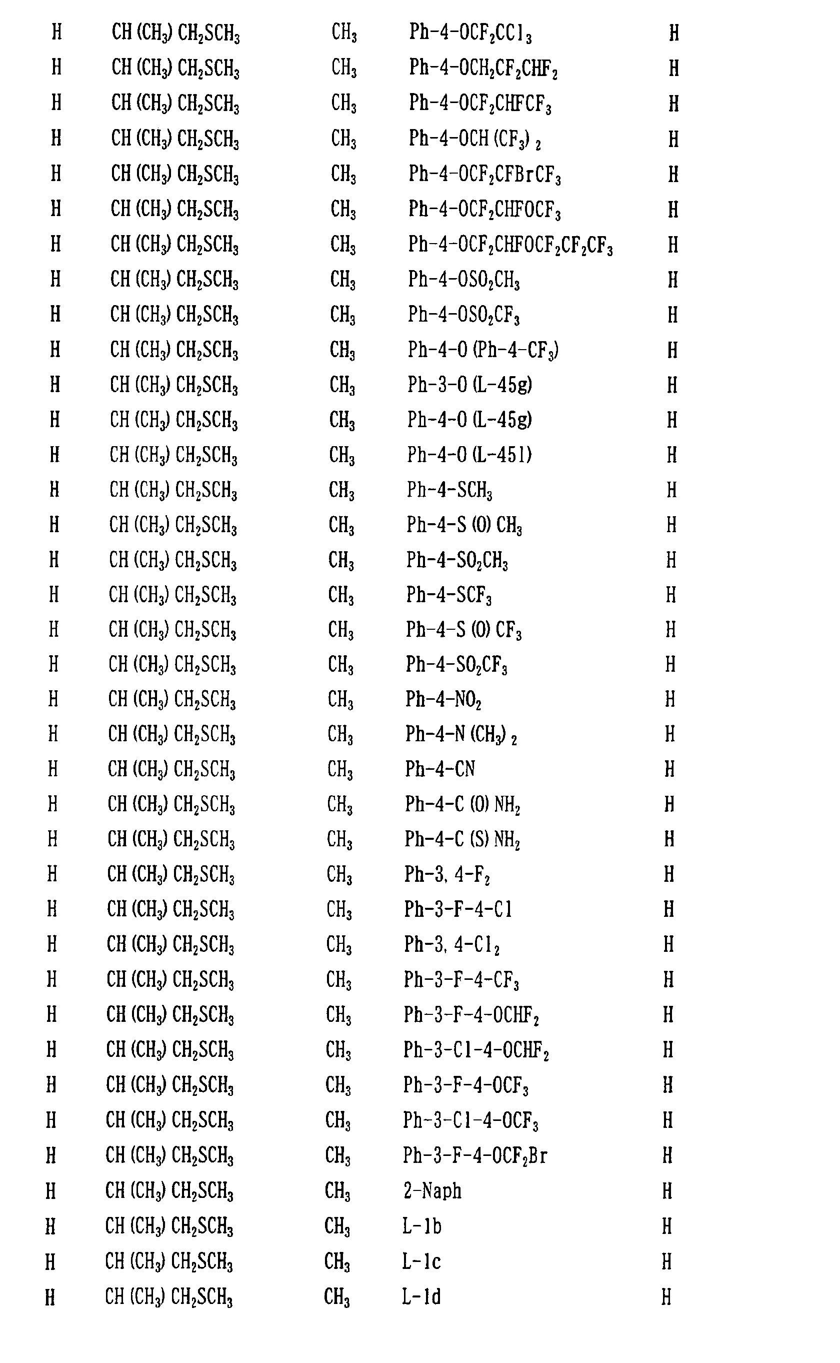

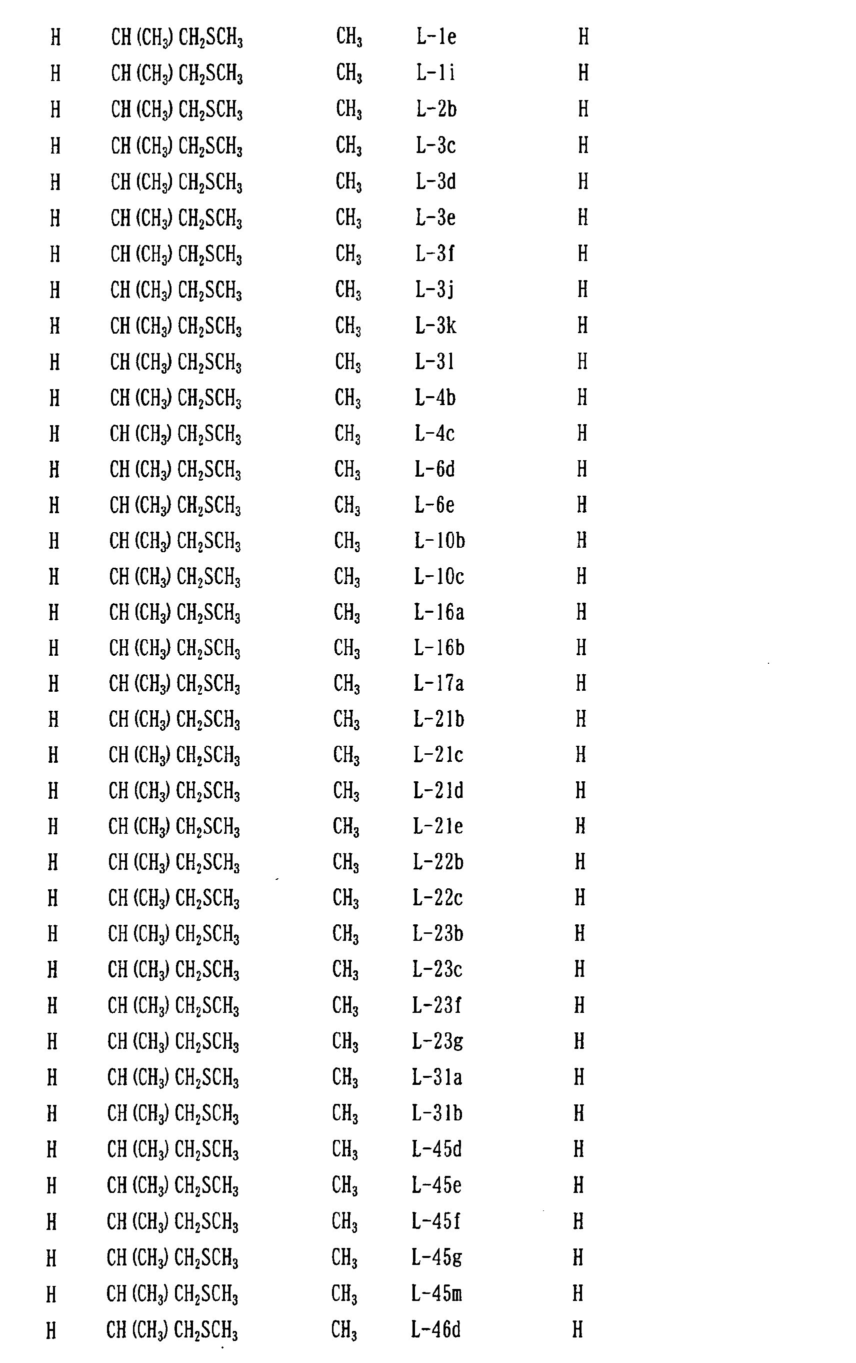

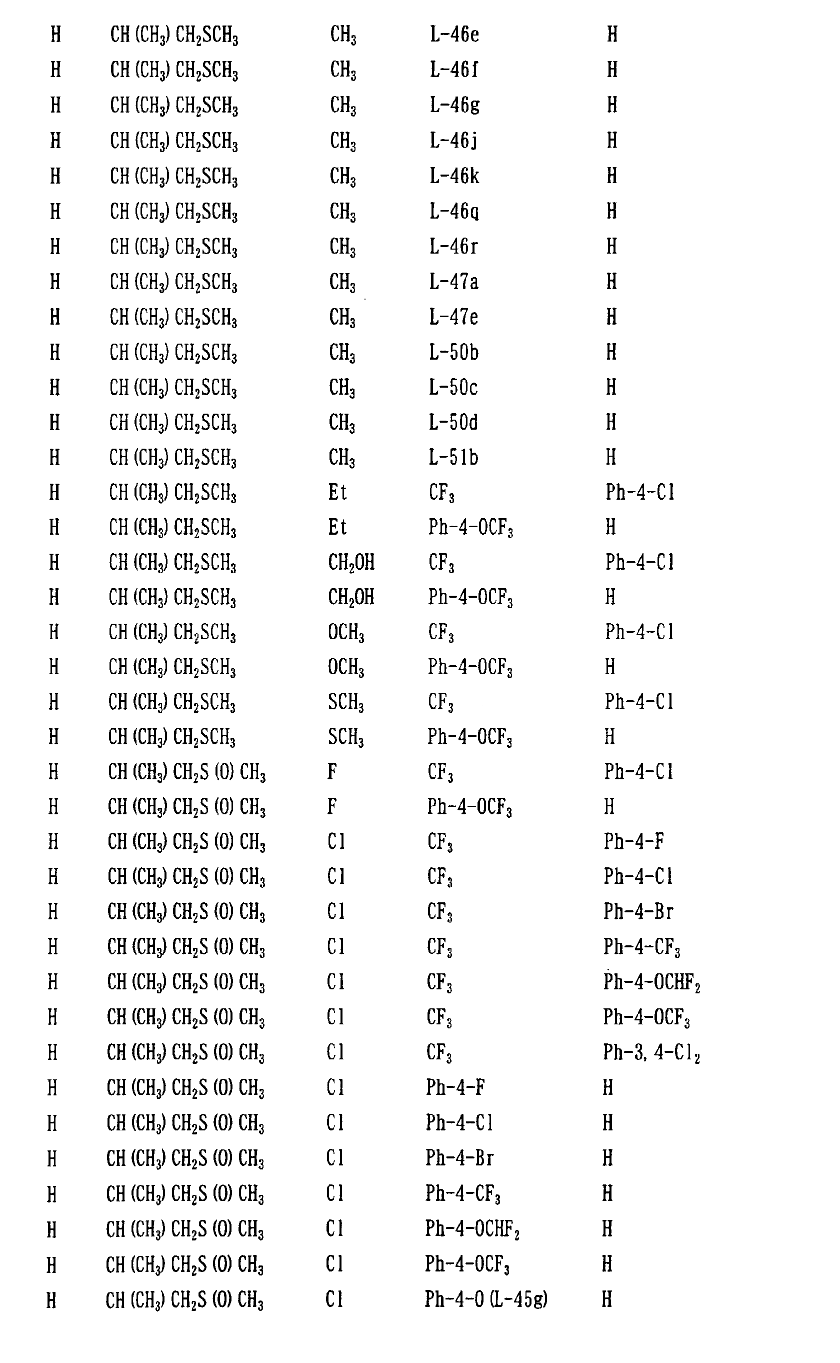

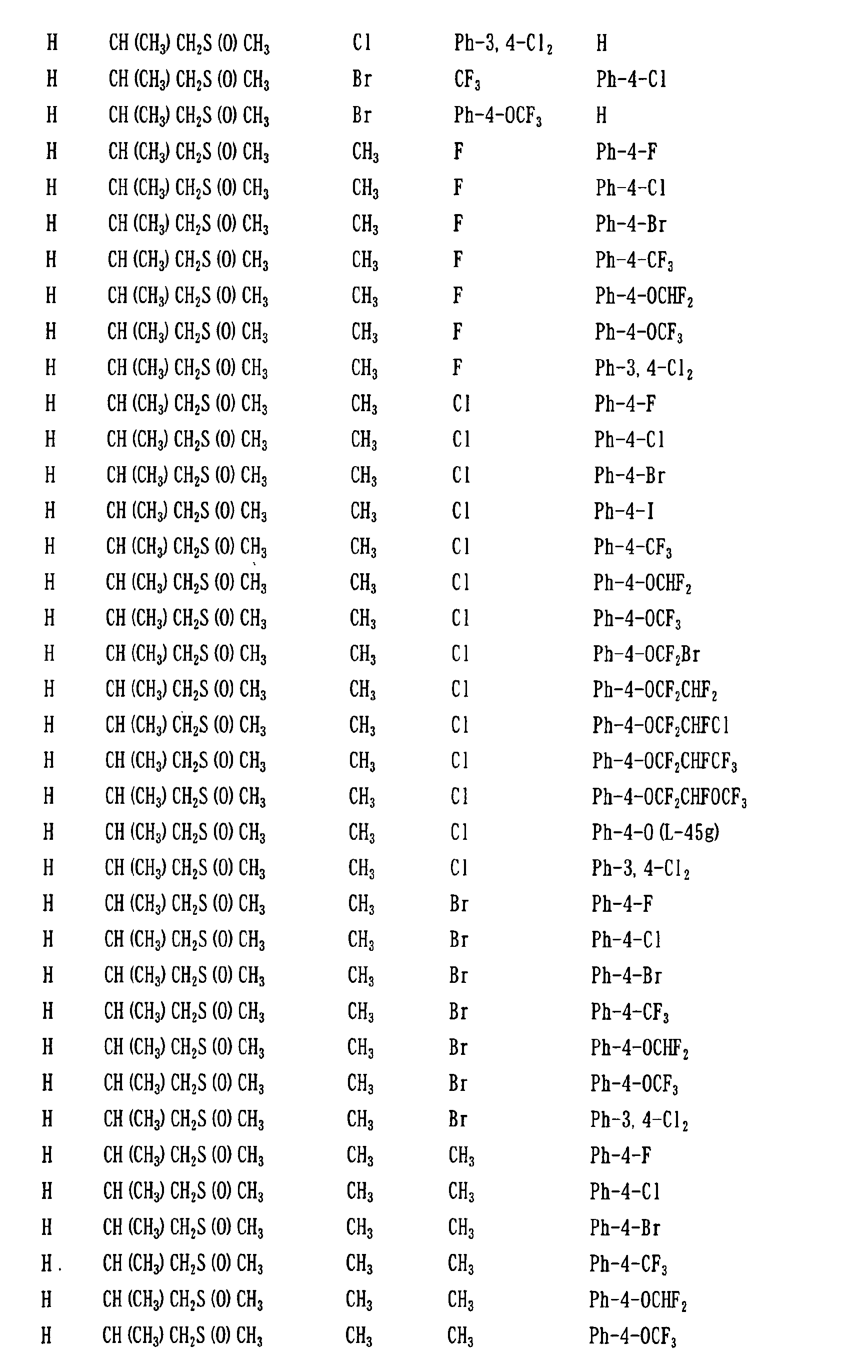

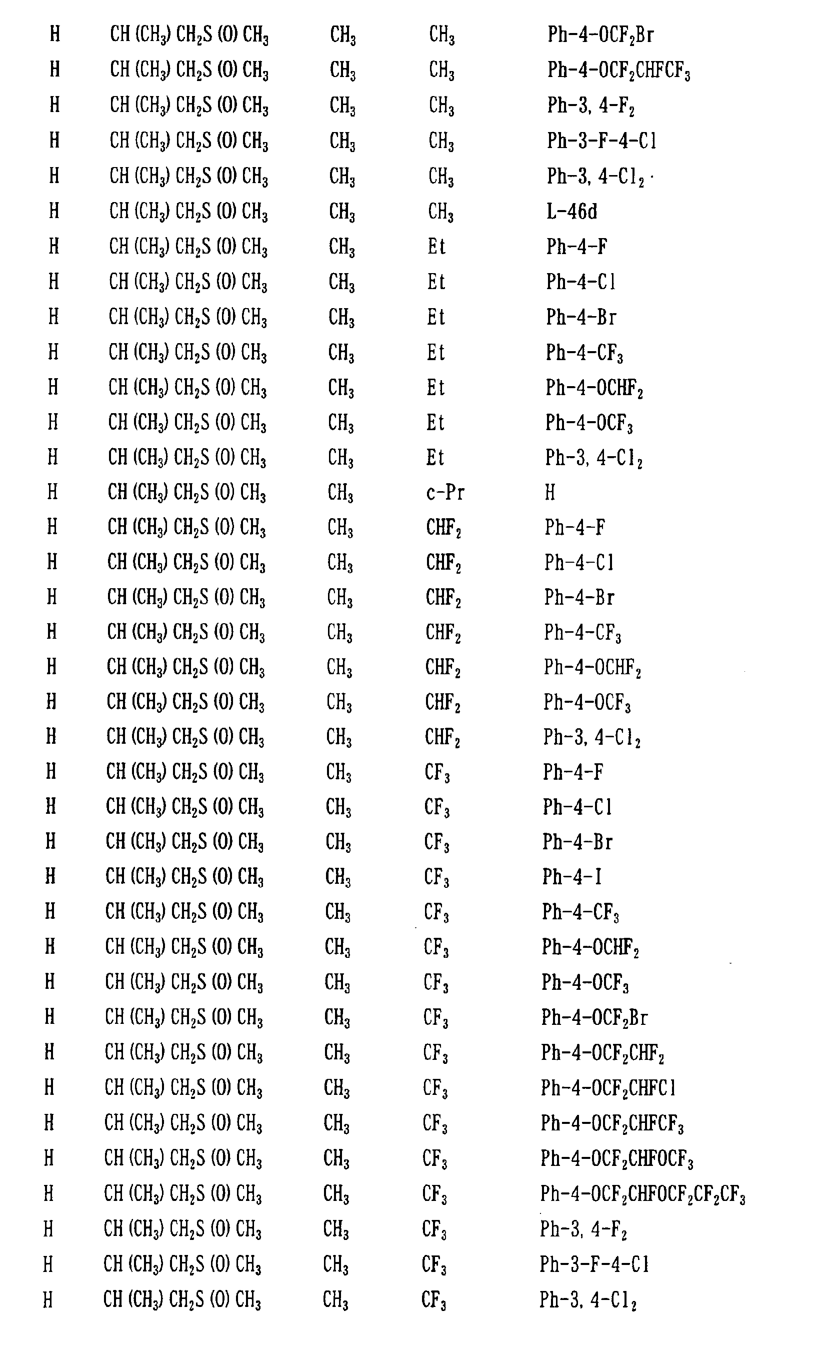

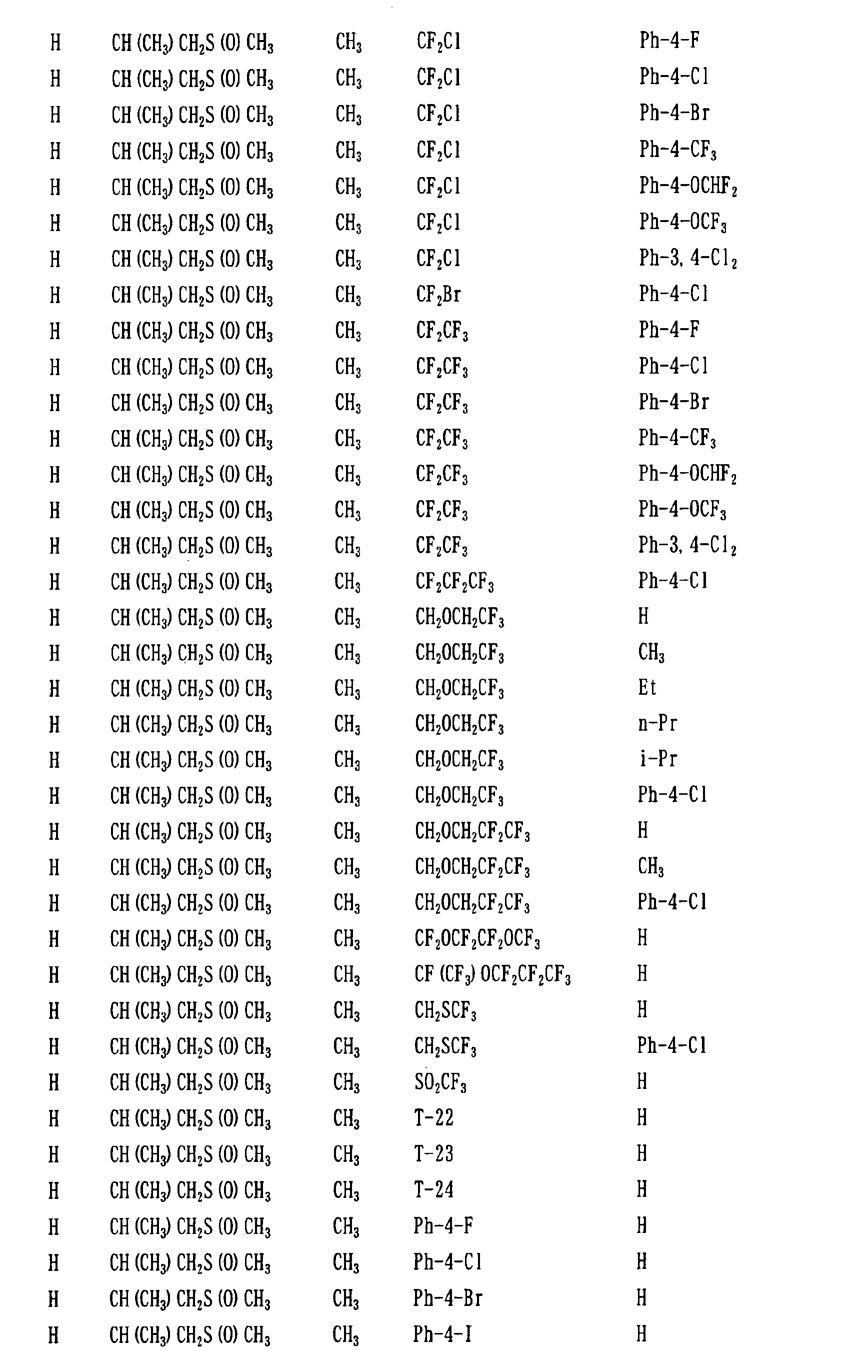

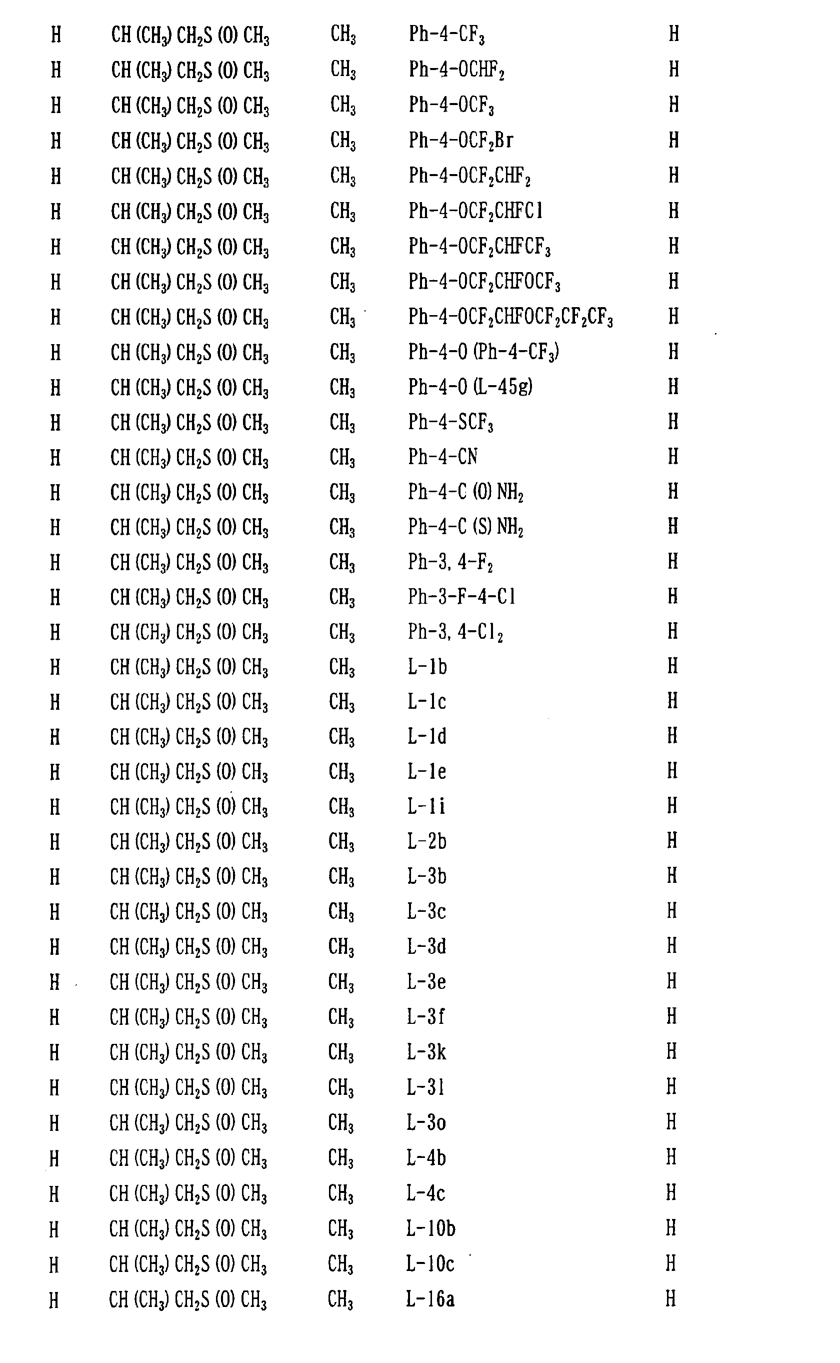

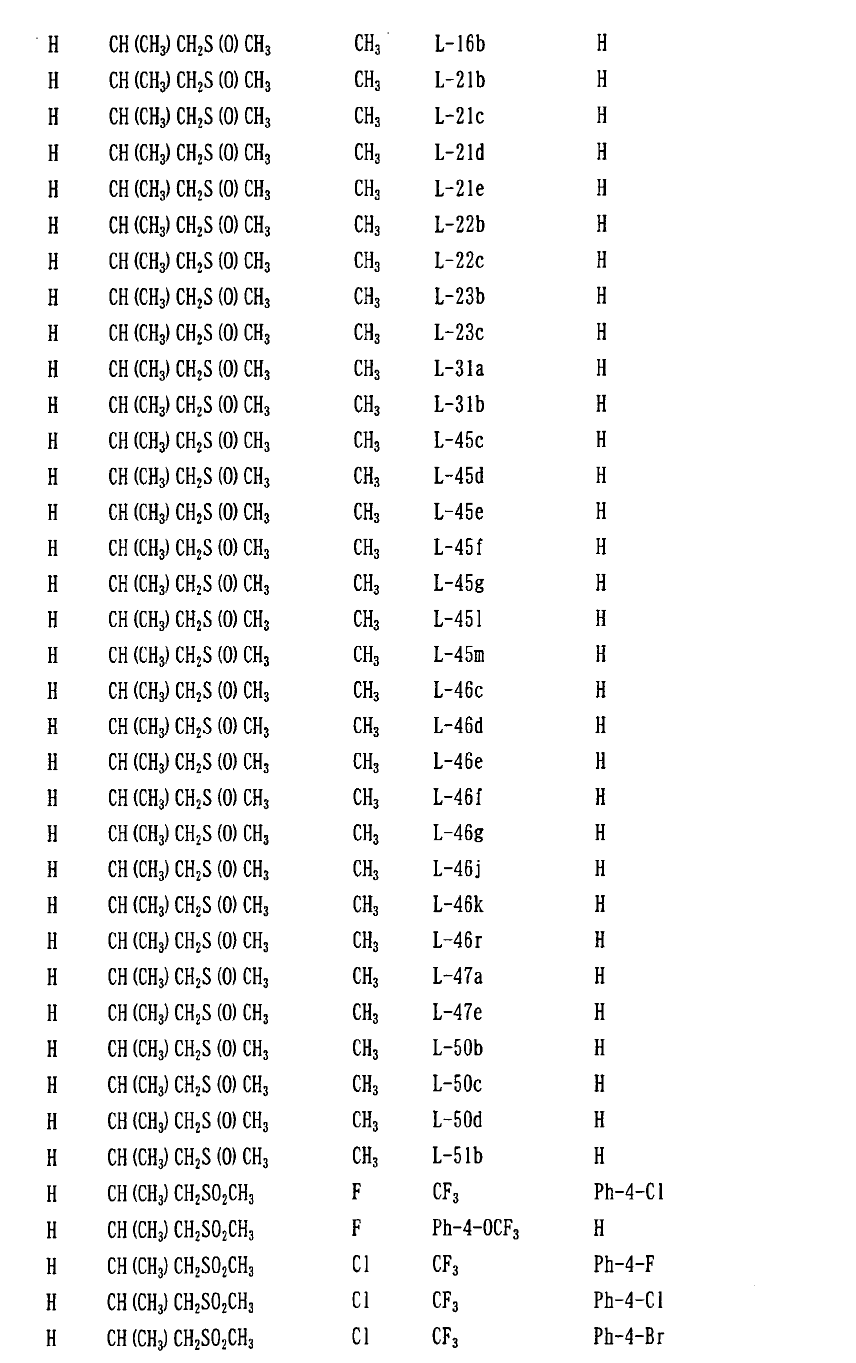

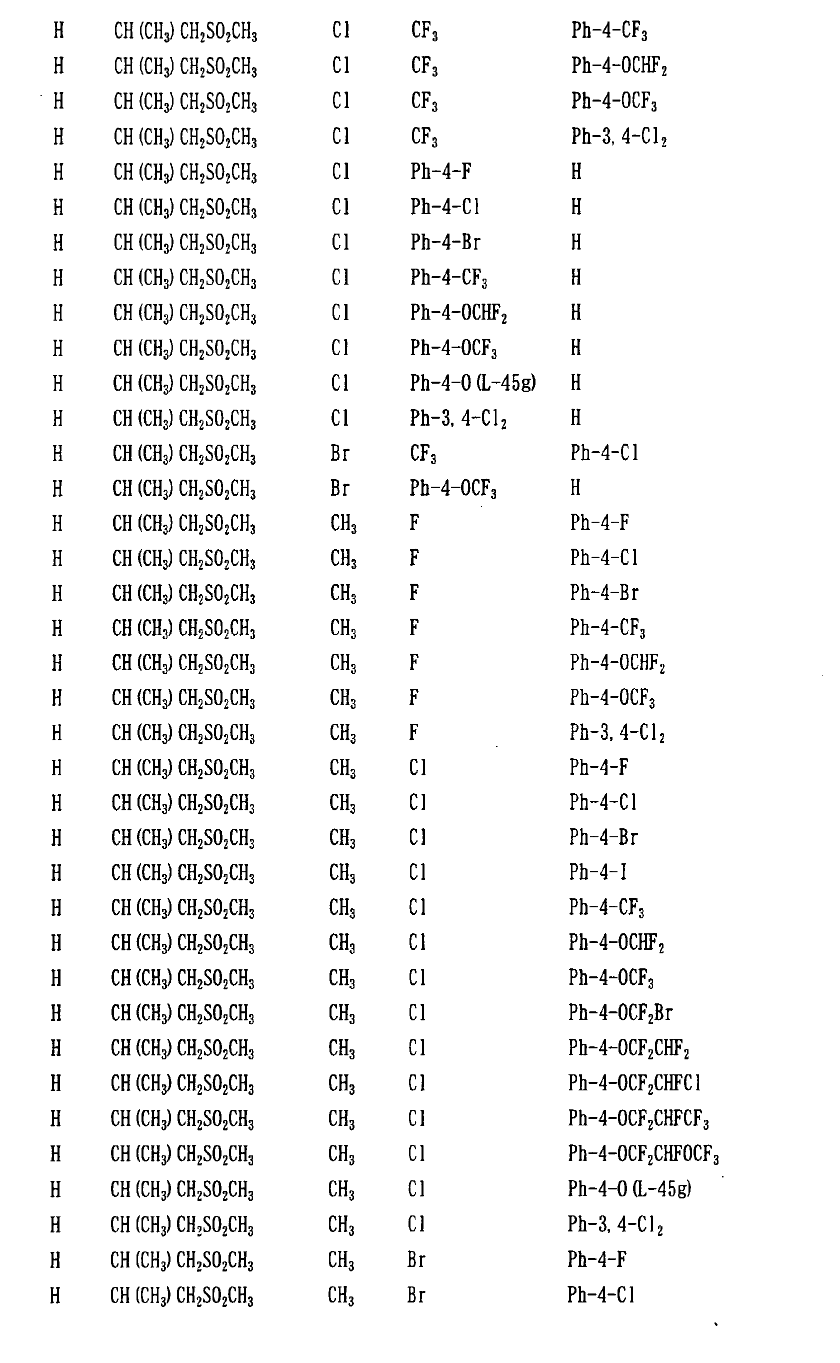

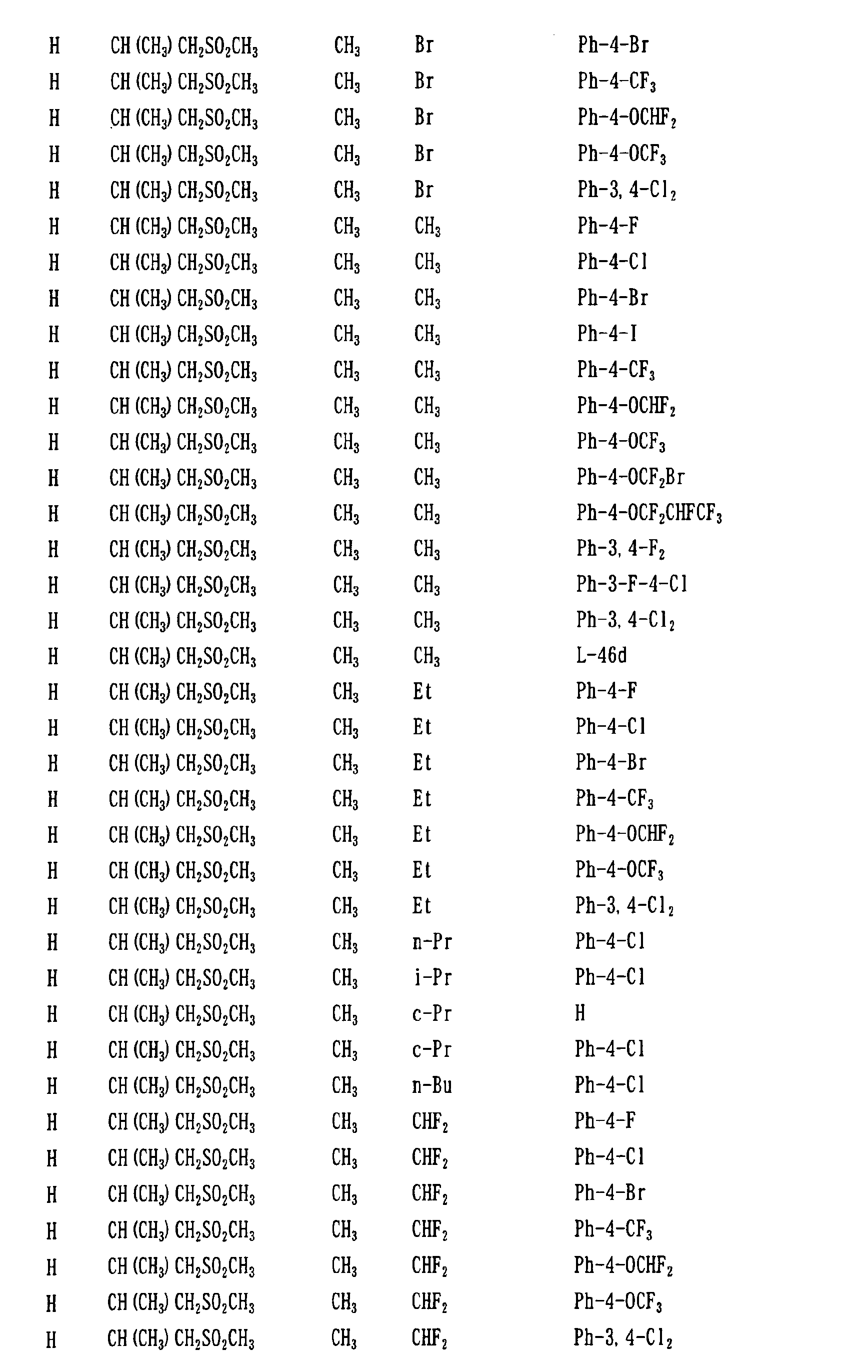

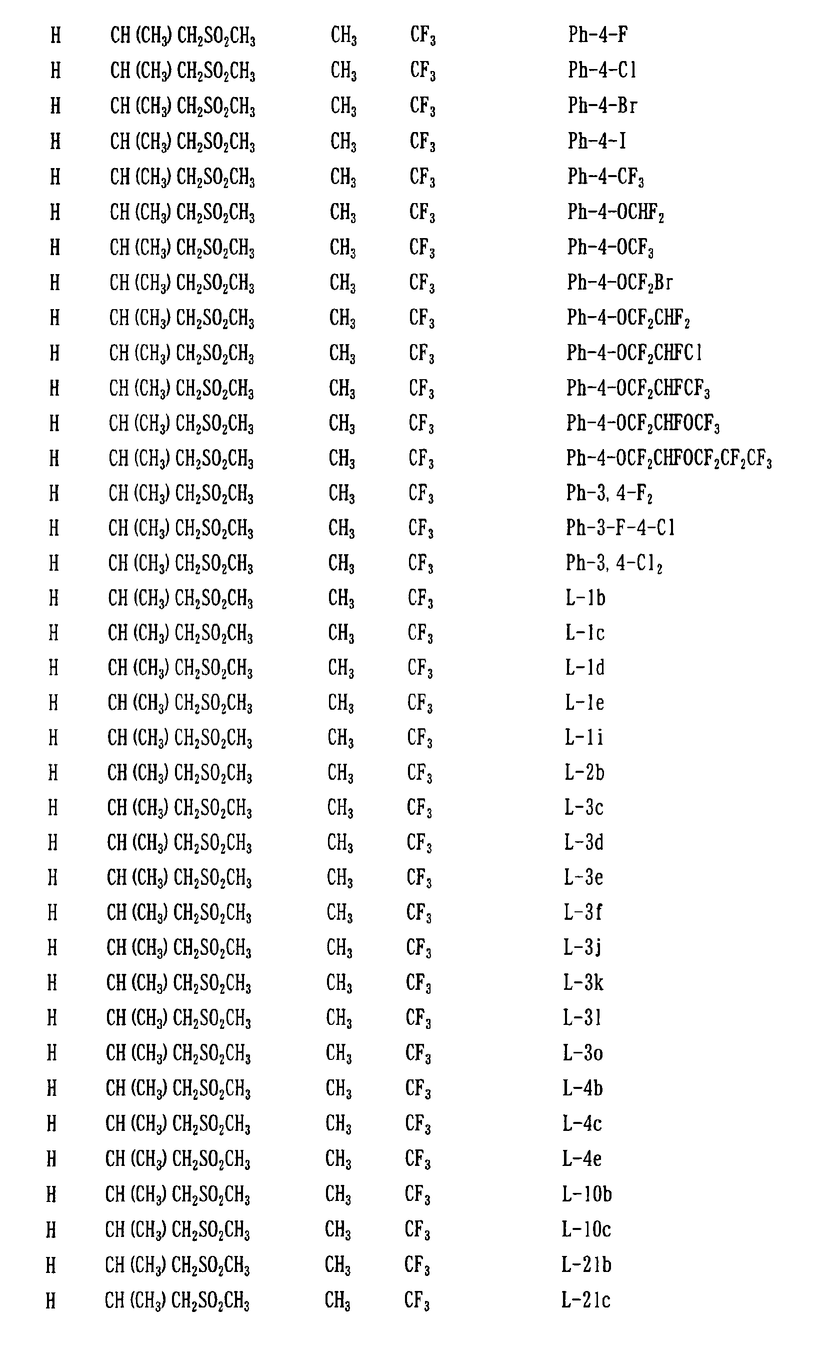

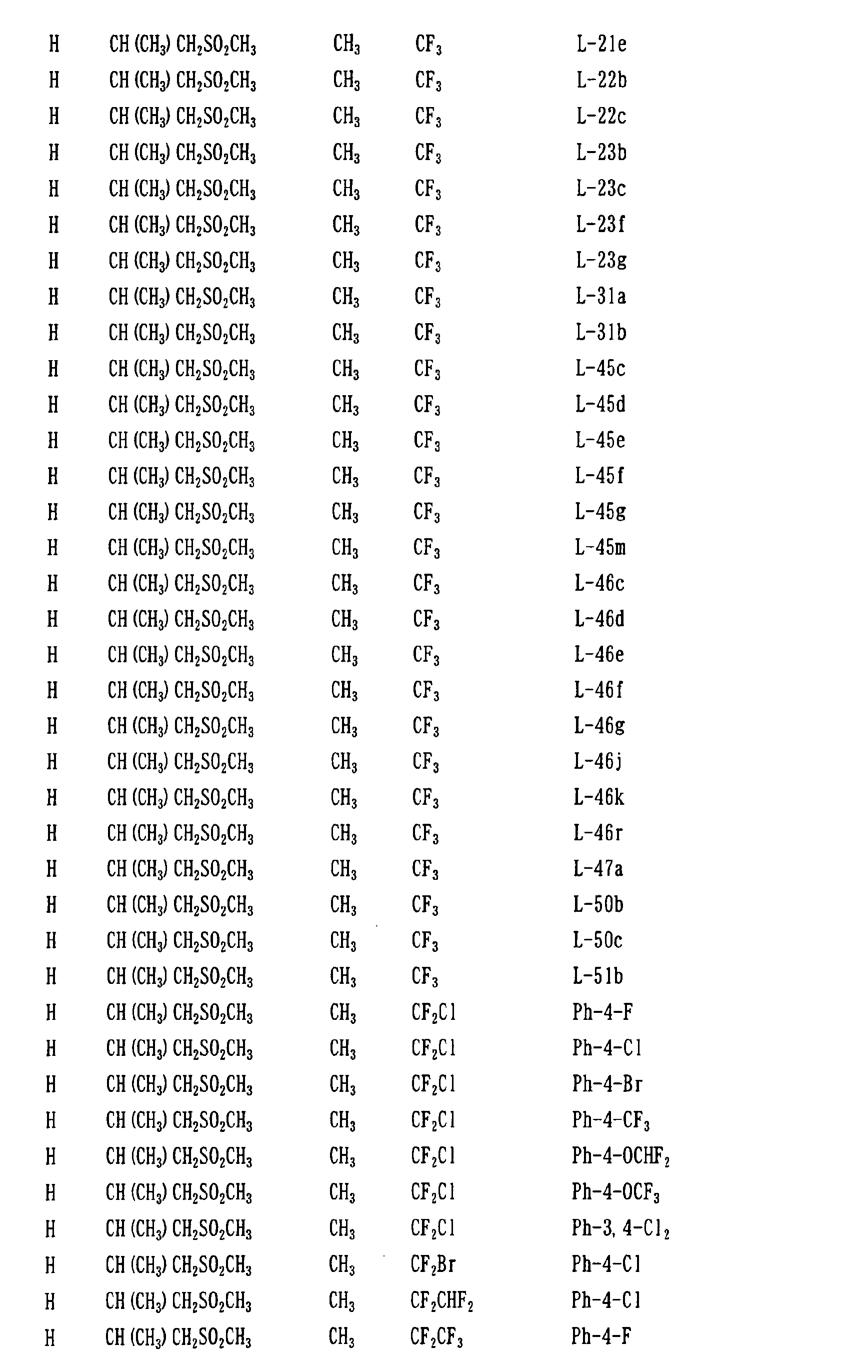

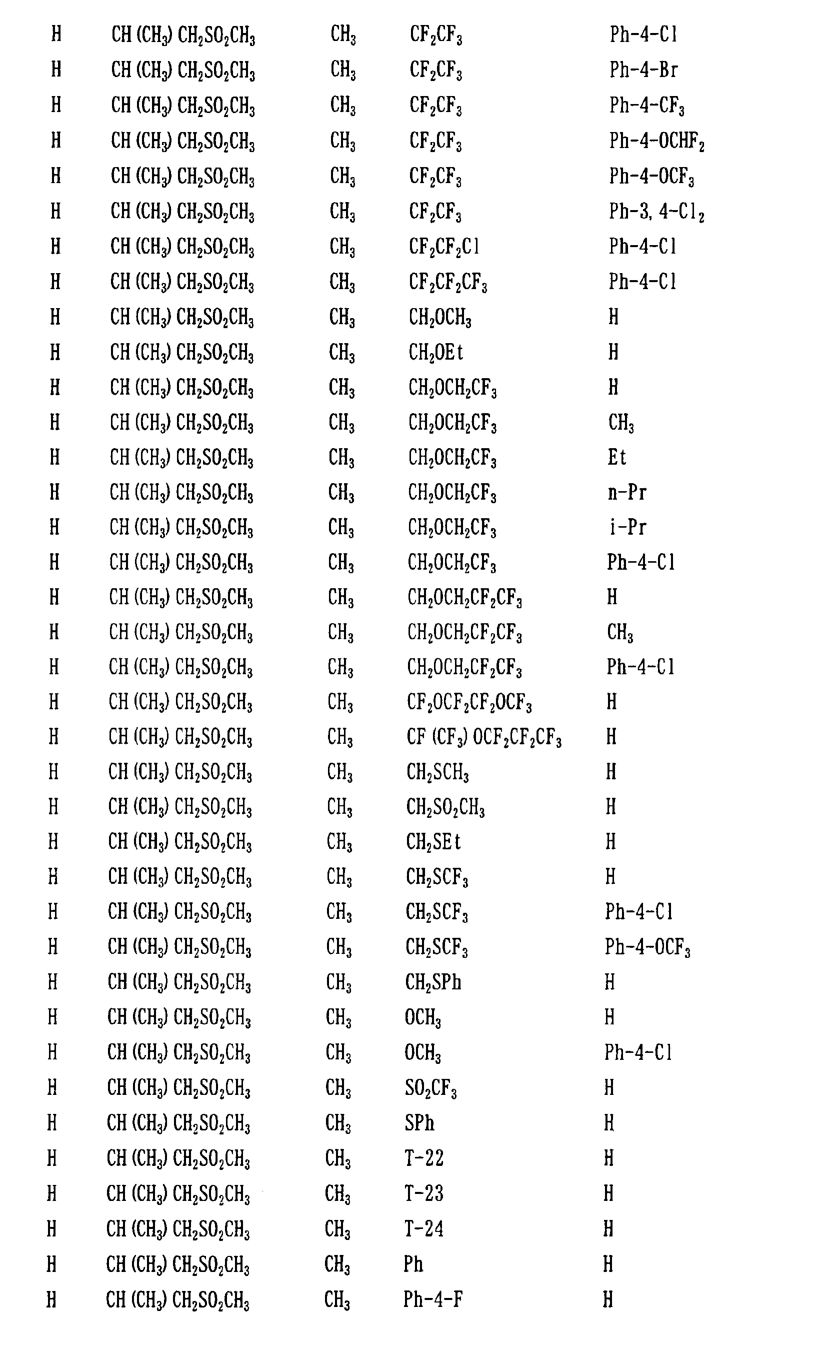

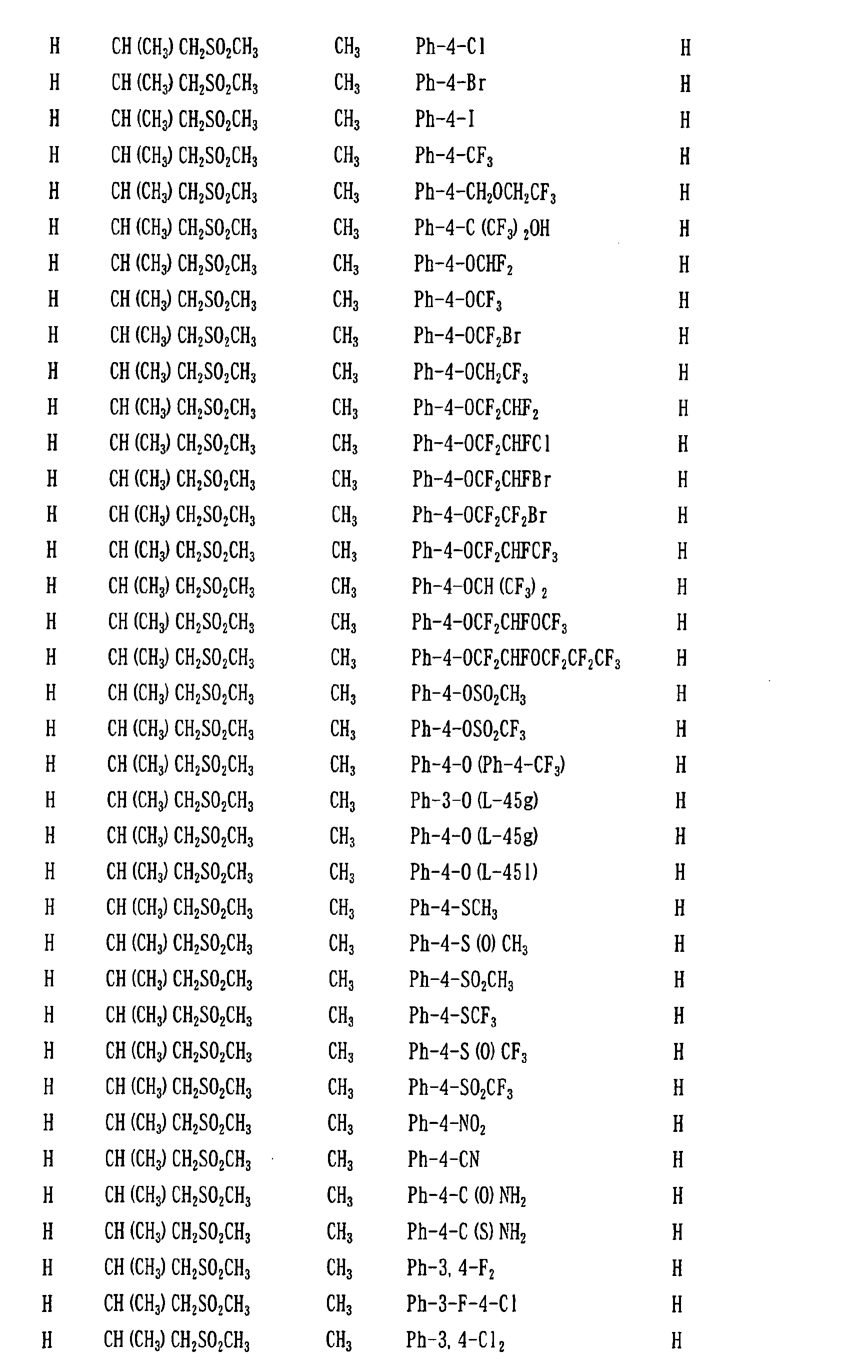

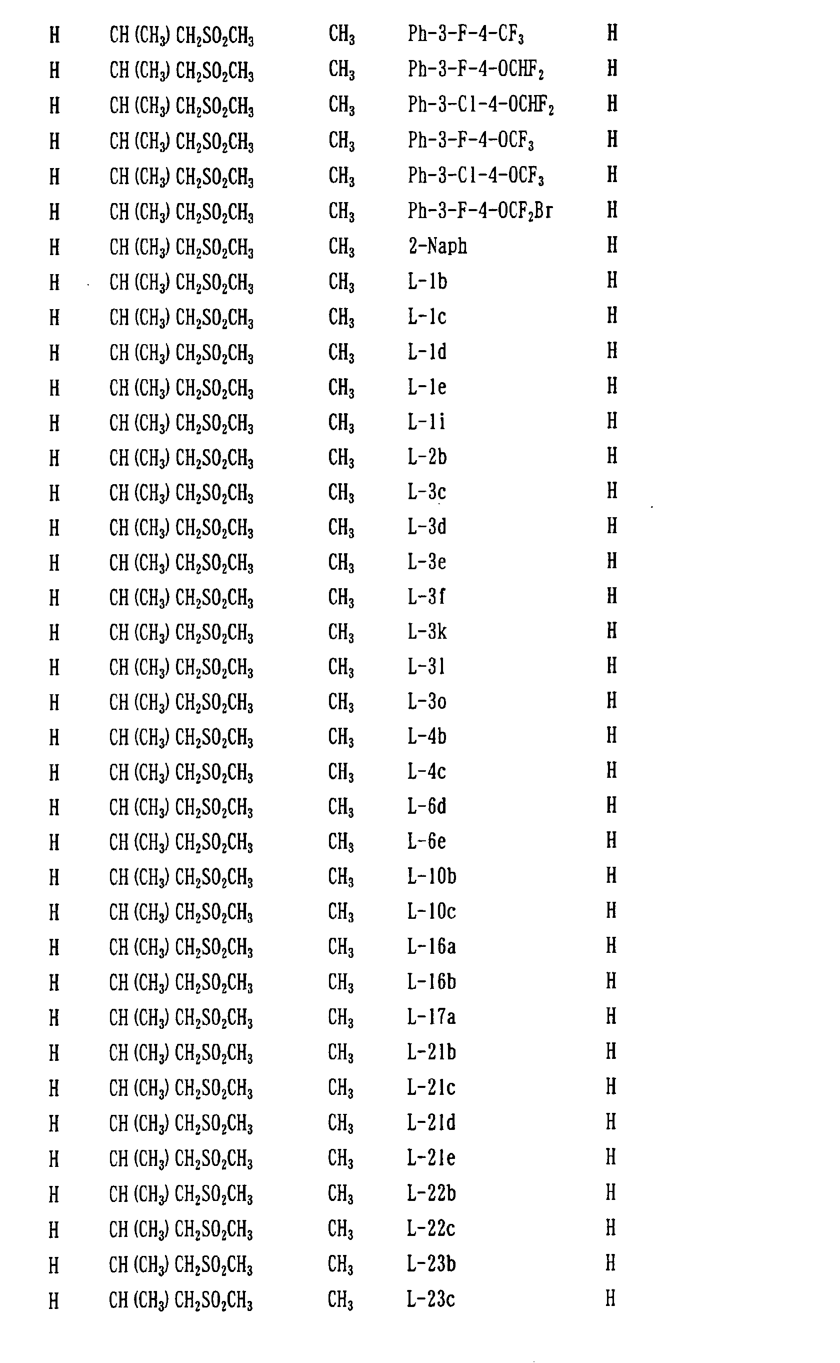

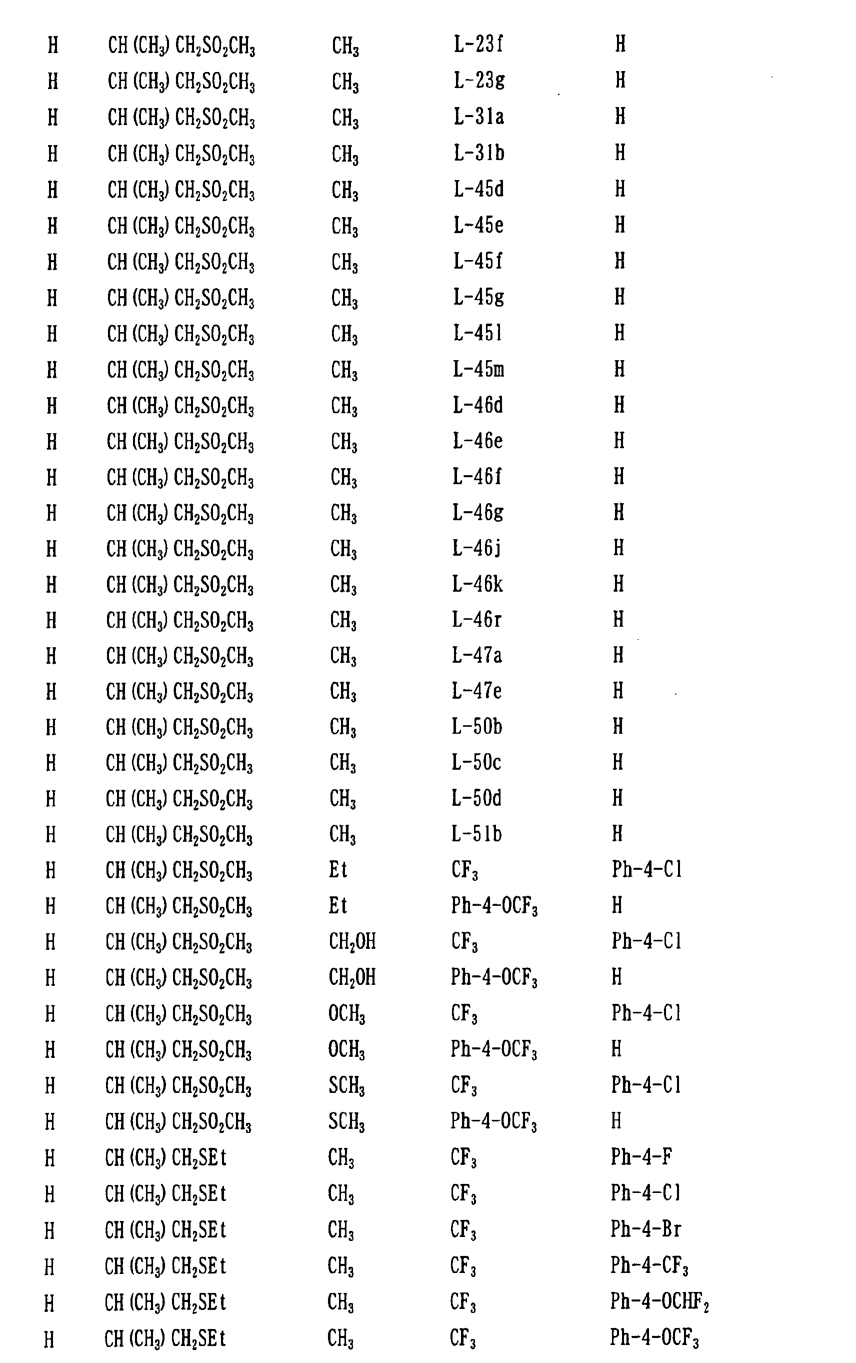

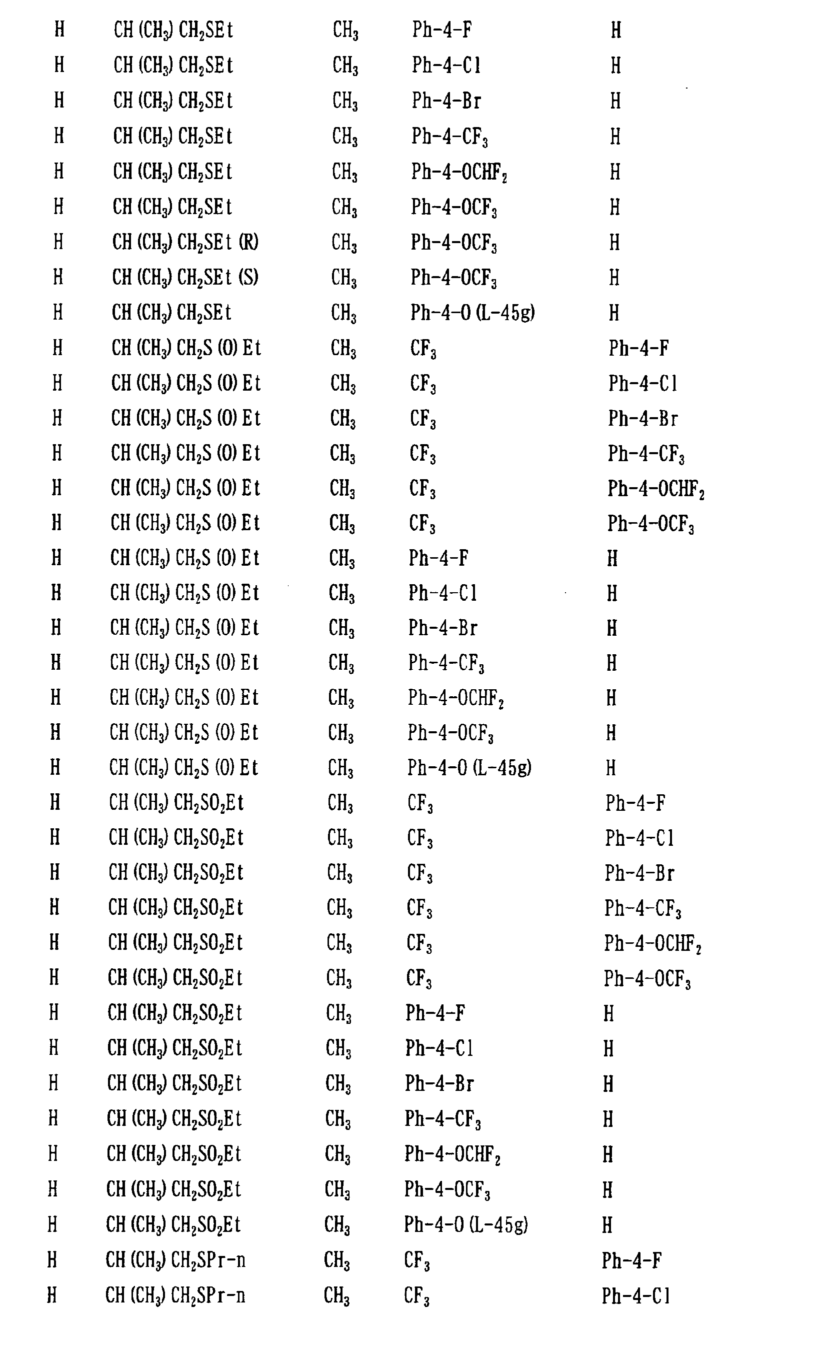

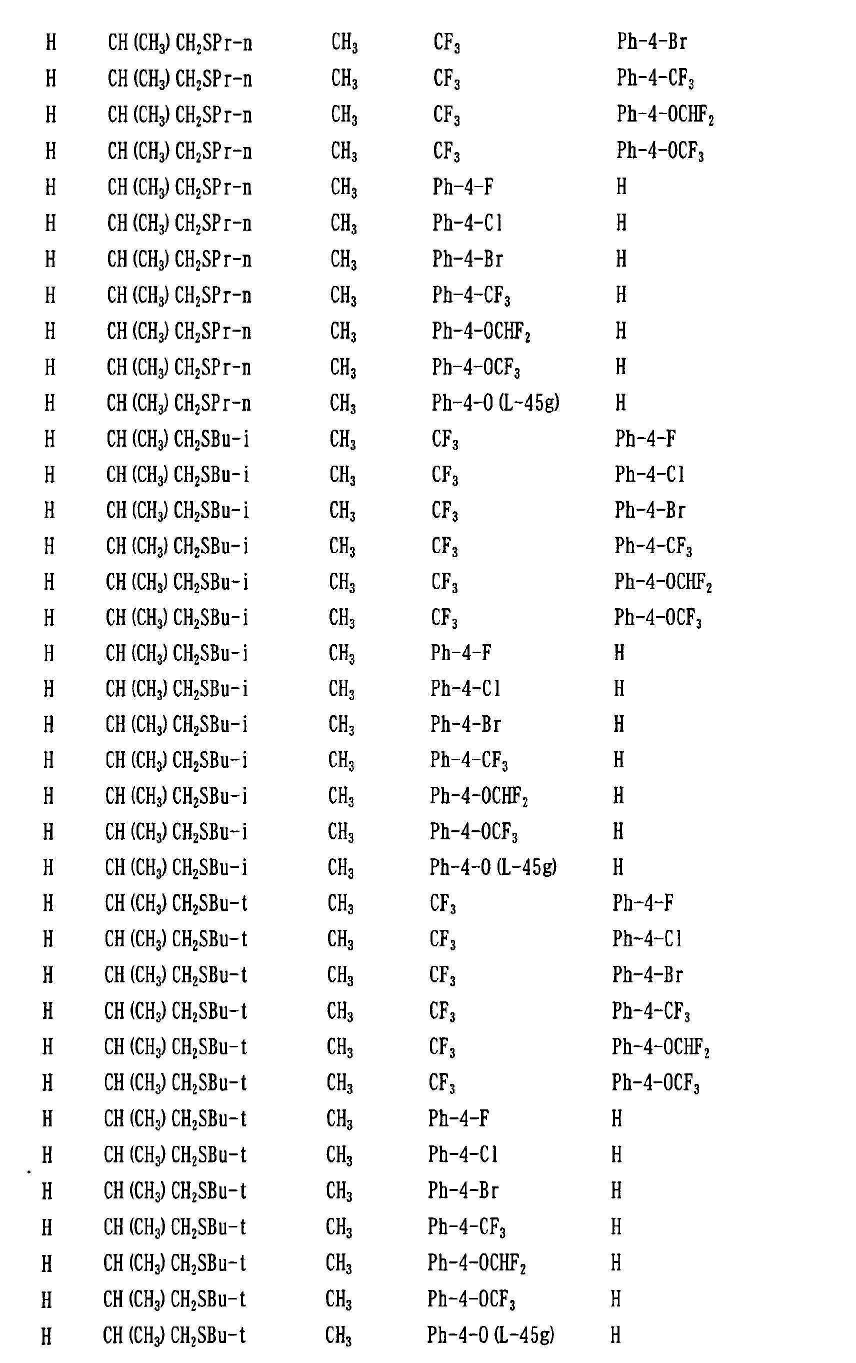

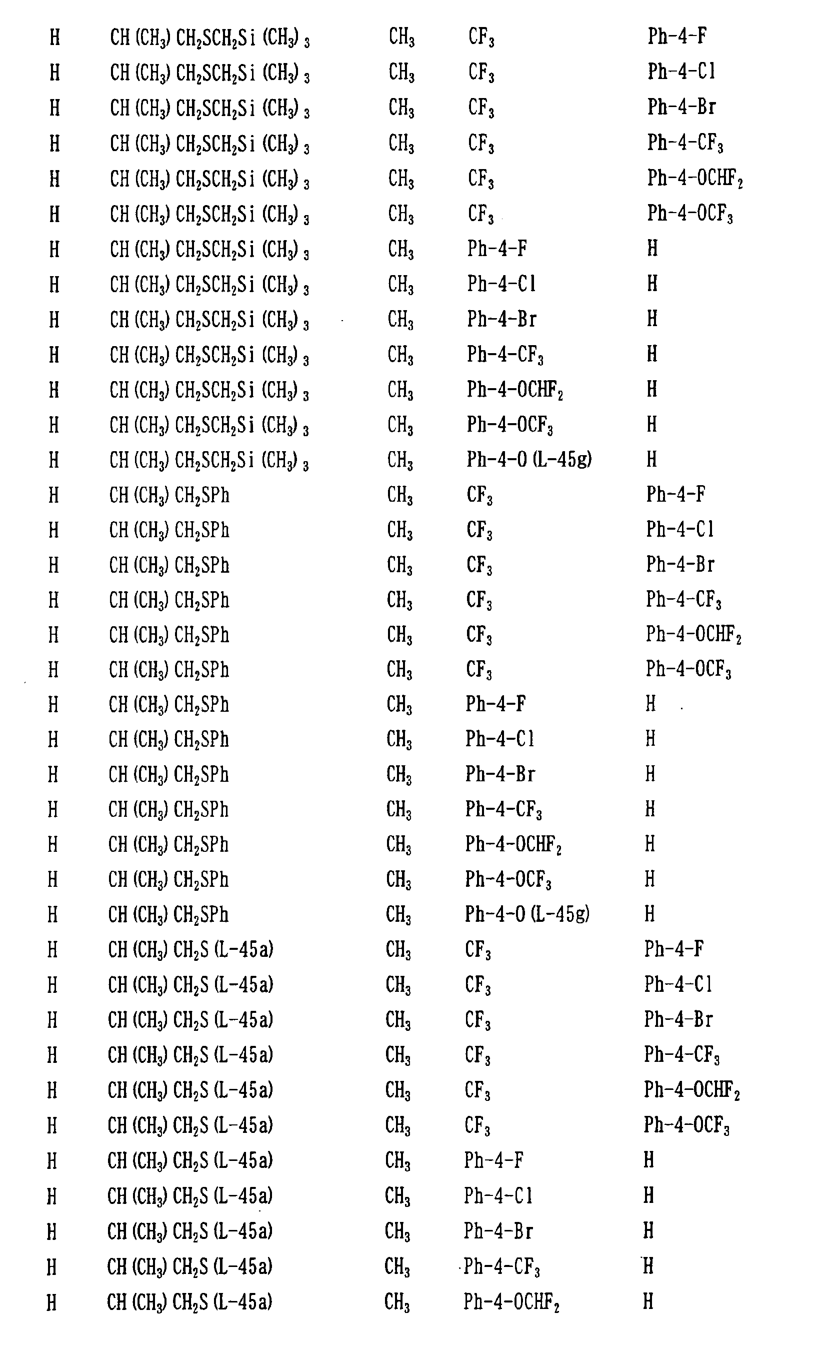

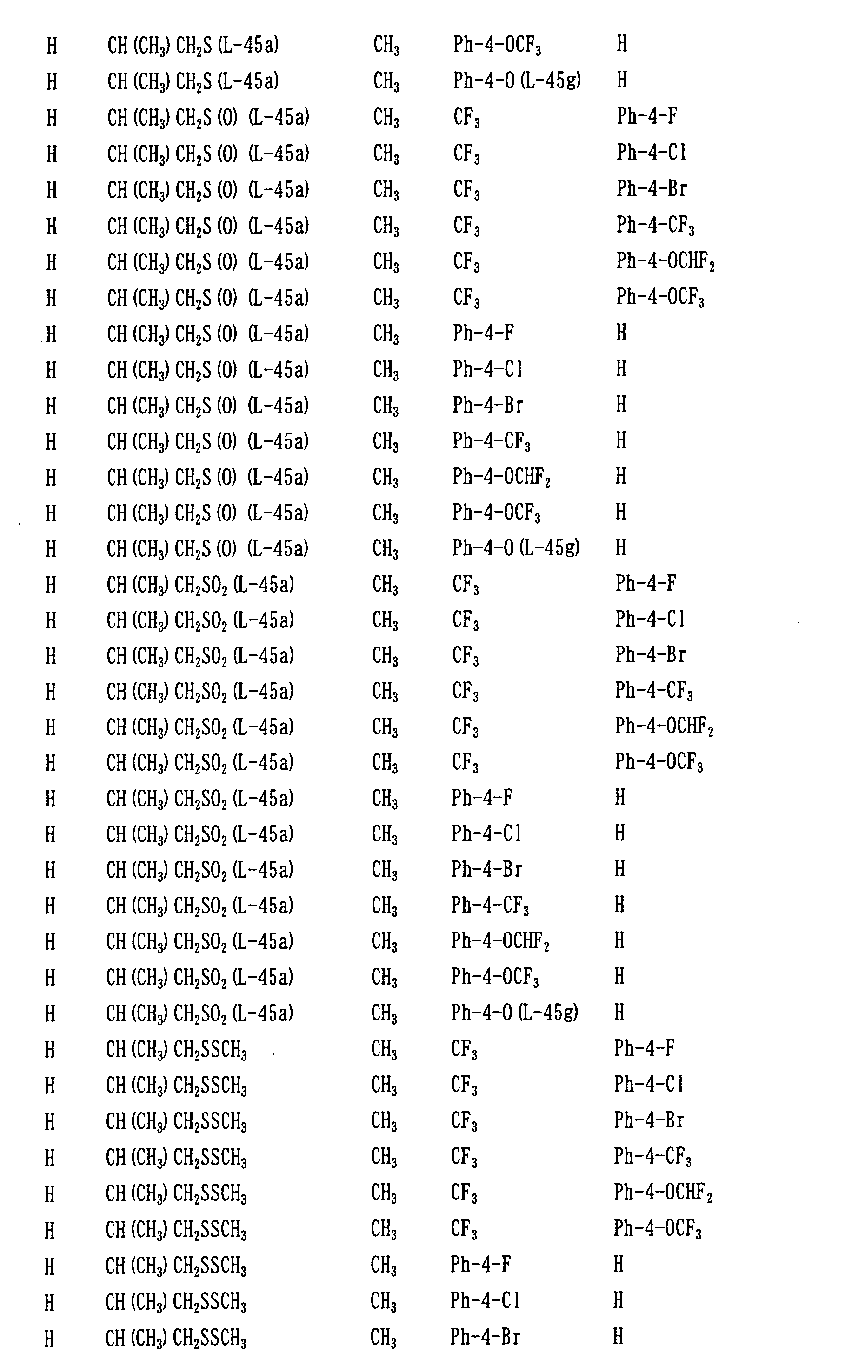

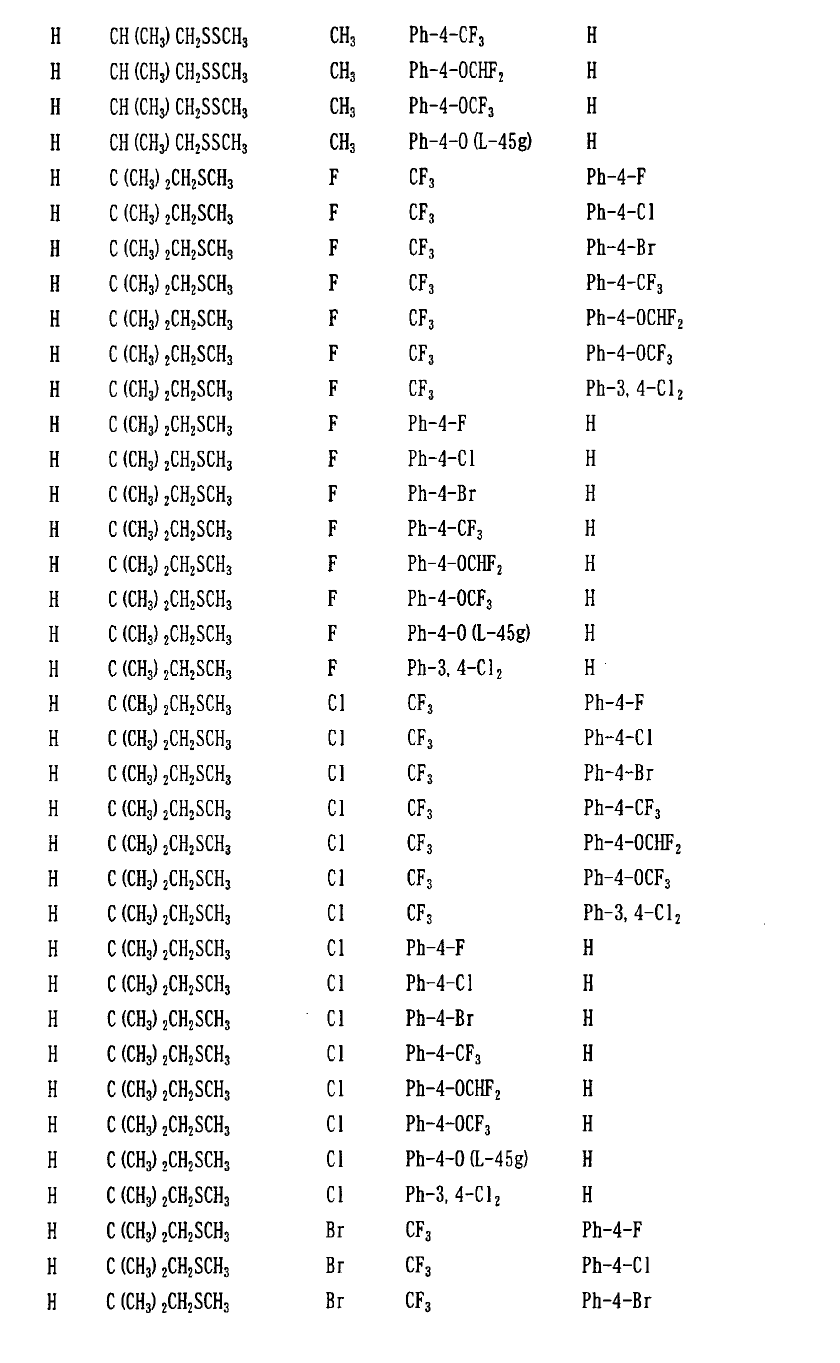

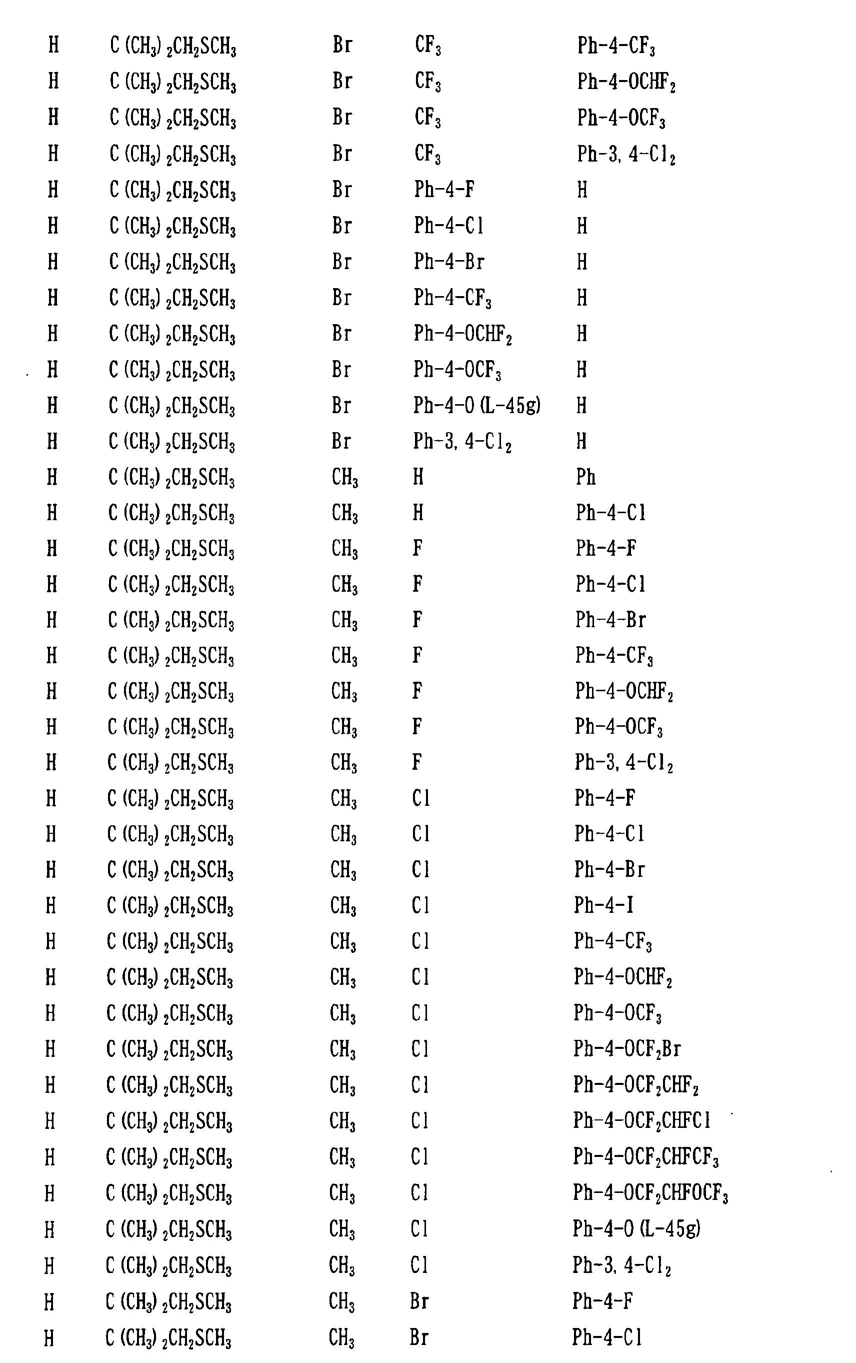

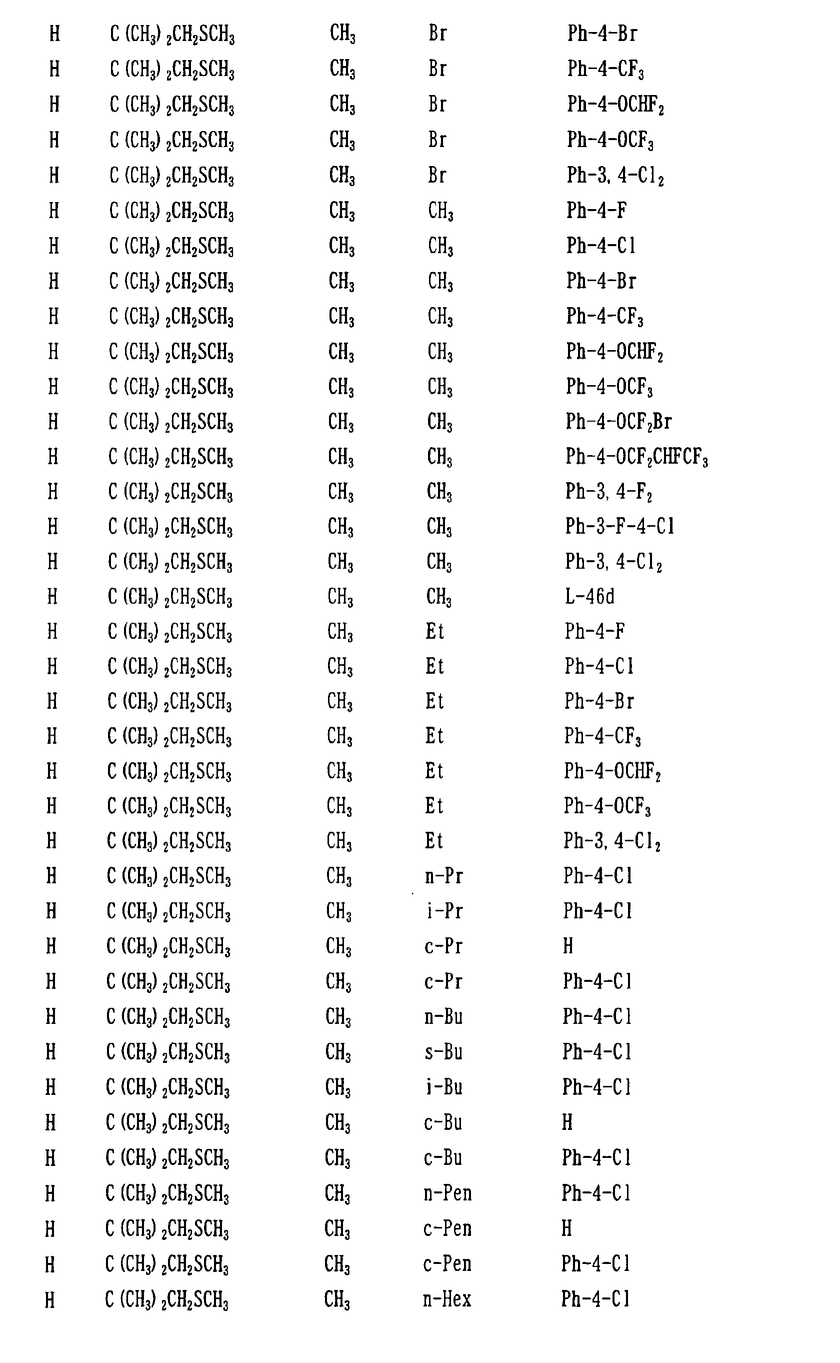

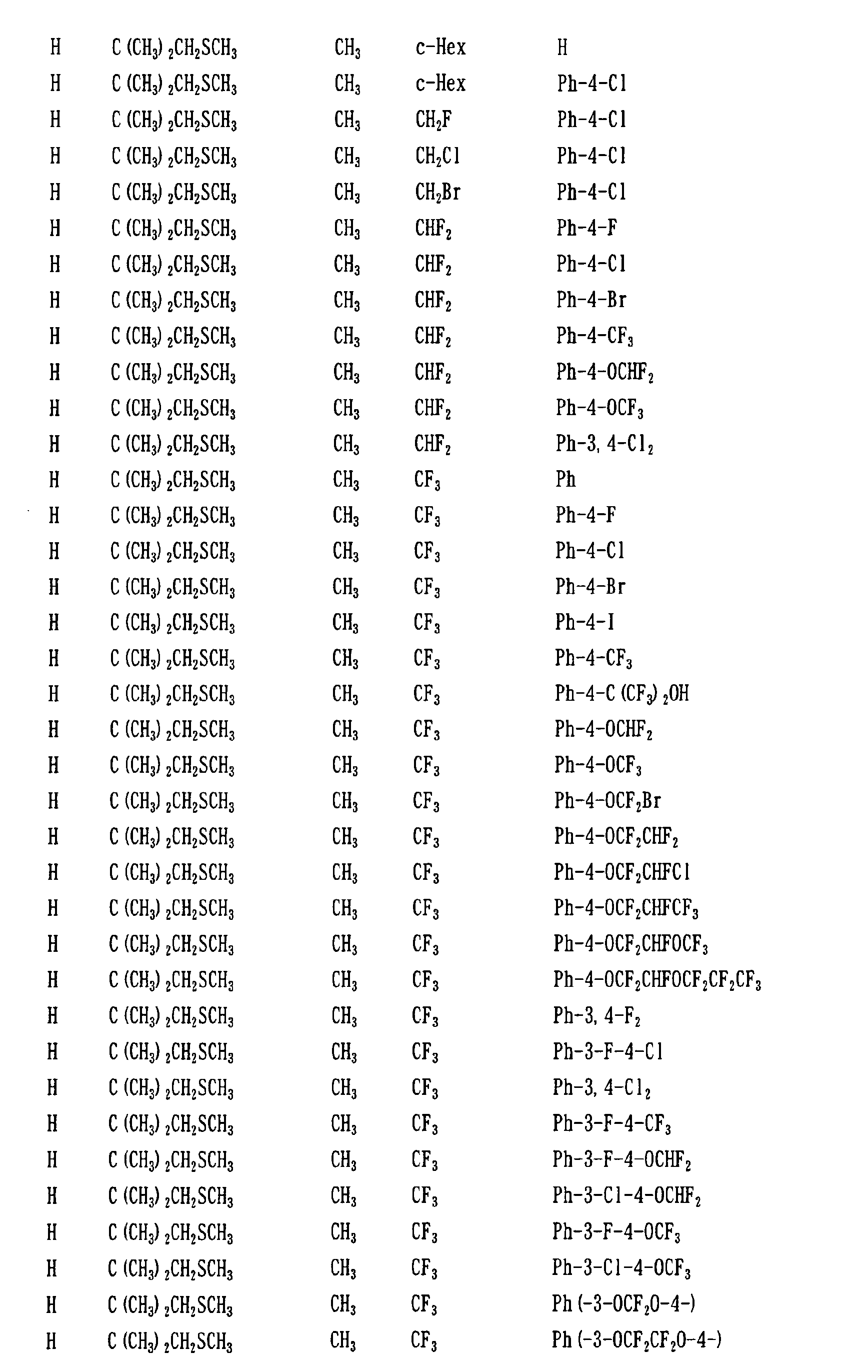

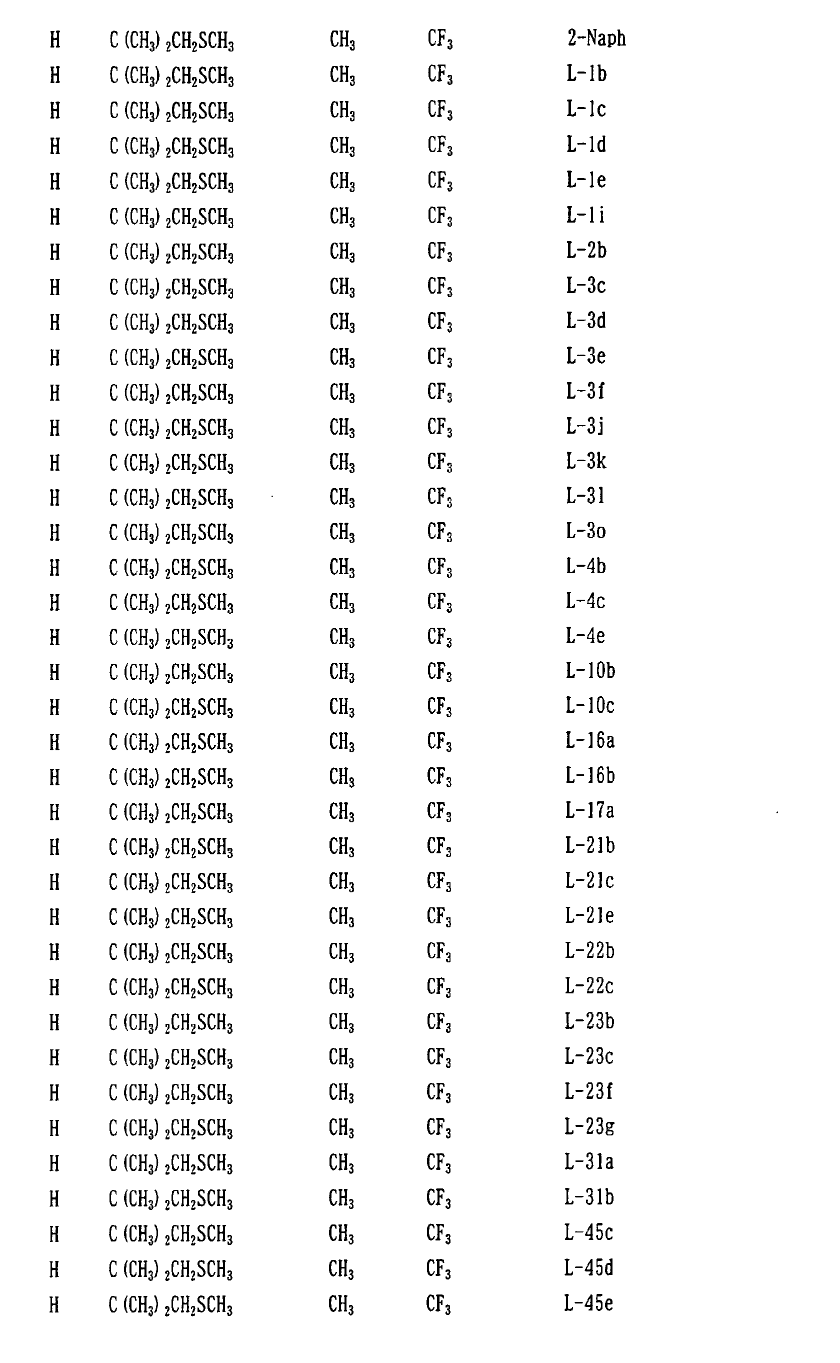

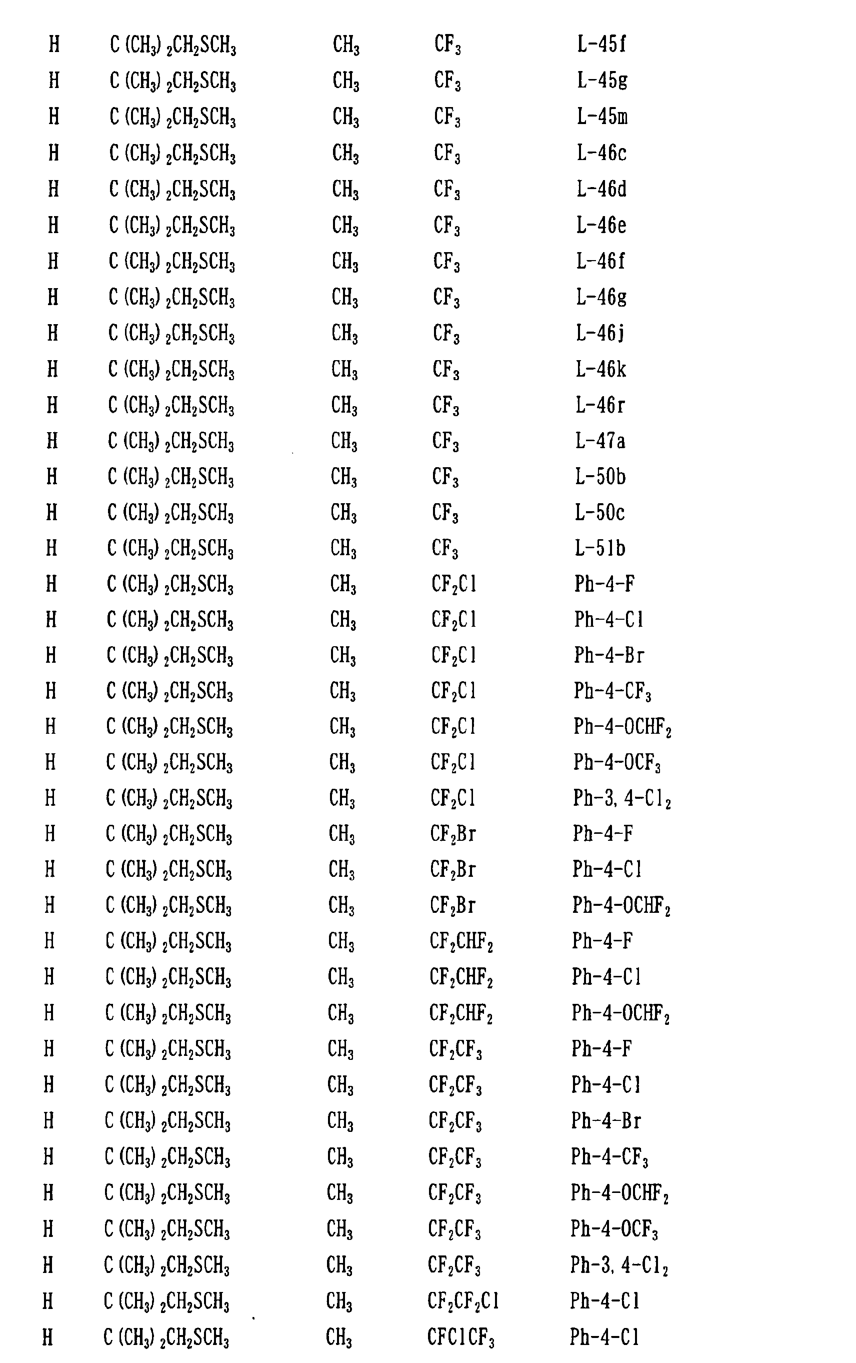

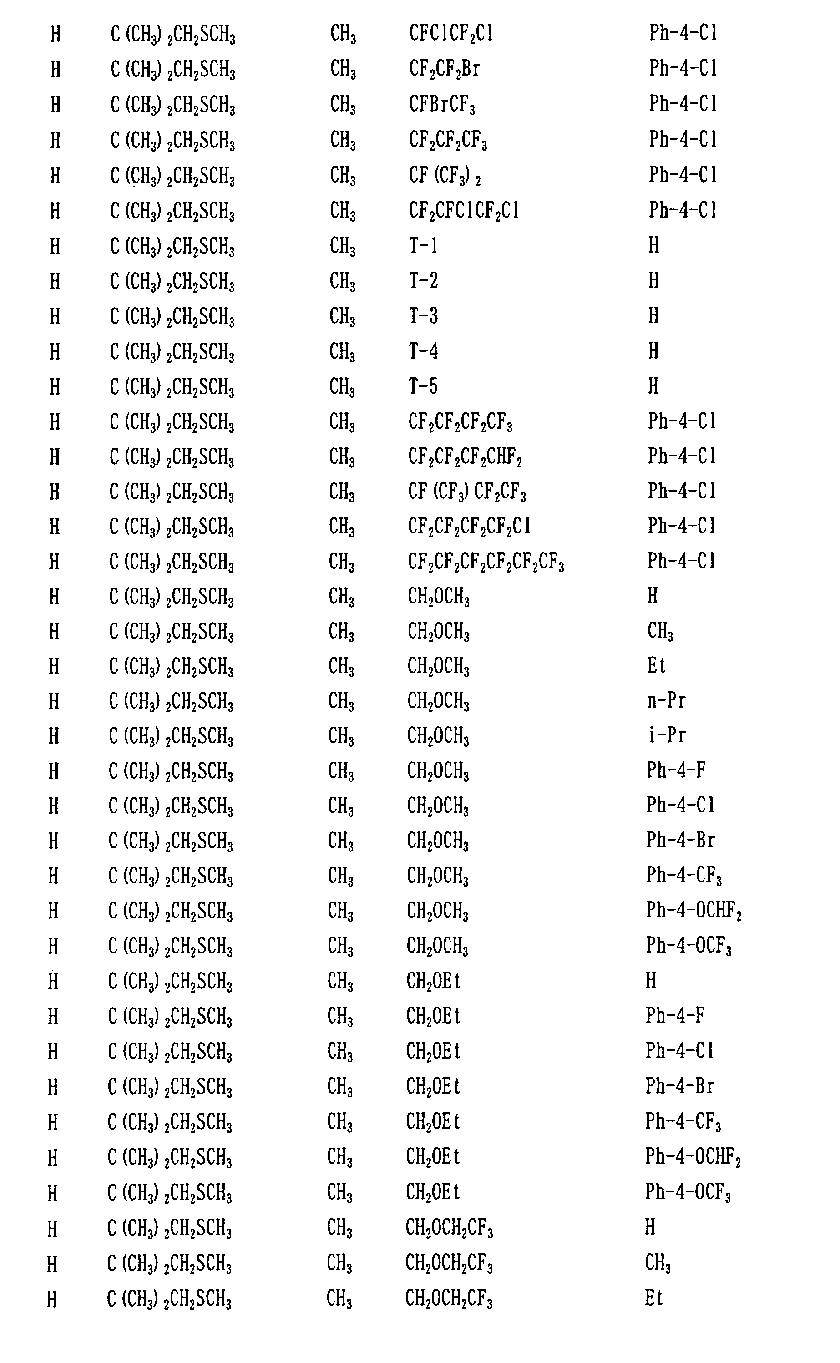

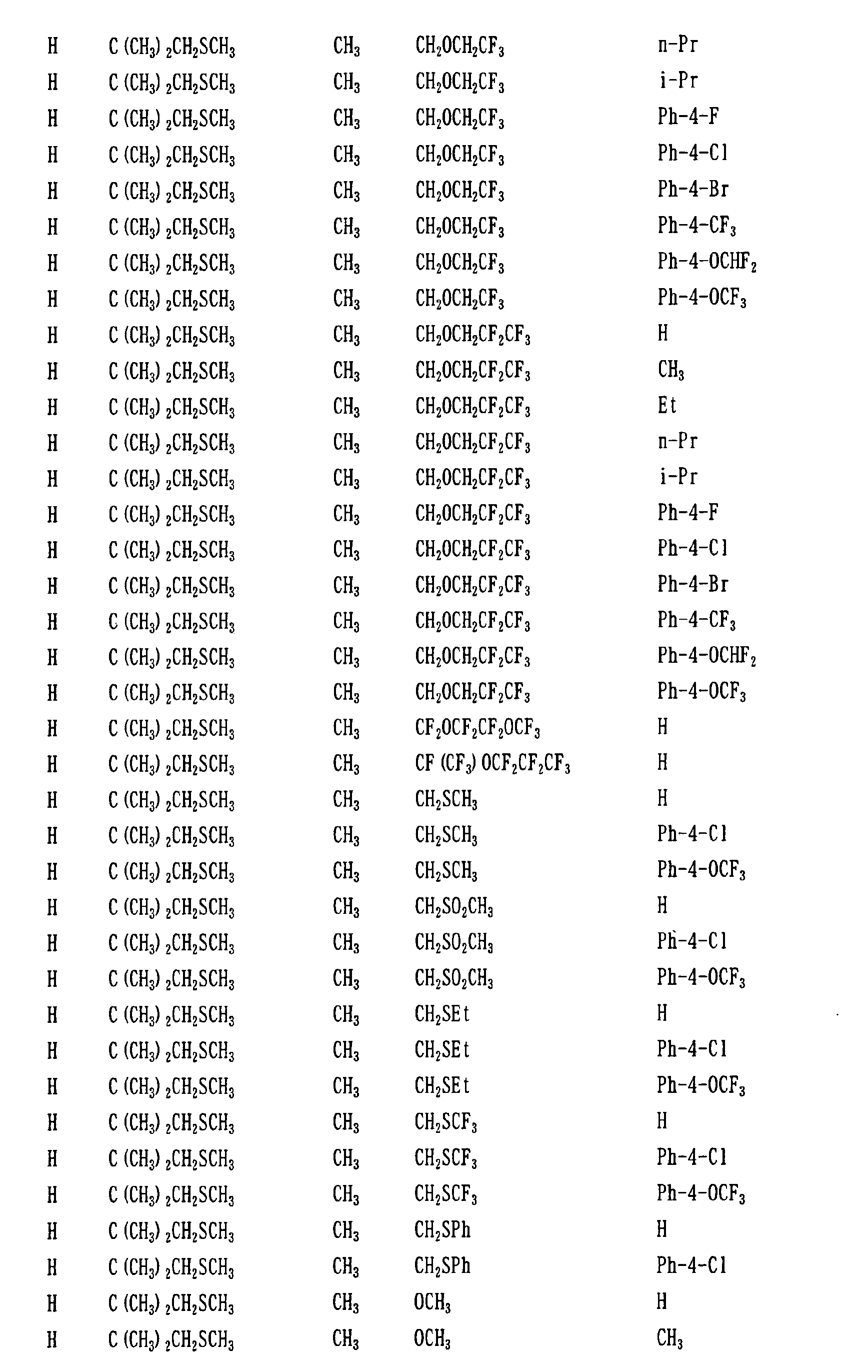

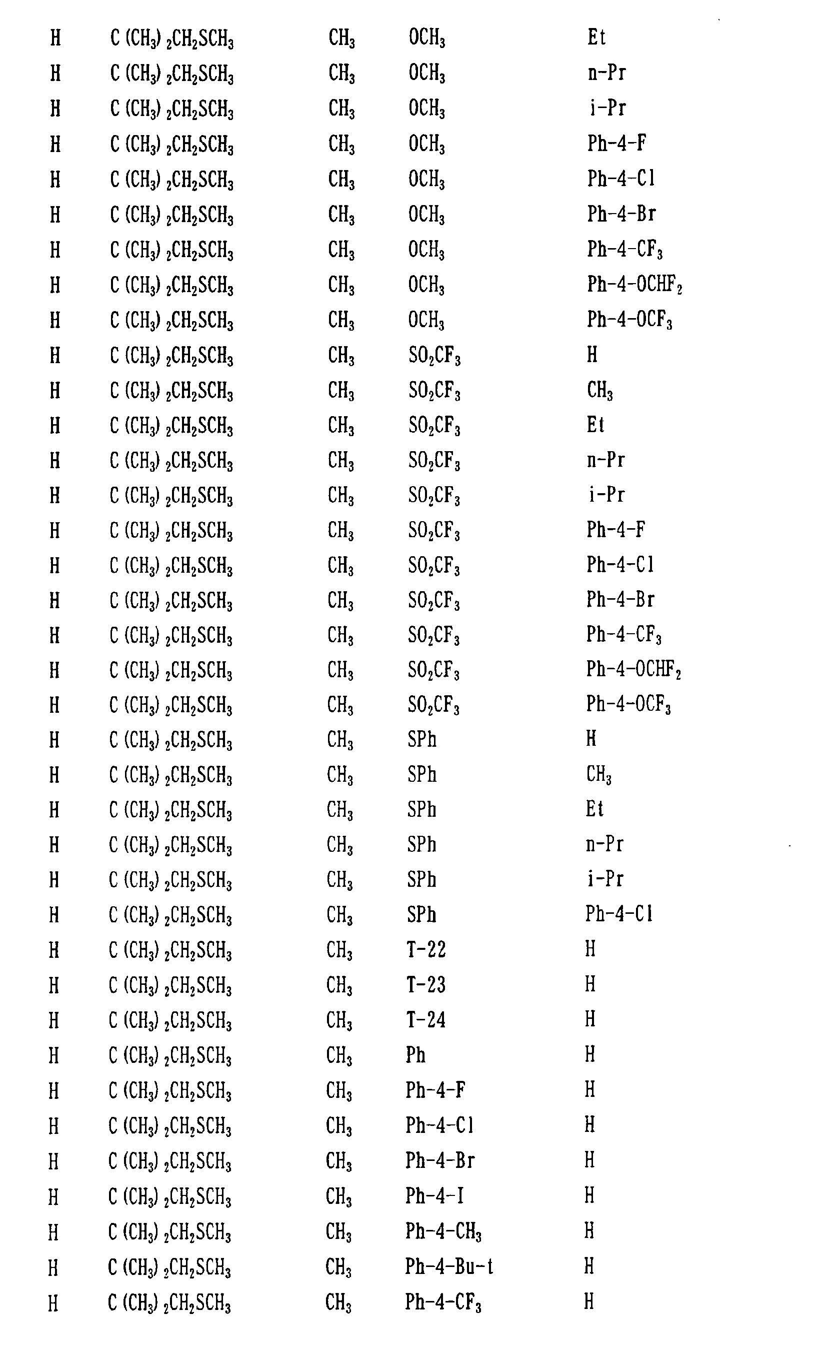

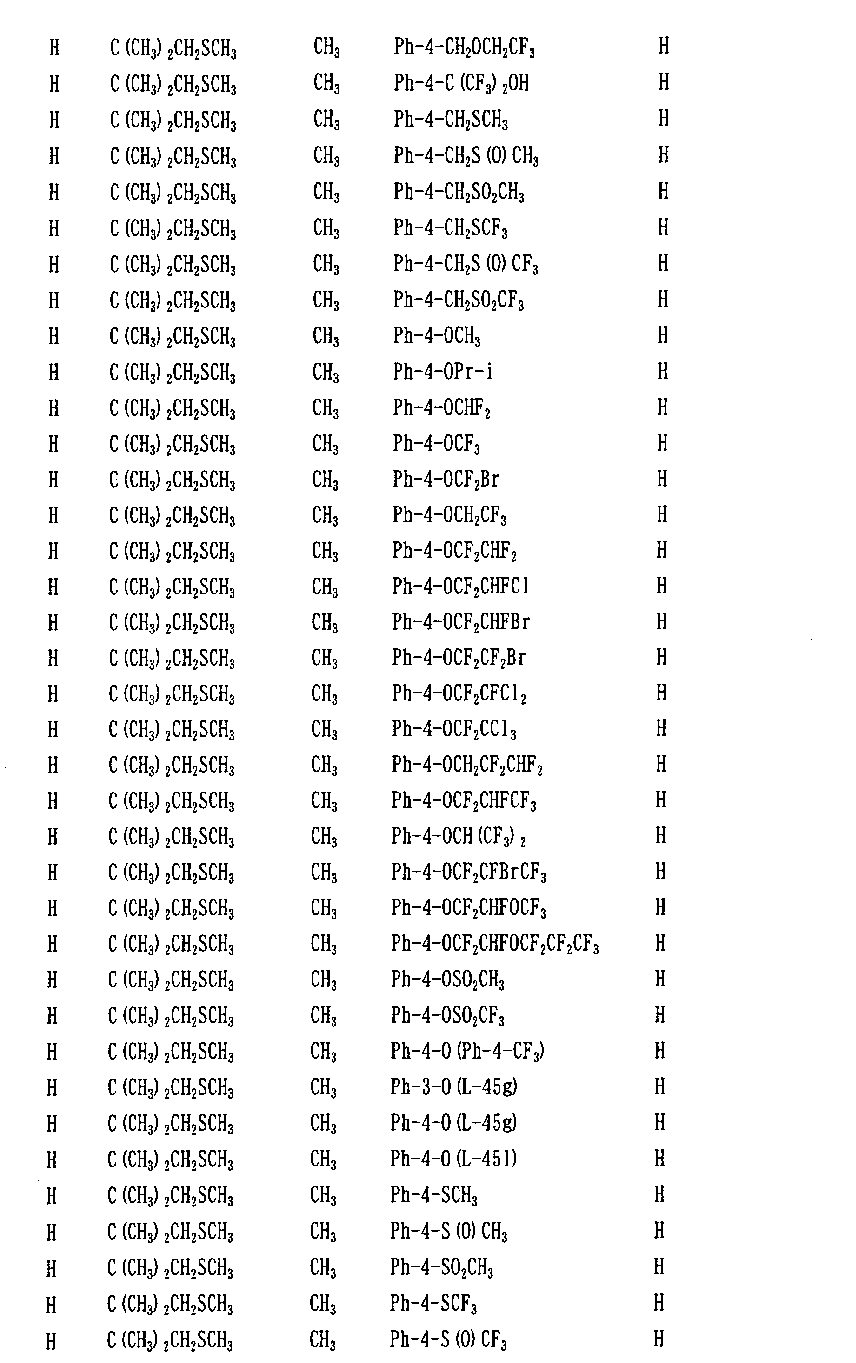

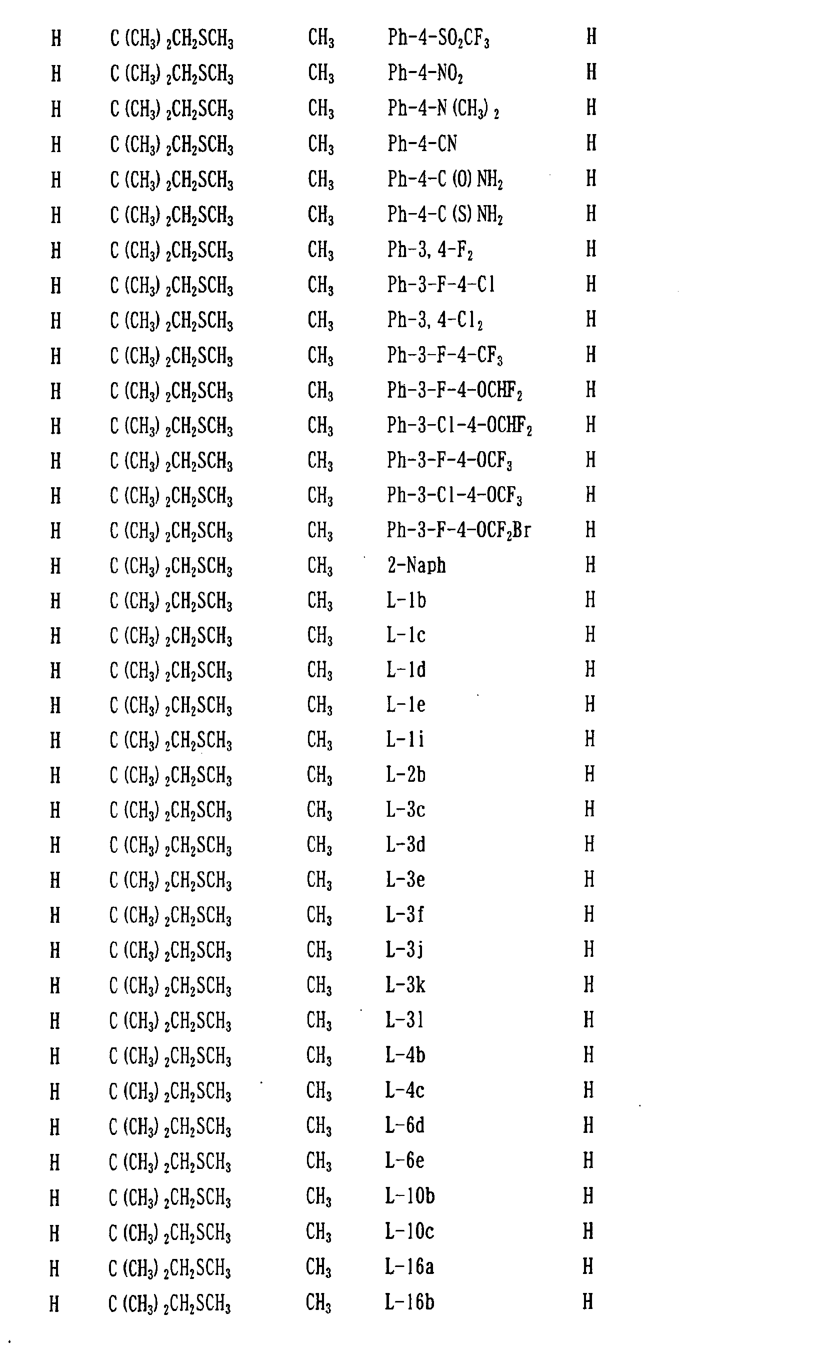

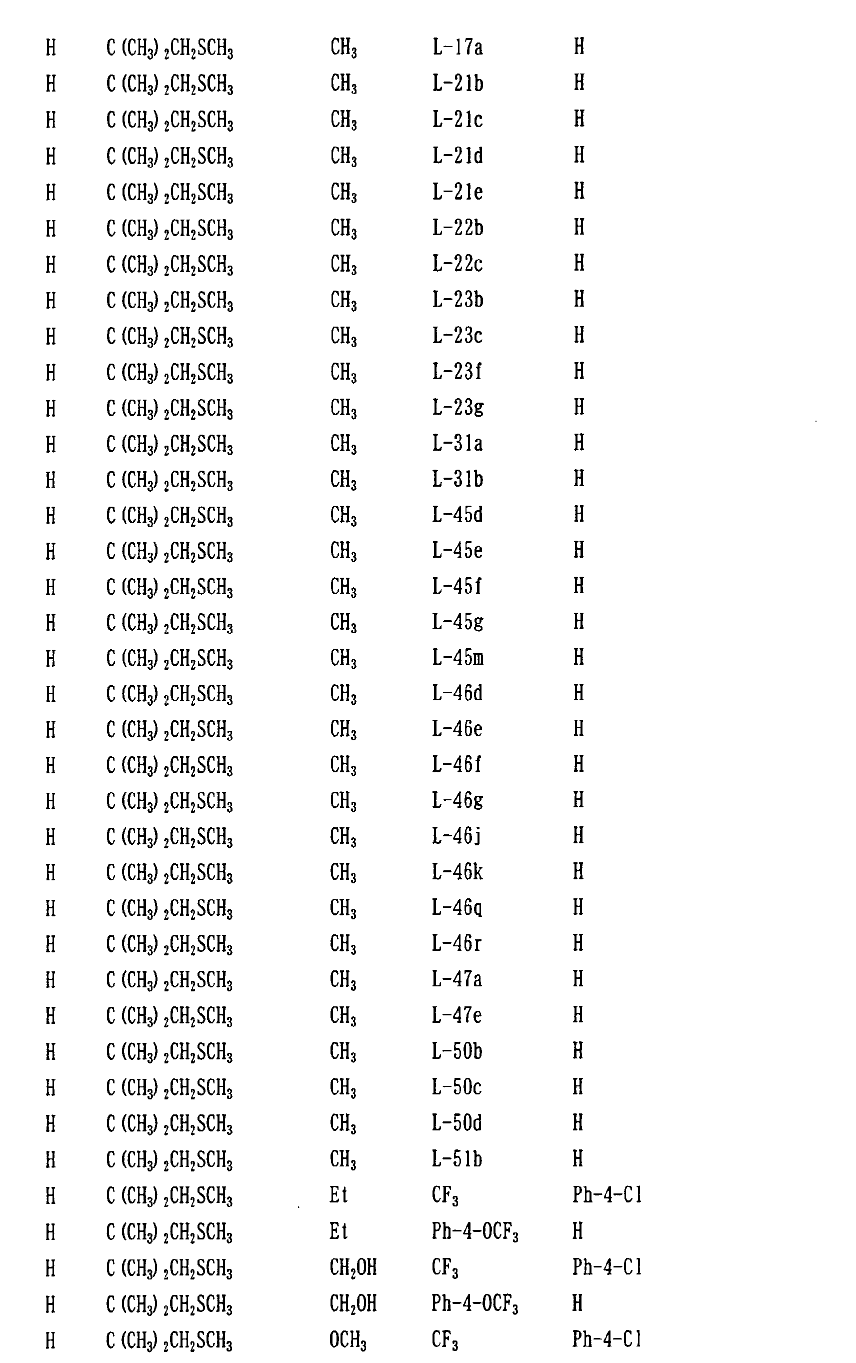

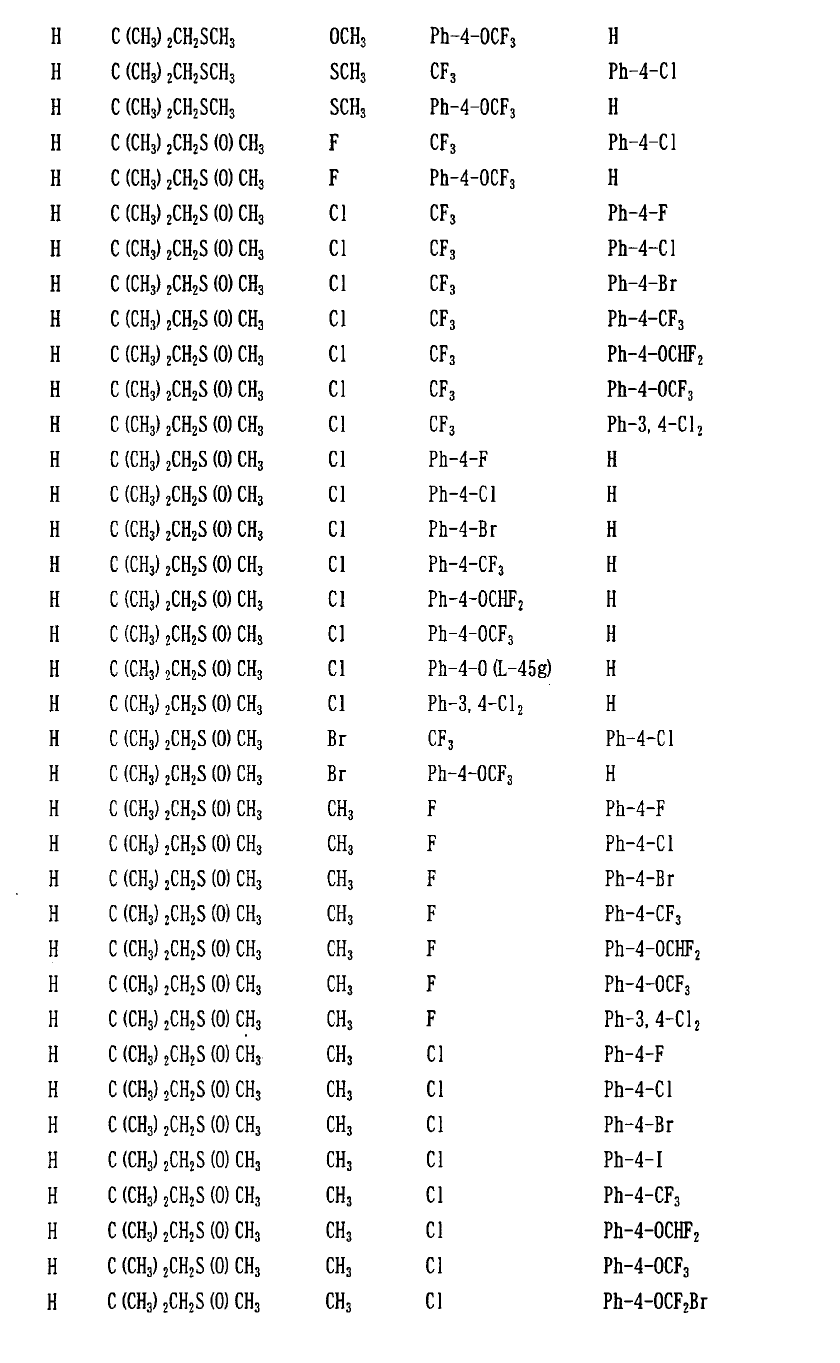

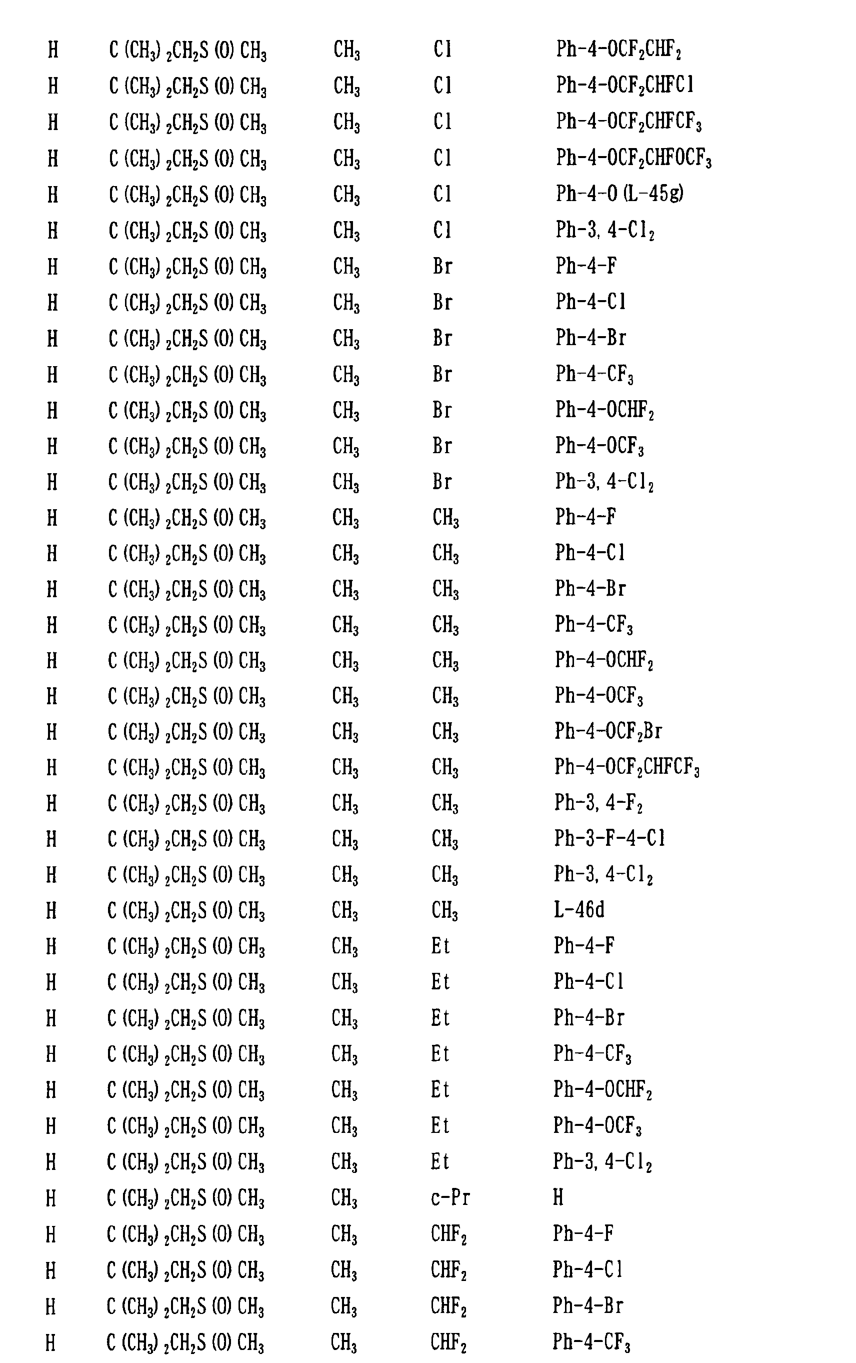

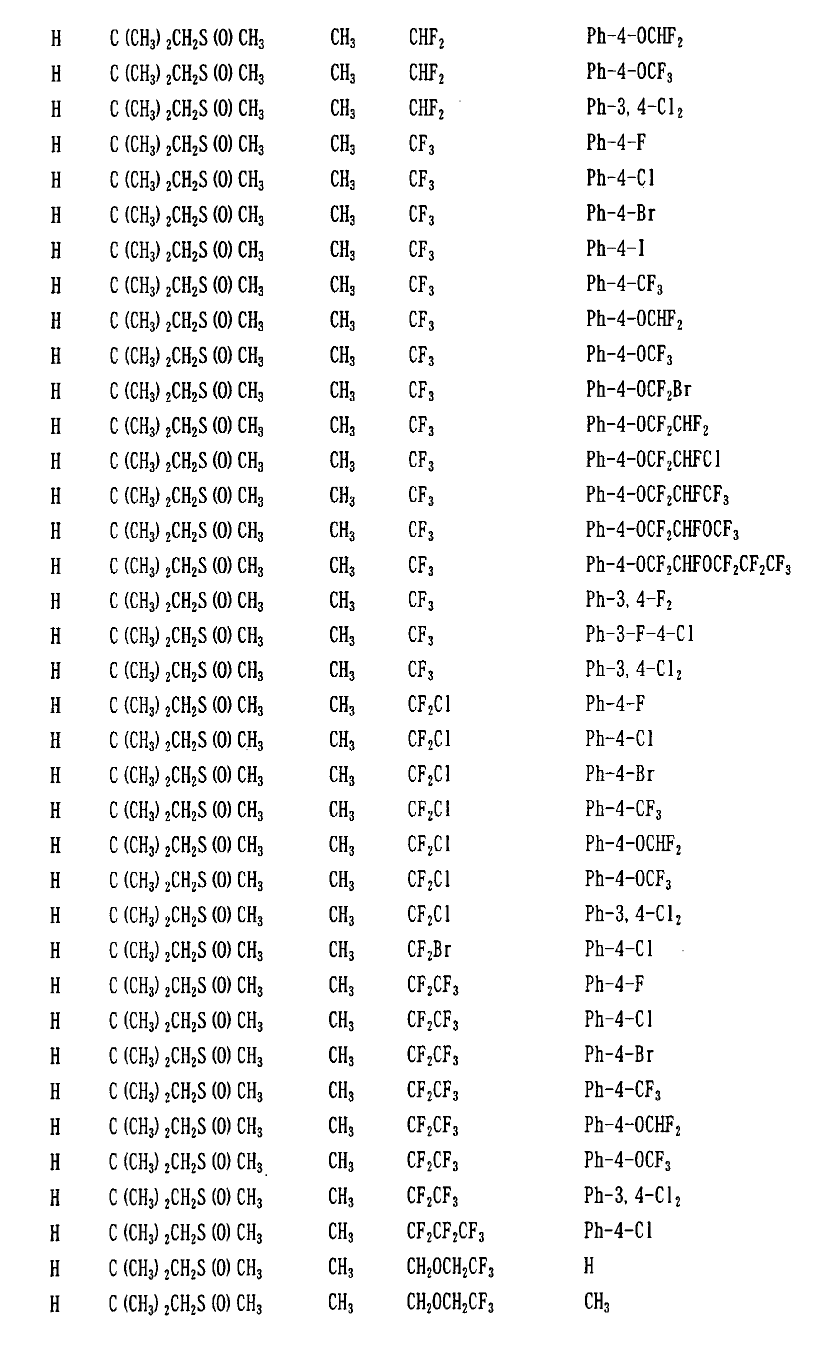

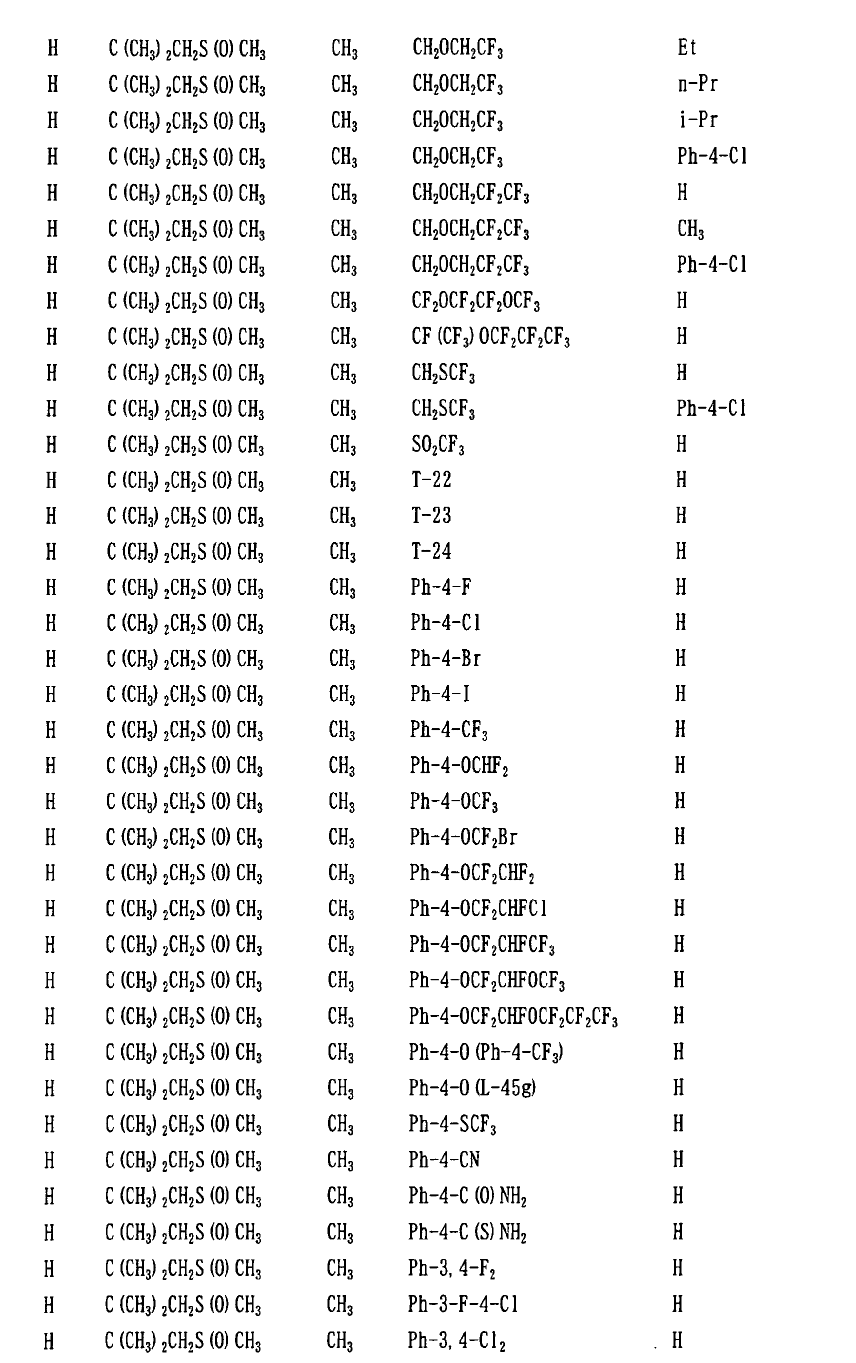

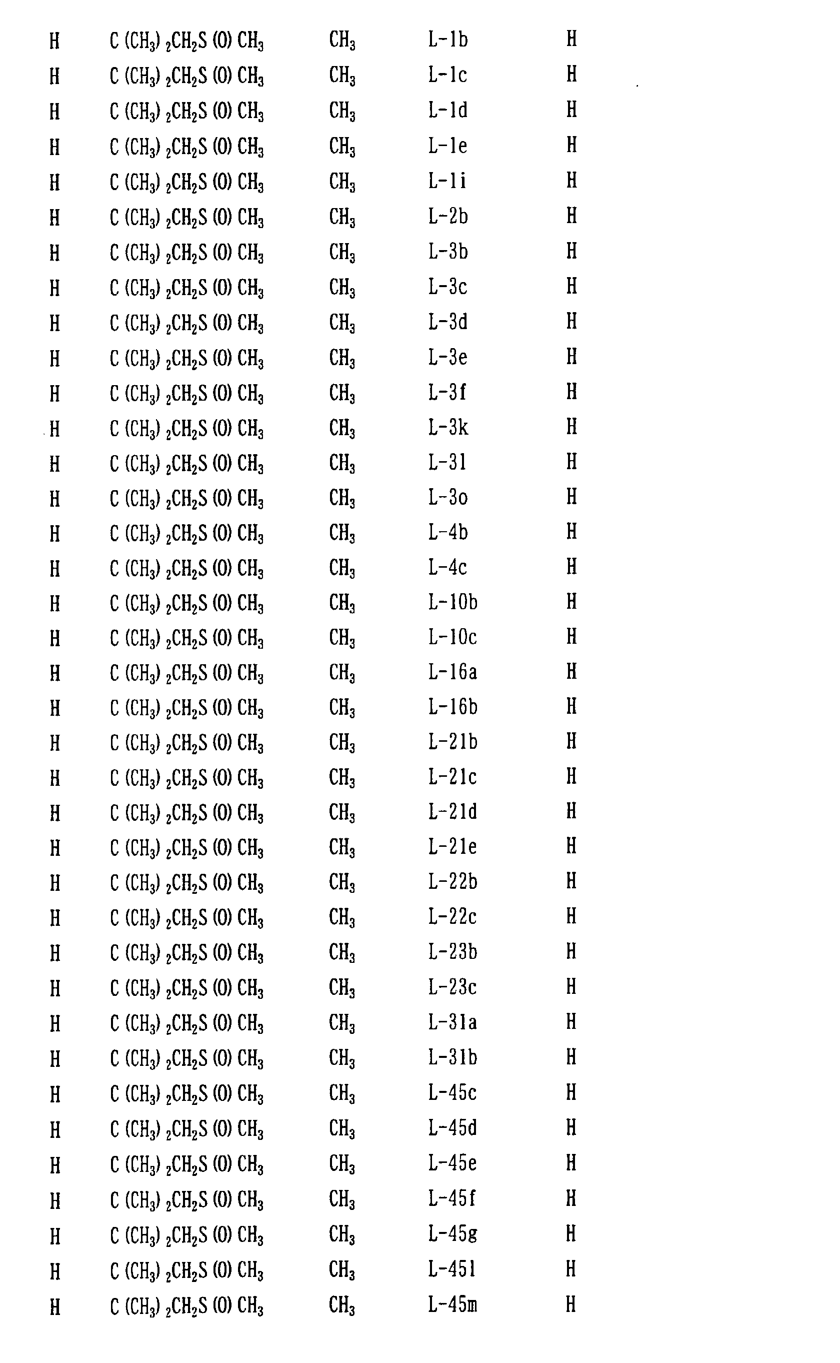

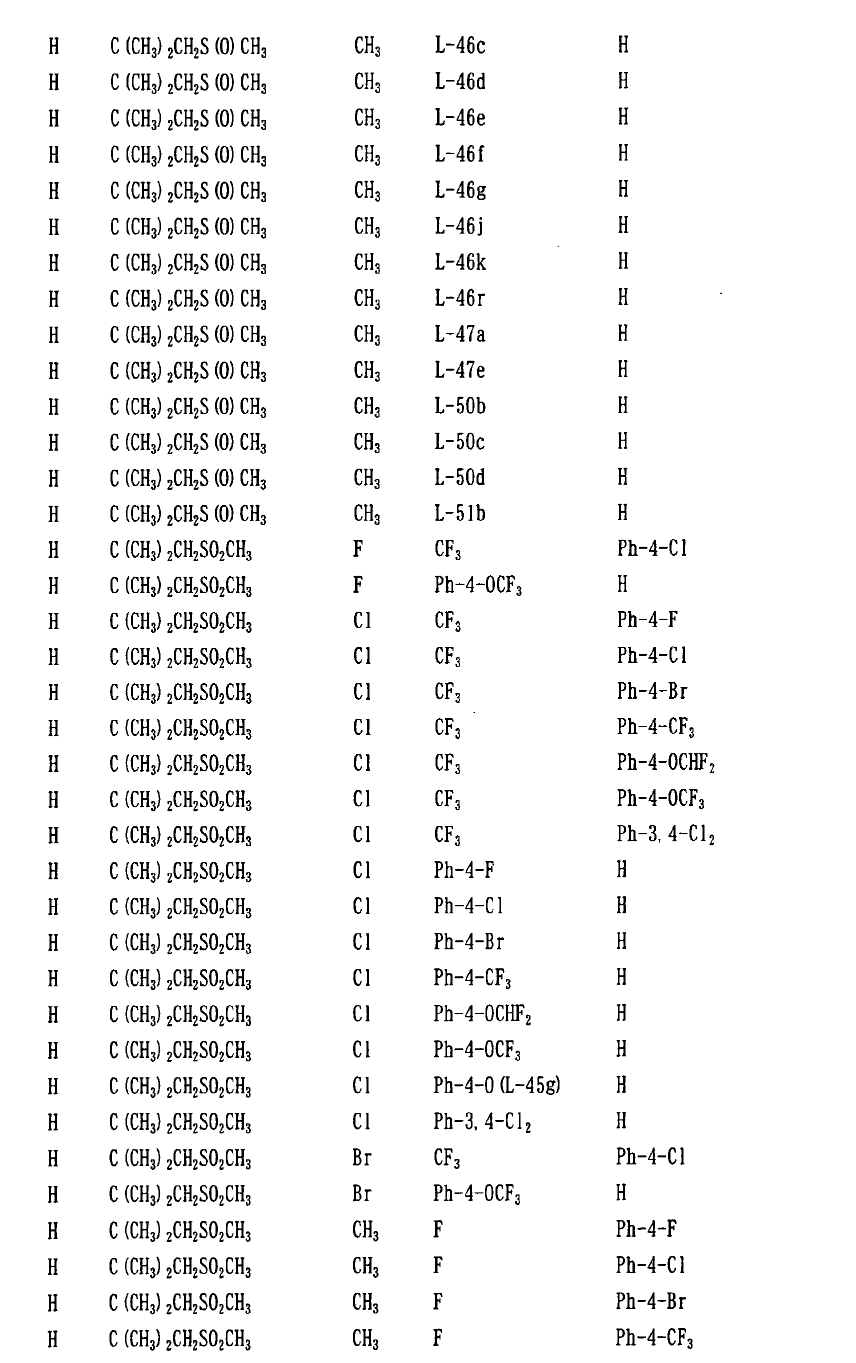

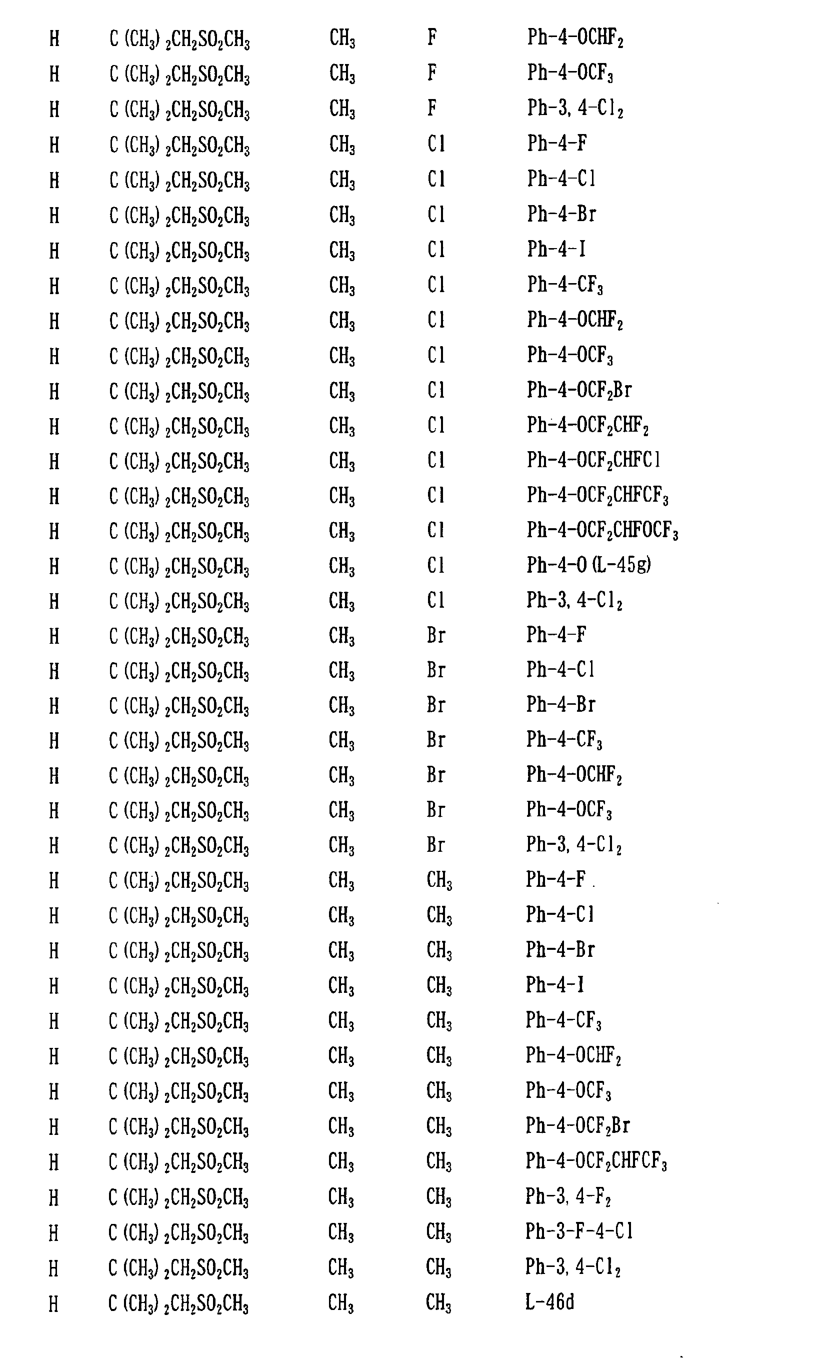

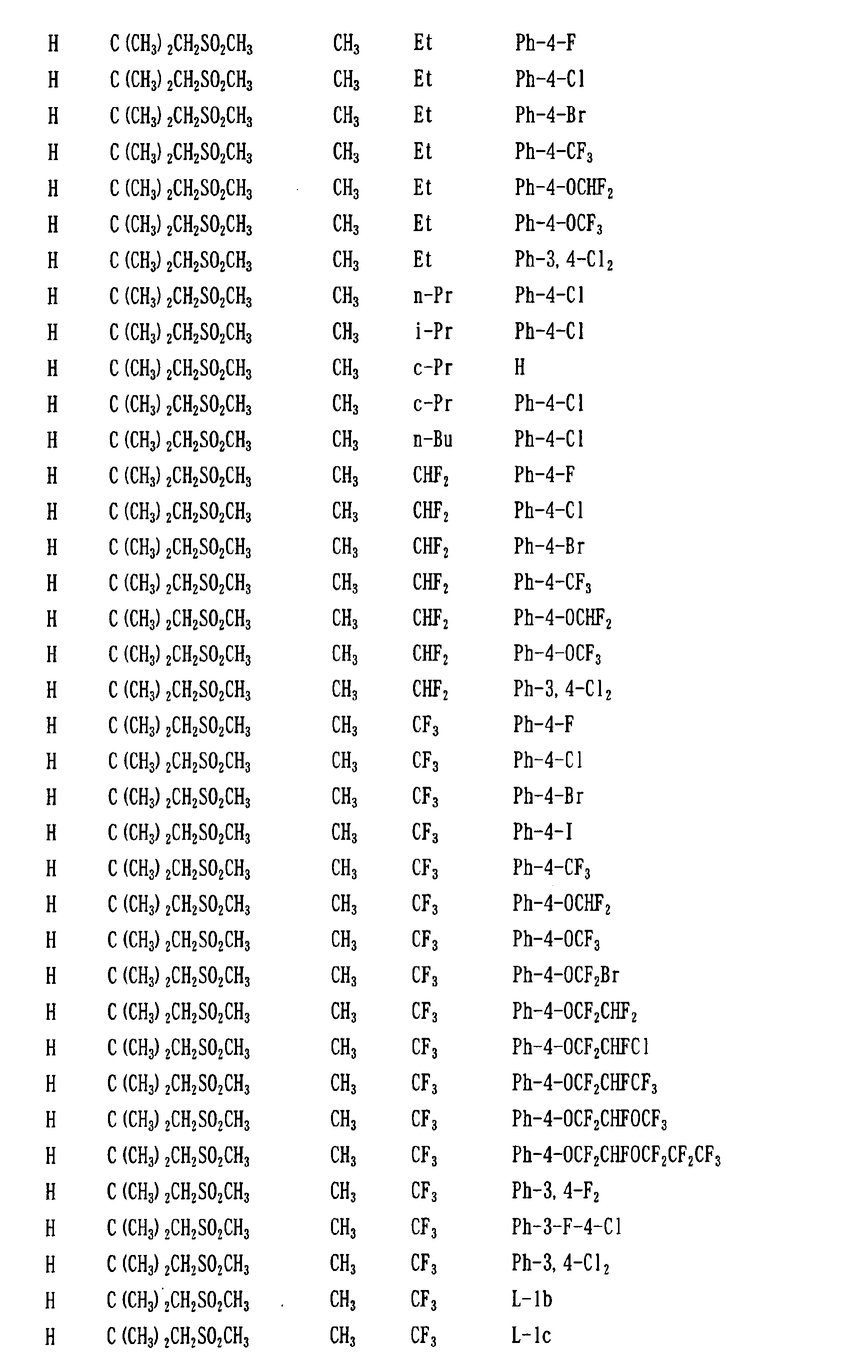

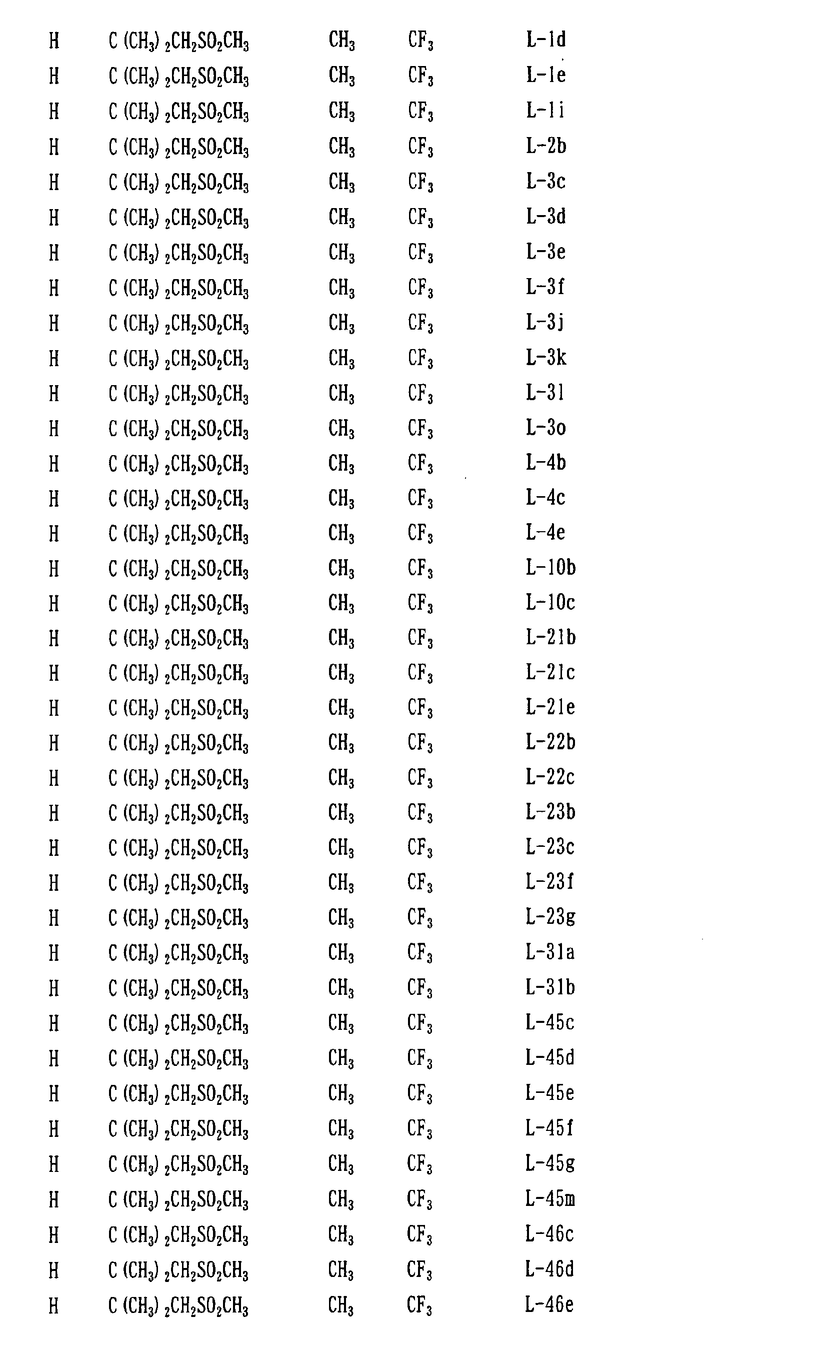

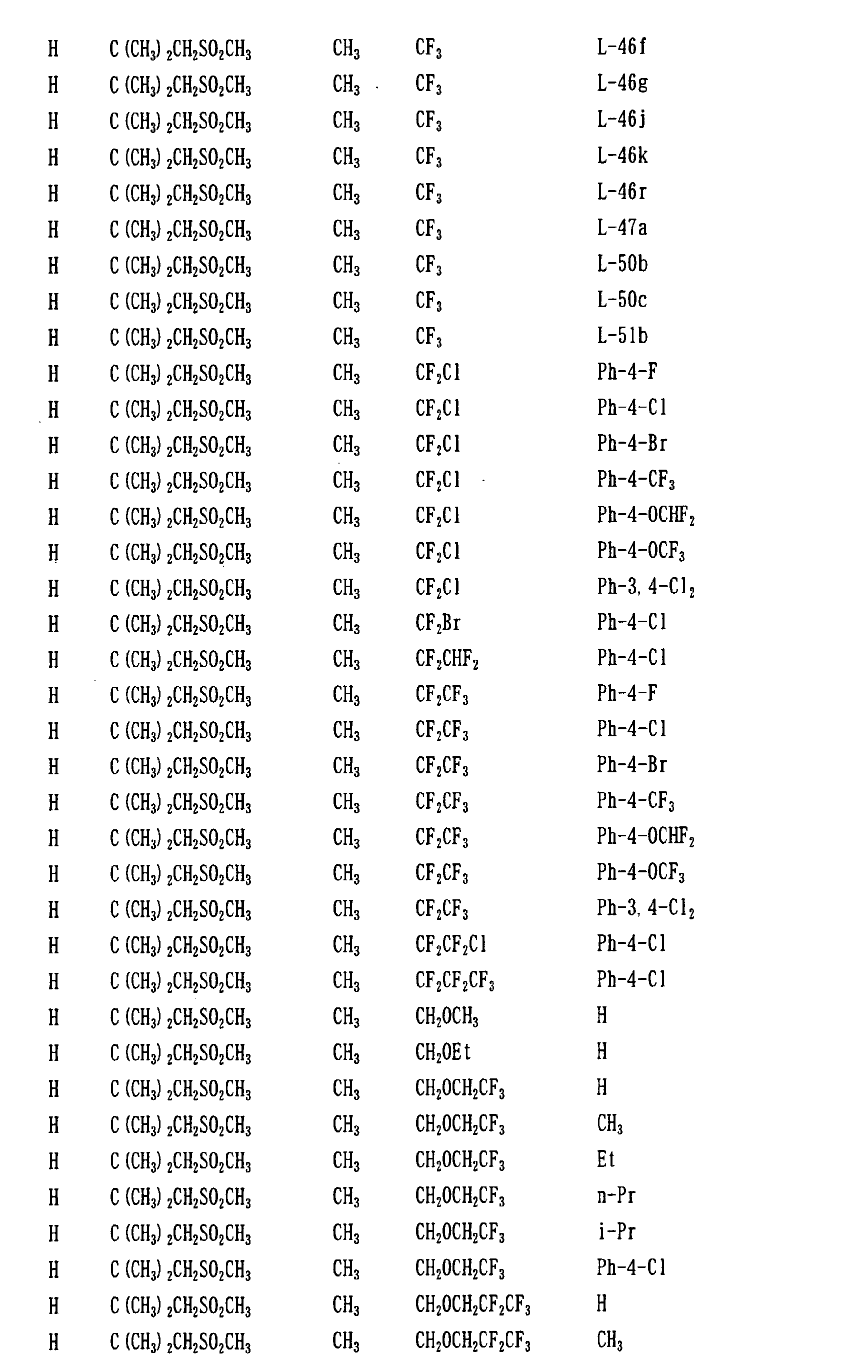

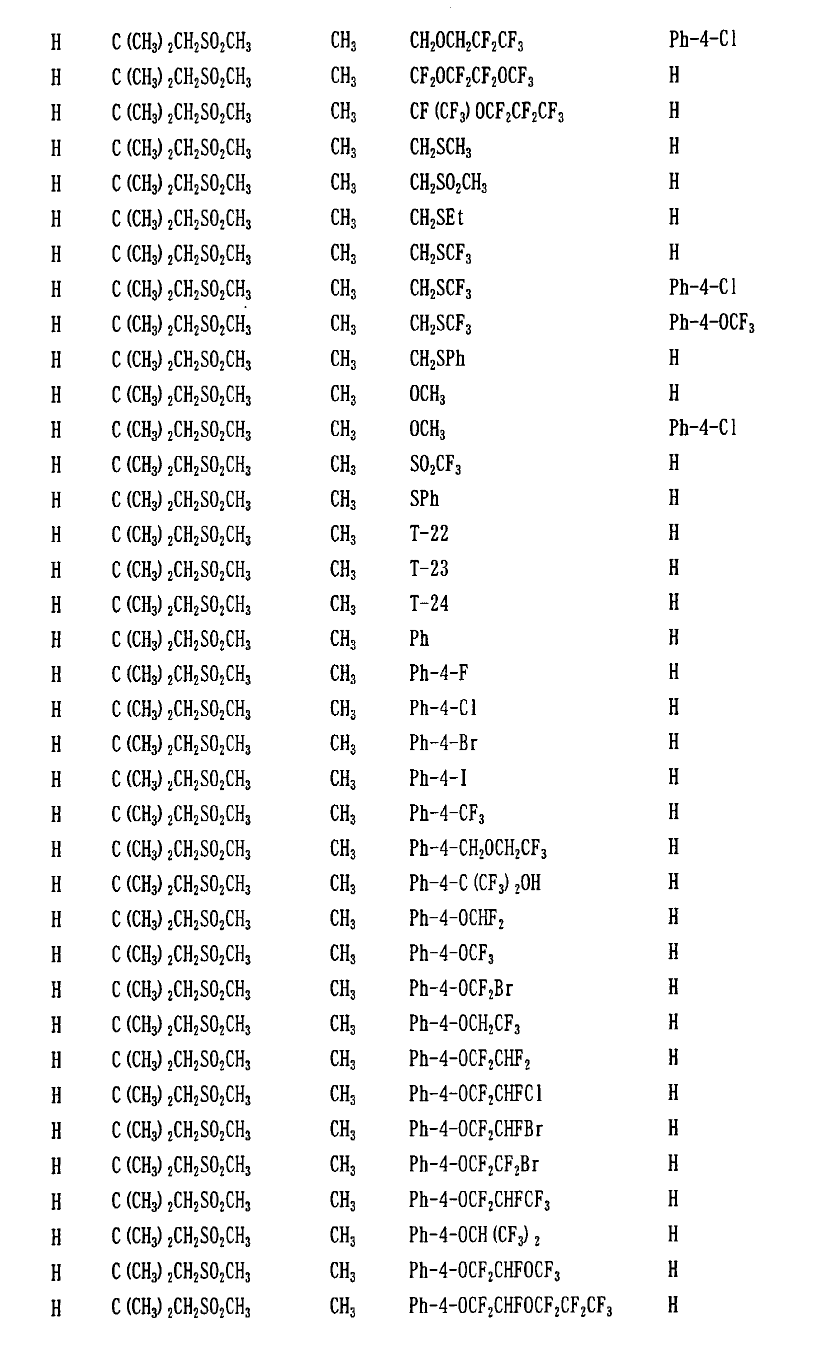

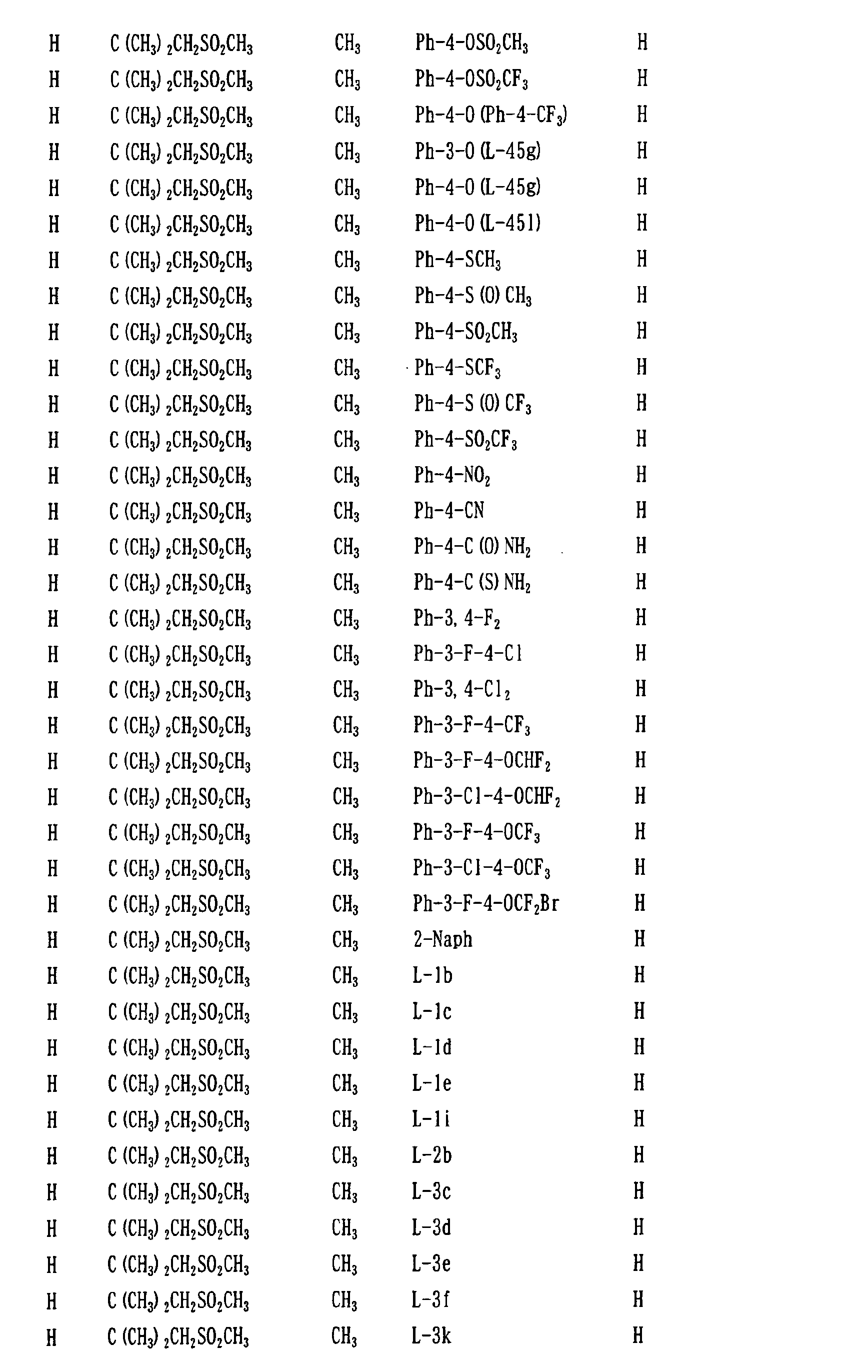

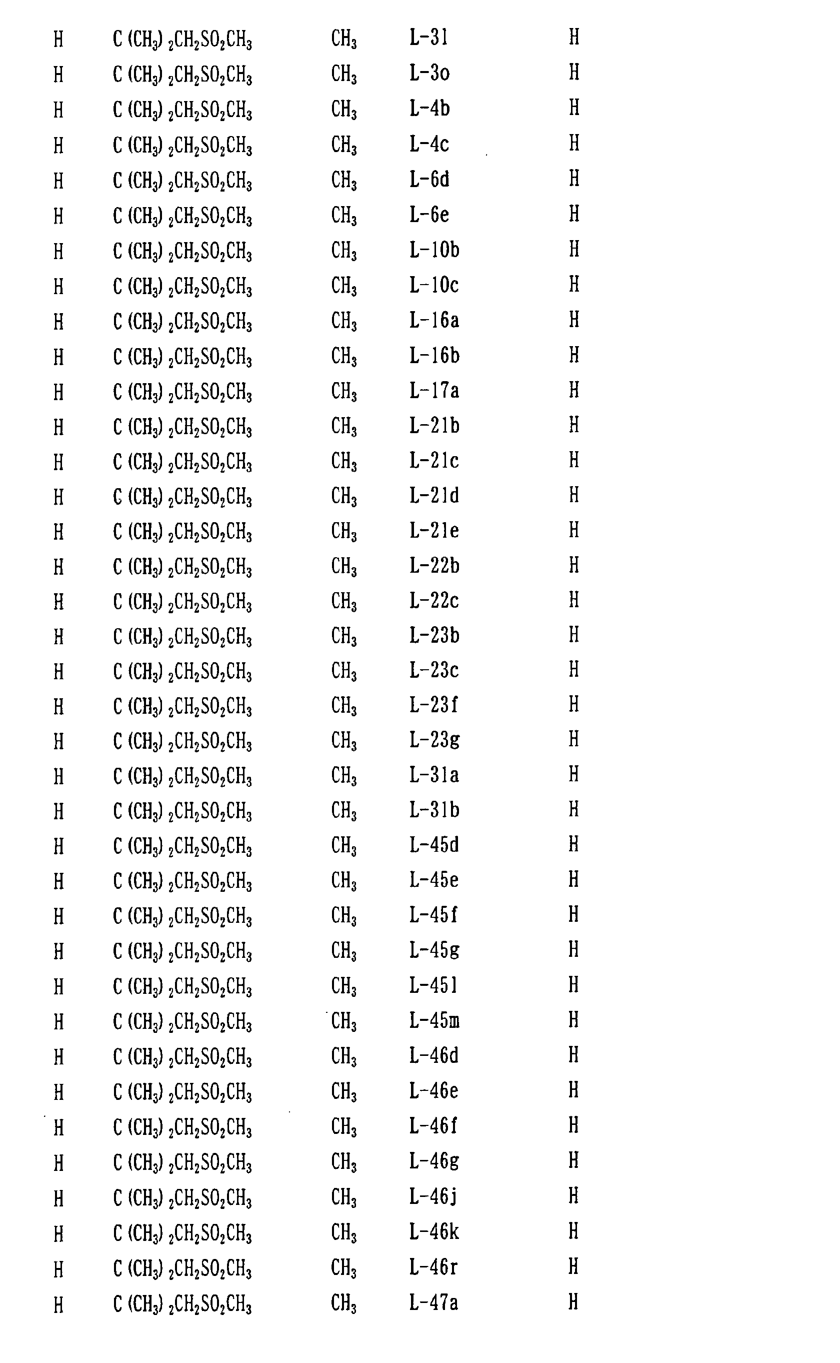

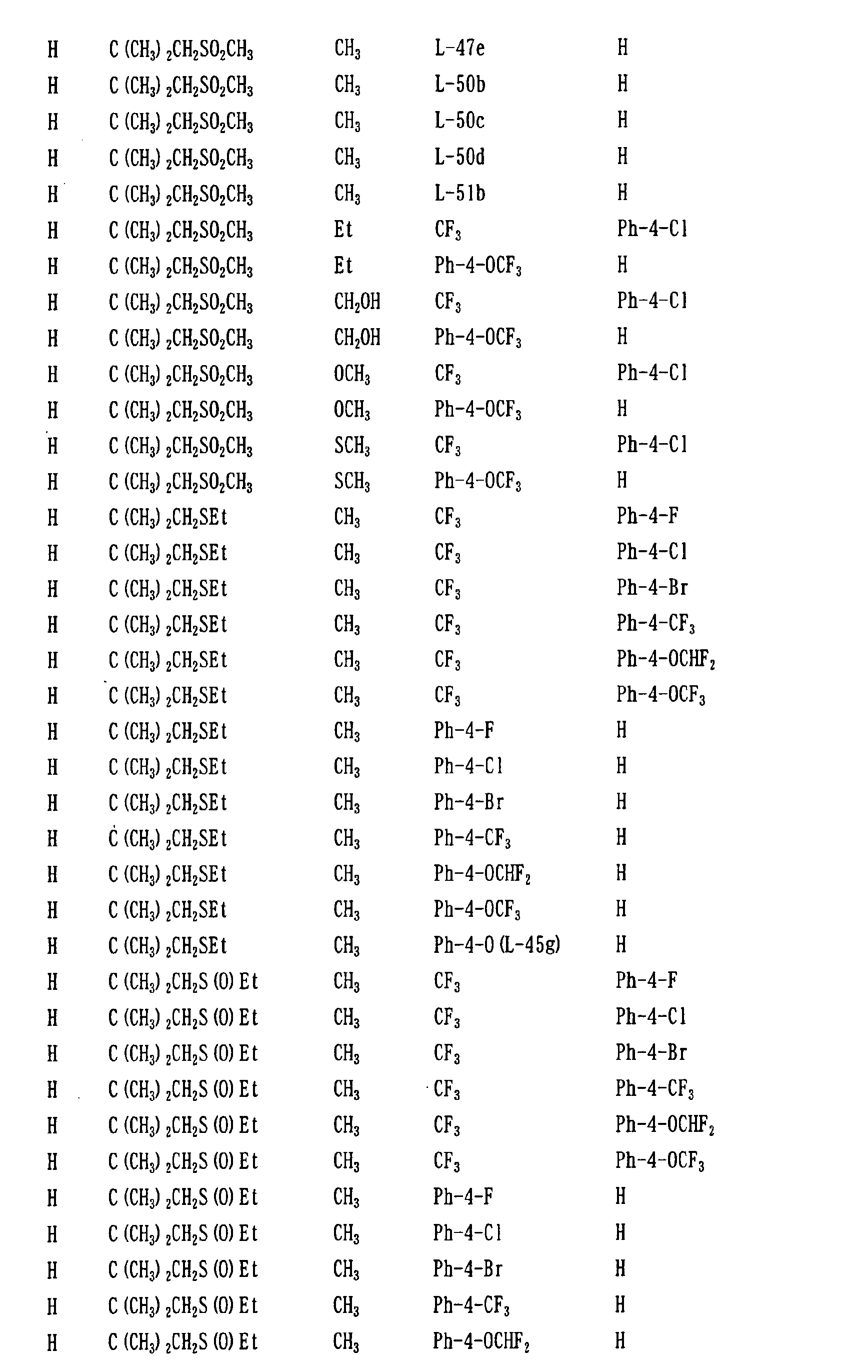

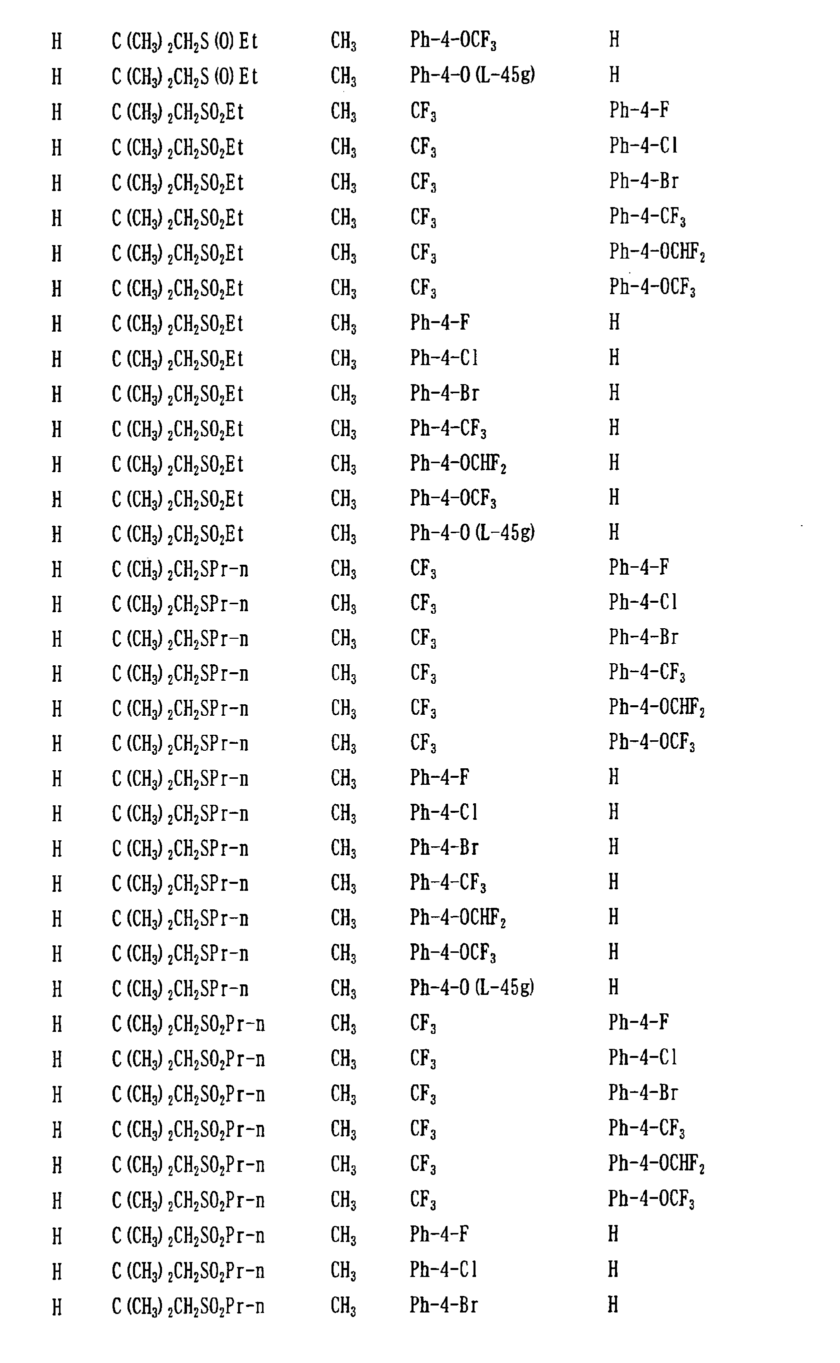

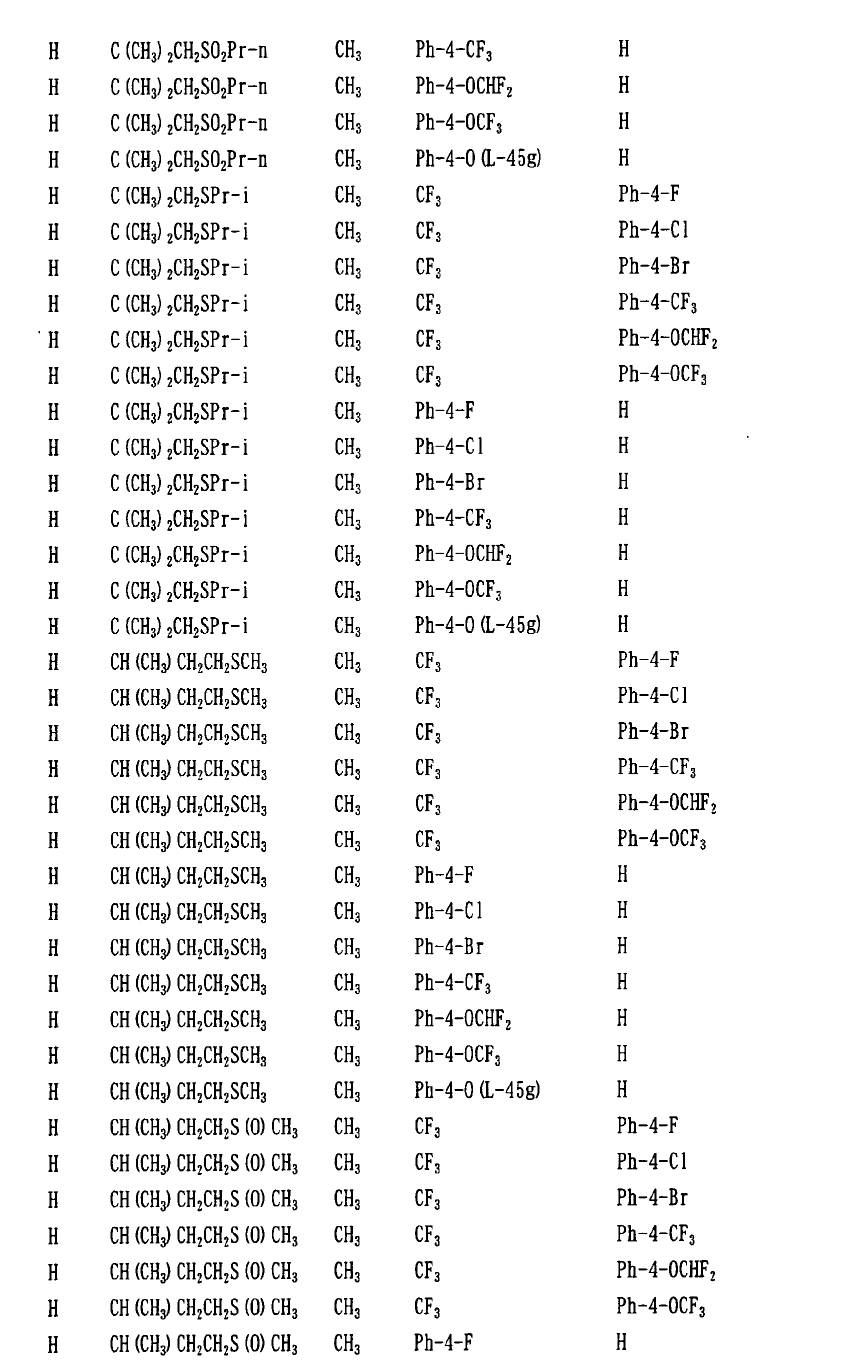

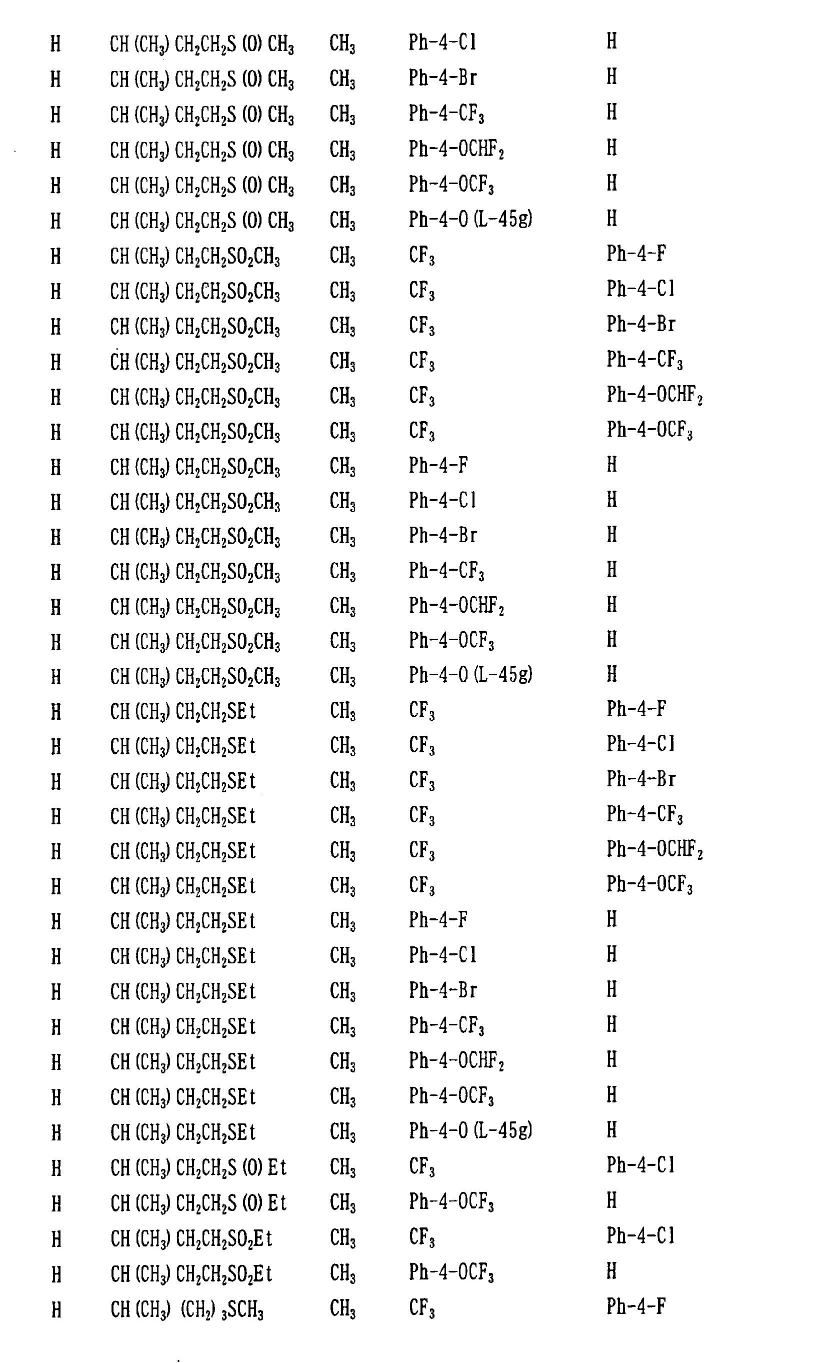

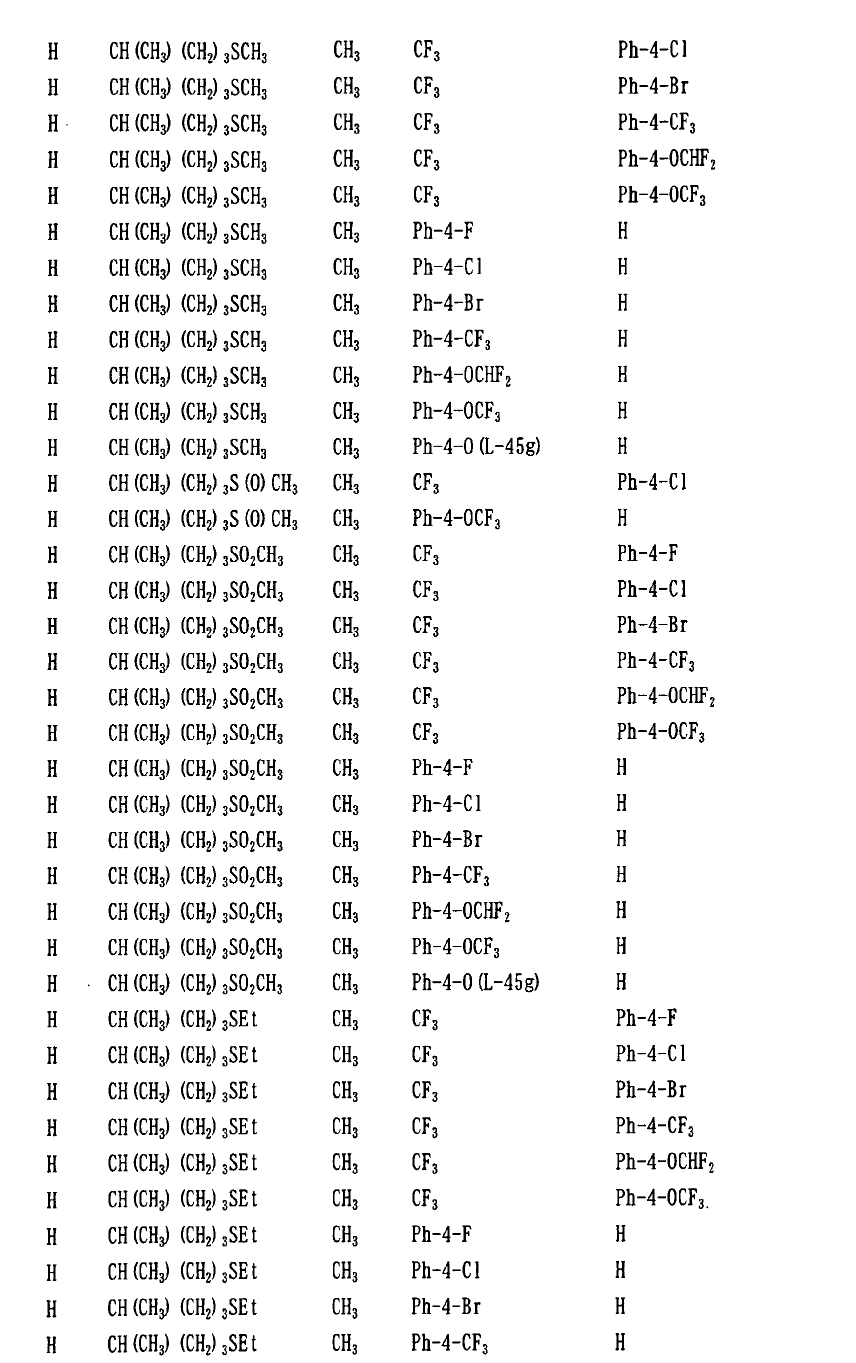

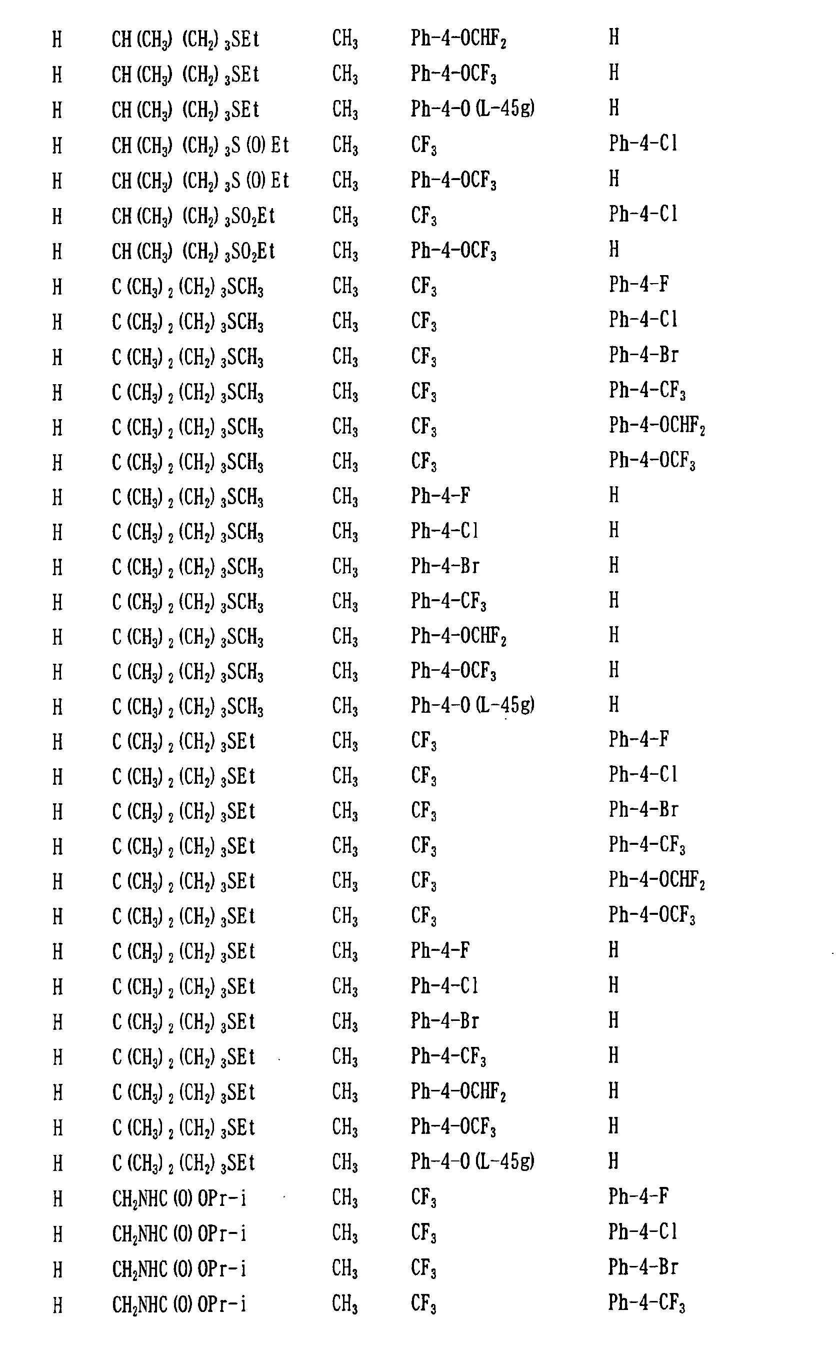

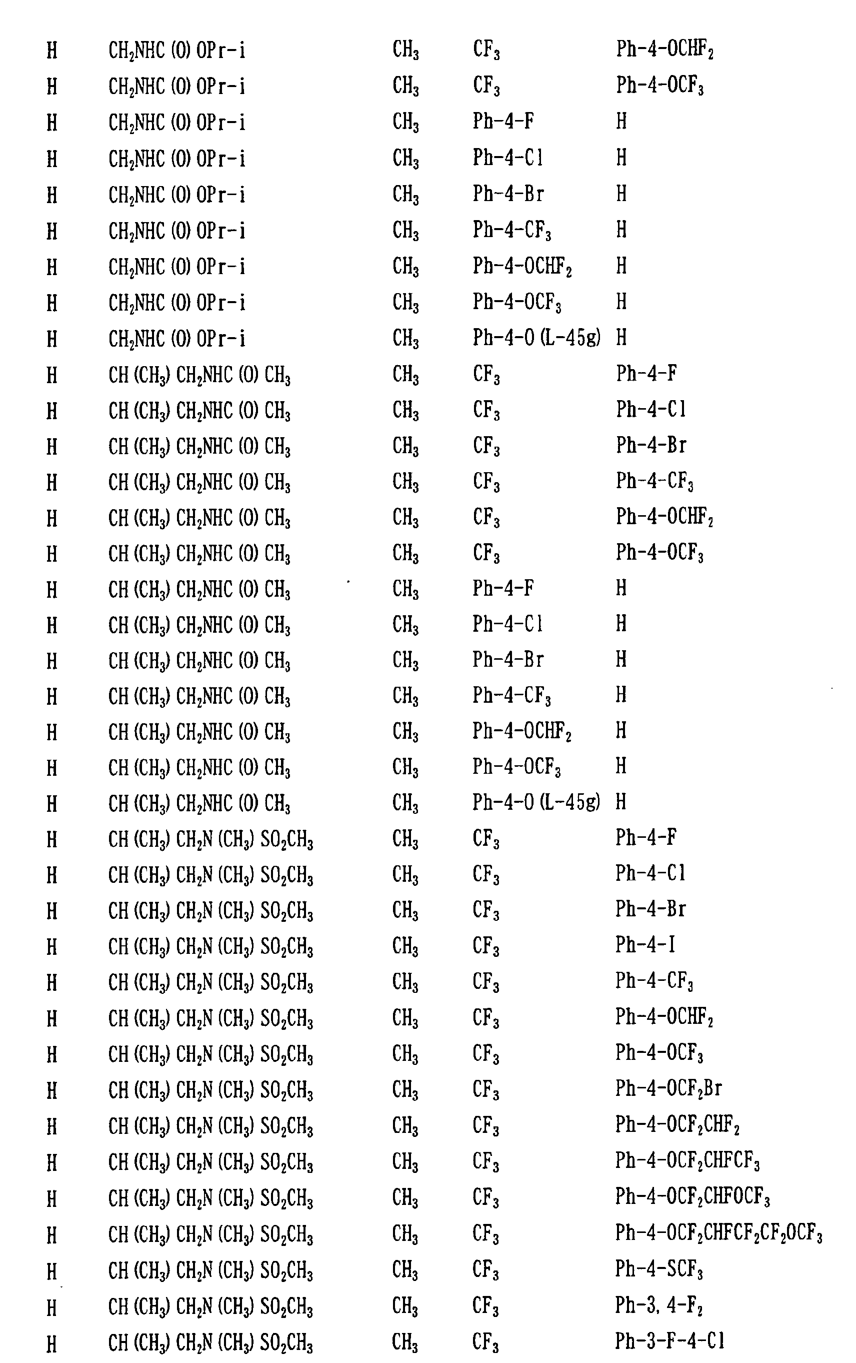

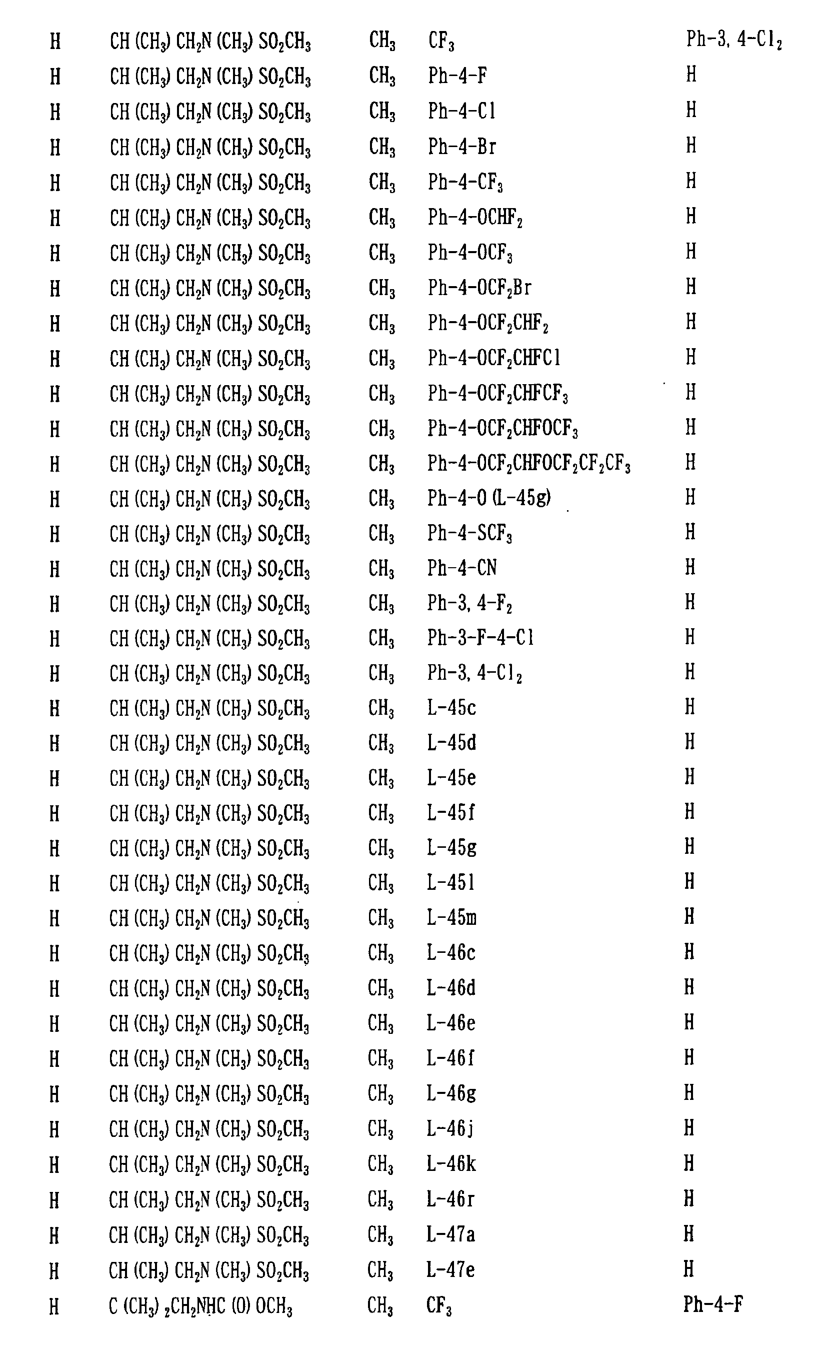

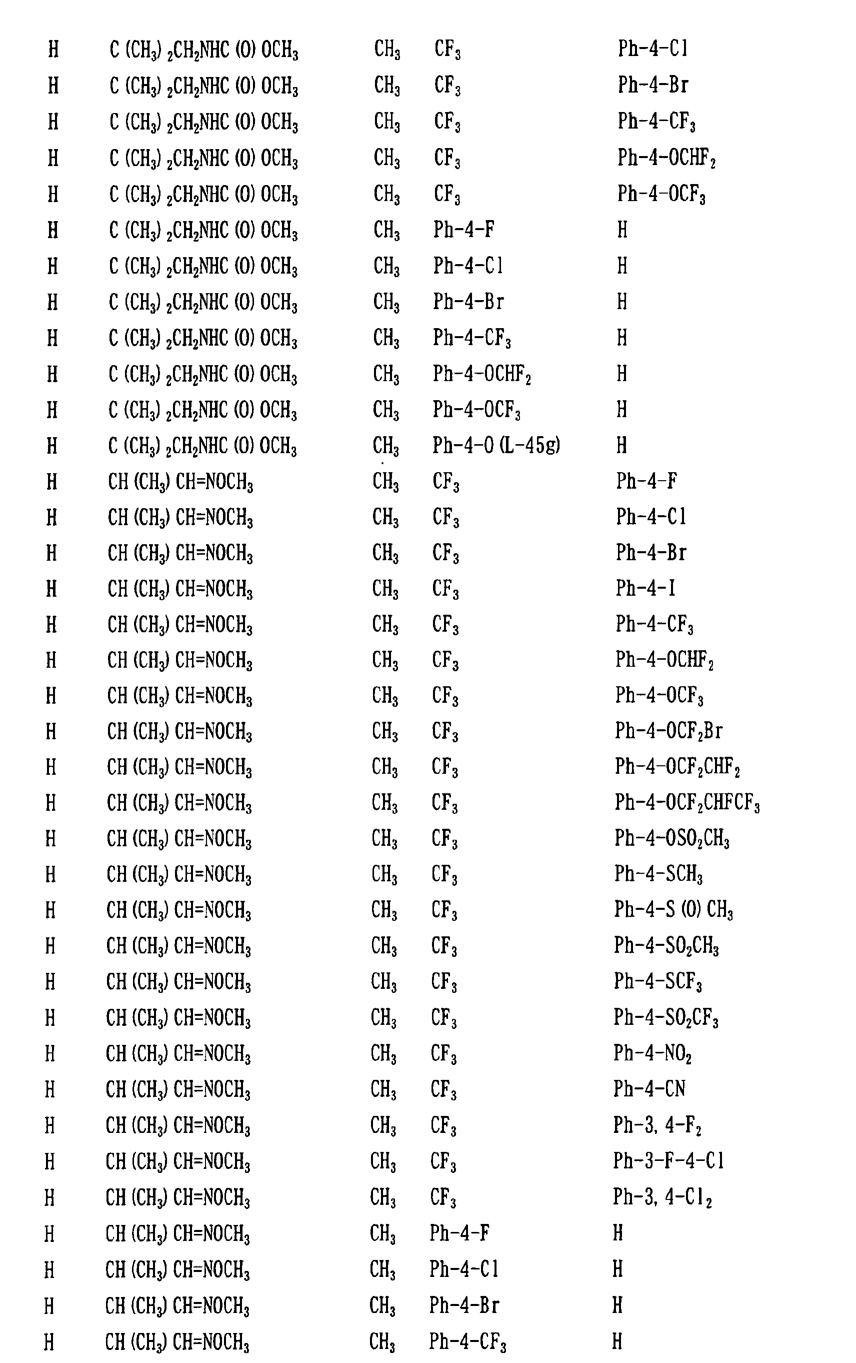

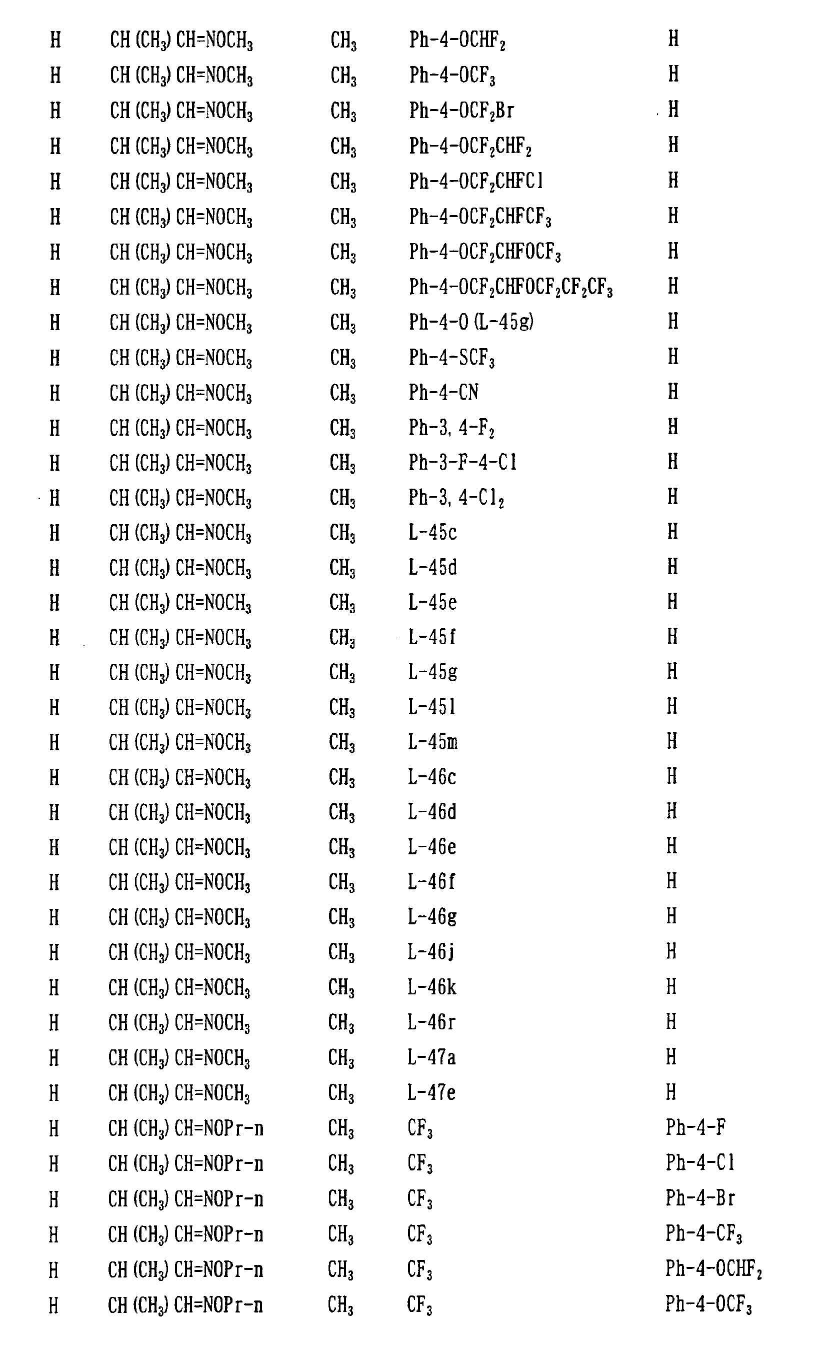

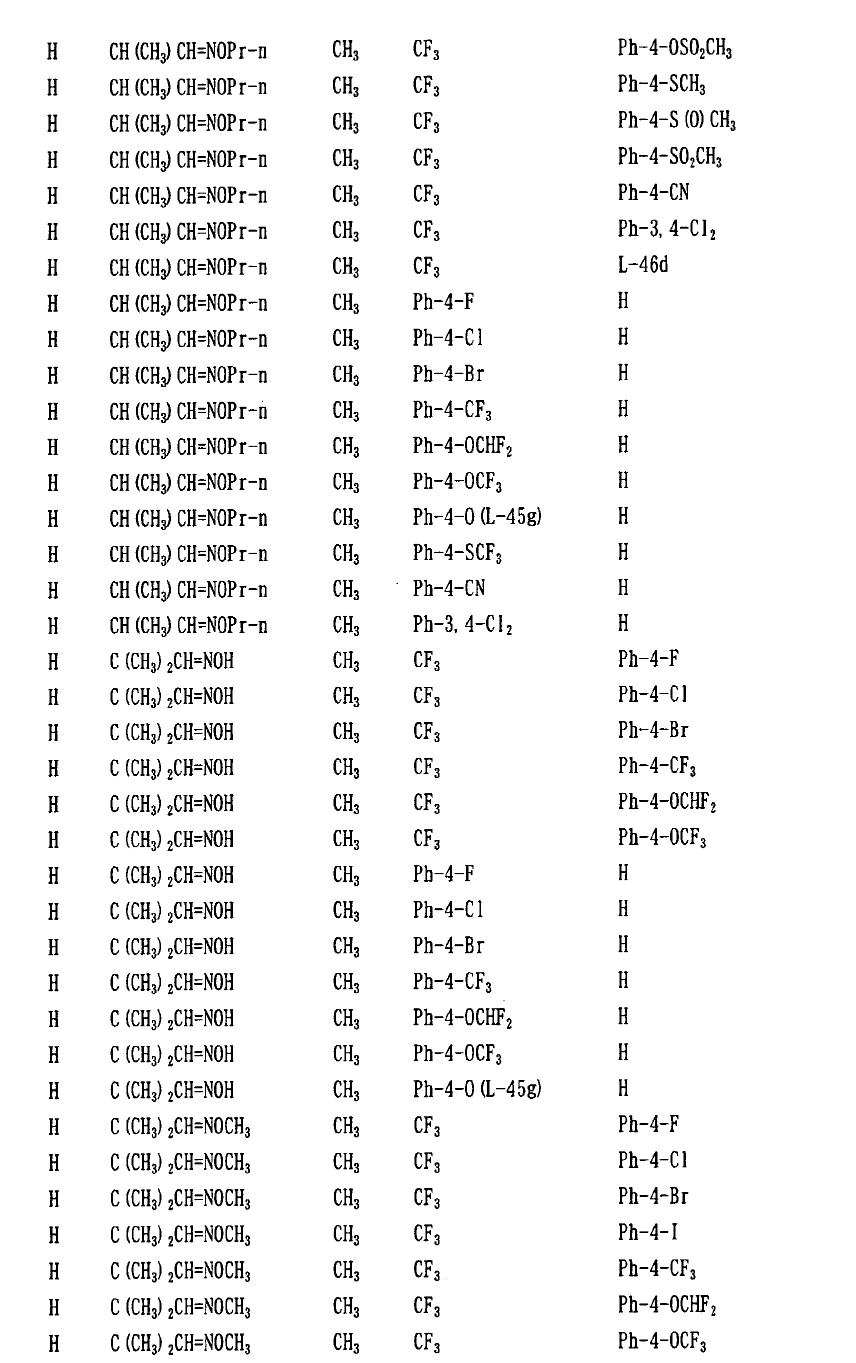

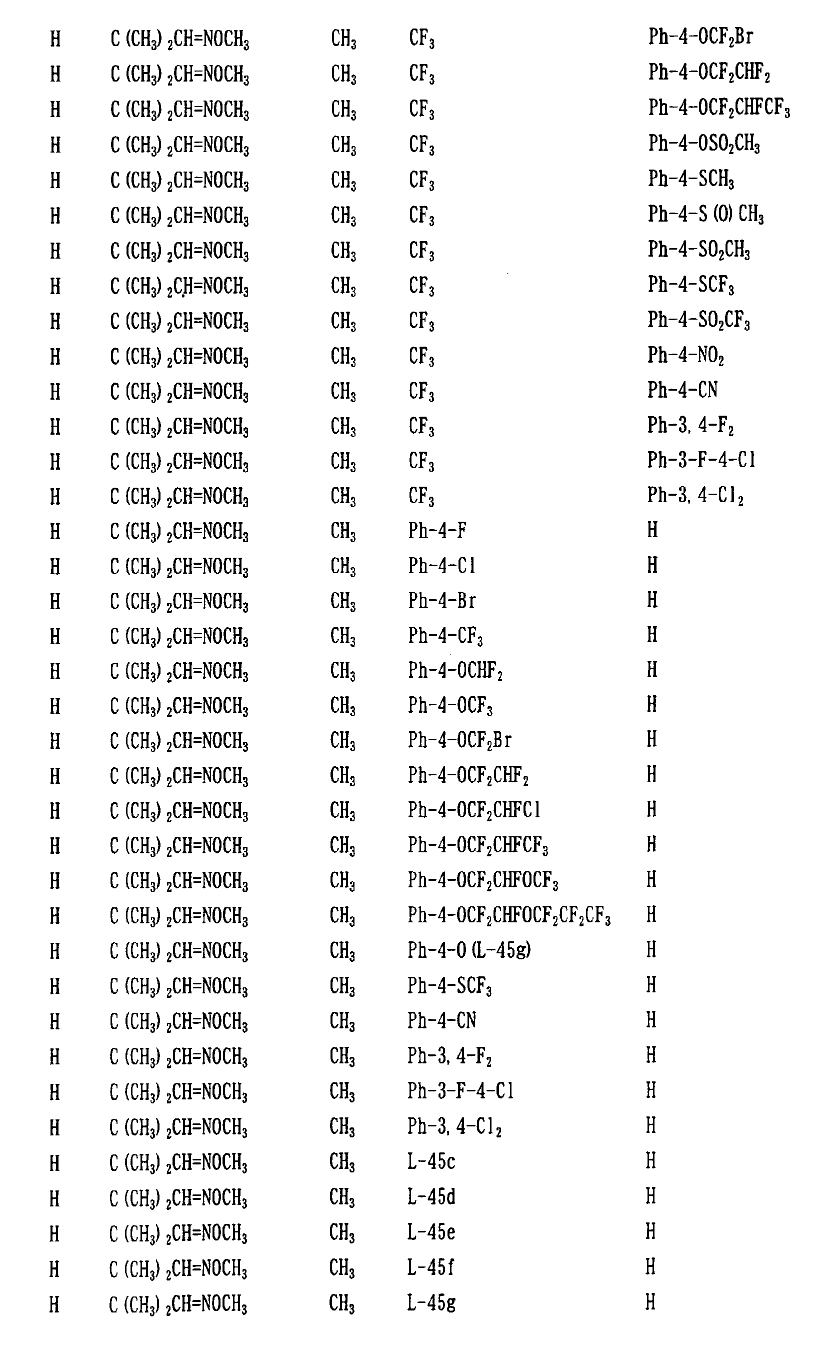

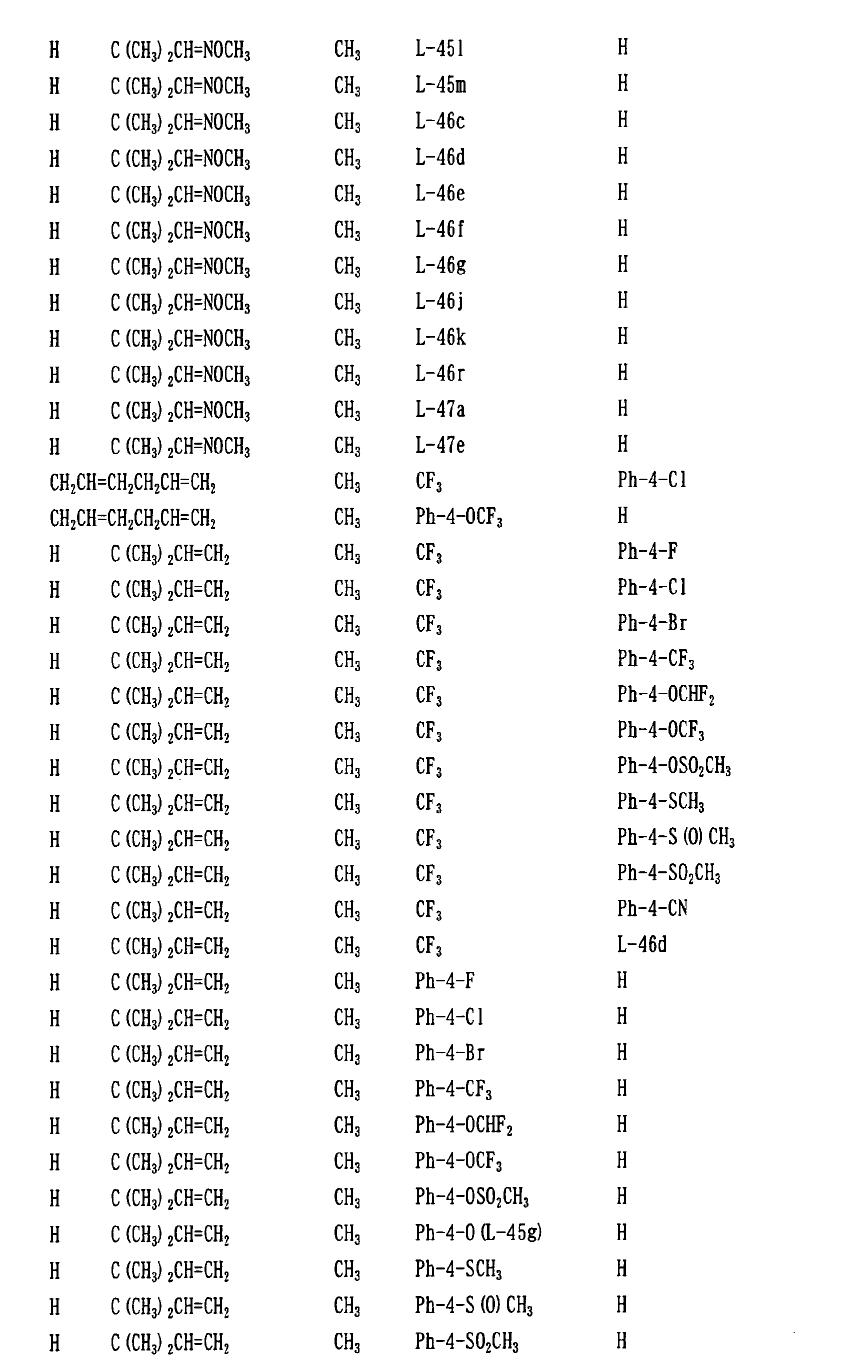

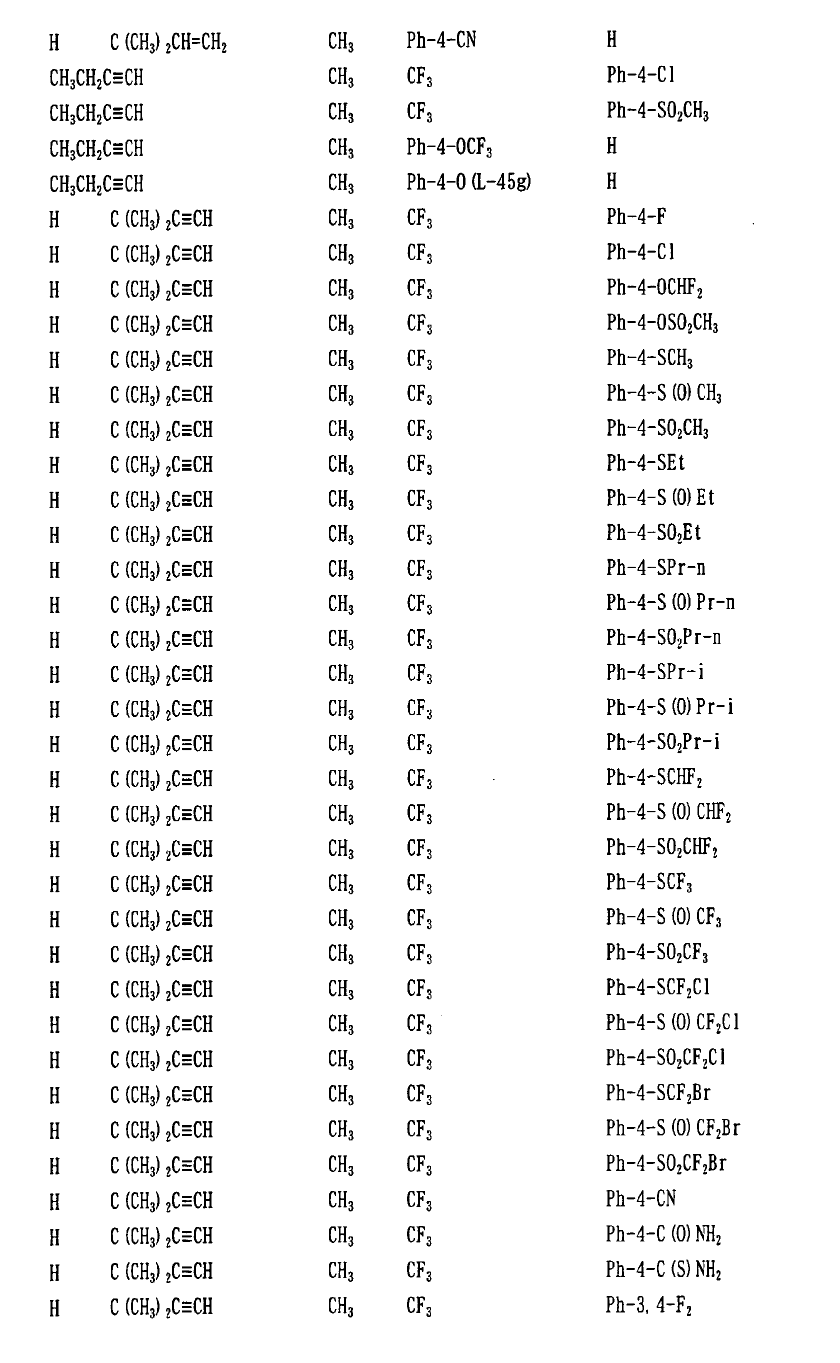

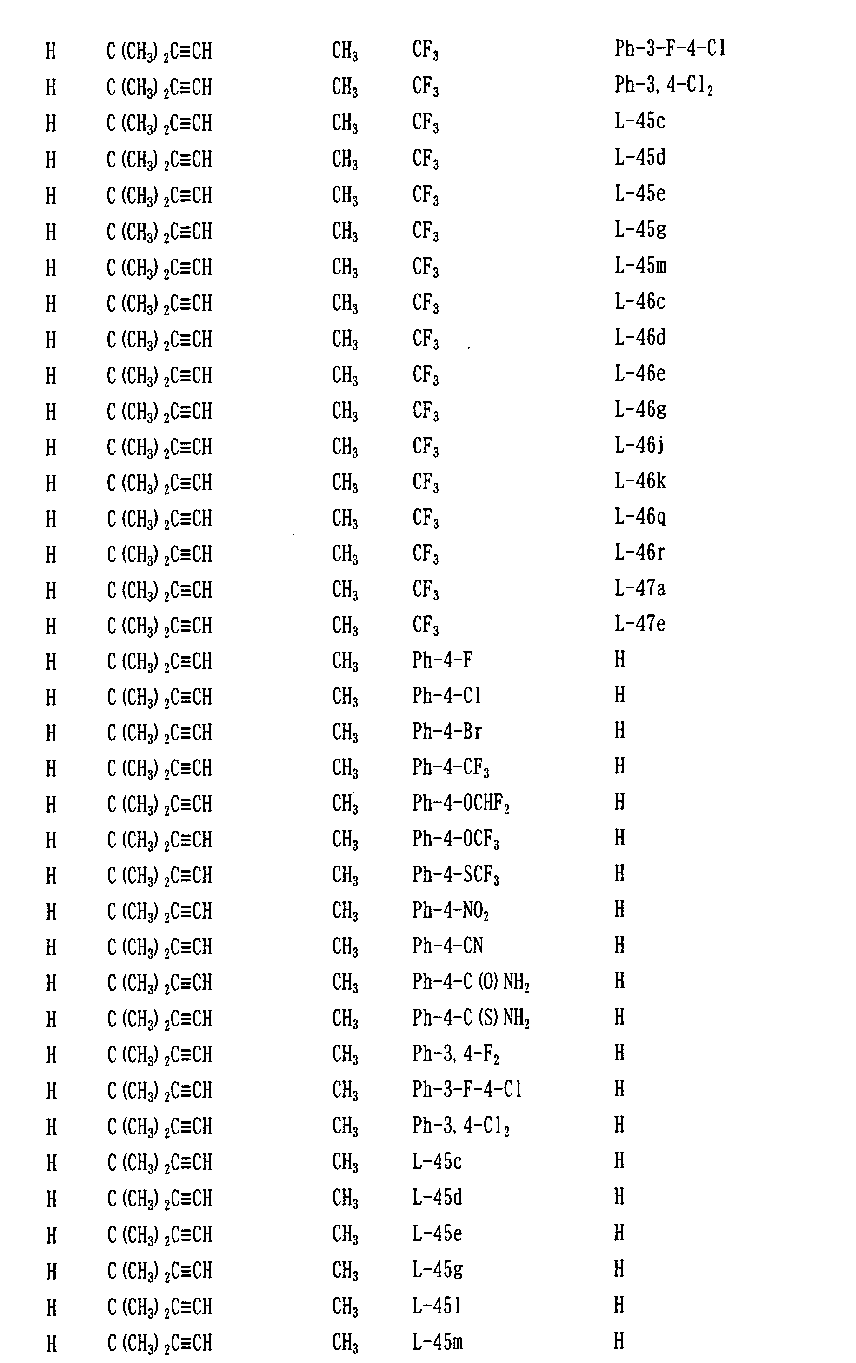

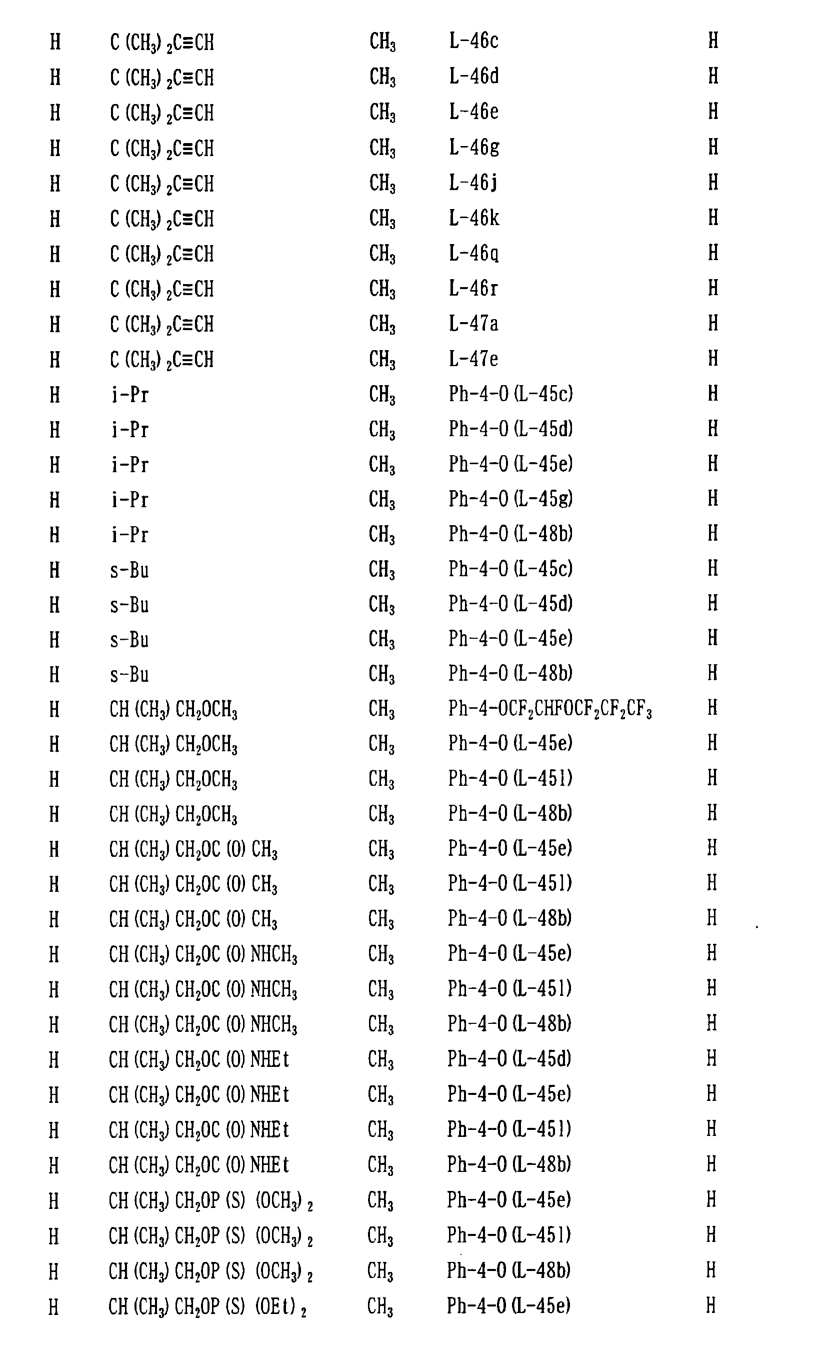

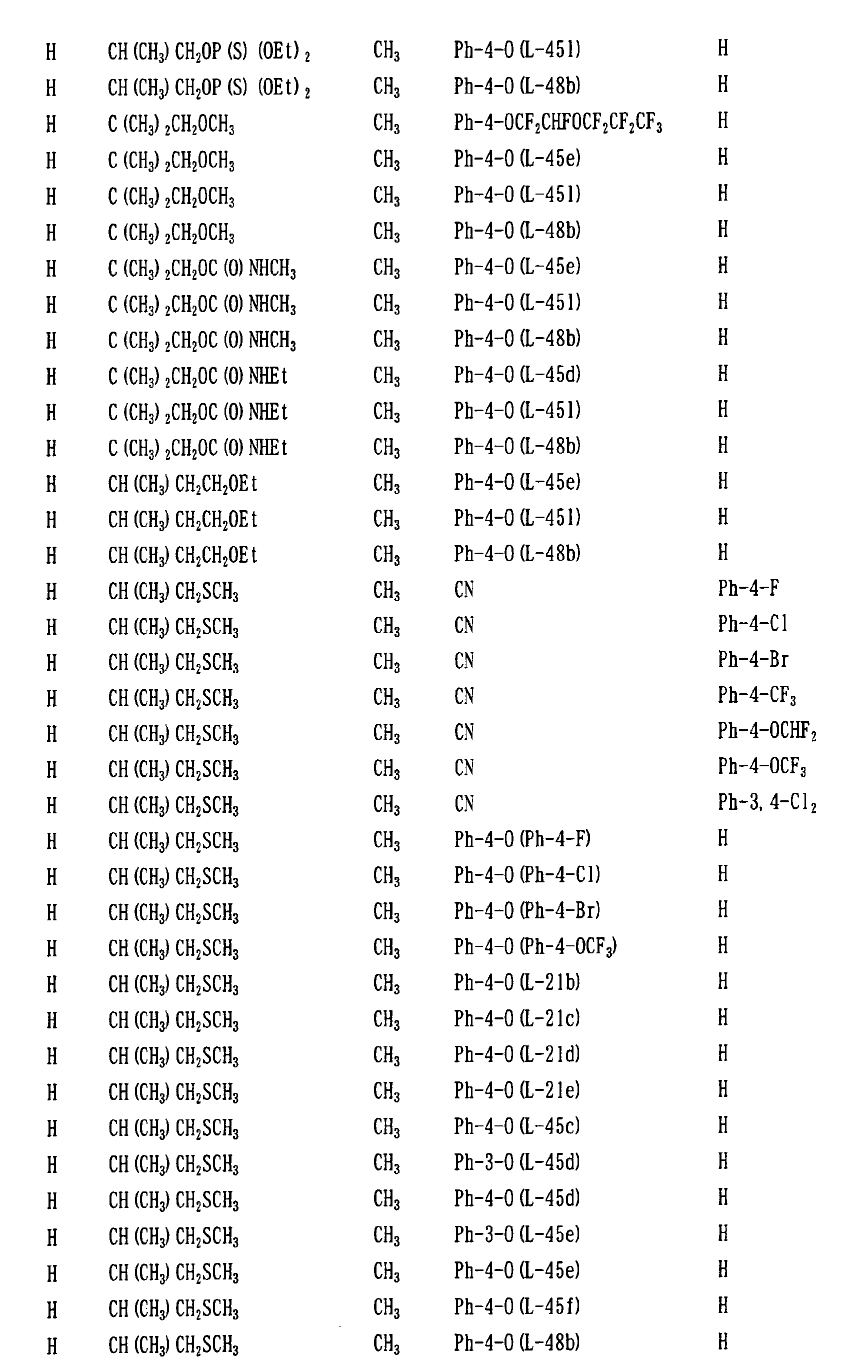

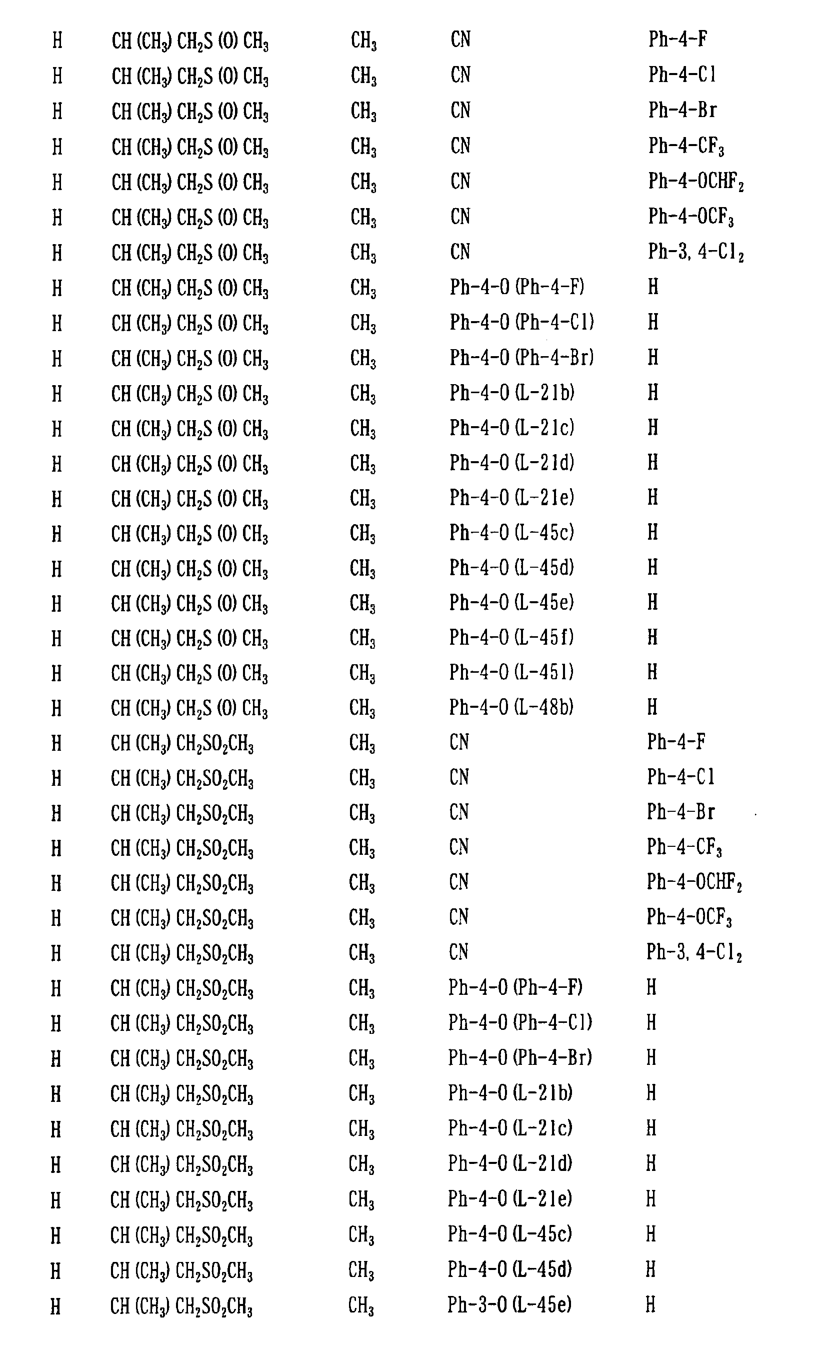

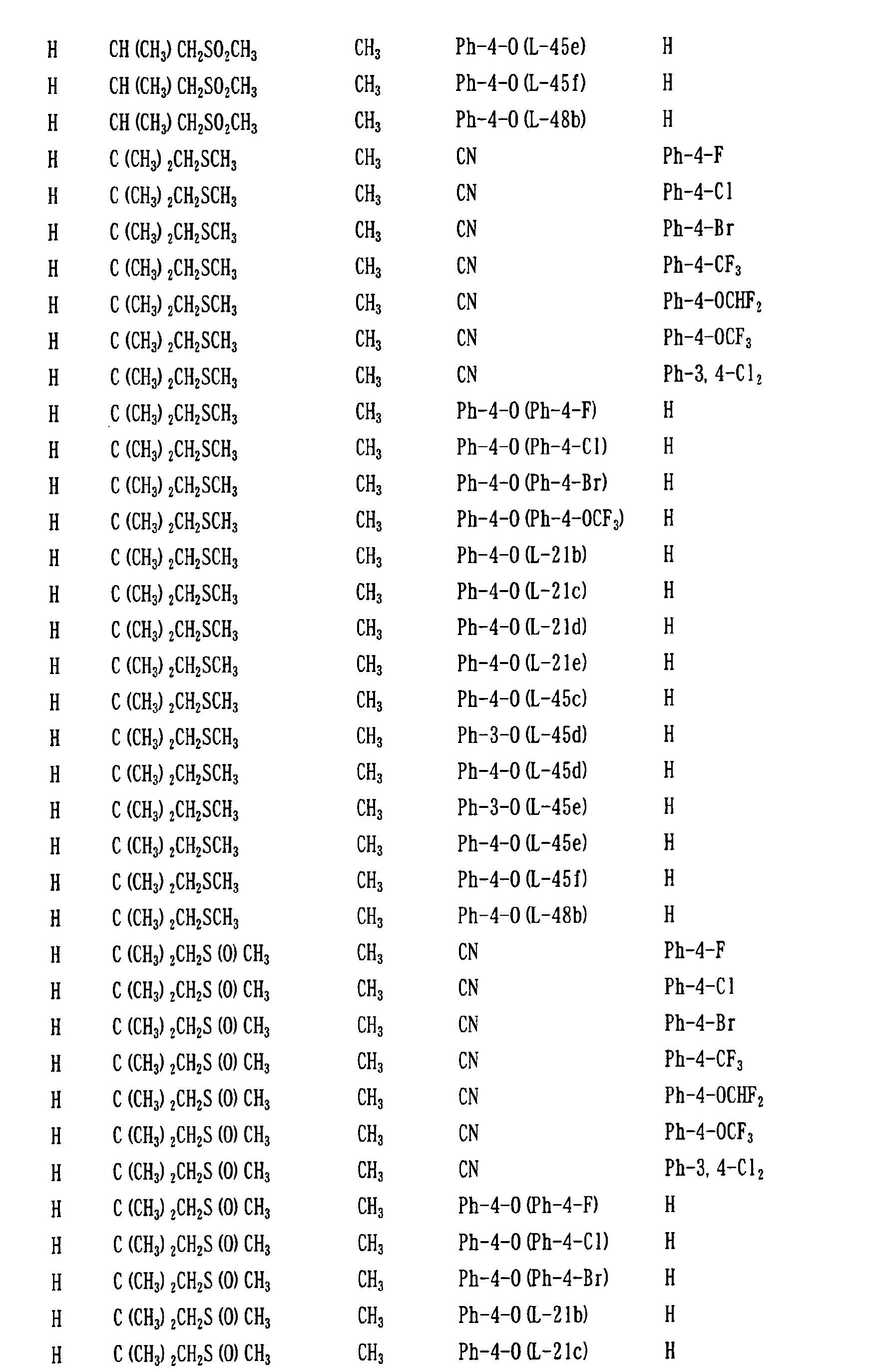

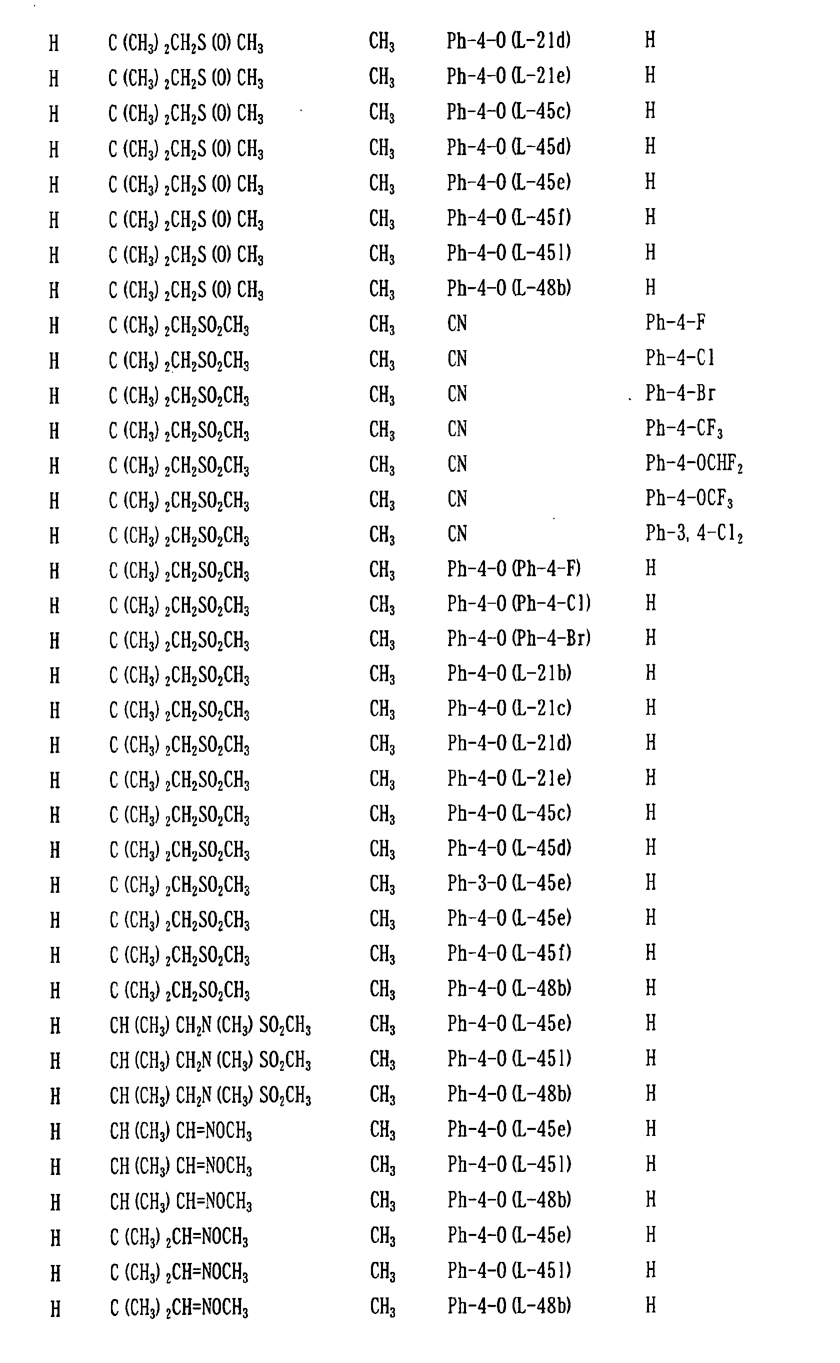

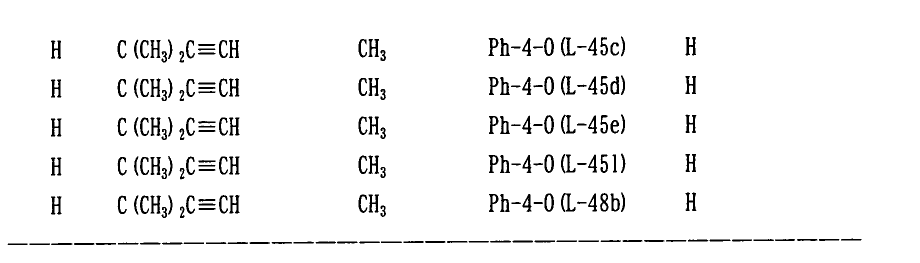

In Table 5 mentioned below, the symbols showing the positions of the substituents (X)ₘ and (Y)ₙ correspond to the positions shown in the following structural formulae, respectively.

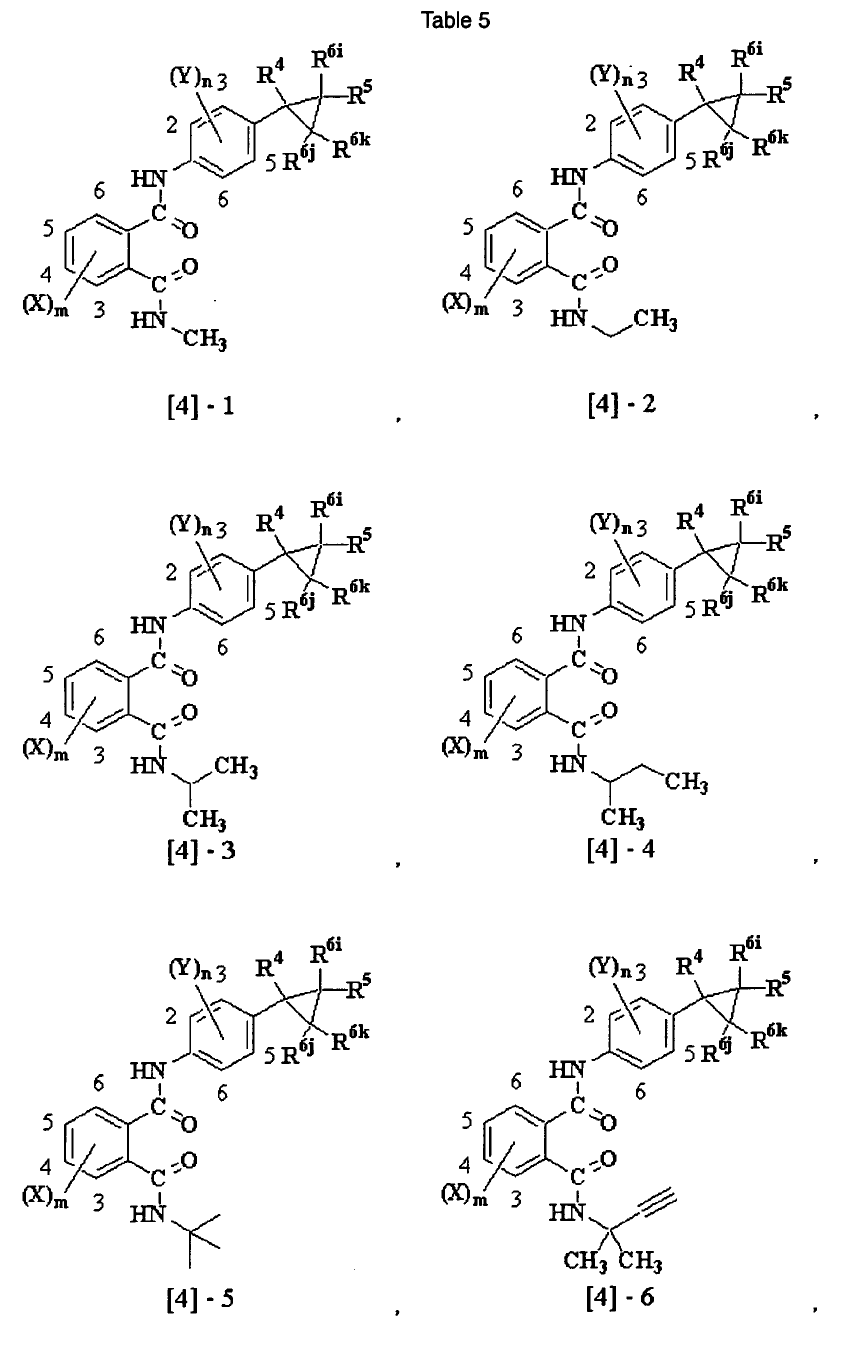

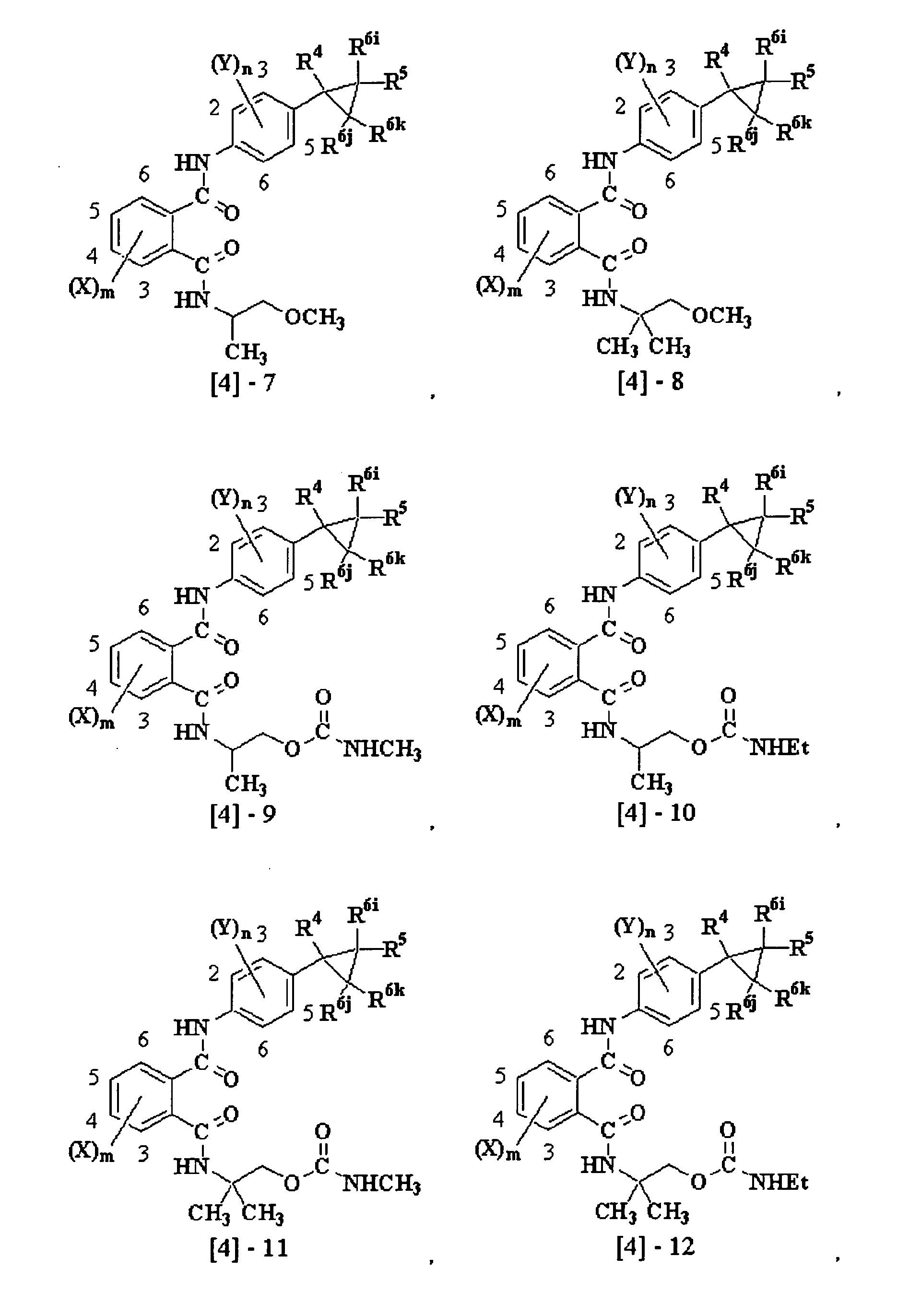

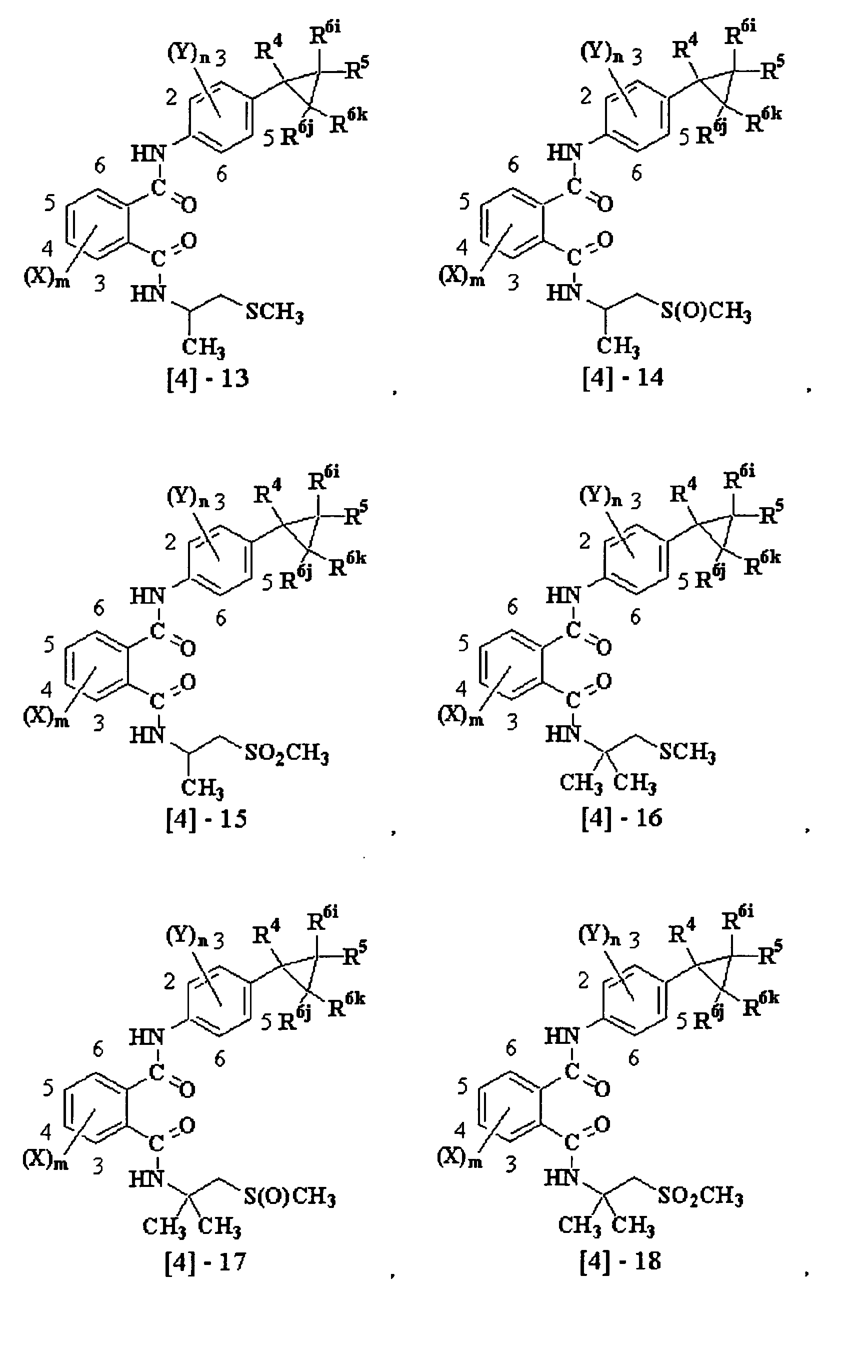

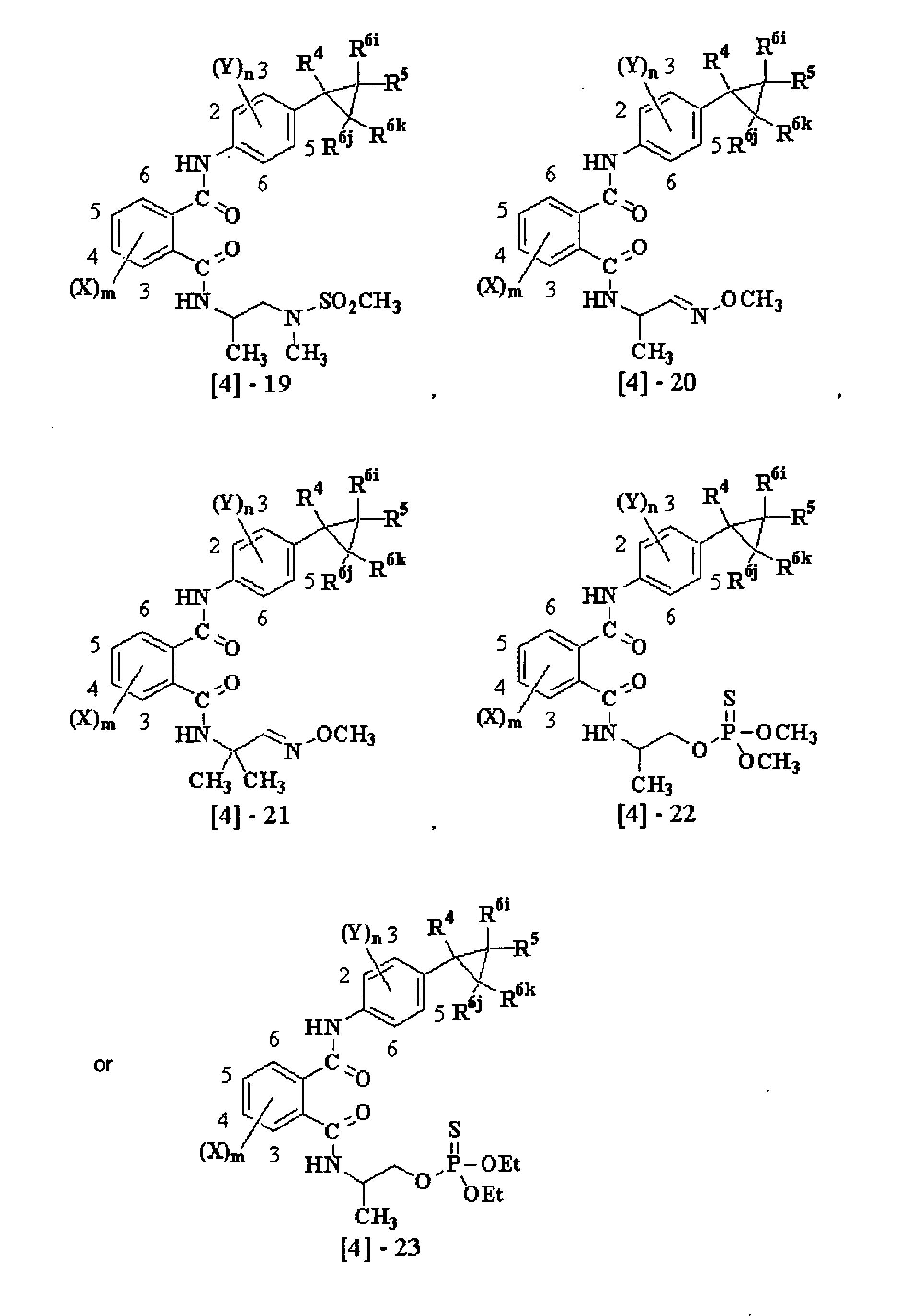

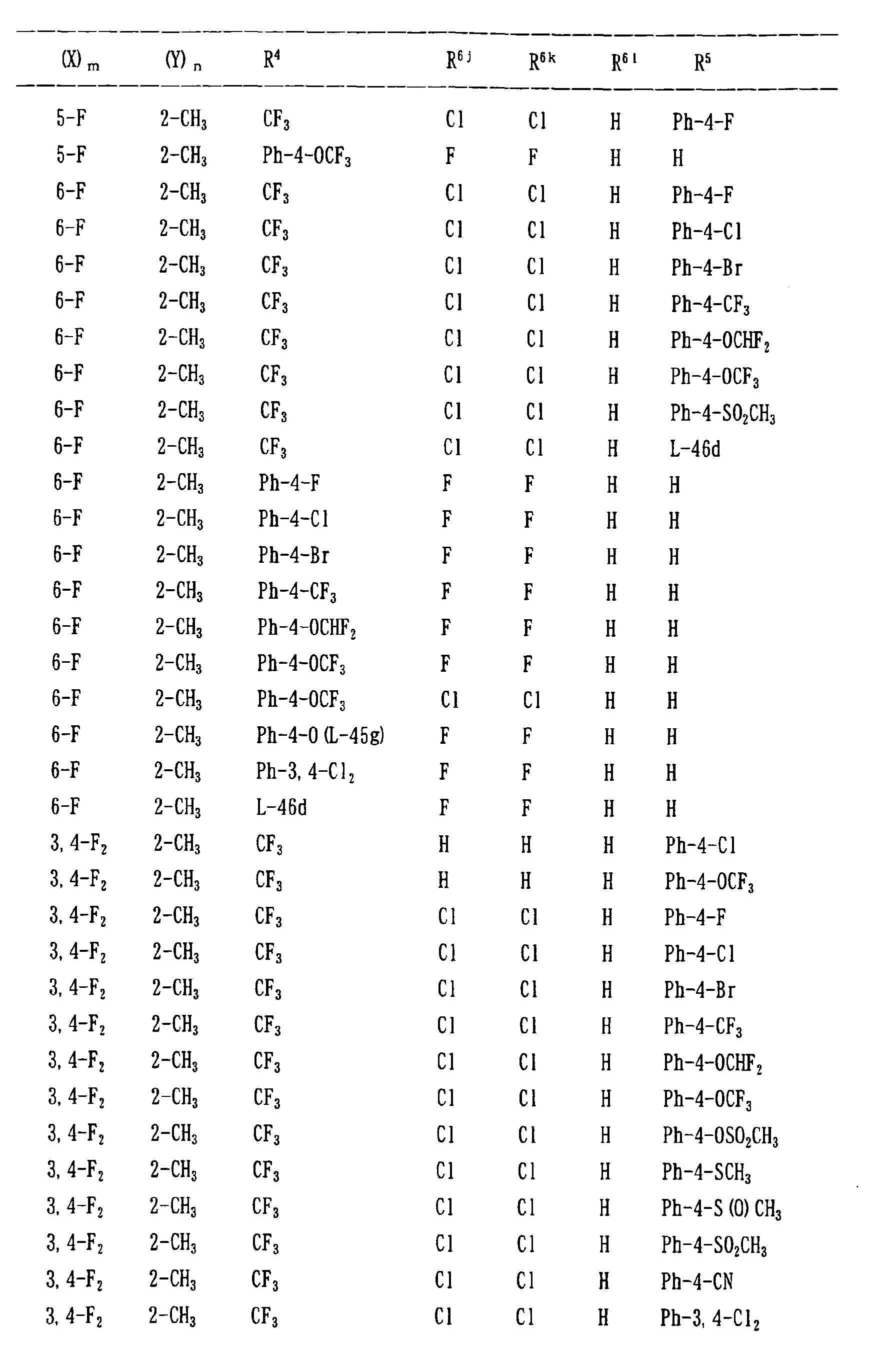

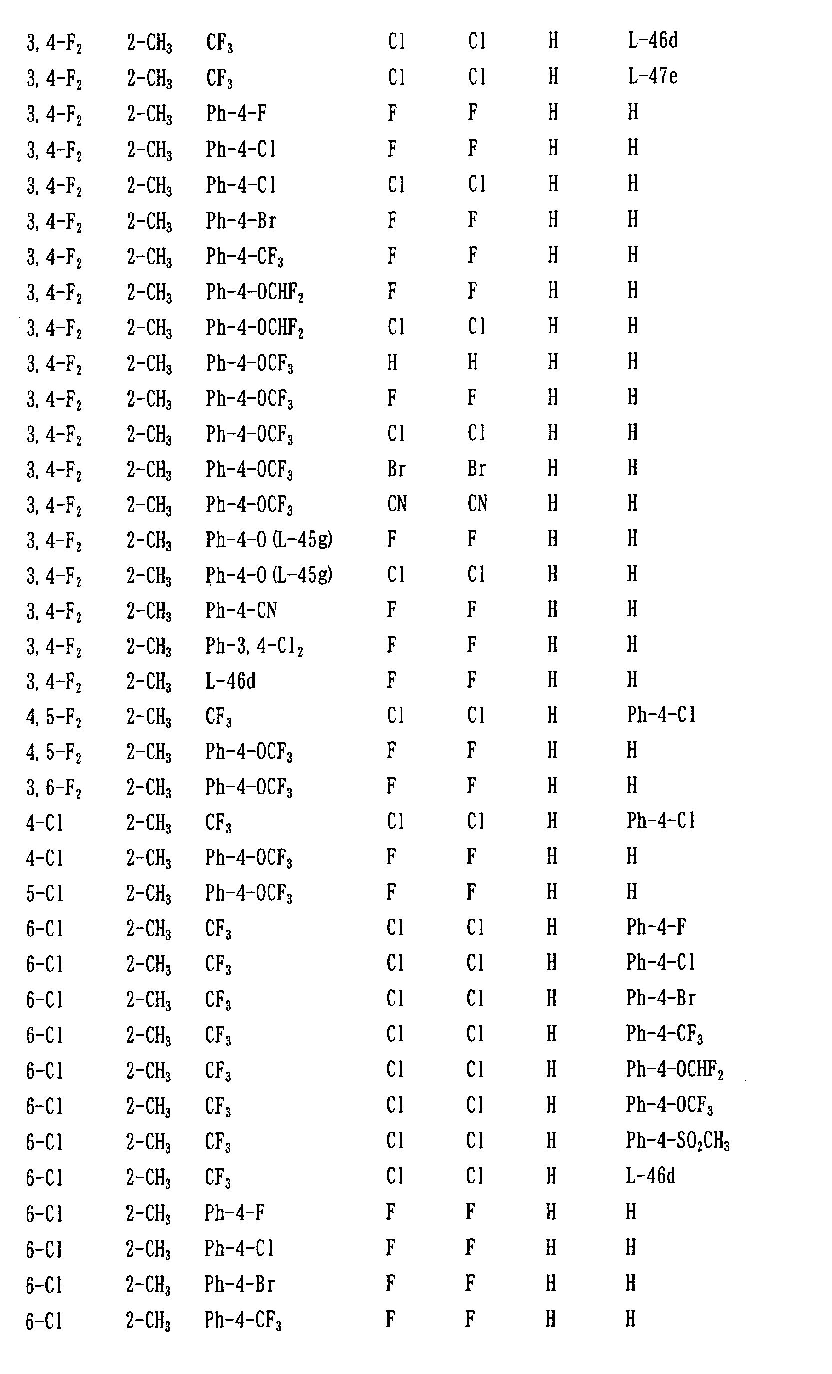

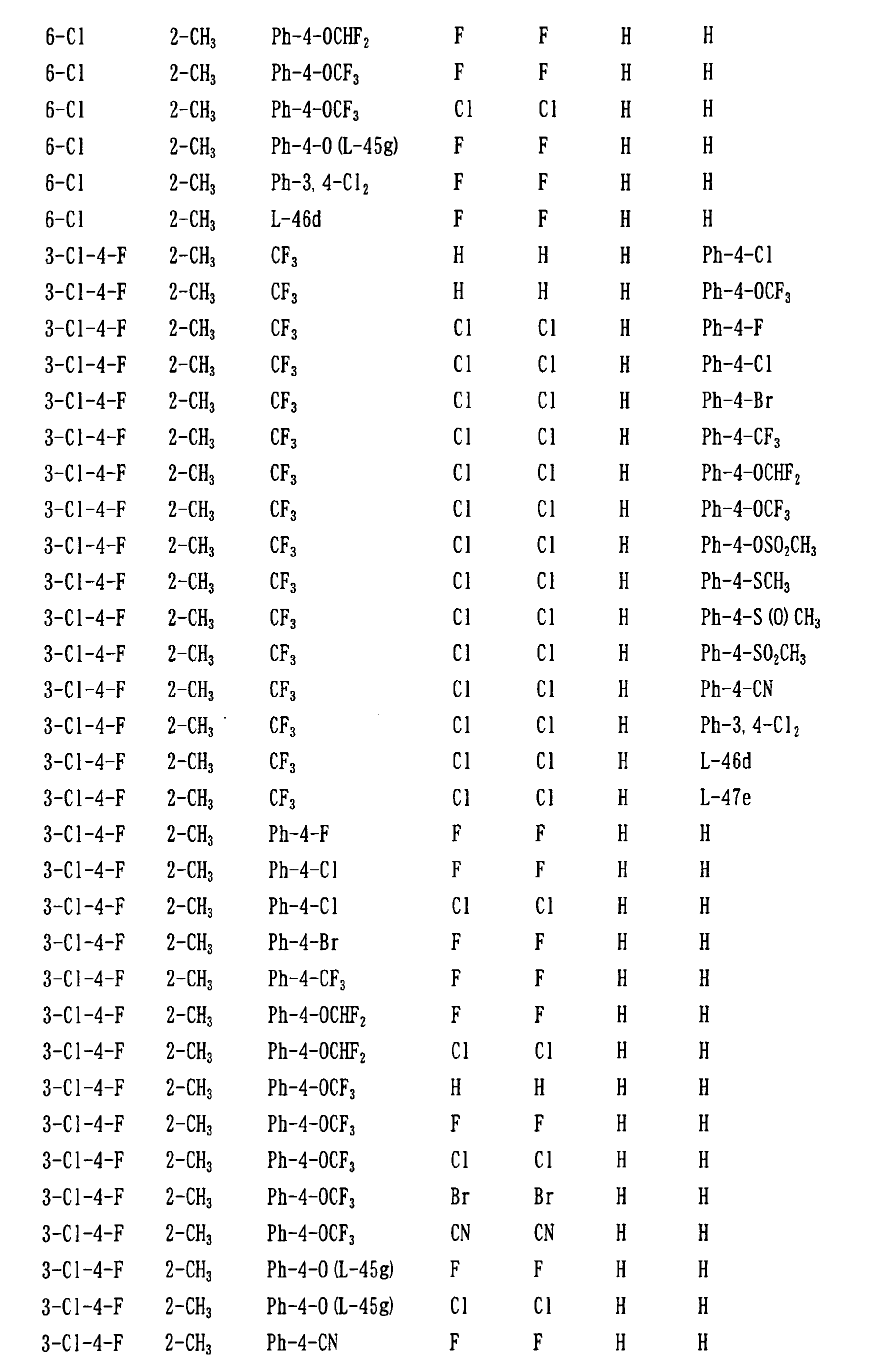

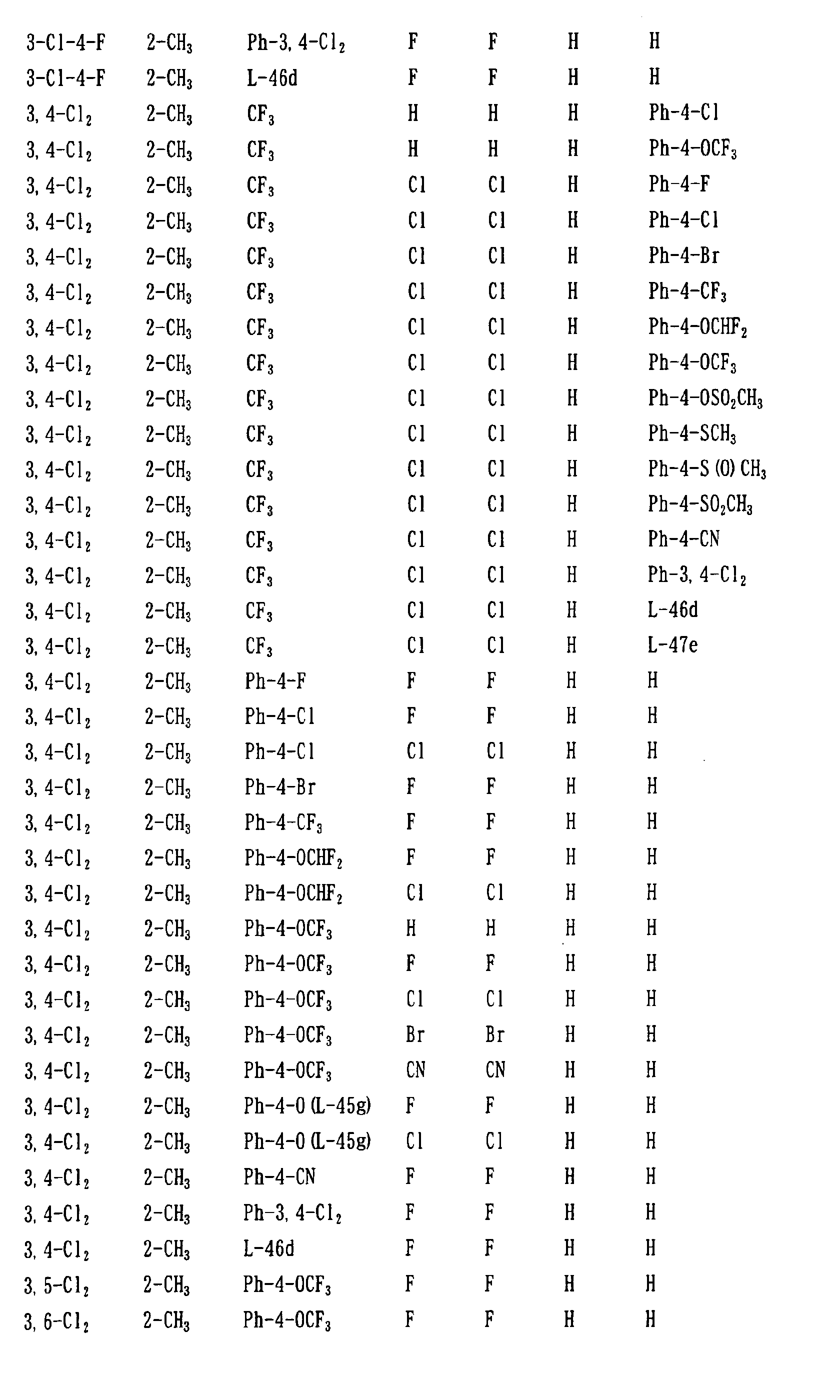

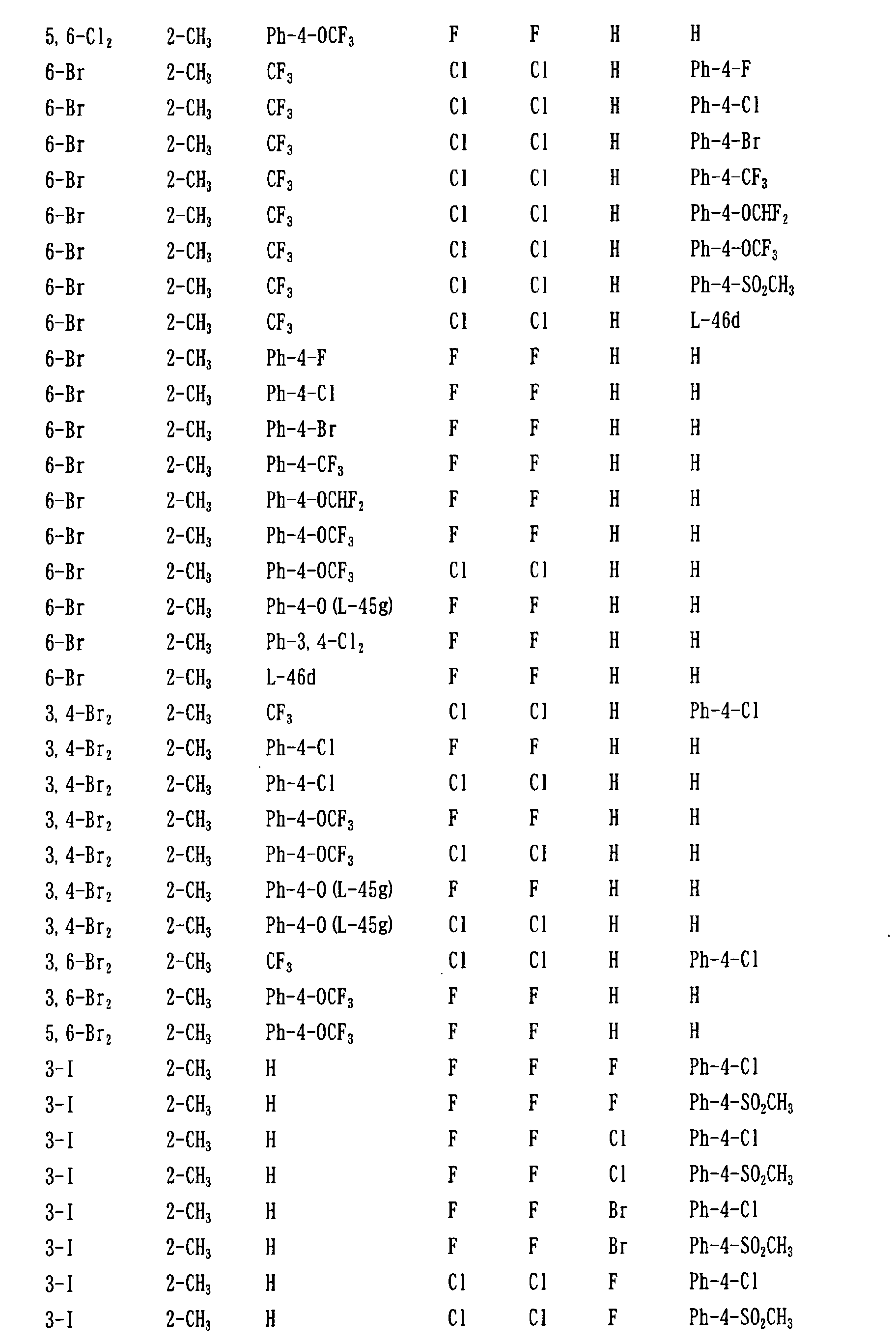

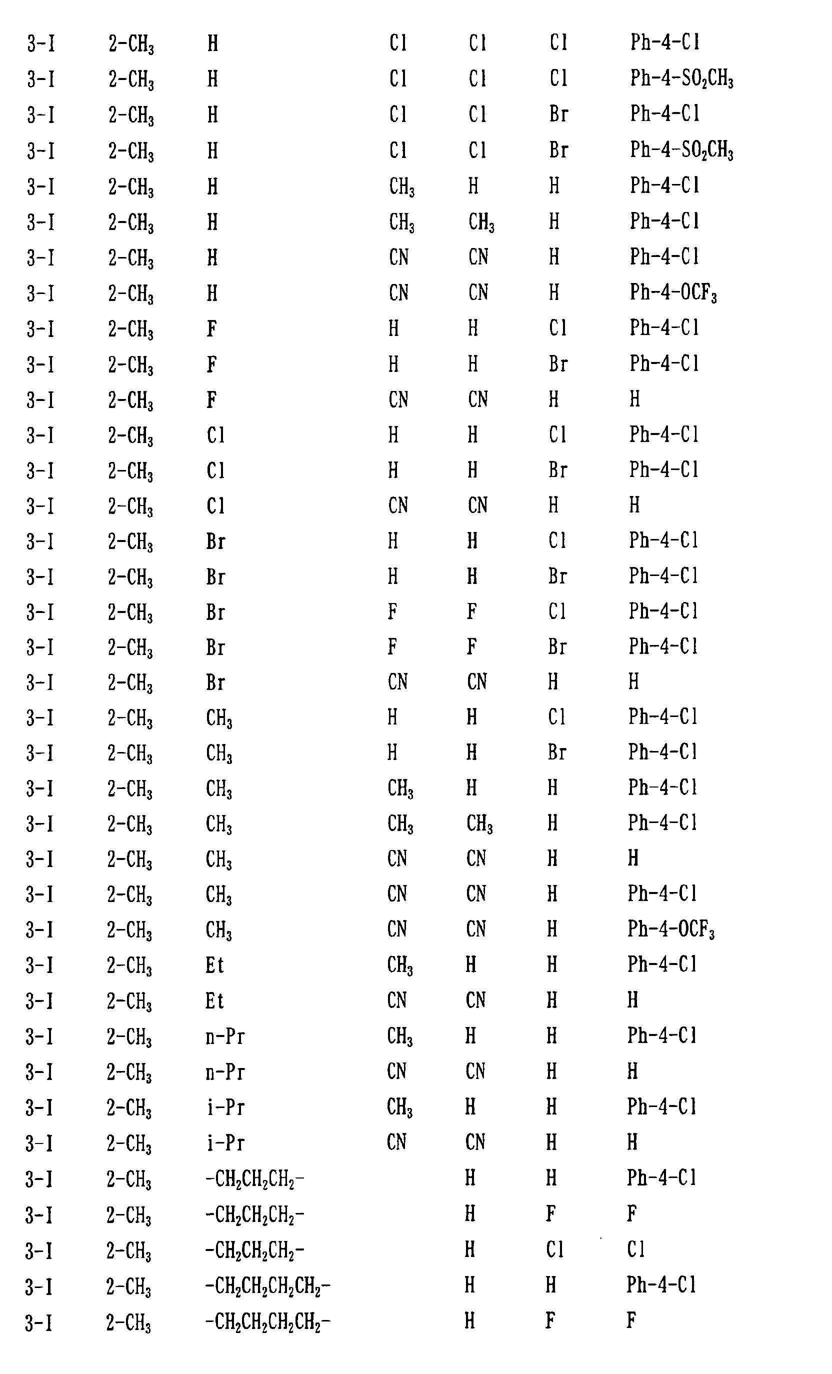

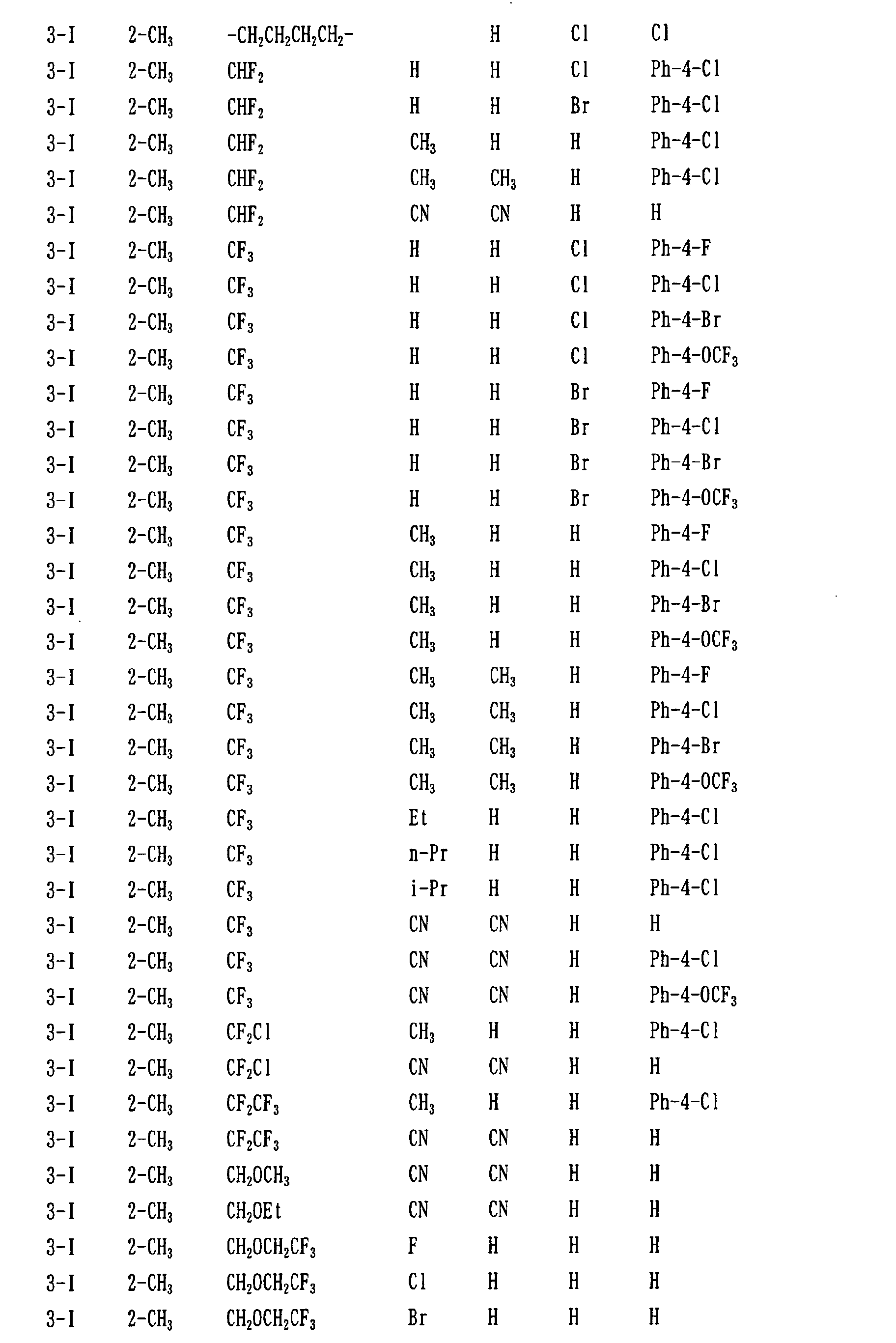

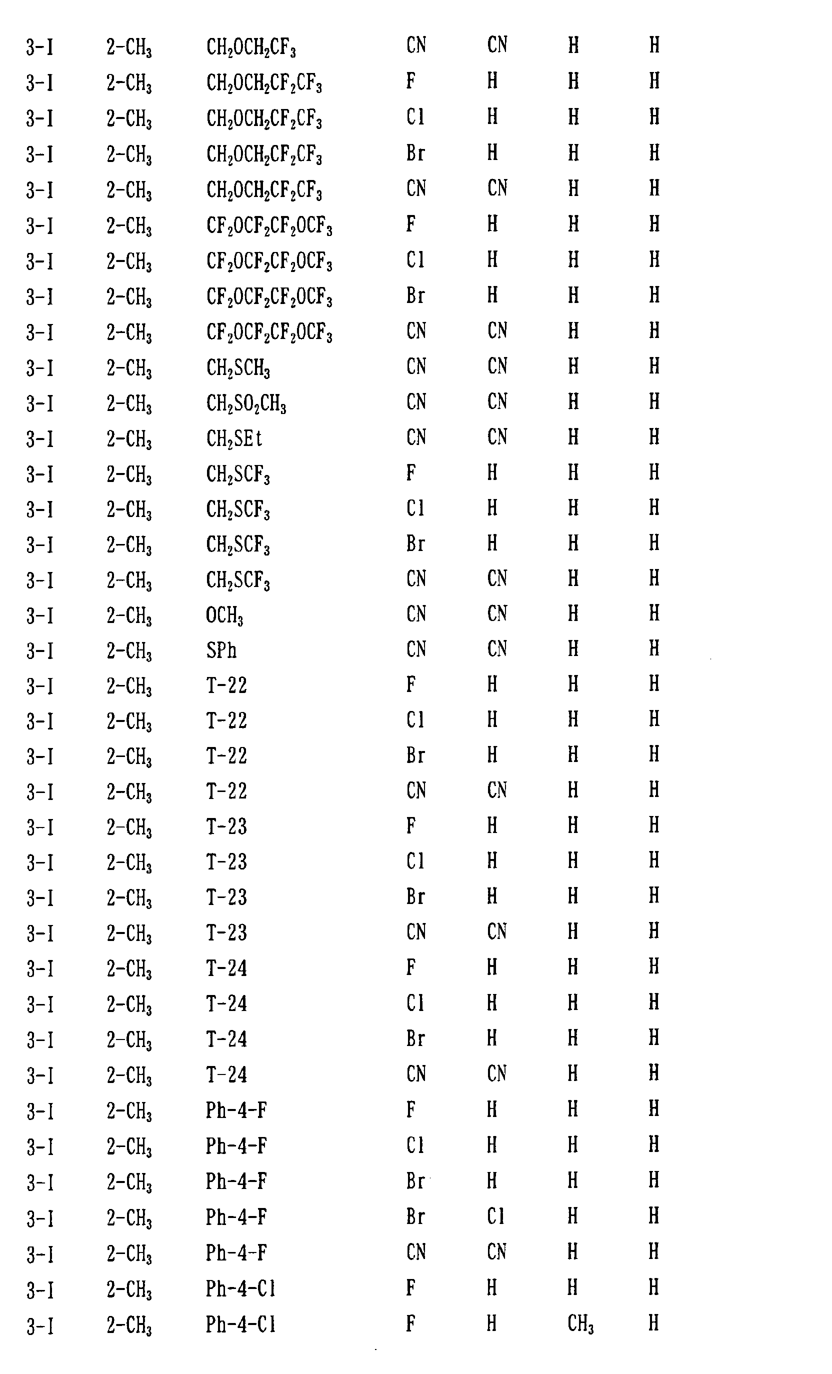

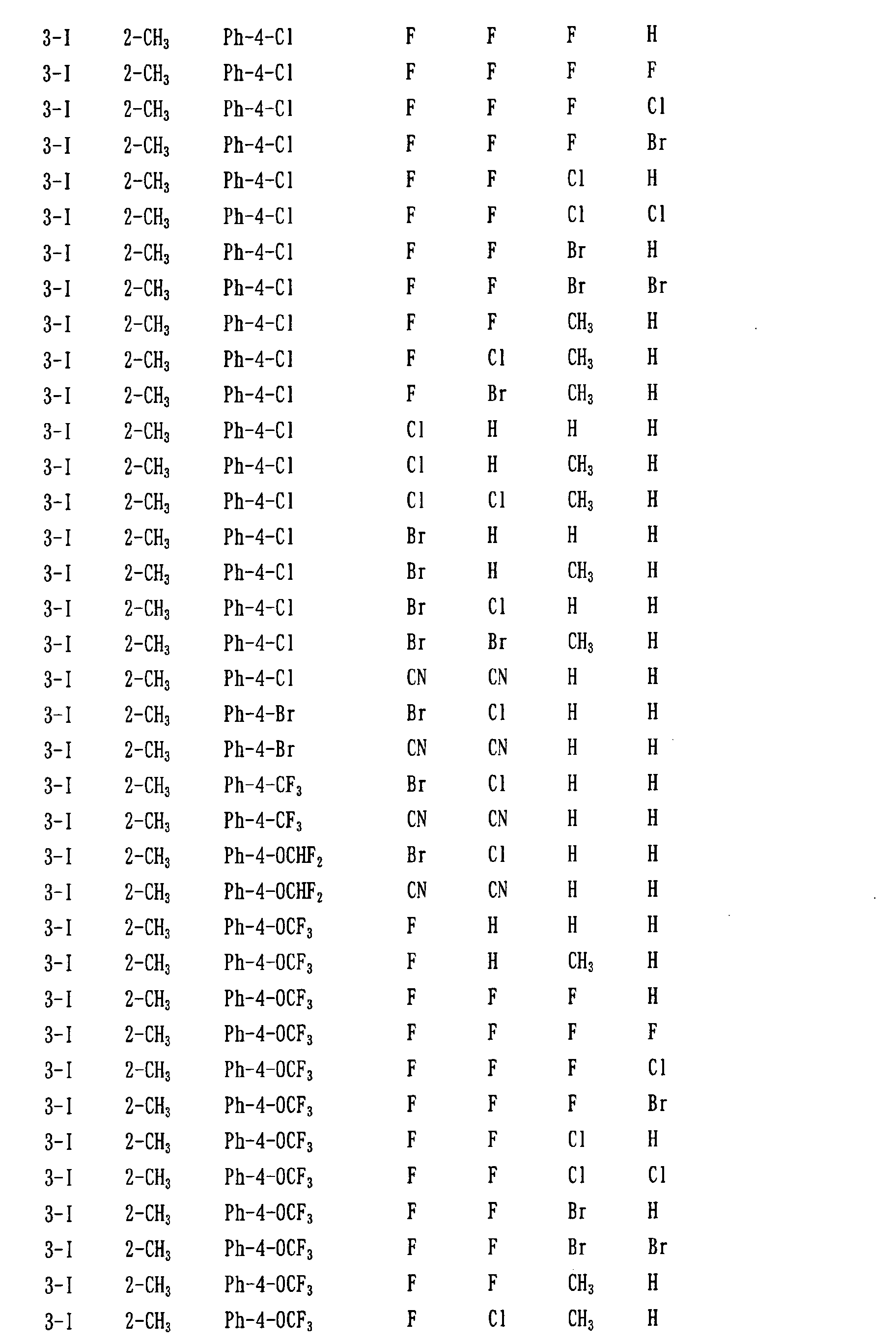

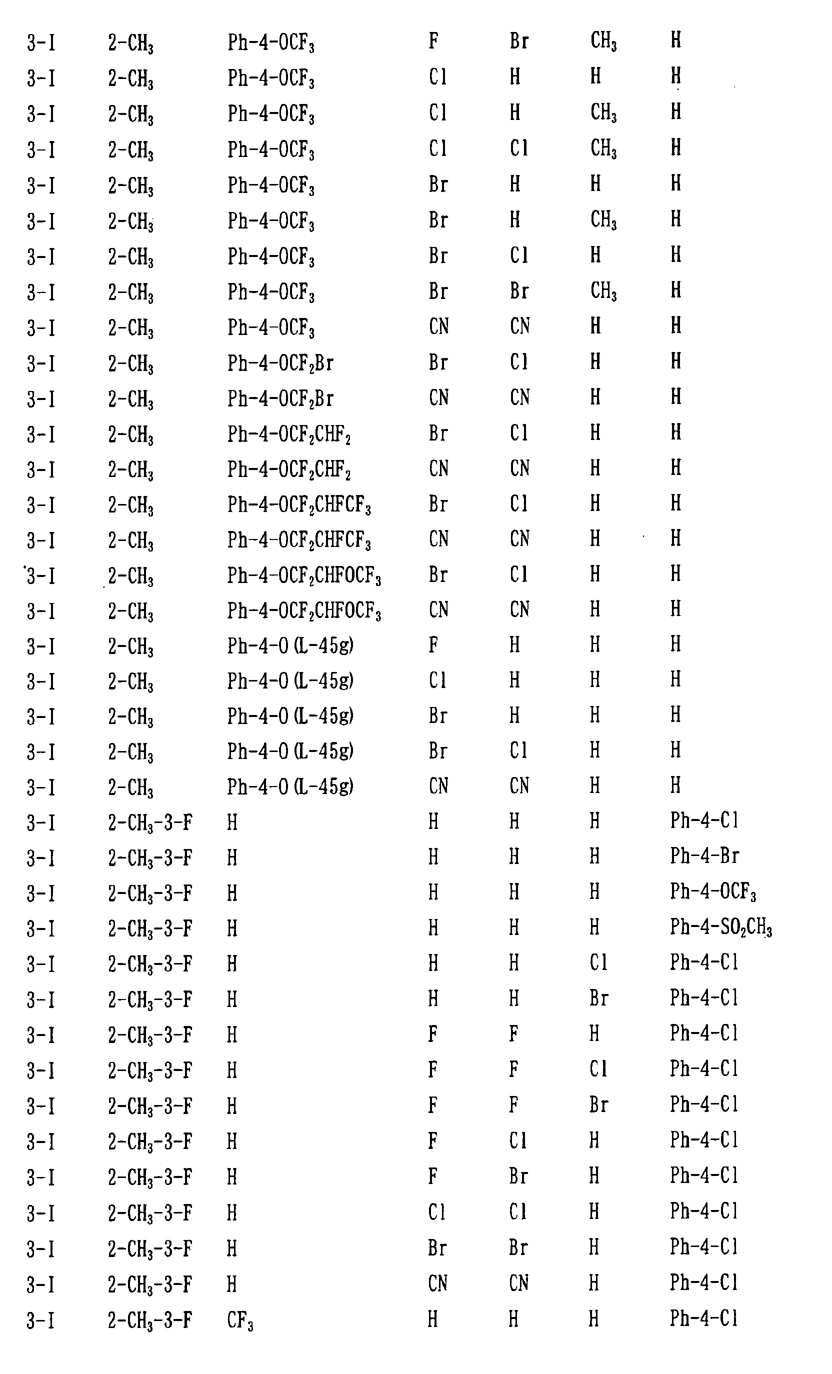

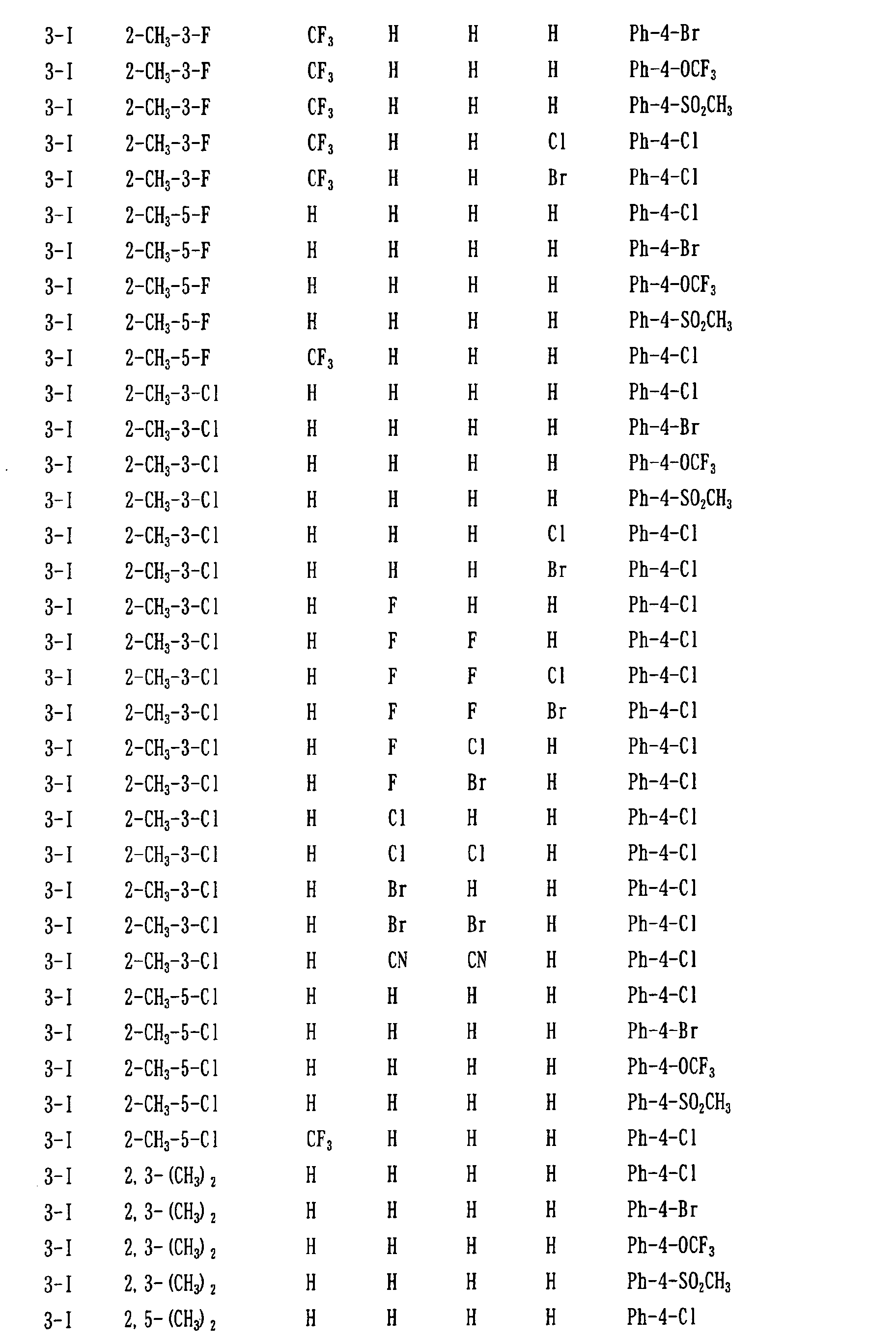

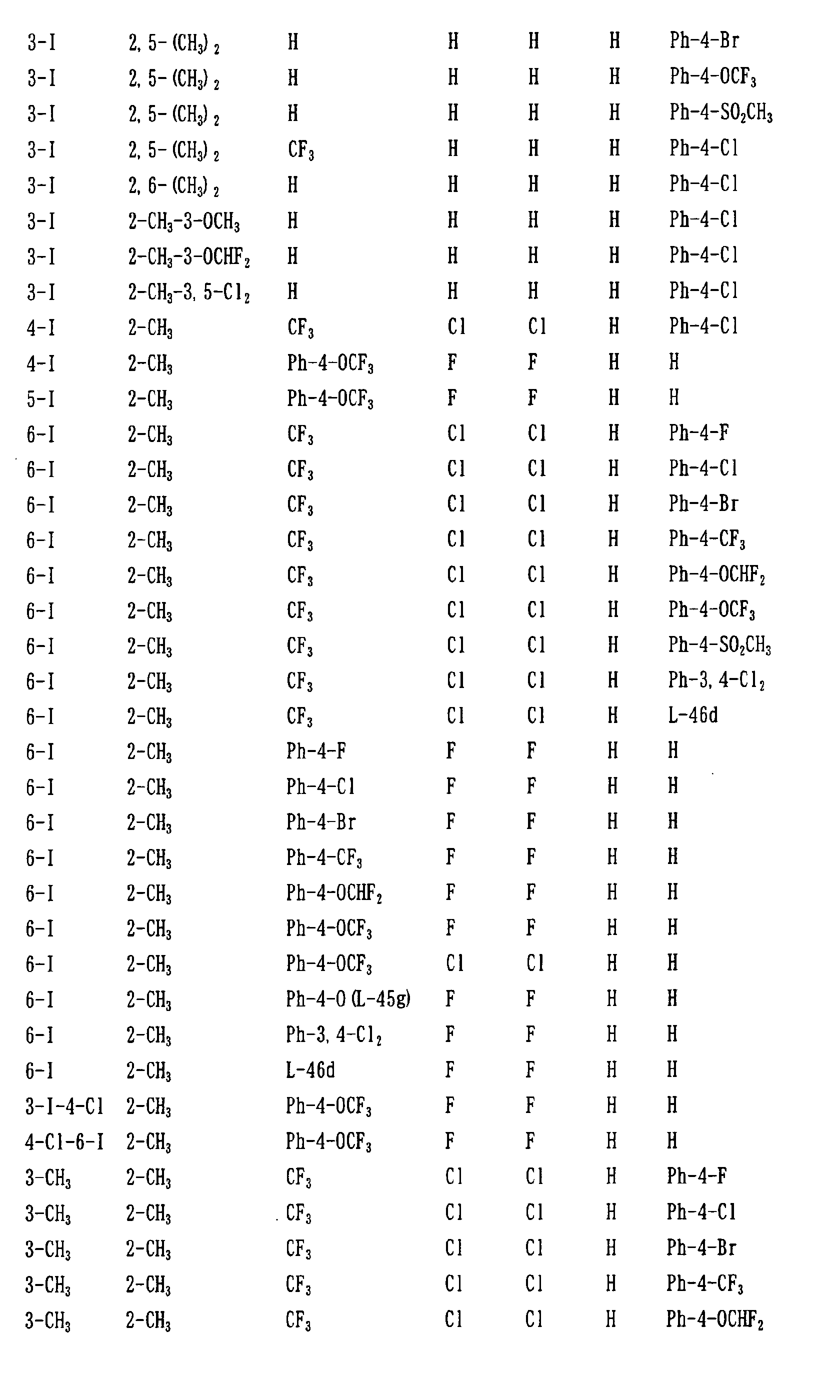

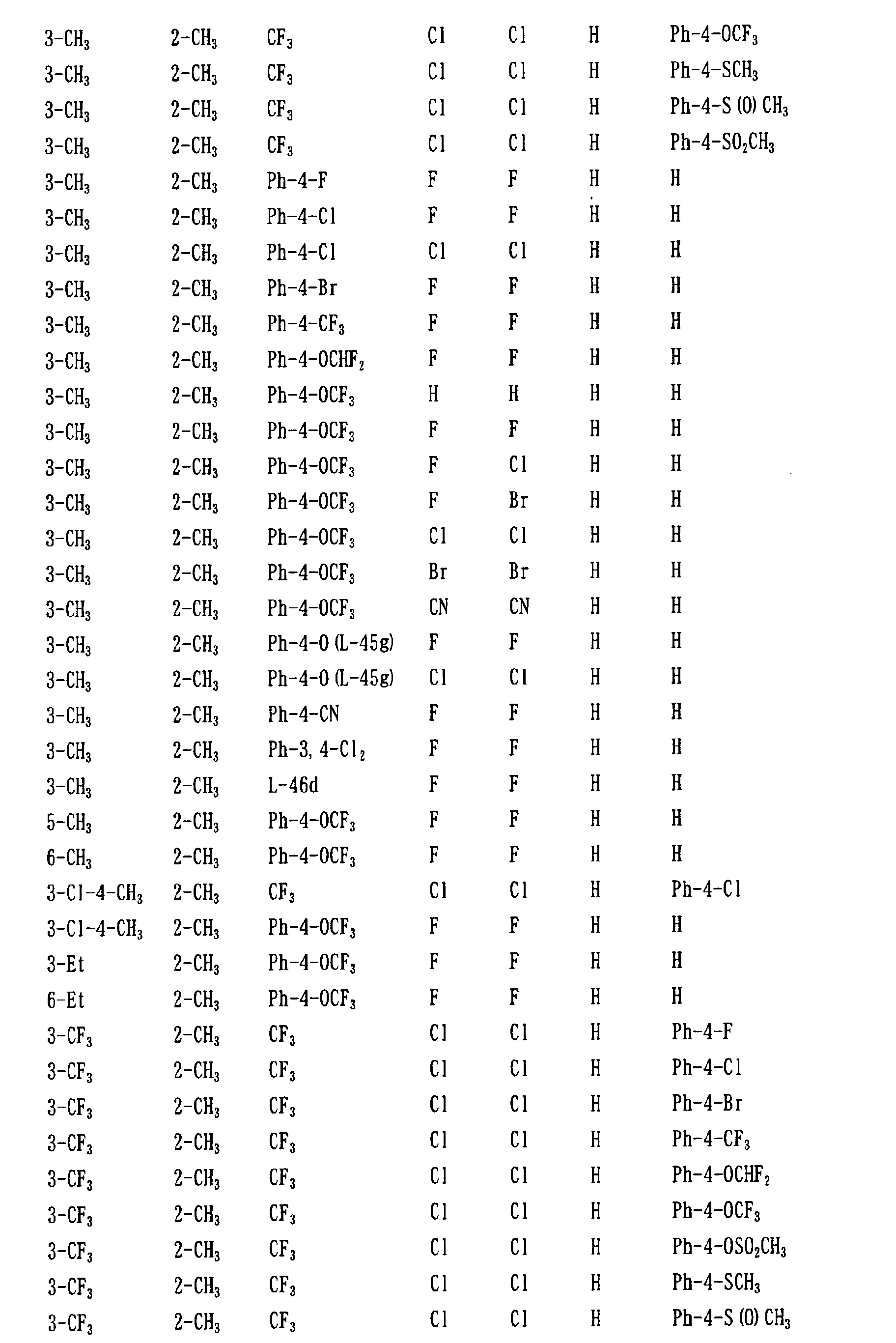

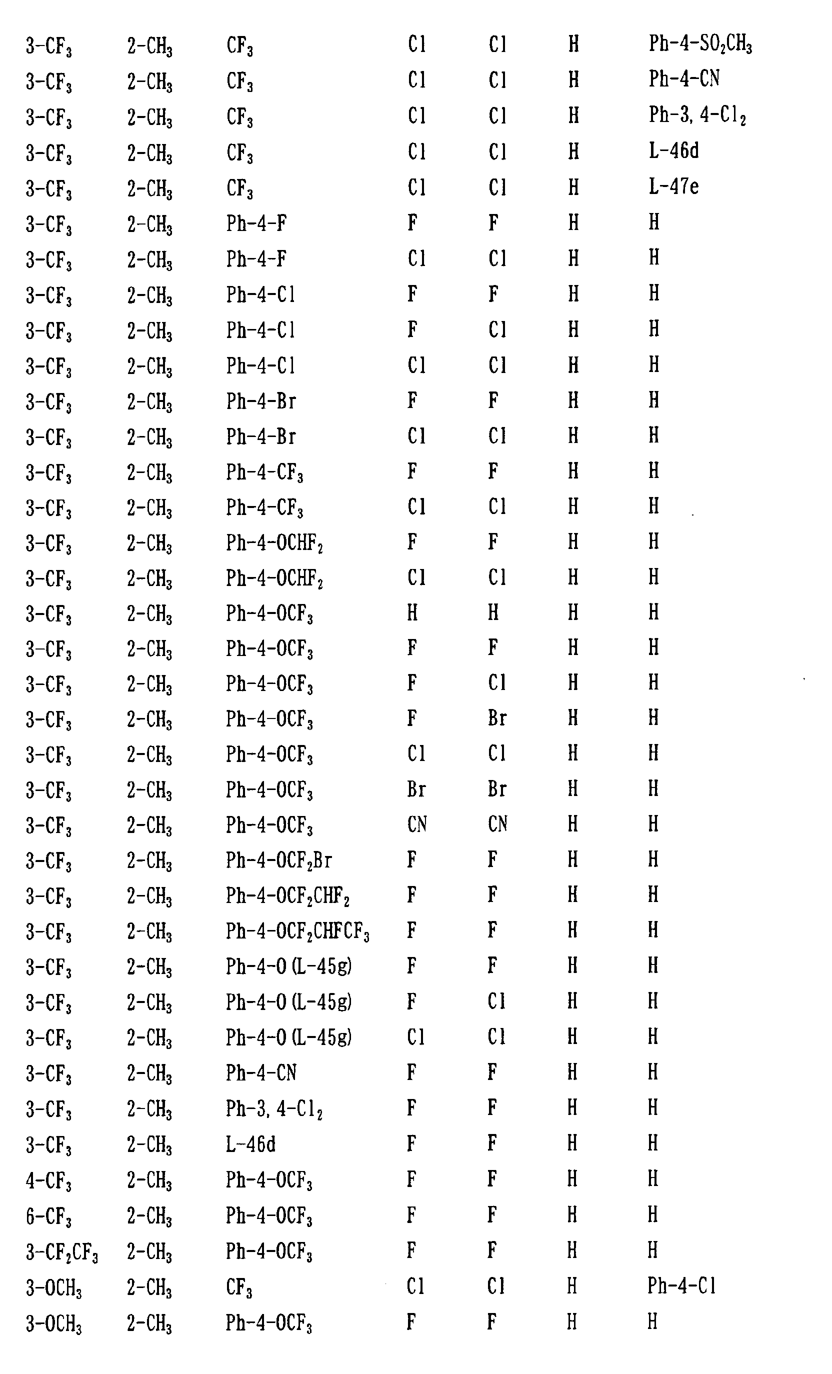

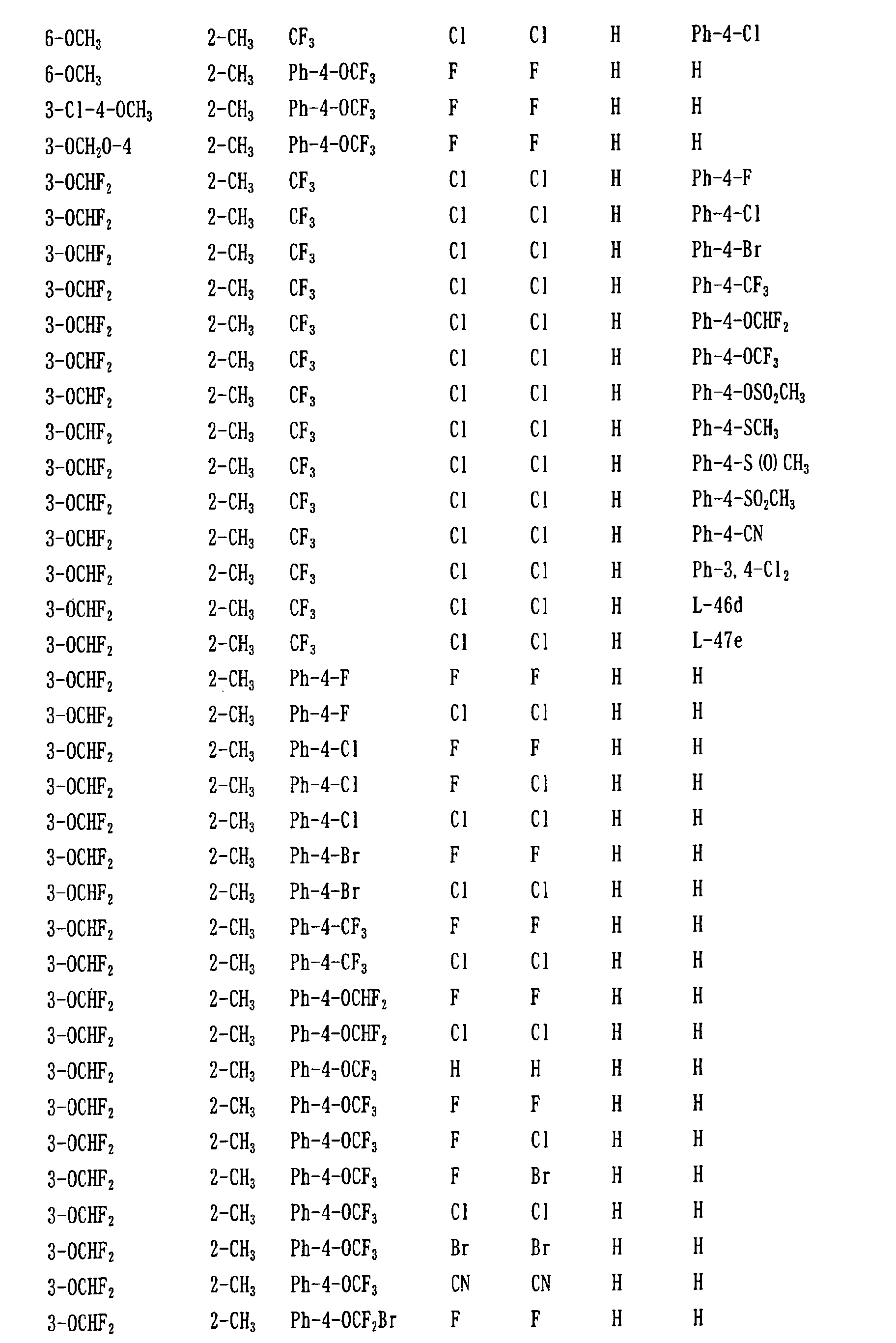

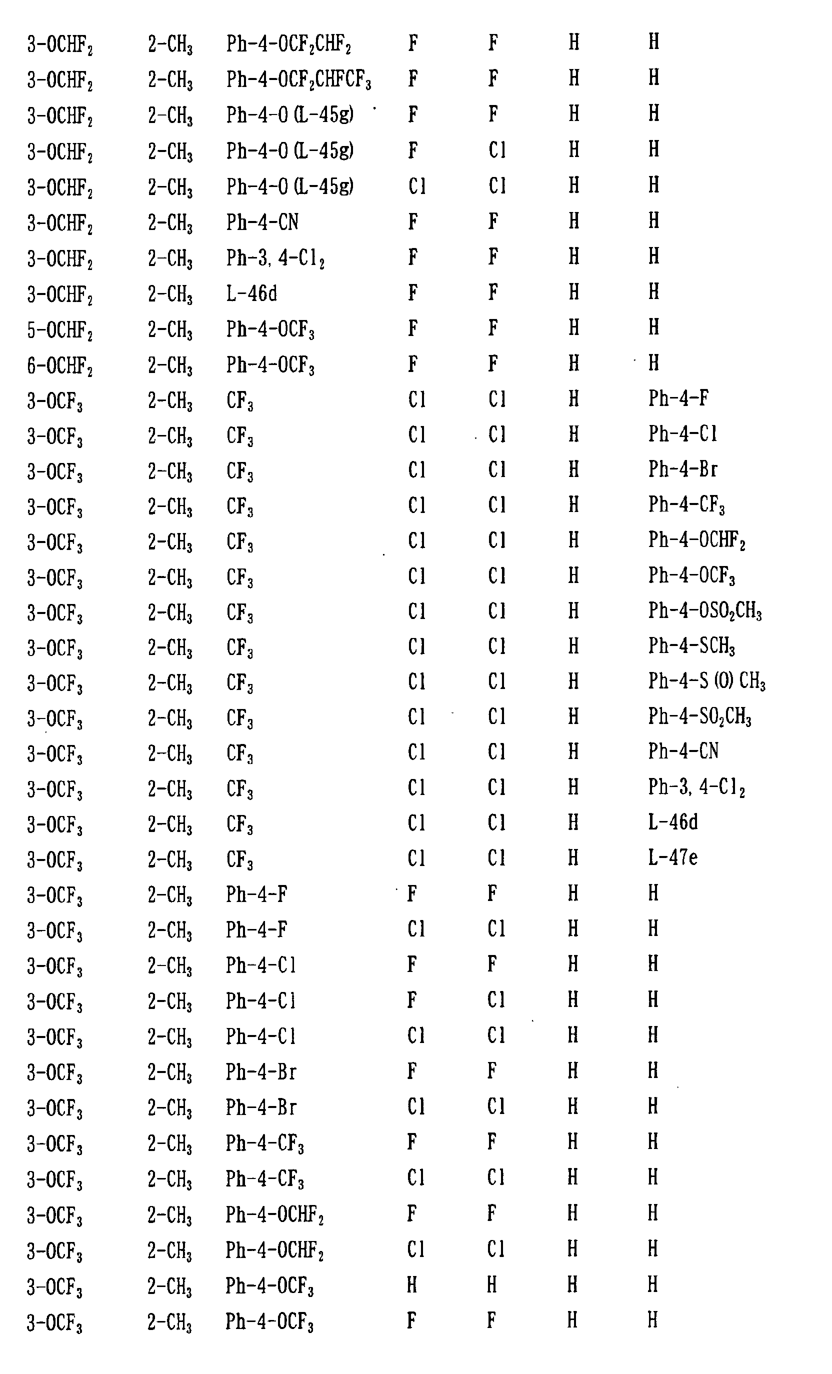

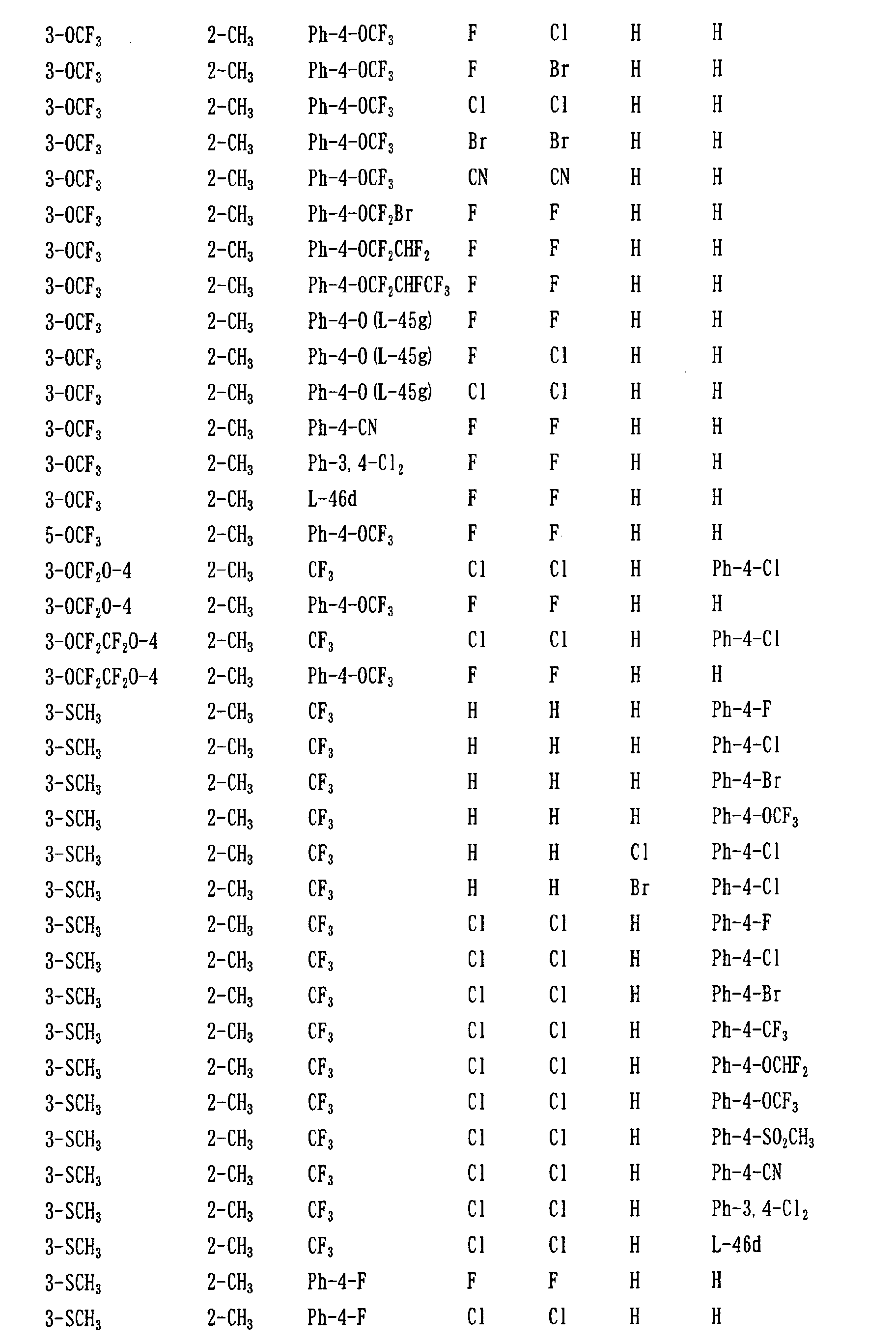

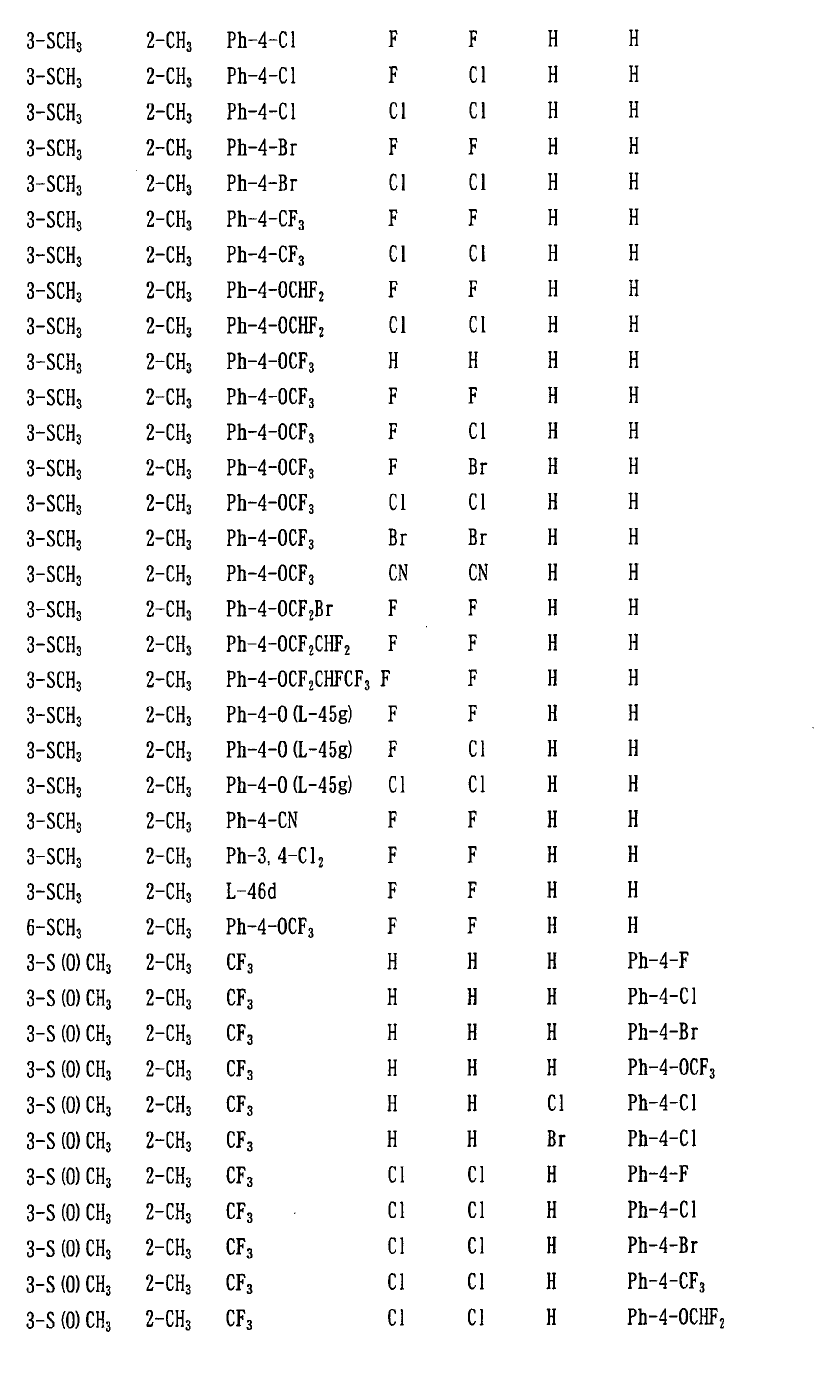

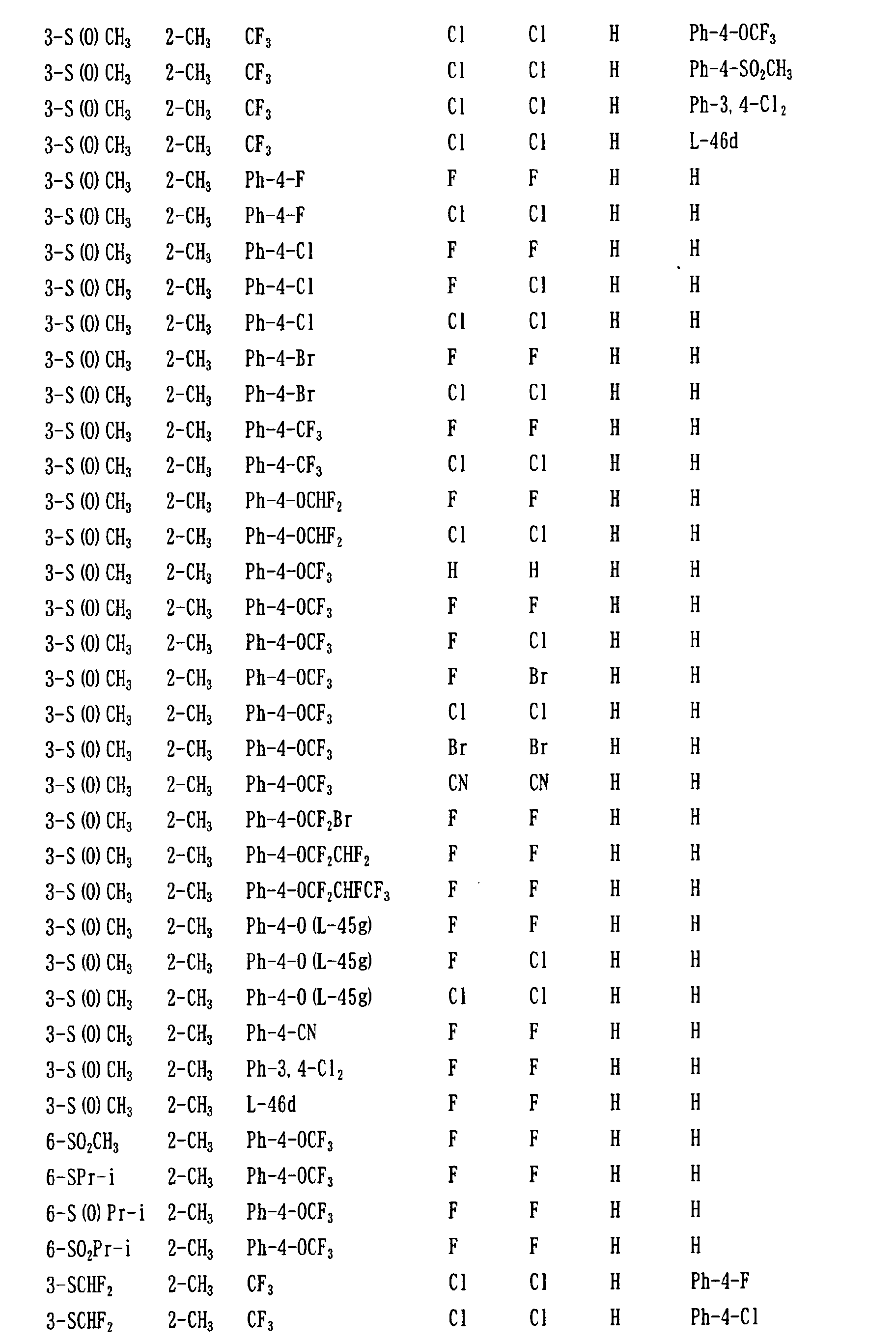

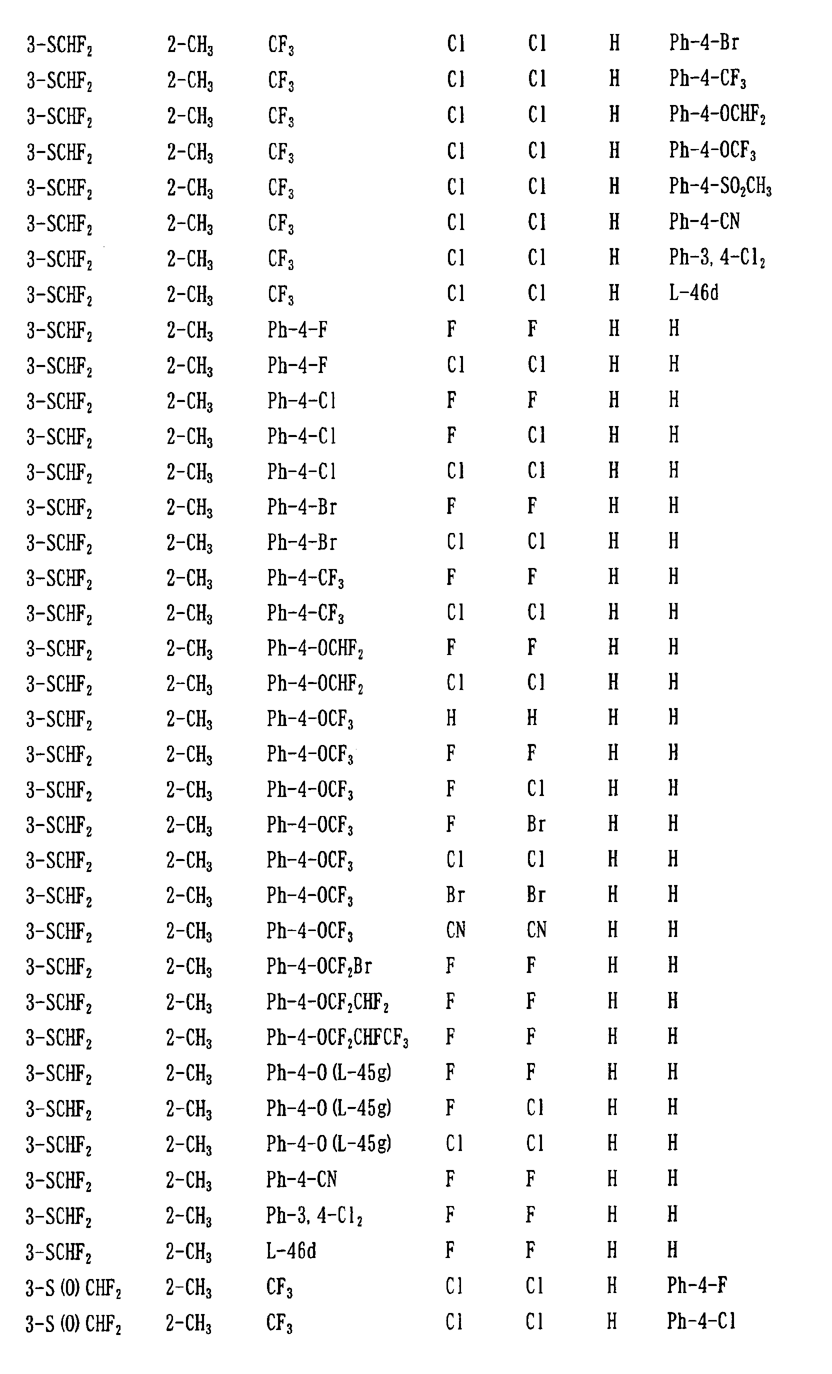

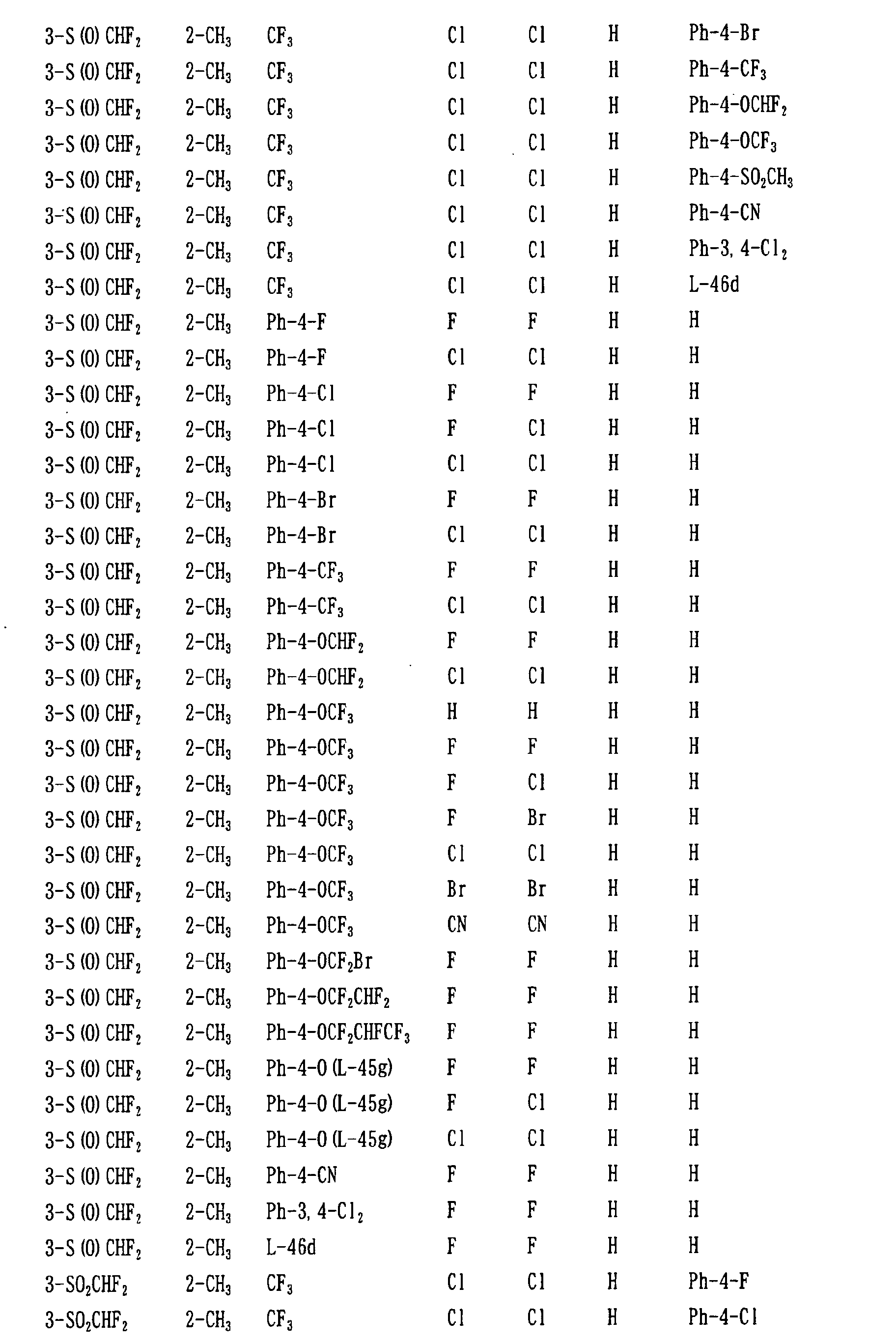

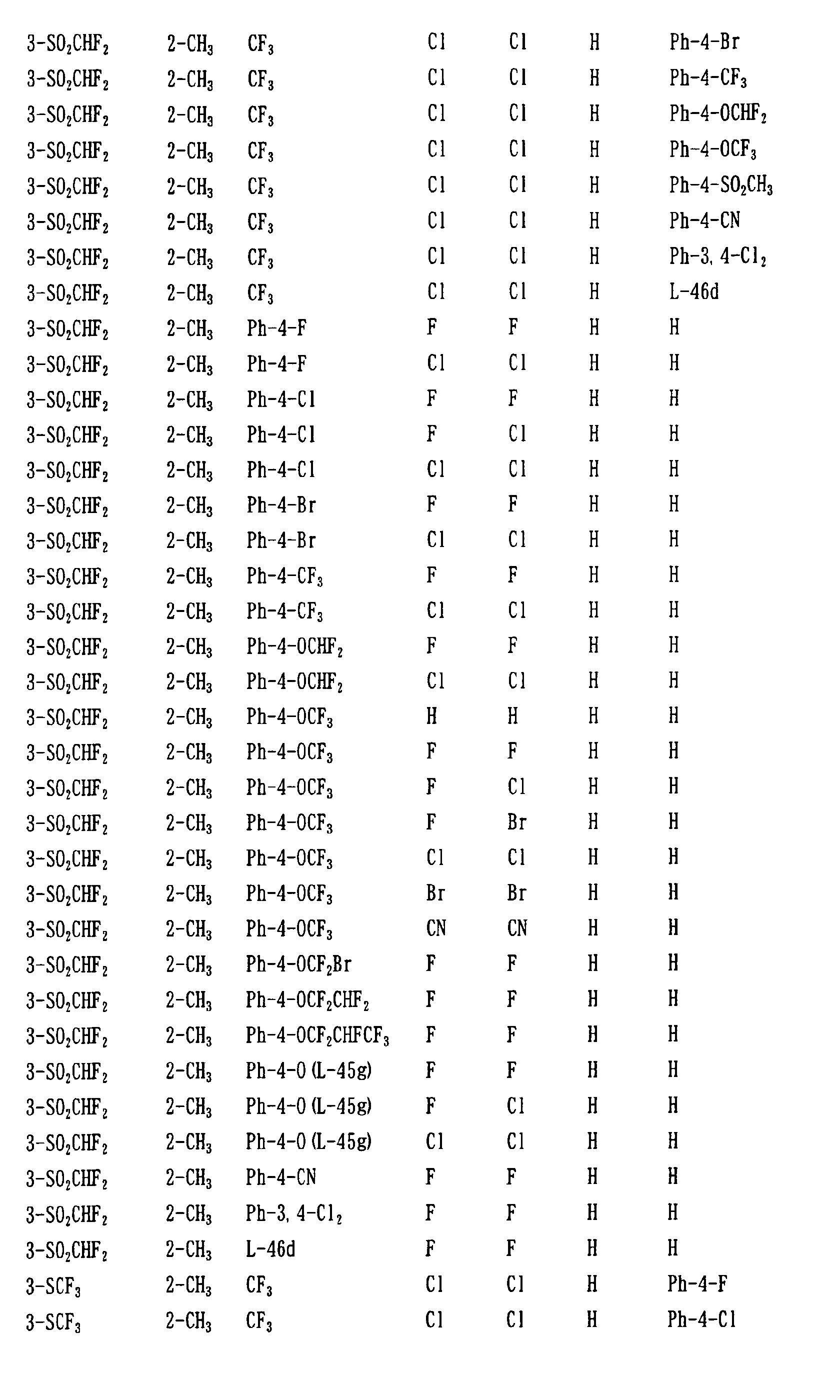

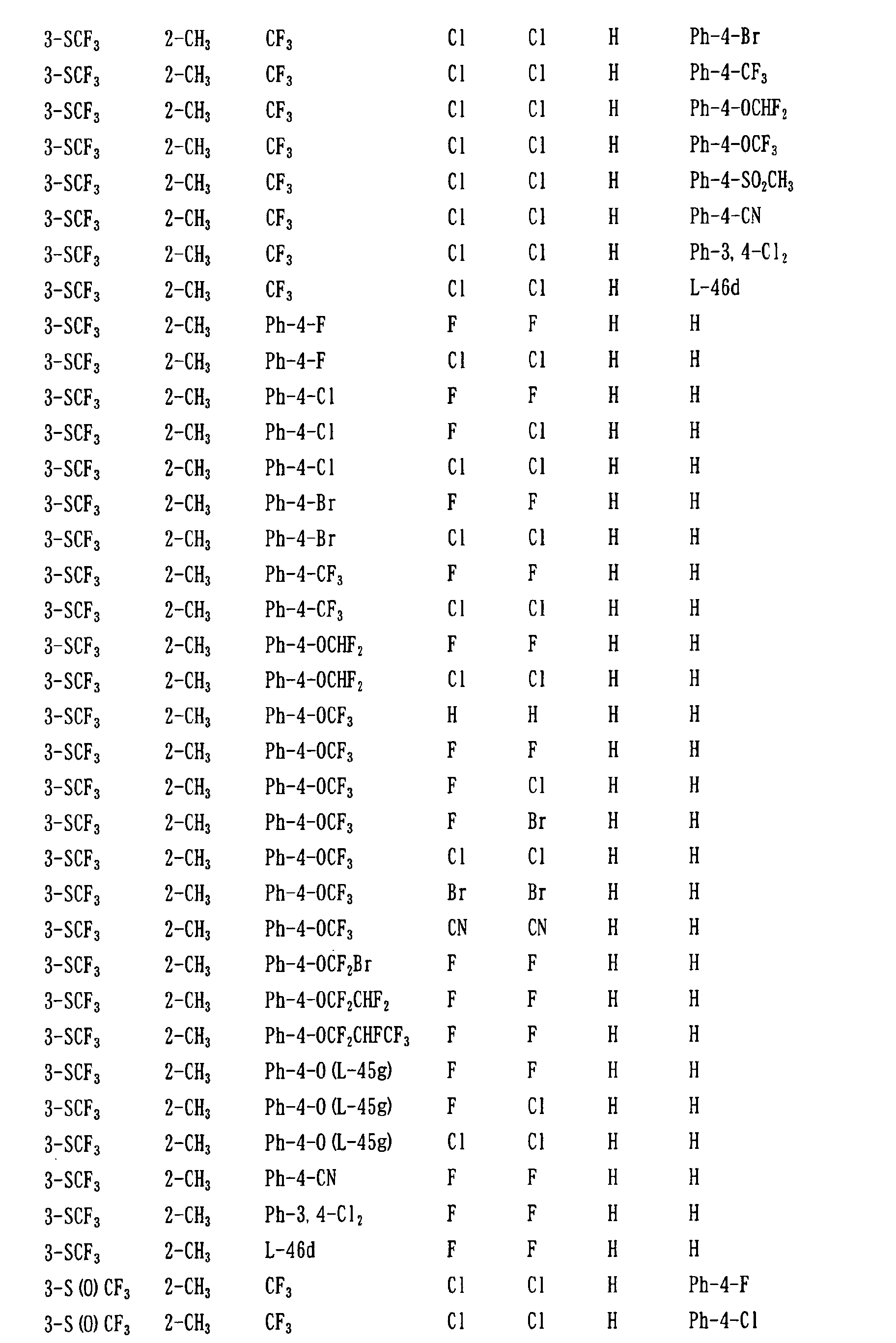

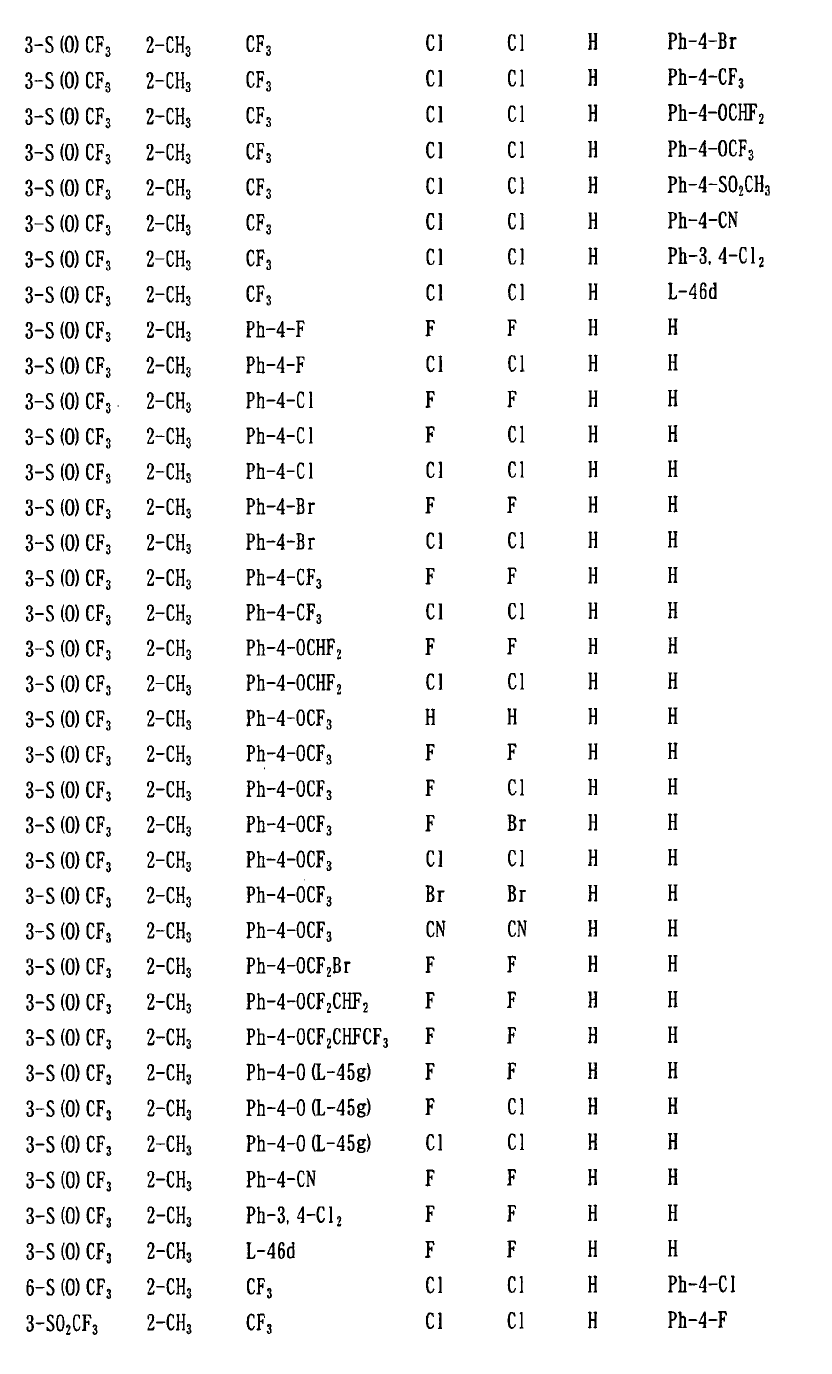

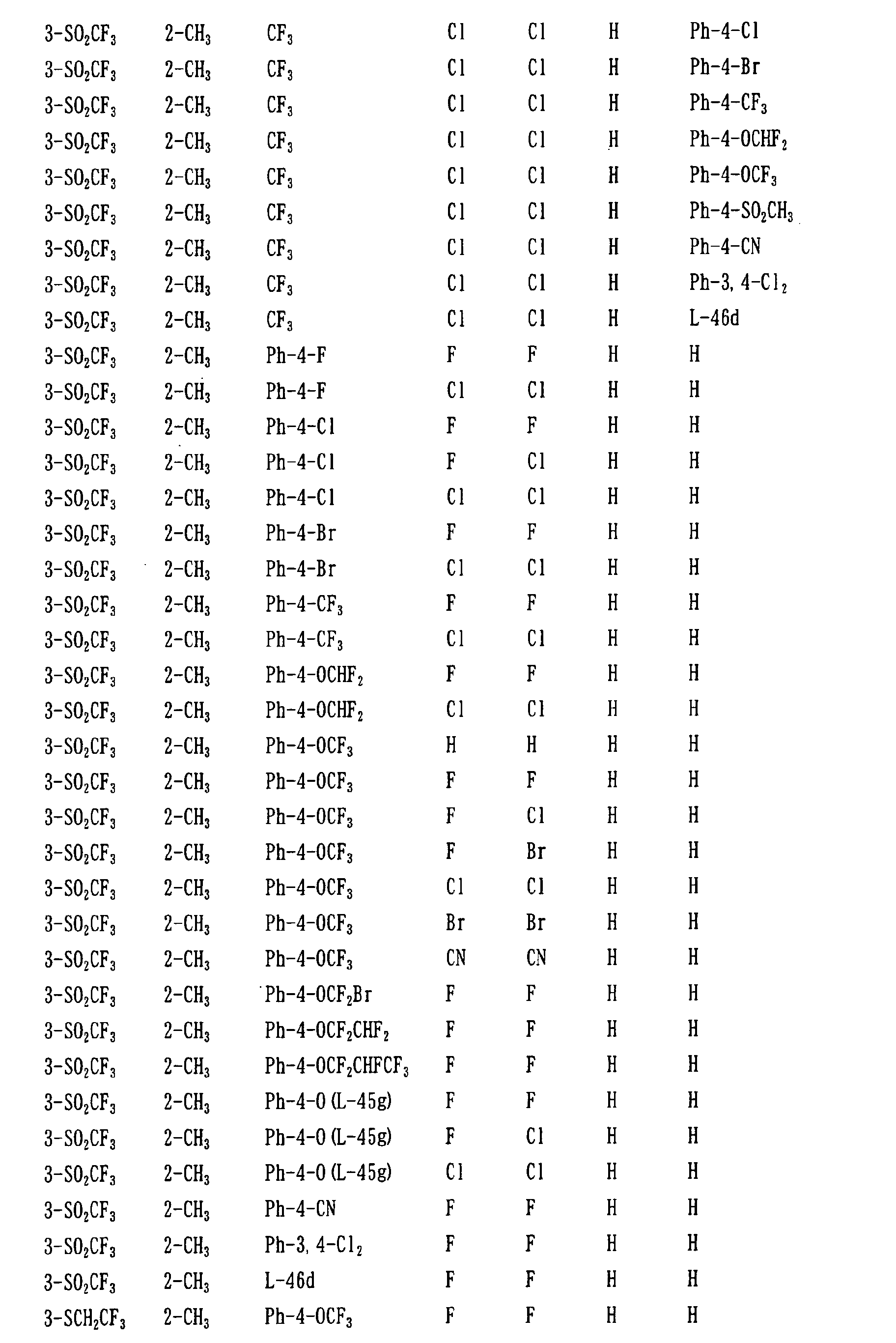

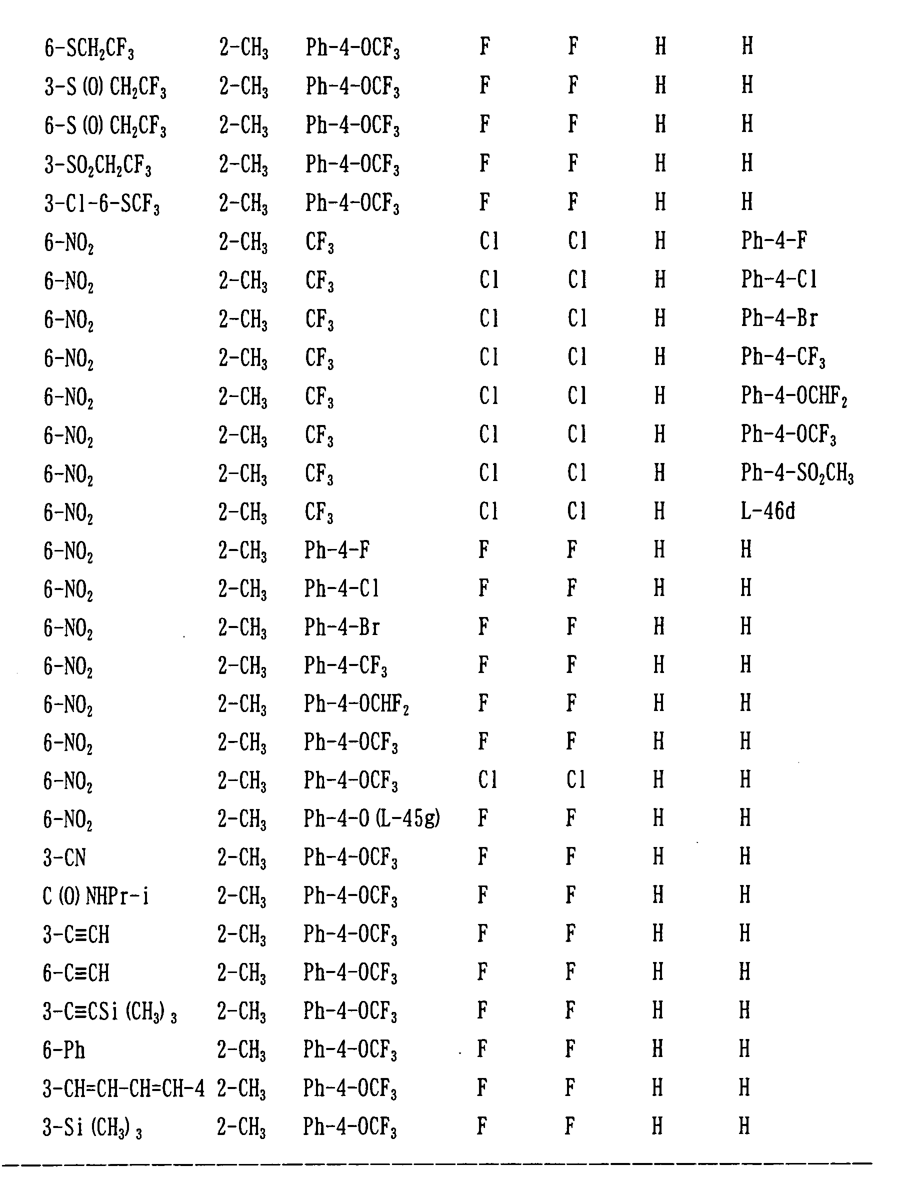

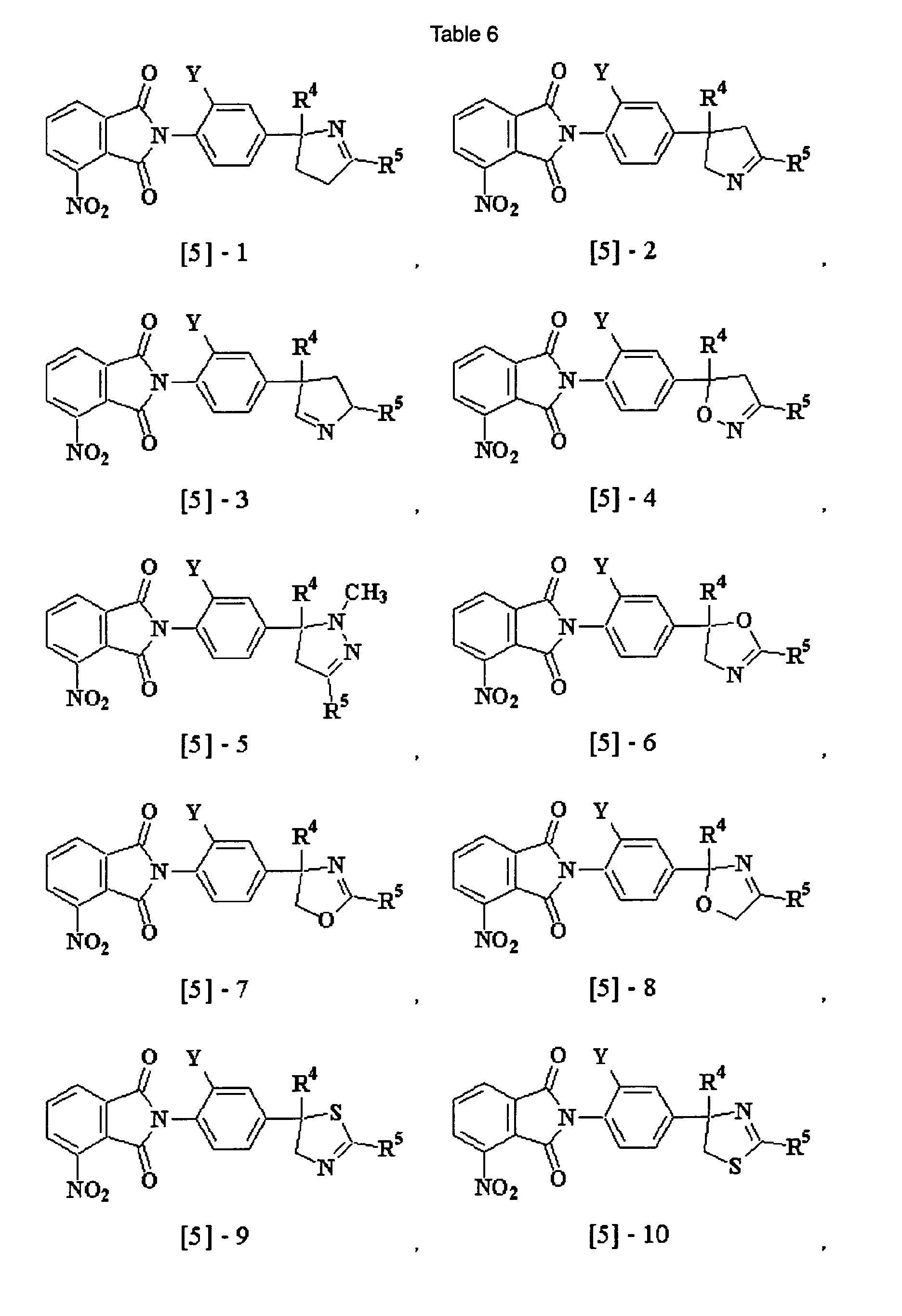

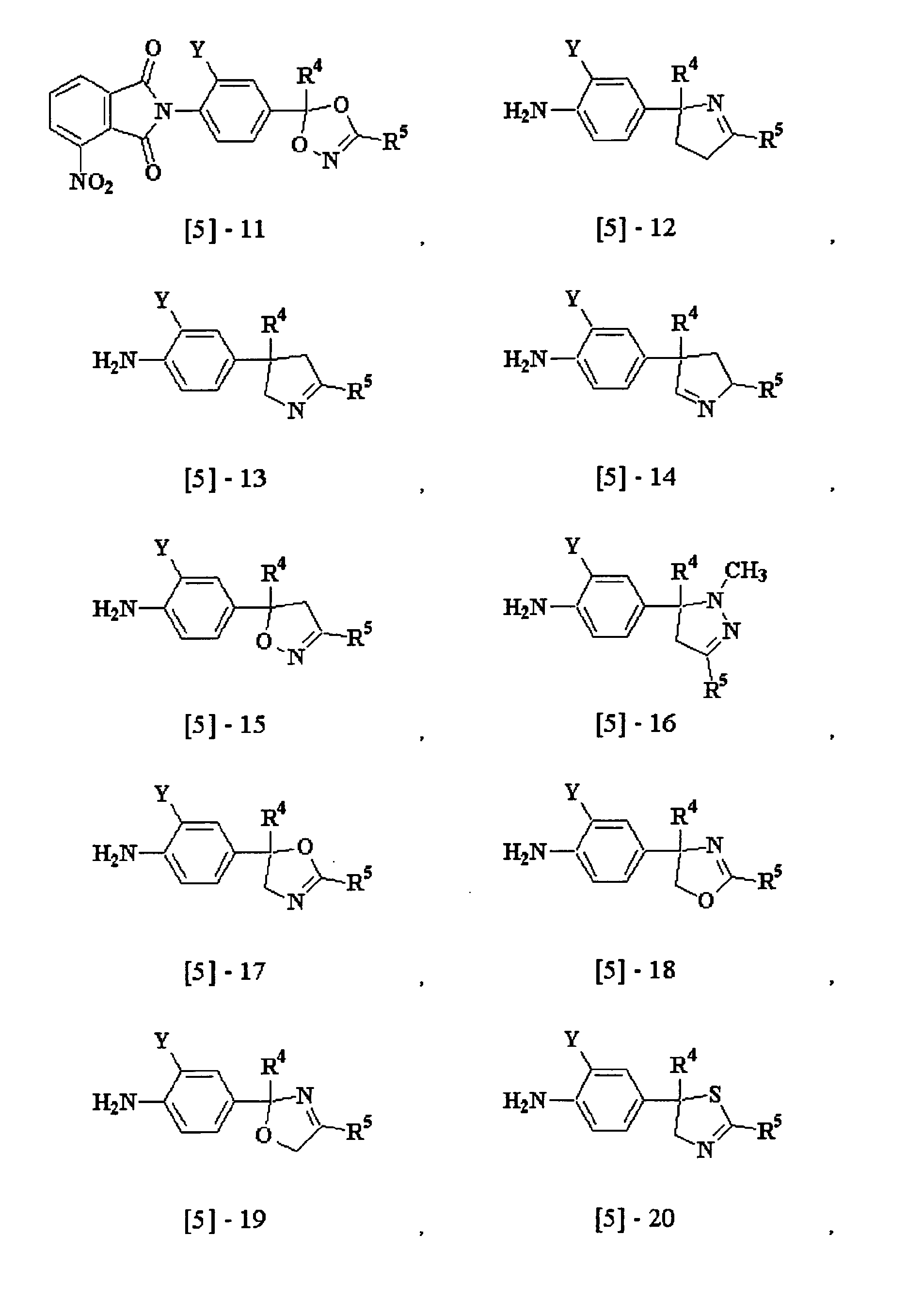

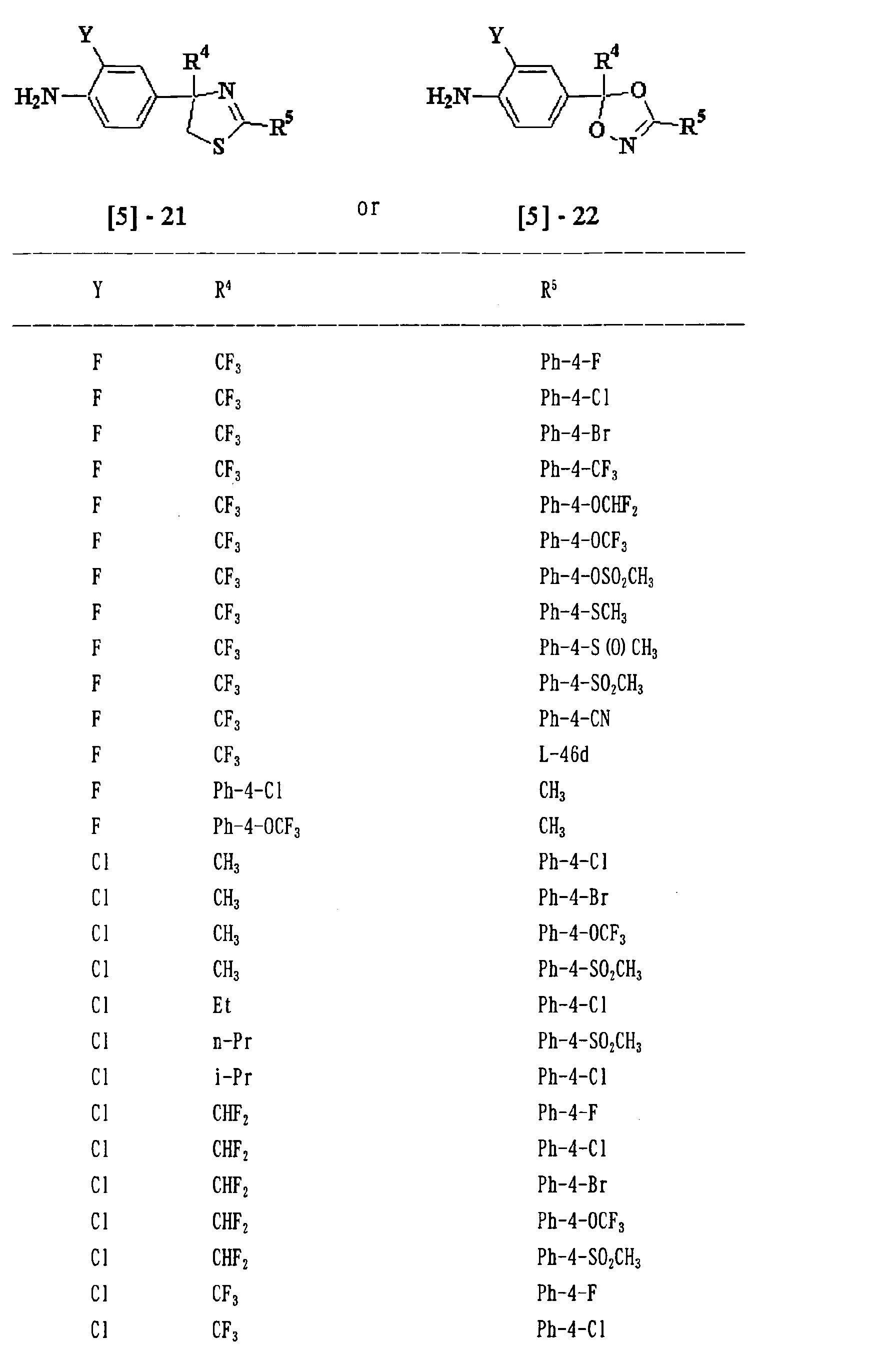

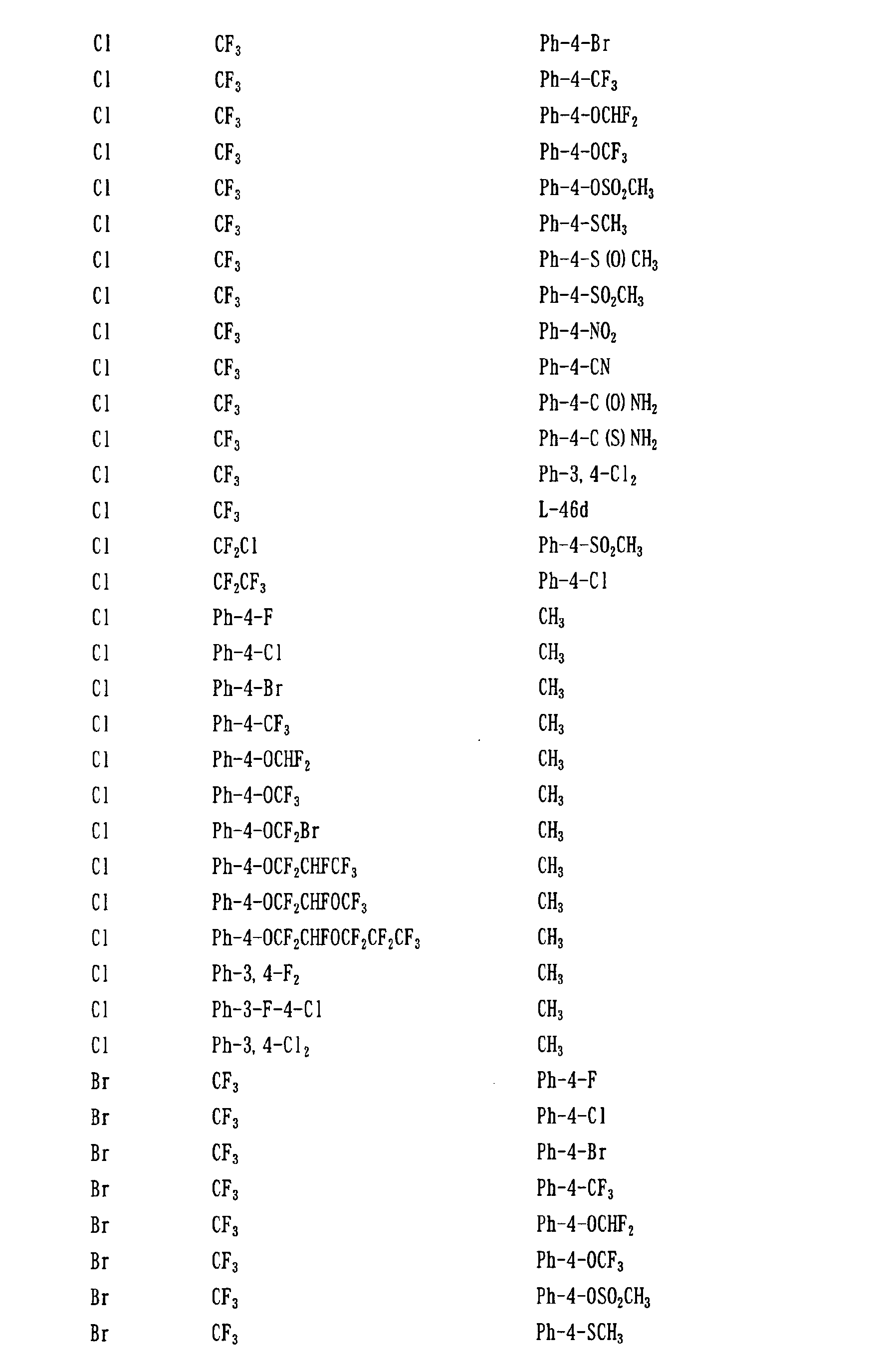

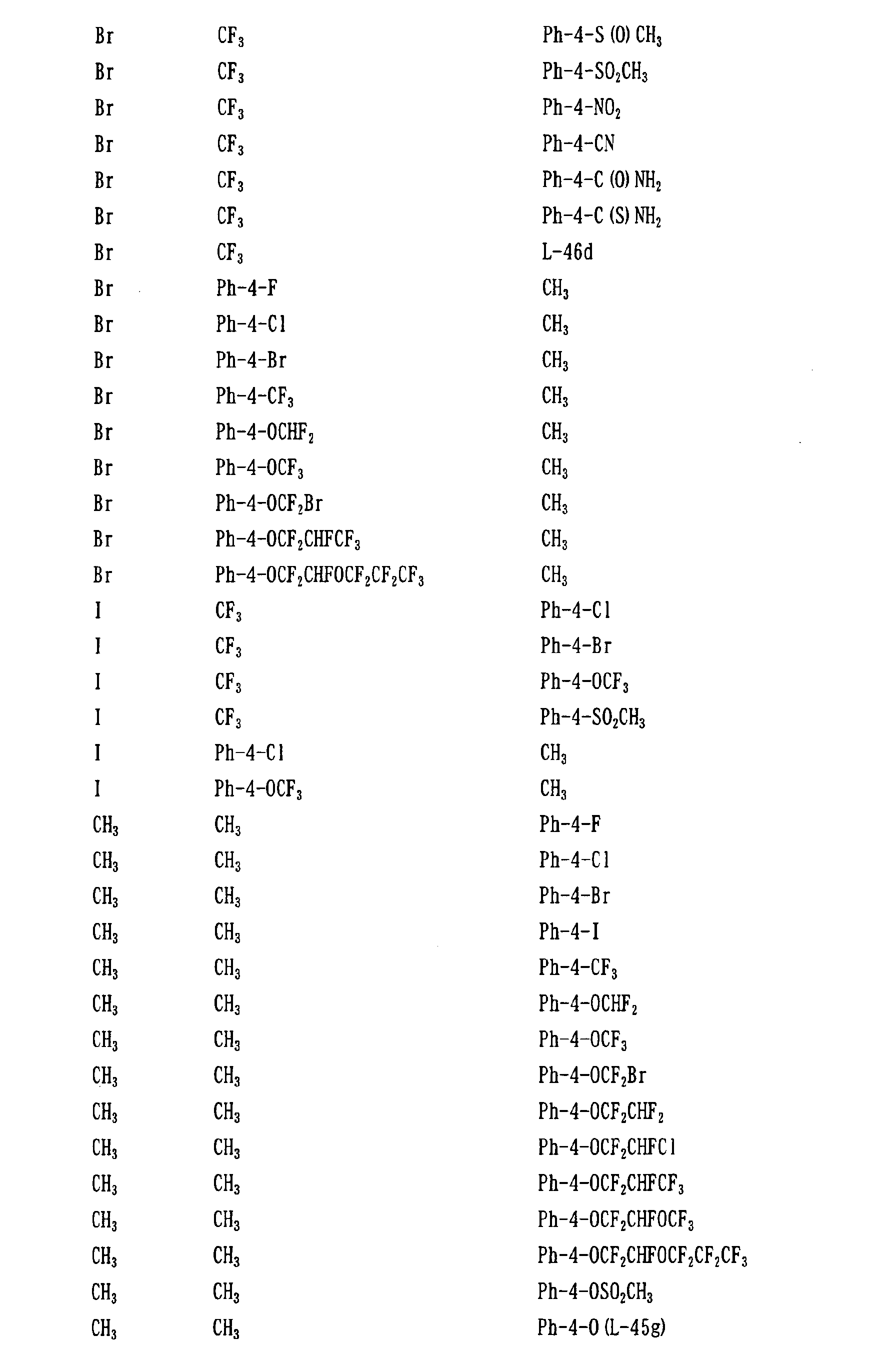

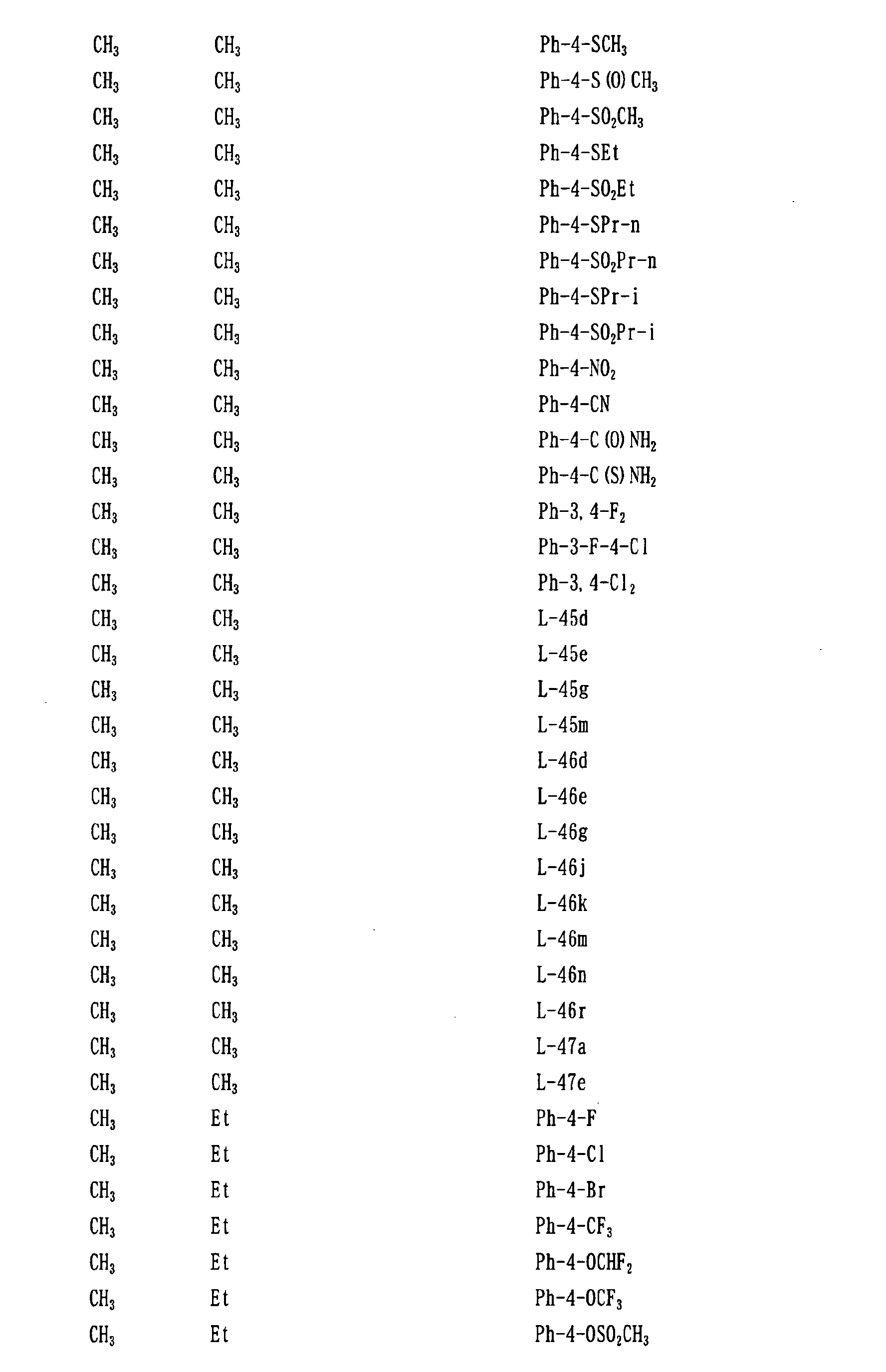

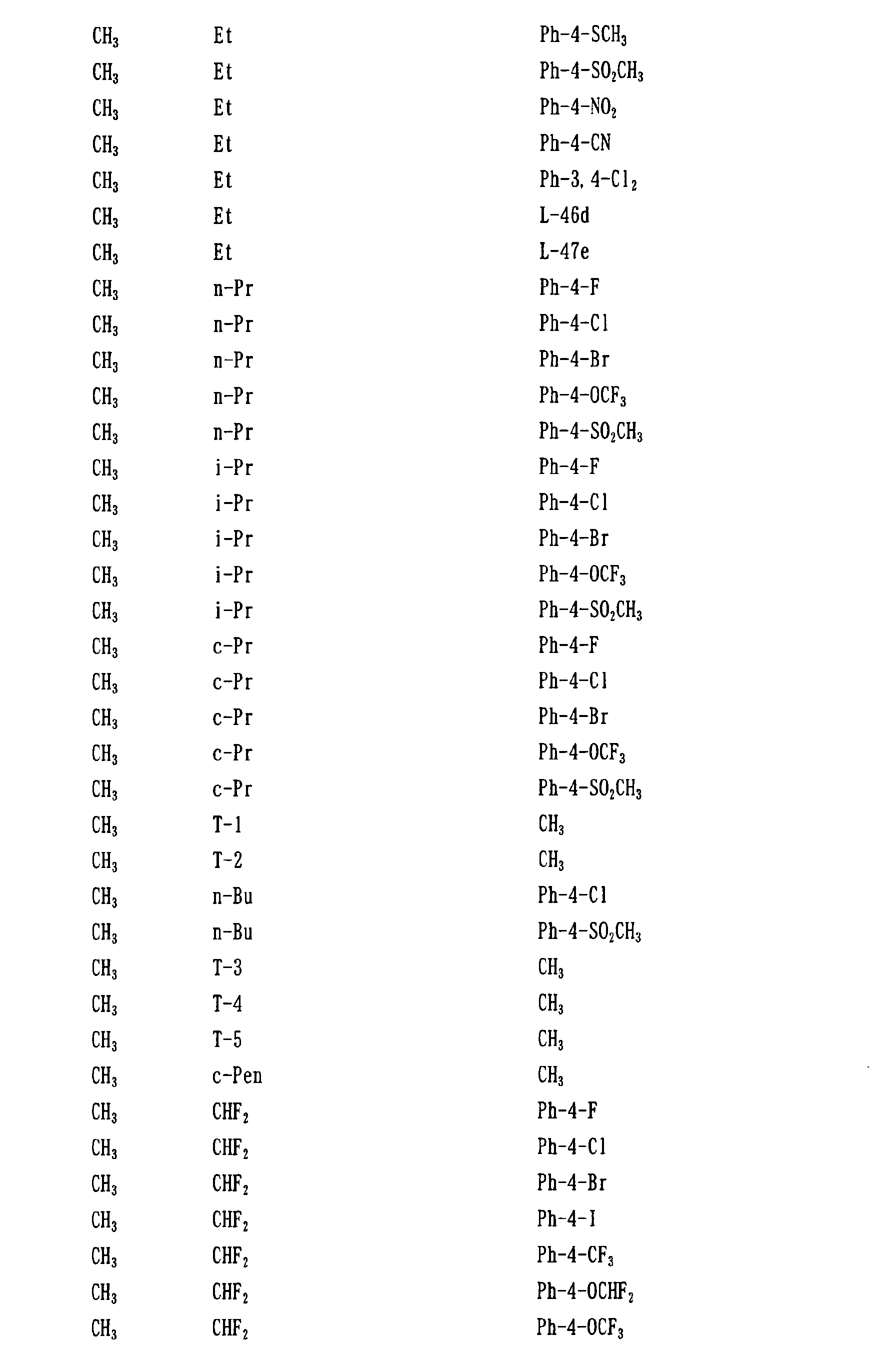

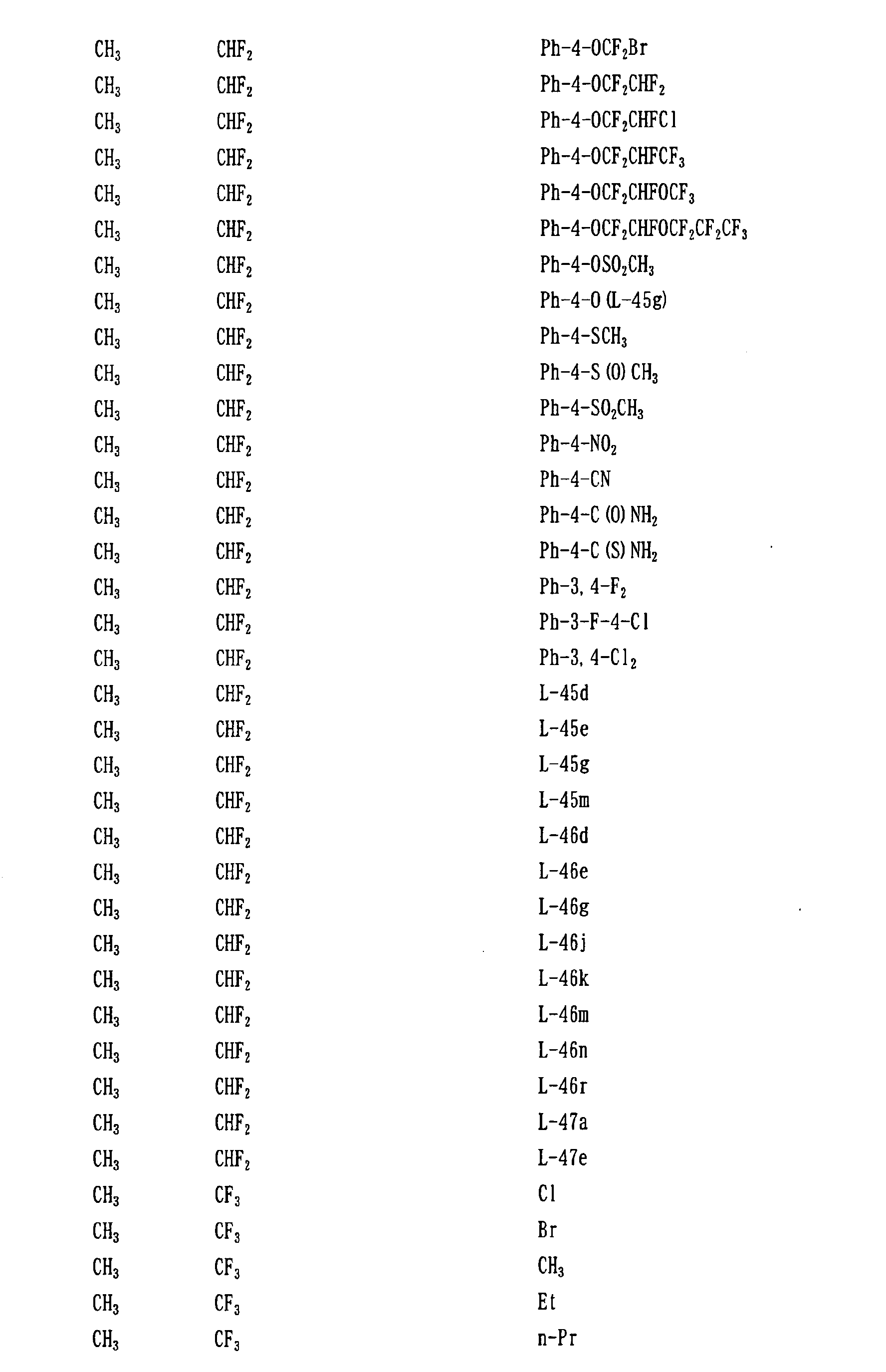

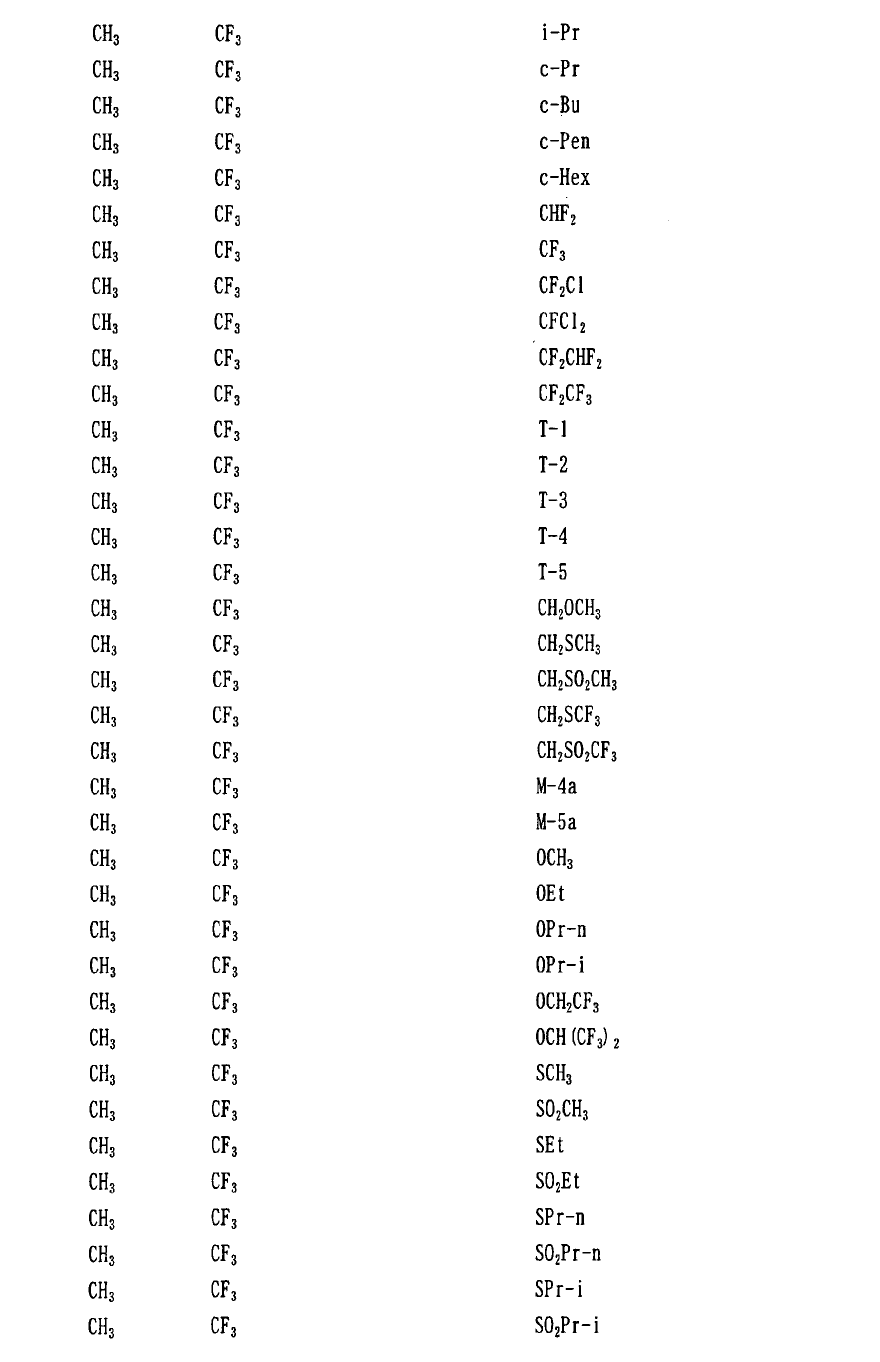

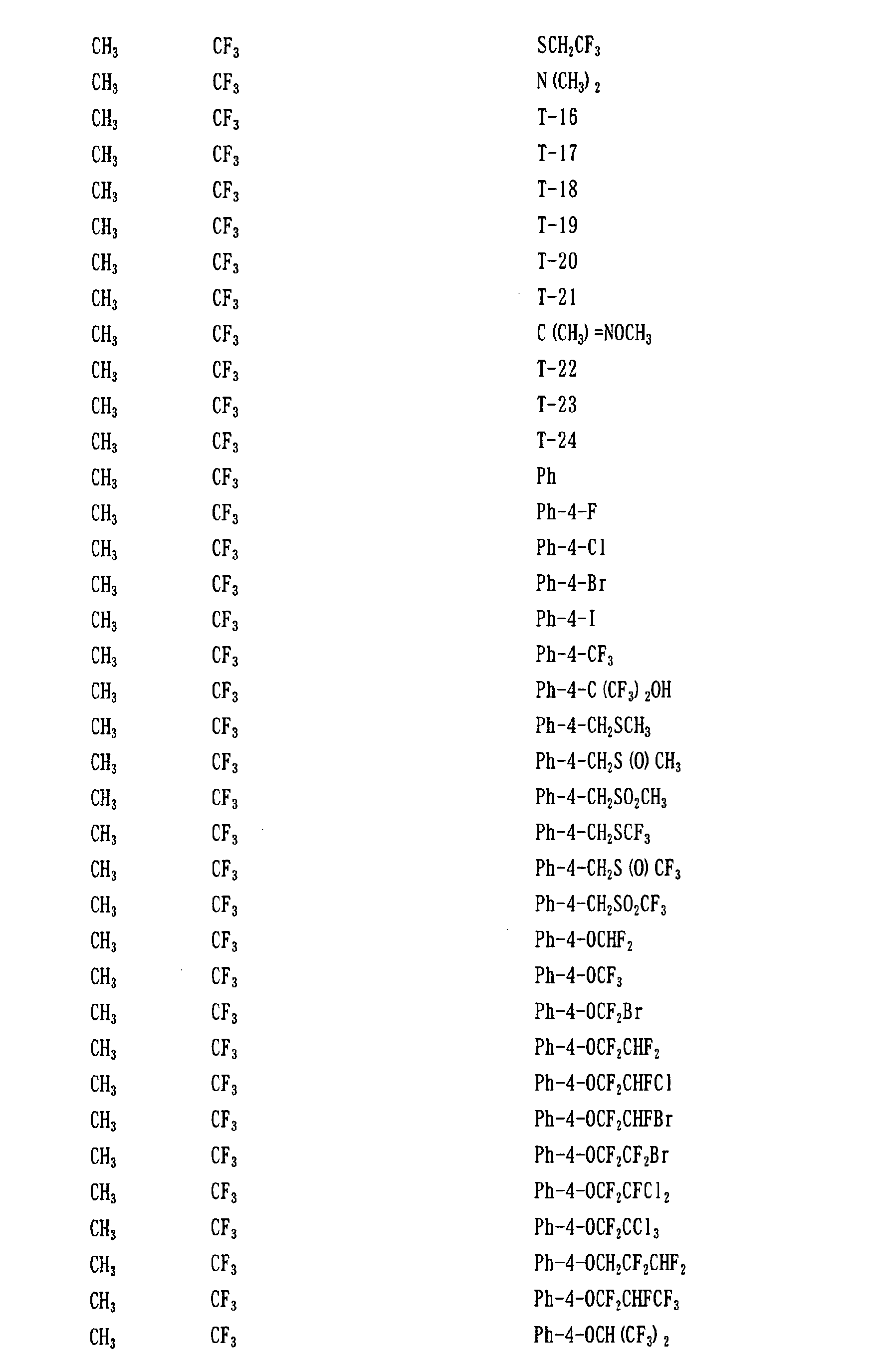

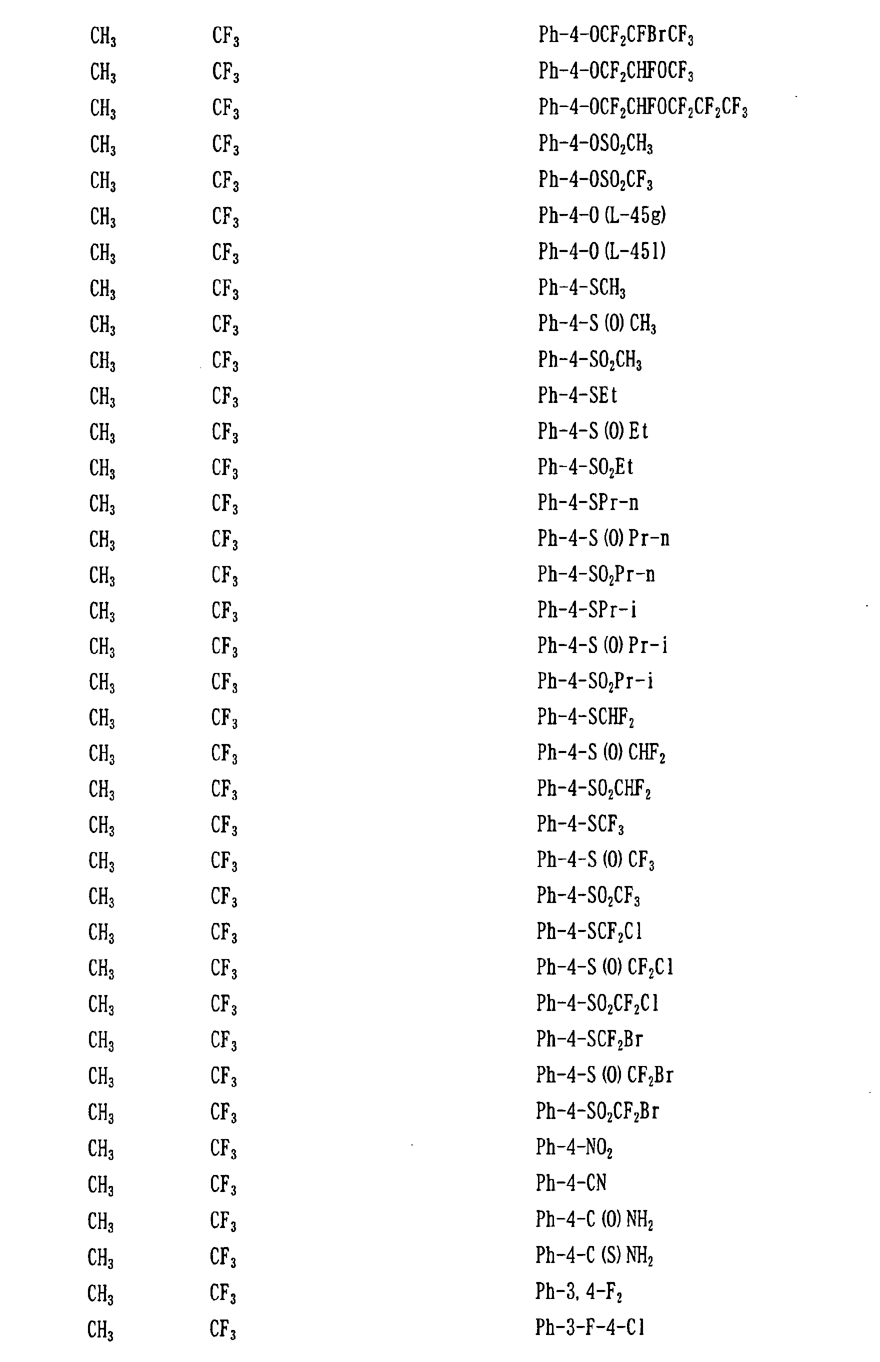

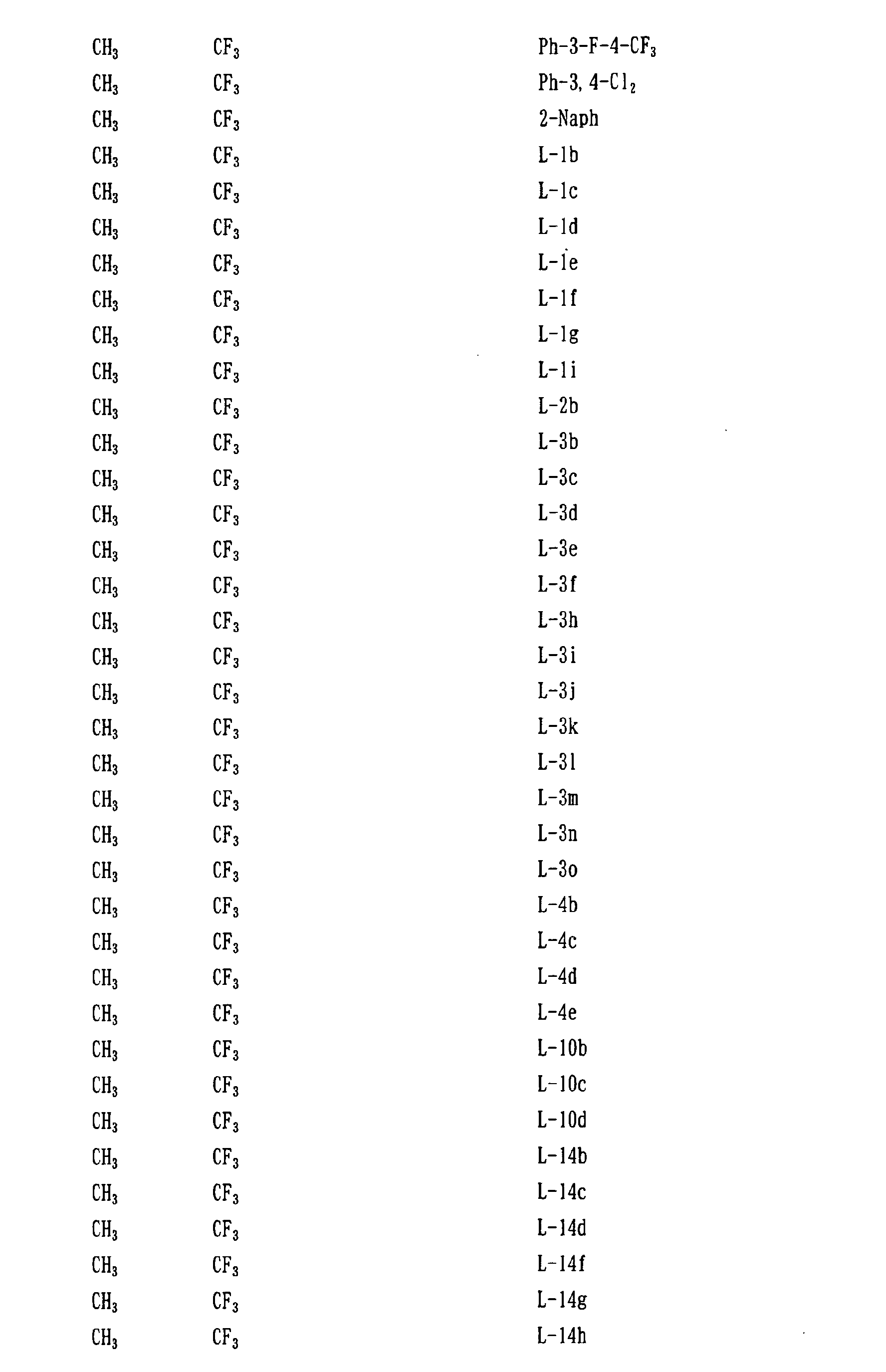

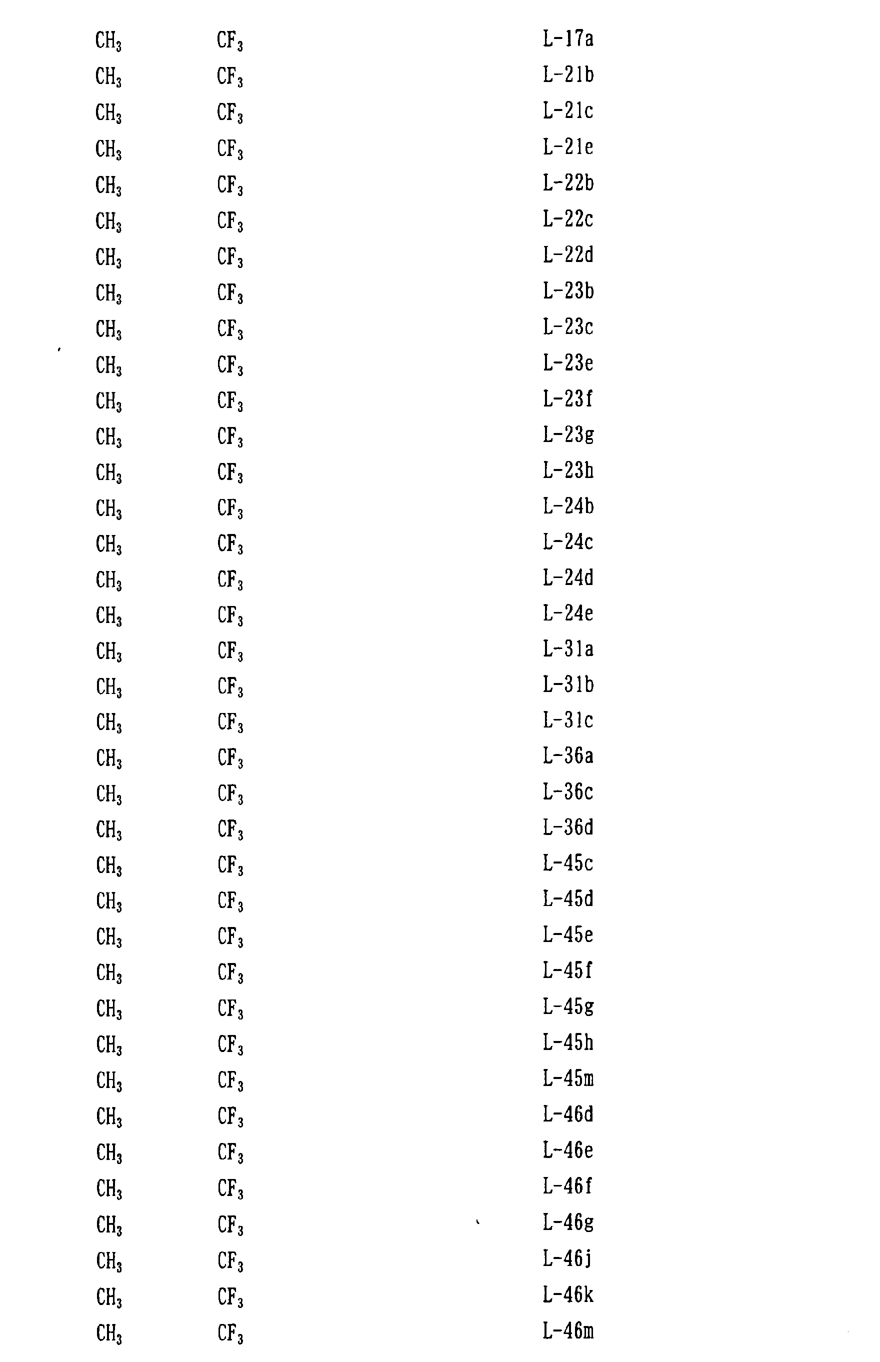

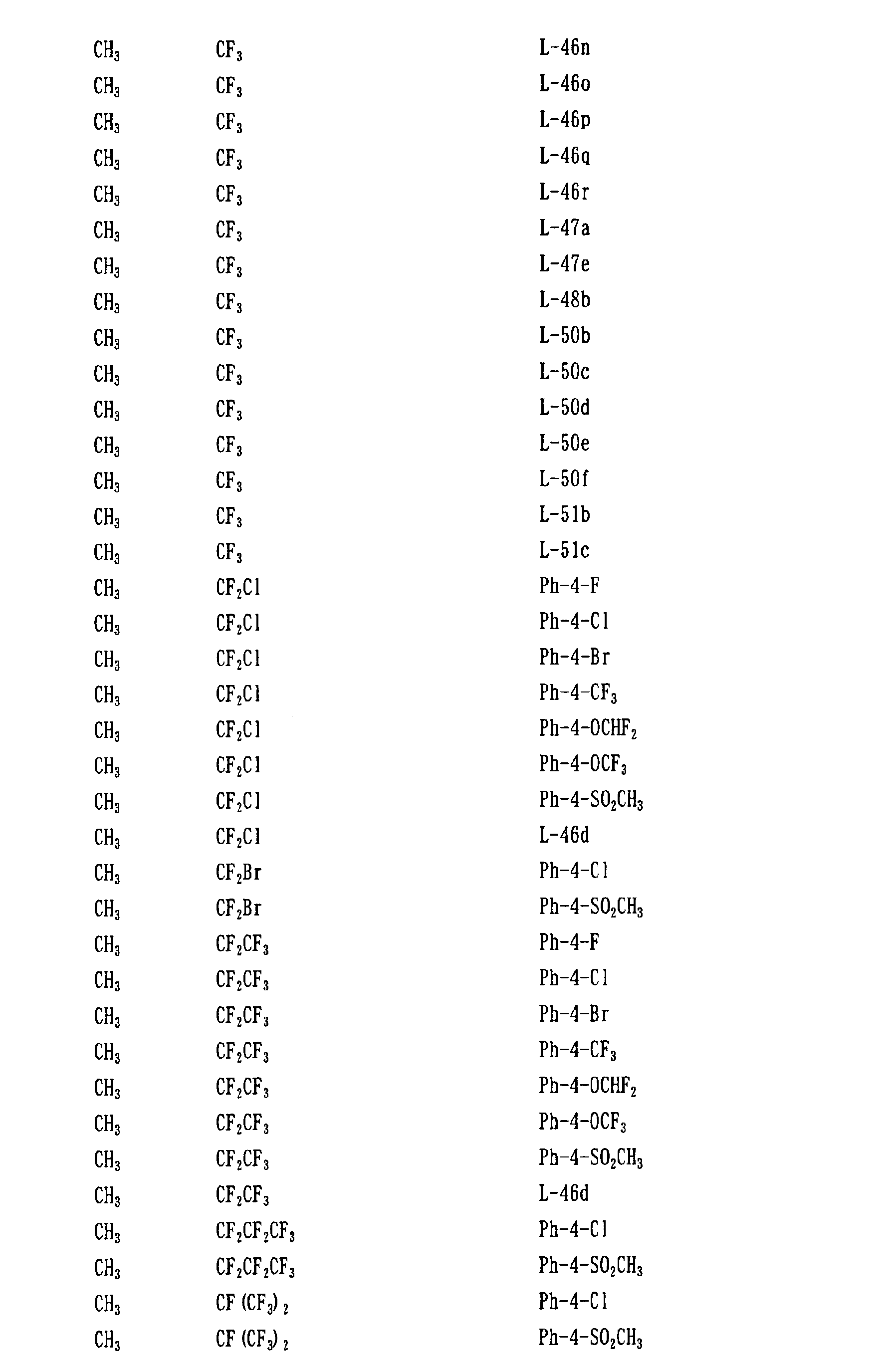

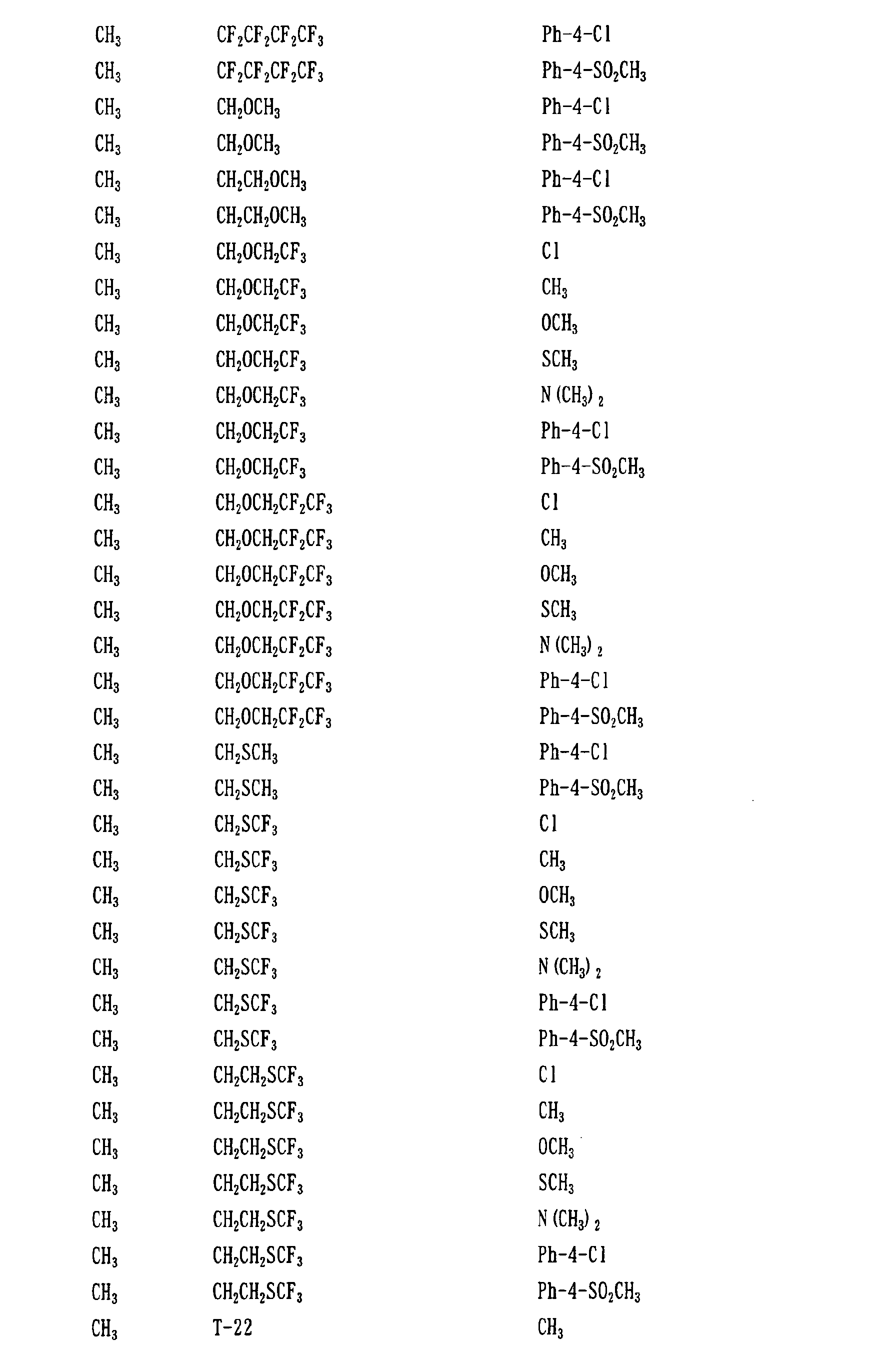

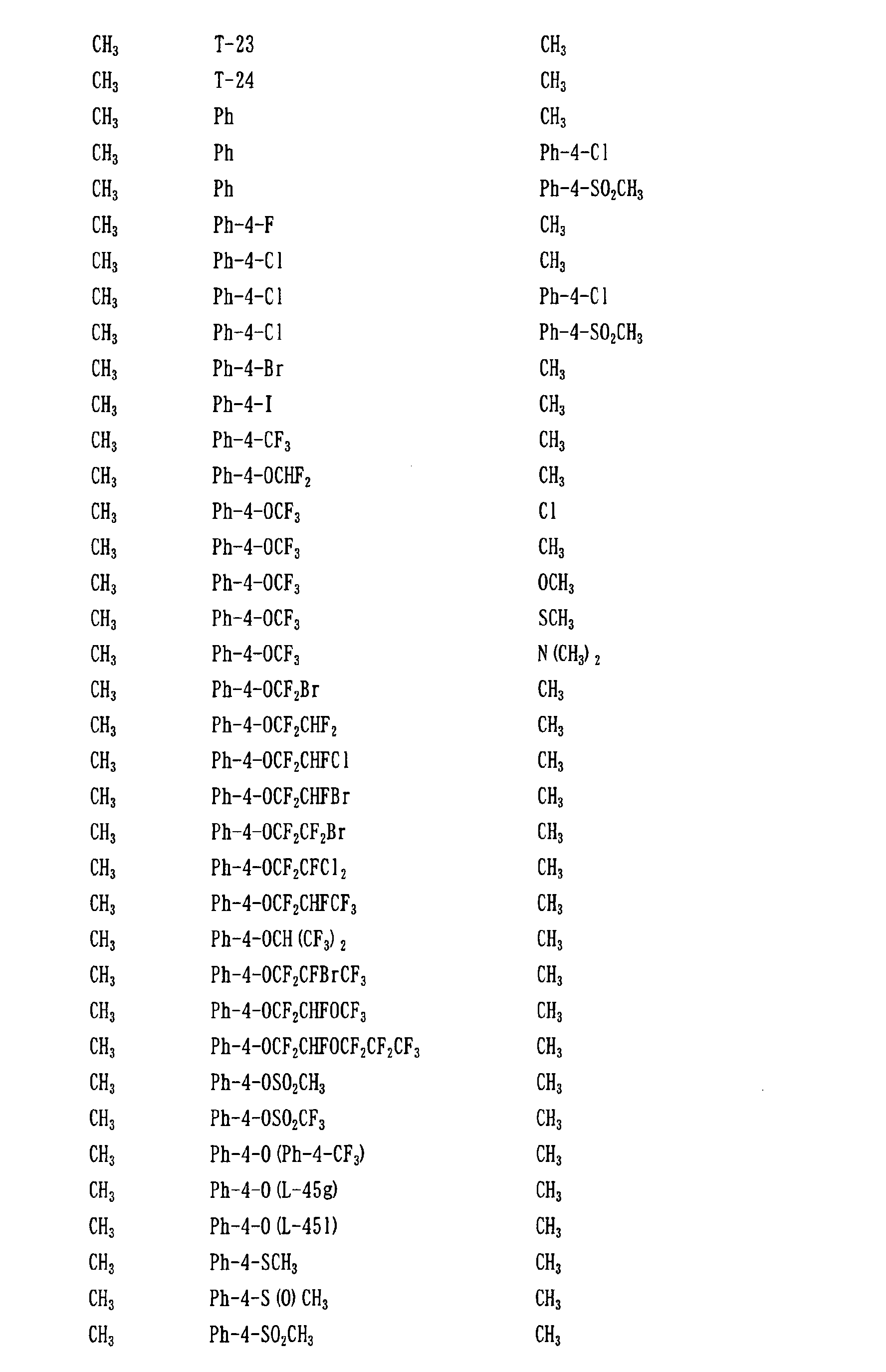

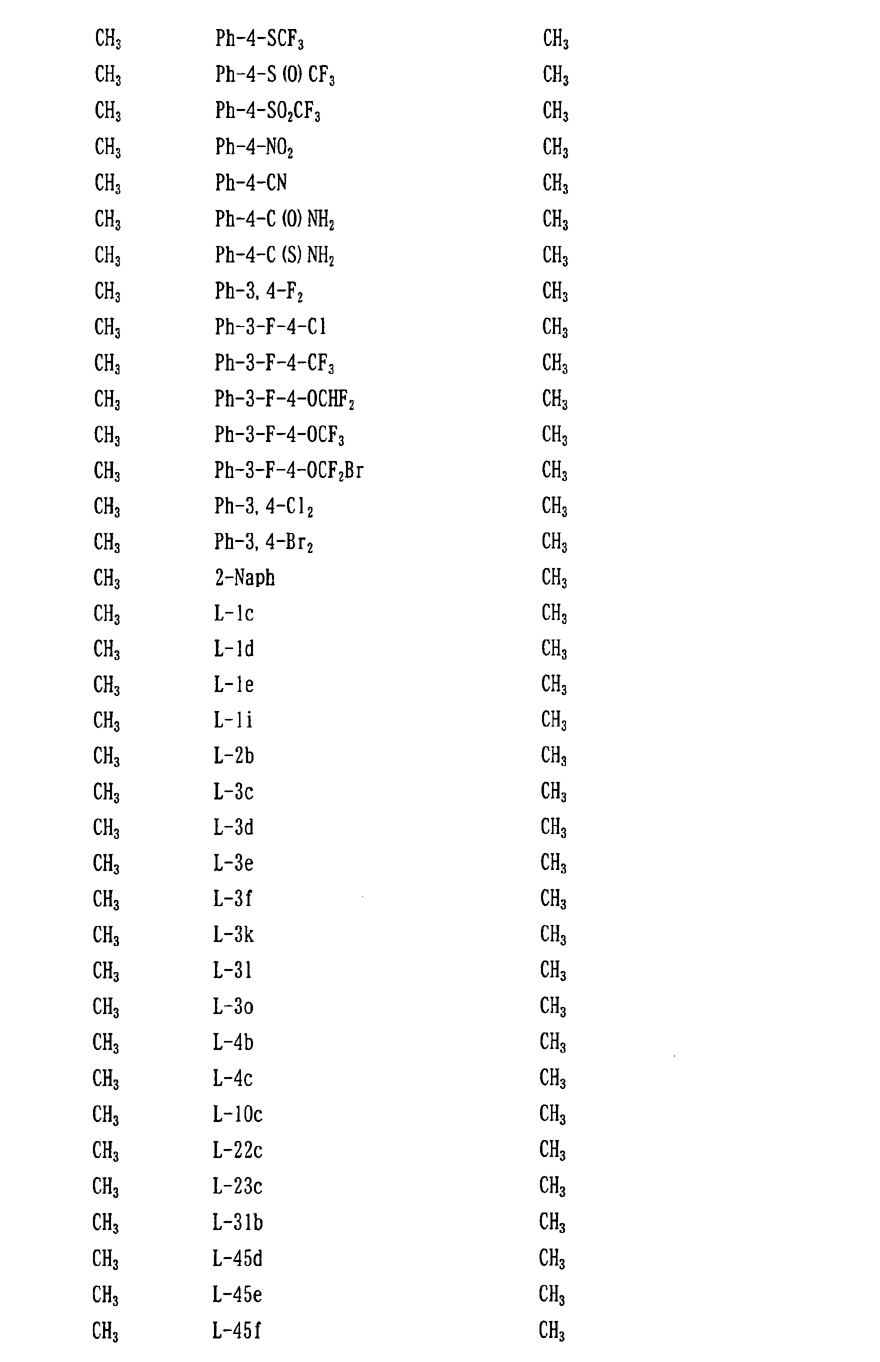

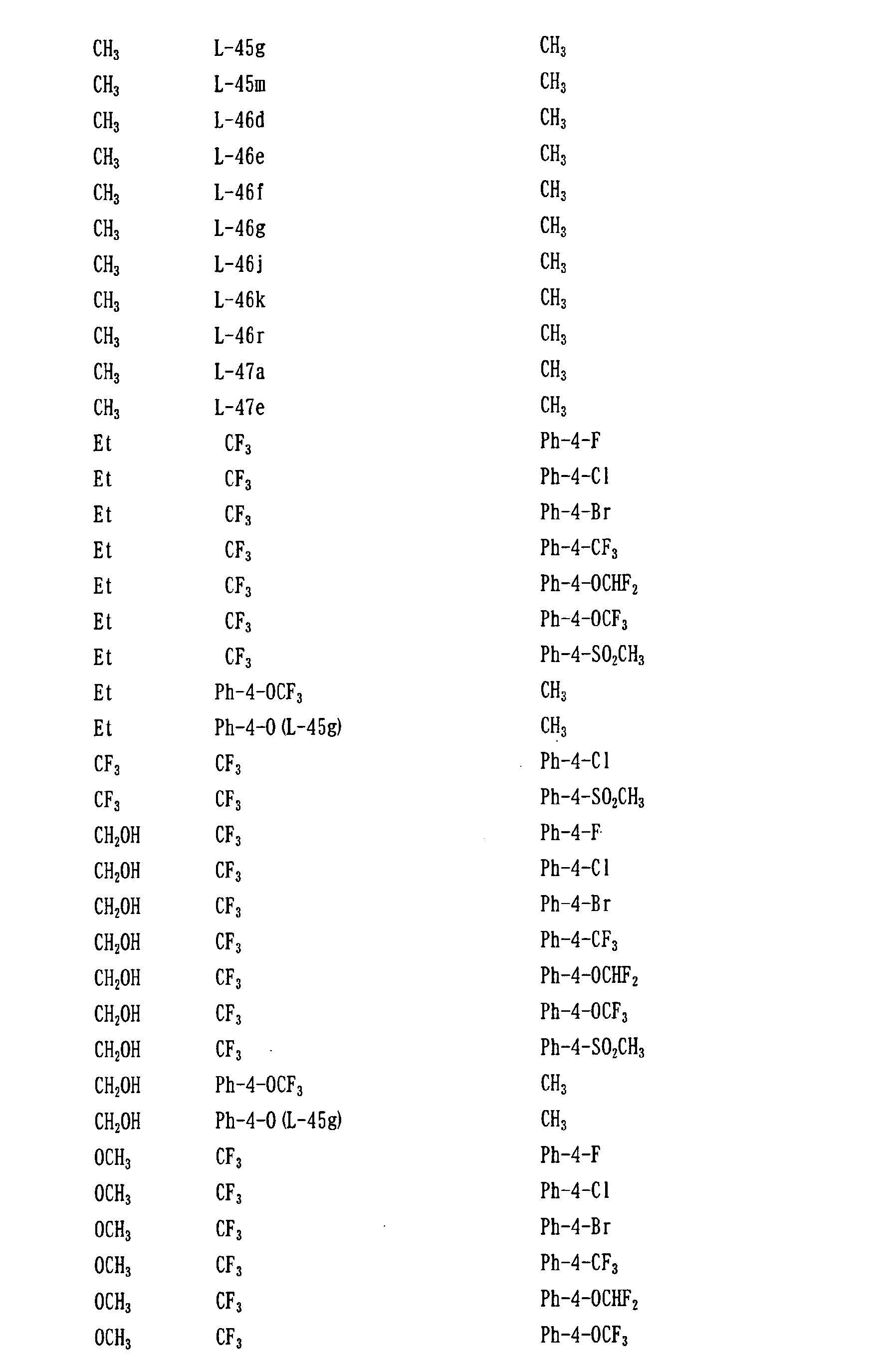

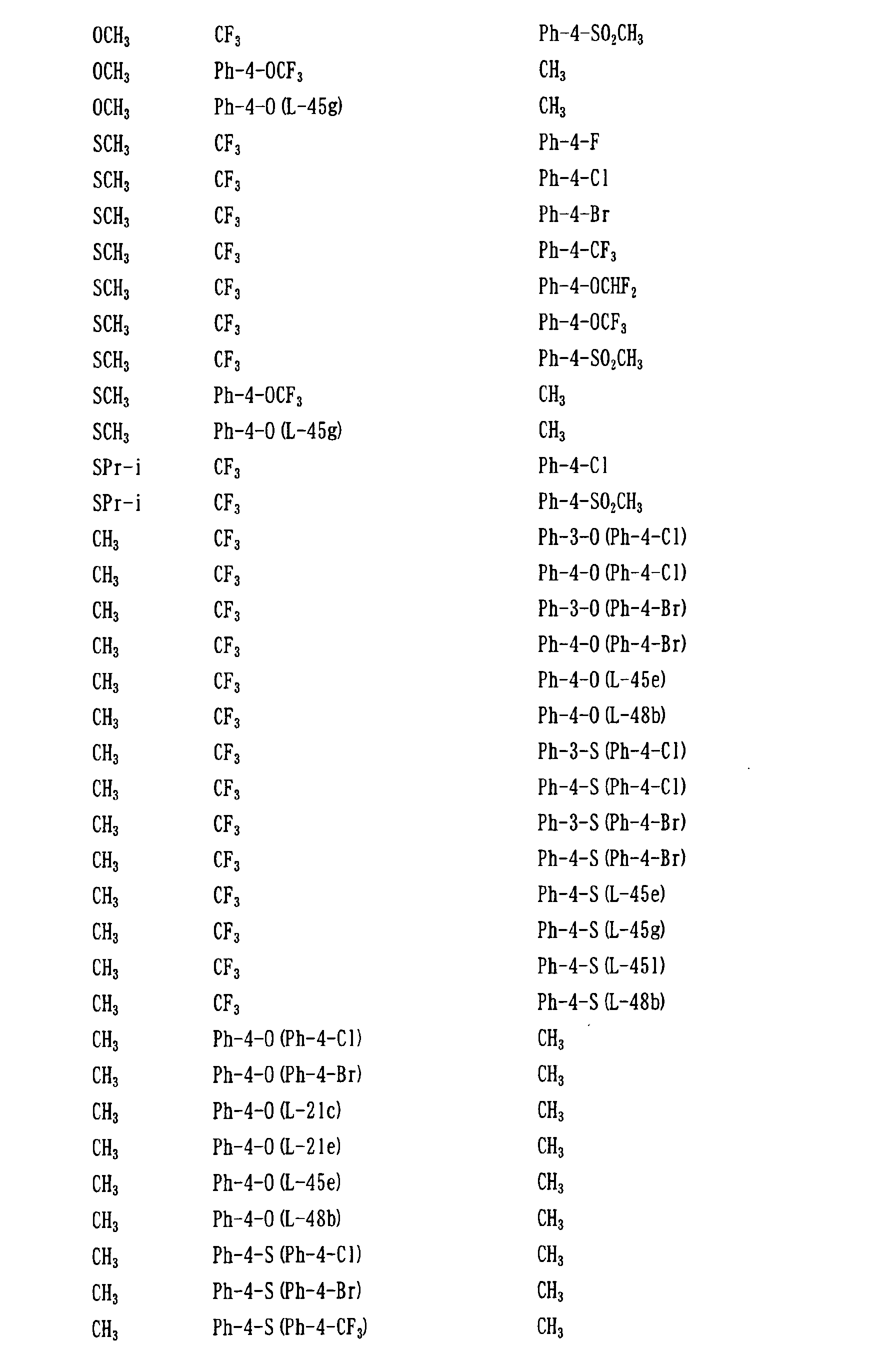

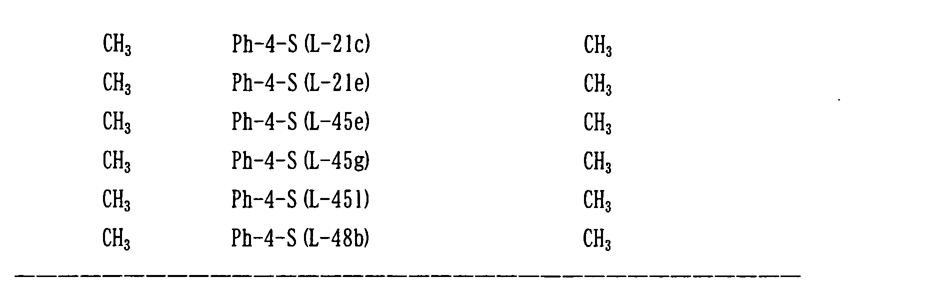

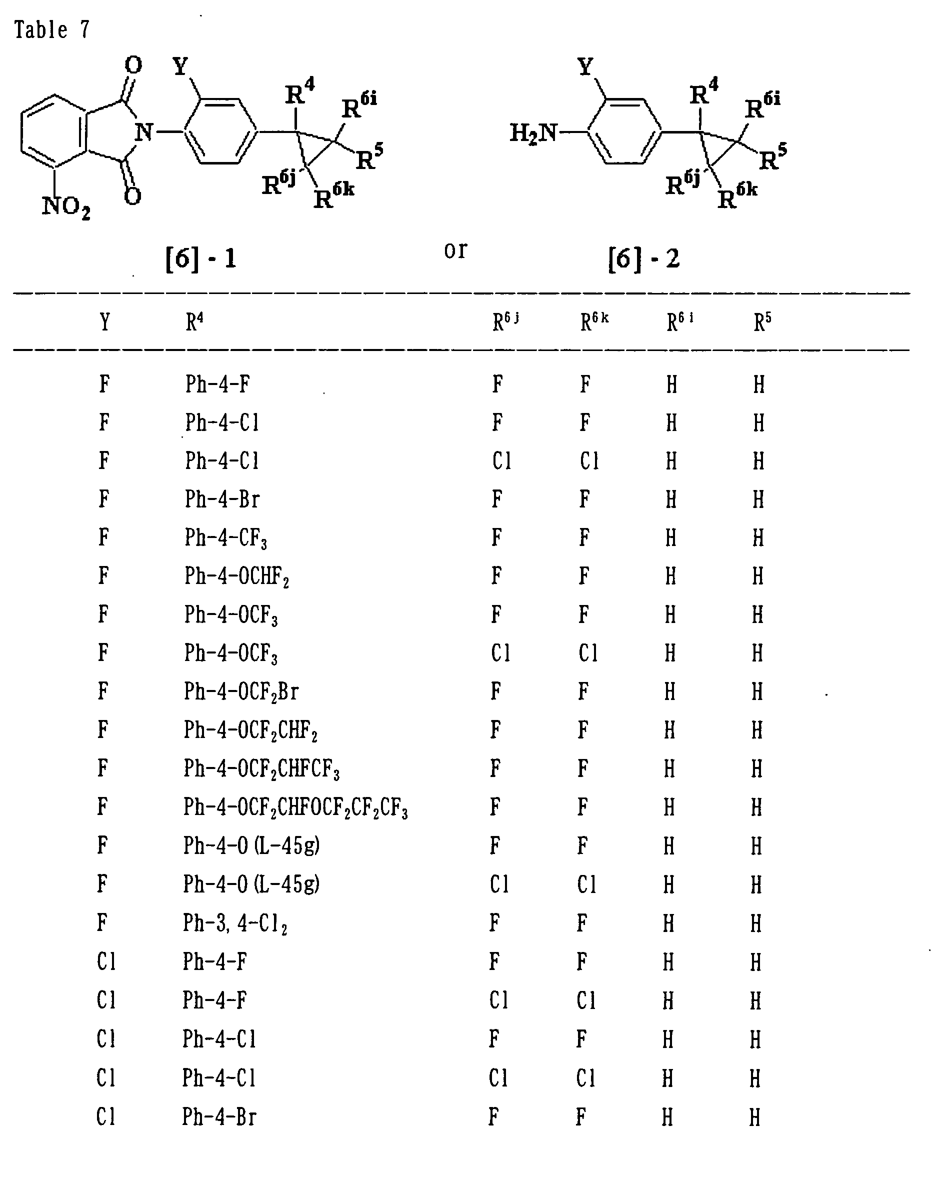

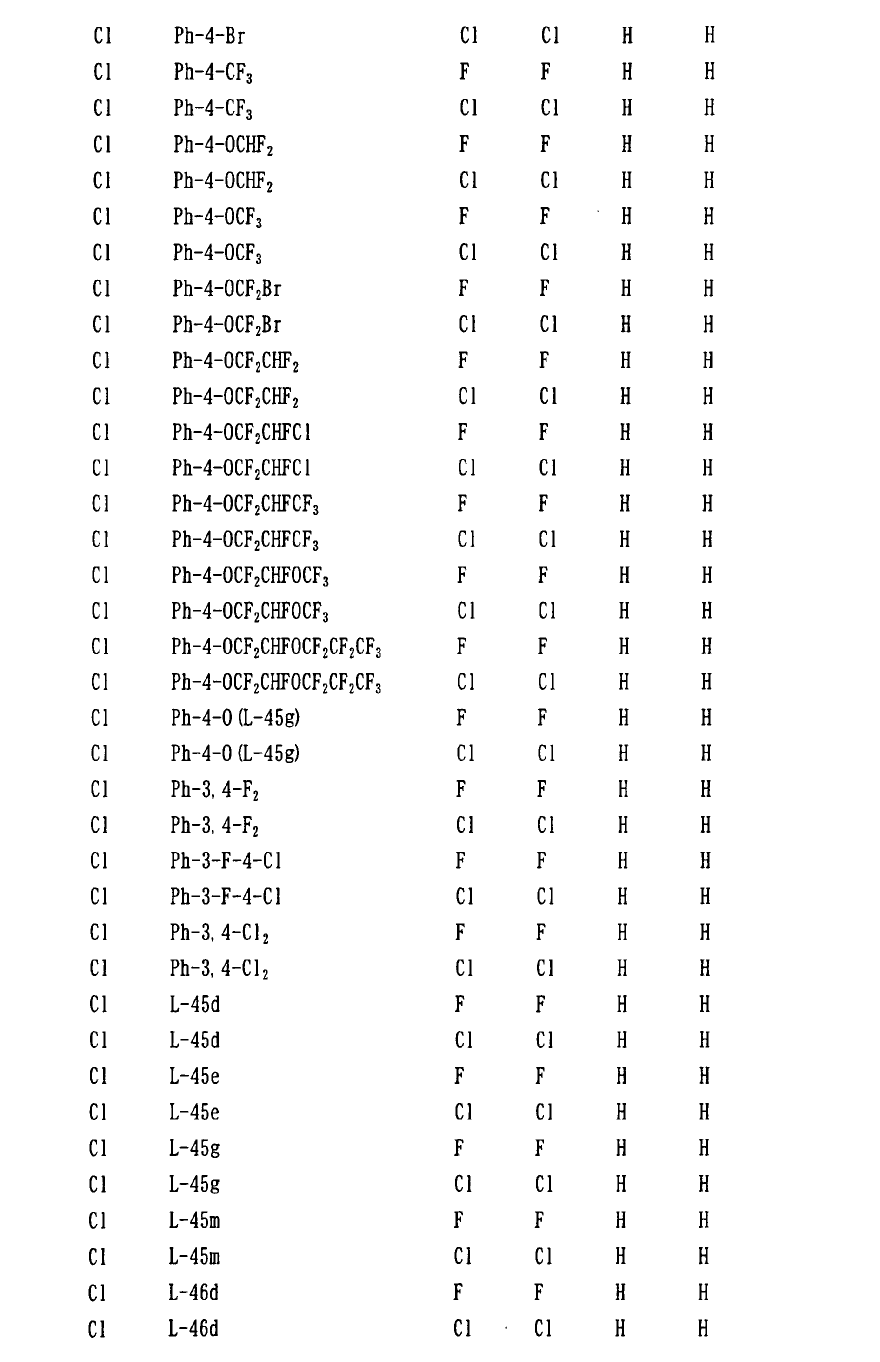

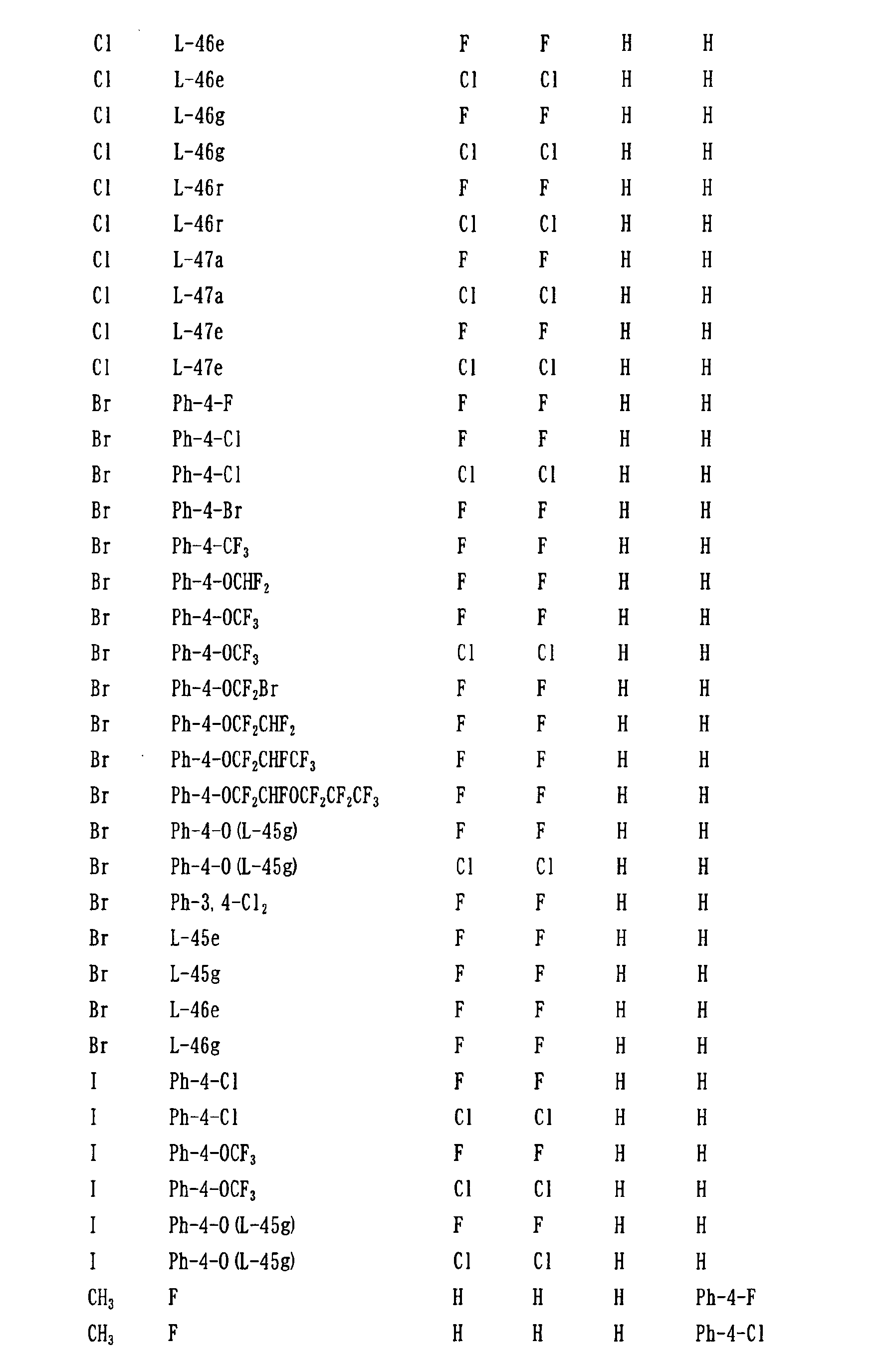

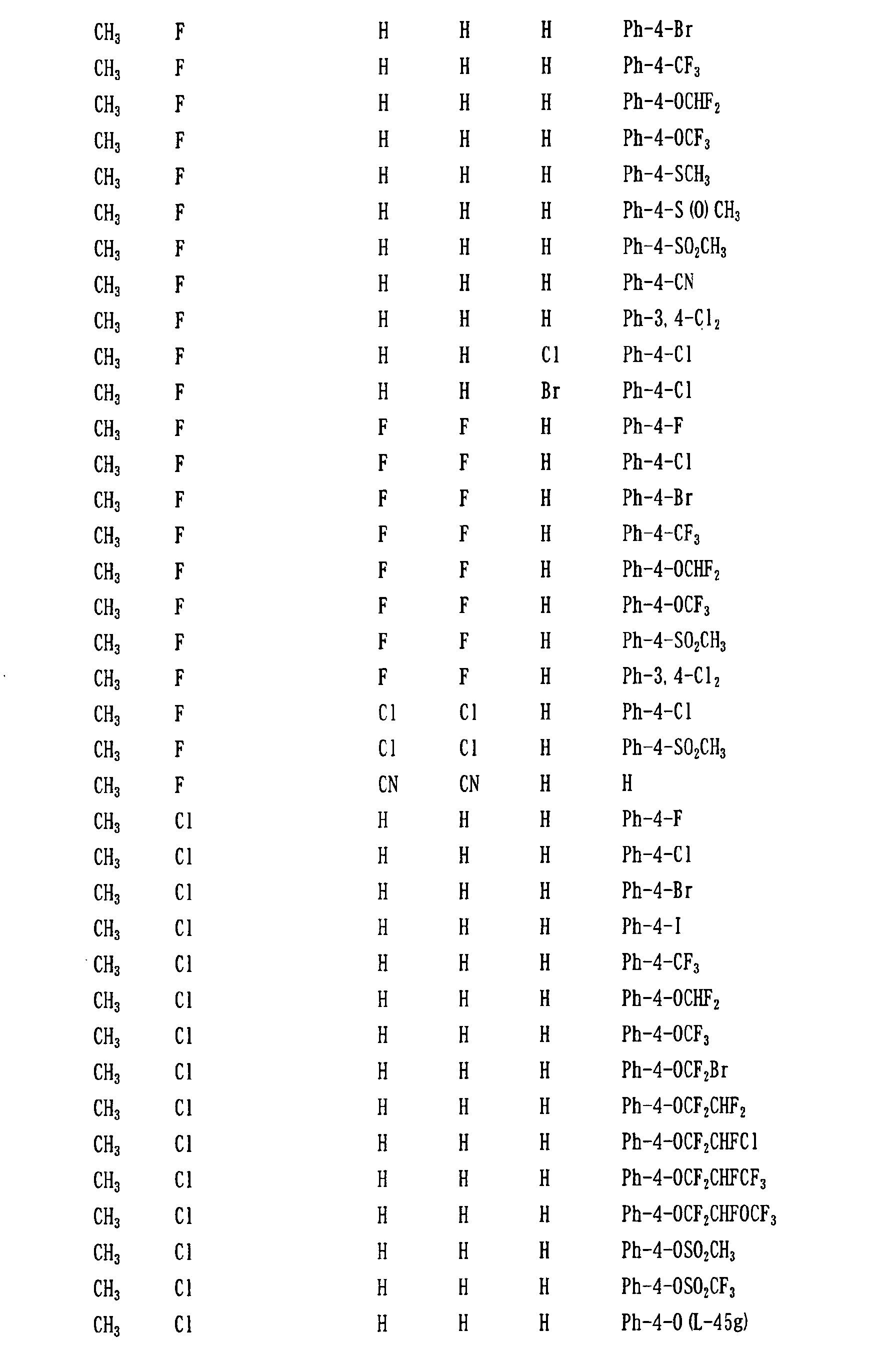

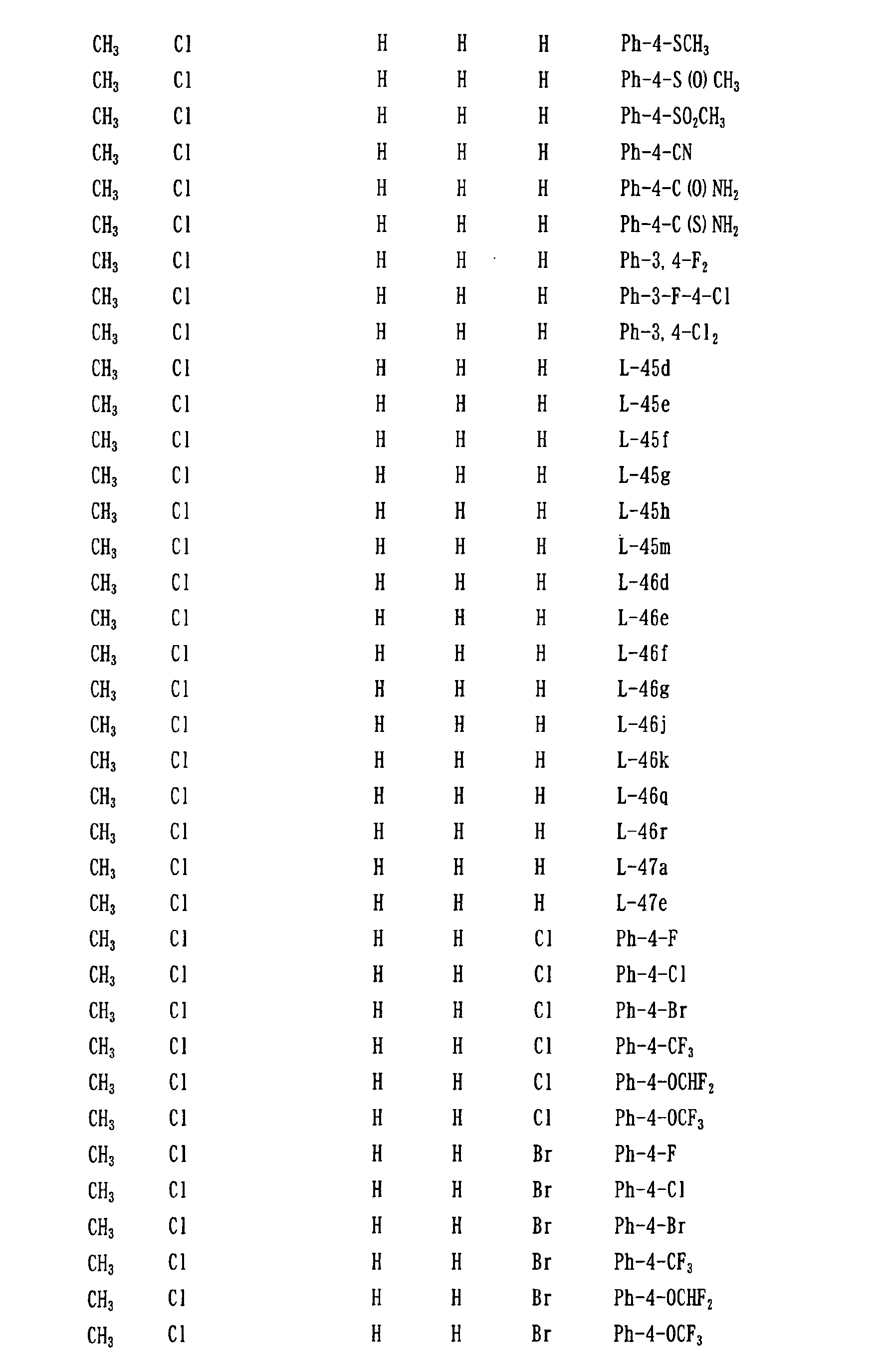

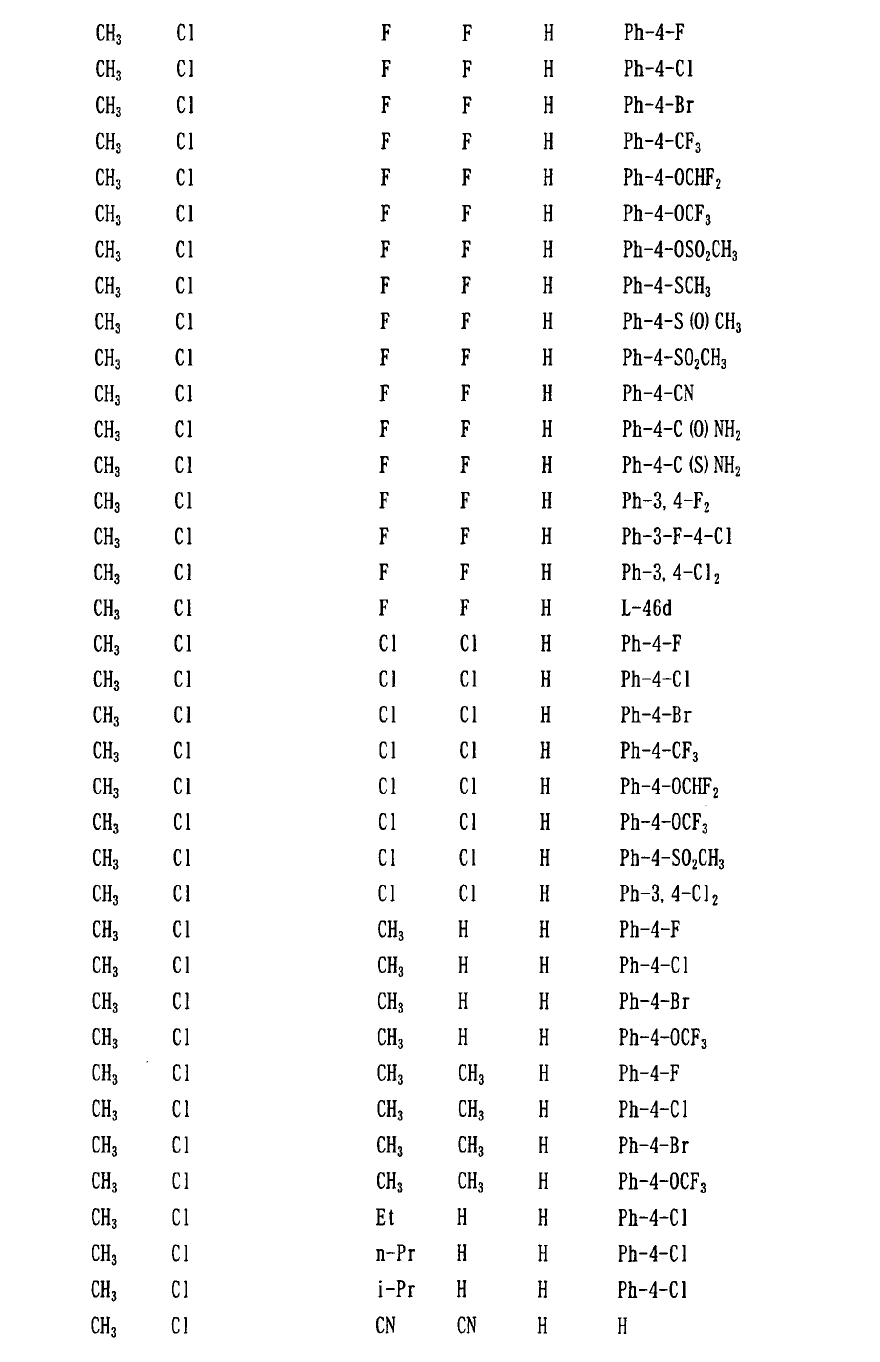

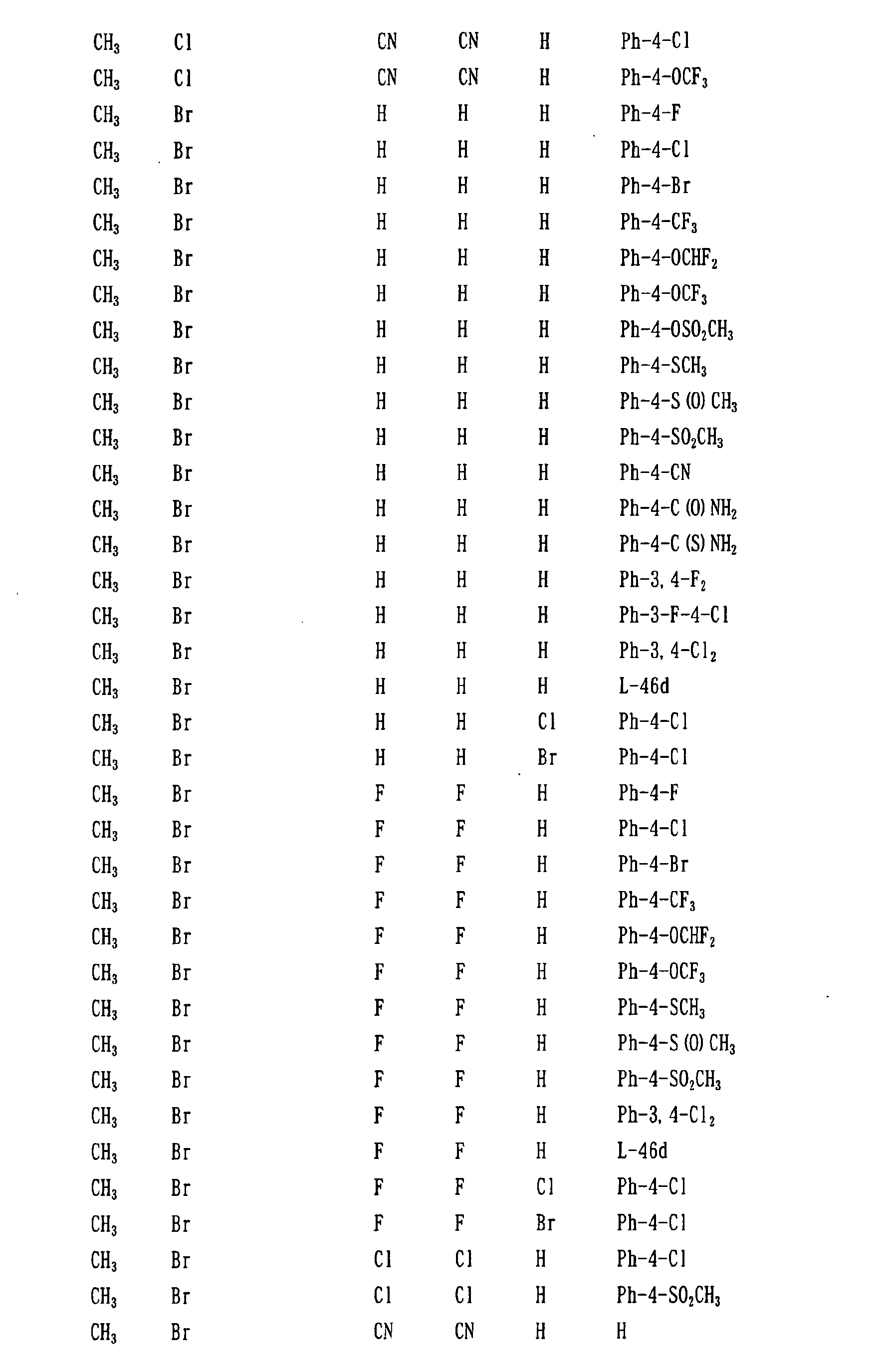

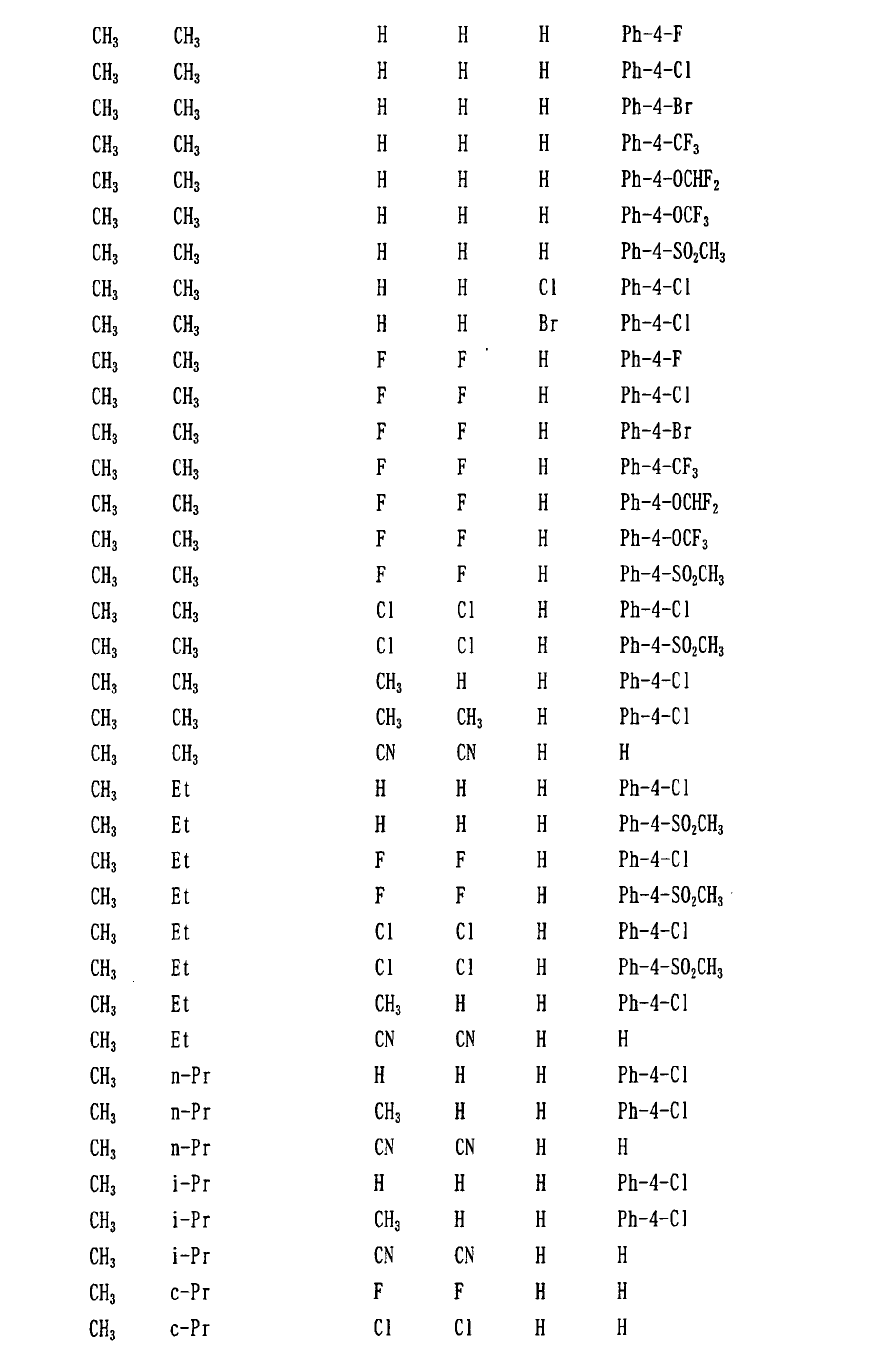

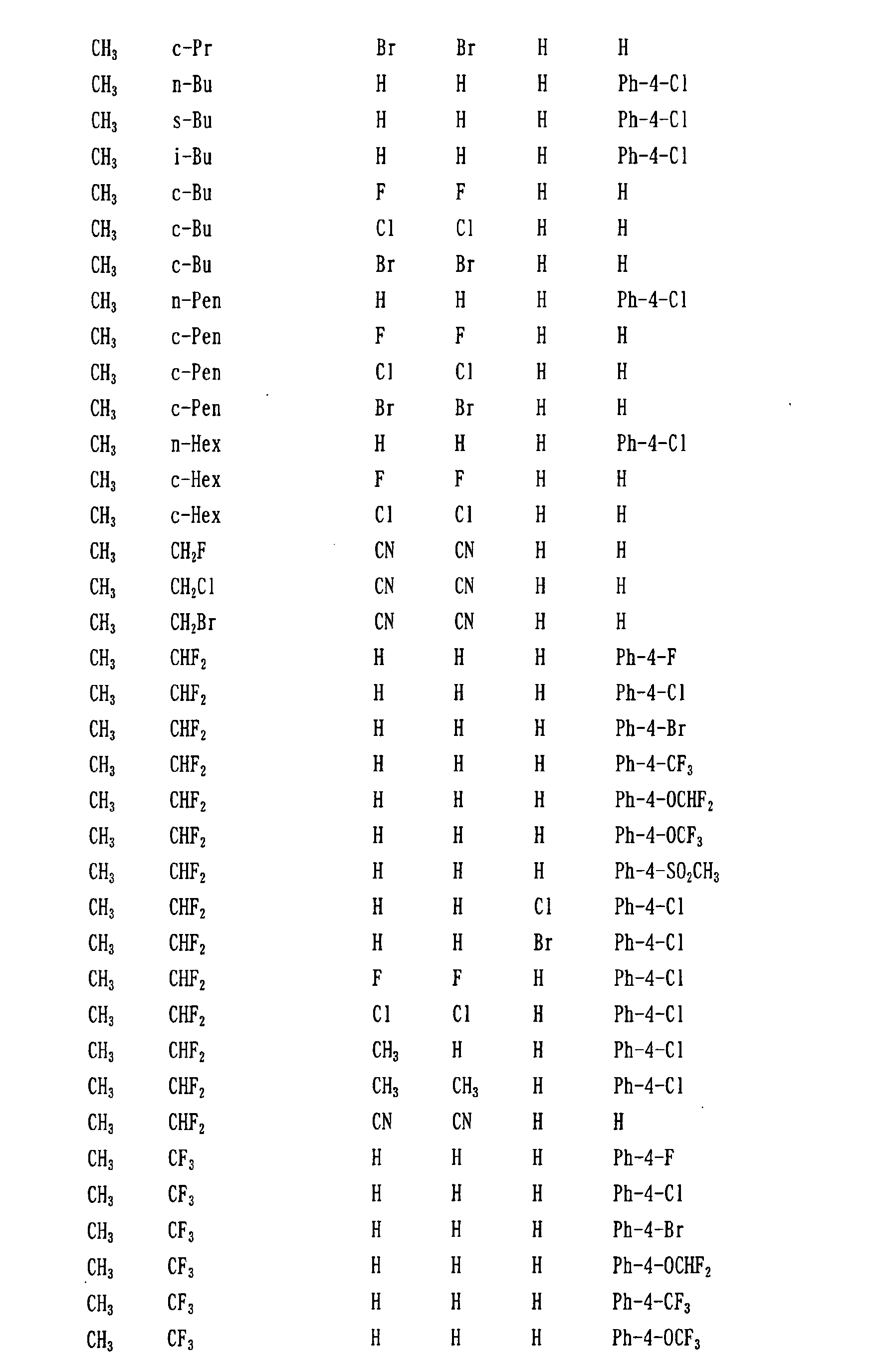

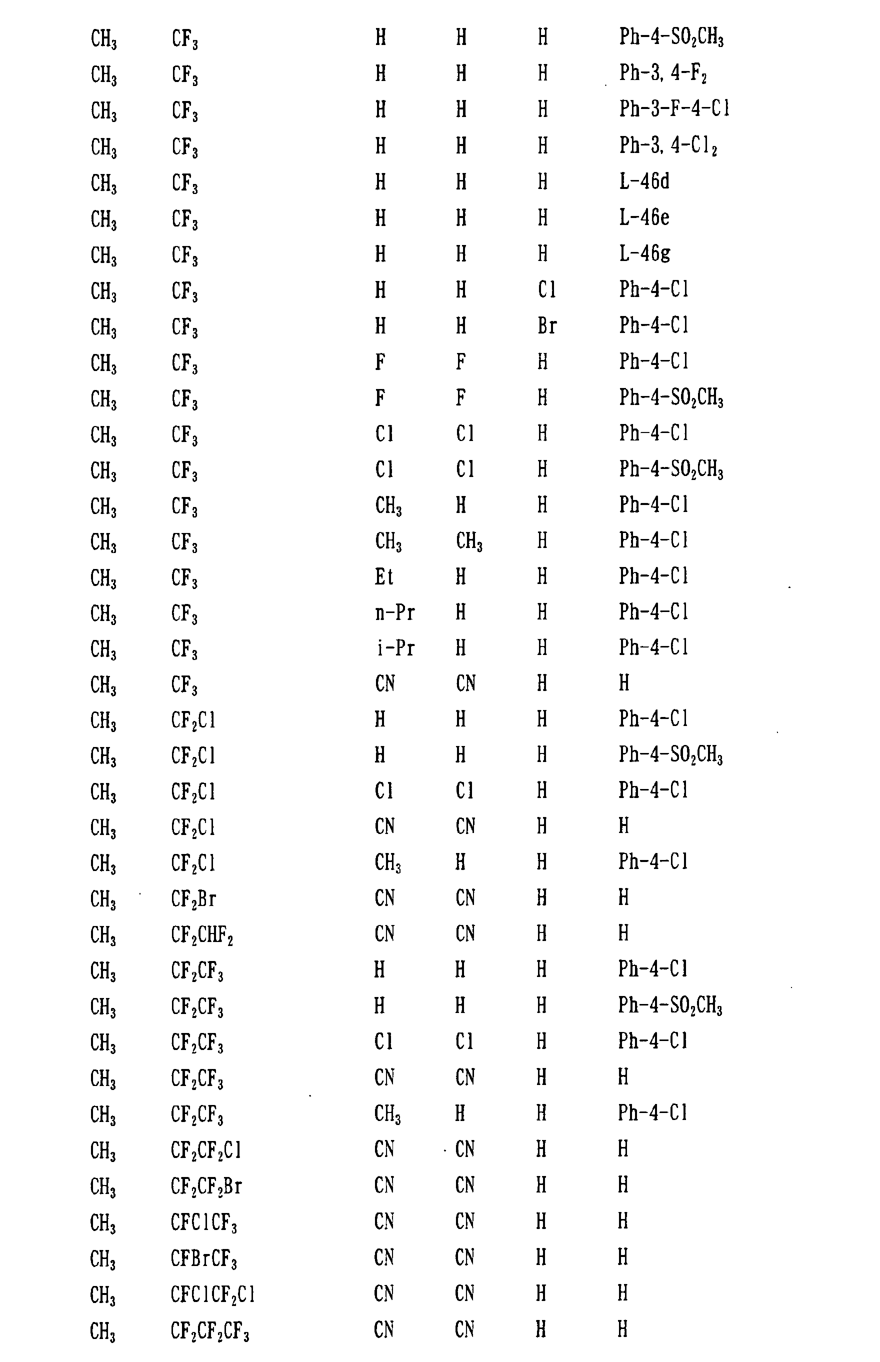

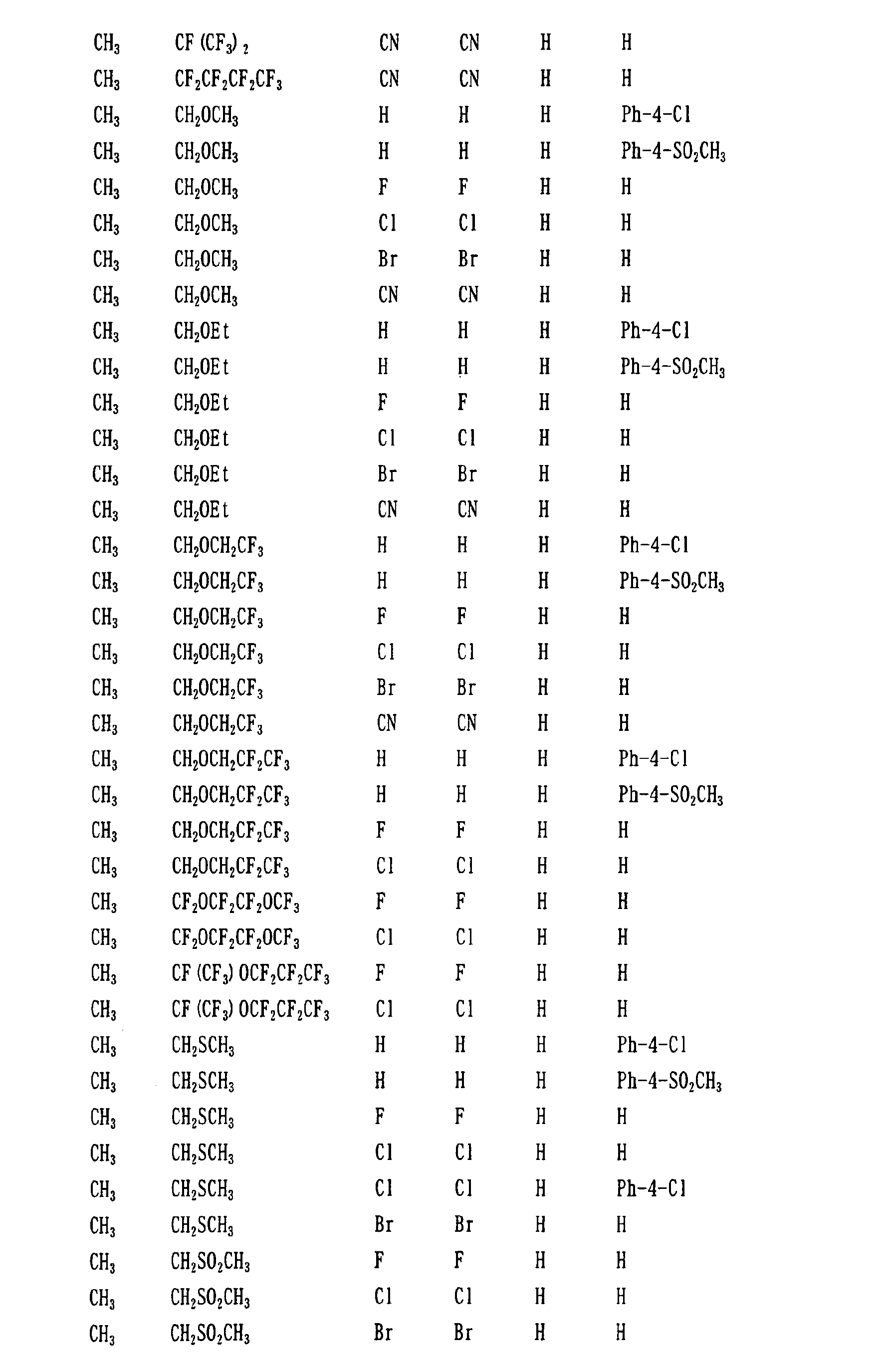

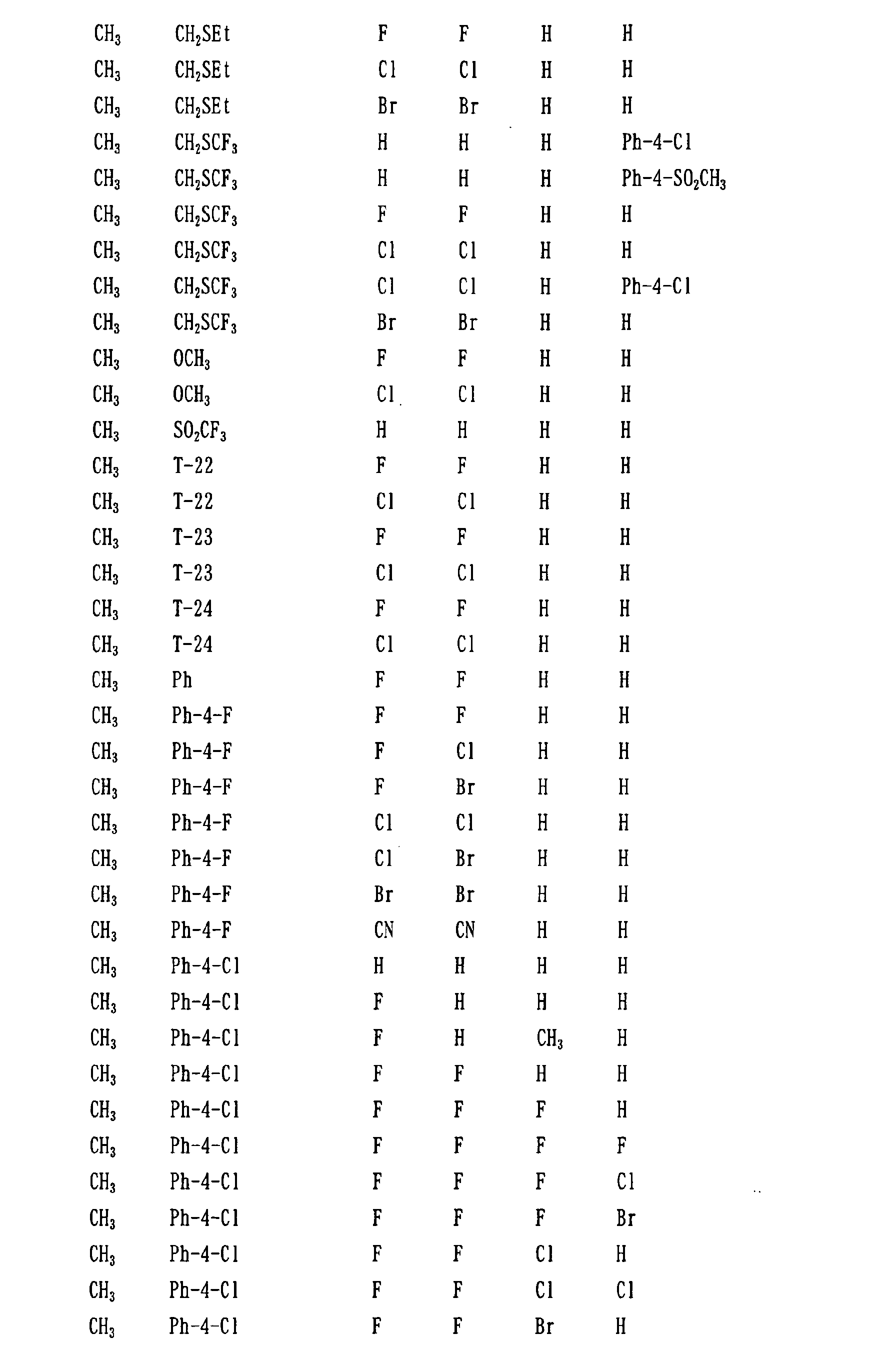

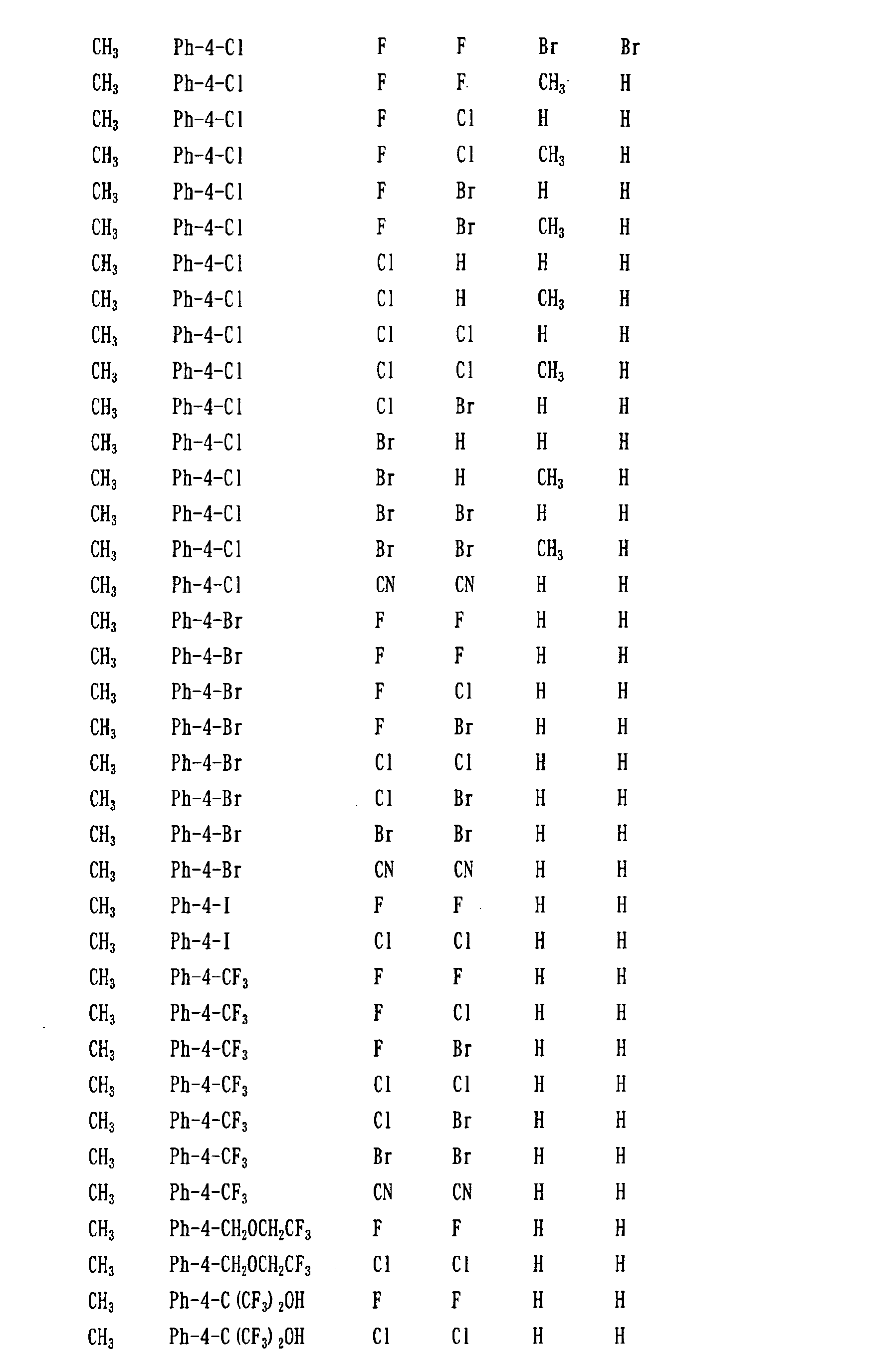

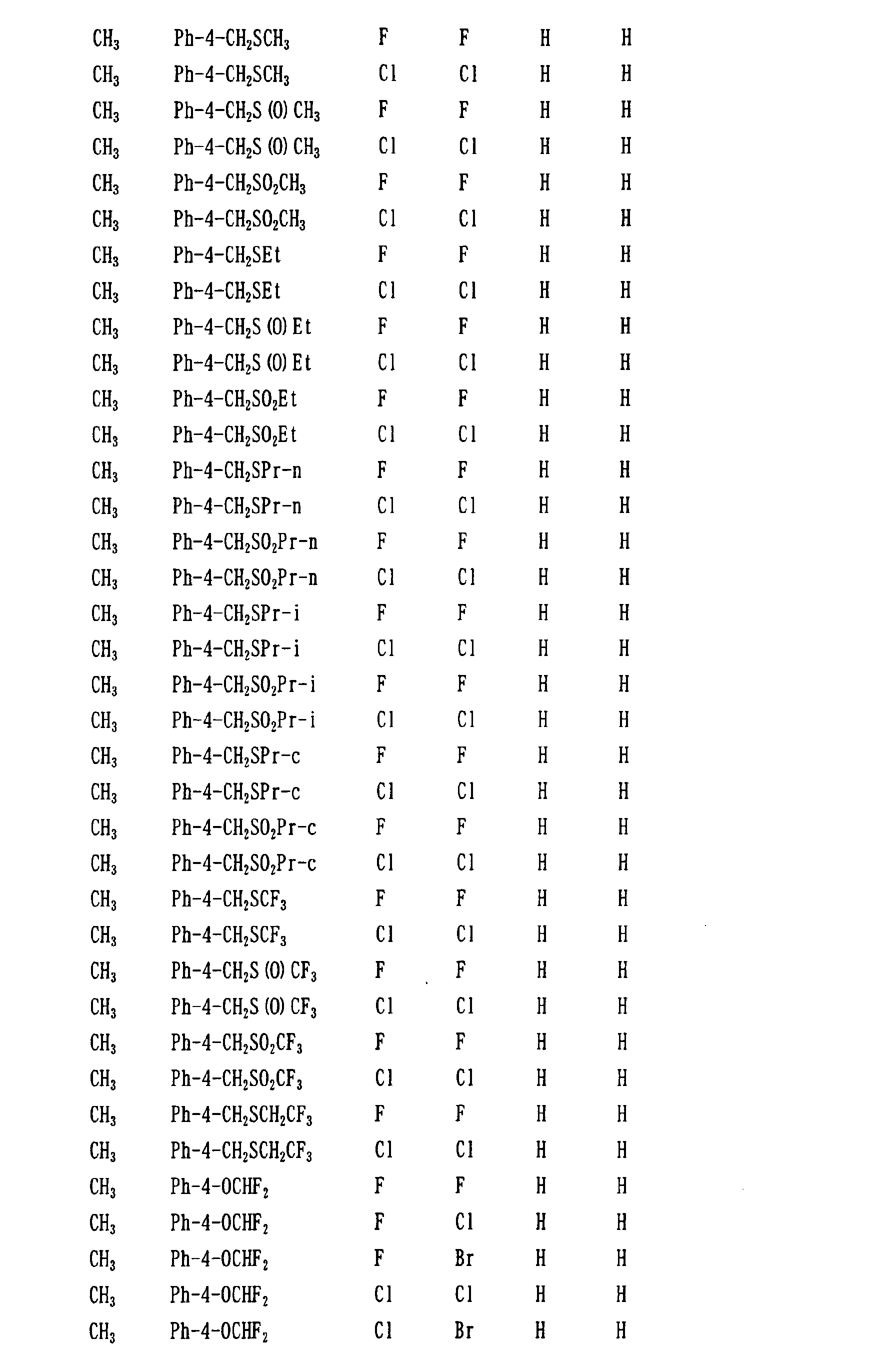

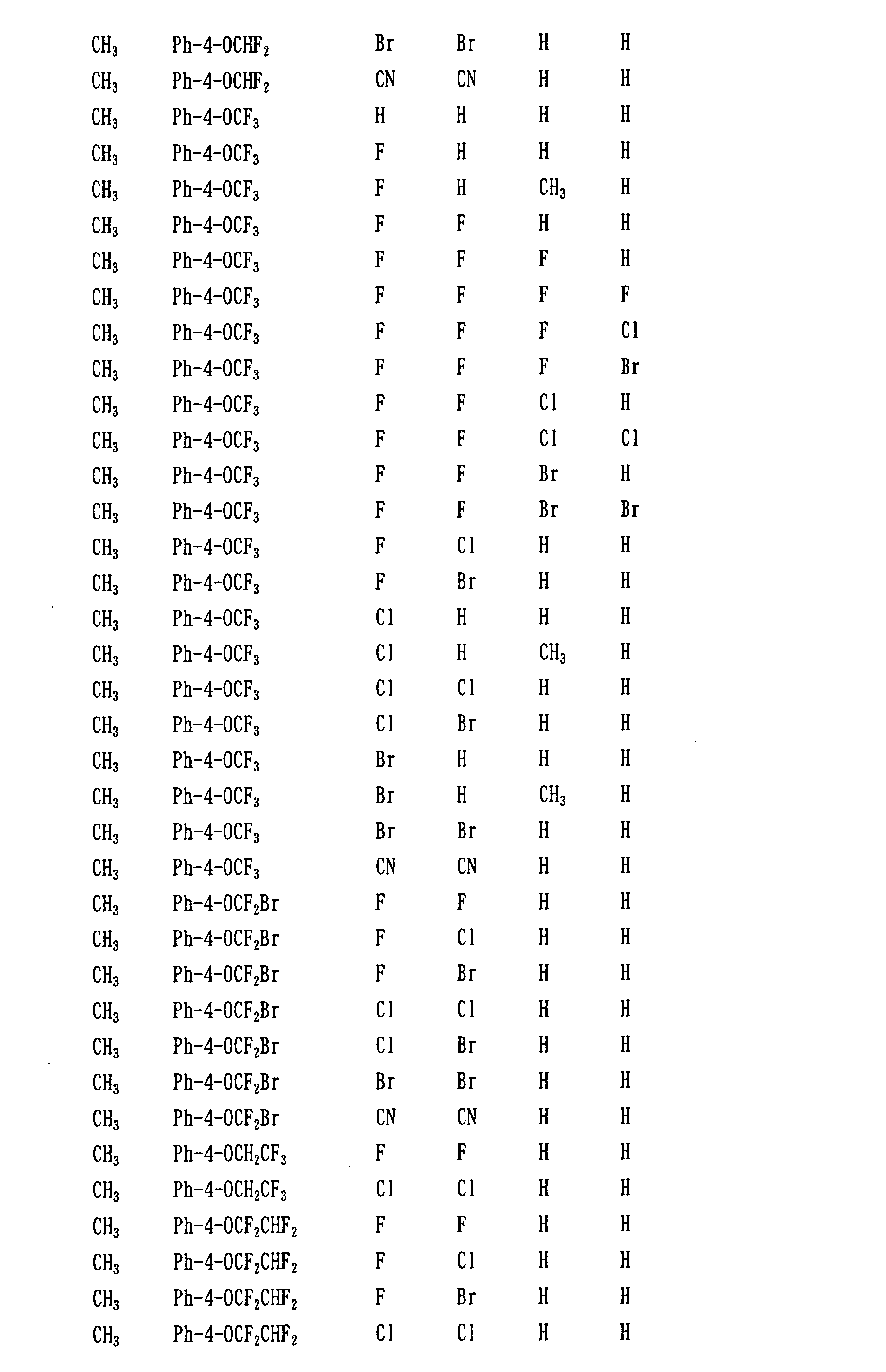

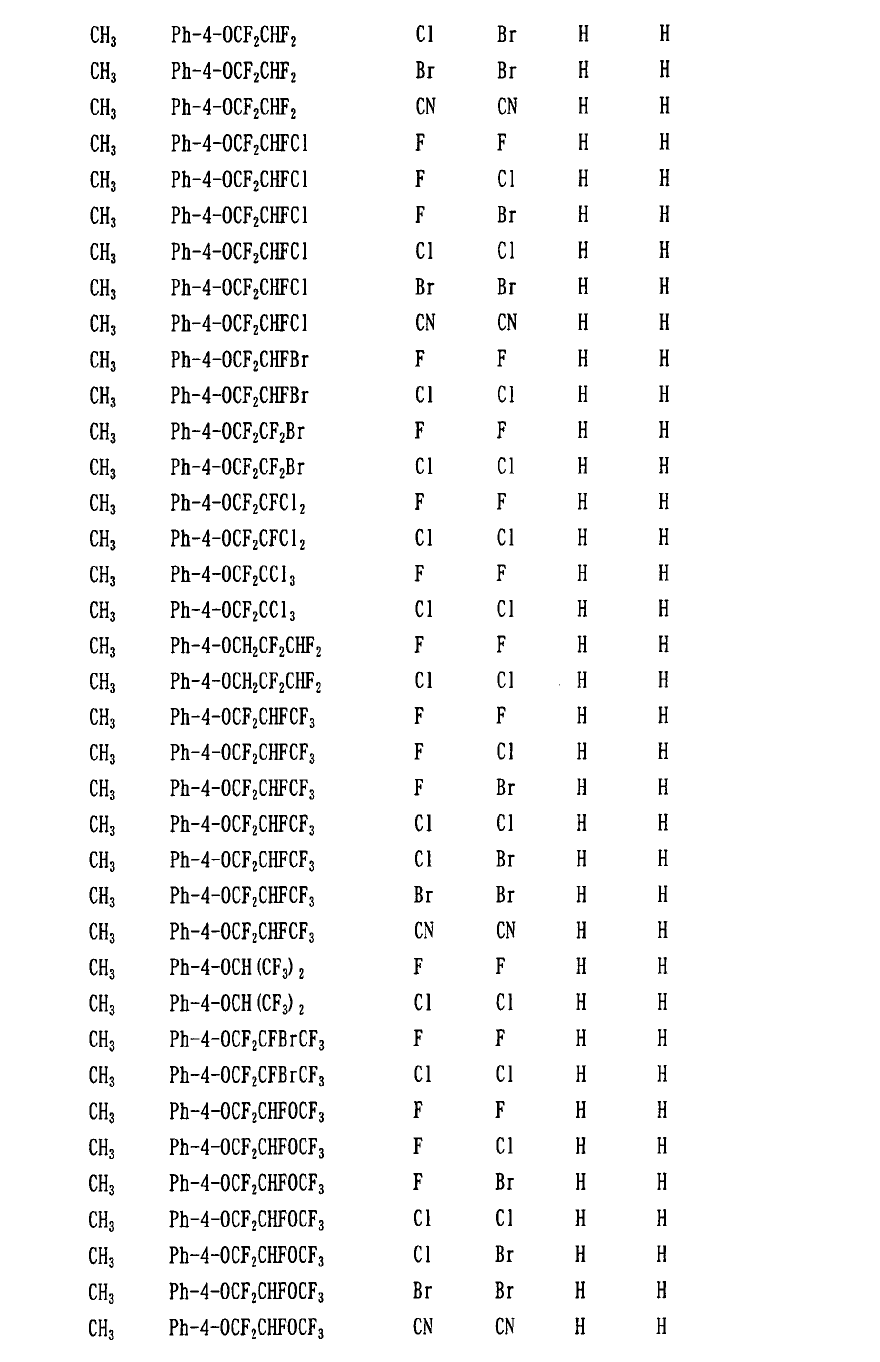

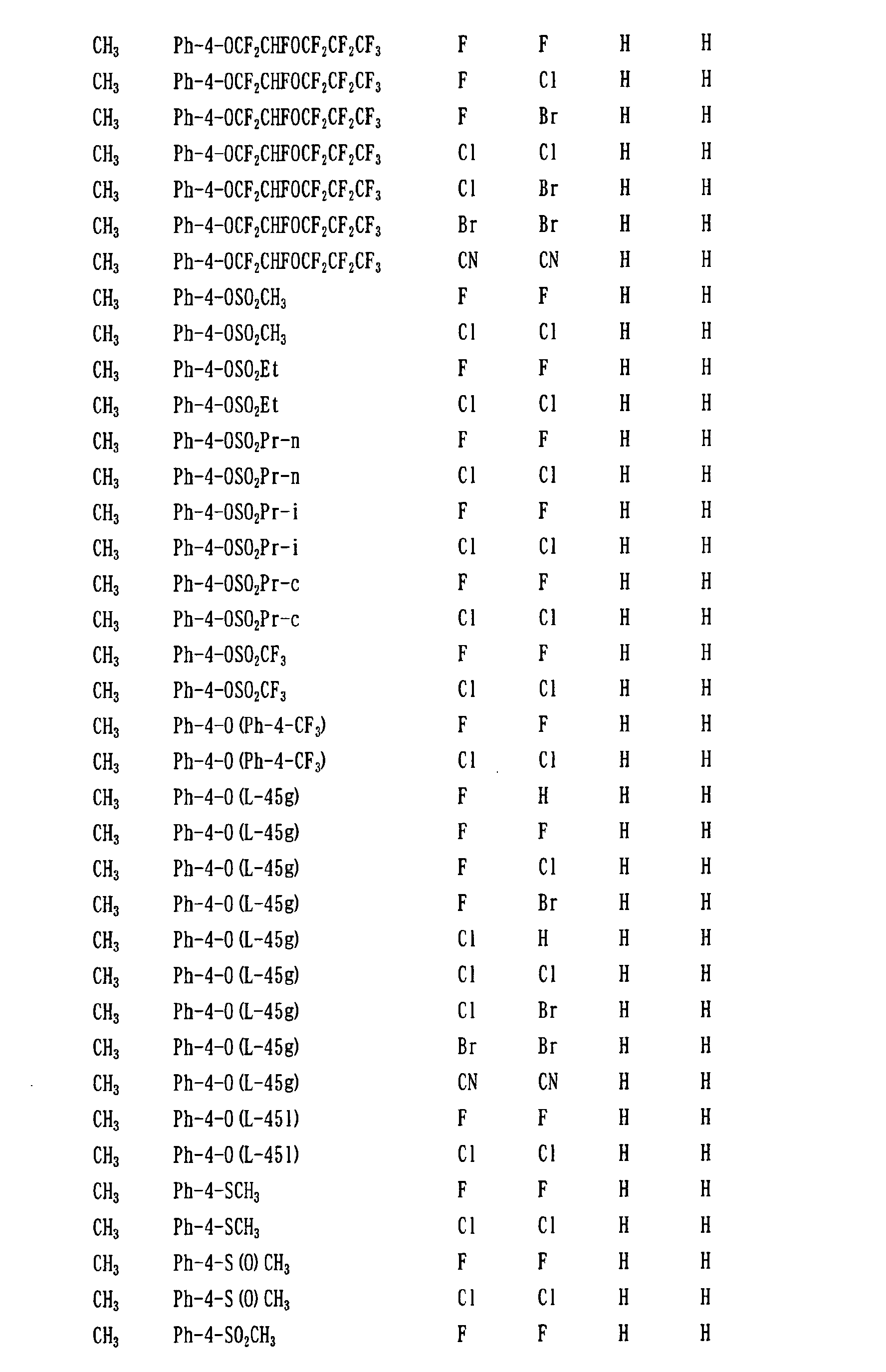

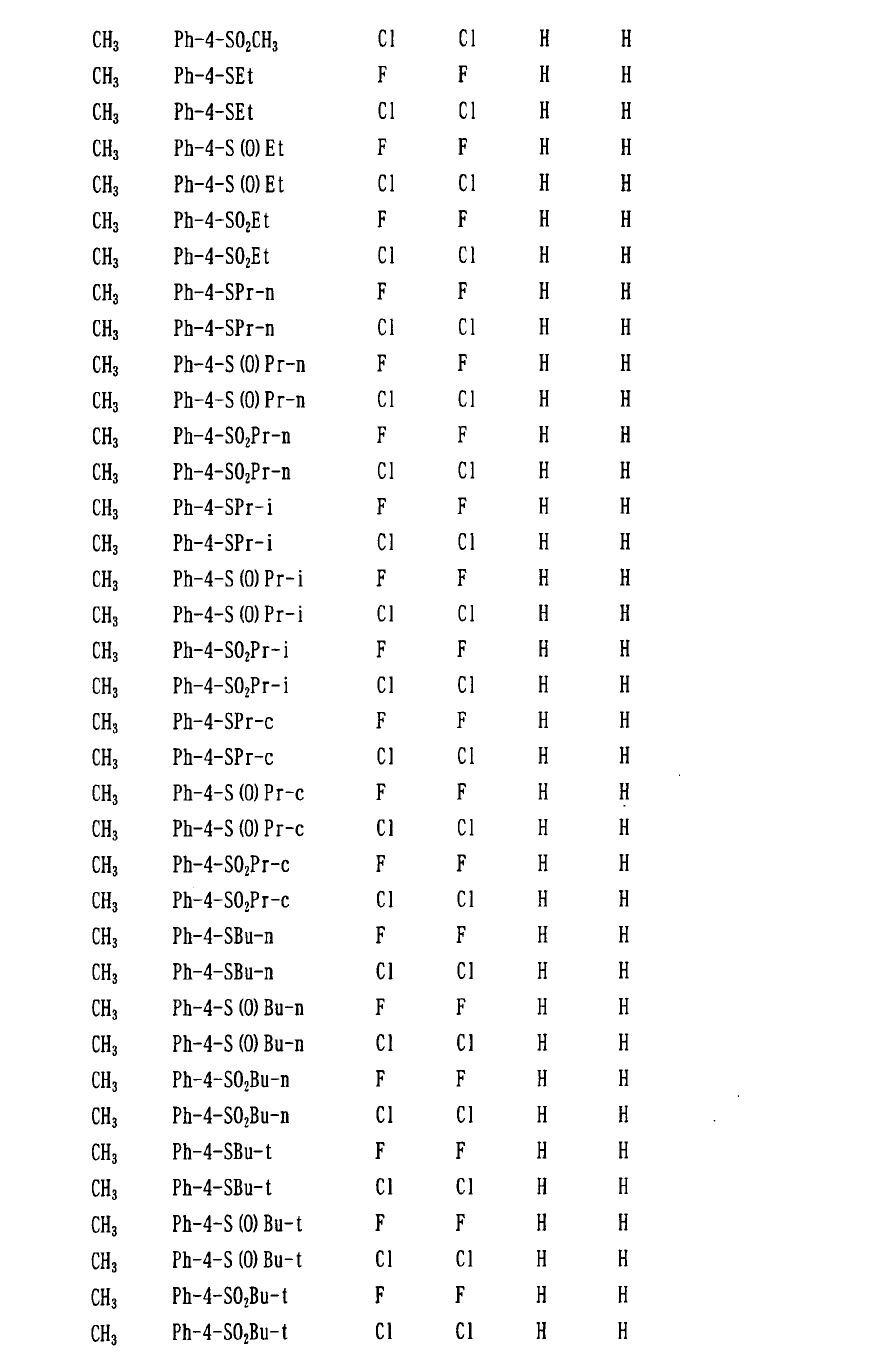

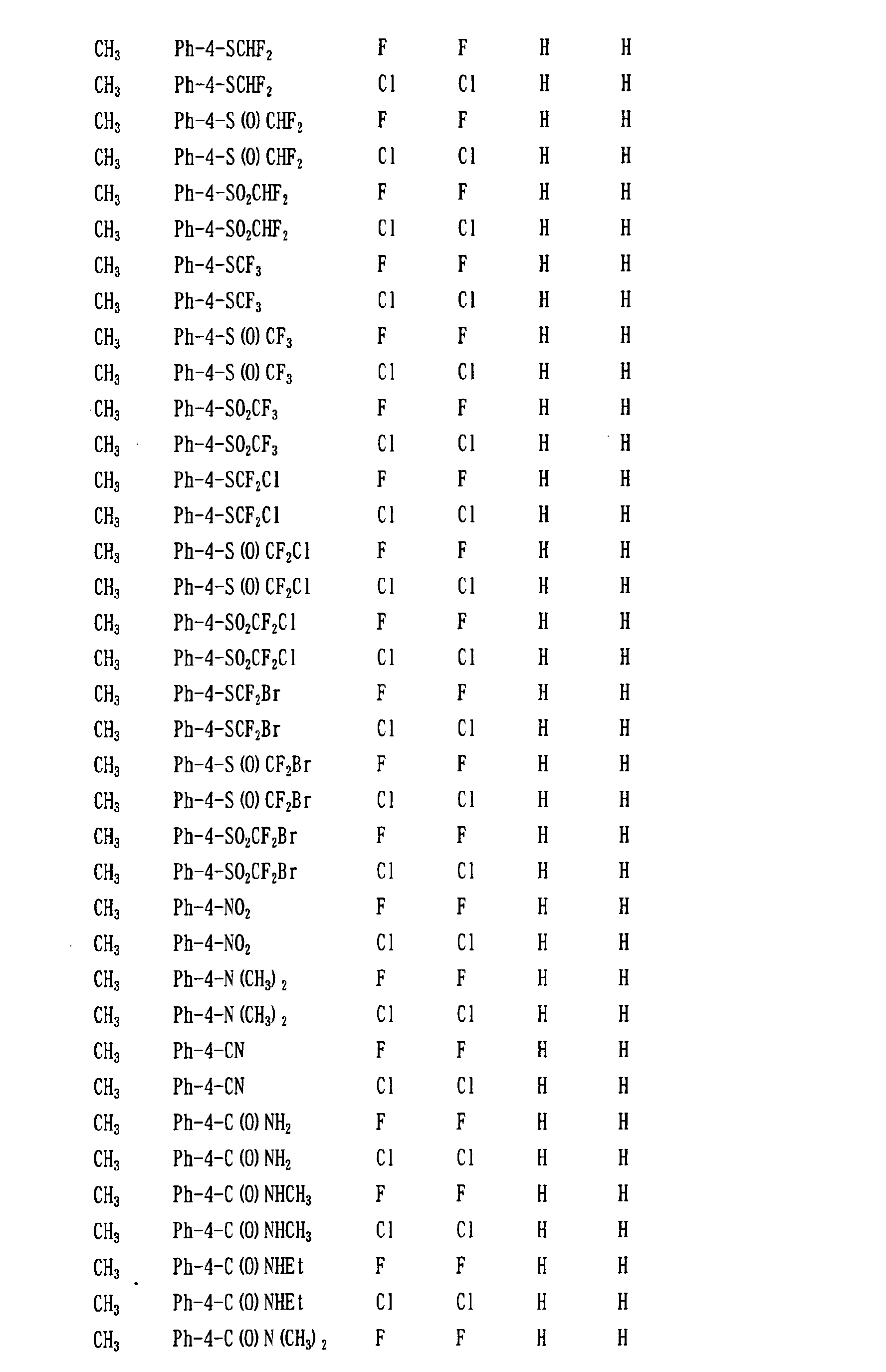

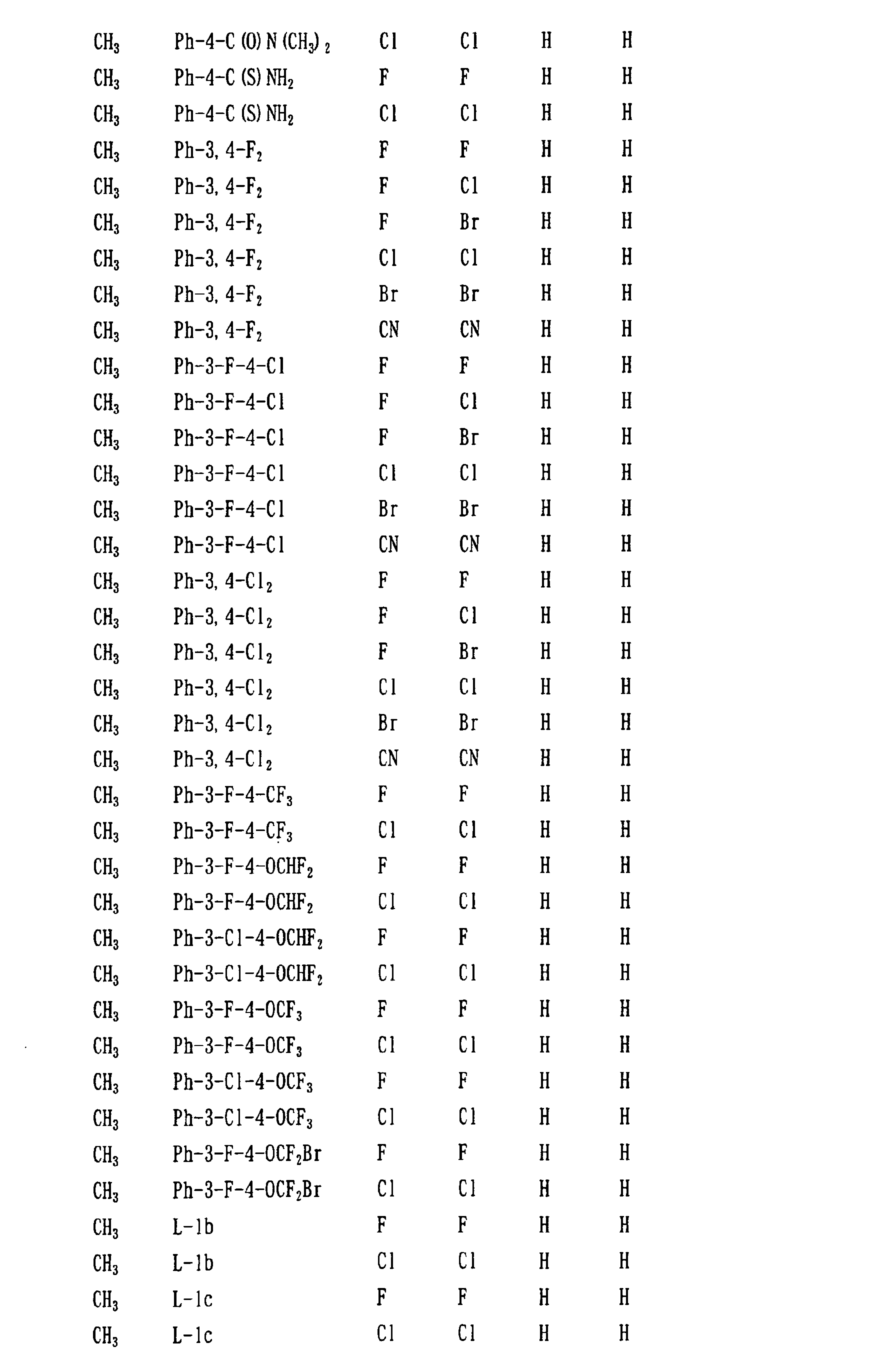

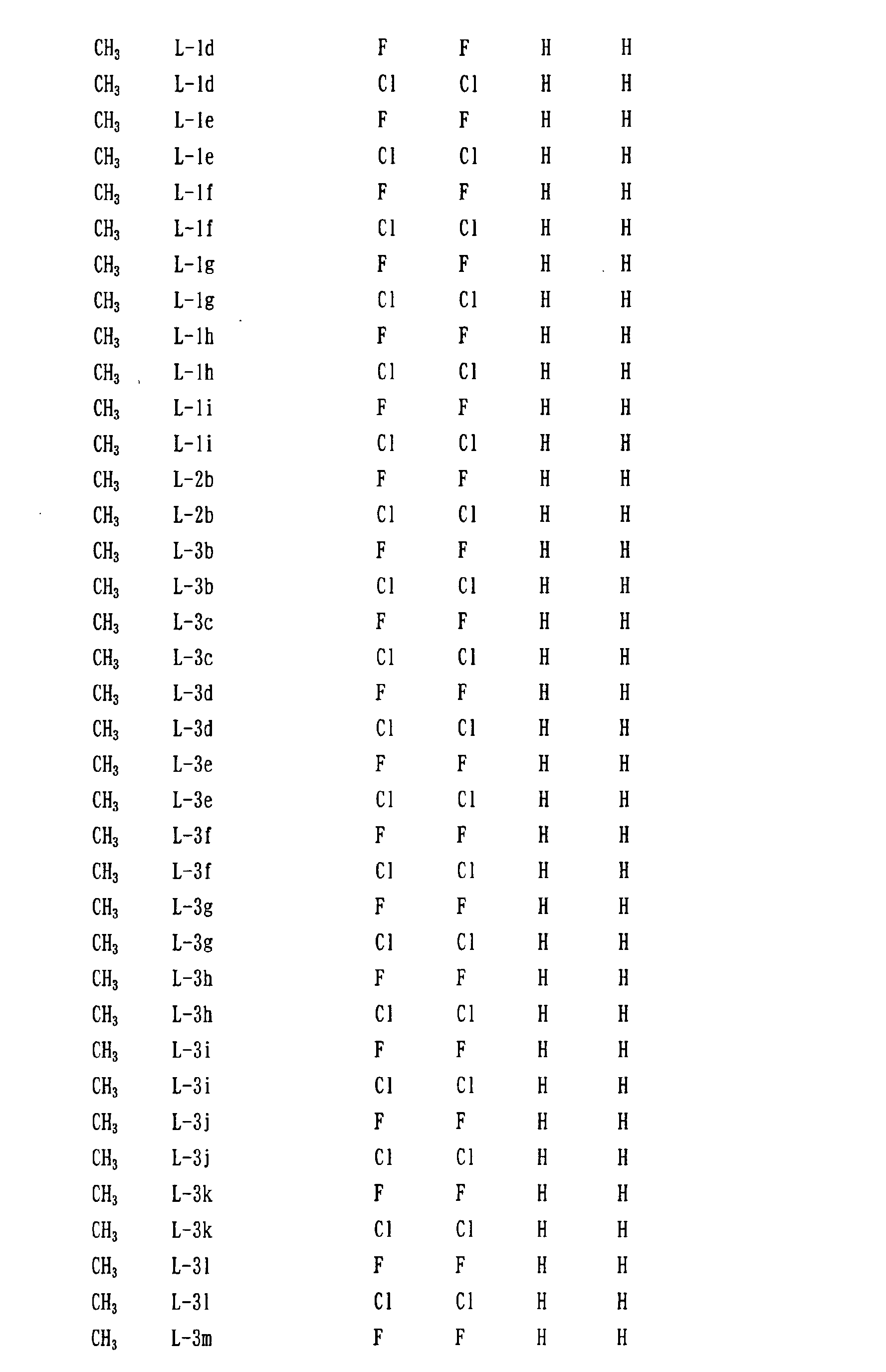

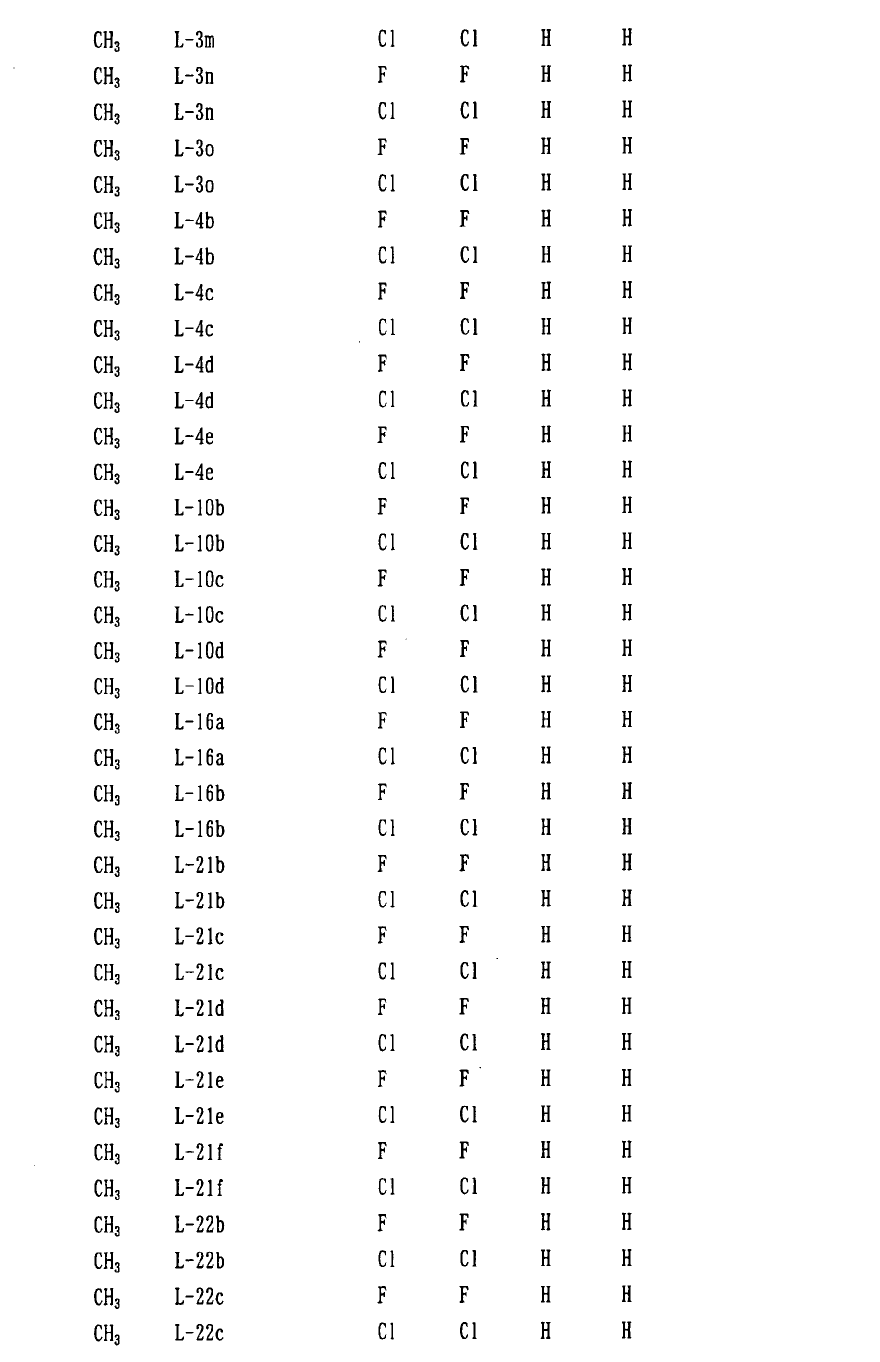

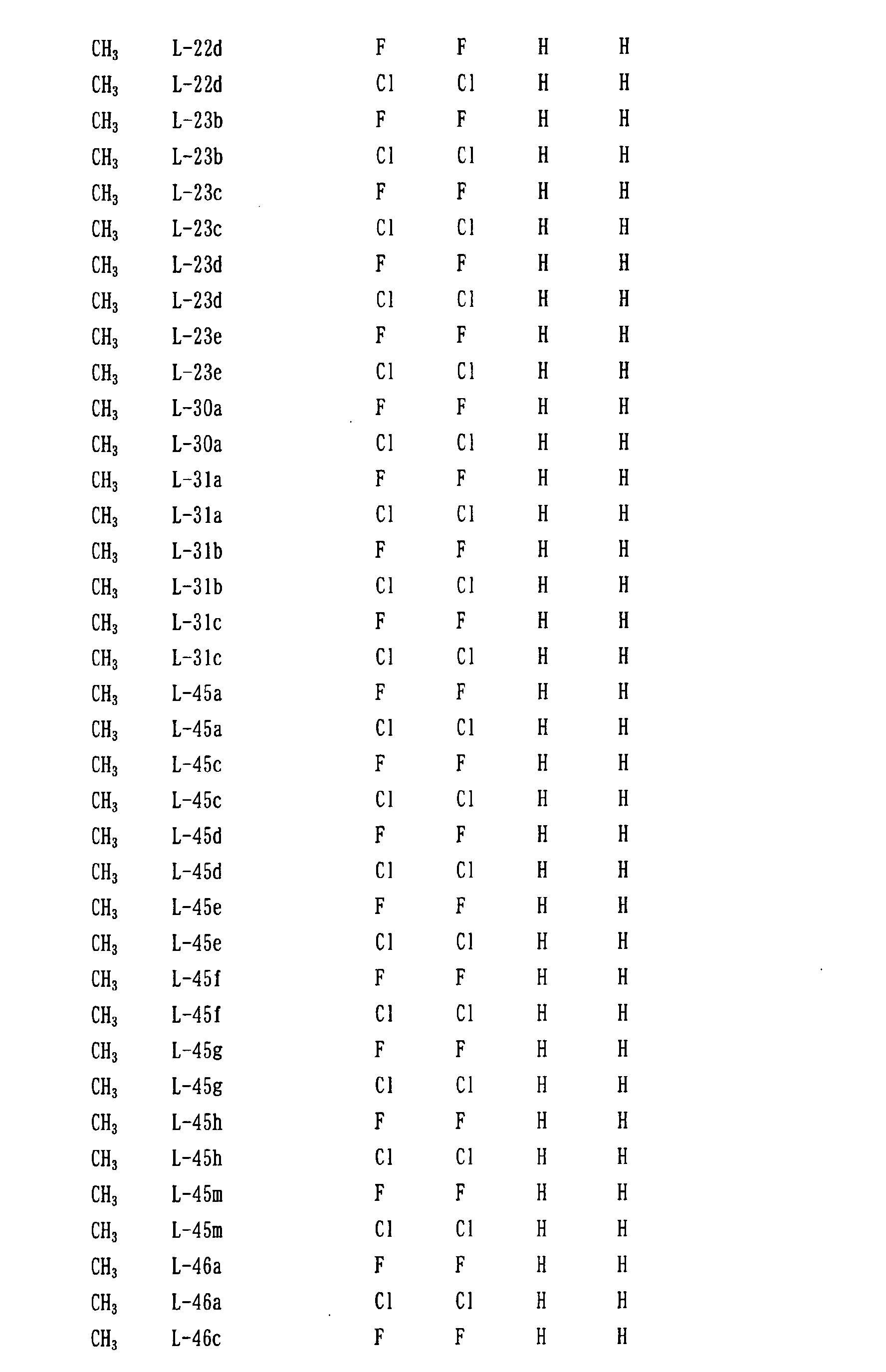

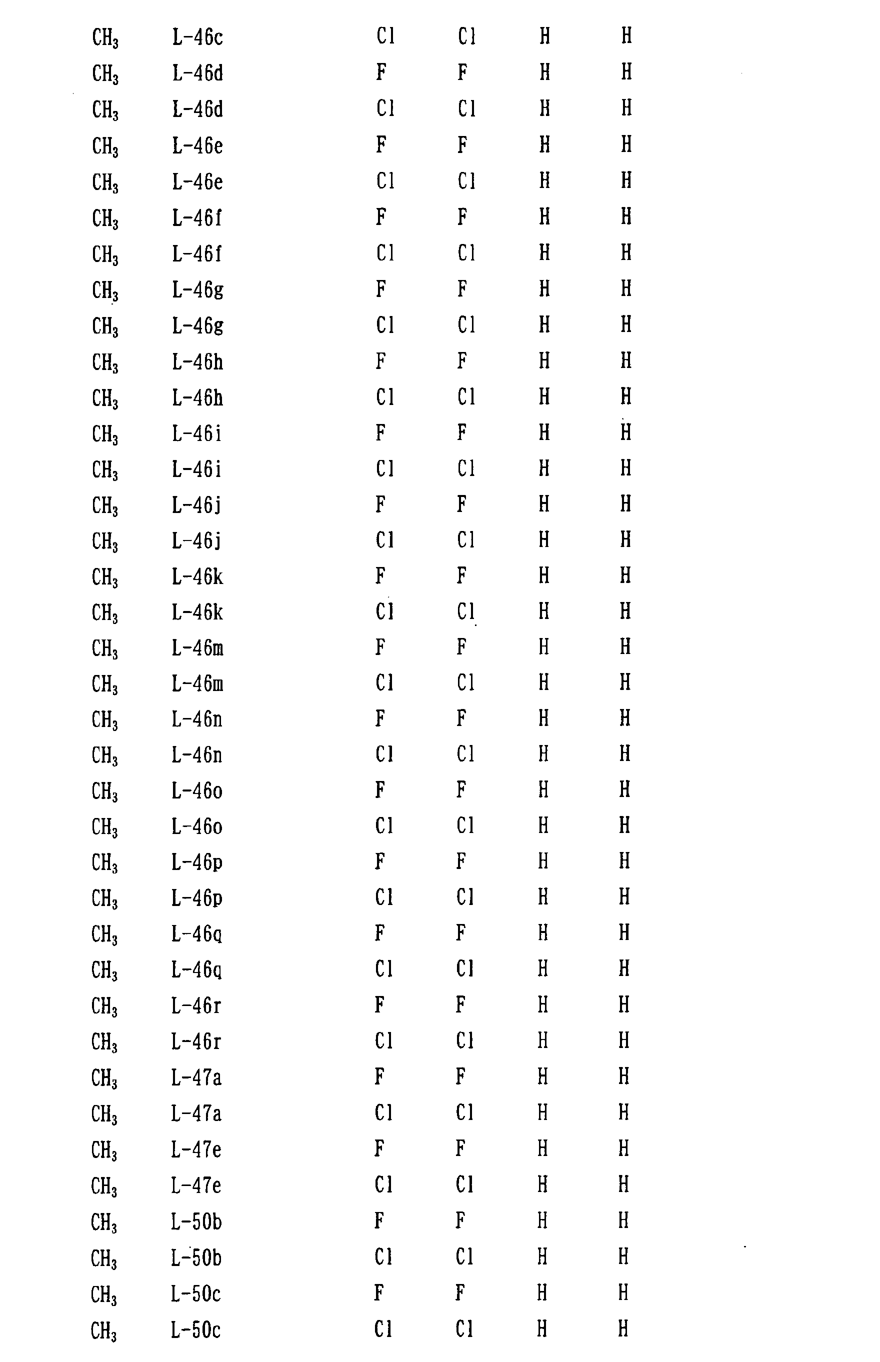

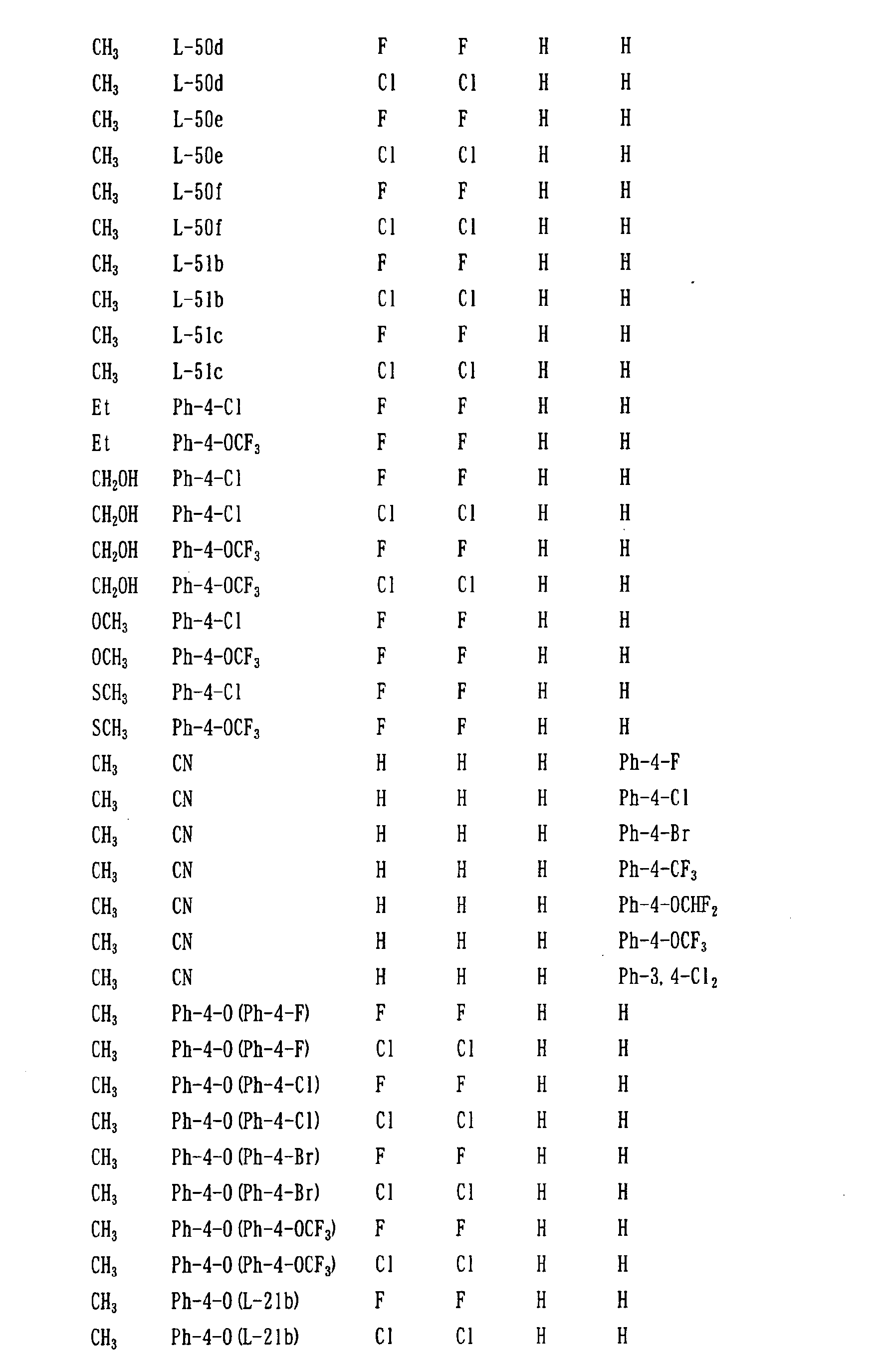

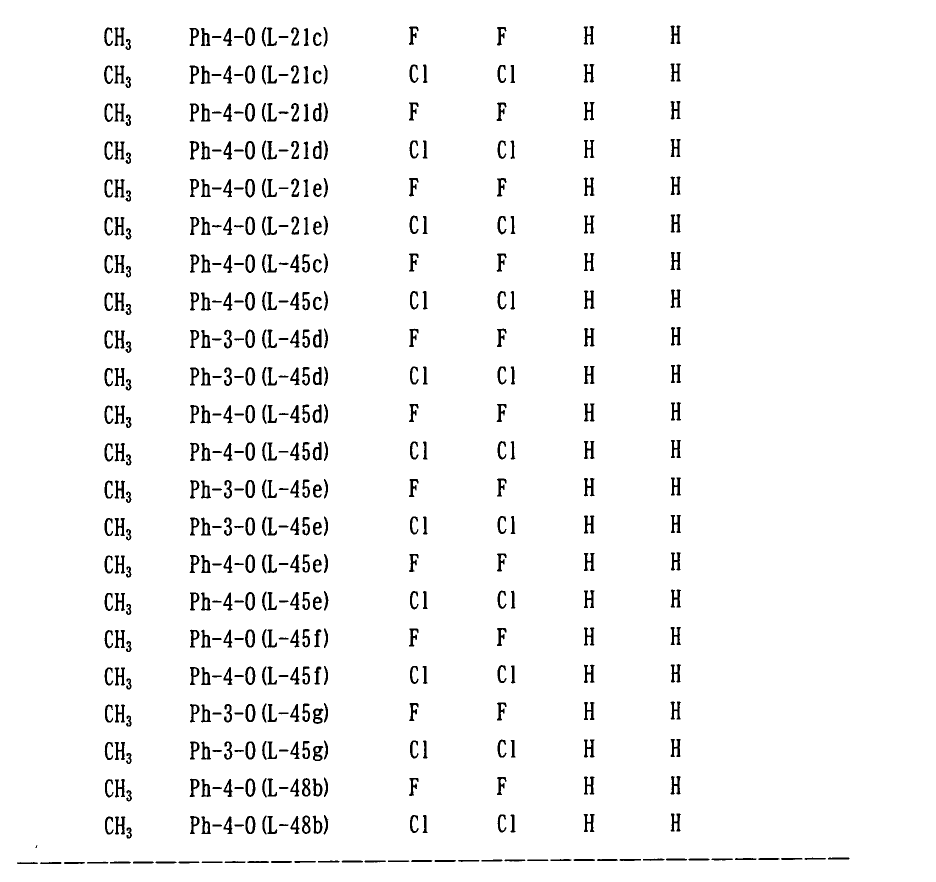

The compound of the present invention can prevent from and exterminate either of harmful insects with a low concentration such as the so-called agricultural harmful insects which injure agricultural and horticultural crops and trees, the so-called harmful insects against domestic animals which are parasitic on domestic animals and domestic dowls, the so-called hygiene harmful insects which provide various bad influences on a human life environment such as houses, etc., the so-called harmful insects against stored grains which injure grains stored in a storehouse, and mites, nematodes, mollusks and crustaceans which generate in the same situation and injure.

In the insects, mites, nematodes, molluscs and crustaceans which can be prevented and exterminated by using the compound of the present invention, there may be specifically mentioned, for example,
Lepidoptera harmful insects such as diamondback moth (Plutella xylostella), black cutworm (Agrotis ipsilon), Turnip moth (Agrotis segetum), corn earworm (Helicoverpa armigera), Oriental tobacco budworm (Helicoverpa assulta), cotton bollworm (Helicoverpa zea), tabacco budworm (Heliothis virescens), cabbage armyworm (Mamestra brassicae), green rice caterpillar (Naranga aenescens), beet semilooper (Autographa nigrisigna), rice armyworm (Mythimna separata), beet armyworm (Spodoptera exigua), common cutworm (Spodoptera litura), cotton leafworm (Spodoptera littoralis), fall armyworm (Spodoptera frugiperda), southern armyworm (Spodoptera eridania), tomato hornworm (Manduca quinquemaculata), tabacco hornworm (Manduca sexta), grapeberry moth (Endopiza viteana), apple lyonetid (Lyonetia prunifoliella malinella), tea leafroller (Caloptilia theivora), apple leafminer (Phyllonorycter ringoniella), citrus leafminer (Phyllocnistis citrella), pink bollworm (Pectinophora gossypiella), peach fruit moth (Carposina sasakii), summer fruit tortrix (Adoxophyes orana faciata), smaller tea tortrix (Adoxophyes honmai), apple tortrix (Archips fuscocupreanus), oriental tea tortrix (Homona magnamina), codling moth (Cydia pomonella), Oriental fruit moth (Grapholita molesta), rice stem borer (Chilo suppressalis), rice leafroller (Cnaphalocrocis medinalis), cabbage webworm (Hellula undalis), oriental corn borer (Ostrinia furnacalis), European corn borer (Ostrinia nubilalis), soybean looper (Pseudoplusia includens), cabbage looper (Trichoplusia ni), fall wedworm (Hyphantria cunea), common white (Pieris rapae crucivora), rice skipper (Parnara guttata), tea tussock moth (Euproctis pseudoconspersa), etc.,
Coleoptera harmful insects such as cupreous chafer beetle (Anomala cuprea), soybean beetle (Anomala rufocuprea), japanese beetle (Popillia japonica), colorado potato beetle (Lepinotarsa decemlineata), mexican bean beetle (Epilachna varivestis), sugarcane wireworm (Melanotus tamsuyensis), cigarette beetle (Lasioderma serricorne), Himehirata Nitidulidae (Epuraea domina), twentyeight-spotted ladybird (Epilachna vigintioctopunctata), yellow mealworm (Tenebrio molitor), red flour beetle (Tribolium castaneum), whitespotted longicorn beetle (Anoplophora malasiaca), japanese pine sawyer (Monochamus alternatus), azuki bean weevil (Callosobruchus chinensis), cucurbit leaf beetle (Aulacophora femoralis), rice leaf beetle (Oulema oryzae), striped flea beetle (Phyllotreta striolata), sweetpotato weevil (Cylas formicarius), boll weevil (Anthonomus grandis), rice curculio (Echinocnemus squameus), alfalfa weevil (Hypera postica), rice water weevil (Lissohoptrus oryzophilus), maize weevil (Sitophilus zeamais), hunting billbug (Sphenophorus venatus vestitus), granary weevil (Sitophilus granarius), southern corn rootworm (Diabrotica undecimpunctata), western corn rootworm (Diabrotica virgifera), northern corn rootworm (Diabrotica barberi), rove beetle (Paederus fuscipes), etc.,
Hemiptera harmful insects such as cabbage bug (Eurydema rugosum), whitespotted bug (Eysarcoris ventralis), brown marmorated stink bug (Halyomorpha halys), southern green stink bug (Nezara viridula), rice bug (Leptocorisa chinensis), bean bug (Riptortus clavatus), small wing gourd bug (Togo hemipterus), tarnished plant bug (Lygus lineolaris), cotton fleahopper (Pseudatomoscelis seriatus), azalea lace bug (Stephanitis pyrioides), grape leafhopper (Erythroneura spp.), tea green leafhopper (Empoasca onukii), potato leafhopper (Empoasca fabae), green rice leafhopper (Nephotettix cinctinceps), small brown planthopper (Laodelphax striatella), brown rice planthopper (Nilaparvata lugens), whitebacked rice planthopper (Sogatella furcifera), Asiatic citrus psylla (Diaphorina citri), pear psylla (Psylla pyrisuga), silverleaf whitefly (Bemisia argentifolii), sweetpotato whitefly (Bemisia tabaci), citrus whitefly (Dialeurodes citri), greenhouse whitefly (Trialeurodes vaporariorum), cotton aphid (Aphis gossypii), spiraea aphid (Aphis spiraecola), green peach aphid (Myzus persicae), giant margarodid mealybug (Drosicha corpulenta), cottony cushion scale (Icerya purchasi), citrus mealybug (Planococcus citri), comstock mealybug (Pseudococcus comstocki), red wax scale (Ceroplastes rubens), arrowhead scale (Unaspis yanonensis), bed bug (Cimex lectularius), etc.,
Thysanoptera harmful insects such as western flower thrips (Frankliniella occidentalis), flower thrips (Frankliniella intonsa), yellow tea thrips (Scirtothrips dorsalis), melon thrips (Thrips palmi), onion thrips (Thrips tabaci), etc.,
Diptera harmful insects such as melon fly (Bactrocera cucurbitae), oriental fruit fly (Bactrocera dorsalis), mediterranean fruit fly (Ceratitis capitata), rice leaf miner (Hydrellia griseola), garden pea leaf miner (Chromatomyia horticola), bryony leaf miner (Liriomyza bryoniae), serpentine leaf miner (Liriomyza trifolii), seedcorn maggot (Delia platura), apple maggot (Rhagoletis pomonella), hessian fly (Mayetiola destructor), house fly (Musca domestica), stable fly (Stomoxys calcitrans), sheep ked (Melophagus ovinus), northern cattle grub (Hypoderma bovis), common cattle grub (Hypoderma lineatum), sheep nasal bot fly (Oestrus ovis), tsetse fly (Glossina palpalis, Glossina morsitans), yellow-leg giant gnat (Prosimulium yezoensis), cattle hoursefly (Tabanus trigonus), filter fly or bathroom_fly (Telmatoscopus albipunctatus), tokunaganuka mosquito (Leptoconops nipponensis), brown house mosquito (Culex pipiens pallens), yellow fever mosquito (Aedes aegypti), asian tiger mosquito (Aedes albopictus), chinese spotted mosquito (Anopheles hyracanus sinesis), etc.,
Hymenoptera harmful insects such as chestnut sawfly (Apethymus kuri), turnip sawfly (Athalia rosae), rose argid sawfly (Arge pagana), european pine sawfly (Neodiprion sertifer), chestnut gall wasp (Dryocosmus kuriphilus), american army ants (Eciton burchelli, Eciton schmitti), red carpenter ant (Camponotus japonicus), giant asian hornet (Vespa mandarina), bulldog ants (Myrmecia spp.), fire ants (Solenopsis spp.), pharaoh ant (Monomorium pharaonis), etc.,
Blattaria harmful insects such as smokybrown cockroach (Periplaneta fuliginosa), Japanese cockroach (Periplaneta japonica), German cockroach (Blattella germanica), etc.,
Orthoptera harmful insects such as emma field cricket (Teleogryllus emma), mole cricket (Gryllotalpa orientalis), migratory locust (Locusta migratoria), rice grasshopper (Oxya yezoensis), desert locust (Schistocerca gregaria), etc.,
Isoptera harmful insects such as formosan subterranean termite (Coptotermes formosanus), Japanese termite (Reticulitermes speratus), Taiwanese termite (Odontotermes formosanus), etc.,
Isoptera harmful insects such as cat flea (Ctenocephalides felis), human flea (Pulex irritans), Oriental rat flea (Xenopsylla cheopis), etc.,
Mallophage harmful insects such as chicken body louse (Menacanthus stramineus), cattlebiting louse (Bovicola bovis), etc.,
Anoplura harmful insects such as shortnosed cattle louse (Haematopinus eurysternus), pig louse (Haematopinus suis), longnosed cattle louse (Linognathus vituli), tubercle-bearing louse (Solenopotes capillatus), etc.,
Tarsonemidae such as cyclamen mite (Phytonemus pallidus), broad mite (Polyphagotarsonemus latus), etc.,
Tetranychidae such as citrus red mite (Panonychus citri), European red mite (Panonychus ulmi), kanzawa spider mite (Tetranychus kanzawai), two-spotted spider mite (Tetranychus urticae), etc.,
Eriophyidae such as pink tea rust mite (Acaphylla theavagrans), wheat curl mite (Aceria tulipae), tomato russet mite (Aculops lycopersici), pink citrus rust mite (Aculops pelekassi), apple rust mite (Aculus schlechtendali), Japanese pear rust mite (Eriophyes chibaensis), citrus rust mite (Phyllocoptruta oleivora), etc.,
Acaridae such as bulb mite (Rhizoglyphus robini), mould mite (Tyrophagus putrescentiae), etc.,
Varroidae such as honeybee varroa mite (Varroa jacobsoni), etc.,
Metastigmata such as southern cattle tick (Boophilus microplus), bush tick (Haemaphysalis longicornis), etc.,
scab mites such as sheep scab mite (Psoroptes ovis), etc.,
Sarcoptes scabiei such as scabies mite (Sarcoptes scabiei), etc.,
crustaceans such as pill bug (Armadillidium vulgare), etc.,
nematodes such as coffee root-lesion nematode (Pratylenchus coffeae), cobb's root-lesion nematode (Pratylenchus penetrans), walnut root-lesion nematode (Pratylenchus vulnus), potato cyst nematode (Globodera rostochiensis), soybean cyst nematode (Heterodera glycines), northern root-knot nematode (Meloidogyne hapla), southern root-knot nematode (Meloidogyne incognita), pinewood nematode (Bursaphelenchus lignicolus), etc.,
mollusks such as channeled apple snail (Pomacea canaliculata), slug (Meghimatium bilineatum), Korean round snail (Acusta despecta sieboldiana), Japanese land snail (Euhadra peliomphala), etc., but the present invention is not limited by these alone.

Moreover, the compound of the present invention is also effective to harmful insects which are improved in resistance against already presenting insecticides such as organophosphurus type compounds, carbamate type compounds or pyrethroid type compounds, etc.

That is, the compound of the present invention can be effectively present and exterminate harmful insects of Orthoptera, Order Thysanoptera, Hemiptera, Lepidoptera, Coleoptera, Hymenoptera, Thysanoptera, Blattaria, Isoptera, Isoptera, mites and lice, and nematodes with a low concentration. On the other hand, the compound of the present invention has extremely useful characteristics that it causes substantially no bad effect against mammals, foshes, crustaceans and useful insects.

For the purpose of using the compound of the present invention, by mixing with a suitable solid carrier or a liquid carrier, and further, if desired, by adding a surfactant, a penetarnt, a spreading agent, a thicknening agent, an antifreezing agent, a binder, a non-caking agent, a discipient, an antifoaming agent, an antiseptic agent and a decomposition preventing agent, etc., it can be practically applied in an optional formulations such as soluble concentrate, emulsifiable concentrate, wettable powder, water soluble powder, water dispersible granule, water soluble granule, suspension concentrate, concentrated emulsion, suspoemulsion, microemulsion, dustable powder, granule, tablet and emulsifiable gel, etc. Also, in the viewpoints of labor saving and improvement in safety, the above-mentioned formulations in an optional form are encapsulated in a water-soluble container such as a bag of a water-soluble capsule and water-soluble film, etc. and applied practically.

As the solid carrier, there may be mentioned, for example, natural minerals such as quartz, calcite, sepiolite, dolomite, chalk, kaolinite, pyrophyylite, sericite, halocite, metahalocite, Kibushi clay, Gaerome clay, kaolin, zeeklite, allophane, white sand (loam), mica, talc, bentonite, active china clay, acidic china clay, pumice, attapulgite, zeolite and diatomaceous earth, etc., calcined products of natural minerals such as calcined clay, perlite, white sand balloon (loam balloon), vermiculite, attapulgus clay and calcined diatomaceous earth, etc., inorganic salts such as magnesium carbonate, calcium carbonate, sodium carbonate, sodium hydrogen carbonate , ammonium sulfate, sodium sulfate, magnesium sulfate, diammonium hydrogen phosphate, ammonium dihydrogen phosphate and potassium chloride, etc., saccharides such as glucose, fructose, sucrose and lactose, etc., polysaccharides such as starch, powder cellulose and dextrin, etc., organic materials such as urea, urea derivatives, benzoic acid and a salt of benzoic acid, etc., plants such as wood powder, cork powder, corn head stem, walnut shell and tobacco stem, etc., fly ash, white carbon (e.g., hydrated synthetic silica, anhydrous synthetic silica and hydrated synthetic silicate, etc.) and feritilizers, etc.

As the liquid carrier, there may be mentioned, for example, aromatic hydrocarbons such as xylene, alkyl(C₉ or C₁₀, etc.)benzene, phenylxylylethane and alkyl(C₁ or C₃, etc.)naphthalene, etc., aliphatic hydrocarbons such as machine oil, normal paraffin, isoparaffin and naphthene, etc., a mixture of aromatic hydrocarbons and aliphatic hydrocarbons such as kerosene, etc., alcohols such as ethanol, isopropanol, cyclohexanol, phenoxyethanol and benzylalcohol, etc., polyvalent alcohols such as ethylene glycol, propyleneglycol, diethylene glycol, hexylene glycol, polyethylene glycol and polypropyleneglycol, etc., ethers such as propyl cellosolve, butyl cellosolve, phenyl cellosolve, propyleneglycol monomethyl ether, propyleneglycol monoethyl ether, propyleneglycol monopropyl ether, propyleneglycol monobutyl ether and propyleneglycol monophenyl ether, etc., ketones such as acetophenone, cyclohexanone and Y-butyrolactone, etc., esters such as aliphatic acid methyl ester, dialkyl succinate, dialkyl glutamate, dialkyl adipate and dialkyl phthalate, etc., acid amides such as N-alkyl(C₁, C₈ or C₁₂, etc.)pyrrolidone, etc., oil and fats such as soybean oil, linseed oil, rapeseed oil, coconut oil, cottonseed oil and caster oil, etc., dimethylsulfoxide and water.

These solid and liquid carriers may be used alone or in combination of two or more kinds in combination.

As the surfactant, there may be mentioned, for example, nonionic surfactants such as polyoxyethylene alkyl ether, polyoxyethylene alkyl (mono- or di-)phenyl ether, polyoxyethylene (mono-, di- or tri-)styrylphenyl ether, polyoxyethylene polyoxypropylene block copolymer, polyoxyethylene fatty acid (mono- or di-)ester, sorbitan fatty acid ester, polyoxyethylene sorbitan fatty acid ester, caster oil-ethylene oxide adducts, acetylene glycol, acetylene alcohol, ethylene oxide adducts of acetylene glycol, ethylene oxide adducts of acetylene alcohol and alkyl glycoside, etc., anionic surfactants such as alkyl sulfate, alkylbenzenesulfonate, lignine sulfonate, alkylsulfosuccinate, naphthalene sulfonate, alkyl naphthalene sulfonate, formalin condensate salt of naphthalene sulfonic acid, formalin condensate salt of alkylnaphthalene sulfonic acid, polyoxyethylene alkyl ether sulfate or phosphate, polyoxyethylene (mono- or di-)alkylphenyl ether sulfate or phosphate, polyoxyethylene (mono-, di- or tri-)styrylphenyl ether sulfate or phosphate, polycarboxylate (e.g., polyacryaltes, polymaleates and copolymer materials of maleic acid and olefin, etc.) and polystyrenesulfonate, etc., cationic surfactants such as alkylamine salt and alkyl quaternary ammonium salt, etc., amphoteric surfactants such as amino acid type and betaine type, etc., silicone type surfactants and fluorine type surfactants.

A content of these surfactants is not specifically limited, and it is desirably in the range of 0.05 to 20 parts by weight in general based on 100 parts by weight of the preparation according to the present invention. Also, these surfactants may be used alone or in combination of two or more kinds in combination.

A dose of the compound of the present invention to be applied may vary depending on the place to be applied, time to be applied, method to be applied, crops to cultivate, etc., and in general, it is suitable in an amount of about 0.005 to 50 kg or so per a hectare (ha) as an amount of the effective ingredient.

Next, Formulation examples of the preparation when the compound of the present invention is used are shown below. Provided that Formulation examples of the present invention are not limited by these. Incidentally, in the following Formulation examples, all "part(s)" mean part(s) by weight.

| [Wettable powder] | |
|---|---|
| Compound of the present invention | 0.1 to 80 parts |
| Solid carrier | 5 to 98.9 parts |
| Surfactant | 1 to 10 parts |
| Others | 0 to 5 parts |

As other components, there may be mentioned, for example, a non-caking agent, a decomposition preventing agent, and the like.

| [Emulsifiable concentrate] | |
|---|---|
| Compound of the present invention | 0.1 to 30 parts |
| Liquid carrier | 45 to 95 parts |
| Surfactant | 4.9 to 15 parts |
| Others | 0 to 10 parts |

As other components, there may be mentioned, for example, a spreading agent, a decomposition preventing agent, and the like.

| [Suspension concentrate] | |
|---|---|
| Compound of the present invention | 0.1 to 70 parts |
| Liquid carrier | 15 to 98.89 parts |
| Surfactant | 1 to 12 parts |
| Others | 0.01 to 30 parts |

As other components, there may be mentioned, for example, an antifreezing agent, a thicknening agent, and the like.

| [Water dispersible granule] | |
|---|---|
| Compound of the present invention | 0.1 to 90 parts |
| Solid carrier | 0 to 98.9 parts |
| Surfactant | 1 to 20 parts |
| Others | 0 to 10 parts |

As other components, there may be mentioned, for example, a binder, a decomposition preventing agent, and the like.

| [Soluble concentrate] | |
|---|---|
| Compound of the present invention | 0.01 to 70 parts |
| Liquid carrier | 20 to 99.99 parts |
| Others | 0 to 10 parts |

As other components, there may be mentioned, for example, an antifreezing agent, a spreading agent, and the like.

| [Granule] | |
|---|---|
| Compound of the present invention | 0.01 to 80 parts |
| Solid carrier | 10 to 99.99 parts |
| Others | 0 to 10 parts |

As other components, there may be mentioned, for example, a binder, a decomposition preventing agent, and the like.

| [Dustable powder] | |
|---|---|
| Compound of the present invention | 0.01 to 30 parts |
| Solid carrier | 65 to 99.99 parts |
| Others | 0 to 5 parts |

As other components, there may be mentioned, for example, a drift preventing agent, a decomposition preventing agent, and the like.

Next, Formulation examples using the compound of the present invention as an effective ingredient are described in more detail, but the present invention is not limited by these.

Incidentally, in the following Formulation examples, "part(s)" means part(s) by weight.

| [Formulation example 1] Wettable powder | |
|---|---|
| Present compound No. 2-124 | 20 parts |
| Pyrophyylite | 74 parts |
| Solpol 5039 | 4 parts |
| (a mixture of a nonionic surfactant and an anionic surfactant: available from TOHO Chemical Industry Co., LTD, Tradename) | |
| CARPREX #80D | 2 parts |
| (Synthetic hydrated silicic acid: available from Shionogi & Co., Ltd., Tradename) | |

The above materials are uniformly mixed and pulverized to make wettable powder.

| [Formulation example 2] Emulsion | |
|---|---|
| Present compound No. 2-124 | 5 parts |
| xylene | 75 parts |
| N-methylpyrrolidone | 15 parts |
| Solpol 2680 | 5 parts |
| (a mixture of a nonionic surfactant and an anionic surfactant: available from TOHO Chemical Industry Co., LTD, Tradename) | |

The above materials are uniformly mixed to make emulsifiable concentrate.

| [Formulation example 3] Suspension concentrate | |
|---|---|
| Present compound No. 2-124 | 25 parts |
| Agrisol S-710 | 10 parts |
| (a nonionic surfactant: available from KAO CORPORATION, Tradename) | |
| Lunox 1000C | 0.5 part |
| (an anionic surfactant: available from TOHO Chemical Industry Co., LTD, Tradename) | |
| Xanthan gum | 0.2 part |
| Water | 64.3 parts |

The above materials are uniformly mixed and pulverized, and then, wet pulverized to make suspension concentrate.

| [Formulation example 4] Water dispersible granule | |
|---|---|
| Present compound No. 2-124 | 75 parts |
| HITENOL NE-15 | 5 parts |
| (an anionic surfactant: available from DAI-ICHI KOGYO SEIYAKU CO., LTD., Tradename) | |
| VANILLEX N | 10 parts |
| (an anionic surfactant: available from Nippon Paper Chemicals Co., Ltd., Tradename) | |
| CARPREX #80D | 10 parts |
| (Synthetic hydrated silicic acid: available from Shionogi & Co., Ltd., Tradename) | |

The above materials are uniformly mixed and pulverized, and then, a small amount of water is added to the mixture and the resulting mixture is mixed under stirring, granulated by an extrusion granulator, and dried to make water dispersible granule.

| [Formulation example 5] Granule | |
|---|---|
| Present compound No. 2-124 | 5 parts |
| Bentonite | 50 parts |
| Talc | 45 parts |

The above materials are uniformly mixed and pulverized, and then, a small amount of water is added to the mixture and the resulting mixture is mixed under stirring, granulated by an extrusion granulator, and dried to make granule.

| [Formulation example 6] Dustable powder | |
|---|---|
| Present compound No. 2-124 | 3 parts |
| CARPREX #80D | 0.5 parts |
| (Synthetic hydrated silicic acid: available from Shionogi & Co., Ltd., Tradename) | |
| Caolinite | 95 parts |
| Diisopropyl phosphate | 1.5 parts |

The above materials are uniformly mixed and pulverized to make dustable powder.

For the purpose of use, the above-mentioned formulations are spread by diluting to 1 to 10000-folds with water, or directly spread without dilution.

Also, when the compound of the present invention is used as an agricultural chemicals, it may be mixed with other kinds of herbicides, various kinds of insecticides, acaricides, nematocides, fungicides, vegetable growth regulators, synergists, fertilizers, soil improvers, etc., and applied, at the time of preparing the formulation or at the time of spreading, if necessary.

In particular, by mixing with the other agricultural chemicals or plant hormones and applying the mixture, it can be expected that a cost is reduced due to reduction in a dose to be applied, enlargement in insecticidal spectrum or higher prevention and extinction effect of noxious organisms due to synergistic effect by mixing agricultural chemicals. At this time, it is possible to use the compound with a plural number of the conventionally known agricultural chemicals in combination simultaneously. As the kinds of the agricultural chemicals to be used in admixture with the compound of the present invention, there may be mentioned, for example, the compounds described in Farm Chemicals Handbook, 1999^{th} Edition and the like. Specific examples of the general names can be enumerated below, but the invention is not necessarily limited by these alone.

Fungicide: acibenzolar-S-methyl, acylaminoberizamide, ambam (amobam), ampropylfos (ampropyfos), anilazine, azaconazole, azoxystrobin, benalaxyl, benodanil, benomyl, benthiazole, benzamacril, binapacryl, biphenyl, bitertanol, bethoxazine, bordeaux mixture, blasticidin-S, bromoconazole, bupirimate, buthiobate, calcium polysulfide, captafol, captan, copper oxychloride, carpropamid, carbendazim, carboxin, CGA-279202 (Test name), chinomethionat, chlobenthiazone, chlorfenazol, chloroneb, chlorothalonil, chlozolinate, cufraneb, cymoxanil, cyproconazol, cyprodinil, cyprofuram, dazomet, debacarb, dichlorophen, diclobutrazol, dichlorfluanid, diclomedine, dicloran, diethofencarb, diclocymet, difenoconazole, diflumetorim, dimethirimol, dimethomorph, diniconazole, diniconazole-M, dinocap, diphenylamine, dipyrithione, ditalimfos, dithianon, dodemorph, dodine, drazoxolon, edifenphos, epoxiconazole, etaconazole, ethirimol, etridianole (etridiazole), famoxazone (famoxadone), fenarimol, febuconazole, fenamidone, fendazosulam, fenfuram, fenhexamid, fenpiclonil, fenpropidin, fenpropimorph, fentin, ferbam, ferimzone, fluazinam, fludioxonil, fluoroimide, fluquinconazole, flusilazole, flusulfamide, flutolanil, flutriafol, folpet, fosetyl-aluminium, fuberidazole, furalaxyl, furametpyr, guazatine, hexachlorobenzene, hexaconazole, hymexazol, imazalil, imibenconazole, iminoctadine, ipconazole, iprobenfos, iprodione, isoprothiolane, iprovalicarb, kasugamycin, kresoxim-methyl, mancopper, mancozeb, maneb, mepanipyrim, mepronil, metalaxyl, metconazole, methasulfocarb, metiram, metominostrobin, myclobutanil, MTF-753 (Test name), nabam, nickel bis(dimethyldithiocarbamate), nitrothal-isopropyl, nuarimol, NNF-9425 (Test name), octhilinone, ofurace, oxadixyl, oxycarboxin, oxpoconazole fumarate, pefurzoate, penconazole, pencycuron, phthalide, piperalin, polyoxins, potassium hydrogen carbonate, probenazole, prochloraz, procymidone, propamocarb hydrochloride, propiconazole, propineb, pyrazophos, pyrifenox, pyrimethanil, pyroquilon, quinomethionate, quinoxyfen, quintozene, RH7281 (Test name), sodium hydrogen carbonate, sodium hypochlorite, sulfur, spiroxamine, tebuconazole, tecnazene, tetraconazole, thiabendazole, thiadiazin/milneb, thifluzamide, thiophanate- methyl, thiram, tolclofos-methyl, tolyfluranid, triadimefon, triadimenol (toriadimenol), triazoxide, tricyclazole, tridemorph, triflumizole, triforine, triticonazole, validamycin, vinclozolin, zinc sulfate, zineb, ziram and shiitake mushroom hyphae extract, etc.

Bactericide: streptomycin, tecloftalam, oxytetracyclin and oxolinic acid, etc.

Nematocide: aldoxycarb, cadusafos, fosthiazate, fosthietan, oxamyl and fenamiphos, etc.

Acaricide: acequinocyl, amitraz, bifenazate, bromopropylate, chinomethionat, chlorobezilate, clofentezine, cyhexatine, dicofol, dienochlor, etoxazole, fenazaquin, fenbutatin oxide, fenpropathrin, fenproximate, halfenprox, hexythiazox, milbemectin, propargite, pyridaben, pyrimidifen and tebufenpyrad, etc.

Insecticide: abamectin, acephate, acetamipirid, aldicarb, allethrin, azinphos-methyl, bendiocarb, benfuracarb, bensultap, bifenthrin, buprofezin, butocarboxim, carbaryl, carbofuran, carbosulfan, cartap, chlorfenapyr, chlorpyrifos, chlorfenvinphos, chlorfluazuron, clothianidin, chromafenozide, chlorpyrifos-methyl, cycloprothrin, cyfluthrin, beta-cyfluthrin, cypermethrin, cyromazine, cyhalothrin, lambda-cyhalothrin, deltamethrin, diafenthiuron, diazinon, diacloden, diflubenzuron, dimethylvinphos, diofenolan, disulfoton, dimethoate, emamectin-benzoate, EPN, esfenvalerate, ethiofencarb, ethiprole, etofenprox, etrimfos, fenitrothion, fenobucarb, fenoxycarb, fenpropathrin, fenvalerate, fipronil, fluacrypyrim, flucythrinate, flufenoxuron, flufenprox, tau-fluvalinate, fonophos, formetanate, formothion, furathiocarb, halofenozide, hexaflumuron, hydramethylnon, imidacloprid, isofenphos, indoxacarb, isoprocarb, isoxathion, lufenuron, malathion, metaldehyde, methamidophos, methidathion, methacrifos, metalcarb, methomyl, methoprene, methoxychlor, methoxyfenozide, monocrotophos, muscalure, nidinotefuran, nitenpyram, omethoate, oxydemeton-methyl, oxamyl, parathion, parathion-methyl, permethrin, phenthoate, phoxim, phorate, phosalone, phosmet, phosphamidon, pirimicarb, pirimiphos-methyl, profenofos, protrifenbute, pymetrozine, pyraclofos, pyriproxyfen, rotenone, sulprofos, silafluofen, spinosad, sulfotep, tebfenozide, teflubenzuron, tefluthorin, terbufos, tetrachlorvinphos, thiacloprid, thiocyclam, thiodicarb, thiamethoxam, thiofanox, thiometon, tolfenpyrad, tralomethrin, trichlorfon, triazuron, triflumuron and vamidothion, etc.

### Examples

In the following, the present invention will be explained in more detail by specifically referring to Synthetic examples and Test Examples of the compound of the present invention as Examples, but the present invention is not limited by these.

### [Synthetic examples]

### Synthetic example 1

N¹-[4-[3-(4-fluorophenyl)-5-trifluoromethyl-4,5-dihydroisoxazol-5-yl]-2-methylphenyl]-3-nitro-N²-isopropylphthalic diamide (Present compound No. 2-167).
Step 1; Preparation of t-butyl 4-iodo-2-methylcarbanilate.

To 30 ml of a toluene solution containing 10.0 g of 4-iodo-2-methylaniline was added 14.0 g of di-t-butyl dicarbonate, and the mixture was stirred under reflux for 2 hours. After completion of the reaction, 30 ml of water was added to the mixture and the resulting mixture was refluxed for 15 minutes, cooled to room temperature by allowing to stand, and extracted by 100 ml of diethyl ether. The organic layer was washed with water, dried over anhydrous magnesium sulfate, the solvent was removed under reduced pressure, and the residual solid was washed with hexane to obtain 11.5 g of the objective material as white crystals.
Melting point 101.0 to 103.0°C
¹H NMR (CDCl₃, Me₄Si, 300MHz) δ 7.63 (d, J=8.4Hz, 1 H), 7.45-7.5 (m, 2H), 6.22 (bs, 1 H), 2.19 (s, 3H), 1.52 (s, 9H).
Step 2; Preparation of t-butyl 2-methyl-4-(1-trifluoromethylethenyl)carbanilate.

Under nitrogen atmosphere, to 9.5 g of zinc powder (Tetrahedron Letters [Tetrahedron Lett.] 1981, vol. 22, p. 649) dispersed in 80 ml of tetrahydrofuran and activated by silver acetate was added 2 ml of chlorotrimethylsilane, and the mixture was stirred at room temperature for 10 minutes, then, 16.2 g of 2-bromo-3,3,3-trifluoropropene and 22 ml of N,N,N',N'-tetramethylethylenediamine were added to the mixture, and the resulting mixture was continued to stirring at 60°C for 12 hours. After removing the precipitated insoluble materials by decantation, 10.0 g of t-butyl 4-iodo-2-methylcarbanilate and 1.0 g of tetrakistriphenylphosphine palladium were added to the reaction mixture, and the resulting mixture was stirred at 60°C for further 4 hours. After completion of the reaction, the solvent was removed under reduced pressure, 200 ml of water was added to the residue, and the mixture was extracted with diethyl ether (200 mlx2). After the organic layer was washed with water, dried over anhydrous magnesium sulfate, the solvent was removed under reduced pressure, and the residue was purified by silica gel column chromatography eluted by diethyl ether and alumina column chromatography eluted by diethyl ether to obtain 8.4 g of the objective material as pale yellowish crystals.
Melting point 86.0 to 88.0°C
¹H NMR (CDCl₃, Me₄Si, 300MHz) δ 7.89 (d, J=8.8Hz, 1 H), 7.29 (d, J=8.8Hz, 1 H), 7.24 (s, 1 H), 6.32 (bs, 1 H), 5.88 (s, 1 H), 5.70 (s, 1 H), 2.30 (s, 3H), 1.53 (s, 9H).
Step 3; Preparation of t-butyl 4-[3-(4-fluorophenyl)-5-trifluoromethyl-4,5-dihydroisoxazol-5-yl]-2-methylcarbanilate.

To 10 ml of a N,N-dimethylformamide solution containing 4.1 g of 4-fluorophenylaldoxime was added 4.0 g of N-chlorosuccinic imide, and the resulting mixture was stirred at room temperature for 90 minutes. Then, to the reaction mixture were added 3.0 g of t-butyl 2-methyl-4-(1-trifluoromethylethenyl)carbanilate and 3.0 g of triethylamine dissolved in 10 ml of N,N-dimethylformamide, and stirring of the mixture was continued at room temperature for further 50 minutes. After completion of the reaction, the reaction mixture was poured into 200 ml of water, extracted with ethyl acetate (100 mlx2), the organic layer was washed with water, washed with saturated brine and then dried over anhydrous magnesium sulfate in this order, and the solvent was removed under reduced pressure. The residual solid was purified by silica gel column chromatography eluting with ethyl acetate-hexane (8:1) to obtain 3.3 g of the objective material as white crystals.
Melting point 149.0 to 150.0°C
¹H NMR (CDCl₃, Me₄Si, 300MHz) δ 7.95 (d, J=8.7Hz, 1 H), 7.65-7.7 (m, 2H), 7.41 (s, 1H), 7.36 (d, J=8.7Hz, 1 H), 7.05-7.15 (m, 2H), 6.33 (s, 1 H), 4.02 (d, J=17.1 Hz, 1 H), 3.70 (d, J=17.1 Hz, 1 H), 2.28 (s, 3H), 1.52 (s, 9H).
Step 4; Preparation of 4-[3-(4-fluorophenyl)-5-trifluoromethyl-4,5-dihydroisoxazol-5-yl]-2-methylaniline.

To 2.6 g of t-butyl 4-[3-(4-fluorophenyl)-5-trifluoromethyl-4,5-dihydroisoxazol-5-yl]-2-methylcarbanilate was added dropwise 10 ml of trifluoroacetic acid under ice-cooling and stirring. At the same temperature, stirring was continued for 10 minutes, then, the solvent was removed under reduced pressure, 100 ml of diethyl ether was added to the residual oily substance, and the mixture was washed with 100 ml of an aqueous saturated sodium hydrogen carbonate solution. After collecting the organic layer by separation, the aqueous layer was extracted with diethyl ether (100 mlx2), and the organic layers were combined and washed with 100 ml of an aqueous saturated sodium hydrogen carbonate solution, dehydrated by saturated brine and dried over anhydrous magnesium sulfate in this order, and the solvent was removed under reduced pressure. The residual solid was purified by silica gel column chromatography eluting with ethyl acetate-hexane (1:3) to obtain 1.7 g of the objective material as white crystals.
Melting point 108.5 to 110.0°C
¹H NMR (CDCl₃, Me₄Si, 300MHz) δ 7.6-7.7 (m, 2H), 7.2-7.3 (m, 2H), 7.05-7.15 (m, 2H), 6.68 (d, J=8.4Hz, 1 H), 3.98 (d, J=17.1Hz, 1 H), 3.69 (d, J=17.1Hz, 1 H), 3.72 (bs, 2H), 2.18 (s, 3H).
Step 5; Preparation of N-[4-[3-(4-fluorophenyl)-5-trifluoromethyl-4,5-dihydroisoxazol-5-yl]-2-methylphenyl]-3-nitrophthalimide.

12 ml of an acetic acid solution containing 0.84 g of 3-nitrophthalic anhydride and 1.18 g of 4-[3-(4-fluorophenyl)-5-trifluoromethyl-4,5-dihydroisoxazol-5-yl]-2-methylaniline was stirred at 100°C for 5 hours. After completion of the reaction, the solvent was removed under reduced pressure, and the residual solid was washed with diethyl ether to obtain 1.54 g of the objective material as white crystals.
Melting point 221.0 to 222.0°C
¹H NMR (CDCl₃, Me₄Si, 300MHz) δ 8.15-8.25 (m, 2H), 8.00 (t, J=7.8Hz, 1 H), 7.65-7.7 (m, 3H), 7.5-7.65 (m, 1 H), 7.28 (d, J=8.4Hz, 1 H), 7.1-7.15 (m, 2H), 4.10 (d, J=17.1Hz, 1 H), 3.70 (d, J=17.1 Hz, 1 H), 2.27 (s, 3H).
Step 6; Preparation of N¹-[4-[3-(4-fluorophenyl)-5-trifluoromethyl-4,5-dihydroisoxazol-5-yl]-2-methylphenyl]-N²-isopropyl-3-nitrophthalic diamide.

To 20 ml of a 1,4-dioxane solution containing 1.47 g of N-[4-[3-(4-fluorophenyl)-5-trifluoromethyl-4,5-dihydroisoxazol-5-yl]-2-methylphenyl]-3-nitrophthalimide was added 1.5 g of isopropylamine, and the mixture was stirred at room temperature for 18 hours. After completion of the reaction, the solvent was removed under reduced pressure, and the residual solid was washed with diethyl ether to obtain 1.42 g of the objective material as beige color crystals.
Melting point 152.0 to 153.0°C
¹H NMR (CDCl₃, Me₄Si, 300MHz) δ 9.89 (s, 1 H), 8.41 (d, J=7.8Hz, 1 H), 8.17 (d, J=7.8Hz, 1 H), 8.00 (d, J=7.5Hz, 1 H), 7.75-7.85 (m, 2H), 7.75 (t, J=7.8Hz, 1 H), 7.61 (d, J=8.1 Hz, 1 H), 7.46 (s, 1 H), 7.43 (d, J=8.7Hz, 1 H), 7.3-7.35 (m, 2H), 4.34 (d, J=18.0Hz, 1 H), 4.15 (d, J=18.0Hz, 1 H), 3.85-3.95 (m, 1 H), 2.32 (s, 3H), 1.02 (d, J=6.6Hz, 6H).

### Synthetic example 2

3-Amino-N¹-[4-[3-(4-fluorophenyl)-5-trifluoromethyl-4,5-dihydroisoxazol-5-yl]-2-methylphenyl]-N²-isopropylphthalic diamide (Present compound No. 2-170).

To 25 ml of a methanol solution containing 1.30 g of N¹-[4-[3-(4-fluorophenyl)-5-trifluoromethyl-4,5-dihydroisoxazol-5-yl]-2-methylphenyl]-N²-isopropyl-3-nitrophthalic diamide (Present compound No. 2-167) was added 0.11 g of 5% active carbon-carried palladium, and the mixture was stirred at normal temperature and normal pressure under hydrogen atmosphere for 2.5 hours. After completion of the reaction, insoluble materials were filtered off by Celite, the solvent was removed under reduced pressure and the thus obtained solid was purified by silica gel column chromatography eluting with ethyl acetate to obtain 1.19 g of the objective material as pale yellowish crystals. Melting point 122.0 to 124.0°C
¹H NMR (CDCl₃, Me₄Si, 300MHz) δ 8.25 (d, J=8.1Hz, 1 H), 7.65-7.7 (m, 2H), 7.56 (s, 1 H), 7.4-7.45 (m, 2H), 7.15-7.3 (m, 1 H), 7.1-7.15 (m, 2H), 6.95 (d, J=7.2Hz, 1 H), 6.81 (d, J=7.2Hz, 1 H), 6.19 (d, J=8.1 Hz, 1 H), 4.62 (bs, 2H), 4.05-4.2 (m, 1 H), 4.05 (d, J=17.1Hz, 1 H), 3.73 (d, J=17.1Hz, 1 H), 2.30 (s, 3H), 1.06 (d, J=6.6Hz, 6H).

### Synthetic example 3

N¹-[4-[3-(4-fluorophenyl)-5-trifluoromethyl-4,5-dihydroisoxazol-5-yl]-2-methylphenyl]-3-iodo-N²-isopropylphthalic diamide (Present compound No. 2-016).

To 13 ml of an acetic acid solution containing 1.02 g of 3-amino-N¹-[4-[3-(4-fluorophenyl)-5-trifluoromethyl-4,5-dihydroisoxazol-5-yl]-2-methylphenyl]-N²-isopropylp hthalic diamide (Present compound No. 2-170) was added 0.55 g of conc. sulfuric acid under ice-cooling and stirring, then, 0.5 ml of aqueous solution containing 0.13 g of sodium nitrite was added dropwise to the mixture with a rate not to exceed the temperature of the reaction mixture over 15°C. After completion of the dropwise addition, stirring was continued at the same temperature for 20 minutes, then, the mixture was added dropwise to a mixture comprising a solution containing 0.37 g of potassium iodide dissolved in 40 ml of water and 40 ml of chloroform maintained at 40° C over 25 minutes, and further stirring was continued at 40° C for 50 minutes. After completion of the reaction, the organic layer was collected by separation, washed with 100 ml of an aqueous sodium thiosulfate solution, then, dehydrated with saturated brine and dried over anhydrous sodium sulfate in this order, and the solvent was removed under reduced pressure. The residual solid was purified by silica gel column chromatography eluting with ethyl acetate-hexane (1:3) to obtain 0.56 g of the objective material as white solid.
Melting point 128.0 to 130.0°C
¹H NMR (CDCl₃, Me₄Si, 300MHz) δ 8.46 (s, 1H), 8.00 (d, J=8.4Hz, 1 H), 7.94 (dd, J=8.1, 1.2Hz, 1H), 7.73 (d, J=7.2Hz, 1 H), 7.65-7.7 (m, 2H), 7.45 (s, 1 H), 7.40 (d, J=8.4Hz, 1 H), 7.05-7.2 (m, 3H), 6.04 (d, J=8.1 Hz, 1 H), 4.1-4.3 (m, 1 H), 4.05 (d, J=17.1 Hz, 1 H), 3.73 (d, J=17.1 Hz, 1 H), 2.35 (s, 3H), 1.16 (d, J=6.6Hz, 6H).

### Synthetic example 4

3-lodo-N²-isopropyl-N¹-[2-methyl-4-(3-methyl-5-trifluoromethyl-4,5-dihydroisoxazol-5-yl)phenyl]phthalic diamide (Present compound No. 1-006) and 6-iodo-N²-isopropyl-N¹-[2-methyl-4-(3-methyl-5-trifluoromethyl-4,5-dihydroisoxazol-5-yl)-phenyl]phthalic diamide (Present compound No. 1-007).
Step 1; Preparation of t-butyl 2-methyl-4-(3-methyl-5-trifluoromethyl-4,5-dihydroisoxazol-5-yl)carbanilate.

To 30 ml of N,N-dimethylformamide solution containing 0.5 g of acetoaldoxime was added 1.1 g of N-chlorosuccinic imide, and the mixture was stirred at room temperature for 30 minutes. Then, to the reaction mixture were added 1.0 g of t-butyl 2-methyl-4-(1-trifluoromethylethenyl)carbanilate synthesized in Step 1 to Step 2 of Synthetic example 1 and 0.8 g of triethylamine, and stirring was continued at room temperature for further 5 hours. After completion of the reaction, the reaction mixture was poured into 200 ml of water, extracted with diethyl ether (100 mlx2), the organic layer was washed with water, dried over anhydrous magnesium sulfate, and the solvent was removed under reduced pressure. The residual solid was purified by silica gel column chromatography eluting with diethyl ether and alumina column chromatography eluting with diethyl ether to obtain 0.9 g of the objective material as pale yellowish crystals.
Step 2; Preparation of 2-methyl-4-(3-methyl-5-trifluoromethyl-4,5-dihydroisoxazol-5-yl)-aniline.

To 0.9 g of t-butyl 2-methyl-4-(3-methyl-5-trifluoromethyl-4,5-dihydroisoxazol-5-yl)carbanilate was added dropwise 5 ml of trifluoroacetic acid under ice-cooling and stirring. After stirring was continued at room temperature for 10 minutes, the solvent was removed under reduced pressure to obtain 0.8 g of the crude objective material as brownish oily substance. This product was used in the next step as such without purification.
Step 3; Preparation of 3-iodo-N²-isopropyl-N¹-[2-methyl-4-(3-methyl-5-trifluoromethyl-4,5-dihydroisoxazol-5-yl)phenyl]phthalic diamide and 6-iodo-N²-isopropyl-N¹-[2-methyl-4-(3-methyl-5-trifluoromethyl-4,5-dihydroisoxazol-5-yl)phenyl]phthalic diamide.

To 10 ml of a toluene solution containing 1.0 g of 3-iodo-N-isopropylphthalamidic acid was added dropwise 1.0 g of trifluoroacetic anhydride at room temperature under stirring. The mixture was stirred at the same temperature for 30 minutes, then, the solvent was removed under reduced pressure, and the residual yellowish oily substance was dissolved in 20 ml of tetrahydrofuran, 0.8 g of crude 2-methyl-4-(3-methyl-5-trifluoromethyl-4,5-dihydroisoxazol-5-yl)aniline was added to the solution, and stirring was continued at room temperature for 17 hours. After completion of the reaction, the solvent was removed under reduced pressure, and the residual solid was purified by silica gel column chromatography eluting with ethyl acetate-chloroform (1:4) to obtain 0.45 g of 3-iodo-N²-isopropyl-N¹-[2-methyl-4-(3-methyl-5-trifluoromethyl-4,5-dihydroisoxazol-5-yl)phenyl]phthalic diamide as white crystals and 0.35 g of 6-iodo-N²-isopropyl-N¹-[2-methyl- 4-(3-methyl-5-trifluoromethyl-4,5-dihydroisoxazol-5-yl)-phenyl]phthalic diamide as white crystals, respectively.
3-lodo-N²-isopropyl-N'-[2-methyl-4-(3-methyl-5-trifluoromethyl-4,5-dihydroisoxazol-5-yl) phenyl]phthalic diamide;
Melting point 236.0 to 238.0°C
¹H NMR (CDCl₃, Me₄Si, 300MHz) δ 8.39 (bs, 1 H), 8.10 (d, J=8.5Hz, 1 H), 7.96 (d, J=8.0Hz, 1 H), 7.79 (d, J=8.5Hz, 1 H), 7.1-7.4 (m, 3H), 5.87 (d, J=8.0Hz, 1 H), 4.1-4.3 (m, 1H), 3.64 (d, J=17.6Hz, 1 H), 3.32 (d, J=17.6Hz, 1 H), 2.34 (s, 3H), 2.02 (s, 3H), 1.17 (d, J=6.3Hz, 6H).
6-Iodo-N²-isopropyl-N¹-[2-methyl-4-(3-methyl-5-trifluoromethyl-4,5-dihydroisoxazol-5-yl) phenyl]phthalic diamide;
Melting point 186.0 to 188.0°C
¹H NMR (CDCl₃, Me₄Si, 300MHz) δ 7.98 (bs, 1 H), 7.92 (d, J=8.3Hz, 1 H), 7.79 (d, J=7.5Hz, 1 H), 6.9-7.4 (m, 4H), 6.52 (d, J=6.0Hz, 1 H), 3.9-4.2 (m, 1 H), 3.58 (d, J=17.6Hz, 1 H), 3.26 (d, J=17.6Hz, 1 H), 2.27 (s, 3H), 1.95 (s, 3H), 1.03 (d, J=6.6Hz, 6H).

### Synthetic example 5

N¹-[4-[3-(4-chlorophenyl)-5-trifluoromethyl-4,5-dihydroisoxazol-5-yl]-2-methylphenyl]-3-iodo-N²-(1-methyl-2-methylthioethyl)phthalic diamide (Present compound No. 2-095).
Step 1; Preparation of 4-chlorophenylhydroximic acid chloride.

To 8 ml of a 1,4-dioxane solution containing 0.46 g of 4-chlorophenylaldoxime was added 4 ml of conc. hydrochloric acid, and 3.0 g of a 8% sodium hypochlorite aqueous solution was added dropwise to the above mixture over 10 minutes under ice-cooling and stirring. After completion of the dropwise addition, stirring was continued at the same temperature for 10 minutes, then, 30 ml of water was added to the mixture, and the resulting mixture was further stirred for 30 minutes. After completion of the reaction, the formed solid was collected by filtration, washed with water (30 mlx2), and dried under reduced pressure to obtain 0.4 g of the objective material as white crystals.
Melting point 86.0 to 87.0°C
¹H NMR (CDCl₃, Me₄Si, 300MHz) δ 8.00 (s, 1 H), 7.75-7.85 (m, 2H), 7.35-7.40 (m, 2H). Step 2; Preparation of t-butyl 4-[3-(4-chlorophenyl)-5-trifluoromethyl-4,5-dihydroisoxazol-5-yl]-2-methylcarbanilate.

To 25 ml of a N,N-dimethylformamide solution containing 3.0 g of t-butyl 2-methyl-4-(1-trifluoromethylethenyl)carbanilate synthesized in Step 1 to Step 2 of Synthetic example 1 was added 2.33 g of 4-chlorophenylhydroximic acid chloride, and 4.2 ml of triethylamine was added dropwise over 10 minutes under ice-cooling and stirring. After stirring was continued at room temperature for 5 hours, the reaction mixture was poured into 100 ml of water, extracted with ethyl acetate (100 mlx2), and the organic layer was washed with water, dried over anhydrous magnesium sulfate, and the solvent was removed under reduced pressure. The residual solid was purified by silica gel column chromatography eluting with ethyl acetate-hexane (1:1) to obtain 4.2 g of the objective material as white crystals.
¹H NMR (CDCl₃, Me₄Si, 300MHz) δ 7.95 (s, 1 H), 7.5-7.6 (m, 2H), 7.3-7.4 (m, 4H), 6.33 (bs, 1 H), 3.99 (d, J=16.8Hz, 1 H), 3.68 (d, J=17.4Hz, 1 H), 2.28 (s, 3H), 1.52 (s, 9H). Step 3; Preparation of 4-[3-(4-chlorophenyl)-5-trifluoromethyl-4,5-dihydroisoxazol-5-yl]-2-methylaniline.

To 3.2 g of t-butyl 4-[3-(4-chlorophenyl)-5-trifluoromethyl-4,5-dihydroisoxazol-5-yl]-2-methylcarbanilate was added dropwise 15 ml of trifluoroacetic acid under ice-cooling and stirring. After stirring was continued at the same temperature for 10 minutes, the solvent was removed under reduced pressure, and 100 ml of diethyl ether was added to the residual oily substance, and the resulting mixture was washed with 100 ml of an aqueous saturated sodium hydrogen carbonate solution. After the organic layer was collected by separation, the aqueous layer was extracted with diethyl ether (100 mlx2), and the organic layers were combined and washed with 100 ml of an aqueous saturated sodium hydrogen carbonate solution, dehydrated with saturated brine and then dried over anhydrous magnesium sulfate in this order, and the solvent was removed under reduced pressure. The residual solid was purified by silica gel column chromatography eluting with ethyl acetate-hexane (1:3) to obtain 1.9 g of the objective material as white crystals.
Melting point 117.5 to 119.0° C
¹H NMR (CDCl₃, Me₄Si, 300MHz) δ 7.55-7.65 (m, 2H), 7.35-7.4 (m, 2H), 7.25 (s, 1 H), 7.22 (d, J=8.1 Hz, 1 H), 6.69 (d, J=8.4Hz, 1 H), 3.97 (d, J=17.1Hz, 1 H), 3.69 (d, J=17.4Hz, 1 H), 3.73 (bs, 2H), 2.18 (s, 3H).
Step 4; Preparation of N¹-[4-[3-(4-chlorophenyl)-5-trifluoromethyl-4,5-dihydroisoxazol-5-yl]-2-methylphenyl]-3-iodo-N²-(1-methyl-2-methylthioethyl)phthalic diamide.

To 4 ml of a dichloromethane solution containing 0.3 g of 3-iodo-N-(1-methyl-2-methylthioethyl)phthalamidic acid were added 0.12 ml of pyridine and 0.18 g of trifluoroacetic anhydride under ice-cooling and stirring, and the mixture was stirred at the same temperature for 2 hours. Then, 0.28 g of 4-[3-(4-chlorophenyl)-5-trifluoromethyl-4,5-dihydroisoxazol-5-yl]-2-methylaniline was added to the mixture, and stirring was continued at room temperature for 12 hours. After completion of the reaction, the reaction mixture was poured into 10 ml of water, extracted with chloroform (10 mlx2), and the organic layer was washed with water and dried over anhydrous magnesium sulfate, and the solvent was removed under reduced pressure. The residual solid was purified by silica gel column chromatography eluting with ethyl acetate-hexane (1:2) to obtain 0.11 g of the objective material as white crystals.
Melting point 116.5 to 120.3°C
¹H NMR (CDCl₃, Me₄Si, 300MHz) δ 8.39 (d, J=2.1 Hz, 1 H), 8.22 (dd, J=8.7, 2.1 Hz, 1H), 7.96 (d, J=7.5Hz, 1 H), 7.77 (d, J=7.8Hz, 1 H), 7.55-7.65 (m, 2H), 7.35-7.5 (m, 4H), 7.20 (t, J=8.1 Hz, 1 H), 6.21 (d, J=8.1Hz, 1 H), 4.25-4.35 (m, 1 H), 4.04 (d, J=17.1Hz, 1 H), 3.72 (d, J=17.4Hz, 1 H), 2.5-2.65 (m, 2H), 2.36 (s, 3H), 1.91 (d, J=1.8Hz, 3H), 1.25 (d, J=6.6Hz, 3H).

### Synthetic example 6

N²,N²-diethyl-3-iodo-N¹-[4-[3-(4-methylsulfonylphenyl)-5-trifluoromethyl-4,5-dihydroisoxazol-5-yl]-2-methylphenyl]phthalic diamide (Present compound No. 2-162). Step 1; Preparation of 3-iodo-N,N-diethylphthalamidic acid .

To 15 ml of an acetonitrile solution containing 2.0 g of 3-iodophthalic anhydride was added dropwise 0.7 g of diethylamine under ice-cooling and stirring, and after completion of the dropwise addition, the mixture was stirred at room temperature for 2 hours. After completion of the reaction, the precipitated solid were collected by filtration, washed with a small amount of acetonitrile to obtain 1.5 g of the objective material as pale yellowish crystals.
Melting point 120.0 to 122.0°C
¹H NMR (CDCl₃, Me₄Si, 300MHz) δ 8.28 (bs, 1 H), 8.05 (d, J=8.5Hz, 1 H), 7.98 (d, J=8.5Hz, 1 H), 7.0-7.2 (m, 1 H), 3.67 (q, J=7.2Hz, 2H), 3.49 (q, J=7.2Hz, 2H), 1.12 (t, J=7.2Hz, 3H), 1.10 (t, J=7.2Hz, 3H).
Step 2; Preparation of N²,N²-diethyl-3-iodo-N¹-[4-[3-(4-methylsulfonylphenyl)-5-trifluoromethyl-4,5-dihydroisoxazol-5-yl]-2-methylphenyl]phthalic diamide.

To 10 ml of a chloroform solution containing 0.5 g of 3-iodo-N,N-diethylphthalamidic acid was added 1.0 g of thionyl chloride, and the mixture was stirred under reflux for 4 hours. After completion of the reaction, the solvent was removed under reduced pressure, the residue was dissolved in 10 ml of acetonitrile, 0.5 g of 4-[3-(4-methylsulfonylphenyl)-5-trifluoromethyl-4,5-dihydroisoxazol-5-yl]-2-methylaniline synthesized in the same manner as in Step 1 to Step 2 of Synthetic example 5 from 4-methylsulfonylphenylaldoxime was added to te mixture, and the resulting mixture was stirred at room temperature for 5 hours. After completion of the reaction, the precipitated solid was collected by filtration, and washed with a small amount of acetonitrile to obtain 0.2 g of the objective material as white crystals.
Melting point 155.0 to 157.0°C
¹H NMR (CDCl₃, Me₄Si, 300MHz) δ 8.19 (d, J=8.6Hz, 1 H), 8.01 (d, J=8.2Hz, 2H), 7.93 (d, J=8.6Hz, 1 H), 7.88 (d, J=8.2Hz, 2H), 7.64 (bs, 1 H), 7.1-7.5 (m, 4H), 4.08 (d, J=17.3Hz, 1 H), 3.77 (d, J=17.3Hz, 1 H), 3.46 (q, J=7.1Hz, 2H), 3.19 (q, J=7.1 Hz, 2H), 3.07 (s, 3H), 2.41 (s, 3H), 1.05 (t, J=7.1 Hz, 3H), 1.04 (t, J=7.1 Hz, 3H).

### Synthetic example 7

N¹-[4-[3-(4-carbamoylphenyl)-5-trifluoromethyl-4,5-dihydroisoxazol-5-yl]-2-methylphenyl]-3-iodo-N²-isopropylphthalic diamide (Present compound No. 2-048).

To 1 ml of a dimethylsulfoxide solution containing 0.22 g of N¹-[4-[3-(4-cyanophenyl)-5-trifluoromethyl-4,5-dihydroisoxazol-5-yl]-2-methylphenyl]-3-iodo-N²-isopropylphthalic diamide (Present compound No. 2-046) was added 0.14 g of anhydrous potassium carbonate, and 0.11 g of 35% aqueous hydrogen peroxide was added dropwise to the mixture at room temperature and under stirring, and after completion of the dropwise addition, stirring was continued at the same temperature for further 1 hour. After completion of the reaction, the reaction mixture was diluted by 5 ml of water, and extracted with 5 ml of ethyl acetate. The organic layer was dried over anhydrous sodium sulfate, and the solvent was removed under reduced pressure to obtain 0.12 g of the objective material as pale yellowish glass-state solid.
Melting point 173.0 to 175.0°C
¹H NMR (CDCl₃, Me₄Si, 300MHz) δ 9.59 (bs, 1 H), 8.70 (bs, 1 H), 8.12 (d, J=8.4Hz, 1 H), 7.9-8.0 (m, 3H), 7.7-7.85 (m, 3H), 7.44 (bs, 2H), 7.20 (t, J=8.0Hz, 1 H), 7.05 (d, J=8.4Hz, 1 H), 6.07 (bs, 1 H), 4.15-4.25 (m, 1 H), 4.09 (d, J=17.2Hz, 1 H), 3.79 (d, J=17.2Hz, 1 H), 3.00 (s, 3H), 1.15 (d, J=6.8Hz, 6H).

### Synthetic example 8

3-lodo-N²-isopropyl-N¹-[4-[3-(4-methylsulfinylphenyl)-5-trifluoromethyl-4,5-dihydroisoxazol-5-yl]-2-methylphenyl]phthalic diamide (Present compound No. 2-037). Step 1; Preparation of t-butyl 2-methyl-4-[3-(4-methylthiophenyl)-5-trifluoromethyl-4,5-dihydroisoxazol-5-yl]carbanilate.

To 50 ml of a N,N-dimethylformamide solution containing 2.0 g of t-butyl 4-[3-(4- fluorophenyl)-5-trifluoromethyl-4,5-dihydroisoxazol-5-yl]-2-methylcarbanilate synthesized in Step 1 to Step 3 of Synthetic example 1 was added 1.0 g of sodium thiomethoxide, and the mixture was stirred at room temperature for 15 hours. After completion of the reaction, the reaction mixture was diluted by 100 ml of ethyl acetate, washed with water (100 mlx2), and the organic layer was dehydrated by saturated brine and then dried over anhydrous magnesium sulfate in this order, and the solvent was removed under reduced pressure. The residue was crystallized by using a mixed solvent of diisopropyl ether-hexane to obtain 2.0 g of the objective material as white crystals.
Melting point 147.0 to 149.0°C
¹H NMR (CDCl₃, Me₄Si, 300MHz) δ 7.95 (d, J=8.5Hz, 1 H), 7.57 (d, J=8.5Hz, 2H), 7.42 (s, 1 H), 7.36 (d, J=8.5Hz, 1 H), 7.24 (d, J=8.5Hz, 2H), 6.33 (bs, 1 H), 4.02 (d, J=17.0Hz, 1 H), 3.70 (d, J=17.0Hz, 1 H), 2.50 (s, 3H), 2.28 (s, 3H), 1.52 (s, 9H).
Step 2; Preparation of t-butyl 2-methyl-4-[3-(4-methylsulfinylphenyl)-5-trifluoromethyl-4,5-dihydroisoxazol-5-yl]carbanilate.

To 50 ml of a dichloromethane solution containing 1.0 g of t-butyl 2-methyl-4-[3-(4-methylthiophenyl)-5-trifluoromethyl-4,5-dihydroisoxazol-5-yl]carbanilate was added 0.57 g of 3-chloroperbenzoic acid under ice-cooling and stirring, and the mixture was stirred at the same temperature for 15 minutes. After completion of the reaction, the reaction mixture was washed with an aqueous saturated sodium hydrogen carbonate solution (30 mlx3), the organic layer was dehydrated by saturated brine and then dried over anhydrous magnesium sulfate in this order, and the solvent was removed under reduced pressure. The residue was purified by using preparative liquid chromatography (acetonitrile:water=4:1) to obtain 0.9 g of the objective material as white glass-state solid.
Melting point 76.5 to 88.5°C
¹H NMR (CDCl₃, Me₄Si, 300MHz) δ 7.97 (d, J=8.5Hz, 1 H), 7.84 (d, J=8.5Hz, 2H), 7.40 (d, J=8.5Hz, 2H), 7.41 (bs, 1 H), 7.37 (d, J=8.5Hz, 1 H), 6.40 (bs, 1 H), 4.07 (d, J=17.0Hz, 1 H), 3.76 (d, J=17.0Hz, 1 H), 2.74 (s, 3H), 2.29 (s, 3H), 1.53 (s, 9H).
Step 3; Preparation of 2-methyl-4-[3-(4-methylsulfinylphenyl)-5-trifluoromethyl-4,5-dihydroisoxazol-5-yl]aniline.

To 0.4 g of t-butyl 2-methyl-4-[3-(4-methylsulfinylphenyl)-5-trifluoromethyl-4,5-dihydroisoxazol-5-yl]carbanilate was added dropwise 2 ml of trifluoroacetic acid at room temperature under stirring. After stirring was continued at the same temperature for 30 minutes, the solvent was removed under reduced pressure, and the residue was dissolved in 50 ml of ethyl acetate, and washed with 30 ml of an aqueous saturated sodium hydrogen carbonate solution. The organic layer was dehydrated by saturated brine and then dried over anhydrous magnesium sulfate in this order, and the solvent was removed under reduced pressure to obtain 0.3 g of the crude objective material as pale yellowish glass-state solid. This product was used in the next step as such without purification.
Melting point 68.5 to 77.0° C
¹H NMR (CDCl₃, Me₄Si, 300MHz) δ 7.83 (d, J=8.5Hz, 2H), 7.69 (d, J=8.5Hz, 2H), 7.26 (s, 1 H), 7.23 (d, J=8.2Hz, 1 H), 6.67 (d, J=8.2Hz, 1 H), 4.03 (d, J=17.0Hz, 1 H), 3.7-3.8 (m, 3H), 2.74 (s, 3H), 2.19 (s, 3H).
Step 4; Preparation of 3-iodo-N²-isopropyl-N¹-[2-methyl-4-[3-(4-methylsulfinylphenyl)-5-trifluoromethyl-4,5-dihydroisoxazol-5-yl]phenyl]phthalic diamide.

To 30 ml of a toluene solution containing 0.3 g of 3-iodo-N-isopropylphthalamidic acid was added dropwise 0.23 g of trifluoroacetic anhydride at room temperature under stirring. After stirring the mixture at the same temperature for 30 minutes, the solvent was removed under reduced pressure, the residual yellowish oily substance was dissolved in 5 ml of acetonitrile, and 0.3 g of 2-methyl-4-[3-(4-methylsulfinylphenyl)-5-trifluoromethyl-4,5- dihydroisoxazol-5-yl]aniline was added to the mixture, and stirring was continued at room temperature for 5 hours. After completion of the reaction, the solvent was removed under reduced pressure, the residual solid was purified by silica gel column chromatography eluting with ethyl acetate to obtain 0.4 g of the objective material as white glass-state solid.
Melting point 134.0 to 151.5°C
¹H NMR (CDCl₃, Me₄Si, 300MHz) δ 8.45 (s, 1 H), 8.16 (d, J=8.1 Hz, 1 H), 7.96 (d, J=7.8Hz, 1 H), 7.85 (d, J=8.7Hz, 2H), 7.79 (d, J=7.8Hz, 1 H), 7.70 (d, J=8.7Hz, 2H), 7.46 (s, 1 H), 7.42 (d, J=8.1 Hz, 1 H), 7.20 (t, J=7.8Hz, 1 H), 5.95 (d, J=7.8Hz, 1 H), 4.1-4.3 (m, 1 H), 4.09 (d, J=17.1Hz, 1 H), 3.78 (d, J=17.1Hz, 1 H), 2.74 (s, 3H), 2.37 (s, 3H), 1.17 (d, J=6.6Hz, 6H).

### Synthetic example 9

3-Iodo-N²-isopropyl-N¹-[2-methyl-4-(3,5-bistrifluoromethyl-4,5-dihydroisoxazol-5-yl)phenyl]phthalic diamide (Present compound No. 1-013).
Step 1; Preparation of 2-methyl-4-(1-trifluoromethylethenyl)aniline.

To 1.5 g of t-butyl 2-methyl-4-(1-trifluoromethylethenyl)carbanilate synthesized in Step 1 to Step 2 of Synthetic example 1 was added dropwise 5 ml of trifluoroacetic acid under ice-cooling and stirring. After stirring was continued at room temperature for 10 minutes, the solvent was removed under reduced pressure to obtain 1.3 g of the crude objective material as brownish oily substance. This product was used in the next step as such without purification.
Step 2; Preparation of 3-iodo-N²-isopropyl-N²-[2-methyl-4-(1-trifluoromethylethenyl)-phenyl]phthalic diamide.

To 30 ml of a toluene solution containing 2.0 g of 3-iodo-N-isopropylphthalamidic acid was added dropwise 1.8 g of trifluoroacetic anhydride at room temperature under stirring. After stirring the mixture at the same temperature for 30 minutes, the solvent was removed under reduced pressure, and the residual yellowish oily substance was dissolved in 30 ml of tetrahydrofuran, 1.2 g of crude 2-methyl-4-(1-trifluoromethylethenyl)aniline was added to the solution, and stirring was continued at room temperature for 5 hours. After completion of the reaction, the solvent was removed under reduced pressure, and the residual solid was purified by silica gel column chromatography eluting with ethyl acetate-chloroform (1:9) to obtain 2.2 g of the objective material as white crystals.
Melting point 201.0 to 203.0°C
¹H NMR (CDCl₃, Me₄Si, 300MHz) δ 8.37 (bs, 1 H), 8.10 (d, J=6.0Hz, 1 H), 7.96 (d, J=6.9Hz, 1 H), 7.79 (d, J=6.0Hz, 1 H), 7.1-7.4 (m, 3H), 5.92 (s, 1 H), 5.88 (d, J=6.0Hz, 1 H), 5.76 (s, 1 H), 4.1-4.3 (m, 1 H), 2.34 (s, 3H), 1.17 (d, J=6.6Hz, 6H).
Step 3; Preparation of 3-iodo-N²-isopropyl-N'-[2-methyl-4-(3,5-bistrifluoromethyl-4,5-dihydroisoxazol-5-yl)-phenyl]phthalic diamide.

To 30 ml of a 1,2-dimethoxyethane solution containing 1.0 g of 2,2,2-trifluoroacetoaldoxime was added 1.0 g of N-chlorosuccinic imide, and the mixture was stirred at 40°C for 1 hour. Then, to this the reaction mixture were added 1.0 g of 3-iodo-N²-isopropyl-N¹-[2-methyl-4-(1-trifluoromethylethenyl)phenyl]phthalic diamide, 6.0 g of potassium hydrogen carbonate and 1 drop of water, and stirring was continued at room temperature for further 5 hours. After completion of the reaction, the solvent was removed under reduced pressure, to the residue was added 200 ml of water and the mixture was extracted with chloroform (100 mlx2), the organic layer was washed with water and then dried over anhydrous magnesium sulfate, and the solvent was removed under reduced pressure. The residual solid was purified by silica gel column chromatography eluting with ethyl acetate-chloroform (1:9) to obtain 0.42 g of the objective material as white crystals.
Melting point 240.0 to 242.0°C
¹H NMR (CDCl₃, Me₄Si, 300MHz) δ 8.44 (bs, 1 H), 8.21 (d, J=6.0Hz, 1 H), 7.96 (d, J=6.9Hz, 1H), 7.78 (d, J=6.0Hz, 1 H), 7.1-7.5 (m, 3H), 5.90 (d, J=8.0Hz, 1 H), 4.40 (d, J=12.4Hz, 1H), 4.18 (d, J=12.4Hz, 1 H), 2.37 (s, 3H), 1.16 (d, J=6.6Hz, 6H).

### Synthetic example 10

N¹-[4-(3-ethoxycarbonyl-5-trifluoromethyl-4,5-dihydroisoxazol-5-yl)-2-methylphenyl]-6-iodo-N²-isopropylphthalic diamide (Present compound No. 1-020).
Step 1; Preparation of 3-iodo-N-[2-methyl-4-(1-trifluoromethylethenyl)phenyl]-phthalimide.

30 ml of an acetic acid solution containing 1.6 g of 2-methyl-4-(1-trifluoromethylethenyl)aniline synthesized in Step 1 of Synthetic example 9 and 2.2 g of 3-iodophthalic anhydride were stirred under reflux for 2 hours. After completion of the reaction, the solvent was removed under reduced pressure, and the residue was purified by silica gel column chromatography eluting with ethyl acetate-hexane (3:7) to obtain 2.8 g of the objective material as white crystals.
Melting point 143.0 to 145.0°C
¹H NMR (CDCl₃, Me₄Si, 300MHz) δ 8.20 (d, J=9.0Hz, 1 H), 7.96 (d, J=9.0Hz, 1 H), 7.2-7.6 (m, 4H), 6.02 (s, 1 H), 5.83 (s, 1 H), 2.24 (s, 3H).
Step 2; Preparation of N-[4-(3-ethoxycarbonyl-5-trifluoromethyl-4,5-dihydroisoxazol-5-yl)-2-methylphenyl]-3-iodophthalimide.

To 40 ml of a 1,2-dimethoxyethane solution containing 0.4 g of 3-iodo-N-[2-methyl-4-(1-trifluoromethylethenyl)phenyl]phthalimide 0.8 g and 2-chloro-2-hydroxyiminoethyl acetate were added 3.0 g of potassium hydrogen carbonate and 1 drop of water, and the mixture was stirred at room temperature for 18 hours. After completion of the reaction, the solvent was removed under reduced pressure, and 100 ml of water was added to the residue and the resulting mixture was extracted with ethyl acetate (100 mlx2), the organic layer was dehydrated by saturated brine and then dried over anhydrous sodium sulfate in this order, and the solvent was removed under reduced pressure. The residual solid was purified by silica gel column chromatography eluting with ethyl acetate-hexane (3:7) to obtain 0.45 g of the objective material as white crystals.
Step 3; Preparation of N¹-[4-(3-ethoxycarbonyl-5-trifluoromethyl-4,5-dihydroisoxazol-5-yl)-2-methylphenyl]-6-iodo-N²-isopropylphthalic diamide.

To 20 ml of a 1,4-dioxane solution containing 0.45 g of N-[4-(3-ethoxycarbonyl-5-trifluoromethyl-4,5-dihydroisoxazol-5-yl)-2-methylphenyl]-3-iodophthalimide was added 2.0 ml of isopropylamine, and the mixture was stirred at room temperature for 3 days. After completion of the reaction, the solvent was removed under reduced pressure, and the residual solid was purified by silica gel column chromatography eluting with ethyl acetate- chloroform (3:7) to obtain 0.26 g of the objective material as white crystals.
Melting point 172.0 to 174.0°C
¹H NMR (CDCl₃, Me₄Si, 300MHz) δ 8.10 (d, J=6.5Hz, 1 H), 7.91 (d, J=6.0Hz, 1 H), 7.79 (bs, 1 H), 7.53 (d, J=7.0Hz, 1 H), 7.1-7.4 (m, 3H), 6.40 (d, J=7.7Hz, 1 H), 4.36 (q, J=7.2Hz, 2H), 4.0-4.2 (m, 1H), 3.95 (d, J=18.1 Hz, 1 H), 3.65 (d, J=18.1 Hz, 1 H), 2.36 (s, 3H), 1.21 (t, J=7.2Hz, 3H), 1.11 (d, J=6.6Hz, 6H).

### Synthetic example 11

3-lodo-N²-isopropyl-N¹-[4-[3-(1,2,4-triazol-1-yl)-5-trifluoromethyl-4,5-dihydroisoxazol-5-yl]-2-methylphenyl]phthalic diamide (Present compound No. 1-032).
Step 1; Preparation of t-butyl 4-(3-chloro-5-trifluoromethyl-4,5-dihydroisoxazol-5-yl)-2-methylcarbanilate.

To 80 ml of a 1,2-dimethoxyethane solution containing 5.0 g of hydroxyiminoacetic acid was added 15.0 g of N-chlorosuccinic imide, and the mixture was stirred at 75°C for 1 hour. After the reaction mixture was cooled to room temperature, 3.0 g of t-butyl 2-methyl-4-(1-trifluoromethylethenyl)carbanilate synthesized in Step 1 to Step 2 of Synthetic example 1 and 20.0 g of potassium hydrogen carbonate were added to the mixture, and stirring was continued at room temperature for further 18 hours. After completion of the reaction, the solvent was removed under reduced pressure, 50 ml of chloroform was added to the residue, insoluble materials were filtered off, the chloroform layer was washed with 1 N hydrochloric acid, dehydrated by saturated brine and then dried over anhydrous magnesium sulfate in this order, and the solvent was removed under reduced pressure. The residual solid was purified by silica gel column chromatography eluting with chloroform-hexane (4:6) to obtain 1.1 g of the objective material as white crystals.
Melting point 83.0 to 85.0°C
¹H NMR (CDCl₃, Me₄Si, 300MHz) δ 7.97 (d, J=8.5Hz, 1 H), 7.2-7.3 (m, 2H), 6.34 (bs, 1 H), 3.83 (d, J=17.5Hz, 1 H), 3.55 (d, J=17.5Hz, 1 H), 2.28 (s, 3H), 1.53 (s, 9H).
Step 2; Preparation of t-butyl 2-methyl-4-[3-(1,2,4-triazol-1-yl)-5-trifluoromethyl-4,5-dihydroisoxazol-5-yl]carbanilate.

To 20 ml of a N,N-dimethylformamide solution containing 0.23 g of 1,2,4-triazole was added 0.13 g of 60% oily sodium hydride, and the mixture was stirred at room temperature for 15 minutes. After generation of a hydrogen gas was stopped, 0.5 g of t-butyl 4-(3-chloro-5-trifluoromethyl-4,5-dihydroisoxazol-5-yl)-2-methylcarbanilate was added to the mixture, and stirring was continued at 50°C for 90 minutes. After completion of the reaction, the reaction mixture was poured into 200 ml of water and extracted with ethyl acetate (100 mlx2), the organic layer was dehydrated by saturated brine and then dried over anhydrous magnesium sulfate in this order, and the solvent was removed under reduced pressure. The residual solid was purified by silica gel column chromatography eluting with ethyl acetate-hexane (3:7) to obtain0.4 g of the objective material as white crystals.
Melting point 46.0 to 48.0°C
¹H NMR (CDCl₃, Me₄Si, 300MHz) δ 8.79 (s, 1 H), 8.08 (s, 1 H), 8.00 (d, J=8.8Hz, 1 H), 7.3-7.4 (m, 2H), 6.36 (bs, 1 H), 4.27 (d, J=17.8Hz, 1 H), 4.01 (d, J=17.8Hz, 1 H), 2.29 (s, 3H), 1.51 (s, 9H).
Step 3; Preparation of 2-methyl-4-[3-(1,2,4-triazol-1-yl)-5-trifluoromethyl-4,5-dihydroisoxazol-5-yl]aniline.

To 0.4 g of t-butyl 2-methyl-4-[3-(1,2,4-triazol-1-yl)-5-trifluoromethyl-4,5-dihydroisoxazol-5-yl]carbanilate was added dropwise 5 ml of trifluoroacetic acid under ice-cooling and stirring. After stirring was continued at room temperature for 10 minutes, the solvent was removed under reduced pressure, and the residue was dissolved in 50 ml of diethyl ether and washed with 50 ml of an aqueous saturated sodium hydrogen carbonate solution. The organic layer was dehydrated by anhydrous magnesium sulfate, and the solvent was removed under reduced pressure to obtain 0.3 g of the crude objective material as pale yellowish oily substance. This product was used in the next step as such without purification.
Step 4; Preparation of 3-iodo-N²-isopropyl-N¹-[2-methyl-4-[3-(1,2,4-triazol-1-yl)-5-trifluoromethyl-4,5-dihydroisoxazol-5-yl]phenyl]phthalic diamide.

To 10 ml of a toluene solution containing 0.35 g of 3-iodo-N-isopropylphthalamidic acid was added dropwise 0.35 g of trifluoroacetic anhydride at room temperature under stirring. After the mixture was stirred at the same temperature for 30 minutes, the solvent was removed under reduced pressure, and the residue was dissolved in 10 ml of tetrahydrofuran, 0.3 g of 2-methyl-4-[3-(1,2,4-triazol-1-yl)-5-trifluoromethyl-4,5-dihydroisoxazol-5-yl]aniline was added to the solution, and stirring was continued at room temperature for 18 hours. After completion of the reaction, the solvent was removed under reduced pressure, and the residual solid was purified by silica gel column chromatography eluting with ethyl acetate-chloroform (4:6) to obtain 0.4 g of the objective material as white crystals.
Melting point 131.0 to 133.0°C
¹H NMR (CDCl₃, Me₄Si, 300MHz) δ 8.80 (s, 1 H), 8.44 (bs, 1 H), 8.21 (d, J=7.8Hz, 1 H), 8.08 (s, 1 H), 7.98 (d, J=6.8Hz, 1 H), 7.82 (d, J=6.8Hz, 1 H), 7.2-7.5 (m, 3H), 5.83 (d, J=8.0Hz, 1 H), 4.29 (d, J=17.8Hz, 1 H), 4.1-4.3 (m, 1 H), 4.00 (d, J=17.8Hz, 1 H), 2.38 (s, 3H), 1.16 (d, J=6.6Hz, 6H).

### Synthetic example 12

N¹-[4-[3-(4-chlorophenyl)-5-cyclopropyl-4,5-dihydroisoxazol-5-yl]-2-methylphen yl]-3-iodo-N²-isopropylphthalic diamide (Present compound No. 2-013).
Step 1; Preparation of t-butyl 4-cyclopropylcarbonyl-2-methylcarbanilate.

Under nitrogen atmosphere, to 48 ml of a t-butyl methyl ether solution containing 3.6 g of t-butyl 4-iodo-2-methylcarbanilate was added dropwise 15.1 ml of n-butyl lithium (1.6M) at -30°C under stirring, and after completion of the dropwise addition, the mixture was raised to 0°C and stirred for further 10 minutes. Then, this reaction mixture was cooled to -78°C, 2.7 g of methylcyclopropanecarboxylate was added to the mixture, and stirring was continued at the same temperature for 4 hours, and then, at 0°C for 2 hours. After completion of the reaction, 100 ml of a saturated aqueous ammonium chloride solution was added to the reaction mixture, the resulting mixture was extracted with diethyl ether (100 mlx2), the organic layer was dehydrated by saturated brine and then dried over anhydrous magnesium sulfate in this order, and the solvent was removed under reduced pressure. The residual solid was purified by silica gel column chromatography eluting with ethyl acetate-hexane (1:5) to obtain 0.93 g of the objective material as white crystals.
¹H NMR (CDCl₃, Me₄Si, 300MHz) δ 8.10 (d, J=8.7Hz, 1 H), 7.89 (dd, J=8.7, 2.1 Hz, 1H), 7.82 (d, J=1.5Hz, 1 H), 6.50 (s, 1 H), 2.6-2.7 (m, 1 H), 2.30 (s, 3H), 1.54 (s, 9H), 1.15-1.25 (m, 2H), 0.9-1.05 (m, 2H).
Step 2; Preparation of t-butyl 4-(1-cyclopropylethenyl)-2-methylcarbanilate.

Under nitrogen atmosphere, to 25 ml of a tetrahydrofuran suspension containing 3.0 g of triphenylmethylphosphonium bromide was added dropwise 5.1 ml of n-butyl lithium (1.6M) under ice-cooling and stirring, and the mixture was stirred at the same temperature for 50 minutes. Then, this reaction mixture was added dropwise to 8 ml of a tetrahydrofuran solution containing 0.93 g of t-butyl 4-cyclopropylcarbonyl-2-methylcarbanilate under nitrogen atmosphere under ice-cooling and stirring, and after completion of the dropwise addition, stirring was continued at room temperature for 5 hours. After completion of the reaction, 100 ml of water was added to the reaction mixture, the resulting mixture was extracted with diethyl ether (100 mlx2), the organic layer was dehydrated by saturated brine and then dried over anhydrous magnesium sulfate in this order, and the solvent was removed under reduced pressure. The residual solid was purified by silica gel column chromatography eluting with ethyl acetate-hexane (1:10) to obtain 0.73 g of the objective material as white crystals.
¹H NMR (CDCl₃, Me₄Si, 300MHz) δ 7.77 (d, J=8.1 Hz, 1 H), 7.42 (dd, J=8.1, 1.8Hz, 1 H), 7.37 (d, J=1.8Hz, 1 H), 6.26 (s, 1 H), 5.21 (s, 1 H), 4.87 (s, 1 H), 2.26 (s, 3H), 1.55-1.7 (m, 1 H), 1.53 (s, 9H), 0.75-0.85 (m, 2H), 0.5-0.6 (m, 2H).
Step 3; Preparation of t-butyl 4-[3-(4-chlorophenyl)-5-cyclopropyl-4,5-dihydroisoxazol-5-yl]-2-methylcarbanilate.

To 25 ml of a 1,2-dimethoxyethane solution containing 0.8 g of 4-chlorophenylaldoxime was added 0.68 g of N-chlorosuccinic imide, and the mixture was stirred at 70°C for 1 hour. After the reaction mixture was ice-cooled, 0.55 g of t-butyl 4-(1-cyclopropylethenyl)-2-methylcarbanilate and 3.6 g of potassium hydrogen carbonate were added to the mixture, and stirring was continued at room temperature for further 6 hours. After completion of the reaction, the solvent was removed under reduced pressure, to the residue were added 30 ml of diethyl ether and 30 ml of water, the diethyl ether layer was collected by separation, and the aqueous layer was extracted with 30 ml of diethyl ether. The organic layers were combined, dehydrated with saturated brine and then dried over anhydrous magnesium sulfate in this order, the solvent was removed under reduced pressure, and the residual solid was purified by silica gel column chromatography eluting with ethyl acetate-hexane (1:10) to obtain 0.6 g of the objective material as white crystals.
¹H NMR (CDCl₃, Me₄Si, 300MHz) δ 7.81 (d, J=9.3Hz, 1 H), 7.58 (dt, J=9.0, 2.1 Hz, 2H), 7.25-7.4 (m, 4H), 6.25 (bs, 1 H), 3.49 (s, 2H), 2.26 (s, 3H), 1.52 (s, 9H), 1.4-1.5 (m, 1 H), 0.45-0.6 (m, 4H).
Step 4; Preparation of 4-[3-(4-chlorophenyl)-5-cyclopropyl-4,5-dihydroisoxazol-5-yl]-2-methylaniline.

To 0.59 g of t-butyl 4-[3-(4-chlorophenyl)-5-cyclopropyl-4,5-dihydroisoxazol-5-yl]- 2-methylcarbanilate was added dropwise 5 ml of trifluoroacetic acid under ice-cooling and stirring. After stirring was continued at the same temperature for 5 minutes, the solvent was removed under reduced pressure to obtain the crude objective material as brownish oily substance. This product was used in the next step as such without purification.
Step 5; Preparation of N¹-[4-[3-(4-chlorophenyl)-5-cyclopropyl-4,5-dihydroisoxazol-5-yl]-2-methylphenyl]-3-iodo-N²-isopropylphthalic diamide.

To 10 ml of a toluene solution containing 0.53 g of 3-iodo-N-isopropylphthalamidic acid was added dropwise 0.37 ml of trifluoroacetic anhydride at room temperature under stirring. After the mixture was stirred at the same temperature for 30 minutes, the solvent was removed under reduced pressure, the residual yellowish oily substance was dissolved in 17 ml of tetrahydrofuran, crude 4-[3-(4-chlorophenyl)-5-cyclopropyl-4,5-dihydroisoxazol- 5-yl]-2-methylaniline obtained in Step 4 was added to the mixture, and stirring was continued at room temperature for 17 hours. After completion of the reaction, the solvent was removed under reduced pressure, and the residual solid was purified by silica gel column chromatography eluting with ethyl acetate-hexane (1:2) to obtain 0.21 g of the objective material as white crystals.
Melting point 178.0 to 180.0°C
¹H NMR (CDCl₃, Me₄Si, 300MHz) δ 8.32 (bs, 1 H), 7.96 (t, J=8.4Hz, 2H), 7.77 (d, J=7.2Hz, 1 H), 7.58 (dt, J=8.4, 1.8Hz, 2H), 7.3-7.4 (m, 4H), 7.18 (t, J=7.5Hz, 1 H), 5.91 (d, J=8.1 Hz, 1 H), 4.15-4.3 (m, 1 H), 3.51 (s, 2H), 2.32 (s, 3H), 1.4-1.5 (m, 1 H), 1.18 (d, J=6.6Hz, 6H), 0.45-0.65 (m, 4H).

### Synthetic example 13

N¹-[4-[3-(4-chlorophenyl)-5-heptafluoropropyl-4,5-dihydroisoxazol-5-yl]-2-methylphenyl]-3-iodo-N²-isopropylphthalic diamide (Present compound No. 2-059).
Step 1; Preparation of t-butyl 4-(2,2,3,3,4,4,4-heptafluoro-1-hydroxy-1-methylbutyl)-2-methylcarbanilate.

Under nitrogen atmosphere, to 100 ml of a diethyl ether solution containing 4.2 g of t-butyl 4-iodo-2-methylcarbanilate was added dropwise 17 ml of n-butyl lithium (1.57M hexane solution) at -20°C under stirring, and after completion of the dropwise addition, the mixture was raised to 0°C and stirred for further 15 minutes. Then, this reaction mixture was cooled to -78°C, 2.0 g of heptafluoropropyl methyl ketone was added to the mixture, and the temperature of the mixture was gradually raised to at room temperature, and stirring was continued at room temperature for further 2 hours. After completion of the reaction, to the reaction mixture was added 100 ml of a saturated aqueous ammonium chloride solution, the organic layer was collected by separation, dehydrated by saturated brine and then dried over anhydrous magnesium sulfate in this order, and the solvent was removed under reduced pressure. The residual solid was purified by silica gel column chromatography eluting with ethyl acetate-hexane (1:9) to obtain 1.3 g of the objective material as pale yellowish oily substance.
n_{D}^{20.3°C} 1.4557
¹H NMR (CDCl₃, Me₄Si, 300MHz) δ 7.88 (d, J=9.3Hz, 1 H), 7.34 (m, 2H), 6.31 (bs, 1 H), 2.59 (bs, 1 H), 2.27 (s, 3H), 1.78 (s, 3H), 1.53 (s, 9H).
Step 2; Preparation of t-butyl 4-(1-heptafluoropropylethenyl)-2-methylcarbanilate.

To 5 ml of a tetrahydrofuran solution containing 1.0 g of t-butyl 4-(2,2,3,3,4,4,4-heptafluoro-1-hydroxy-1-methylbutyl)-2-methylcarbanilate were added 0.56 g of potassium t-butoxide and 1.1 g of trifluoroacetic acid anhydride under ice-cooling and stirring, and the mixture was stirred at the same temperature for 15 minutes. Moreover, to the reaction mixture were additionally added 1.7 g of potassium t-butoxide and 1.1 g of trifluoroacetic acid anhydride, the mixture was heated to at room temperature and stirring was continued at room temperature for further 3 hours. After completion of the reaction, the reaction mixture was poured into 50 ml of ice-water and extracted with ethyl acetate (30 mlx3), the organic layer was dehydrated by saturated brine and then dried over anhydrous magnesium sulfate in this order, and the solvent was removed under reduced pressure. The residual solid was purified by silica gel column chromatography eluting with ethyl acetate-hexane (3:17) to obtain 0.8 g of the objective material as white crystals.
Melting point 50.0 to 53.5°C
¹H NMR (CDCl₃, Me₄Si, 300MHz) δ 7.88 (d, J=8.7Hz, 1 H), 7.20 (d, J=8.7Hz, 1 H), 7.15 (s, 1 H), 6.31 (bs, 1 H), 5.92 (s, 1 H), 5.75 (s, 1 H), 2.26 (s, 3H), 1.53 (s, 9H).
Step 3; Preparation of t-butyl 4-[3-(4-chlorophenyl)-5-heptafluoropropyl-4,5-dihydroisoxazol-5-yl]-2-methylcarbanilate.

To 20 ml of a N,N-dimethylformamide solution containing 0.4 g of t-butyl 4-(1-heptafluoropropylethenyl)-2-methylcarbanilate and 0.29 g of 4-chlorophenylhydroximic acid chloride synthesized in Step 1 of Synthetic example 5 was added 0.16 g of triethylamine, and the mixture was stirred at room temperature for 2 hours. Then, 0.29 g of 4-chlorophenylhydroximic acid chloride and 0.16 g of triethylamine were additionally added to the mixture, and stirring was further continued at room temperature for 3.5 hours. After completion of the reaction, the reaction mixture was diluted by 80 ml of ethyl acetate, washed with 50 ml of water, dehydrated by saturated brine and then dried over anhydrous magnesium sulfate in this order, and the solvent was removed under reduced pressure. The residue was purified by silica gel column chromatography eluting with ethyl acetate- hexane (1:4) to obtain 0.5 g of the objective material as yellowish oily substance.
¹H NMR (CDCl₃, Me₄Si, 300MHz) δ 7.97 (d, J=8.8Hz, 1 H), 7.59 (d, J=8.8Hz, 2H), 7.35-7.45 (m, 4H), 6.33 (bs, 1H), 4.20 (d, J=17.0Hz, 1 H), 3.68 (d, J=17.0Hz, 1 H), 2.28 (s, 3H), 1.52 (s, 9H).
Step 4; Preparation of 4-[3-(4-chlorophenyl)-5-heptafluoropropyl-4,5-dihydroisoxazol-5-yl]-2-methylaniline.

To 0.45 g of t-butyl 4-[3-(4-chlorophenyl)-5-heptafluoropropyl-4,5-dihydroisoxazol- 5-yl]-2-methylcarbanilate was added dropwise 3 ml of trifluoroacetic acid under stirringat room temperature. After stirring was continued at the same temperature for 30 minutes, the solvent was removed under reduced pressure, the residue was dissolved in 50 ml of ethyl acetate, washed with 50 ml of an aqueous saturated sodium hydrogen carbonate solution and then with 50 ml of water, dehydrated by saturated brine and then dried over anhydrous magnesium sulfate in this order, and the solvent was removed under reduced pressure. the residue was purified by silica gel column chromatography eluting with ethyl acetate-hexane (1:4) to obtain 0.34 g of the objective material as yellowish oily substance.
¹H NMR (CDCl₃, Me₄Si, 300MHz) δ 7.59 (d, J=6.9Hz, 2H), 7.38 (d, J=6.9Hz, 2H), 7.2-7.3 (m, 2H), 6.68 (d, J=8.1 Hz, 1 H), 4.16 (d, J=17.1 Hz, 1 H), 3.65-3.75 (m, 3H), 2.19 (s, 3H).
Step 5; Preparation of N¹-[4-[3-(4-chlorophenyl)-5-heptafluoropropyl-4,5-dihydroisoxazol-5-yl]-2-methylphenyl]-3-iodo-N²-isopropylphthalic diamide.

To 5 ml of a toluene solution containing 0.22 g of 3-iodo-N-isopropylphthalamidic acid was added dropwise 0.21 g of trifluoroacetic anhydride under ice-cooling and stirring. After the mixture was stirred at the same temperature for 30 minutes, the solvent was removed under reduced pressure, the remaining yellowish oily substance was dissolved in 5 ml of acetonitrile, 5 ml of an acetonitrile solution containing 0.3 g of 4-[3-(4-chlorophenyl)-5-heptafluoropropyl-4,5-dihydroisoxazol-5-yl]-2-methylaniline obtained in Step 4 was added dropwise to the solution at room temperature, and stirring was continued at the same temperature for further 5 hours. After completion of the reaction, insoluble materials were separated by filtration, and the solvent was removed under reduced pressure. The residue was crystallized from 5 ml of acetonitrile to carry out purification to obtain 0.22 g of the objective material as white crystals.
Melting point 155.5 to 158.5°C
¹H NMR (CDCl₃, Me₄Si, 300MHz) δ 8.42 (bs, 1 H), 8.16 (d, J=8.4Hz, 1 H), 7.95 (d, J=7.8Hz, 1 H), 7.74 (d, J=7.8Hz, 1 H), 7.60 (d, J=8.4Hz, 2H), 7.35-7.5 (m, 4H), 7.17 (t, J=7.8Hz, 1 H), 6.05 (d, J=8.1 Hz, 1 H), 4.15-4.3 (m, 2H), 3.71 (d, J=17.4Hz, 1 H), 2.35 (s, 3H), 1.13 (d, J=4.8Hz, 6H).

### Synthetic example 14

N¹-[4-[5-(4-chlorophenyl)-3-methyl-4,5-dihydroisoxazol-5-yl]-2-methylphenyl]-3-iodo-N²-isopropylphthalic diamide (Present compound No. 3-006).
Step 1; Preparation of tertiary butyl 4-[1-(4-chlorophenyl)-1-hydroxyethyl]-2-methylcarbanilate.

Under nitrogen atmosphere, 40 ml of a t-butyl methyl ether solution containing 3.0 g of t-butyl 4-iodo-2-methylcarbanilate was added dropwise 12 ml of n-butyl lithium (1.57M hexane solution) at -50°C under stirring, and after completion of the dropwise addition, the mixture was warmed to 0°C, and the mixture was stirred for further 30 minutes. Then, this reaction mixture was cooled to -78°C, 1.39 g of 4-chloroacetophenone was added to the mixture, and the resulting mixture was gradually warmed up to 0°C, and further stirring was continued at the same temperature for 1 hour. After completion of the reaction, 100 ml of a saturated aqueous ammonium chloride solution was added to the reaction mixture, the organic layer was collected by separation, and the aqueous layer was extracted with 100 ml of ethyl acetate. The organic layers were combined and dried over anhydrous magnesium sulfate, and the solvent was removed under reduced pressure. The residue was purified by silica gel column chromatography eluting with ethyl acetate-hexane (1:9 to 2:3) to obtain 1.7 g of the objective material as yellowish oily substance.
¹H NMR (CDCl₃, Me₄Si, 300MHz) δ 7.75 (d, J=8.3Hz, 1 H), 7.1-7.4 (m, 6H), 6.25 (bs, 1 H), 2.21 (s, 3H), 2.16 (bs, 1 H), 1.89 (s, 3H), 1.51 (s, 9H).
Step 2; Preparation of t-butyl 4-[1-(4-chlorophenyl)ethenyl]-2-methylcarbanilate.

To 20 ml of a 1,2-dichloroethane solution containing 3.1 g of t-butyl 4-[1-(4-chlorophenyl)-1-hydroxyethyl]-2-methylcarbanilate was added 0.33 g of paratoluenesulfonic acid monohydrate, and the mixture was stirred at room temperature for 30 minutes. After completion of the reaction, the reaction mixture was poured into 50 ml of water and extracted with 50 ml of chloroform, and after the organic layer was washed with 50 ml of an aqueous saturated sodium hydrogen carbonate solution, it was dehydrated by saturated brine and then dried over anhydrous sodium sulfate in this order, and the solvent was removed under reduced pressure. The residue was purified by silica gel column chromatography eluting with ethyl acetate-hexane (1:4) to obtain 2.6 g of the objective material as yellow crystals.
Melting point 101.5 to 104.0°C
¹H NMR (CDCl₃, Me₄Si, 300MHz) δ 7.80 (d, J=8.4Hz, 1 H), 7.2-7.35 (m, 4H), 7.14 (d, J=8.4Hz, 1 H), 7.08 (s, 1 H), 6.29 (bs, 1 H), 5.40 (d, J=1.0Hz, 1 H), 5.36 (d, J=1.0Hz, 1 H), 2.23 (s, 3H), 1.52 (s, 9H).
Step 3; Preparation of t-butyl 4-[5-(4-chlorophenyl)-3-methyl-4,5-dihydroisoxazol-5-yl]-2-methylcarbanilate.

To 5 ml of a N,N-dimethylformamide solution containing 0.41 g of acetoaldoxime was added 0.93 g of N-chlorosuccinic imide, and the mixture was stirred at 50°C for 1 hour. Then, to this reaction mixture were added 0.8 g of t-butyl 4-[1-(4-chlorophenyl)ethenyl]-2- methylcarbanilate and 0.71 g of triethylamine, and stirring was continued at room temperature for further 13 hours. After completion of the reaction, the reaction mixture was poured into 30 ml of water, extracted with 50 ml of ethyl acetate and dried over anhydrous magnesium sulfate, and then, the solvent was removed under reduced pressure. The residue was purified by silica gel column chromatography eluting with ethyl acetate- hexane (2:3) to obtain 0.46 g of the objective material as yellowish oily substance.
¹H NMR (CDCl₃, Me₄Si, 300MHz) δ 7.79 (d, J=8.2Hz, 1 H), 7.1-7.35 (m, 6H), 6.26 (bs, 1 H), 3.56 (d, J=16.7Hz, 1 H), 3.42 (d, J=16.7Hz, 1 H), 2.21 (s, 3H), 1.99 (s, 3H), 1.51 (s, 9H).
Step 4; Preparation of 4-[5-(4-chlorophenyl)-3-methyl-4,5-dihydroisoxazol-5-yl]-2-methylaniline.

To 0.46 g of t-butyl 4-[5-(4-chlorophenyl)-3-methyl-4,5-dihydroisoxazol-5-yl]-2-methylcarbanilate was added dropwise 5 ml of trifluoroacetic acid under ice-cooling and stirring. After stirring was continued at room temperature for 20 minutes, the solvent was removed under reduced pressure, the residue was dissolved in 30 ml of ethyl acetate and washed with 20 ml of an aqueous saturated sodium hydrogen carbonate solution. The organic layer was dried over anhydrous sodium sulfate, and the solvent was removed under reduced pressure to obtain 0.34 g of the crude objective material as brownish oily substance. This product was used in the next step as such without purification.
¹H NMR (CDCl₃, Me₄Si, 300MHz) δ 7.25-7.4 (m, 4H), 7.04 (s, 1 H), 6.97 (d, J=8.1 Hz, 1 H), 6.61 (d, J=8.1 Hz, 1 H), 3.5-3.7 (m, 3H), 3.35 (d, J=16.8Hz, 1 H), 2.13 (s, 3H), 1.99 (s, 3H).
Step 5; Preparation of N¹-[4-[5-(4-chlorophenyl)-3-methyl-4,5-dihydroisoxazol-5-yl]-2-methylphenyl]-3-iodo-N²-isopropylphthalic diamide.

To 5 ml of a toluene solution containing 0.46 g of 3-iodo-N-isopropylphthalamidic acid was added dropwise 0.37 ml of trifluoroacetic anhydride at room temperature under stirring. After the mixture was stirred at the same temperature for 2 hours, the solvent was removed under reduced pressure, the residue was dissolved in 2.5 ml of acetonitrile, 0.34 g of crude 4-[5-(4-chlorophenyl)-3-methyl-4,5-dihydroisoxazol-5-yl]-2-methylaniline obtained in Step 4 was added to the solution, and stirring was continued at room temperature for 2.5 hours. After completion of the reaction, the precipitated crystals were collected by filtration and washed with a small amount of acetonitrile to obtain 0.36 g of the objective material as white crystals.
Melting point 145.5 to 148.5°C
¹H NMR (CDCl₃, Me₄Si, 300MHz) δ 8.31 (bs, 1H), 7.98 (d, J=8.7Hz, 1 H), 7.94 (d, J=8.1 Hz, 1 H), 7.77 (d, J=7.5Hz, 1 H), 7.1-7.4 (m, 7H), 5.85 (d, J=8.1 Hz, 1 H), 4.15-4.3 (m, 1 H), 3.56 (d, J=16.5Hz, 1 H), 3.43 (d, J=16.5Hz, 1 H), 2.28 (s, 3H), 2.00 (s, 3H), 1.17 (d, J=6.6Hz, 6H).

### Synthetic example 15

N¹-[4-[2-(4-chlorophenyl)-5-trifluoromethyl-4,5-dihydroxazol-5-yl]-2-methylphenyl]-3-iodo-N²-isopropylphthalic diamide (Present compound No. 4-02)
Step 1; Preparation of t-butyl 2-methyl-4-(1-trifluoromethyloxylan-1-yl)carbanilate.

To 50 ml of a chloroform solution containing 3.0 g of t-butyl 2-methyl-4-(1-trifluoromethylethenyl)carbanilate synthesized in Step 1 to Step 2 of Synthetic example 1 was added 3.5 g of 3-chloroperbenzoic acid, and the mixture was stirred at room temperature for 3 days. After completion of the reaction, the reaction mixture was washed with 50 ml of 10% aqueous sodium hydrogen sulfite solution, 50 ml of an aqueous saturated sodium hydrogen carbonate solution, and 50 ml of water in this order. After the organic layer was dried over anhydrous magnesium sulfate, the solvent was removed under reduced pressure to obtain 2.9 g of the objective material as pale yellowish crystals.
Melting point 88.0 to 90.0°C
¹H NMR (CDCl₃, Me₄Si, 300MHz) δ 7.93 (d, J=8.5Hz, 1 H), 7.3-7.4 (m, 2H), 6.33 (bs, 1 H), 3.38 (d, J=5.2Hz, 1 H), 2.89 (d, J=5.2Hz, 1 H), 2.27 (s, 3H), 1.53 (s, 9H).
Step 2; Preparation of t-butyl 4-(2-amino-1-hydroxy-1-trifluoromethylethyl)-2-methylcarbanilate.

To 30 ml of a tetrahydrofuran solution containing 2.9 g of t-butyl 2-methyl-4-(1-trifluoromethyloxylan-1-yl)carbanilate was added 20 ml of a 10% ammonia-ethanol solution, and the mixture was stirred at room temperature for 3 days. After completion of the reaction, the solvent was removed under reduced pressure to obtain 3.1 g of the objective material as pale yellow oil. This product was used in the next step as such without purification.
Step 3; Preparation of t-butyl 4-[2-(4-chlorobenzoylamino)-1-hydroxy-1-trifluoromethylethyl]-2-methylcarbanilate.

To 30 ml of a diethyl ether solution containing 2.0 g of t-butyl 4-(2-amino-1-hydroxy-1-trifluoromethylethyl)-2-methylcarbanilate were added 30 ml of water, 1.25 g of sodium carbonate and 1.25 g of 4-chlorobenzoyl chloride, and the mixture was stirred at room temperature for 3 hours. After completion of the reaction, the organic layer was collected by separation, washed with 50 ml of water, then, dried over anhydrous magnesium sulfate, and the solvent was removed under reduced pressure. The residual solid was recrystallized from diisopropyl ether to obtain 1.8 g of the objective material as colorless crystals.
Melting point 160.0 to 162.0°C
¹H NMR (CDCl₃, Me₄Si, 300MHz) δ 7.85 (d, J=8.8Hz, 1 H), 7.58 (d, J=8.5Hz, 2H), 7.36 (d, J=8.5Hz, 2H), 7.3-7.5 (m, 2H), 6.4-6.6 (m, 1 H), 6.31 (bs, 1 H), 5.52 (bs, 1 H), 4.23 (dd, J=13.0, 6.6Hz, 1 H), 3.87 (dd, J=13.0, 6.6Hz, 1 H), 2.24 (s, 3H), 1.52 (s, 9H).
Step 4; Preparation of t-butyl 4-[2-(4-chlorophenyl)-5-trifluoromethyl-4,5-dihydroxazol-5-yl]-2-methylcarbanilate.

To 10 ml of a pyridine solution containing 0.5 g of t-butyl 4-[2-(4-chlorobenzoylamino)-1-hydroxy-1-trifluoromethylethyl]-2-methylcarbanilate was added dropwise 0.32 g of phosphorus oxychloride, and the mixture was stirred at 60°C for 2 hours. After completion of the reaction, the solvent was removed under reduced pressure, the residue was poured into 50 ml of water, extracted with diethyl ether (50 mlx2), the organic layer was washed with water dried over anhydrous magnesium sulfate, and the solvent was removed under reduced pressure. The residue was purified by silica gel column chromatography eluting with ethyl acetate-hexane (1:4) to obtain 0.35 g of the objective material as colorless resinous substance.
¹H NMR (CDCl₃, Me₄Si, 300MHz) δ 7.98 (d, J=8.8Hz, 2H), 7.96 (d, J=8.5Hz, 1 H), 7.44 (d, J=8.8Hz, 2H), 7.2-7.4 (m, 2H), 6.33 (bs, 1 H), 4.71 (d, J=15.4Hz, 1 H), 4.33 (d, J=15.4Hz, 1 H), 2.28 (s, 3H), 1.53 (s, 9H).
Step 5; Preparation of 4-[2-(4-chlorophenyl)-5-trifluoromethyl-4,5-dihydroxazol-5-yl]-2-methylaniline.

To 0.35 g of t-butyl 4-[2-(4-chlorophenyl)-5-trifluoromethyl-4,5-dihydroxazol-5-yl]-2-methylcarbanilate was added dropwise 5 ml of trifluoroacetic acid under ice-cooling and stirring. After stirring was continued at room temperature for 10 minutes, the solvent was removed under reduced pressure, 50 ml of diethyl ether was added to the remaining oily substance, and the resulting material was washed with 50 ml of an aqueous saturated sodium hydrogen carbonate solution and then 50 ml of water. The organic layer was dried over anhydrous magnesium sulfate, and the solvent was removed under reduced pressure to obtain 0.33 g of the objective material as pale yellowish resinous substance.
¹H NMR (CDCl₃, Me₄Si, 300MHz) δ 7.99 (d, J=8.8Hz, 2H), 7.44 (d, J=8.8Hz, 2H), 7.1-7.3 (m, 2H), 6.69 (d, J=9.1 Hz, 1 H), 4.68 (d, J=15.4Hz, 1 H), 4.34 (d, J=15.4Hz, 1 H), 3.00 (bs, 2H), 2.19 (s, 3H).
Step 6; Preparation of N¹-[4-[2-(4-chlorophenyl)-5-trifluoromethyl-4,5-dihydroxazol-5-yl]-2-methylphenyl]-3-iodo-N²-isopropylphthalic diamide.

To 10 ml of a toluene solution containing 0.4 g of 3-iodo-N-isopropylphthalamidic acid was added 0.4 g of trifluoroacetic anhydride under ice-cooling and stirring, and the mixture was stirred at room temperature for 30 minutes. After the solvent was removed under reduced pressure, the residue was dissolved in 10 ml of tetrahydrofuran, 0.33 g of 4-[2-(4-chlorophenyl)-5-trifluoromethyl-4,5-dihydroxazol-5-yl]-2-methylaniline was added to the solution, and the mixture was stirred at room temperature for 12 hours. After completion of the reaction, the solvent was removed under reduced pressure, and the residual solid was purified by silica gel column chromatography eluting with ethyl acetate-chloroform (1:9) to obtain 0.3 g of the objective material as colorless crystals. Melting point 138.0 to 140.0°C
¹H NMR (CDCl₃, Me₄Si, 300MHz) δ 8.41 (bs, 1 H), 8.18 (d, J=8.5Hz, 1 H), 7.98 (d, J=8.8Hz, 2H), 7.9-8.0 (m, 1 H), 7.46 (d, J=8.8Hz, 2H), 7.1-7.4 (m, 4H), 5.79 (d, J=8.0Hz, 1 H), 4.73 (d, J=15.7Hz, 1 H), 4.34 (d, J=15.7Hz, 1 H), 4.1-4.3 (m, 1 H), 2.37 (s, 3H), 1.13 (d, J=6.1 Hz, 6H).

### Synthetic example 16

N¹-[4-[2-(4-chlorophenyl)-5-trifluoromethyl-4,5-dihydrothiazol-5-yl]-2-methylphenyl]-3-iodo-N²-isopropylphthalic diamide (Present compound No. 4-04)
Step 1; Preparation of 4-[2-(4-chlorophenyl)-5-trifluoromethyl-4,5-dihydrothiazol-5-yl]-2-methylaniline.

To 20 ml of a toluene solution containing 1.0 g of t-butyl 4-[2-(4-chlorobenzoylamino)-1-hydroxy-1-trifluoromethylethyl]-2-methylcarbanilate synthesized in Step 1 to Step 3 of Synthetic example 15 was added 1.0 g of Lawesson's Reagent, and the mixture was stirred under reflux for 1 hour. After completion of the reaction, the mixture was cooled to room temperature by allowing to stand, insoluble materials were separated by filtration, the filtrate was washed with 50 ml of water and dried over anhydrous magnesium sulfate, and the solvent was removed under reduced pressure. The residue was purified by silica gel column chromatography eluting with ethyl acetate-hexane (1:4) to obtain 0.4 g of the objective material as colorless resinous substance.
¹H NMR (CDCl₃, Me₄Si, 300MHz) δ 7.76 (d, J=8.5Hz, 2H), 7.41 (d, J=8.5Hz, 2H), 7.0-7.2 (m, 2H), 6.6-6.7 (m, 1 H), 5.82 (d, J=17.0Hz, 1 H), 4.71 (d, J=17.0Hz, 1 H), 3.73 (bs, 2H), 2.19 (s, 3H).
Step 2; Preparation of N¹-[4-[2-(4-chlorophenyl)-5-trifluoromethyl-4,5-dihydrothiazol-5-yl]-2-methylphenyl]-3-iodo-N²-isooropylphthalic diamide.

To 10 ml of a toluene solution containing 0.4 g of 3-iodo-N-isopropylphthalamidic acid was added 0.4 g of trifluoroacetic anhydride under ice-cooling and stirring, and the mixture was stirred at room temperature for 30 minutes. After the solvent was removed under reduced pressure, the residue was dissolved in 10 ml of acetonitrile, 0.4 g of 4-[2-(4-chlorophenyl)-5-trifluoromethyl-4,5-dihydrothiazol-5-yl]-2-methylaniline was added to the solution, and the mixture was stirred at room temperature for 5 hours. After completion of the reaction, the solvent was removed under reduced pressure, and the residue was purified by silica gel column chromatography eluting with ethyl acetate-hexane (2:3) to obtain 0.35 g of the objective material as colorless crystals. Melting point 121.0 to 123.0°C
¹H NMR (CDCl₃, Me₄Si, 300MHz) δ 8.41 (bs, 1 H), 8.27 (d, J=6.6Hz, 1 H), 7.97 (d, J=6.6Hz, 1 H), 7.78 (d, J=8.5Hz, 2H), 7.7-7.9 (m, 1 H), 7.42 (d, J=8.5Hz, 2H), 7.1-7.3 (m, 3H), 5.86 (d, J=8.2Hz, 1 H), 5.23 (d, J=17.0Hz, 1 H), 4.73 (d, J=17.0Hz, 1 H), 4.1-4.2 (m, 1 H), 2.35 (s, 3H), 1.15 (d, J=6.0Hz, 6H).

### Synthetic example 17

N¹-[4-[1-(4-chlorophenyl)-3-methyl-4,5-dihydropyrazol-5-yl]-2-methylphenyl]-3-iodo-N²-isopropylphthalic diamide (Present compound No. 6-02)
Step 1; Preparation of 4-(3-oxo-1-butenyl)-2-methylcarbanilate-t-butyl.

To 20 ml of a N-methylpyrrolidone solution containing 3.0 g of t-butyl 4-iodo-2-methylcarbanilate and 1.26 g of methyl vinyl ketone were added 0.126 g of dichlorobistriphenylphosphine palladium and 1.51 g of sodium hydrogen carbonate, and the mixture was stirred in an autoclave at 130°C for 90 minutes. Then, to this reaction mixture were additionally added 1.26 g of methyl vinyl ketone, 0.126 g of palladium dichlorobistriphenylphosphine and 1.51 g of sodium hydrogen carbonate, and stirring was continued at the same temperature for further 90 minutes. After completion of the reaction, the reaction mixture was poured into 100 ml of water and extracted with 100 ml of diethyl ether, the organic layer was dried over anhydrous magnesium sulfate, and the solvent was removed under reduced pressure. The residue was purified by silica gel column chromatography eluting with ethyl acetate-hexane (1:9 to 2:3) to obtain 2.0 g of the objective material as brown solid.
Melting point 116.5 to 119.0°C
¹H NMR (CDCl₃, Me₄Si, 300MHz) δ 7.99 (d, J=8.4Hz, 1 H), 7.44 (d, J=16.5Hz, 1 H), 7.39 (d, J=8.4Hz, 1 H), 7.34 (s, 1 H), 6.63 (d, J=16.5Hz, 1 H), 6.41 (bs, 1 H), 2.36 (s, 3H), 2.27 (s, 3H), 1.54 (s, 9H).
Step 2; Preparation of 4-[1-(4-chlorophenyl)-3-methyl-4,5-dihydropyrazol-5-yl]-2-methylaniline.

To 4 ml of a toluene solution containing 0.5 g of t-butyl 4-(3-oxo-1-butenyl)-2-methylcarbanilate were added 0.36 g of 4-chlorophenylhydrazine hydrochloride and 0.31 g of p-toluene sulfonic acid monohydrate, and the mixture was stirred under reflux for 1 hour. After completion of the reaction, the mixture was cooled up to room temperature by allowing to stand, to the reaction mixture was added 50 ml of an aqueous saturated sodium hydrogen carbonate solution, the resulting mixture was extracted with 50 ml of ethyl acetate, the organic layer was dried over anhydrous magnesium sulfate, and the solvent was removed under reduced pressure. The remaining brown solid was washed with 3 ml of diisopropyl ether and 1 ml of diethyl ether to obtain 0.43 g of the objective material as brown solid.
Melting point 180.0 to 184.5°C
¹H NMR (CDCl₃, Me₄Si, 300MHz) δ 7.06 (d, J=9.0Hz, 2H), 6.9-7.0 (m, 2H), 6.85 (d, J=9.0Hz, 2H), 6.62 (d, J=8.1 Hz, 1 H), 4.84 (dd, J=11.7, 8.1 Hz, 1 H), 3.66 (bs, 2H), 3.35 (dd, J=17.7, 11.7Hz, 1 H), 2.68 (dd, J=17.7, 8.1Hz, 1 H), 2.13 (s, 3H), 2.05 (s, 3H).
Step 3; Preparation of N¹-[4-[1-(4-chlorophenyl)-3-methyl-4,5-dihydropyrazol-5-yl]-2-methylphenyl]-3-iodo-N²-isopropylphthalic diamide.

To 5 ml of a toluene solution containing 0.27 g of 3-iodo-N-isopropylphthalamidic acid was added 0.2 g of trifluoroacetic anhydride at room temperature under stirring, and the mixture was stirred at the same temperature for 1 hour. After the solvent was removed under reduced pressure, the residue was dissolved in 4 ml of acetonitrile, 0.2 g of 4-[1-(4-chlorophenyl)-3-methyl-4,5-dihydropyrazol-5-yl]-2-methylaniline was added to the solution, and the mixture was stirred at room temperature for 17 hours. After completion of the reaction, the precipitated solid was collected by filtration, and washed with a small amount of acetonitrile to obtain 0.3 g of the objective material as white crystals.
Melting point 222.5 to 224.5°C
¹H NMR (CDCl₃, Me₄Si, 300MHz) δ 8.28 (bs, 1 H), 7.9-8.0 (m, 2H), 7.77 (d, J=7.8Hz, 1 H), 7.0-7.25 (m, 5H), 6.83 (d, J=9.3Hz, 2H), 5.85 (d, J=8.1 Hz, 1 H), 4.91 (dd, J=12.0, 8.1 Hz, 1 H), 4.15-4.3 (m, 1 H), 3.40 (dd, J=17.7, 12.0Hz, 1 H), 2.71 (dd, J=17.7, 8.1 Hz, 1 H), 2.27 (s, 3H), 2.06 (s, 3H), 1.17 (dd, J=6.6, 3.0Hz, 6H).

### Synthetic example 18

N¹-[4-[3-(4-chlorophenyl)-1-methyl-5-trifluoromethyl-4,5-dihydropyrazol-5-yl]-2-methylphenyl]-3-iodo-N²-isopropylphthalic diamide (Present compound No. 6-04)
Step 1; Preparation of 4-chlorophenacyltriphenyl phosphonium bromide.

To 30 ml of a tetrahydrofuran solution containing 5.0 g of 4-chlorophenacyl bromide was added 5.6 g of triphenylphosphine, and the mixture was stirred at 50°C for 3 hours. After completion of the reaction, the solid was filtered and washed with tetrahydrofuran to obtain 9.0 g of the objective material as white crystals.
Melting point >255.0°C (decomposed)
¹H NMR (CDCl₃, Me₄Si, 300MHz) δ 8.38 (d, J=8.5Hz, 2H), 7.6-8.1 (m, 15H), 7.49 (d, J=8.5Hz, 2H), 6.39 (d, J=12.4Hz, 2H).
Step 2; Preparation of 1-(4-chlorophenyl)-4-trifluoro-2-triphenylphosphranyliden-1,3-butanedione.

To a mixture of 5.0 g of 4-chlorophenacyltriphenyl phosphonium bromide and 1.2 g of triethylamine in 50 ml of chloroform was added dropwise 2.4 g of trifluoroacetic anhydride under ice-cooling and stirring, and after completion of the dropwise addition, the mixture was stirred at room temperature for 24 hours. After completion of the reaction, the mixture was washed with 50 ml of water and dried over anhydrous magnesium sulfate, and the solvent was removed under reduced pressure. The residue was purified by silica gel column chromatography eluting with ethyl acetate-chloroform (1:4) to obtain 3.8 g of the objective material as pale yellowish crystals.
Melting point 174.0 to 176.0°C
¹H NMR (CDCl₃, Me₄Si, 300MHz) δ 7.78 (d, J=8.5Hz, 2H), 7.4-7.7 (m, 15H), 7.33 (d, J=8.5Hz, 2H).
Step 3; Preparation of t-butyl 4-[3-(4-chlorophenyl)-3-oxo-1-trifluoromethyl-1-propenyl]-2-methylcarbanilate.

Under nitrogen atmosphere, to 100 ml of a diethyl ether solution containing 3.0 g of t-butyl 4-iodo-2-methylcarbanilate was added dropwise 13.5 ml of n-butyl lithium (1.5M hexane solution) at -10°C and under stirring, and after completion of the dropwise addition, the mixture was stirred at the same temperature for 20 minutes. Then, this reaction mixture was cooled to -78°C, 4.6 g of 1-(4-chlorophenyl)-4-trifluoro-2-triphenylphosphoraniliden-1,3-butanedione was added to the mixture, warmed up to 0°C over 3 hours, added dropwise 50 ml of 2N hydrochloric acid at 0°C to the mixture, and further stirring was continued vigorously at room temperature for 3 hours. After completion of the reaction, insoluble materials were separated by filtration, the organic layer of the filtrate was collected by separation, washed with 50 ml of water and dried over anhydrous magnesium sulfate, and the solvent was removed under reduced pressure. The residue was purified by silica gel column chromatography eluting with ethyl acetate-hexane (1:4) to obtain 1.3 g of the objective material as pale yellowish crystals. Melting point 72.0 to 74.0°C
¹H NMR (CDCl₃, Me₄Si, 300MHz) δ 7.86 (d, J=8.0Hz, 1 H), 7.74 (d, J=8.5Hz, 2H), 7.35 (d, J=8.5Hz, 2H), 7.0-7.2 (m, 3H), 6.26 (bs, 1 H), 2.15 (s, 3H), 1.49 (s, 9H).
Step 4; Preparation of t-butyl 4-[3-(4-chlorophenyl)-1-methyl-5-trifluoromethyl-4,5-dihydropyrazol-5-yl]-2-methylcarbanilate.

To 50 ml of an ethanol solution containing 0.8 g of t-butyl 4-[3-(4-chlorophenyl)-3-oxo-1-trifluoromethyl-1-propenyl]-2-methylcarbanilate was added 1.0 g of methylhydrazine, and the mixture was stirred at 45°C for 4 hours. After completion of the reaction, the reaction mixture was poured into 300 ml of ice-water and extracted with diethyl ether (100 mlx3), the organic layer was dehydrated by saturated brine and then dried over anhydrous magnesium sulfate in this order, and the solvent was removed under reduced pressure. The residue was purified by silica gel column chromatography eluting with ethyl acetate-hexane (1:9) to obtain 0.5 g of the objective material as colorless resinous substance.
¹H NMR (CDCl₃, Me₄Si, 300MHz) δ 7.88 (d, J=8.2Hz, 1 H), 7.53 (d, J=8.5Hz, 2H), 7.33 (d, J=8.5Hz, 2H), 7.2-7.5 (m, 2H), 6.30 (bs, 1 H), 3.82 (d, J=17.6Hz, 1 H), 3.39 (d, J=17.6Hz, 1 H), 2.98 (s, 3H), 2.26 (s, 3H), 1.52 (s, 9H).
Step 5; Preparation of 4-[3-(4-chlorophenyl)-1-methyl-5-trifluoromethyl-4,5-dihydropyrazol-5-yl]-2-methylaniline.

To 0.4 g of t-butyl 4-[3-(4-chlorophenyl)-1-methyl-5-trifluoromethyl-4,5-dihydropyrazol-5-yl]-2-methylcarbanilate was added dropwise5 ml of trifluoroacetic acid under ice-cooling and stirring. After stirring was continued at room temperature for 10 minutes, the solvent was removed under reduced pressure, 50 ml of diethyl ether was added to the remaining oily substance, and the mixture was washed with 50 ml of an aqueous saturated sodium hydrogen carbonate solution and then with 50 ml of water. After the organic layer was dried over anhydrous magnesium sulfate, the solvent was removed under reduced pressure to obtain 0.35 g of the objective material as colorless resinous substance.
¹H NMR (CDCl₃, Me₄Si, 300MHz) δ 8.45 (bs, 2H), 7.49 (d, J=8.5Hz, 2H), 7.3-7.5 (m, 3H), 7.35 (d, J=8.5Hz, 2H), 3.89 (d, J=17.6Hz, 1 H), 3.37 (d, J=17.6Hz, 1 H), 3.03 (s, 3H), 2.42 (s, 3H).
Step 6; Preparation of N¹-[4-[3-(4-chlorophenyl)-1-methyl-5-trifluoromethyl-4,5-dihydropyrazol-5-yl]-2-methylphenyl]-3-iodo-N²-isopropylphthalic diamide.

To 10 ml of a toluene solution containing 0.4 g of 3-iodo-N-isopropylphthalamidic acid was added 0.4 g of trifluoroacetic anhydride under ice-cooling and stirring, and the mixture was stirred at room temperature for 30 minutes. After the solvent was removed under reduced pressure, the residue was dissolved in 10 ml of acetonitrile, 0.3 g of 4-[3-(4-chlorophenyl)-1-methyl-5-trifluoromethyl-4,5-dihydropyrazol-5-yl]-2-methylaniline was added to the solution, and the mixture was stirred at room temperature for 5 hours. After completion of the reaction, the solvent was removed under reduced pressure, and the residue was purified by silica gel column chromatography eluting with ethyl acetate- chloroform (3:7) to obtain 0.3 g of the objective material as colorless crystals.
Melting point 90.0 to 92.0°C
¹H NMR (CDCl₃, Me₄Si, 300MHz) δ 8.38 (bs, 1 H), 8.09 (d, J=8.4Hz, 1 H), 7.96 (d, J=8.0Hz, 1 H), 7.80 (d, J=8.4Hz, 1 H), 7.53 (d, J=8.5Hz, 2H), 7.34 (d, J=8.5Hz, 2H), 7.1-7.4 (m, 3H), 5.85 (d, J=8.2Hz, 1 H), 4.1-4.3 (m, 1H), 3.85 (d, J=17.6Hz, 1 H), 3.42 (d, J=17.6Hz, 1 H), 2.99 (s, 3H), 2.33 (s, 3H), 1.17 (d, J=6.6Hz, 6H).

### Synthetic example 19

3-lodo-N²-isopropyl-N¹-[2-methyl-4-(4-phenyl-2-trifluoromethyl-2,5-dihydroxazo l- 2-yl)phenyl]phthalic diamide (Present compound No. 5-01)
Step 1; Preparation of t-butyl 2-methyl-4-trifluoroacetylcarbanilate.

Under nitrogen atmosphere, to 300 ml of a diethyl ether solution containing 10.0 g of t-butyl 4-iodo-2-methylcarbanilate was added dropwise 45.0 ml of n-butyllithium (1.5M) at -10°C and under stirring, and after completion of the dropwise addition, the mixture was stirred at the same temperature for 15 minutes. Then, this reaction mixture was cooled to -78°C, 9.5 g of trifluoroethyl acetate was added dropwise to the mixture, and after completion of the dropwise addition, stirring was continued at the same temperature for further 1 hour. Then, the mixture was warmed up to -10°C, 100 ml of 2N hydrochloric acid was added to the mixture and the resulting mixture was vigorously stirred, the organic layer was collected by separation, dehydrated by saturated brine and then dried over anhydrous magnesium sulfate in this order, and the solvent was removed under reduced pressure. The residue was purified by silica gel column chromatography eluting with diethyl ether-hexane (1:4) to obtain 3.2 g of the objective material as white crystals.
Melting point 85.0 to 87.0°C
¹H NMR (CDCl₃, Me₄Si, 300MHz) δ 8.25 (d, J=8.8Hz, 1 H), 7.8-8.0 (m, 2H), 6.62 (bs, 1 H), 2.32 (s, 3H), 1.55 (s, 9H).
Step 2; Preparation of t-butyl 2-methyl-4-(4-phenyl-2-trifluoromethyl-2,3,4,5-tetrahydroxazol-2-yl)carbanilate.

To 20 ml of a xylene solution containing 2.0 g of t-butyl 2-methyl-4-trifluoroacetylcarbanilate and 0.92 g of 2-amino-2-phenylethanol was added 0.16 g of pyridinium- p-toluene sulfonate, and while removing the formed water by using a Dean-Stark tube, the mixture was stirred under reflux for 8 hours. After completion of the reaction, the reaction mixture was poured into 100 ml of water and extracted with ethyl acetate (100 mlx2), the organic layer was washed with water and dried over anhydrous magnesium sulfate, and the solvent was removed under reduced pressure. The residue was purified by silica gel column chromatography eluting with ethyl acetate-hexane (1:5) to obtain 0.3 g of the objective material as reddish oily substance.
¹H NMR (CDCl₃, Me₄Si, 300MHz) δ 7.92 (d, J=8.4Hz, 1 H), 7.25-7.55 (m, 7H), 6.32 (bs, 1 H), 4.6-4.75 (m, 1 H), 4.53 (t, J=7.2Hz, 1 H), 4.35-4.45 (m, 1 H), 3.75 (t, J=8.4Hz, 1 H), 2.28 (s, 3H), 1.53 (s, 9H).
Step 3; Preparation of t-butyl 2-methyl-4-(4-phenyl-2-trifluoromethyl-2,5-dihydroxazol-2-yl)carbanilate.

To 10 ml of a diethyl ether solution containing 0.2 g of t-butyl 2-methyl-4-(4-phenyl-2-trifluoromethyl-2,3,4,5-tetrahydroxazol-2-yl)carbanilate were added 0.08 g of potassium hydrogen carbonate and 0.1 g of t-butyl hypochlorite under ice-cooling and stirring. The reaction mixture was warmed to room temperature, and after stirring was continued at the same temperature for further 2 hours, insoluble materials were removed by Celite filtration, and then, the solvent was removed under reduced pressure. The residue was dissolved in 5 ml of diethyl ether, 0.07 g of potassium dioxide and 0.01 g of 18-crown-6-ether were added to the solution, the mixture was stirred at room temperature for 2 hours, and after completion of the reaction, insoluble materials were removed by Celite filtration, and the solvent was removed under reduced pressure. The residue was purified by silica gel column chromatography eluting with ethyl acetate-hexane (1:5) to obtain 0.15 g of the objective material as reddish oily substance.
¹H NMR (CDCl₃, Me₄Si, 300MHz) δ 7.91 (d, J=8.4Hz, 1 H), 7.8-7.85 (m, 2H), 7.4-7.65 (m, 5H), 6.32 (bs, 1 H), 5.30 (d, J=13.7Hz, 1 H), 5.14 (d, J=13.6Hz, 1 H), 2.28 (s, 3H), 1.52 (s, 9H).
Step 4; Preparation of 2-methyl-4-(4-phenyl-2-trifluoromethyl-2,5-dihydroxazol-2-yl)-aniline.

To 0.12 g of t-butyl 2-methyl-4-(4-phenyl-2-trifluoromethyl-2,5-dihydroxazol-2-yl)carbanilate was added dropwise 3 ml of trifluoroacetic acid under ice-cooling and stirring. After stirring was continued at room temperature for 30 minutes, the solvent was removed under reduced pressure, to the remaining oily substance was added 50 ml of ethyl acetate, and the mixture was washed with 50 ml of an aqueous saturated sodium hydrogen carbonate solution and then with 50 ml of water. After drying over anhydrous sodium sulfate, the solvent was removed under reduced pressure to obtain 0.09 g of the objective material as brownish oily substance.
¹H NMR (CDCl₃, Me₄Si, 300MHz) δ 7.8-7.85 (m, 2H), 7.4-7.55 (m, 5H), 6.68 (d, J=8.1 Hz, 1 H), 5.28 (d, J=13.6Hz, 1 H), 5.14 (d, J=13.7Hz, 1 H), 3.70 (bs, 2H), 2.19 (s, 3H).
Step 5; Preparation of 3-iodo-N²-isopropyl-N¹-[2-methyl-4-(4-phenyl-2-trifluoromethyl-2,5-dihydroxazol-2-yl)phenyl]phthalic diamide.

To 10 ml of a toluene solution containing 0.14 g of 3-iodo-N-isopropylphthalamidic acid was added 0.13 g of trifluoroacetic anhydride under ice-cooling and stirring, and the mixture was stirred at room temperature for 30 minutes. After the solvent was removed under reduced pressure, the residue was dissolved in 10 ml of acetonitrile at room temperature under stirring, 0.09 g of 2-methyl-4-(4-phenyl-2-trifluoromethyl-2,5-dihydroxazol-2-yl)aniline was added to the solution, and stirring was continued at the same temperature for 12 hours. After completion of the reaction, precipitated solid was collected by filtration and washed with 3 ml of acetonitrile to obtain 0.06 g of the objective material as white crystals.
Melting point 222.0 to 223.0° C
¹H NMR (CDCl₃, Me₄Si, 300MHz) δ 8.35 (bs, 1 H), 8.12 (d, J=8.2Hz, 1 H), 7.96 (d, J=7.9Hz, 1 H), 7.8-7.9 (m, 2H), 7.79 (d, J=7.7Hz, 1 H), 7.6-7.7 (m, 2H), 7.45-7.6 (m, 3H), 7.20 (t, J=7.9Hz, 1 H), 5.83 (d, J=8.2Hz, 1 H), 5.32 (d, J=13.7Hz, 1 H), 5.17 (d, J=13.6Hz, 1 H), 4.15-4.25 (m, 1 H), 2.36 (s, 3H), 1.17 (d, J=6.6Hz, 6H).

### Synthetic example 20

N¹-[4-[3-(4-fluorophenyl)-5-trifluoromethyl-1,4,2-dioxazolin-5-yl]-2-methylpheny l]- 3-iodo-N²-isopropylphthalic diamide (Present compound No. 4-05) Step 1; Preparation of t-butyl 4-[3-(4-fluorophenyl)-5-trifluoromethyl-1,4,2-dioxazolin-5-yl]-2-methylcarbanilate.

To 30 ml of a N,N-dimethylformamide solution containing 0.5 g of t-butyl 2-methyl-4-trifluoroacetylcarbanilate synthesized in Step 1 of Synthetic example 19 and 1.2 g of 4-fluorophenylhydroximic acid chloride (synthesized from 4-fluorophenylaldoxime in the same manner as in Step 1 of Synthetic example 5) was added dropwise 0.7 g of triethylamine at room temperature under stirring, and after completion of the dropwise addition, stirring was continued at the same temperature for further 20 hours. After completion of the reaction, the reaction mixture was diluted with 80 ml of ethyl acetate and washed with water (50 mlx2), then, the organic layer was dehydrated by saturated brine and then dried over anhydrous magnesium sulfate in this order, and the solvent was removed under reduced pressure. The residue was purified by silica gel column chromatography eluting with ethyl acetate-hexane (1:3) to obtain 1.3 g of the objective material as pale yellowish resinous substance.
Step 2; Preparation of 4-[3-(4-fluorophenyl)-5-trifluoromethyl-1,4,2-dioxazolin-5-yl]-2-methylaniline.

To 1.3 g of t-butyl 4-[3-(4-fluorophenyl)-5-trifluoromethyl-1,4,2-dioxazolin-5-yl]-2-methylcarbanilate was added dropwise 5 ml of trifluoroacetic acid at room temperature under stirring. After stirring was continued at room temperature for 30 minutes, the solvent was removed under reduced pressure, to the remaining oily substance was added 50 ml of ethyl acetate, and the resulting mixture was washed with 30 ml of an aqueous saturated sodium hydrogen carbonate solution and then with 30 ml of water, dehydrated by saturated brine and then dried over anhydrous magnesium sulfate in this order, and the solvent was removed under reduced pressure. The residue was purified by silica gel column chromatography eluting with ethyl acetate-hexane (1:4) to obtain 0.4 g of the objective material as pale yellowish crystals.
Melting point 78.0 to 83.0°C
¹H NMR (CDCl₃, Me₄Si, 300MHz) δ 7.85 (dd, J=9.0, 5.4Hz, 2H), 7.3-7.35 (m, 2H), 7.15 (dd, J=9.0, 8.4Hz, 2H), 6.71 (d, J=9.0Hz, 1 H), 3.84 (bs, 2H), 2.20 (s, 3H).
Step 3; Preparation of N¹-[4-[3-(4-fluorophenyl)-5-trifluoromethyl-1,4,2-dioxazolin-5-yl]-2-methylphenyl]-3-iodo-N²-isopropylphthalic diamide.

To 30 ml of a toluene solution containing 0.35 g of 3-iodo-N-isopropylphthalamidic acid was added 0.28 g of trifluoroacetic anhydride at room temperature under stirring, and the mixture was stirred at room temperature for 1 hour. After the solvent was removed under reduced pressure, the residue was dissolved in 1 ml of acetonitrile, 3 ml of an acetonitrile solution containing 0.34 g of 4-[3-(4-fluorophenyl)-5-trifluoromethyl-1,4,2-dioxazolin-5-yl]-2-methylaniline was added to the solution at room temperature under stirring, and stirring was continued at the same temperature for 2 hours. After completion of the reaction, the solvent was removed under reduced pressure, and the residue was purified by silica gel column chromatography eluting with ethyl acetate-chloroform (1:3) to obtain 0.55 g of the objective material as white glass-state solid.
Melting point 99.0 to 112.0°C
¹H NMR (CDCl₃, Me₄Si, 300MHz) δ 8.49 (bs, 1 H), 8.24 (d, J=8.4Hz, 1 H), 7.96 (d, J=7.8Hz, 1 H), 7.87 (dd, J=8.7, 5.4Hz, 2H), 7.78 (d, J=7.5Hz, 1 H), 7.5-7.55 (m, 2H), 7.1-7.25 (m, 3H), 5.94 (d, J=7.8Hz, 1 H), 4.1-4.3 (m, 1 H), 2.38 (s, 3H), 1.16 (d, J=6.6Hz, 6H).

### Synthetic example 21

N¹-[4-[4-(4-chlorophenyl)-2-trifluoromethyl-1,3-dioxolan-2-yl]-2-methylphenyl]-3-iodo-N²-isopropylphthalic diamide (Present compound No. 7-03)
Step 1; Preparation of 2-chloro-1-(4-chlorophenyl)ethanol.

To 50 ml of an ethanol solution containing 3.0 g of 4-chlorophenacyl chloride was added 1.5 g of sodium hydrogen carbonate under ice-cooling and stirring, then, 3 ml of an aqueous suspension containing 0.3 g of sodium borohydride was added dropwise to the mixture, and after completion of the dropwise addition, stirring was continued at the same temperature for further 1 hour. After completion of the reaction, the reaction mixture was carefully poured into 100 ml of 1 N hydrochloric acid and extracted with ethyl acetate (100 mlx2), the organic layer was washed with water and dried over anhydrous sodium sulfate, and the solvent was removed under reduced pressure to obtain 2.5 g of the objective material as pale yellowish oily substance.
¹H NMR (CDCl₃, Me₄Si, 300MHz) δ 7.3-7.4 (m, 4H), 4.88 (dd, J=8.6, 3.3Hz, 1 H), 3.72 (dd, J=11.4, 3.5Hz, 1 H), 3.60 (dd, J=11.2, 8.6Hz, 1 H), 2.70 (bs, 1 H).
Step 2; Preparation of t-butyl 4-[4-(4-chlorophenyl)-2-trifluoromethyl-1,3-dioxolan-2-yl]-2-methylcarbanilate.

To 5 ml of a dimethylsulfoxide solution containing 0.6 g of t-butyl 2-methyl-4-trifluoroacetylcarbanilate synthesized in Step 1 of Synthetic example 19 and 0.38 g of 2-chloro-1-(4-chlorophenyl)ethanol was carefully added 0.09 g of 60% oily sodium hydride at 10°C and under stirring, and the mixture was stirred at room temperature for 12 hours. After completion of the reaction, the reaction mixture was poured into 100 ml of water and extracted with ethyl acetate (100 mlx2), the organic layer was washed with water and dried over anhydrous sodium sulfate, and the solvent was removed under reduced pressure. The residue was purified by silica gel column chromatography eluting with ethyl acetate- hexane (1:8) to obtain 0.57 g of the objective material as yellowish oily substance.
¹H NMR (CDCl₃, Me₄Si, 300MHz) δ 7.97 (d, J=8.4Hz, 1 H), 7.52 (dd, J=9.5, 1.8Hz, 1 H), 7.44 (bs, 1 H), 7.15-7.35 (m, 4H), 6.35 (bs, 1 H), 5.40 (dd, J=8.5, 6.4Hz, 1 H), 4.57 (dd, J=8.1, 6.0Hz, 1 H), 3.68 (t, J=8.4Hz, 1 H), 2.29 (s, 3H), 1.54 (s, 9H).
Step 3; Preparation of 4-[4-(4-chlorophenyl)-2-trifluoromethyl-1,3-dioxolan-2-yl]-2-methylaniline.

To 0.5 g of t-butyl 4-[4-(4-chlorophenyl)-2-trifluoromethyl-1,3-dioxolan-2-yl]-2-methylcarbanilate was added dropwise 5 ml of trifluoroacetic acid under ice-cooling and stirring. After stirring was continued at room temperature for 30 minutes, the solvent was removed under reduced pressure, to the remaining oily substance was added 50 ml of ethyl acetate, the resulting mixture was washed with 50 ml of an aqueous saturated sodium hydrogen carbonate solution and then with 50 ml of water and dried over anhydrous sodium sulfate, and the solvent was removed under reduced pressure. The residue was purified by silica gel column chromatography eluting with ethyl acetate-hexane (1:3) to obtain 0.25 g of the objective material as brownish oily substance.
¹H NMR (CDCl₃, Me₄Si, 300MHz) δ 7.3-7.4 (m, 2H), 7.15-7.35 (m, 4H), 6.70 (d, J=8.8Hz, 1 H), 5.39 (dd, J=8.4, 6.4Hz, 1 H), 4.55 (dd, J=8.0, 6.4Hz, 1 H), 3.76 (bs, 2H), 3.69 (t, J=8.4Hz, 1 H), 2.20 (s, 3H).
Step 4; Preparation of N¹-[4-[4-(4-chlorophenyl)-2-trifluoromethyl-1,3-dioxolan-2-yl]-2-methylphenyl]-3-iodo-N²-isopropylphthalic diamide.

To 10 ml of a toluene solution containing 0.14 g of 3-iodo-N-isopropylphthalamidic acid was added 0.13 g of trifluoroacetic anhydride under ice-cooling and stirring, and the mixture was stirred at room temperature for 30 minutes. After the solvent was removed under reduced pressure, the residue was dissolved in 10 ml of acetonitrile, 0.1 g of 4-[4-(4-chlorophenyl)-2-trifluoromethyl-1,3-dioxolan-2-yl]-2-methylaniline was added to the solution at room temperature under stirring, and stirring was continued at the same temperature for 12 hours. After completion of the reaction, the solvent was removed under reduced pressure, and the residue was purified by silica gel column chromatography eluting with ethyl acetate-hexane (1:2) to obtain 0.14 g of the objective material as white crystals.
Melting point 112.0 to 114.0°C
¹H NMR (CDCl₃, Me₄Si, 300MHz) δ 8.38 (bs, 1 H), 8.17 (d, J=8.8Hz, 1 H), 7.99 (d, J=7.9Hz, 1 H), 7.83 (d, J=7.5Hz, 1 H), 7.55 (d, J=8.6Hz, 1 H), 7.50 (bs, 1 H), 7.15-7.35 (m, 5H), 5.7-5.9 (m, 1 H), 5.42 (dd, J=8.1, 7.1 Hz, 1 H), 4.59 (dd, J=7.9, 7.1 Hz, 1 H), 4.2-4.35 (m, 1 H), 3.70 (t, J=8.4Hz, 1 H), 2.37 (s, 3H), 1.19 (d, J=6.6Hz, 6H).

### Synthetic example 22

3-Iodo-N²-isopropyl-N¹-[2-methyl-4-(2-phenyl-4-trifluoromethyl-3,4-dihydro-2H-pyrrol-4-yl)phenyl]phthalic diamide (Present compound No. 7-04)
Step 1; Preparation of t-butyl 2-methyl-4-(2-phenyl-4-trifluoromethyl-3,4-dihydro-2H -pyrrol-4-yl)carbanilate.

To 50 ml of a toluene solution containing 3.0 g of t-butyl 2-methyl-4-(1-trifluoromethylethenyl)carbanilate synthesized in Step 1 to Step 2 of Synthetic example 1 were added 0.78 g of benzyl isocyanide and 0.03 g of cuprous oxide, and the mixture was stirred under reflux for 10 hours. After completion of the reaction, the reaction mixture was poured into 100 ml of water and extracted with ethyl acetate (100 mlx2), the organic layer was washed with water and dried over anhydrous sodium sulfate, and the solvent was removed under reduced pressure. The residue was purified by silica gel column chromatography eluting with ethyl acetate-hexane (1:5) to obtain 1.8 g of the objective material (diastereomer mixture) as reddish brown resinous substance.
¹H NMR (CDCl₃, Me₄Si, 300MHz) δ 8.05 and 8.04 (d, J=12.3Hz, 1 H), 7.85-7.95 (m, 1H), 7.15-7.4 (m, 7H), 6.30 (d, J=9.2Hz, 1 H), 5.40 and 5.00 (td, J=8.0, 2.8Hz, 1H), 3.19 and 2.83 (dd, J=13.9, 7.9Hz, 1 H), 2.1-2.4 (m, 1 H), 2.28 and 2.25 (s, 3H), 1.53 and 1.52 (s, 9H).
Step 2; Preparation of 2-methyl-4-(2-phenyl-4-trifluoromethyl-3,4-dihydro-2H-pyrrol-4-yl)aniline.

To 0.7 g of t-butyl 2-methyl-4-(2-phenyl-4-trifluoromethyl-3,4-dihydro-2H-pyrrol-4-yl)carbanilate was added dropwise 10 ml of trifluoroacetic acid under ice-cooling and stirring. After stirring was continued at room temperature for 1 hour, the solvent was removed under reduced pressure, to the remaining oily substance was added 50 ml of ethyl acetate, the resulting mixture was washed with 50 ml of an aqueous saturated sodium hydrogen carbonate solution and then with 50 ml of water, dried over anhydrous sodium sulfate, and the solvent was removed under reduced pressure. The residue was purified by silica gel column chromatography eluting with ethyl acetate-hexane (1:2) to obtain 0.26 g of the objective material as reddish brown resinous substance.
¹H NMR (CDCl₃, Me₄Si, 300MHz) δ 8.05 (d, J=3.1 Hz, 2H), 7.25-7.4 (m, 4H), 7.05-7.1 (m, 2H), 6.66 (d, J=9.0Hz, 1 H), 4.95-5.05 (m, 1 H), 3.71 (bs, 2H), 2.83 (dd, J=13.2, 7.0Hz, 1 H), 2.30 (dd, J=13.2, 9.3Hz, 1 H), 2.17 (s, 3H).
Step 3; Preparation of 3-iodo-N²-isopropyl-N¹-[2-methyl-4-(2-phenyl-4-trifluoromethyl-3,4-dihydro-2H-pyrrol-4-yl)phenyl]phthalic diamide.

To 15 ml of a toluene solution containing 0.24 g of 3-iodo-N-isopropylphthalamidic acid was added 0.23 g of trifluoroacetic anhydride under ice-cooling and stirring, and the mixture was stirred at room temperature for 1 hour. After the solvent was removed under reduced pressure, the residue was dissolved in 15 ml of acetonitrile, 0.15 g of 2-methyl- 4-(2-phenyl-4-trifluoromethyl-3,4-dihydro-2H-pyrrol-4-yl)aniline was added to the solution at room temperature under stirring, and stirring was continued at the same temperature for 12 hours. After completion of the reaction, the solvent was removed under reduced pressure, and the residue was purified by silica gel column chromatography eluting with ethyl acetate-hexane (1:1) to obtain 0.09 g of the objective material as white crystals.
Melting point 118.0 to 120.0°C
¹H NMR (CDCl₃, Me₄Si, 300MHz) δ 8.46 (bs, 1 H), 8.05-8.1 (m, 2H), 7.95 (d, J=7.9Hz, 1 H), 7.76 (d, J=7.7Hz, 1 H), 7.15-7.4 (m, 8H), 6.10 (d, J=7.5Hz, 1 H), 4.95-5.05 (m, 1 H), 4.2-4.3 (m, 1 H), 2.86 (dd, J=13.2, 7.1 Hz, 1 H), 2.3-2.4 (m, 1 H), 2.35 (s, 3H), 1.18 (d, J=6.6Hz, 6H).

### Synthetic example 23

N¹-[4-(3-cyano-2-phenyl-5-trifluoromethyl-4,5-dihydrofuran-5-yl)-2-methylphen yl]-3-iodo-N²-isopropylphthalic diamide (Present compound No. 4-01)
Step 1; Preparation of t-butyl 4-(3-cyano-2-phenyl-5-trifluoromethyl-4,5-dihydrofuran-5-yl)-2-methylcarbanilate.

To 45 ml of an acetic acid solution containing 1.0 g of t-butyl 2-methyl-4-(1-trifluoromethylethenyl)carbanilate synthesized in Step 1 to Step 2 of Synthetic example 1 and 0.96 g of benzoylacetonitrile was carefully added 2.67 g of manganese triacetate dehydrate at 100°C and under stirring, and the mixture was stirred under reflux for futher 2 hours. After completion of the reaction, the solvent was removed under reduced pressure, the residue was poured into 100 ml of water and extracted with ethyl acetate (100 mlx2), the organic layer was washed with water and dried over anhydrous sodium sulfate, and the solvent was removed under reduced pressure. The residue was purified by silica gel column chromatography eluting with ethyl acetate-hexane (1:3) to obtain 0.35 g of the objective material as pale yellowish crystals.
Melting point 46.0 to 49.0°C
¹H NMR (CDCl₃, Me₄Si, 300MHz) δ 8.0-8.05 (m, 2H), 7.98 (d, J=8.6Hz, 1 H), 7.45-7.6 (m, 3H), 7.36 (d, J=8.6Hz, 1 H), 7.31 (bs, 1 H), 6.39 (bs, 1H), 3.77 (d, J=15.4Hz, 1 H), 3.47 (d, J=15.4Hz, 1 H), 2.29 (s, 3H), 1.52 (s, 9H).
Step 2; Preparation of 4-(3-cyano-2-phenyl-5-trifluoromethyl-4,5-dihydrofuran-5-yl)-2-methylaniline.

To 0.27 g of t-butyl 4-(3-cyano-2-phenyl-5-trifluoromethyl-4,5-dihydrofuran-5-yl)-2-methylcarbanilate was added dropwise 5 ml of trifluoroacetic acid under ice-cooling and stirring. After stirring was continued at room temperature for 1 hour, the solvent was removed under reduced pressure, to the remaining oily substance was added 50 ml of ethyl acetate, the resulting mixture was washed with 50 ml of an aqueous saturated sodium hydrogen carbonate solution and then with 50 ml of water and dried over anhydrous sodium sulfate, and the solvent was removed under reduced pressure to obtain 0.15 g of the objective material as reddish resinous substance.
¹H NMR (CDCl₃, Me₄Si, 300MHz) δ 8.0-8.05 (m, 2H), 7.45-7.55 (m, 3H), 7.15-7.25 (m, 2H), 6.70 (d, J=9.0Hz, 1 H), 3.78 (bs, 2H), 3.74 (d, J=15.2Hz, 1 H), 3.47 (d, J=15.2Hz, 1 H), 2.19 (s, 3H).
Step 3; Preparation of N¹-[4-(3-cyano-2-phenyl-5-trifluoromethyl-4,5-dihydrofuran-5-yl)-2-methylphenyl]-3-iodo-N²-isopropylphthalic diamide.

To 10 ml of a toluene solution containing 0.15 g of 3-iodo-N-isopropylphthalamidic acid was added 0.15 g of trifluoroacetic anhydride under ice-cooling and stirring, and the mixture was stirred at room temperature for 2 hours. After the solvent was removed under reduced pressure, the residue was dissolved in 10 ml of acetonitrile, 0.1 g of 4-(3-cyano-2-phenyl-5-trifluoromethyl-4,5-dihydrofuran-5-yl)-2-methylaniline was added to the solution at room temperature under stirring, and stirring was continued at the same temperature for 12 hours. After completion of the reaction, the precipitated solid was collected by filtration and washed with 5 ml of acetonitrile to obtain 0.07 g of the objective material as white crystals.
Melting point 214.0 to 215.0°C
¹H NMR (CDCl₃, Me₄Si, 300MHz) δ 8.42 (bs, 1 H), 8.23 (d, J=8.6Hz, 1 H), 8.0-8.1 (m, 2H), 7.95-8.0 (m, 1 H), 7.8-7.85 (m, 1 H), 7.45-7.6 (m, 3H), 7.35-7.45 (m, 2H), 7.2-7.3 (m, 1 H), 5.75 (d, J=8.1 Hz, 1 H), 4.15-4.3 (m, 1 H), 3.80 (d, J=15.4Hz, 1 H), 3.49 (d, J=15.4Hz, 1 H), 2.38 (s, 3H), 1.17 (d, J=6.6Hz, 6H).

### Synthetic example 24

N¹-[4-[6-(4-chlorophenyl)-2-methyl-4-trifluoromethyl-3,4-dihydropyrimidin-4-yl]-2-methylphenyl]-3-iodo-N²-isopropylphthalic diamide (Present compound No. 8-01) Step 1; Preparation of t-butyl 4-[6-(4-chlorophenyl)-2-methyl-4-trifluoromethyl-3,4-dihydropyrimidin-4-yl]-2-methylcarbanilate.

To 50 ml of an ethanol solution containing 1.0 g of t-butyl 4-[3-(4-chlorophenyl)-3-oxo-1-trifluoromethyl-1-propenyl]-2-methylcarbanilate synthesized in Step 1 to Step 3 of Synthetic example 18 were added 0.4 g of acetamidine hydrochloride and 2.3 g of sodium methoxide, and the mixture was stirred under reflux for 5 hours. After completion of the reaction, the solvent was removed under reduced pressure, to the residue was added 50 ml of water and the resultimg mixture was extracted with ethyl acetate (50 mlx2), the organic layer was dehydrated by saturated brine and then dried over anhydrous magnesium sulfate in this order, and the solvent was removed under reduced pressure. The residue was purified by silica gel column chromatography eluting with ethyl acetate-hexane (1:1) to obtain 0.7 g of the objective material as colorless resinous substance.
¹H NMR (CDCl₃, Me₄Si, 300MHz) δ 7.3-7.9 (m, 7H), 6.38 (bs, 1 H), 6.26 (bs, 1 H), 5.29 (s, 1 H), 2.26 (s, 3H), 2.22 (s, 3H), 1.51 (s, 9H).
Step 2; Preparation of 4-[6-(4-chlorophenyl)-2-methyl-4-trifluoromethyl-3,4-dihydropyrimidin-4-yl]-2-methylaniline.

To 0.5 g of t-butyl 4-[6-(4-chlorophenyl)-2-methyl-4-trifluoromethyl-3,4-dihydropyrimidin-4-yl]-2-methylcarbanilate was added dropwise 3 ml of trifluoroacetic acid under ice-cooling and stirring. After stirring was continued at room temperature for 1 hour, the solvent was removed under reduced pressure, to the residue was added 30 ml of water and the resulting mixture was extracted with ethyl acetate (30 mlx2). The organic layer was washed with 30 ml of an aqueous saturated sodium hydrogen carbonate solution, then, dehydrated by saturated brine and then dried over anhydrous magnesium sulfate in this order, and the solvent was removed under reduced pressure to obtain 0.4 g of the objective material as colorless resinous substance.
¹H NMR (CDCl₃, Me₄Si, 300MHz) δ 7.48 (d, J=8.5Hz, 2H), 7.37 (d, J=8.5Hz, 2H), 7.2-7.3 (m, 3H), 6.65 (d, J=8.0Hz, 1 H), 5.34 (s, 1 H), 3.40 (bs, 2H), 2.20 (s, 3H), 2.17 (s, 3H).
Step 3; Preparation of N¹-[4-[6-(4-chlorophenyl)-2-methyl-4-trifluoromethyl-3,4-dihydropyrimidin-4-yl]-2-methylphenyl]-3-iodo-N²-isopropylphthalic diamide.

To 10 ml of a toluene solution containing 0.4 g of 3-iodo-N-isopropylphthalamidic acid was added 0.4 g of trifluoroacetic anhydride under ice-cooling and stirring, and the mixture was stirred at room temperature for 30 minutes. After the solvent was removed under reduced pressure, the residue was dissolved in 10 ml of acetonitrile, 0.4 g of 4-[6-(4-chlorophenyl)-2-methyl-4-trifluoromethyl-3,4-dihydropyrimidin-4-yl]-2-methylaniline was added to the solution and the mixture was stirred at room temperature for 5 hours. After completion of the reaction, the solvent was removed under reduced pressure, the residue was purified by silica gel column chromatography eluting with ethyl acetate-chloroform (1:1) to obtain 0.2 g of the objective material as colorless crystals.
Melting point 137.0 to 139.0°C
¹H NMR (CDCl₃, Me₄Si, 300MHz) δ 8.37 (bs, 1 H), 7.9-8.05 (m, 2H), 7.76 (d, J=9.0Hz, 1 H), 7.3-7.5 (m, 3H), 7.26 (s, 4H), 7.19 (t, J=7.5Hz, 1 H), 5.85 (d, J=8.0Hz, 1 H), 5.33 (s, 1H), 4.1-4.3 (m, 1 H), 2.32 (s, 3H), 2.23 (s, 3H), 1.17 (d, J=6.5Hz, 6H).

### Synthetic example 25

N¹-[4-(2,2-dichloro-1-(4-chlorophenyl)cyclopropyl)-2-methylphenyl]-3-iodo-N²-( 1-methyl-2-methylthioethyl)phthalic diamide (Present compound No. 9-020).
Step 1; Preparation of 4'-chloro-3-methyl-4-nitrobenzophenone.

To 100 ml of a chlorobenzene suspension containing 50.0 g of 3-methyl-4-nitrobenzoic acid were added 30.2 ml of thionyl chloride and 1 ml of N,N-dimethylformamide, and after the mixture was stirred at 95°C for 1.5 hours, excess thionyl chloride was removed under reduced pressure. To the reaction mixture was added 30 ml of chlorobenzene, 44.0 g of aluminum chloride was carefully added to the mixture under ice-cooling and stirring, and then, stirring was continued at 80°C for 2 hours. After completion of the reaction, the reaction mixture cooled to room temperature by allowing to stand was poured into 500 ml of ice-water, and further 50 ml of conc. hydrochloric acid was added thereto, the mixture was extracted with ethyl acetate (150 mlx2). The organic layer was washed with water, dehydrated by saturated brine and then dried over anhydrous magnesium sulfate in this order, and the solvent was removed under reduced pressure. The residual solid was washed with diisopropyl ether to obtain 63.5 g of the objective material as pale yellowish crystals.
Melting point 105.0 to 107.5°C
¹H NMR (CDCl₃, Me₄Si, 300MHz) δ 8.04 (d, J=8.1 Hz, 1 H), 7.65-7.8 (m, 4H), 7.50 (d, J=8.7Hz, 2H), 2.66 (s, 3H).
Step 2; Preparation of 4-amino-4'-chloro-3-methylbenzophenone.

To 220 ml of an ethyl acetate solution containing 22.0 g of 4'-chloro-3-methyl-4-nitrobenzophenone were added 220 ml of acetic acid, 220 ml of water and 26.4 g of iron powder, and the mixture was stirred under reflux for 4.5 hours. After completion of the reaction, the reaction mixture was subjected to Celite filtration, and the solvent was removed under reduced pressure. The residue was dissolved in 200 ml of ethyl acetate and washed with 200 ml of an aqueous saturated sodium hydrogen carbonate solution, the organic layer was dehydrated by saturated brine and then dried over anhydrous magnesium sulfate in this order, and the solvent was removed under reduced pressure. The residue was crystallized from diisopropyl ether to obtain 16.5 g of the objective material as yellow crystals.
Melting point 113.0 to 115.5°C
¹H NMR (CDCl₃, Me₄Si, 300MHz) δ 7.67 (d, J=8.4Hz, 2H), 7.60 (s, 1 H), 7.53 (d, J=8.4Hz, 1 H), 7.43 (d, J=8.4Hz, 2H), 6.66 (d, J=8.4Hz, 1 H), 4.15 (bs, 2H), 2.19 (s, 3H). Step 3; Preparation of t-butyl N-t-butoxycarbonyl-4-(4-chlorobenzoyl)-2-methylcarbanilate.

To 500 ml of a N,N-dimethylformamide solution containing 42.5 g of 4-amino-4'-chloro-3-methylbenzophenone were added 105.0 g of di-t-butyl dicarbonate and 72.0 g of potassium carbonate, and the mixture was stirred at 100°C for 3 hours. After completion of the reaction, the mixture was cooled to room temperature by allowing to stand, poured into 500 ml of ice-water and extracted with ethyl acetate (350 mlx2). The organic layer was washed with water (300 mlx2), then dehydrated by saturated brine and then dried over anhydrous magnesium sulfate in this order, and the solvent was removed under reduced pressure. The residue was crystallized from hexane to obtain 70.0 g of the objective material as white solid.
¹H NMR (CDCl₃, Me₄Si, 300MHz) δ 7.74 (d, J=9.0Hz, 2H), 7.67 (s, 1 H), 7.60 (d, J=8.1 Hz, 1 H), 7.47 (d, J=9.0Hz, 2H), 7.20 (d, J=8.1 Hz, 1 H), 2.27 (s, 3H), 1.43 (s, 18H). Step 4; Preparation of t-butyl N-t-butoxycarbonyl-4-[1-(4-chlorophenyl)ethenyl]-2-methylcarbanilate.

To 300 ml of tetrahydrofuran solution containing 10.1 g of potassium t-butoxide was added 32.15 g of methyltriphenyl phosphonium bromide, and the mixture was stirred at room temperature for 30 minutes, then, 50 ml of a tetrahydrofuran solution containing 20.05 g of t-butyl N-t-butoxycarbonyl-4-(4-chlorobenzoyl)-2-methylcarbanilate was added dropwise to the mixture, and stirring was continued at the same temperature for further 90 minutes. After completion of the reaction, the reaction mixture was poured into 200 ml of a saturated aqueous ammonium chloride solution, and the mixture was stirred for 15 minutes and then extracted with 50 ml of ethyl acetate1. The organic layer was dehydrated by saturated brine and then dried over anhydrous magnesium sulfate in this order, the solvent was removed under reduced pressure, and the residue was purified by silica gel column chromatography eluting with ethyl acetate-hexane (1:9) to obtain 19.0 g of the objective material as yellowish oily substance.
n_{D}^{21.3°C} 1.5398
¹H NMR (CDCl₃, Me₄Si, 300MHz) δ 7.25-7.35 (m, 4H), 7.1-7.15 (m, 2H), 7.04 (d, J=7.8Hz, 1 H), 5.46 (bs, 1 H), 5.44 (bs, 1 H), 2.18 (s, 3H), 1.44 (s, 18H).
Step 5; Preparation of t-butyl N-t-butoxycarbonyl-4-[2,2-dichloro-1-(4-chlorophenyl)-cyclopropyl]-2-methylcarbanilate.

To 5 ml of a chloroform solution containing 1.0 g of t-butyl N-t-butoxycarbonyl-4-[1-(4-chlorophenyl)ethenyl]-2-methylcarbanilate was added 0.005 g of hexadecyl-Itrimethyl ammonium chloride, and 2 ml of an aqueous solution containing 1.0 g of sodium hydroxide was added dropwise to the mixture at 50° C under stirring. After stirring was continued at the same temperature for 90 minutes, the reaction mixture was diluted by water and extracted with chloroform (20 mlx2). The organic layer was washed with water (30 mlx1), then, dehydrated by saturated brine and then dried over anhydrous magnesium sulfate in this order, and the solvent was removed under reduced pressure to obtain 1.0 g of the objective material as yellowish oily substance.
¹H NMR (CDCl₃, Me₄Si, 300MHz) δ 7.41 (d, J=8.4Hz, 2H), 7.25-7.3 (m, 4H), 7.01 (d, J=8.7Hz, 1 H), 2.30 (d, J=7.2Hz, 1 H), 2.22 (d, J=7.2Hz, 1 H), 2.17 (s, 3H), 1.37 (s, 18H).
Step 6; Preparation of 4-[2,2-dichloro-1-(4-chlorophenyl)cyclopropyl]-2-methylaniline.

To 1.0 g of t-butyl N-t-butoxycarbonyl-4-[2,2-dichloro-1-(4-chlorophenyl)cyclopropyl]-2-methylcarbanilate was added dropwise 4 ml of trifluoroacetic acid at room temperature under stirring. After stirring was continued at the same temperature for 15 minutes, excess trifluoroacetic acid was removed under reduced pressure, the remaining oily substance was dissolved in 30 ml of ethyl acetate and washed with 20 ml of an aqueous saturated sodium hydrogen carbonate solution. The organic layer was dehydrated by saturated brine and then dried over anhydrous magnesium sulfate in this order, and the solvent was removed under reduced pressure to obtain 0.6 g of the objective material as greenish oily substance.
¹H NMR (CDCl₃, Me₄Si, 300MHz) δ 7.39 (d, J=6.1 Hz, 2H), 7.26 (d, J=6.1 Hz, 2H), 7.1-7.15 (m, 2H), 6.60 (d, J=7.7Hz, 1 H), 3.26 (bs, 2H), 2.23 (d, J=7.1 Hz, 1 H), 2.17 (d, J=7.1 Hz, 1 H), 2.13 (s, 3H).
Step 7; Preparation of N¹-[4-(2,2-dichloro-1-(4-chlorophenyl)cyclopropyl)-2-methylphenyl]-3-iodo-N²-(1-methyl-2-methylthioethyl)phthalic diamide.

To 10 ml of a toluene solution containing 0.4 g of 3-iodo-N-(1-methyl-2-methylthioethyl)phthalamidic acid was added dropwise 0.3 g of trifluoroacetic anhydride at room temperature under stirring. After the mixture was stirred at the same temperature for 15 minutes, the solvent was removed under reduced pressure, the residue was dissolved in 2 ml of acetonitrile, and 3 ml of an acetonitrile solution containing 0.35 g of 4-[2,2-dichioro-1-(4-chiorophenyl)cyclopropyl]-2-methylaniline was added dropwise to the mixture. After stirring was continued at room temperature for 30 minutes, the reaction mixture was ice-cooled, and the precipitated solid was collected by filtration and washed with a small amount of acetonitrile to obtain 0.5 g of the objective material as white crystals.
Melting point 223.5 to 225.0°C
¹H NMR (CDCl₃, Me₄Si, 300MHz) δ 8.23 (bs, 1 H), 8.09 (d, J=8.1 Hz, 1 H), 7.95 (d, J=7.8Hz, 1 H), 7.75 (d, J=7.5Hz, 1 H), 7.41 (d, J=8.4Hz, 2H), 7.15-7.35 (m, 5H), 6.13 (d, J=8.7Hz, 1 H), 4.25-4.35 (m, 1 H), 2.58 (dd, J=13.5, 4.5Hz, 1 H), 2.50 (dd, J=13.5, 6.3Hz, 1 H), 2.29 (s, 3H), 2.28 (d, J=7.2Hz, 1 H), 2.23 (d, J=7.2Hz, 1 H), 1.83 (s, 3H), 1.23 (d, J=6.6Hz, 3H).

### Synthetic example 26

N¹-[4-(2,2-difluoro-1-(4-chlorophenyl)cyclopropyl)-2-methylphenyl]-3-iodo-N²-(1-methyl-2-methylthioethyl)phthalic diamide (Present compound No. 9-019).
Step 1; Preparation of 4-[2,2-difluoro-1-(4-chlorophenyl)cyclopropyl]-2-methylaniline.

To 5 ml of a diethylene glycol dimethyl ether solution containing 1.7 g of t-butyl N-t-butoxycarbonyl-4-[1-(4-chlorophenyl)ethenyl]-2-methylcarbanilate synthesized in Step 1 to Step 4 of Synthetic example 25 was added 7.0 g of sodium dichlorofluoroacetate little by little over 30 minutes at 170°C and under stirring, and after completion of addition, stirring was continued at 180°C for further 30 minutes. After completion of the reaction, the mixture was cooled to room temperature by allowing to stand, the reaction mixture was dissolved in 50 ml of ethyl acetate and washed with water (50 mlx1), then, dehydrated by saturated brine and then dried over anhydrous magnesium sulfate in this order, and the solvent was removed under reduced pressure. To the residue was added dropwise 10 ml of trifluoroacetic acid at room temperature under stirring, and stirring was continued at the same temperature for further 15 minutes. After completion of the reaction, excess trifluoroacetic acid was removed under reduced pressure, the remaining oily substance dissolved in 30 ml of ethyl acetate and washed with 20 ml of an aqueous saturated sodium hydrogen carbonate solution. The organic layer was dehydrated by saturated brine and then dried over anhydrous magnesium sulfate in this order, the solvent was removed under reduced pressure, and the residue was purified by high performance liquid chromatography eluting with acetonitrile-water (85:15) to obtain 0.35 g of the objective material as pale yellowish oily substance. n_{D}^{21.3°C} 1.5726
¹H NMR (CDCl₃, Me₄Si, 300MHz) δ 7.30 (d, J=8.7Hz, 2H), 7.24 (d, J=8.7Hz, 2H), 7.0-7.05 (m, 2H), 6.59 (d, J=8.7Hz, 1 H), 3.53 (bs, 2H), 2.11 (s, 3H), 1.9-2.05 (m, 2H). Step 2; Preparation of N¹-[4-(2,2-difluoro-1-(4-chlorophenyl)cyclopropyl)-2-methylphenyl]-3-iodo-N²-(1-methyl-2-methylthioethyl)phthalic diamide.

To 8 ml of a toluene solution containing 0.28 g of 3-iodo-N-(1-methyl-2-methylthioethyl)phthalamidic acid was added dropwise 0.2 g of trifluoroacetic anhydride at room temperature under stirring. After the mixture was stirred at the same temperature for 15 minutes, the solvent was removed under reduced pressure, the residue was dissolved in 2 ml of acetonitrile, and 5 ml of an acetonitrile solution containing 0.2 g of 4-[2,2-difluoro-1-(4-chlorophenyl)cyclopropyl]-2-methylaniline was added to the mixture. After stirring was continued at room temperature for 2 hours, the reaction mixture was ice-cooled, the precipitated solid was collected by filtration and washed with a small amount of acetonitrile to obtain 0.32 g of the objective material as white crystals.
Melting point 229.0 to 232.0°C
¹H NMR (CDCl₃, Me₄Si, 300MHz) δ 8.26 (bs, 1 H), 8.04 (d, J=8.7Hz, 1 H), 7.94 (d, J=8.1 Hz, 1 H), 7.74 (d, J=7.5Hz, 1 H), 7.15-7.35 (m, 7H), 6.22 (d, J=8.1 Hz, 1 H), 4.25-4.35 (m, 1 H), 2.61 (dd, J=13.5, 6.0Hz, 1 H), 2.52 (dd, J=13.5, 6.3Hz, 1 H), 2.28 (s, 3H), 1.95-2.15 (m, 2H), 1.88 (s, 3H), 1.25 (d, J=6.6Hz, 3H).

### Synthetic example 27

N¹-[4-(2,2-difluoro-1-(4-chlorophenyl)cyclopropyl)-2-methylphenyl]-3-iodo-N²-(1-methyl-2-methylsulfonylethyl)phthalic diamide (Present compound No. 9-036).

To 20 ml of a dichloromethane solution containing 0.15 g of N¹-[4-(2,2-difluoro-1-(4-chlorophenyl)cyclopropyl)-2-methylphenyl]-3-iodo-N²-(1-methyl-2-methylthioethyl)phthalic diamide (Present compound No. 9-019) synthesized in Synthetic example 26 was added 0.12 g of 3-chloroperbenzoic acid under ice-cooling and stirring, and stirring was continued at room temperature for 4.5 hours. After completion of the reaction, the reaction mixture was washed with 30 ml of an aqueous sodium hydrogen sulfite solution and then with 30 ml of an aqueous saturated sodium hydrogen carbonate solution, then, dehydrated by saturated brine and then dried over anhydrous magnesium sulfate in this order, and the solvent was removed under reduced pressure to obtain 0.15 g of the objective material as colorless resinous substance.
¹H NMR (CDCl₃, Me₄Si, 300MHz) δ 8.26 (bs, 1 H), 7.80 (d, J=7.8Hz, 1 H), 7.74 (d, J=9.0Hz, 1 H), 7.55 (d, J=7.5Hz, 1 H), 7.05-7.35 (m, 7H), 6.94 (d, J=7.5Hz, 1 H), 4.4-4.5 (m, 1 H), 3.24 (dd, J=14.1, 4.2Hz, 1 H), 3.00 (dd, J=14.1, 6.9Hz, 1 H), 2.52 (s, 3H), 2.23 (s, 3H), 1.95-2.15 (m, 2H), 1.41 (d, J=6.3Hz, 3H).

### Synthetic example 28

N¹-[4-(2,2-dicyano-1-trifluoromethylcyclopropyl)-2-methylphenyl]-3-iodo-N²-isopropylphthalic diamide (Present compound No. 9-001).
Step 1; Preparation of t-butyl 4-(2,2-dicyano-1-trifluoromethylcyclopropyl)-2-methylcarbanilate.

To 50 ml of a chloroform solution containing 2.0 g of t-butyl 2-methyl-4-(1-trifluoromethylethenyl)carbanilate synthesized in Step 1 to Step 2 of Synthetic example 1 was added dropwise 0.34 ml of bromine at room temperature under stirring, and stirring was continued at the same temperature for 15 minutes. After completion of the reaction, the solvent was removed under reduced pressure to obtain crude t-butyl 4-(2,2-dibromo-1-trifluoromethylethyl)-2-methylcarbanilate. Then, to 0.75 g of potassium t-butoxide suspended in 3 ml of toluene was added dropwise 5 ml of a toluene solution containing 0.5 g of malononitrile at room temperature under stirring, and the mixture was stirred at the same temperature for 1 hour. To the mixture was added dropwise 5 ml of a toluene solution containing crude t-butyl 4-(2,2-dibromo-1-trifluoromethylethyl)-2-methylcarbanilate and stirring was continued. After 1 hour, 1.5 g of potassium t-butoxide was additionally added to the mixture, and stirring was further continued for 4 hours. After completion of the reaction, the reaction mixture was washed with 30 ml of dil. hydrochloric acid, then, dehydrated by saturated brine and then dried over anhydrous magnesium sulfate in this order, and the solvent was removed under reduced pressure. The residue was purified by silica gel column chromatography eluting with ethyl acetate-hexane (1:4) to obtain 1.15 g of the objective material as white crystals.
Melting point 179.5 to 182.0°C
¹H NMR (CDCl₃, Me₄Si, 300MHz) δ 8.08 (d, J=8.7Hz, 1 H), 7.25-7.3 (m, 2H), 6.41 (bs, 1 H), 2.59 (d, J=6.9Hz, 1 H), 2.39 (d, J=6.9Hz, 1 H), 2.29 (s, 3H), 1.53 (s, 9H).
Step 2; Preparation of 4-(2,2-dicyano-1-trifluoromethylcyclopropyl)-2-methylaniline.

To 1.05 g of t-butyl 4-(2,2-dicyano-1-trifluoromethylcyclopropyl)-2-methylcarbanilate was added dropwise 5 ml of trifluoroacetic acid at room temperature under stirring. After stirring was continued at room temperature for 1 hour, the solvent was removed under reduced pressure, the residue was dissolved in 50 ml of ethyl acetate and washed with 30 ml of an aqueous saturated sodium hydrogen carbonate solution, then, dehydrated by saturated brine and then dried over anhydrous magnesium sulfate in this order, and the solvent was removed under reduced pressure to obtain 0.78 g of the objective material as brownish resinous substance.
¹H NMR (CDCl₃, Me₄Si, 300MHz) δ 7.11 (bs, 2H), 6.70 (d, J=8.7Hz, 1 H), 3.07 (bs, 2H), 2.55 (d, J=6.3Hz, 1 H), 2.3-2.4 (m, 1 H), 2.18 (s, 3H).
Step 3; Preparation of N¹-[4-(2,2-dicyano-1-trifluoromethylcyclopropyl)-2-methylphenyl]-3-iodo-N²-isopropylphthalic diamide.

To 5 ml of a toluene solution containing 0.27 g of 3-iodo-N-isopropylphthalamidic acid was added 0.2 g of trifluoroacetic anhydride at room temperature under stirring, and the mixture was stirred at room temperature for 15 minutes. After the solvent was removed under reduced pressure, the residue was dissolved in 2 ml of acetonitrile, 5 ml of an acetonitrile solution containing 0.2 g of 4-(2,2-dicyano-1-trifluoromethylcyclopropyl)-2-methylaniline was added dropwise to the mixture, and the mixture was stirred at room temperature for 2 hours. After completion of the reaction, the solvent was removed under reduced pressure, and the residue was purified by silica gel column chromatography eluting with ethyl acetate-hexane (1:1) to obtain 0.2 g of the objective material as white crystals.
Melting point 218.0 to 221.0°C
¹H NMR (CDCl₃, Me₄Si, 300MHz) δ 8.52 (bs, 1 H), 8.34 (d, J=8.7Hz, 1 H), 7.97 (d, J=8.1 Hz, 1 H), 7.77 (d, J=7.2Hz, 1 H), 7.15-7.35 (m, 3H), 5.94 (d, J=8.1 Hz, 1 H), 4.1-4.3 (m, 1 H), 2.62 (d, J=6.6Hz, 1 H), 2.35-2.5 (m, 1 H), 2.38 (s, 3H), 1.13 (q, J=6.6Hz, 6H).

### Synthetic example 29

N¹-[4-[4-(4-chlorophenyl)-2-trifluoromethyl-1,3-oxathiolan-2-yl]-2-methylphenyl]-3-iodo-N²-isopropylphthalic diamide (Present compound No. 7-09)
Step 1; Preparation of 2-(4-chlorophenyl)-2-(3-methyl-4-nitrophenyl)-1,3-oxathiolane.

To 6 ml of a 1,2-dichloroethane solution containing 1.0 g of 4'-chloro-3-methyl-4-nitrobenzophenone synthesized in Step 1 of Synthetic example 25 and 5 ml of 2-mercaptoethanol was added 2.0 g of chlorotrimethylsilane, and the mixture was stirred at 60°C for 3.5 hours. After completion of the reaction, the reaction mixture was poured into 50 ml of water and extracted with chloroform (30 mlx2), the organic layer was dehydrated and washed with water, saturated brine and then anhydrous sodium sulfate in this order, and the solvent was removed under reduced pressure to obtain1.4 g of the crude objective material as yellowish oily substance. This product was used in the next step as such without purification.
Step 2; Preparation of 4-[2-(4-chlorophenyl)-1,3-oxathiolan-2-yl]-2-methylaniline.

To 100 ml of a methanol suspension containing 1.4 g of crude 2-(4-chlorophenyl)-2-(3-methyl-4-nitrophenyl)-1,3-oxathiolane and 1.08 g of cuprous chloride was added 1.37 g of potassium borohydride over 30 minutes little by little at room temperature under stirring. After stirring was continued at the same temperature for 1 hour, 1.08 g of cuprous chloride and 1.37 g of potassium borohydride were additionally added to the mixture over 30 minutes little by little, and stirring was continued at the same temperature for further 2 hours. After completion of the reaction, insoluble materials were removed by Celite filtration, and the solvent was removed under reduced pressure. To the remaining oily substance was added 50 ml of ethyl acetate, the resulting mixture was washed with 50 ml of dil. aqueous ammonia, then, dehydrated by saturated brine and dried over anhydrous sodium sulfate in this order, and the solvent was removed under reduced pressure. The residue was purified by silica gel column chromatography eluting with ethyl acetate-hexane (1:2) to obtain 0.2 g of the objective material as yellowish oily substance.
¹H NMR (CDCl₃, Me₄Si, 300MHz) δ 7.43 (d, J=8.4, 2H), 7.05-7.3 (m, 4H), 6.58 (d, J=8.4Hz, 1 H), 4.15-4.2 (m, 2H), 3.63 (bs, 2H), 3.2-3.25 (m, 2H), 2.13 (s, 3H).
Step 3; Preparation of N¹-[4-[2-(4-chlorophenyl)-1,3-oxathiolan-2-yl]-2-methylphenyl]-3-iodo-N²-isopropylphthalic diamide.

To 5 ml of a toluene solution containing 0.3 g of 3-iodo-N-isopropylphthalamidic acid was added 0.23 g of trifluoroacetic anhydride at room temperature under stirring, and the mixture was stirred at the same temperature for 15 minutes. After the solvent was removed under reduced pressure, the residue was dissolved in 2 ml of acetonitrile, and at room temperature under stirring, 5 ml of an acetonitrile solution containing 0.2 g of 4-[2-(4-chlorophenyl)-1,3-oxathiolan-2-yl]-2-methylaniline was added to the solution, and stirring was continued at the same temperature for 1 hour. After completion of the reaction, the precipitated solid was collected by filtration, and washed with a small amount of acetonitrile to obtain 0.25 g of the objective material as white crystals.
Melting point 213.5 to 217.0°C
¹H NMR (CDCl₃, Me₄Si, 300MHz) δ 8.31 (bs, 1 H), 7.9-8.0 (m, 2H), 7.75 (d, J=7.8Hz, 1 H), 7.43 (d, J=8.7Hz, 2H), 7.15-7.35 (m, 5H), 5.91 (d, J=7.8Hz, 1 H), 4.15-4.25 (m, 3H), 3.2-3.3 (m, 2H), 2.28 (s, 3H), 1.17 (d, J=6.6Hz, 6H).

The compounds of the present invention can be prepared in accordance with the above-mentioned Preparation methods and Examples. Examples of such compounds are shown in Table 8 to Table 24, but the present invention is not limited by these.

Incidentally, in the tables, the description Et means an ethyl group, and similarly n-Pr or Pr-n means normal propyl group, i-Pr or Pr-i means isopropyl group, c-Pr or Pr-c means cyclopropyl group, n-Bu or Bu-n means normal butyl group, s-Bu or Bu-s means secondary butyl group, i-Bu or Bu-i means isobutyl group, t-Bu or Bu-t means tertiary butyl group, c-Pen or Pen-c means cyclopentyl group, c-Hex or Hex-c means cyclohexyl group, Ph means phenyl group, respectively, and

in the tables, the aromatic heterocyclic rings represented by L-1a to L-47e are each represented by the following structures.

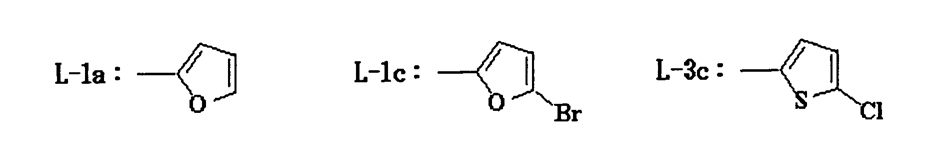

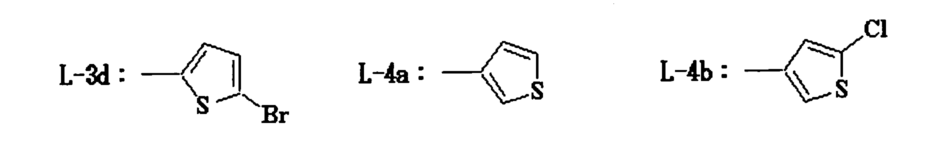

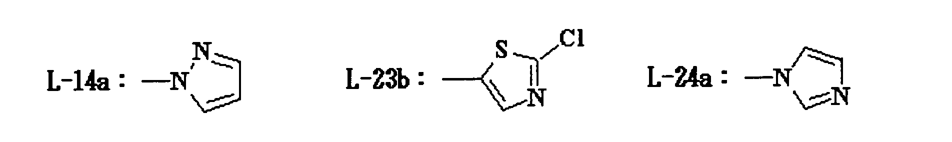

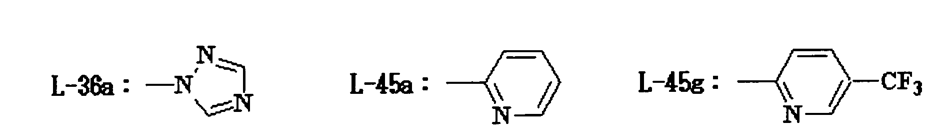

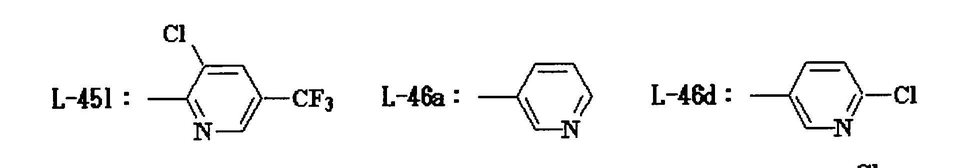

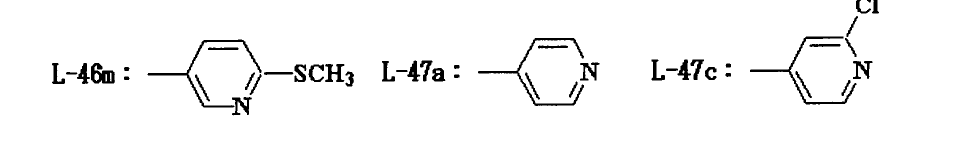

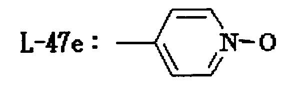

Also, in the tables, *1 means "resinous state", *2 means "oily", and *3 means "decomposed".

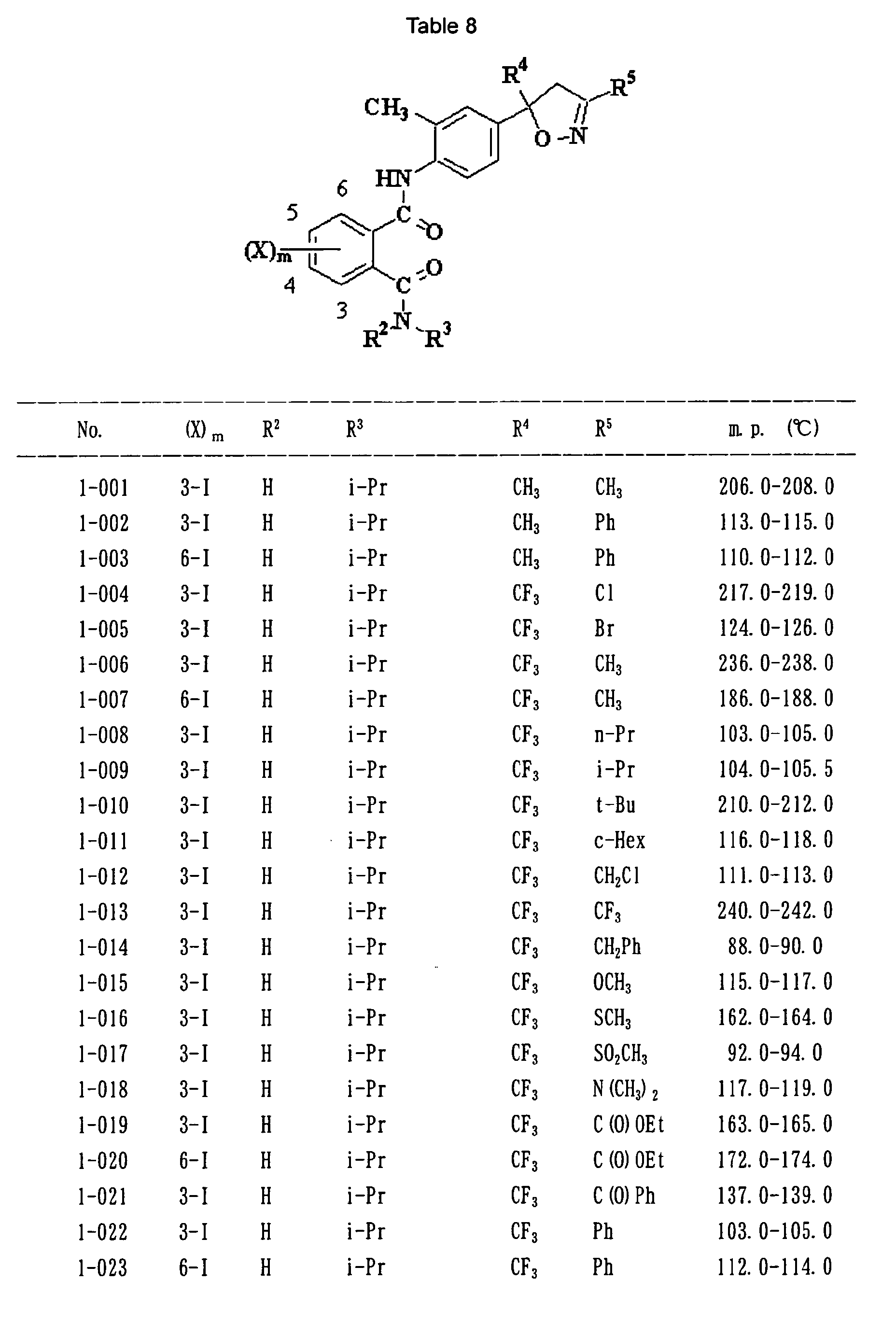

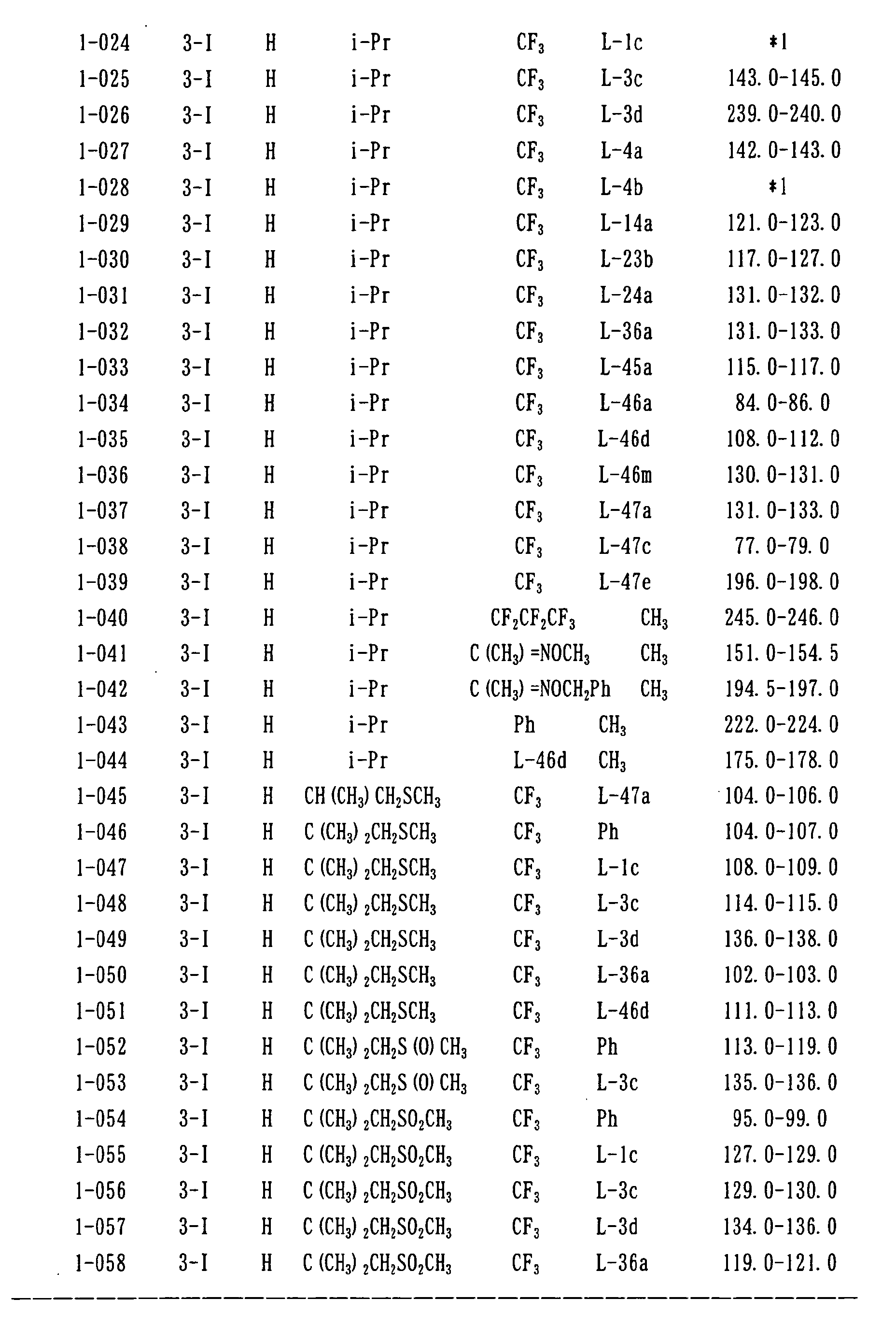

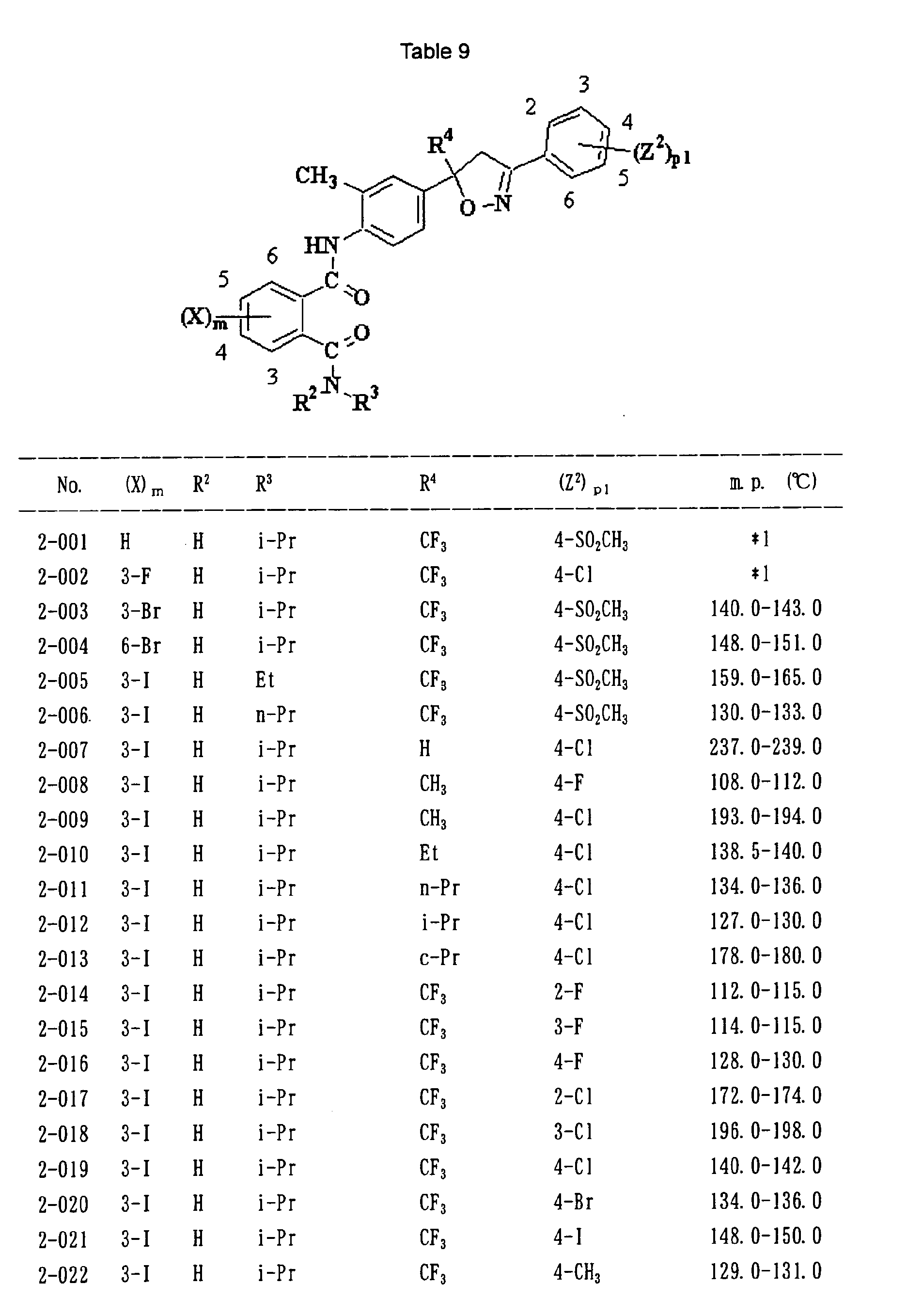

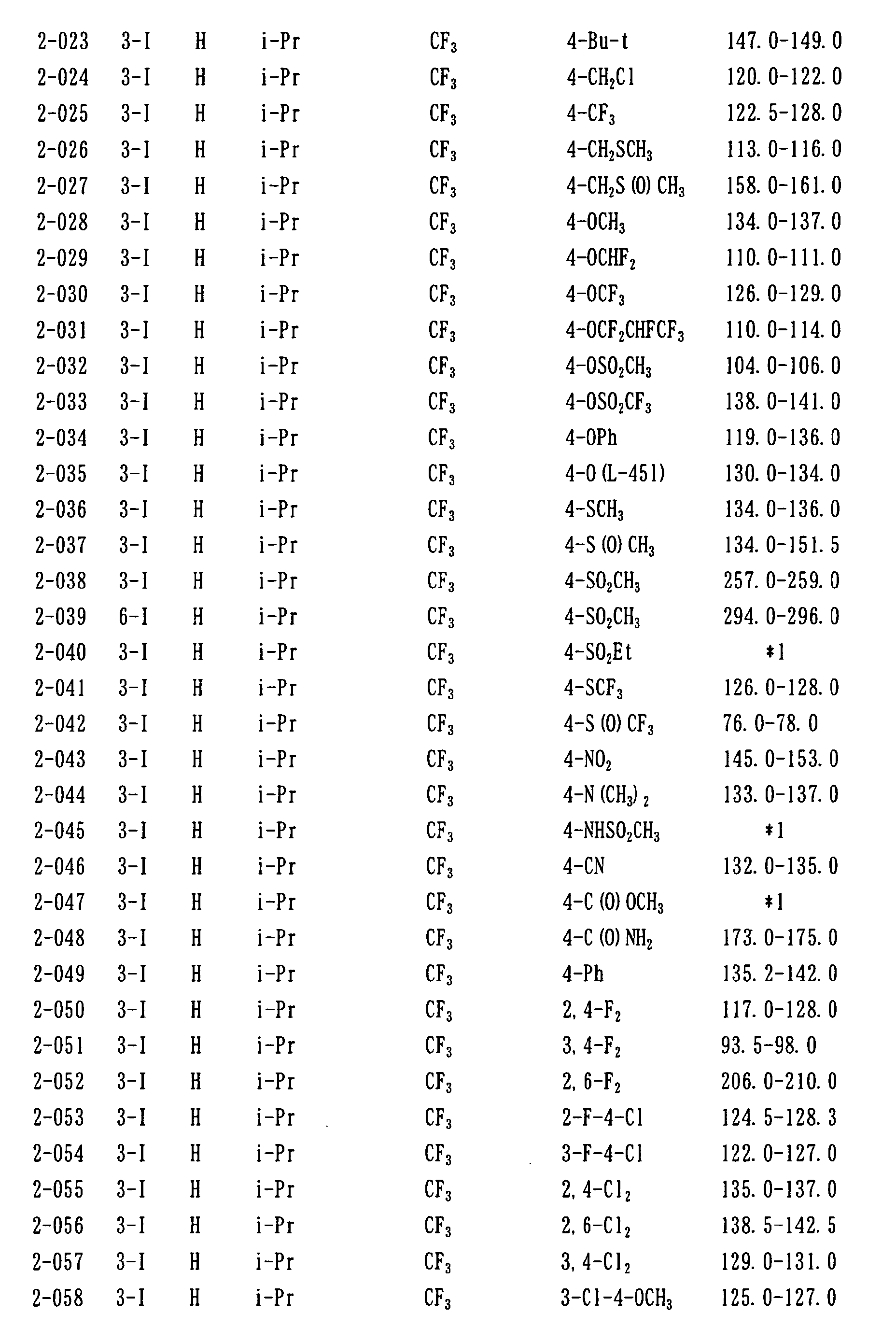

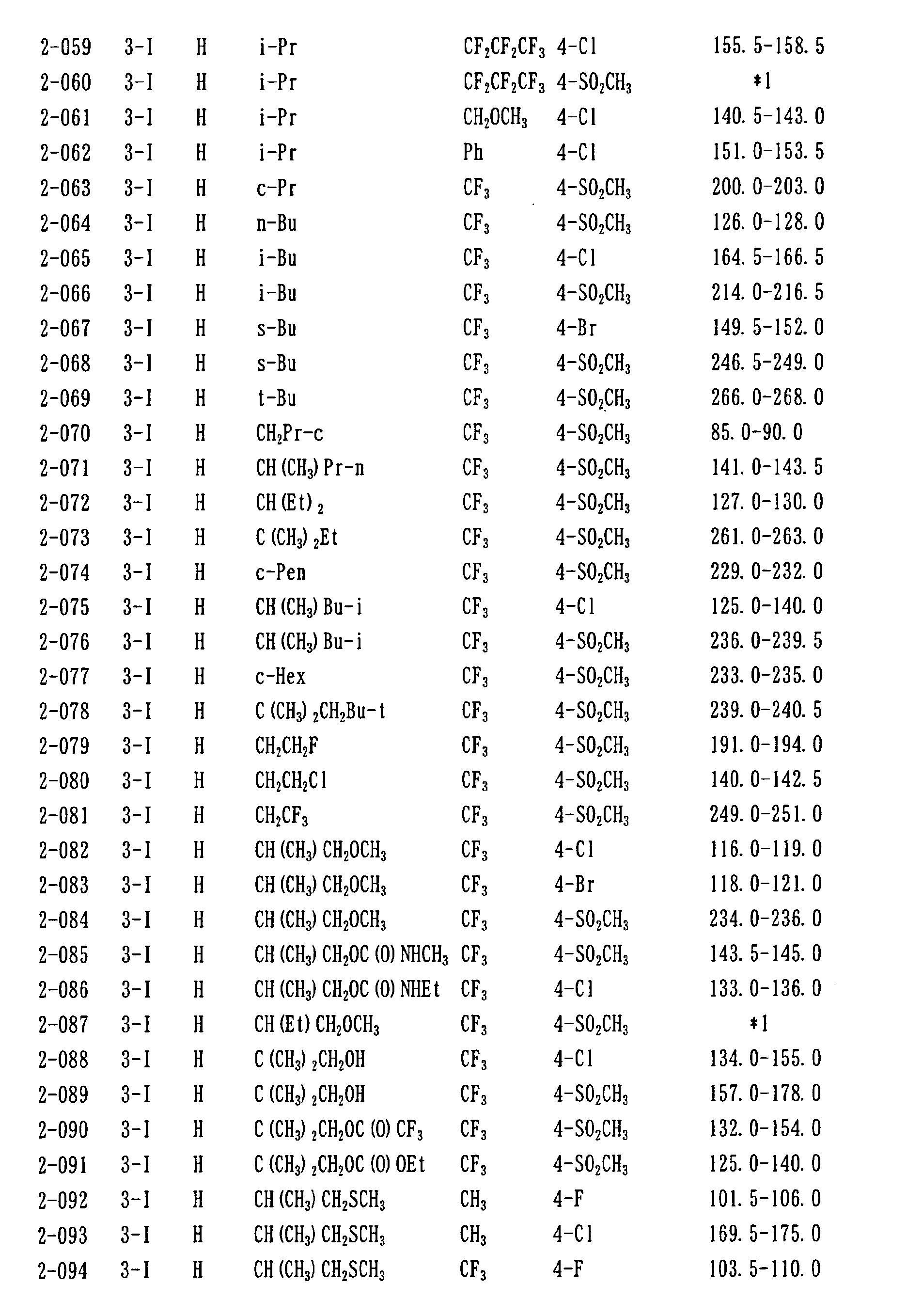

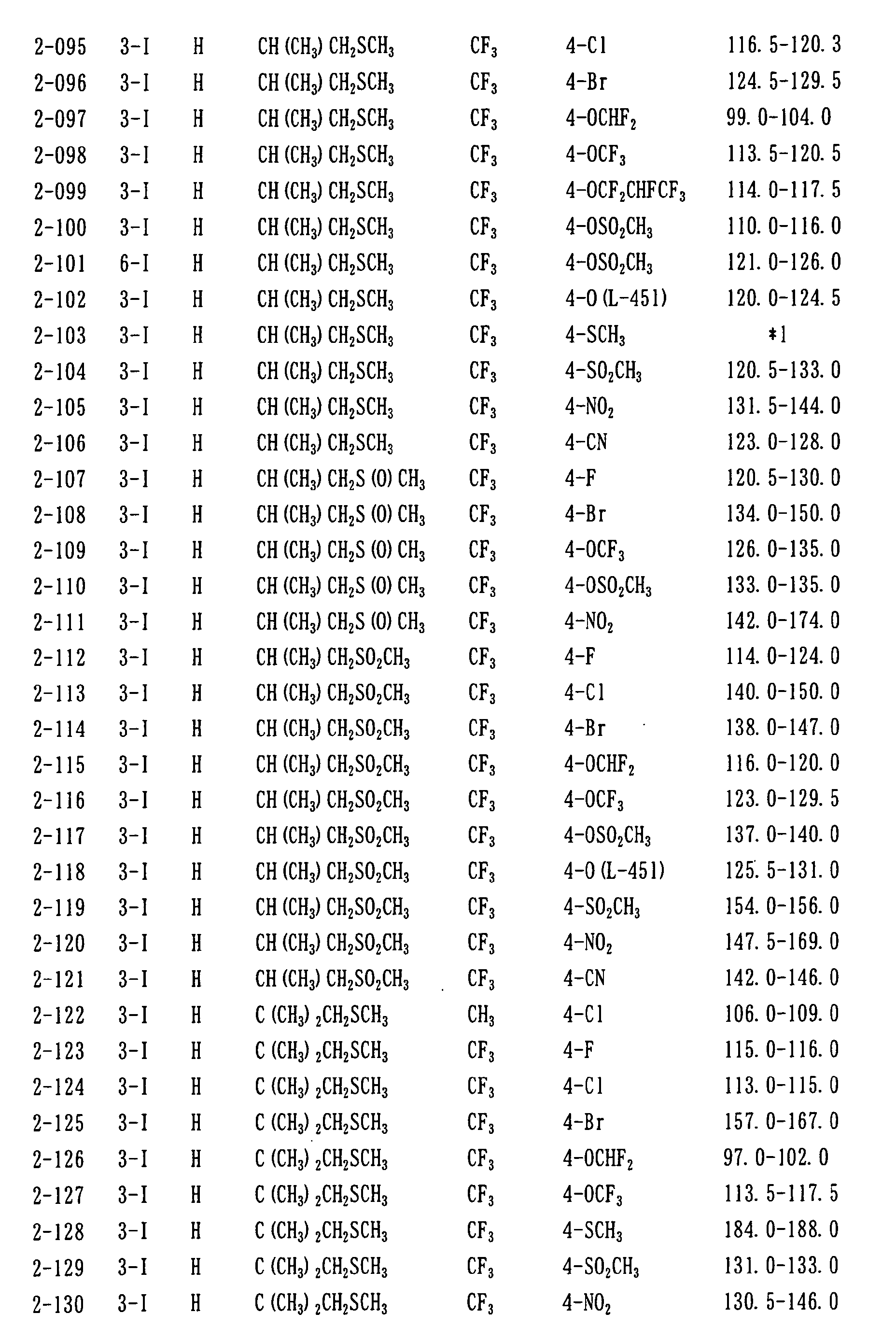

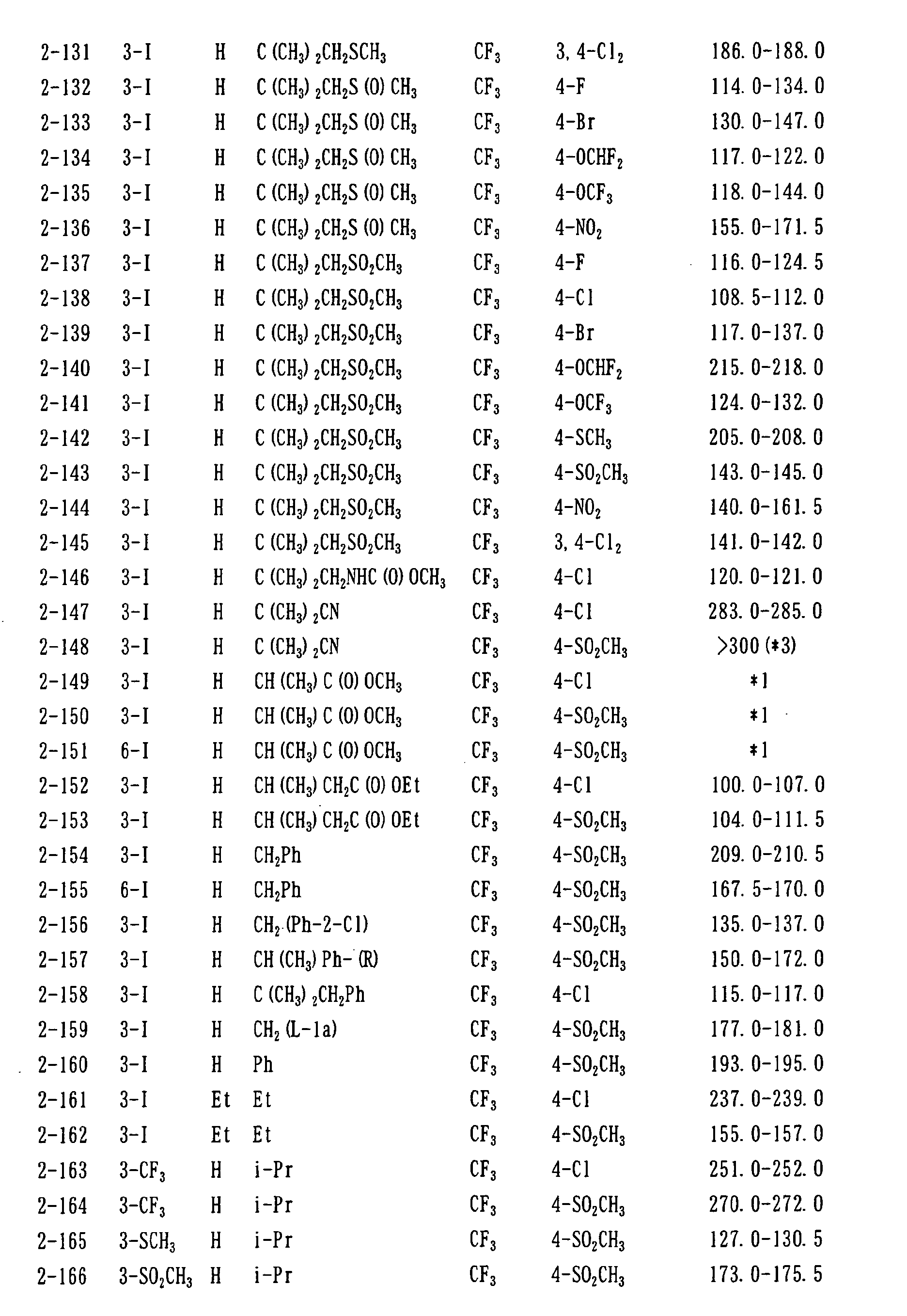

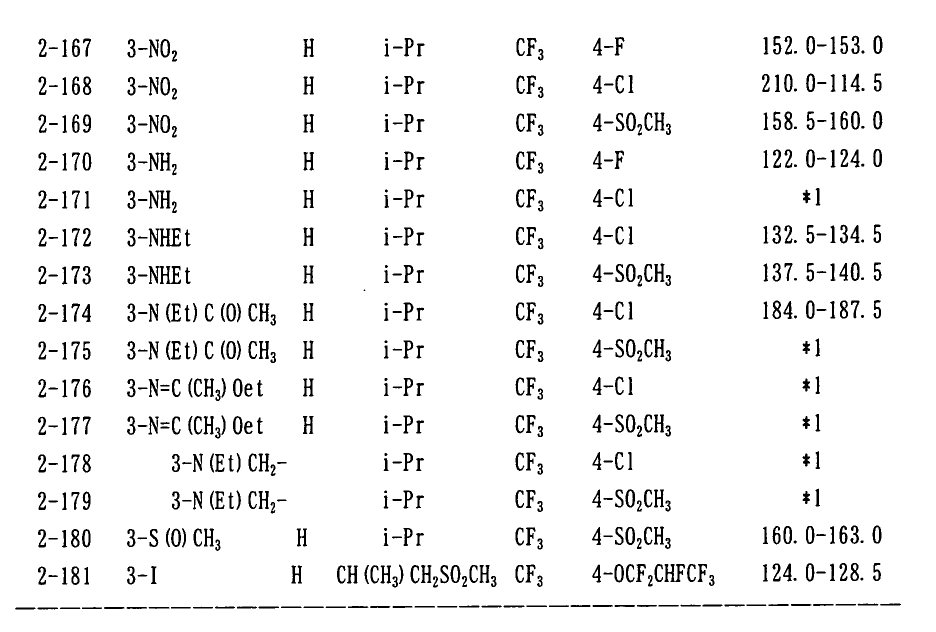

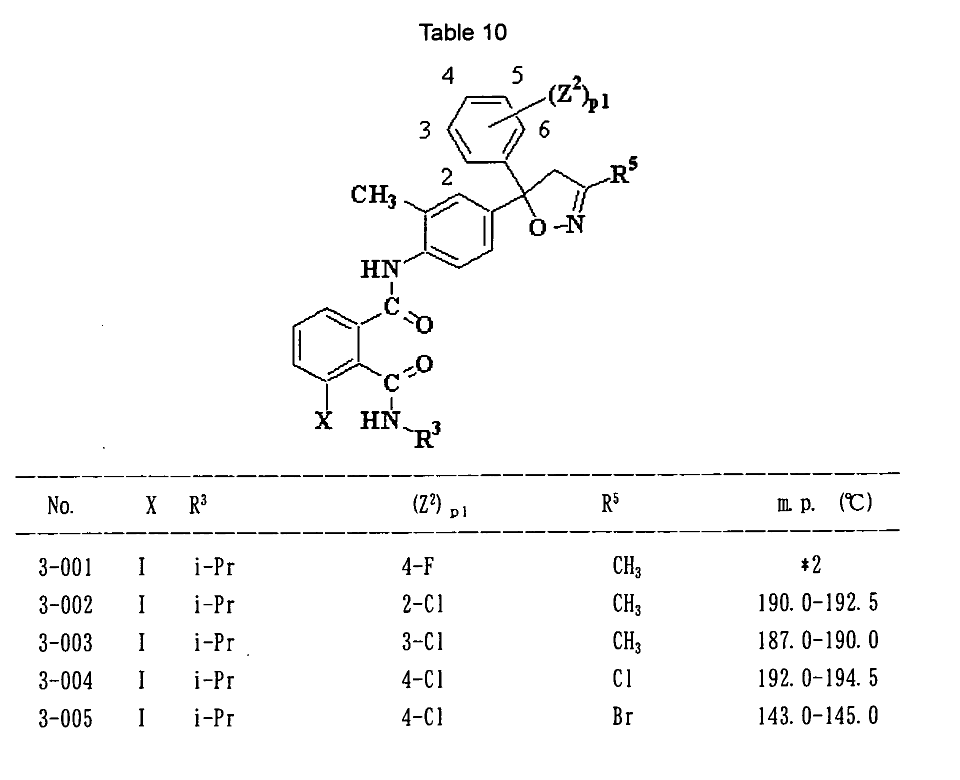

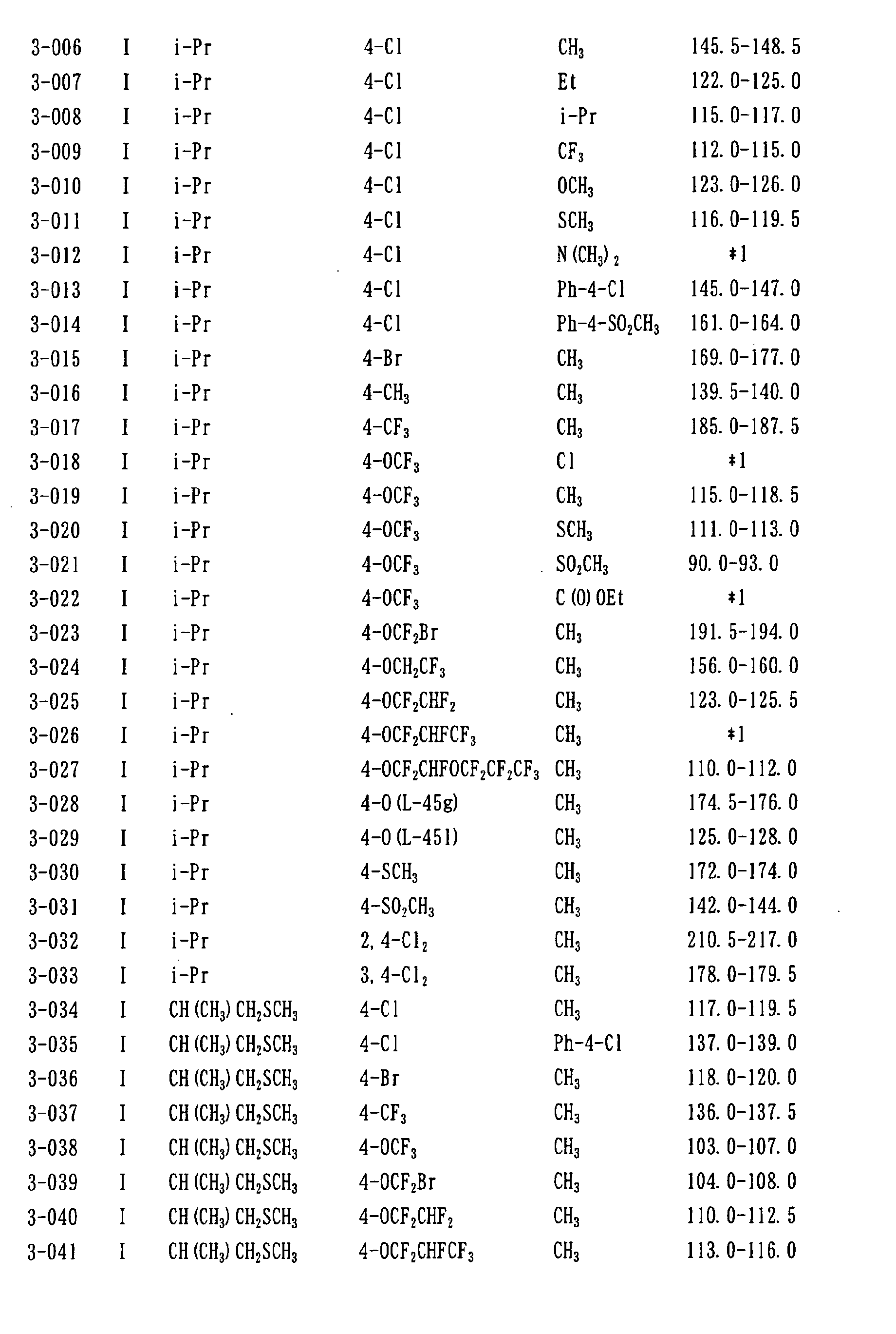

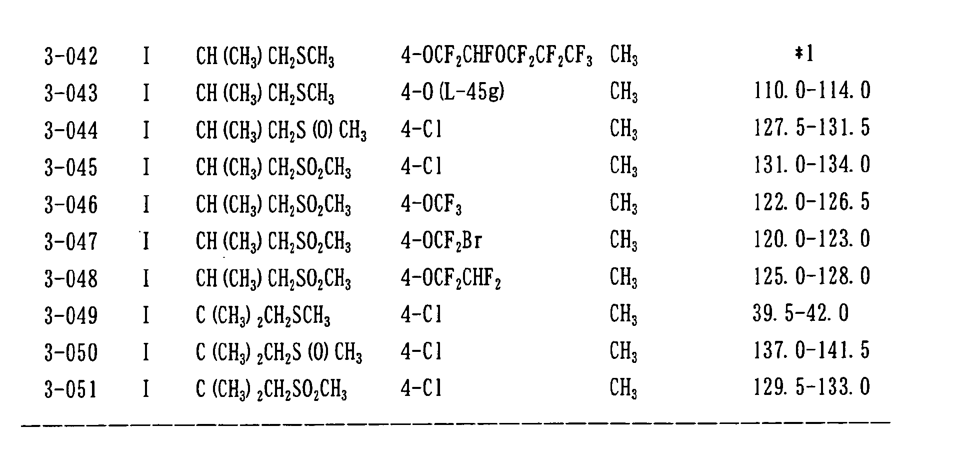

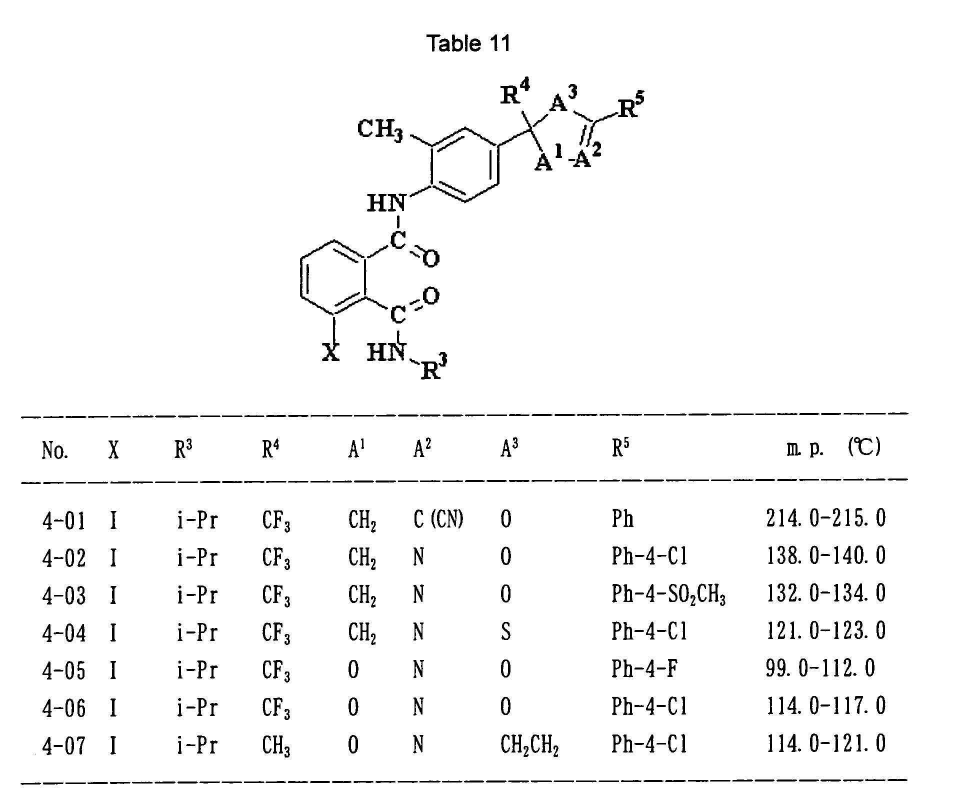

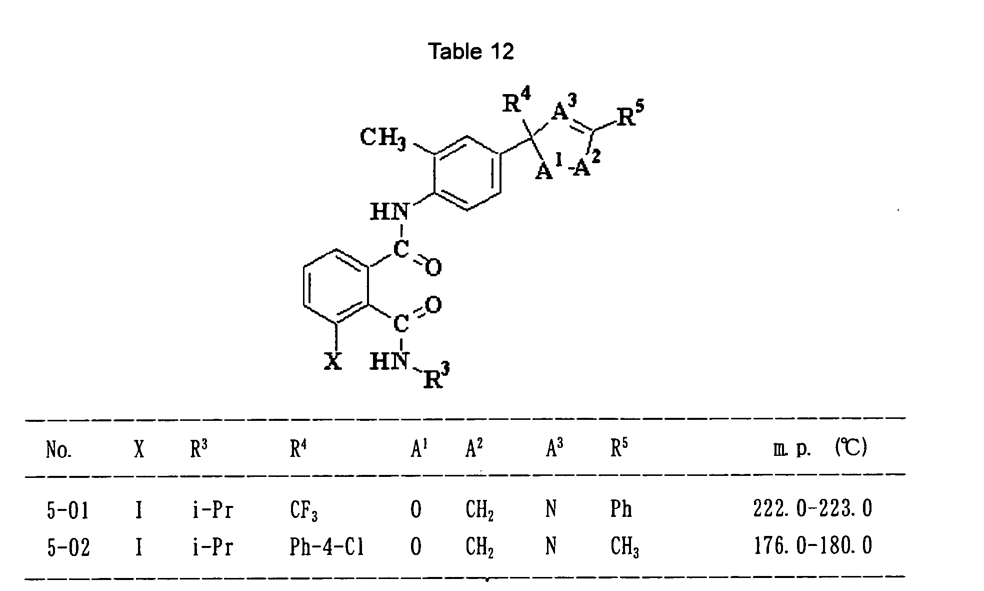

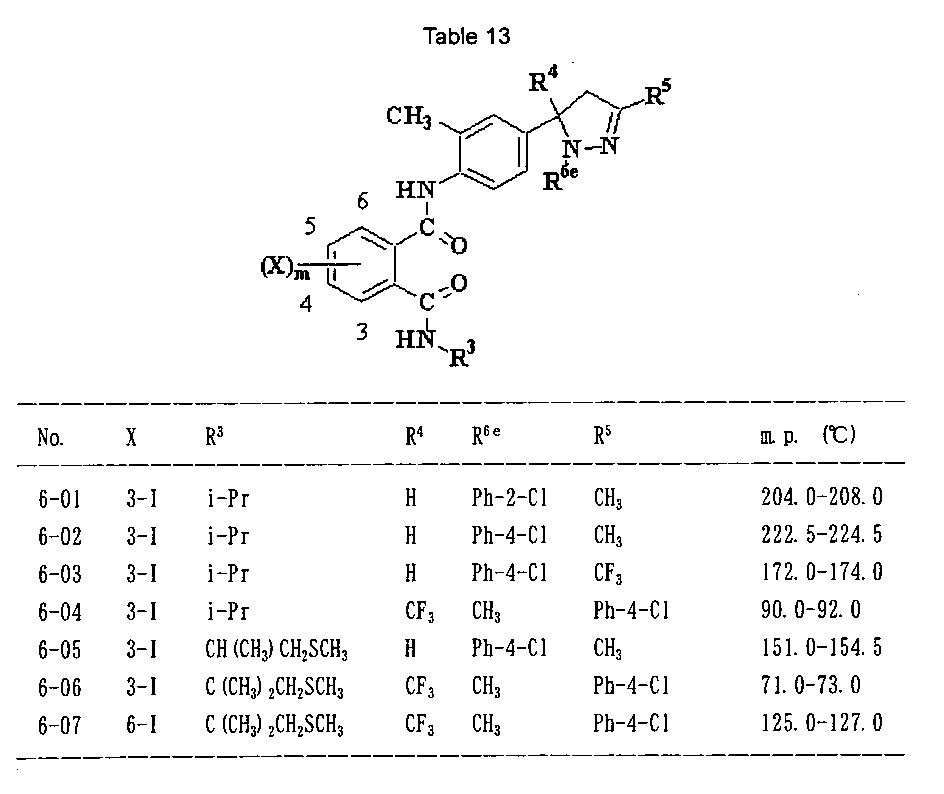

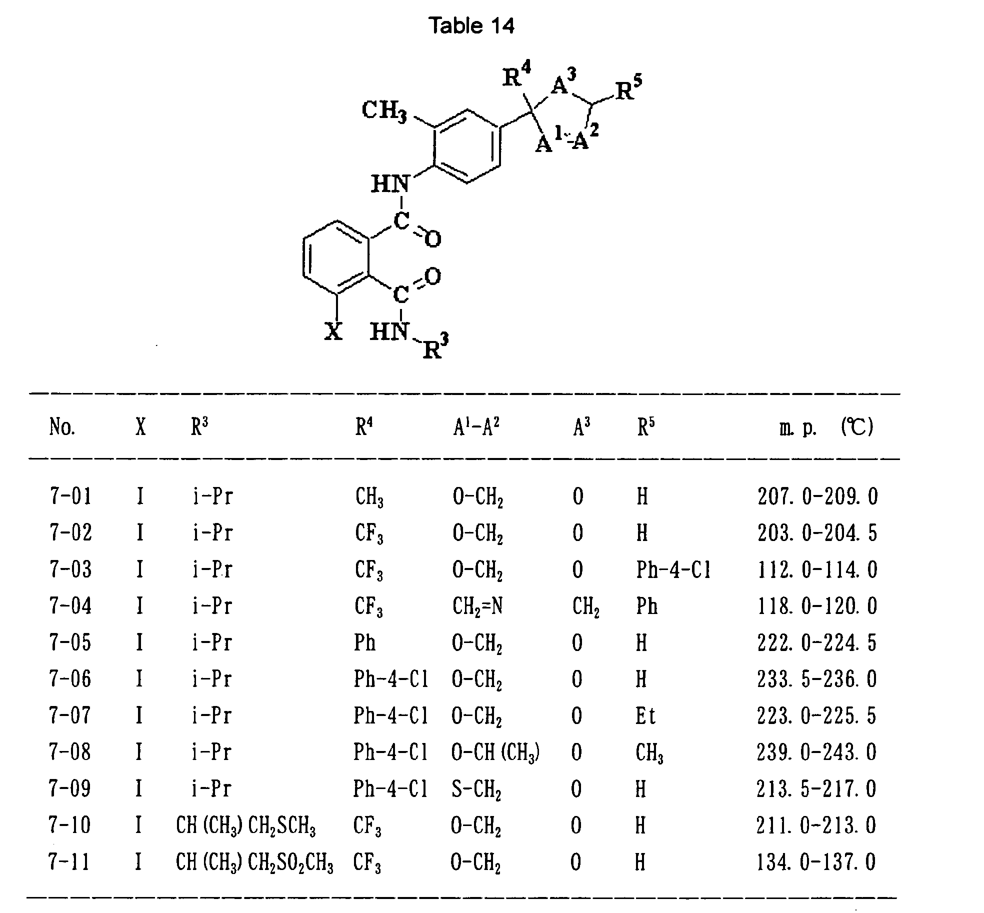

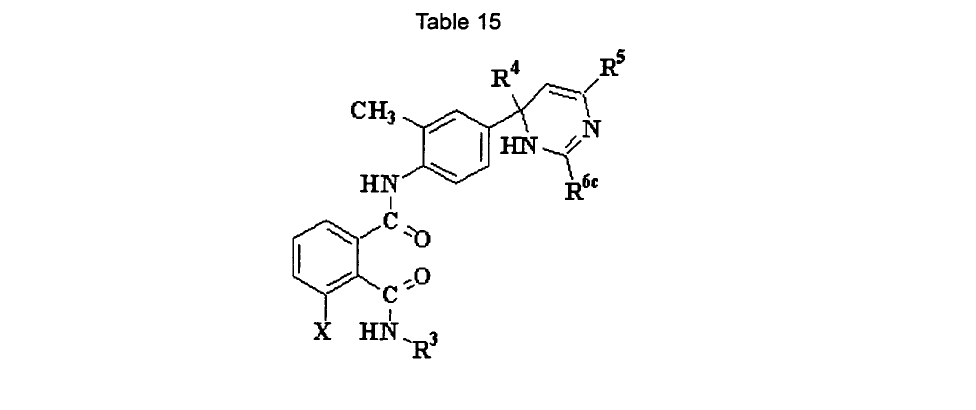

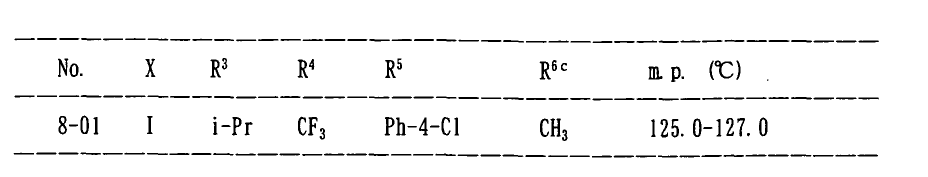

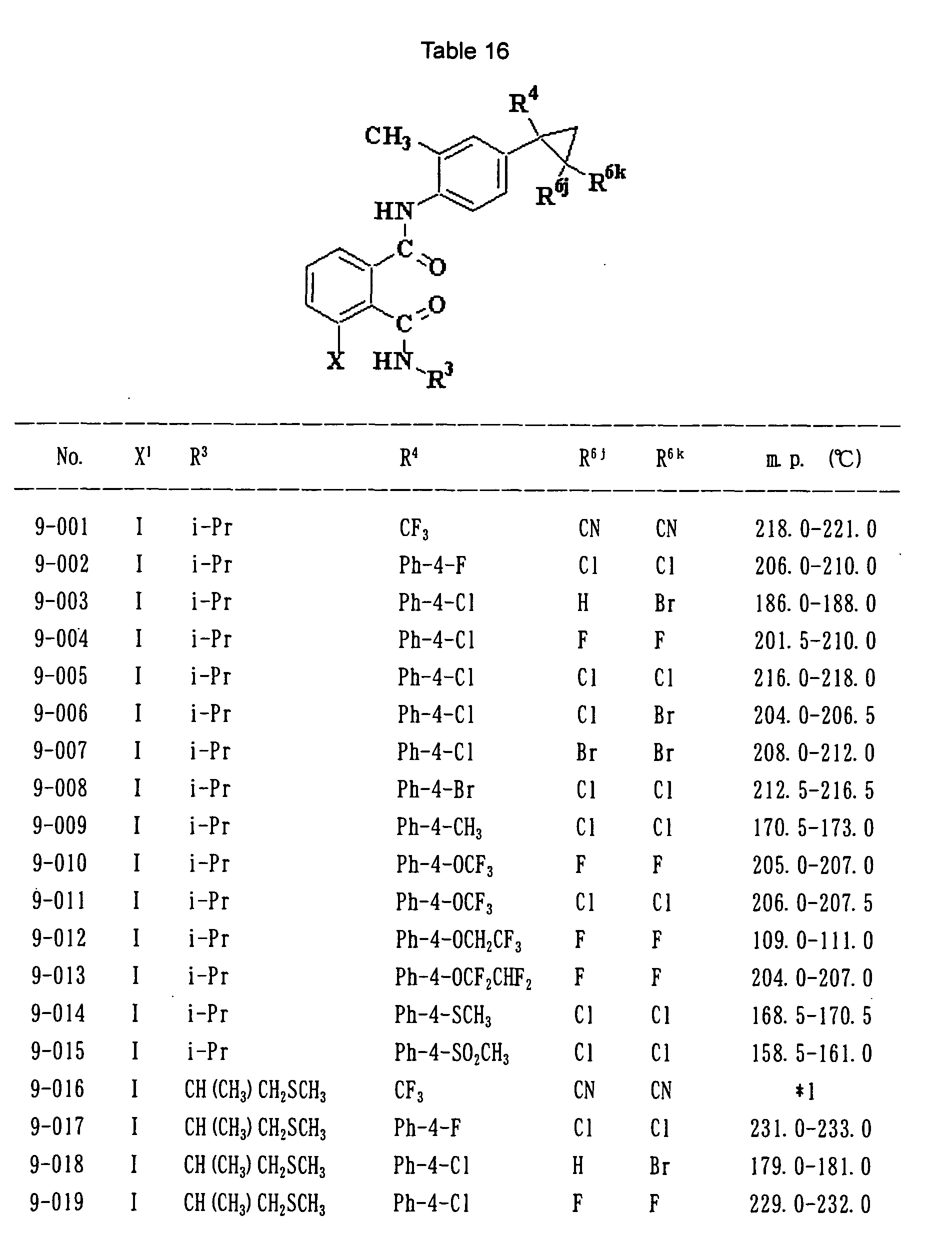

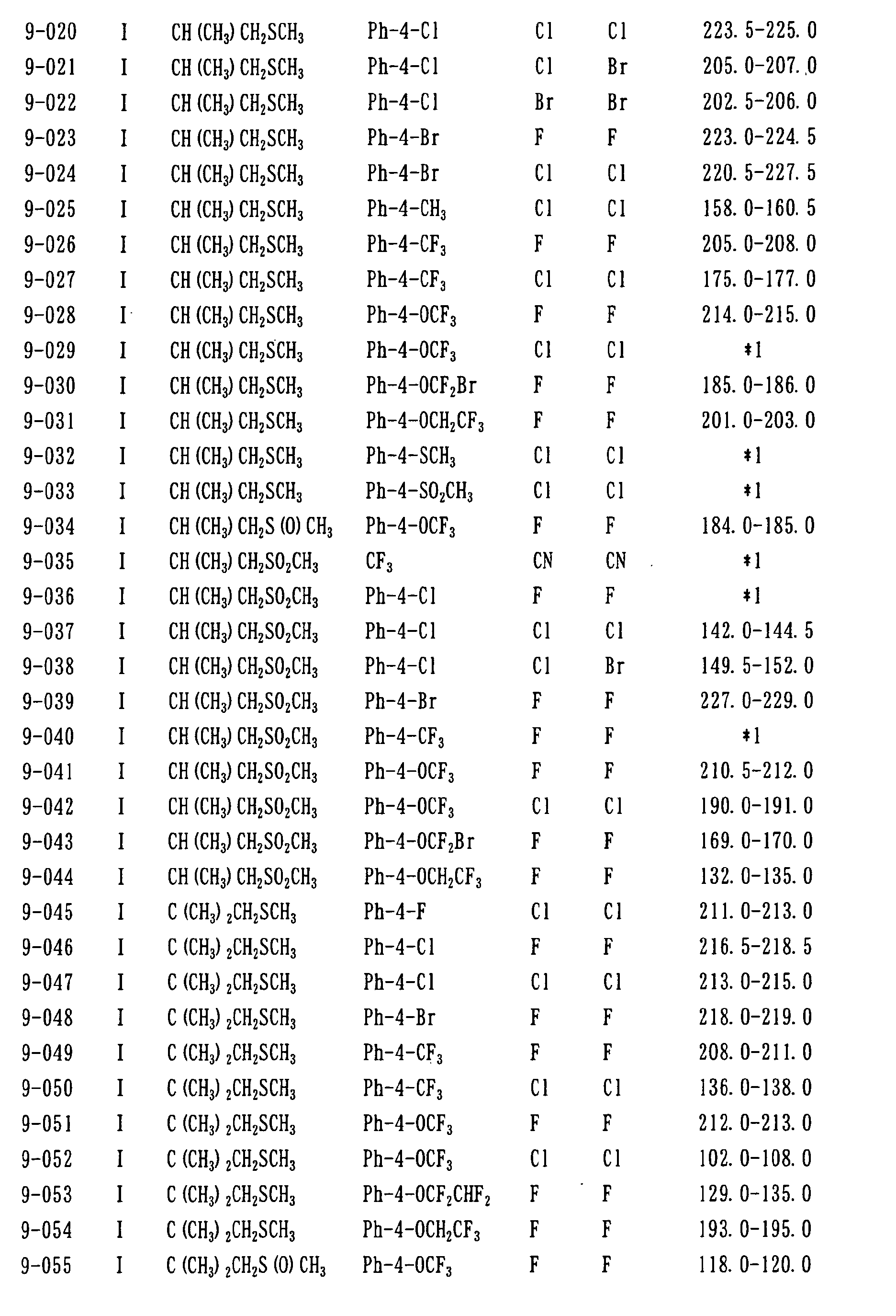

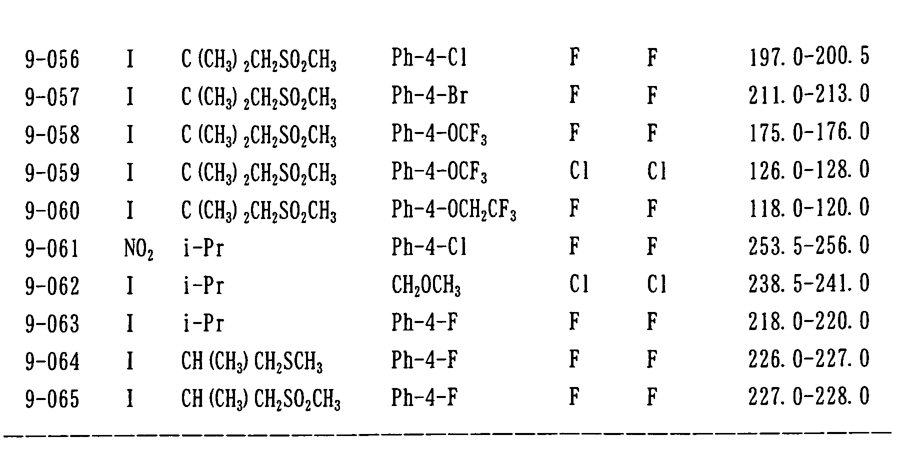

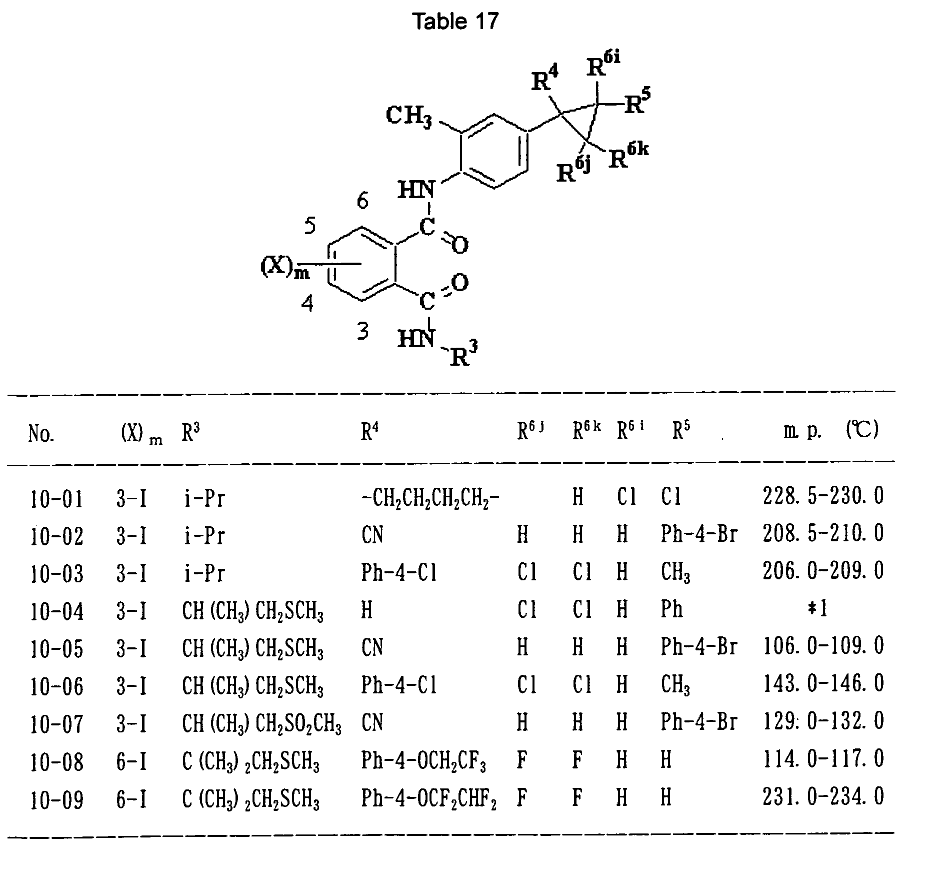

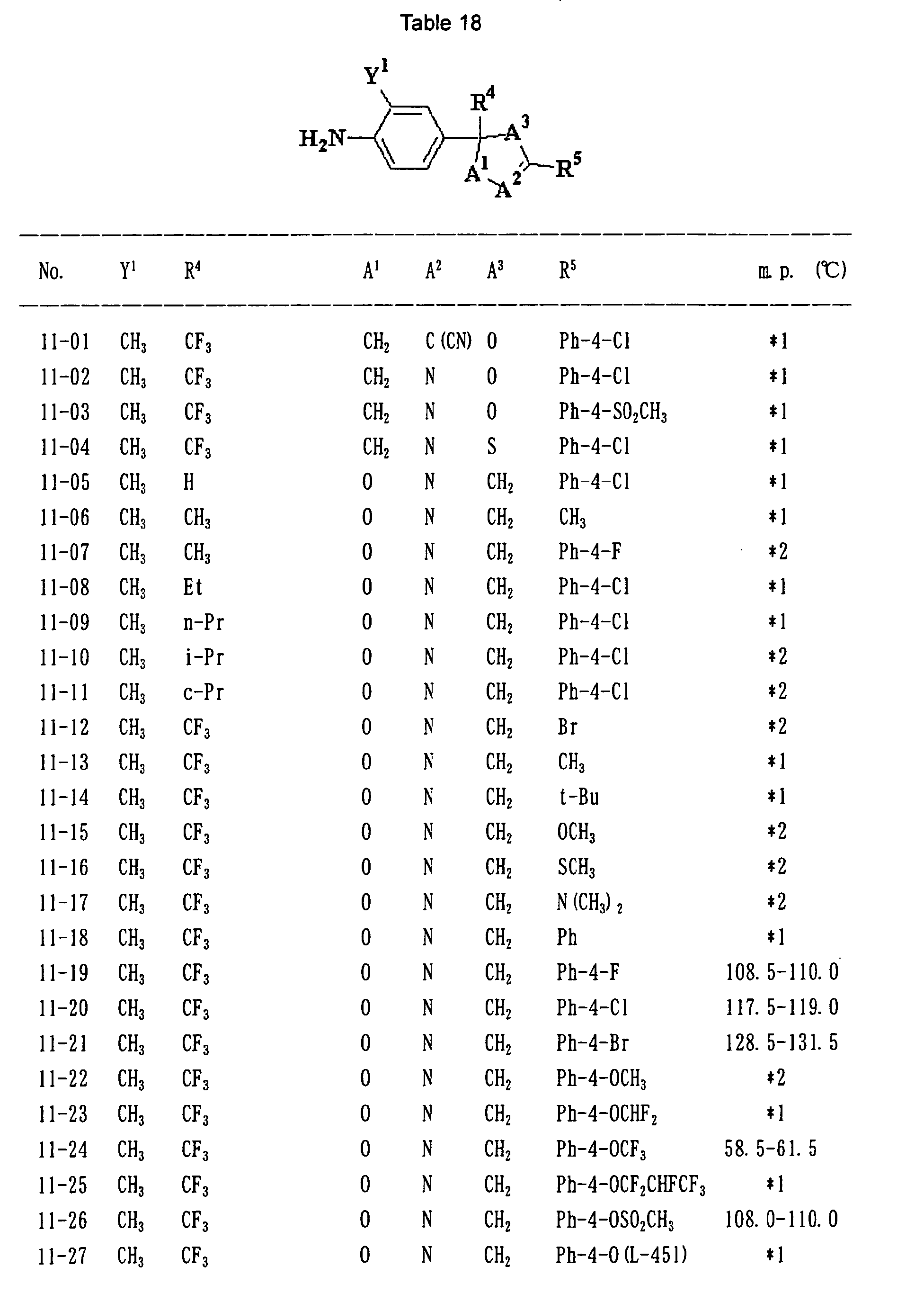

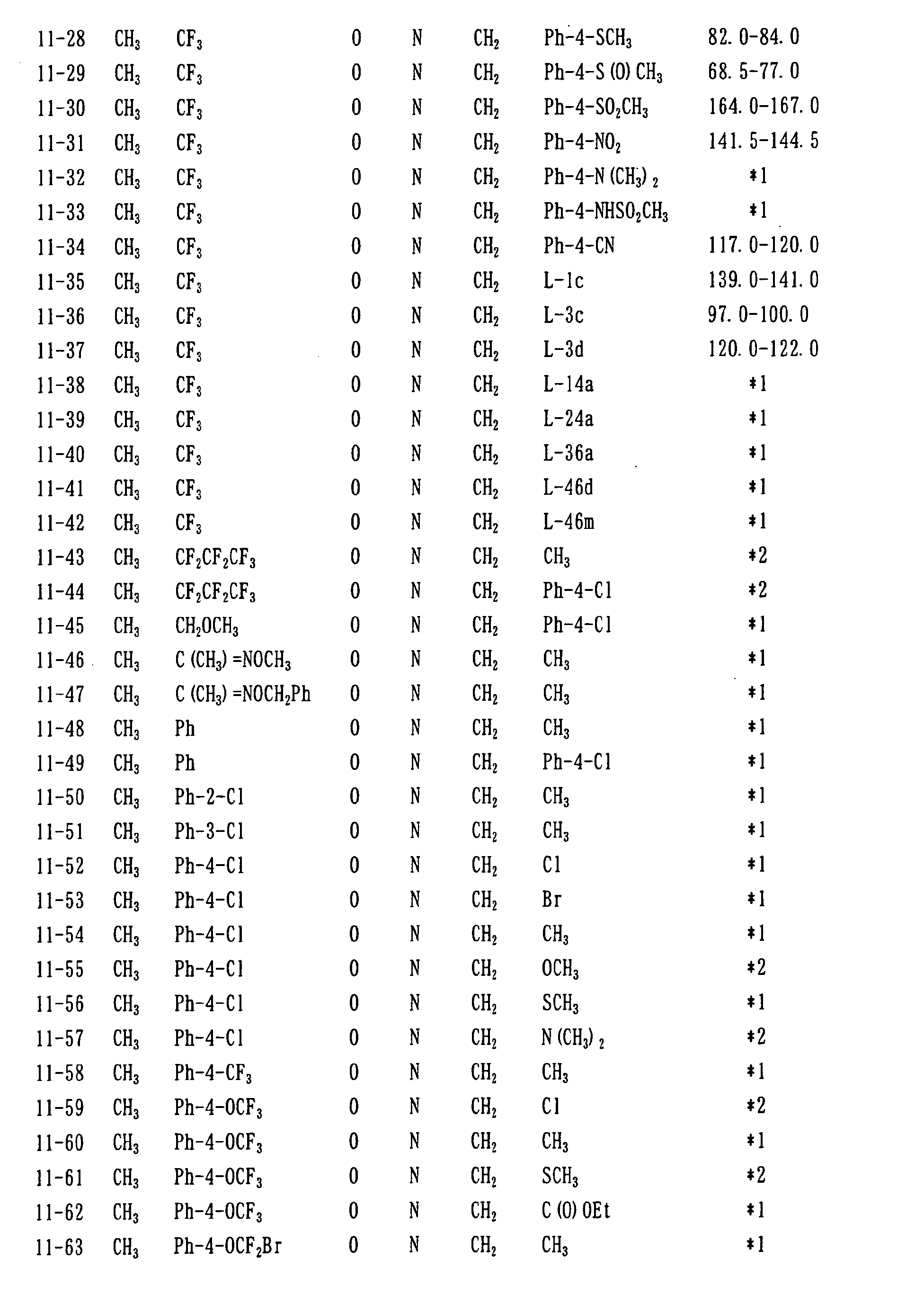

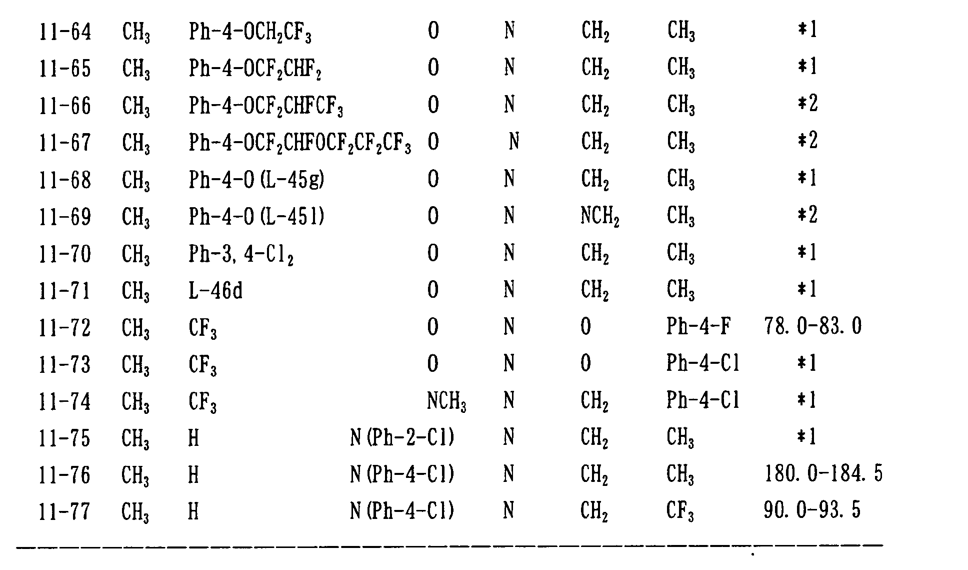

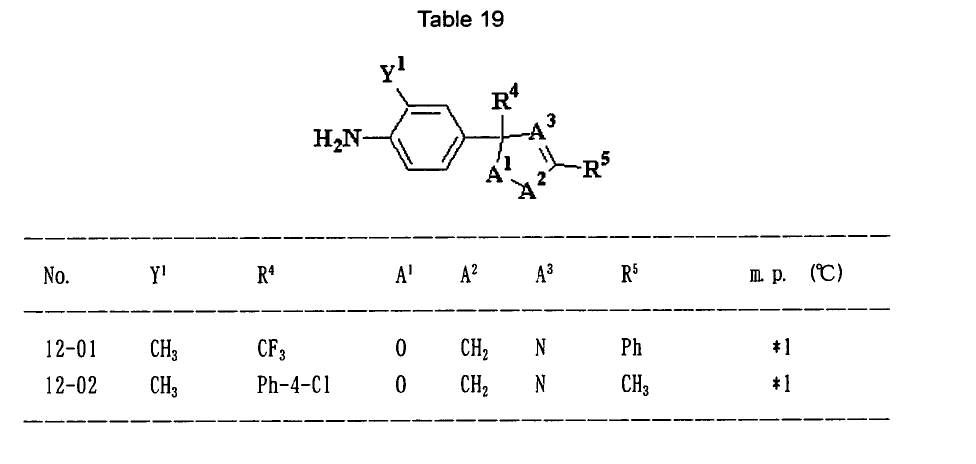

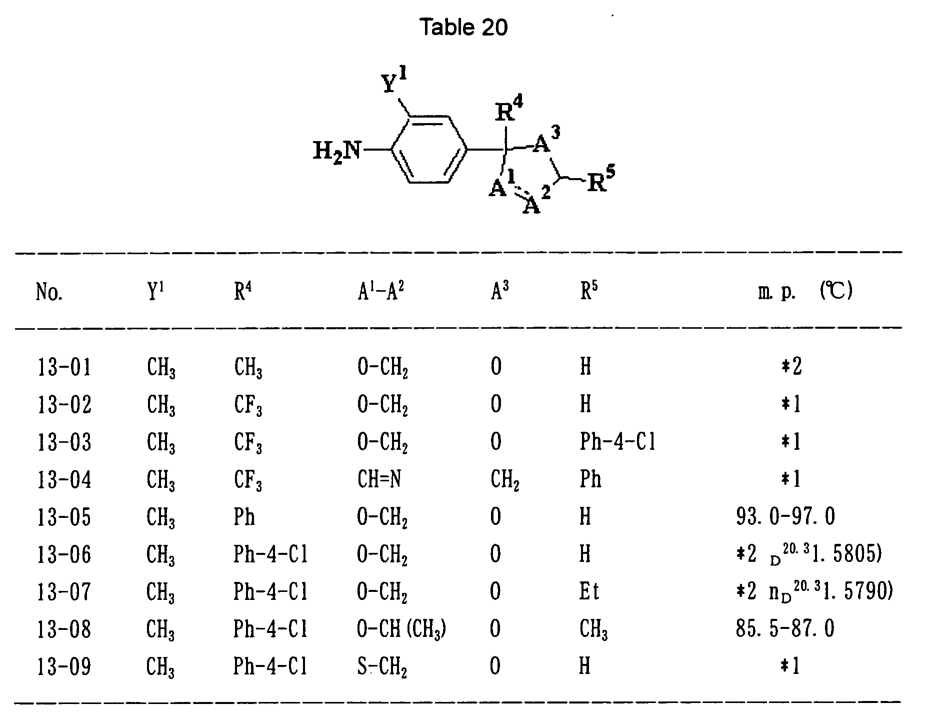

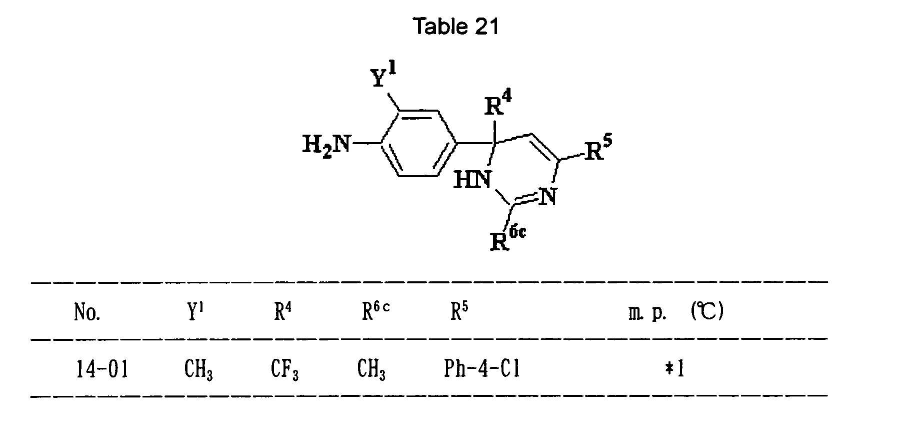

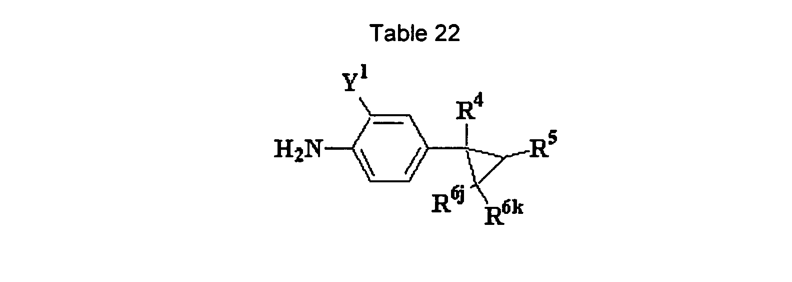

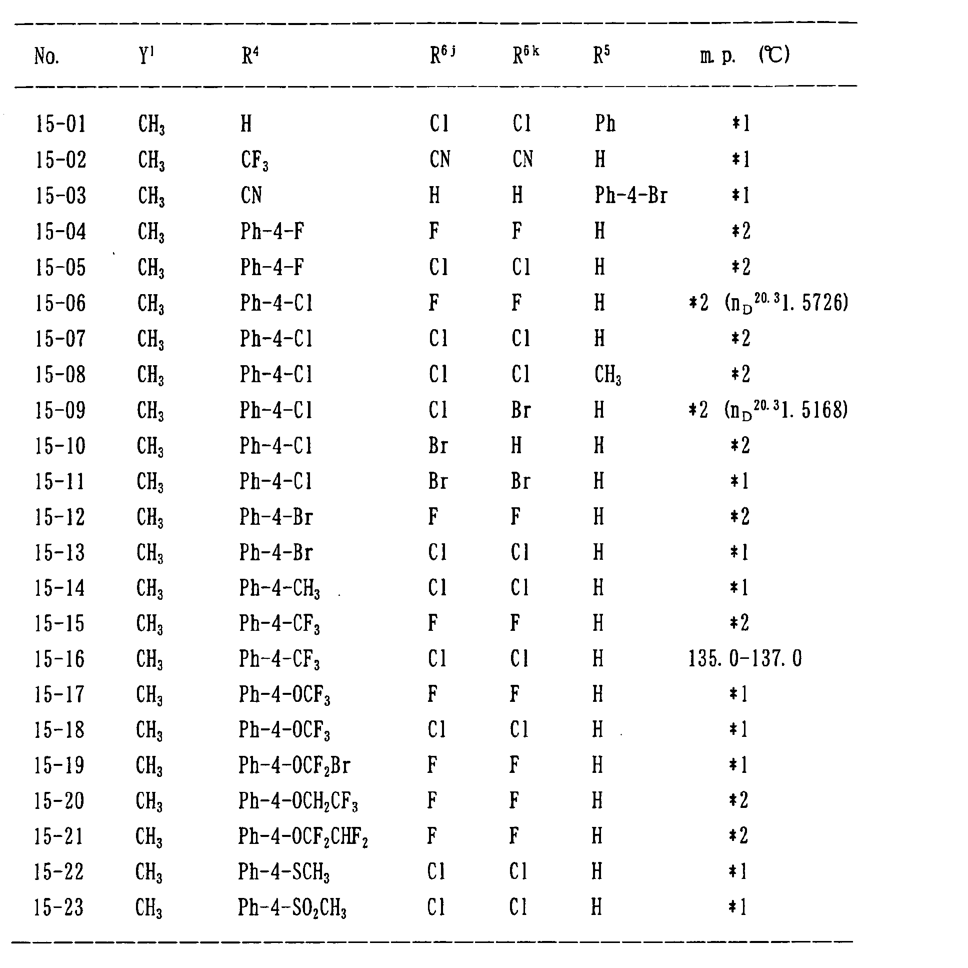

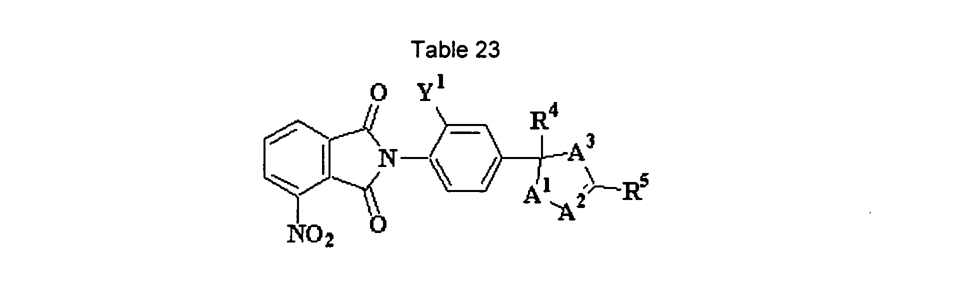

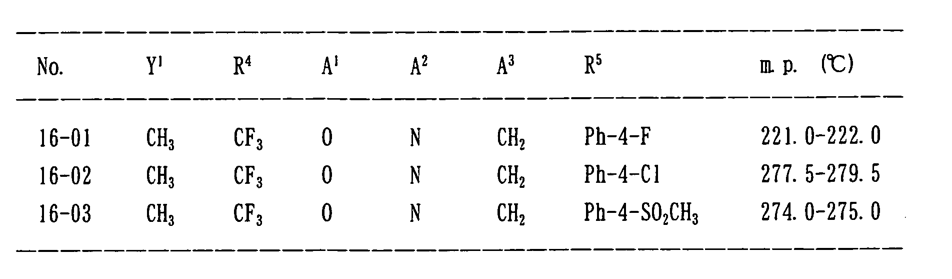

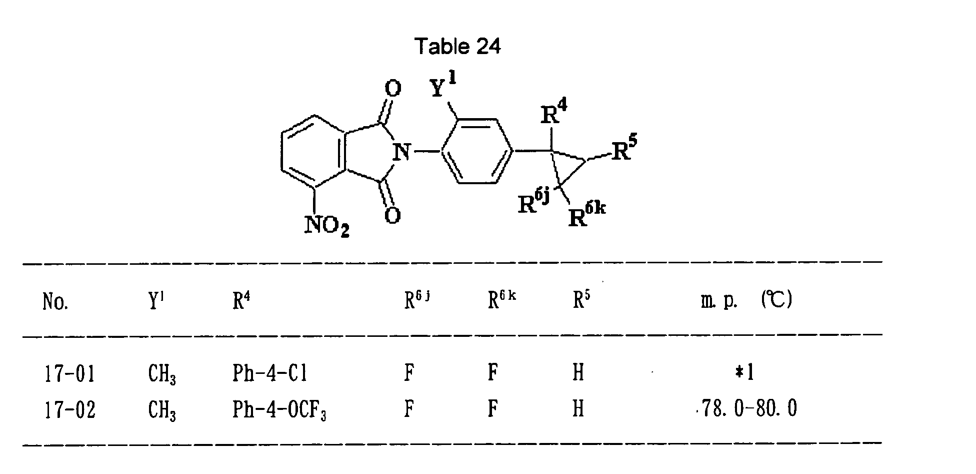

### [Test Examples]

Next, usefulness of the compound of the present invention as a noxious organism controlling agent is specifically explained in the following Test Examples, but the present invention is not limited by these alone.

### Test Example 1 Insecticidal test against Common cutworm

A 10% emulsifiable concentrate (depending on the compounds, 25% wettable powder was applied for the test) of the compound of the present invention was diluted with water containing a spreading agent to prepare a chemical solution with a concentration of 100 ppm. To the chemical solution was dipped leaves of Chinese olive for about 10 seconds, and after air-drying, they were placed in a laboratory dish, then, 10 Common cutworms (Spodoptera litura) with second instar larvae per the dish were released therein, and the dish was covered with a lid having holes and contained at a thermostat chamber at 25°C. A number of dead insect(s) after 6 days was counted and a rate of dead insects was calculated by the following calculation formula. Incidentally, the test was carried out with two districts.

Rate of dead insects (%) = (Number of dead insects/Number of released insects)x100

As a result, the following compounds showed 80% or more of insecticidal rate.

The compounds of the present invention: No. 1-004, 1-006, 1-012, 1-015, 1-016, 1-022, 1-024, 1-025, 1-026, 1-027, 1-028, 1-030, 1-031, 1-032, 1-033, 1-034, 1-035, 1-036, 1-037, 1-038, 1-039, 1-043, 1-044, 1-046, 1-047, 1-048, 1-049, 1-050, 1-051, 1-052, 1-053, 1-054, 1-057, 1-058, 2-001, 2-005, 2-007, 2-008, 2-009, 2-010, 2-011, 2-012, 2-013, 2-014, 2-015, 2-016, 2-017, 2-018, 2-019, 2-020, 2-021, 2-022, 2-024, 2-025, 2-026, 2-027, 2-028, 2-029, 2-030, 2-032, 2-033, 2-036, 2-037, 2-038, 2-040, 2-041, 2-042, 2-043, 2-044, 2-045, 2-046, 2-047, 2-048, 2-050, 2-051, 2-053, 2-054, 2-055, 2-057, 2-059, 2-060, 2-061, 2-062, 2-065, 2-066, 2-067, 2-068, 2-069, 2-070, 2-073, 2-075, 2-080, 2-082, 2-083, 2-084, 2-085, 2-086, 2-092, 2-093, 2-094, 2-095, 2-096, 2-097, 2-098, 2-100, 2-103, 2-104, 2-105, 2-106, 2-107, 2-108, 2-109, 2-110, 2-111, 2-112, 2-113, 2-114, 2-115, 2-116, 2-117, 2-119, 2-120, 2-121, 2-122, 2-123, 2-124, 2-125, 2-126, 2-127, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-152, 2-163, 2-164, 2-167, 2-169, 2-170, 2-171, 2-173, 2-174, 2-176, 2-179, 3-001, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-023, 3-024, 3-025, 3-026, 3-027, 3-028, 3-029, 3-032, 3-033, 3-034, 3-035, 3-037, 3-038, 3-039, 3-040, 3-041, 3-042, 3-043, 3-044, 3-045, 3-046, 3-047, 3-048, 3-049, 3-050, 3-051, 4-02, 4-03, 4-04, 4-05, 4-06, 5-01, 6-03, 6-04, 6-05, 6-06, 7-02, 7-03, 7-04, 7-10, 8-01, 9-002, 9-003, 9-004, 9-005, 9-006, 9-007, 9-008, 9-010, 9-011, 9-012, 9-013, 9-014, 9-015, 9-017, 9-019, 9-020, 9-021, 9-022, 9-023, 9-024, 9-026, 9-027, 9-028, 9-029, 9-030, 9-031, 9-034, 9-036, 9-037, 9-038, 9-039, 9-040, 9-041, 9-042, 9-043, 9-045, 9-046, 9-047, 9-048, 9-049, 9-050, 9-051, 9-052, 9-053, 9-054, 9-055, 9-056, 9-057, 9-058, 9-059, 9-060, 10-02, 10-05

### Test Example 2 Insecticidal test against diamondback moth

A 10% emulsifiable concentrate (depending on the compounds, 25% wettable powder was applied for the test) of the compound of the present invention was diluted with water containing a spreading agent to prepare a chemical solution with a concentration of 100 ppm. To the chemical solution was dipped leaves of Chinese olive for about 10 seconds, and after air-drying, they were placed in a laboratory dish, then, 10 diamondback moths (Plutella xylostella) with second instar larvae per the dish were released therein, and the dish was covered with a lid having holes and contained at a thermostat chamber at 25°C. A number of dead insect(s) after 6 days was counted and a rate of dead insects was calculated in the same manner as in Test Example 1. Incidentally, the test was carried out with two districts.

As a result, the following compounds showed 80% or more of insecticidal rate.

The compounds of the present invention: No. 1-001, 1-002, 1-004, 1-005, 1-006, 1-007, 1-008, 1-009, 1-011, 1-012, 1-013, 1-014, 1-015, 1-016, 1-017, 1-018, 1-019, 1-020, 1-021, 1-022, 1-024, 1-025, 1-026, 1-027, 1-028, 1-029, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 2-001, 2-002, 2-005, 2-006, 2-007, 2-008, 2-009, 2-010, 2-011, 2-012, 2-013, 2-014, 2-015, 2-016, 2-017, 2-018, 2-019, 2-020, 2-021, 2-022, 2-023, 2-024, 2-025, 2-026, 2-027, 2-028, 2-029, 2-030, 2-032, 2-033, 2-034, 2-036, 2-037, 2-038, 2-039, 2-040, 2-041, 2-042, 2-043, 2-044, 2-045, 2-046, 2-047, 2-048, 2-049, 2-050, 2-051, 2-052, 2-053, 2-054, 2-055, 2-057, 2-058, 2-059, 2-060, 2-061, 2-062, 2-063, 2-064, 2-065, 2-066, 2-067, 2-068, 2-069, 2-070, 2-071, 2-072, 2-073, 2-074, 2-075, 2-076, 2-077, 2-078, 2-079, 2-080, 2-082, 2-083, 2-084, 2-085, 2-086, 2-087, 2-088, 2-089, 2-090, 2-091, 2-092, 2-093, 2-094, 2-095, 2-096, 2-097, 2-098, 2-100, 2-101, 2-103, 2-104, 2-105, 2-106, 2-107, 2-108, 2-109, 2-110, 2-111, 2-112, 2-113, 2-114, 2-115, 2-116, 2-117, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-156, 2-157, 2-161, 2-162, 2-163, 2-164, 2-167, 2-168, 2-169, 2-170, 2-171, 2-172, 2-173, 2-174, 2-175, 2-176, 2-177, 2-178, 2-179, 3-001, 3-002, 3-003, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 3-033, 3-034, 3-035, 3-036, 3-037, 3-038, 3-039, 3-040, 3-041, 3-042, 3-043, 3-044, 3-045, 3-046, 3-047, 3-048, 3-049, 3-050, 3-051, 4-01, 4-02, 4-03, 4-04, 4-05, 4-06, 4-07, 5-01, 5-02, 6-01, 6-02, 6-03, 6-04, 6-05, 6-06, 7-01, 7-02, 7-03, 7-04, 7-05, 7-06, 7-07, 7-08, 7-09, 7-10, 7-11, 8-01, 9-001, 9-002, 9-003, 9-004, 9-005, 9-006, 9-007, 9-008, 9-009, 9-010, 9-011, 9-013, 9-014, 9-015, 9-016, 9-017, 9-018, 9-019, 9-020, 9-021, 9-022, 9-023, 9-024, 9-025, 9-026, 9-027, 9-028, 9-029, 9-032, 9-033, 9-034, 9-035, 9-036, 9-037, 9-038, 9-039, 9-041, 9-045, 9-046, 9-047, 9-048, 9-049, 9-050, 9-051, 9-053, 9-055, 9-056, 9-057, 9-058, 9-061, 10-01, 10-02, 10-03, 10-04, 10-05, 10-06, 10-09

### Utilizability in industry

The substituted benzanilide compounds according to the present invention are extremely useful compounds showing an excellent noxious organism controlling activity, particularly an insecticidal and acaricidal activity, and causing substantiall no bad effect against non-target organisms such as mammals, fishes and useful insects.

## Claims

1. A substituted benzanilide compound represented by the formula (1): 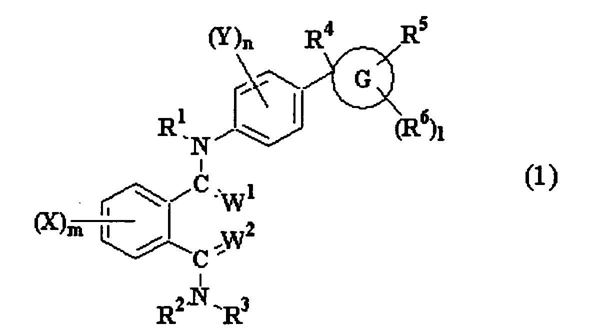 [wherein G represents a 5-membered or 6-membered non-aromatic heterocyclic ring containing at least one atom selected from an oxygen atom, a sulfur atom and a nitrogen atom, and having at least one double bond in the ring, a 5-membered or 6-membered saturated heterocyclic ring containing two atoms selected from an oxygen atom, a sulfur atom and a nitrogen atom or a 3-membered to 6-membered cycloalkyl ring,
W¹ and W² each independently represent an oxygen atom or a sulfur atom,
X represents a halogen atom, cyano, nitro, azide, -SCN, -SF₅, a C₁ to C₆ alkyl, a (C₁ to C₆)alkyl which may be optionally substituted by R⁷, a C₃ to C₈ cycloalkyl, a (C₃ to C₈)cycloalkyl which may be optionally substituted by R⁷, C₂ to C₆ alkenyl, a (C₂ to C₆)alkenyl which may be optionally substituted by R⁷, a C₃ to C₈ cycloalkenyl, a C₃ to C₈ halocycloalkenyl, a C₂ to C₆ alkynyl, a (C₂ to C₆)alkynyl which may be optionally substituted by R⁷, -OH, -OR⁸, -OS(O)₂R⁸, -SH, -S(O)ᵣR⁸, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, C(S)N(R¹⁰)R⁹, -CH=NOR¹¹, -C(R⁹)=NOR¹¹, -S(O)₂OR⁹, -S(O)₂NHR¹⁰, -S(O)₂N(R¹⁰)R⁹, -Si(R¹³)(R¹⁴)R¹², phenyl which may be substituted by (Z¹)ₚ₁, L or M, when m represents 2, 3 or 4, each of X's may be the same with each other or may be different from each other, further, when two Xs are adjacent to each other, the adjacent two Xs may form -CH₂CH₂CH₂-, -CH₂CH₂O-, -CH₂OCH₂-, -OCH₂O-, -CH₂CH₂S-, -CH₂SCH₂-, -CH₂CH₂N(R¹⁵)-, -CH₂N(R¹⁵)CH₂-, -CH₂CH₂CH₂CH₂-, -CH₂CH₂CH₂O-, -CH₂CH₂OCH₂-, -CH₂OCH₂O-, -OCH₂CH₂O-, -OCH₂CH₂S-, -CH₂CH=CH-, -OCH=CH-, -SCH=CH-, -N(R¹⁵)CH=CH-, -OCH=N-, -SCH=N-, -N(R¹⁵)CH=N-, -N(R¹⁵)N=CH-, -CH=CHCH=CH-, -OCH₂CH=CH-, -N=CHCH=CH-, -N=CHCH=N- or -N=CHN=CH-, so that the two Xs form a 5-membered ring or 6-membered ring with the carbon atoms to which the two Xs are bonded, and at this time, the hydrogen atom(s) bonded to each of the carbon atom(s) which form(s) the ring which may be optionally substituted by Z¹, and further, when it is substituted by two or more Z¹s simultaneously, each of Z¹s may be the same with each other or may be different from each other,
and, when G represents a 5-membered or 6-membered non-aromatic heterocyclic ring containing at least one atom selected from an oxygen atom, a sulfur atom and a nitrogen atom, and having at least one double bond in the ring, X may further represent -N(R¹⁷)R¹⁶, -N=CHOR¹² or -N=C(R⁹)OR¹²,
Y represents a halogen atom, cyano, nitro, azide, -SCN, -SF₅, a C₁ to C₆ alkyl, a (C₁ to C₆)alkyl which may be optionally substituted by R⁷, a C₃ to C₈ cycloalkyl, a (C₃ to C₈)cycloalkyl which may be optionally substituted by R⁷, -OH, -OR⁸, -OS(O)₂R⁸, -SH, -S(O)ᵣR⁸, -NH₂, a C₁ to C₆ alkylamino, a di(C₁ to C₆ alkyl)amino, -Si(R¹³)(R¹⁴)R¹², phenyl which may be substituted by (Z¹)ₚ₁, L or M, when n represents 2, 3 or 4, each of Ys may be the same with each other or may be different from each other, and further, when two Ys are adjacent to each other, the adjacent two Ys may form -CH₂CH₂CH₂-, -CH₂CH₂O-, -CH₂OCH₂-, -OCH₂O-, -CH₂CH₂S-, -CH₂SCH₂-, -SCH₂S-, -CH₂CH₂CH₂CH₂-, -CH₂CH₂CH₂O-, -CH₂CH₂OCH₂-, -CH₂OCH₂O-, -OCH₂CH₂O-, -OCH₂CH₂S-, -SCH₂CH₂S-, -OCH=N- or -SCH=N-, so that the two Ys may form a 5-membered ring or 6-membered ring with carbon atoms to which the two Ys are bonded, and at this time, the hydrogen atom(s) bonded to each of the carbon atom(s) which form(s) the ring which may be optionally substituted by Z¹, and further, when it is substituted by two or more Z¹s simultaneously, each of Z¹s may be the same with each other or may be different from each other,
R¹, R² and R³ each independently represent a hydrogen atom, cyano, a C₁ to C₁₂ alkyl, a (C₁ to C₁₂)alkyl which may be optionally substituted by R¹⁸, a C₃ to C₁₂ cycloalkyl, a (C₃ to C₁₂)cycloalkyl which may be optionally substituted by R¹⁸, a C₃ to C₁₂ alkenyl, a (C₃ to C₁₂)alkenyl which may be optionally substituted by R¹⁸, C₃ to C₁₂ a cycloalkenyl, C₃ to C₁₂ halocycloalkenyl, a C₃ to C₁₂ alkynyl, a (C₃ to C₁₂)alkynyl which may be optionally substituted by R¹⁸, -OH, a C₁ to C₈ alkoxy, a C₃ to C₈ alkenyloxy, a C₃ to C₈ haloalkenyloxy, phenoxy which may be substituted by (Z¹)ₚ₁, a phenyl(C₁ to C₄)alkoxy which may be substituted by (Z¹)ₚ₁, a C₁ to C₆ alkylthio, a C₁ to C₆ haloalkylthio, phenylthio which may be substituted by (Z¹)ₚ₁, -S(O)₂R⁹, -SN(R²⁰)R¹⁹, -S(O)₂N(R¹⁰)R⁹, -N(R²²)R²¹, -C(O)R⁹, -C(O)OR⁹, -C(O)SR⁹, -C(O)N(R¹⁰)R⁹, -C(S)OR⁹, -C(S)SR⁹, -C(S)N(R¹⁰)R⁹, phenyl which may be substituted by (Z¹)ₚ₁, L or M, or R² and R³ are combined to form a C₂ to C₆ alkylene chain, so that they may form a 3- to 7-membered ring with the nitrogen atom(s) to which they are bonded, the alkylene chain at this time may contain one oxygen atom, sulfur atom or nitrogen atom, and may be optionally substituted by a halogen atom, a C₁ to C₆ alkyl group, a C₁ to C₆ haloalkyl group, a C₁ to C₆ alkoxy group, a C₁ to C₆ alkylcarbonyl group or a C₁ to C₆ alkoxycarbonyl group,
R⁴ represents a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², -P(O)(OR²⁴)₂, phenyl which may be substituted by (Z²)ₚ₁, 1-naphthyl, 2-naphthyl, L or M,
R⁵ represents
(a). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, phenyl which may be substituted by (Z²)ₚ₁, 1-naphthyl, 2-naphthyl, L or M, when G represents a 5-membered or 6-membered non-aromatic heterocyclic ring containing at least one atom selected from an oxygen atom, a sulfur atom and a nitrogen atom, and having at least one double bond in the ring, or a 5-membered or 6-membered saturated heterocyclic ring containing two atoms selected from an oxygen atom, a sulfur atom and a nitrogen atom,
(b). cyano, a (C₁ to C₆)alkyl which may be optionally substituted by R²³, a (C₁ to C₆)-haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents cyano, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -CH=NOR¹¹, -C(R⁹)=NOR¹¹, -Si(R¹³)(R¹⁴)R¹², -P(O)(OR²⁴)₂, phenyl which may be substituted by (Z²)ₚ₁, 1-naphthyl, 2-naphthyl, L or M,
R⁶ represents a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₁ to C₆ alkoxy, a C₁ to C₆ haloalkoxy, a C₁ to C₆ alkylthio, a C₁ to C₆ haloalkylthio, -S(O)₂R⁹, -C(O)R⁹, -C(O)OR⁹, -C(O)SR⁹, -C(S)OR⁹, -C(S)SR⁹, -C(O)N(R¹⁰)R⁹, -C(S)N(R¹⁰)R⁹, -P(O)(OR²⁴)₂, -P(S)(OR²⁴)₂, phenyl which may be substituted by (Z²)ₚ₁, 1-naphthyl, 2-naphthyl, L or M, when I represents an integer of 2 or more, each of R⁶s may be the same with each other or may be different from each other, and when R⁶ and R⁴ are adjacent to each other, the adjacent R⁴ and R⁶ are combined to form a C₃ to C₅ alkylene chain, so that they may form a 5 to 7-membered ring with atoms to which they are bonded, and the alkylene chain at this time may contain one oxygen atom or sulfur atom, and may be which may be optionally substituted by a C₁ to C₆ alkyl group,
L represents an aromatic heterocyclic ring represented by either one of the formula L-1 to the formula L-58, 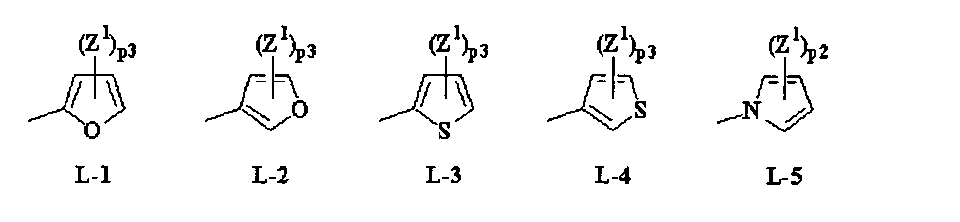 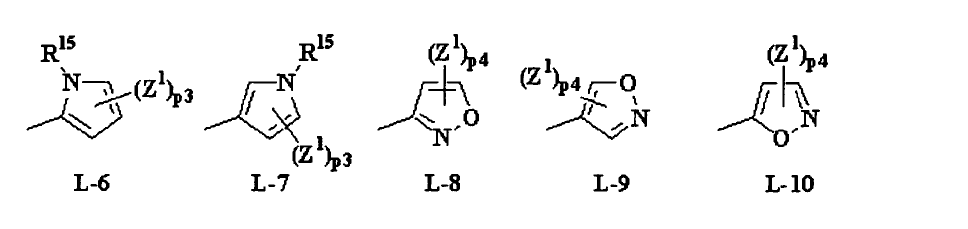 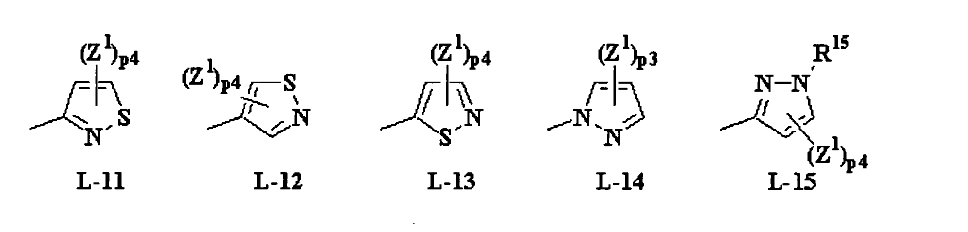 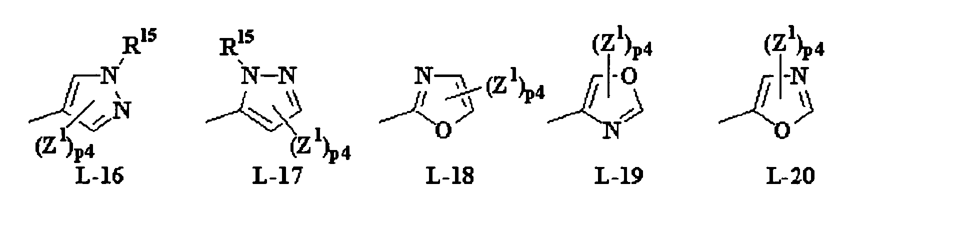 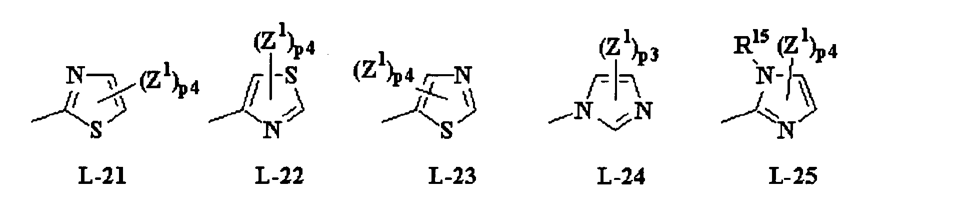 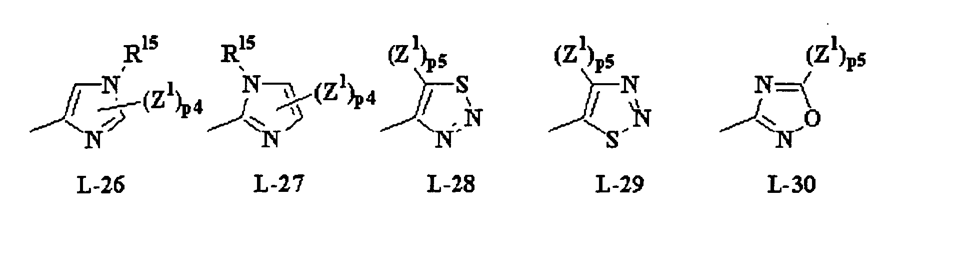 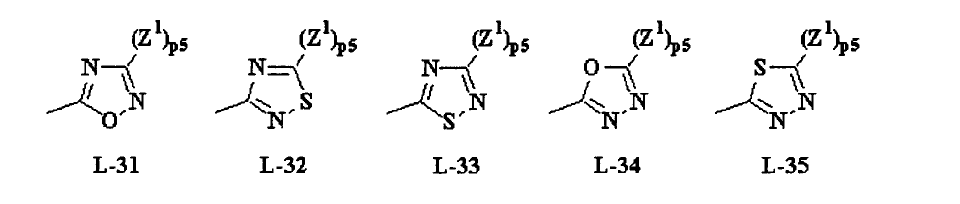 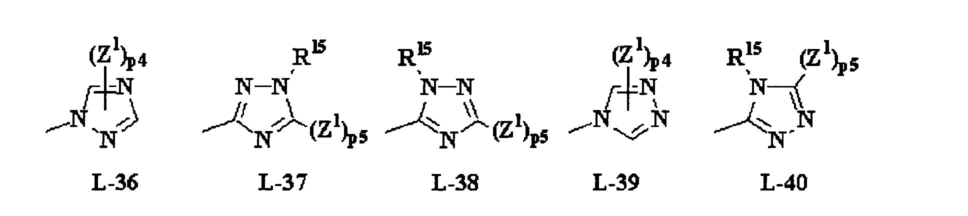 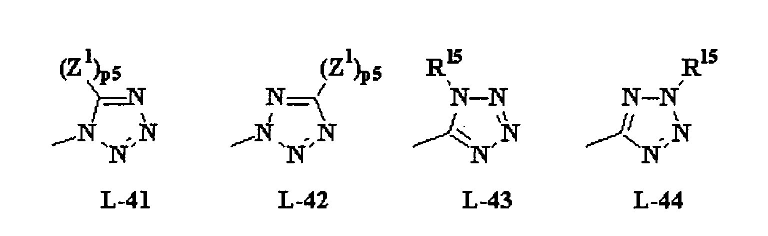 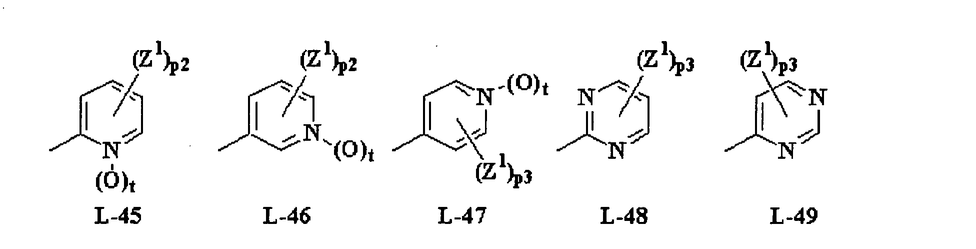 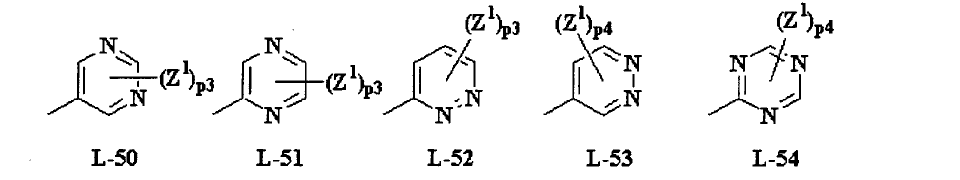 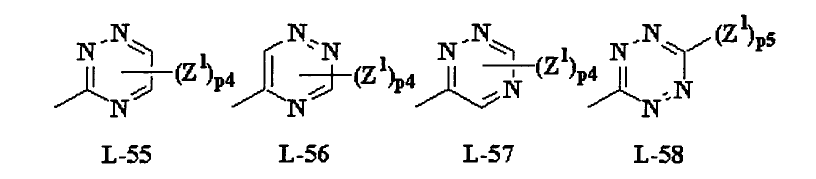 M represents a saturated heterocyclic ring represented by either one of the formula M-1 to the formula M-28, 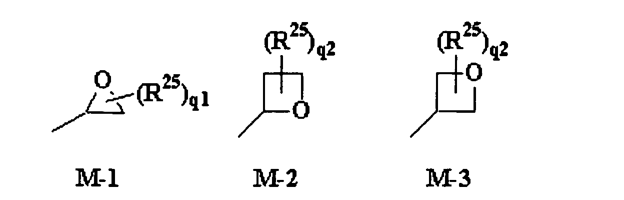 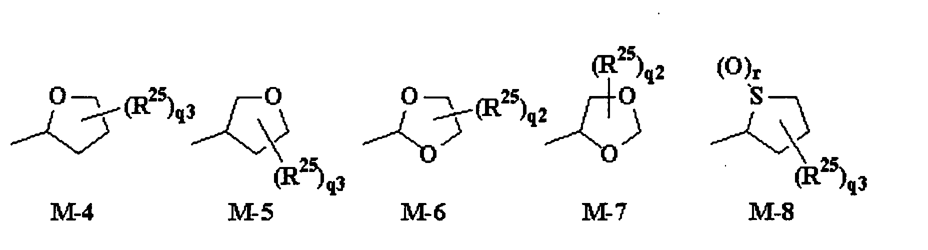 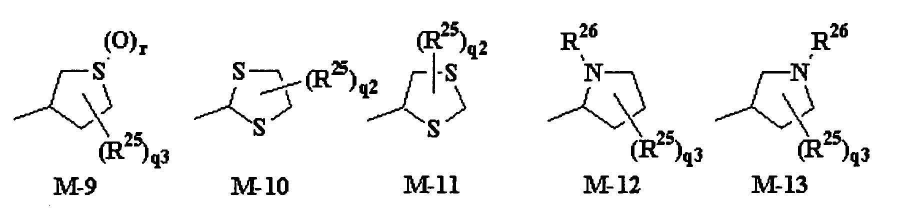 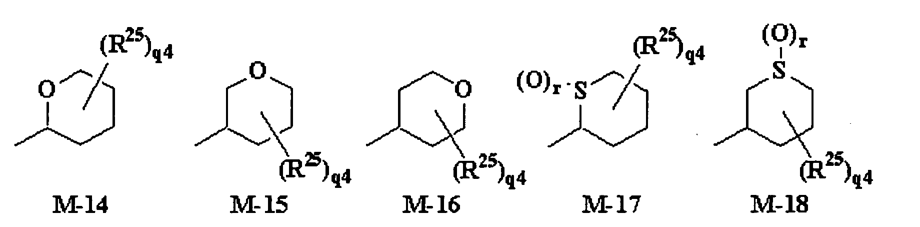 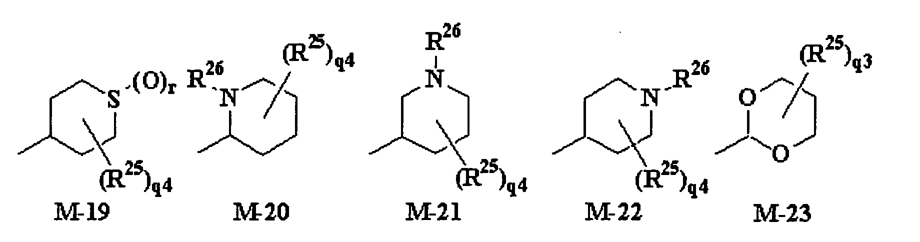 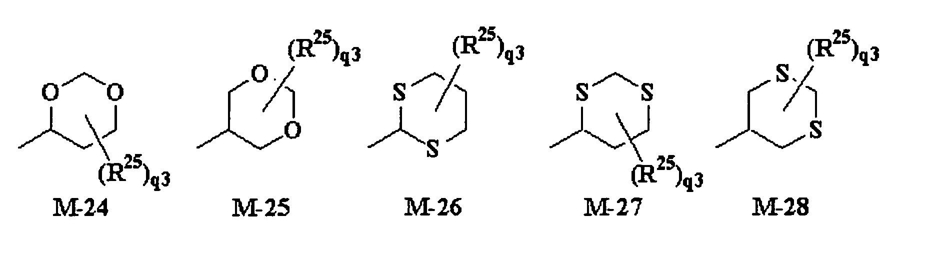 Z¹ represents a halogen atom, cyano, nitro, a C₁ to C₆ alkyl, a C₁ to C₆ haloalkyl, a C₃ to C₆ cycloalkyl, a C₃ to C₆ halocycloalkyl, a C₂ to C₆ alkenyl, a C₂ to C₆ haloalkenyl, a C₂ to C₆ alkynyl, a C₂ to C₆ haloalkynyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a C₁ to C₆ alkylamino, a di(C₁ to C₆ alkyl)amino, a C₁ to C₆ alkoxycarbonyl, a C₁ to C₆ haloalkoxycarbonyl or phenyl which may be optionally substituted by a halogen atom, when p1, p2, p3 or p4 represents an integer of 2 or more, each of Z¹s may be the same with each other or may be different from each other,
Z² represents a halogen atom, cyano, nitro, amino, azide, -SCN, -SF₅, a C₁ to C₆ alkyl, a (C₁ to C₆)alkyl which may be optionally substituted by R⁷, a C₃ to C₈ cycloalkyl, a (C₃ to C₈)cycloalkyl which may be optionally substituted by R⁷, a C₂ to C₆ alkenyl, a (C₂ to C₆)alkenyl which may be optionally substituted by R⁷, a C₃ to C₈ cycloalkenyl, a C₃ to C₈ halocycloalkenyl, a C₂ to C₆ alkynyl, a (C₂ to C₆)alkynyl which may be optionally substituted by R⁷, -OH, -OR⁸, -OS(O)₂R⁸, -SH, -S(O)ᵣR⁸, -N(R¹⁰)R⁹, -N(R¹⁰)CHO, -N(R¹⁰)C(O)R⁹, -N(R¹⁰)C(O)OR⁹, -N(R¹⁰)C(O)SR⁹, -N(R¹⁰)C(S)OR⁹, -N(R¹⁰)C(S)SR⁹, -N(R¹⁰)S(O)₂R⁹, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -S(O)₂OR⁹, -S(O)₂NHR¹⁰, -S(O)₂N(R¹⁰)R⁹, -Si(R¹³)(R¹⁴)R¹², phenyl which may be substituted by (Z¹)ₚ₁, L or M, when p1 represents an integer of 2 or more, each of Z²s may be the same with each other or may be different from each other, and further, when two Z²s are adjacent to each other, the adjacent two Z²s form -CH₂CH₂CH₂-, -CH₂CH₂O-, -CH₂OCH₂-, -OCH₂O-, -CH₂CH₂S-, -CH₂SCH₂-, -CH₂CH₂CH₂CH₂-, -CH₂CH₂CH₂O-, -CH₂CH₂OCH₂-, -CH₂OCH₂O-, -OCH₂CH₂O- or -OCH₂CH₂S-, so that the two Z²s may form a 5-membered ring or 6-membered ring with carbon atoms to which the two Z²s are bonded, and at this time, the hydrogen atom(s) bonded to each of the carbon atom(s) which form(s) the ring may be optionally substituted by a halogen atom or a C₁ to C₆ alkyl group,
R⁷ represents a halogen atom, cyano, a C₃ to C₈ cycloalkyl, a C₃ to C₈ halocycloalkyl, -OH, -OR⁸, -SH, -S(O)ᵣR⁸, -N(R¹⁰)R⁹, -N(R¹⁰)CHO, -N(R¹⁰)C(O)R⁹, -N(R¹⁰)C(O)OR⁹, -N(R¹⁰)C(O)SR⁹, -N(R¹⁰)C(S)OR⁹, -N(R¹⁰)C(S)SR⁹, -N(R¹⁰)S(O)₂R⁹, -C(O)OR⁹, -C(O)N(R¹⁰)R⁹, -Si(R¹³)(R¹⁴)R¹², phenyl which may be substituted by (Z¹)ₚ₁, L or M,
R⁸ represents a C₁ to C₆ alkyl, a (C₁ to C₆)alkyl which may be optionally substituted by R²⁷, a C₃ to C₈ cycloalkyl, a (C₃ to C₈)cycloalkyl which may be optionally substituted by R²⁷, a C₂ to C₆ alkenyl, a (C₂ to C₆)alkenyl which may be optionally substituted by R²⁷, a C₃ to C₈ cycloalkenyl, a C₃ to C₈ halocycloalkenyl, a C₃ to C₆ alkynyl, a (C₃ to C₆)alkynyl which may be optionally substituted by R²⁷, phenyl which may be substituted by (Z¹)ₚ₁, L or M,
R⁹ represents a C₁ to C₆ alkyl, a C₁ to C₆ haloalkyl, a C₃ to C₆ cycloalkyl(C₁ to C₄)alkyl, a C₁ to C₆ alkoxy(C₁ to C₄)alkyl, a C₁ to C₆ alkylthio(C₁ to C₄)alkyl, a cyano(C₁ to C₆)alkyl, a phenyl(C₁ to C₄)alkyl which may be substituted by (Z¹)ₚ₁, a L-(C₁ to C₄)alkyl, a M-(C₁ to C₄)alkyl, a C₃ to C₈ cycloalkyl, a C₃ to C₈ halocycloalkyl, a C₃ to C₆ alkenyl, a C₃ to C₆ haloalkenyl, a C₃ to C₆ alkynyl or phenyl which may be substituted by (Z¹)ₚ₁,
R¹⁰ represents a hydrogen atom or a C₁ to C₆ alkyl, or R⁹ and R¹⁰ are combined to form a C₂ to C₆ alkylene chain, so that they may form a 3- to 7-membered ring with the atom(s) to which they are bonded, and the alkylene chain at this time may contain one oxygen atom, sulfur atom or nitrogen atom, and may be optionally substituted by a halogen atom, a C₁ to C₆ alkyl group, a C₁ to C₆ alkoxy group, formyl group, a C₁ to C₆ alkylcarbonyl group or a C₁ to C₆ alkoxycarbonyl group,
R¹¹ represents a hydrogen atom, a C₁ to C₆ alkyl, a C₁ to C₆ haloalkyl, a phenyl(C₁ to C₄)alkyl which may be substituted by (Z¹)ₚ₁, a C₃ to C₆ alkenyl, a C₃ to C₆ haloalkenyl, a C₃ to C₆ alkynyl or a C₃ to C₆ haloalkynyl, or R⁹ and R¹¹ are combined to form a C₂ to C₄ alkylene chain, so that they may form a 5 to 7-membered ring with the atom(s) to which they are bonded, and the alkylene chain at this time may contain one oxygen atom, sulfur atom or nitrogen atom, and may be optionally substituted by a halogen atom or a C₁ to C₆ alkyl group,
R¹² represents a C₁ to C₆ alkyl, a C₁ to C₆ haloalkyl, a C₃ to C₆ alkenyl or phenyl which may be substituted by (Z¹)ₚ₁,
R¹³ and R¹⁴ each independently represent a C₁ to C₆ alkyl or a C₁ to C₆ haloalkyl,
R¹⁵ represents a hydrogen atom, a C₁ to C₆ alkyl, a C₁ to C₆ haloalkyl, a C₁ to C₆ alkoxycarbonyl(C₁ to C₄)alkyl, a C₁ to C₆ haloalkoxycarbonyl(C₁ to C₄)alkyl, a phenyl(C₁ to C₄)alkyl which may be substituted by (Z¹)ₚ₁, a C₃ to C₆ alkenyl, a C₃ to C₆ haloalkenyl, a C₃ to C₆ alkynyl, a C₃ to C₆ haloalkynyl, a C₁ to C₆ alkoxy, a C₁ to C₆ alkoxycarbonyl, a C₁ to C₆ haloalkoxycarbonyl or phenyl which may be substituted by (Z¹)ₚ₁,
R¹⁶ represents a hydrogen atom, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ alkylthio(C₁ to C₄)alkyl, a phenylthio(C₁ to C₄)alkyl which may be substituted by (Z¹)ₚ₁, a cyano(C₁ to C₆)alkyl, a C₁ to C₆ alkoxycarbonyl(C₁ to C₄)alkyl, a C₁ to C₆ alkylaminocarbonyl(C₁ to C₄)alkyl, a di(C₁ to C₆ alkyl)aminocarbonyl(C₁ to C₄)alkyl, a phenyl(C₁ to C₄)alkyl which may be substituted by (Z¹)ₚ₁, a L-(C₁ to C₄)alkyl, a M-(C₁ to C₄)alkyl, a C₃ to C₆ cycloalkyl, a C₃ to C₆ alkenyl, a C₃ to C₆ haloalkenyl, a C₃ to C₆ alkynyl, a C₃ to C₆ haloalkynyl, -OH, a C₁ to C₆ alkylcarbonyloxy, a C₁ to C₆ alkylthio, a C₁ to C₆ haloalkylthio, phenylthio which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, -SN(R²⁰)R¹⁹, a C₁ to C₆ alkylaminosulfonyl, a di(C₁ to C₆ alkyl)aminosulfonyl, -CHO, -C(O)R⁹, -C(O)OR⁹, -C(O)SR⁹, -C(O)NHR¹⁰, -C(O)N(R¹⁰)R⁹, -C(S)OR⁹, -C(S)SR⁹, -C(S)NHR¹⁰, -C(S)N(R¹⁰)R⁹, -C(O)C(O)R⁹, -C(O)C(O)OR⁹, -P(O)(OR²⁴)₂ or -P(S)(OR²⁴)₂,
R¹⁷ represents a hydrogen atom, a C₁ to C₆ alkyl, a C₁ to C₆ haloalkyl, a C₃ to C₈ cycloalkyl(C₁ to C₆)alkyl, a C₃ to C₈ cycloalkyl, a C₃ to C₆ alkenyl, a C₃ to C₆ haloalkenyl, a C₃ to C₆ alkynyl, a C₃ to C₆ haloalkynyl, -CHO, a C₁ to C₆ alkylcarbonyl, a C₁ to C₆ haloalkylcarbonyl or a C₁ to C₆ alkoxycarbonyl, or R¹⁶ and R¹⁷ are combined to form a C₂ to C₆ alkylene chain, so that they may form a 3- to 7-membered ring with the nitrogen(s) atom to which they are bonded, and the alkylene chain at this time may contain one oxygen atom, sulfur atom or nitrogen atom, and may be optionally substituted by a halogen atom, a C₁ to C₆ alkyl group or a C₁ to C₆ haloalkyl group, or further, R¹⁷ is combined with R² to form a C₁ to C₂ alkylene chain, so that they may form a 6-membered or 7-membered hetero ring which fuses with a benzene ring with the atom(s) to which they are bonded, and the alkylene chain at this time may be optionally substituted by an oxygen atom or a C₁ to C₆ alkyl group,
R¹⁸ represents a halogen atom, cyano, nitro, a C₃ to C₈ cycloalkyl, a C₃ to C₈ halocycloalkyl, -OR²⁸, -N(R²⁹)R²⁸, -SH, -S(O)ᵣR³⁰, -CHO, -C(O)R³¹, -C(O)OH, -C(O)OR³¹, -C(O)SR³¹, -C(O)NHR³², -C(O)N(R³²)R³¹, -C(O)C(O)OR³¹, -C(R³⁴)=NOH, -C(R³⁴)=NOR³³, -Si(R¹³)(R¹⁴)R¹², -P(O)(OR²⁴)₂, -P(S)(OR²⁴)₂, -P(phenyl)₂, -P(O)(phenyl)₂, phenyl which may be substituted by (Z¹)ₚ₁, L or M,
R¹⁹ represents a C₁ to C₁₂ alkyl, a C₁ to C₁₂ haloalkyl, a C₁ to C₁₂ alkoxy(C₁ to C₁₂)alkyl, a cyano(C₁ to C₁₂)alkyl, a C₁ to C₁₂ alkoxycarbonyl(C₁ to C₁₂)alkyl, a phenyl(C₁ to C₄)alkyl which may be substituted by (Z¹)ₚ₁, a C₃ to C₁₂ alkenyl, a C₃ to C₁₂ haloalkenyl, a C₃ to C₁₂ alkynyl, a C₃ to C₁₂ haloalkynyl, a C₁ to C₁₂ alkylcarbonyl, a C₁ to C₁₂ alkoxycarbonyl or phenyl which may be substituted by (Z¹)ₚ₁,
R²⁰ represents a C₁ to C₁₂ alkyl, a C₁ to C₁₂ haloalkyl, a C₁ to C₁₂ alkoxy(C₁ to C₁₂)alkyl, a cyano(C₁ to C₁₂)alkyl, a C₁ to C₁₂ alkoxycarbonyl(C₁ to C₁₂)alkyl, a phenyl(C₁ to C₄)alkyl which may be substituted by (Z¹)ₚ₁, a C₃ to C₁₂ alkenyl, a C₃ to C₁₂ haloalkenyl, a C₃ to C₁₂ alkynyl, a C₃ to C₁₂ haloalkynyl or phenyl which may be substituted by (Z¹)ₚ₁, or R¹⁹ and R²⁰ are combined to form a C₄ to C₇ alkylene chain, so that they may form a 5 to 8-membered ring with the nitrogen atom(s) to which they are bonded, and the alkylene chain at this time may contain one oxygen atom or sulfur atom, and may be optionally substituted by a C₁ to C₄ alkyl group or a C₁ to C₄ alkoxy group,
R²¹ represents a hydrogen atom, a C₁ to C₆ alkyl, a C₁ to C₆ haloalkyl, a phenyl(C₁ to C₄)alkyl which may be substituted by (Z¹)ₚ₁, a C₃ to C₆ alkenyl, a C₃ to C₆ haloalkenyl, a C₃ to C₆ alkynyl, -CHO, a C₁ to C₆ alkylcarbonyl, a C₁ to C₆ haloalkylcarbonyl, a C₁ to C₆ alkoxycarbonyl, a C₁ to C₆ haloalkoxycarbonyl, a phenyl(C₁ to C₄)alkoxycarbonyl which may be substituted by (Z¹)ₚ₁, phenoxycarbonyl which may be substituted by (Z¹)ₚ₁, phenylcarbonyl which may be substituted by (Z¹)ₚ₁ or phenyl which may be substituted by (Z¹)ₚ₁,
R²² represents a hydrogen atom, a C₁ to C₆ alkyl, -CHO, a C₁ to C₆ alkylcarbonyl, a C₁ to C₆ haloalkylcarbonyl or a C₁ to C₆ alkoxycarbonyl,
R²³ represents cyano, a C₃ to C₈ cycloalkyl, a C₃ to C₈ halocycloalkyl, -OH, -OR⁸, -SH, -S(O)ᵣR⁸, -N(R¹⁰)R⁹, -N(R¹⁰)CHO, -N(R¹⁰)C(O)R⁹, -N(R¹⁰)C(O)OR⁹, -N(R¹⁰)C(O)SR⁹, -N(R¹⁰)C(S)OR⁹, -N(R¹⁰)C(S)SR⁹, -N(R¹⁰)S(O)₂R⁹, -C(O)OR⁹, -C(O)N(R¹⁰)R⁹, -Si(R¹³)(R¹⁴)R¹², phenyl which may be substituted by (Z¹)ₚ₁, L or M,
R²⁴ represents a C₁ to C₆ alkyl or a C₁ to C₆ haloalkyl,
R²⁵ represents a halogen atom, cyano, a C₁ to C₆ alkyl, a C₁ to C₆ haloalkyl, a hydroxy(C₁ to C₆)alkyl, a C₁ to C₄ alkoxy(C₁ to C₄)alkyl, a C₁ to C₄ alkoxycarbonyl(C₁ to C₄)alkyl, a C₁ to C₆ alkoxy, a C₁ to C₆ alkoxycarbonyl or phenyl which may be substituted by (Z¹)ₚ₁, when q1, q2, q3 or q4 represents an integer of 2 or more, each of R²⁵s may be the same with each other or may be different from each other,
R²⁶ represents a hydrogen atom, a C₁ to C₆ alkyl, a C₁ to C₆ haloalkyl, -CHO, a C₁ to C₆ alkylcarbonyl, a C₁ to C₆ haloalkylcarbonyl, a phenyl(C₁ to C₄)alkylcarbonyl which may be substituted by (Z¹)ₚ₁, a C₁ to C₆ alkoxycarbonyl, a C₁ to C₆ haloalkoxycarbonyl, a phenyl(C₁ to C₄)alkoxycarbonyl which may be substituted by (Z¹)ₚ₁, a C₁ to C₆ alkylaminocarbonyl, a di(C₁ to C₆ alkyl)aminocarbonyl, a C₁ to C₆ alkylaminothiocarbonyl, a di(C₁ to C₆ alkyl)aminothiocarbonyl, phenylcarbonyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, -P(O)(OR²⁴)₂ or -P(S)(OR²⁴)₂,
R²⁷ represents a halogen atom, cyano, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfonyl, a C₁ to C₆ haloalkylsulfonyl, a C₁ to C₆ alkylamino, a di(C₁ to C₆ alkyl)amino, -CHO, a C₁ to C₆ alkylcarbonyl, a C₁ to C₆ haloalkylcarbonyl, a C₁ to C₆ alkoxycarbonyl, a C₁ to C₆ haloalkoxycarbonyl, -CH=NOR¹¹, -C(R⁹)=NOR¹¹, phenyl which may be substituted by (Z¹)ₚ₁, L or M,
R²⁸ represents a hydrogen atom, a C₁ to C₈ alkyl, a (C₁ to C₈)alkyl which may be optionally substituted by R³⁵, a C₃ to C₈ cycloalkyl, a (C₃ to C₈)cycloalkyl which may be optionally substituted by R³⁵, a C₃ to C₈ alkenyl, a (C₃ to C₈)alkenyl which may be optionally substituted by R³⁵, a C₃ to C₈ alkynyl, a (C₃ to C₈)alkynyl which may be optionally substituted by R³⁵, -CHO, -C(O)R³¹, -C(O)OR³¹, -C(O)SR³¹, -C(O)NHR³², -C(O)N(R³²)R³¹, -C(O)C(O)R³¹, -C(O)C(O)OR³¹, -C(S)R³¹, -C(S)OR³¹, -C(S)SR³¹, -C(S)NHR³², -C(S)N(R³²)R³¹, -S(O)₂R³¹, -S(O)₂N(R³²)R³¹, -Si(R¹³)(R¹⁴)R¹², -P(O)(OR²⁴)₂, -P(S)(OR²⁴)₂, phenyl which may be substituted by (Z¹)ₚ₁, L or M,
R²⁹ represents a hydrogen atom, a C₁ to C₆ alkyl, a C₁ to C₆ haloalkyl, a C₃ to C₆ cycloalkyl or a C₁ to C₆ alkoxy, or R²⁸ and R²⁹ are combined to form a C₂ to C₅ alkylene chain, so that they may form a 3- to 6-membered ring with the nitrogen atom(s) to which they are bonded, the alkylene chain at this time may contain one oxygen atom or sulfur atom, and may be optionally substituted by a halogen atom, C₁ to C₆ alkyl group, C₁ to C₆ alkoxy group or a phenyl group which may be substituted by (Z¹)ₚ₁,
R³⁰ represents a C₁ to C₈ alkyl, a (C₁ to C₈)alkyl which may be optionally substituted by R³⁵, a C₃ to C₈ cycloalkyl, a (C₃ to C₈)cycloalkyl which may be optionally substituted by R³⁵, a C₃ to C₈ alkenyl, a (C₃ to C₈)alkenyl which may be optionally substituted by R³⁵, a C₃ to C₈ alkynyl, a (C₃ to C₈)alkynyl which may be optionally substituted by R³⁵, -SH, a C₁ to C₆ alkylthio, a C₁ to C₆ haloalkylthio, phenylthio which may be substituted by (Z¹)ₚ₁, -CHO, -C(O)R³¹, -C(O)OR³¹, -C(O)SR³¹, -C(O)NHR³², -C(O)N(R³²)R³¹, -C(O)C(O)R³¹, -C(O)C(O)OR³¹, -C(S)R³¹, -C(S)OR³¹, -C(S)SR³¹, -C(S)NHR³², -C(S)N(R³²)R³¹, -P(O)(OR²⁴)₂, -P(S)(OR²⁴)₂, phenyl which may be substituted by (Z¹)ₚ₁, L-18, L-21, L-25, L-30 to L-35, L-45, L-48, L-49 or M,
R³¹ represents a C₁ to C₆ alkyl, a C₁ to C₆ haloalkyl, a C₃ to C₈ cycloalkyl(C₁ to C₄)alkyl, a C₁ to C₆ alkoxy(C₁ to C₄)alkyl, a C₁ to C₆ haloalkoxy(C₁ to C₄)alkyl, a C₁ to C₆ alkylthio(C₁ to C₄)alkyl, a C₁ to C₆ haloalkylthio(C₁ to C₄)alkyl, a C₁ to C₆ alkylsulfonyl(C₁ to C₄)alkyl, a C₁ to C₆ haloalkylsulfonyl(C₁ to C₄)alkyl, a cyano(C₁ to C₆)alkyl, a C₁ to C₆ alkylcarbonyl(C₁ to C₄)alkyl, a C₁ to C₆ haloalkylcarbonyl(C₁ to C₄)alkyl, a C₁ to C₆ alkoxycarbonyl(C₁ to C₄)alkyl, a di(C₁ to C₆ alkyl)aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a L-(C₁ to C₄)alkyl, a M-(C₁ to C₄)alkyl, a C₃ to C₈ cycloalkyl, a C₃ to C₈ halocycloalkyl, a C₂ to C₆ alkenyl(C₃ to C₈)cycloalkyl, a C₂ to C₆ haloalkenyl(C₃ to C₈)cycloalkyl, a C₂ to C₆ alkenyl, a C₂ to C₆ haloalkenyl, a C₂ to C₆ alkynyl, a C₂ to C₆ haloalkynyl, phenyl which may be substituted by (Z¹)ₚ₁, L or M,
R³² represents a hydrogen atom, a C₁ to C₆ alkyl, a C₁ to C₆ haloalkyl or phenyl which may be substituted by (Z¹)ₚ₁, or R³¹ and R³² are combined to form a C₂ to C₅ alkylene chain, so that they may form a 3- to 6-membered ring with the nitrogen atom(s) to which they are bonded, and the alkylene chain at this time may contain one oxygen atom, sulfur atom or nitrogen atom, and may be optionally substituted by a halogen atom, a C₁ to C₆ alkyl group, a C₁ to C₆ alkoxy group, formyl group, a C₁ to C₆ alkylcarbonyl group, a C₁ to C₆ alkoxycarbonyl group or a phenyl group which may be substituted by (Z¹)ₚ₁,
R³³ represents a hydrogen atom, a C₁ to C₈ alkyl, a (C₁ to C₈)alkyl which may be optionally substituted by R³⁵, a C₃ to C₈ cycloalkyl, a C₃ to C₈ alkenyl, a (C₃ to C₈)-alkenyl which may be optionally substituted by R³⁵, a C₃ to C₈ alkynyl or a (C₃ to C₈)-alkynyl which may be optionally substituted by R³⁵,
R³⁴ represents a hydrogen atom, a C₁ to C₆ alkyl, a C₁ to C₆ haloalkyl, a C₃ to C₈ cycloalkyl(C₁ to C₄)alkyl, a C₁ to C₆ alkoxy(C₁ to C₄)alkyl, a C₁ to C₆ haloalkoxy(C₁ to C₄)alkyl, a C₁ to C₆ alkylthio(C₁ to C₄)alkyl, a C₁ to C₆ haloalkylthio(C₁ to C₄)alkyl, a C₁ to C₆ alkylsulfonyl(C₁ to C₄)alkyl, a C₁ to C₆ haloalkylsulfonyl(C₁ to C₄)alkyl, a phenyl(C₁ to C₄)alkyl which may be substituted by (Z¹)ₚ₁, or phenyl which may be substituted by (Z¹)ₚ₁,
R³⁵ represents a halogen atom, cyano, nitro, a C₃ to C₈ cycloalkyl, a C₃ to C₈ halocycloalkyl, -OH, -OR³⁶, -SH, -S(O)ᵣR³⁶, -NHR³⁷, -N(R³⁷)R³⁶, -CHO, -C(O)R³¹, -C(O)OR³¹, -C(O)SR³¹, -C(O)NHR³², -C(O)N(R³²)R³¹, -C(O)C(O)OR³¹, -CH=NOR¹¹, -C(R⁹)=NOR¹¹, -Si(R¹³)(R¹⁴)R¹², -P(O)(OR²⁴)₂, -P(S)(OR²⁴)₂, -P(phenyl)₂, -P(O)(phenyl)₂, phenyl which may be substituted by (Z¹)ₚ₁, L or M,
R³⁶ represents a C₁ to C₆ alkyl, a C₁ to C₆ haloalkyl, a C₁ to C₆ alkoxy(C₁ to C₄)alkyl, a C₁ to C₆ alkylthio(C₁ to C₄)alkyl, a phenyl(C₁ to C₄)alkyl which may be substituted by (Z¹)ₚ₁, a C₃ to C₈ cycloalkyl, a C₃ to C₈ halocycloalkyl, a C₃ to C₆ alkenyl, a C₃ to C₆ haloalkenyl, a C₃ to C₈ cycloalkenyl, a C₃ to C₈ halocycloalkenyl, a C₃ to C₆ alkynyl, a C₃ to C₆ haloalkynyl, -CHO, a C₁ to C₆ alkylcarbonyl, a C₁ to C₆ haloalkylcarbonyl, a C₁ to C₆ alkoxycarbonyl, a C₁ to C₆ haloalkoxycarbonyl, a C₁ to C₆ alkylaminocarbonyl, a di(C₁ to C₆ alkyl)aminocarbonyl, phenylcarbonyl which may be substituted by (Z¹)ₚ₁, a C₁ to C₆ alkylaminothiocarbonyl, a di(C₁ to C₆ alkyl)aminothiocarbonyl, phenyl which may be substituted by (Z¹)ₚ₁, L or M,
R³⁷ represents a hydrogen atom, a C₁ to C₆ alkyl, a C₁ to C₆ haloalkyl, a C₃ to C₈ cycloalkyl, a C₃ to C₆ alkenyl, a C₃ to C₆ alkynyl, a C₁ to C₆ alkylcarbonyl, a C₁ to C₆ haloalkylcarbonyl, a C₁ to C₆ alkoxycarbonyl, a C₁ to C₆ haloalkoxycarbonyl, phenoxycarbonyl which may be substituted by (Z¹)ₚ₁, phenylcarbonyl which may be substituted by (Z¹)ₚ₁, a C₁ to C₆ alkylsulfonyl, a C₁ to C₆ haloalkylsulfonyl, phenyl which may be substituted by (Z¹)ₚ₁, L or M, or R³⁶ and R³⁷ are combined to form a C₂ to C₅ alkylene chain, so that they may form a 3- to 6-membered ring with the nitrogen atom(s) to which they are bonded, the alkylene chain at this time may contain one oxygen atom or sulfur atom, and may be substituted by a halogen atom or a methyl group,
I represents an integer of 0 to 9,
m represents an integer of 0 to 4,
n represents an integer of 0 to 4,
p1 represents an integer of 1 to 5,
p2 represents an integer of 0 to 4,
p3 represents an integer of 0 to 3,
p4 represents an integer of 0 to 2,
p5 represents an integer of 0 or 1,
q1 represents an integer of 0 to 3,
q2 represents an integer of 0 to 5,
q3 represents an integer of 0 to 7,
q4 represents an integer of 0 to 9,
r represents an integer of 0 to 2,
t represents an integer of 0 or 1.]
or a salt thereof.

2. The substituted benzanilide compound or a salt thereof according to Claim 1, wherein X represents a halogen atom, cyano, nitro, -SF₅, a C₁ to C₆ alkyl, a C₁ to C₆ haloalkyl, a C₂ to C₆ alkynyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a C₁ to C₆ alkoxycarbonyl, a C₁ to C₆alkylaminocarbonyl, a di(C₁ to C₆ alkyl)aminocarbonyl or phenyl which may be substituted by (Z¹)ₚ₁, when m represents 2, 3 or 4, each of Xs may be the same with each other or may be different from each other, and further, when two Xs are adjacent to each other, the adjacent two Xs may form -CH₂CH₂CH₂-, -CH₂CH₂O-, -CH₂OCH₂-, -OCH₂O-, -CH₂CH₂S-, -CH₂SCH₂-, -CH₂CH₂CH₂CH₂-, -CH₂CH₂CH₂O-, -CH₂CH₂OCH₂-, -CH₂OCH₂O-, -OCH₂CH₂O-, -OCH₂CH₂S- or -CH=CHCH=CH-, so that the two Xs may form a 5-membered ring or 6-membered ring with the carbon atom(s) to which they are bonded, and at this time, the hydrogen atom(s) bonded to the respective carbon atoms which form the ring may be optionally substituted by a halogen atom, a C₁ to C₄ alkyl group or a C₁ to C₄ haloalkyl group,
and when G represents a 5-membered or 6-membered non-aromatic heterocyclic ring containing at least one atom selected from an oxygen atom, a sulfur atom and a nitrogen atom, and having at least one double bond in the ring, X may further represent -N(R¹⁷)R¹⁶, -N=CHOR¹² or -N=C(R⁹)OR¹²,
Y represents a halogen atom, cyano, a C₁ to C₆ alkyl, a C₁ to C₆ haloalkyl, a hydroxy(C₁ to C₆)alkyl, a C₁ to C₃ alkoxy(C₁ to C₃)alkyl, a C₁ to C₆ alkoxy, a C₁ to C₆ haloalkoxy, a C₁ to C₆ alkylthio, a C₁ to C₆ haloalkylthio, a C₁ to C₆ alkylamino, a di(C₁ to C₆ alkyl)amino, phenyl which may be substituted by (Z¹)ₚ₁ or phenoxy which may be substituted by (Z¹)ₚ₁, and when n represents 2, 3 or 4, each of Ys may be the same with each other or may be different from each other, and further, when two Ys are adjacent to each other, the adjacent two Ys may form -CH₂CH₂CH₂-, -CH₂CH₂O-, -CH₂OCH₂-, -OCH₂O-, -CH₂CH₂CH₂CH₂-, -CH₂CH₂CH₂O-, -CH₂CH₂OCH₂-, -CH₂OCH₂O-, -OCH₂CH₂O-, -OCH=N- or -SCH=N-, so that the two Ys may form a 5-membered ring or 6-membered ring with carbon atoms to which the two Ys are bonded, and at this time, the hydrogen atom(s) bonded to each of the carbon atom(s) which form(s) the ring may be optionally substituted by a halogen atom, a C₁ to C₄ alkyl group or a C₁ to C₄ haloalkyl group,
R¹ and R² each independently represent a hydrogen atom, a C₁ to C₆ alkyl, a C₁ to C₆ haloalkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfonyl(C₁ to C₄)alkyl, a C₁ to C₄ haloalkylsulfonyl(C₁ to C₄)alkyl, phenylthio(C₁ to C₄)alkyl which may be substituted by (Z¹)ₚ₁, a C₃ to C₆ alkenyl, a C₃ to C₆ haloalkenyl, a C₃ to C₆ alkynyl, a C₃ to C₆ haloalkynyl, a C₁ to C₆ alkylthio, a C₁ to C₆ haloalkylthio, phenylthio which may be substituted by (Z¹)ₚ₁ or -SN(R²⁰)R¹⁹,
R³ represents a C₁ to C₈ alkyl, a (C₁ to C₈)alkyl which may be optionally substituted by R¹⁸, a C₃ to C₈ cycloalkyl, a C₁ to C₄ alkylthio(C₃ to C₆)cycloalkyl, a C₁ to C₄ alkylsulfinyl(C₃ to C₆)cycloalkyl, a C₁ to C₄ alkylsulfonyl(C₃ to C₆)cycloalkyl, a hydroxymethyl(C₃ to C₆)cycloalkyl, a C₁ to C₄ alkoxymethyl(C₃ to C₆)cycloalkyl, a C₁ to C₄ alkylthiomethyl(C₃ to C₆)cycloalkyl, a C₁ to C₄ alkylsulfinylmethyl(C₃ to C₆)cycloalkyl, a C₁ to C₄ alkylsulfonylmethyl(C₃ to C₆)cycloalkyl, a C₃ to C₈ alkenyl, a C₁ to C₆ alkylaminocarbonyl(C₃ to C₆)alkenyl, a phenyl(C₃ to C₆)alkenyl which may be substituted by (Z¹)ₚ₁, a C₃ to C₈ alkynyl, a phenyl(C₃ to C₆)alkynyl which may be substituted by (Z¹)ₚ₁, a naphthyl(C₃ to C₆)alkynyl, a L-(C₃ to C₆)alkynyl, a C₁ to C₈ alkoxy, a C₃ to C₈ haloalkenyloxy, M-4, M-5, M-8, M-9, M-13 to M-19, M-21, M-22, M-25 or M-28, or R² and R³ are combined to form a C₂ to C₆ alkylene chain, so that they may form a 3- to 7-membered ring with a nitrogen atom(s) to which they are bonded, the alkylene chain at this time may contain one oxygen atom or sulfur atom, and may be optionally substituted by a C₁ to C₆ alkyl group,
R⁴ represents a hydrogen atom, a halogen atom, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, a C₁ to C₆ alkoxycarbonyl, -CH=NOR¹¹, -C(R⁹)=NOR¹¹, -Si(R¹³)(R¹⁴)R¹², -P(O)(OR²⁴)₂, phenyl which may be substituted by (Z²)ₚ₁, 1-naphthyl, 2-naphthyl, L or M,
R⁵ represents
(a). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)SR⁹, -C(O)N(R¹⁰)R⁹, -C(S)N(R¹⁰)R⁹, -CH=NOR¹¹, -C(R⁹)=NOR¹¹, phenyl which may be substituted by (Z²)ₚ₁, 1-naphthyl, 2-naphthyl, L or M, when G represents a 5-membered or 6-membered non-aromatic heterocyclic ring containing at least one atom selected from an oxygen atom, a sulfur atom and a nitrogen atom, and having at least one double bond in the ring, or a 5-membered or 6-membered saturated heterocyclic ring containing two atoms selected from an oxygen atom, a sulfur atom and a nitrogen atom,
(b). cyano, a (C₁ to C₆)alkyl which may be optionally substituted by R²³, a (C₁ to C₆)-haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a 3-membered to 6-membered cycloalkyl ring, and R⁴ represents cyano, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, a C₃ to C₆ alkenyl, a C₃ to C₆ haloalkenyl, a C₃ to C₆ alkynyl, a C₃ to C₆ haloalkynyl, -OR⁸, -S(O)ᵣR⁸, -N(R¹⁰)R⁹, a C₁ to C₆ alkoxycarbonyl, -Si(R¹³)(R¹⁴)R¹², -P(O)(OR²⁴)₂, phenyl which may be substituted by (Z²)ₚ₁, 1-naphthyl, 2-naphthyl, L or M,
R⁶ represents a hydrogen atom, a halogen atom, cyano, a C₁ to C₆ alkyl, a C₁ to C₆ haloalkyl, a (C₁ to C₆)alkyl which may be optionally substituted by R²³, a (C₁ to C₆)haloalkyl which may be optionally substituted by R²³, a C₃ to C₈ cycloalkyl, a C₃ to C₈ halocycloalkyl, a (C₃ to C₈)cycloalkyl which may be optionally substituted by R²³, a (C₃ to C₈)halocycloalkyl which may be optionally substituted by R²³, -S(O)₂R⁹, a C₁ to C₆ alkylcarbonyl, a C₁ to C₆ haloalkylcarbonyl, phenylcarbonyl which may be substituted by (Z¹)ₚ₁, -C(O)OR⁹, -C(O)SR⁹, -C(O)N(R¹⁰)R⁹, a di(C₁ to C₆ alkyl)phosphoryl, a di(C₁ to C₆ alkyl)thiophosphoryl, phenyl which may be substituted by (Z²)ₚ₁, L or M, or when R⁶ and R⁴ are adjacent to each other, the adjacent R⁴ and R⁶ are combined to form a C₃ to C₅ alkylene chain, so that they may form a 5- to 7-membered ring with atoms to which they are bonded, and the alkylene chain at this time may contain one oxygen atom or sulfur atom, and may be optionally substituted by a C₁ to C₆ alkyl group,
Z² represents a halogen atom, cyano, nitro, amino, a C₁ to C₆ alkyl, a C₁ to C₆ haloalkyl, a C₁ to C₃ alkoxy(C₁ to C₃)alkyl, a C₁ to C₃ haloalkoxy(C₁ to C₃)alkyl, a C₁ to C₃ alkylthio(C₁ to C₃)alkyl, a C₁ to C₃ haloalkylthio(C₁ to C₃)alkyl, a C₁ to C₃ alkylsulfinyl(C₁ to C₃)alkyl, a C₁ to C₃ haloalkylsulfinyl(C₁ to C₃)alkyl, a C₁ to C₃ alkylsulfonyl(C₁ to C₃)alkyl, a C₁ to C₃ haloalkylsulfonyl(C₁ to C₃)alkyl, cyano(C₁ to C₆)alkyl, hydroxy(C₁ to C₃)haloalkyl, a C₁ to C₃ alkoxy(C₁ to C₃)haloalkyl, a C₁ to C₃ haloalkoxy-(C₁ to C₃)haloalkyl, a C₃ to C₈ cycloalkyl, a C₃ to C₈ halocycloalkyl, a C₁ to C₆ alkoxy, a C₁ to C₆ haloalkoxy, a C₁ to C₃ haloalkoxy(C₁ to C₃) haloalkoxy, a C₁ to C₆ alkylsulfonyloxy, a C₁ to C₆ haloalkylsulfonyloxy, phenoxy which may be substituted by (Z¹)ₚ₁, -O(L-45), a C₁ to C₆ alkylthio, a C₁ to C₆ haloalkylthio, a C₃ to C₈ cycloalkylthio, phenylthio which may be substituted by (Z¹)ₚ₁, -S(L-45), a C₁ to C₆ alkylsulfinyl, a C₁ to C₆ haloalkylsulfinyl, a C₃ to C₈ cycloalkylsulfinyl, phenylsulfinyl which may be substituted by (Z¹)ₚ₁, -S(O)(L-45), a C₁ to C₆ alkylsulfonyl, a C₁ to C₆ haloalkylsulfonyl, a C₃ to C₈ cycloalkylsulfonyl, phenylsulfonyl which may be substituted by (Z¹)ₚ₁, -SO₂(L-45), a C₁ to C₆ alkylamino, a di(C₁ to C₆ alkyl)amino, a C₁ to C₆ alkylaminosulfonyl, a di(C₁ to C₆ alkyl)aminosulfonyl, a C₁ to C₆ alkylsulfonylamino, a C₁ to C₆ haloalkylsulfonylamino, -C(O)NH₂, a C₁ to C₆ alkylaminocarbonyl, a di(C₁ to C₆ alkyl)aminocarbonyl, -C(S)NH₂, -Si(R¹⁵)(R¹⁶)R¹⁴, phenyl which may be substituted by (Z¹)ₚ₁, L-5, L-14, L-24, L-36, L-39, L-41, L-42, L-43, L-44 or M, when p1 represents an integer of 2 or more, each of Z²s may be the same with each other or may be different from each other, and further, when the two Z²s are adjacent to each other, the adjacent two Z²s form -CF₂CF₂O-, -CF₂OCF₂- or -OCF₂O-, so that they may form a 5-membered ring with the carbon atoms each of which are bonded to,
R⁸ represents a C₁ to C₆ alkyl, a C₁ to C₆ haloalkyl, a C₃ to C₈ cycloalkyl(C₁ to C₄)alkyl, a C₃ to C₈ cycloalkyl, a C₃ to C₈ halocycloalkyl, a C₂ to C₆ alkenyl, a C₂ to C₆ haloalkenyl, a C₃ to C₈ cycloalkenyl, a C₃ to C₈ halocycloalkenyl, a C₃ to C₆ alkynyl, a C₃ to C₆ haloalkynyl, phenyl which may be substituted by (Z¹)ₚ₁, L or M,
R⁹ represents a C₁ to C₆ alkyl, a C₁ to C₆ haloalkyl, a C₃ to C₆ cycloalkyl(C₁ to C₄)alkyl, a phenyl(C₁ to C₄)alkyl which may be substituted by (Z¹)ₚ₁, a L-(C₁ to C₄)alkyl, a M-(C₁ to C₄)alkyl, a C₃ to C₈ cycloalkyl, a C₃ to C₈ halocycloalkyl or phenyl which may be substituted by (Z¹)ₚ₁,
R¹⁰ represents a hydrogen atom or a C₁ to C₆ alkyl, or R⁹ and R¹⁰ are combined to form a C₂ to C₆ alkylene chain, so that they may form a 3- to 7-membered ring with a nitrogen atom(s) to which they are bonded, and the alkylene chain at this time may contain one oxygen atom or sulfur atom,
R¹¹ represents a hydrogen atom, a C₁ to C₆ alkyl, a C₁ to C₆ haloalkyl or phenyl(C₁ to C₄)alkyl which may be substituted by (Z¹)ₚ₁, or R⁹ and R¹¹ are combined to form a C₂ to C₃ alkylene chain, so that they may form a 5-membered ring or 6-membered ring with atoms to which they are bonded, and the alkylene chain at this time may contain one oxygen atom, sulfur atom or nitrogen atom, and may be optionally substituted by a C₁ to C₆ alkyl group,
R¹² represents a C₁ to C₆ alkyl or phenyl which may be substituted by (Z¹)ₚ₁,
R¹³ and R¹⁴ each independently represent a C₁ to C₆ alkyl,
R¹⁵ represents a C₁ to C₆ alkyl, a C₁ to C₆ haloalkyl, a phenyl(C₁ to C₄)alkyl which may be substituted by (Z¹)ₚ₁ or phenyl which may be substituted by (Z¹)ₚ₁,
R¹⁶ represents a hydrogen atom, a C₁ to C₆ alkyl, a C₃ to C₆ cycloalkyl(C₁ to C₄)alkyl, a C₁ to C₄ alkoxy(C₁ to C₄)alkyl, a C₁ to C₄ alkylthio(C₁ to C₄)alkyl, a C₃ to C₆ cycloalkyl, a C₃ to C₆ alkenyl, a C₃ to C₆ haloalkenyl, a C₃ to C₆ alkynyl, a C₃ to C₆ haloalkynyl, a C₁ to C₆ alkylsulfonyl, a C₁ to C₆ haloalkylsulfonyl, -CHO, -C(O)R⁹, -C(O)OR⁹, -C(O)SR⁹, -C(O)N(R¹⁰)R⁹, -C(S)OR⁹, -C(S)SR⁹ or -C(S)N(R¹⁰)R⁹,
R¹⁷ represents a hydrogen atom or a C₁ to C₆ alkyl, or R¹⁷ and R² are combined to form a C₁ to C₂ alkylene chain, so that they may form a 6-membered or 7-membered hetero ring which fuses with a benzene ring with atoms to which they are bonded, and the alkylene chain at this time may be optionally substituted by a C₁ to C₆ alkyl group,
R¹⁸ represents a halogen atom, cyano, a C₃ to C₆ cycloalkyl, -OR²⁸, -N(R²⁹)R²⁸, -SH, -S(O)ᵣR³⁰, -CHO, a C₁ to C₆ alkylcarbonyl, a C₁ to C₆ alkoxycarbonyl, -C(O)N(R³²)R³¹, -C(R³⁴)=NOH, -C(R³⁴)=NOR³³, -Si(R¹³)(R¹⁴)R¹², phenyl which may be substituted by (Z¹)ₚ₁, L-1, L-2, L-3, L-4, L-45, L-46, L-47 or M,
R¹⁹ represents a C₁ to C₆ alkyl, a C₁ to C₆ alkoxycarbonyl(C₁ to C₄)alkyl or a C₁ to C₆ alkoxycarbonyl,
R²⁰ represents a C₁ to C₆ alkyl or, R¹⁹ and R²⁰ are combined to form a C₄ to C₅ alkylene chain, so that they may form a 5-membered ring or 6-membered ring with the nitrogen atom(s) to which they are bonded, and the alkylene chain at this time may contain one oxygen atom or sulfur atom, and may be optionally substituted by a methyl group or a methoxy group,
R²³ represents cyano, a C₃ to C₈ cycloalkyl, a C₃ to C₈ halocycloalkyl, a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, a C₁ to C₆ alkoxycarbonyl, a C₁ to C₆ haloalkoxycarbonyl, a di(C₁ to C₆ alkyl)-aminocarbonyl, -Si(R¹³)(R¹⁴)R¹², phenyl which may be substituted by (Z¹)ₚ₁, L or M,
R²⁵ represents a C₁ to C₆ alkyl, a C₁ to C₆ haloalkyl or a C₁ to C₆ alkoxy, when q1, q2, q3 or q4 represents an interger of 2 or more, each of R²⁵s may be the same with each other or may be different from each other,
R²⁶ represents -CHO, a C₁ to C₆ alkylcarbonyl, a C₁ to C₆ alkoxycarbonyl, a C₁ to C₆ alkylaminocarbonyl, a di(C₁ to C₆ alkyl)aminocarbonyl, phenylcarbonyl which may be substituted by (Z¹)ₚ₁, a C₁ to C₆ alkylsulfonyl or a C₁ to C₆ haloalkylsulfonyl,
R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₃ to C₈ cycloalkyl, a C₃ to C₈ alkenyl, a C₃ to C₈ alkynyl, -CHO, a C₁ to C₆ alkylcarbonyl, a C₁ to C₆ haloalkylcarbonyl, a C₃ to C₆ cycloalkylcarbonyl, phenylcarbonyl which may be substituted by (Z¹)ₚ₁, a C₁ to C₆ alkoxycarbonyl, -C(O)N(R³²)R³¹, -C(S)N(R³²)R³¹, a C₁ to C₆ alkylsulfonyl, a C₁ to C₆ haloalkylsulfonyl, phenylsulfonyl which may be substituted by (Z¹)ₚ₁, a di(C₁ to C₆ alkyl)aminosulfonyl, a di(C₁ to C₆ alkyl)phosphoryl, a di(C₁ to C₆ alkyl)thiophosphoryl, -Si(R¹³)(R¹⁴)R¹² or phenyl which may be substituted by (Z¹)ₚ₁,
R²⁹ represents a hydrogen atom, a C₁ to C₆ alkyl or a C₁ to C₆ alkoxy,
R³⁰ represents a C₁ to C₆ alkyl, a (C₁ to C₄)alkyl which may be optionally substituted by R³⁵, a C₃ to C₆ cycloalkyl, a C₃ to C₆ alkenyl, a C₃ to C₆ alkynyl, a C₁ to C₆ alkylthio, phenylthio which may be substituted by (Z¹)ₚ₁, a C₁ to C₆ alkylcarbonyl, a C₁ to C₆ alkylaminocarbonyl, a di(C₁ to C₄ alkyl)aminocarbonyl, a C₁ to C₆ alkylaminothiocarbonyl, a di(C₁ to C₄ alkyl)aminothiocarbonyl, phenyl which may be substituted by (Z¹)ₚ₁, L-21, L-35, L-45 or L-48,
R³¹ represents a C₁ to C₆ alkyl, a C₁ to C₆ haloalkyl, a C₃ to C₈ cycloalkyl(C₁ to C₄)alkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a (L-45)-(C₁ to C₄)alkyl, a (L-46)-(C₁ to C₄)alkyl, a (L-47)-(C₁ to C₄)alkyl, a C₃ to C₆ cycloalkyl, a C₃ to C₆ alkenyl, a C₂ to C₆ alkynyl or phenyl which may be substituted by (Z¹)ₚ₁,
R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, and the alkylene chain at this time may contain one oxygen atom, sulfur atom or nitrogen atom, and may be optionally substituted by a C₁ to C₆ alkyl group, a C₁ to C₆ alkoxy group, formyl group, a C₁ to C₆ alkylcarbonyl group or a C₁ to C₆ alkoxycarbonyl group,
R³³ represents a C₁ to C₆ alkyl, a C₃ to C₆ cycloalkyl(C₁ to C₄)alkyl, a C₁ to C₄ alkoxy(C₁ to C₄)alkyl, a C₁ to C₄ alkylthio(C₁ to C₄)alkyl, a C₁ to C₄ alkoxycarbonyl(C₁ to C₄)alkyl, a di(C₁ to C₄ alkyl)aminocarbonyl(C₁ to C₄)alkyl, a C₃ to C₆ alkenyl or a phenyl(C₁ to C₄)alkyl which may be substituted by (Z¹)ₚ₁,
R³⁴ represents a hydrogen atom, a C₁ to C₆ alkyl or phenyl which may be substituted by (Z¹)ₚ₁,
R³⁵ represents a halogen atom, -OH, a C₁ to C₄ alkoxy, a C₁ to C₆ alkylcarbonyloxy, a C₁ to C₄ haloalkylcarbonyloxy, a C₁ to C₄ alkylthio, a C₁ to C₄ alkylcarbonyl, a C₁ to C₄ alkoxycarbonyl, a di(C₁ to C₄ alkyl)aminocarbonyl, -Si(R¹³)(R¹⁴)R¹² or phenyl which may be substituted by (Z¹)ₚ₁.

3. The substituted benzanilide compound or a salt thereof according to Claim 2, wherein G represents a non-aromatic heterocyclic ring represented by either one of the formula G-1, the formula G-4, the formula G-5, the formula G-6, the formula G-7, the formula G-8, the formula G-11, the formula G-12, the formula G-13, the formula G-14, the formula G-15, the formula G-17, the formula G-18, the formula G-21, the formula G-22, the formula G-23, the formula G-32, the formula G-33, the formula G-40, the formula G-41, the formula G-42, the formula G-53 or the formula G-54, a saturated heterocyclic ring represented by the formula G-55 or the formula G-56, or a cycloalkyl ring represented by the formula G-71, 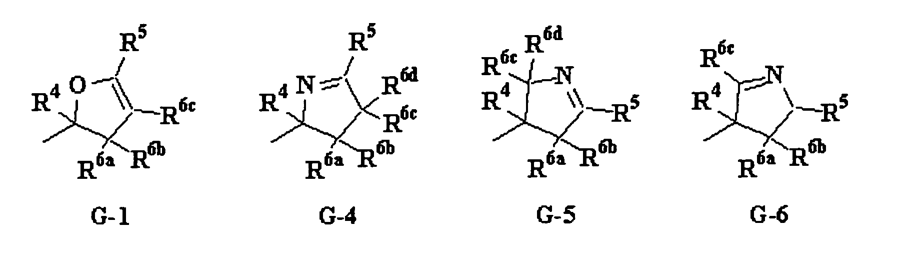 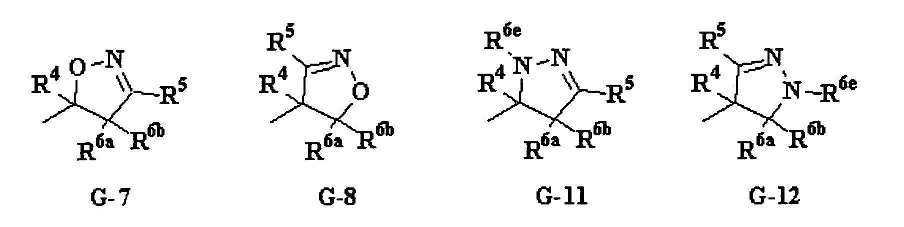 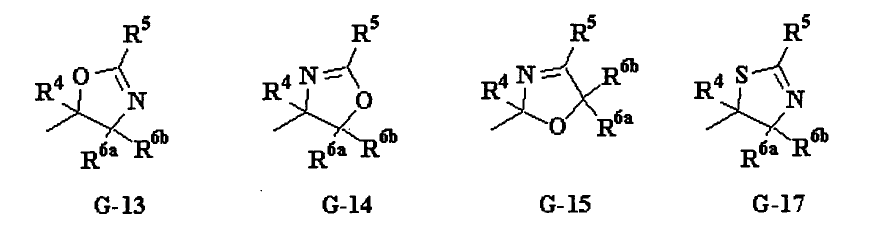 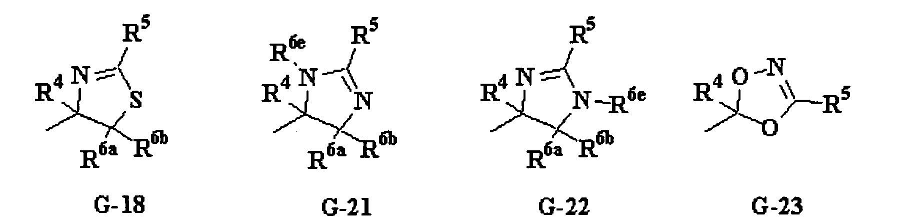 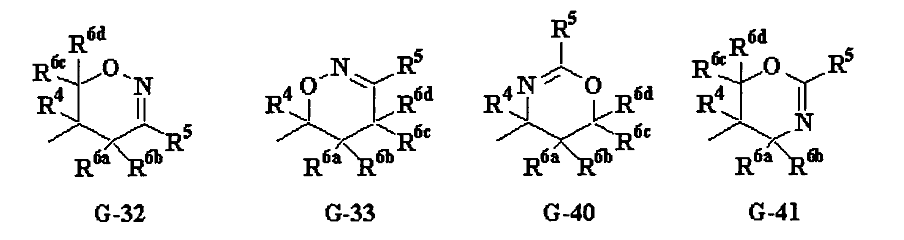 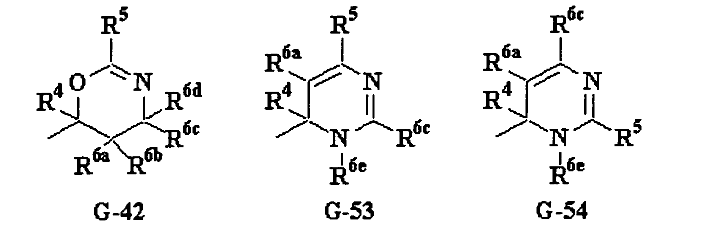 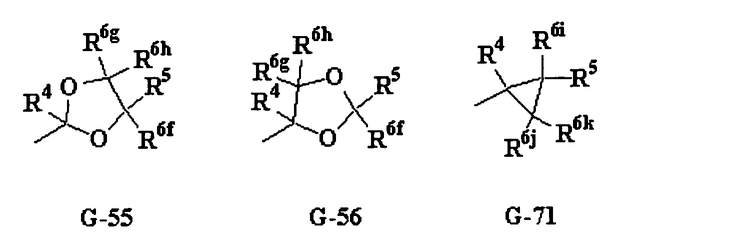 W¹ and W² each represent an oxygen atom,
X represents a halogen atom, nitro, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl or a C₁ to C₆ haloalkylsulfonyl, when m represents 2 or 3, each of Xs may be the same with each other or may be different from each other,
and further, when two Xs are adjacent to each other, the adjacent two Xs may form -OCF₂O- or -OCF₂CF₂O-, so that they may form a 5-membered ring or 6-membered ring with carbon atoms to which they are bonded,
and, when G represents a non-aromatic heterocyclic ring represented by either of the formula G-1, the formula G-4, the formula G-5, the formula G-6, the formula G-7, the formula G-8, the formula G-11, the formula G-12, the formula G-13, the formula G-14, the formula G-15, the formula G-17, the formula G-18, the formula G-21, the formula G-22, the formula G-23, the formula G-32, the formula G-33, the formula G-40, the formula G-41, the formula G-42, the formula G-53 or the formula G-54, X may further represent -N(R¹⁷)R¹⁶,
Y represents a halogen atom, a C₁ to C₆ alkyl, a C₁ to C₆ haloalkyl, a hydroxy(C₁ to C₆)alkyl, a C₁ to C₃ alkoxy(C₁ to C₃)alkyl, a C₁ to C₆ alkoxy or a C₁ to C₆ alkylthio, and when n represents 2 or 3, each of Ys may be the same with each other or may be different from each other,
R¹ represents a hydrogen atom,
R² represents a hydrogen atom, a C₁ to C₆ alkyl, a C₁ to C₄ alkoxy(C₁ to C₄)alkyl or a C₃ to C₆ alkenyl,
R³ represents a C₁ to C₈ alkyl, a (C₁ to C₈)alkyl which may be optionally substituted by R¹⁸, a C₃ to C₈ cycloalkyl, a C₃ to C₈ alkenyl, a C₁ to C₆ alkylaminocarbonyl(C₃ to C₆)alkenyl, a phenyl(C₃ to C₆)alkenyl which may be substituted by (Z¹)ₚ₁, a C₃ to C₈ alkynyl, a phenyl(C₃ to C₆)alkynyl which may be substituted by (Z¹)ₚ₁, a naphthalen-1-yl-(C₃ to C₆)alkynyl, a naphthalen-2-yl-(C₃ to C₆)alkynyl, a (L-1)-(C₃ to C₆)alkynyl, a (L-2)-(C₃ to C₆)alkynyl, a (L-3)-(C₃ to C₆)alkynyl, a (L-4)-(C₃ to C₆)alkynyl, a (L-45)-(C₃ to C₆)alkynyl, a (L-46)-(C₃ to C₆)alkynyl, a (L-47)-(C₃ to C₆)alkynyl, M-4, M-5, M-8, M-9, M-13 to M-19, M-21 or M-22, or R² and R³ are combined to form a C₂ to C₆ alkylene chain, so that they may form a 3- to 7-membered ring with a nitrogen atom(s) to which they are bonded, and the alkylene chain at this time may contain one oxygen atom or sulfur atom,
R⁴ represents a hydrogen atom, a C₁ to C₆ alkyl, a C₁ to C₆ haloalkyl, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₁ to C₄ haloalkoxy(C₁ to C₄)haloalkyl, a C₃ to C₈ cycloalkyl, a C₃ to C₈ halocycloalkyl, -CH=NOR¹¹, -C(R⁹)=NOR¹¹, phenyl which may be substituted by (Z²)ₚ₁, 1-naphthyl, 2-naphthyl, L-1 to L-4, L-8 to L-13, L-15 to L-23, L-25 to L-35, L-37, L-38, L-40, L-43 to L-58, M-4, M-5, M-8, M-9, M-14 to M-18 or M-19,
and when G represents a cycloalkyl ring represented by the formula G-71, R⁴ may further represent a halogen atom, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl or a C₁ to C₆ haloalkylsulfonyl,
R⁵ represents
(a). a hydrogen atom, a halogen atom, cyano, a C₁ to C₆ alkyl, a C₁ to C₆ haloalkyl, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, cyano(C₁ to C₆)alkyl, a C₃ to C₈ cycloalkyl, a C₃ to C₈ halocycloalkyl, a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfinyl, a C₁ to C₆ haloalkylsulfinyl, phenylsulfinyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, a C₁ to C₆ alkylamino, a di(C₁ to C₆ alkyl)amino, pyrrolidin-1-yl, oxazolidin-3-yl, thiazolidin-3-yl, piperidin-1-yl, morpholin-1-yl, -C(O)R⁹, -C(O)OR⁹, -C(O)SR⁹, -C(O)N(R¹⁰)R⁹, -C(S)N(R¹⁰)R⁹, -CH=NOR¹¹, -C(R⁹)=NOR¹¹, phenyl which may be substituted by (Z²)ₚ₁, 1-naphthyl, 2-naphthyl, L or M, when G represents a non-aromatic heterocyclic ring represented by either one of the formula G-1, the formula G-4, the formula G-5, the formula G-6, the formula G-7, the formula G-8, the formula G-11, the formula G-12, the formula G-13, the formula G-14, the formula G-15, the formula G-17, the formula G-18, the formula G-21, the formula G-22, the formula G-23, the formula G-32, the formula G-33, the formula G-40, the formula G-41, the formula G-42, the formula G-53 or the formula G-54, or a saturated heterocyclic ring represented by the formula G-55 or the formula G-56,
(b). cyano, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₃ to C₈ cycloalkyl, a C₃ to C₈ halocycloalkyl, phenoxy which may be substituted by (Z¹)ₚ₁, phenylthio which may be substituted by (Z¹)ₚ₁, phenylsulfinyl which may be substituted by (Z¹)ₚ₁, phenylsulfonyl which may be substituted by (Z¹)ₚ₁, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a cycloalkyl ring represented by the formula G-71, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₃ to C₈ cycloalkyl, a C₃ to C₈ halocycloalkyl, a C₁ to C₆ alkoxy, a C₁ to C₆ haloalkoxy, phenoxy which may be substituted by (Z¹)ₚ₁, a C₁ to C₆ alkylthio, a C₁ to C₆ haloalkylthio, phenylthio which may be substituted by (Z¹)ₚ₁, a C₁ to C₆ alkylsulfinyl, a C₁ to C₆ haloalkylsulfinyl, phenylsulfinyl which may be substituted by (Z¹)ₚ₁, a C₁ to C₆ alkylsulfonyl, a C₁ to C₆ haloalkylsulfonyl, phenylsulfonyl which may be substituted by (Z¹)ₚ₁, -CHO, -C(O)R⁹, -C(O)OR⁹, -C(O)NHR¹⁰, -C(O)N(R¹⁰)R⁹, -C(S)NHR¹⁰, -C(S)N(R¹⁰)R⁹, -CH=NOR¹¹, -C(R⁹)=NOR¹¹, -Si(R¹³)(R¹⁴)R¹², phenyl which may be substituted by (Z²)ₚ₁, 1-naphthyl, 2-naphthyl, L or M, when G represents a cycloalkyl ring represented by the formula G-71, and R⁴ represents a C₃ to C₆ cycloalkyl(C₁ to C₄)alkyl, a C₃ to C₆ halocycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio-(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a cyano(C₁ to C₆)alkyl, a C₁ to C₄ haloalkoxy(C₁ to C₄)haloalkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, phenyl which may be substituted by (Z²)ₚ₁, 1-naphthyl, 2-naphthyl, L-1 to L-4, L-8 to L-13, L-15 to L-23, L-25 to L-35, L-37, L-38, L-40, L-43 to L-58, M-4, M-5, M-8, M-9, M-14 to M-18 or M-19,
R^{6a}, R^{6b}, R^{6c} and R^{6d} each independently represent a hydrogen atom, a halogen atom, cyano, a C₁ to C₆ alkyl, a C₁ to C₆ haloalkyl or phenyl which may be substituted by (Z²)ₚ₁,
R^{6e} represents a hydrogen atom, a C₁ to C₆ alkyl, a C₁ to C₆ alkylsulfonyl, a C₁ to C₆ haloalkylsulfonyl, phenylsulfonyl which may be substituted by (Z¹)ₚ₁, a C₁ to C₆ alkylcarbonyl, a C₁ to C₆ haloalkylcarbonyl, phenylcarbonyl which may be substituted by (Z¹)ₚ₁, a C₁ to C₆ alkoxycarbonyl, a C₁ to C₆ haloalkoxycarbonyl, phenoxycarbonyl which may be substituted by (Z¹)ₚ₁, a di(C₁ to C₆ alkyl)phosphoryl or phenyl which may be substituted by (Z²)ₚ₁,
R^{6f}, R^{6g} and R^{6h} each independently represent a hydrogen atom, a C₁ to C₆ alkyl, a C₁ to C₆ haloalkyl or phenyl which may be substituted by (Z²)ₚ₁,
R⁶ⁱ represents a hydrogen atom, a halogen atom, cyano, a C₁ to C₆ alkyl, a C₁ to C₆ haloalkyl, a C₁ to C₆ alkoxycarbonyl or a C₁ to C₆ haloalkoxycarbonyl,
R^{6j} and R^{6k} each independently represent hydrogen atom, a halogen atom, cyano, a C₁ to C₆ alkyl or a C₁ to C₆ haloalkyl,
Z² represents a halogen atom, cyano, nitro, amino, a C₁ to C₆ alkyl, a C₁ to C₆ haloalkyl, a C₁ to C₃ alkoxy(C₁ to C₃)alkyl, a C₁ to C₃ haloalkoxy(C₁ to C₃)alkyl, a C₁ to C₃ alkylthio(C₁ to C₃)alkyl, a C₁ to C₃ haloalkylthio(C₁ to C₃)alkyl, a C₁ to C₃ alkylsulfinyl(C₁ to C₃)alkyl, a C₁ to C₃ haloalkylsulfinyl(C₁ to C₃)alkyl, a C₁ to C₃ alkylsulfonyl-(C₁ to C₃)alkyl, a C₁ to C₃ haloalkylsulfonyl(C₁ to C₃)alkyl, a cyano(C₁ to C₆)alkyl, a C₃ to C₈ cycloalkyl, a C₃ to C₈ halocycloalkyl, a C₁ to C₆ alkoxy, a C₁ to C₆ haloalkoxy, a C₁ to C₃ haloalkoxy(C₁ to C₃) haloalkoxy, a C₁ to C₆ alkylsulfonyloxy, a C₁ to C₆ haloalkylsulfonyloxy, phenoxy which may be substituted by (Z¹)ₚ₁, -O(L-45), a C₁ to C₆ alkylthio, a C₁ to C₆ haloalkylthio, a C₃ to C₈ cycloalkylthio, a C₁ to C₆ alkylsulfinyl, a C₁ to C₆ haloalkylsulfinyl, a C₃ to C₈ cycloalkylsulfinyl, a C₁ to C₆ alkylsulfonyl, a C₁ to C₆ haloalkylsulfonyl, a C₃ to C₈ cycloalkylsulfonyl, a C₁ to C₆ alkylamino, a di(C₁ to C₆ alkyl)amino, a C₁ to C₆ alkylaminosulfonyl, a di(C₁ to C₆ alkyl)aminosulfonyl, -C(O)NH₂, a C₁ to C₆ alkylaminocarbonyl, a di(C₁ to C₆ alkyl)aminocarbonyl, -C(S)NH₂ or a tri(C₁ to C₆ alkyl)silyl, when p1 represents an integer of 2 or more, each of Z²s may be the same with each other or may be different from each other,
and further, when the two Z²s are adjacent to each other, the adjacent two Z²s form -CF₂CF₂O-, -CF₂OCF₂- or -OCF₂O-, so that they may form a 5-membered ring with the carbon atoms each of which are bonded to,
R⁹ represents a C₁ to C₆ alkyl, a C₁ to C₆ haloalkyl, a phenyl(C₁ to C₄)alkyl which may be substituted by (Z¹)ₚ₁, a C₃ to C₈ cycloalkyl or phenyl which may be substituted by (Z¹)ₚ₁,
R¹⁰ represents a hydrogen atom or a C₁ to C₆ alkyl, or R⁹ and R¹⁰ are combined to form a C₄ to C₅ alkylene chain, so that they may form a 5-membered ring or 6-membered ring with the nitrogen atom(s) to which they are bonded, and the alkylene chain at this time may contain one oxygen atom or sulfur atom,
R¹¹ represents a C₁ to C₆ alkyl, a C₁ to C₆ haloalkyl or a phenyl(C₁ to C₄)alkyl which may be substituted by (Z¹)ₚ₁, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group,
R¹⁵ represents a C₁ to C₆ alkyl or phenyl which may be substituted by (Z¹)ₚ₁,
R¹⁶ represents a hydrogen atom, a C₁ to C₆ alkyl, a C₁ to C₄ alkoxy(C₁ to C₄)alkyl, a C₁ to C₄ alkylthio(C₁ to C₄)alkyl, -CHO, a C₁ to C₆ alkylcarbonyl, a C₁ to C₆ haloalkylcarbonyl, a C₃ to C₆ cycloalkylcarbonyl, a C₁ to C₆ alkoxycarbonyl, a C₁ to C₆ haloalkoxycarbonyl or a C₁ to C₆ alkylthiocarbonyl,
R¹⁷ represents a hydrogen atom or a C₁ to C₆ alkyl, or R¹⁷ may be combined with R² to form a -CH₂-,
R¹⁸ represents a halogen atom, cyano, a C₃ to C₆ cycloalkyl, -OR²⁸, -N(R²⁹)R²⁸, -S(O)ᵣR³⁰, a C₁ to C₆ alkoxycarbonyl, -C(O)N(R³²)R³¹, -C(R³⁴)=NOH, -C(R³⁴)=NOR³³, a tri(C₁ to C₄ alkyl)silyl, phenyl which may be substituted by (Z¹)ₚ₁, L-1, L-2, L-3, L-4, L-45, L-46, L-47 or M,
R²⁵ represents a C₁ to C₄ alkyl,
R²⁶ represents a C₁ to C₆ alkylcarbonyl or a C₁ to C₆ alkoxycarbonyl,
R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₁ to C₆ alkylcarbonyl, a C₃ to C₆ cycloalkylcarbonyl, a C₁ to C₆ alkoxycarbonyl, -C(O)N(R³²)R³¹, a C₁ to C₆ alkylsulfonyl, a di(C₁ to C₆ alkyl)-aminosulfonyl, phenylsulfonyl which may be substituted by (Z¹)ₚ₁, a di(C₁ to C₆ alkyl)-phosphoryl, a di(C₁ to C₆ alkyl)thiophosphoryl, a tri(C₁ to C₄ alkyl)silyl or phenyl which may be substituted by (Z¹)ₚ₁,
R²⁹ represents a hydrogen atom or a C₁ to C₆ alkyl,
R³⁰ represents a C₁ to C₆ alkyl, a C₁ to C₆ haloalkyl, a hydroxy(C₁ to C₄)alkyl, a C₁ to C₄ alkoxy(C₁ to C₄)alkyl, a C₁ to C₄ alkylthio(C₁ to C₄)alkyl, a C₁ to C₄ alkylcarbonyl(C₁ to C₄)alkyl, a C₁ to C₄ alkoxycarbonyl(C₁ to C₄)alkyl, a di(C₁ to C₄ alkyl)-aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a C₃ to C₆ alkenyl, a C₃ to C₆ alkynyl, a C₁ to C₆ alkylthio, phenyl which may be substituted by (Z¹)ₚ₁, L-21 or L-45,
R³¹ represents a C₁ to C₆ alkyl, a C₁ to C₄ alkylthio(C₁ to C₄)alkyl, a phenyl(C₁ to C₄)alkyl which may be substituted by (Z¹)ₚ₁, a C₃ to C₆ cycloalkyl, a C₃ to C₆ alkenyl or phenyl which may be substituted by (Z¹)ₚ₁,
R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, and the alkylene chain at this time may contain one oxygen atom or sulfur atom,
R³³ represents a C₁ to C₆ alkyl or a phenyl(C₁ to C₄)alkyl which may be substituted by (Z¹)ₚ₁,
R³⁴ represents a hydrogen atom or a C₁ to C₆ alkyl,
m represents an integer of 0 to 3,
n represents an integer of 0 to 3,
q2 represents an integer of 0 to 3,
q3 represents an integer of 0 to 2, and
q4 represents an integer of 0 to 2.

4. The substituted benzanilide compound or a salt thereof according to Claim 3, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, or a cycloalkyl ring represented by the formula G-71,
X represents a halogen atom, nitro, a C₁ to C₄ alkyl, a C₁ to C₄ haloalkyl, a C₁ to C₄ alkoxy, a C₁ to C₄ haloalkoxy, a C₁ to C₄ alkylthio, a C₁ to C₄ haloalkylthio, a C₁ to C₄ alkylsulfinyl, a C₁ to C₄ haloalkylsulfinyl, a C₁ to C₄ alkylsulfonyl or a C₁ to C₄ haloalkylsulfonyl, and when m represents 2, each of Xs may be the same with each other or may be different from each other,
Y represents a halogen atom, a C₁ to C₄ alkyl, a C₁ to C₄ haloalkyl, a C₁ to C₄ alkoxy or a C₁ to C₄ alkylthio, and when n represents 2, each of Ys may be the same with each other or may be different from each other,
R² represents a hydrogen atom or a C₁ to C₆ alkyl,
R³ represents a C₁ to C₈ alkyl, a (C₁ to C₈)alkyl which may be optionally substituted by R¹⁸, a C₃ to C₈ alkenyl or a C₃ to C₈ alkynyl,
R⁴ represents a hydrogen atom, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₃ to C₈ cycloalkyl, a C₃ to C₈ halocycloalkyl, phenyl which may be substituted by (Z²)ₚ₁, 2-naphthyl, L-1 to L-4, L-15 to L-17, L-19, L-20, L-22, L-23, L-45 to L-47, L-50, M-4, M-5, M-8, M-9, M-14 to M-18 or M-19,
and when G represents a cycloalkyl ring represented by the formula G-71, R⁴ may further represent a halogen atom, a C₁ to C₆ alkoxy, a C₁ to C₆ haloalkoxy, a C₁ to C₆ alkylthio, a C₁ to C₆ haloalkylthio, a C₁ to C₆ alkylsulfonyl or a C₁ to C₆ haloalkylsulfonyl,
R⁵ represents
(a). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a di(C₁ to C₆ alkyl)amino, pyrrolidin-1-yl, oxazolidin-3-yl, thiazolidin-3-yl, piperidin-1-yl, morpholin-1-yl, -C(R⁹)=NOR¹¹, phenyl which may be substituted by (Z²)ₚ₁, 1-naphthyl, 2-naphthyl, L, M-4, M-5, M-8, M-9, M-14 to M-18 or M-19, when G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
(b). a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, -C(R⁹)=NOR¹¹, -Si(R¹³)(R¹⁴)R¹², phenyl which may be substituted by (Z²)ₚ₁, 1-naphthyl, 2-naphthyl, L, M-4, M-5, M-8, M-9, M-14 to M-18 or M-19, when G represents a cycloalkyl ring represented by the formula G-71, and R⁴ represents a hydrogen atom, a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, -C(R⁹)=NOR¹¹, -Si(R¹³)(R¹⁴)R¹², M-4, M-5, M-8, M-9, M-14 to M-18 or M-19, when G represents a cycloalkyl ring represented by the formula G-71, and R⁴ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfonyl, a C₁ to C₆ haloalkylsulfonyl, M-4, M-5, M-8, M-9, M-14 to M-18 or M-19,
(d). a hydrogen atom, a halogen atom, cyano, a C₁ to C₆ alkyl or a C₁ to C₆ haloalkyl, when G represents a cycloalkyl ring represented by the formula G-71, and R⁴ represents phenyl which may be substituted by (Z²)ₚ₁, 1-naphthyl, 2-naphthyl, L-1 to L-4, L-15 to L-17, L-19, L-20, L-22, L-23, L-45 to L-47 or L-50,
R^{6a}, R^{6b}, R^{6c} and R^{6d} each represent a hydrogen atom,
R^{6e} represents a C₁ to C₆ alkyl, a C₁ to C₆ alkylsulfonyl, a C₁ to C₆ alkoxycarbonyl, a di(C₁ to C₆ alkyl)phosphoryl or phenyl which may be substituted by (Z²)ₚ₁,
R⁶ⁱ represents a hydrogen atom, a halogen atom or a C₁ to C₆ alkyl,
R^{6j} and R^{6k} each independently represent a hydrogen atom, a halogen atom, cyano or methyl,
R⁹ represents a C₁ to C₆ alkyl, a C₁ to C₆ haloalkyl, a C₃ to C₈ cycloalkyl or phenyl which may be substituted by (Z¹)ₚ₁,
R¹¹ represents a C₁ to C₆ alkyl or a C₁ to C₆ haloalkyl, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group,
R¹⁸ represents a -OR²⁸, -N(R²⁹)R²⁸, -S(O)ᵣR³⁰, -C(R³⁴)=NOH, -C(R³⁴)=NOR³³ or phenyl which may be substituted by (Z¹)ₚ₁,
R²⁸ represents a hydrogen atom, a C₁ to C₆ alkyl, a C₁ to C₆ alkylcarbonyl, a C₁ to C₆ alkoxycarbonyl, a C₁ to C₆ alkylaminocarbonyl, a C₃ to C₆ cycloalkylaminocarbonyl, a di(C₁ to C₆ alkyl)aminocarbonyl, phenylaminocarbonyl which may be substituted by (Z¹)ₚ₁, a phenyl(C₁ to C₄)alkylaminocarbonyl which may be substituted by (Z¹)ₚ₁, a C₁ to C₆ alkylsulfonyl, a di(C₁ to C₆ alkyl)thiophosphoryl or phenyl which may be substituted by (Z¹)ₚ₁,
R³⁰ represents a C₁ to C₆ alkyl, a C₁ to C₆ alkylthio, phenyl which may be substituted by (Z¹)ₚ₁ or L-45,
R³³ represents a C₁ to C₆ alkyl,
R³⁴ represents a hydrogen atom,
m represents an integer of 0 to 2, and
n represents an integer of 0 to 2.

5. The substituted benzanilide compound or a salt thereof according to any one of Claims 1 to 4, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R⁵ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, pyrrolidin-1-yl, oxazolidin-3-yl, thiazolidin-3-yl, piperidin-1-yl, morpholin-1-yl, -C(R⁹)=NOR¹¹, phenyl which may be substituted by (Z²)ₚ₁, 1-naphthyl, 2-naphthyl, L, M-4, M-5, M-8, M-9, M-14 to M-18 or M-19.

6. The substituted benzanilide compound or a salt thereof according to any one of Claims 1 to 4, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, and
R⁴ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₃ to C₈ cycloalkyl, a C₃ to C₈ halocycloalkyl, phenyl which may be substituted by (Z²)ₚ₁, 2-naphthyl, L-1 to L-4, L-15 to L-17, L-19, L-20, L-22, L-23, L-45 to L-47, L-50, M-4, M-5, M-8, M-9, M-14 to M-18 or M-19.

7. The substituted benzanilide compound or a salt thereof according to any one of Claims 1 to 3, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R³ represents a C₁ to C₈ alkyl, a C₁ to C₈ haloalkyl, a cyano(C₁ to C₈)alkyl, a C₃ to C₆ cycloalkyl(C₁ to C₈)alkyl, a tri(C₁ to C₄ alkyl)silyl(C₁ to C₈)alkyl, a phenyl(C₁ to C₈)alkyl which may be substituted by (Z¹)ₚ₁, a (L-1)-(C₁ to C₈)alkyl, a (L-2)-(C₁ to C₈)alkyl, a (L-3)-(C₁ to C₈)alkyl, a (L-4)-(C₁ to C₈)alkyl, a (L-45)-(C₁ to C₈)alkyl, a (L-46)-(C₁ to C₈)alkyl, a (L-47)-(C₁ to C₈)alkyl, a C₃ to C₈ cycloalkyl, a C₃ to C₈ alkenyl, a phenyl(C₃ to C₆)alkenyl which may be substituted by (Z¹)ₚ₁, a C₃ to C₈ alkynyl, a phenyl(C₃ to C₆)-alkynyl which may be substituted by (Z¹)ₚ₁, a naphthalen-1-yl-(C₃ to C₆)alkynyl, a naphthalen-2-yl-(C₃ to C₆)alkynyl, a (L-1)-(C₃ to C₆)alkynyl, a (L-2)-(C₃ to C₆)alkynyl, a (L-3)-(C₃ to C₆)alkynyl, a (L-4)-(C₃ to C₆)alkynyl, a (L-45)-(C₃ to C₆)alkynyl, a (L-46)-(C₃ to C₆)alkynyl or a (L-47)-(C₃ to C₆)alkynyl, or R² and R³ are combined to form a C₂ to C₆ alkylene chain, so that they may form a 3- to 7-membered ring with the nitrogen atoms to which they are bonded.

8. The substituted benzanilide compound or a salt thereof according to Claim 4, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23, and
R³ represents a C₁ to C₈ alkyl, a phenyl(C₁ to C₈)alkyl which may be substituted by (Z¹)ₚ₁, a C₃ to C₈ alkenyl or a C₃ to C₈ alkynyl.

9. The substituted benzanilide compound or a salt thereof according to any one of Claims 1 to 3, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R³ represents a (C₁ to C₈)alkyl which may be optionally substituted by R¹⁸, a C₁ to C₆ alkylaminocarbonyl(C₃ to C₆)alkenyl, M-4, M-5, M-8, M-9, M-13 to M-19, M-21 or M-22, or R² and R³ are combined to form a C₂ to C₆ alkylene chain containing one oxygen atom or sulfur atom, so that they may form a 3- to 7-membered ring with a nitrogen atom(s) to which they are bonded,
R⁴ represents a hydrogen atom, a C₁ to C₆ alkyl or a C₁ to C₆ haloalkyl,
R⁵ represents a hydrogen atom, a halogen atom, cyano, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl or a di(C₁ to C₆ alkyl)amino,
R¹⁸ represents -OR²⁸, -N(R²⁹)R²⁸, -S(O)ᵣR³⁰, a C₁ to C₆ alkoxycarbonyl, a C₁ to C₆ alkylaminocarbonyl, -C(O)N(R³²)R³¹, -C(R³⁴)=NOH, -C(R³⁴)=NOR³³ or M,
R²⁸ represents a hydrogen atom, a C₁ to C₆ alkyl, a C₁ to C₆ haloalkyl, a C₁ to C₄ alkoxy(C₁ to C₄)alkyl, a C₁ to C₄ alkylthio(C₁ to C₄)alkyl, a phenyl(C₁ to C₄)alkyl which may be substituted by (Z¹)ₚ₁, a C₁ to C₆ alkylcarbonyl, a C₃ to C₆ cycloalkylcarbonyl, a C₁ to C₆ alkoxycarbonyl, -C(O)N(R³²)R³¹, a C₁ to C₆ alkylsulfonyl, a di(C₁ to C₆ alkyl)-aminosulfonyl, phenylsulfonyl which may be substituted by (Z¹)ₚ₁, a di(C₁ to C₆ alkyl)-phosphoryl, a di(C₁ to C₆ alkyl)thiophosphoryl, a tri(C₁ to C₄ alkyl)silyl or phenyl which may be substituted by (Z¹)ₚ₁,
R²⁹ represents a hydrogen atom or a C₁ to C₆ alkyl,
R³⁰ represents a C₁ to C₆ alkyl, a C₁ to C₆ haloalkyl, a hydroxy(C₁ to C₄)alkyl, a C₁ to C₄ alkoxy(C₁ to C₄)alkyl, a C₁ to C₄ alkylthio(C₁ to C₄)alkyl, a C₁ to C₄ alkylcarbonyl(C₁ to C₄)alkyl, a C₁ to C₄ alkoxycarbonyl(C₁ to C₄)alkyl, a di(C₁ to C₄ alkyl)aminocarbonyl(C₁ to C₄)alkyl, a tri(C₁ to C₄ alkyl)silyl(C₁ to C₄)alkyl, a phenyl(C₁ to C₄)alkyl which may be substituted by (Z¹)ₚ₁, a C₃ to C₆ alkenyl, a C₃ to C₆ alkynyl, a C₁ to C₆ alkylthio, phenyl which may be substituted by (Z¹)ₚ₁, L-21 or L-45,
R³¹ represents a C₁ to C₆ alkyl, a C₁ to C₄ alkylthio(C₁ to C₄)alkyl, a phenyl(C₁ to C₄)alkyl which may be substituted by (Z¹)ₚ₁, a C₃ to C₆ cycloalkyl, a C₃ to C₆ alkenyl or phenyl which may be substituted by (Z¹)ₚ₁,
R³² represents a hydrogen atom or a C₁ to C₆ alkyl, or R³¹ and R³² are combined to form a C₂ to C₅ alkylene chain, so that they may form a 3- to 6-membered ring with the nitrogen atom(s) to which they are bonded, and further the alkylene chain may contain one oxygen atom or sulfur atom,
R³³ represents a C₁ to C₆ alkyl or a phenyl(C₁ to C₄)alkyl which may be substituted by (Z¹)ₚ₁,
R³⁴ represents a hydrogen atom or a C₁ to C₆ alkyl,
q2 represents an integer of 0 to 3,
q3 represents an integer of 0 to 2, and
q4 represents an integer of 0 to 2.

10. The substituted benzanilide compound or a salt thereof according to Claim 4, wherein G represents a non-aromatic heterocyclic ring represented by either one of the formula G-4, the formula G-5, the formula G-6, the formula G-7, the formula G-11, the formula G-13, the formula G-14, the formula G-15, the formula G-17, the formula G-18, the formula G-21, the formula G-22 or the formula G-23,
R³ represents a C₁ to C₈ alkyl which may be optionally substituted by -OR²⁸, -N(R²⁹)R²⁸, -S(O)ᵣR³⁰, -C(R³⁴)=NOH or -C(R³⁴)=NOR³³,
R⁴ represents a hydrogen atom, a C₁ to C₆ alkyl or a C₁ to C₆ haloalkyl,
R⁵ represents a hydrogen atom, a halogen atom, cyano, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl or a di(C₁ to C₆ alkyl)amino,
R²⁸ represents a hydrogen atom, a C₁ to C₆ alkyl, a C₁ to C₆ alkylcarbonyl, a C₁ to C₆ alkoxycarbonyl, a C₁ to C₆ alkylaminocarbonyl, a C₃ to C₆ cycloalkylaminocarbonyl, a di(C₁ to C₆ alkyl)aminocarbonyl, phenylaminocarbonyl which may be substituted by (Z¹)ₚ₁, a phenyl(C₁ to C₄)alkylaminocarbonyl which may be substituted by (Z¹)ₚ₁, a C₁ to C₆ alkylsulfonyl, a di(C₁ to C₆ alkyl)thiophosphoryl or phenyl which may be substituted by (Z¹)ₚ₁,
R³⁰ represents a C₁ to C₆ alkyl, a C₁ to C₆ alkylthio, phenyl which may be substituted by (Z¹)ₚ₁, or L-45,
R³³ represents a C₁ to C₆ alkyl, and
R³⁴ represents a hydrogen atom.

11. The substituted benzanilide compound or a salt thereof according to any one of Claims 1 to 5, and 7 to 8, wherein G represents a non-aromatic heterocyclic ring represented by either one of the formula G-7, the formula G-13, the formula G-14, the formula G-15, the formula G-17 or the formula G-18,
X represents a halogen atom, nitro, a C₁ to C₄ alkyl, a C₁ to C₄ haloalkyl, a C₁ to C₄ alkylthio, a C₁ to C₄ alkylsulfinyl or a C₁ to C₄ alkylsulfonyl, and when m represents 2, each of Xs may be the same with each other or may be different from each other,
Y represents a halogen atom or a C₁ to C₄ alkyl, and when n represents 2, each of Ys may be the same with each other or may be different from each other,
R⁴ represents a C₁ to C₆ alkyl or a C₁ to C₆ haloalkyl,
R⁵ represents phenyl which may be substituted by (Z²)ₚ₁, 1-naphthyl, 2-naphthyl, L-1 to L-5, L-8 to L-24, L-28 to L-36, L-39, L-41, L-42, L-45 to L-47 or L-50.

12. The substituted benzanilide compound or a salt thereof according to any one of Claims 1 to 4 and 6 to 8, wherein G represents a non-aromatic heterocyclic ring represented by either one of the formula G-7, the formula G-13, the formula G-14, the formula G-15, the formula G-17 or the formula G-18,
X represents a halogen atom, nitro, a C₁ to C₄ alkyl, a C₁ to C₄ haloalkyl, a C₁ to C₄ alkylthio, a C₁ to C₄ alkylsulfinyl or a C₁ to C₄ alkylsulfonyl, and when m represents 2, each of Xs may be the same with each other or may be different from each other,
Y represents a halogen atom or a C₁ to C₄ alkyl, and when n represents 2, each of Ys may be the same with each other or may be different from each other,
R⁴ represents phenyl which may be substituted by (Z²)ₚ₁, 2-naphthyl, L-1 to L-4, L-15 to L-17, L-19, L-20, L-22, L-23, L-45, L-46 or L-47,
R⁵ represents a halogen atom, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ alkylsulfinyl, a C₁ to C₆ alkylsulfonyl or a di(C₁ to C₆ alkyl)amino.

13. The substituted benzanilide compound or a salt thereof according to Claim 4, wherein G represents a cycloalkyl ring represented by the formula G-71,
X represents a halogen atom, nitro, a C₁ to C₄ alkyl, a C₁ to C₄ haloalkyl, a C₁ to C₆ alkylthio, a C₁ to C₆ alkylsulfinyl or a C₁ to C₆ alkylsulfonyl, and when m represents 2, each of Xs may be the same with each other or may be different from each other,
Y represents a halogen atom or a C₁ to C₆ alkyl, and when n represents 2, each of Ys may be the same with each other or may be different from each other,
R³ represents a C₁ to C₈ alkyl which may be optionally substituted by -OR²⁸, -N(R²⁹)R²⁸, -S(O)ᵣR³⁰, -C(R³⁴)=NOH or -C(R³⁴)=NOR³³,
R⁴ represents a hydrogen atom, a halogen atom, a C₁ to C₆ alkyl or a C₁ to C₆ haloalkyl,
R⁵ represents phenyl which may be substituted by (Z²)ₚ₁, 1-naphthyl, 2-naphthyl, L-1 to L-4, L-15 to L-17, L-19, L-20, L-22, L-23, L-45, L-46 or L-47,
R²⁸ represents a hydrogen atom, a C₁ to C₆ alkyl, a C₁ to C₆ alkylcarbonyl, a C₁ to C₆ alkoxycarbonyl, a C₁ to C₆ alkylaminocarbonyl, a C₃ to C₆ cycloalkylaminocarbonyl, a di(C₁ to C₆ alkyl)aminocarbonyl, phenylaminocarbonyl which may be substituted by (Z¹)ₚ₁, a phenyl(C₁ to C₄)alkylaminocarbonyl which may be substituted by (Z¹)ₚ₁, a C₁ to C₆ alkylsulfonyl, a di(C₁ to C₆ alkyl)thiophosphoryl or phenyl which may be substituted by (Z¹)ₚ₁,
R³⁰ represents a C₁ to C₆ alkyl, a C₁ to C₆ alkylthio, phenyl which may be substituted by (Z¹)ₚ₁ or L-45,
R³³ represents a C₁ to C₆ alkyl, and
R³⁴ represents a hydrogen atom.

14. The substituted benzanilide compound or a salt thereof according to Claim 4, wherein G represents a cycloalkyl ring represented by the formula G-71,
R⁴ represents phenyl which may be substituted by (Z²)ₚ₁, 2-naphthyl, L-1 to L-4, L-15 to L-17, L-19, L-20, L-22, L-23, L-45, L-46 or L-47, and
R⁵ represents a hydrogen atom, halogen atom or a C₁ to C₆ alkyl.

15. The substituted benzanilide compound or a salt thereof according to Claim 14, wherein X represents a halogen atom, nitro, a C₁ to C₄ alkyl, a C₁ to C₄ haloalkyl, a C₁ to C₆ alkylthio, a C₁ to C₆ alkylsulfinyl or a C₁ to C₆ alkylsulfonyl, and when m represents 2, each of Xs may be the same with each other or may be different from each other,
Y represents a halogen atom or a C₁ to C₆ alkyl, and when n represents 2, each of Ys may be the same with each other or may be different from each other,
R⁵ represents a hydrogen atom,
R⁶ⁱ represents a hydrogen atom, and
R^{6j} and R^{6k} each independently represent a hydrogen atom, a halogen atom or cyano.

16. An N-substituted phenyl-3-nitrophthalimide or a substituted aniline represented by the formula (2) or the formula (3): 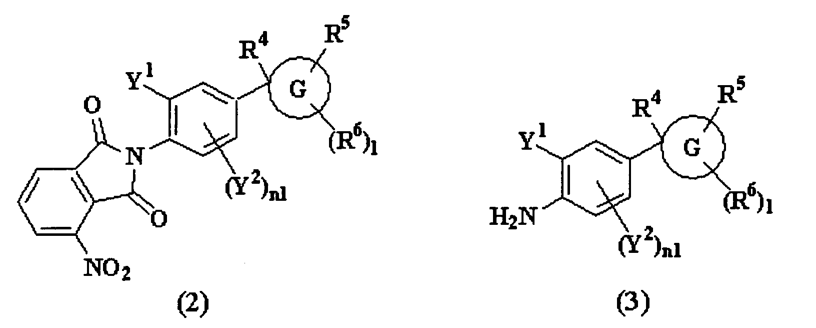 [wherein G represents a non-aromatic heterocyclic ring represented by either of the formula G-4, the formula G-5, the formula G-7, the formula G-11, the formula G-13, the formula G-14, the formula G-15, the formula G-17, the formula G-18 or the formula G-23, or a cycloalkyl ring represented by the formula G-71, 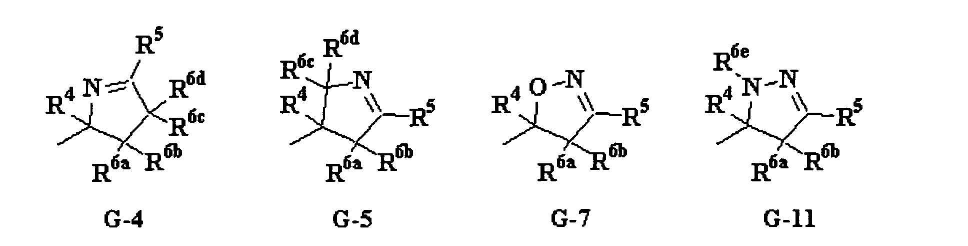 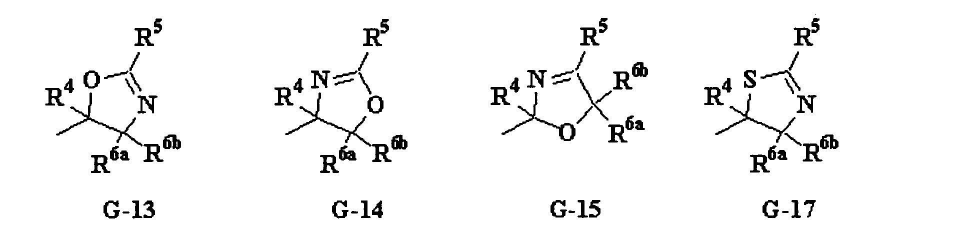 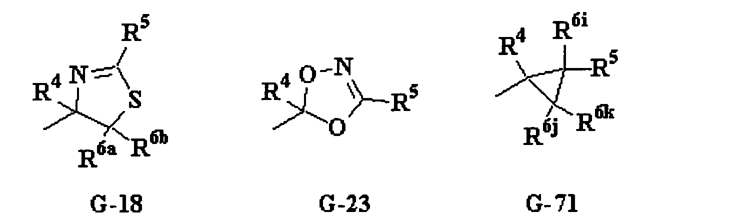 Y¹ represents a halogen atom, a C₁ to C₆ alkyl, a C₁ to C₆ haloalkyl, a hydroxy(C₁ to C₆)alkyl, a C₁ to C₃ alkoxy(C₁ to C₃)alkyl, a C₁ to C₆ alkoxy, a C₁ to C₆ haloalkoxy, a C₁ to C₆ alkylthio or a C₁ to C₆ haloalkylthio,
Y² represents a halogen atom, a C₁ to C₆ alkyl, a C₁ to C₆ alkoxy or a C₁ to C₆ alkylthio, and when n1 represents 2, each of Y²s may be the same with each other or may be different from each other,
R⁴ represents
(a). a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a non-aromatic heterocyclic ring represented by either of the formula G-4, the formula G-5, the formula G-7, the formula G-11, the formula G-13, the formula G-15, the formula G-17, the formula G-18 or the formula G-23,
(b). a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a non-aromatic heterocyclic ring represented by the formula G-14,
(c). a halogen atom, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a cycloalkyl ring represented by the formula G-71,
R⁵ represents
(a). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a di(C₁ to C₆ alkyl)amino, pyrrolidin-1-yl, oxazolidin-3-yl, thiazolidin-3-yl, piperidin-1-yl, morpholin-1-yl, -C(R⁹)=NOR¹¹, Q, 1-naphthyl, 2-naphthyl, L, M-4, M-5, M-8, M-9, M-14 to M-18 or M-19, when G represents a non-aromatic heterocyclic ring represented by either of the formula G-4, the formula G-5, the formula G-7, the formula G-11, the formula G-13, the formula G-14, the formula G-15, the formula G-17, the formula G-18 or the formula G-23,
(b). a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, -C(R⁹)=NOR¹¹, -Si(R¹³)(R¹⁴)R¹², Q, 1-naphthyl, 2-naphthyl, L-1 to L-4, L-8 to L-13, L-15 to L-23, L-30 to L-35, L-45 to L-53, M-4, M-5, M-8, M-9, M-14 to M-18 or M-19, when G represents a cycloalkyl ring represented by the formula G-71, and R⁴ represents a halogen atom, a C₁ to C₆ alkyl or a C₁ to C₆ haloalkyl,
(c). a hydrogen atom, a halogen atom, cyano, a C₁ to C₆ alkyl, a C₁ to C₆ haloalkyl, a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, -C(R⁹)=NOR¹¹, -Si(R¹³)(R¹⁴)R¹², M-4, M-5, M-8, M-9, M-14 to M-18 or M-19, when G represents a cycloalkyl ring represented by the formula G-71, and R⁴ represents a C₃ to C₆ cycloalkyl(C₁ to C₄)alkyl, a C₁ to C₄ alkoxy(C₁ to C₄)alkyl, a C₁ to C₄ haloalkoxy(C₁ to C₄)alkyl, a C₁ to C₄ alkylthio(C₁ to C₄)alkyl, a C₁ to C₄ haloalkylthio(C₁ to C₄)alkyl, a C₁ to C₄ alkylsulfinyl(C₁ to C₄)alkyl, a C₁ to C₄ haloalkylsulfinyl(C₁ to C₄)alkyl, a C₁ to C₄ alkylsulfonyl(C₁ to C₄)alkyl, a C₁ to C₄ haloalkylsulfonyl(C₁ to C₄)alkyl, a C₁ to C₄ haloalkoxy(C₁ to C₄)haloalkyl, a C₃ to C₈ cycloalkyl, a C₃ to C₈ halocycloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, M-4, M-5, M-8, M-9, M-14 to M-18 or M-19,
(d). a hydrogen atom, a halogen atom, cyano, a C₁ to C₆ alkyl or a C₁ to C₆ haloalkyl, when G represents a cycloalkyl ring represented by the formula G-71, and R⁴ represents Q, 1-naphthyl, 2-naphthyl, L-1 to L-4, L-8 to L-13, L-15 to L-23, L-30 to L-35, L-45 to L-52 or L-53,
R^{6a}, R^{6b}, R^{6c} and R^{6d} each represent a hydrogen atom,
R^{6e} represents a C₁ to C₆ alkyl, a C₁ to C₆ alkylsulfonyl, a C₁ to C₆ alkoxycarbonyl, a di(C₁ to C₆ alkyl)phosphoryl or Q,
R⁶ⁱ represents a hydrogen atom, a halogen atom or a C₁ to C₆ alkyl,
R^{6j} and R^{6k} each independently represent a hydrogen atom, a halogen atom, cyano or methyl,
Q represents a phenyl group which may be substituted represented by either of Q-1 to Q-11, 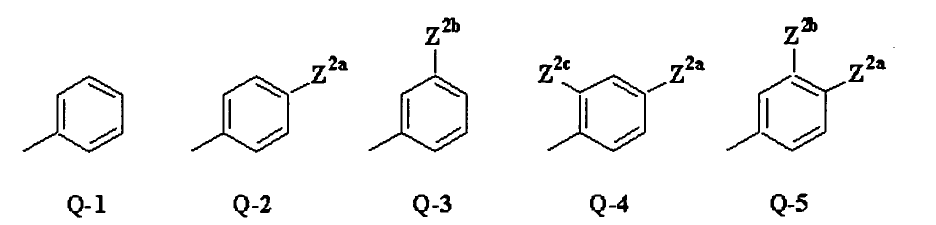 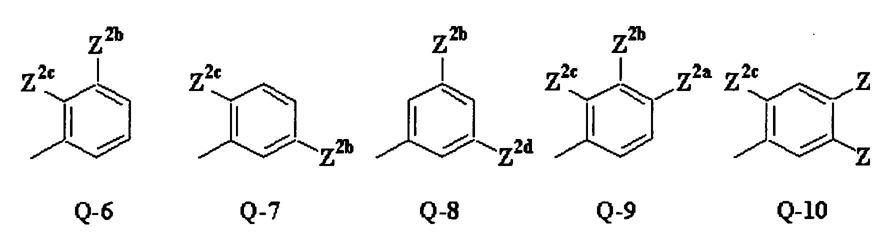 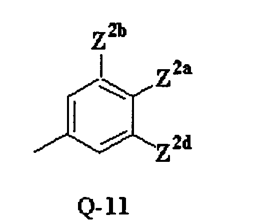 L represents an aromatic heterocyclic ring represented by either of the formula L-1 to the formula L-58, 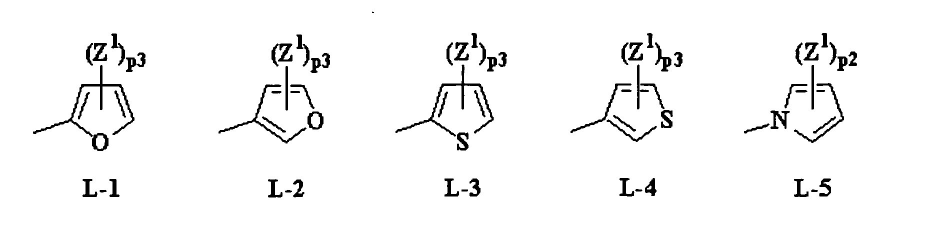 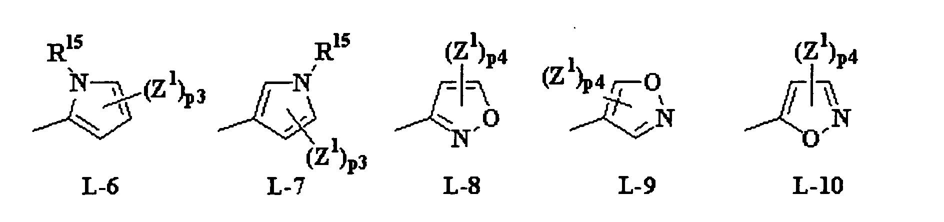 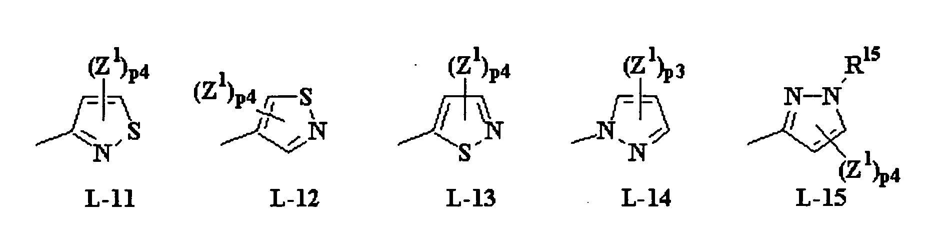 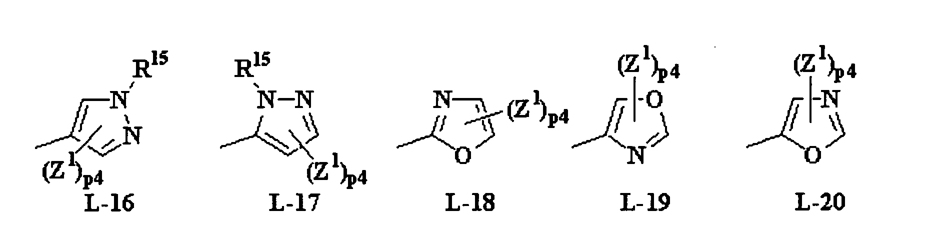 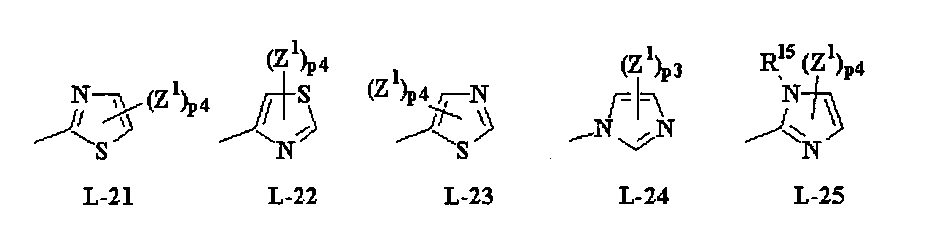 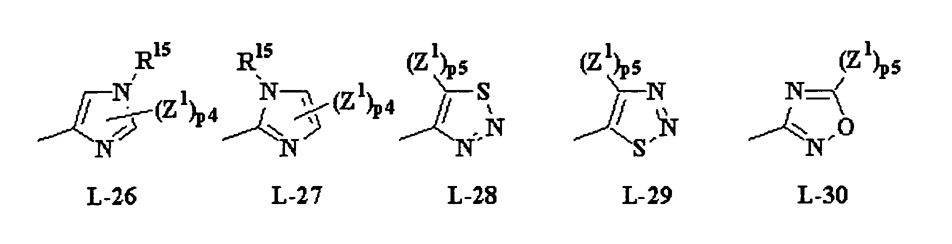 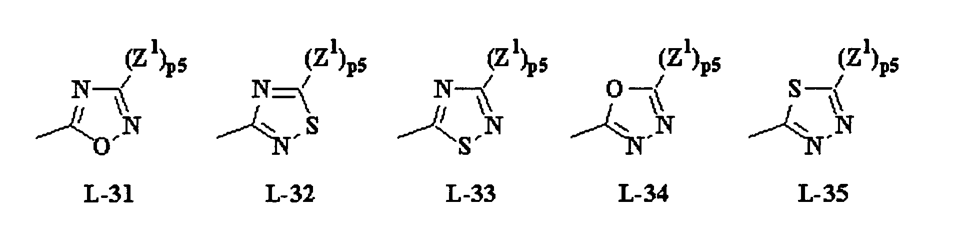 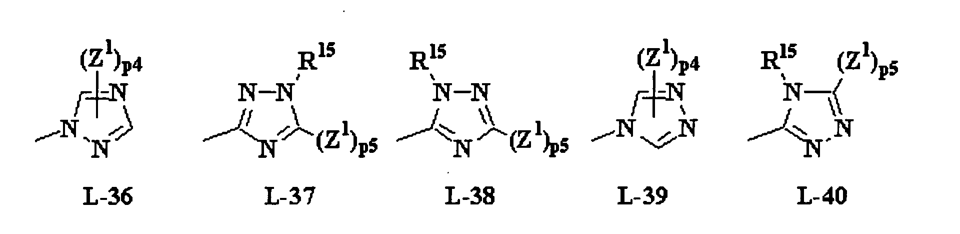 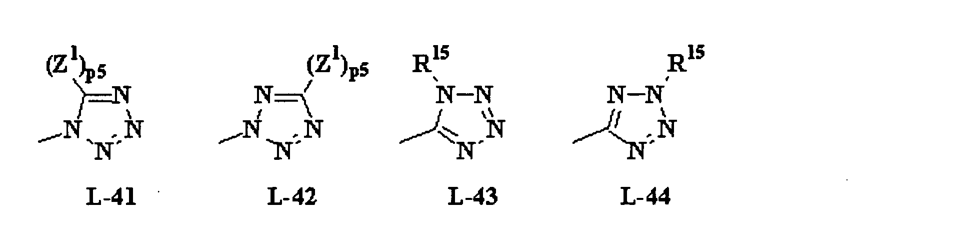 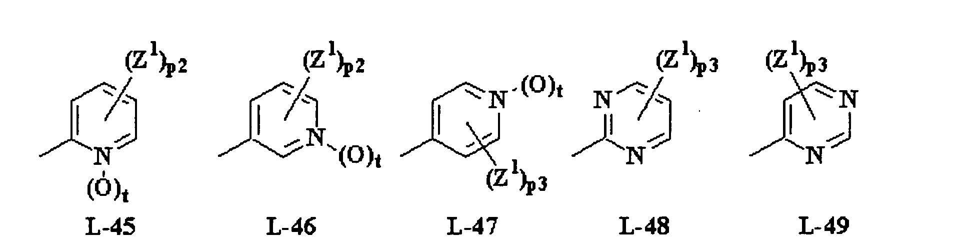 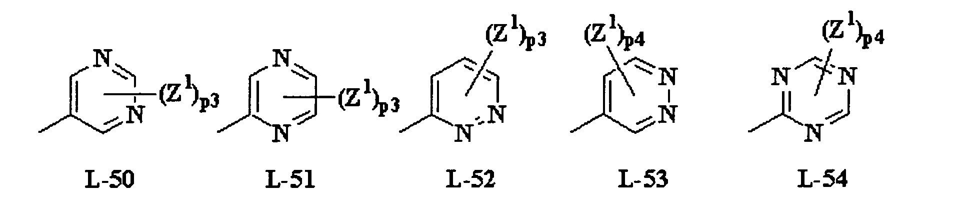 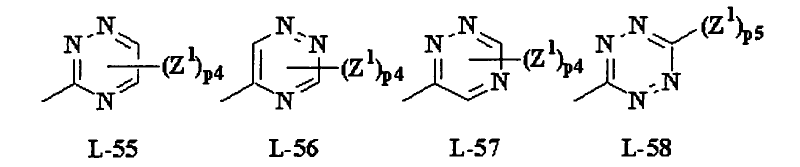 M-4, M-5, M-8, M-9, M-14 to M-18 or M-19 each represent the following saturated heterocyclic ring, 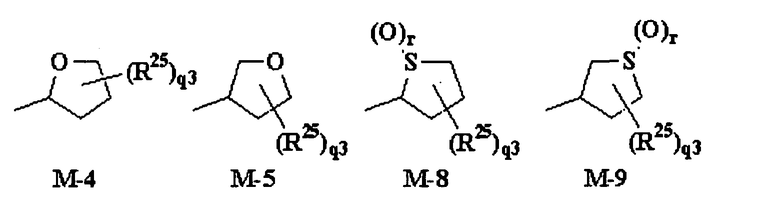 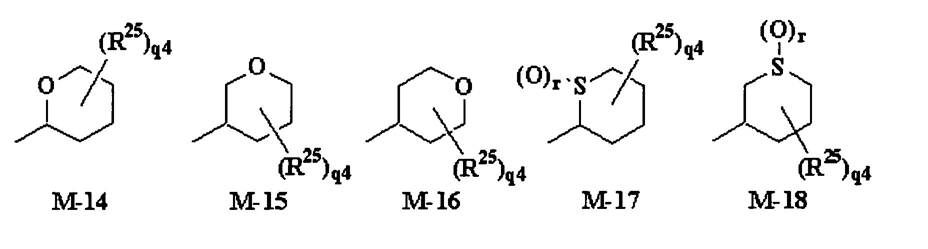 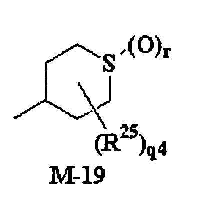 Z¹ represents a halogen atom, cyano, nitro, a C₁ to C₆ alkyl, a C₁ to C₆ haloalkyl, a C₁ to C₆ alkoxy, a C₁ to C₆ haloalkoxy, a C₁ to C₆ alkylthio, a C₁ to C₆ haloalkylthio, a C₁ to C₆ alkylsulfinyl, a C₁ to C₆ haloalkylsulfinyl, a C₁ to C₆ alkylsulfonyl, a C₁ to C₆ haloalkylsulfonyl, a C₁ to C₆ alkylamino or di(C₁ to C₆ alkyl)amino, when p1, p2; p3 or p4 represents an interger of 2 or more, each of Z¹s may be the same with each other or may be different from each other,
Z^{2a}, Z^{2b} and R^{2d} each independently represent a halogen atom, cyano, nitro, amino, a C₁ to C₆ alkyl, a C₁ to C₆ haloalkyl, a C₁ to C₃ alkoxy(C₁ to C₃)alkyl, a C₁ to C₃ haloalkoxy(C₁ to C₃)alkyl, a C₁ to C₃ alkylthio(C₁ to C₃)alkyl, a C₁ to C₃ haloalkylthio(C₁ to C₃)alkyl, a C₁ to C₃ alkylsulfinyl(C₁ to C₃)alkyl, a C₁ to C₃ haloalkylsulfinyl(C₁ to C₃)alkyl, a C₁ to C₃ alkylsulfonyl(C₁ to C₃)alkyl, a C₁ to C₃ haloalkylsulfonyl(C₁ to C₃)alkyl, a cyano(C₁ to C₆)alkyl, a C₃ to C₈ cycloalkyl, a C₃ to C₈ halocycloalkyl, a C₁ to C₆ alkoxy, a C₁ to C₆ haloalkoxy, a C₁ to C₆ alkylsulfonyloxy, a C₁ to C₆ haloalkylsulfonyloxy, phenoxy which may be substituted by (Z¹)ₚ₁, 5-trifluoromethylpyridin-2-yloxy, 3-chloro-5-trifluoromethylpyridin-2-yloxy, a C₁ to C₆ alkylthio, a C₁ to C₆ haloalkylthio, a C₃ to C₈ cycloalkylthio, a C₁ to C₆ alkylsulfinyl, a C₁ to C₆ haloalkylsulfinyl, a C₃ to C₈ cycloalkylsulfinyl, a C₁ to C₆ alkylsulfonyl, a C₁ to C₆ haloalkylsulfonyl, a C₃ to C₈ cycloalkylsulfonyl, a C₁ to C₆ alkylamino, a di(C₁ to C₆ alkyl)amino, a C₁ to C₆ alkylaminosulfonyl, a di(C₁ to C₆ alkyl)aminosulfonyl, -C(O)NH₂, a C₁ to C₆ alkylaminocarbonyl, a di(C₁ to C₆)alkylaminocarbonyl, -C(S)NH₂ or a tri(C₁ to C₆ alkyl)silyl,
and further Z^{2a} and Z^{2b} or Z^{2a} and Z^{2d} may form -OCF₂O-, so that they may form a 5-membered ring with the carbon atoms to which they are bonded,
Z^{2c} represents a halogen atom, a C₁ to C₆ alkyl or a C₁ to C₆ haloalkyl,
R⁹ represents a C₁ to C₆ alkyl, a C₁ to C₆ haloalkyl, a C₃ to C₈ cycloalkyl or phenyl which may be substituted by (Z¹)ₚ₁,
R¹¹ represents a C₁ to C₆ alkyl or a C₁ to C₆ haloalkyl, or R⁹ and R¹¹ are combined to form a C₂ to C₃ alkylene chain, so that they may form a 5-membered ring or 6-membered ring with atoms to which they are bonded, and the alkylene chain at this time may be optionally substituted by a C₁ to C₆ alkyl group,
R¹² represents a C₁ to C₆ alkyl or phenyl which may be substituted by (Z¹)ₚ₁,
R¹³ and R¹⁴ each independently represent a C₁ to C₆ alkyl,
R¹⁵ represents a C₁ to C₆ alkyl or phenyl which may be substituted by (Z¹)ₚ₁,
R²⁵ represents a C₁ to C₄ alkyl,
n1 represents an integer of 0 to 3,
p1 represents an integer of 1 to 5,
p2 represents an integer of 0 to 4,
p3 represents an integer of 0 to 3,
p4 represents an integer of 0 to 2,
p5 represents an integer of 0 or 1,
q3 represents an integer of 0 to 2,
q4 represents an integer of 0 to 2,
r represents an integer of 0 to 2,
t represents an integer of 0 or 1.]
or a salt of these.

17. A noxious organism controlling agent which comprises one or more kinds selected from the group consisting of a substituted benzanilide compound and a salt thereof according to any one of Claims 1 to 15 as an effective ingredient.

18. An agricultural chemical which comprises one or more kinds selected from the group consisting of a substituted benzanilide compound and a salt thereof according to any one of Claims 1 to 15 as an effective ingredient.

19. A insecticide or acaricide which comprises one or more kinds selected from the group consisting of a substituted benzanilide compound and a salt thereof according to any one of Claims 1 to 15 as an effective ingredient.
